# EUROPEAN PATENT SPECIFICATION

(11) **EP 4 251 619 B1**
(45) Date of publication and mention of the grant of the patent: **12.08.2026**
(21) Application number: 21843855.4
(22) Date of filing: 30.11.2021
(51) Int. Cl.: C07D 401/14, C07D 403/12, C07D 405/14, C07D 409/14, C07D 413/14, C07D 417/14, C07D 453/02, C07D 471/04, C07D 471/08, C07D 487/04, C07D 487/08, C07D 493/08, C07D 493/10, C07D 495/08, C07D 498/04

(54) **PYRROLIDINE DERIVATIVES AND METHODS OF USE**
PYRROLIDIN DERIVATE UND VERFAHREN ZU IHRER VERWENDUNG
DÉRIVÉS DE LA PYRROLIDINE ET PROCÉDÉS POUR LEUR UTILISATION

(30) Priority: 30.11.2020 US 202063119621 P
(43) Date of publication of application: 04.10.2023
(73) Proprietor: Genentech, Inc., South San Francisco, CA 94080-4990 (US)
(72) Inventor: FUHRMANN, Jakob, South San Francisco, California 94080-4990 (US); SKELTON, Nicholas John, South San Francisco, California 94080-4990 (US); DRAGOVICH, Peter Scott, South San Francisco, California 94080-4990 (US)
(74) Representative: Jochnowitz, Evan
(86) International application number: PCT/US2021/072644
(87) International publication number: WO 2022/115879

(56) References cited:
- WO-A1-2019/084026
- WO-A1-2019/084030
- WO-A2-2013/106646
- US-A1- 2014 356 322
- D. L. BUCKLEY T. AL.: "Small Molecule Inhibitors of the Interaction Between the E3 Ligase VHL and HIF1alpha", ANGEWANDTE CHEMIE, INTERNATIONAL EDITION, vol. 51, no. 46, 12 November 2012 (2012-11-12), pages 11463 - 11467, XP002728004, DOI: 10.1002/anie.201206231

## Description

### CROSS-REFERENCE TO RELATED APPLICATIONS

This application claims priority to and benefit of U.S. Provisional Patent Application No. 63/119,621, filed November 30, 2020.

### FIELD OF THE DISCLOSURE

The present disclosure relates to compounds and to methods of using such compounds in therapy. The present disclosure further relates to the use of the compounds described herein, or pharmaceutical compositions thereof, to prevent and/or treat a hyperprolifertive disorder such as cancer. The compounds described herein, or pharmaceutical compositions thereof, may also find use in therapy in the treatment of diseases or indications associated with VHL activity.

### BACKGROUND OF THE DISCLOSURE

There exists an ongoing need for small molecule compounds that may find use in therapy, such as treating a hyperproliferative disorder such as cancer. The document WO2013106646 discloses the use of proline derivatives for use in the treatment of conditions associated with Ubiquitin Ligase.

### BRIEF DESCRIPTION OF THE DISCLOSURE

Sections referring to "methods of treatment" in the context of the disclosed invention are directed to the respective compounds for use in the treatment of a medical condition.

In one aspect the present disclosure is directed to compounds of formula (I): 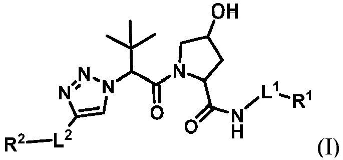 or a stereoisomers or tautomers thereof, or pharmaceutically acceptable salts of any of the foregoing, wherein:
I) L¹-R¹ is methyl;
   L² is a bond or C₁₋₁₂alkyl;
   R² is
      i) -O-C₆₋₂₀aryl, optionally substituted with one or more R^{s}; wherein R^{s} is, independently at each occurrence, halo;
      ii) 4-20 membered heterocyclyl, optionally substituted with one or more R^{s}; wherein R^{s} is, independently at each occurrence, -C(O)O-C₁₋₆alkyl;
      iii) 5-20 membered heteroaryl, wherein the 5-20 membered heteroaryl of R² is substituted with one or more R^{s}; wherein R^{s} is, independently at each occurrence, halo, C₁₋₁₂alkyl;
      iv) C₆₋₂₀cycloalkyl, optionally substituted with one or more R^{s}; wherein R^{s} is, independently at each occurrence, -C(O)O-C₁₋₆alkyl; or
II) L²-R² is cyclopropyl;
   L¹ is a bond or C₁₋₁₂alkyl; wherein the C₁₋₁₂alkyl of L¹ is optionally substituted with one or more R^{e}; wherein R^{e} is, independently at each occurrence, C₁₋₆alkyl or hydroxylC₁₋₆alkyl;
   R¹ is
      i) -L³-C₆₋₂₀aryl; wherein L³ is O or C(O)NH; the C₆₋₂₀aryl of -L³-C₆₋₂₀aryl is optionally substituted with one or more R^{s}, wherein R^{s} is, independently at each occurrence, haloC₁₋₆alkyl;
      ii) C₄₋₂₀cycloalkyl optionally substituted with one or more R^{s}; wherein R^{s} is, independently at each occurrence, halo, 5-20 membered heteroaryl, or S(O)C₁₋₆alkyl;
      iii) C₆₋₂₀aryl, optionally substituted with one or more R^{s}; wherein R^{s} is, independently at each occurrence, halo, cyano, or 5-20 membered heteroaryl;
         wherein the 5-20 membered heteroaryl of R^{s} has one or more annular atoms, independently selected from N and O; and wherein the 5-20 membered heteroaryl of R^{s} is optionally substituted with one or more R^{t}; wherein R^{t} is, independently at each occurrence, C₁₋₆alkyl;
      iv) 5-20 membered heteroaryl, optionally substituted with one or more R^{s}, wherein R^{s} is, independently at each occurrence, halo, amine, C₁₋₆alkyl, or C₆₋₂₀aryl;
         wherein the C₆₋₂₀aryl of R^{s} is optionally substituted with one or more R^{t}; wherein R^{t} is, independently at each occurrence, halo;
      v) 4-20 membered heterocyclyl, optionally substituted with one or more R^{s}, wherein R^{s} is, independently at each occurrence, halo, oxo, hydroxyl, 5-20 membered heteroaryl, C₆₋₂₀aryl, or C₁₋₆alkyl;
         wherein the C₁₋₆alkyl of R^{s} is optionally substituted with one or more R^{t}.
         wherein R^{t} is, independently at each occurrence, C₆₋₂₀aryl,
         wherein the C₆₋₂₀aryl of R^{s} is optionally substituted with one or more R^{u};
         wherein R^{u} is, independently at each occurrence, halo or C₁₋₆alkyl.

In another aspect, the present disclosure is directed to pharmaceutical compositions comprising compounds of formula (I), or stereoisomers or tautomers thereof, or pharmaceutically acceptable salts of any of the foregoing, and one or more pharmaceutically acceptable excipients.

In one aspect, the present disclosure is directed to methods of using a compound of formula (I), or one or more of the compounds described herein, or a stereoisomer or tautomer thereof, or a pharmaceutically acceptable salt of any of the foregoing, or one or more of the pharmaceutical compositions described herein, for treatment of a disease or indication associated with VHL activity.

In a futher aspect, the present disclosure is directed to methods of preventing or treating hyperproliferative disorders, anemia, or ischemia by administering to a subject in need thereof one or more of the compounds described herein, or a stereoisomer or tautomer thereof, or a pharmaceutically acceptable salt of any of the foregoing, or one or more of the pharmaceutical compositions described herein. In some embodiments, the hyperproliferative disorder is cancer and the subject is a human. In some embodiments, the hyperproliferative disorder is a solid tumor. In some such embodiments, the solid tumor is breast cancer, lung cancer, multiple myoloma or renal cell carcinoma.

In another aspect, provided is a compound of formula (II): 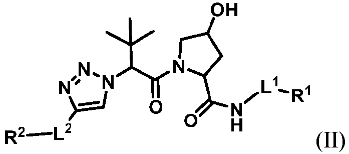 or a stereoisomer or tautomer thereof, or a pharmaceutically acceptable salt of any of the foregoing, wherein:
I) L¹-R¹ is methyl;
   L² is a bond or C₁₋₁₂alkyl;
   R² is
      i) -O-C₆₋₂₀aryl, optionally substituted with one or more R^{s}; wherein R^{s} is, independently at each occurrence, halo;
      ii) 4-20 membered heterocyclyl, optionally substituted with one or more R^{s}; wherein R^{s} is, independently at each occurrence, -C(O)O-C₁₋₆alkyl;
      iii) 5-20 membered heteroaryl, wherein the 5-20 membered heteroaryl of R² is substituted with one or more R^{s}; wherein R^{s} is, independently at each occurrence, halo or C₁₋₁₂alkyl;
      iv) C₆₋₂₀cycloalkyl, optionally substituted with one or more R^{s}; wherein R^{s} is, independently at each occurrence, -C(O)O-C₁₋₆alkyl;
      v) -N(CH₃)-C₆₋₂₀aryl;
      vi) -C₆₋₂₀aryl, optionally substituted with one or more R^{s}; wherein R^{s} is, independently at each occurrence, C₁₋₆haloalkyl; or
II) L²-R² is cyclopropyl;
   L¹ is a bond or C₁₋₁₂alkyl; wherein the C₁₋₁₂alkyl of L¹ is optionally substituted with one or more R^{e}; wherein R^{e} is, independently at each occurrence, C₁₋₆alkyl or hydroxylC₁₋₆alkyl;
   R¹ is
      i) -L³-C₆₋₂₀aryl; wherein L³ is O or C(O)NH; the C₆₋₂₀aryl of -L³-C₆₋₂₀aryl is optionally substituted with one or more R^{s}, wherein R^{s} is, independently at each occurrence, haloC₁₋₆alkyl;
      ii) C₄₋₂₀cycloalkyl optionally substituted with one or more R^{s}; wherein R^{s} is, independently at each occurrence, halo, 5-20 membered heteroaryl, or S(O)C₁₋₆alkyl;
      iii) C₆₋₂₀aryl, optionally substituted with one or more R^{s}; wherein R^{s} is, independently at each occurrence, halo, cyano, or 5-20 membered heteroaryl;
         wherein the 5-20 membered heteroaryl of R^{s} has one or more annular atoms, independently selected from N and O; and wherein the 5-20 membered heteroaryl of R^{s} is optionally substituted with one or more R^{t}; wherein R^{t} is, independently at each occurrence, C₁₋₆alkyl;
      iv) 5-20 membered heteroaryl, optionally substituted with one or more R^{s}, wherein R^{s} is, independently at each occurrence, halo, amine, C₁₋₆alkyl, or C₆₋₂₀aryl;
         wherein the C₆₋₂₀aryl of R^{s} is optionally substituted with one or more R^{t}; wherein R^{t} is, independently at each occurrence, halo; or
      v) 4-20 membered heterocyclyl, optionally substituted with one or more R^{s}, wherein R^{s} is, independently at each occurrence, halo, oxo, hydroxyl, 5-20 membered heteroaryl, C₆₋₂₀aryl, or C₁₋₆alkyl;
         wherein the C₁₋₆alkyl of R^{s} is optionally substituted with one or more R^{t}; wherein R^{t} is, independently at each occurrence, C₆₋₂₀aryl,
wherein the C₆₋₂₀aryl of R^{s} is optionally substituted with one or more R^{u}; wherein R^{u} is, independently at each occurrence, halo or C₁₋₆alkyl.

It should be understood that any methods of making or using a compound of formula (I) will also apply to a compound of formula (II).

### DETAILED DESCRIPTION OF THE DISCLOSURE

The present disclosure is directed to compounds and pharmaceutically acceptable salts thereof.

The presently disclosed subject matter will now be described more fully hereinafter. However, many modifications and other embodiments of the presently disclosed subject matter set forth herein will come to mind to one skilled in the art to which the presently disclosed subject matter pertains having the benefit of the teachings presented in the foregoing descriptions. Therefore, it is to be understood that the presently disclosed subject matter is not to be limited to the specific embodiments disclosed and that modifications and other embodiments are intended to be included within the scope of the appended claims. In other words, the subject matter described herein covers alternatives, modifications, and equivalents. In the event that one or more of the incorporated literature, patents, and similar materials differs from or contradicts this application, including but not limited to defined terms, term usage, described techniques, or the like, this application controls. Unless otherwise defined, technical and scientific terms used herein have the same meaning as commonly understood by one of ordinary skill in the art to which this disclosure belongs, applying that term in context to its use in describing the present disclosure. The terminology used in the description is for describing particular embodiments only and is not intended to be limiting of the disclosure.

### I. Definitions

The term "group" refers to a component that is covalently bound or linked to another component.

The term "covalently bound" or "covalently linked" refers to a chemical bond formed by sharing of one or more pairs of electrons.

As used herein, unless defined otherwise in a claim, the term "optionally" means that the subsequently described event(s) may or may not occur, and includes both event(s) that occur and event(s) that do not occur.

As used herein, unless defined otherwise, the phrase "optionally substituted", "substituted" or variations thereof denote an optional substitution, including multiple degrees of substitution, with one or more substituent group, for example, one, two, three, four or five. The phrase should not be interpreted as duplicative of the substitutions herein described and depicted.

The term "alkyl" as used herein refers to a saturated linear or branched-chain monovalent hydrocarbon radical of any length from one to twelve carbon atoms (C₁-C₁₂), wherein the alkyl radical may be optionally substituted independently with one or more substituents described herein. In another embodiment, an alkyl radical is one to eight carbon atoms (C₁-C₈), or one to six carbon atoms (C₁-C₆), or one to four carbon atoms (C₁-C₄), or one to three carbon atoms (C₁-C₃). Examples of alkyl groups include, but are not limited to, methyl (Me, -CH₃), ethyl (Et, -CH₂CH₃), 1-propyl (n-Pr, n-propyl, -CH₂CH₂CH₃), 2-propyl (i-Pr, i-propyl, isopropyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, tert-butyl, -C(CH₃)₃), 1-pentyl (n-pentyl, - CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH3)₂CH₂CH3), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃, 1-heptyl, 1-octyl, and the like.

The term "alkylene" as used herein refers to a saturated linear or branched-chain divalent hydrocarbon radical of any length from one to twelve carbon atoms (C₁-C₁₂), wherein the alkylene radical may be optionally substituted independently with one or more substituents described herein. In another embodiment, an alkylene radical is one to eight carbon atoms (C₁-C₈), one to six carbon atoms (C₁-C₆), or one to four carbon atoms (C₁-C₄). Examples of alkylene groups include, but are not limited to, methylene (-CH₂-), ethylene (-CH₂CH₂-), propylene (-CH₂CH₂CH₂-), and the like.

The terms "carbocycle", "carbocyclyl", "carbocyclic ring" and "cycloalkyl" refer to a monovalent non-aromatic, saturated or partially unsaturated ring having 3 to 12 carbon atoms (C₃-C₁₂) as a monocyclic ring or 7 to 12 carbon atoms as a polycyclic (e.g., bicyclic) ring. Bicyclic carbocycles having 7 to 12 atoms can be arranged, for example, as a bicyclo [4,5], [5,5], [5,6] or [6,6] system, and bicyclic carbocycles having 9 or 10 ring atoms can be arranged as a bicyclo [5,6] or [6,6] system, or as bridged systems such as bicyclo[2.2.1]heptane, bicyclo[2.2.2]octane and bicyclo[3.2.2]nonane. Polycyclic (e.g., bicyclic) rings that are overall fully saturated or partially unsaturated are encompassed within the definition of the terms "carbocycle", "carbocyclyl", "carbocyclic ring" and "cycloalkyl," incuding when one or more of the fused rings in the polycyclic ring is fully unsaturated (i.e., aromatic). Spiro moieties are also included within the scope of this definition. Examples of monocyclic carbocycles include, but are not limited to, cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, 1-cyclohex-2-enyl, 1-cyclohex-3-enyl, cyclohexadienyl, cycloheptyl, cyclooctyl, cyclononyl, cyclodecyl, cycloundecyl, cyclododecyl, indenyl, indanyl, 1,2-dihydronaphthalene, 1,2,3,4-tetrahydronaphthyl, and the like. Carbocyclyl groups are optionally substituted independently with one or more substituents described herein.

"Aryl" means a monovalent aromatic hydrocarbon radical of 6-20 carbon atoms (C₆-C₂₀) derived by the removal of one hydrogen atom from a single carbon atom of a parent aromatic ring system. Some aryl groups are represented in the exemplary structures as "Ar". Typical aryl groups include, but are not limited to, radicals derived from benzene (phenyl), substituted benzenes, naphthalene, anthracene, and the like. Aryl groups are optionally substituted independently with one or more substituents described herein.

The terms "heterocycle," "heterocyclyl" and "heterocyclic ring" are used interchangeably herein and refer to a saturated or a partially unsaturated (i.e., having one or more double and/or triple bonds within the ring) carbocyclic radical of 3 to about 20 ring atoms in which at least one ring atom is a heteroatom selected from nitrogen, oxygen, phosphorus and sulfur, the remaining ring atoms being C, where one or more ring atoms is optionally substituted independently with one or more substituents described herein. A heterocycle may be a monocycle having 3 to 7 ring members (2 to 6 carbon atoms and 1 to 4 heteroatoms selected from N, O, P, and S) or a bicycle having 7 to 10 ring members (4 to 9 carbon atoms and 1 to 6 heteroatoms selected from N, O, P, and S), for example: a bicyclo [4,5], [5,5], [5,6], or [6,6] system. Heterocycles are described in Paquette, Leo A.; "Principles of Modern Heterocyclic Chemistry" (W.A. Benjamin, New York, 1968), particularly Chapters 1, 3, 4, 6, 7, and 9; "The Chemistry of Heterocyclic Compounds, A series of Monographs" (John Wiley & Sons, New York, 1950 to present), in particular Volumes 13, 14, 16, 19, and 28; and J. Am. Chem. Soc. (1960) 82:5566. "Heterocyclyl" also includes radicals where heterocycle radicals are fused with a saturated, partially unsaturated ring, or aromatic carbocyclic or heterocyclic ring. Examples of heterocyclic rings include, but are not limited to, morpholin-4-yl, piperidin-1-yl, piperazinyl, piperazin-4-yl-2-one, piperazin-4-yl-3-one, pyrrolidin-1-yl, thiomorpholin-4-yl, S-dioxothiomorpholin-4-yl, azocan-1-yl, azetidin-1-yl, octahydropyrido[1,2-a]pyrazin-2-yl, [1,4]diazepan-1-yl, pyrrolidinyl, tetrahydrofuranyl, dihydrofuranyl, tetrahydrothienyl, tetrahydropyranyl, dihydropyranyl, tetrahydrothiopyranyl, piperidino, morpholino, thiomorpholino, thioxanyl, piperazinyl, homopiperazinyl, azetidinyl, oxetanyl, thietanyl, homopiperidinyl, oxepanyl, thiepanyl, oxazepinyl, diazepinyl, thiazepinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinylimidazolinyl, imidazolidinyl, 3-azabicyco[3.1.0]hexanyl, 3-azabicyclo[4.1.0]heptanyl, azabicyclo[2.2.2]hexanyl, 3H-indolyl quinolizinyl and N-pyridyl ureas. Spiro moieties are also included within the scope of this definition. Examples of a heterocyclic group wherein 2 ring atoms are substituted with oxo (=O) moieties are pyrimidinonyl and 1,1-dioxo-thiomorpholinyl. The heterocycle groups herein are optionally substituted independently with one or more substituents described herein.

The term "heteroaryl" refers to a monovalent aromatic radical of 5-, 6-, or 7-membered rings, and includes fused ring systems (at least one of which is aromatic) of 5-20 atoms, containing one or more heteroatoms independently selected from nitrogen, oxygen, and sulfur. Examples of heteroaryl groups are pyridinyl (including, for example, 2-hydroxypyridinyl), imidazolyl, imidazopyridinyl, 1-methyl-1H-benzo[d]imidazole, [1,2,4]triazolo[1,5-a]pyridine, pyrimidinyl (including, for example, 4-hydroxypyrimidinyl), pyrazolyl, triazolyl, pyrazinyl, tetrazolyl, furyl, thienyl, isoxazolyl, thiazolyl, oxadiazolyl, oxazolyl, isothiazolyl, pyrrolyl, quinolinyl, isoquinolinyl, tetrahydroisoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, thiadiazolyl, thiadiazolyl, furazanyl, benzofurazanyl, benzothiophenyl, benzothiazolyl, benzoxazolyl, quinazolinyl, quinoxalinyl, naphthyridinyl, and furopyridinyl. Heteroaryl groups are optionally substituted independently with one or more substituents described herein.

The heterocycle or heteroaryl groups may be carbon (carbon-linked), or nitrogen (nitrogen-linked) bonded where such is possible. By way of example and not limitation, carbon bonded heterocycles or heteroaryls are bonded at position 2, 3, 4, 5, or 6 of a pyridine, position 3, 4, 5, or 6 of a pyridazine, position 2, 4, 5, or 6 of a pyrimidine, position 2, 3, 5, or 6 of a pyrazine, position 2, 3, 4, or 5 of a furan, tetrahydrofuran, thiofuran, thiophene, pyrrole or tetrahydropyrrole, position 2, 4, or 5 of an oxazole, imidazole or thiazole, position 3, 4, or 5 of an isoxazole, pyrazole, or isothiazole, position 2 or 3 of an aziridine, position 2, 3, or 4 of an azetidine, position 2, 3, 4, 5, 6, 7, or 8 of a quinoline or position 1, 3, 4, 5, 6, 7, or 8 of an isoquinoline.

By way of example, and not limitation, nitrogen bonded heterocycles or heteroaryls are bonded at position 1 of an aziridine, azetidine, pyrrole, pyrrolidine, 2-pyrroline, 3-pyrroline, imidazole, imidazolidine, 2-imidazoline, 3-imidazoline, pyrazole, pyrazoline, 2-pyrazoline, 3-pyrazoline, piperidine, piperazine, indole, indoline, 1H-indazole, position 2 of a isoindole, or isoindoline, position 4 of a morpholine, and position 9 of a carbazole, or β-carboline.

The term "oxo" refers to "=O".

A "patient" or "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the patient, individual, or subject is a human. In some embodiments, the patient may be a "cancer patient," i.e. one who is suffering or at risk for suffering from one or more symptoms of cancer.

The terms "cancer" and "cancerous" refer to or describe the physiological condition in mammals that is typically characterized by unregulated cell growth/proliferation. A "tumor" comprises one or more cancerous cells.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration, or palliation of the disease state, and remission or improved prognosis. In some embodiments, the compounds and compositions of the subject matter described herein are used to delay development of a disease or to slow the progression of a disease. In one embodiment, treatment is performed for prophylaxis only. In another embodiment, treatment is performed during the course of clinical pathology only (i.e., not for prophylaxis). In another embodiment, treatment is performed both during the course of clinical pathology and for prophylaxis.

The term "effective" is used to describe an amount of a compound, composition or component which, when used within the context of its intended use, achieves the desired therapeutic or prophylactic result. The term effective subsumes other effective amount or effective concentration terms, including therapeutically effective amounts, which are otherwise described or used in the present application. As used herein, the term "therapeutically effective amount" means any amount which, as compared to a corresponding subject who has not received such amount, results in treatment of a disease, disorder, or side effect, or a decrease in the rate of advancement of a disease or disorder. The term also includes within its scope amounts effective to enhance normal physiological function. For use in therapy, therapeutically effective amounts of a compound of the present disclosure, as well as stereoisomers or tautomes thereof, or pharmaceutically acceptable salts of any of the foregoing, may be administered as the raw chemical. Additionally, the active ingredient may be presented as a pharmaceutical composition.

The term "pharmaceutical formulation" or "pharmaceutical composition" refers to a preparation which is in such form as to permit the biological activity of an active ingredient contained therein to be effective, and which contains no additional components which are unacceptably toxic to a subject to which the formulation would be administered.

A "pharmaceutically acceptable excipient" refers to an ingredient in a pharmaceutical formulation, other than an active ingredient, which is nontoxic to a subject. A pharmaceutically acceptable excipient includes, but is not limited to, a buffer, carrier, stabilizer, or preservative.

The phrase "pharmaceutically acceptable salt," as used herein, refers to pharmaceutically acceptable organic or inorganic salts of a molecule. Exemplary salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p toluenesulfonate, and pamoate (i.e., 1,1' methylene bis -(2 hydroxy 3 naphthoate)) salts. A pharmaceutically acceptable salt may involve the inclusion of another molecule such as an acetate ion, a succinate ion or other counterion. The counterion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counterion.

Other salts, which are not pharmaceutically acceptable, may be useful in the preparation of compounds described herein and these should be considered to form a further aspect of the subject matter. These salts, such as oxalic or trifluoroacetate, while not in themselves pharmaceutically acceptable, may be useful in the preparation of salts useful as intermediates in obtaining the compounds described herein and their pharmaceutically acceptable salts.

The term "stereoisomers" refers to compounds which have identical chemical constitution, but differ with regard to the arrangement of the atoms or groups in space.

"Diastereomer" refers to a stereoisomer with two or more centers of chirality and whose molecules are not mirror images of one another. Diastereomers have different physical properties, e.g. melting points, boiling points, spectral properties, and reactivities. Mixtures of diastereomers may separate under high resolution analytical procedures such as electrophoresis and chromatography.

"Enantiomers" refer to two stereoisomers of a compound which are non-superimposable mirror images of one another.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., Stereochemistry of Organic Compounds (1994) John Wiley & Sons, Inc., New York. Many organic compounds exist in optically active forms, i.e., they have the ability to rotate the plane of plane-polarized light. In describing an optically active compound, the prefixes D and L, or R and S, are used to denote the absolute configuration of the molecule about its chiral center(s). The prefixes d and l or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

The term "compound", as used herein, unless otherwise indicated, refers to any specific chemical compound disclosed herein and includes tautomers, regioisomers, geometric isomers, and where applicable, stereoisomers, including optical isomers (enantiomers) and other stereoisomers (diastereomers) thereof, as well as pharmaceutically acceptable salts and derivatives (including prodrug forms) thereof where applicable, in context. Within its use in context, the term compound generally refers to a single compound, but also may include other compounds such as stereoisomers, regioisomers and/or optical isomers (including racemic mixtures) as well as specific enantiomers or enantiomerically enriched mixtures of disclosed compounds. The term also refers, in context to prodrug forms of compounds which have been modified to facilitate the administration and delivery of compounds to a site of activity. It is noted that in describing the present compounds, numerous substituents and variables associated with same, among others, are described. It is understood by those of ordinary skill that molecules which are described herein are stable compounds as generally described hereunder. When the bond 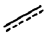 is shown, both a double bond and single bond are represented within the context of the compound shown. When a crossed double bond ( 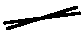 ) is shown, both the *E* and *Z* configurations are represented within the context of the compound shown; and the compound may contain the *E* isomer or the *Z* isomer or a mixture of both the *E* and *Z* isomers.

In certain embodiments disclosed herein, certain groups (e.g., alkyl, alkenyl, alkynyl, cycloalkyl, aryl, heteroaryl or heterocyclyl) are described as "substituted". In some such embodiments, the "substituted" group may be substituted with 1, 2, 3, 4, 5, or more substituents, as indicated herein. In certain embodiments, alkyl, alkenyl, alkynyl, cycloalkyl, aryl, heteroaryl or heterocyclyl may be substituted with one or more substituents independently selected from, but not limited to, alkyl, alkenyl, alkynyl, cycloalkyl heterocyclyl, aryl, heteroaryl, halo (i.e., halogen), haloalkyl, oxo, OH, CN, -O-alkyl, S-alkyl, NH-alkyl, N(alkyl)₂, O-cycloalkyl, S-cycloalkyl, NH-cycloalkyl, N(cycloalkyl)₂, N(cycloalkyl)(alkyl), NH₂, SH, SO₂-alkyl, P(O)(O-alkyl)(alkyl), P(O)(O-alkyl)₂, Si(OH)₃, Si(alkyl)₃, Si(OH)(alkyl)₂, CO-alkyl, CO₂H, NO₂, SF₅, SO₂NH-alkyl, SO₂N(alkyl)₂, SONH-alkyl, SON(alkyl)₂, CONH-alkyl, CON(alkyl)₂, N(alkyl)CONH(alkyl), N(alkyl)CON(alkyl)₂, NHCONH(alkyl), NHCON(alkyl)₂, NHCONH₂, N(alkyl)SO₂NH(alkyl), N(alkyl)SO₂N(alkyl)₂, NHSO₂NH(alkyl), NHSO₂N(alkyl)₂, and NHSO₂NH₂.

Where a range of values is provided, it is understood that each intervening value, to the tenth of the unit of the lower limit unless the context clearly dictates otherwise (such as in the case of a group containing a number of carbon atoms in which case each carbon atom number falling within the range is provided), between the upper and lower limit of that range and any other stated or intervening value in that stated range is encompassed within the disclosure. The upper and lower limits of these smaller ranges may independently be included in the smaller ranges is also encompassed within the disclosure, subject to any specifically excluded limit in the stated range. Where the stated range includes one or both of the limits, ranges excluding either both of those included limits are also included in the disclosure.

The articles "a" and "an" as used herein and in the appended claims are used herein to refer to one or to more than one (i.e., to at least one) of the grammatical object of the article unless the context clearly indicates otherwise. By way of example, "an element" means one element or more than one element.

In the claims, as well as in the specification above, transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

It should also be understood that, in certain methods described herein that include more than one step or act, the order of the steps or acts of the method is not necessarily limited to the order in which the steps or acts of the method are recited unless the context indicates otherwise.

### II. Compounds

In one embodiment, provided herein is a compound of formula (I): 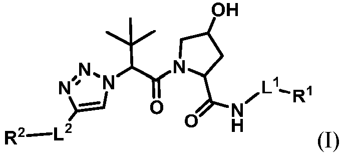 or a stereoisomer or tautomer thereof, or a pharmaceutically acceptable salt of any of the foregoing, wherein:
I) L¹-R¹ is methyl;
   L² is a bond or C₁₋₁₂alkyl;
   R² is
      i) -O-C₆₋₂₀aryl, optionally substituted with one or more R^{s}; wherein R^{s} is, independently at each occurrence, halo;
      ii) 4-20 membered heterocyclyl, optionally substituted with one or more R^{s}; wherein R^{s} is, independently at each occurrence, -C(O)O-C₁₋₆alkyl;
      iii) 5-20 membered heteroaryl, wherein the 5-20 membered heteroaryl of R² is substituted with one or more R^{s}; wherein R^{s} is, independently at each occurrence, halo, C₁₋₁₂alkyl;
      iv) C₆₋₂₀cycloalkyl, optionally substituted with one or more R^{s}; wherein R^{s} is, independently at each occurrence, -C(O)O-C₁₋₆alkyl; or
II) L²-R² is cyclopropyl;
   L¹ is a bond or C₁₋₁₂alkyl; wherein the C₁₋₁₂alkyl of L¹ is optionally substituted with one or more R^{e}; wherein R^{e} is, independently at each occurrence, C₁₋₆alkyl or hydroxylC₁₋₆alkyl;
   R¹ is
      i) -L³-C₆₋₂₀aryl; wherein L³ is O or C(O)NH; the C₆₋₂₀aryl of -L³-C₆₋₂₀aryl is optionally substituted with one or more R^{s}, wherein R^{s} is, independently at each occurrence, haloC₁₋₆alkyl;
      ii) C₄₋₂₀cycloalkyl optionally substituted with one or more R^{s}; wherein R^{s} is, independently at each occurrence, halo, 5-20 membered heteroaryl, or S(O)C₁₋₆alkyl;
      iii) C₆₋₂₀aryl, optionally substituted with one or more R^{s}; wherein R^{s} is, independently at each occurrence, halo, cyano, or 5-20 membered heteroaryl;
         wherein the 5-20 membered heteroaryl of R^{s} has one or more annular atoms, independently selected from N and O; and wherein the 5-20 membered heteroaryl of R^{s} is optionally substituted with one or more R^{t}; wherein R^{t} is, independently at each occurrence, C₁₋₆alkyl;
      iv) 5-20 membered heteroaryl, optionally substituted with one or more R^{s}, wherein R^{s} is, independently at each occurrence, halo, amine, C₁₋₆alkyl, or C₆₋₂₀aryl;
         wherein the C₆₋₂₀aryl of R^{s} is optionally substituted with one or more R^{t}; wherein R^{t} is, independently at each occurrence, halo;
      v) 4-20 membered heterocyclyl, optionally substituted with one or more R^{s}, wherein R^{s} is, independently at each occurrence, halo, oxo, hydroxyl, 5-20 membered heteroaryl, C₆₋₂₀aryl, or C₁₋₆alkyl;
         wherein the C₁₋₆alkyl of R^{s} is optionally substituted with one or more R^{t};
         wherein R^{t} is, independently at each occurrence, C₆₋₂₀aryl, wherein the C₆₋₂₀aryl of R^{s} is optionally substituted with one or more R^{u};
         wherein R^{u} is, independently at each occurrence, halo or C₁₋₆alkyl.

In some embodiments, the compound of formula (I) is a compound of formula (I-a): 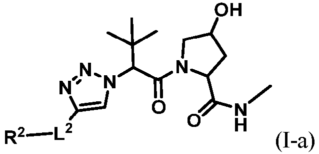 or a stereoisomer or tautomer thereof, or a pharmaceutically acceptable salt of any of the foregoing, wherein:
L² is a bond or C₁₋₁₂alkyl; and
R² is
   i) -O-C₆₋₂₀aryl, optionally substituted with one or more R^{s}; wherein R^{s} is, independently at each occurrence, halo;
   ii) 4-20 membered heterocyclyl, optionally substituted with one or more R^{s}; wherein R^{s} is, independently at each occurrence, -C(O)O-C₁₋₆alkyl;
   iii) 5-20 membered heteroaryl, wherein the 5-20 membered heteroaryl of R² is substituted with one or more R^{s}; wherein R^{s} is, independently at each occurrence, halo or C₁₋₁₂alkyl;
   iv) C₆₋₂₀cycloalkyl, optionally substituted with one or more R^{s}; wherein R^{s} is, independently at each occurrence, -C(O)O-C₁₋₆alkyl.

In some embodiments, the compound of formula (I) is a compound of formula (I-b): 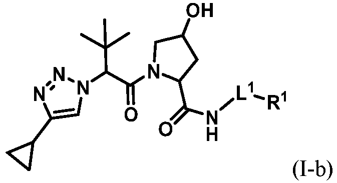 or a stereoisomer or tautomer thereof, or a pharmaceutically acceptable salt of any of the foregoing, wherein:
L¹ is a bond or C₁₋₁₂alkyl; wherein the C₁₋₁₂alkyl of L¹ is optionally substituted with one or more R^{e}; wherein R^{e} is, independently at each occurrence, C₁₋₆alkyl or hydroxylC₁₋₆alkyl; and
R¹ is
   i) -L³-C₆₋₂₀aryl; wherein L³ is O or C(O)NH; the C₆₋₂₀aryl of -L³-C₆₋₂₀aryl is optionally substituted with one or more R^{s}, wherein R^{s} is, independently at each occurrence, haloC₁₋₆alkyl;
   ii) C₄₋₂₀cycloalkyl optionally substituted with one or more R^{s}; wherein R^{s} is, independently at each occurrence, halo, 5-20 membered heteroaryl, or S(O)C₁₋₆alkyl;
   iii) C₆₋₂₀aryl, optionally substituted with one or more R^{s}; wherein R^{s} is, independently at each occurrence, halo, cyano, or 5-20 membered heteroaryl;
      wherein the 5-20 membered heteroaryl of R^{s} has one or more annular atoms, independently selected from N and O; and wherein the 5-20 membered heteroaryl of R^{s} is optionally substituted with one or more R^{t}; wherein R^{t} is, independently at each occurrence, C₁₋₆alkyl;
   iv) 5-20 membered heteroaryl, optionally substituted with one or more R^{s}, wherein R^{s} is, independently at each occurrence, halo, amine, C₁₋₆alkyl, or C₆₋₂₀aryl;
      wherein the C₆₋₂₀aryl of R^{s} is optionally substituted with one or more R^{t}; wherein R^{t} is, independently at each occurrence, halo;
   v) 4-20 membered heterocyclyl, optionally substituted with one or more R^{s}, wherein R^{s} is, independently at each occurrence, halo, oxo, hydroxyl, 5-20 membered heteroaryl, C₆₋₂₀aryl, or C₁₋₆alkyl;
      wherein the C₁₋₆alkyl of R^{s} is optionally substituted with one or more R^{t}.
         wherein R^{t} is, independently at each occurrence, C₆₋₂₀aryl,
      wherein the C₆₋₂₀aryl of R^{s} is optionally substituted with one or more R^{u};
         wherein R^{u} is, independently at each occurrence, halo or C₁₋₆alkyl.

In some embodiments, the compound of formula (I) or formula (I-a) is a compound of formula (I-a-1) or (I-a-2): 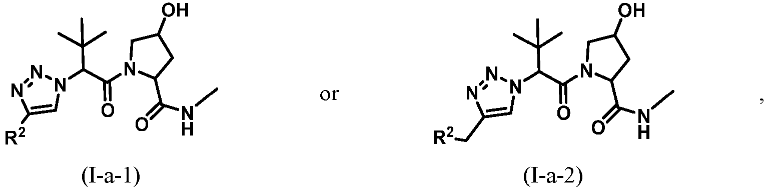 wherein L² and R² are as defined for formula (I). In some embodiments, the compound is a compound of formula (I-a-1). In some embodiments, the compound is a compound of formula (I-a-2).

In some embodiments, the compound of formula (I) or formula (I-b) is a compound of formula (I-b-1), (I-b-2), (I-b-3), (I-b-4), (I-b-5), (I-b-6), (I-b-7), (I-b-8), or (I-b-9): 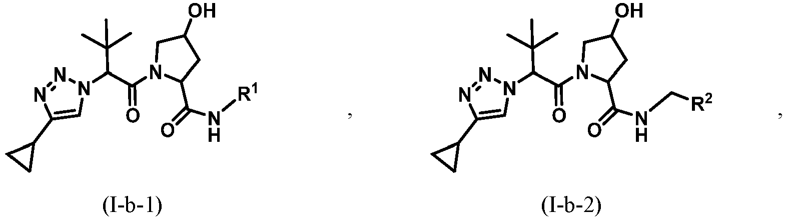 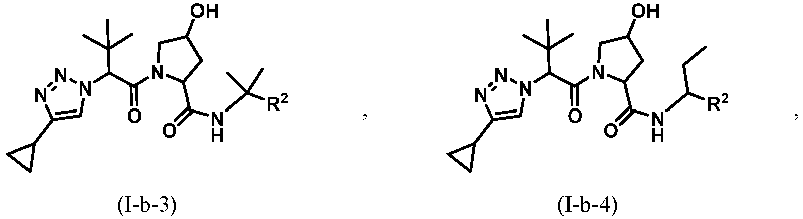 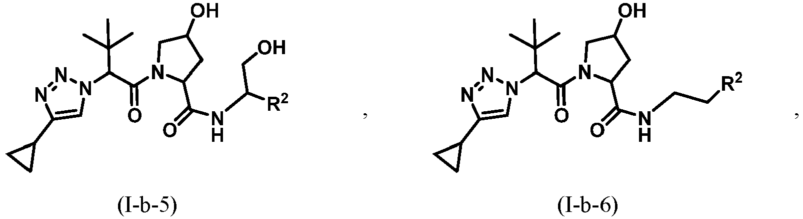 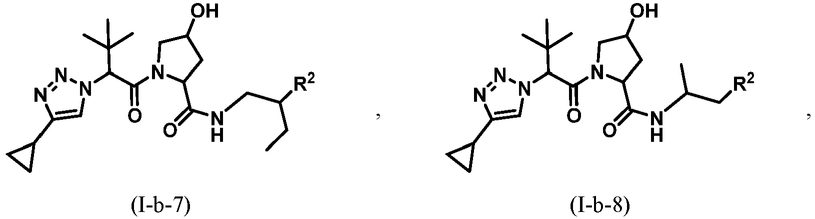 or 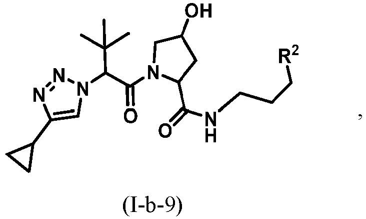 wherein L¹ and R¹ are as defined for formula (I). In some embodiments, the compound is a compound of formula (I-b-1). In some embodiments, the compound is a compound of formula (I-b-2). In some embodiments, the compound is a compound of formula (I-b-3). In some embodiments, the compound is a compound of formula (I-b-4). In some embodiments, the compound is a compound of formula (I-b-5). In some embodiments, the compound is a compound of formula (I-b-6). In some embodiments, the compound is a compound of formula (I-b-7). In some embodiments, the compound is a compound of formula (I-b-8). In some embodiments, the compound is a compound of formula (I-b-9).

In some embodiments, provided herein is a compound of formula (I) or any applicable subformulae thereof, or a stereoisomer or tautomer thereof, or a pharmaceutically acceptable salt of any of the foregoing, wherein:
L¹-R¹ is methyl;
L² is a bond or C₁₋₁₂alkyl; and
R² is -O-C₆₋₂₀aryl, optionally substituted with one or more R^{s}; wherein R^{s} is, independently at each occurrence, halo.

In some embodiments, provided herein is a compound of formula (I) or any applicable subformulae thereof, or a stereoisomer or tautomer thereof, or a pharmaceutically acceptable salt of any of the foregoing, wherein R² is -O-C₆aryl, optionally substituted with one or more halo. In some such embodiments, R² is -O-C₆aryl, optionally substituted with one or more fluoro. In some such embodiments, R² is -O-C₆aryl substituted with one fluoro.

In some embodiments, provided herein is a compound of formula (I) or any applicable subformulae thereof, or a stereoisomer or tautomer thereof, or a pharmaceutically acceptable salt of any of the foregoing, wherein:
L¹-R¹ is methyl;
L² is a bond or C₁₋₁₂alkyl; and
R² is 4-20 membered heterocyclyl, optionally substituted with one or more R^{s}; wherein R^{s} is, independently at each occurrence, -C(O)O-C₁₋₆alkyl.

In some embodiments, provided herein is a compound of formula (I) or any applicable subformulae thereof, or a stereoisomer or tautomer thereof, or a pharmaceutically acceptable salt of any of the foregoing, wherein R² is 6 membered heterocyclyl, optionally substituted with one or more -C(O)O-C₁₋₆alkyl. In some embodiments, R² is 6 membered heterocyclyl, optionally substituted with one or more -C(O)O-CH₃. In some embodiments, R² is 6 membered heterocyclyl substituted with one -C(O)O-CH₃. In some embodiments, R² is 6 membered heterocyclyl comprising 1-3 N heteroatoms, optionally substituted with one or more -C(O)O-C₁₋₆alkyl. In some embodiments, R² is 6 membered heterocyclyl comprising 1-3 N heteroatoms, optionally substituted with one or more -C(O)O-CH₃. In some embodiments, R² is 6 membered heterocyclyl comprising 1-3 N heteroatoms substituted with one -C(O)O-CH₃. In some embodiments, R² is 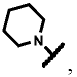 optionally substituted with one or more -C(O)O-C₁₋₆alkyl. In some embodiments, R² is 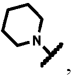 optionally substituted with one or more -C(O)O-CH₃.

In some embodiments, provided herein is a compound of formula (I) or any applicable subformulae thereof, or a stereoisomer or tautomer thereof, or a pharmaceutically acceptable salt of any of the foregoing, wherein:
L¹-R¹ is methyl;
L² is a bond or C₁₋₁₂alkyl; and
R² is 5-20 membered heteroaryl, wherein the 5-20 membered heteroaryl of R² is substituted with one or more R^{s}; wherein R^{s} is, independently at each occurrence, halo or C₁₋₁₂alkyl.

In some embodiments, provided herein is a compound of formula (I) or any applicable subformulae thereof, or a stereoisomer or tautomer thereof, or a pharmaceutically acceptable salt of any of the foregoing, wherein R² is 6-9 membered heteroaryl substituted with one or more R^{s}; wherein R^{s} is, independently at each occurrence, halo or C₁₋₁₂alkyl. In some embodiments, R² is a 6 membered heteroaryl substituted with one or more halo or C₁₋₁₂alkyl. In some embodiments, R² is a 6 membered heteroaryl substituted with one or more fluoro or C₁alkyl. In some embodiments, R² is a 6 membered heteroaryl comprising 1-3 N heteroatoms substituted with one or more halo or C₁₋₁₂alkyl. In some embodiments, R² is 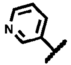 substituted with one or more halo or C₁₋₁₂alkyl. In some embodiments, R² is 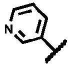 substituted with one or more fluoro or C₁alkyl. In some embodiments, R² is a 9 membered heteroaryl substituted with one or more halo or C₁₋₁₂alkyl. In some embodiments, R² is a 9 membered heteroaryl substituted with one or more C₁alkyl. In some embodiments, R² is a 9 membered heteroaryl comprising 1-3 N heteroatoms substituted with one or more halo or C₁₋₁₂alkyl. In some embodiments, R² is 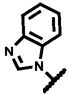 substituted with one or more halo or C₁₋₁₂alkyl. In some embodiments, R² is 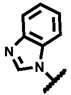 substituted with one or more C₁alkyl.

In some embodiments, provided herein is a compound of formula (I) or any applicable subformulae thereof, or a stereoisomer or tautomer thereof, or a pharmaceutically acceptable salt of any of the foregoing, wherein:
L¹-R¹ is methyl;
L² is a bond or C₁₋₁₂alkyl; and
R² is C₆₋₂₀cycloalkyl, optionally substituted with one or more R^{s}; wherein R^{s} is, independently at each occurrence, -C(O)O-C₁₋₆alkyl.

In some embodiments, provided herein is a compound of formula (I) or any applicable subformulae thereof, or a stereoisomer or tautomer thereof, or a pharmaceutically acceptable salt of any of the foregoing, wherein R² is C₆cycloalkyl, optionally substituted with one or more -C(O)O-C₁₋₆alkyl. In some embodiments, R² is C₆cycloalkyl, optionally substituted with one or more -C(O)O-CH₃. In some embodiments, R² is C₆cycloalkyl substituted with one -C(O)O-CH₃.

In some embodiments, provided herein is a compound of formula (I) or any applicable subformulae thereof, or a stereoisomer or tautomer thereof, or a pharmaceutically acceptable salt of any of the foregoing, wherein:
L²-R² is cyclopropyl;
L¹ is a bond or C₁₋₁₂alkyl; wherein the C₁₋₁₂alkyl of L¹ is optionally substituted with one or more R^{e}; wherein R^{e} is, independently at each occurrence, C₁₋₆alkyl or hydroxylC₁₋₆alkyl; and
R¹ is -L³-C₆₋₂₀aryl; wherein L³ is O or C(O)NH; the C₆₋₂₀aryl of -L³-C₆₋₂₀aryl is optionally substituted with one or more R^{s}, wherein R^{s} is, independently at each occurrence, haloC₁₋₆alkyl.

In some embodiments, provided herein is a compound of formula (I) or any applicable subformulae thereof, or a stereoisomer or tautomer thereof, or a pharmaceutically acceptable salt of any of the foregoing, wherein R¹ is -L³-C₆aryl, L³ is O or C(O)NH, and the C₆aryl of -L³-C₆aryl is optionally substituted with one or more haloC₁₋₆alkyl. In some embodiments, L³ is O. In some embodiments, L³ is C(O)NH. In some embodiments, R¹ is - L³-C₆aryl, wherein L³ is O or C(O)NH, and the C₆aryl of -L³-C₆aryl is optionally substituted with one or more haloC₁alkyl. In some embodiments, R¹ is -L³-C₆aryl, wherein L³ is O or C(O)NH, and the C₆aryl of -L³-C₆aryl is optionally substituted with one or more fluoroC₁₋₆alkyl. In some embodiments, R¹ is -L³-C₆aryl, wherein L³ is O or C(O)NH, and the C₆aryl of -L³-C₆aryl is optionally substituted with one or more -CF₃. In some embodiments, R¹ is - L³-C₆aryl, wherein L³ is O or C(O)NH, and the C₆aryl of -L³-C₆aryl is substituted with one - CF₃. In some embodiments, R¹ is -L³-C₆aryl, wherein L³ is O or C(O)NH, and the C₆aryl of -L³-C₆aryl is unsubstituted

In some embodiments, provided herein is a compound of formula (I) or any applicable subformulae thereof, or a stereoisomer or tautomer thereof, or a pharmaceutically acceptable salt of any of the foregoing, wherein:
L²-R² is cyclopropyl;
L¹ is a bond or C₁₋₁₂alkyl; wherein the C₁₋₁₂alkyl of L¹ is optionally substituted with one or more R^{e}; wherein R^{e} is, independently at each occurrence, C₁₋₆alkyl or hydroxylC₁₋₆alkyl; and
R¹ is C₄₋₂₀cycloalkyl optionally substituted with one or more R^{s}; wherein R^{s} is, independently at each occurrence, halo, 5-20 membered heteroaryl, or S(O)C₁₋₆alkyl.

In some embodiments, provided herein is a compound of formula (I) or any applicable subformulae thereof, or a stereoisomer or tautomer thereof, or a pharmaceutically acceptable salt of any of the foregoing, wherein R¹ is C₆₋₁₁cycloalkyl optionally substituted with one or more R^{s}; wherein R^{s} is, independently at each occurrence, halo, 5-20 membered heteroaryl or S(O)C₁₋₆alkyl. In some embodiments, R¹ is C₆cycloalkyl optionally substituted with one or more R^{s}; wherein R^{s} is, independently at each occurrence, halo, 5-20 membered heteroaryl, or S(O)C₁₋₆alkyl. In some such embodiments, R^{s} is, independently at each occurrence, 9 membered heteroaryl or S(O)C₁₋₆alkyl. In some such embodiments, R^{s} is, independently at each occurrence, 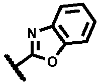 or S(O)CH₂CH₃. In some embodiments, R¹ is C₉cycloalkyl optionally substituted with one or more R^{s}; wherein R^{s} is, independently at each occurrence, halo, 5-20 membered heteroaryl, or S(O)C₁₋₆alkyl. In some such embodiments, R^{s} is, independently at each occurrence, halo. In some such embodiments, R^{s} is chloro. In some embodiments, R¹ is 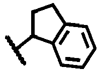 optionally substituted with one or more R^{s}; wherein R^{s} is, independently at each occurrence, halo, 5-20 membered heteroaryl, or S(O)C₁₋₆alkyl. In some such embodiments, R^{s} is, independently at each occurrence, halo. In some such embodiments, R^{s} is chloro. In some embodiments, R¹ is C₁₁cycloalkyl optionally substituted with one or more R^{s}; wherein R^{s} is, independently at each occurrence, halo, 5-20 membered heteroaryl, or S(O)C₁₋₆alkyl. In some such embodiments, R^{s} is, independently at each occurrence, halo. In some such embodiments, R^{s} is, independently at each occurrence, bromo. In some embodiments, R¹ is 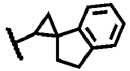 optionally substituted with one or more R^{s}; wherein R^{s} is, independently at each occurrence, halo, 5-20 membered heteroaryl, or S(O)C₁₋₆alkyl. In some such embodiments, R^{s} is, independently at each occurrence, halo. In some such embodiments, R^{s} is, independently at each occurrence, bromo.

In some embodiments, provided herein is a compound of formula (I) or any applicable subformulae thereof, or a stereoisomer or tautomer thereof, or a pharmaceutically acceptable salt of any of the foregoing, wherein:
L²-R² is cyclopropyl;
L¹ is a bond or C₁₋₁₂alkyl; wherein the C₁₋₁₂alkyl of L¹ is optionally substituted with one or more R^{e}; wherein R^{e} is, independently at each occurrence, C₁₋₆alkyl or hydroxylC₁₋₆alkyl; and
R¹ is C₆₋₂₀aryl, optionally substituted with one or more R^{s}; wherein R^{s} is, independently at each occurrence, halo, cyano, or 5-20 membered heteroaryl,
   wherein the 5-20 membered heteroaryl of R^{s} has one or more annular atoms, independently selected from N and O; and wherein the 5-20 membered heteroaryl of R^{s} is optionally substituted with one or more R^{t}; wherein R^{t} is, independently at each occurrence, C₁₋₆alkyl.

In some embodiments, provided herein is a compound of formula (I) or any applicable subformulae thereof, or a stereoisomer or tautomer thereof, or a pharmaceutically acceptable salt of any of the foregoing, wherein R¹ is C₆aryl, optionally substituted with one or more R^{s}; wherein R^{s} is, independently at each occurrence, halo, cyano, 5-20 membered heteroaryl, and wherein the 5-20 membered heteroaryl of R^{s} has one or more annular atoms, independently selected from N and O; and wherein the 5-20 membered heteroaryl of R^{s} is optionally substituted with one or more R^{t}; wherein R^{t} is, independently at each occurrence, C₁₋₆alkyl. In some embodiments, R1 is C₆aryl, optionally substituted with one or more R^{s}; wherein R^{s} is, independently at each occurrence, fluoro, bromo, or cyano.

In some embodiments, provided herein is a compound of formula (I) or any applicable subformulae thereof, or a stereoisomer or tautomer thereof, or a pharmaceutically acceptable salt of any of the foregoing, wherein:
L²-R² is cyclopropyl;
L¹ is a bond or C₁₋₁₂alkyl; wherein the C₁₋₁₂alkyl of L¹ is optionally substituted with one or more R^{e}; wherein R^{e} is, independently at each occurrence, C₁₋₆alkyl or hydroxylC₁₋₆alkyl; and
R¹ is 5-20 membered heteroaryl, optionally substituted with one or more R^{s}, wherein R^{s} is, independently at each occurrence, halo, amine, or C₁₋₆alkyl, C₆₋₂₀aryl,
   wherein the C₆₋₂₀aryl of R^{s} is optionally substituted with one or more R^{t};
wherein R^{t} is, independently at each occurrence, halo.

In some embodiments, provided herein is a compound of formula (I) or any applicable subformulae thereof, or a stereoisomer or tautomer thereof, or a pharmaceutically acceptable salt of any of the foregoing, wherein R¹ is 5-9 membered heteroaryl, optionally substituted with one or more R^{s}, wherein R^{s} is, independently at each occurrence, halo, amine, C₁₋₆alkyl, or C₆₋₂₀aryl; wherein the C₆₋₂₀aryl of R^{s} is optionally substituted with one or more R^{t}; wherein R^{t} is, independently at each occurrence, halo. In some embodiments, R¹ is 5 membered heteroaryl, optionally substituted with one or more R^{s}, wherein R^{s} is, independently at each occurrence, halo, amine, C₁₋₆alkyl, or C₆₋₂₀aryl; wherein the C₆₋₂₀aryl of R^{s} is optionally substituted with one or more R^{t}; wherein R^{t} is, independently at each occurrence, halo. In some such embodiments, R^{s} is, independently at each occurrence, C₁₋₂alkyl or C₆aryl; wherein the C₆aryl of R^{s} is optionally substituted with one or more R^{t}; wherein R^{t} is, independently at each occurrence, halo. In some such embodiments, R^{s} is C₁₋₂alkyl, 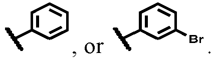 In some embodiments, R¹ is 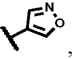 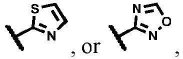 optionally substituted with one or more R^{s}, wherein R^{s} is, independently at each occurrence, halo, amine, C₁₋₆alkyl, or C₆₋₂₀aryl; wherein the C₆₋₂₀aryl of R^{s} is optionally substituted with one or more R^{t}; wherein R^{t} is, independently at each occurrence, halo. In some such embodiments, R^{s} is, independently at each occurrence, C₁₋₂alkyl or C₆aryl; wherein the C₆aryl of R^{s} is optionally substituted with one or more R^{t}; wherein R^{t} is, independently at each occurrence, halo. In some such embodiments, R^{s} is C₁₋₂alkyl, 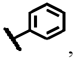 or 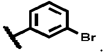 In some embodiments, R¹ is 9 membered heteroaryl, optionally substituted with one or more R^{s}, wherein R^{s} is, independently at each occurrence, halo, amine, C₁₋₆alkyl, or C₆₋₂₀aryl; wherein the C₆₋₂₀aryl of R^{s} is optionally substituted with one or more R^{t}; wherein R^{t} is, independently at each occurrence, halo. In some embodiments, R¹ is 9 membered heteroaryl, optionally substituted with one or more R^{s}, wherein R^{s} is, independently at each occurrence, halo or amine. In some embodiments, R¹ is 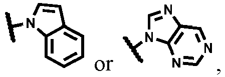 optionally substituted with one or more R^{s}, wherein R^{s} is, independently at each occurrence, halo or amine.

In some embodiments, provided herein is a compound of formula (I) or any applicable subformulae thereof, or a stereoisomer or tautomer thereof, or a pharmaceutically acceptable salt of any of the foregoing, wherein:
L²-R² is cyclopropyl;
L¹ is a bond or C₁₋₁₂alkyl; wherein the C₁₋₁₂alkyl of L¹ is optionally substituted with one or more R^{e}; wherein R^{e} is, independently at each occurrence, C₁₋₆alkyl or hydroxylC₁₋₆alkyl; and
R¹ is 4-20 membered heterocyclyl, optionally substituted with one or more R^{s}, wherein R^{s} is, independently at each occurrence, halo, oxo, hydroxyl, 5-20 membered heteroaryl, C₆₋₂₀aryl, or C₁₋₆alkyl;
   wherein the C₁₋₆alkyl of R^{s} is optionally substituted with one or more R^{t};
      wherein R^{t} is, independently at each occurrence, C₆₋₂₀aryl,
   wherein the C₆₋₂₀aryl of R^{s} is optionally substituted with one or more R^{u};
      wherein R^{u} is, independently at each occurrence, halo or C₁₋₆alkyl.

In some embodiments, provided herein is a compound of formula (I) or any applicable subformulae thereof, or a stereoisomer or tautomer thereof, or a pharmaceutically acceptable salt of any of the foregoing, wherein R¹ is 4-10 membered heterocyclyl, optionally substituted with one or more R^{s}, wherein R^{s} is, independently at each occurrence, halo, oxo, hydroxyl, 5-20 membered heteroaryl, C₆₋₂₀aryl, C₁₋₆alkyl; wherein the C₁₋₆alkyl of R^{s} is optionally substituted with one or more R^{t}; wherein R^{t} is, independently at each occurrence, C₆₋₂₀aryl; and wherein the C₆₋₂₀aryl of R^{s} is optionally substituted with one or more R^{u}; wherein R^{u} is, independently at each occurrence, halo or C₁₋₆alkyl.

In some embodiments, R¹ is 4 membered heterocyclyl, optionally substituted with one or more R^{s}, wherein R^{s} is, independently at each occurrence, halo, oxo, hydroxyl, 5-20 membered heteroaryl, C₆₋₂₀aryl, C₁₋₆alkyl; wherein the C₁₋₆alkyl of R^{s} is optionally substituted with one or more R^{t}; wherein R^{t} is, independently at each occurrence, C₆₋₂₀aryl; and wherein the C₆₋₂₀aryl of R^{s} is optionally substituted with one or more R^{u}; wherein R^{u} is, independently at each occurrence, halo or C₁₋₆alkyl. In some such embodiments, R¹ is 4 membered heterocyclyl, optionally substituted with one or more R^{s}, wherein R^{s} is, independently at each occurrence, hydroxyl, 5-20 membered heteroaryl, or C₁₋₆alkyl optionally substituted with one or more R^{t}; wherein R^{t} is, independently at each occurrence, C₆₋₂₀aryl. In some such embodiments, R^{s} is, independently at each occurrence, hydroxyl, pyridine, or Cialkyl optionally substituted with one or more R^{t}; wherein R^{t} is, independently at each occurrence, C₆aryl. In some embodiments, R¹ is 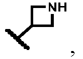 optionally substituted with one or more R^{s}, wherein R^{s} is, independently at each occurrence, hydroxyl, 5-20 membered heteroaryl or C₁₋₆alkyl optionally substituted with one or more R^{t}; wherein R^{t} is, independently at each occurrence, C₆₋₂₀aryl. In some such embodiments, R^{s} is, independently at each occurrence, hydroxyl, pyridine or Cialkyl optionally substituted with one or more R^{t}; wherein R^{t} is, independently at each occurrence, C₆aryl.

In some embodiments, R¹ is 5 membered heterocyclyl, optionally substituted with one or more R^{s}, wherein R^{s} is, independently at each occurrence, halo, oxo, hydroxyl, 5-20 membered heteroaryl, C₆₋₂₀aryl, C₁₋₆alkyl; wherein the C₁₋₆alkyl of R^{s} is optionally substituted with one or more R^{t}; wherein R^{t} is, independently at each occurrence, C₆₋₂₀aryl; and wherein the C₆₋₂₀aryl of R^{s} is optionally substituted with one or more R^{u}; wherein R^{u} is, independently at each occurrence, halo or C₁₋₆alkyl. In some such embodiments, R¹ is 5 membered heterocyclyl, optionally substituted with one or more R^{s}, wherein R^{s} is C₆₋₂₀aryl, and wherein the C₆₋₂₀aryl of R^{s} is optionally substituted with one or more R^{u}; wherein R^{u} is, independently at each occurrence, halo or C₁₋₆alkyl. In some such embodiments, R^{s} is C₆aryl, optionally substituted with one or more R^{u}, wherein R^{u} is, independently at each occurrence, C₁alkyl, or chloro. In some embodiments, R¹ is 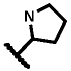 or 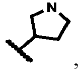 optionally substituted with one or more R^{s}, wherein R^{s} is C₆₋₂₀aryl, and wherein the C₆₋₂₀aryl of R^{s} is optionally substituted with one or more R^{u}; wherein R^{u} is, independently at each occurrence, halo or C₁₋₆alkyl. In some such embodiments, R^{s} is C₆aryl, optionally substituted with one or more R^{u}, wherein R^{u} is, independently at each occurrence, C₁alkyl, or chloro.

In some embodiments, R¹ is 10 membered heterocyclyl, optionally substituted with one or more R^{s}, wherein R^{s} is, independently at each occurrence, halo, oxo, hydroxyl, 5-20 membered heteroaryl, C₆₋₂₀aryl, C₁₋₆alkyl; wherein the C₁₋₆alkyl of R^{s} is optionally substituted with one or more R^{t}; wherein R^{t} is, independently at each occurrence, C₆₋₂₀aryl; and wherein the C₆₋₂₀aryl of R^{s} is optionally substituted with one or more R^{u}; wherein R^{u} is, independently at each occurrence, halo or C₁₋₆alkyl. In some such embodiments, R¹ is 10 membered heterocyclyl, optionally substituted with one or more R^{s}, wherein R^{s} is, independently at each occurrence, oxo, or C₆₋₂₀aryl. In some such embodiments, R^{s} is, independently at each occurrence, oxo, or C₆aryl. In some embodiments, R¹ is 10 membered heterocyclyl comprising one or more heteroatoms selected from the group consisting of O and S, optionally substituted with one or more R^{s}, wherein R^{s} is, independently at each occurrence, oxo, or C₆₋₂₀aryl. In some such embodiments, R^{s} is, independently at each occurrence, oxo, or C₆aryl. In some embodiments, R¹ is 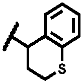 or 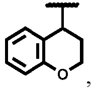 optionally substituted with one or more R^{s}, wherein R^{s} is, independently at each occurrence, oxo, or C₆₋₂₀aryl. In some such embodiments, R^{s} is, independently at each occurrence, oxo, or C₆aryl.

In some embodiments, provided herein is a compound of formula (I) or any applicable subformulae thereof, or a stereoisomer or tautomer thereof, or a pharmaceutically acceptable salt of any of the foregoing, wherein:
L¹-R¹ is methyl;
L² is a bond or C₁₋₁₂alkyl; and
R² is selected from the group consisting of

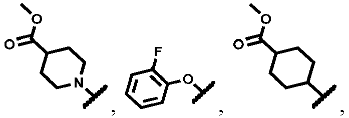

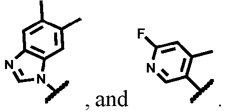

In some embodiments, provided herein is a compound of formula (I) or any applicable subformulae thereof, or a stereoisomer or tautomer thereof, or a pharmaceutically acceptable salt of any of the foregoing, wherein L² is a bond or -CH₂-.

In some embodiments, provided herein is a compound of formula (I) or any applicable subformulae thereof, or a stereoisomer or tautomer thereof, or a pharmaceutically acceptable salt of any of the foregoing, wherein:
L¹-R¹ is methyl;
L² is a bond or -CH₂-; and
R² is selected from the group consisting of

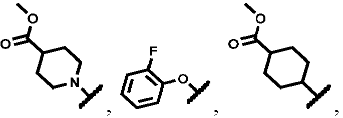

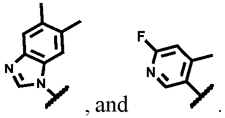

In some embodiments, provided herein is a compound of formula (I) or any applicable subformulae thereof, or a stereoisomer or tautomer thereof, or a pharmaceutically acceptable salt of any of the foregoing, wherein:
L¹-R¹ is methyl;
L² is a bond; and
R² is selected from the group consisting of

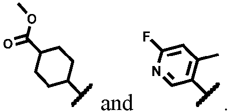

In some embodiments, provided herein is a compound of formula (I) or any applicable subformulae thereof, or a stereoisomer or tautomer thereof, or a pharmaceutically acceptable salt of any of the foregoing, wherein:
L¹-R¹ is methyl;
L² is -CH₂-; and
R² is selected from the group consisting of

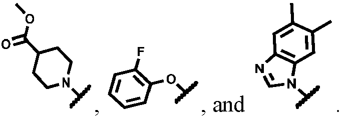

In some embodiments, provided herein is a compound of formula (I) or any applicable subformulae thereof, or a stereoisomer or tautomer thereof, or a pharmaceutically acceptable salt of any of the foregoing, wherein:
L²-R² is cyclopropyl;
L¹ is a bond or C₁₋₁₂alkyl; wherein the C₁₋₁₂alkyl of L¹ is optionally substituted with one or more R^{e}; wherein R^{e} is, independently at each occurrence, C₁₋₆alkyl or hydroxylC₁₋₆alkyl; and
R¹ is selected from the group consisting of

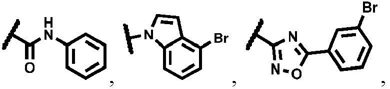

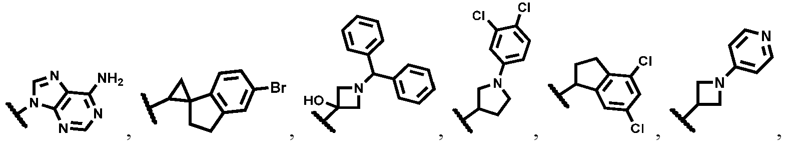

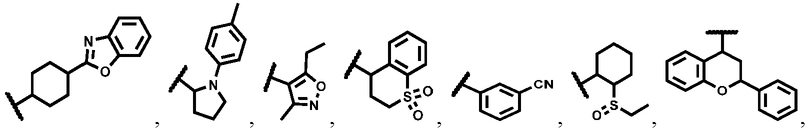

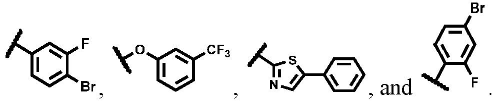

In some embodiments, provided herein is a compound of formula (I) or any applicable subformulae thereof, or a stereoisomer or tautomer thereof, or a pharmaceutically acceptable salt of any of the foregoing, wherein L¹ is selected from the group consisting of a bond, -CH₂-, -CH₂CH₂-, -CH₂CH₂CH₂-, -C (CH₃)₂-, -CH(CH₂OH)-, -CH(CH₂CH₃)-, - CH(CH₃)CH₂-, and -CH(CH₂CH₃)CH₂-.

In some embodiments, provided herein is a compound of formula (I) or any applicable subformulae thereof, or a stereoisomer or tautomer thereof, or a pharmaceutically acceptable salt of any of the foregoing, wherein:
L²-R² is cyclopropyl;
L¹ is selected from the group consisting of a bond, -CH₂-, -CH₂CH₂-, -CH₂CH₂CH₂-, - C (CH₃)₂-, -CH(CH₂OH)-, -CH(CH₂CH₃)-, -CH(CH₃)CH₂-, and -CH(CH₂CH₃)CH₂-; and
R¹ is selected from the group consisting of

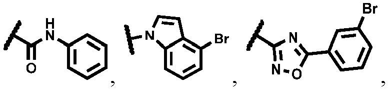

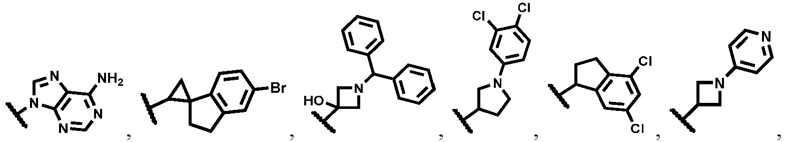

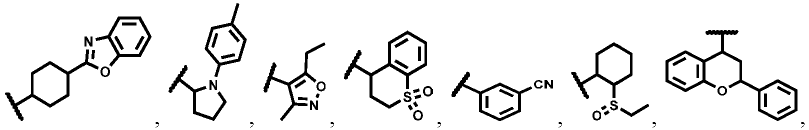

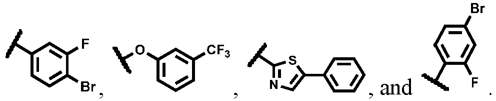

In some embodiments, provided is a compound of formula (I), or a stereoisomer or tautomer thereof, or a pharmaceutically acceptable salt of any of the foregoing, wherein:
L²-R² is cyclopropyl;
L¹ is a bond; and
   R¹ is selected from the group consisting of

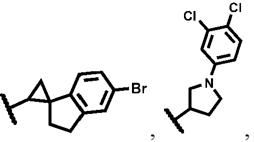

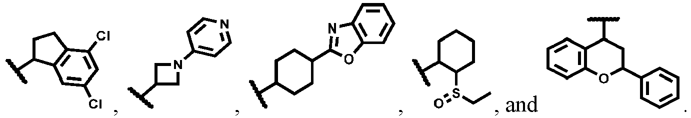

In some embodiments, provided is a compound of formula (I), or a stereoisomer or tautomer thereof, or a pharmaceutically acceptable salt of any of the foregoing, wherein:
L²-R² is cyclopropyl;
L¹ is selected from the group consisting of -CH₂-, -CH₂CH₂-, -CH₂CH₂CH₂-, -C (CH₃)₂-, -CH(CH₂OH)-, -CH(CH₂CH₃)-, -CH(CH₃)CH₂-, and -CH(CH₂CH₃)CH₂-; and
R¹ is selected from the group consisting of

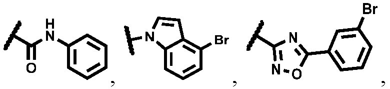

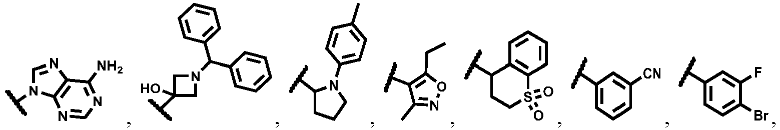

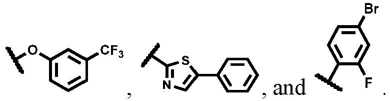

In some embodiments, provided is a compound of formula (I), or a stereoisomer or tautomer thereof, or a pharmaceutically acceptable salt of any of the foregoing, wherein the compound is selected from the group consisting of

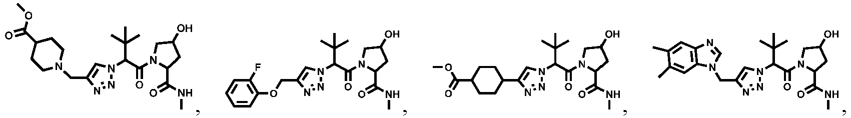

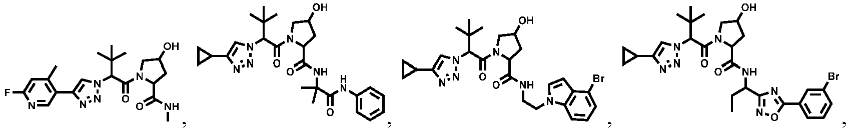

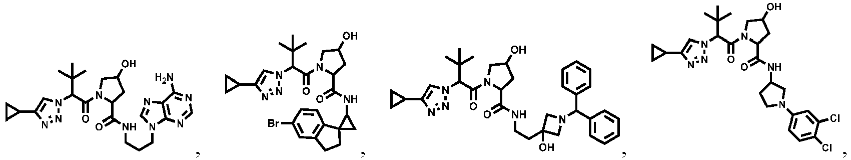

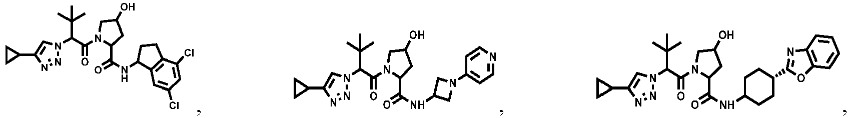

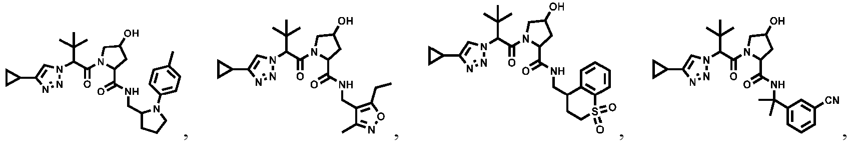

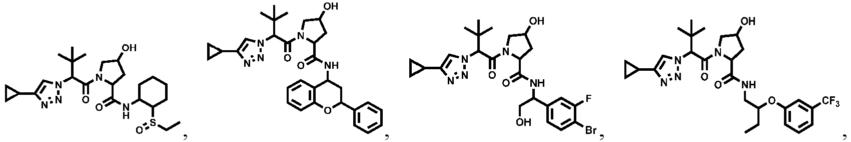

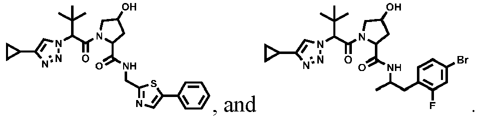

In some embodiments, provided is a compound of formula (I), or a pharmaceutically acceptable salt thereof, wherein the compound is selected from the group consisting of

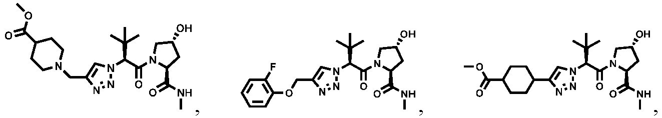

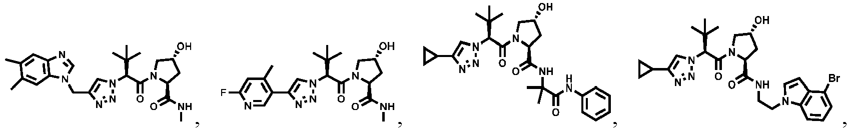

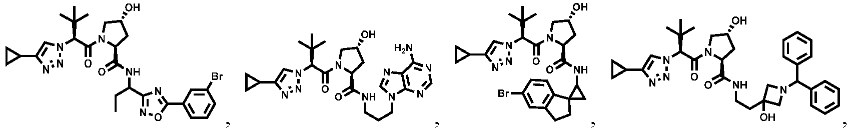

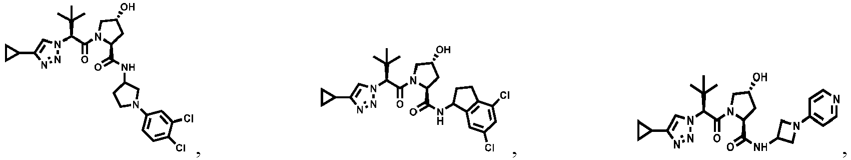

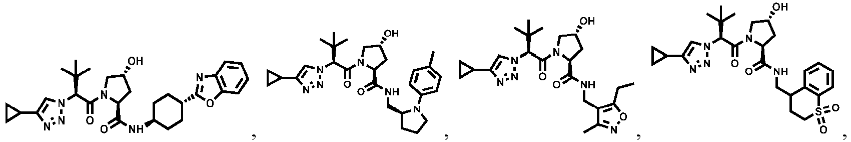

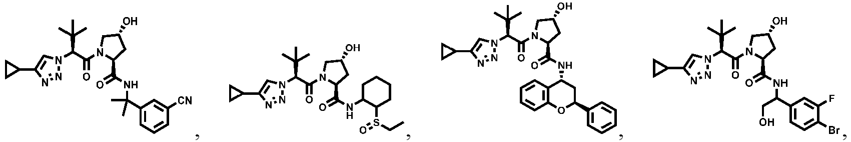

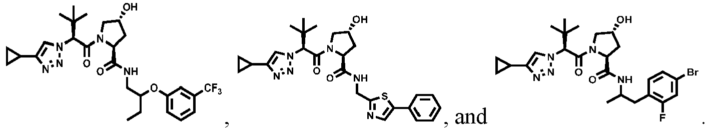

In another embodiment, provided is a compound of formula (II): 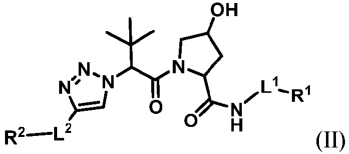 or a stereoisomer or tautomer thereof, or a pharmaceutically acceptable salt of any of the foregoing, wherein:
I) L¹-R¹ is methyl;
   L² is a bond or C₁₋₁₂alkyl;
   R² is
      i) -O-C₆₋₂₀aryl, optionally substituted with one or more R^{s}; wherein R^{s} is, independently at each occurrence, halo;
      ii) 4-20 membered heterocyclyl, optionally substituted with one or more R^{s}; wherein R^{s} is, independently at each occurrence, -C(O)O-C₁₋₆alkyl;
      iii) 5-20 membered heteroaryl, wherein the 5-20 membered heteroaryl of R² is substituted with one or more R^{s}; wherein R^{s} is, independently at each occurrence, halo or C₁₋₁₂alkyl;
      iv) C₆₋₂₀cycloalkyl, optionally substituted with one or more R^{s}; wherein R^{s} is, independently at each occurrence, -C(O)O-C₁₋₆alkyl;
      v) -N(CH₃)-C₆₋₂₀aryl;
      vi) -C₆₋₂₀aryl, optionally substituted with one or more R^{s}; wherein R^{s} is, independently at each occurrence, C₁₋₆haloalkyl; or
   II) L²-R² is cyclopropyl;
      L¹ is a bond or C₁₋₁₂alkyl; wherein the C₁₋₁₂alkyl of L¹ is optionally substituted with one or more R^{e}; wherein R^{e} is, independently at each occurrence, C₁₋₆alkyl or hydroxylC₁₋₆alkyl;
      R¹ is
         i) -L³-C₆₋₂₀aryl; wherein L³ is O or C(O)NH; the C₆₋₂₀aryl of -L³-C₆₋₂₀aryl is optionally substituted with one or more R^{s}, wherein R^{s} is, independently at each occurrence, haloC₁₋₆alkyl;
         ii) C₄₋₂₀cycloalkyl optionally substituted with one or more R^{s}; wherein R^{s} is, independently at each occurrence, halo, 5-20 membered heteroaryl, or S(O)C₁₋₆alkyl;
         iii) C₆₋₂₀aryl, optionally substituted with one or more R^{s}; wherein R^{s} is, independently at each occurrence, halo, cyano, or 5-20 membered heteroaryl;
            wherein the 5-20 membered heteroaryl of R^{s} has one or more annular atoms, independently selected from N and O; and wherein the 5-20 membered heteroaryl of R^{s} is optionally substituted with one or more R^{t}; wherein R^{t} is, independently at each occurrence, C₁₋₆alkyl;
         iv) 5-20 membered heteroaryl, optionally substituted with one or more R^{s}, wherein R^{s} is, independently at each occurrence, halo, amine, C₁₋₆alkyl, or C₆₋₂₀aryl;
            wherein the C₆₋₂₀aryl of R^{s} is optionally substituted with one or more R^{t}; wherein R^{t} is, independently at each occurrence, halo; or
         v) 4-20 membered heterocyclyl, optionally substituted with one or more R^{s}, wherein R^{s} is, independently at each occurrence, halo, oxo, hydroxyl, 5-20 membered heteroaryl, C₆₋₂₀aryl, or C₁₋₆alkyl;
            wherein the C₁₋₆alkyl of R^{s} is optionally substituted with one or more R^{t}; wherein R^{t} is, independently at each occurrence, C₆₋₂₀aryl,
wherein the C₆₋₂₀aryl of R^{s} is optionally substituted with one or more R^{u}; wherein R^{u} is, independently at each occurrence, halo or C₁₋₆alkyl.

It should be understood that any methods of making or using a compound of formula (I) will also apply to a compound of formula (II).

In some embodiments, provided herein is a compound of formula (II) or any applicable subformulae thereof, or a stereoisomer or tautomer thereof, or a pharmaceutically acceptable salt of any of the foregoing, wherein:
L¹-R¹ is methyl;
L² is a bond or C₁₋₁₂alkyl; and
   R² is selected from the group consisting of

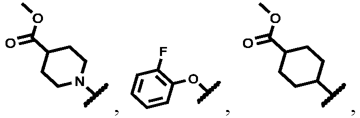

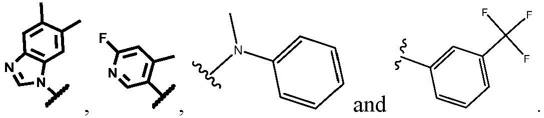

In some embodiments, provided herein is a compound of formula (II) or any applicable subformulae thereof, or a stereoisomer or tautomer thereof, or a pharmaceutically acceptable salt of any of the foregoing, wherein:
L²-R² is cyclopropyl;
L¹ is selected from the group consisting of a bond, -CH₂-, -CH₂CH₂-, -CH₂CH₂CH₂-, - C (CH₃)₂-, -CH(CH₂OH)-, -CH(CH₂CH₃)-, -CH(CH₃)CH₂-, and -CH(CH₂CH₃)CH₂-; and
R¹ is selected from the group consisting of

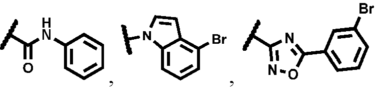

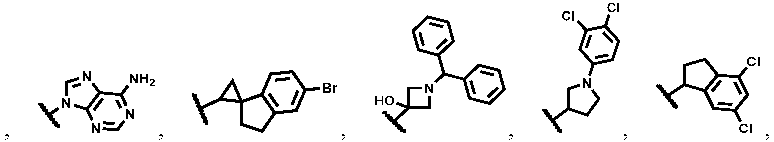

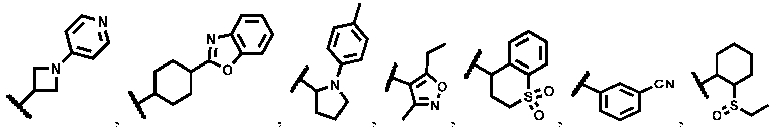

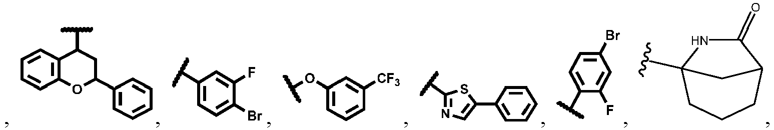

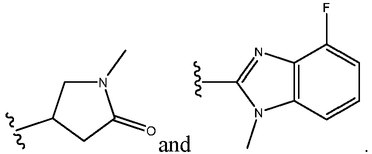

Compounds described herein include a compound of Table 1-A, Table 1-B, or Table 1-C, or a stereoisomer or tautomer thereof, or a pharmaceutically acceptable salt of any of the foregoing.

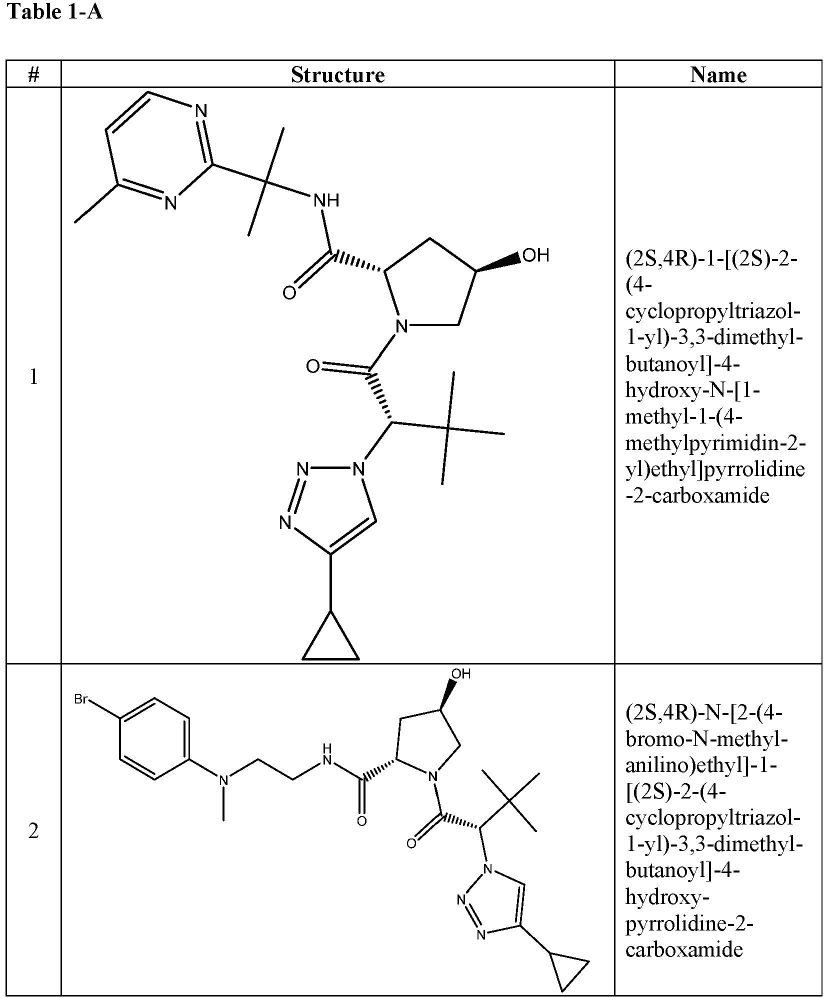

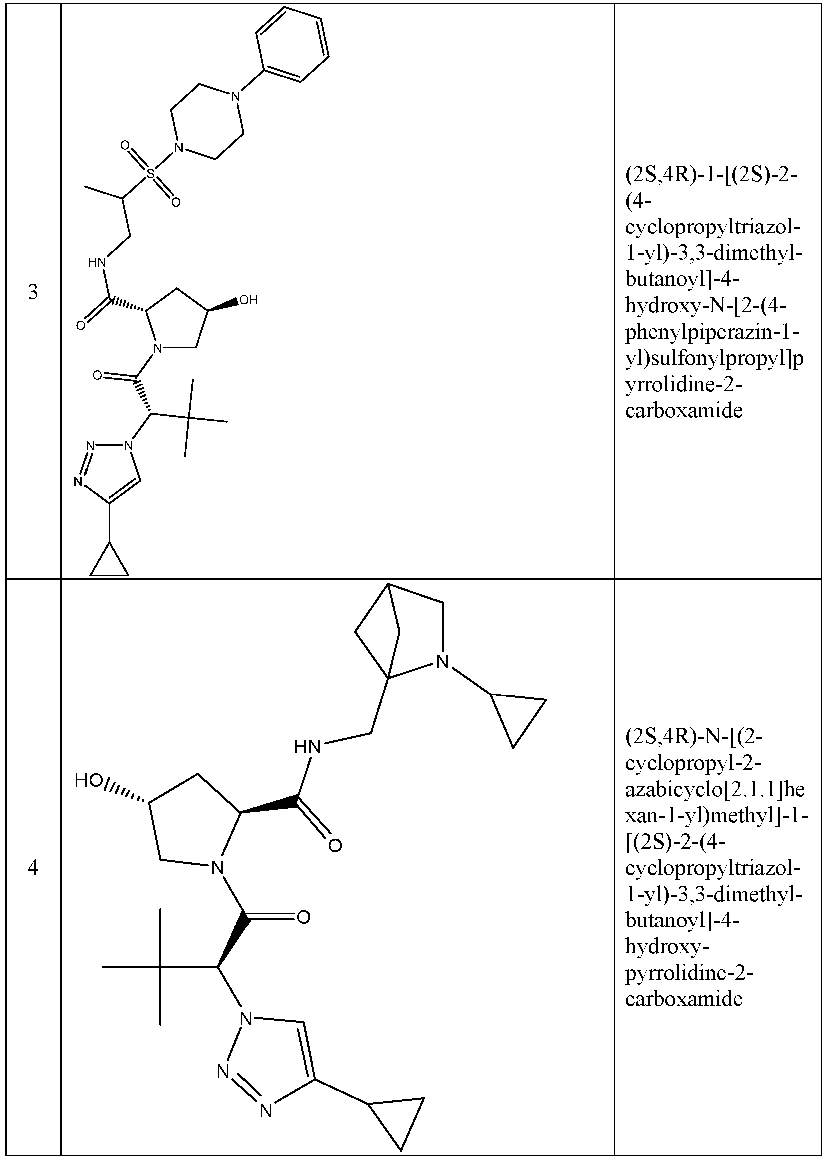

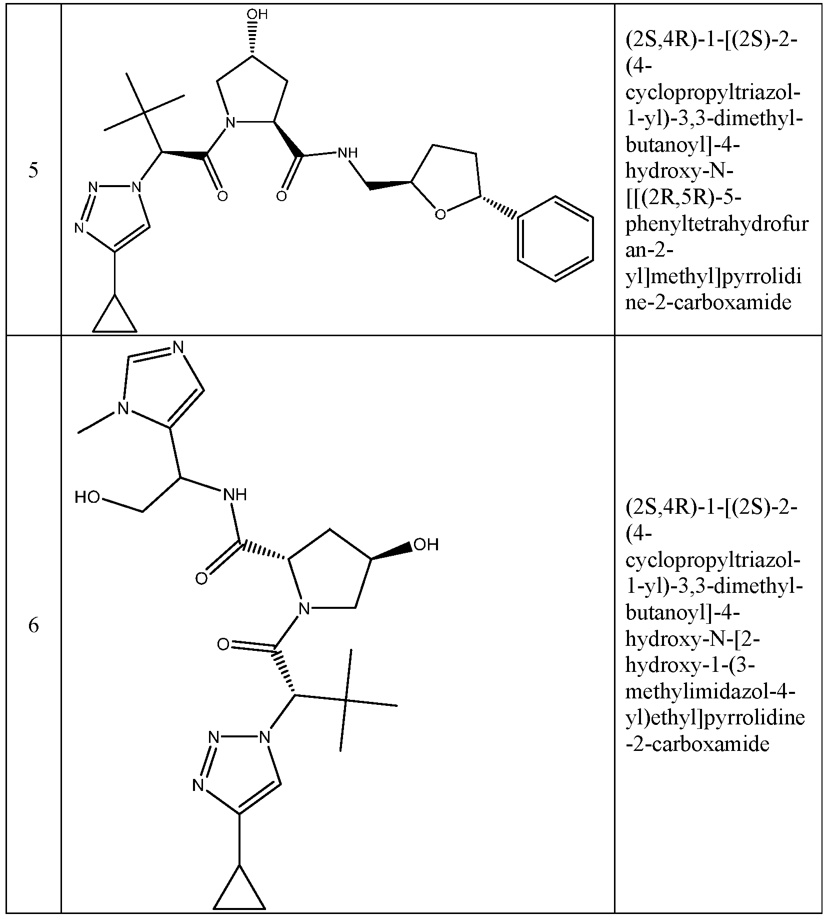

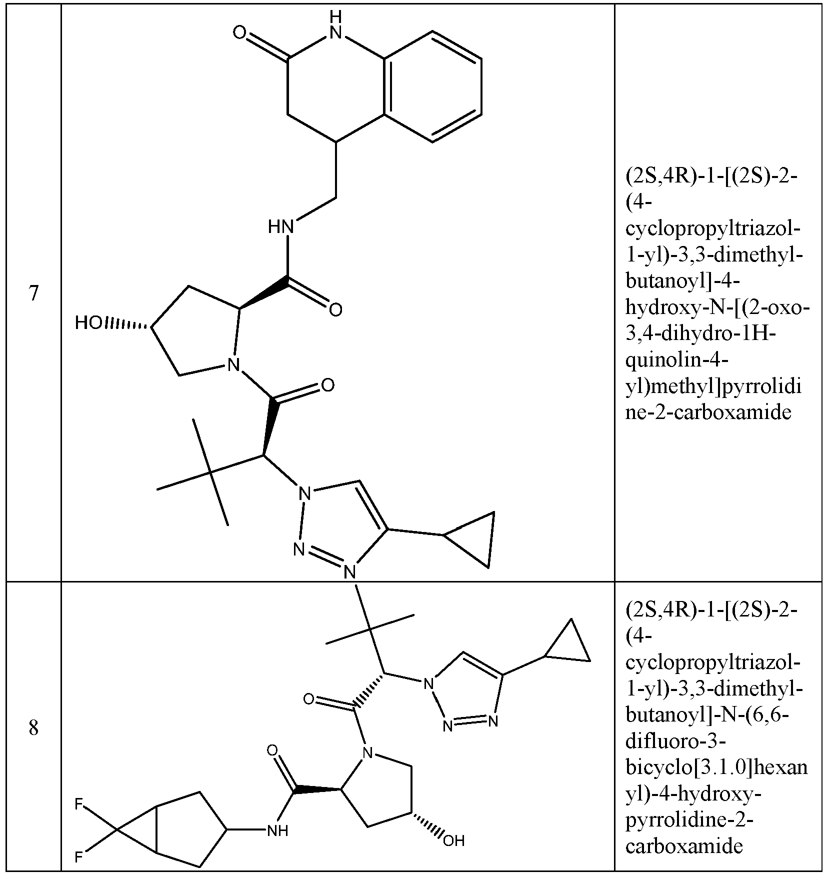

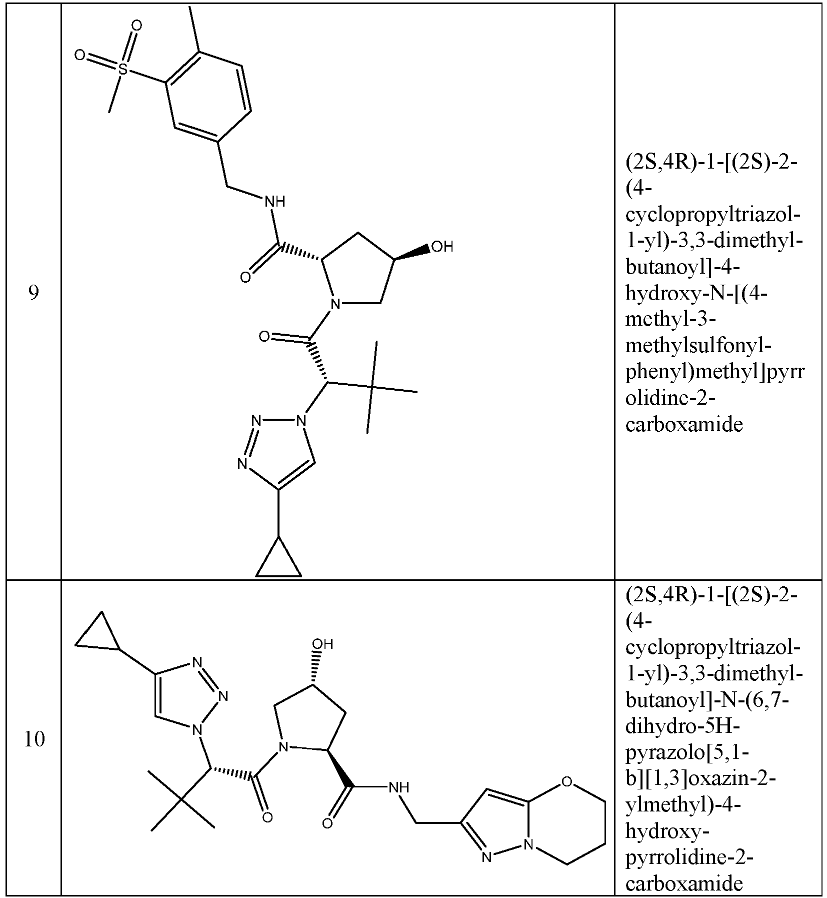

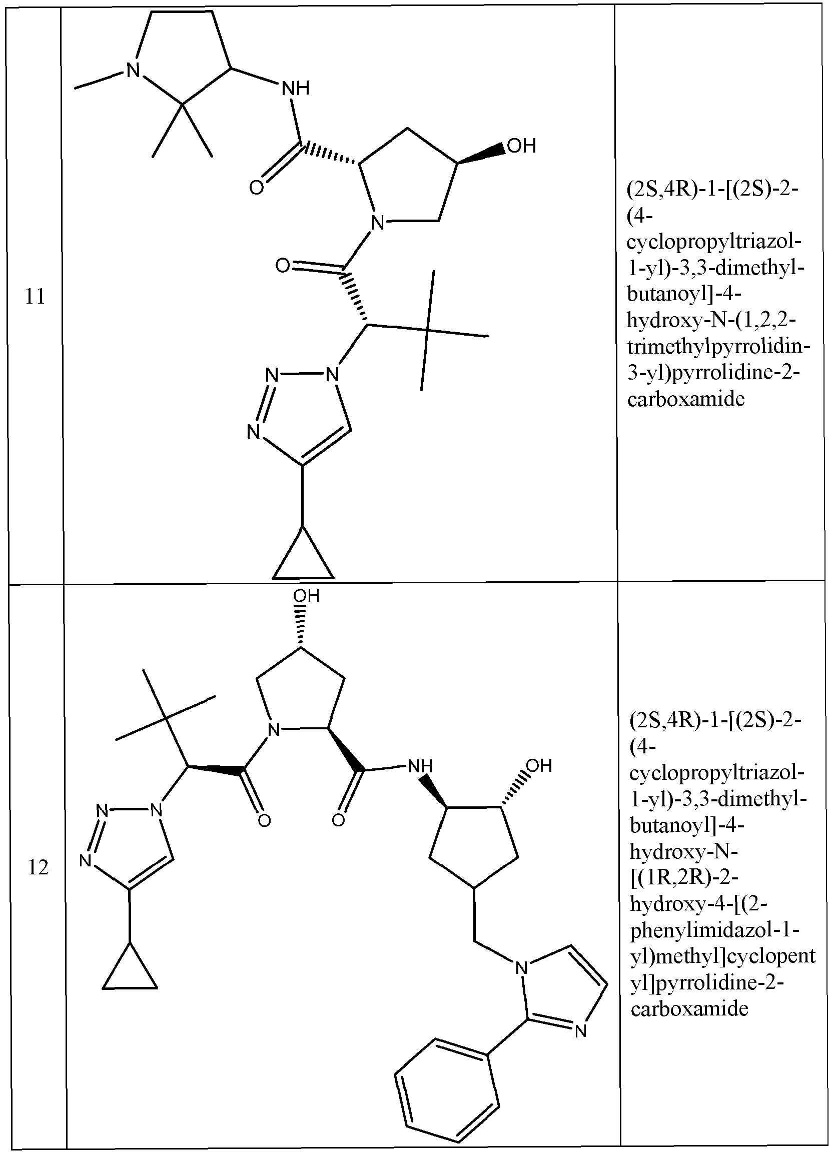

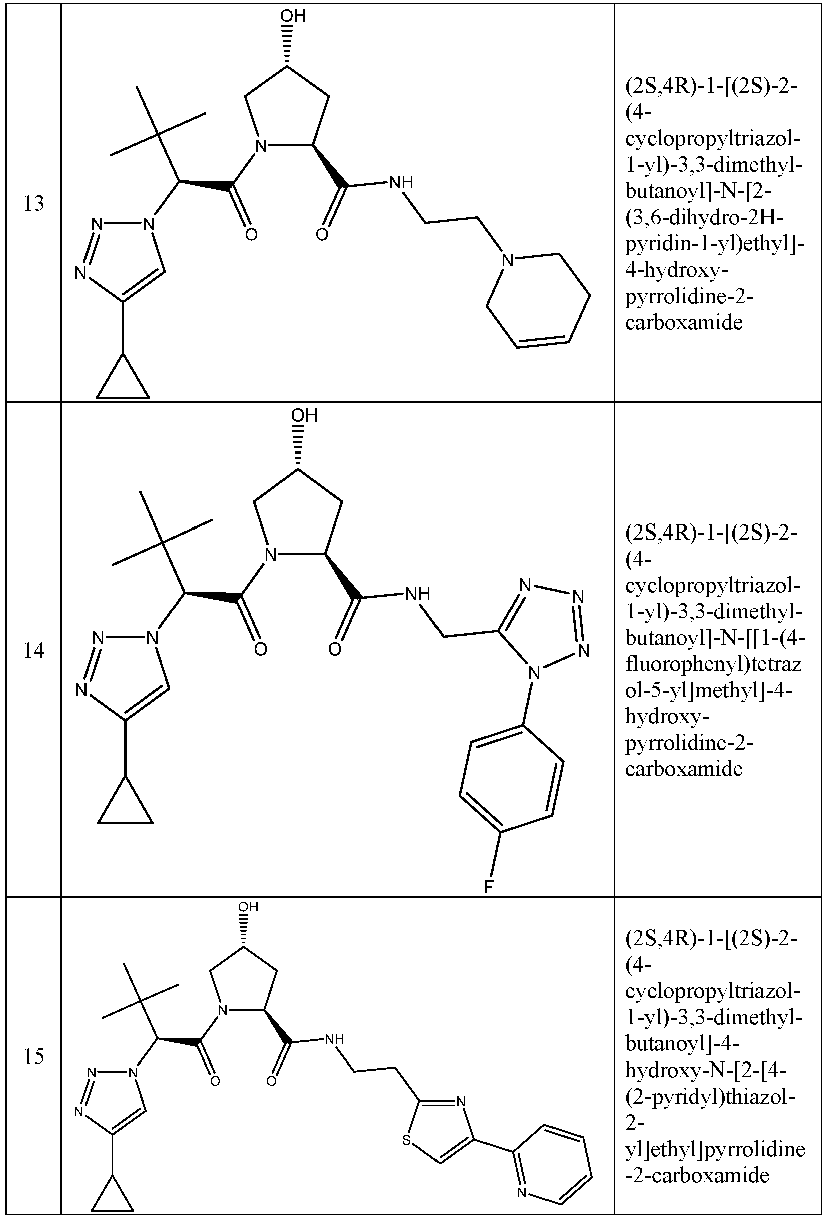

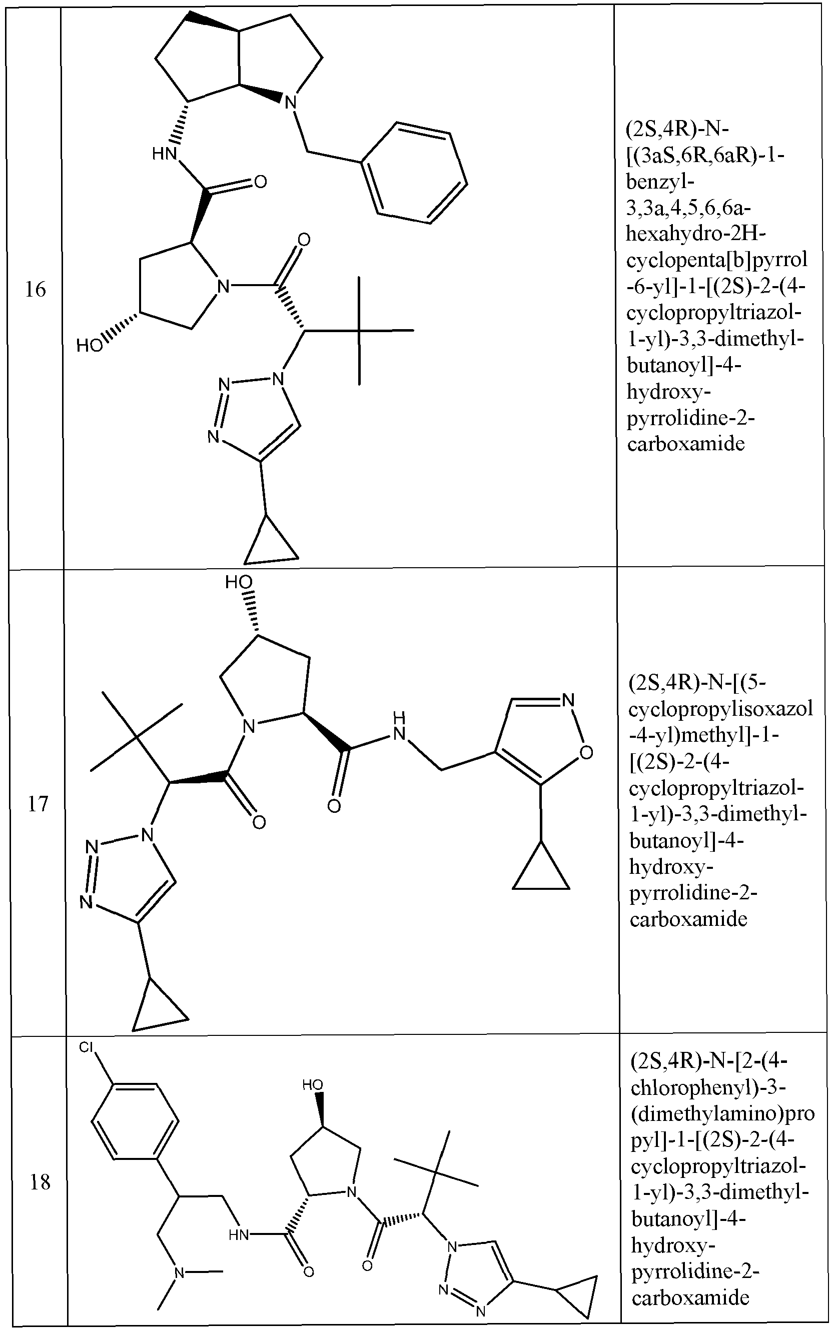

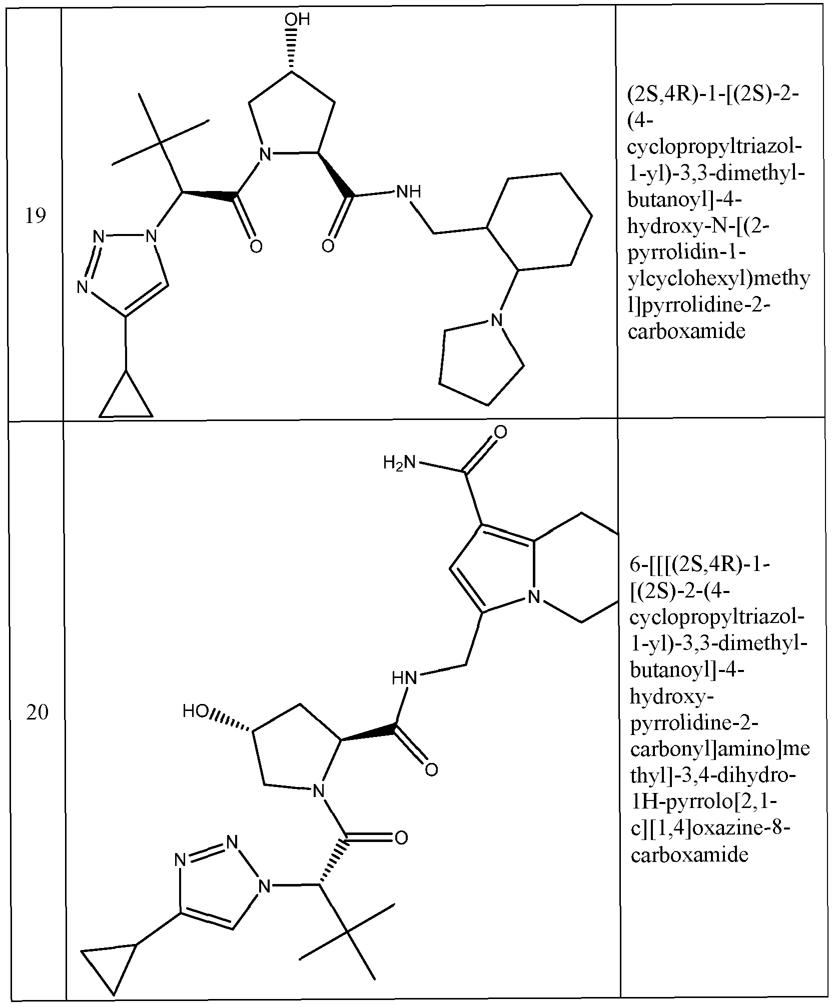

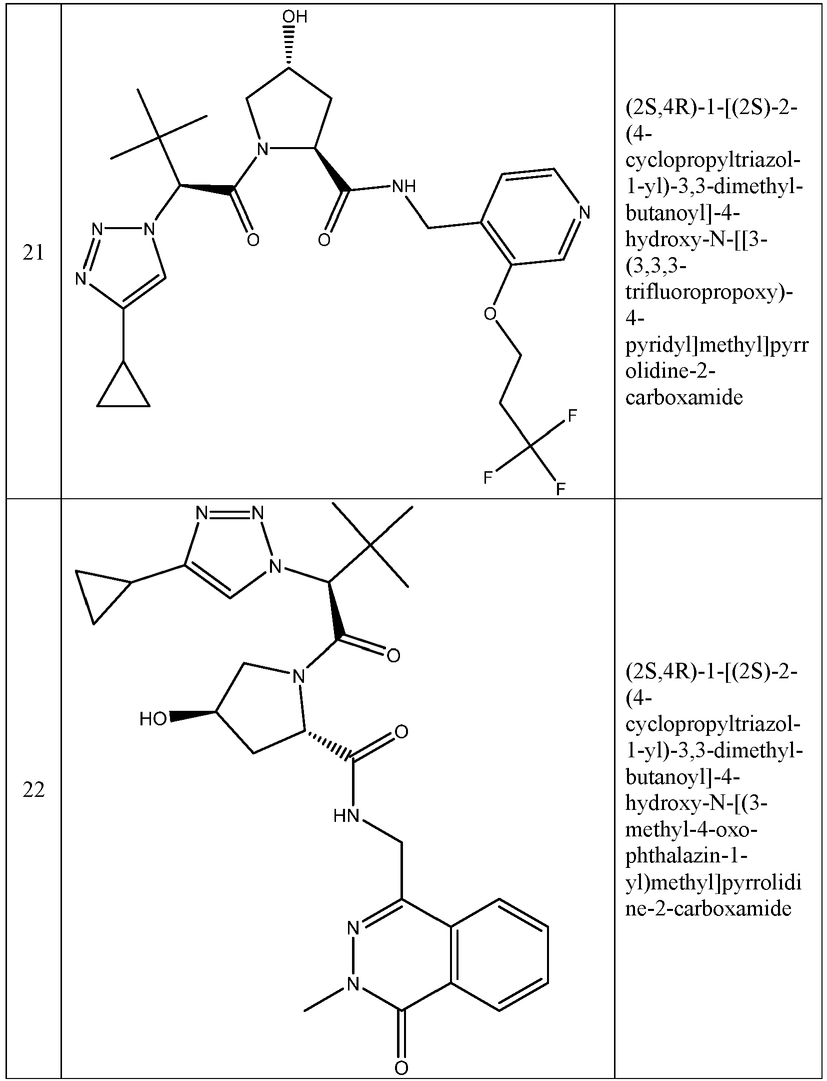

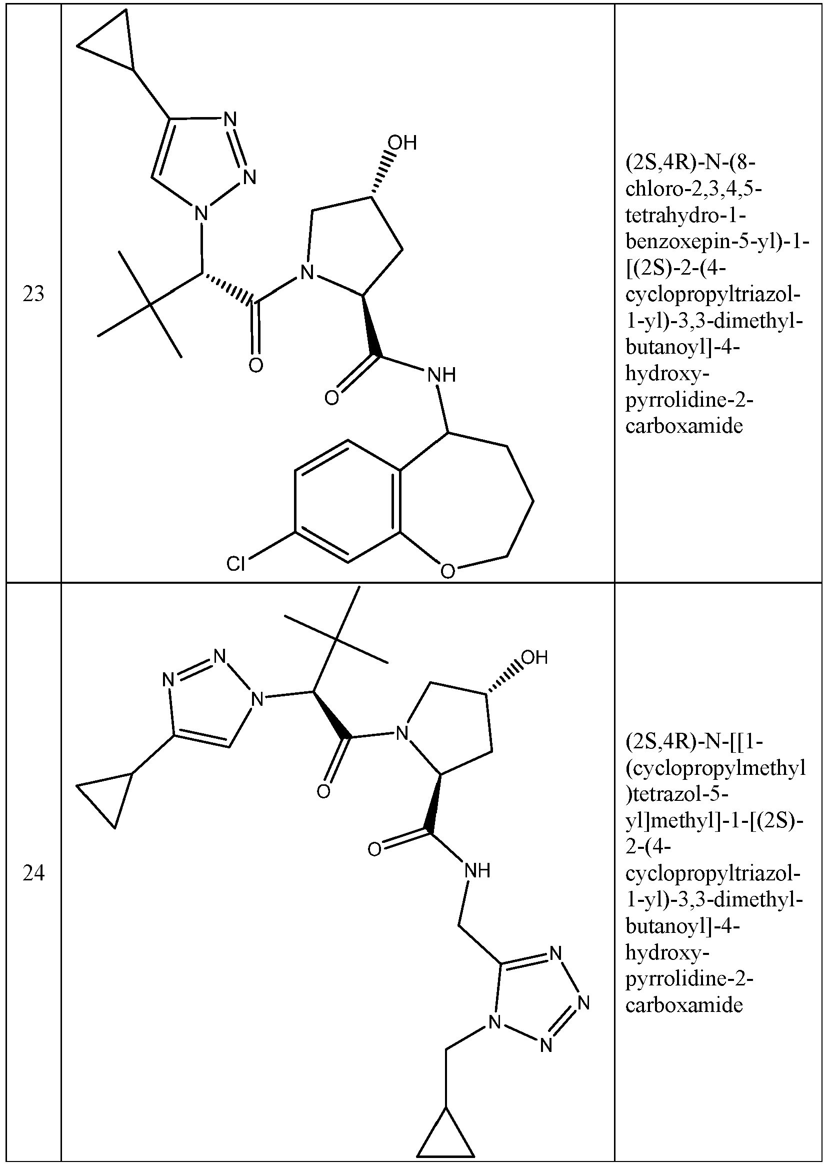

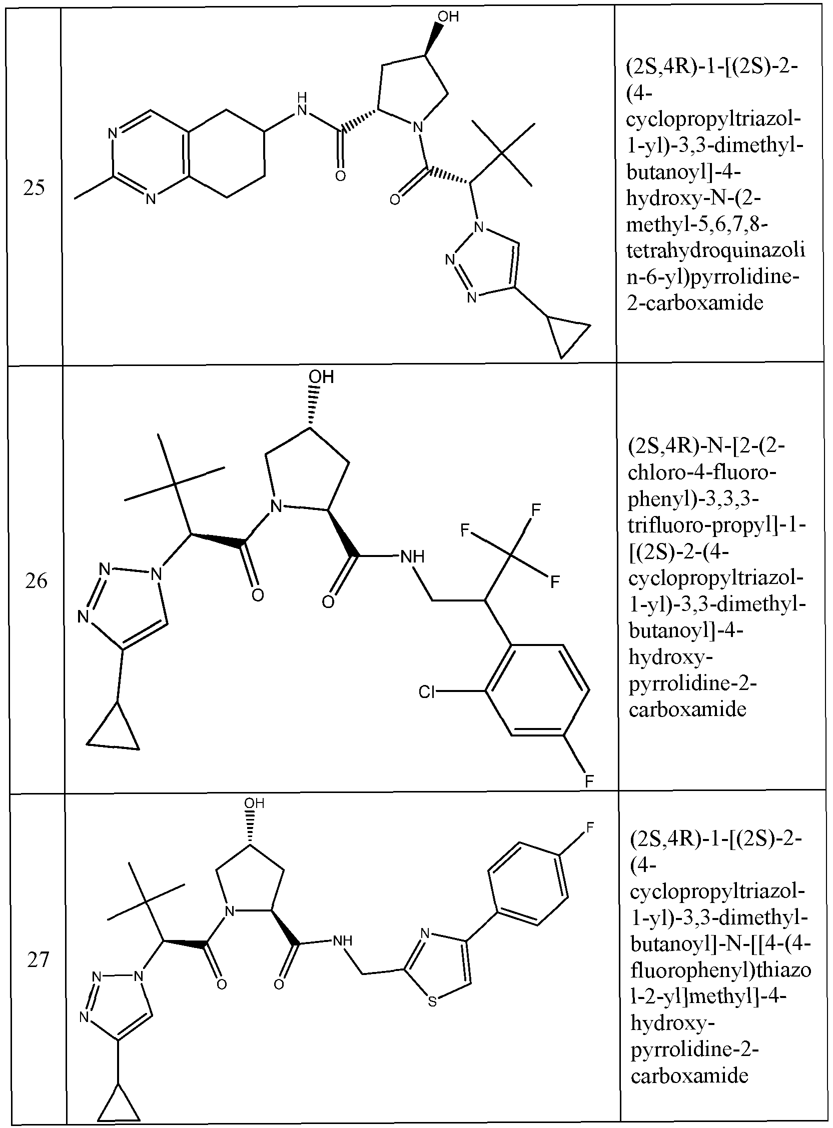

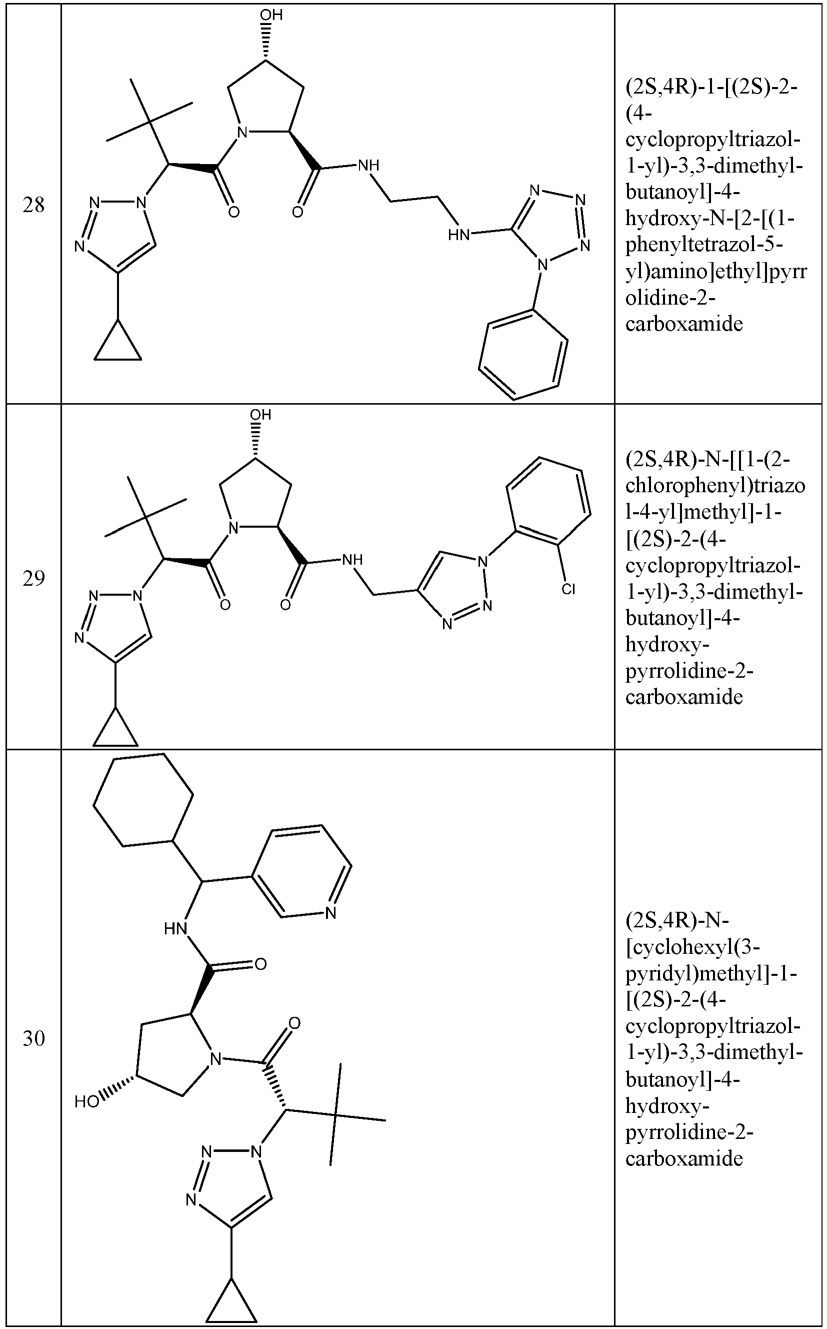

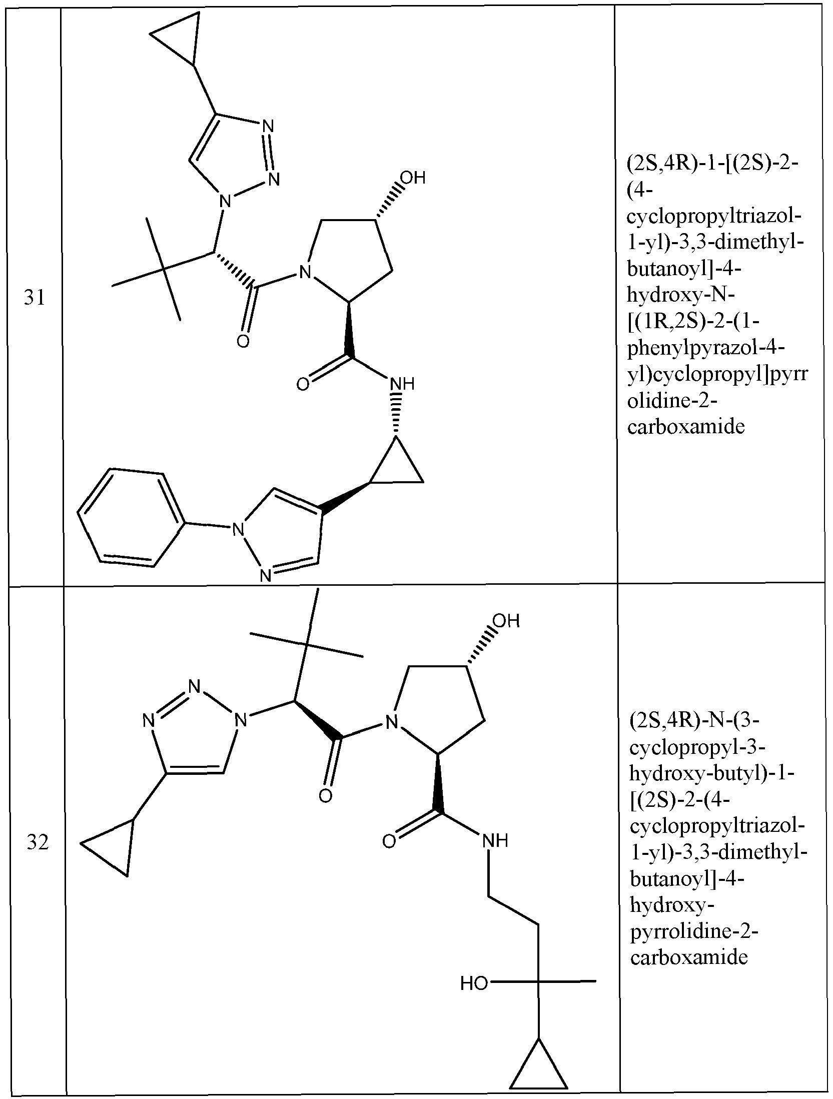

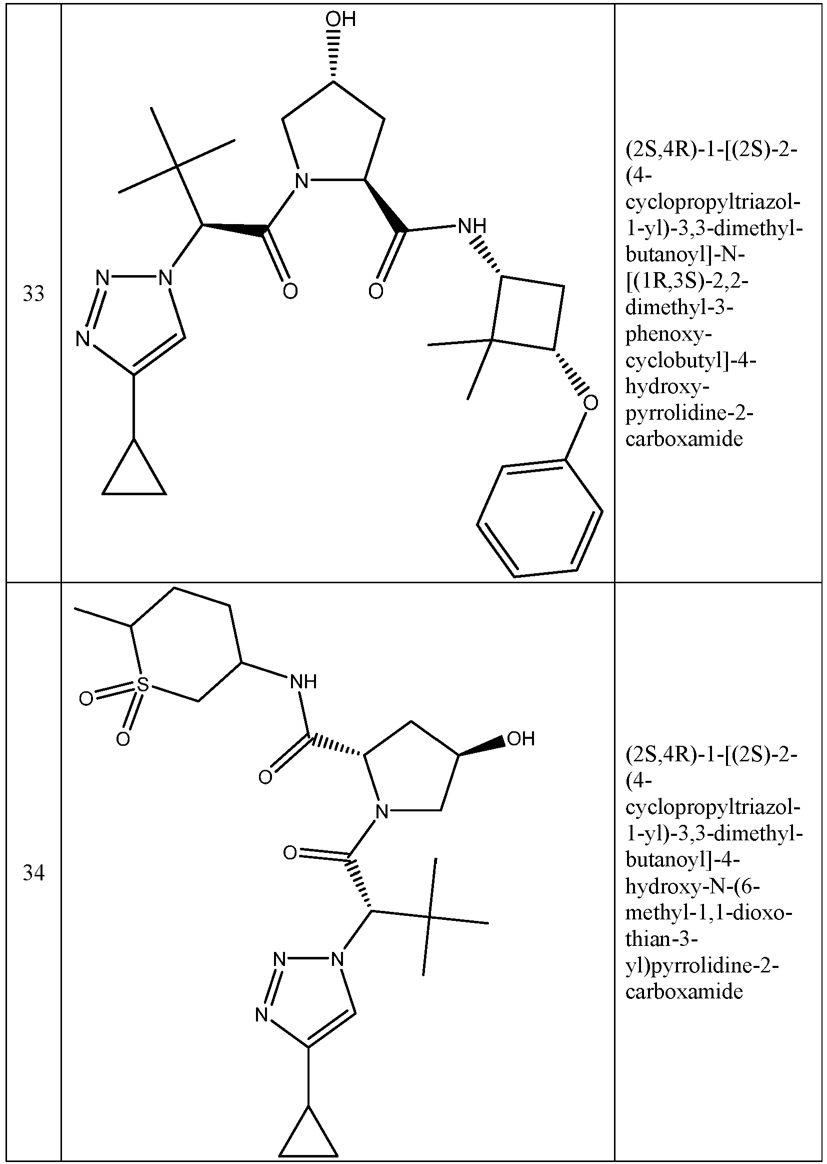

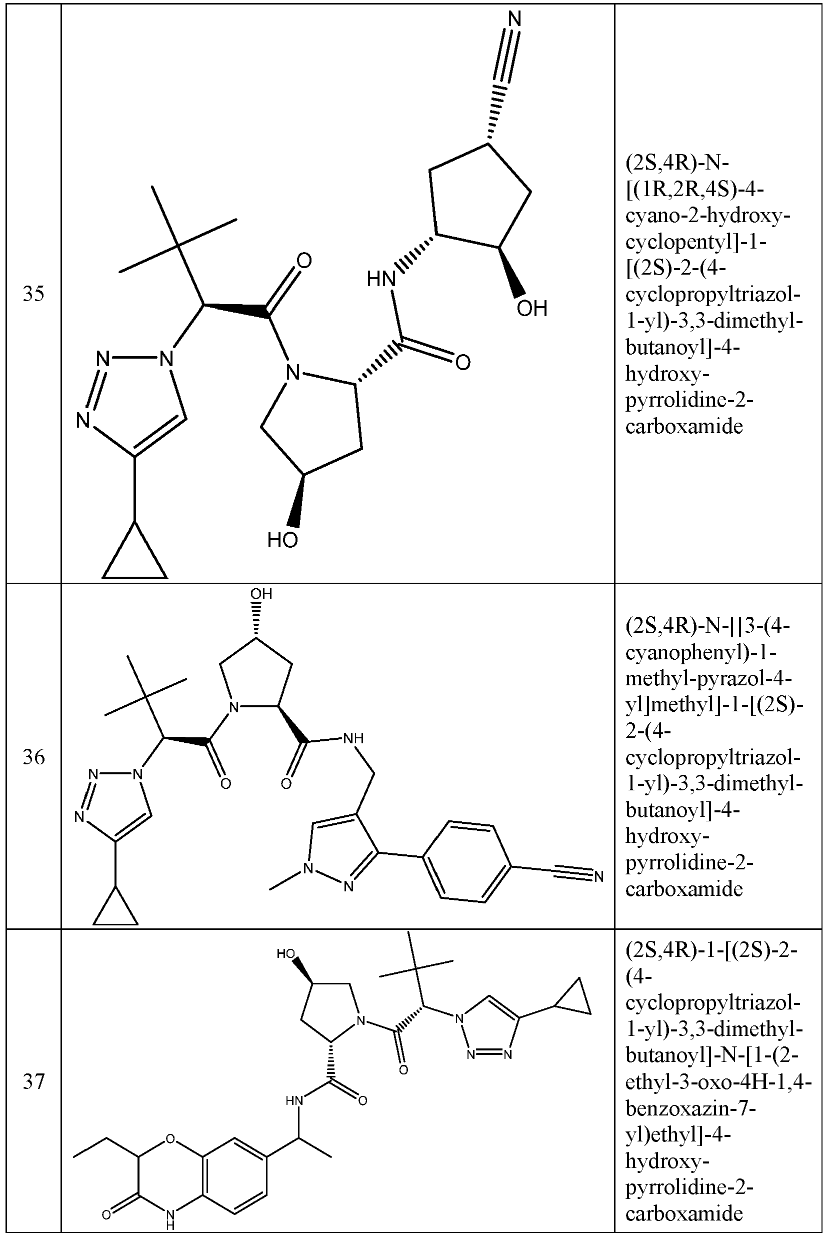

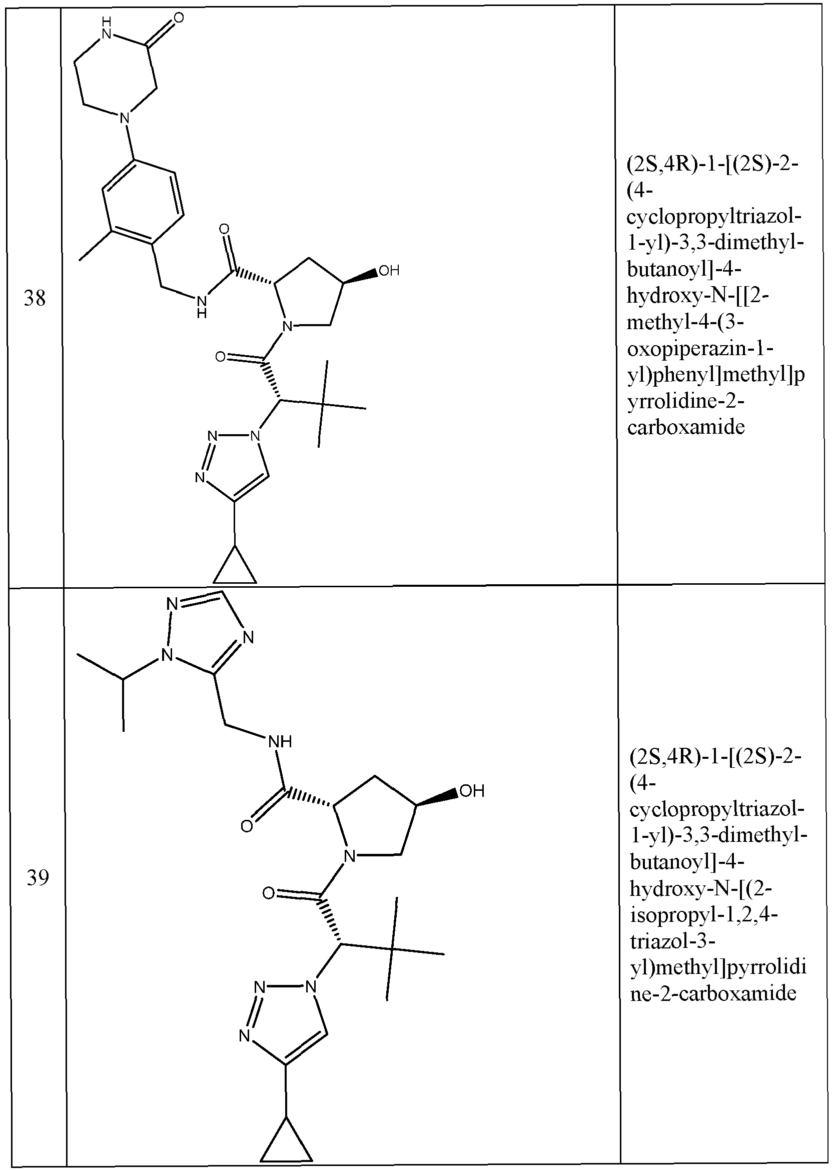

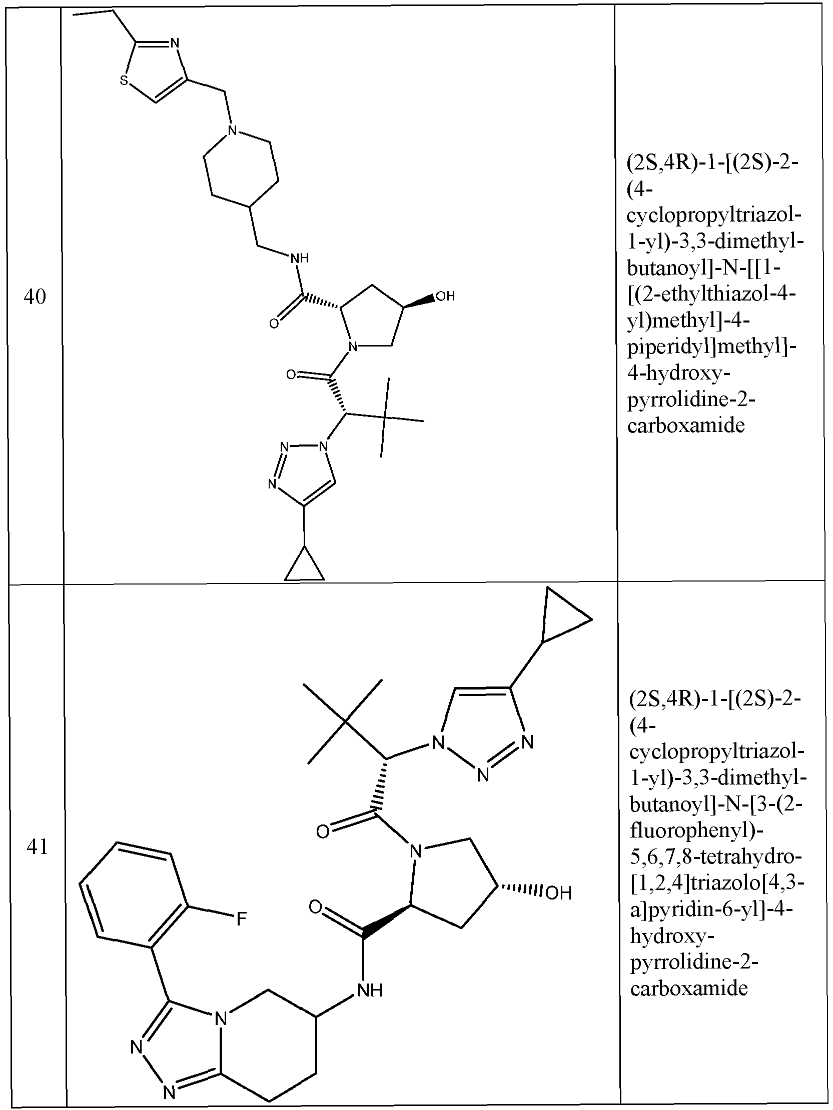

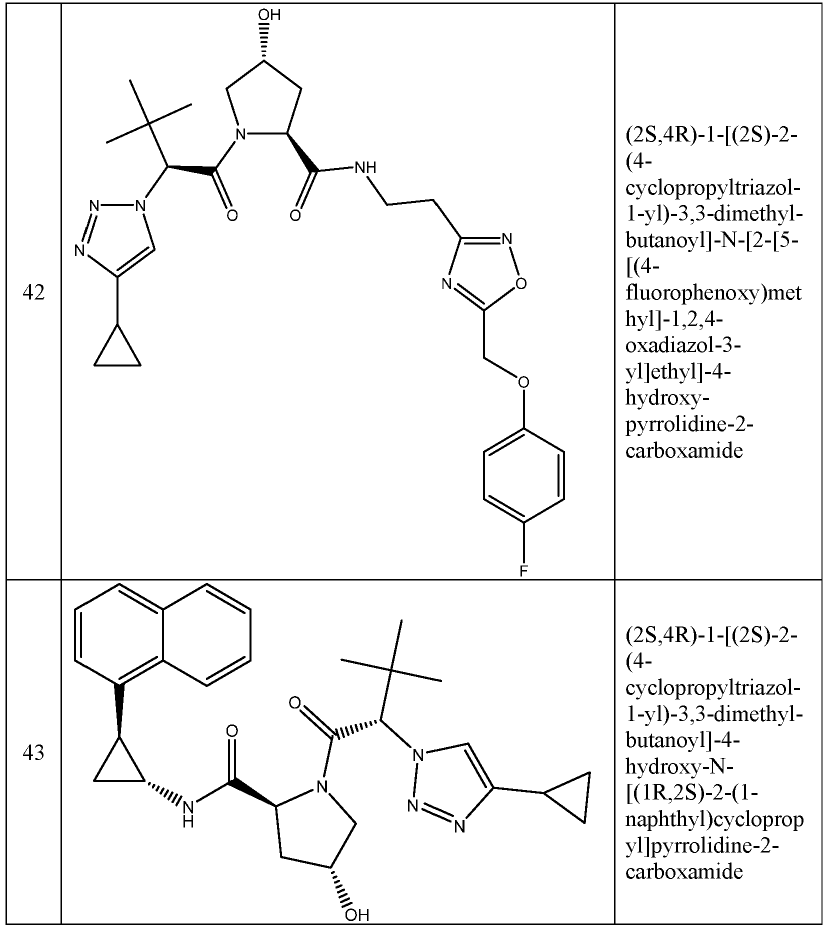

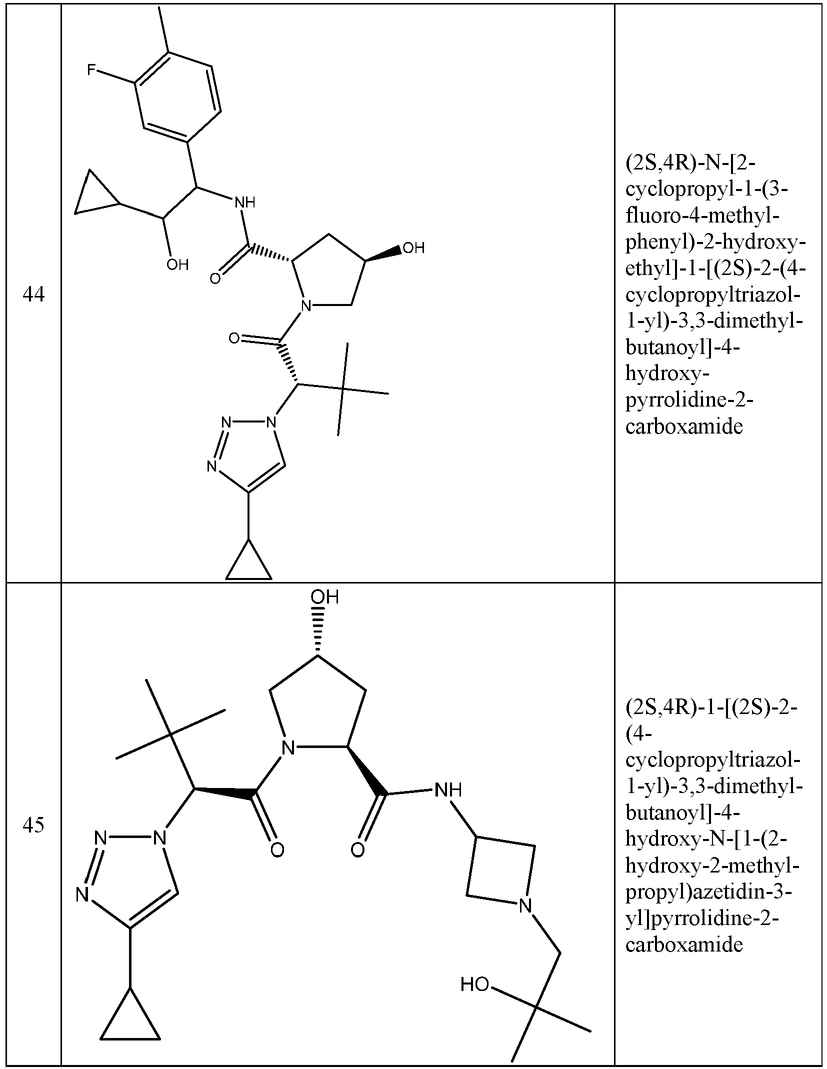

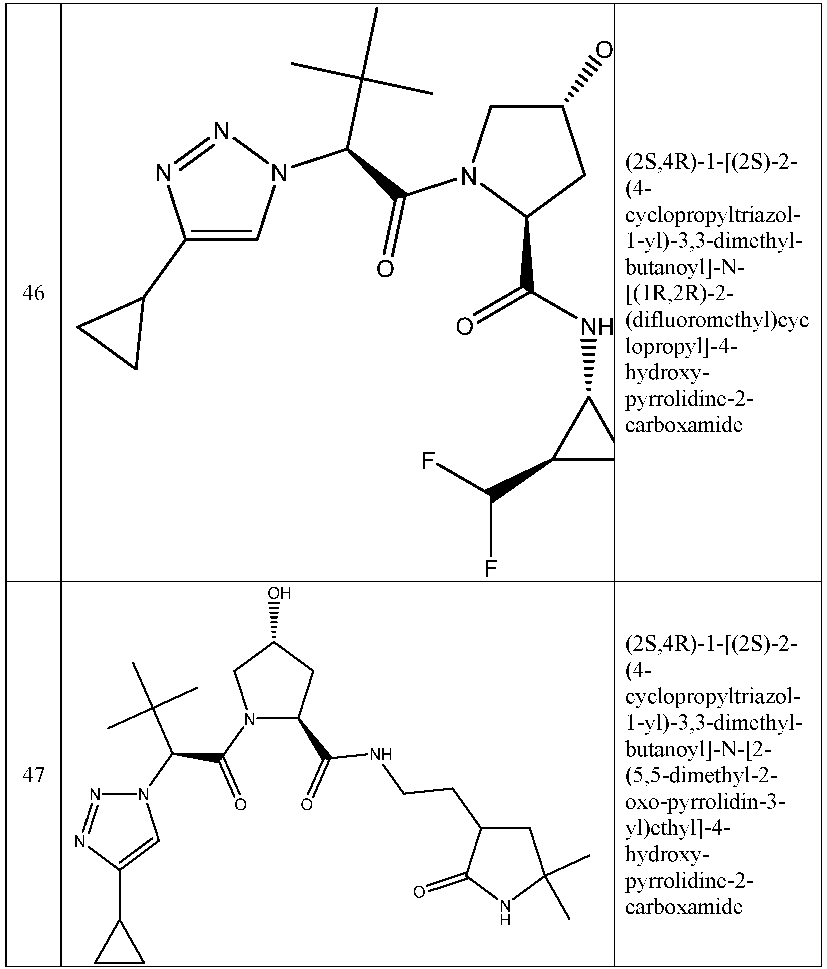

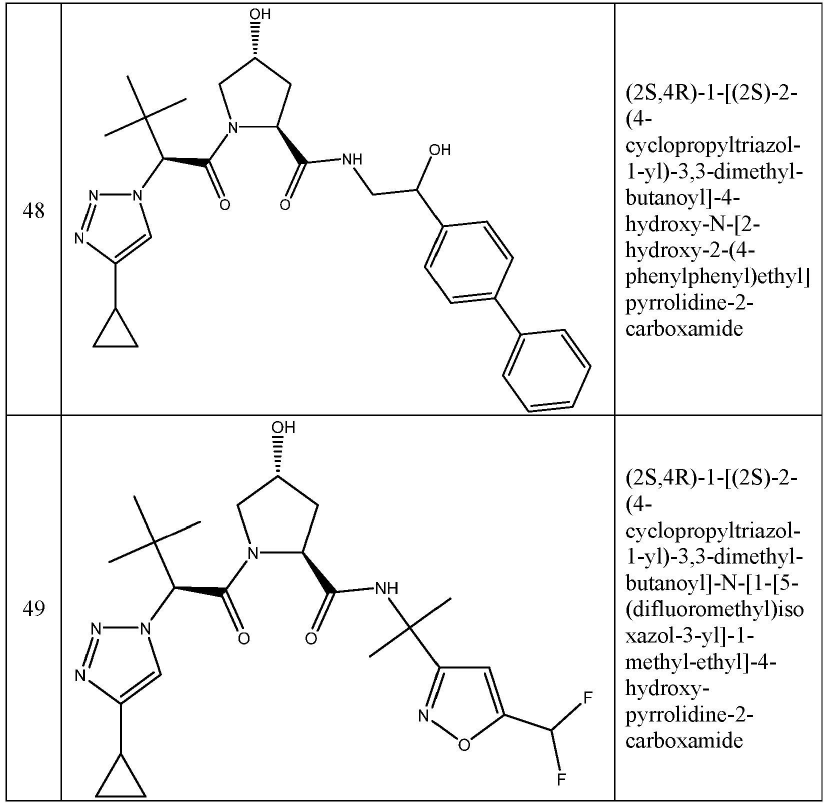

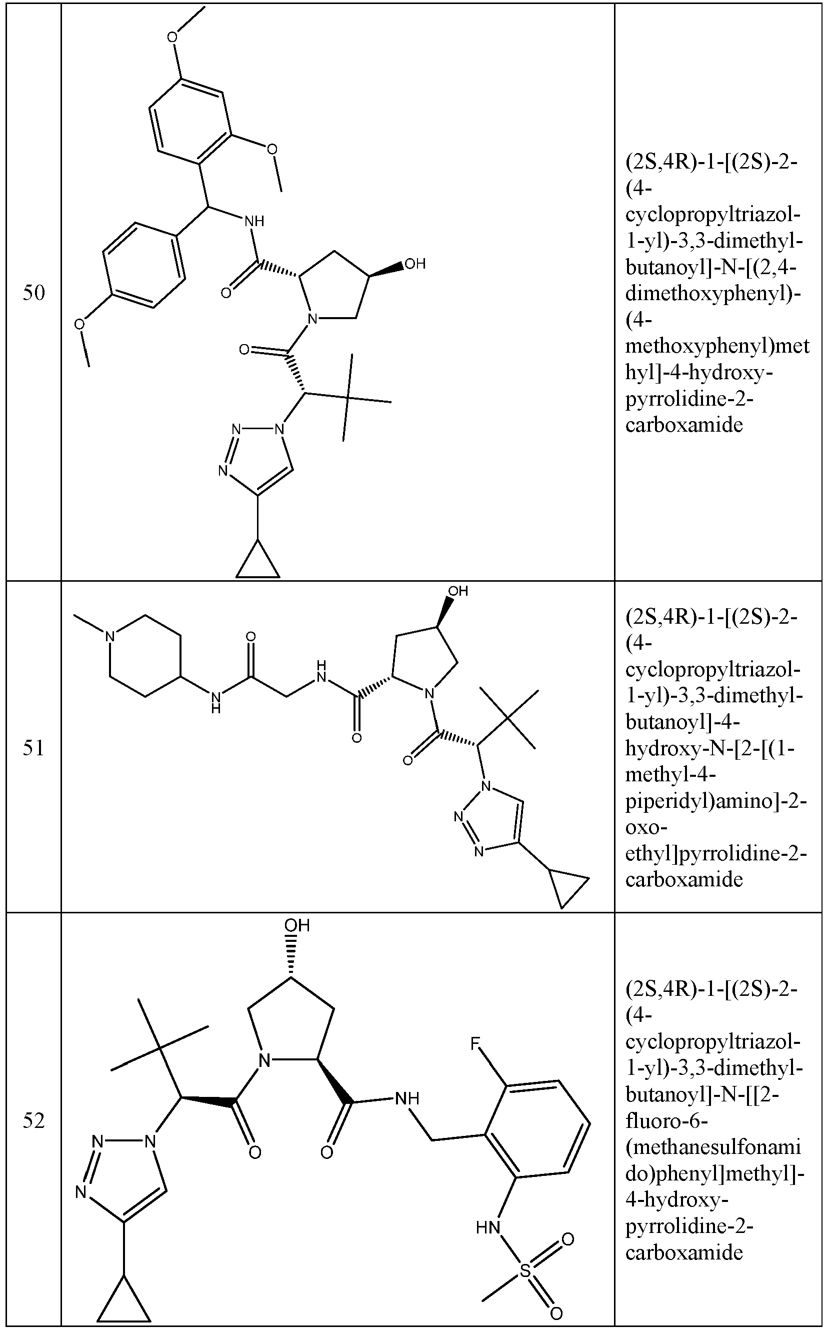

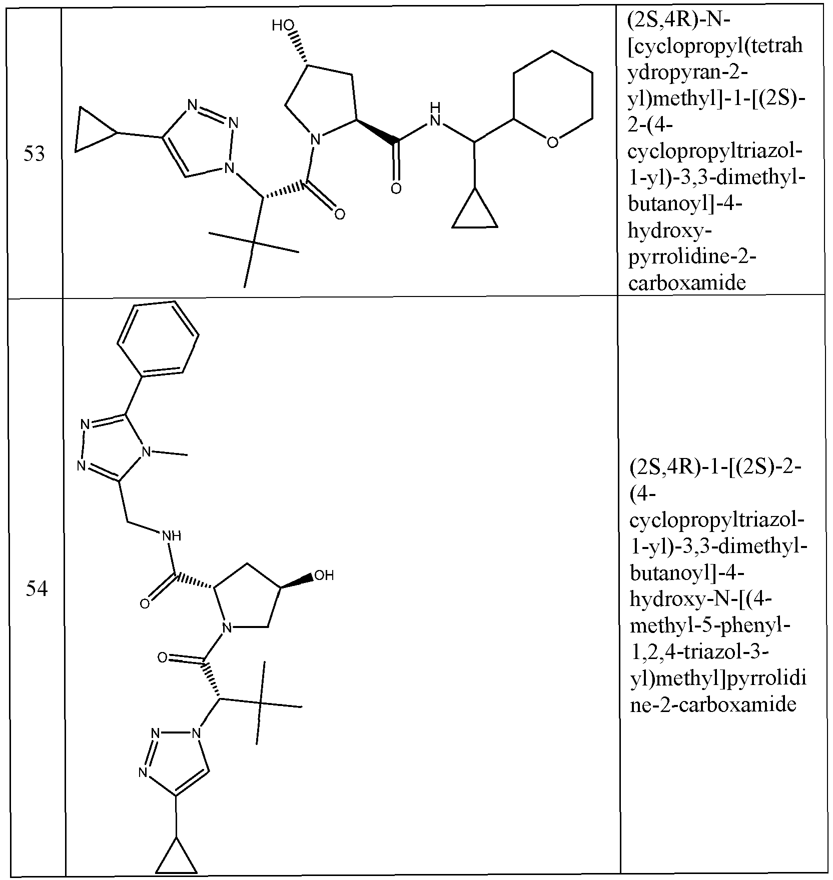

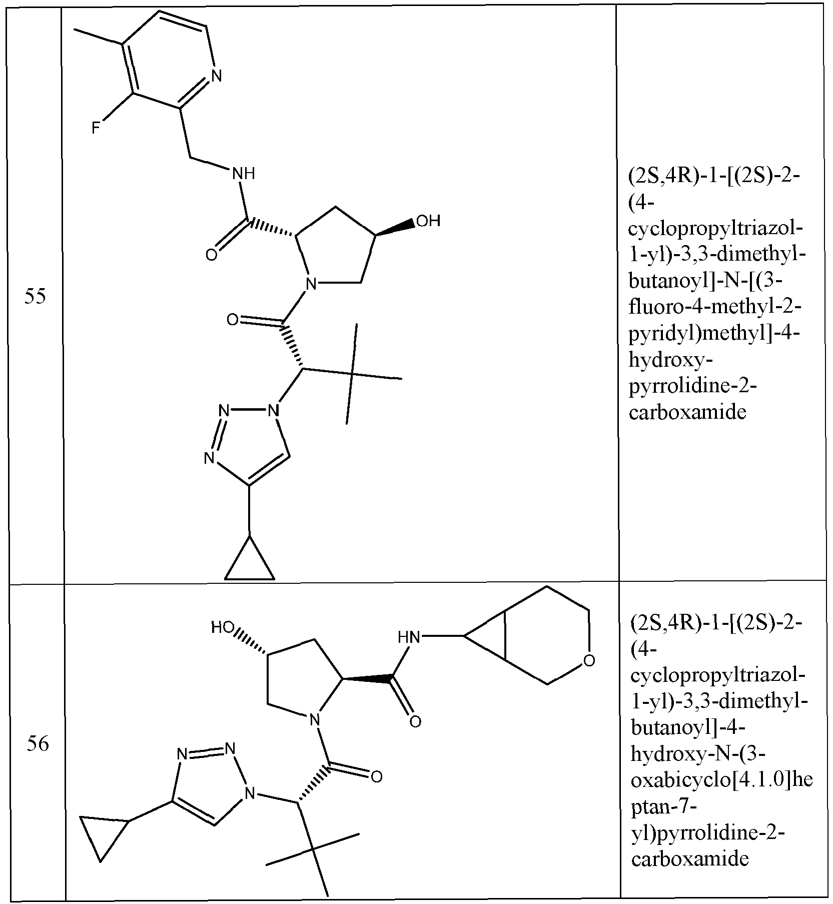

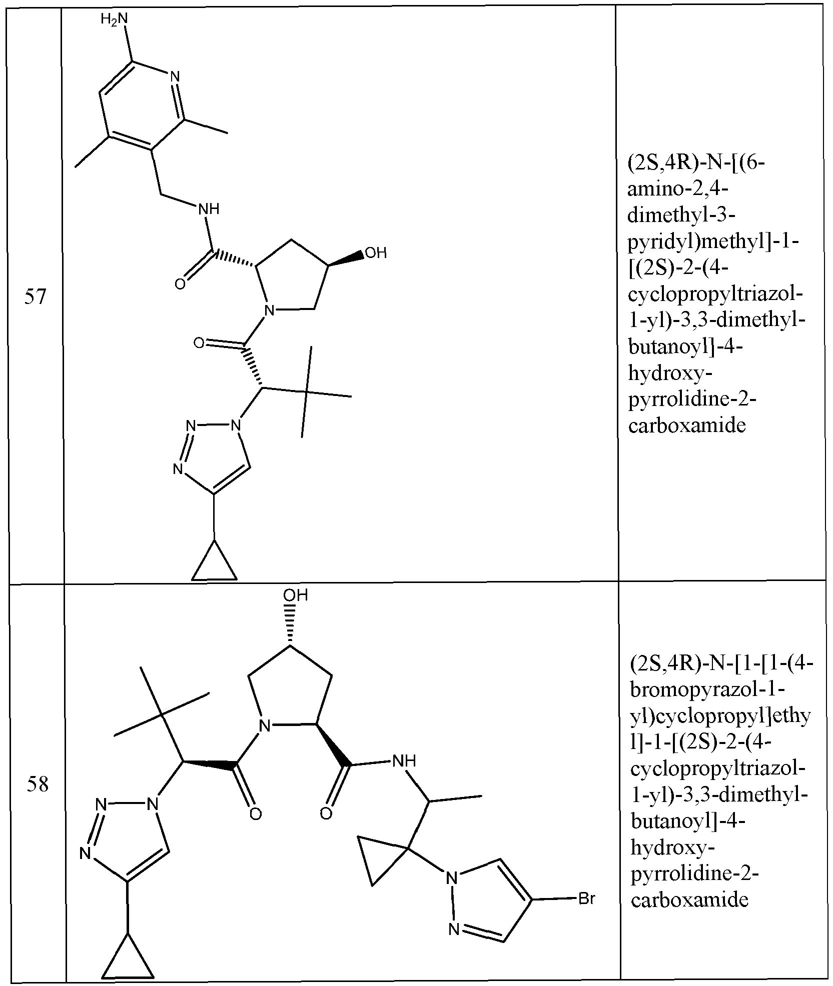

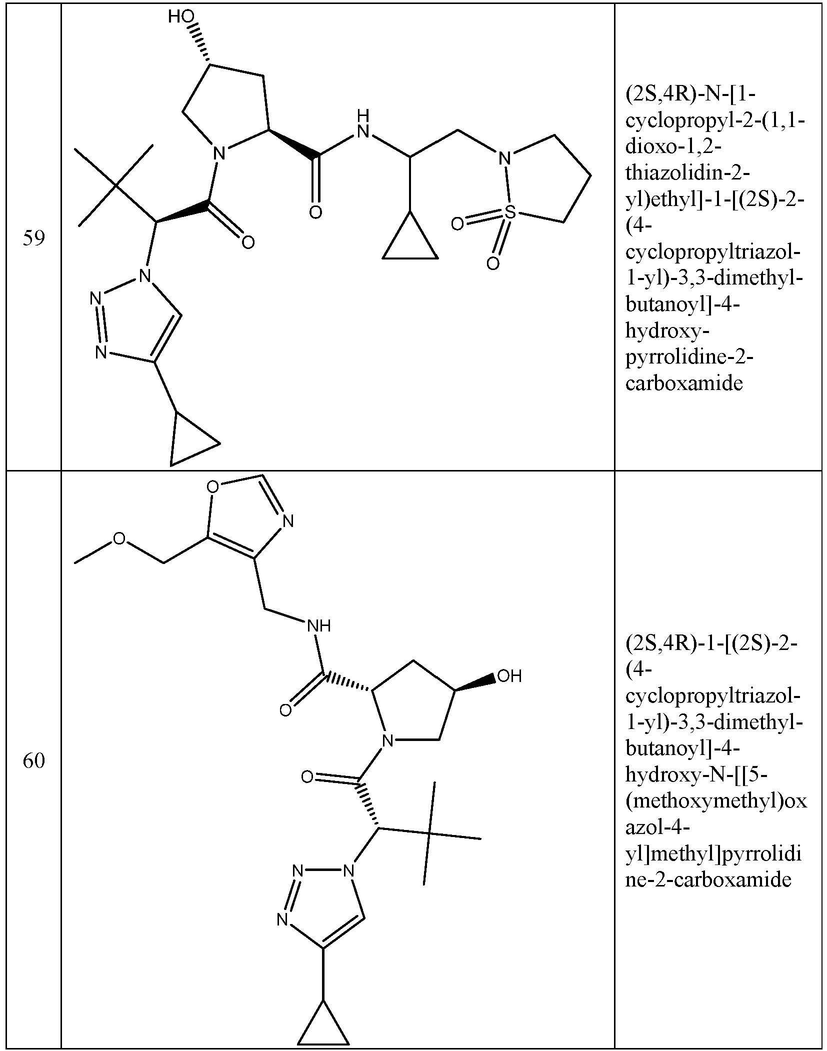

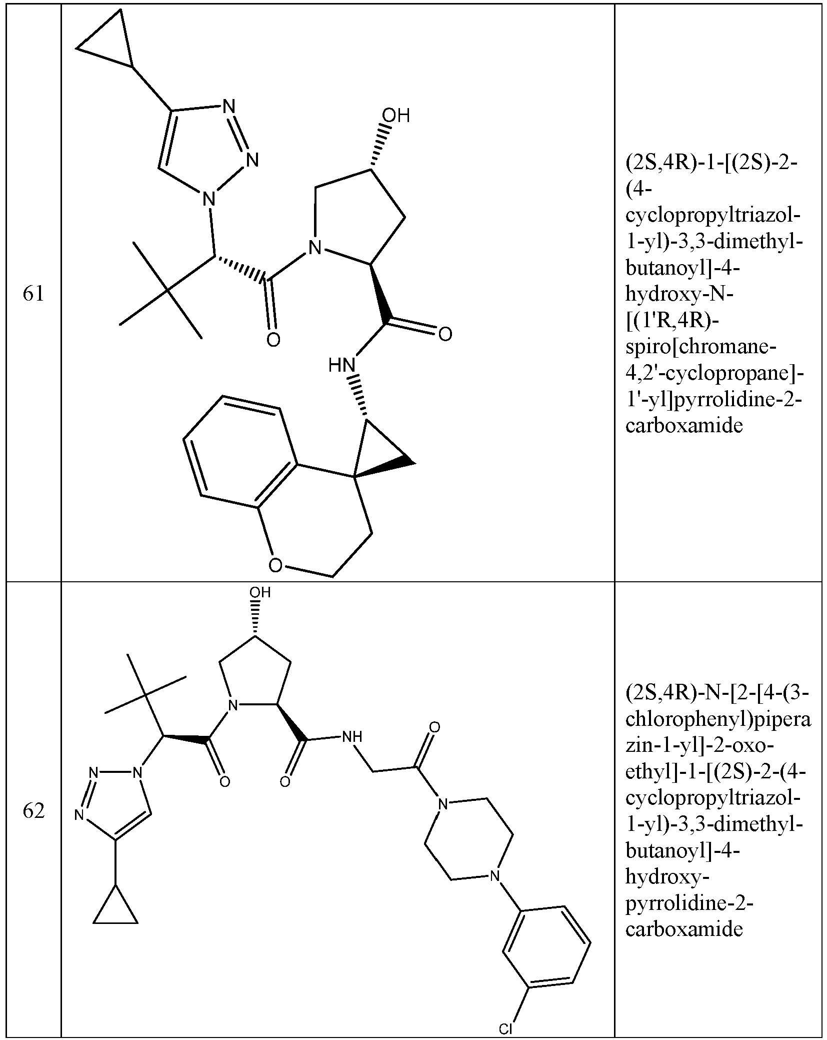

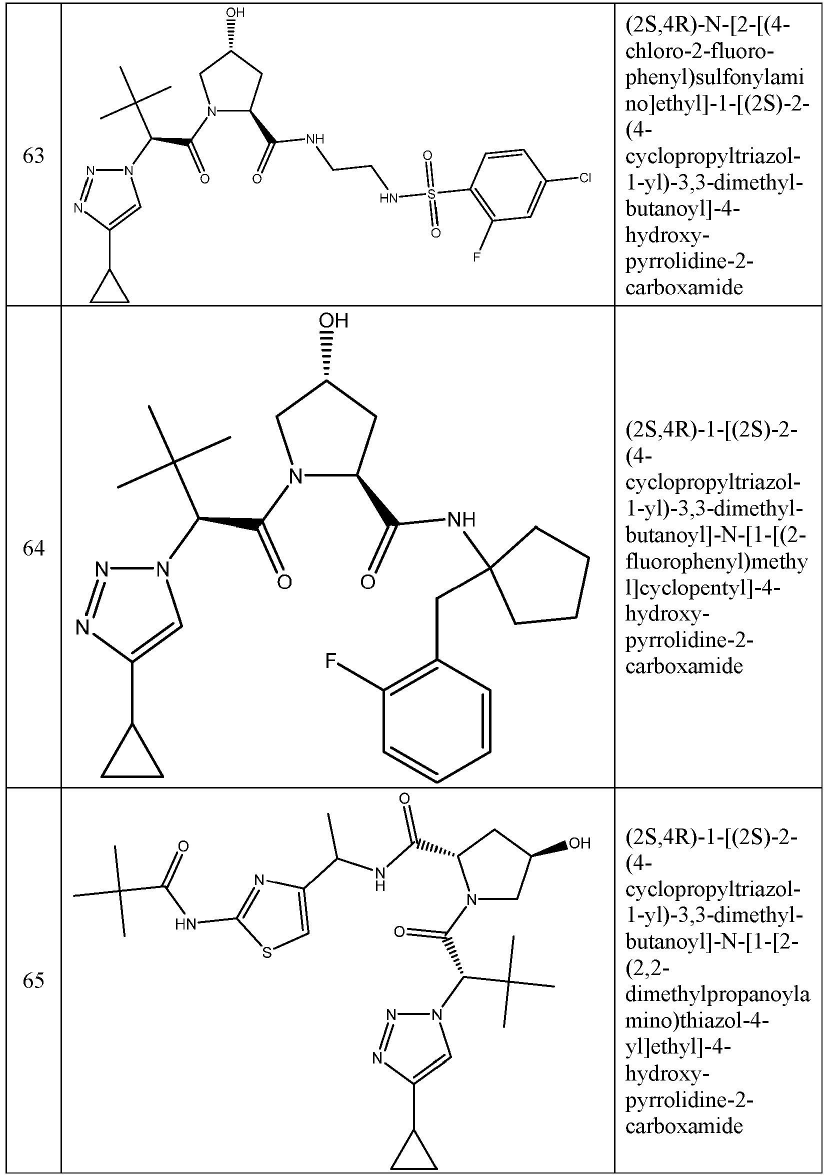

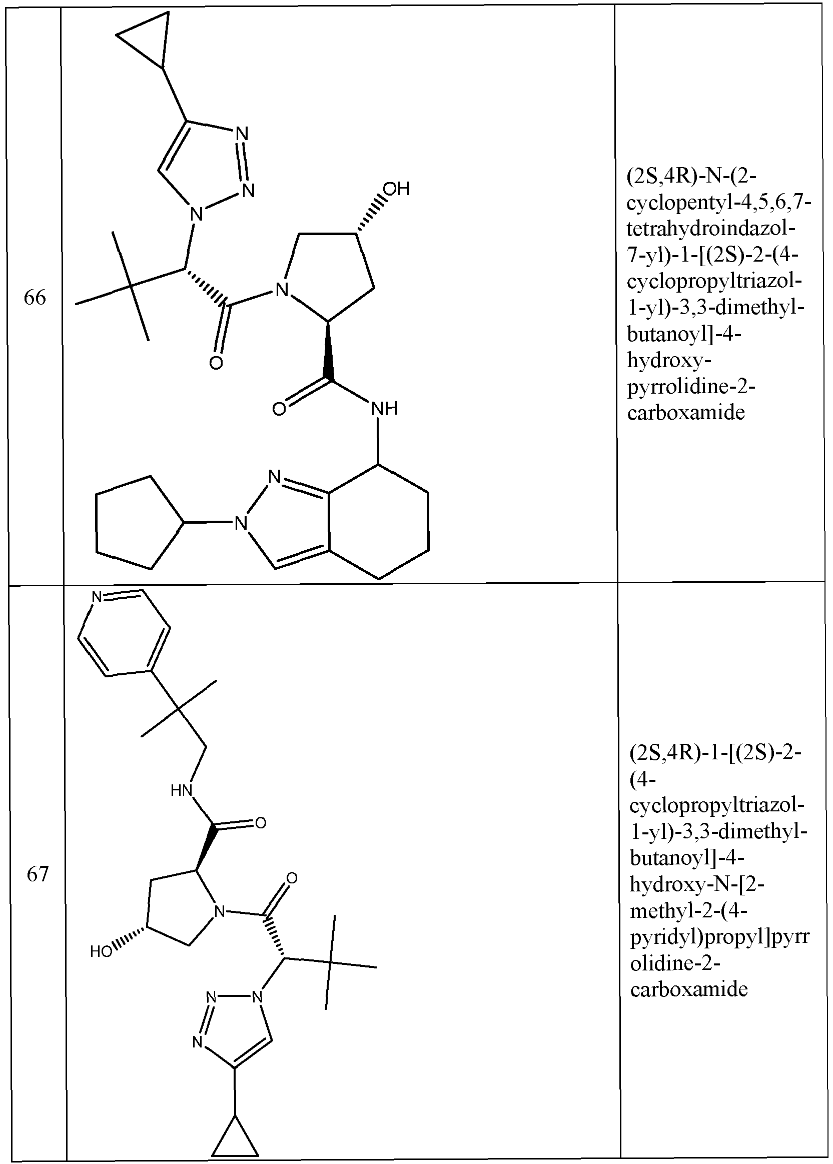

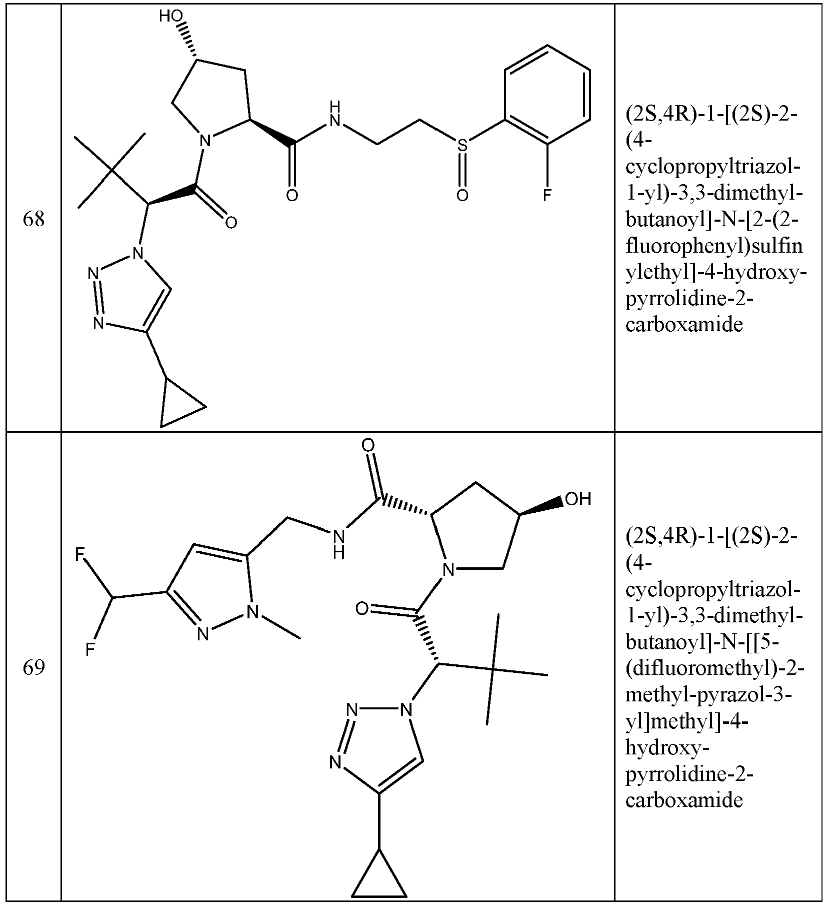

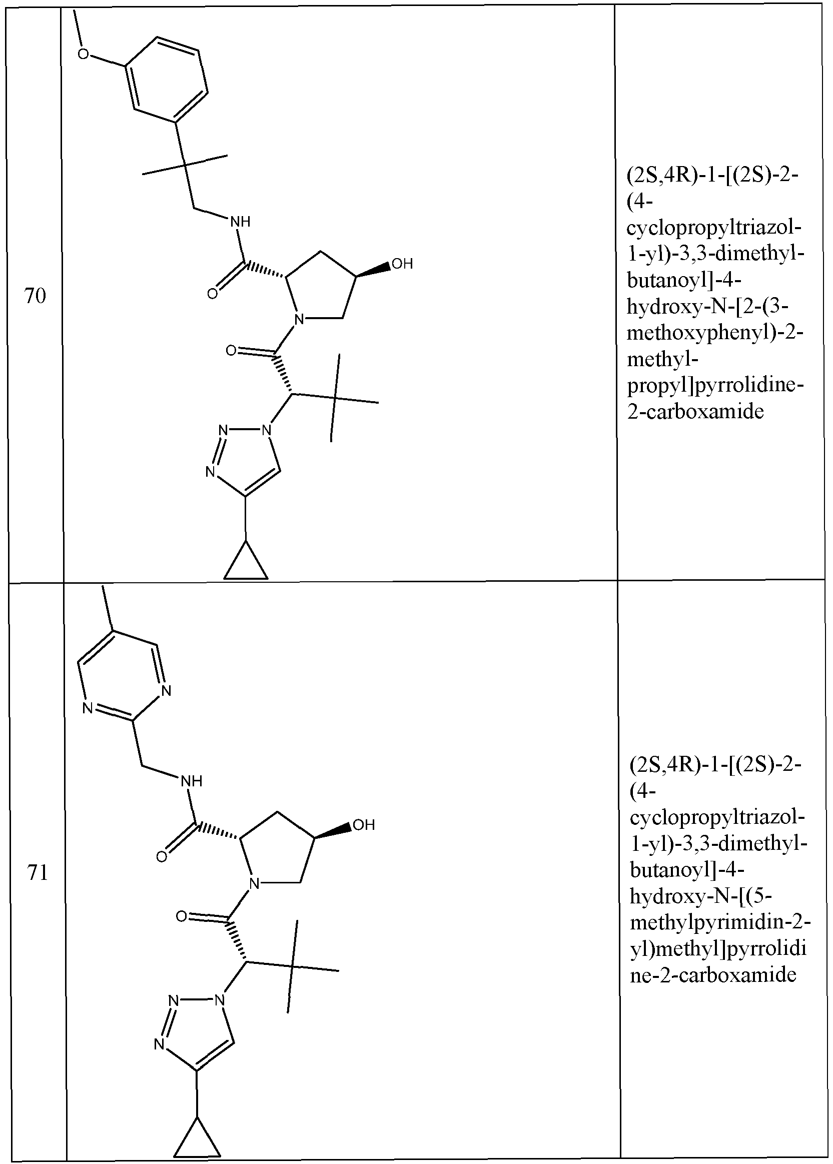

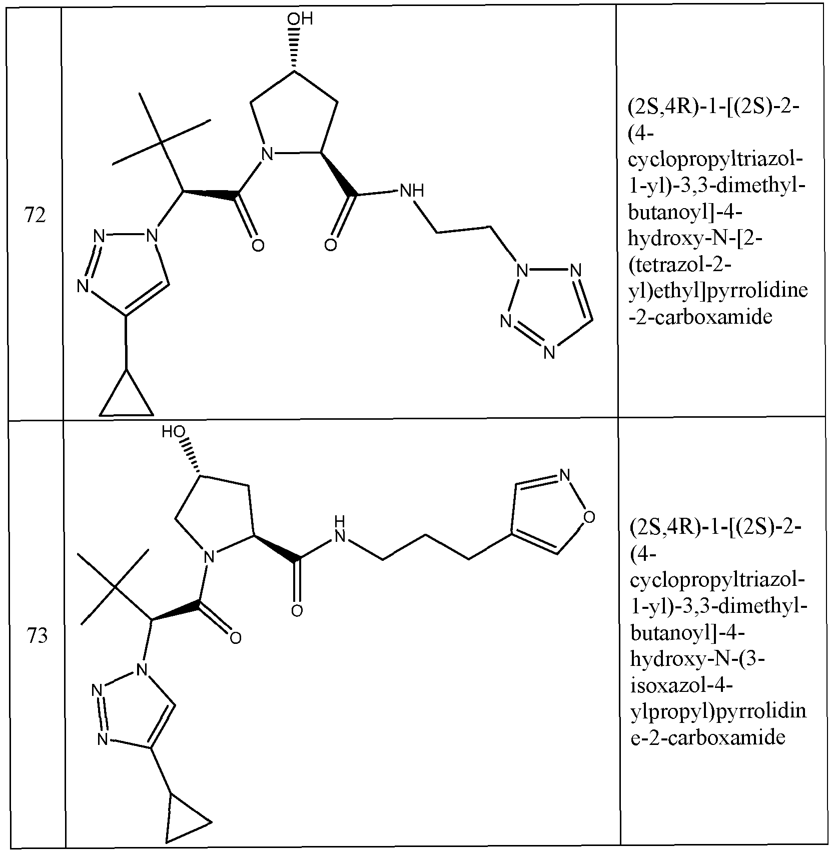

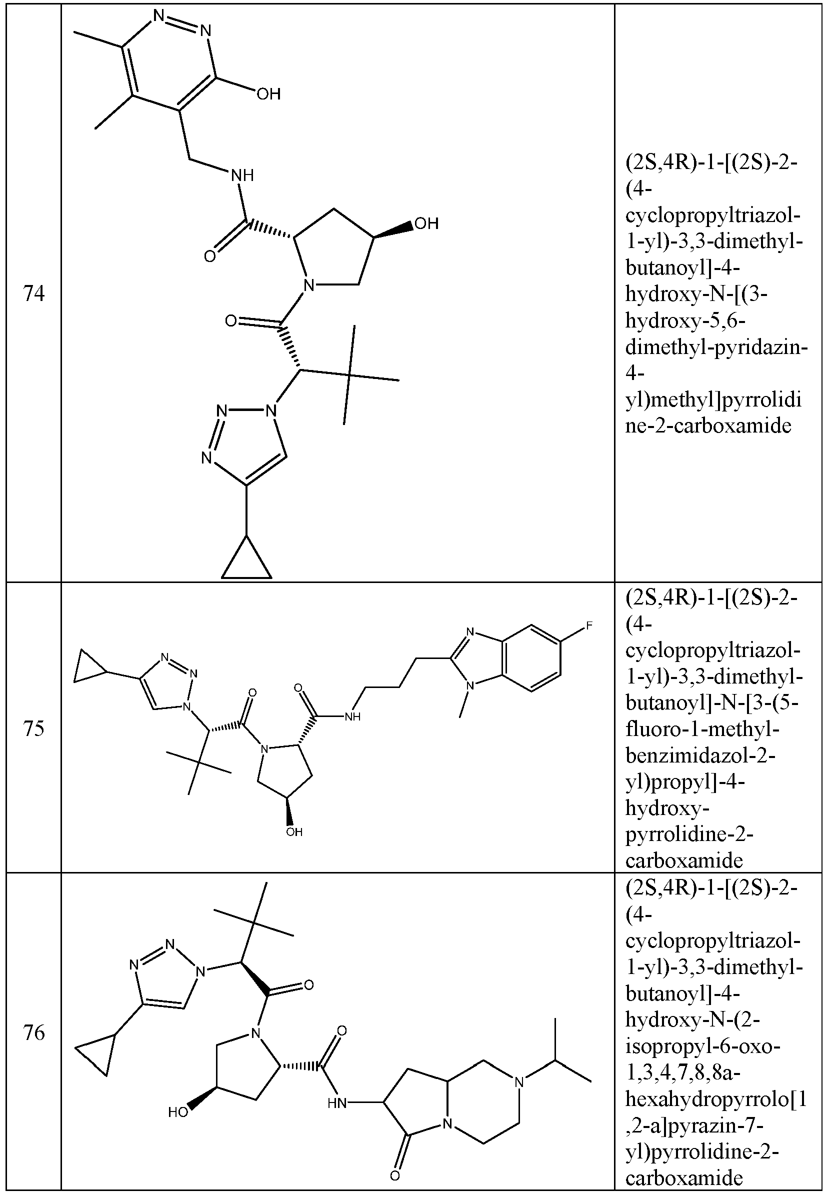

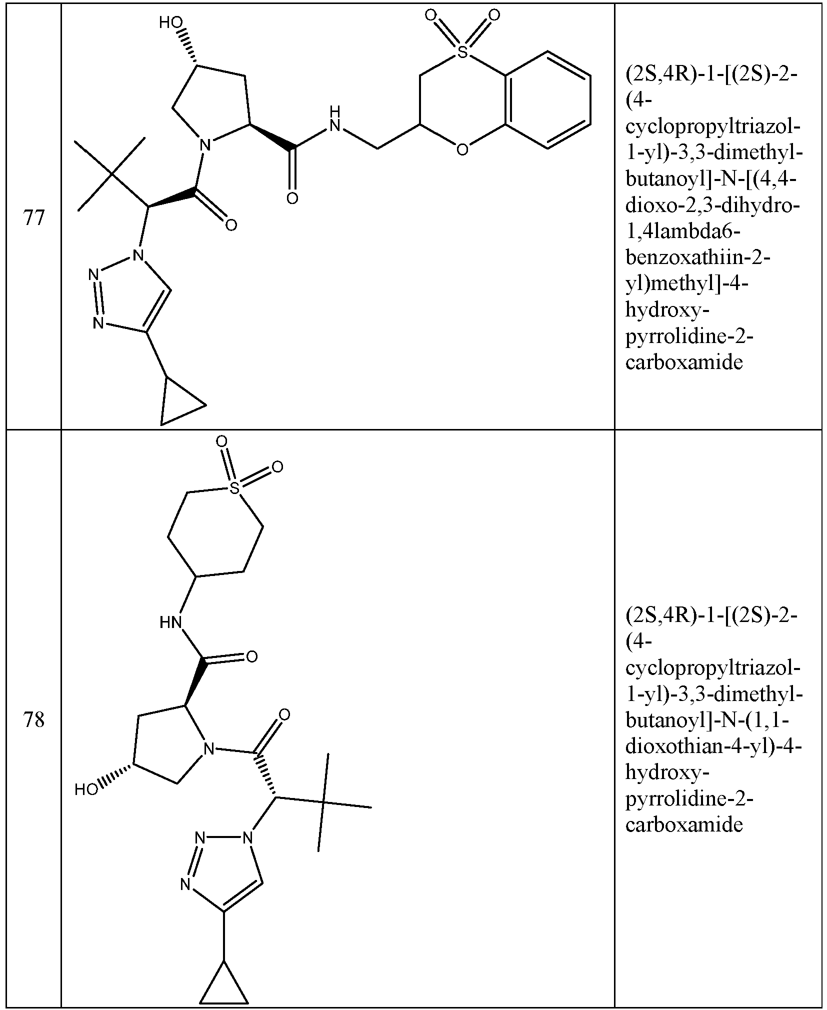

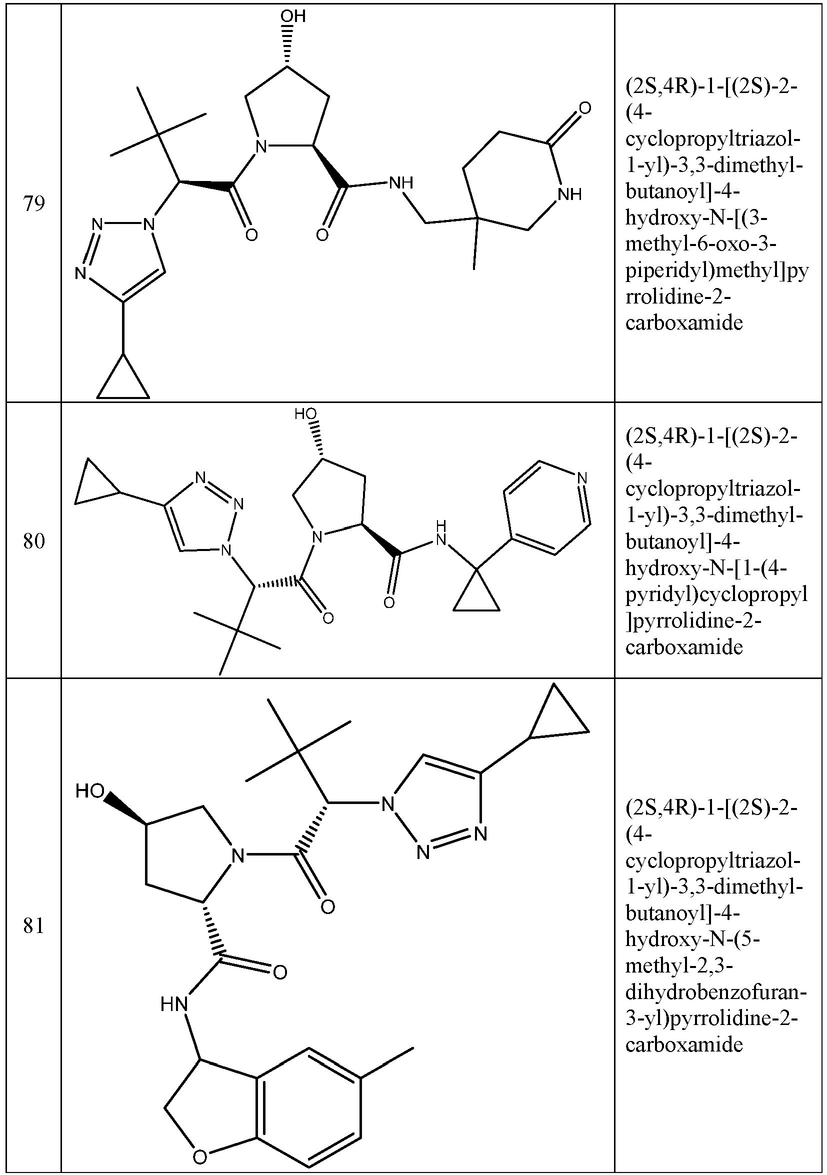

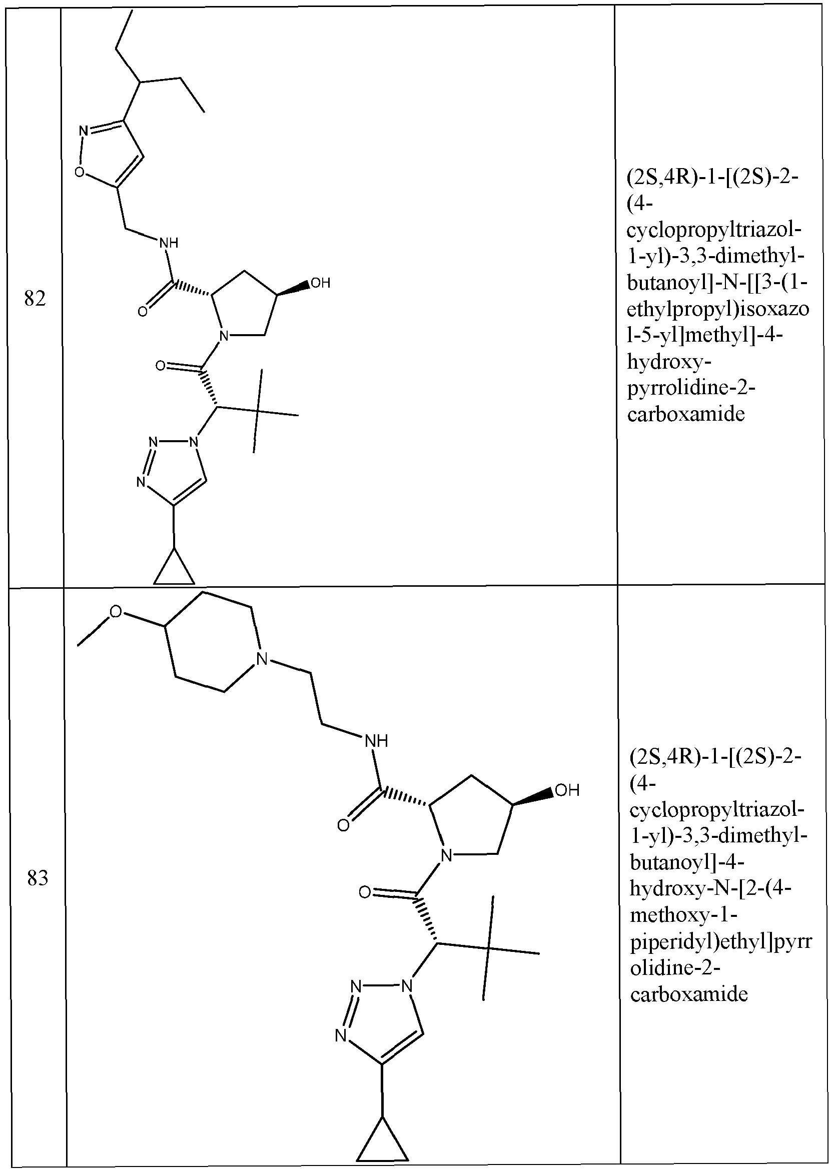

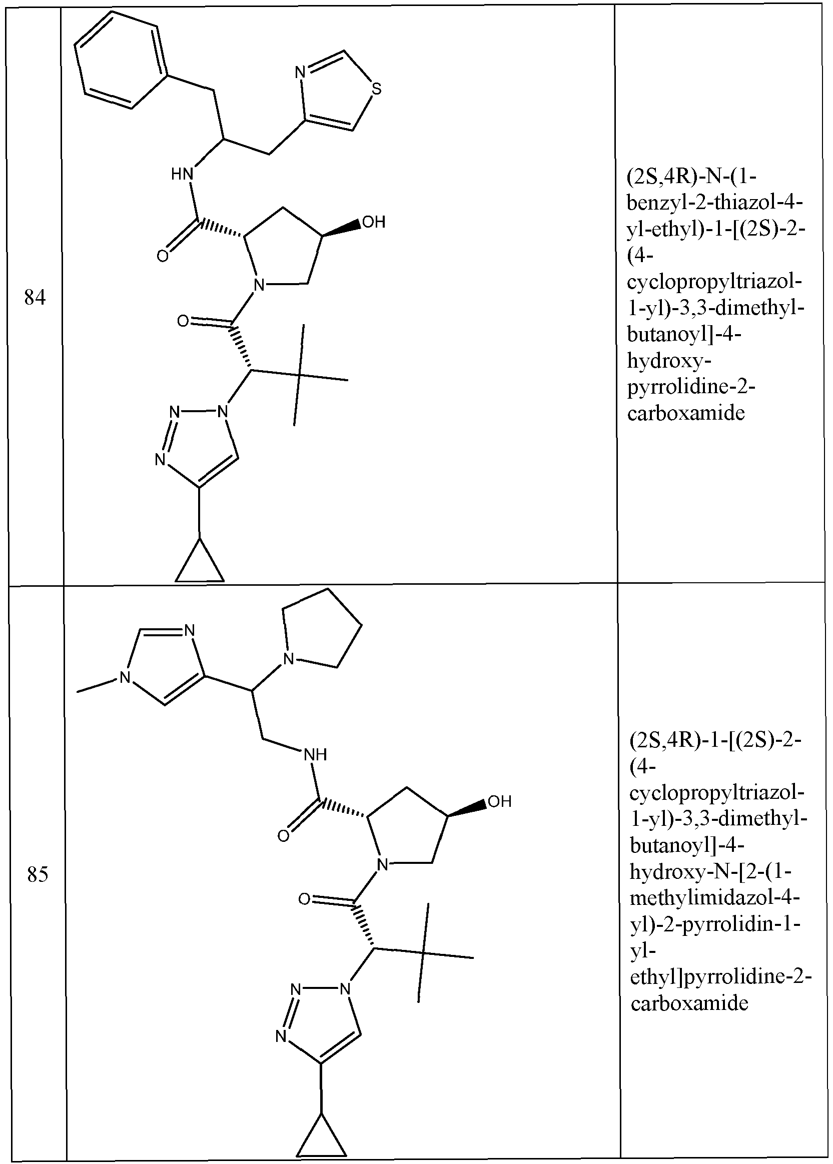

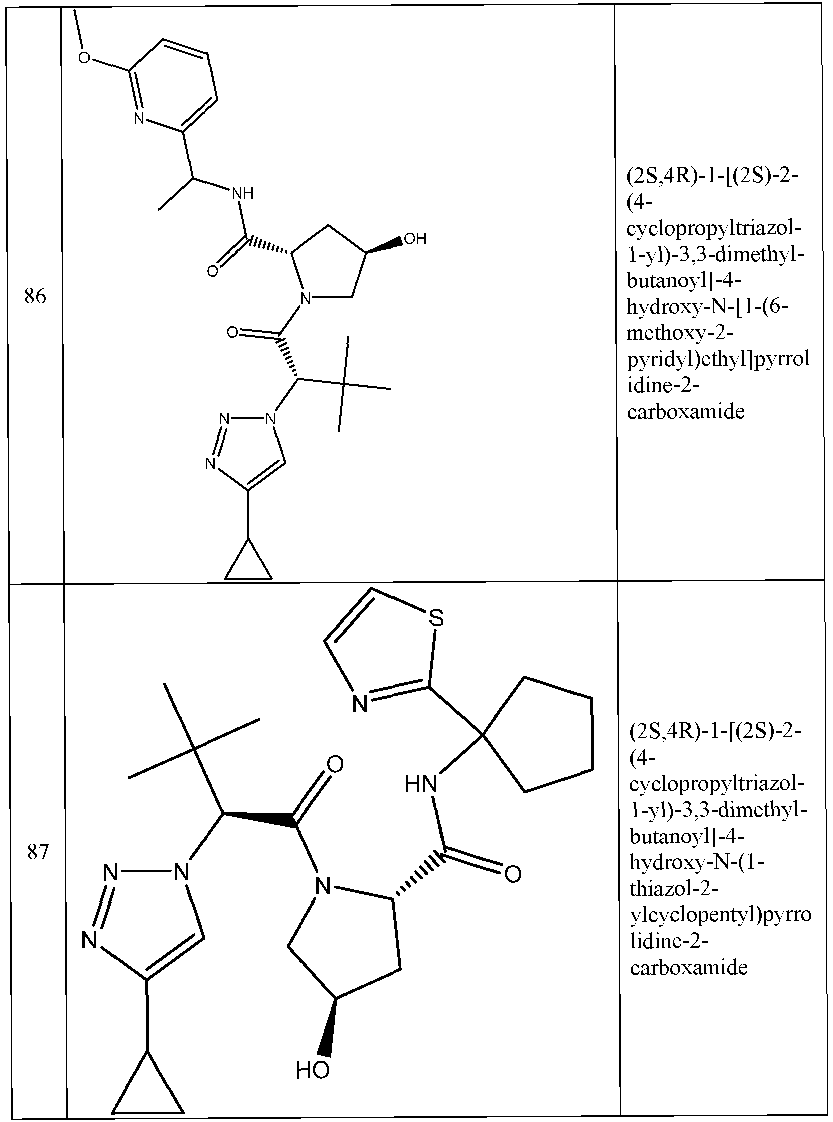

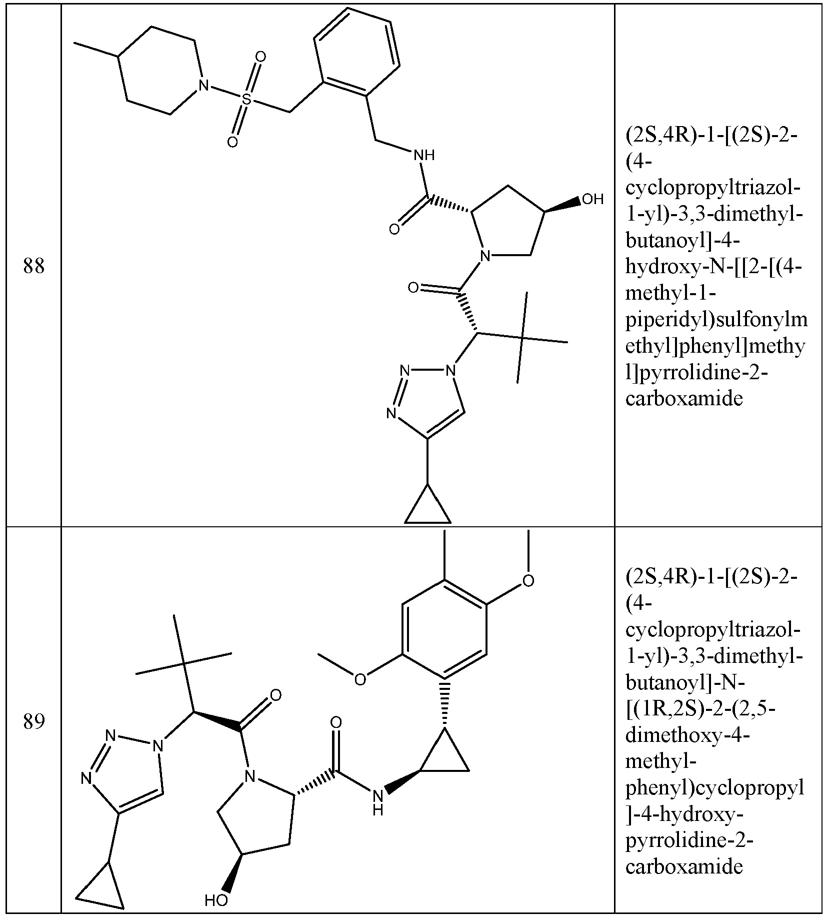

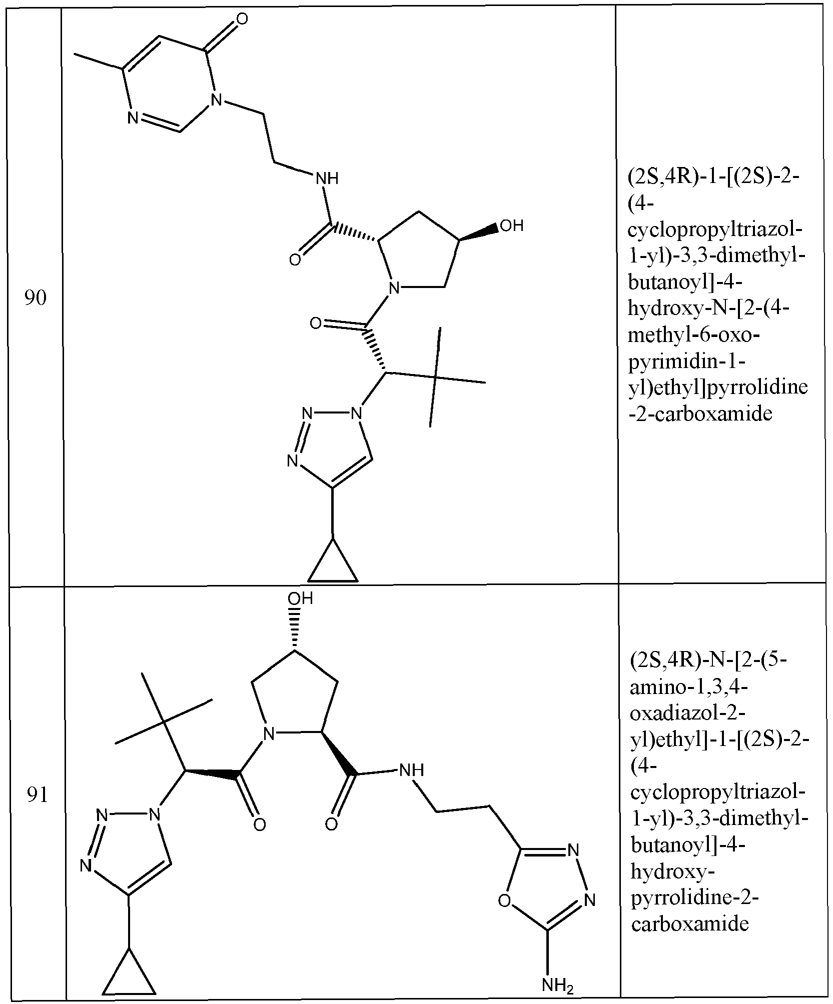

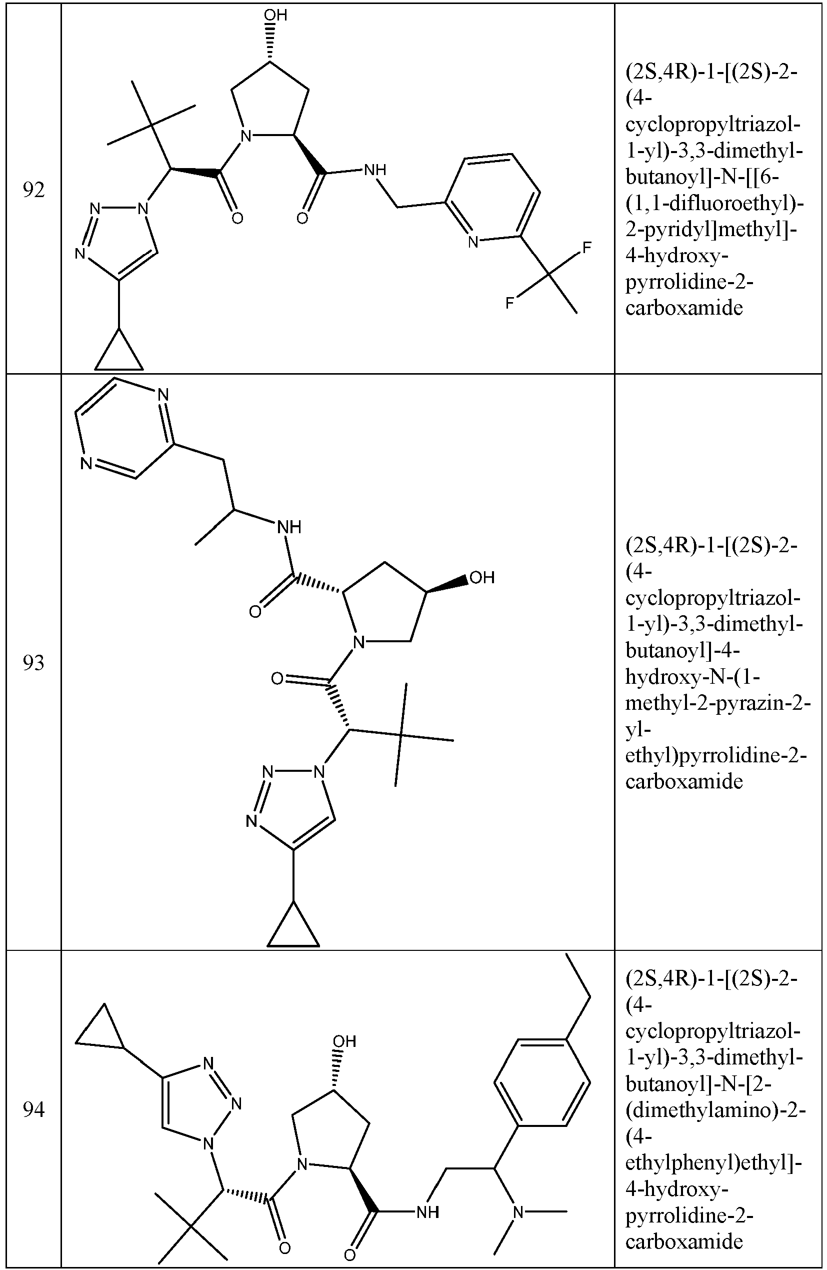

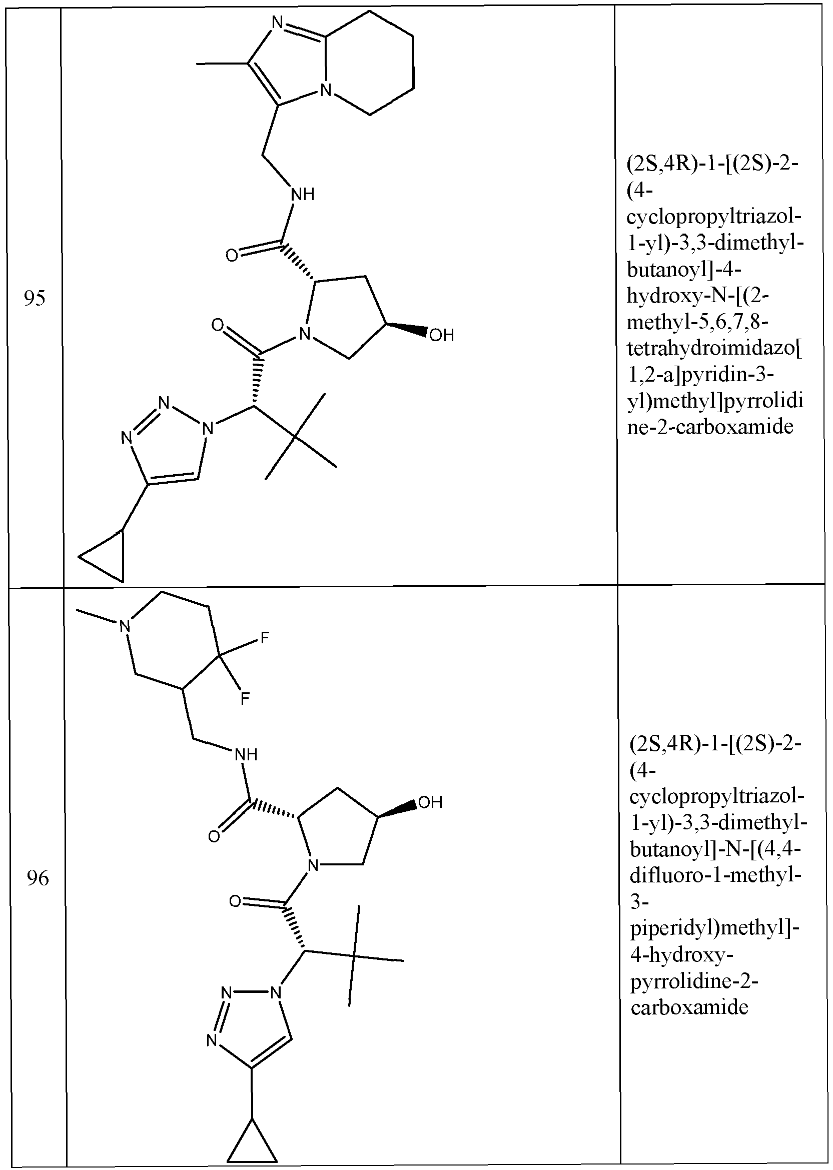

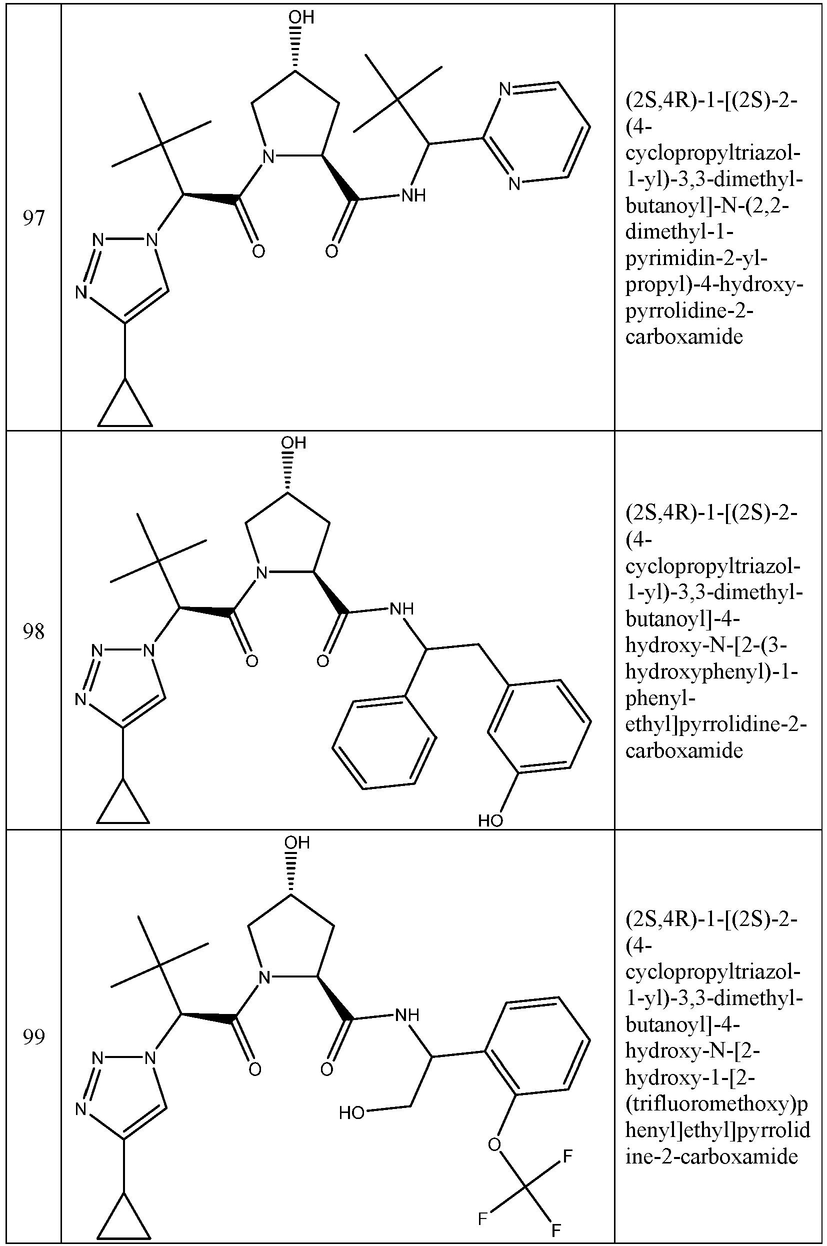

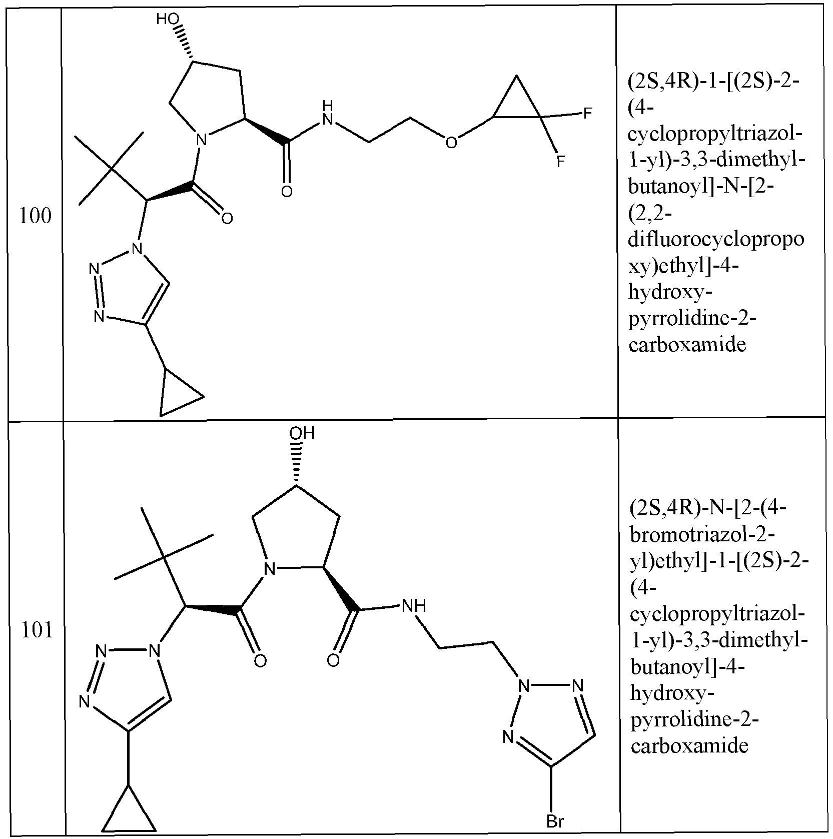

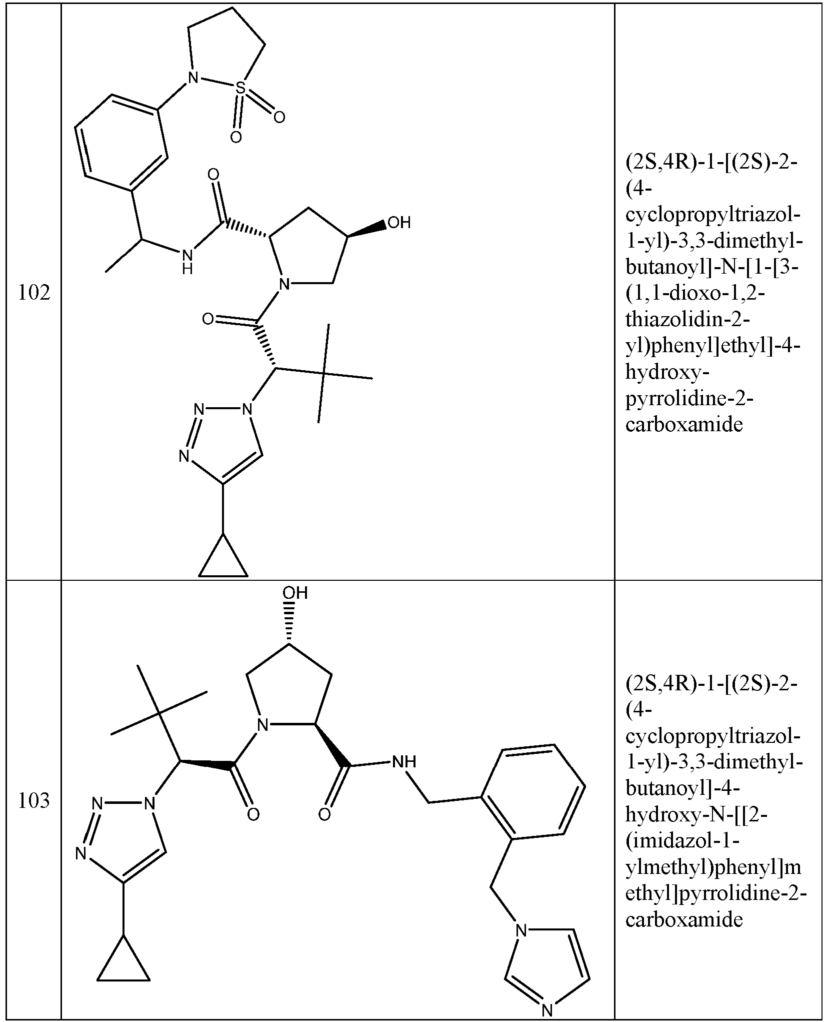

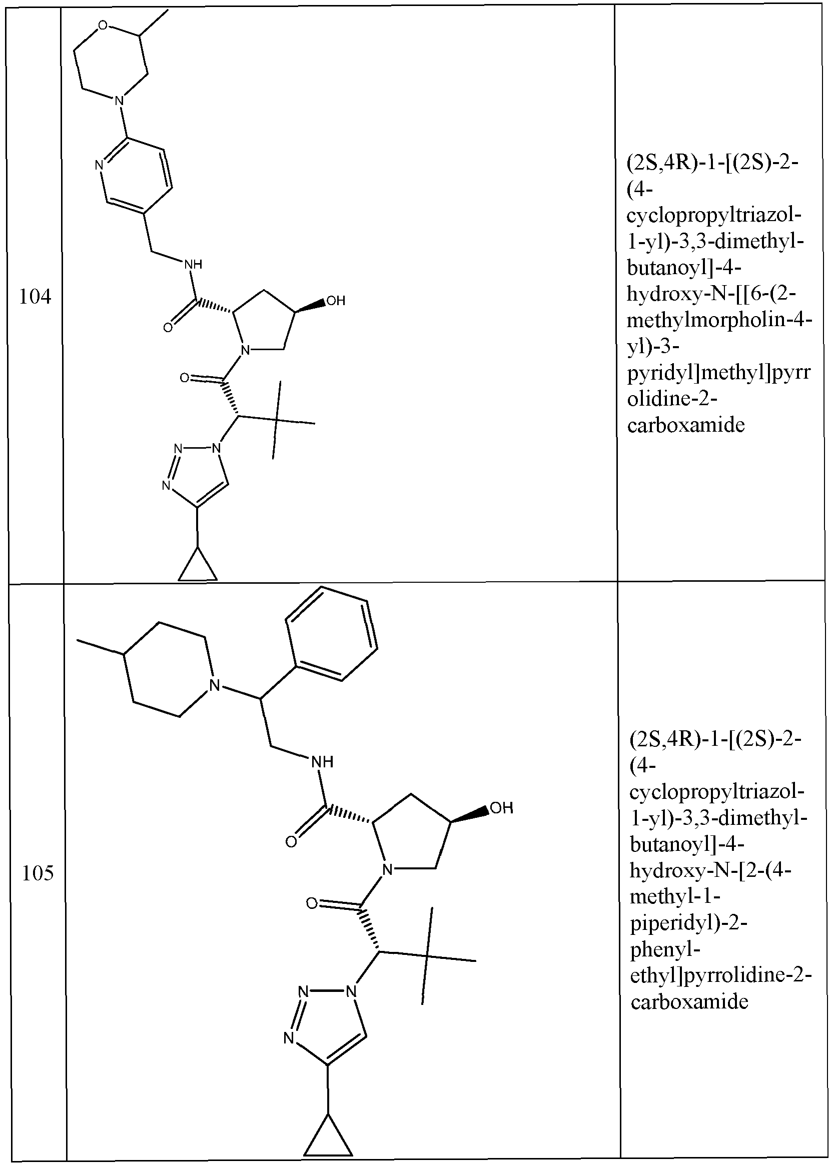

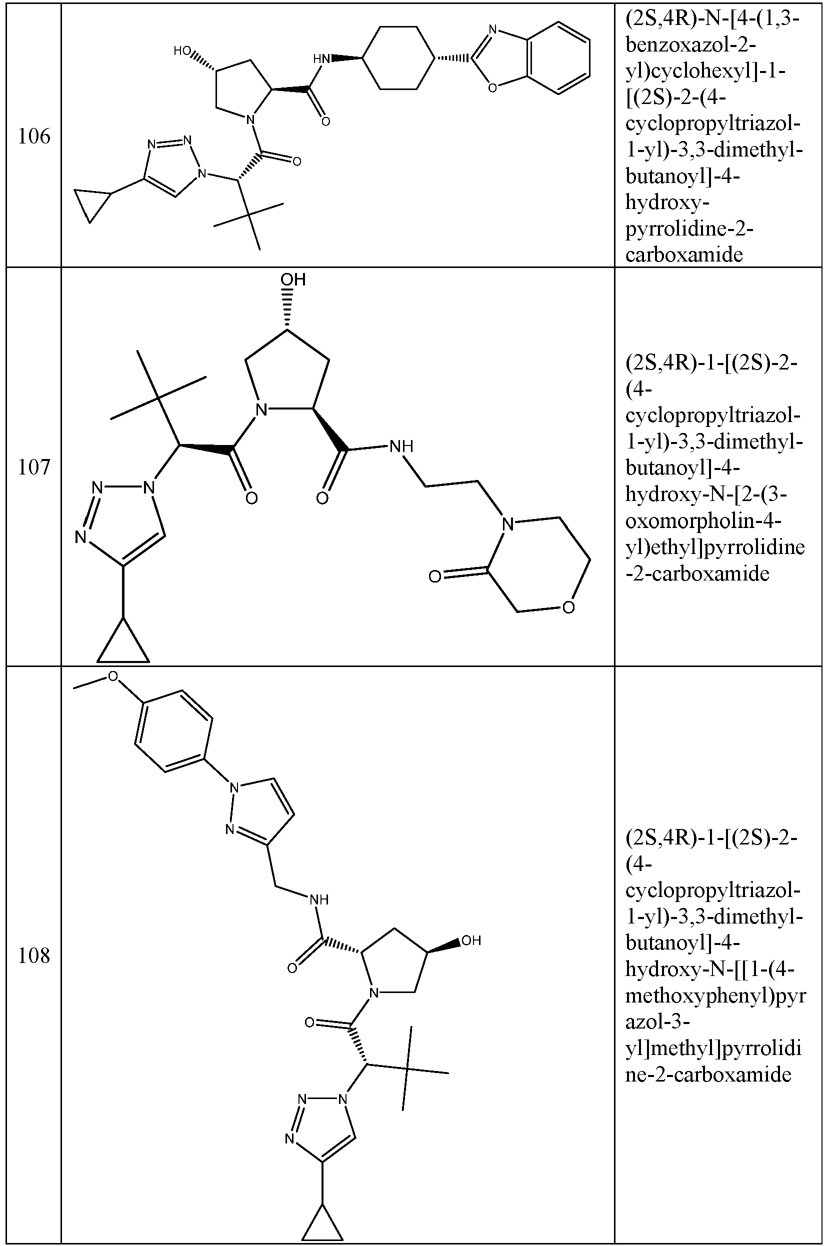

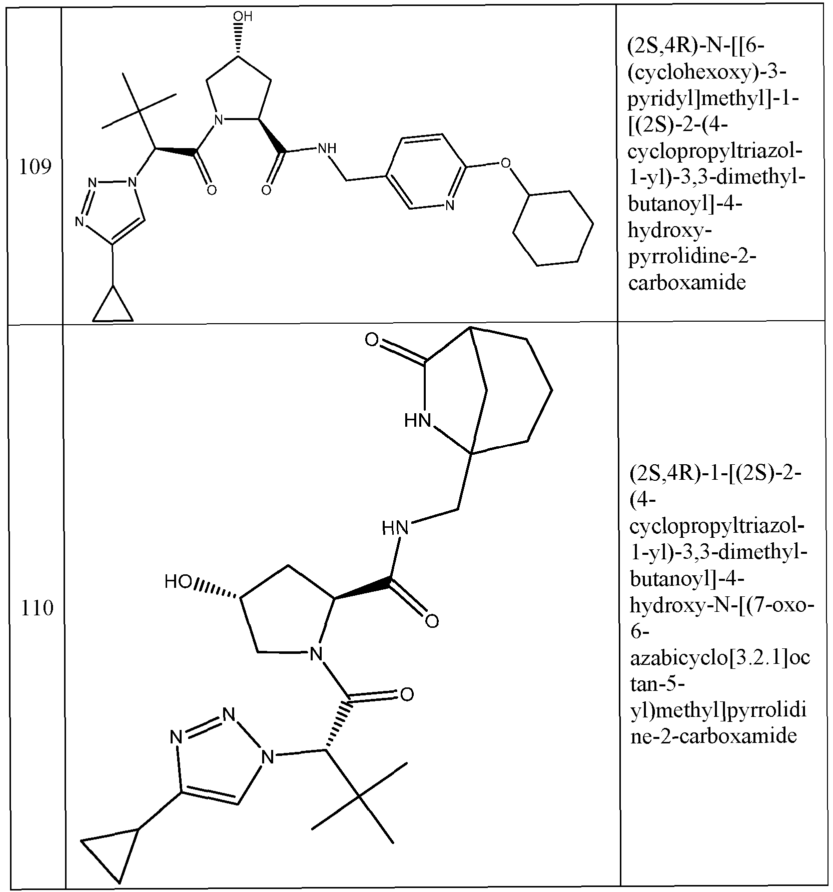

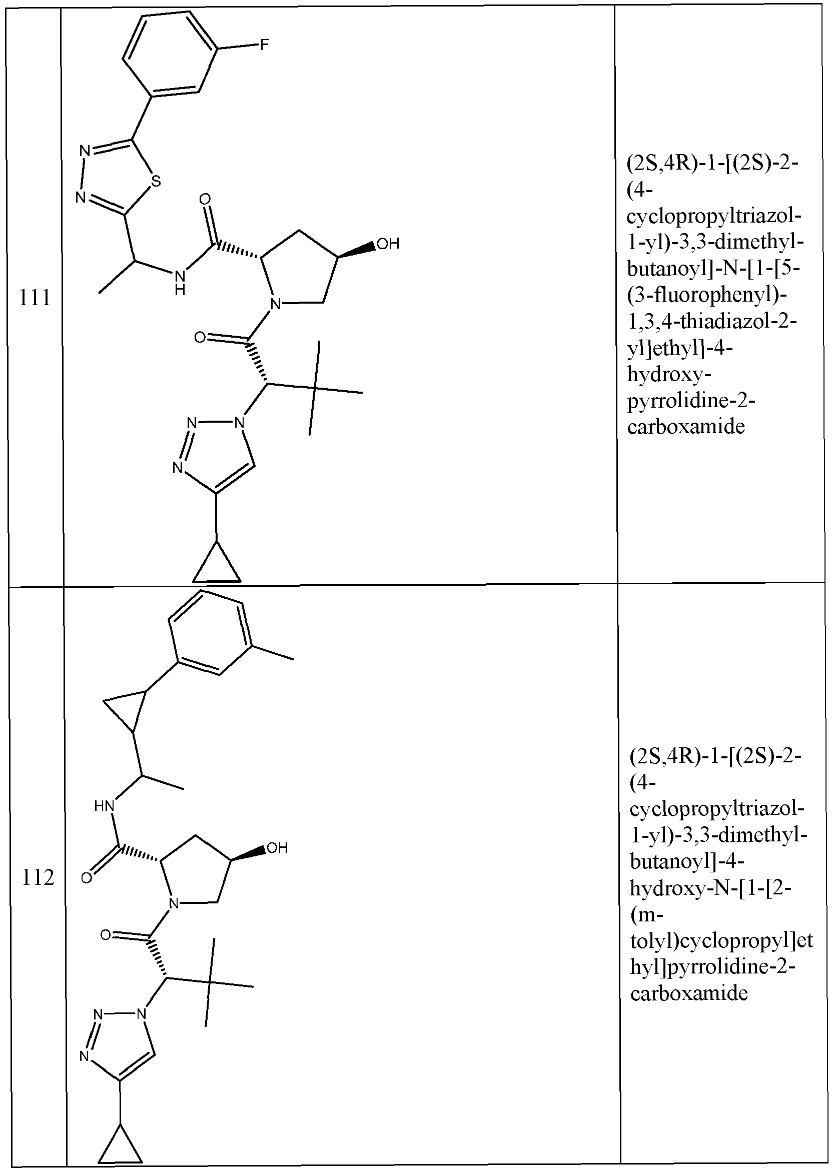

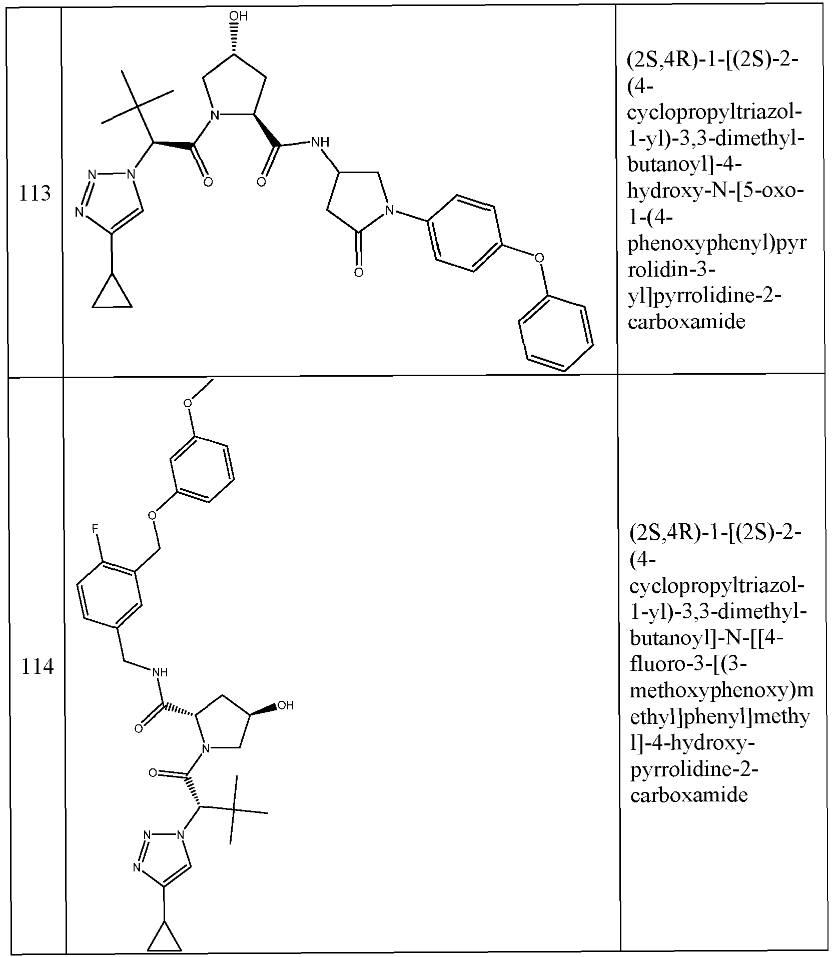

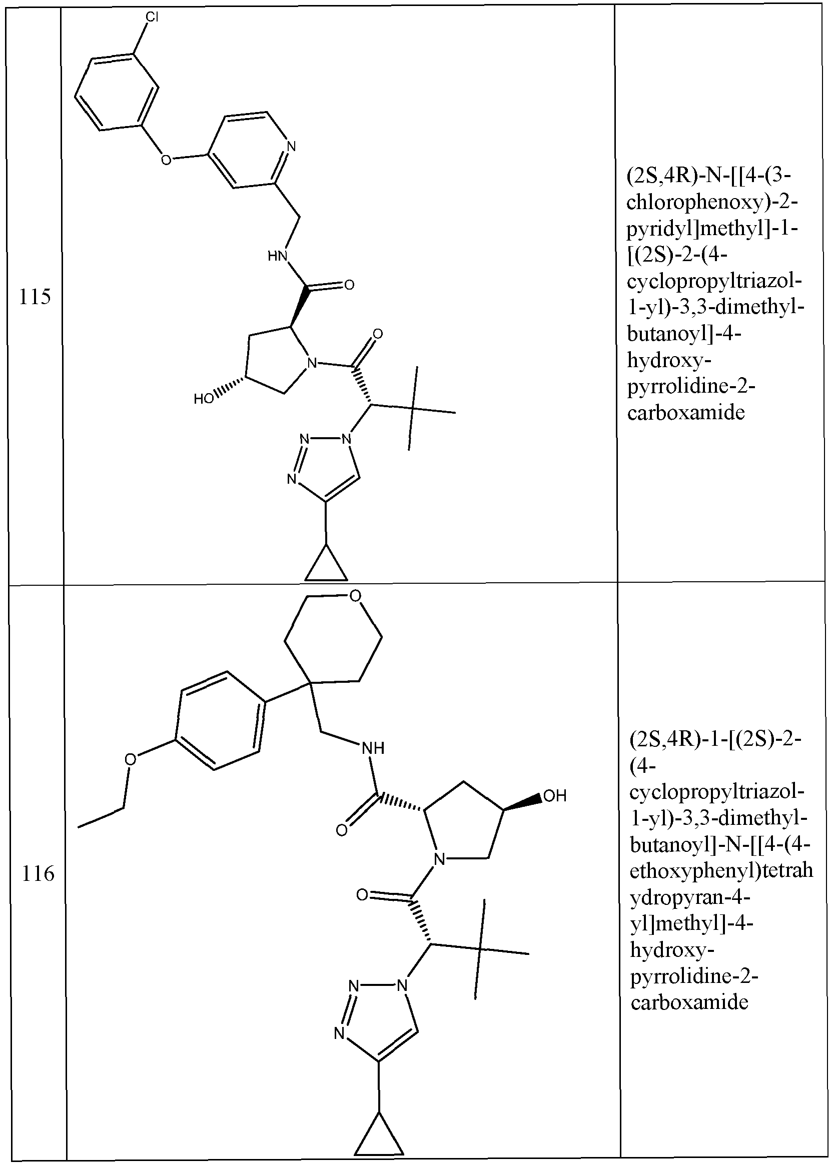

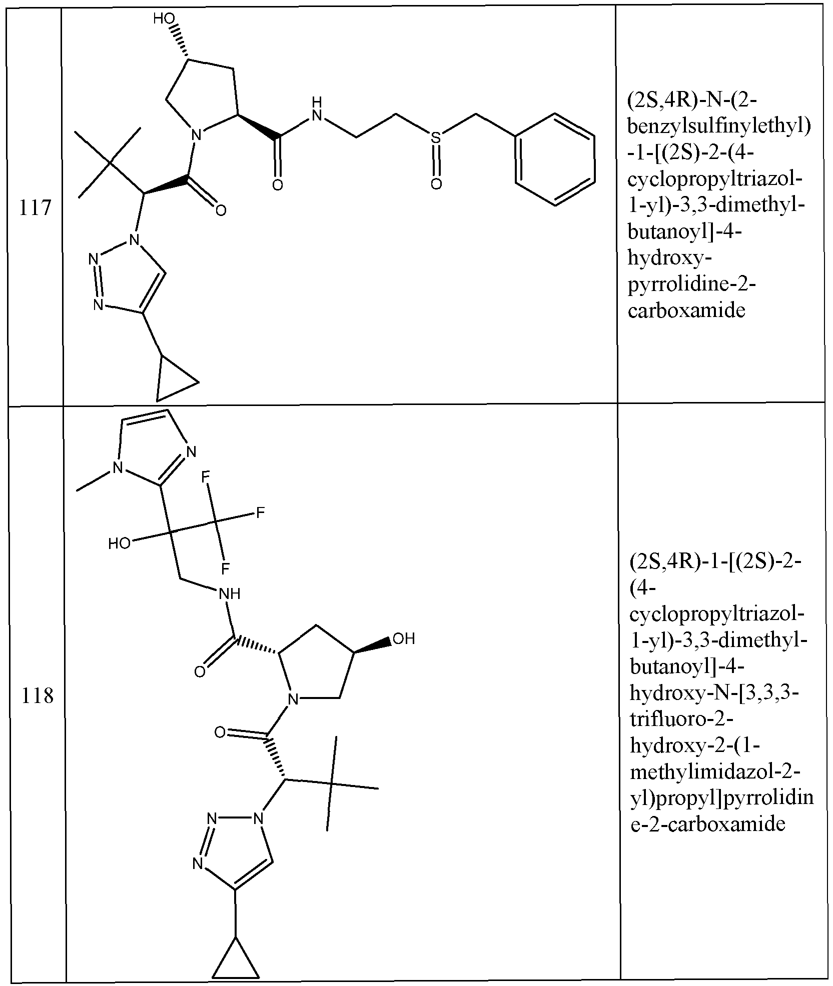

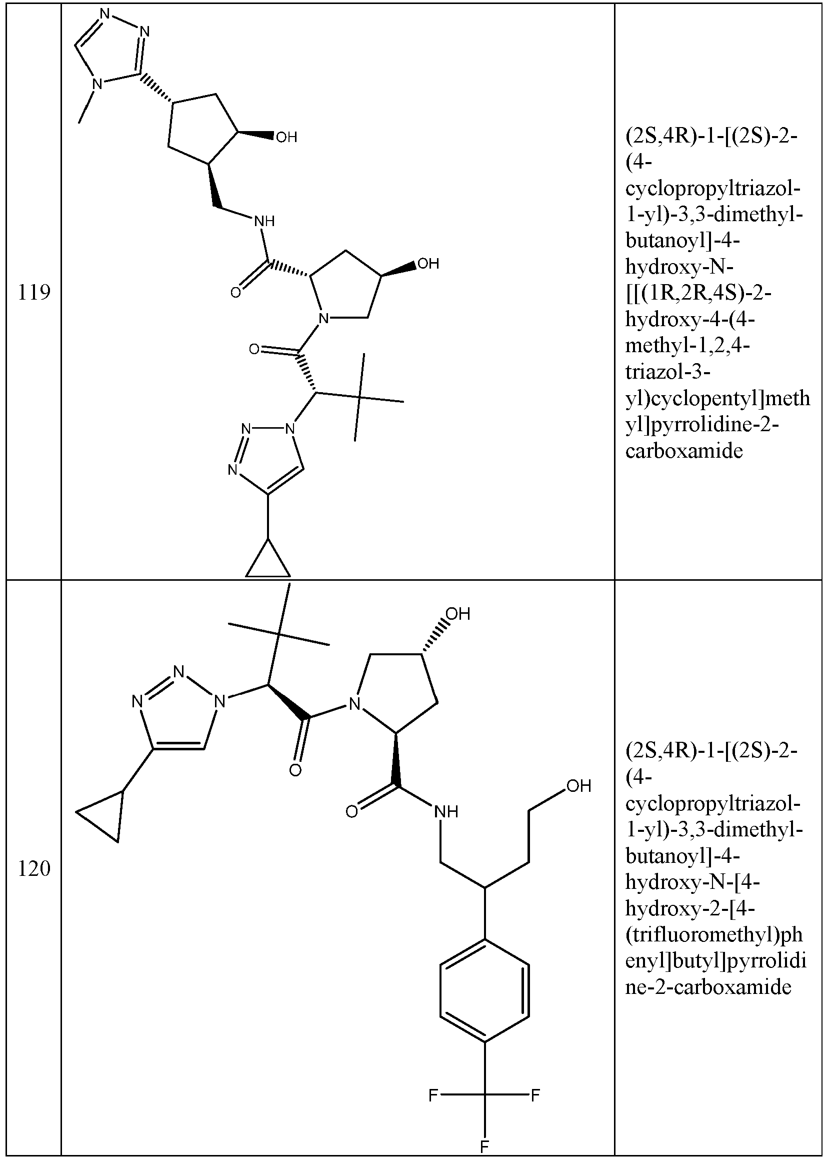

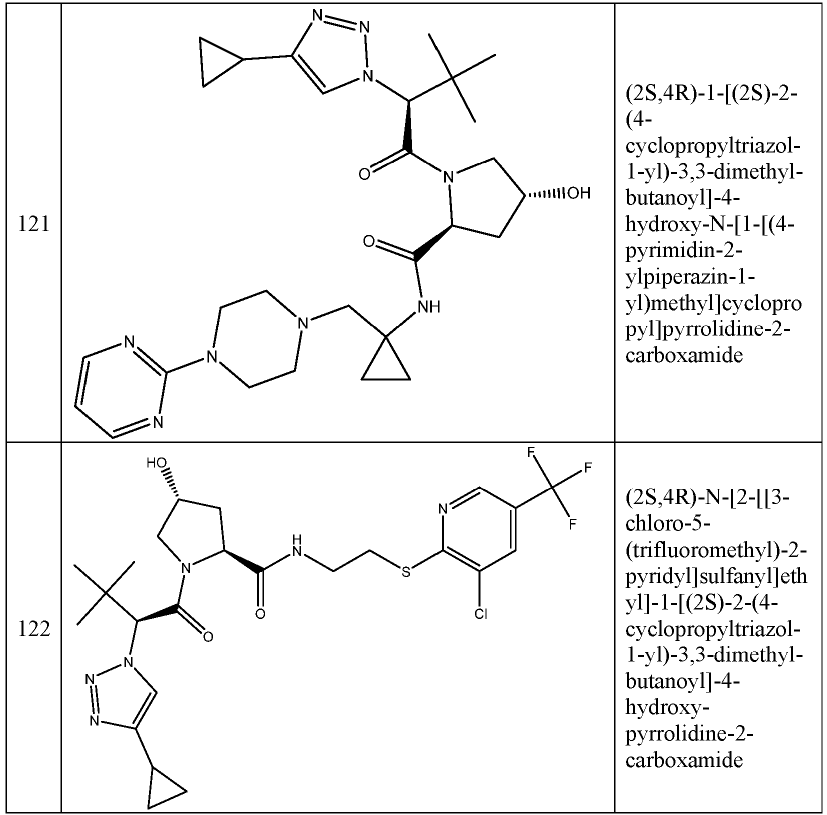

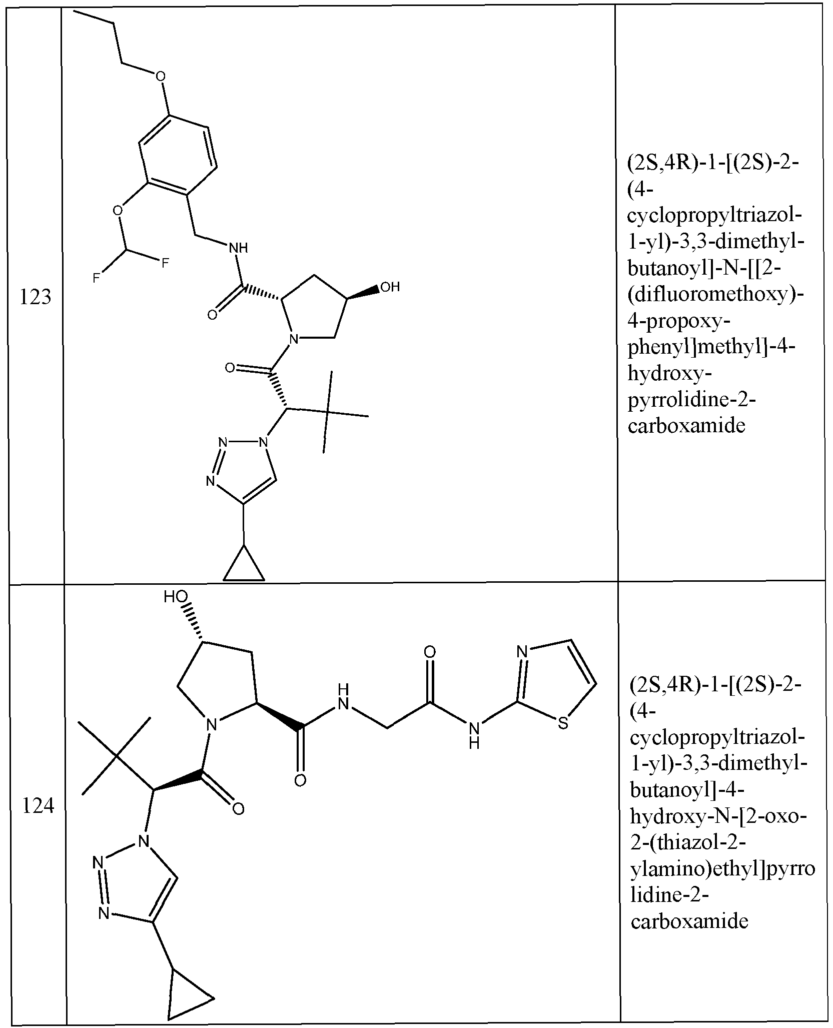

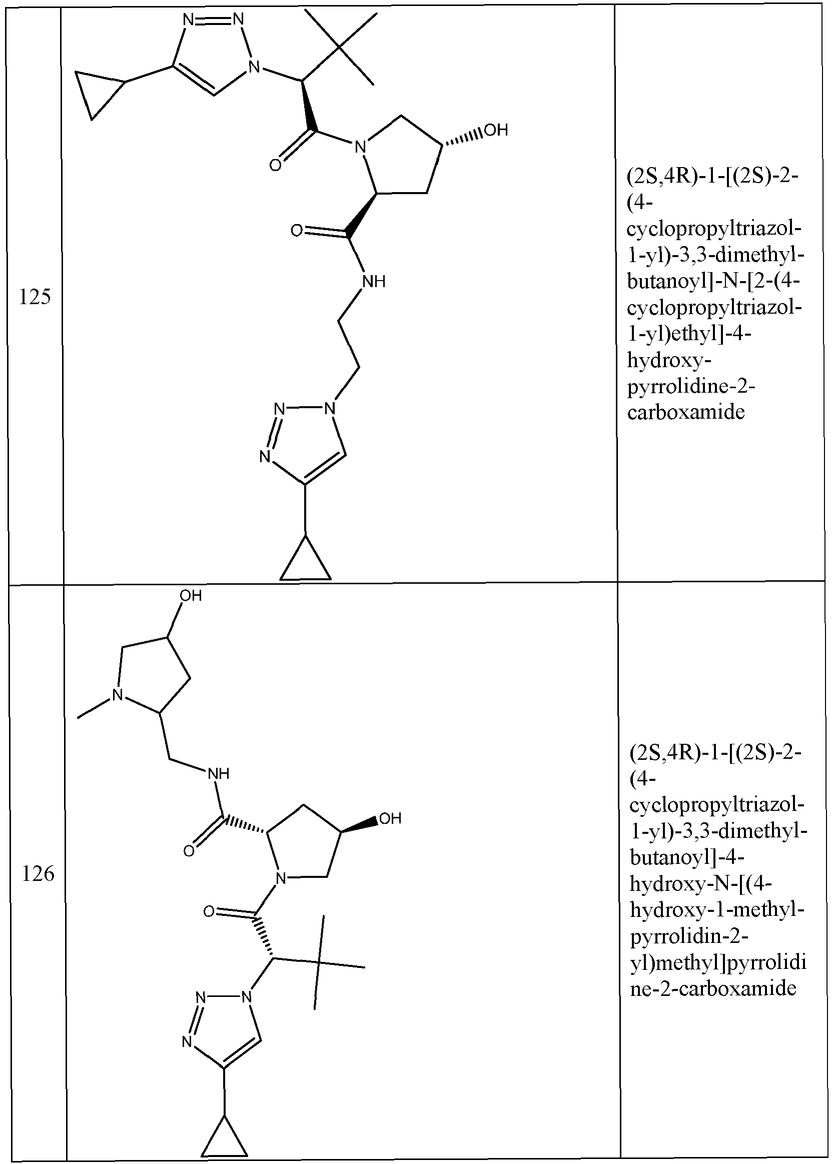

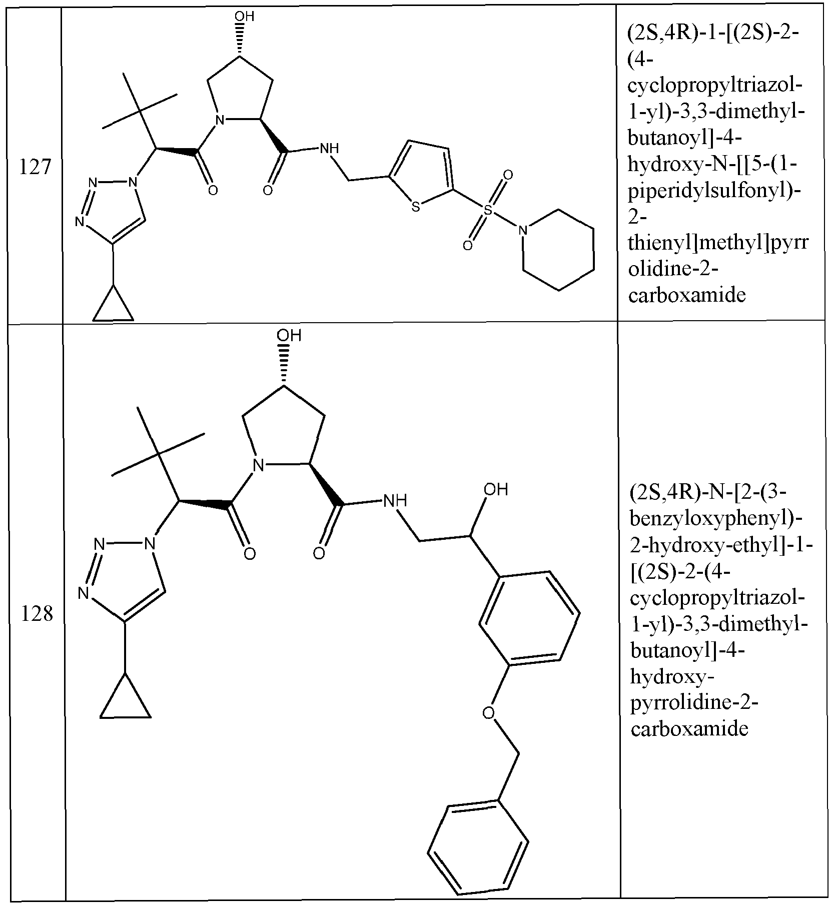

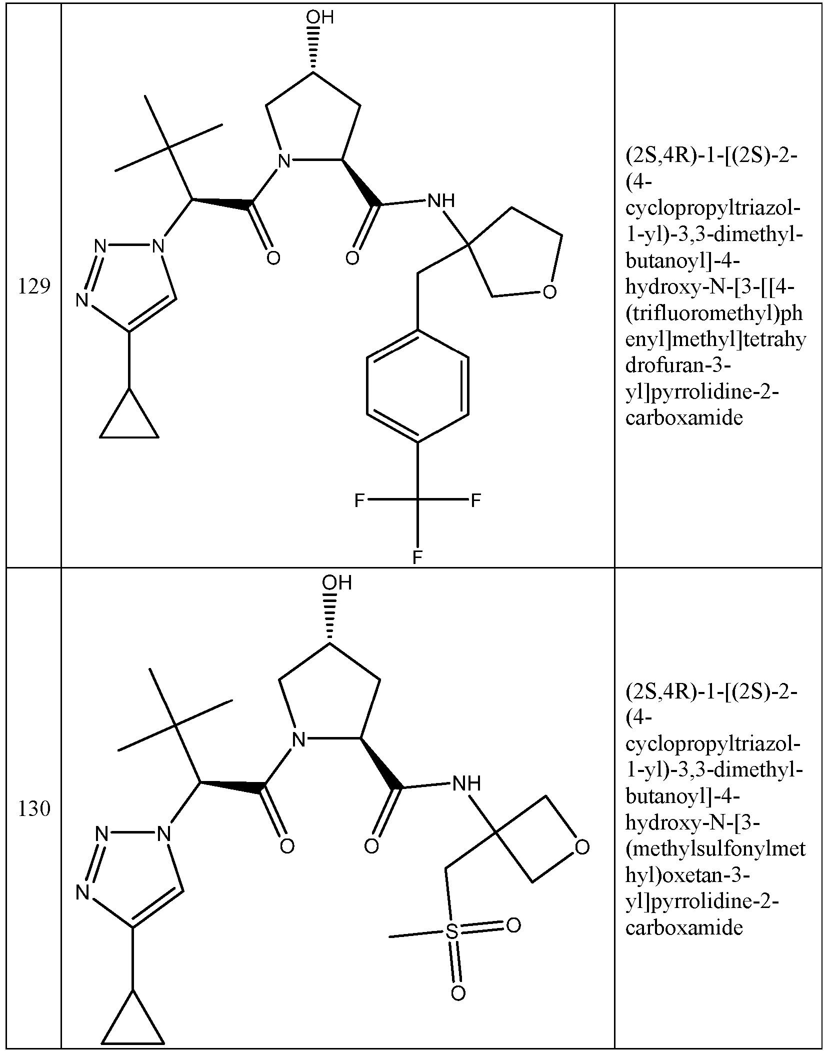

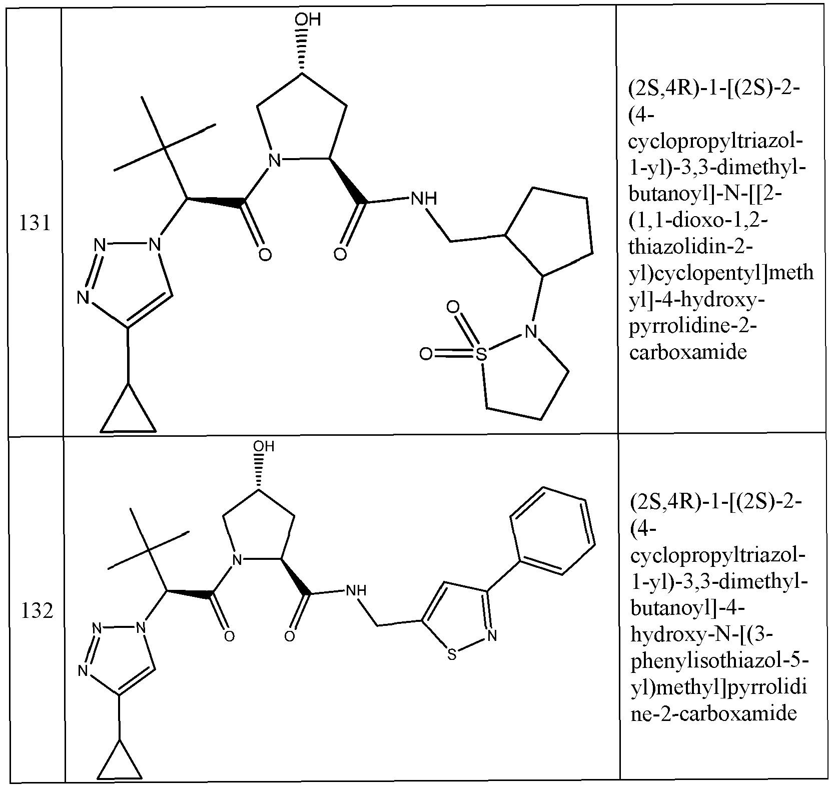

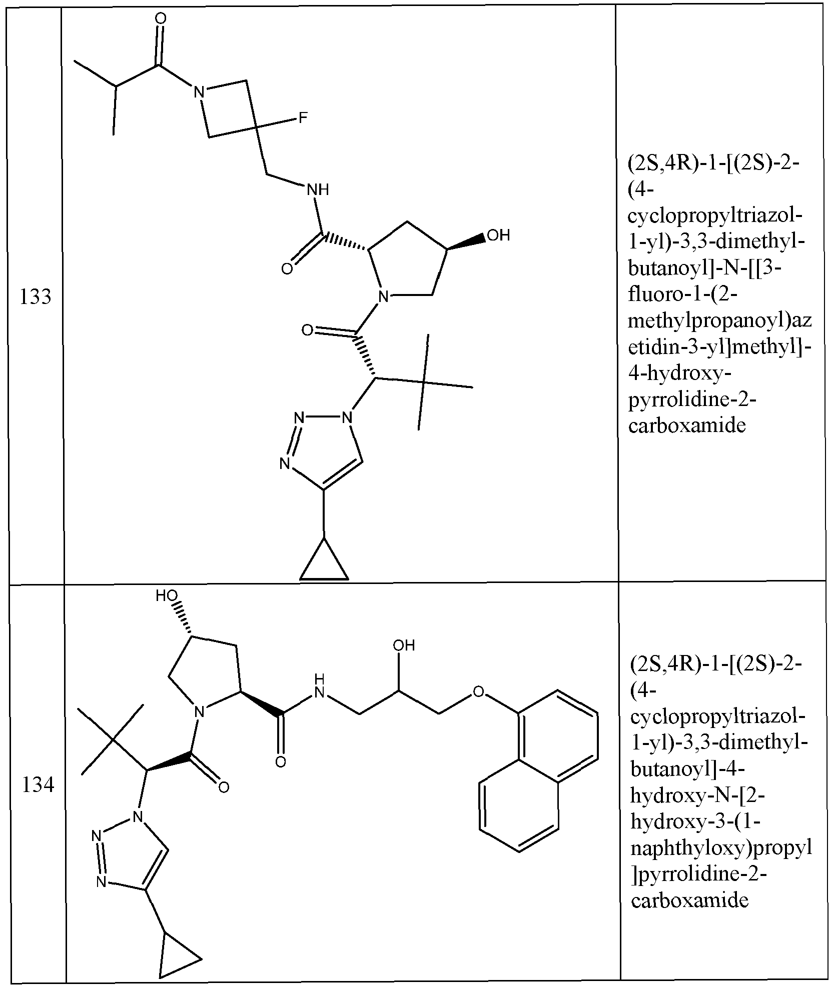

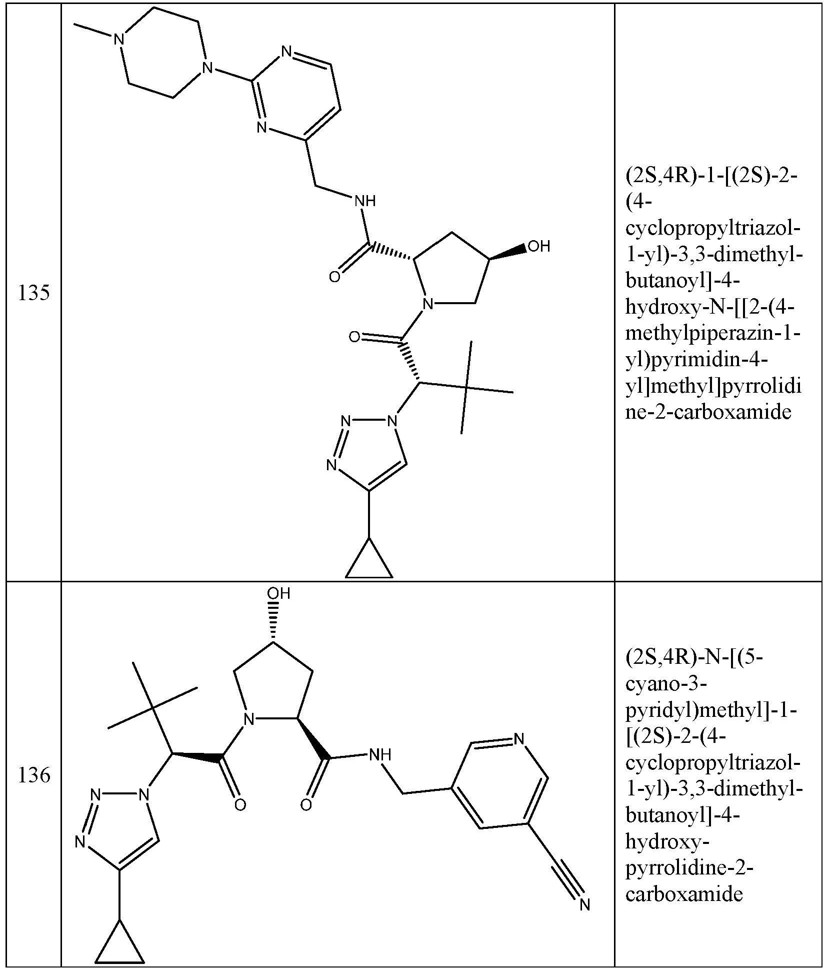

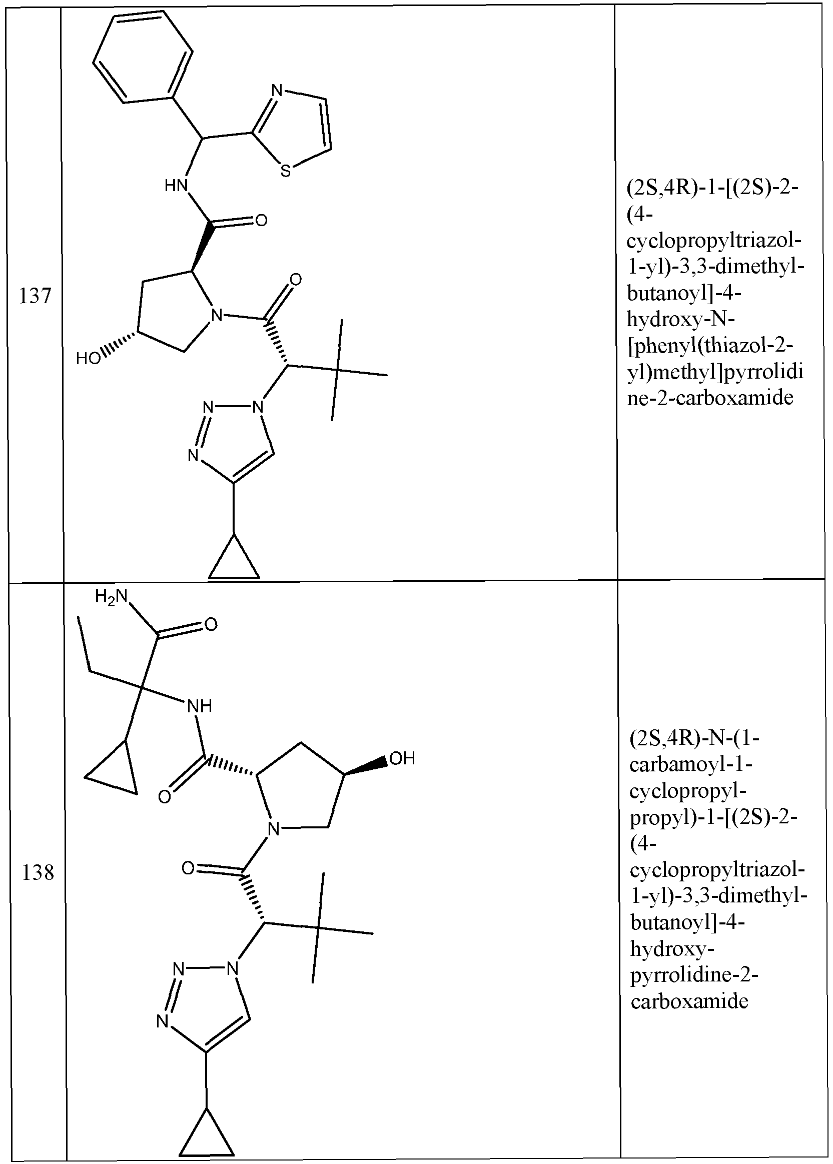

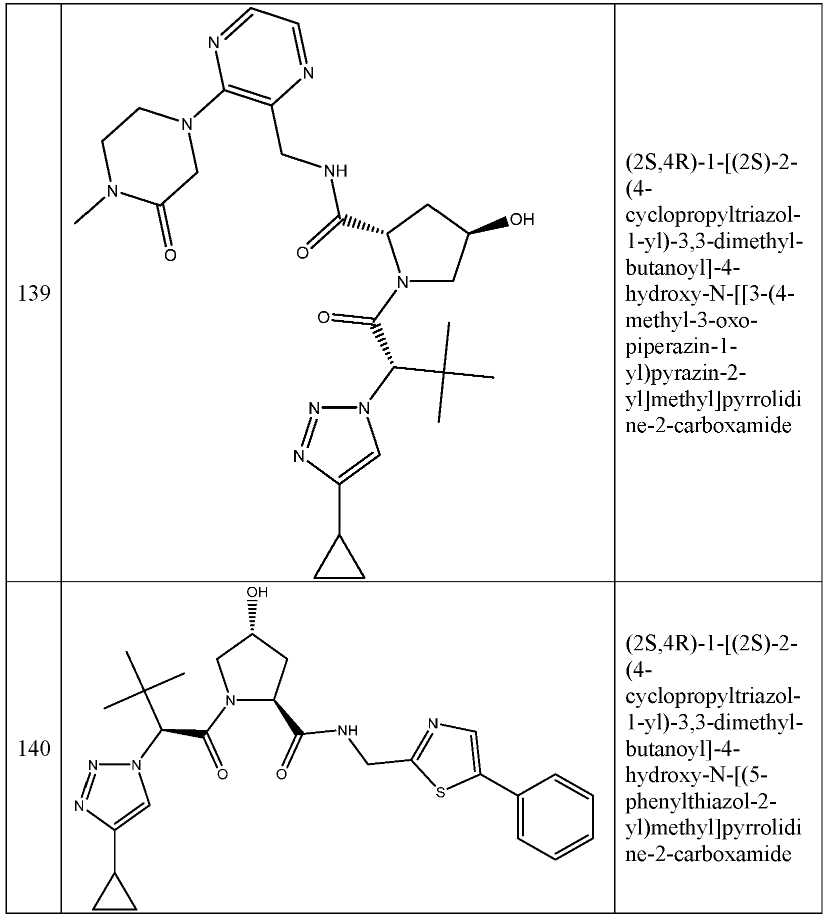

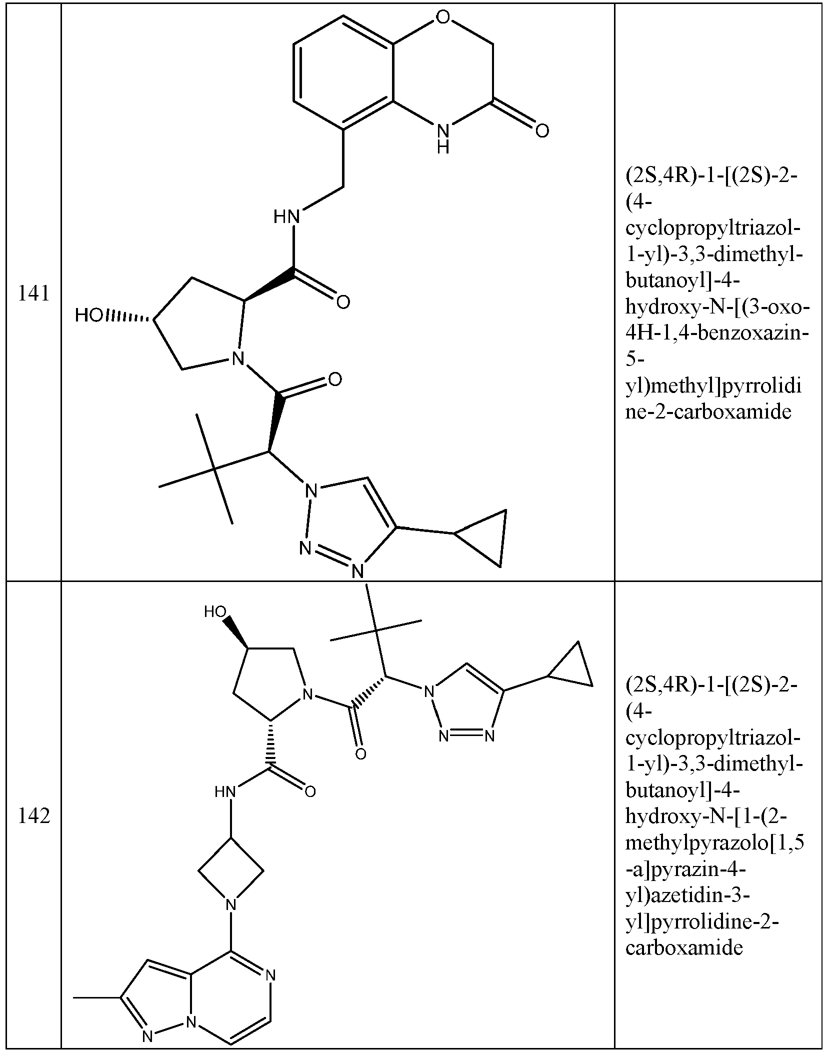

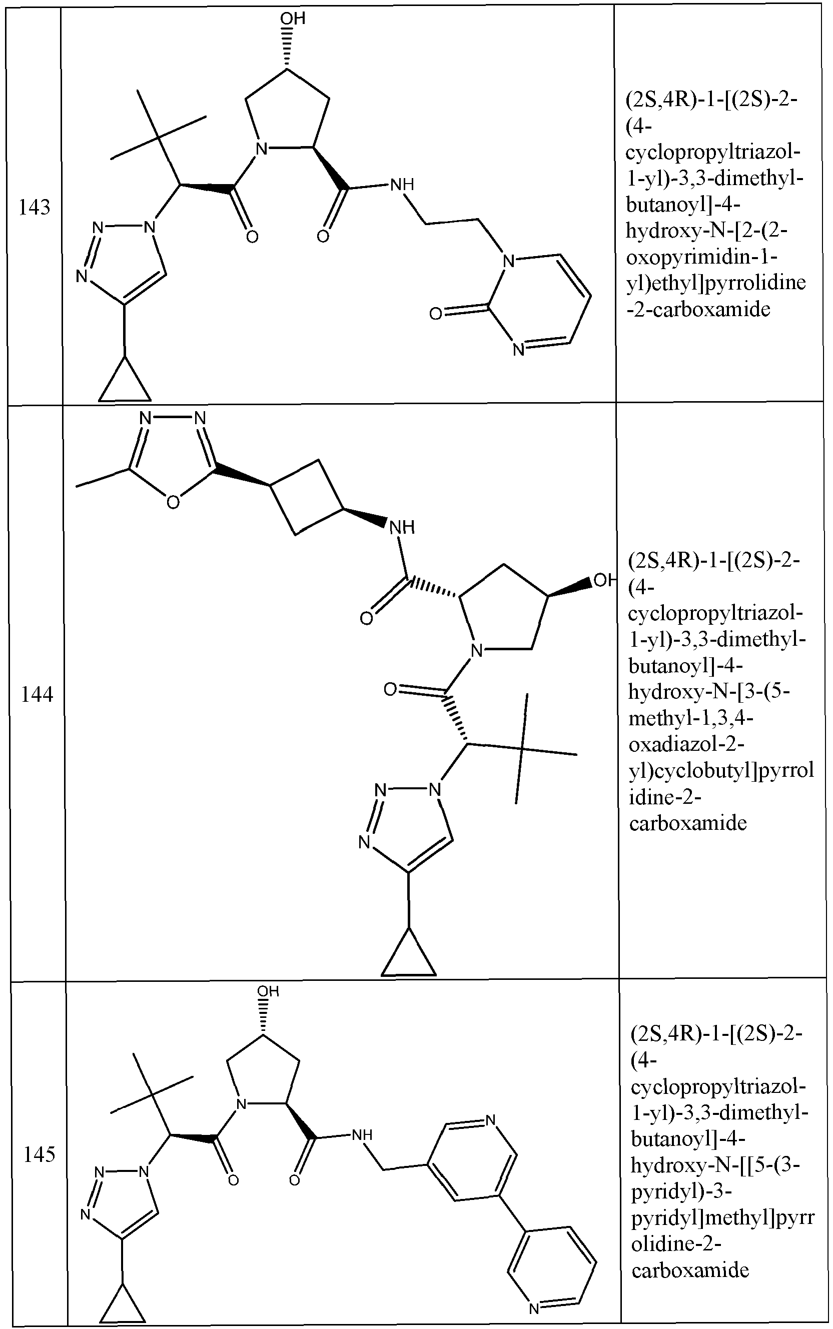

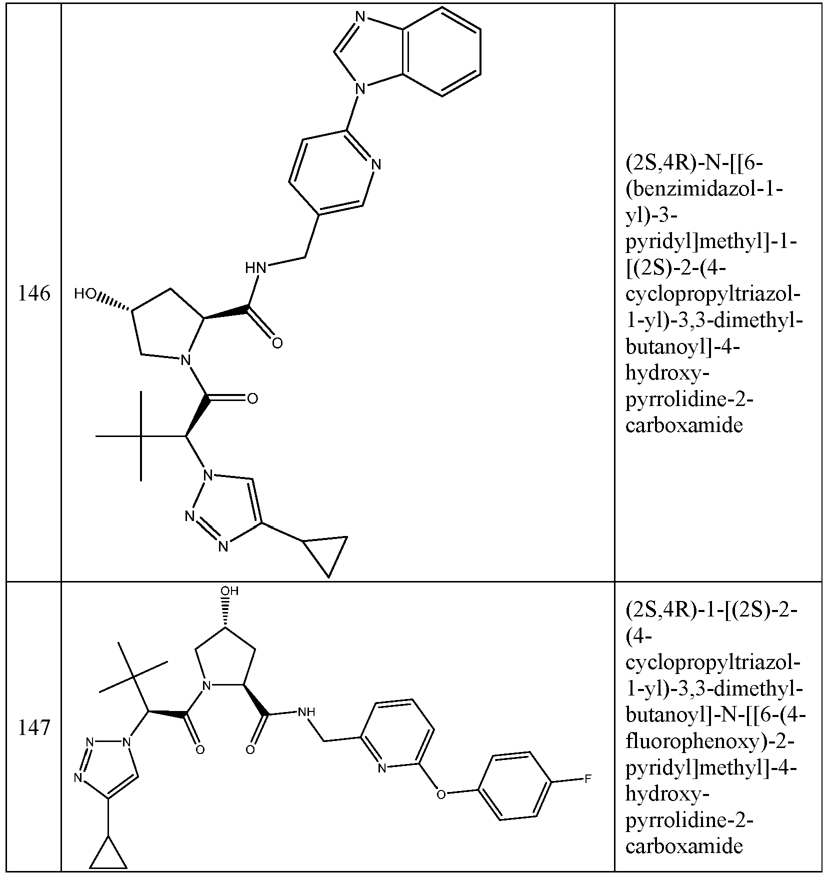

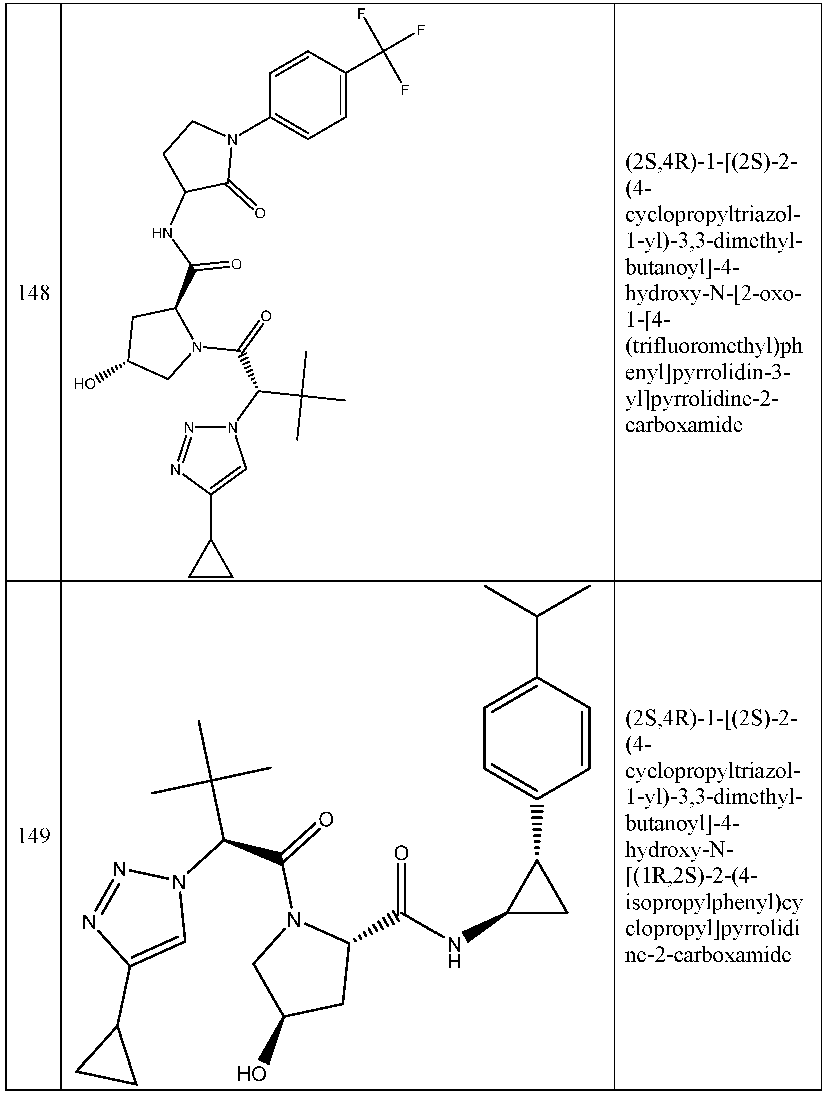

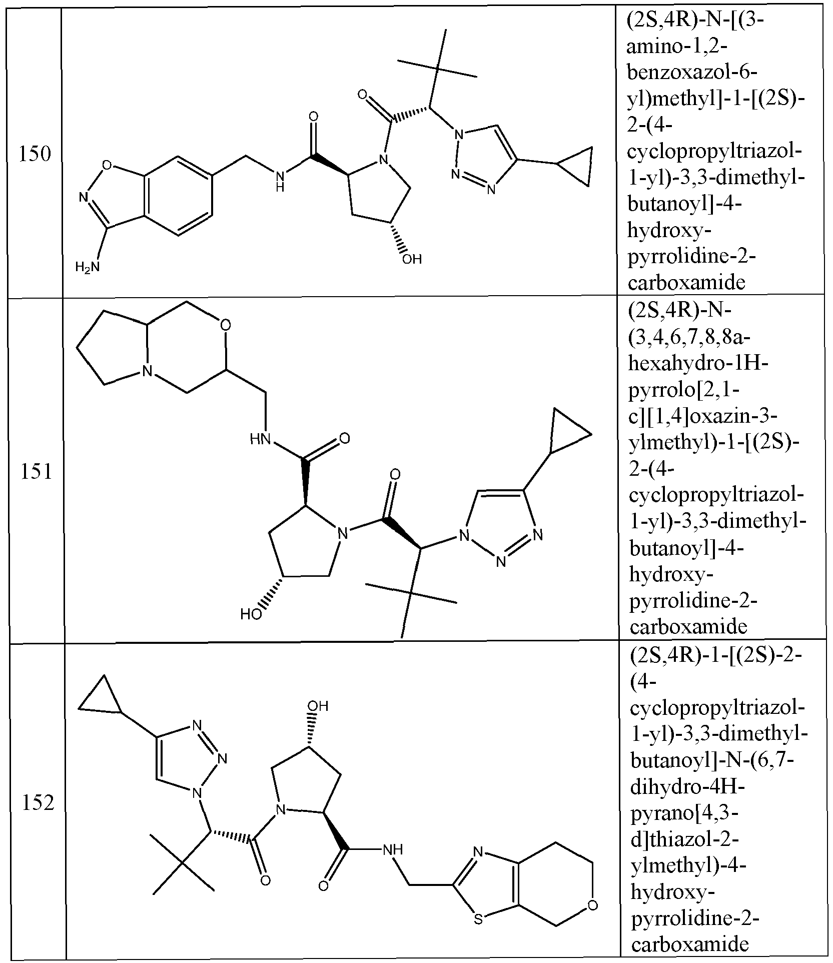

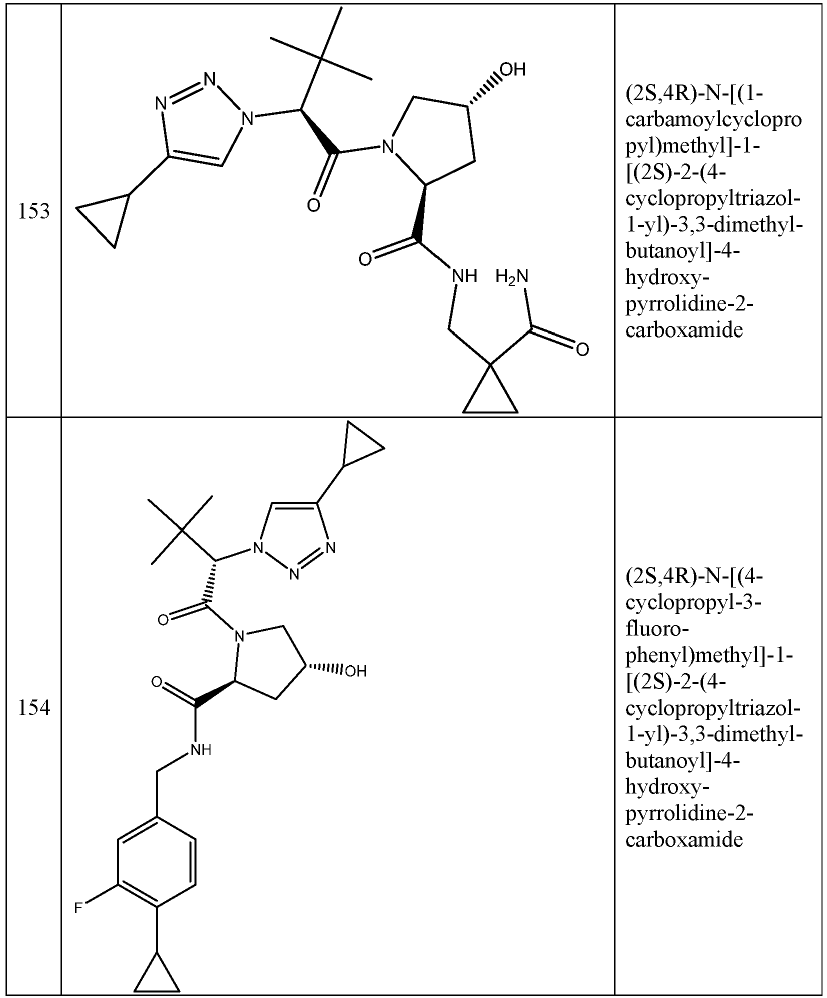

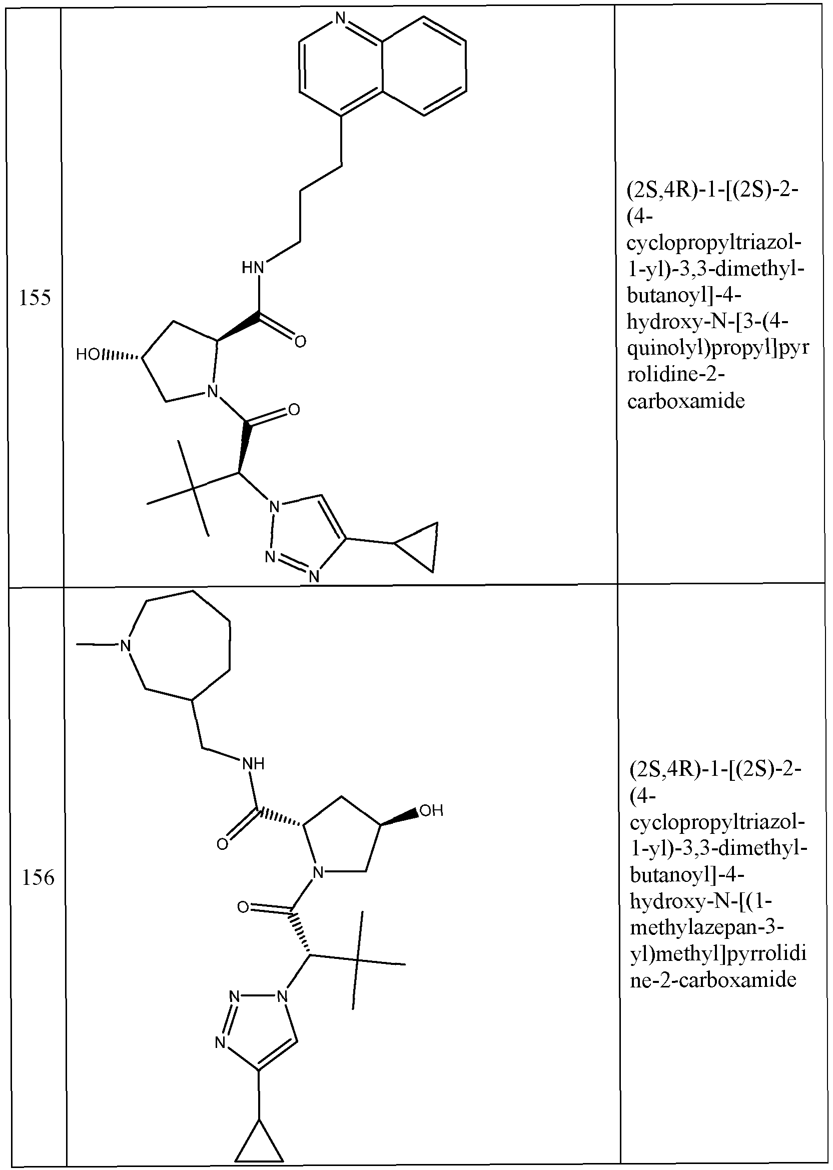

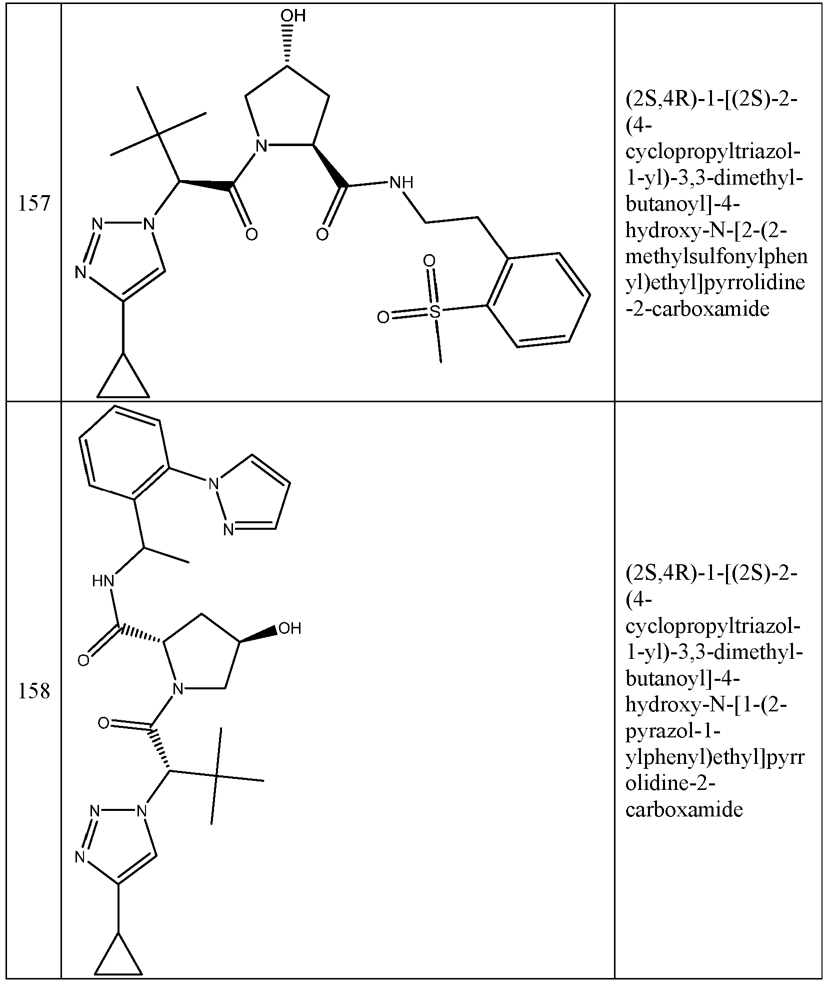

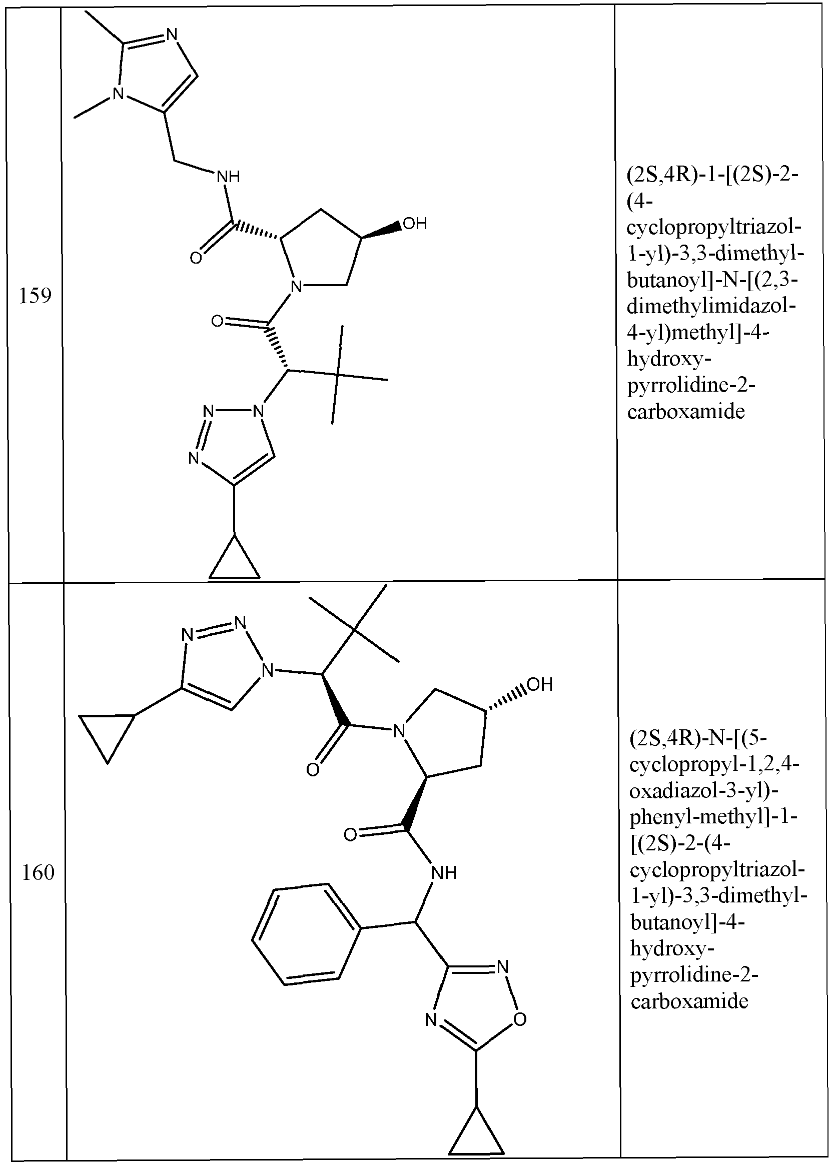

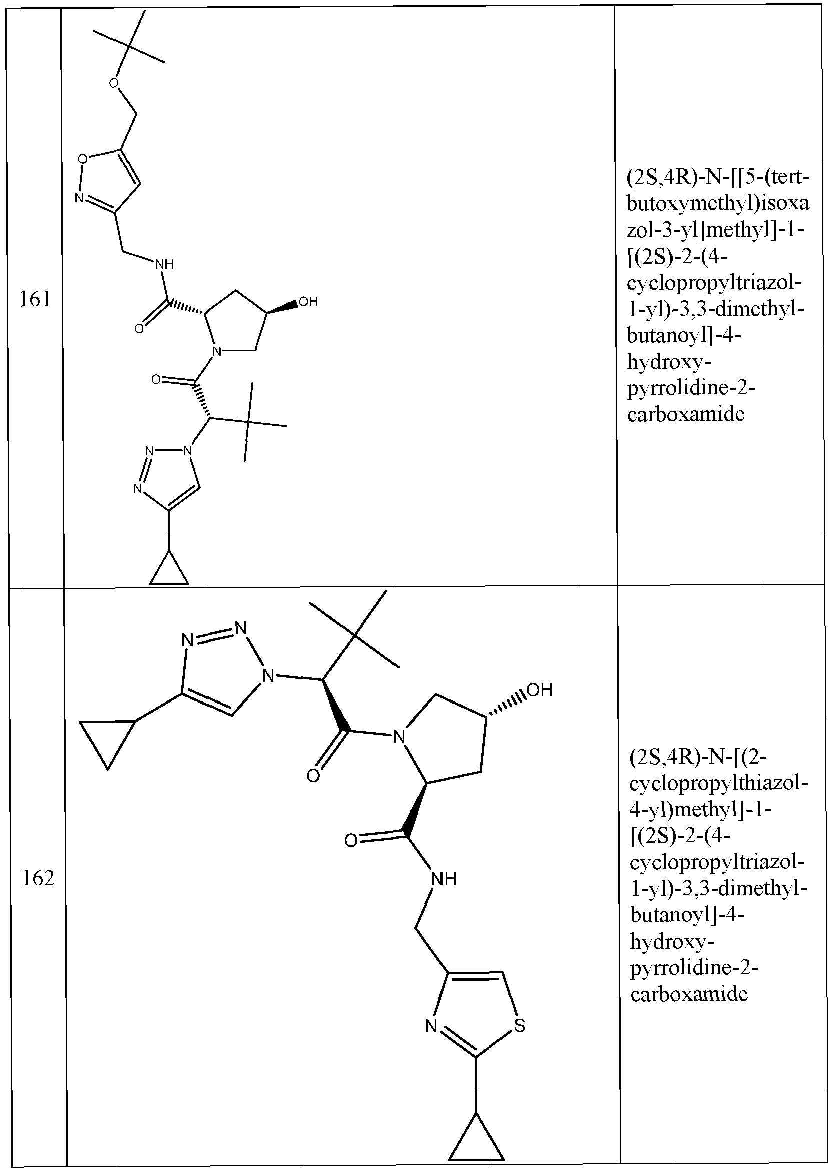

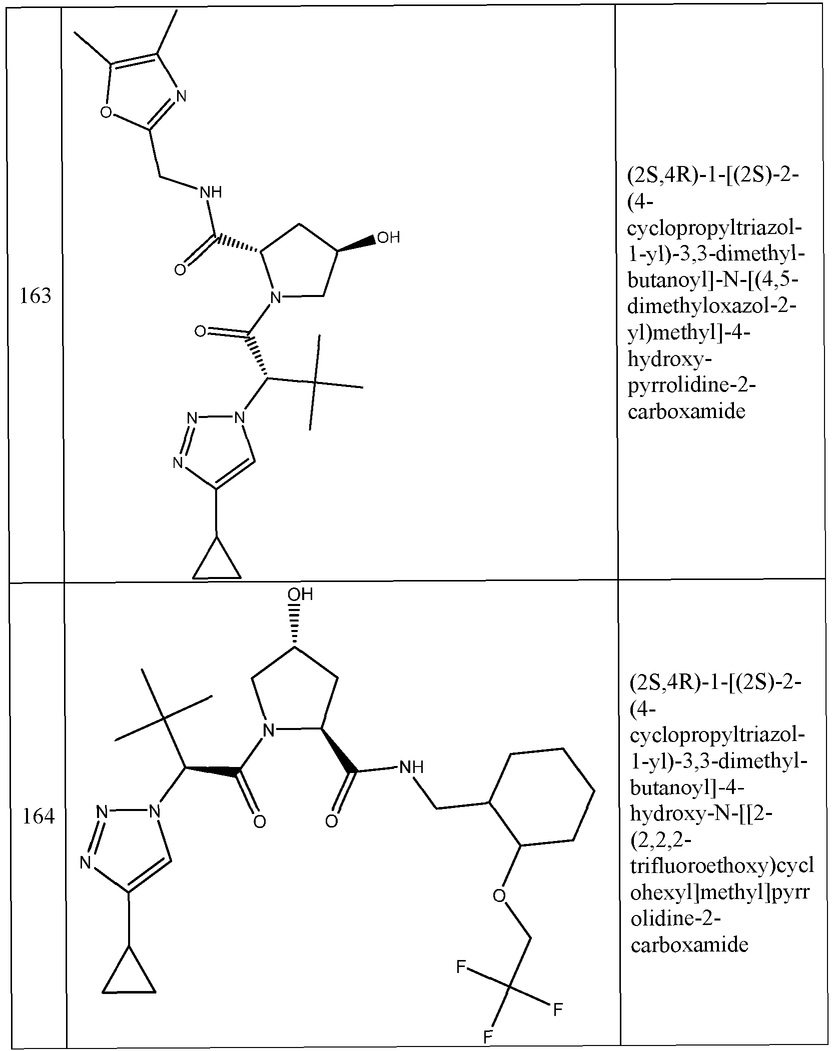

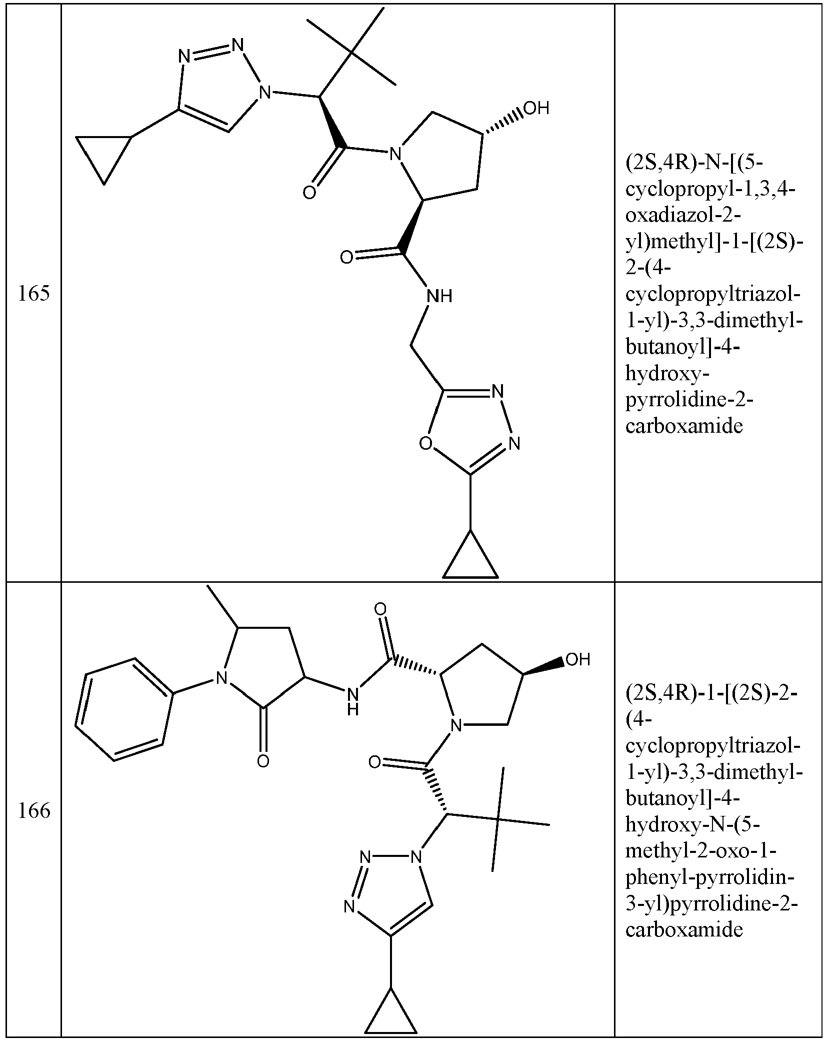

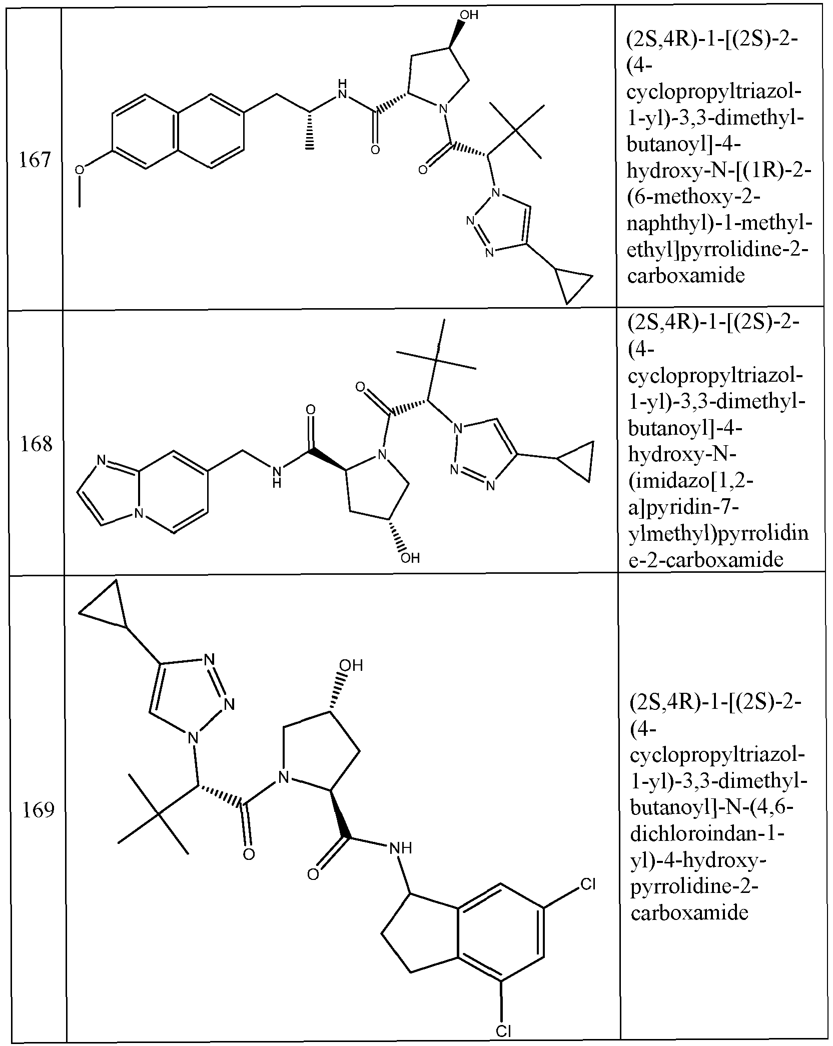

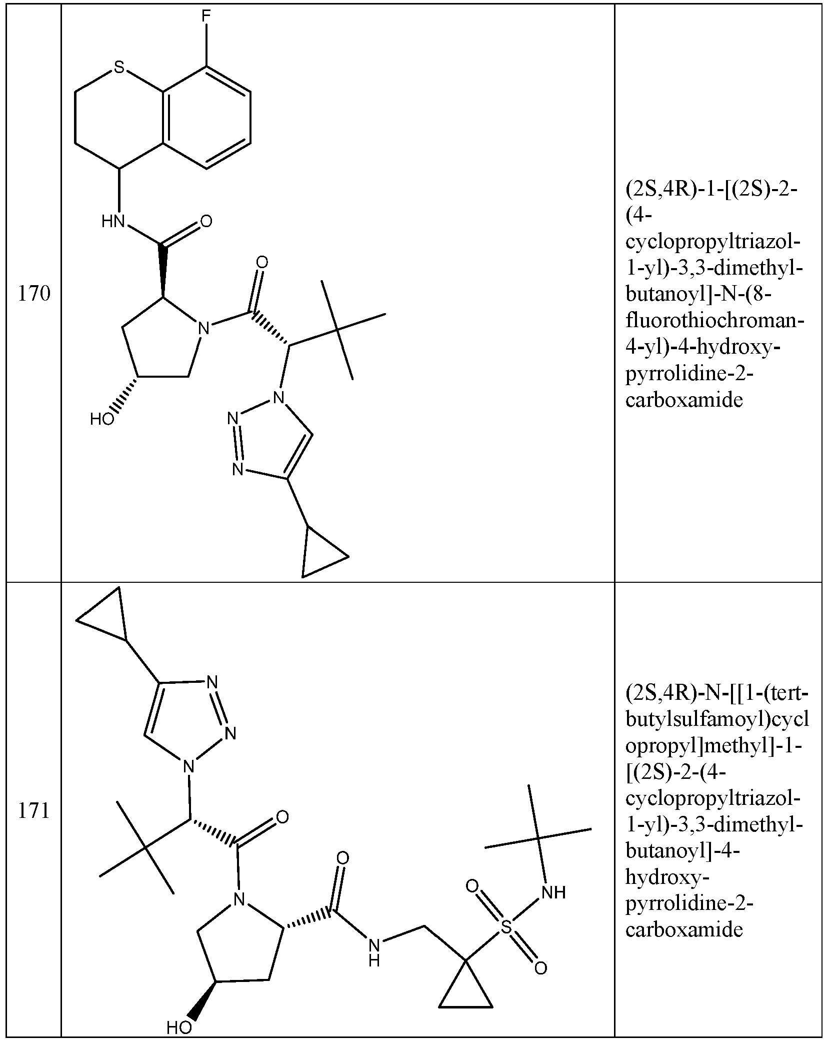

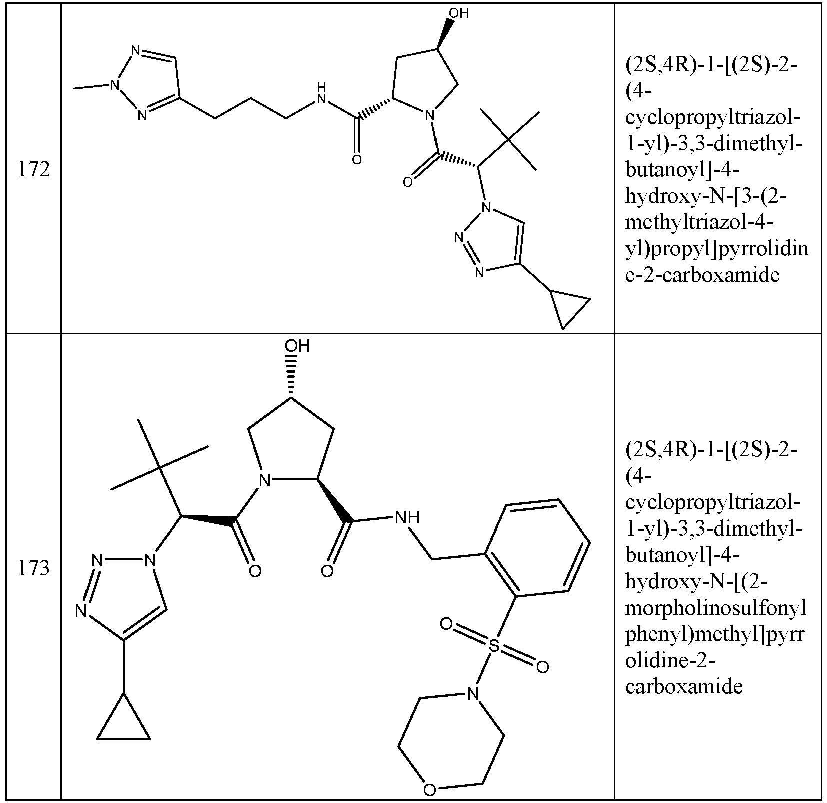

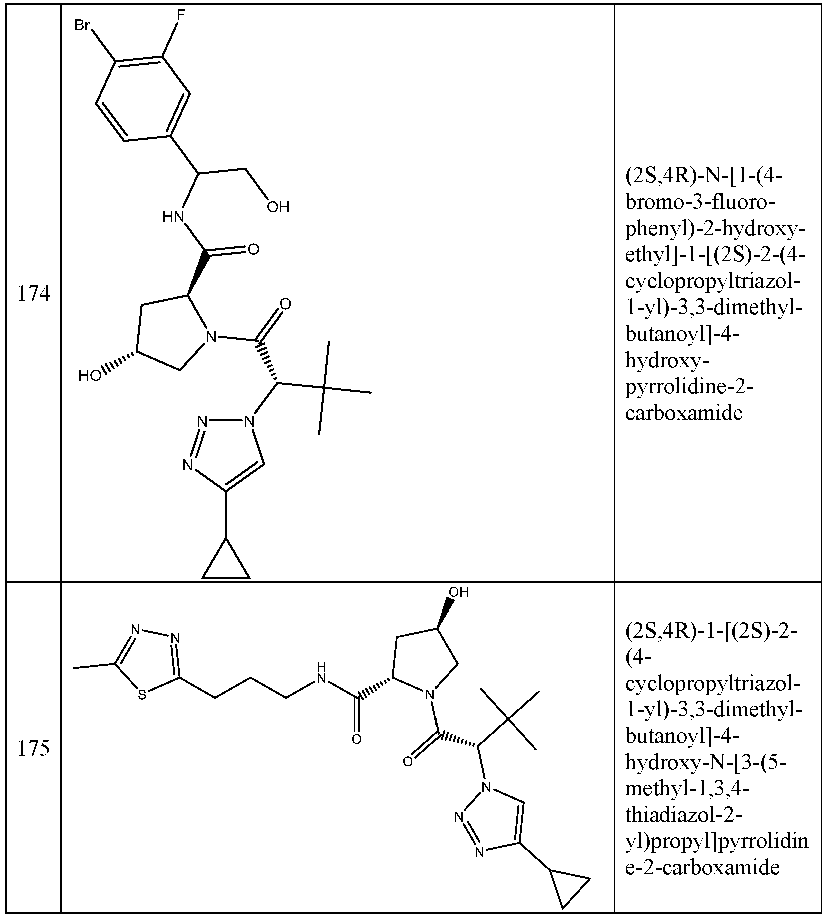

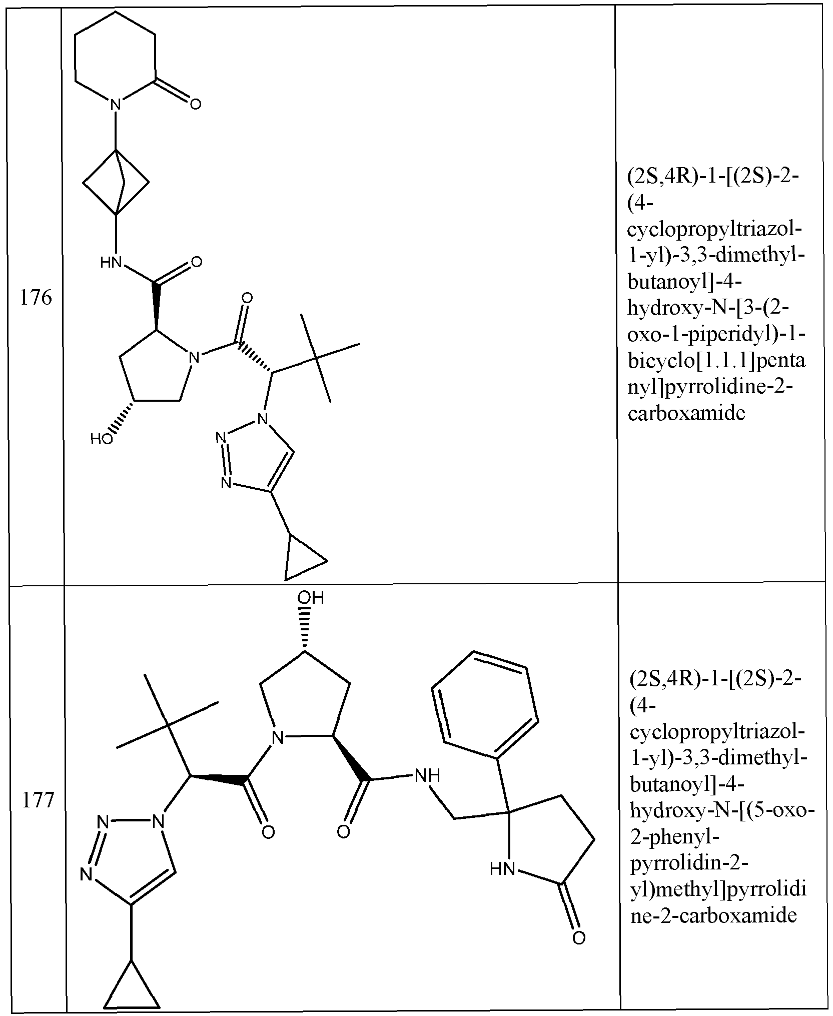

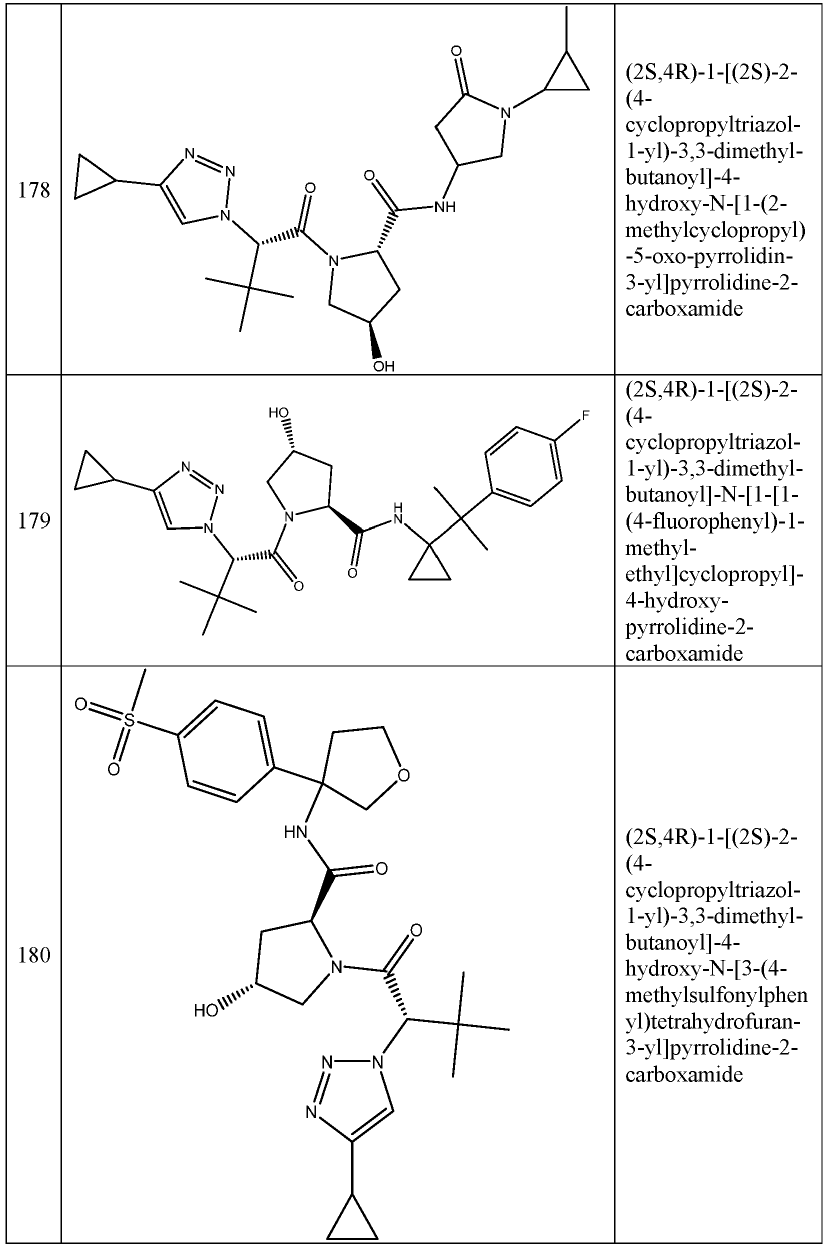

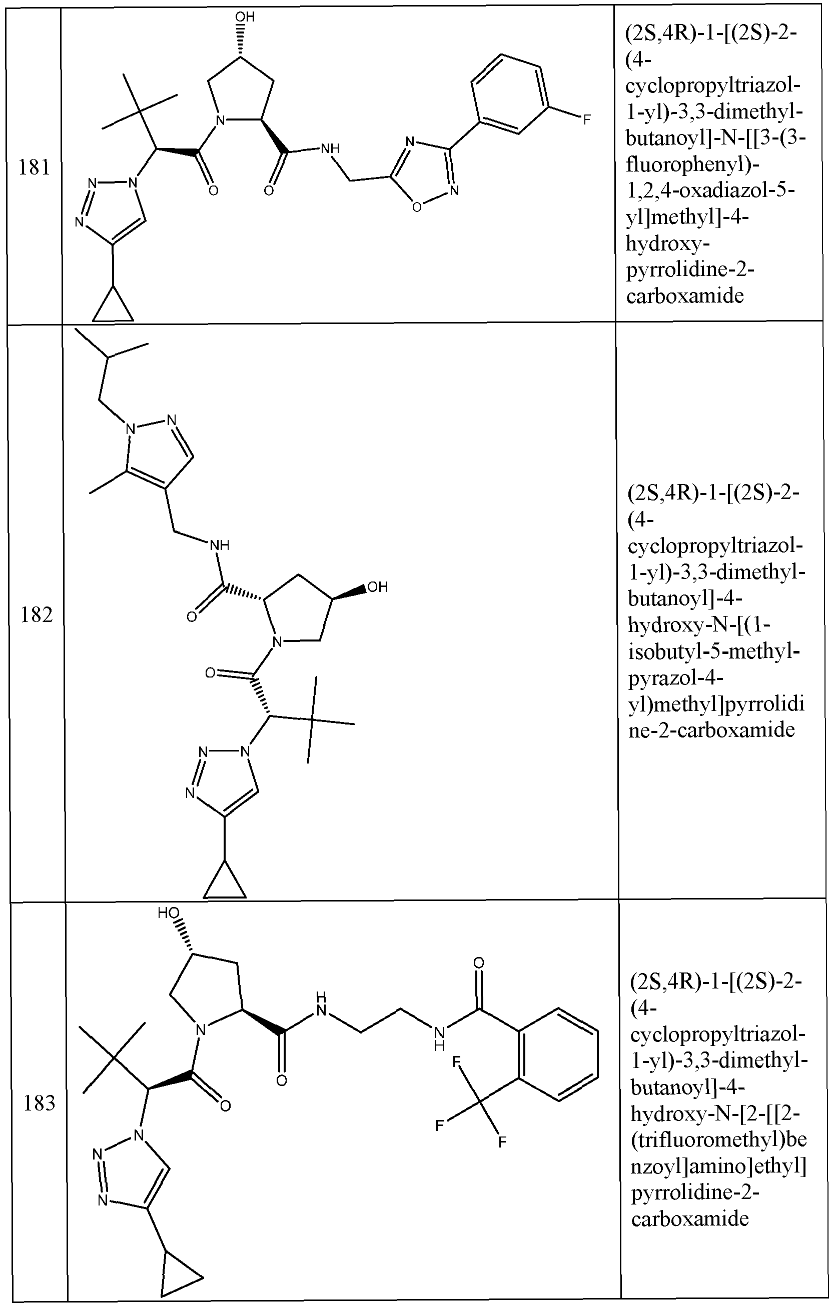

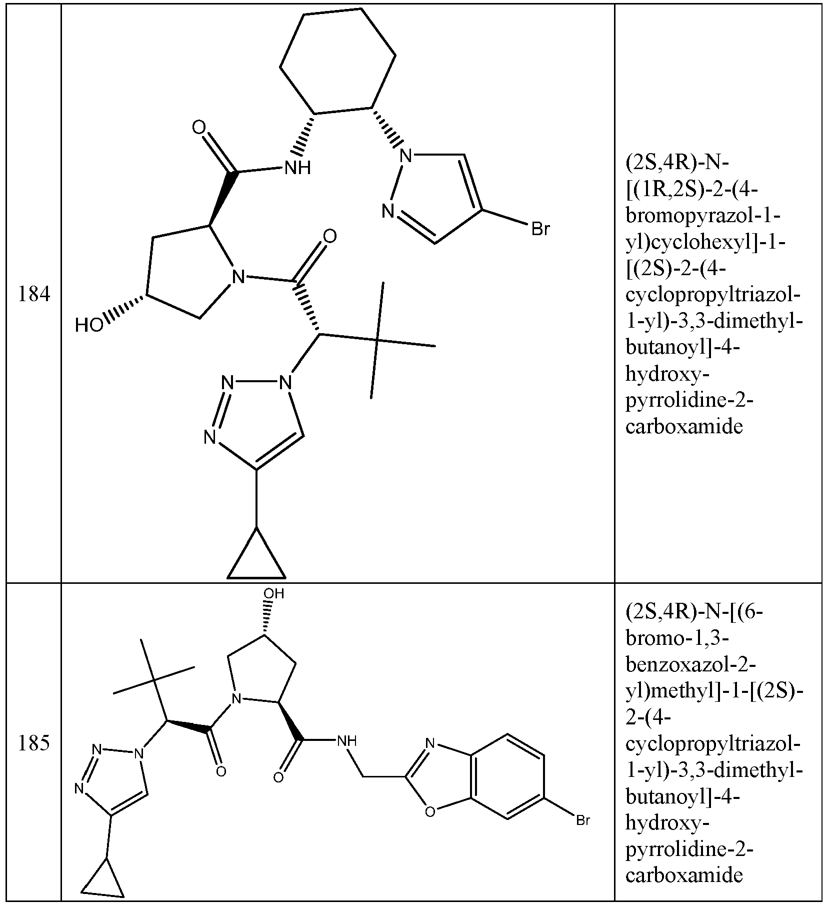

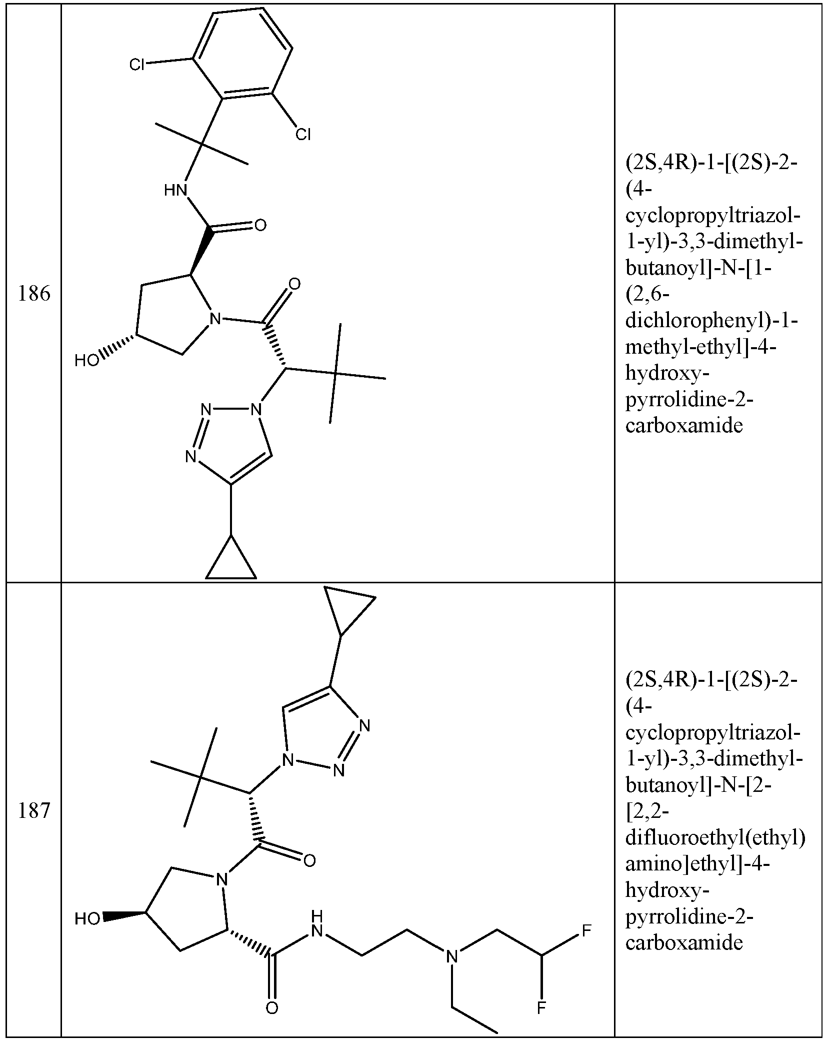

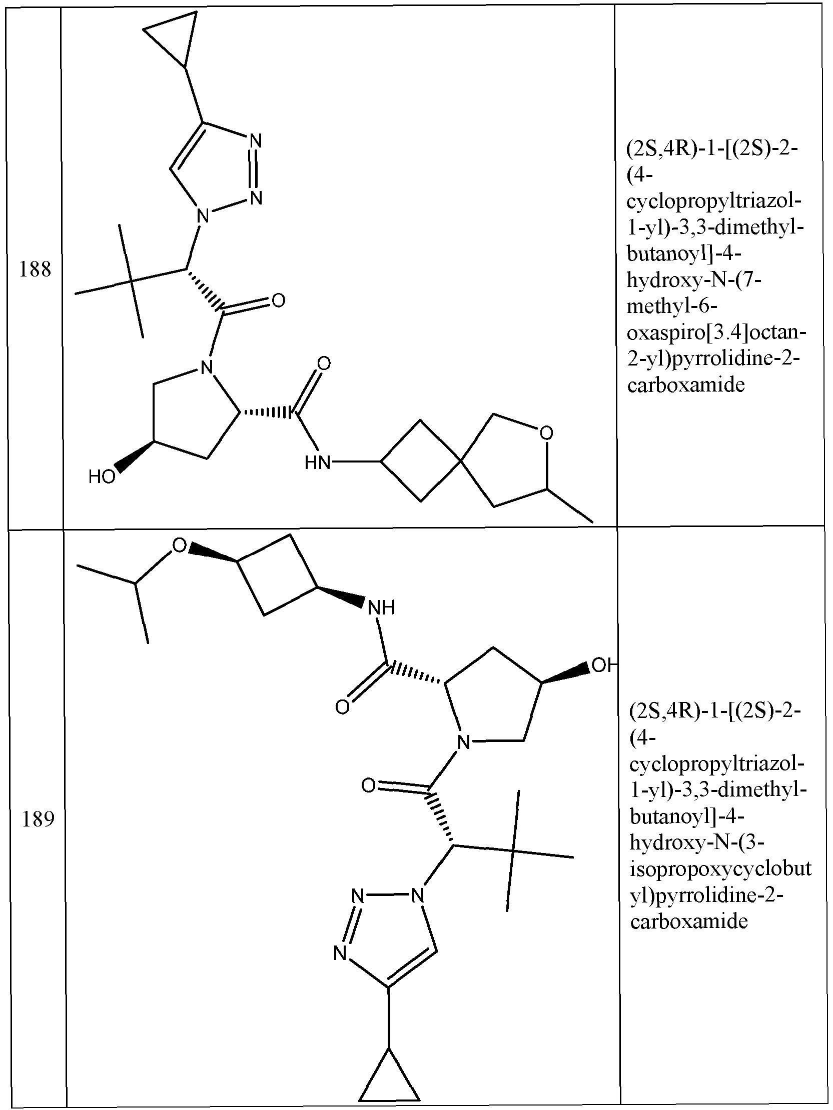

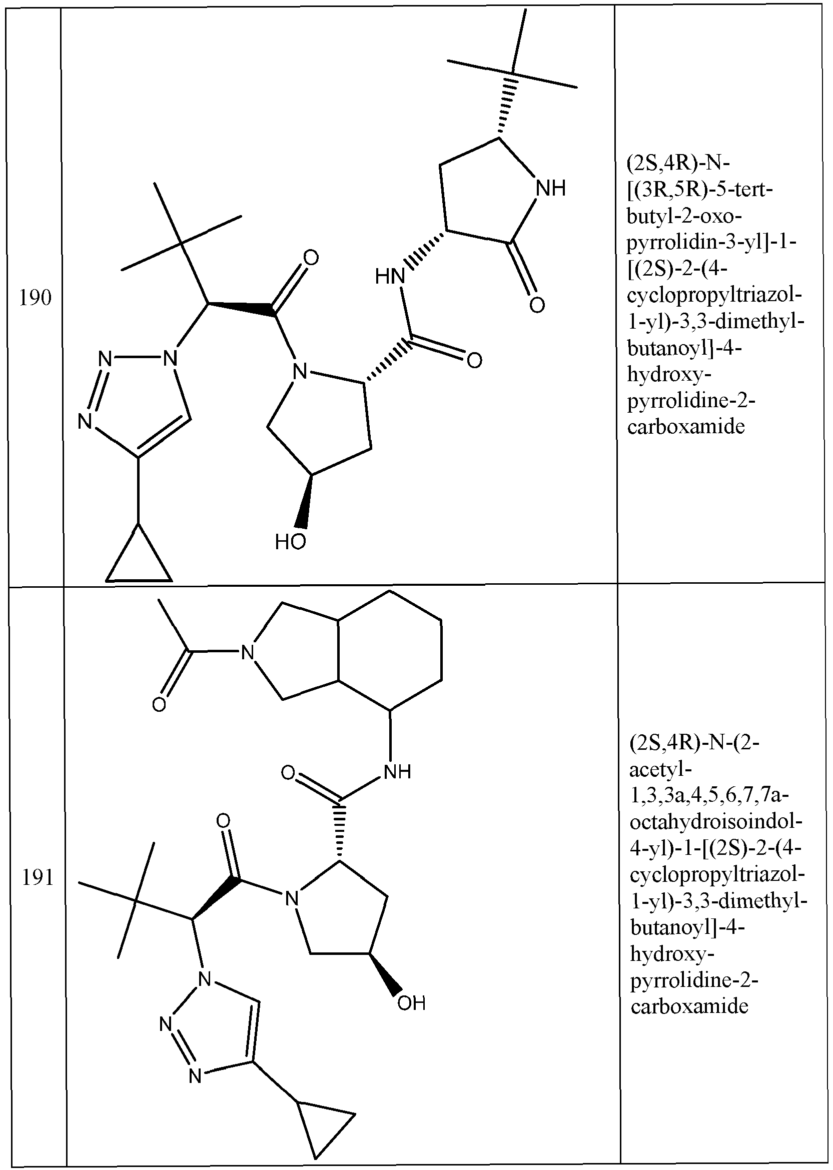

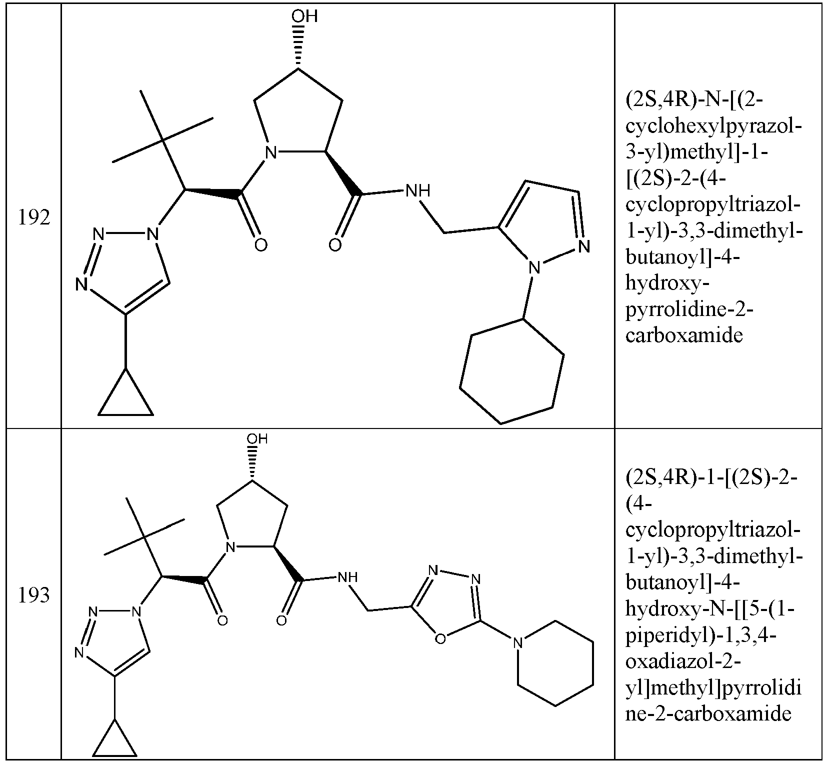

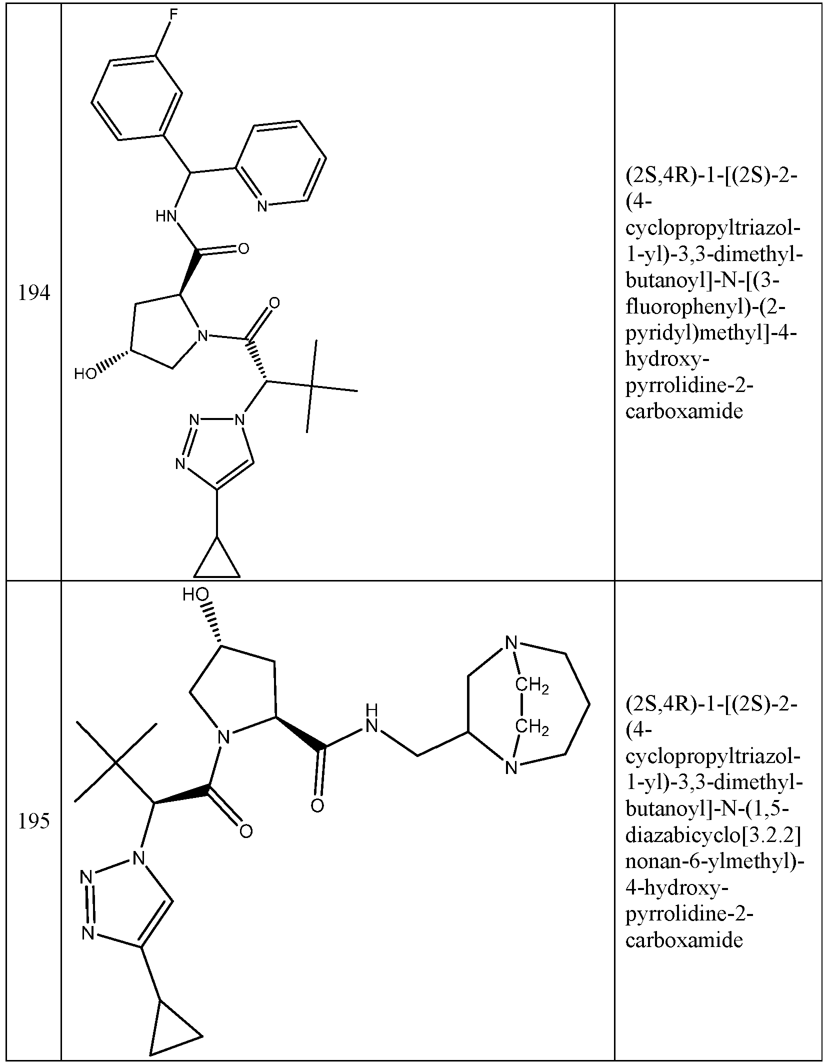

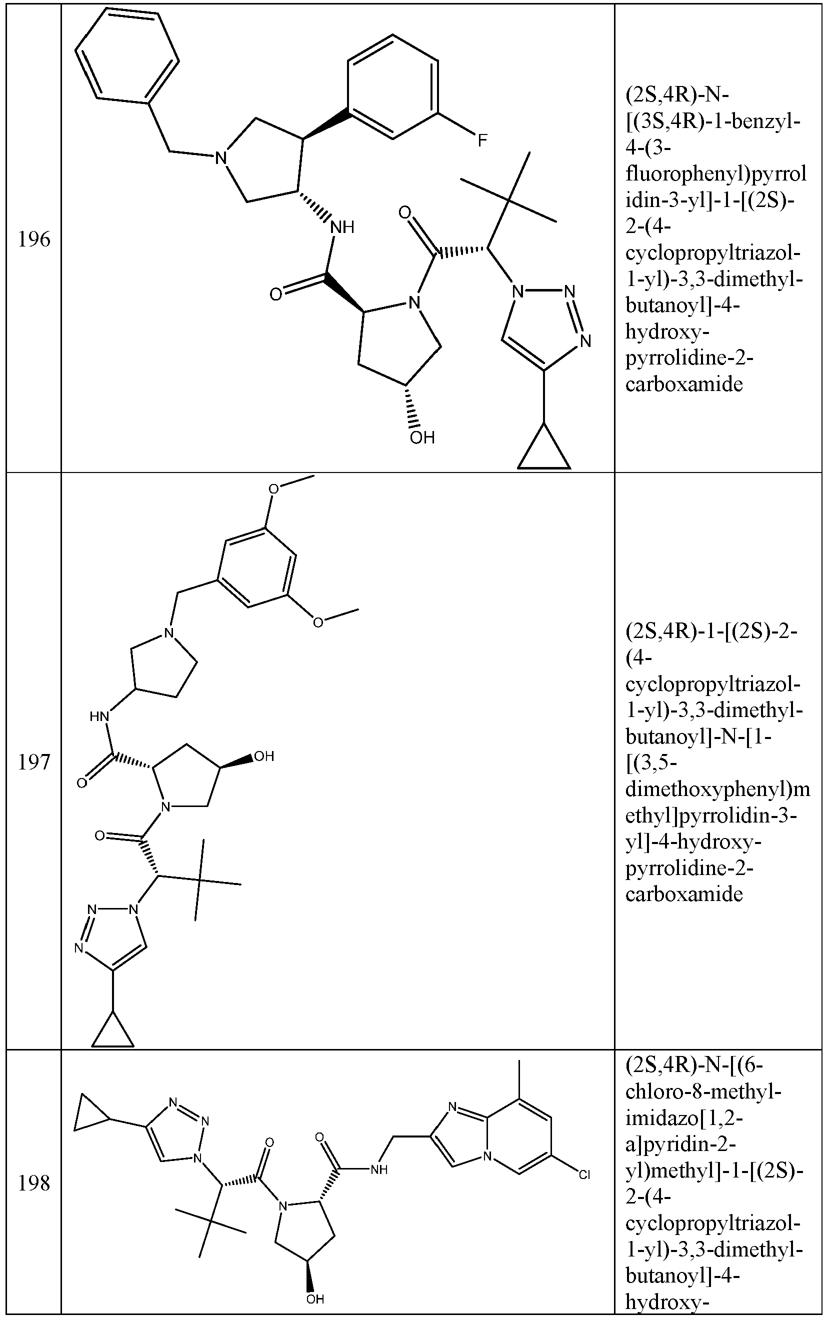

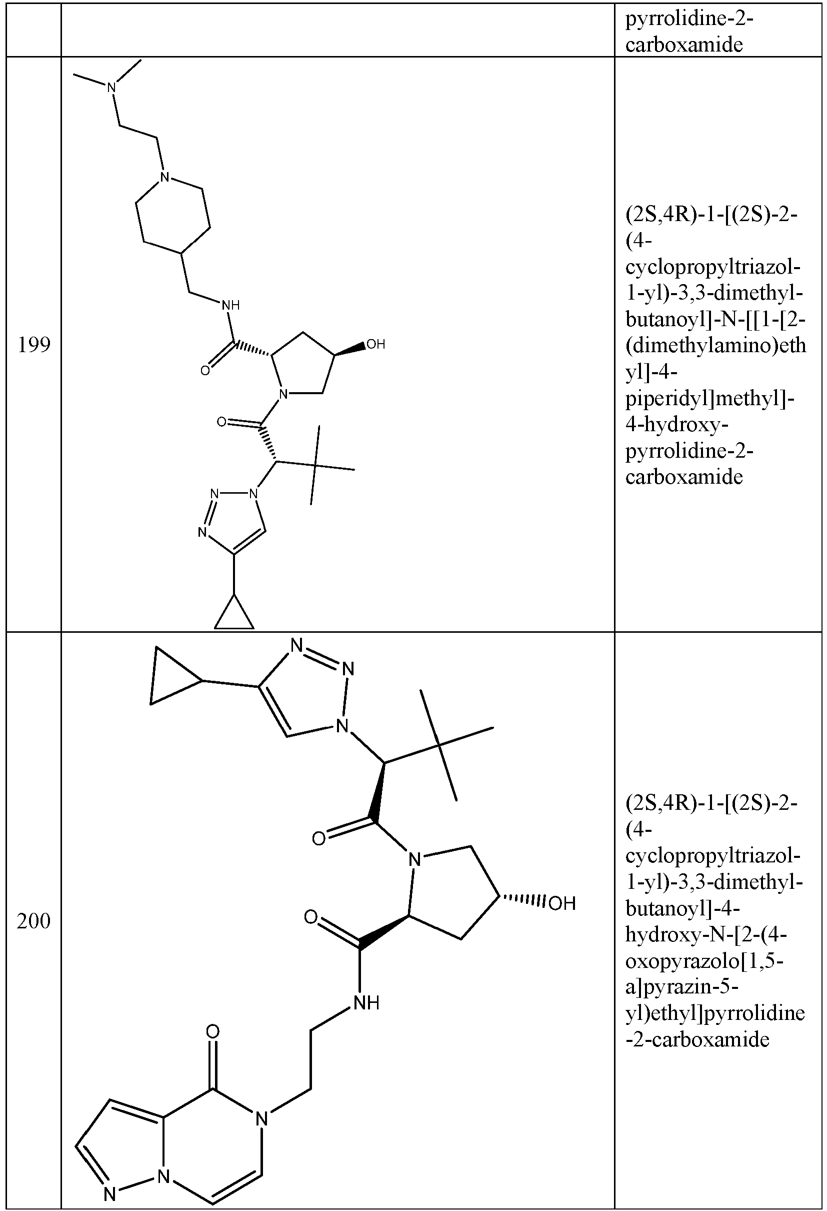

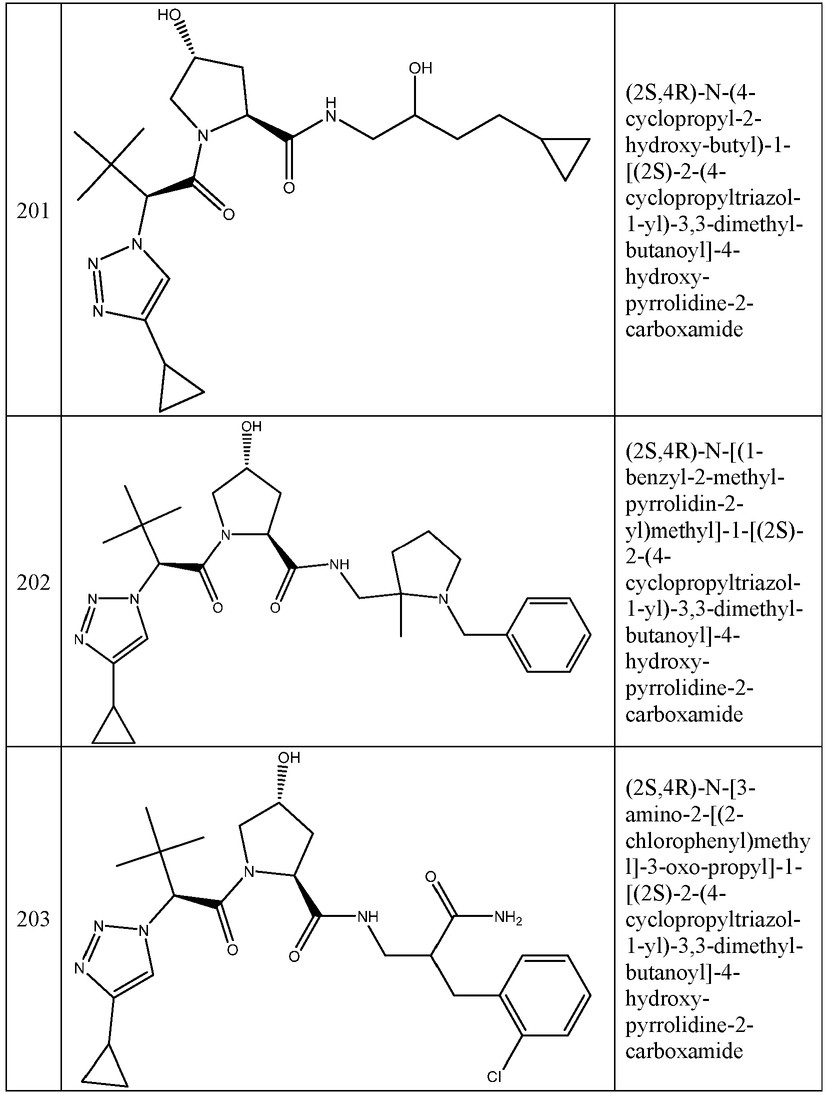

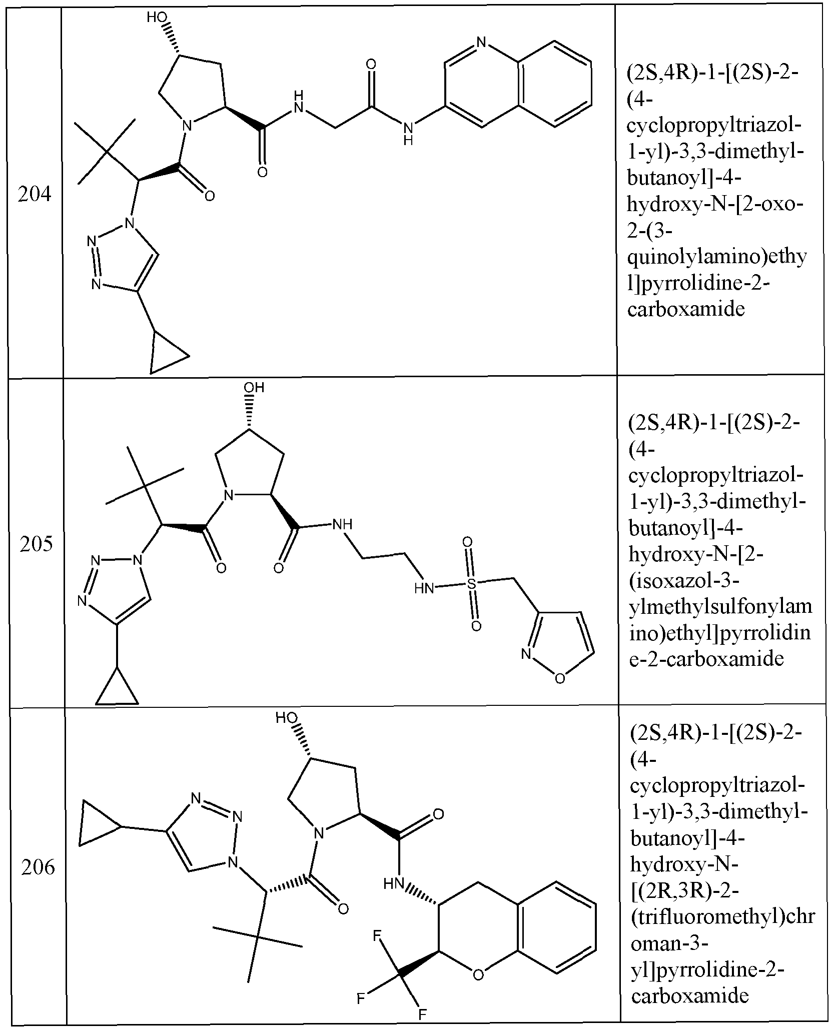

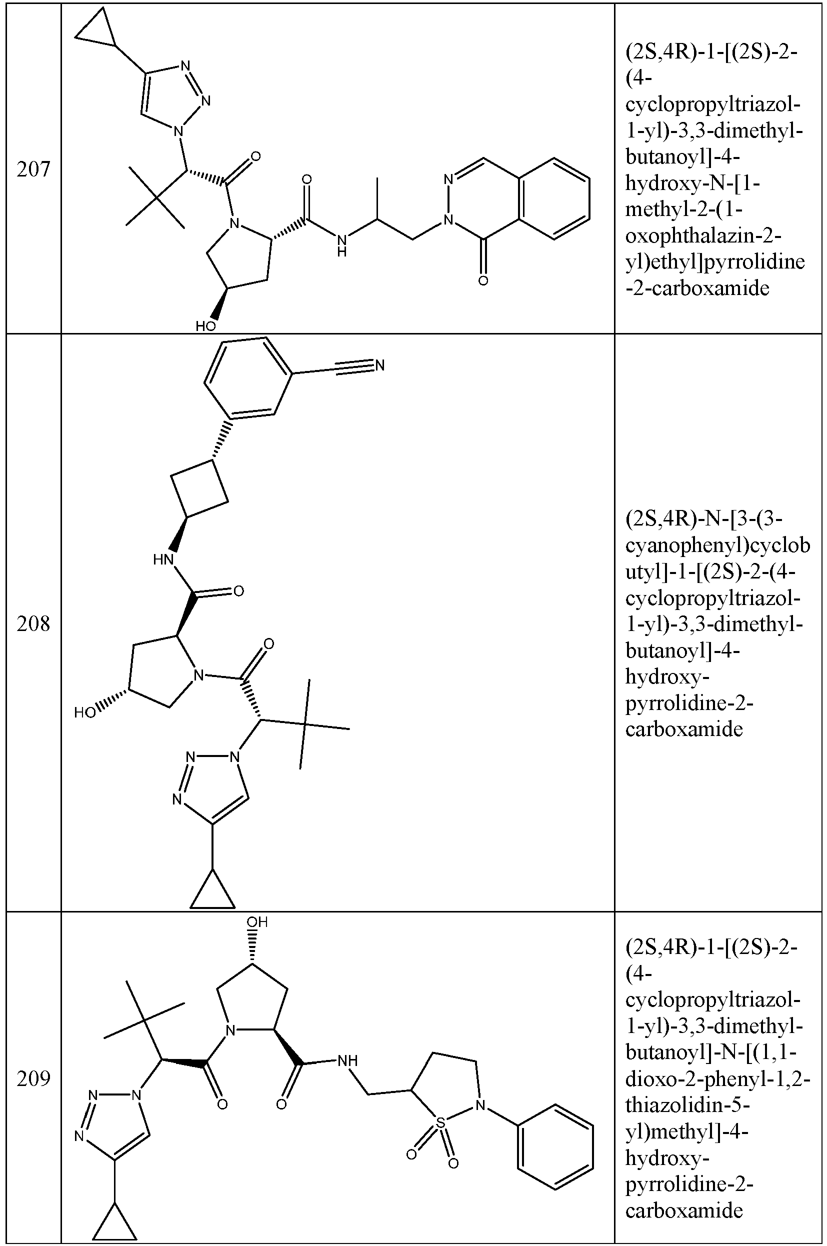

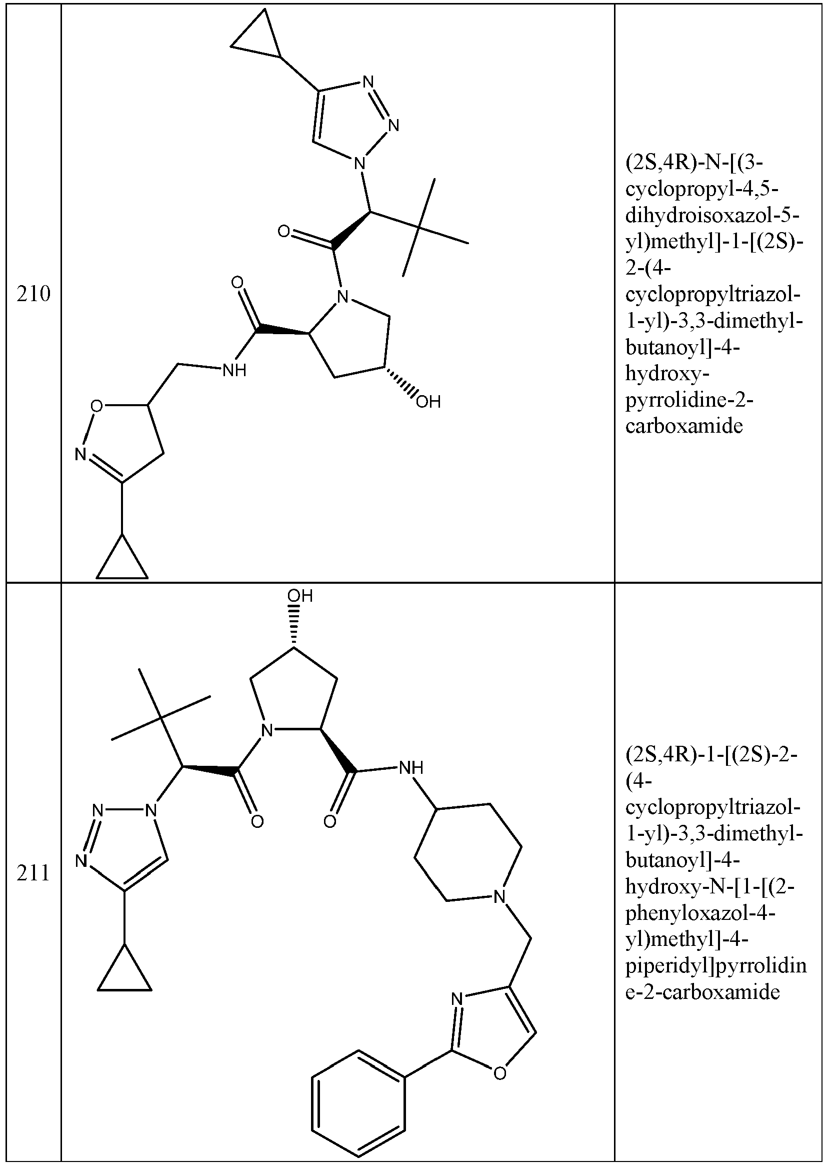

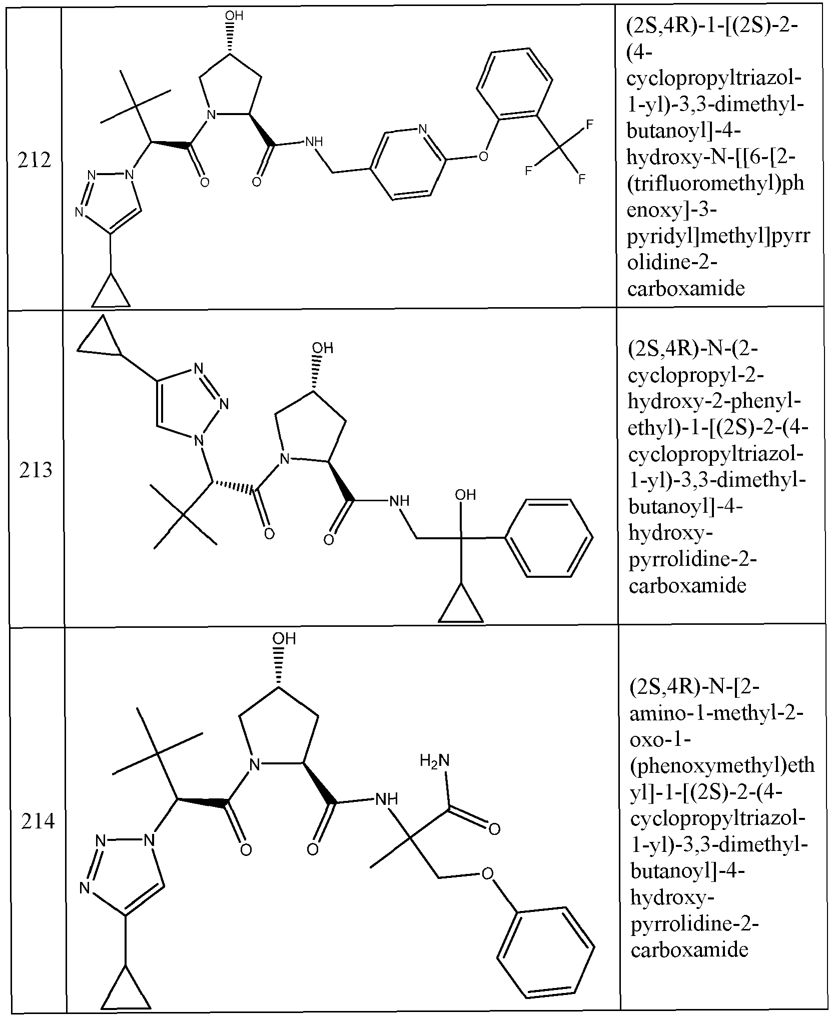

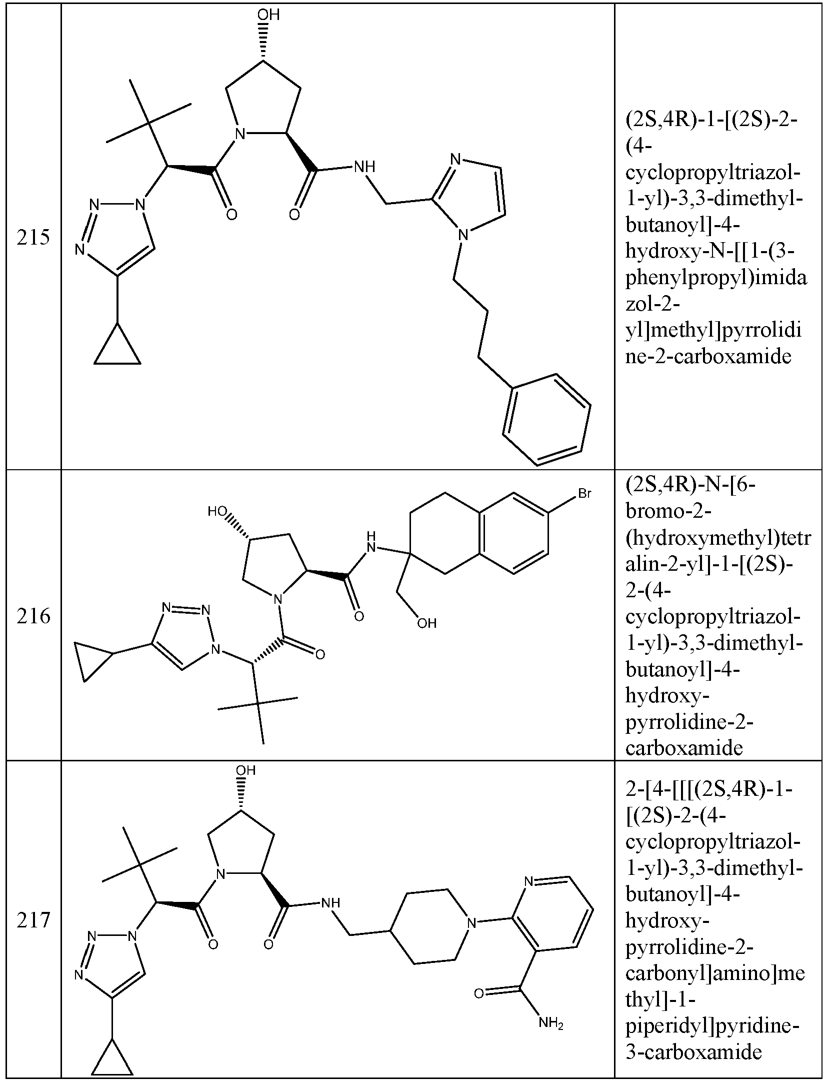

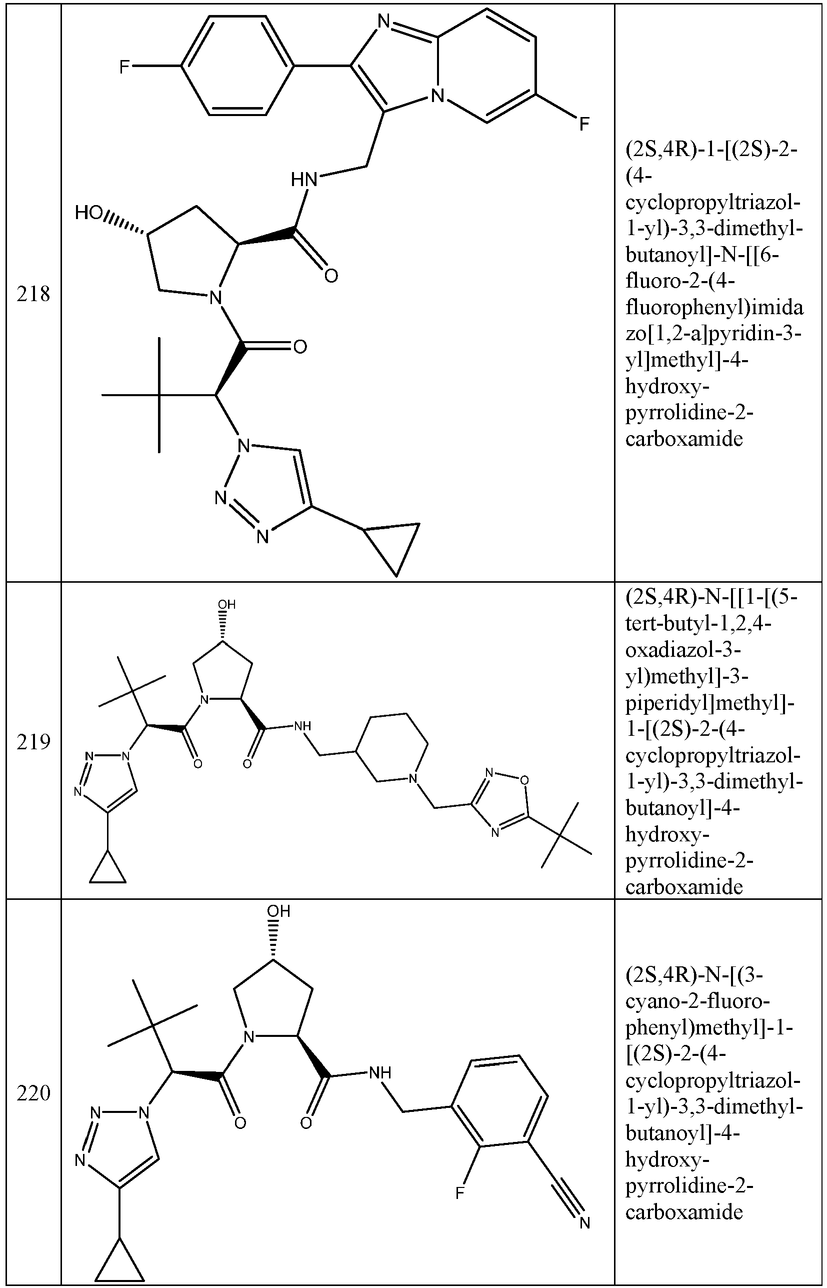

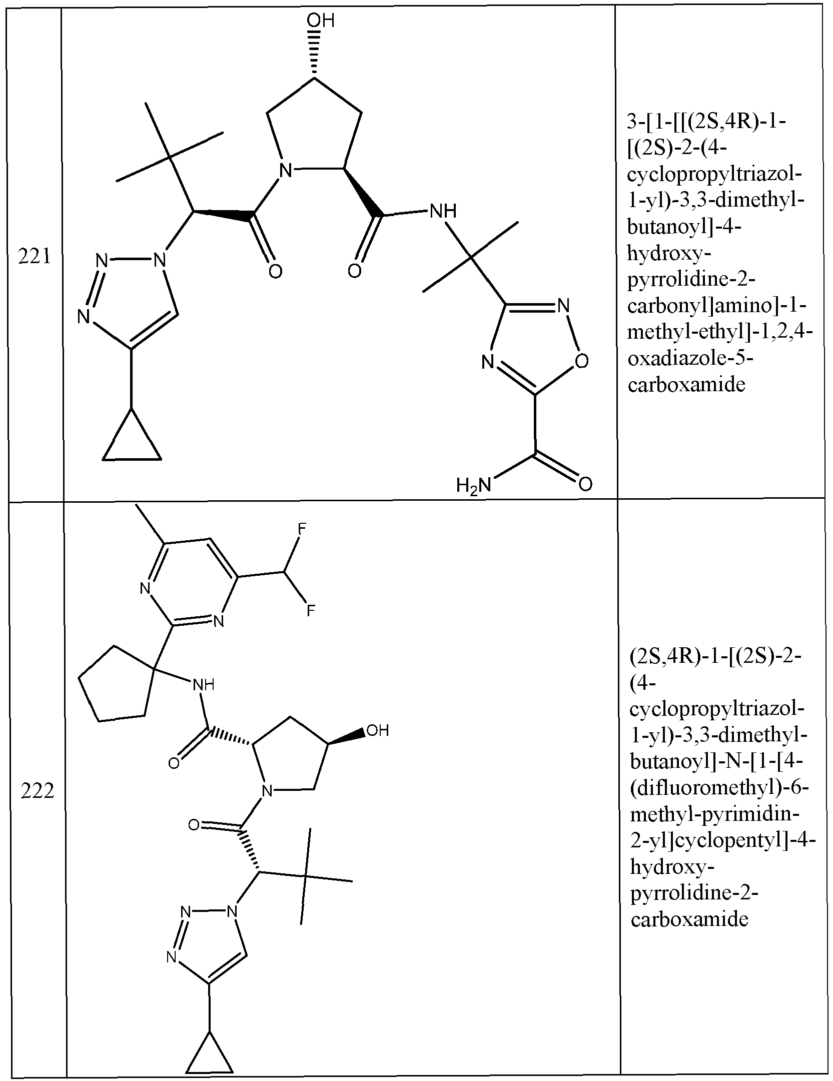

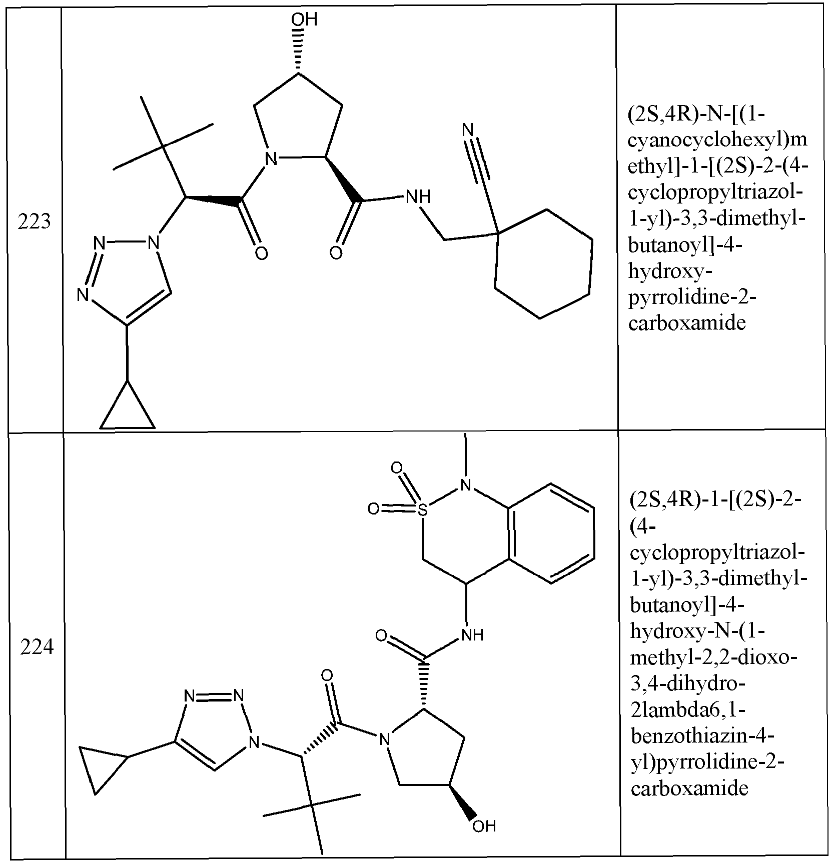

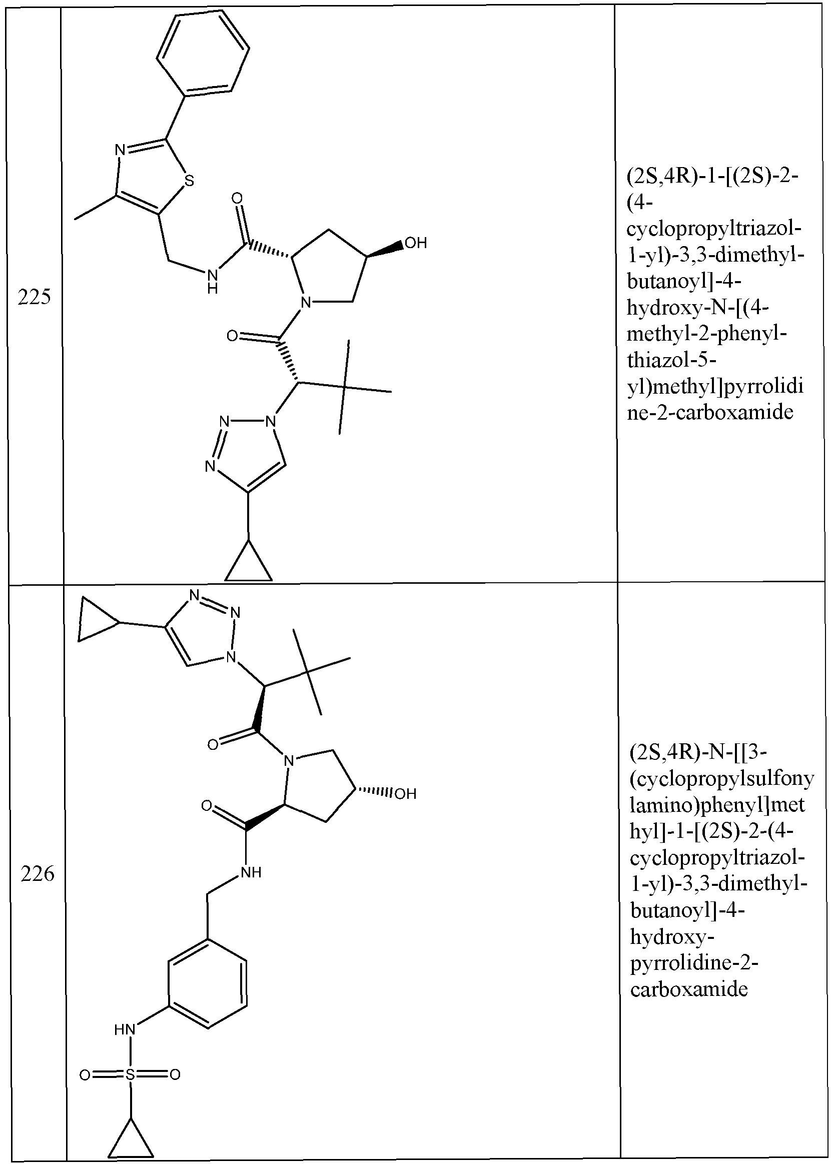

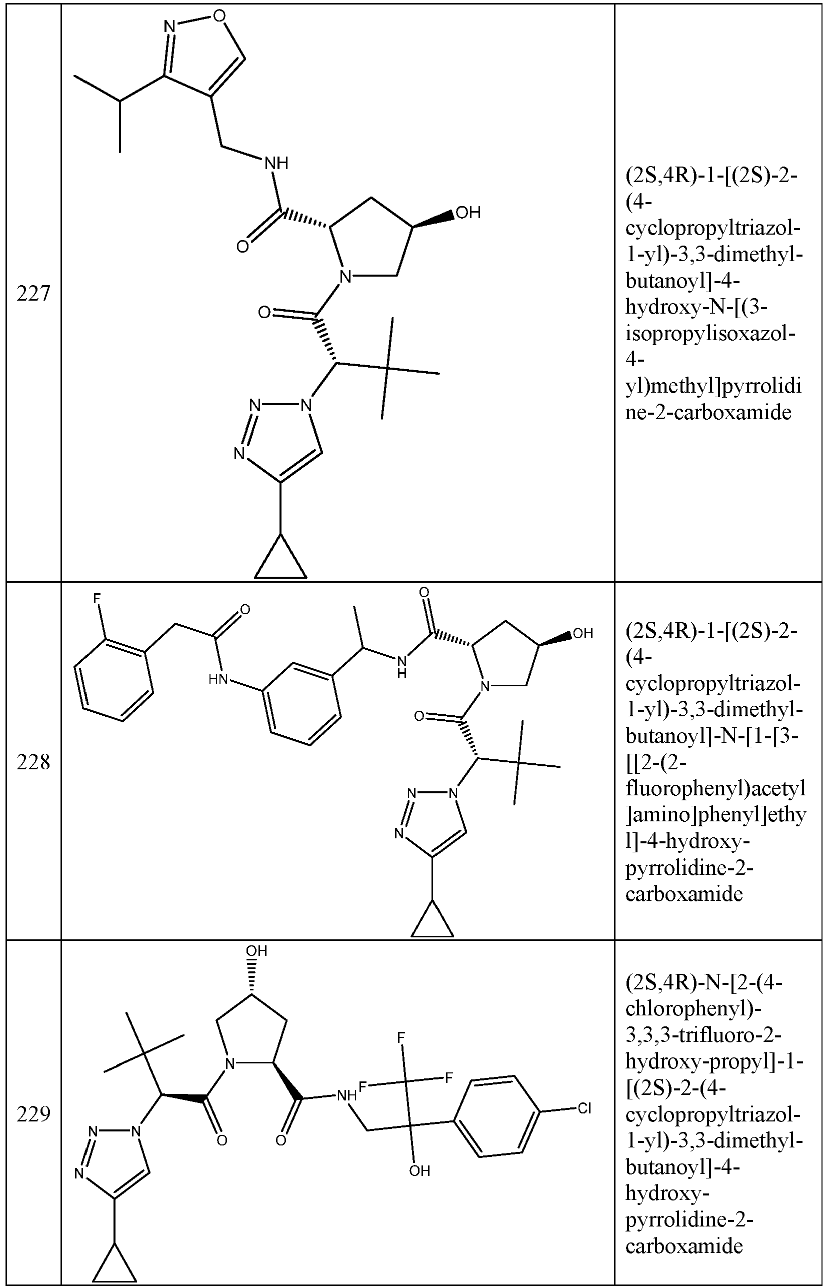

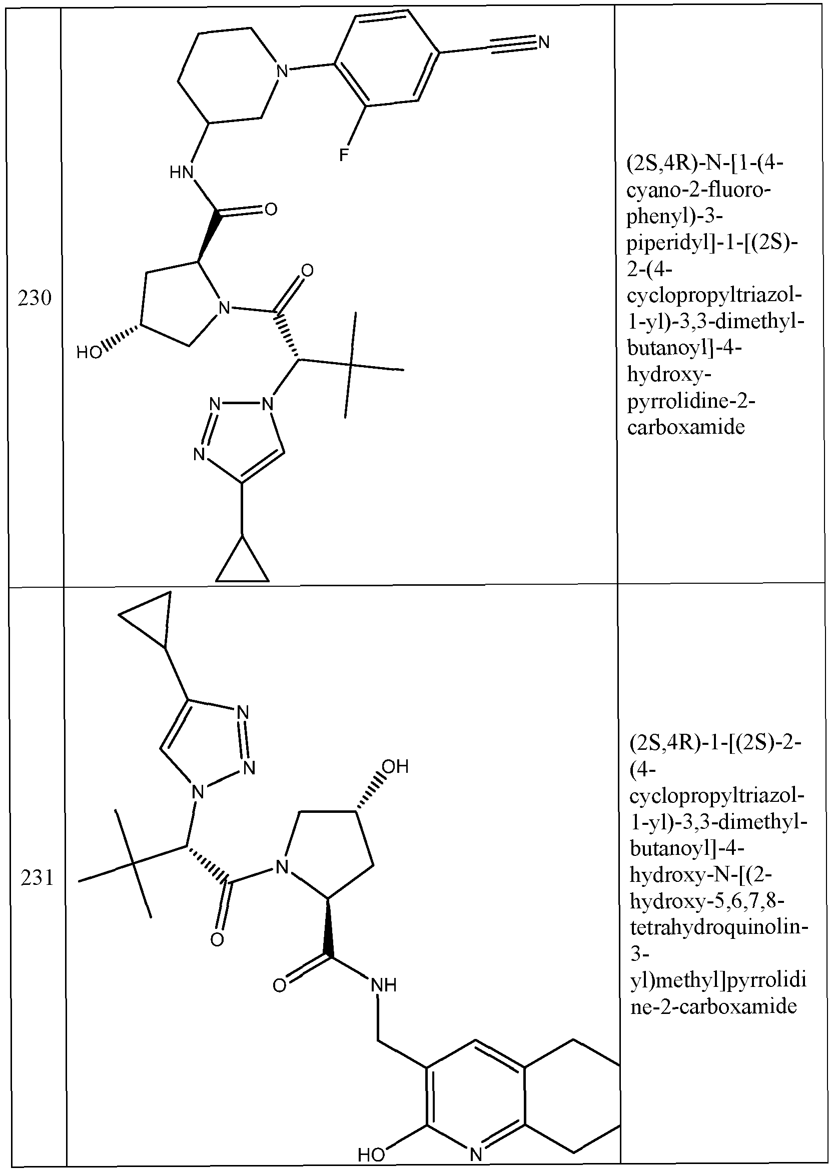

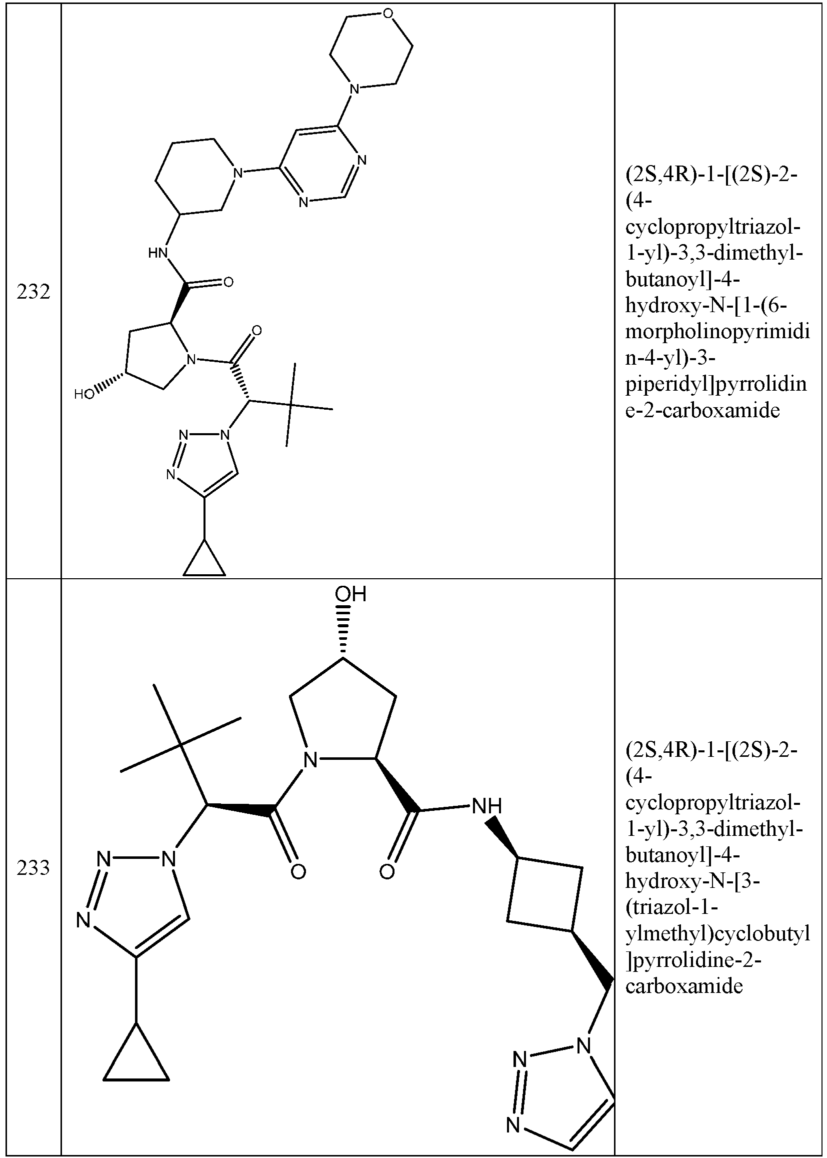

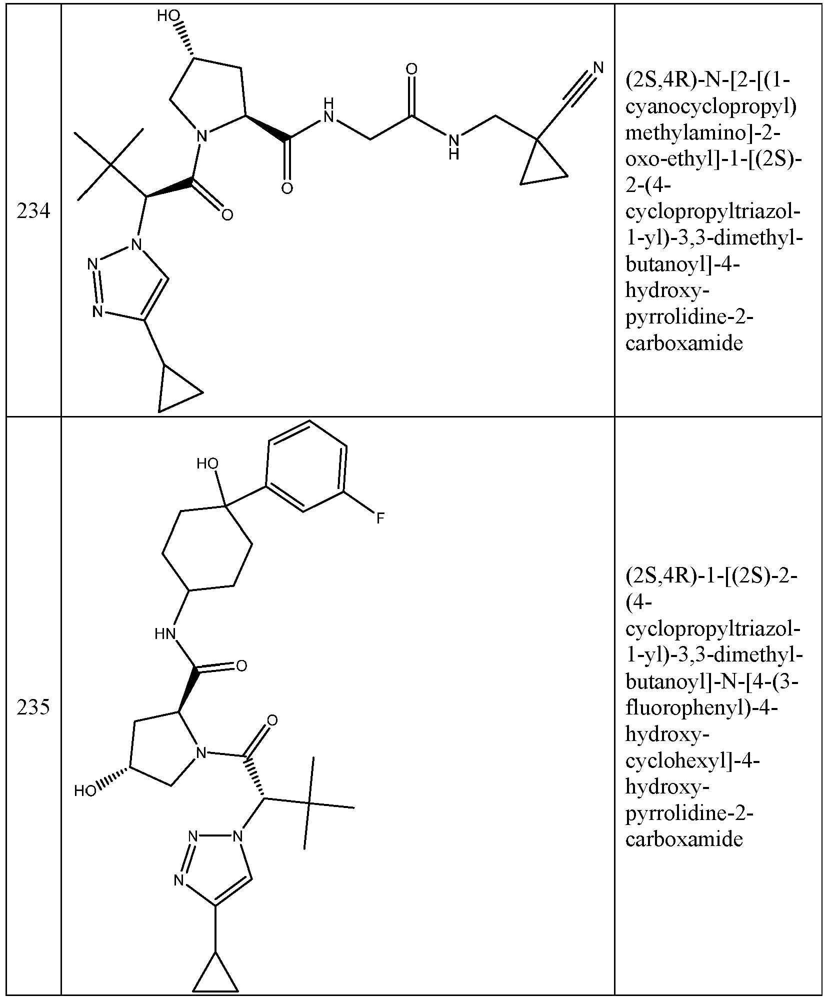

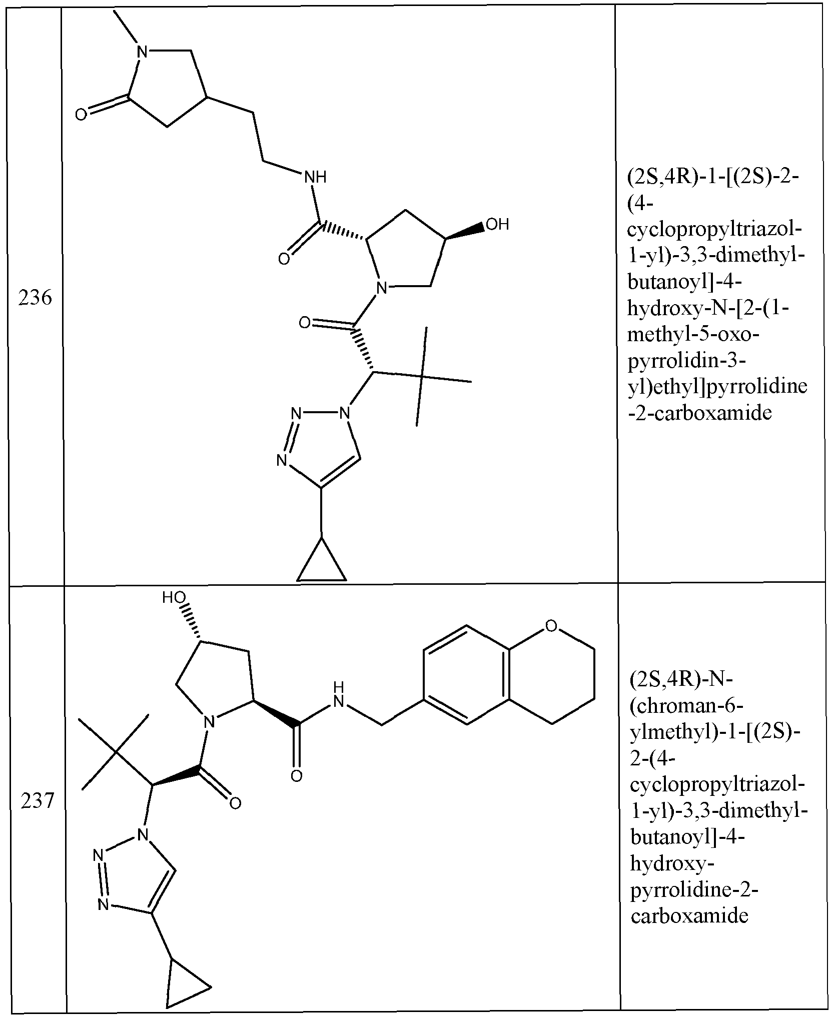

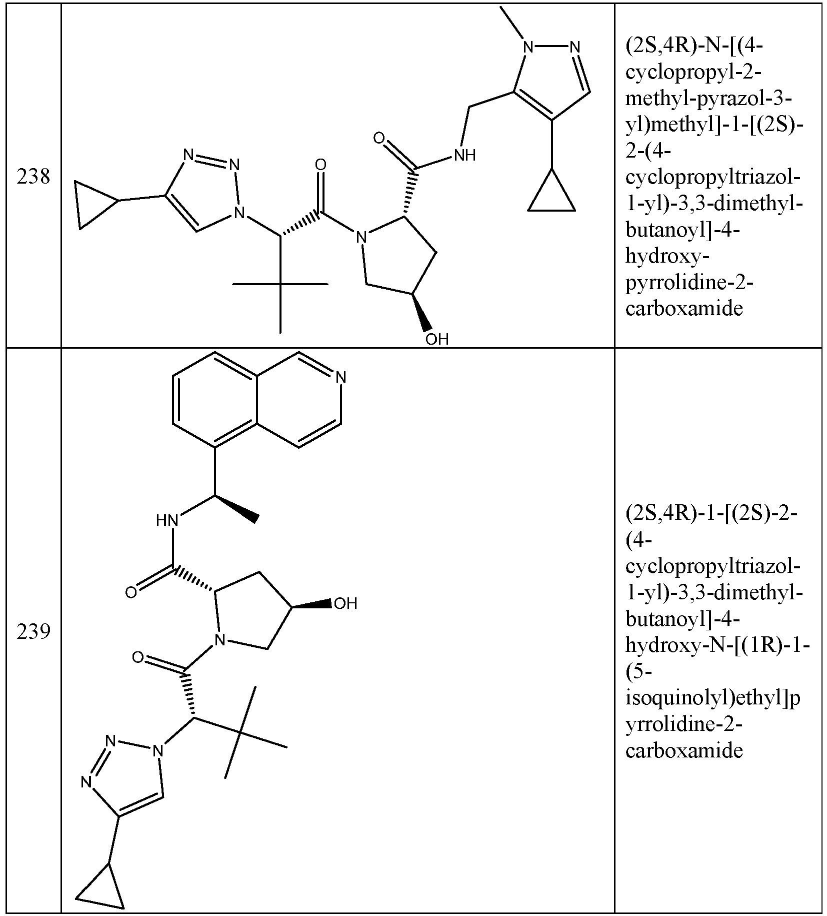

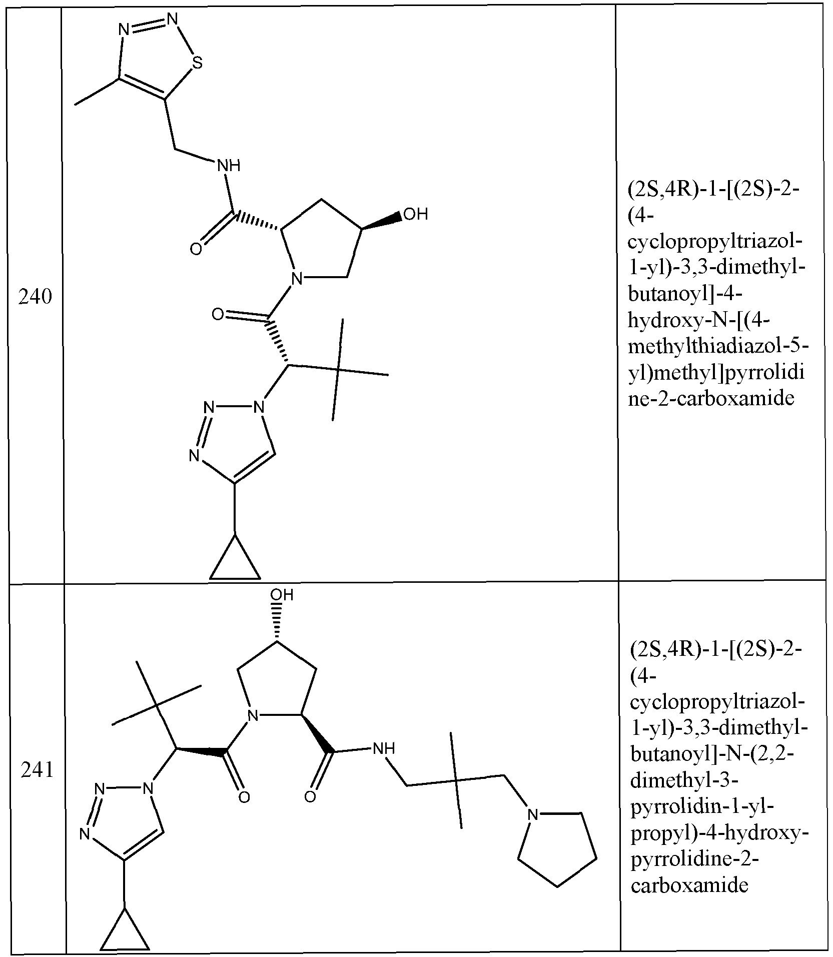

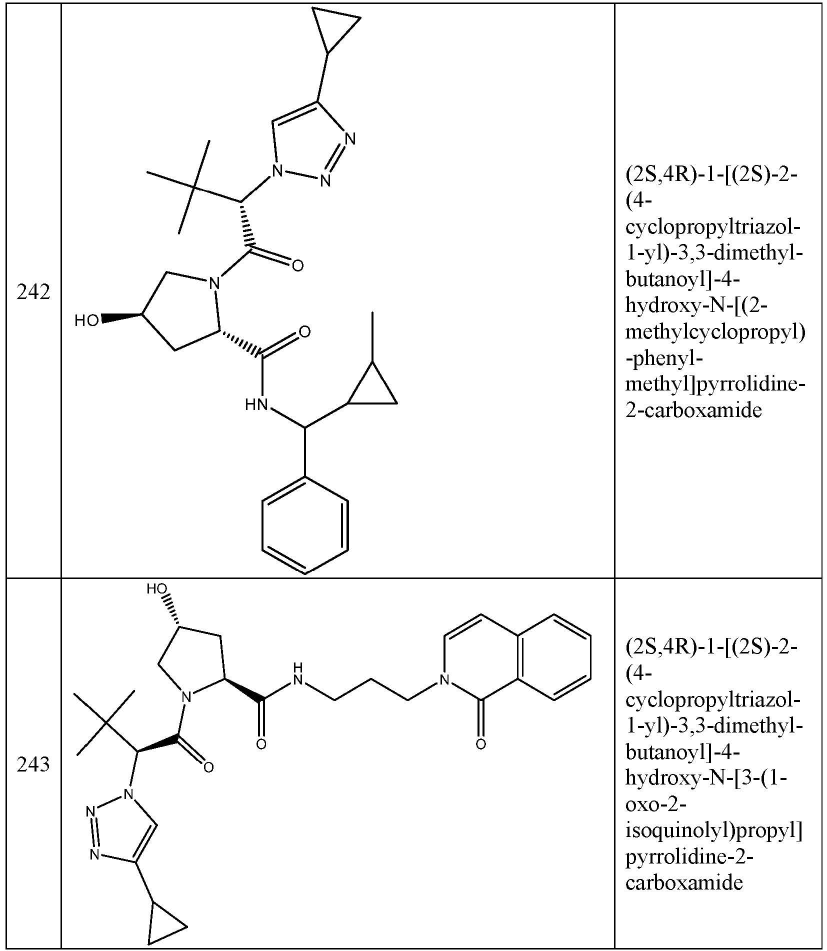

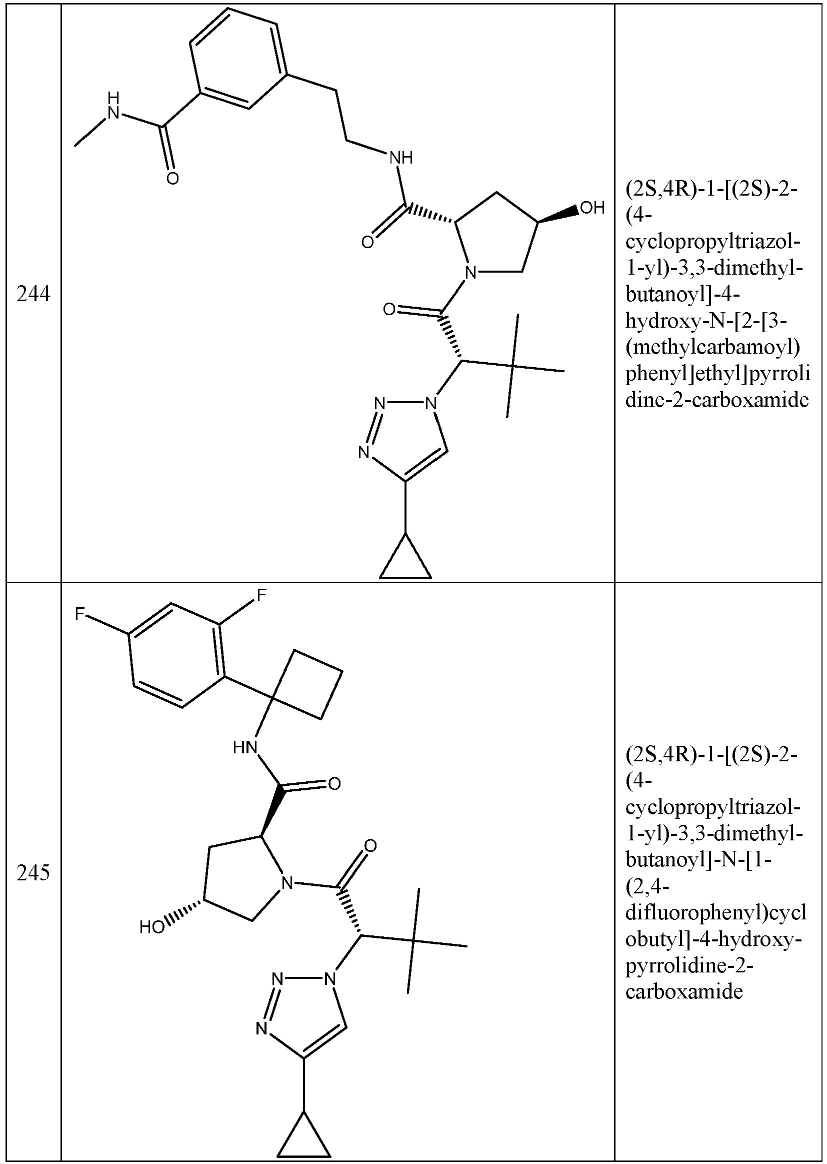

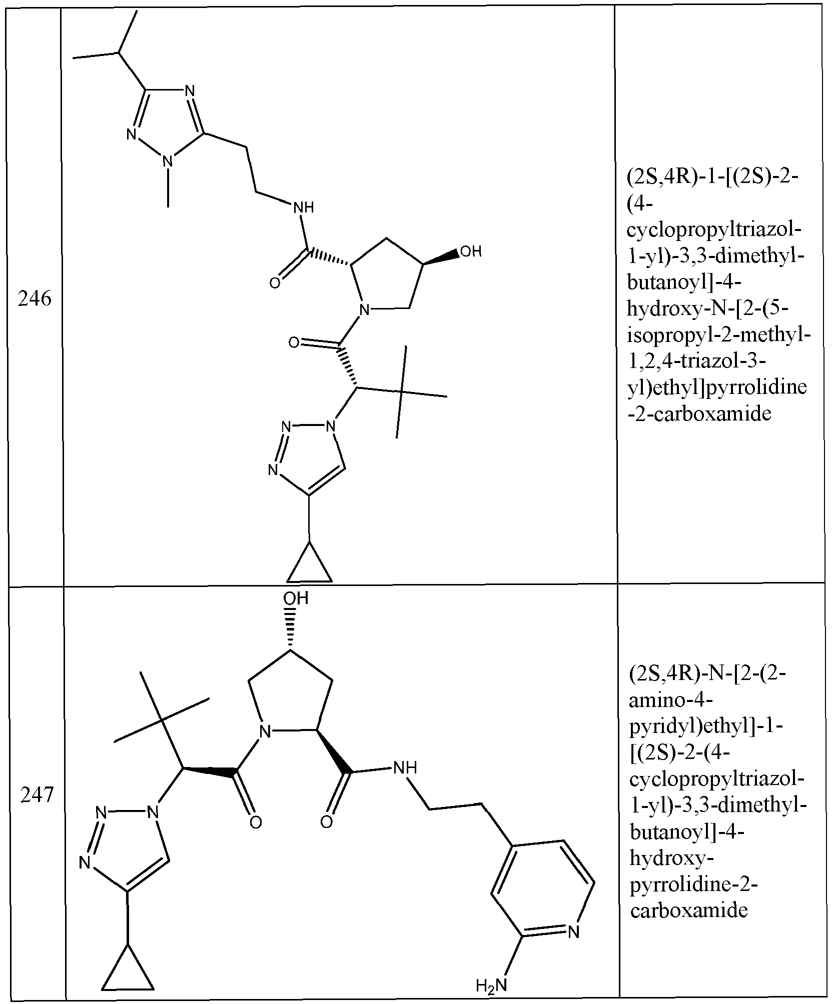

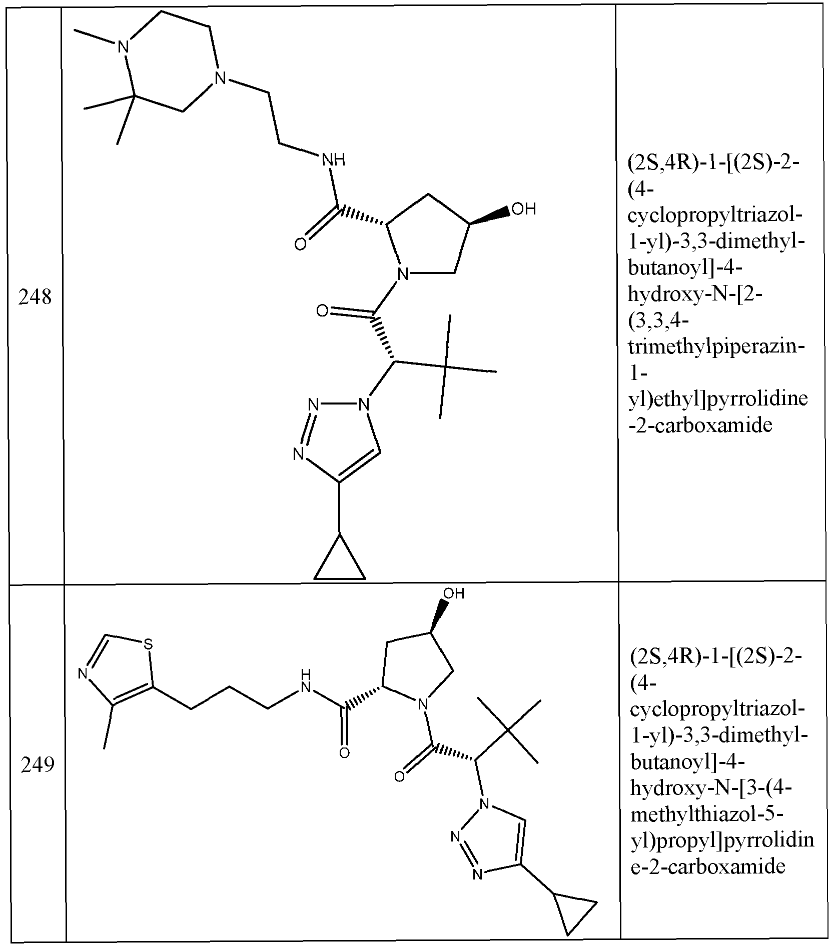

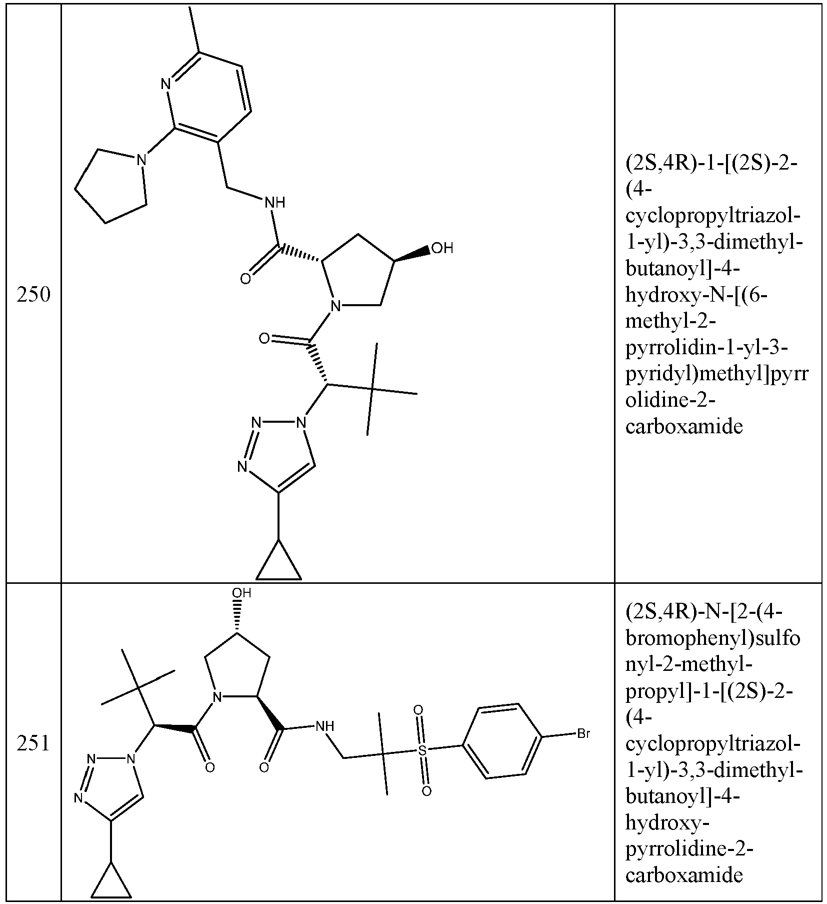

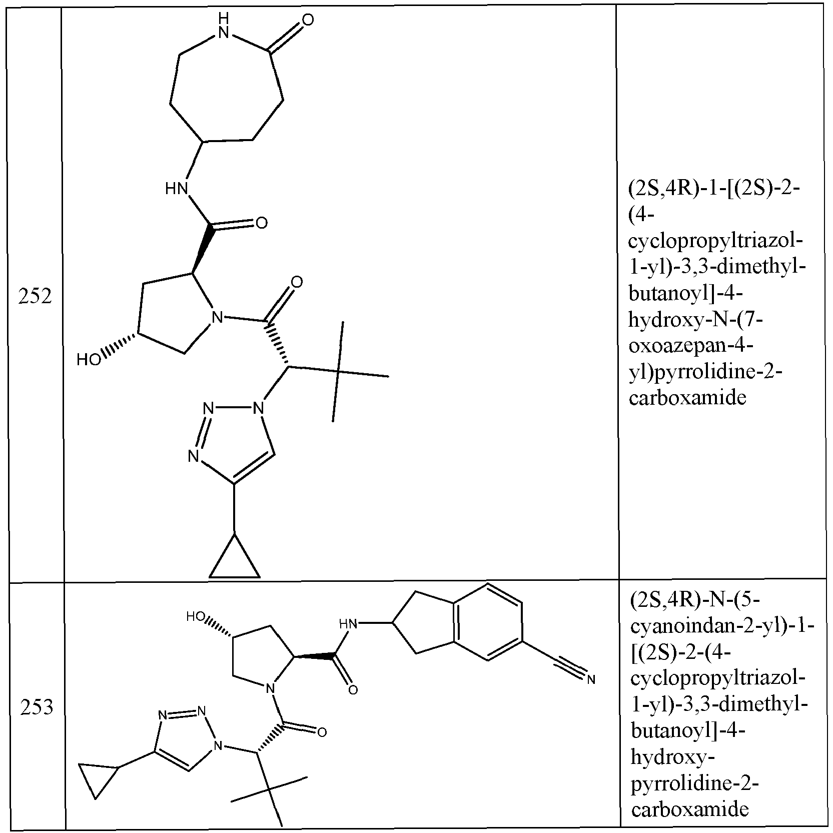

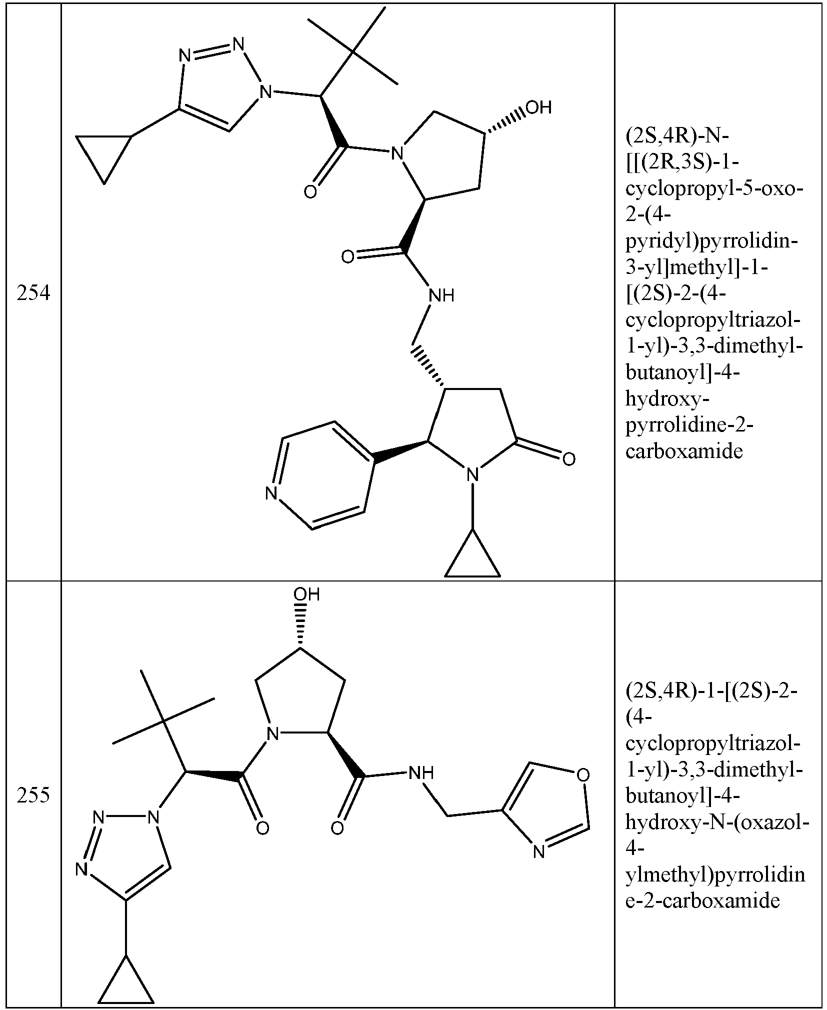

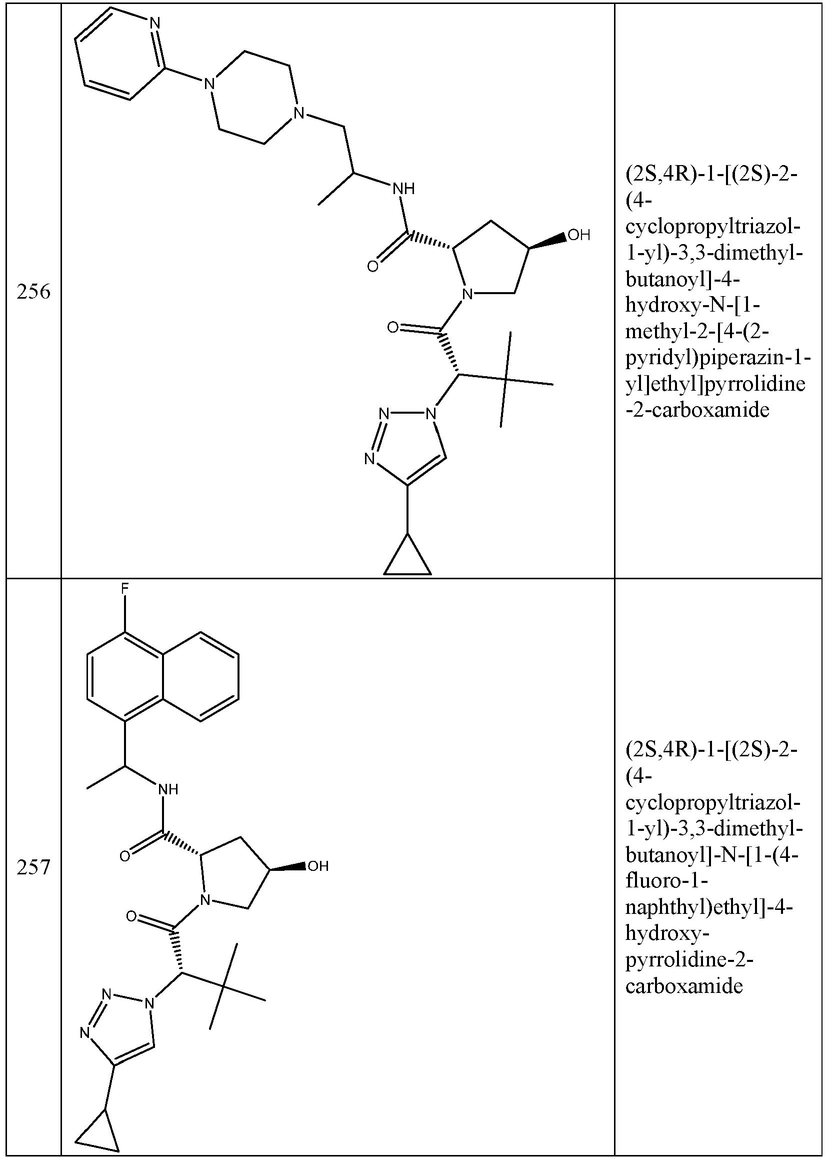

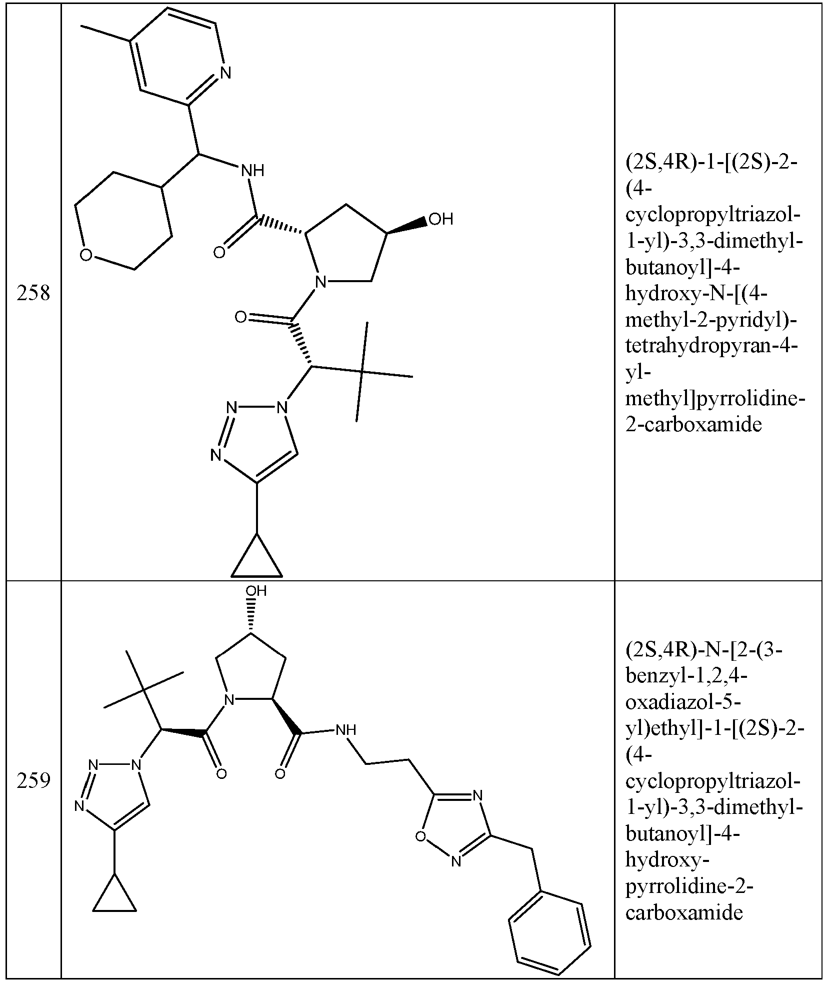

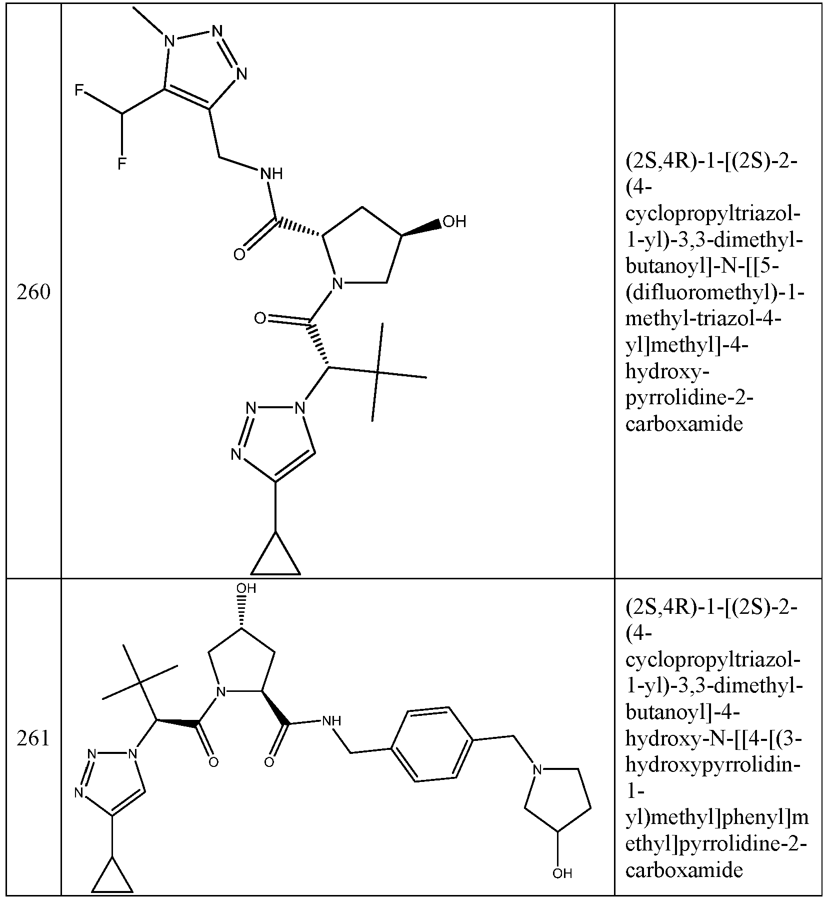

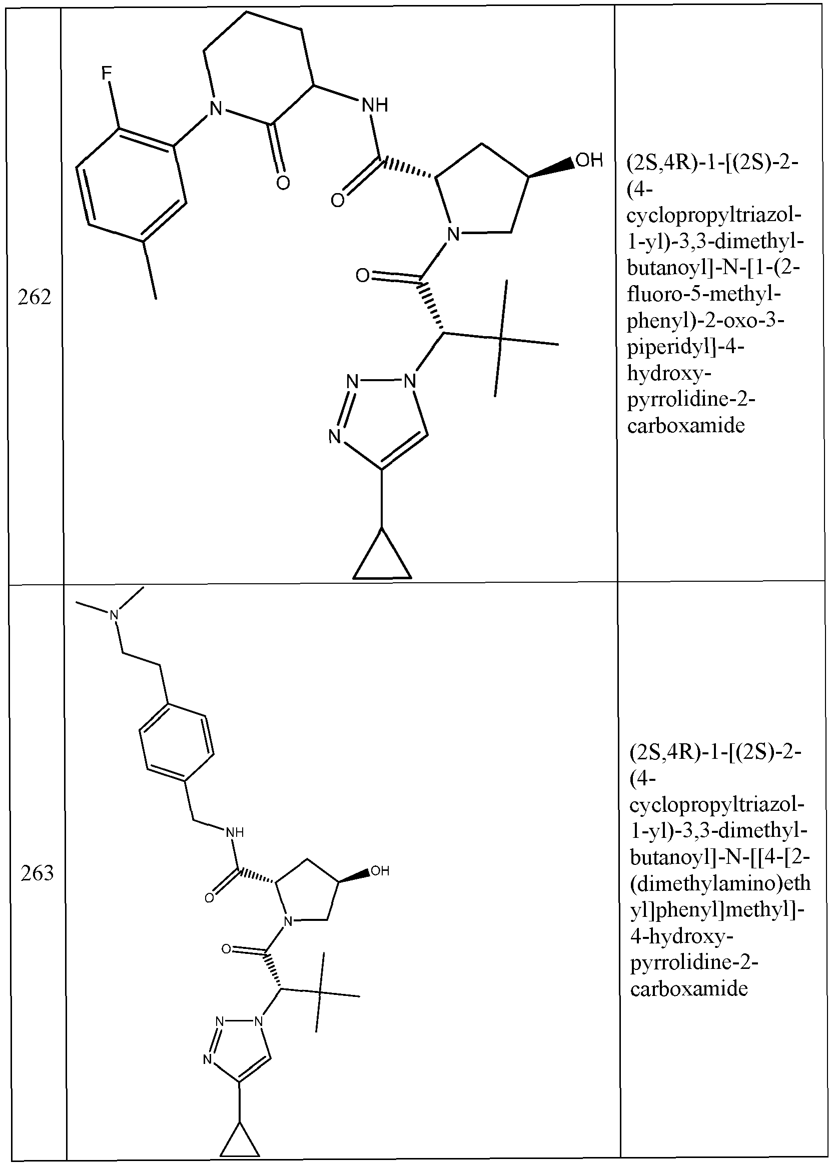

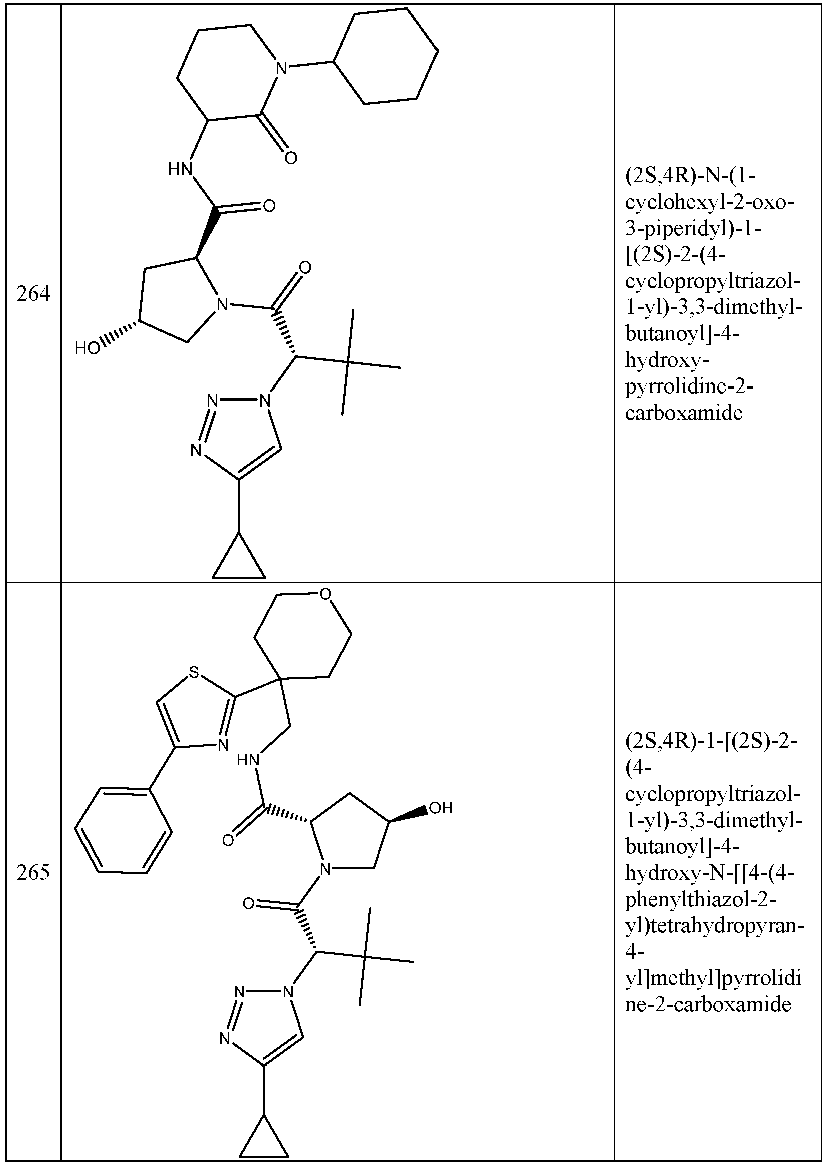

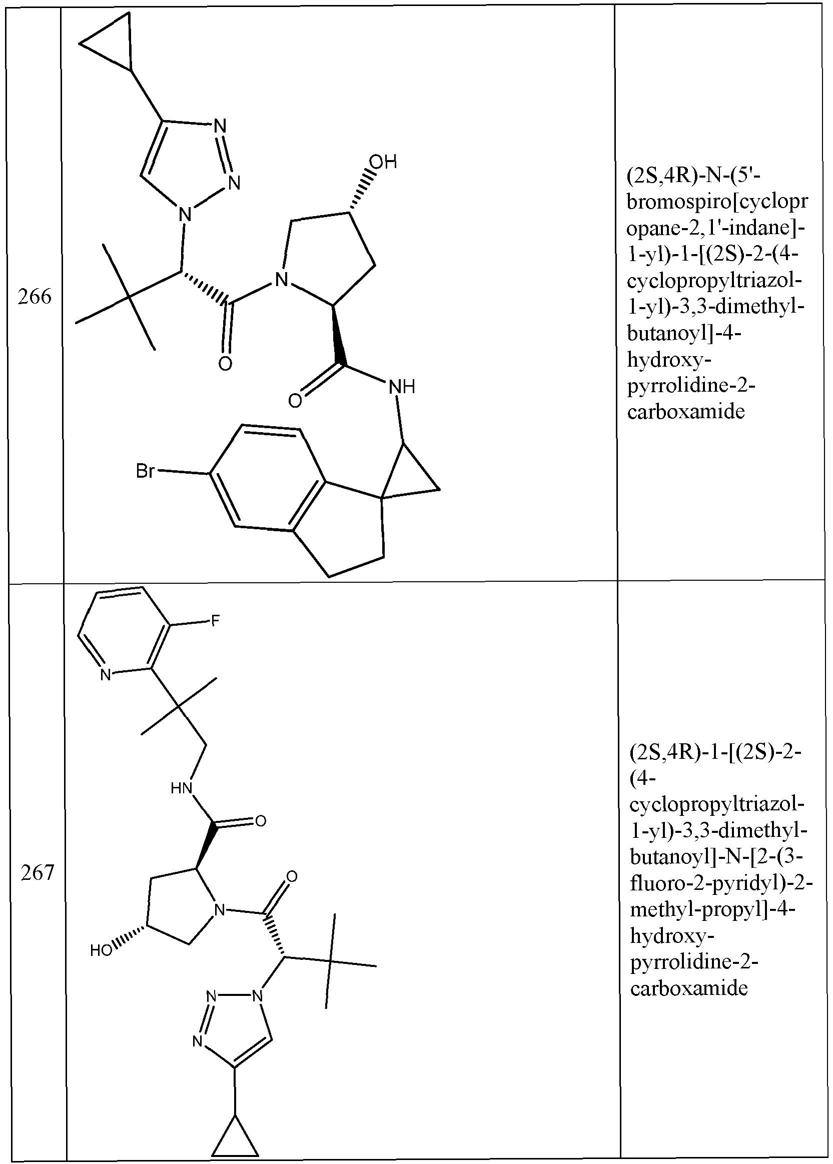

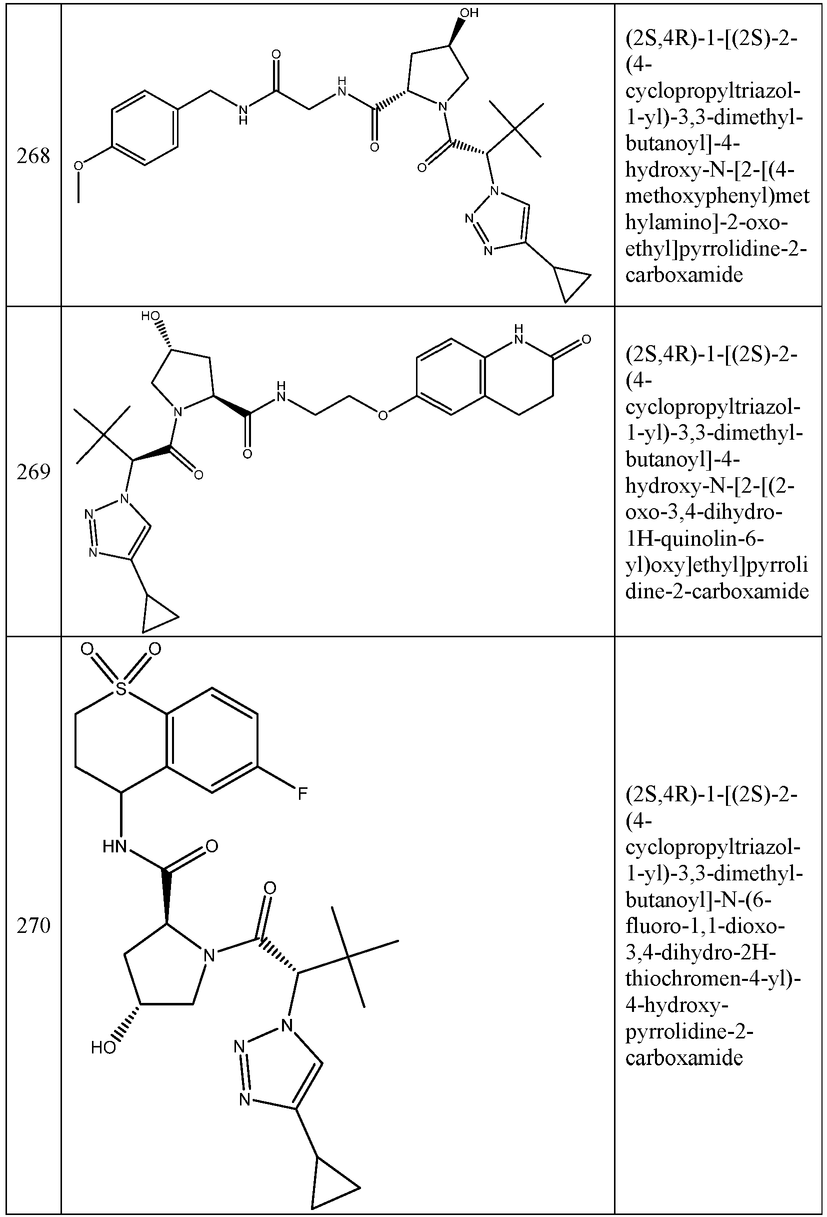

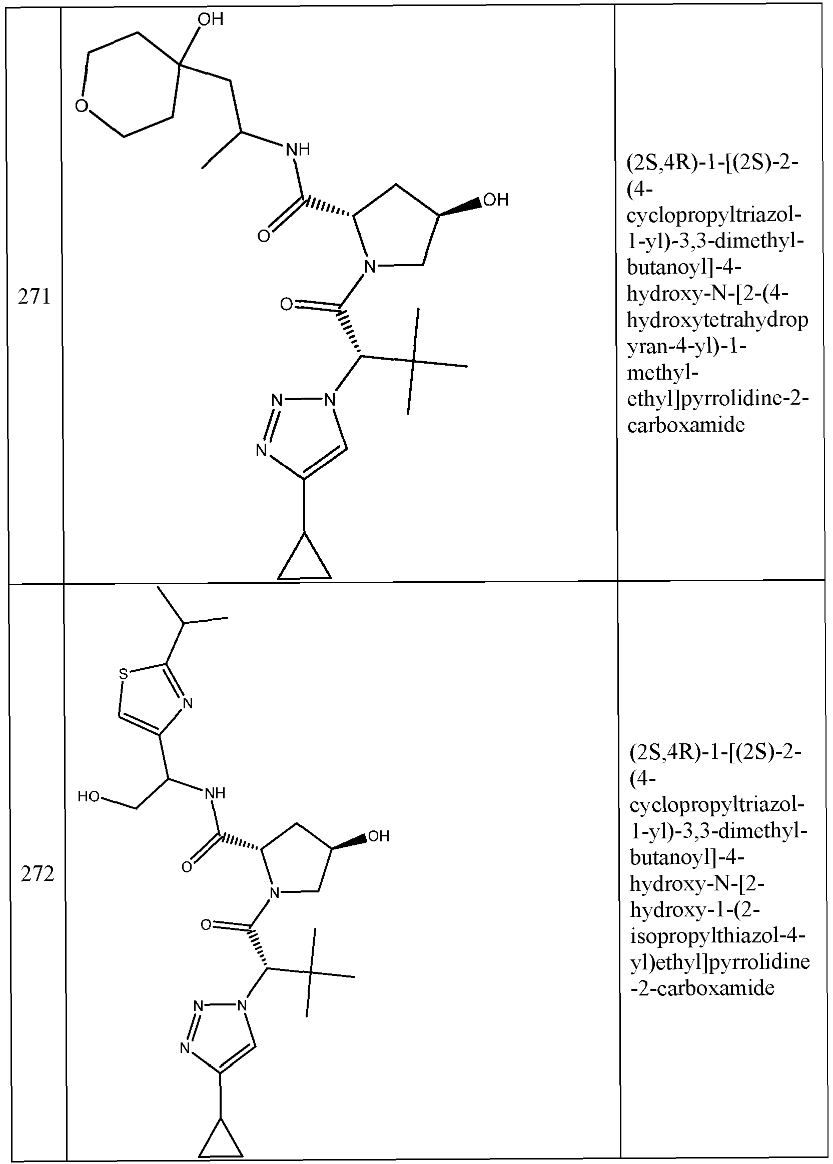

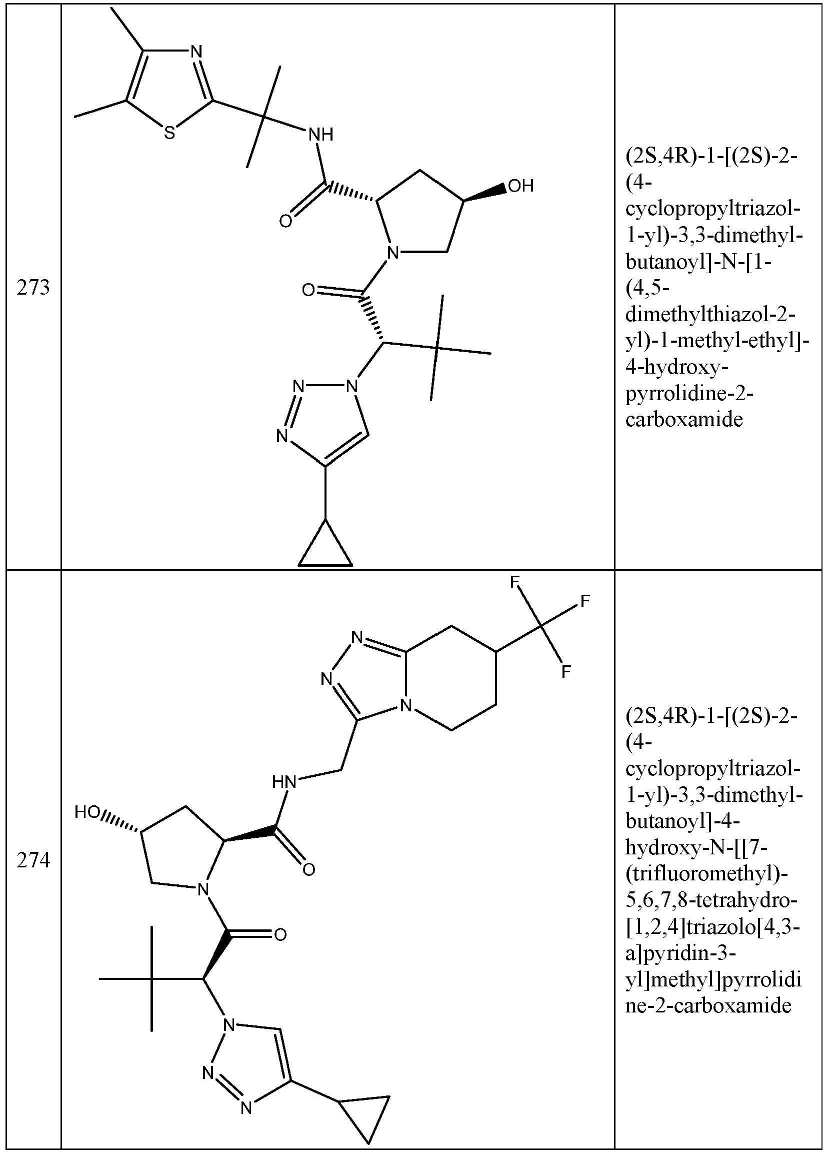

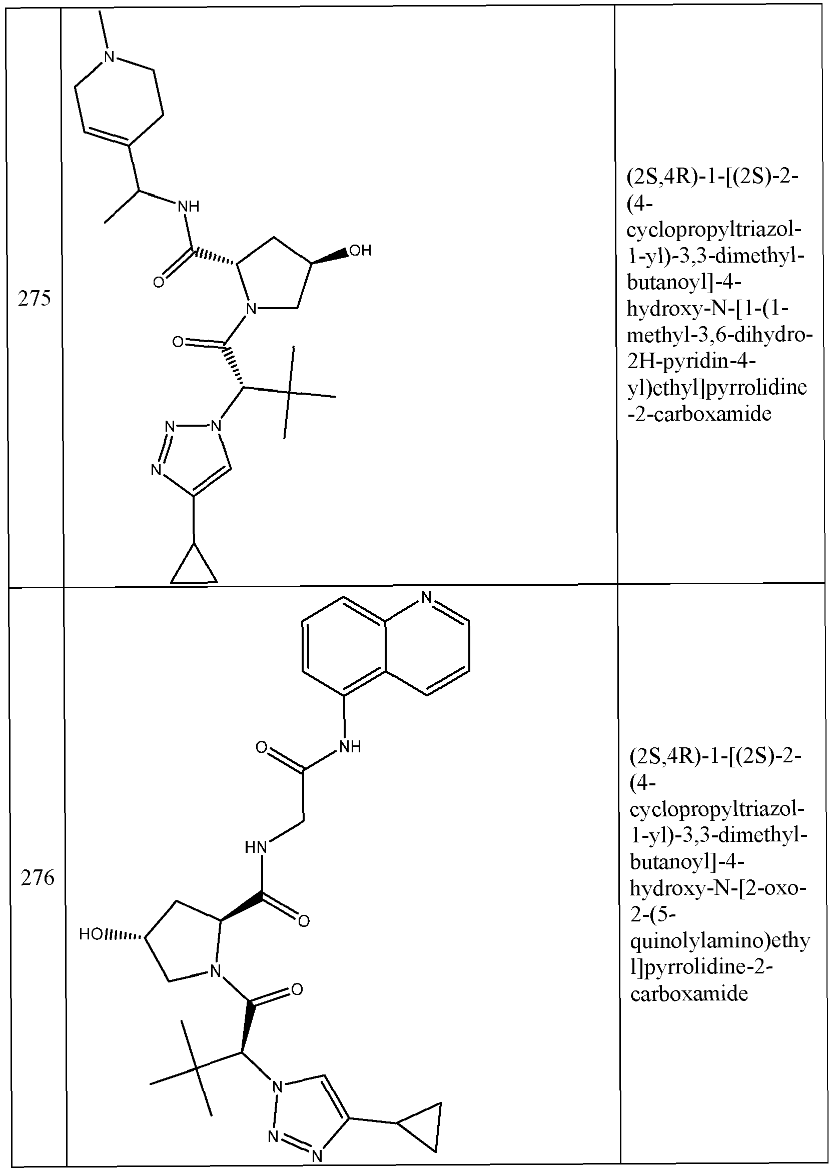

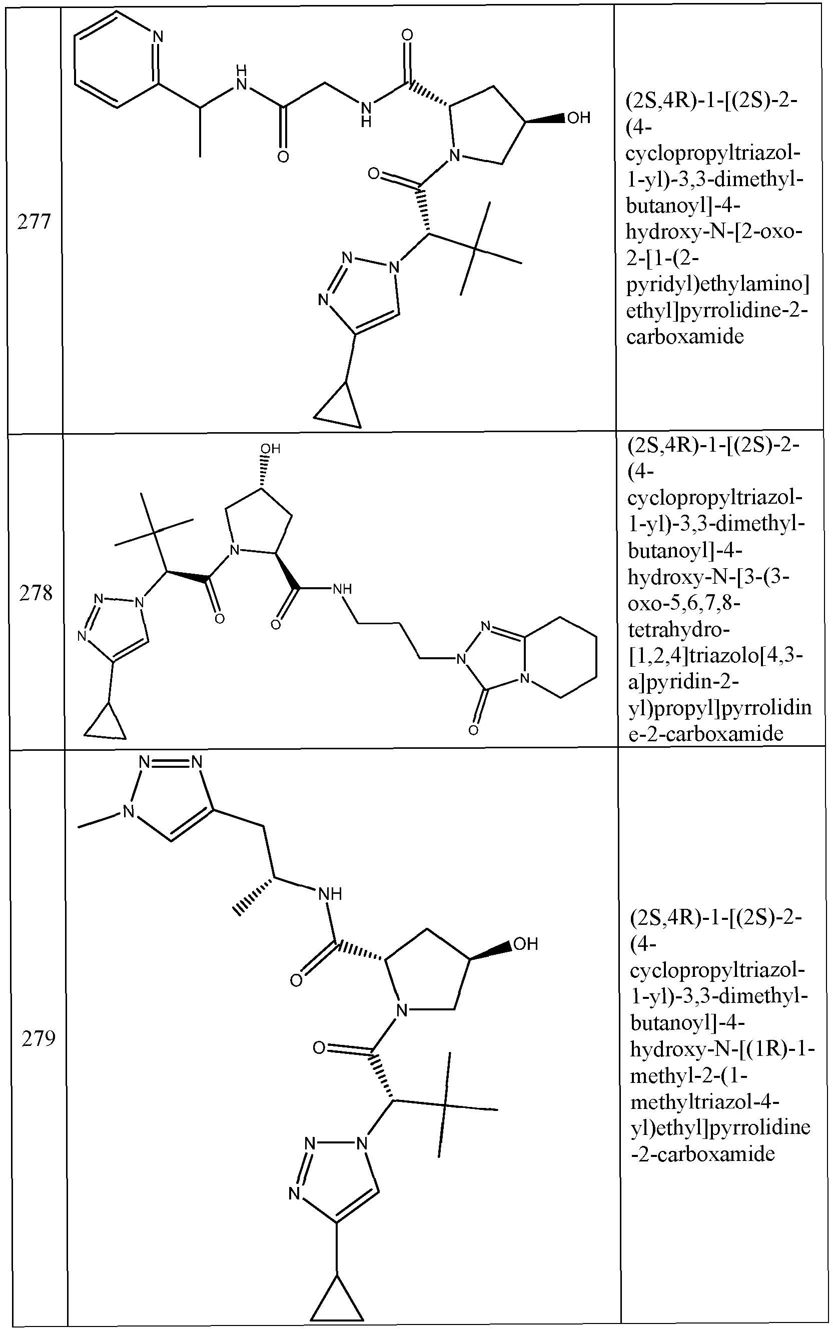

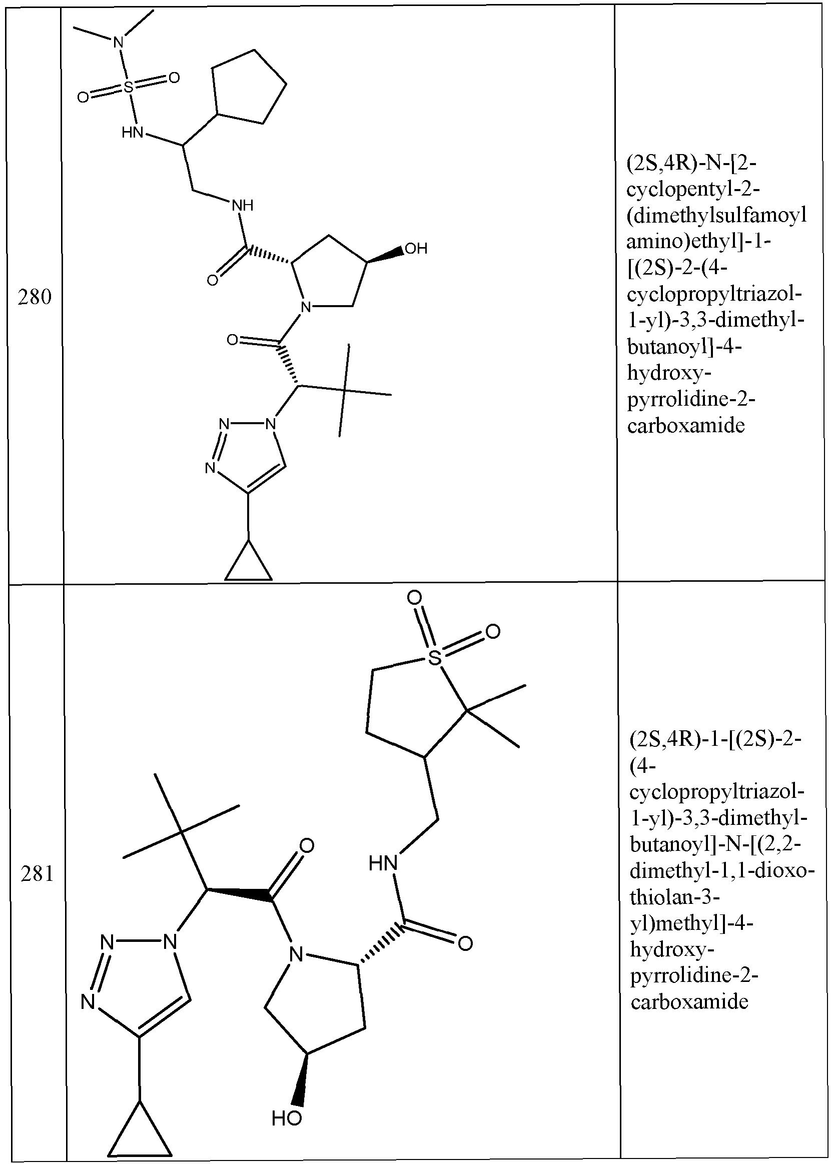

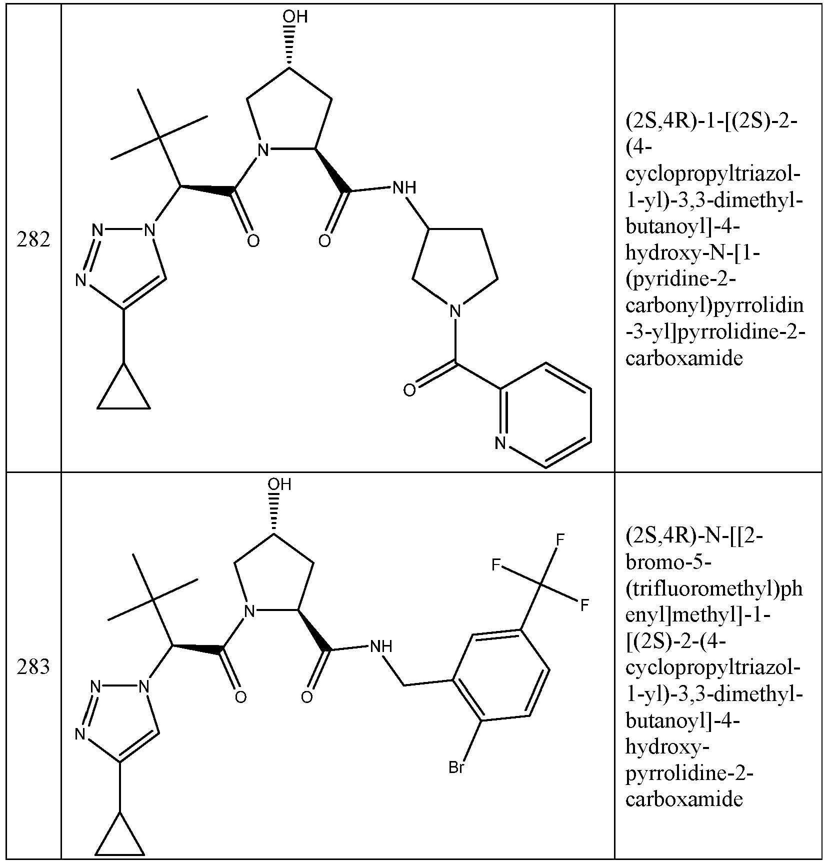

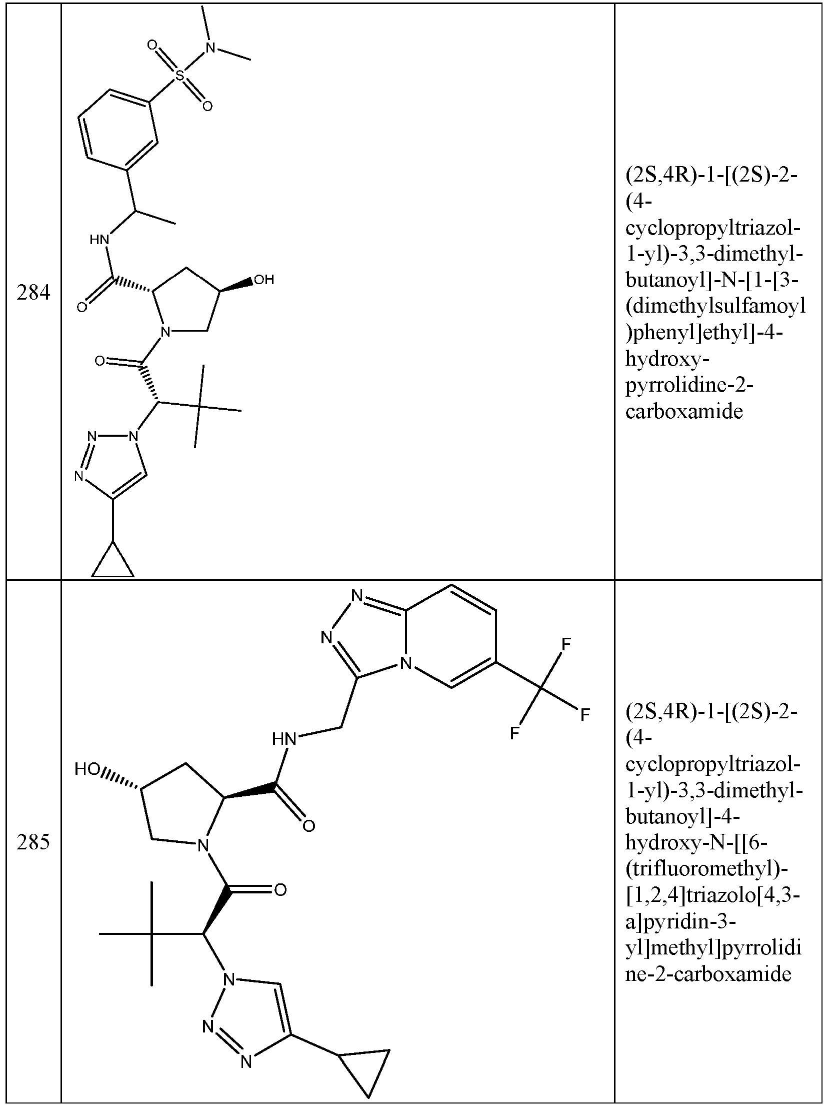

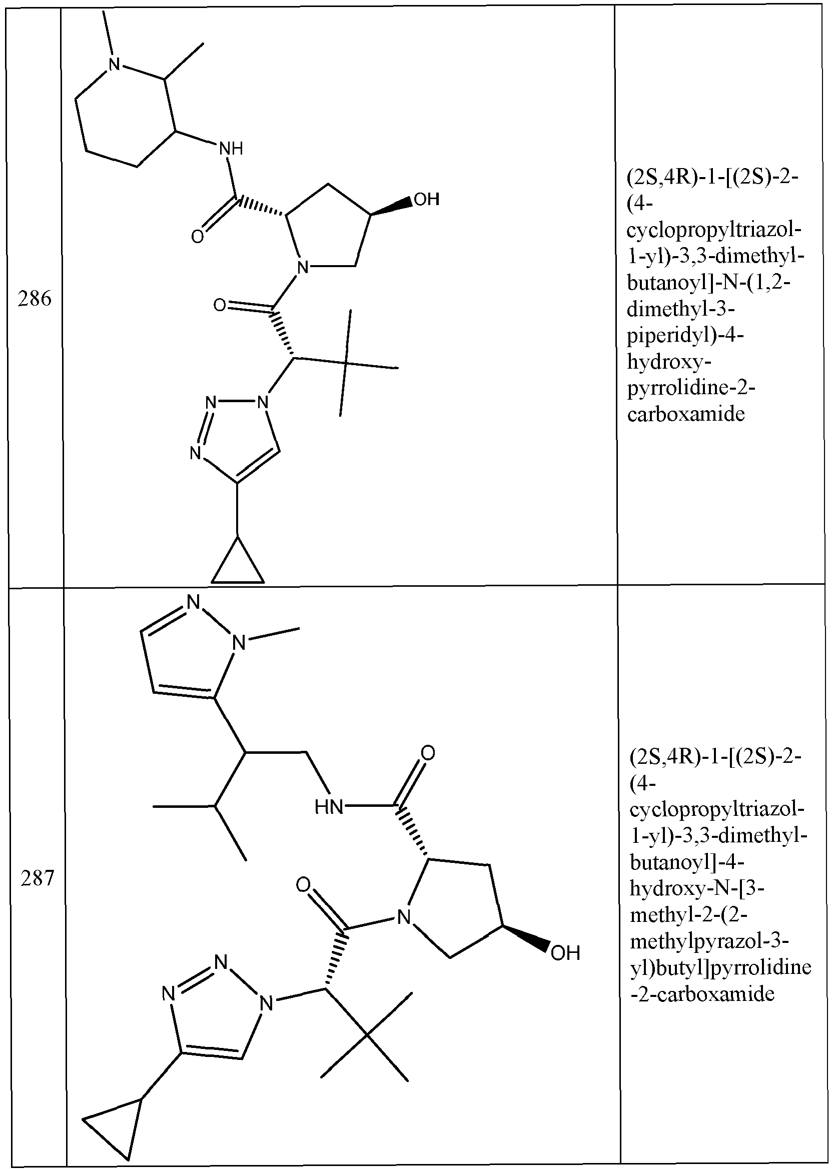

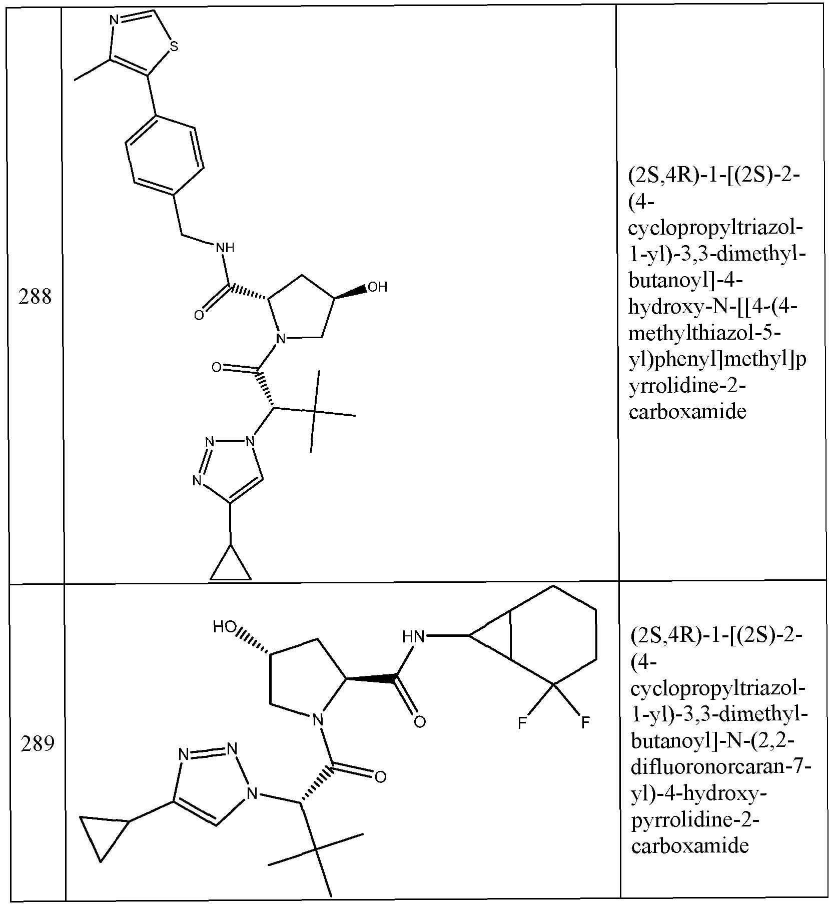

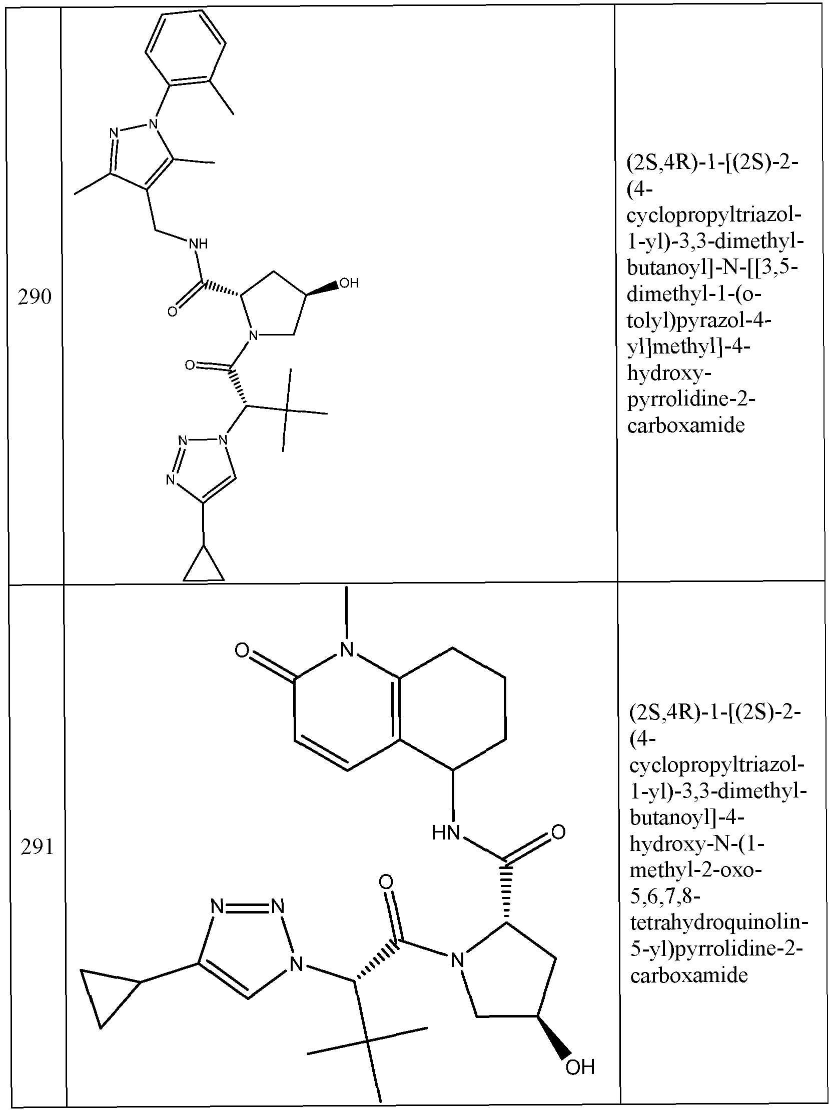

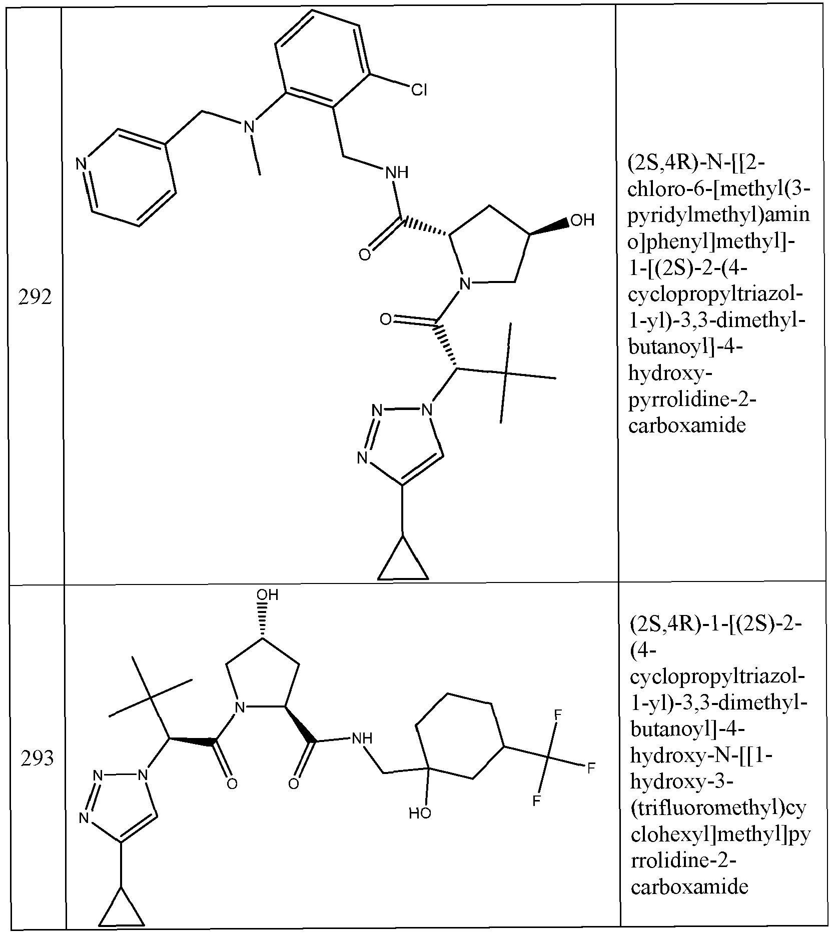

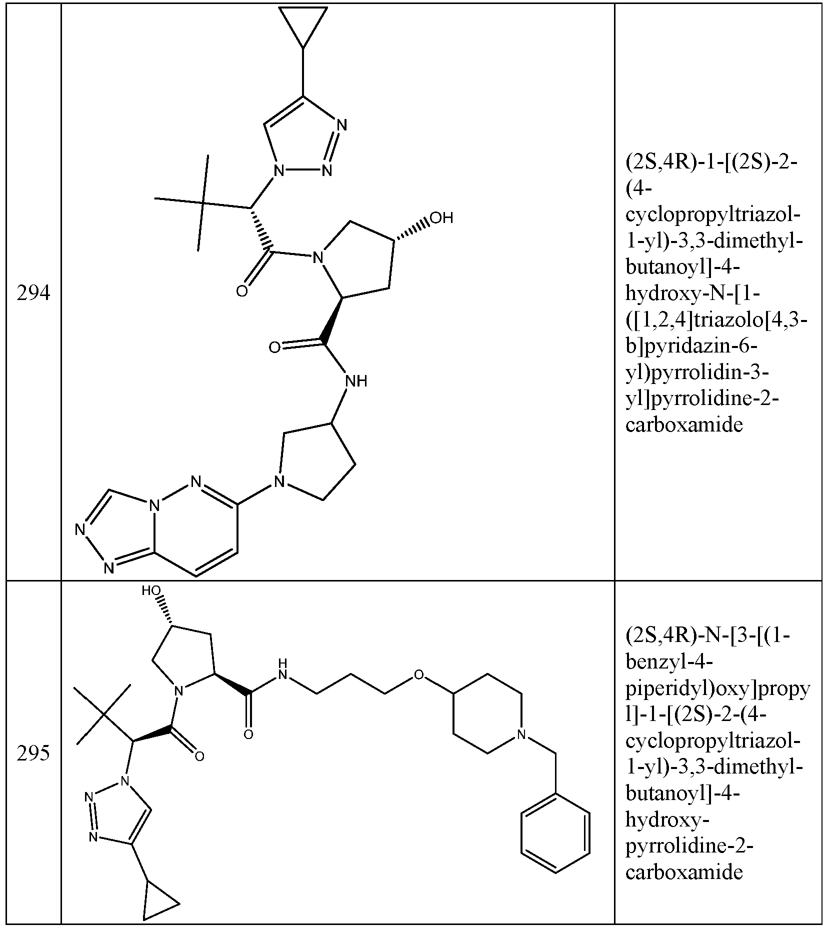

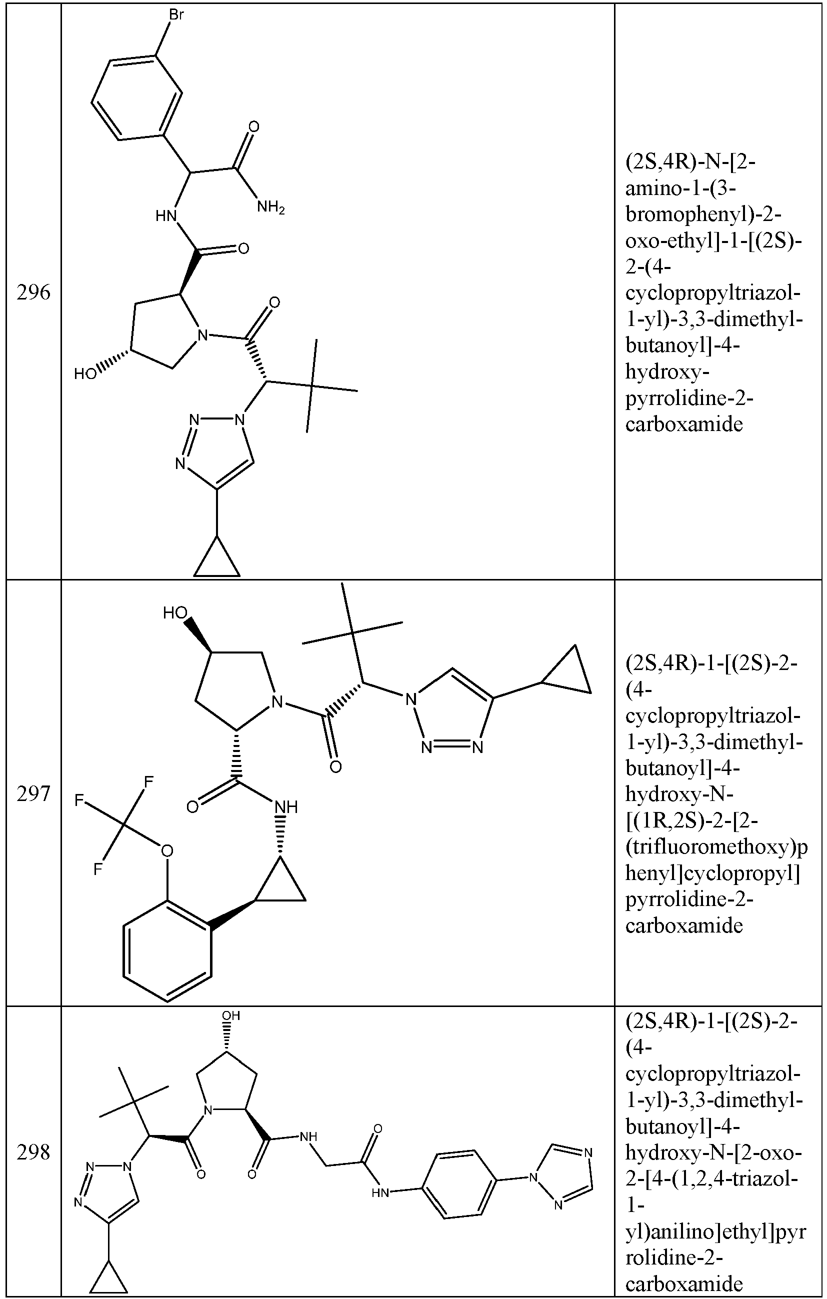

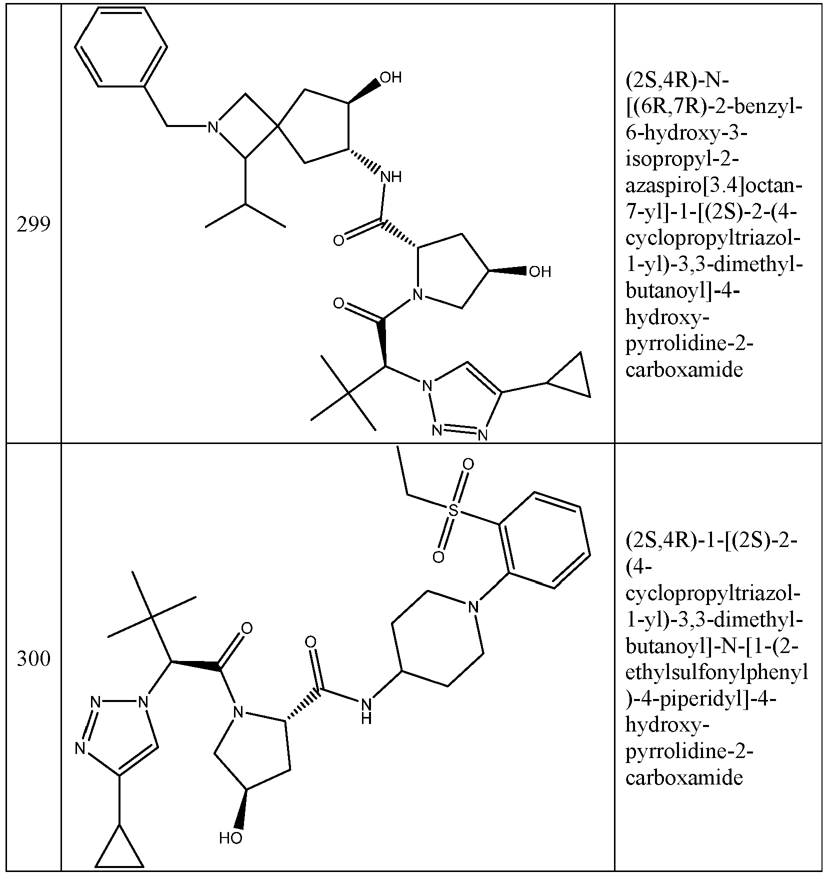

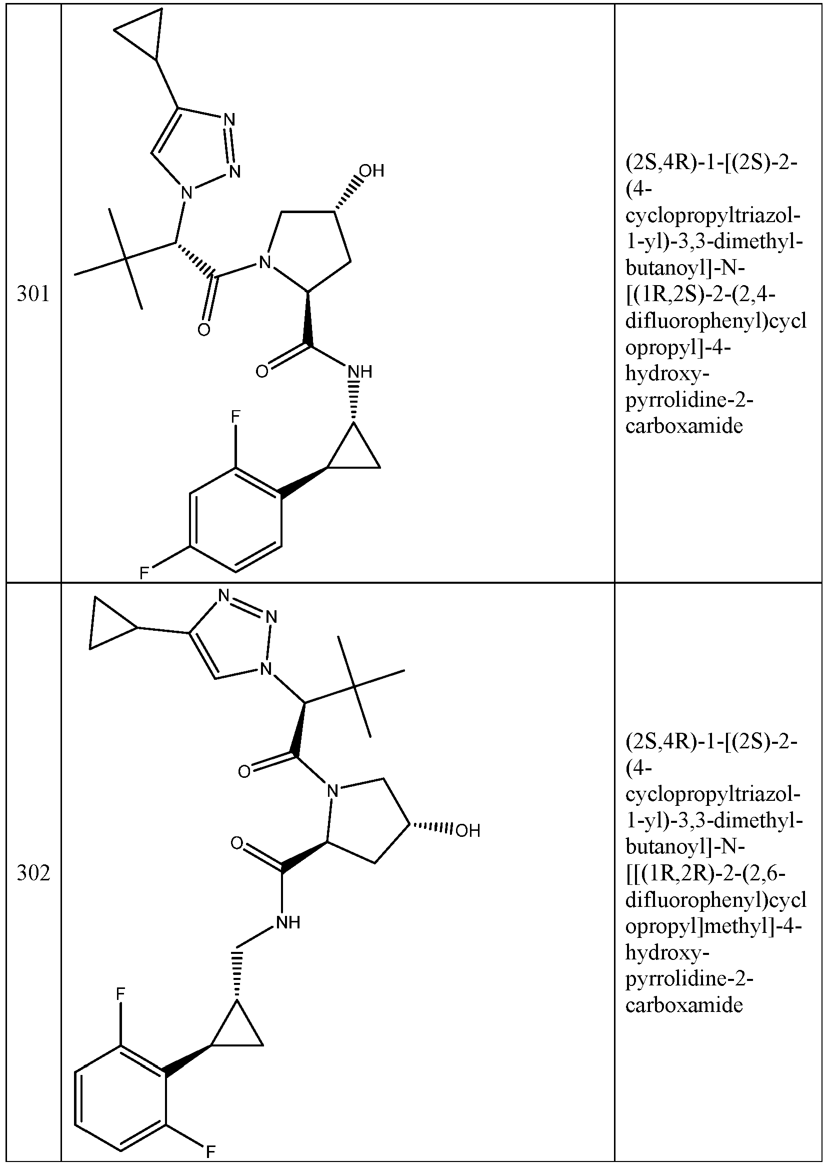

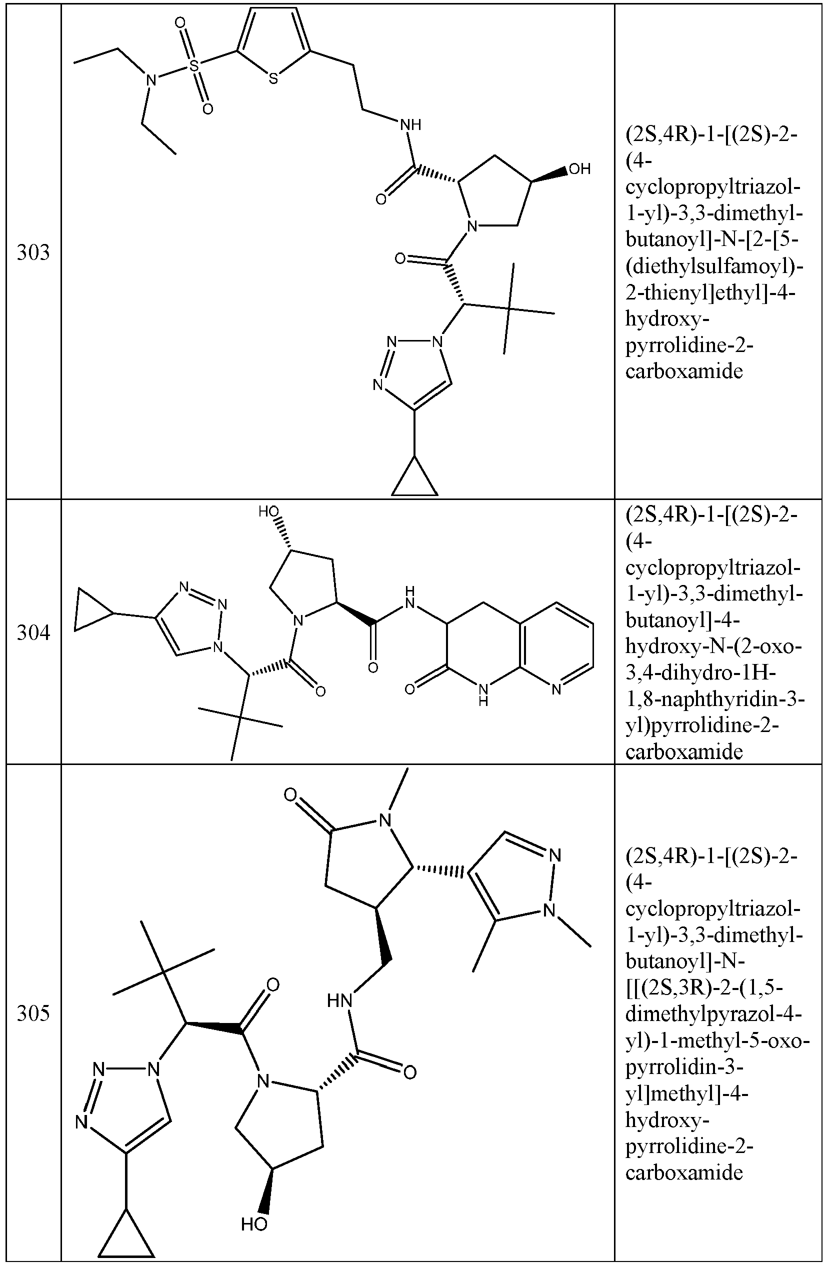

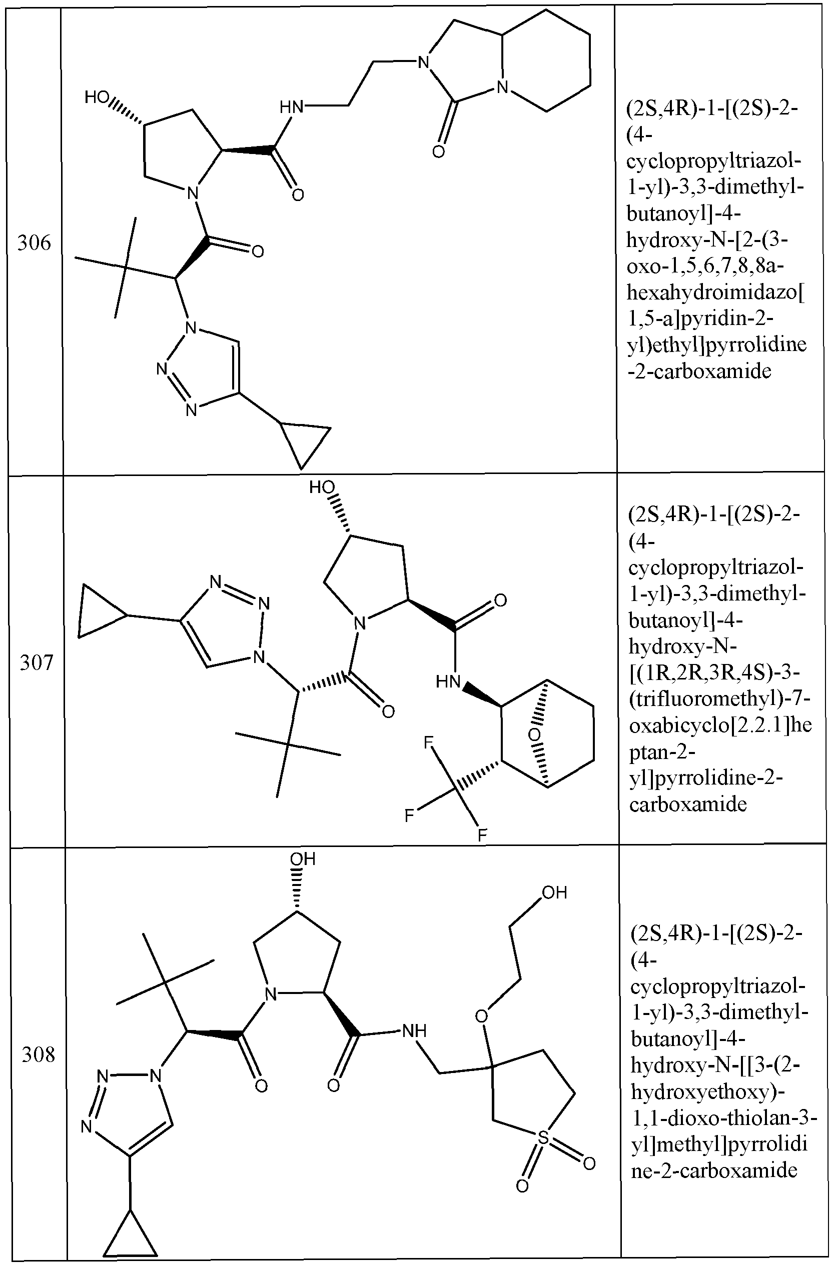

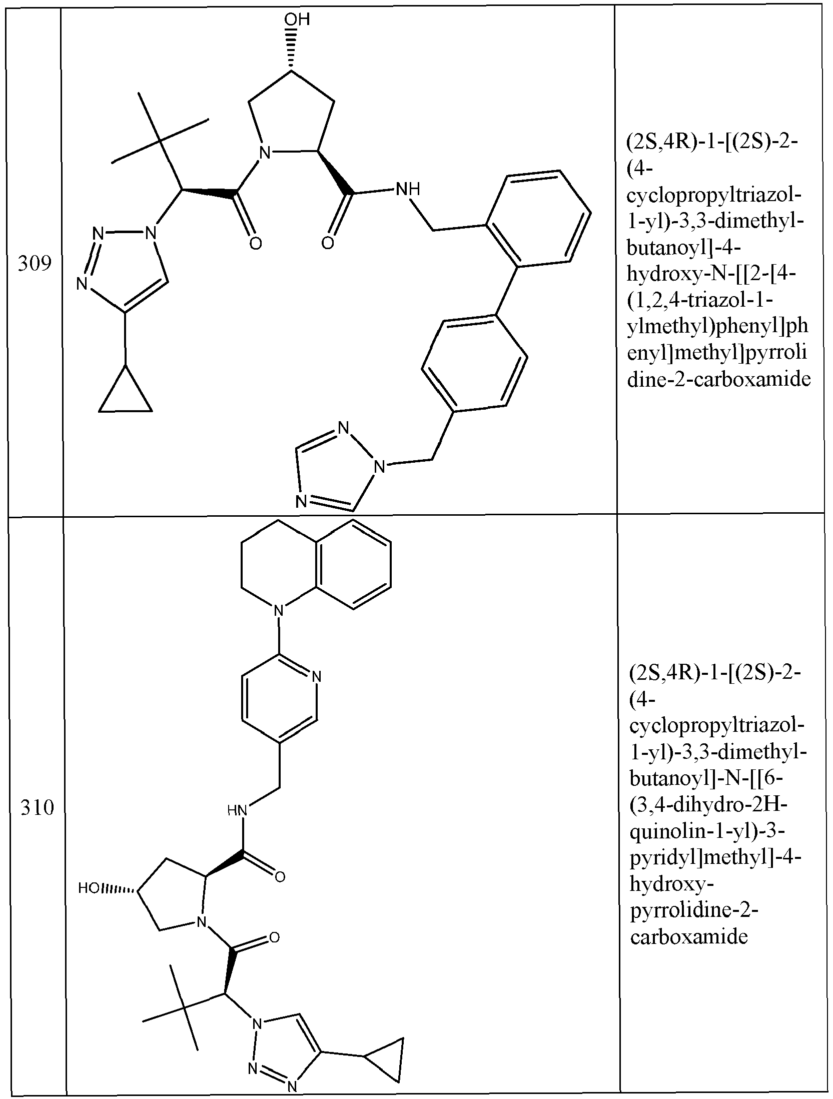

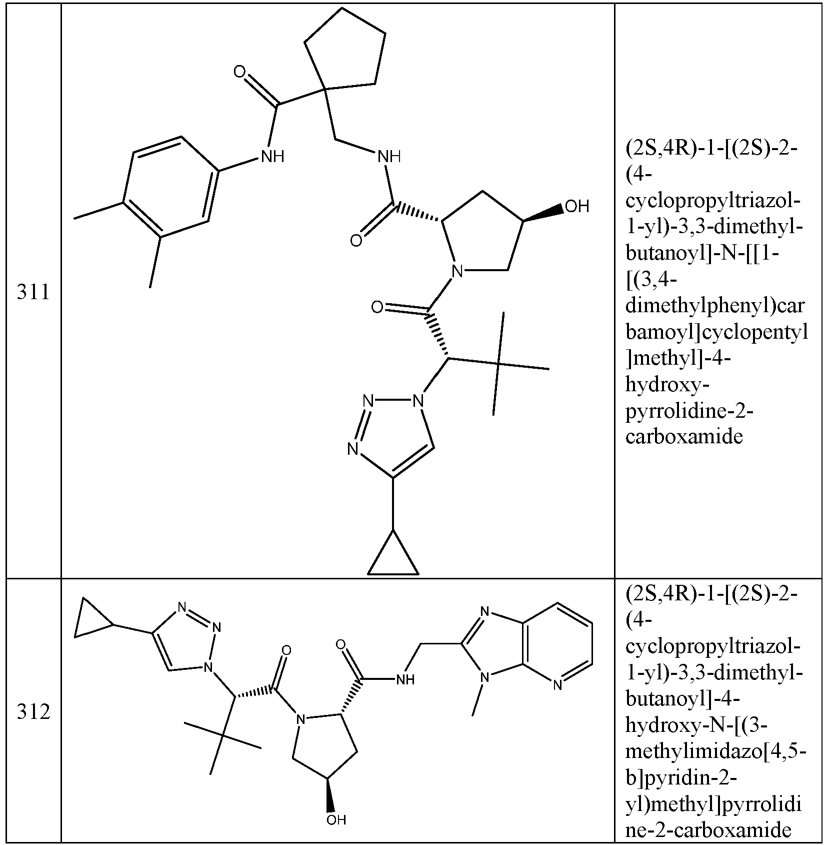

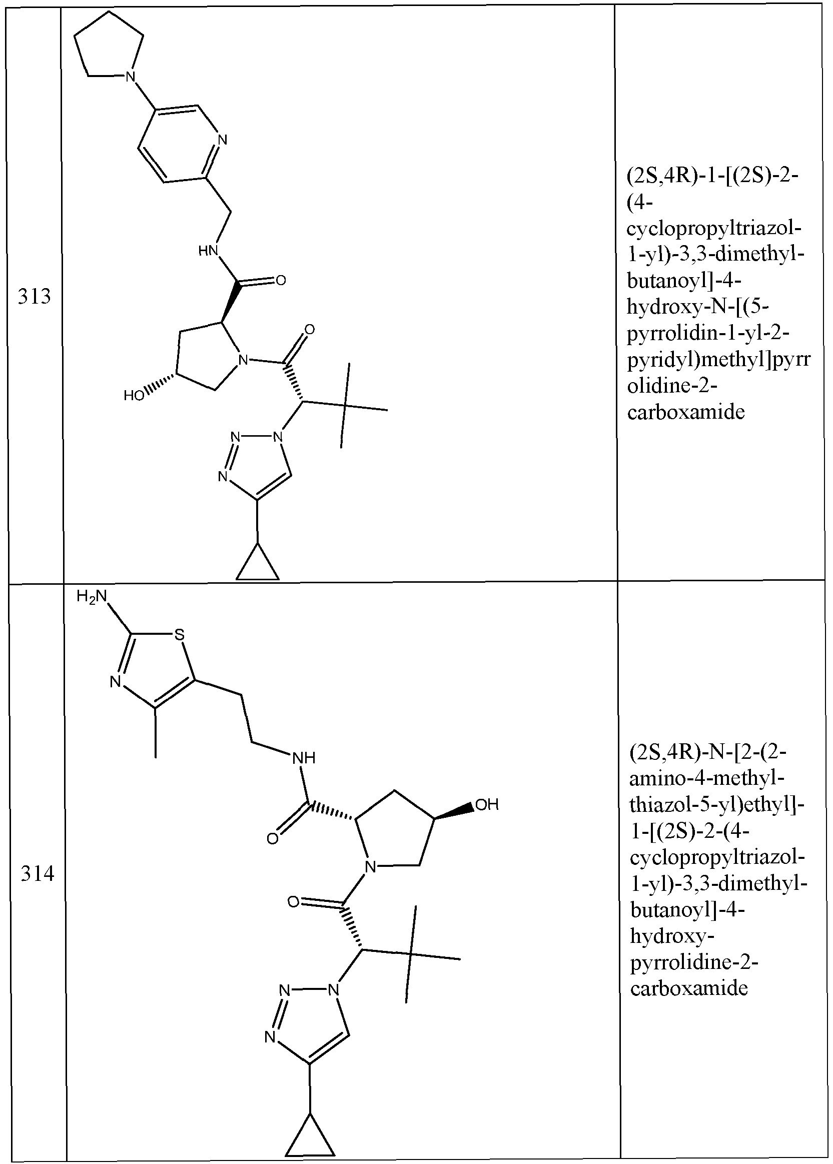

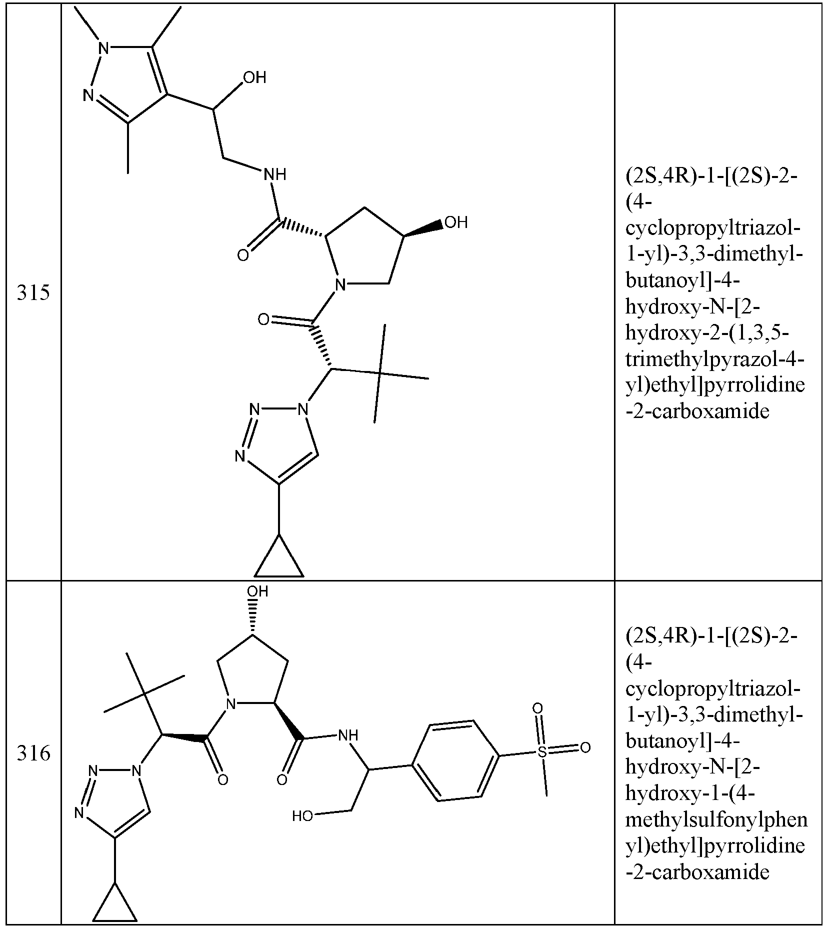

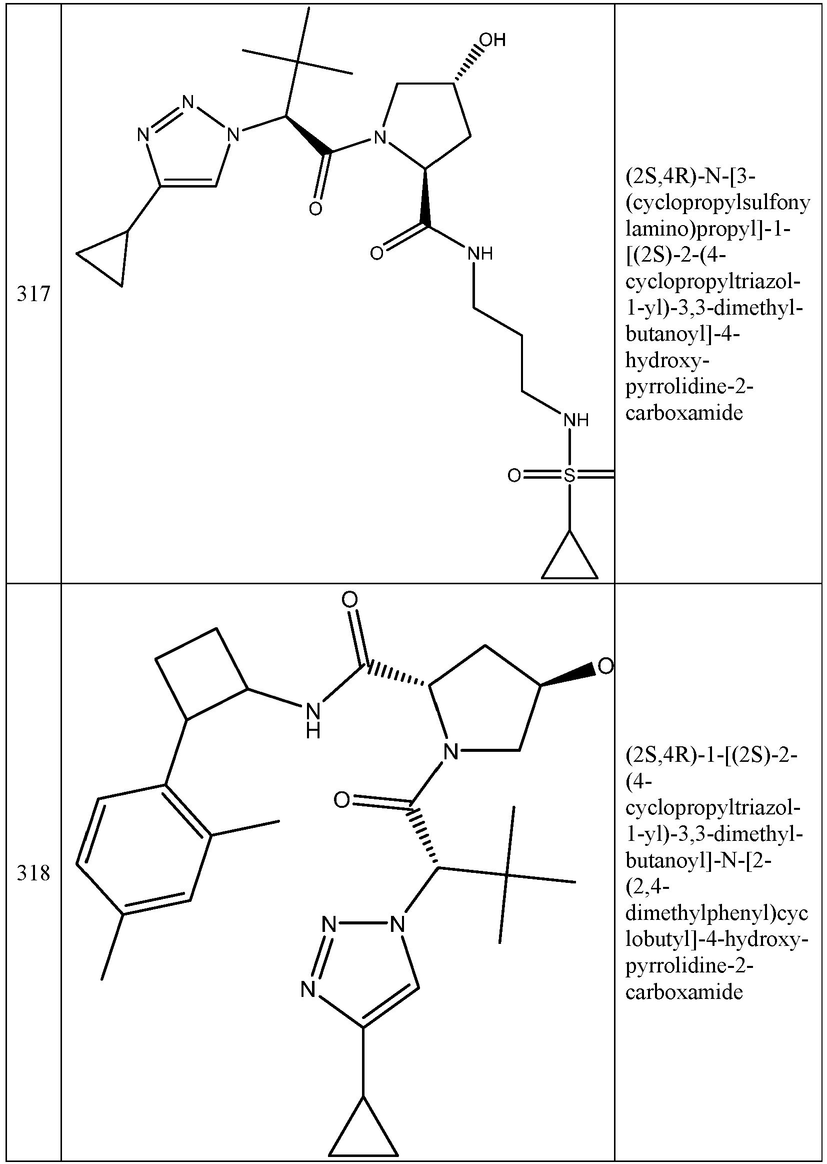

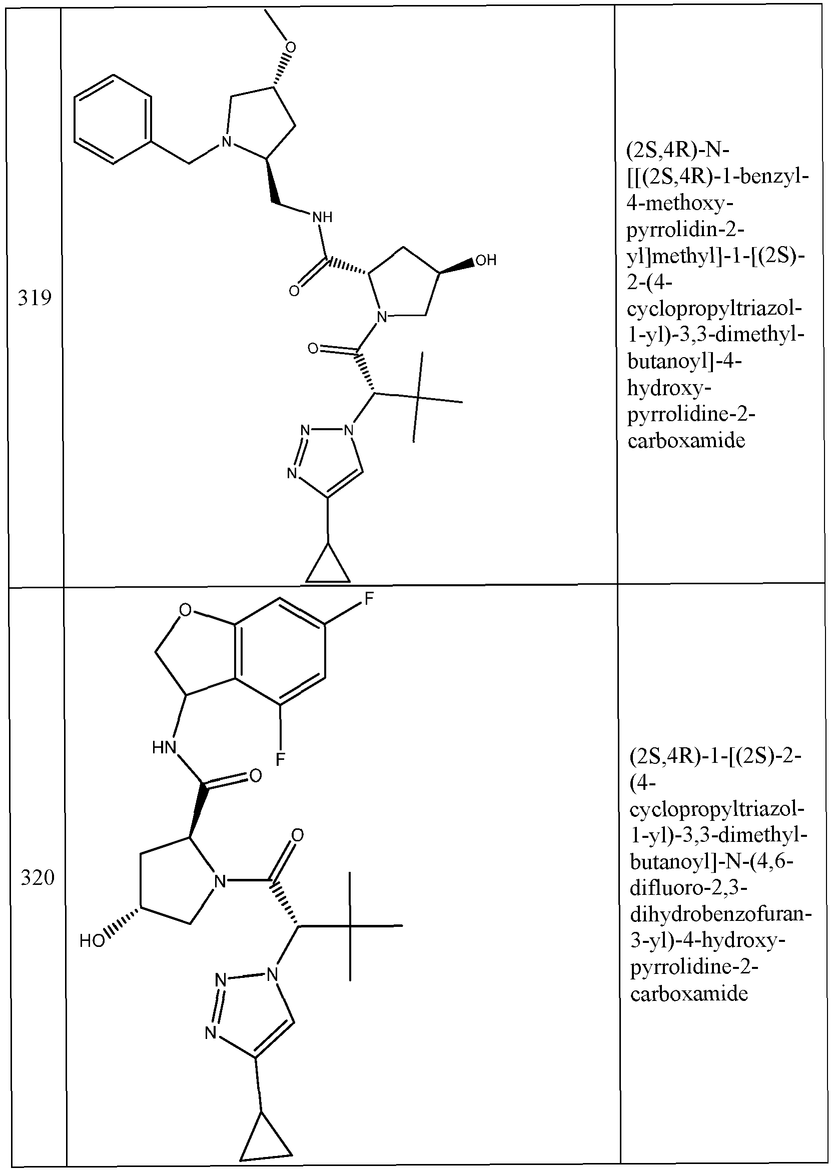

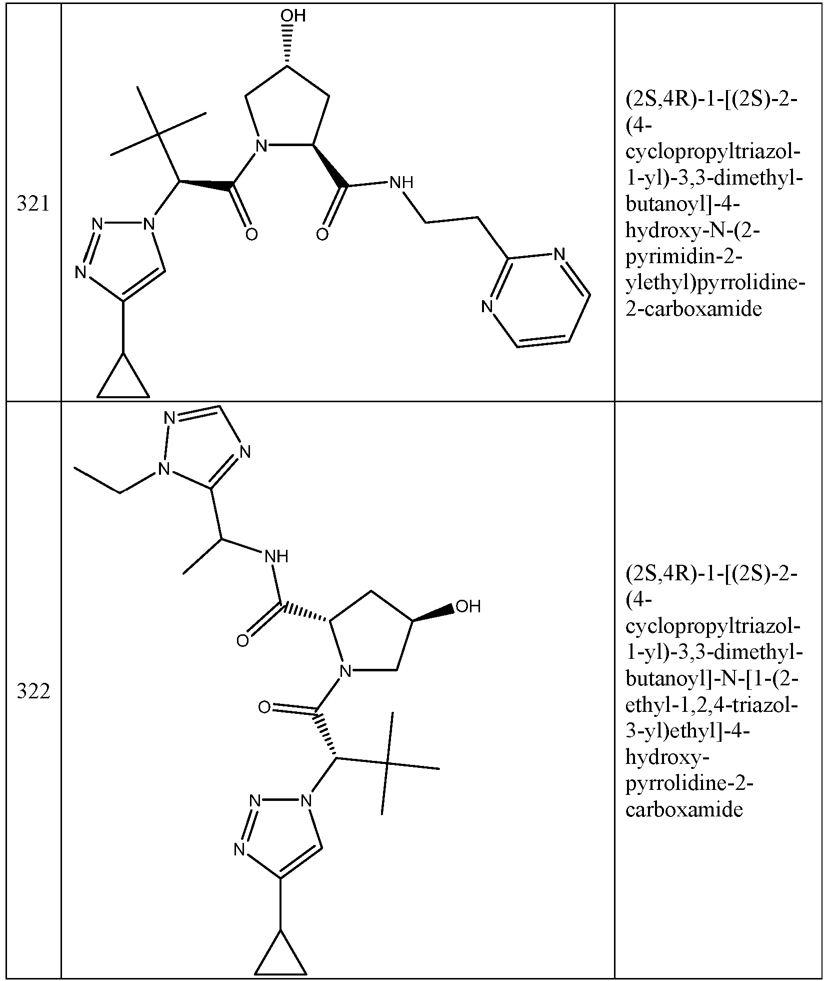

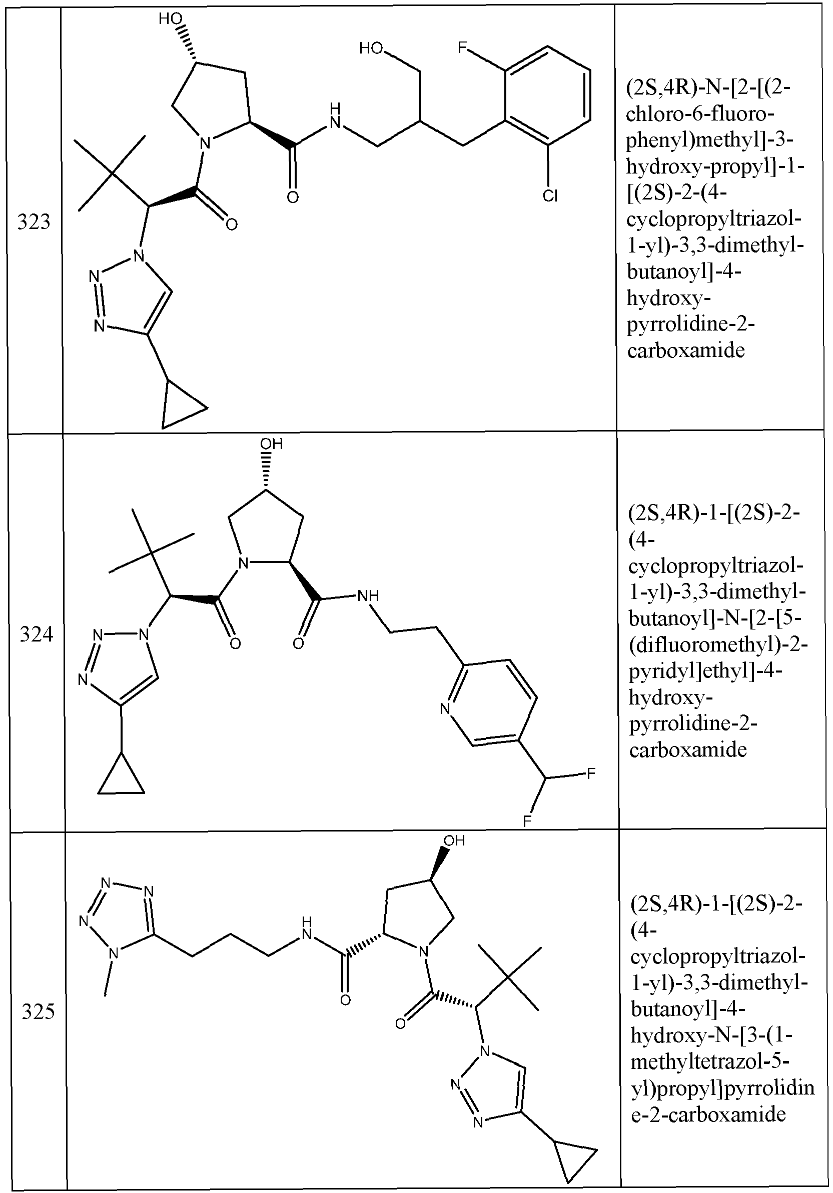

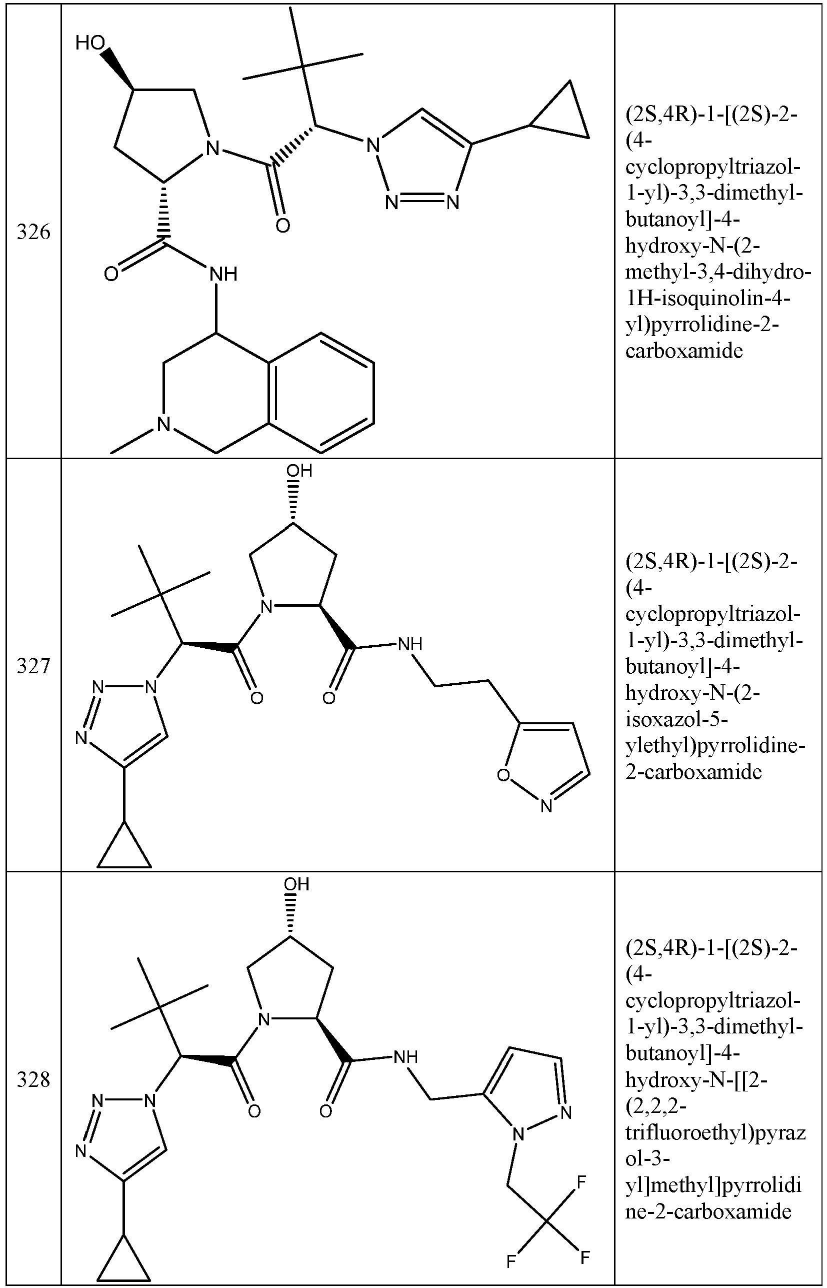

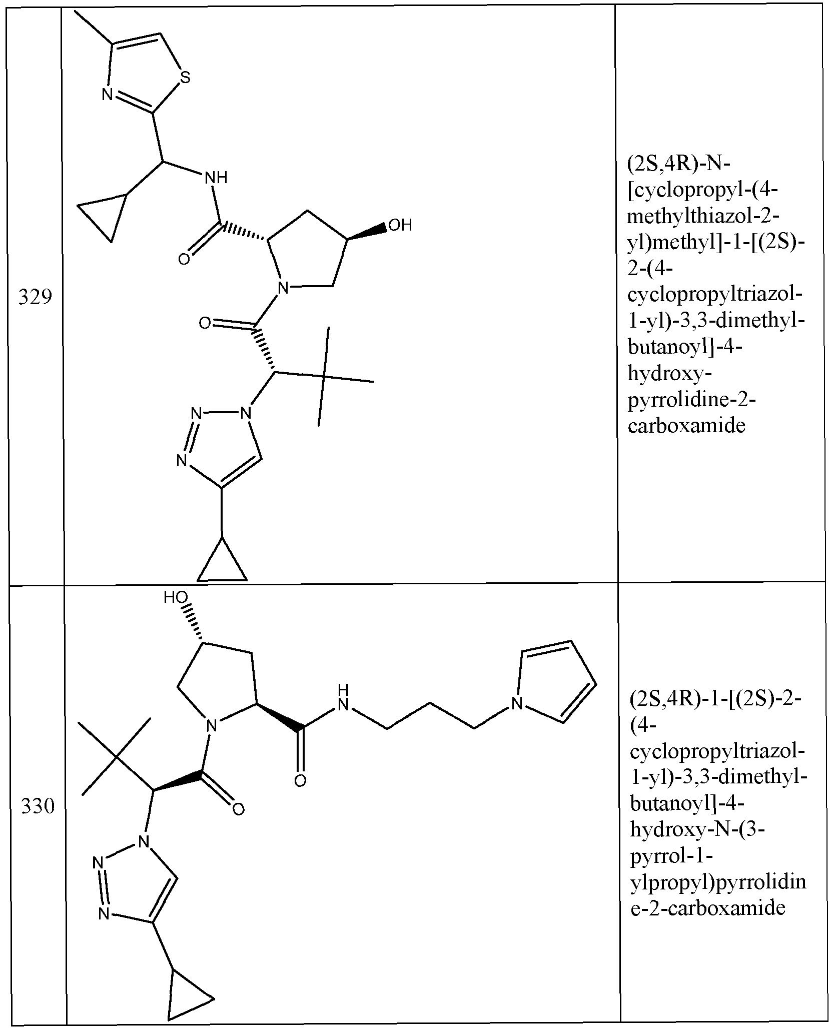

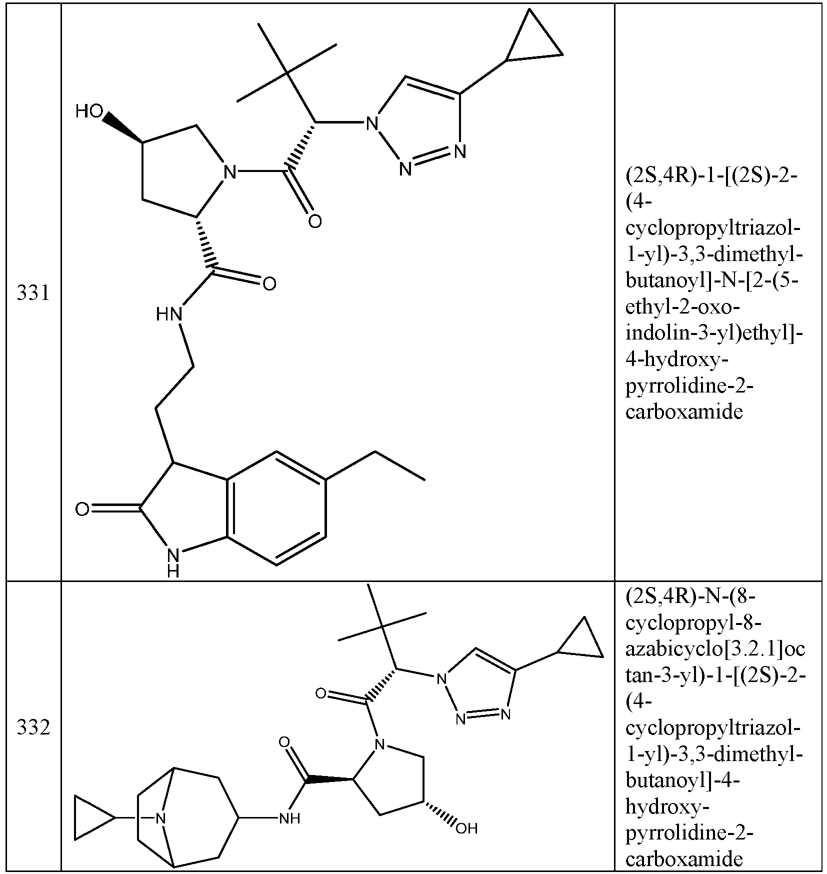

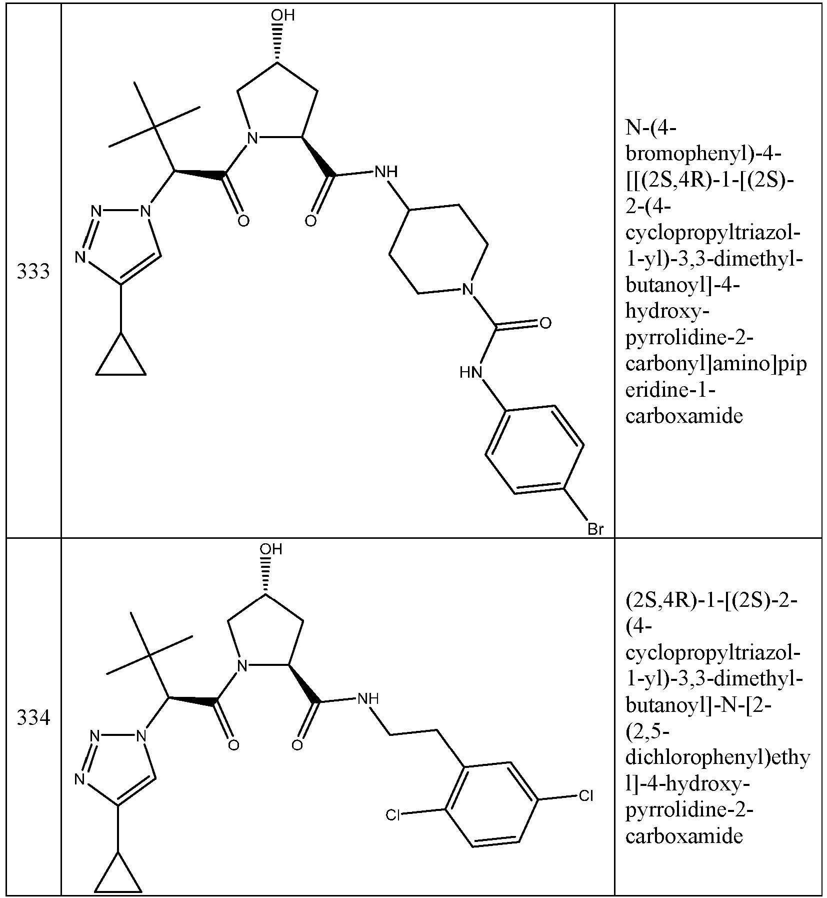

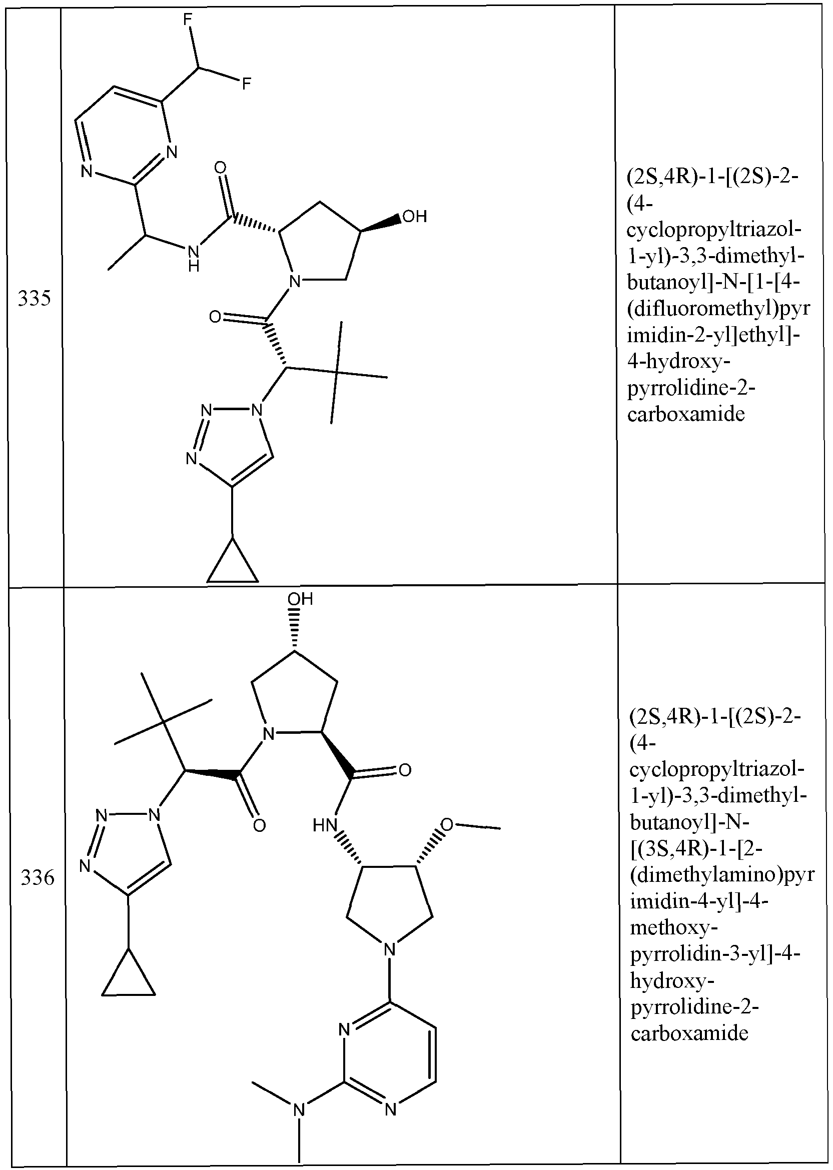

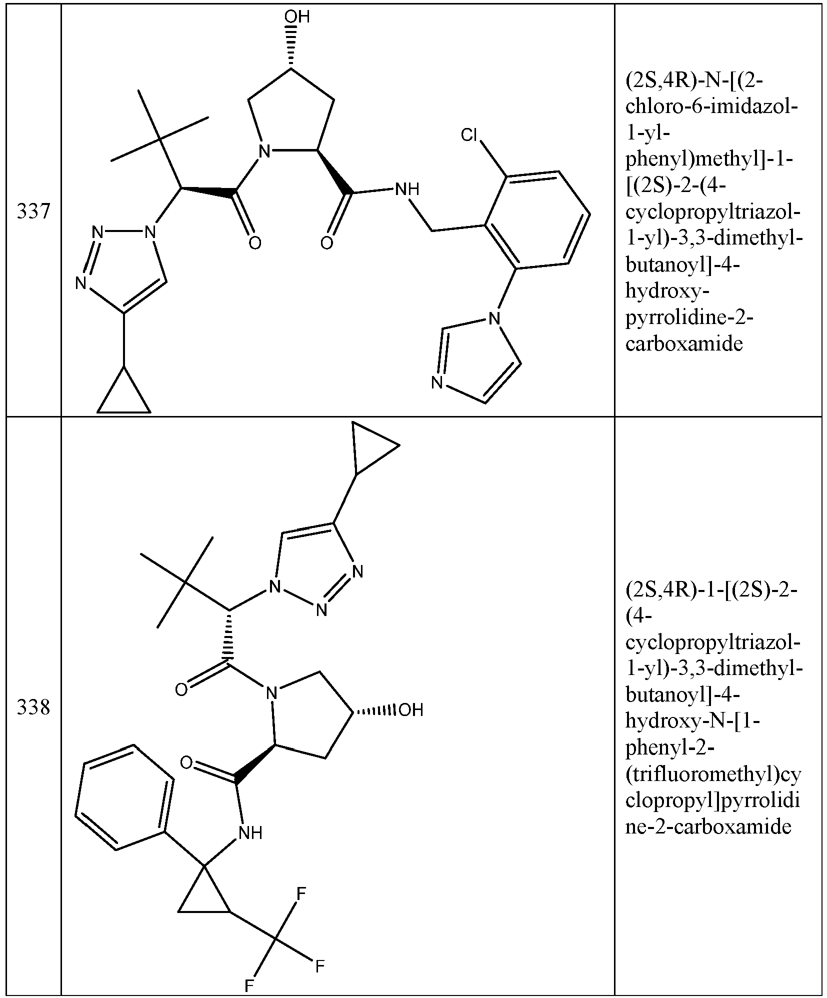

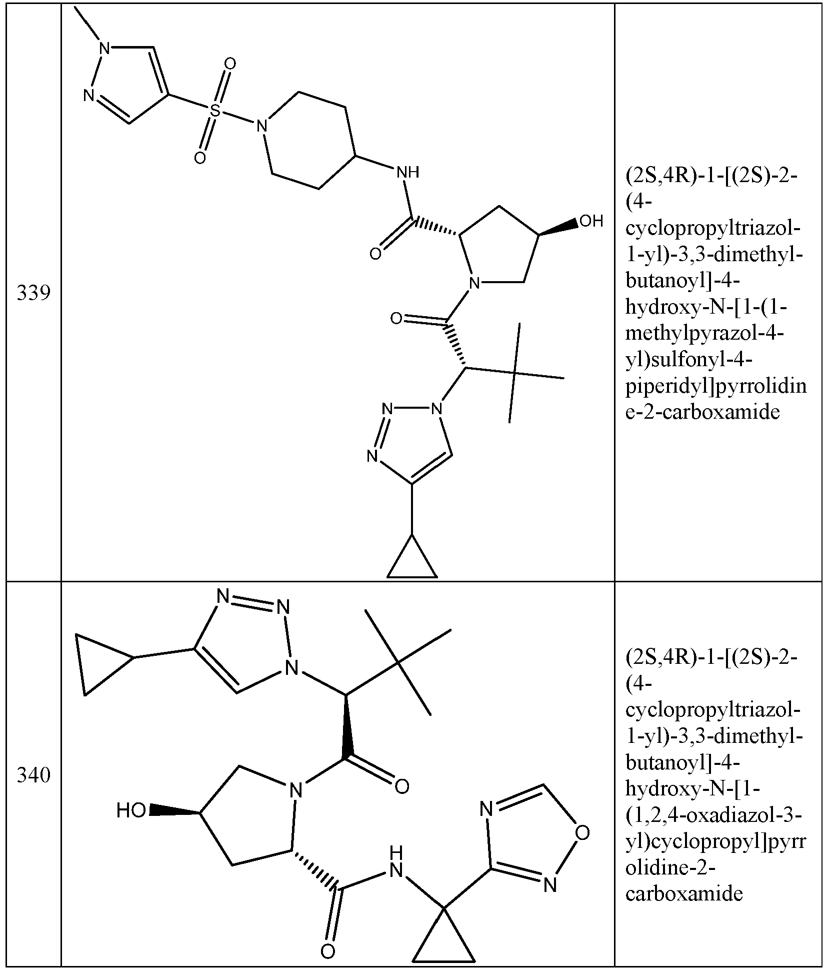

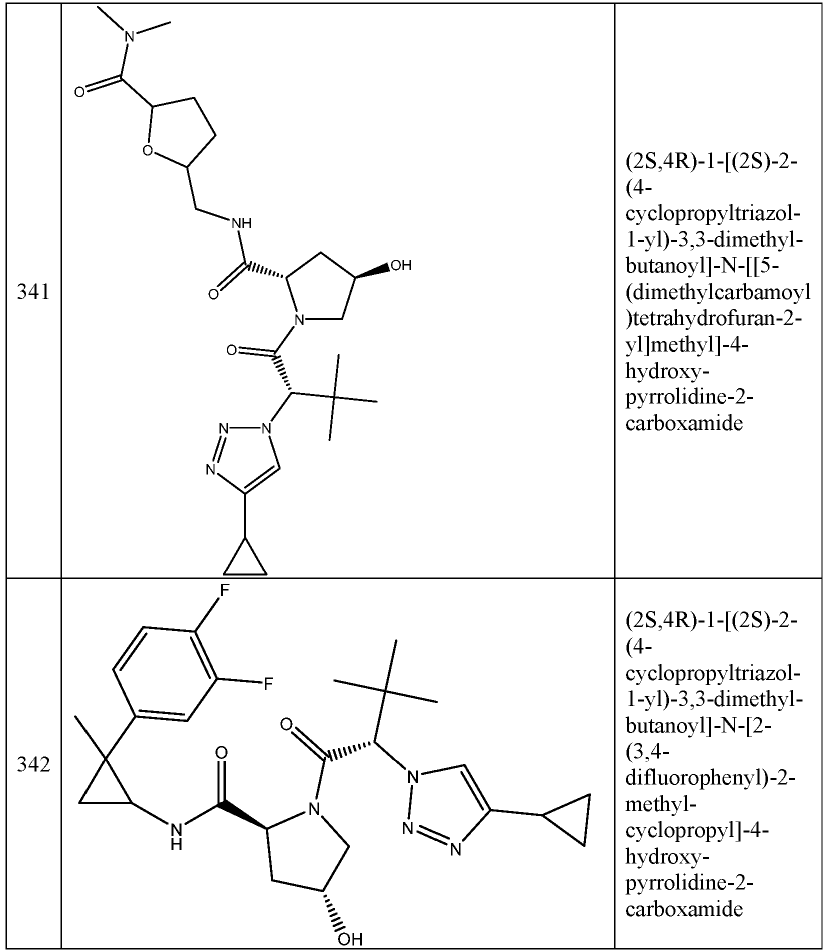

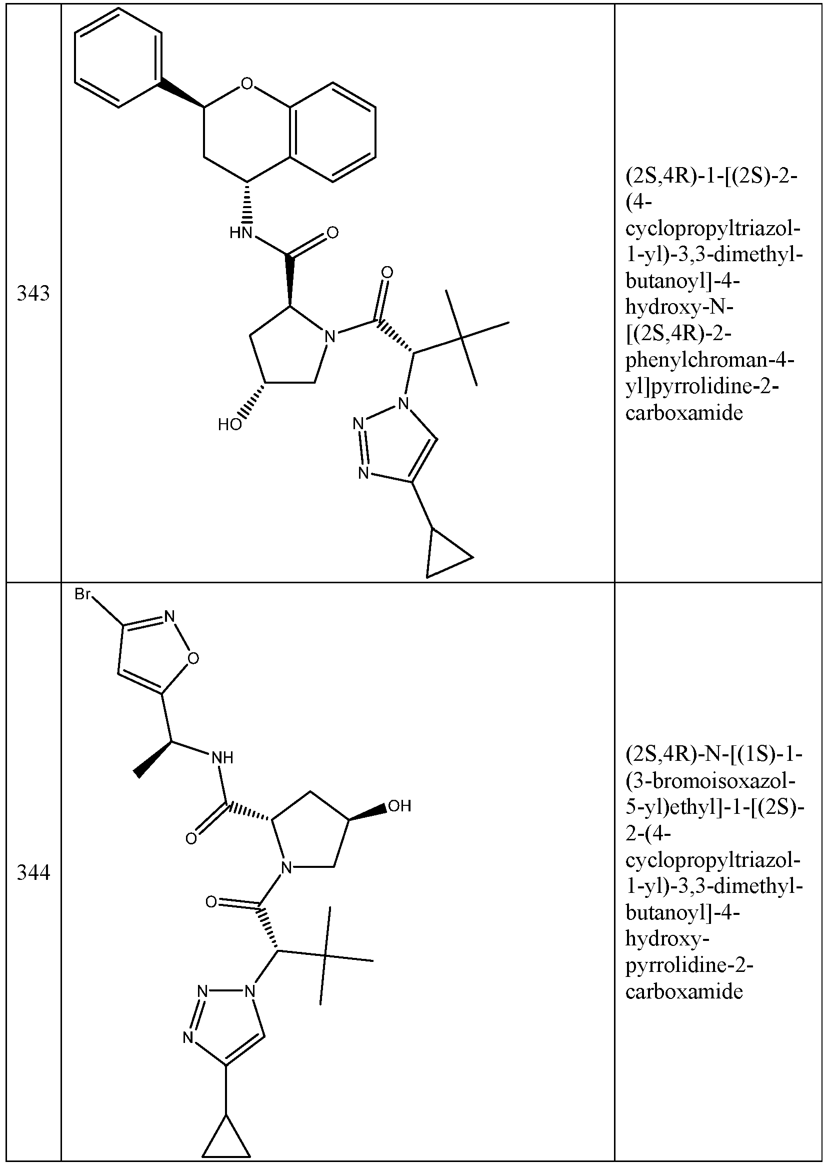

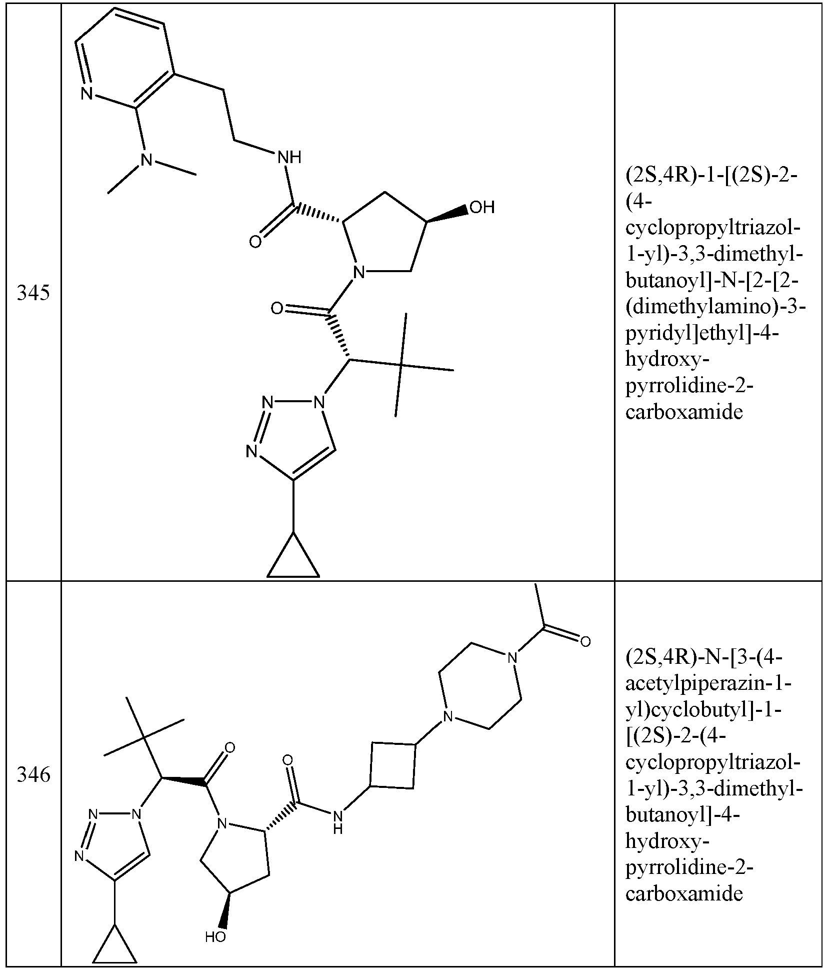

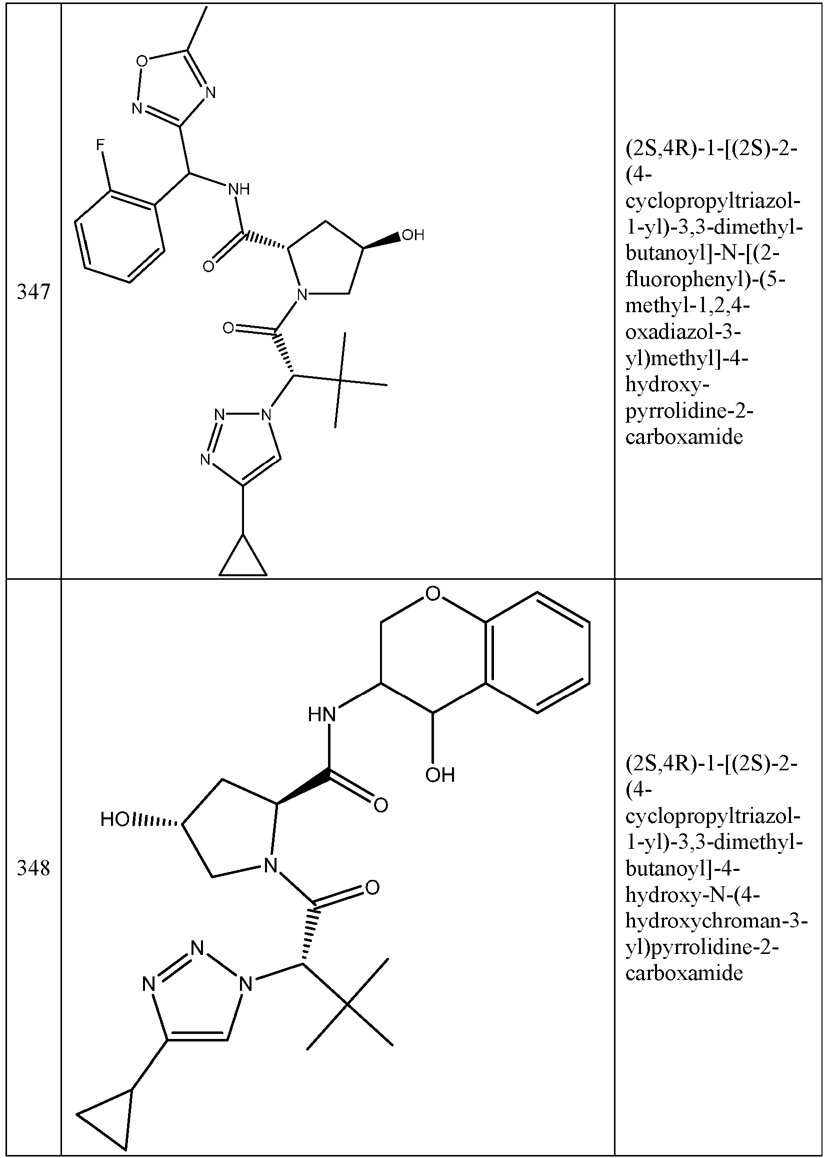

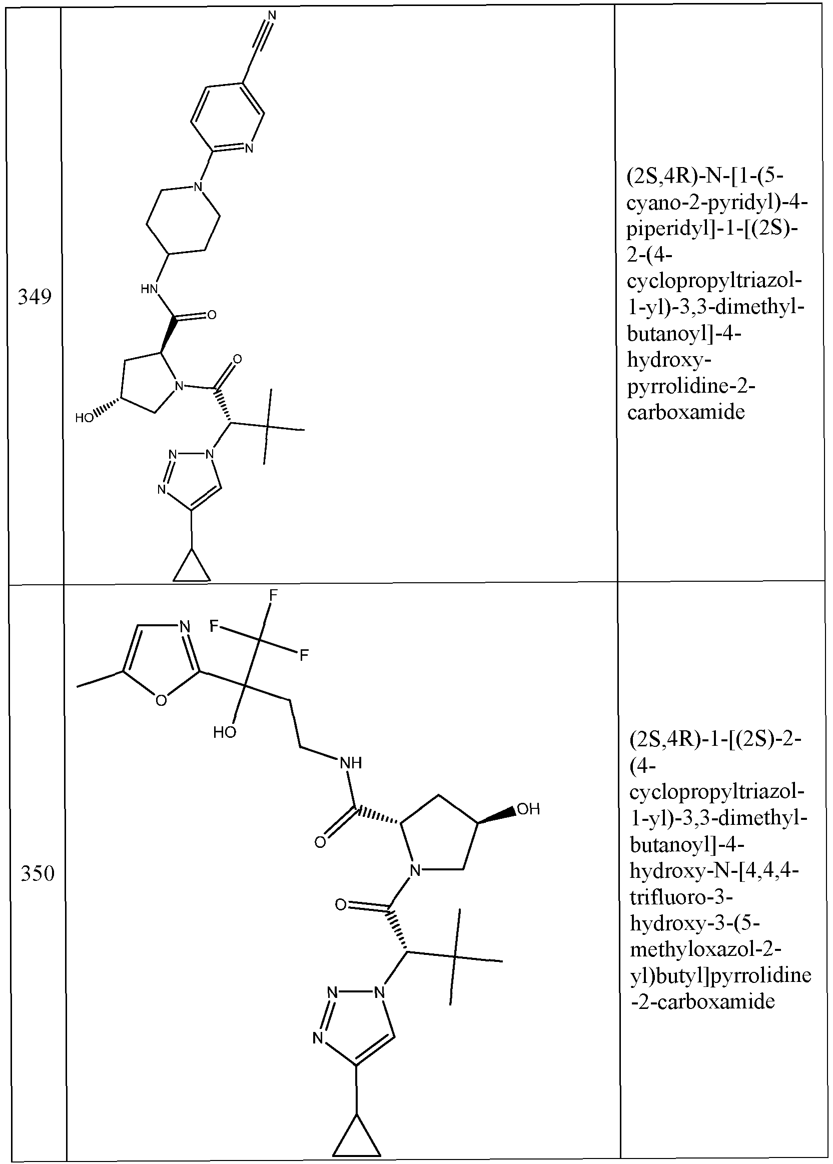

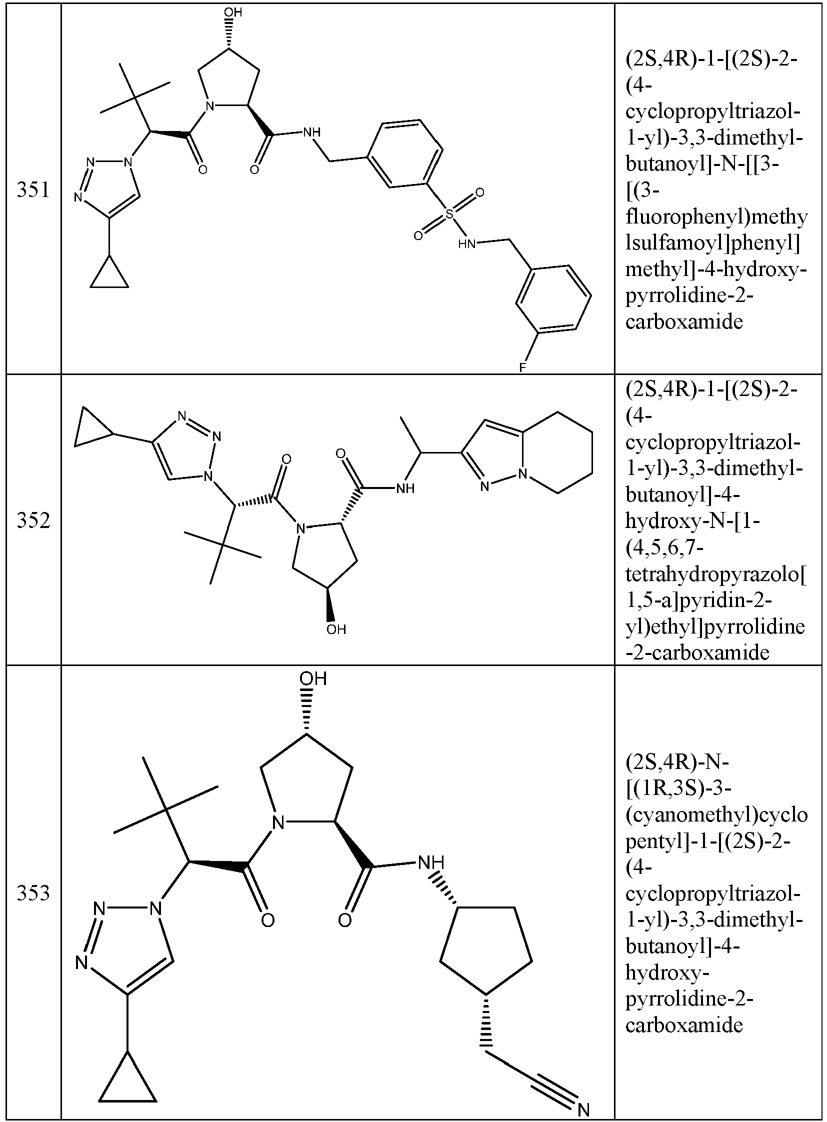

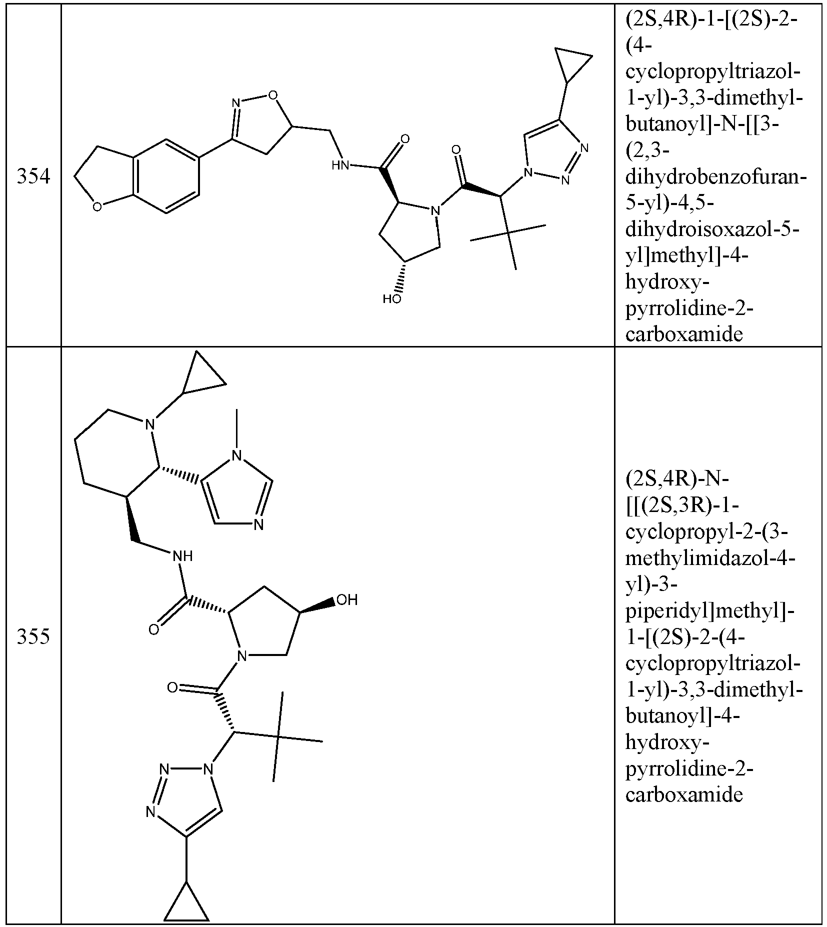

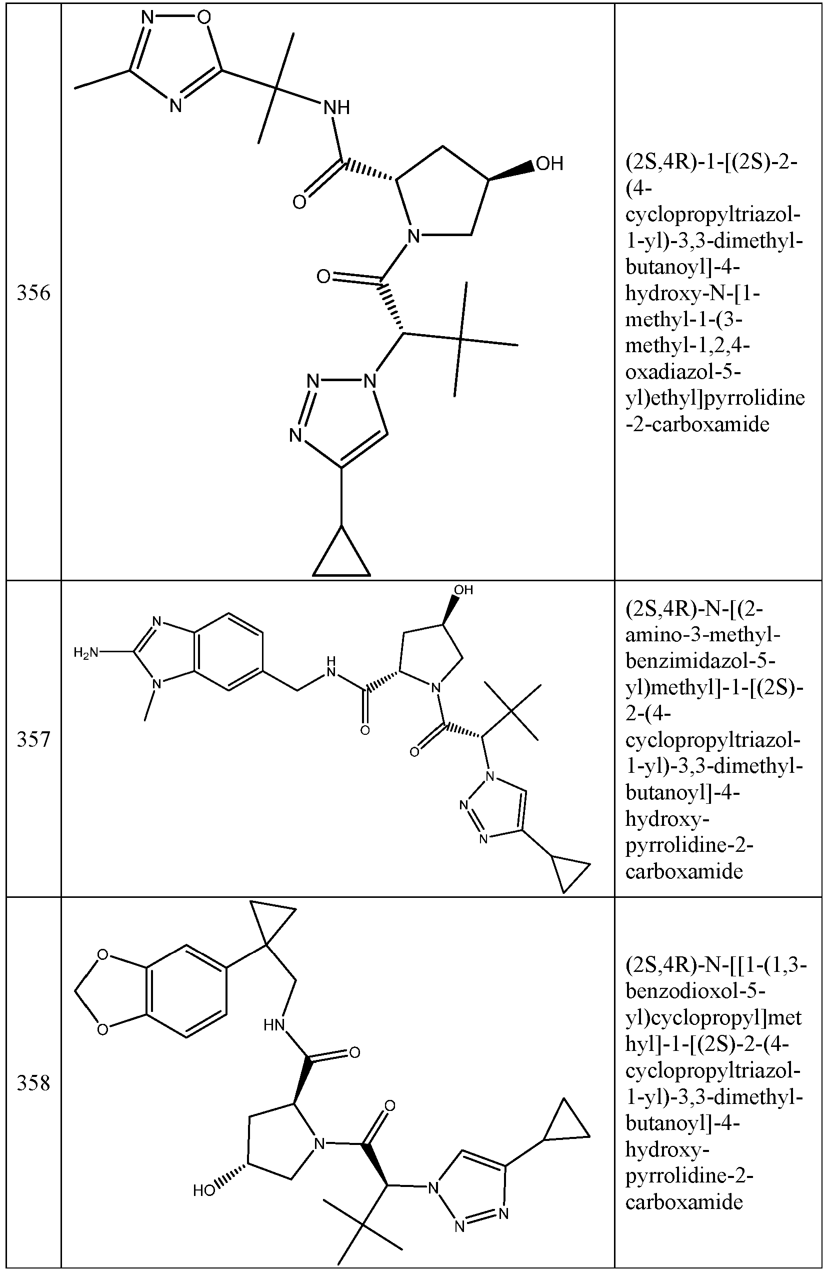

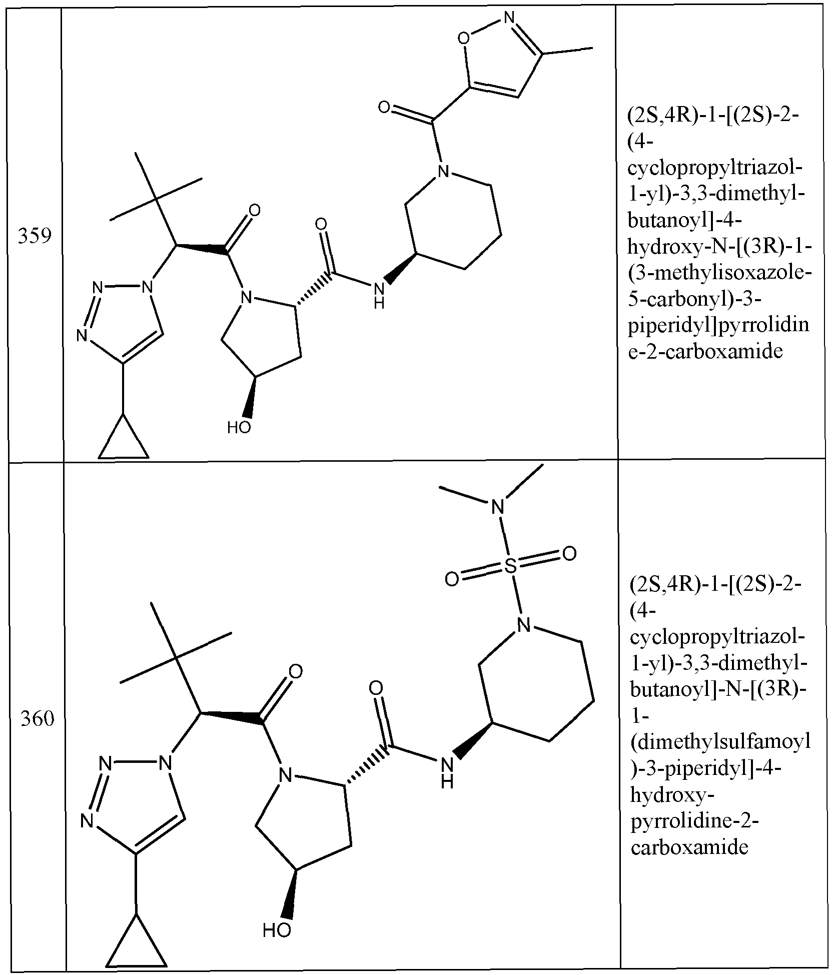

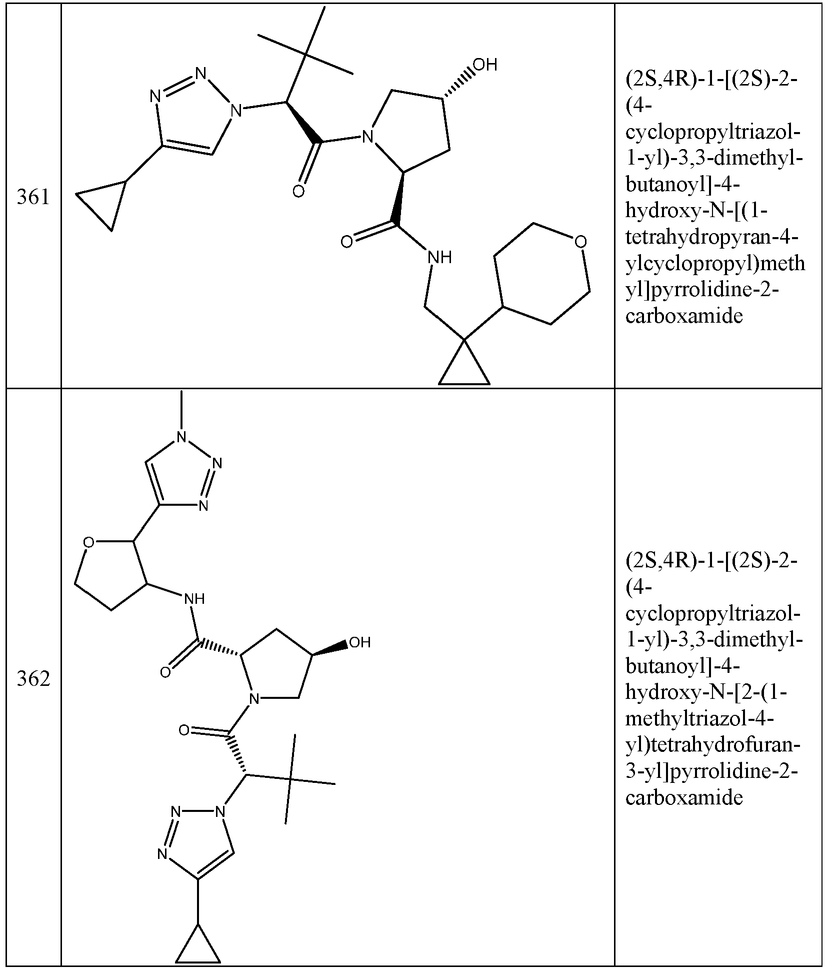

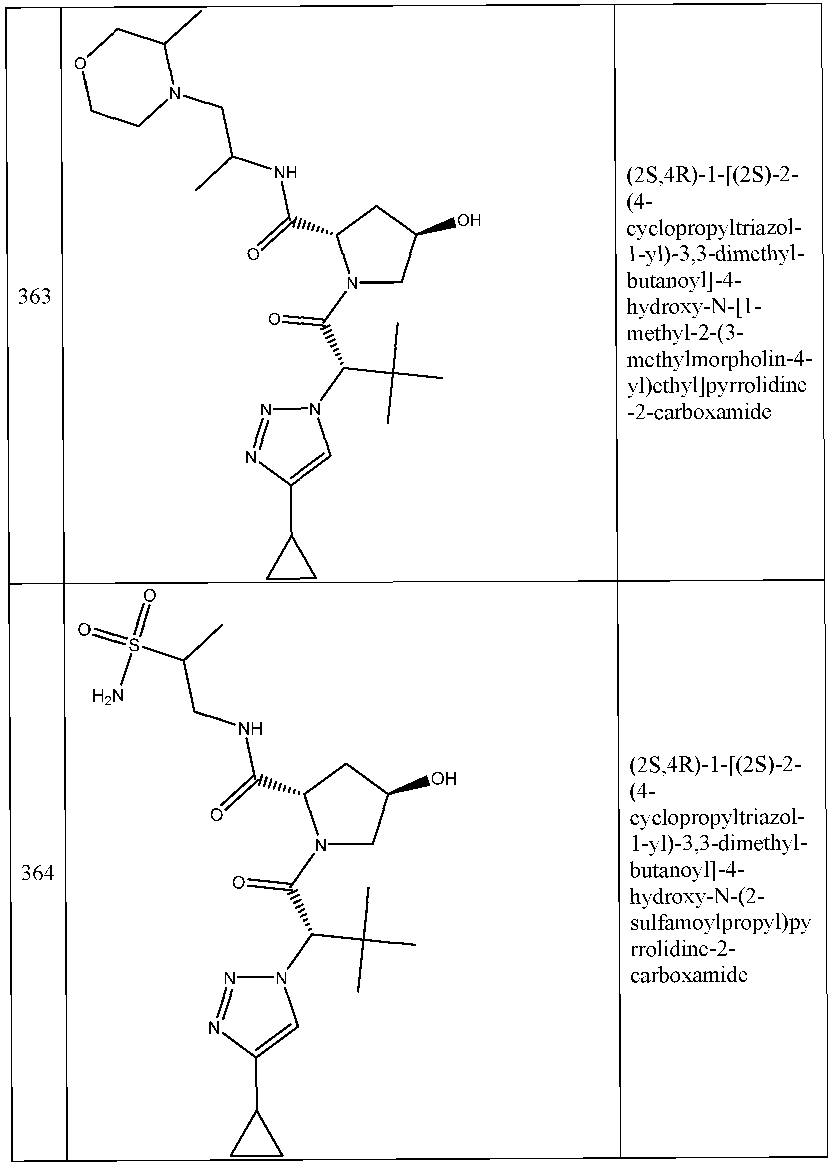

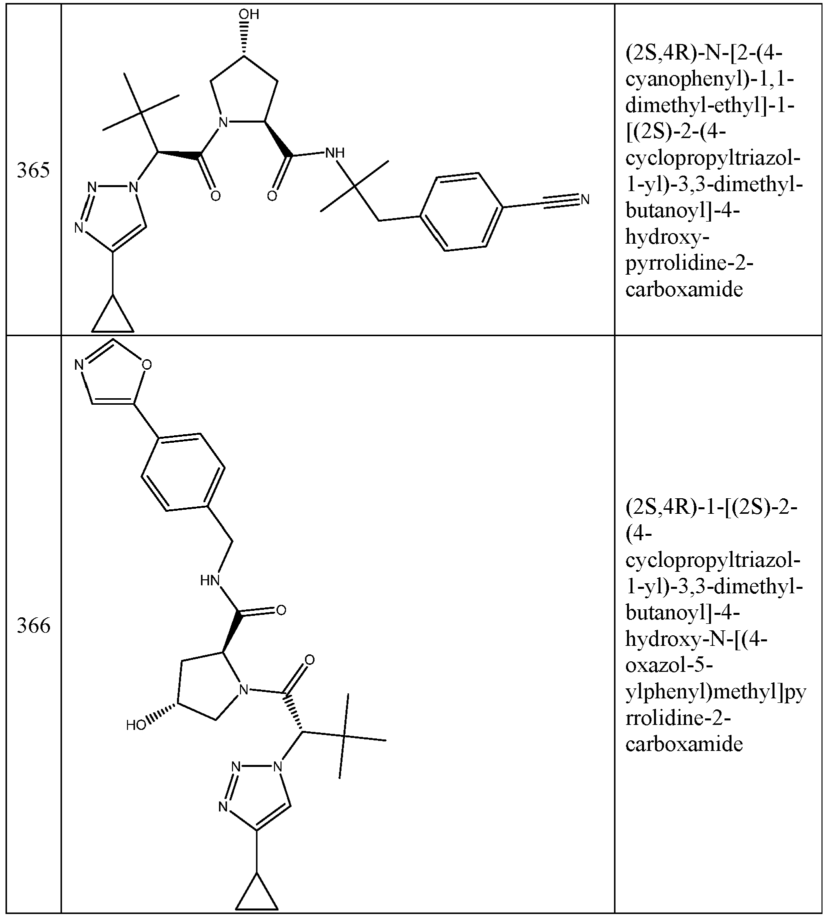

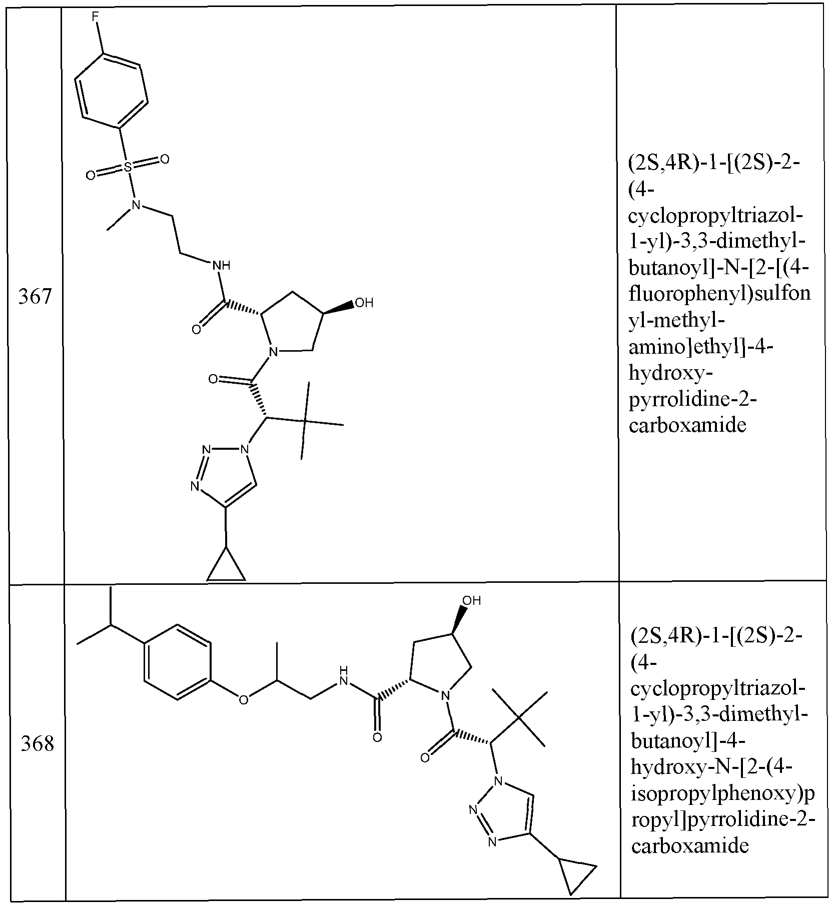

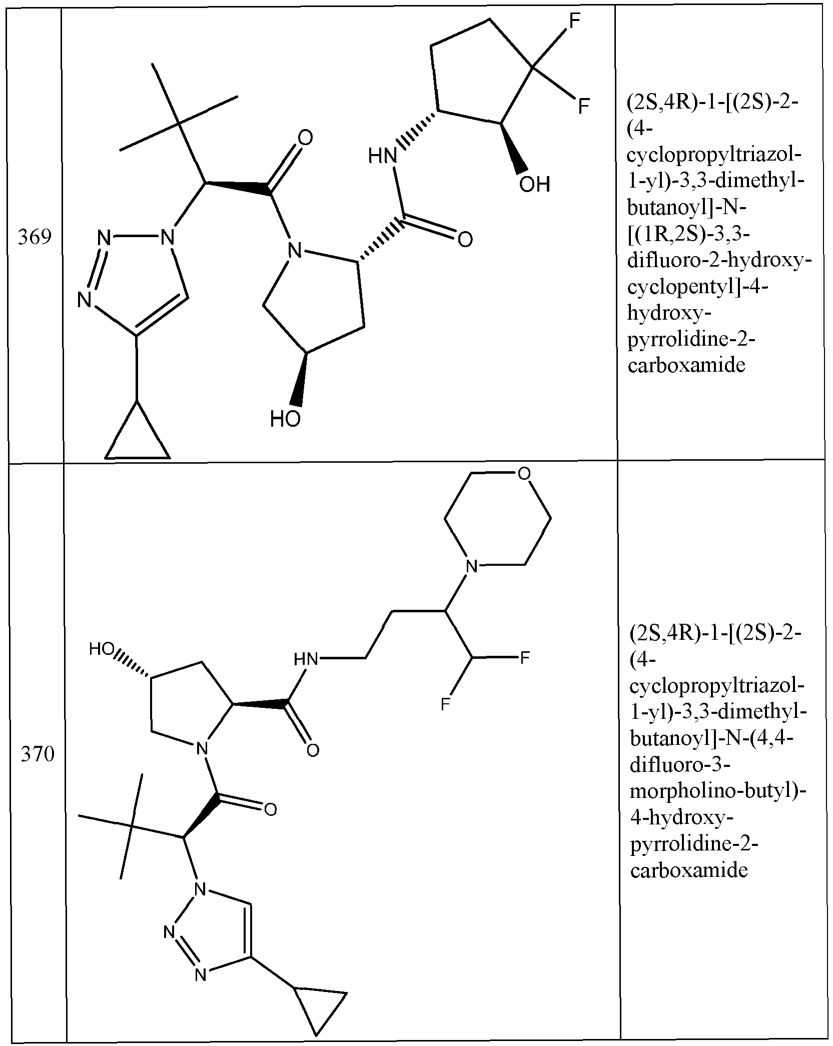

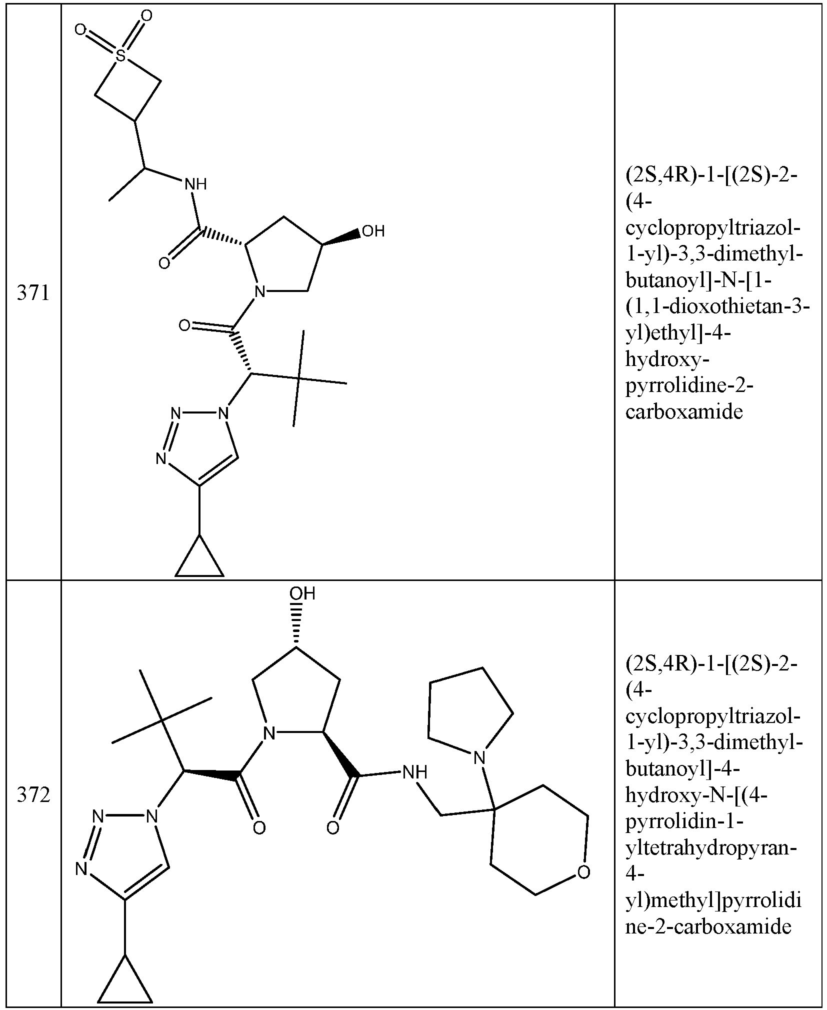

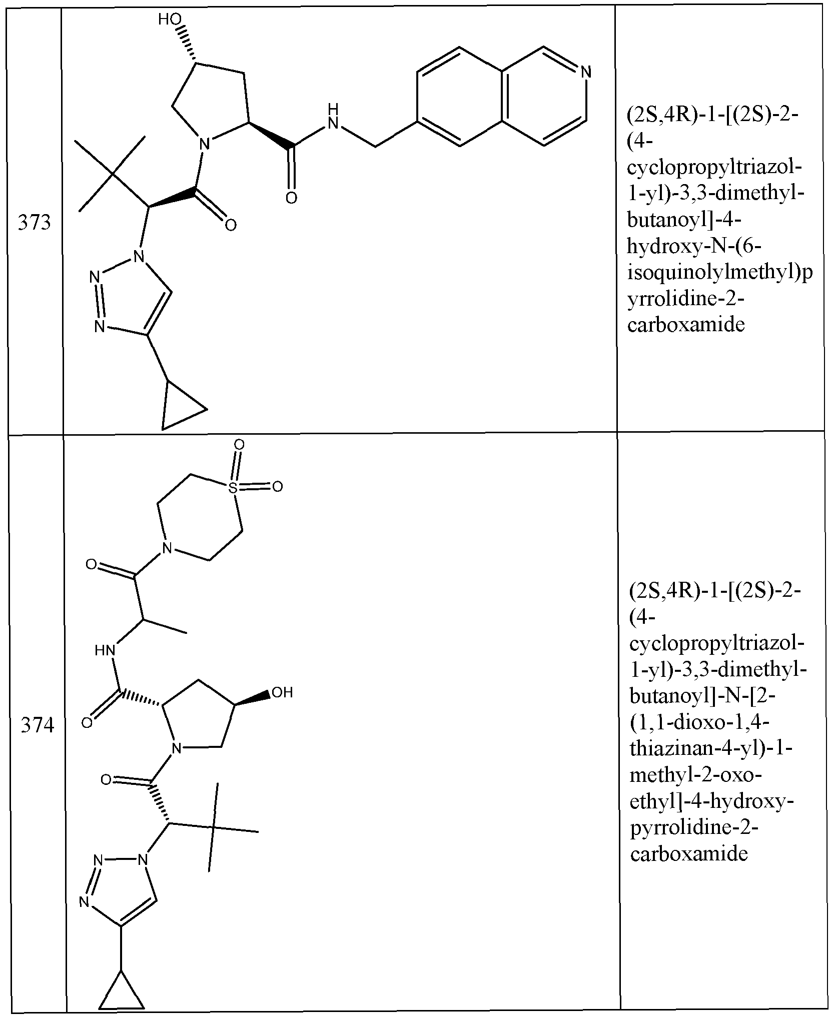

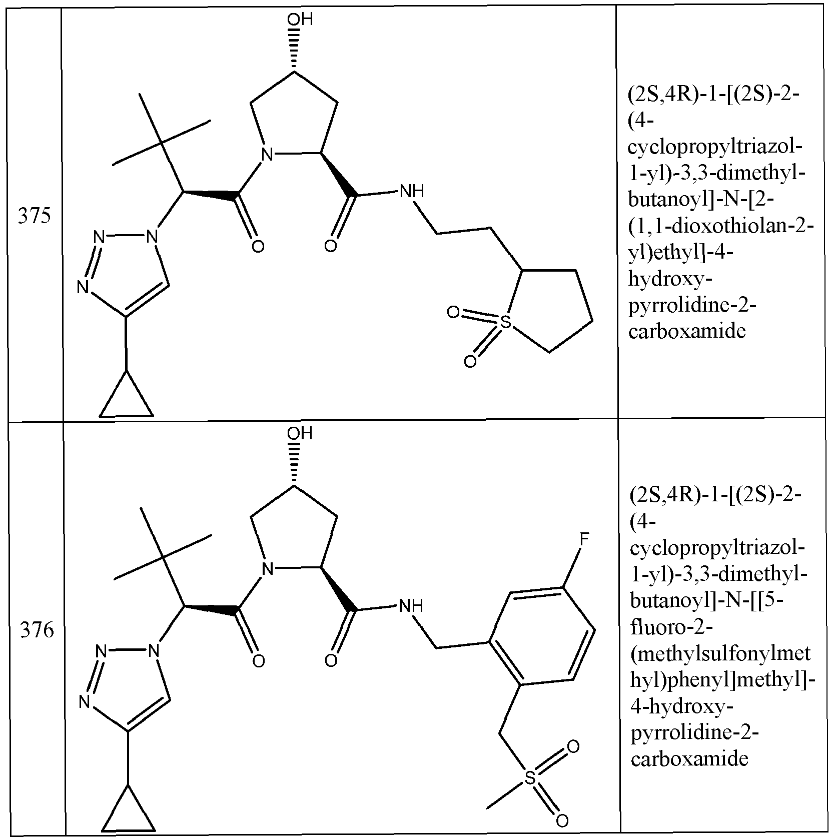

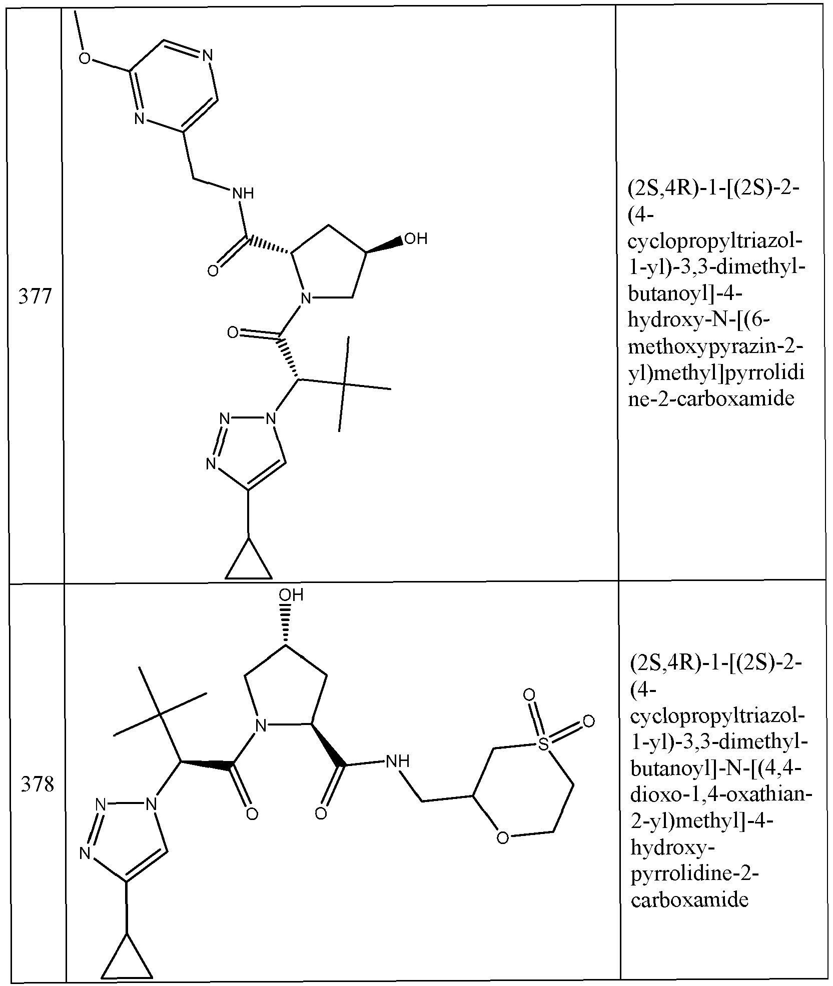

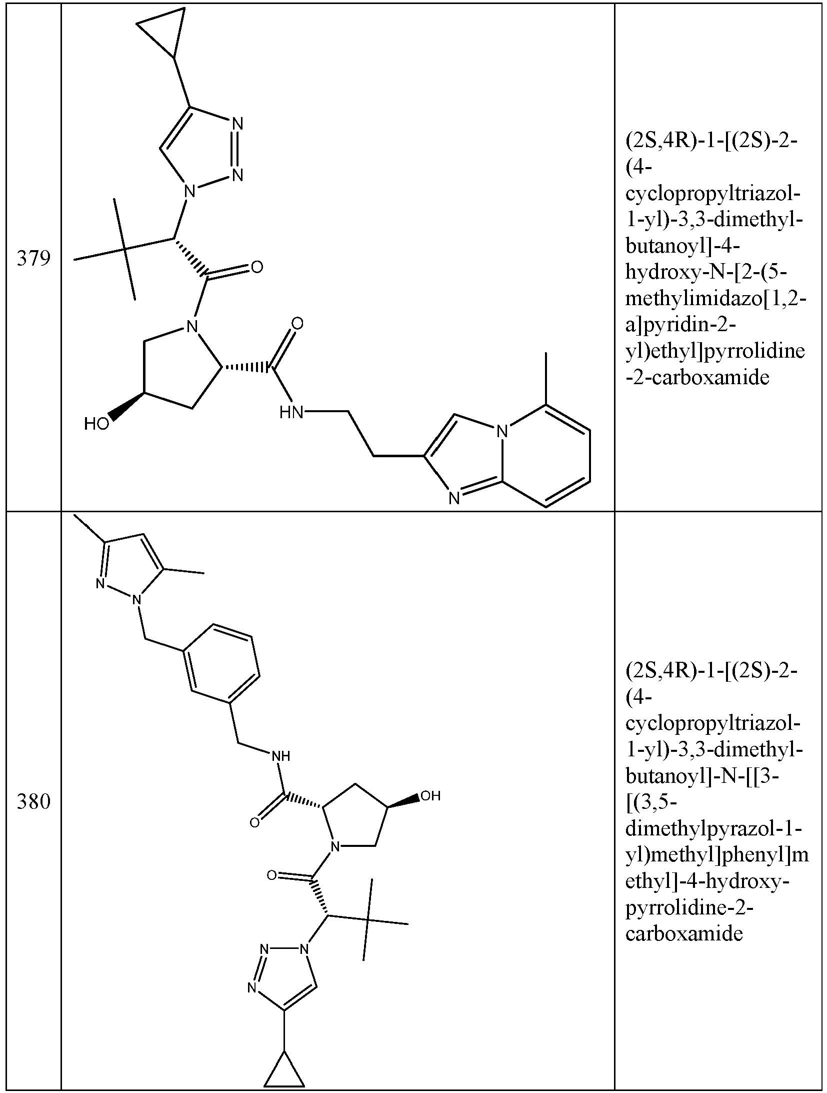

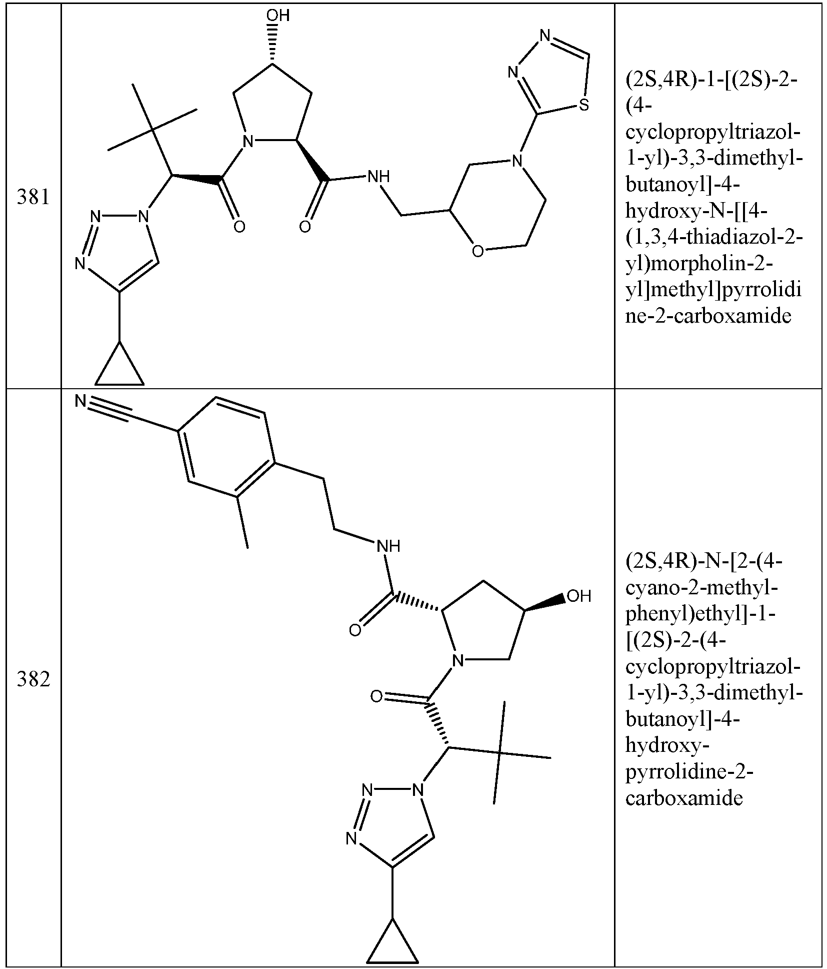

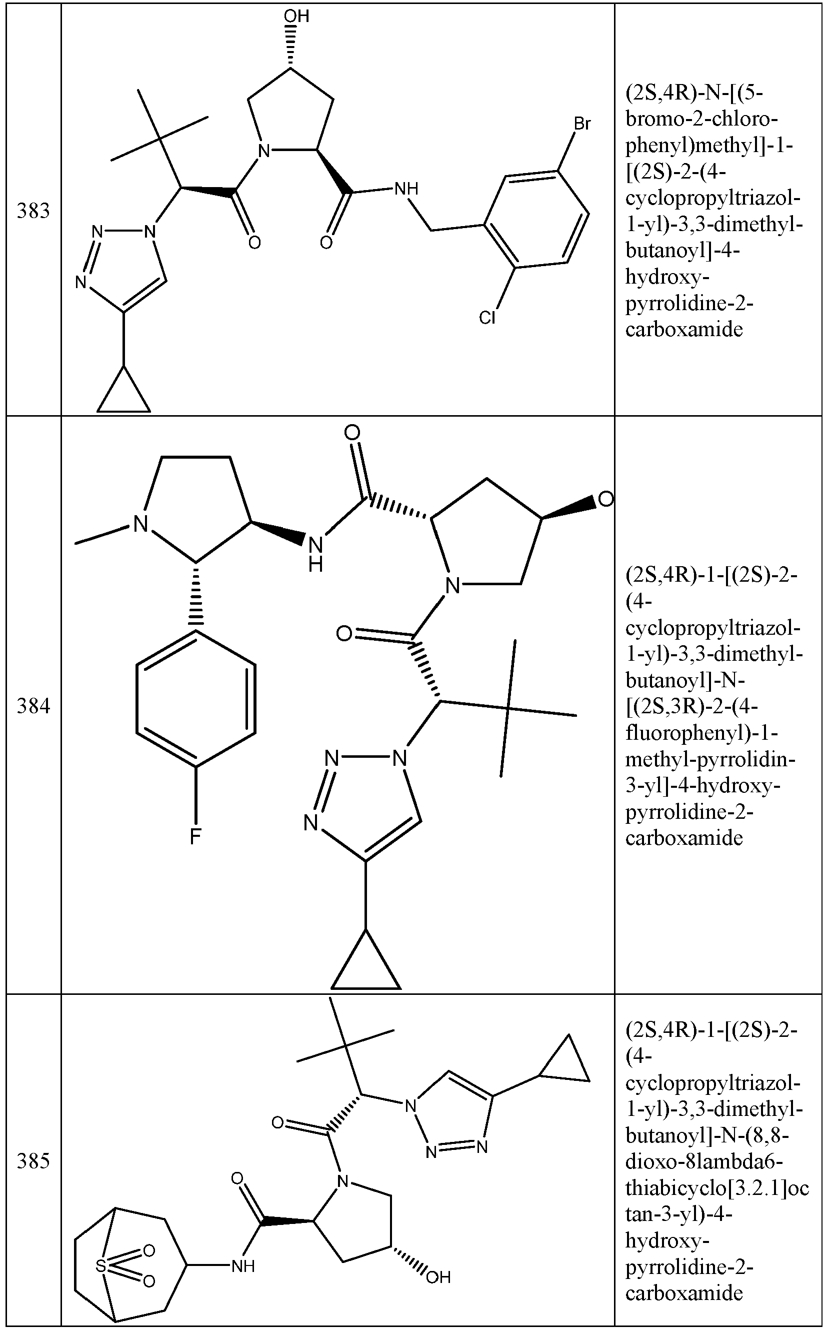

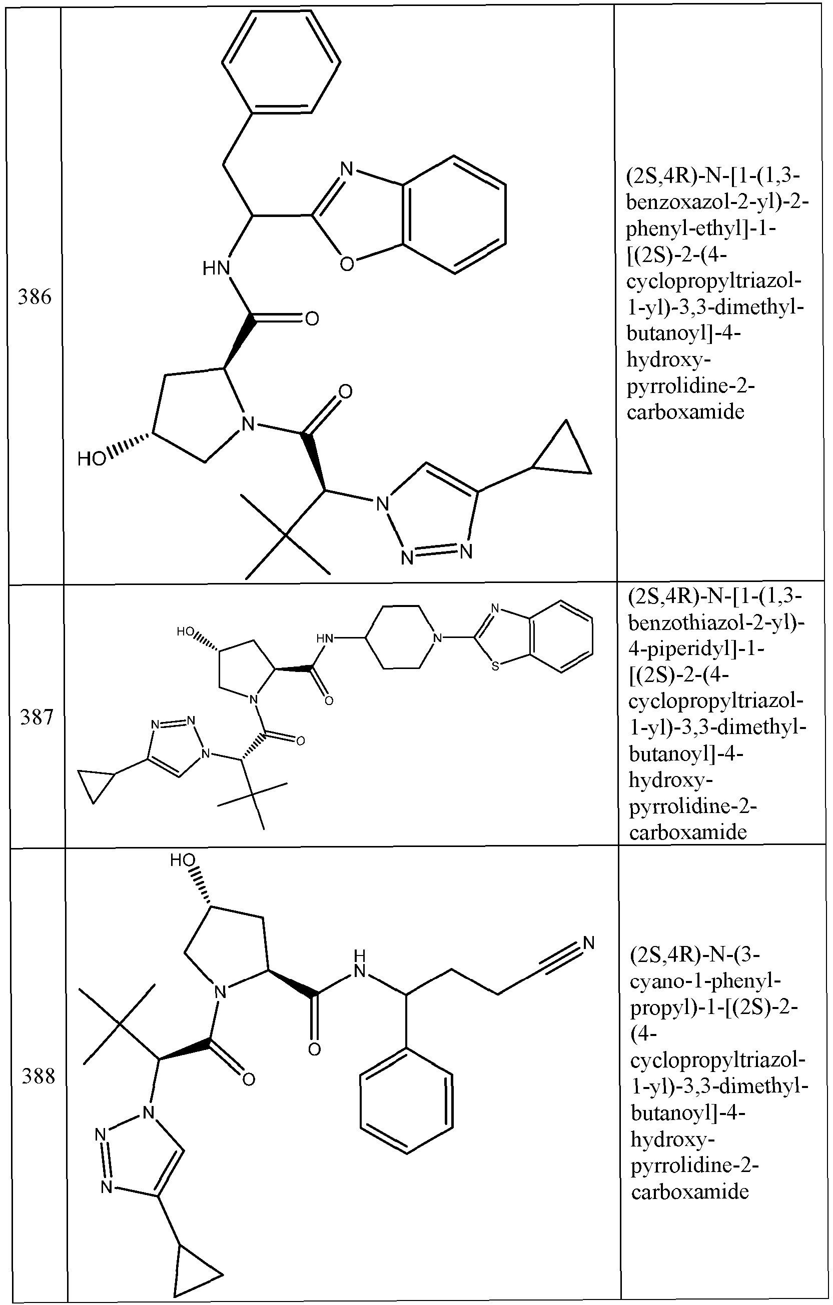

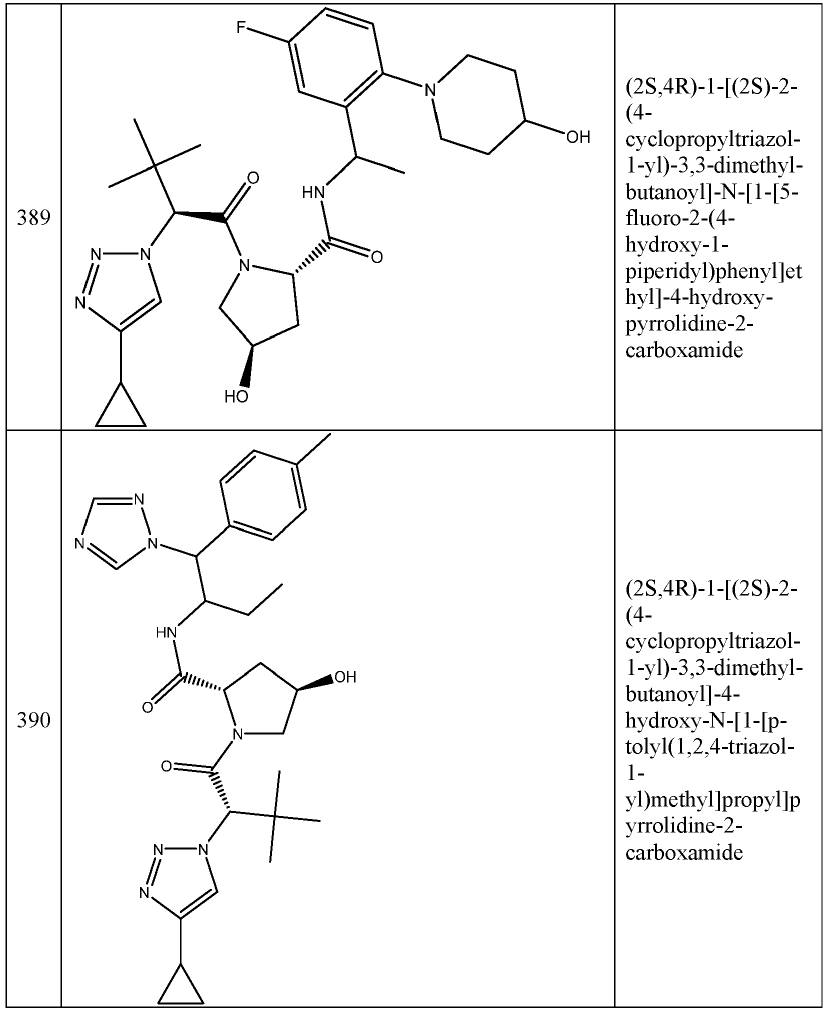

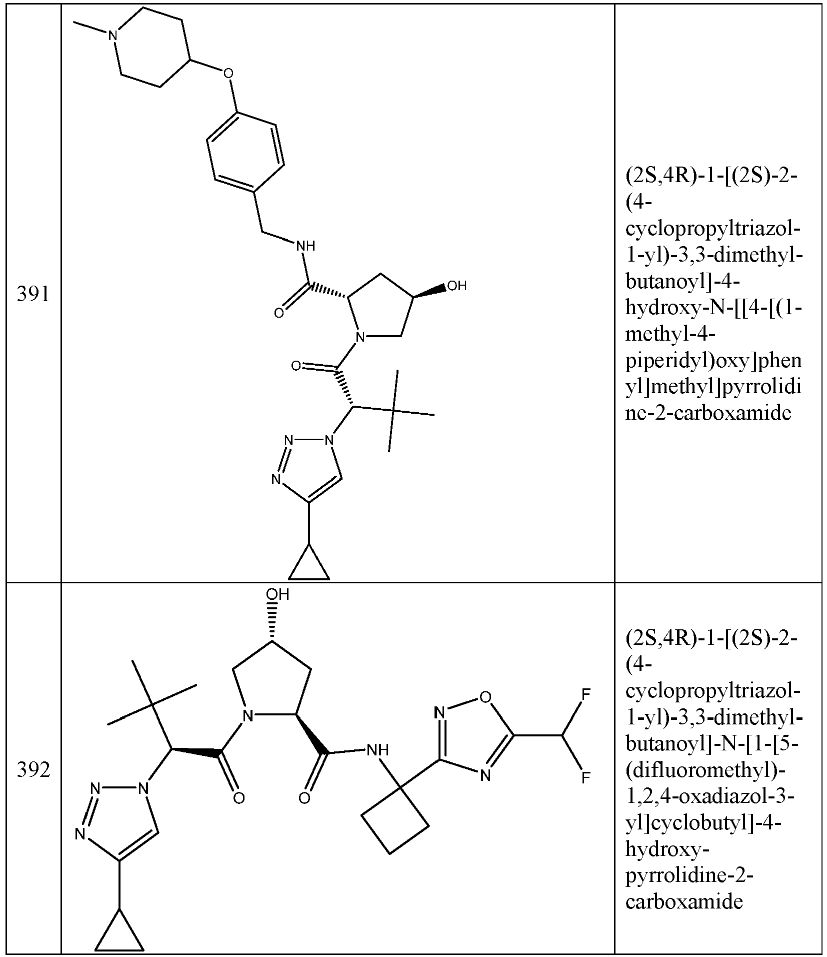

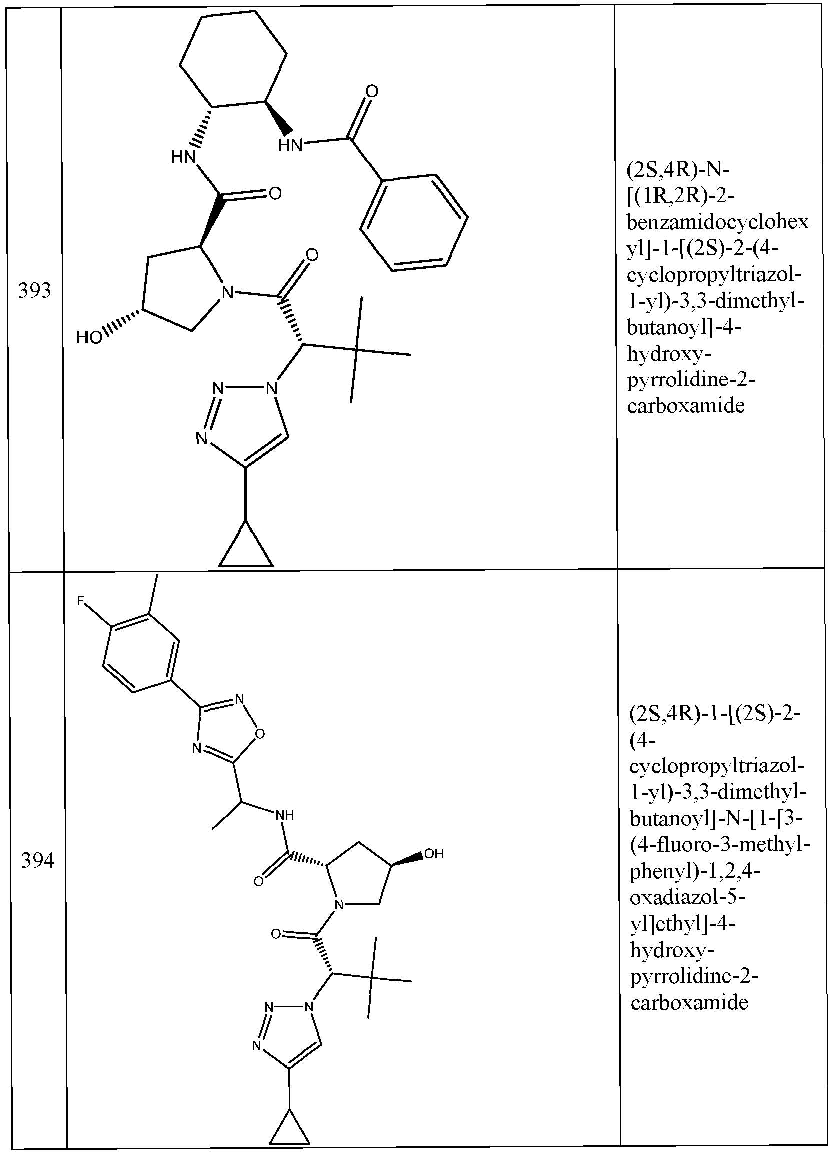

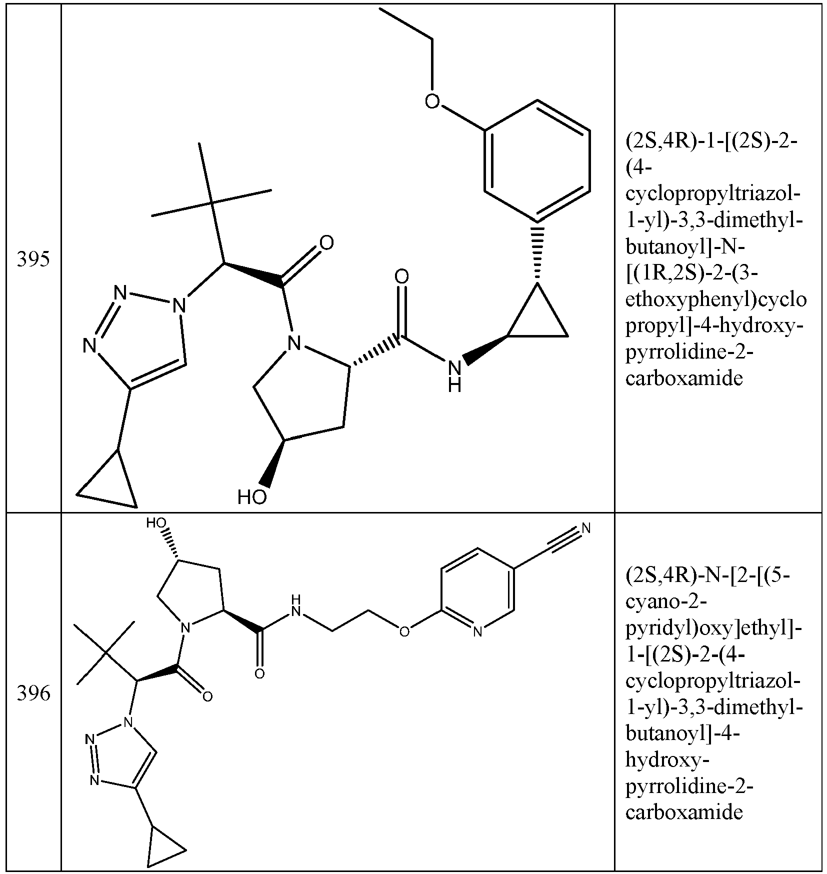

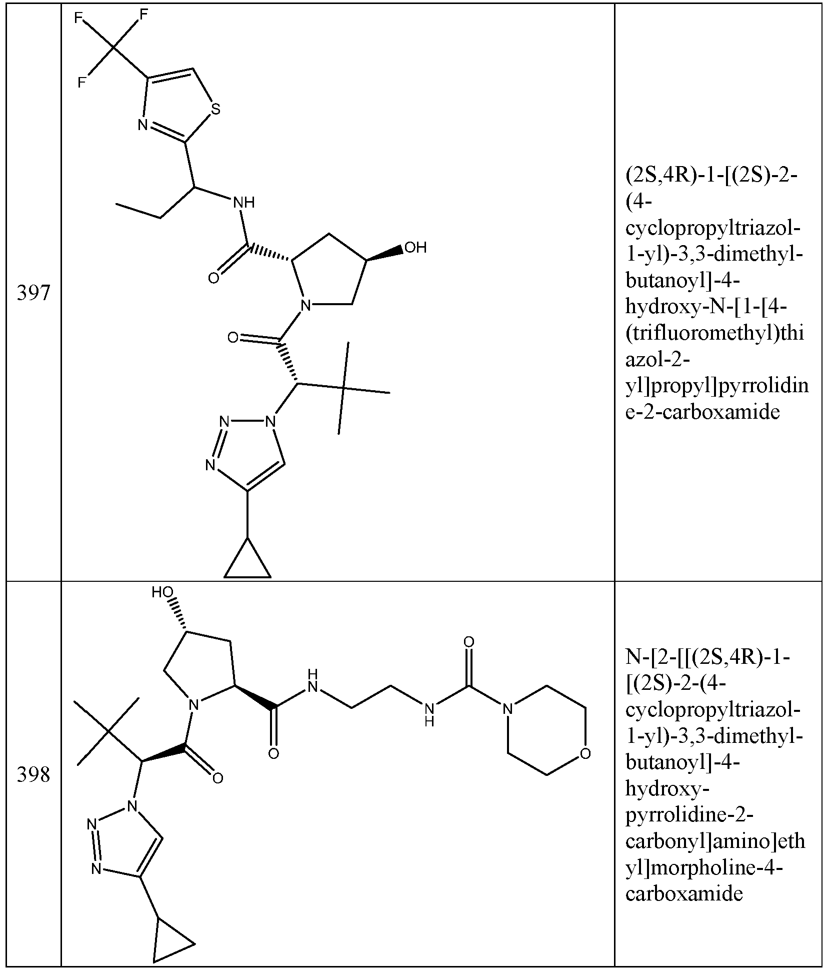

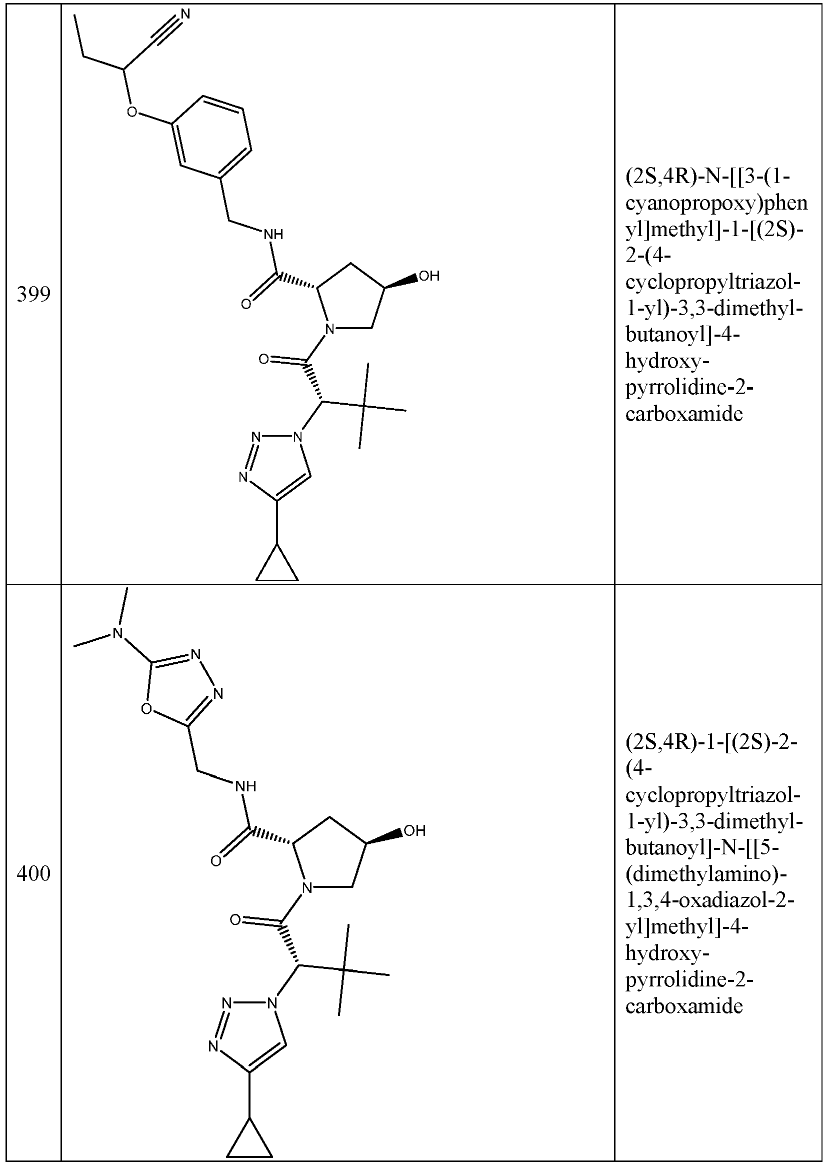

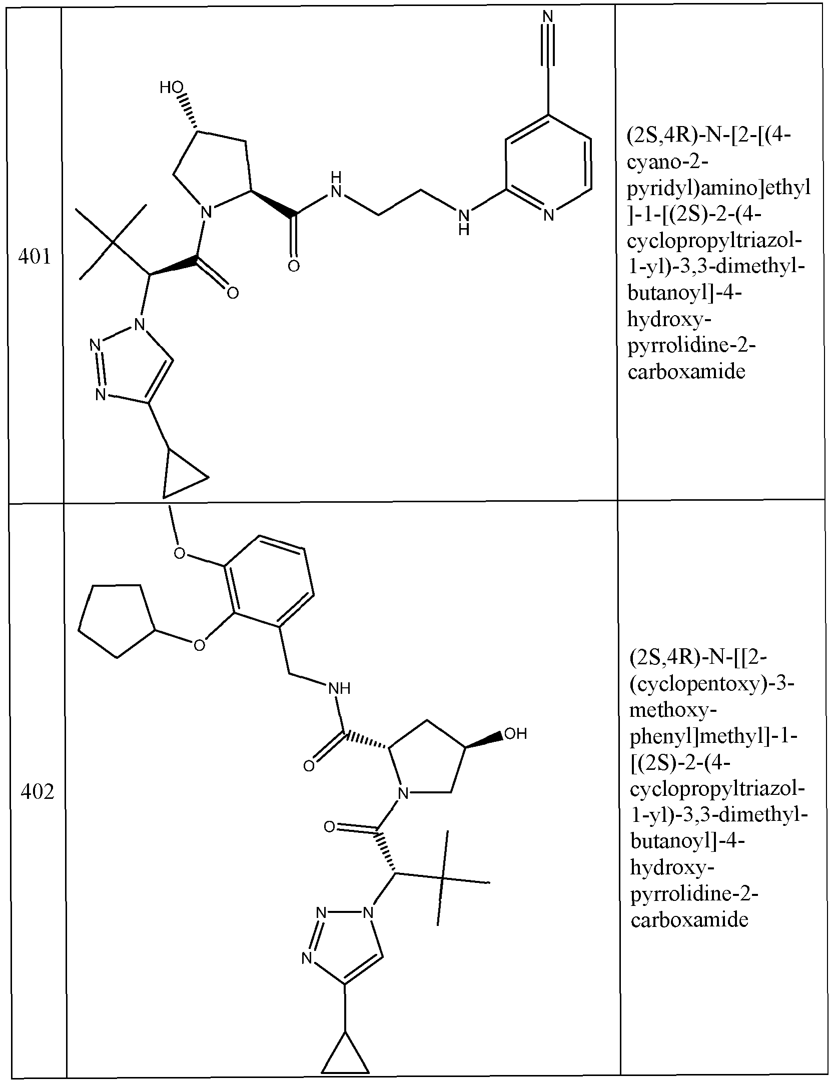

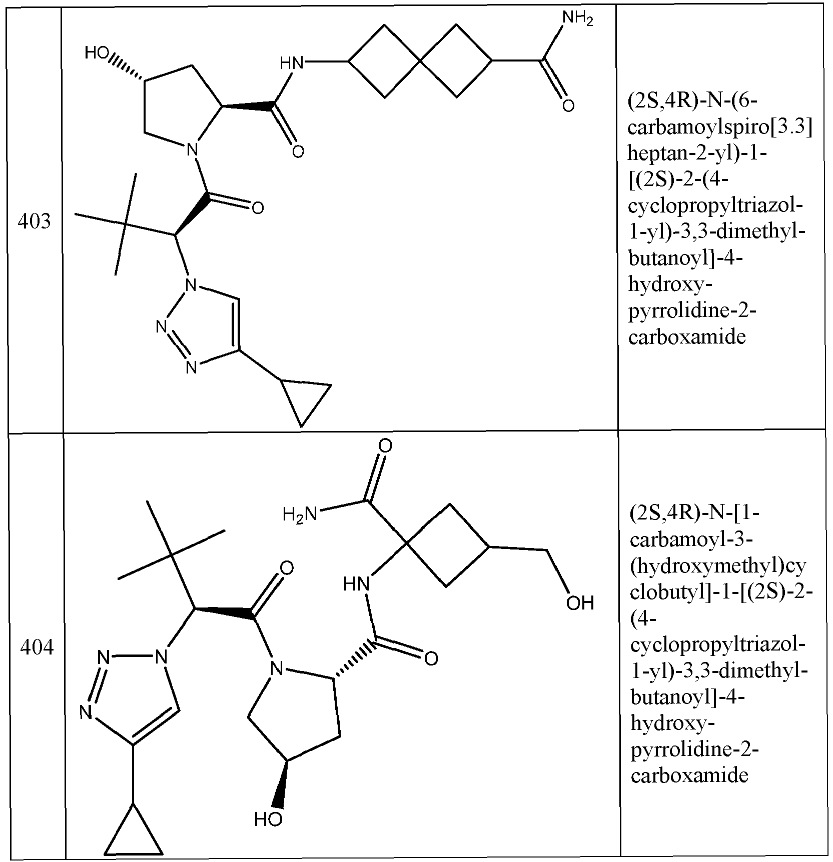

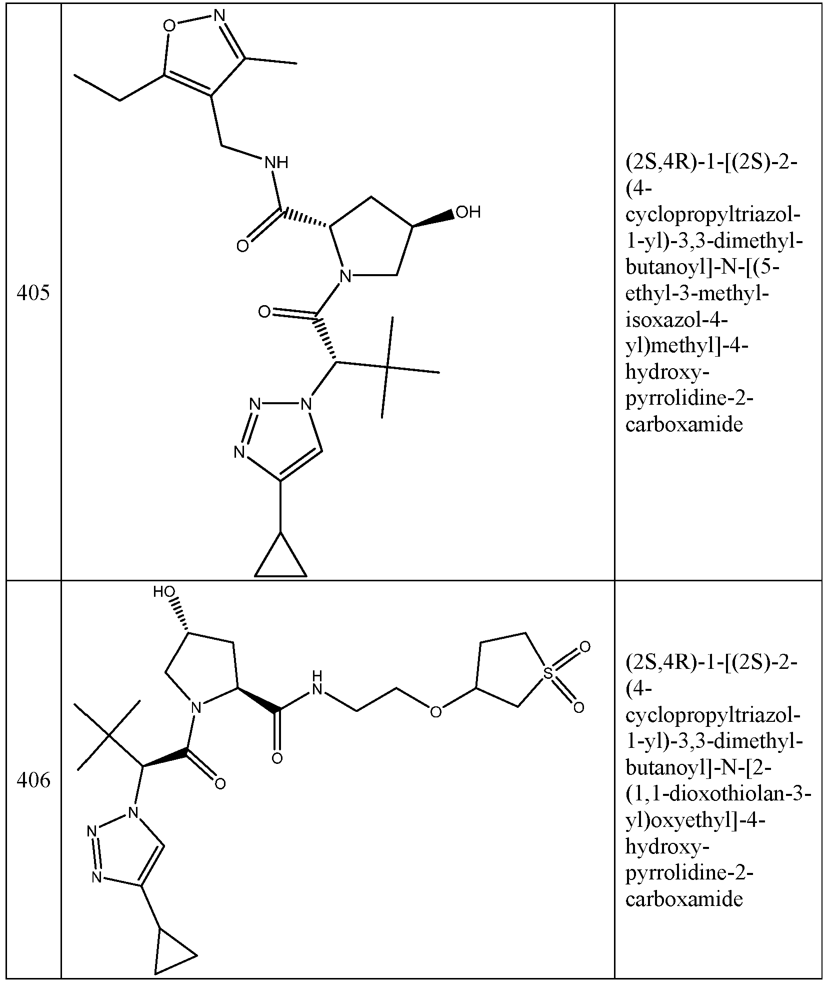

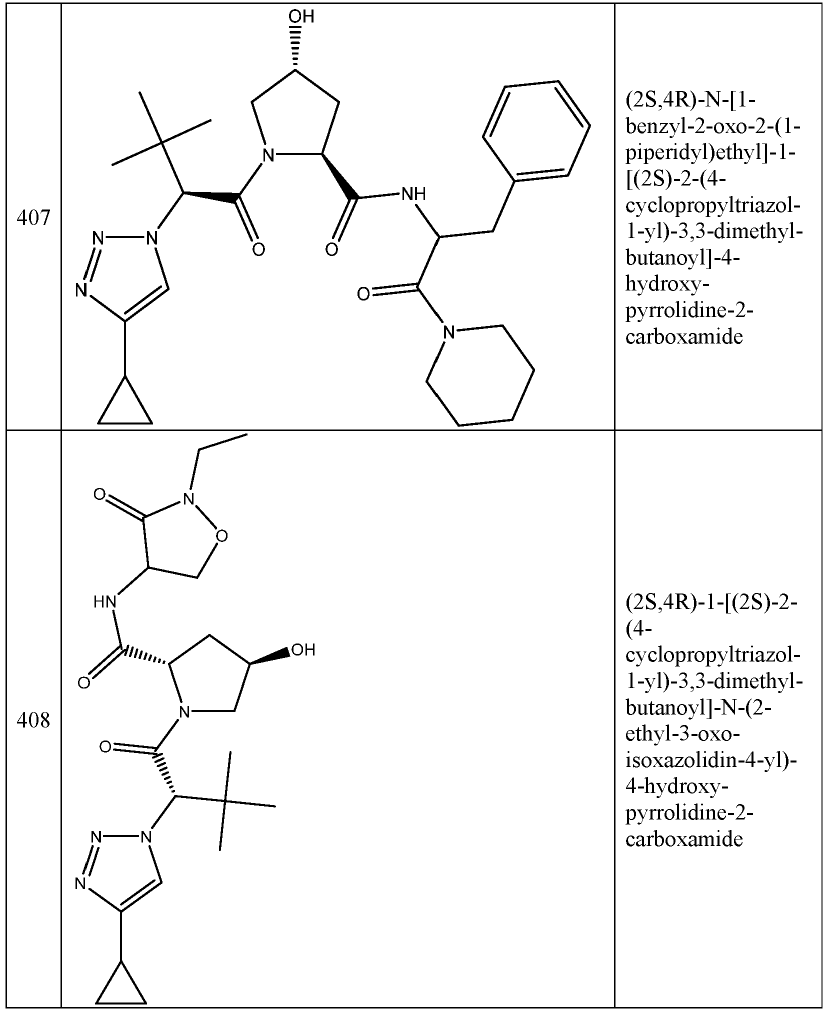

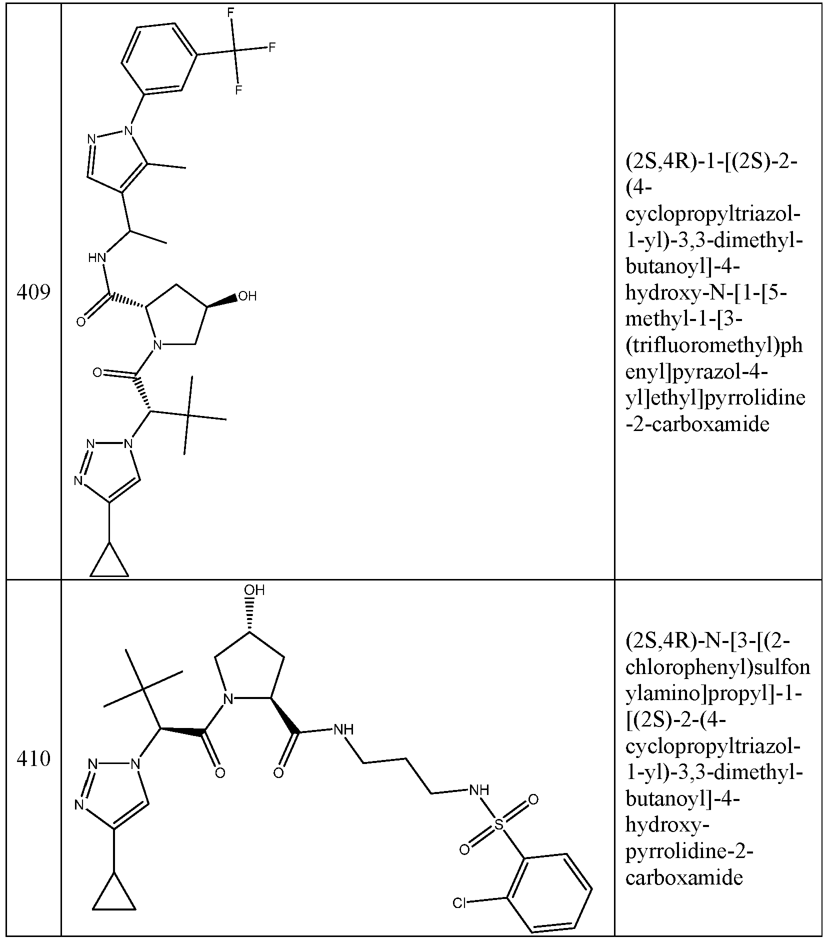

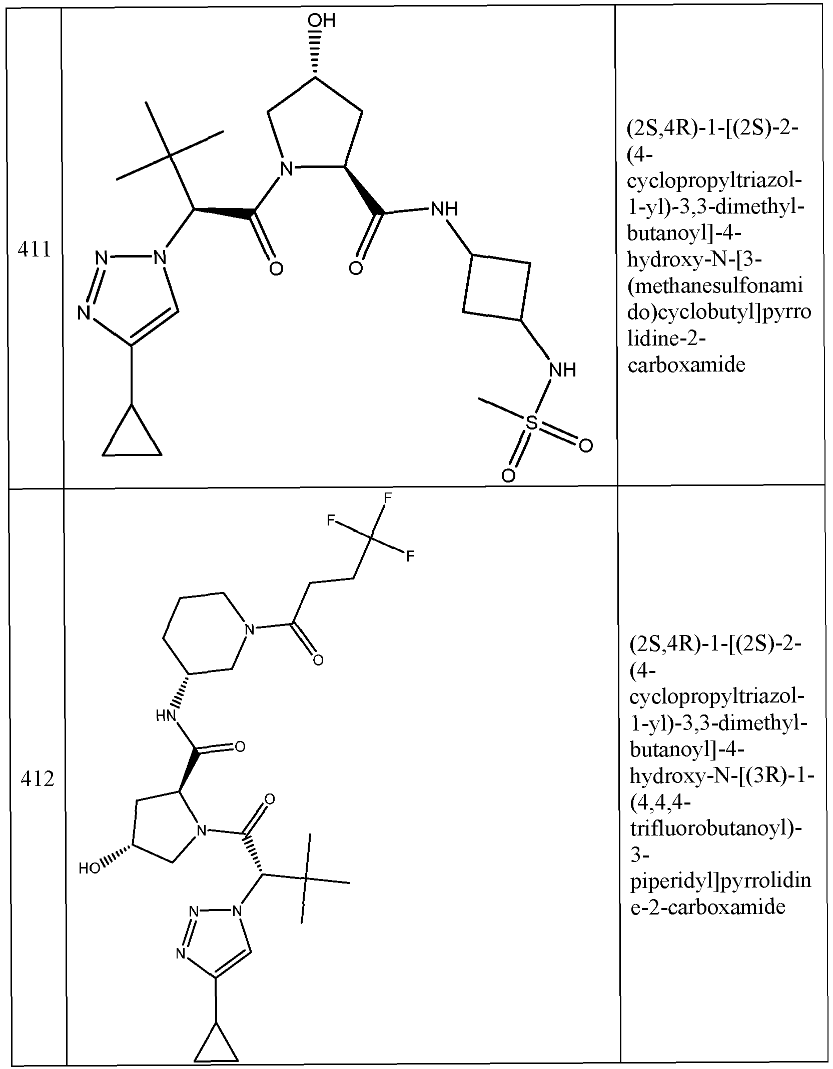

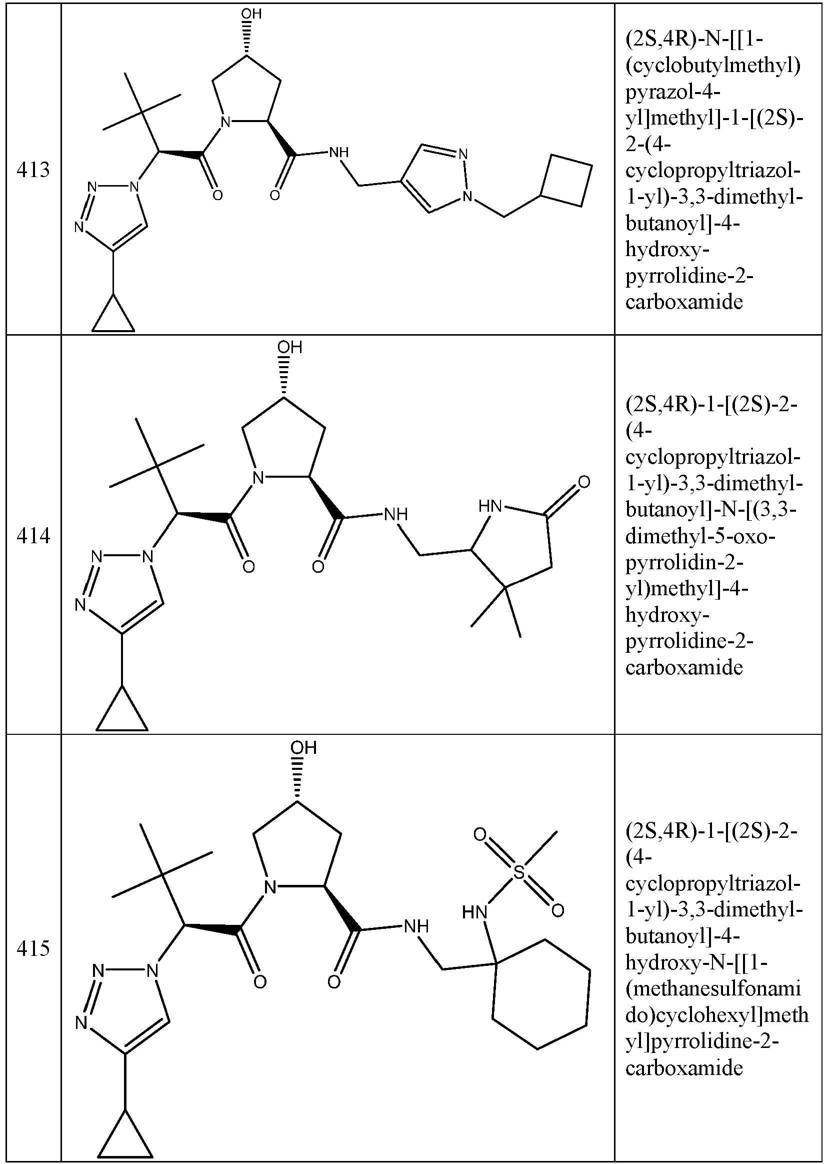

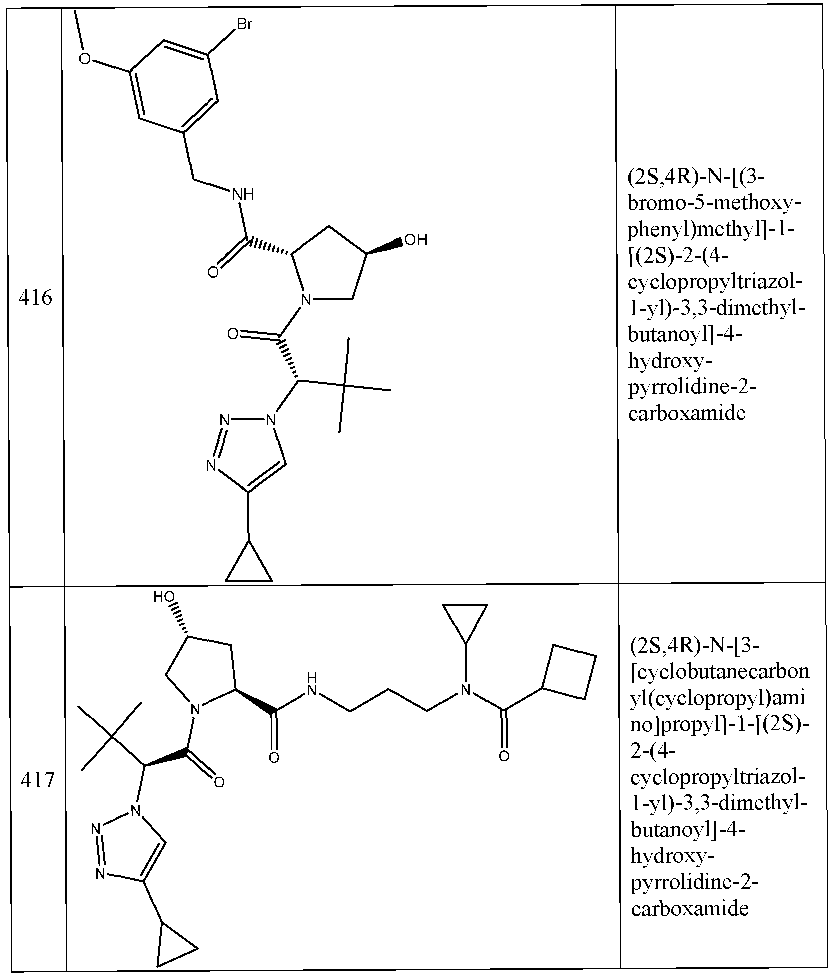

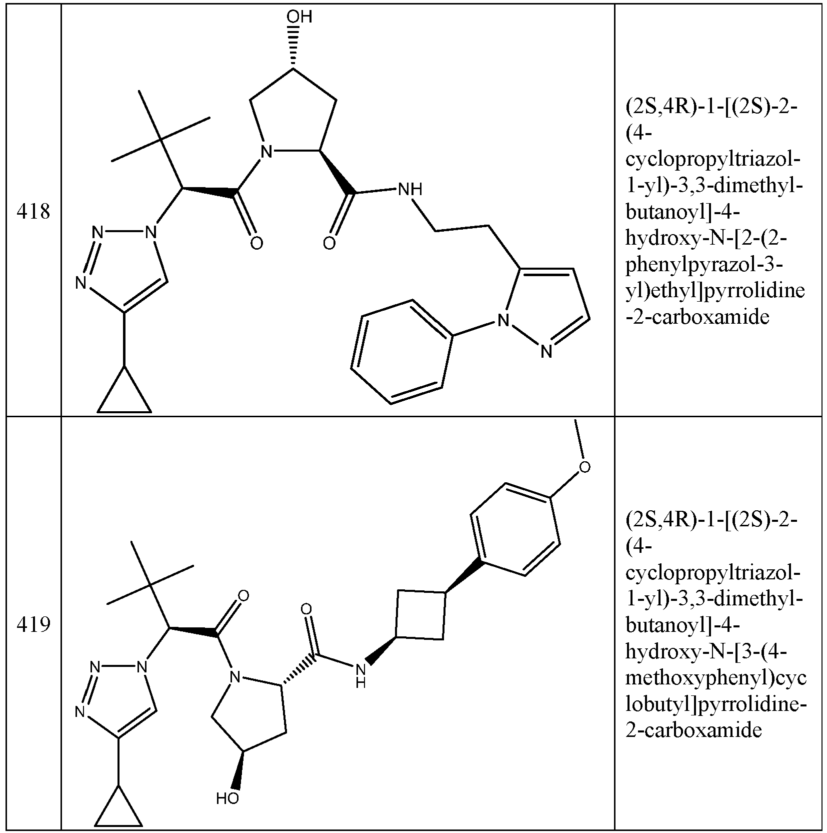

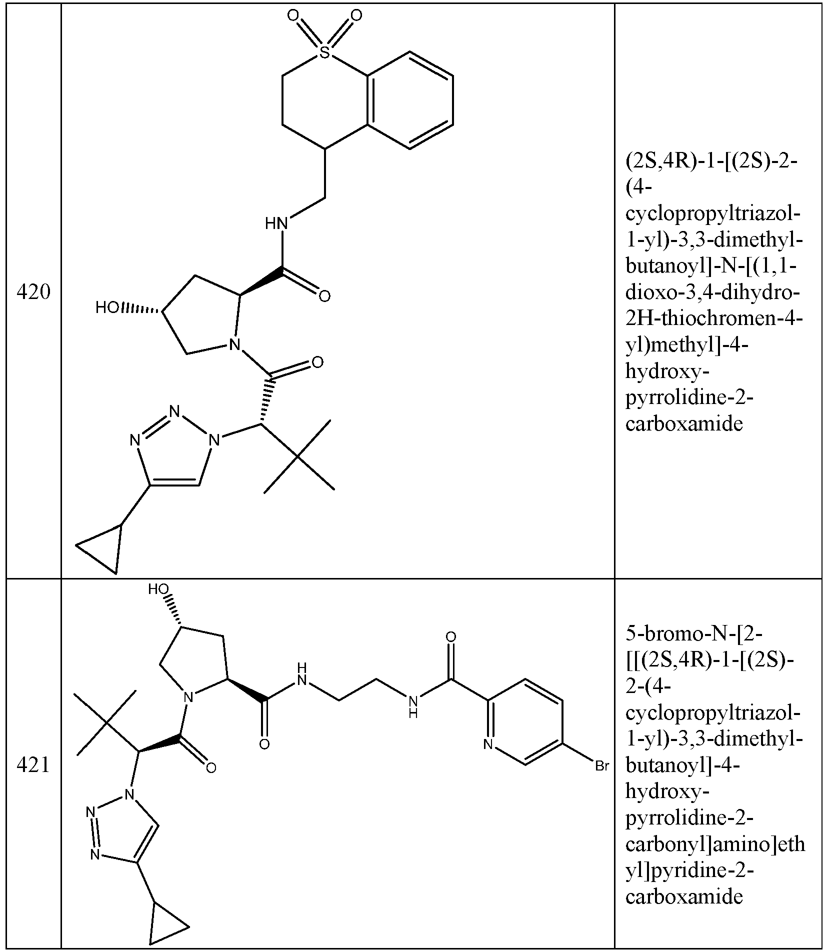

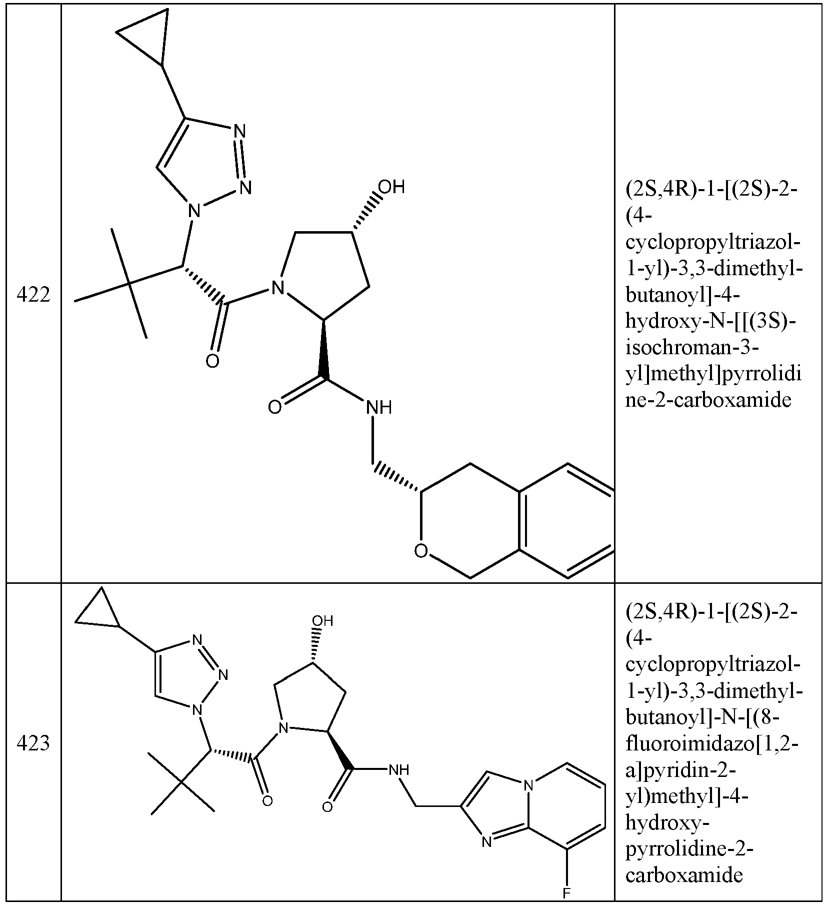

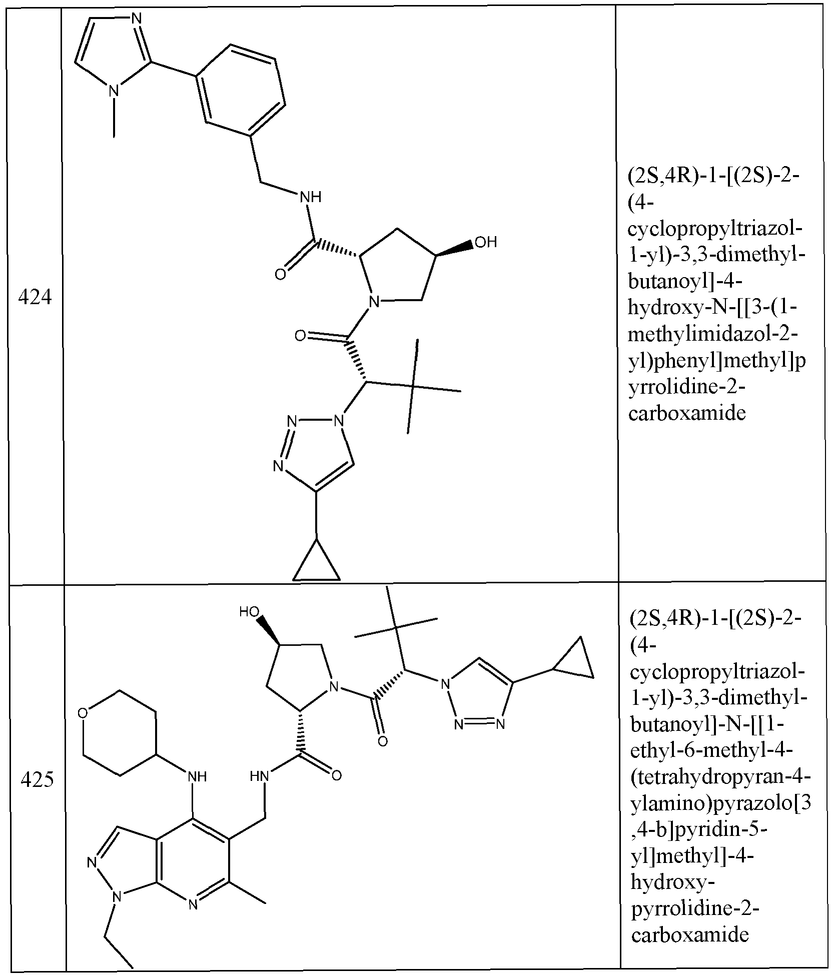

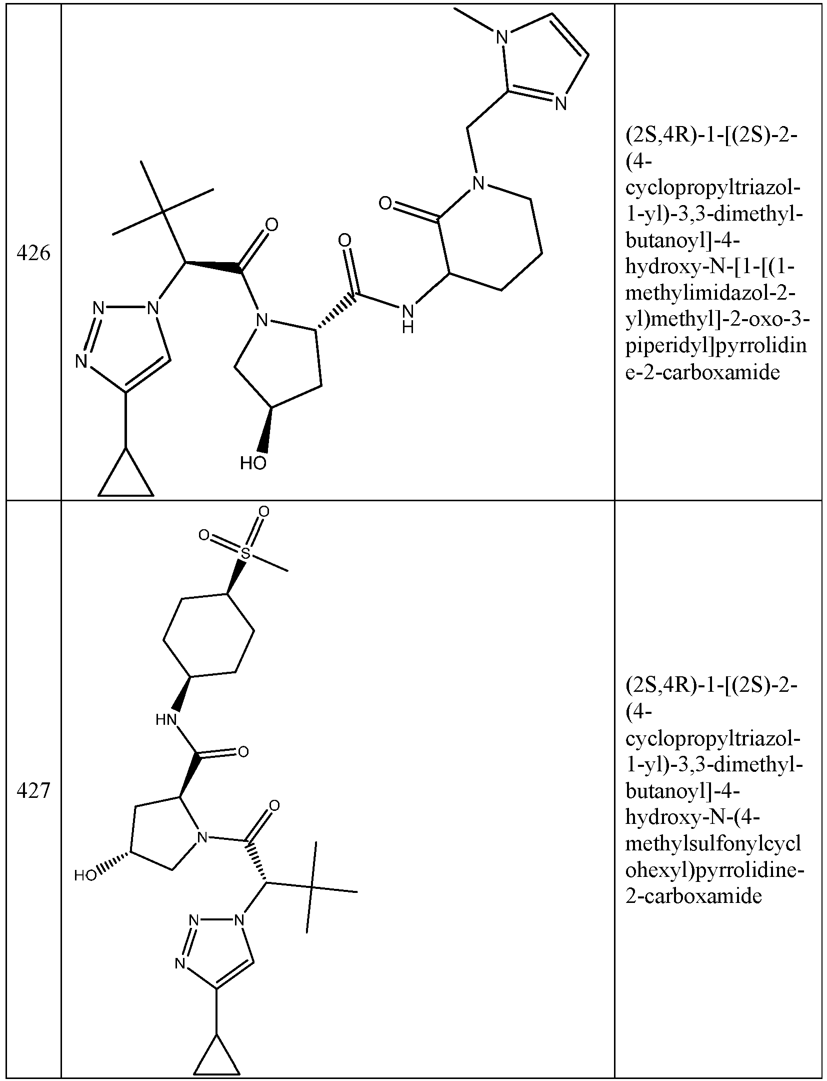

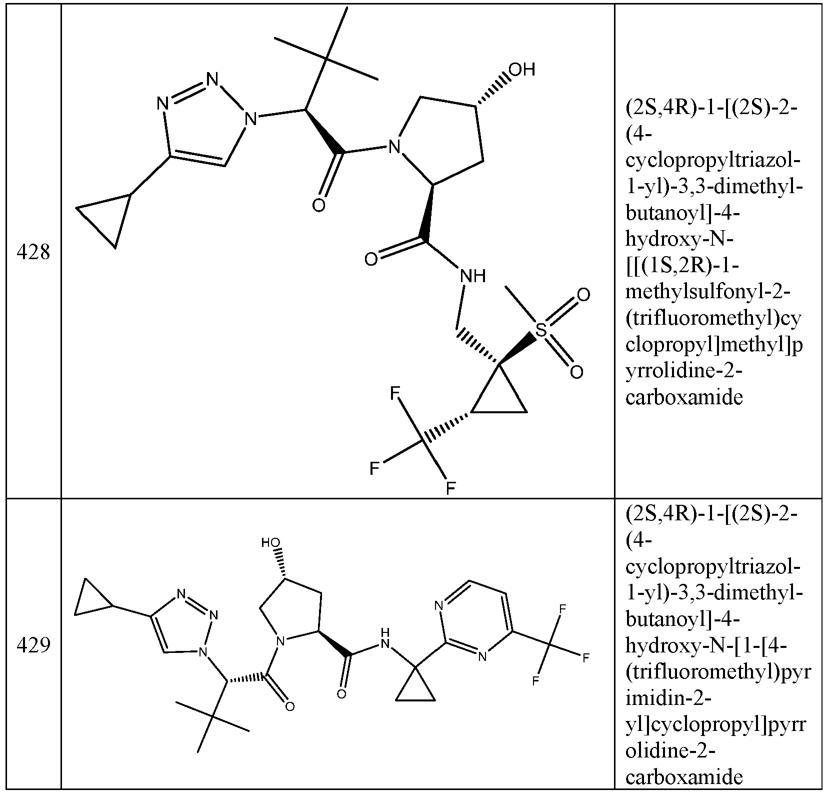

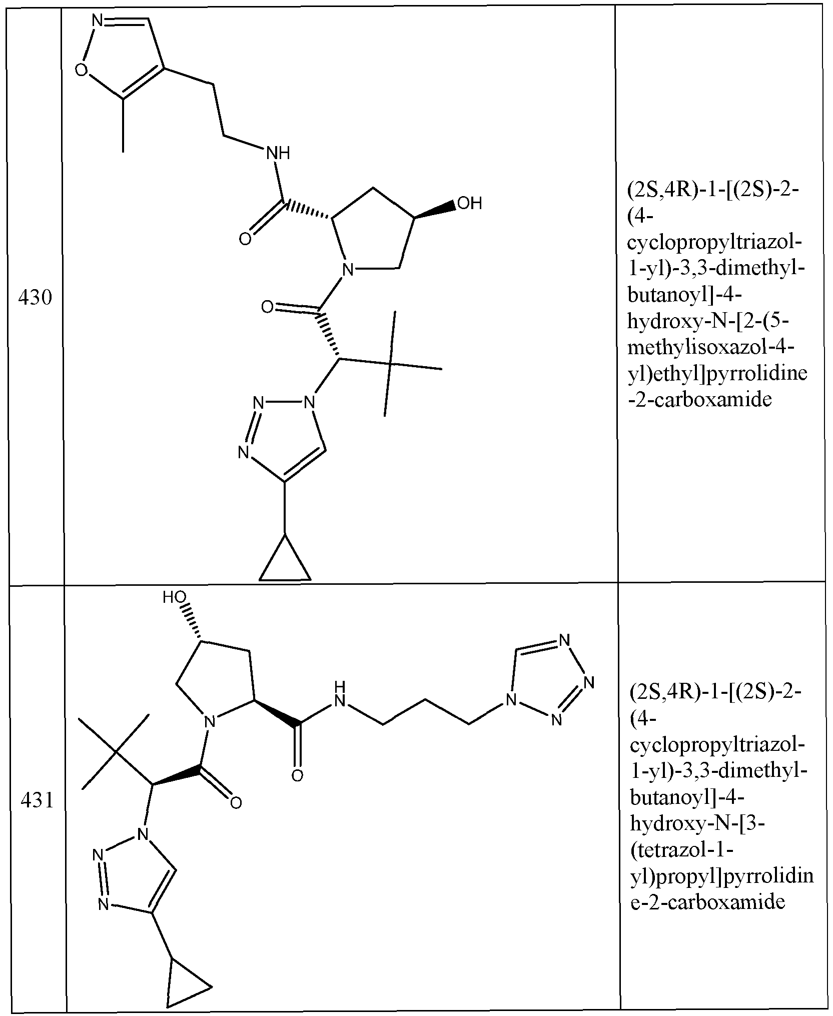

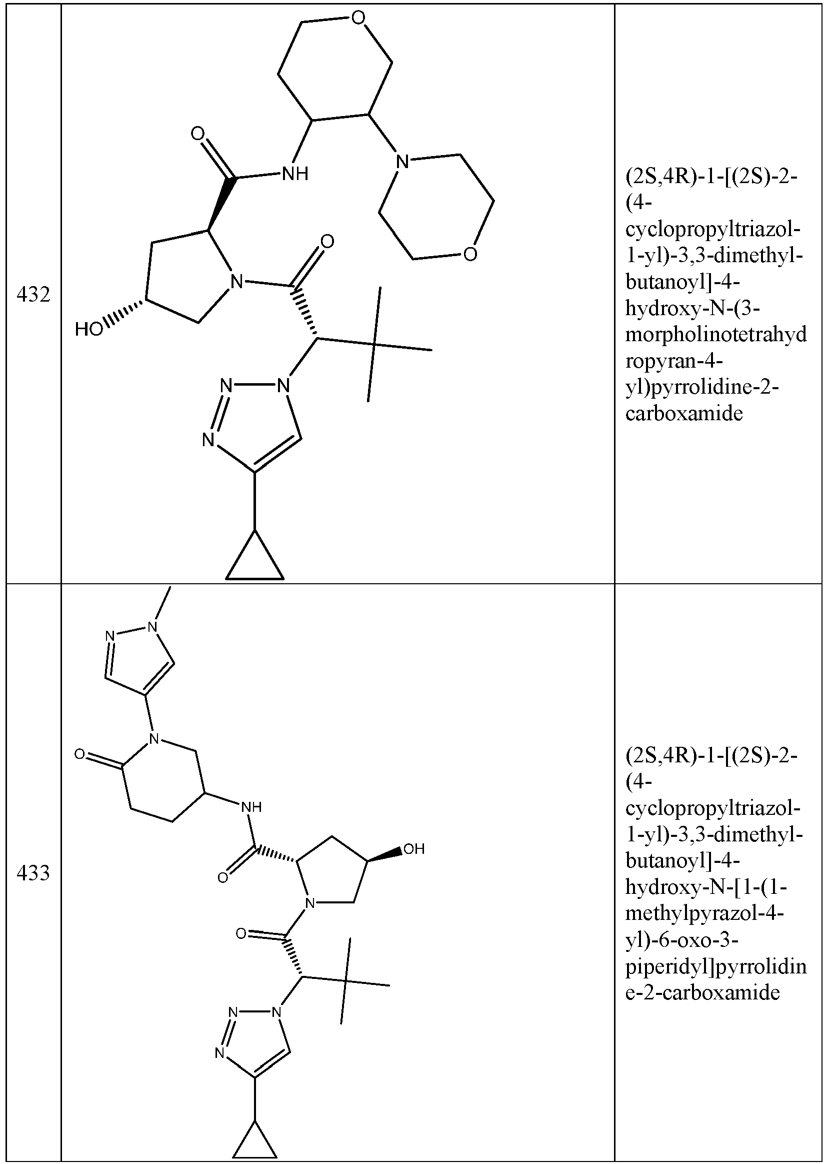

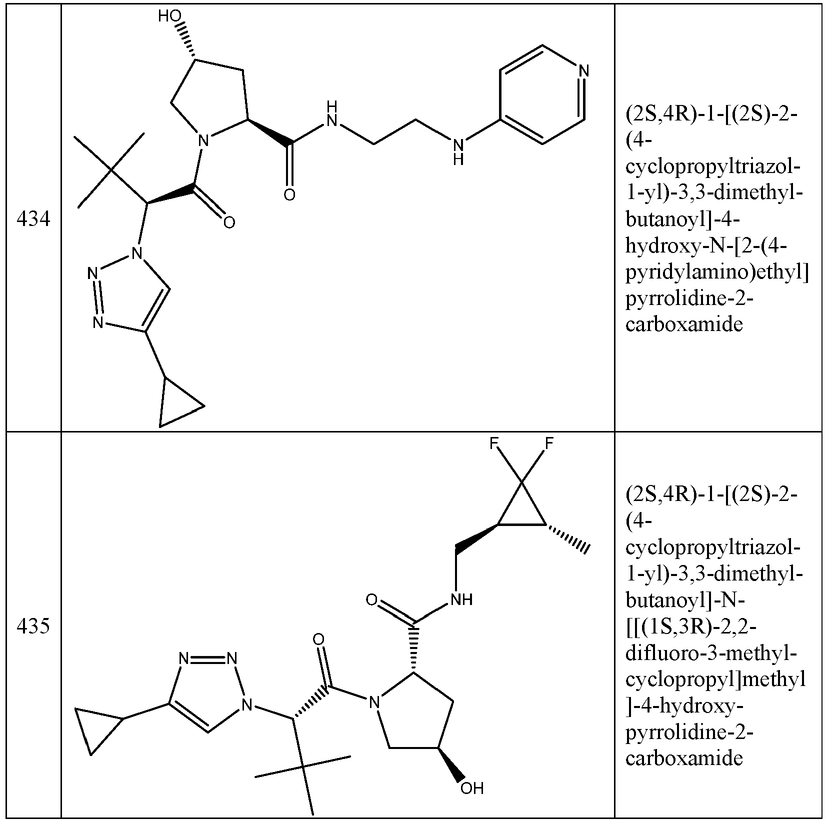

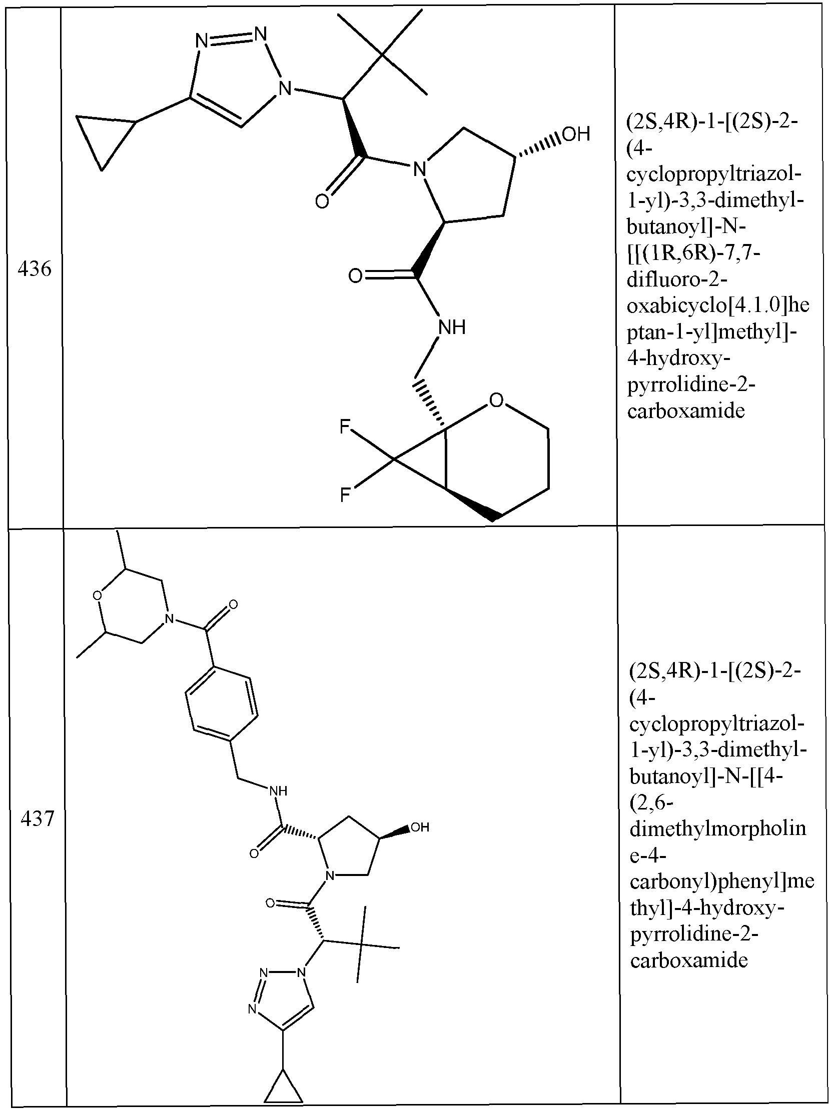

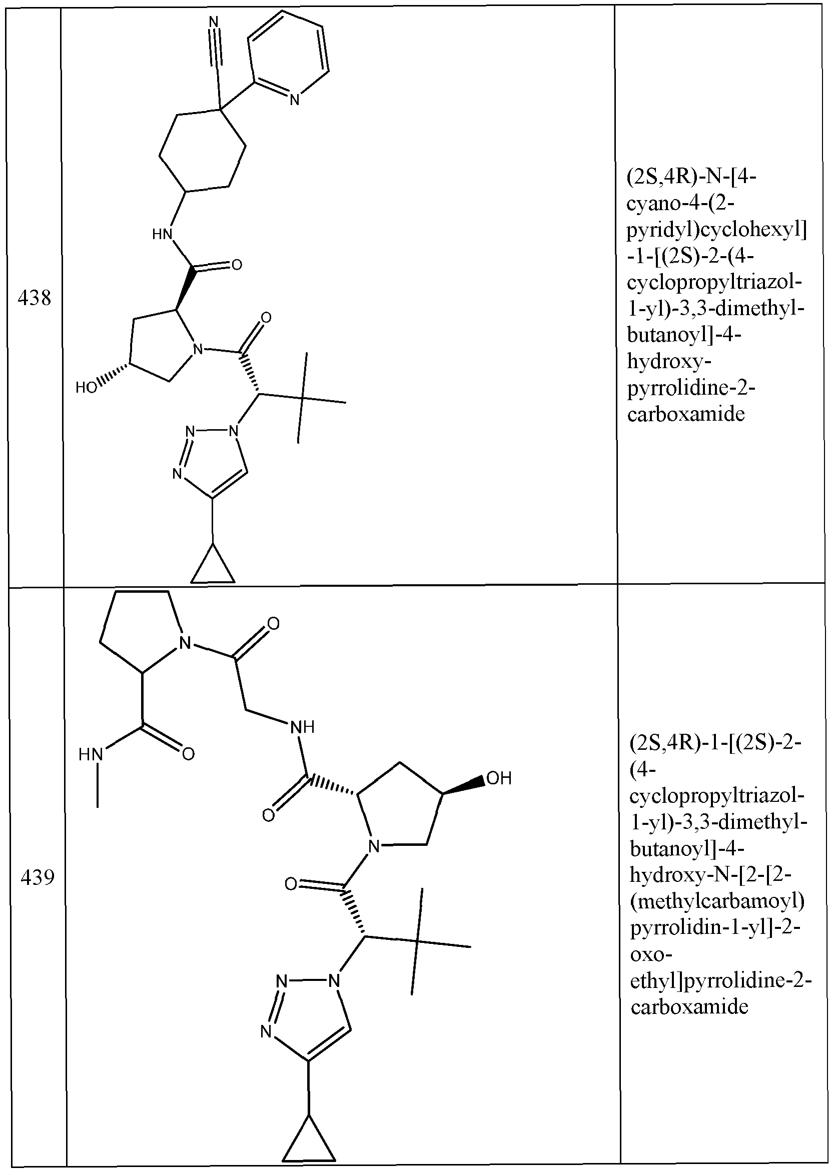

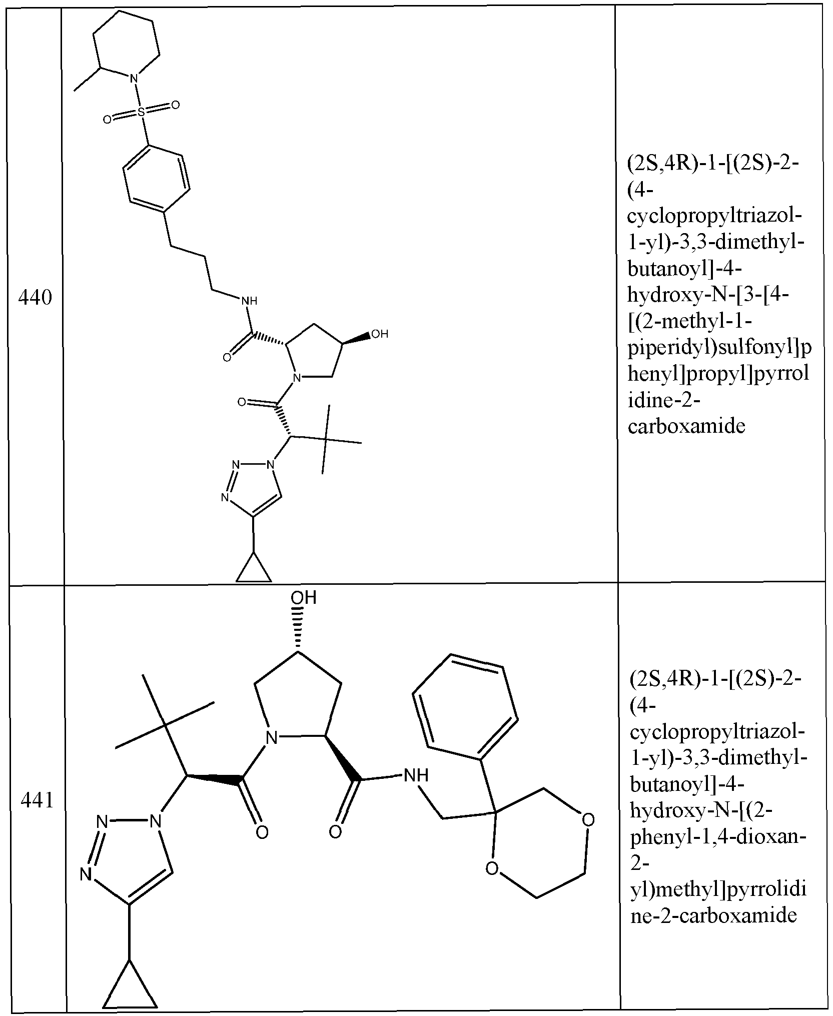

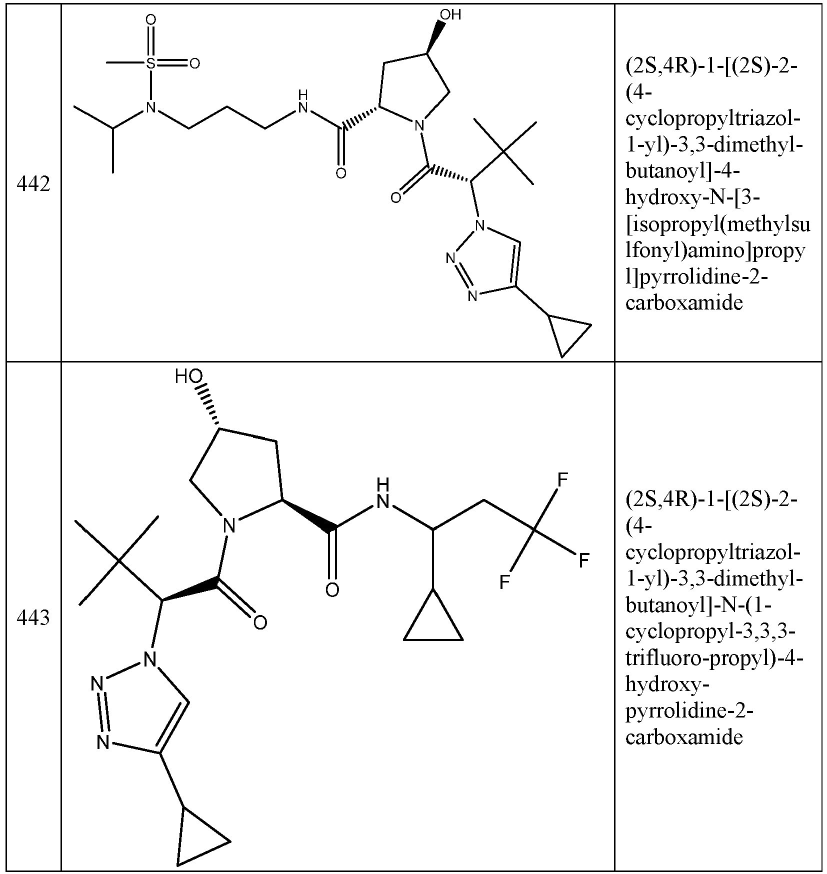

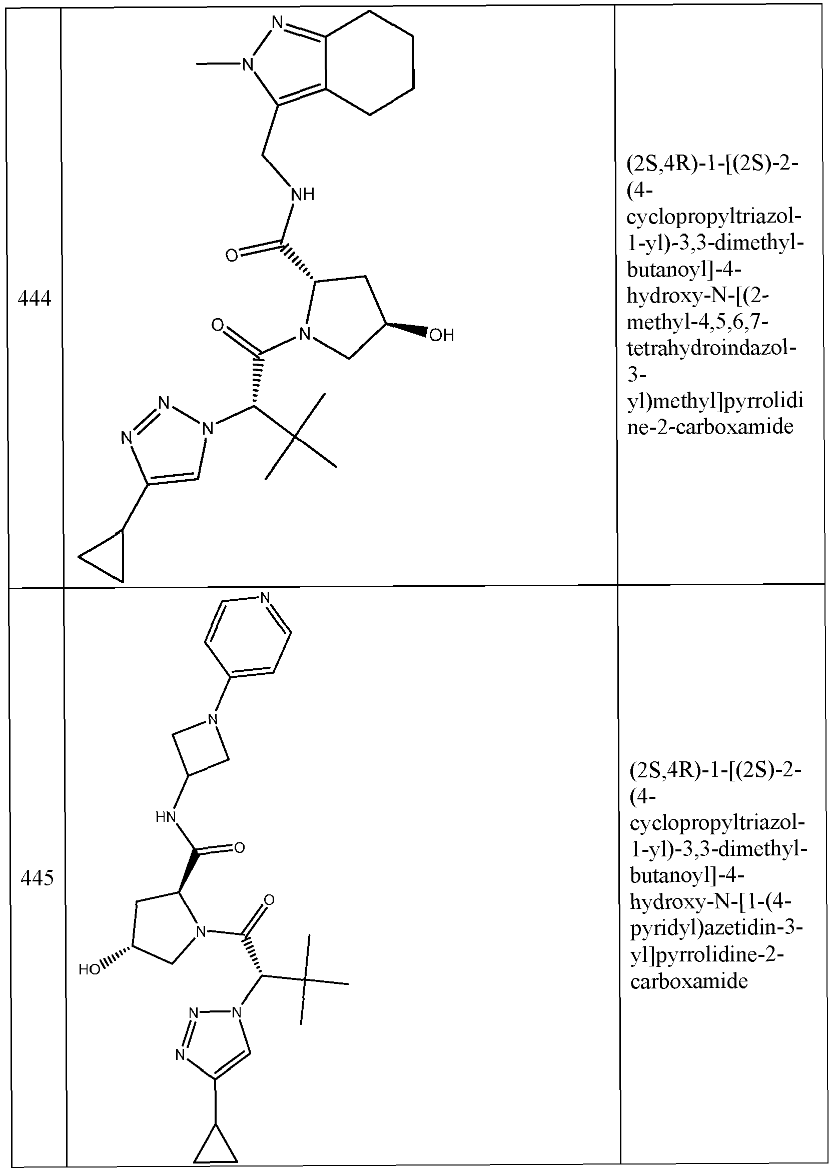

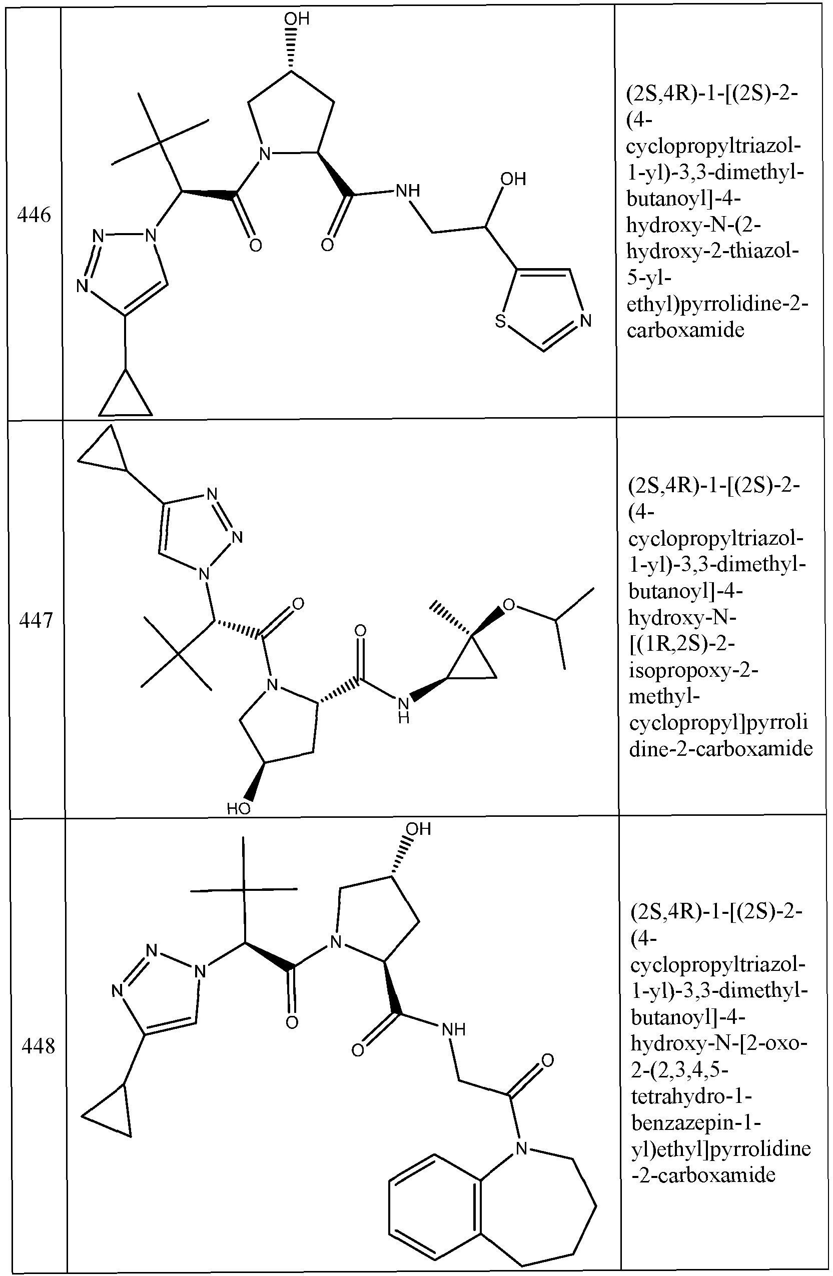

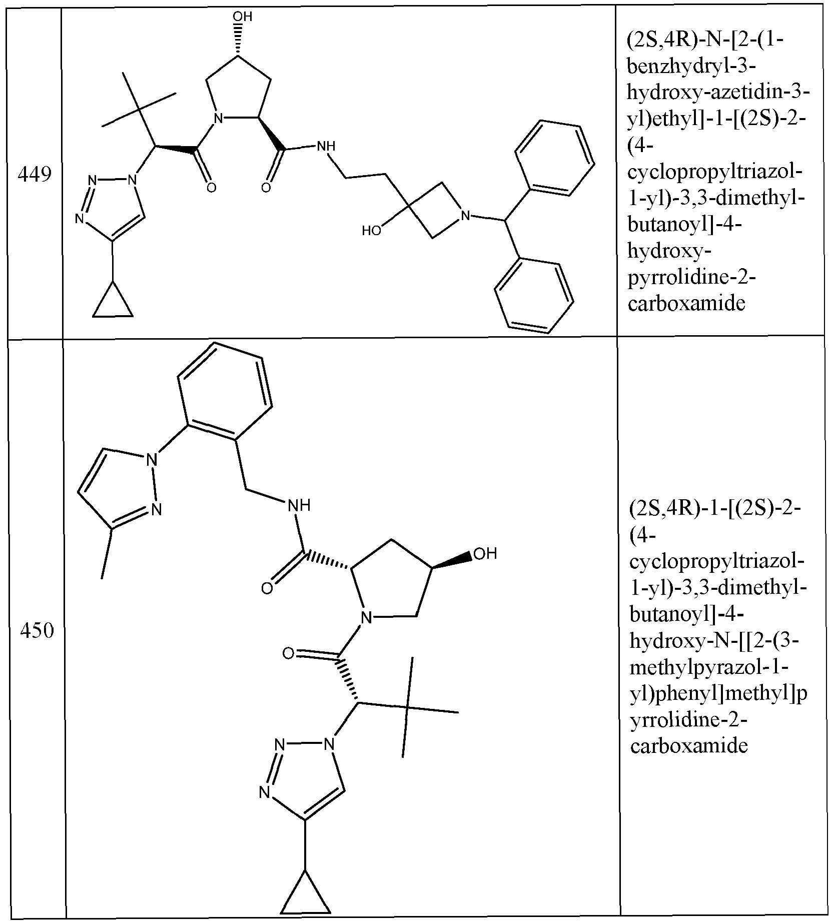

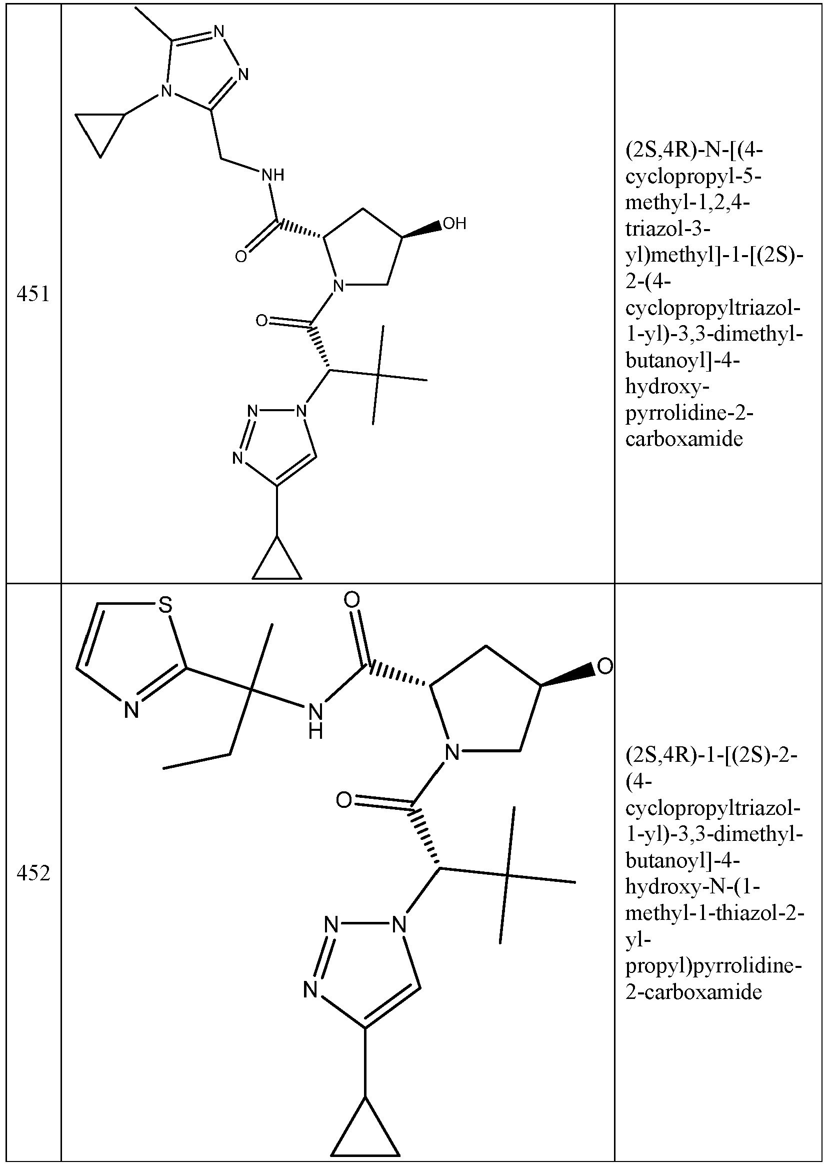

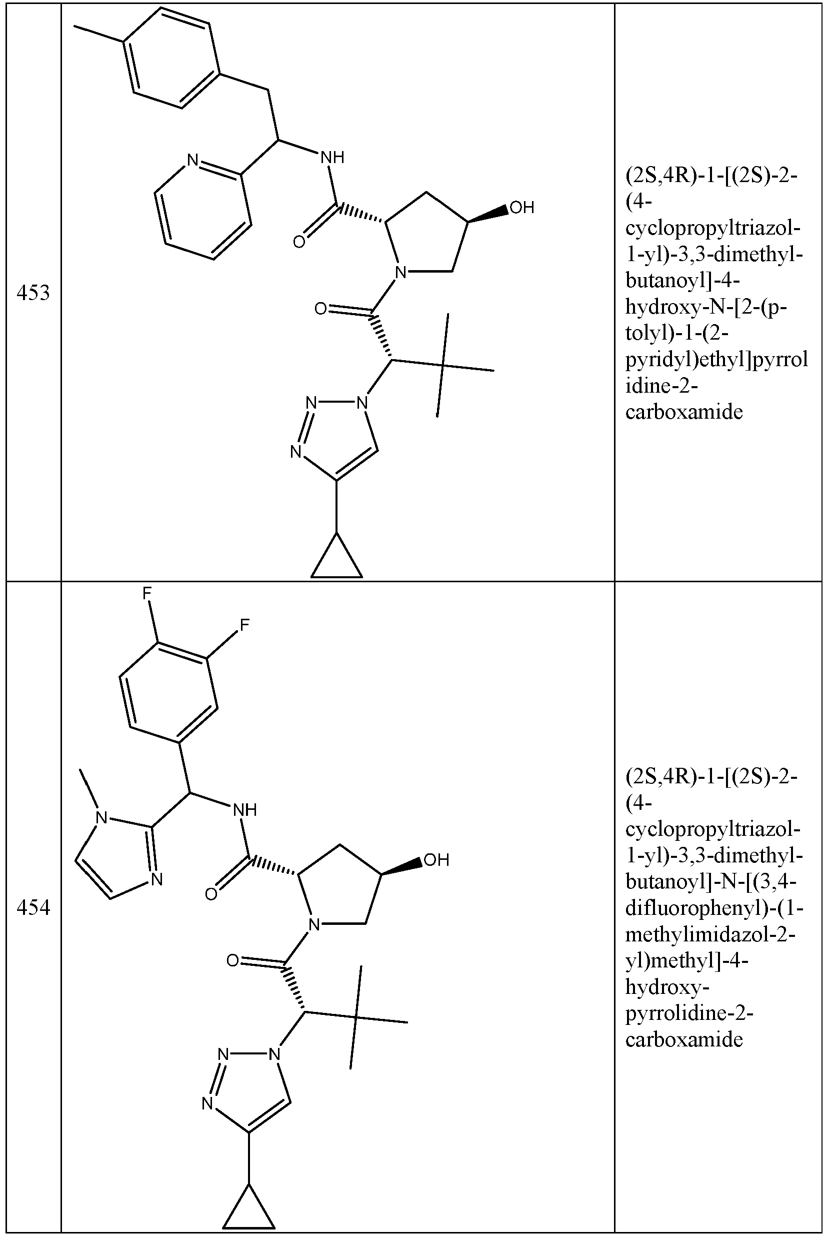

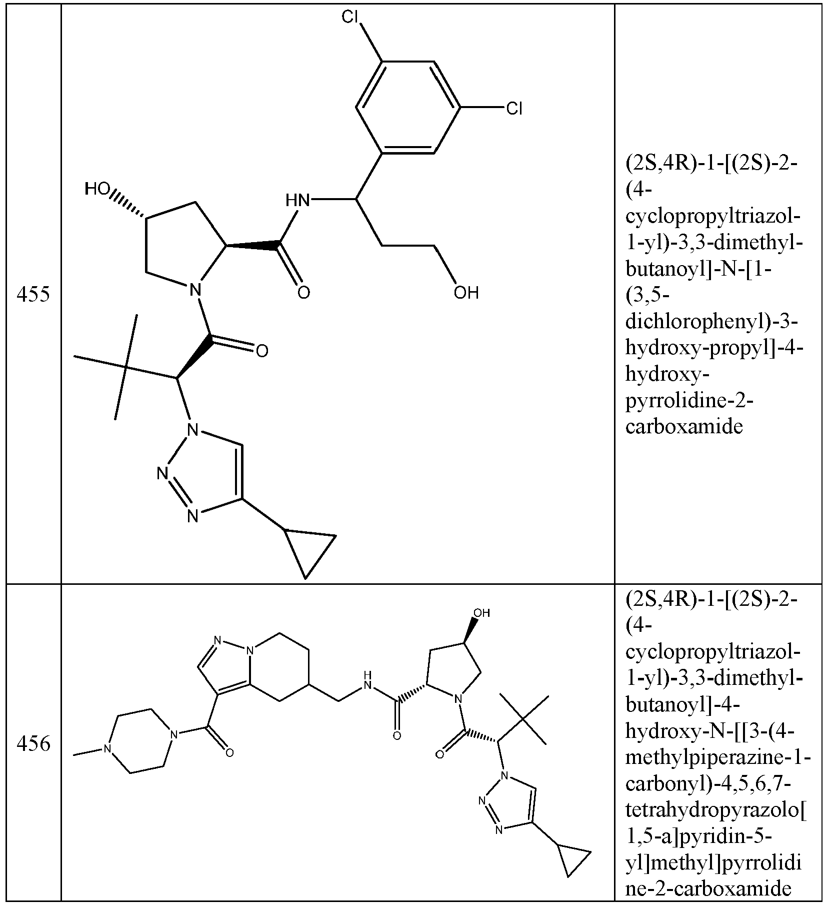

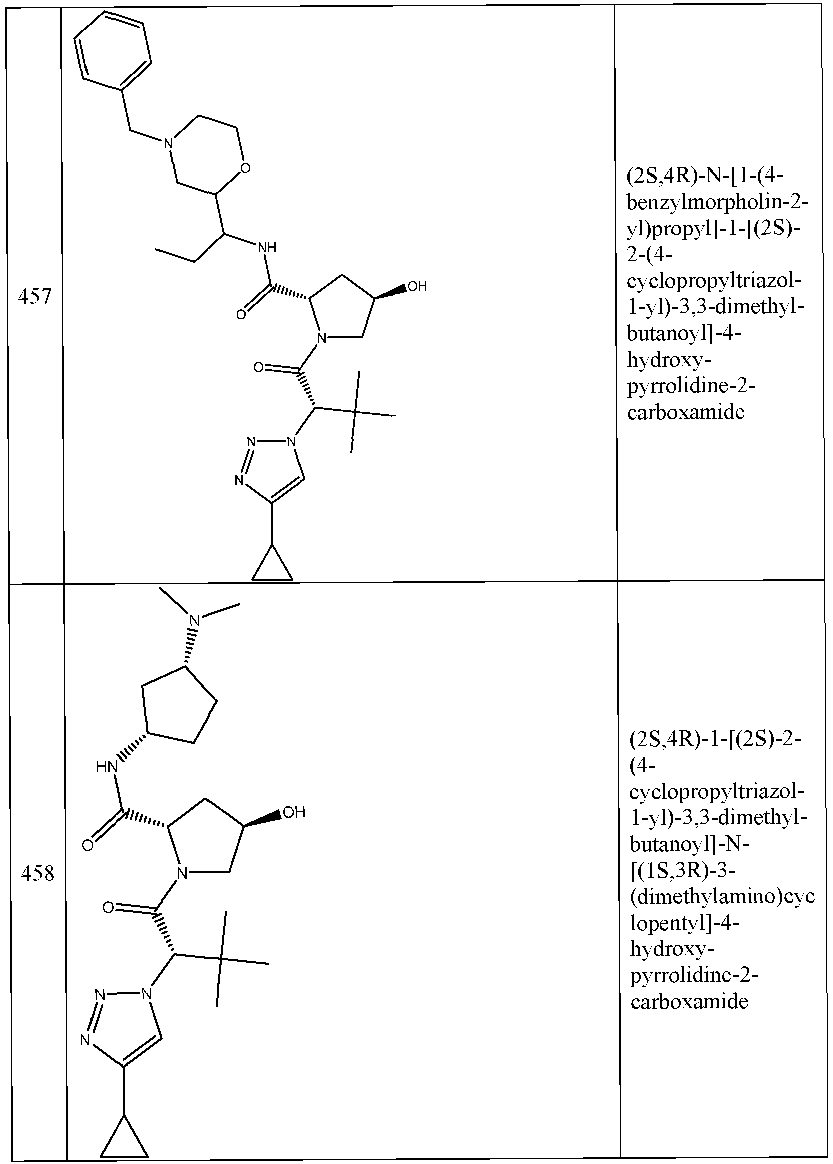

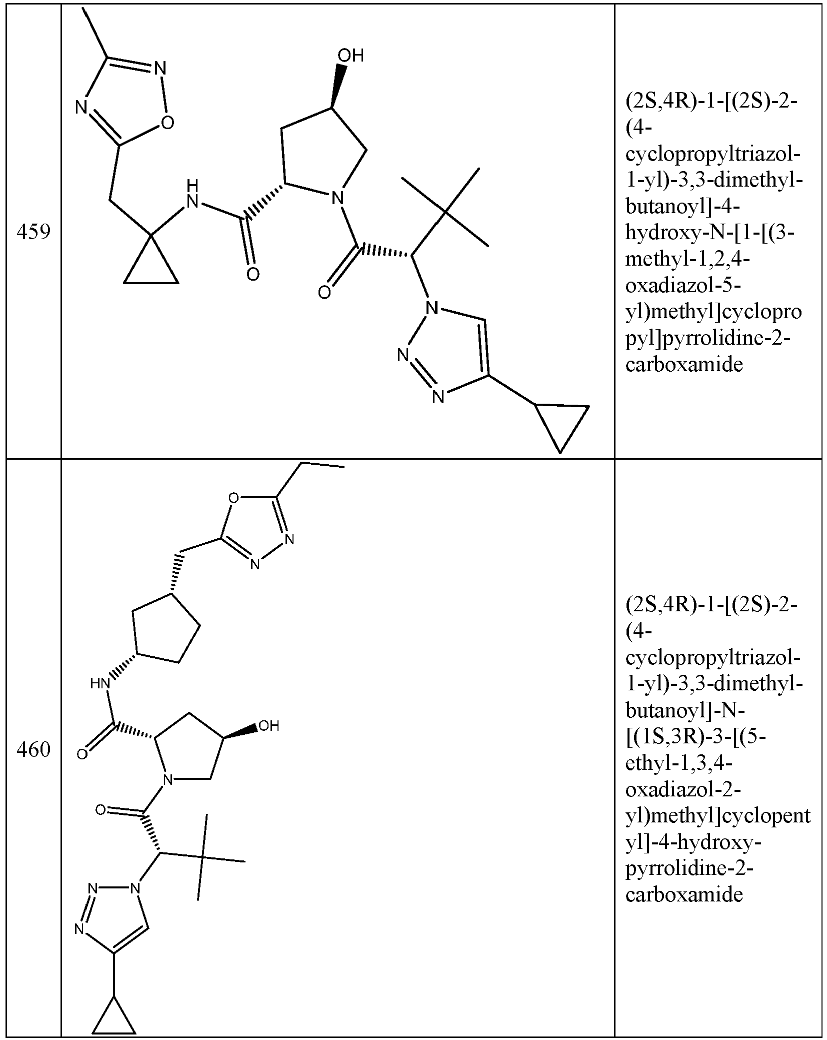

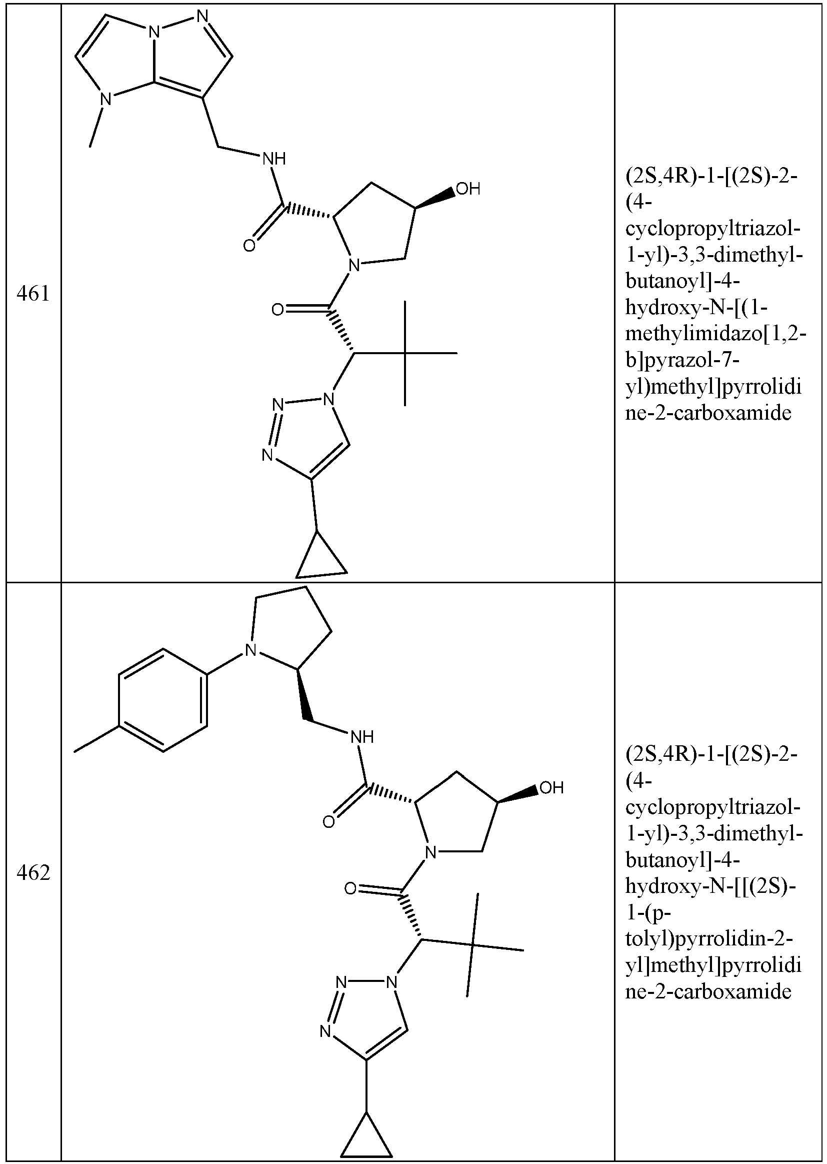

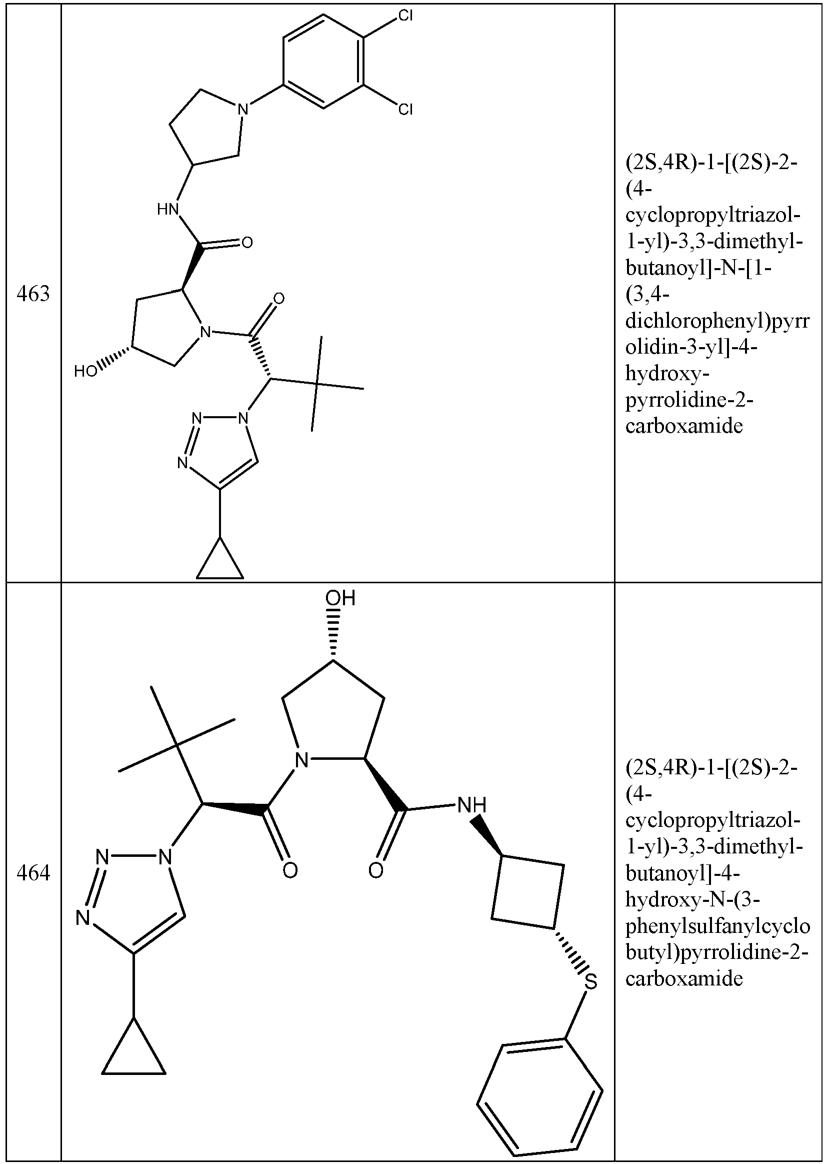

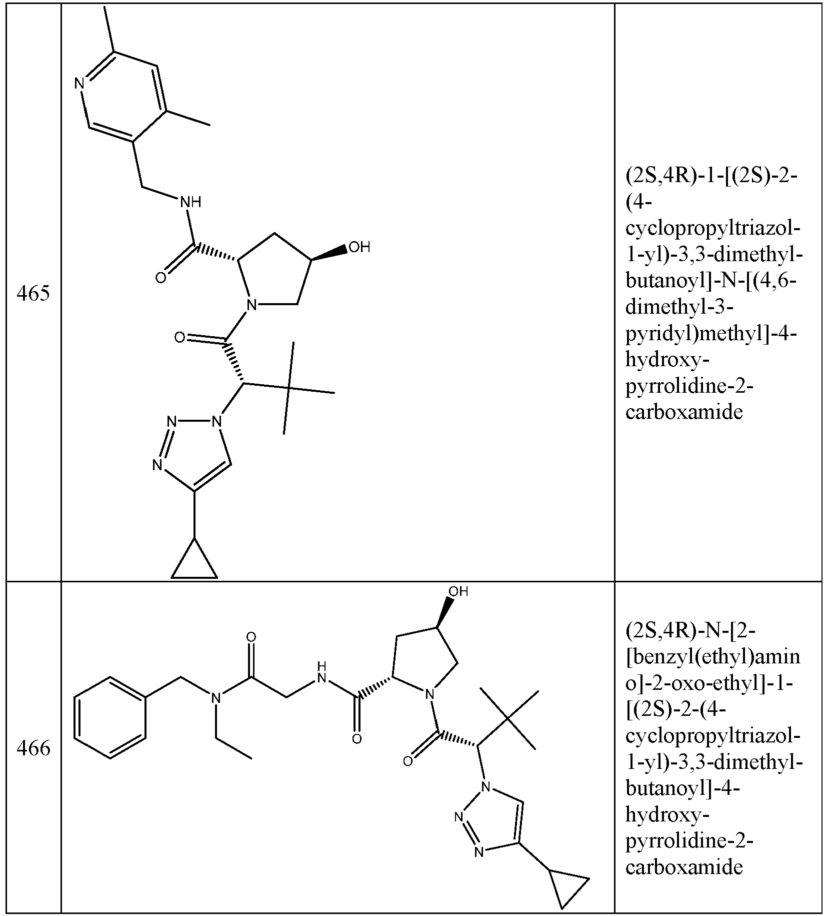

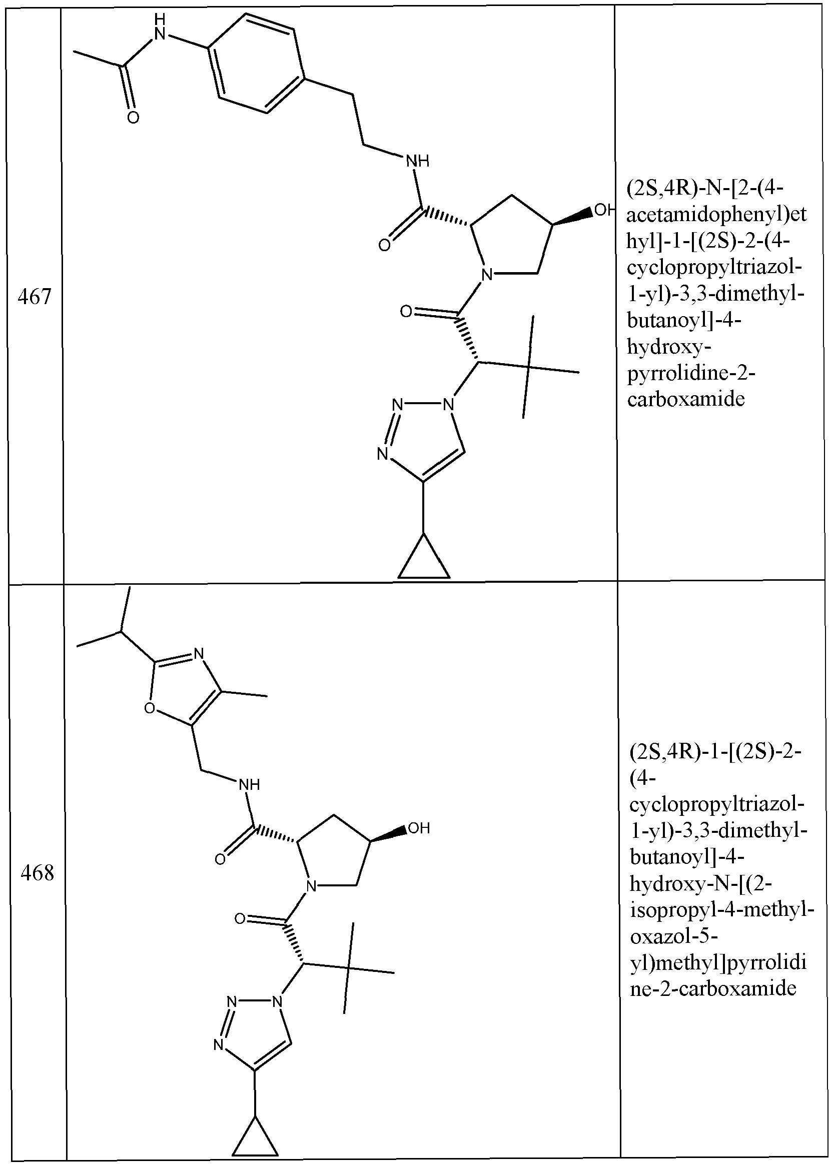

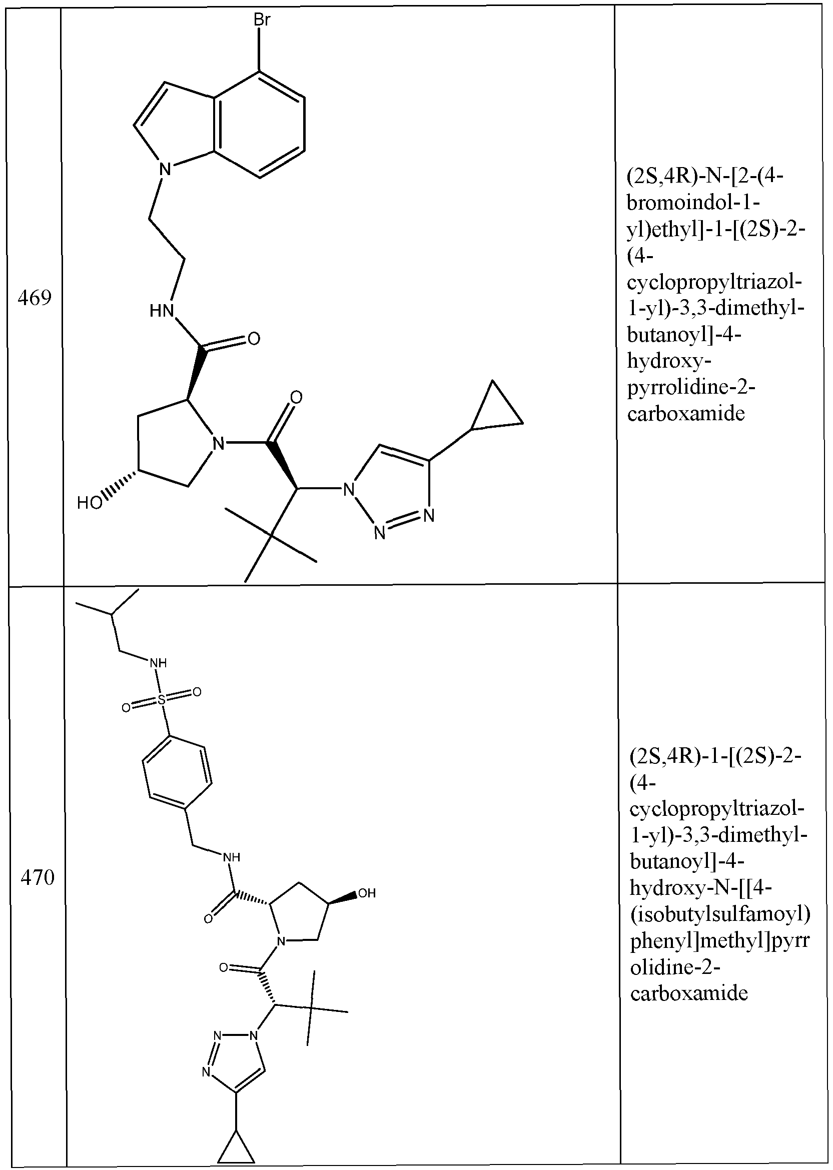

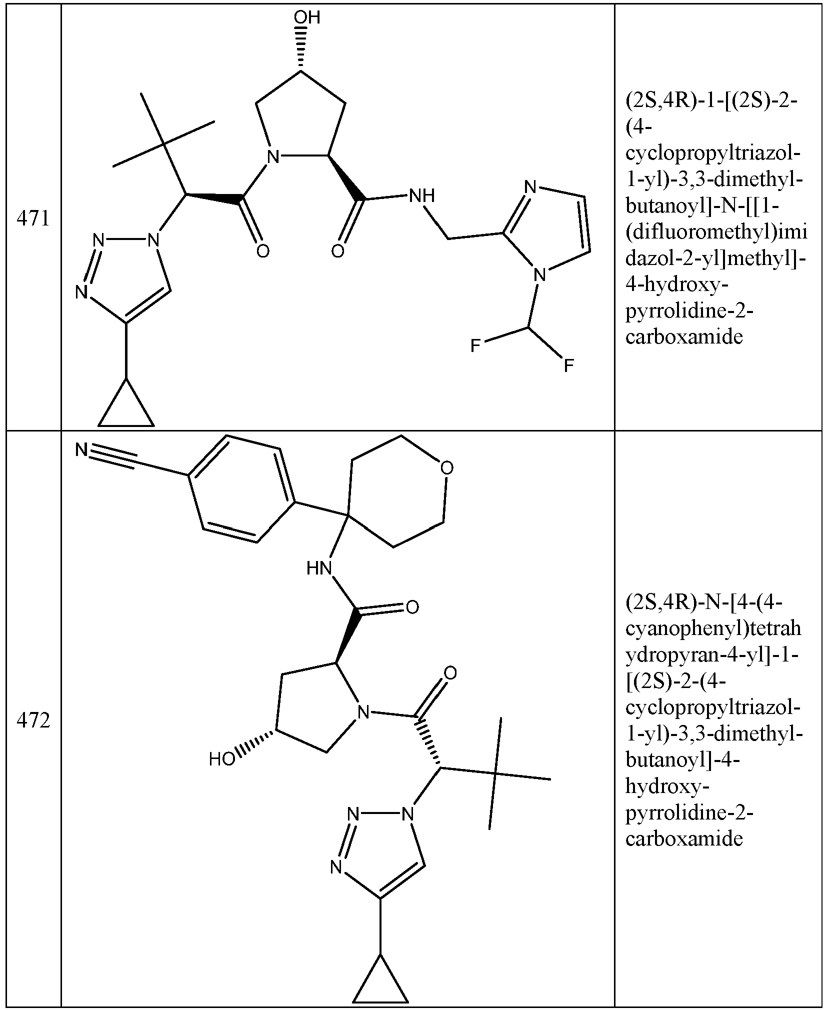

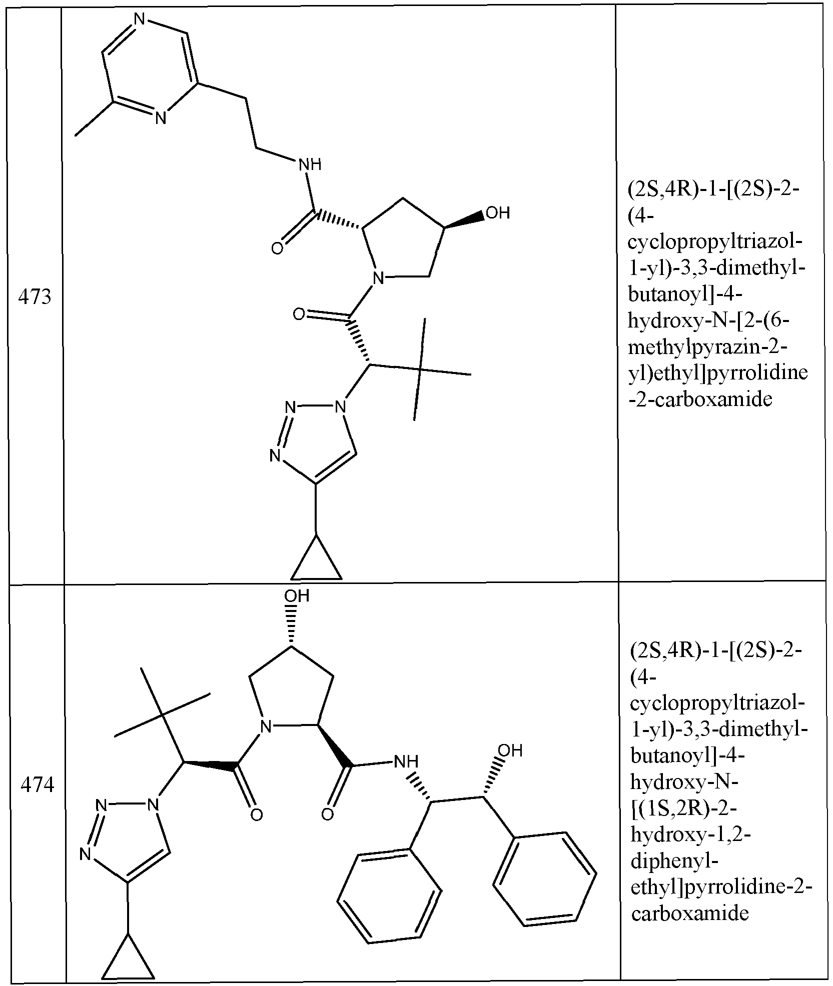

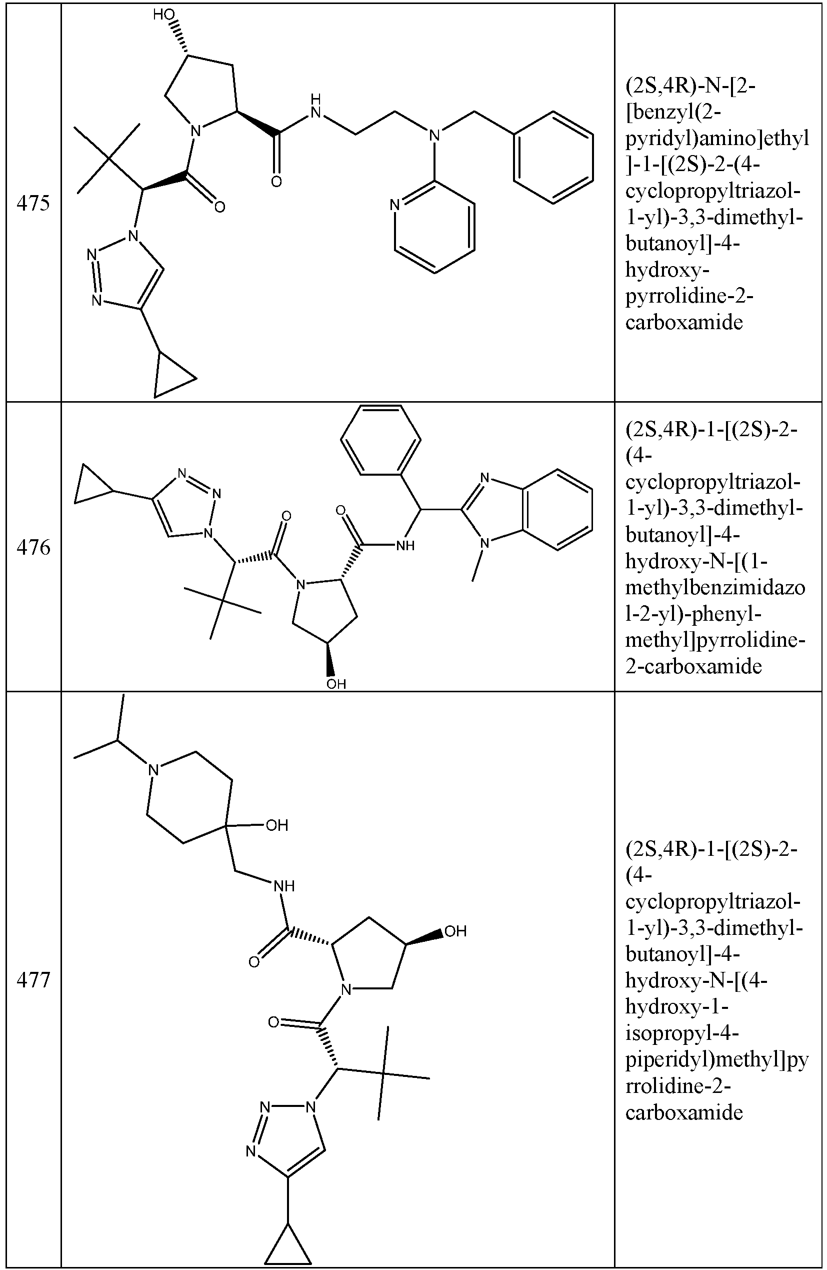

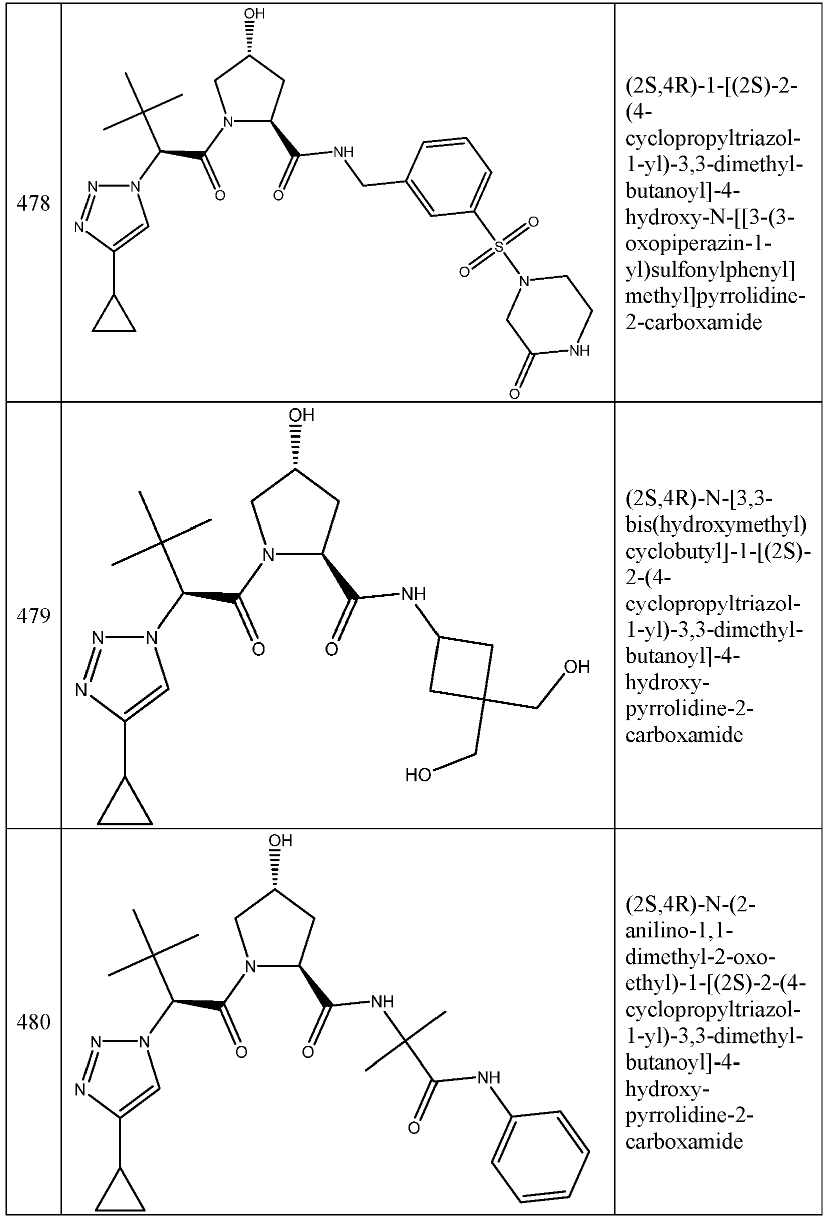

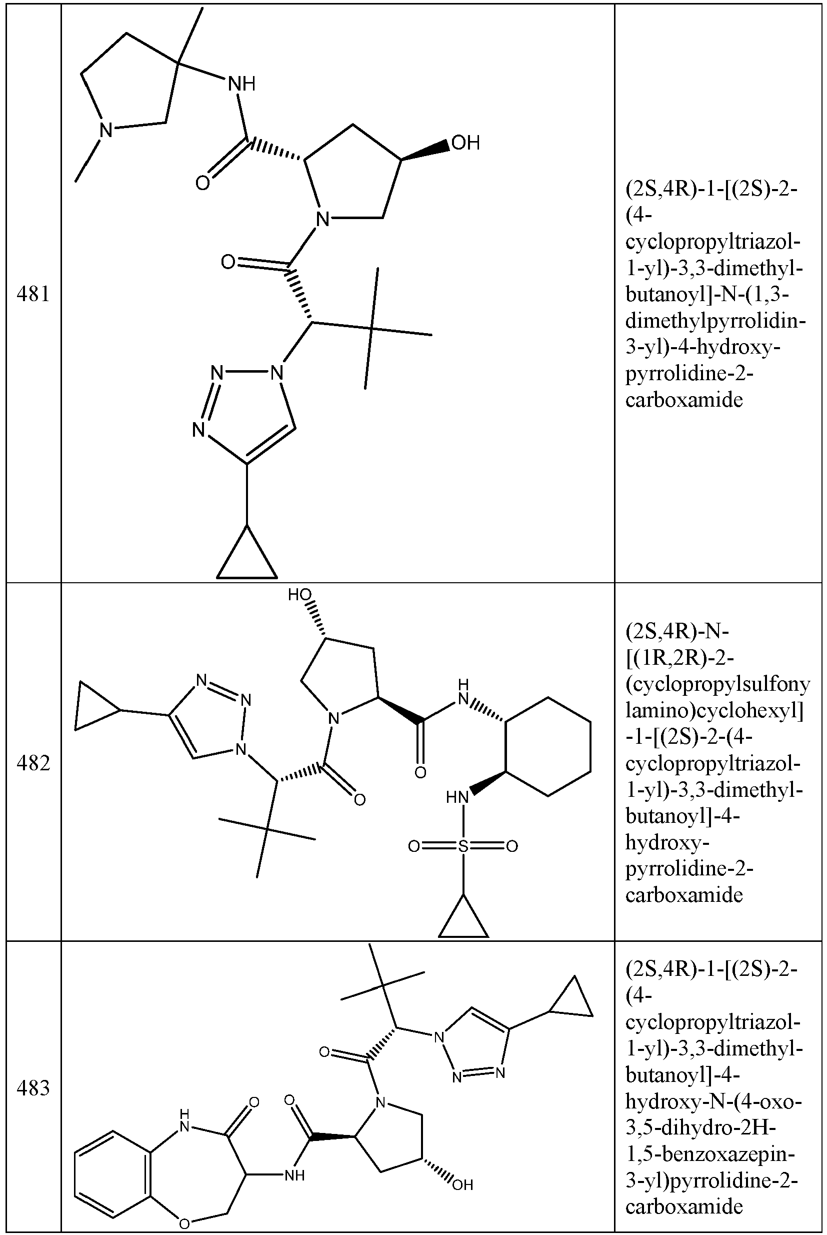

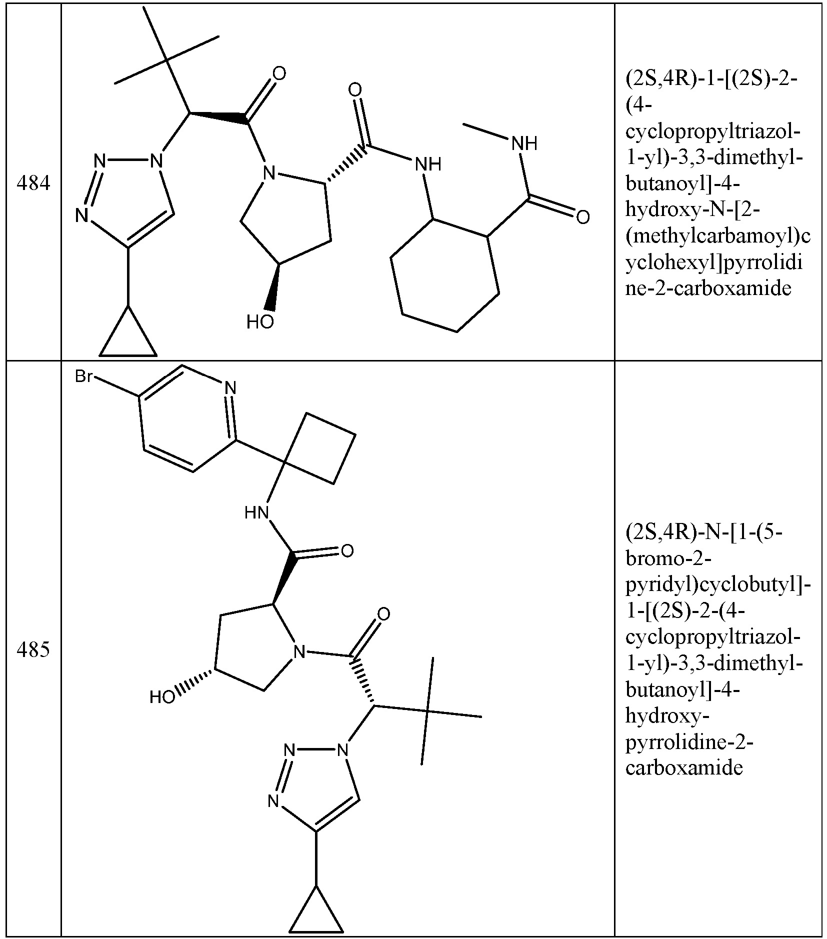

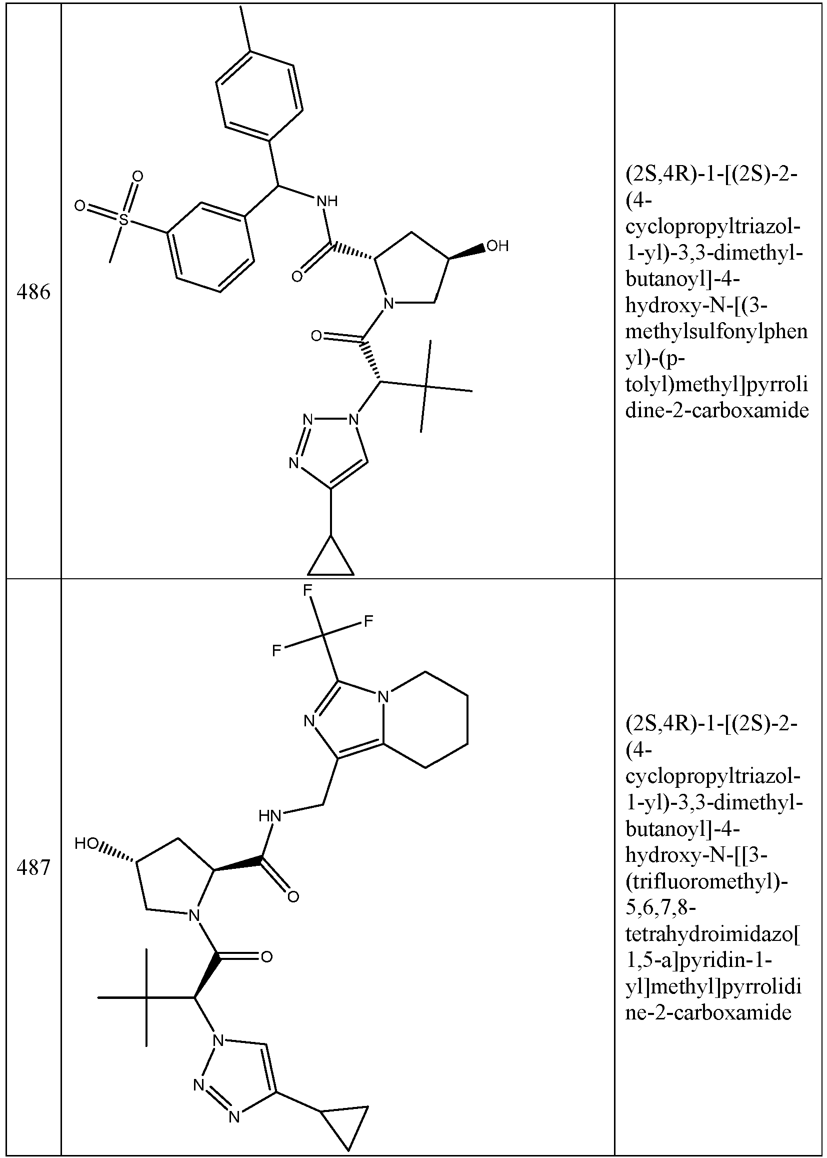

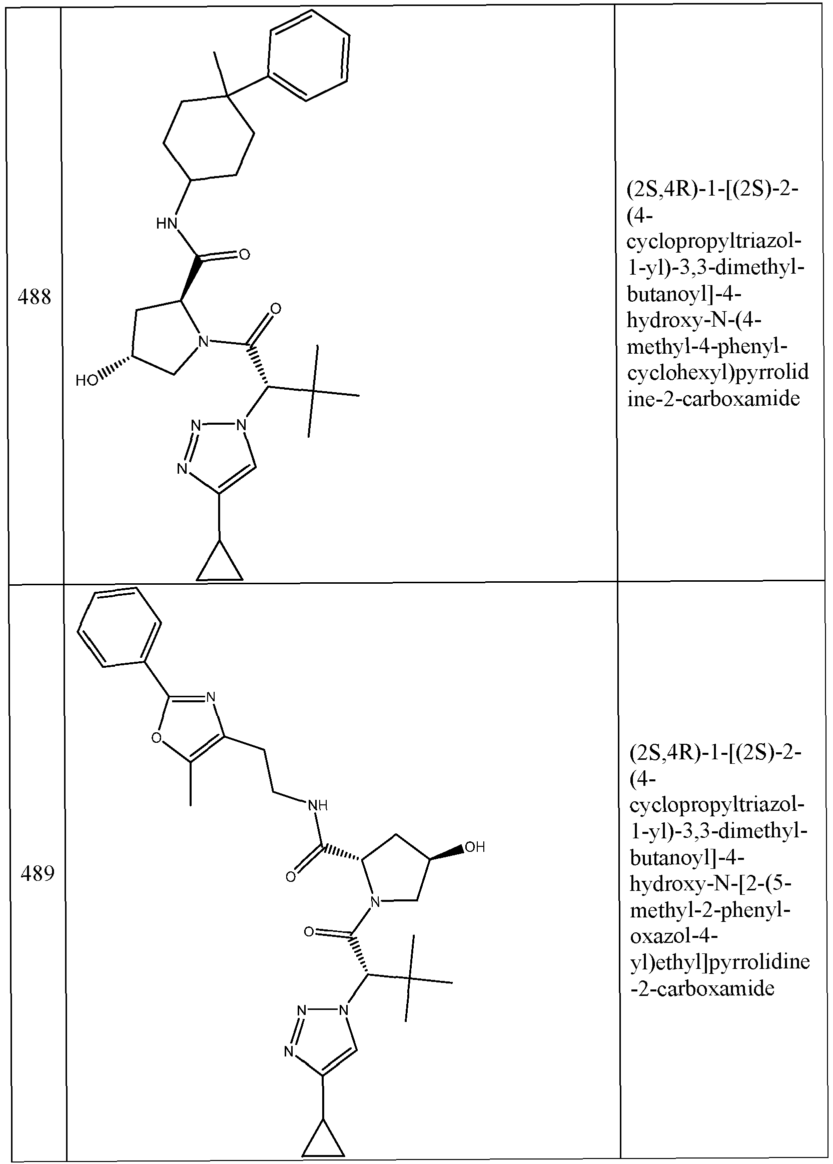

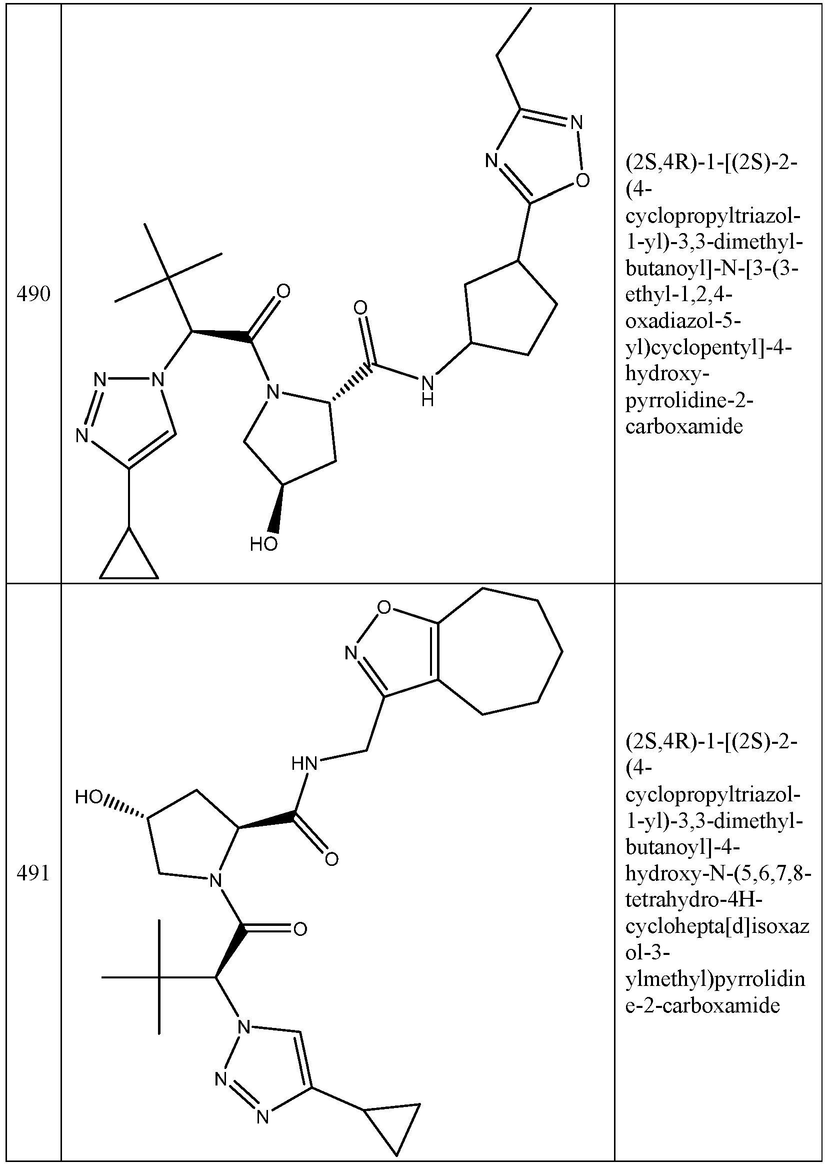

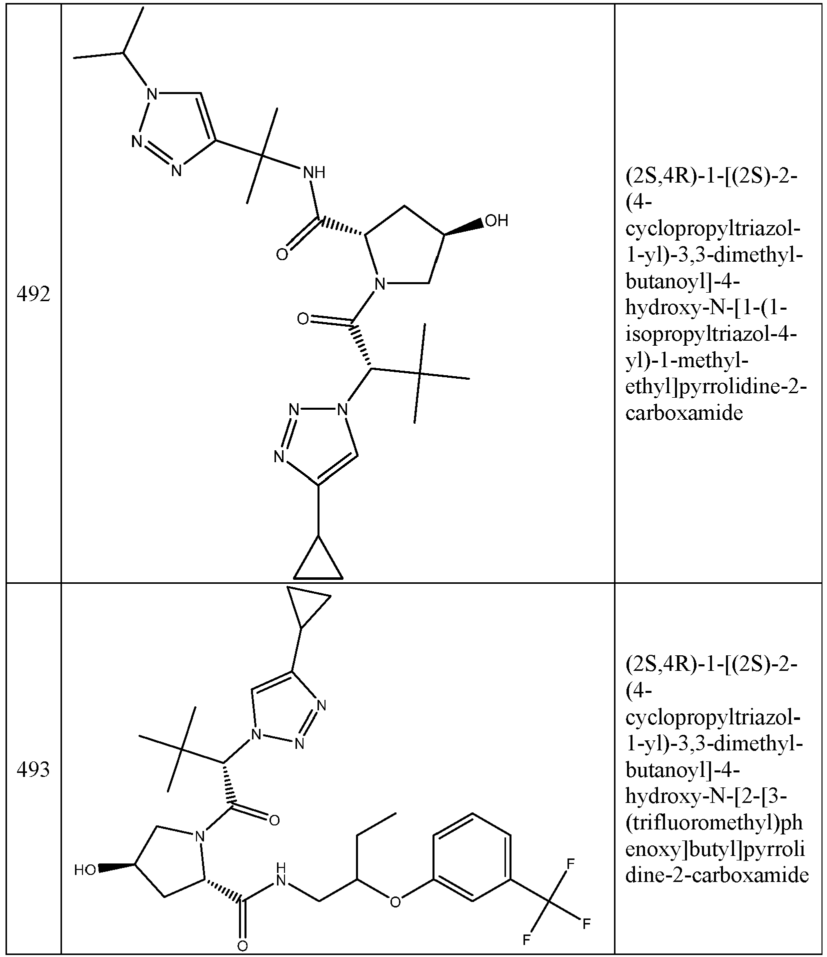

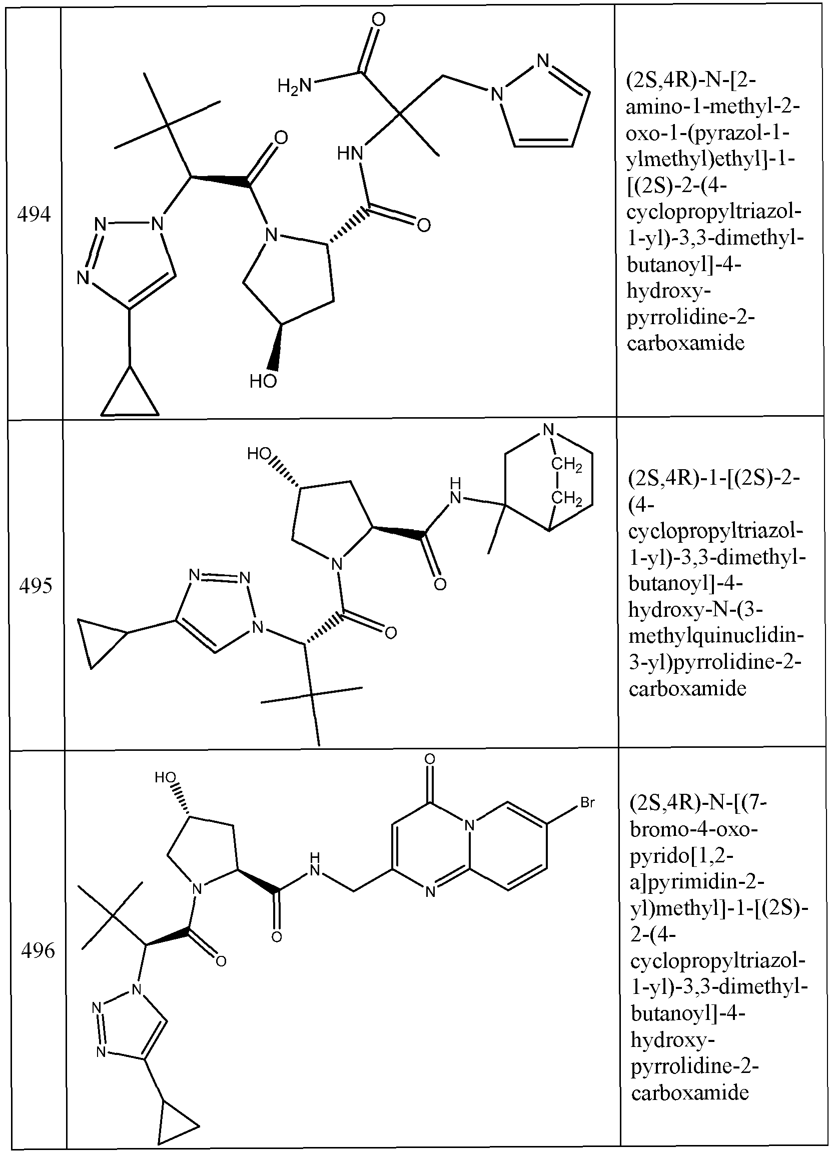

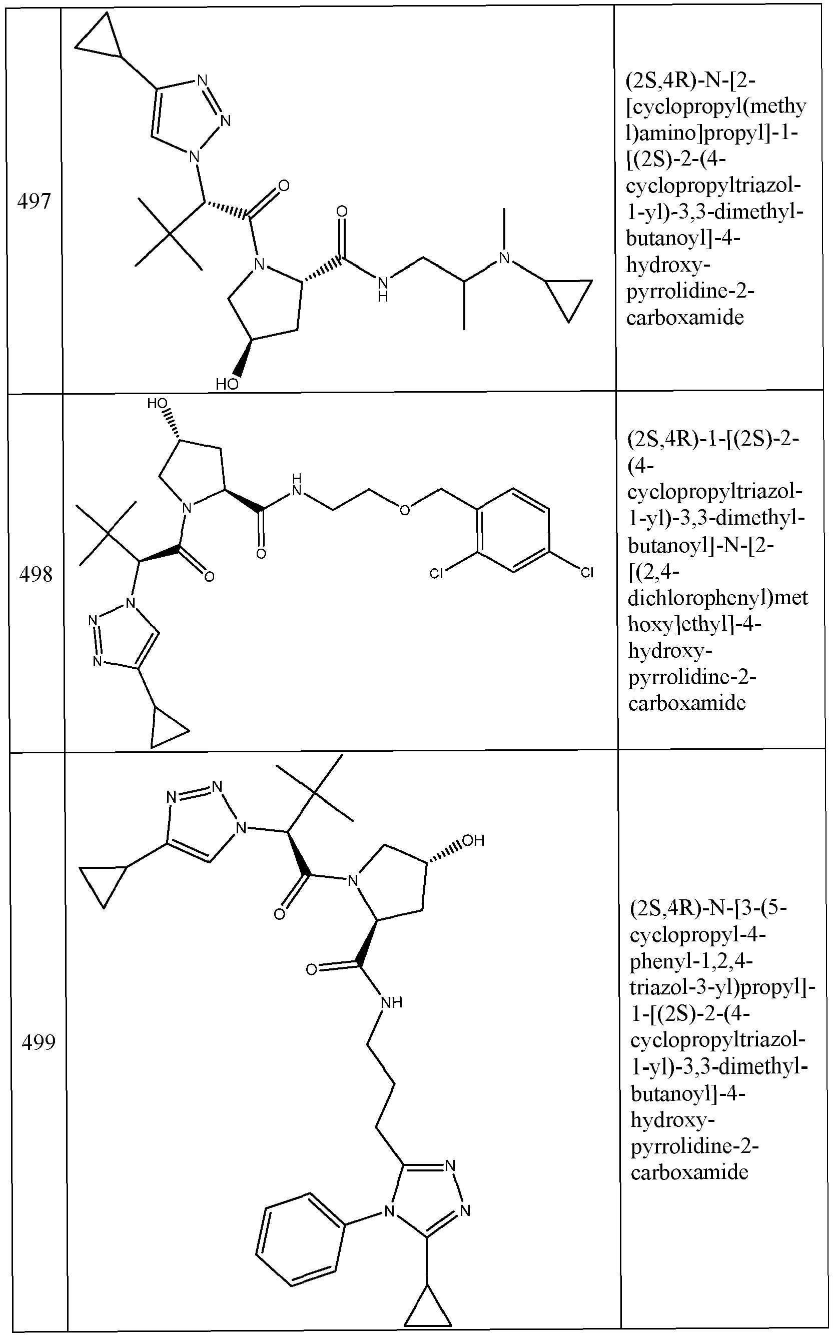

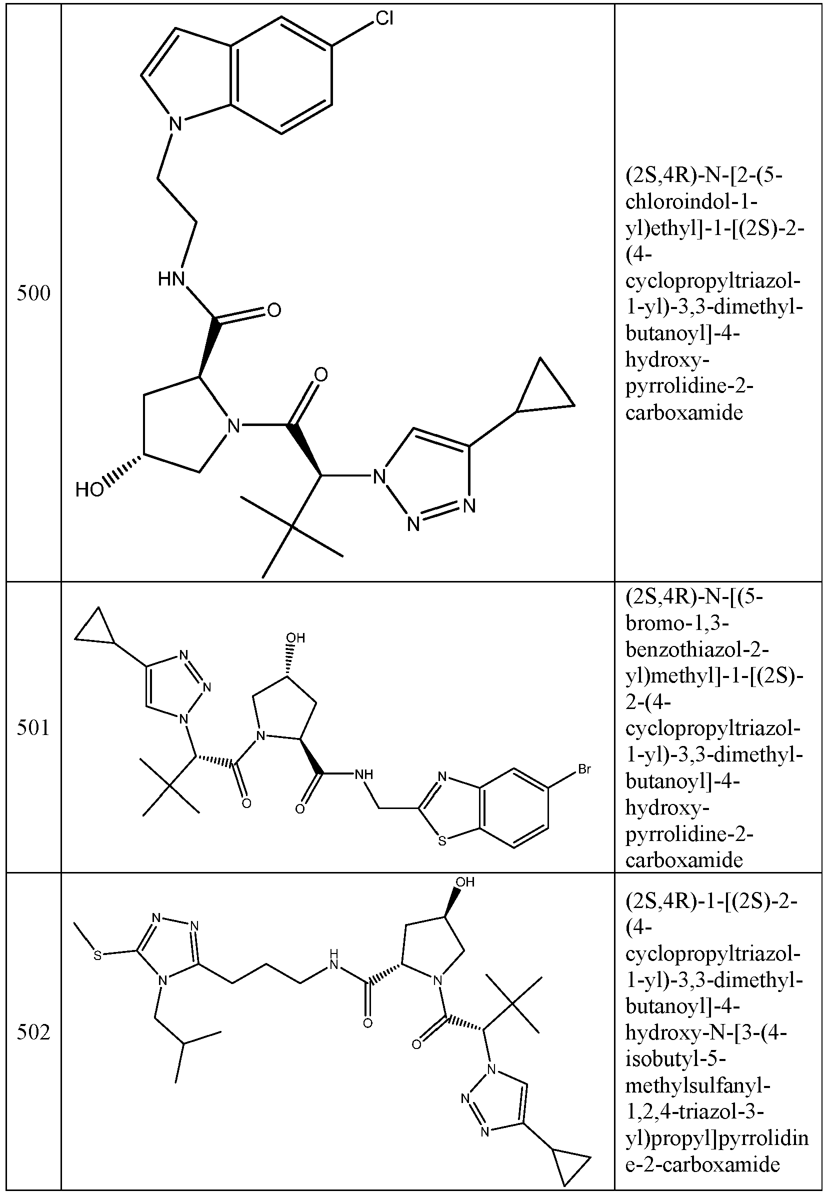

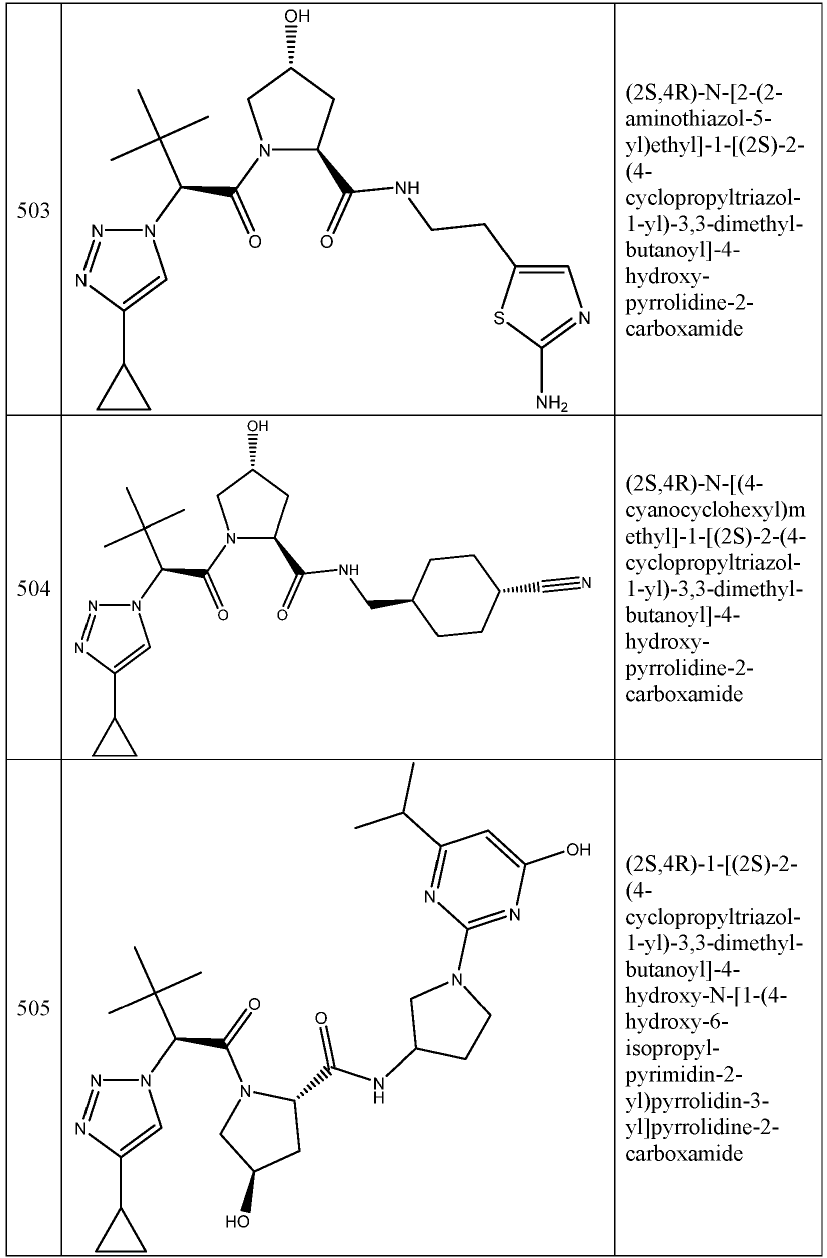

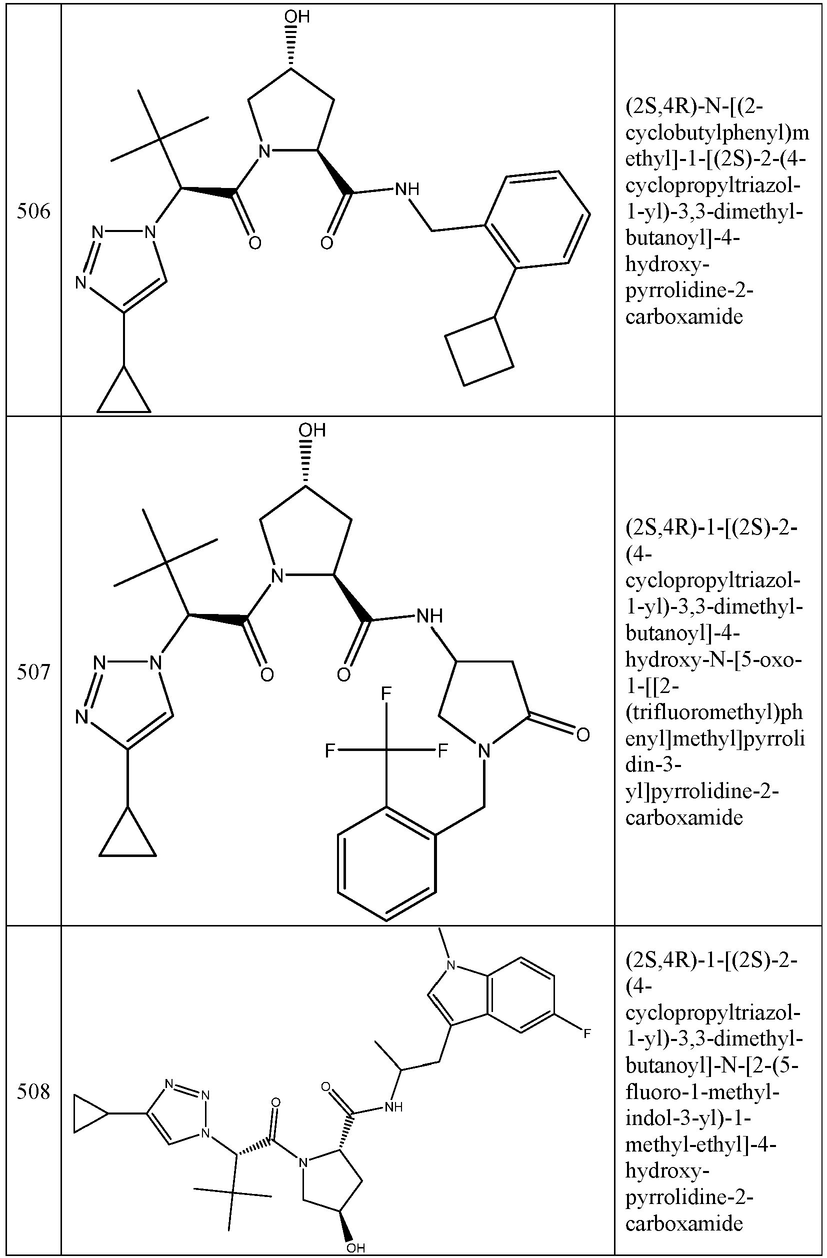

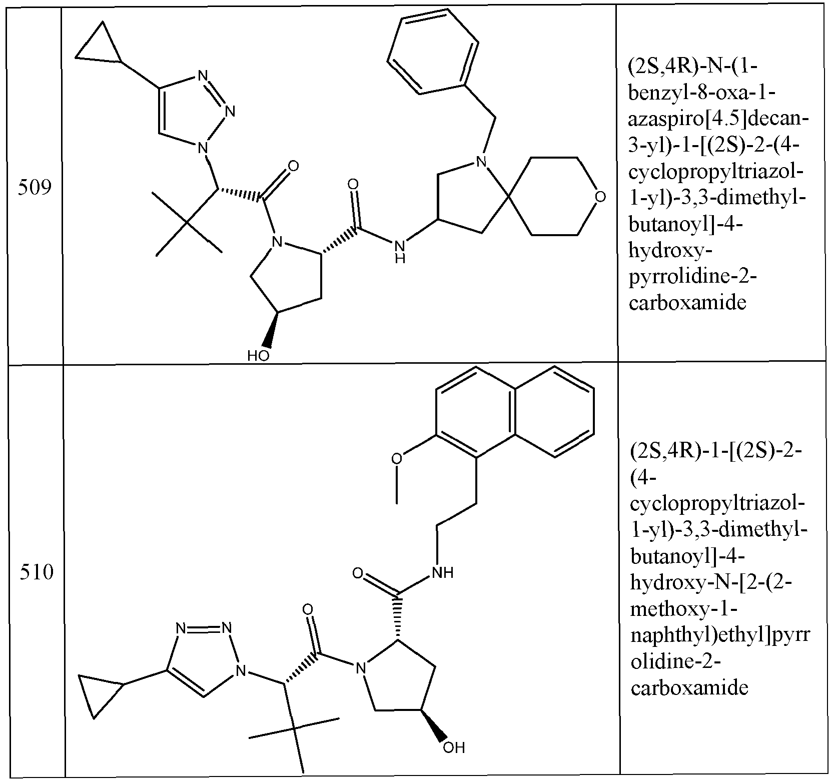

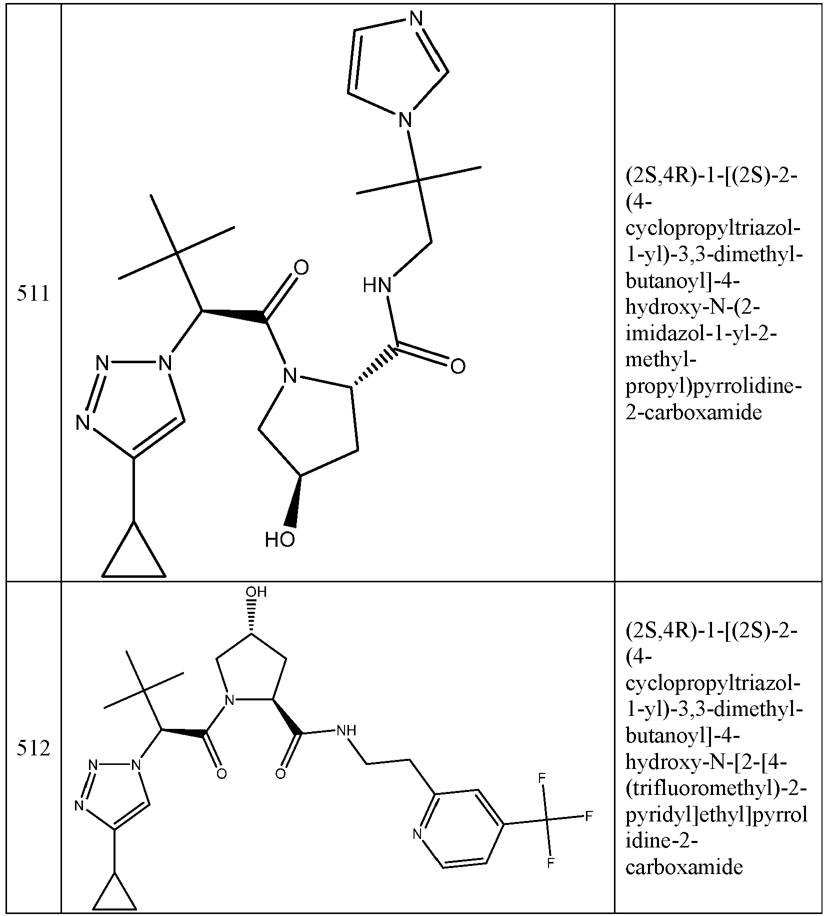

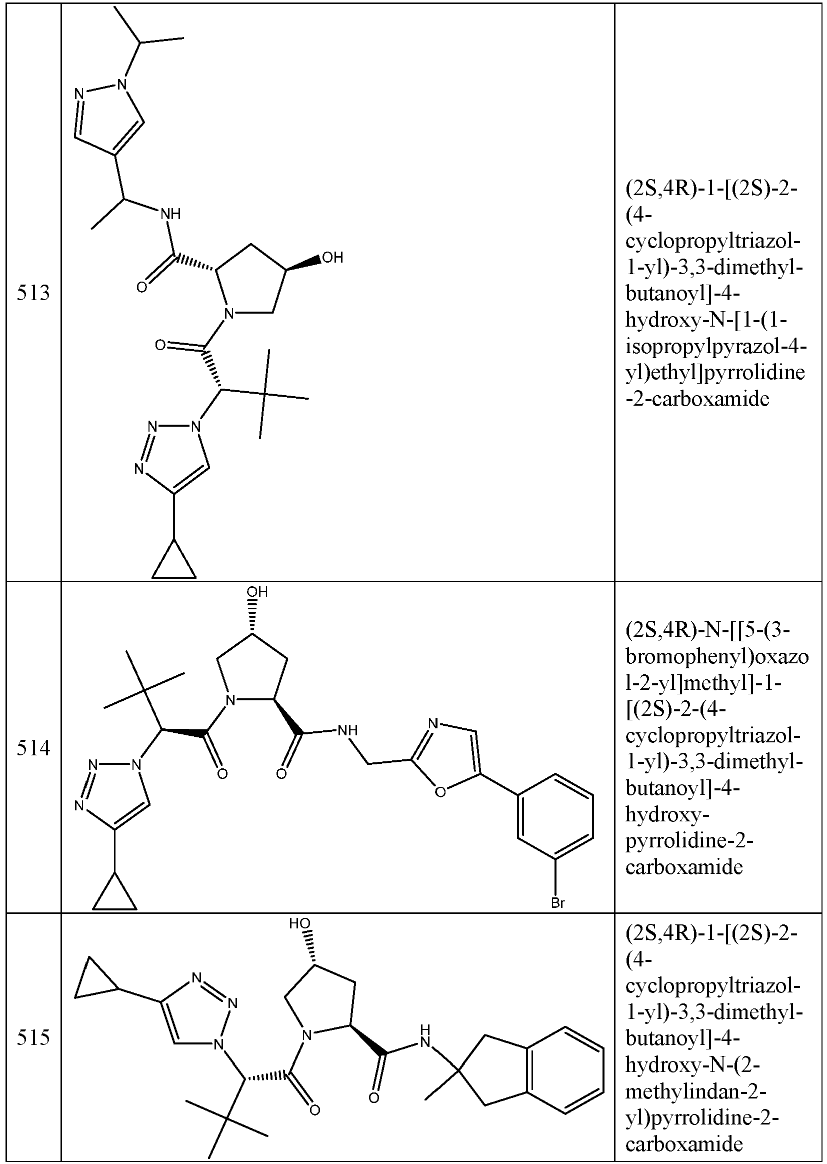

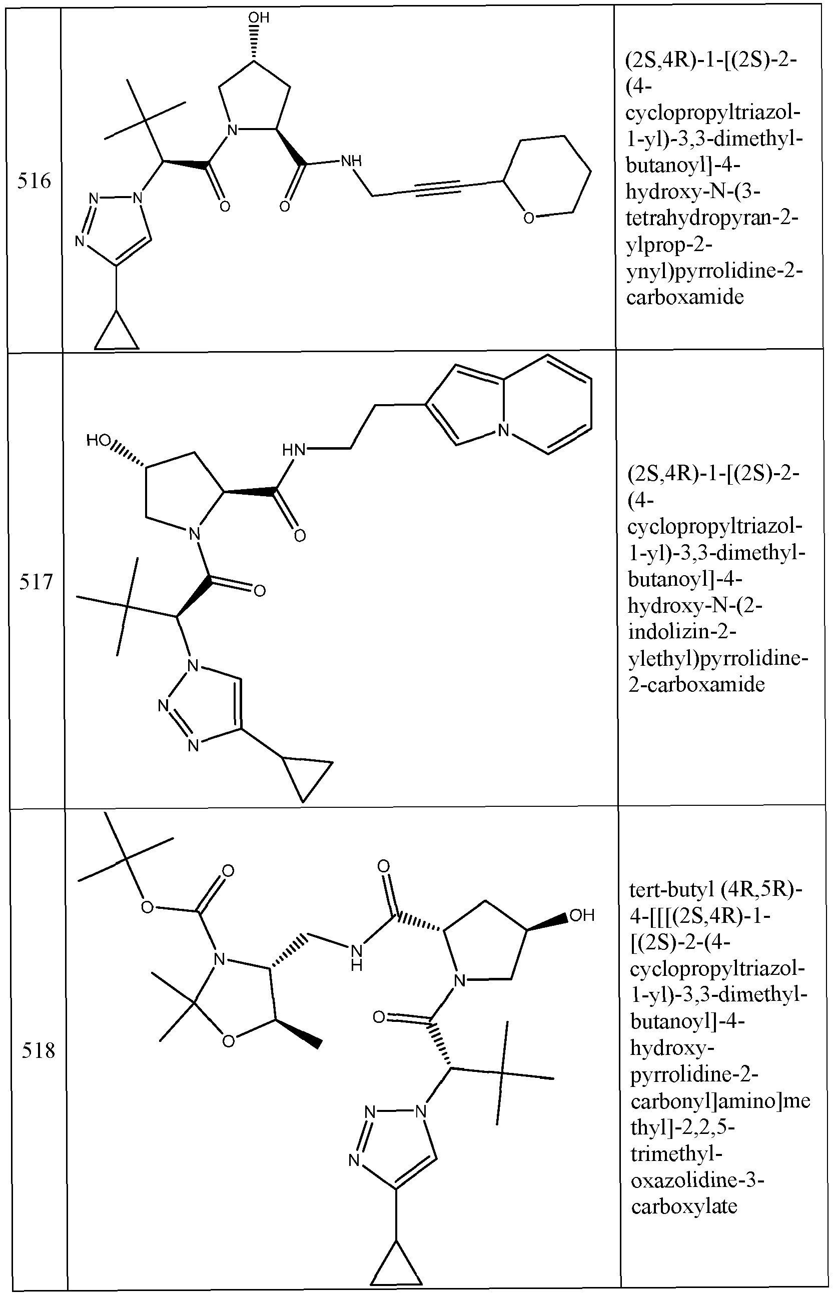

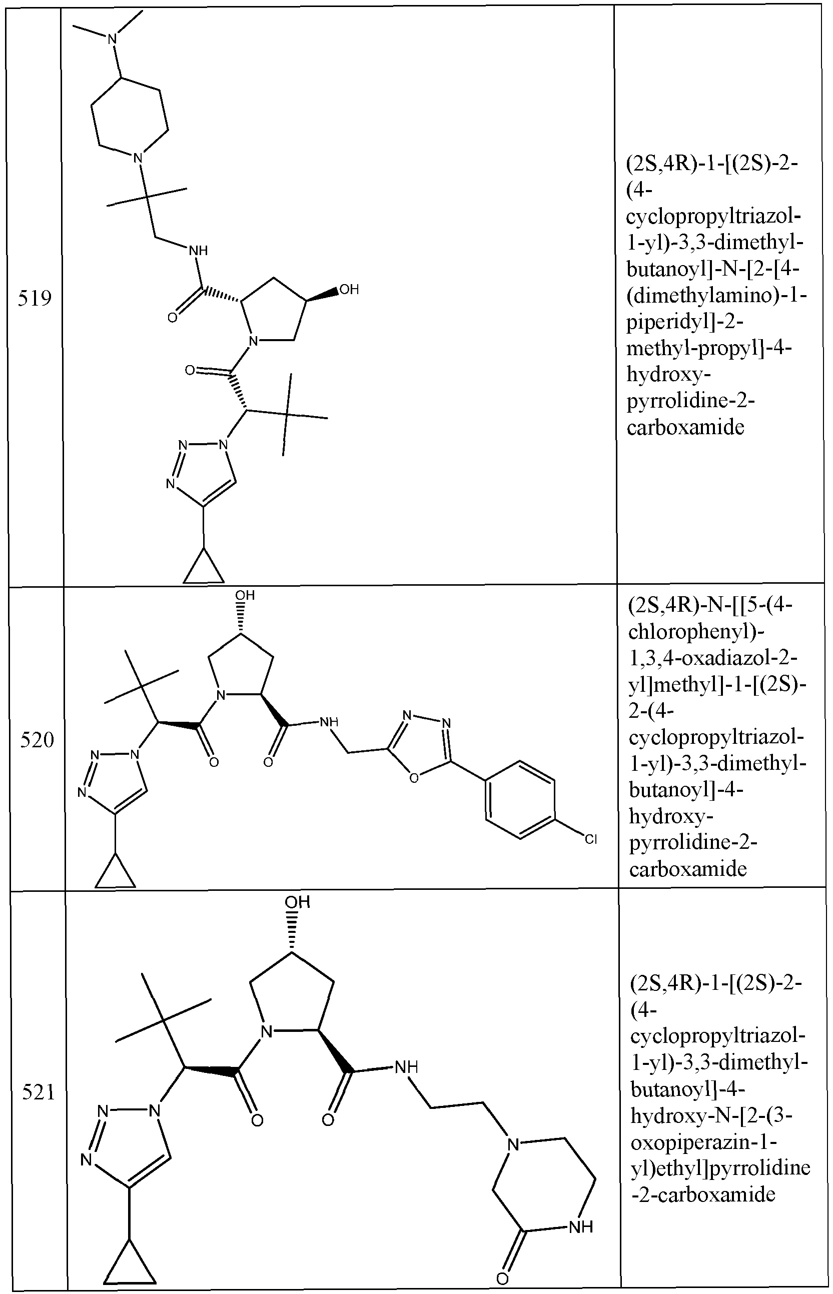

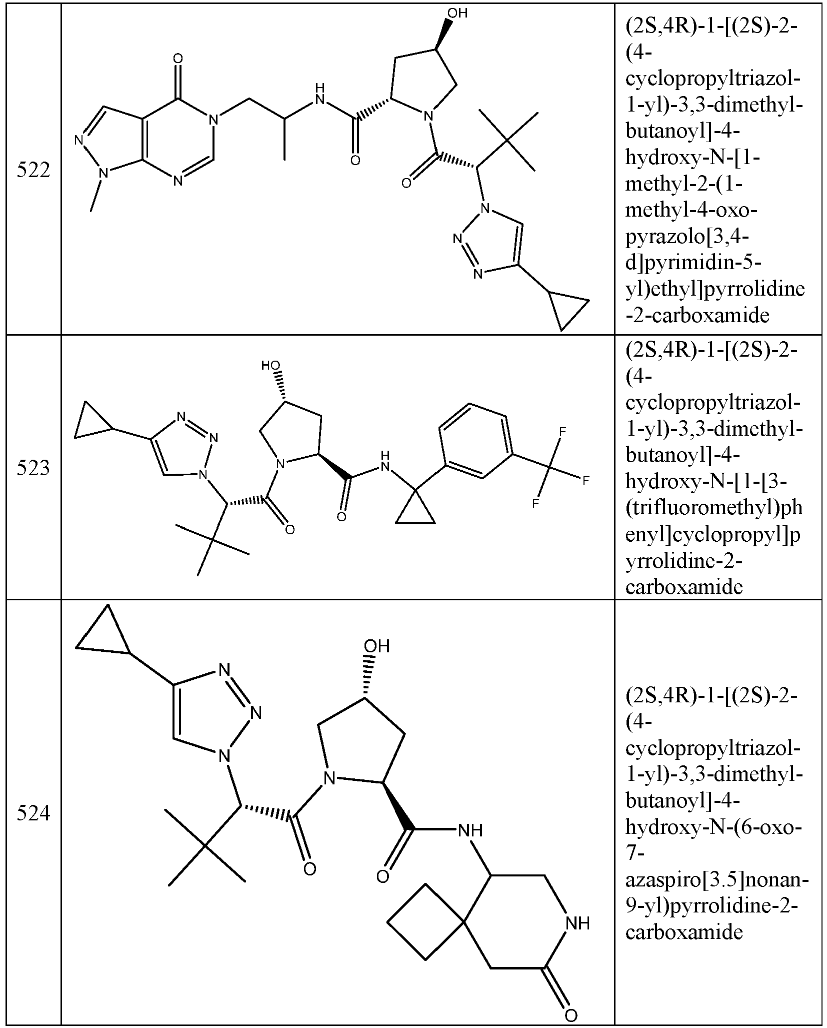

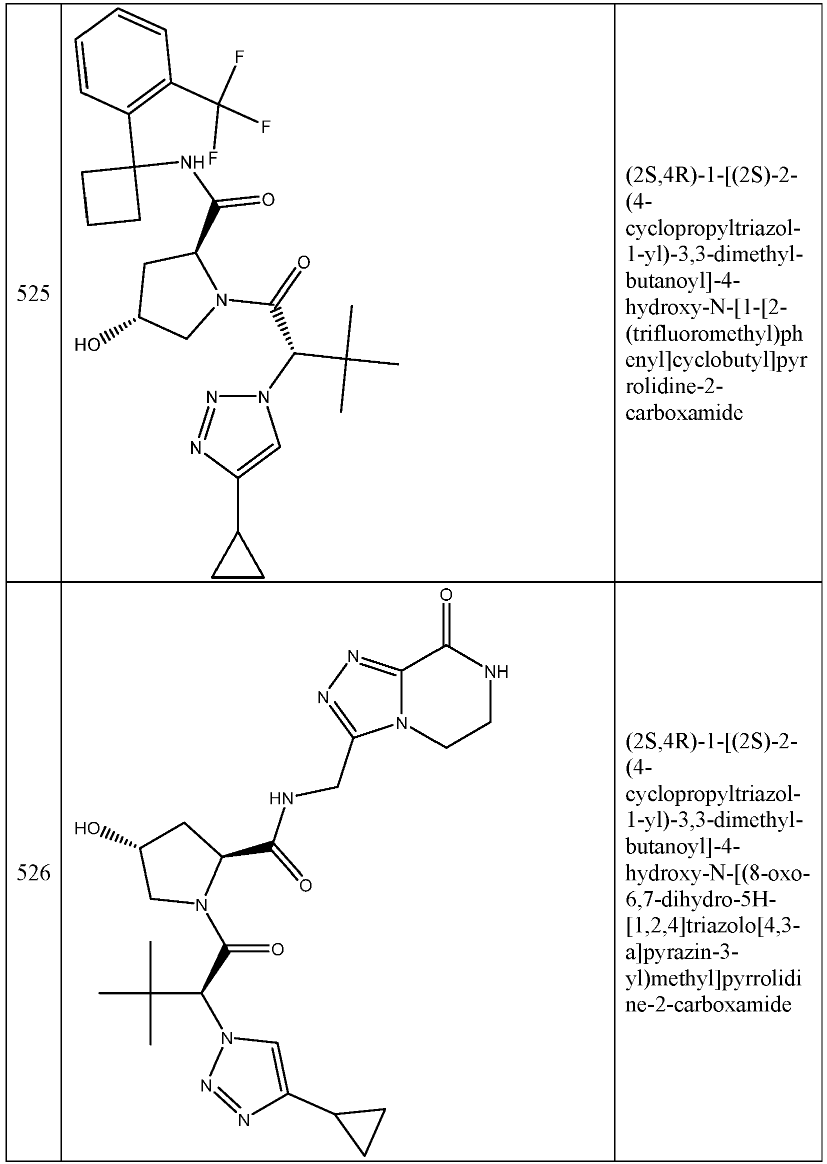

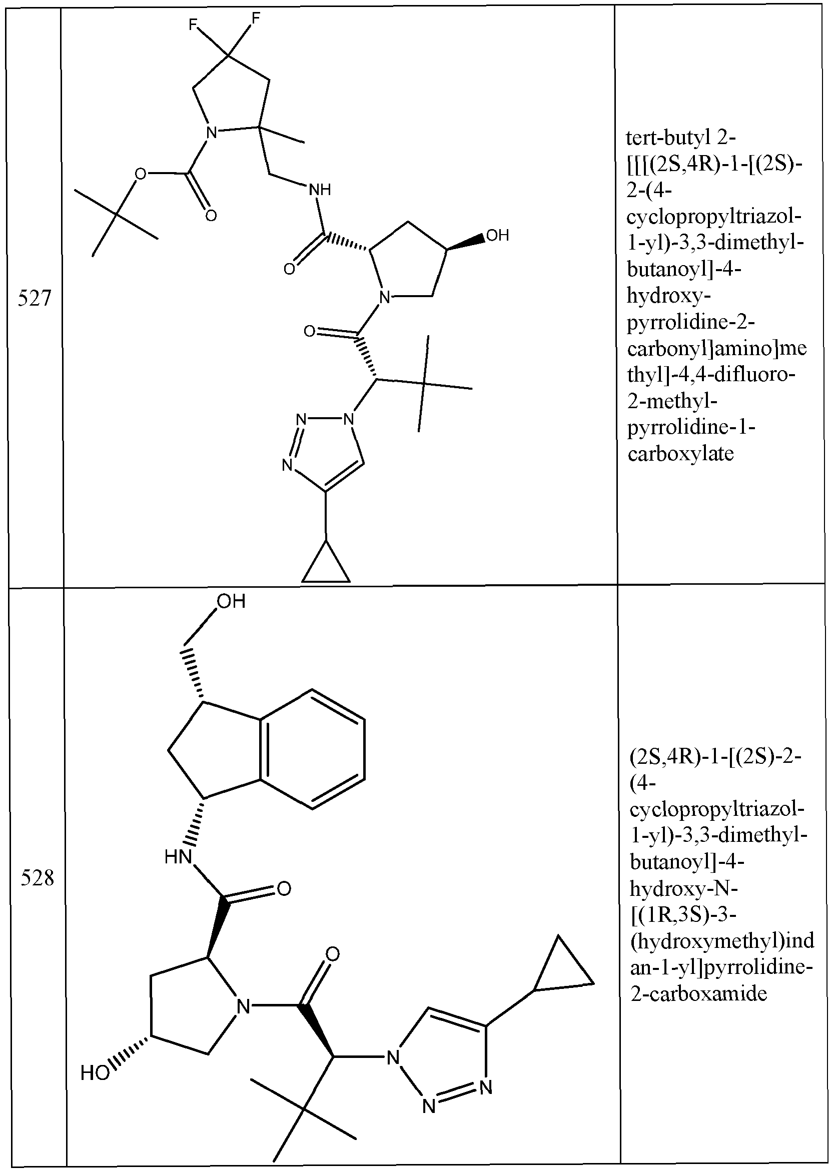

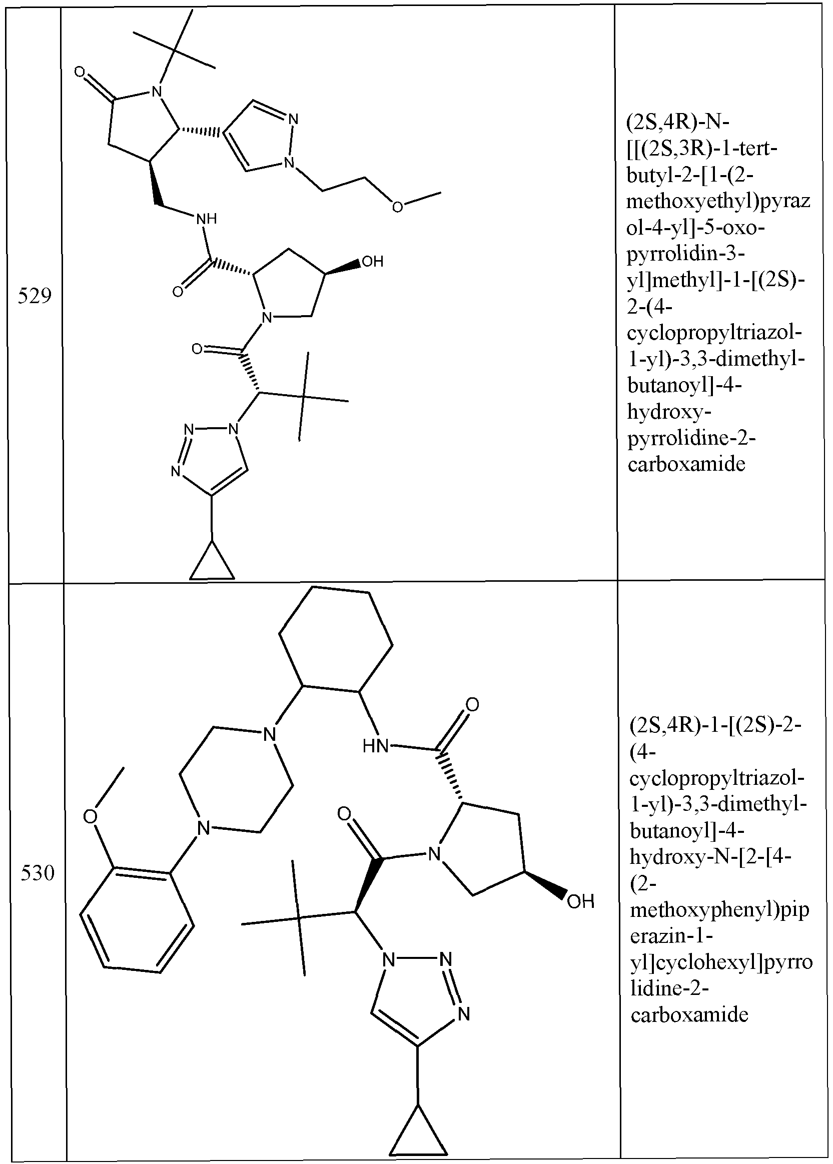

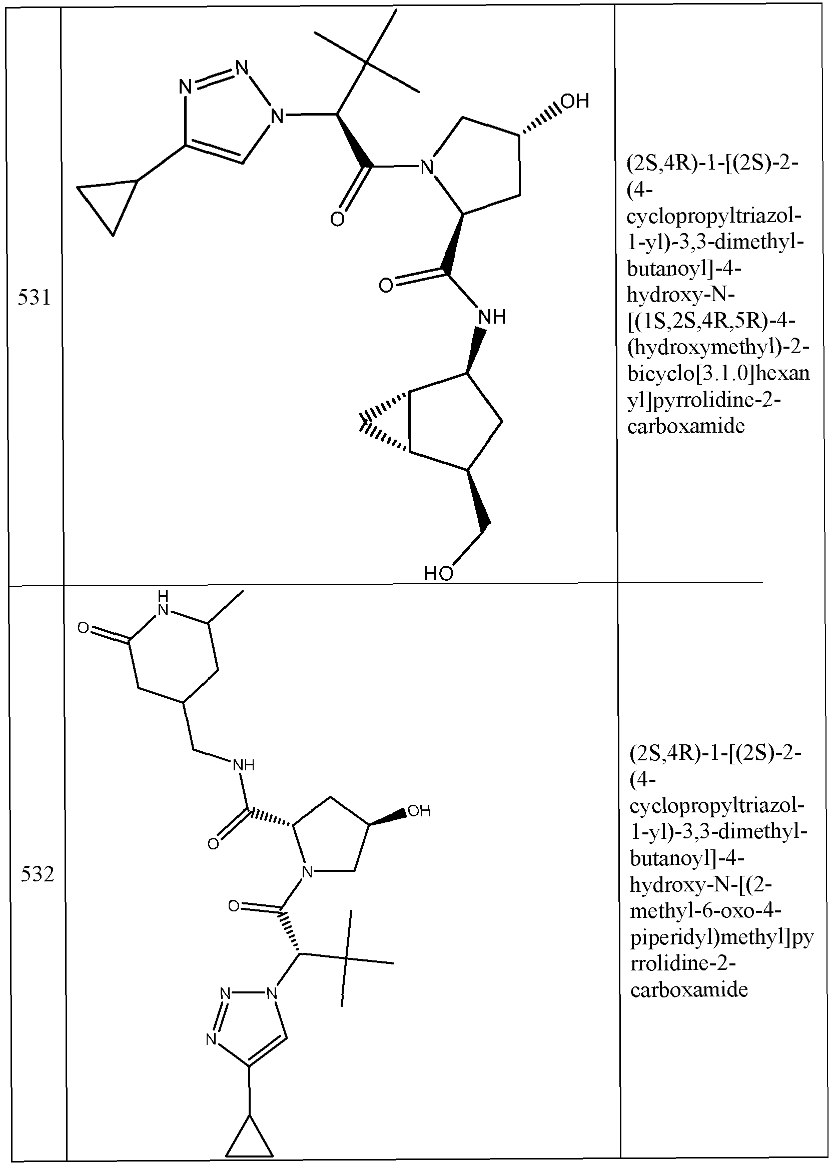

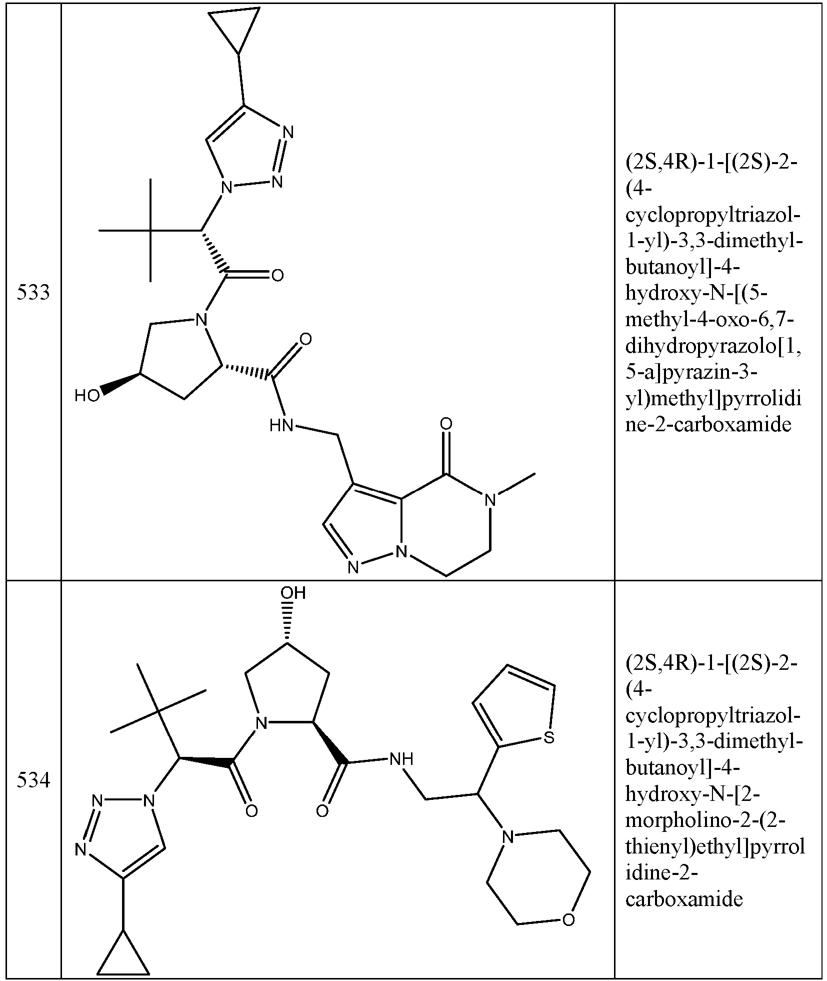

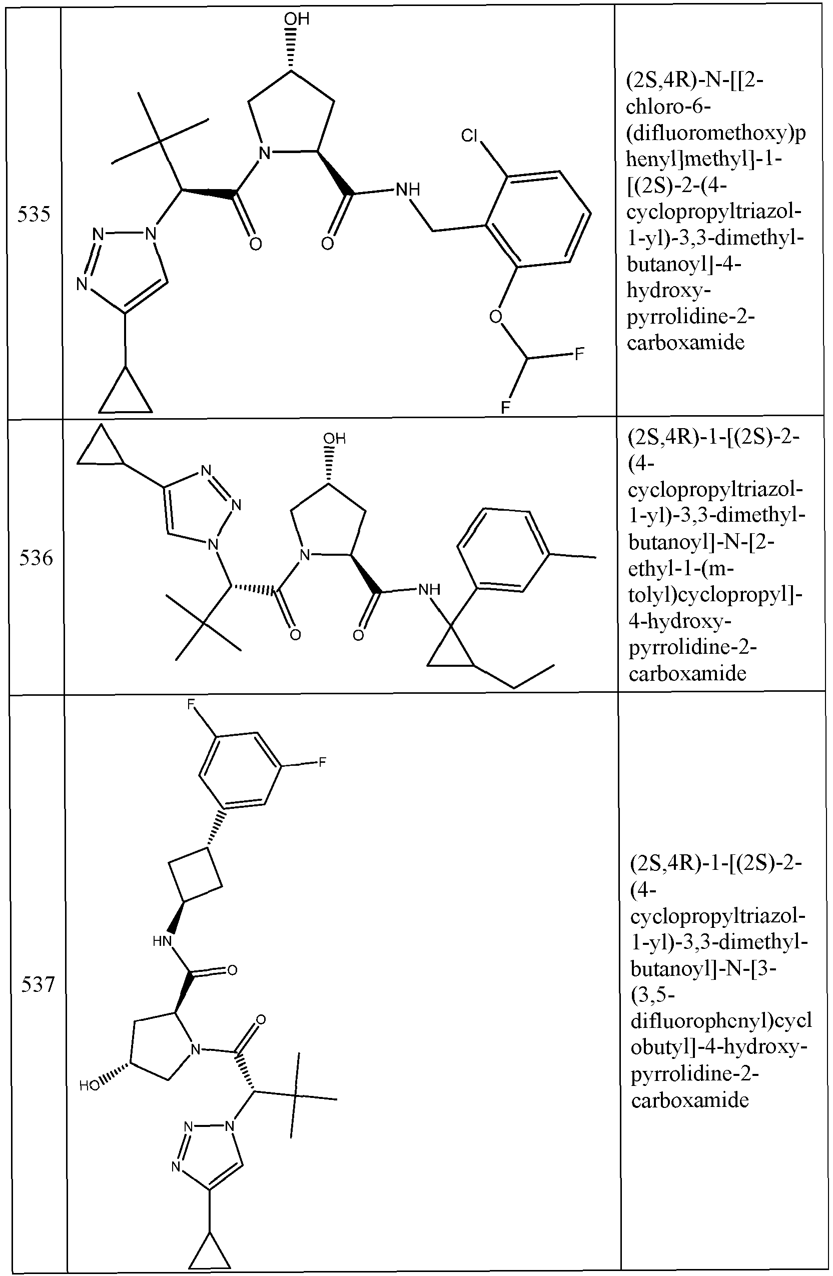

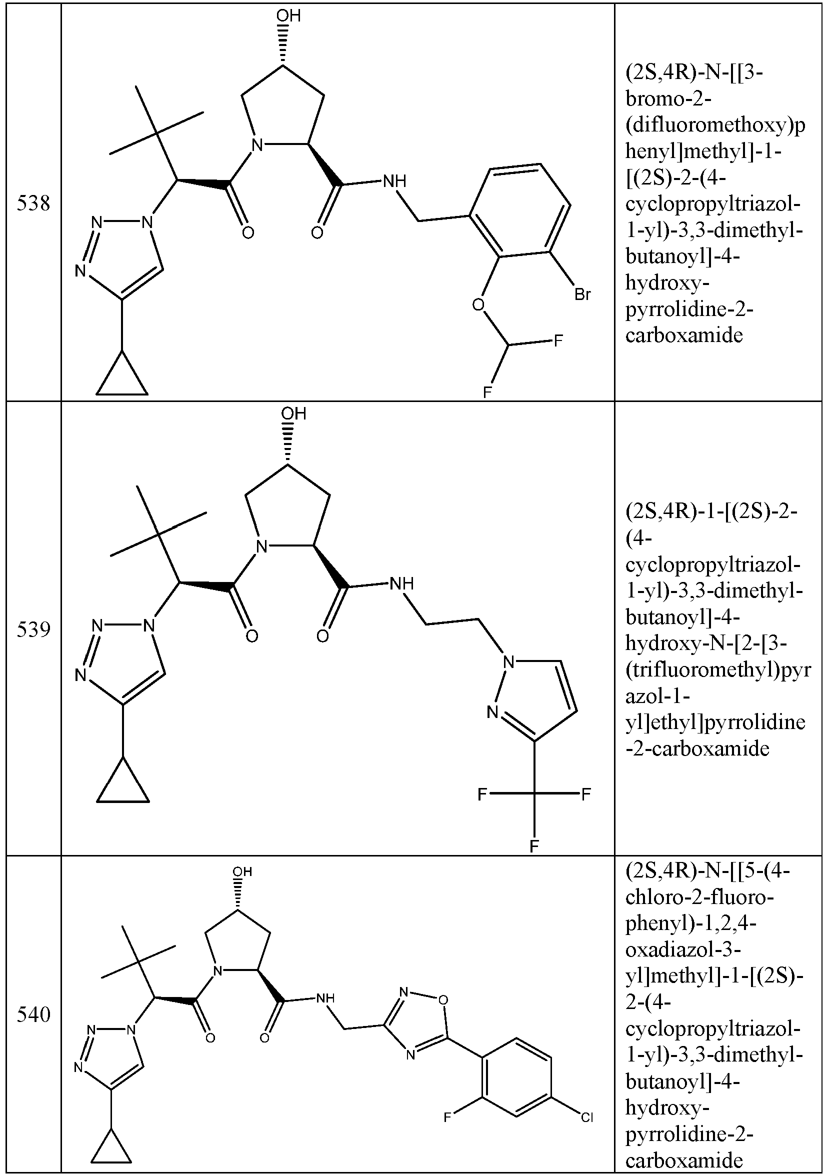

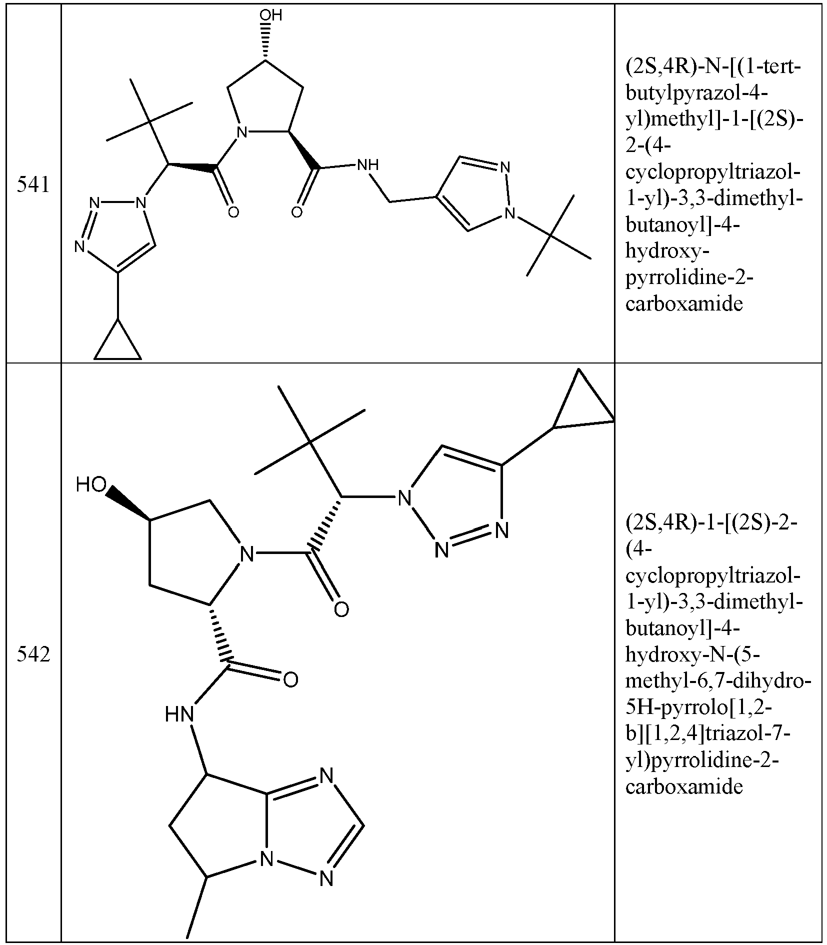

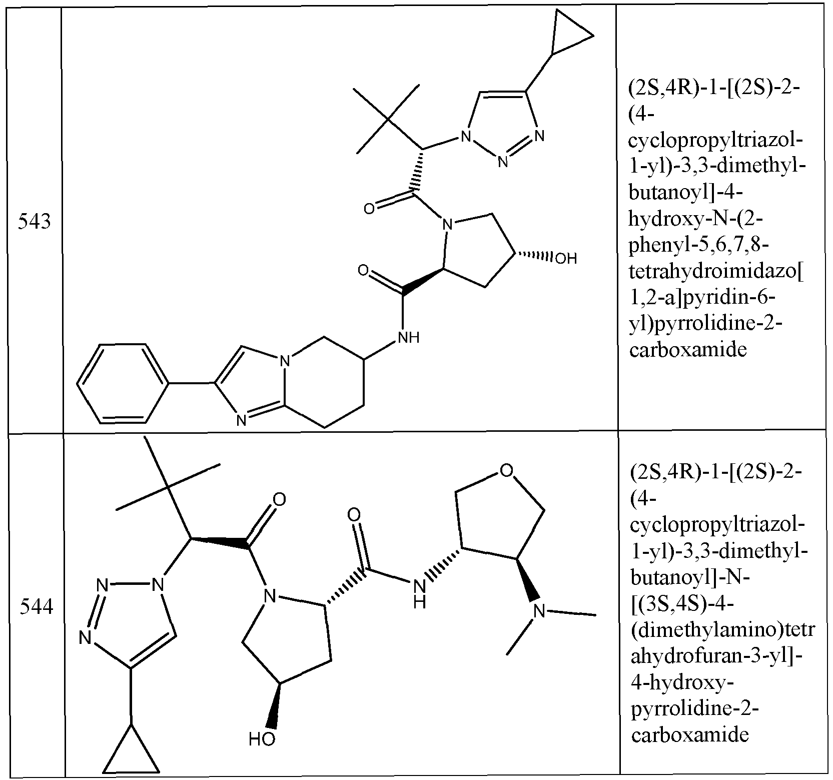

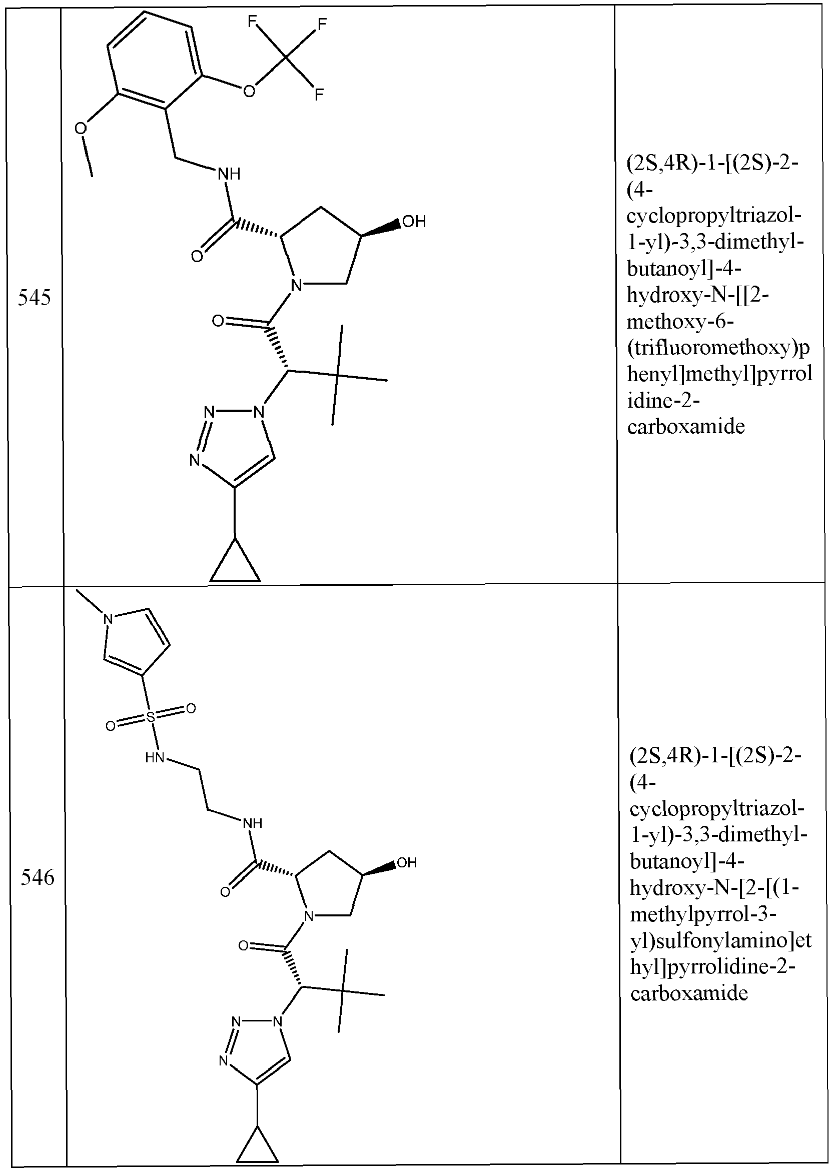

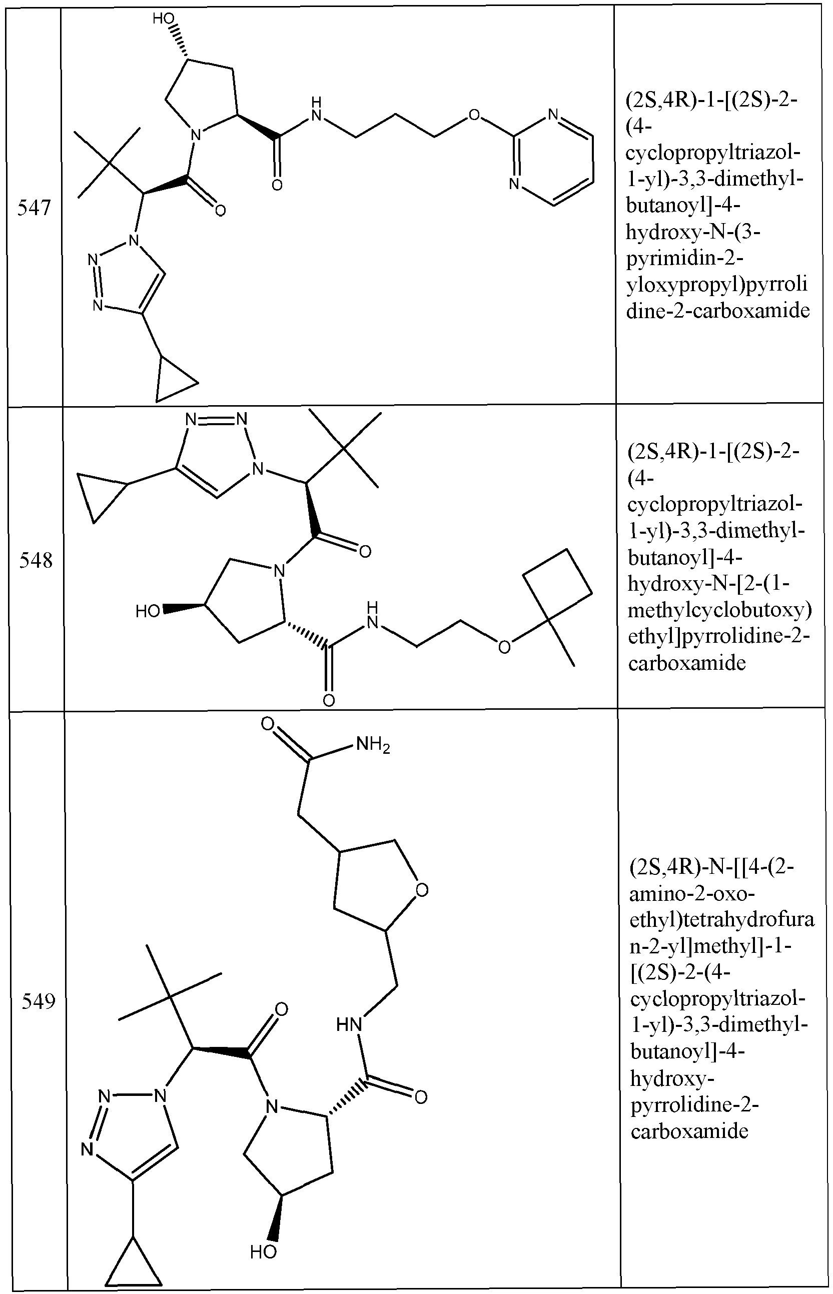

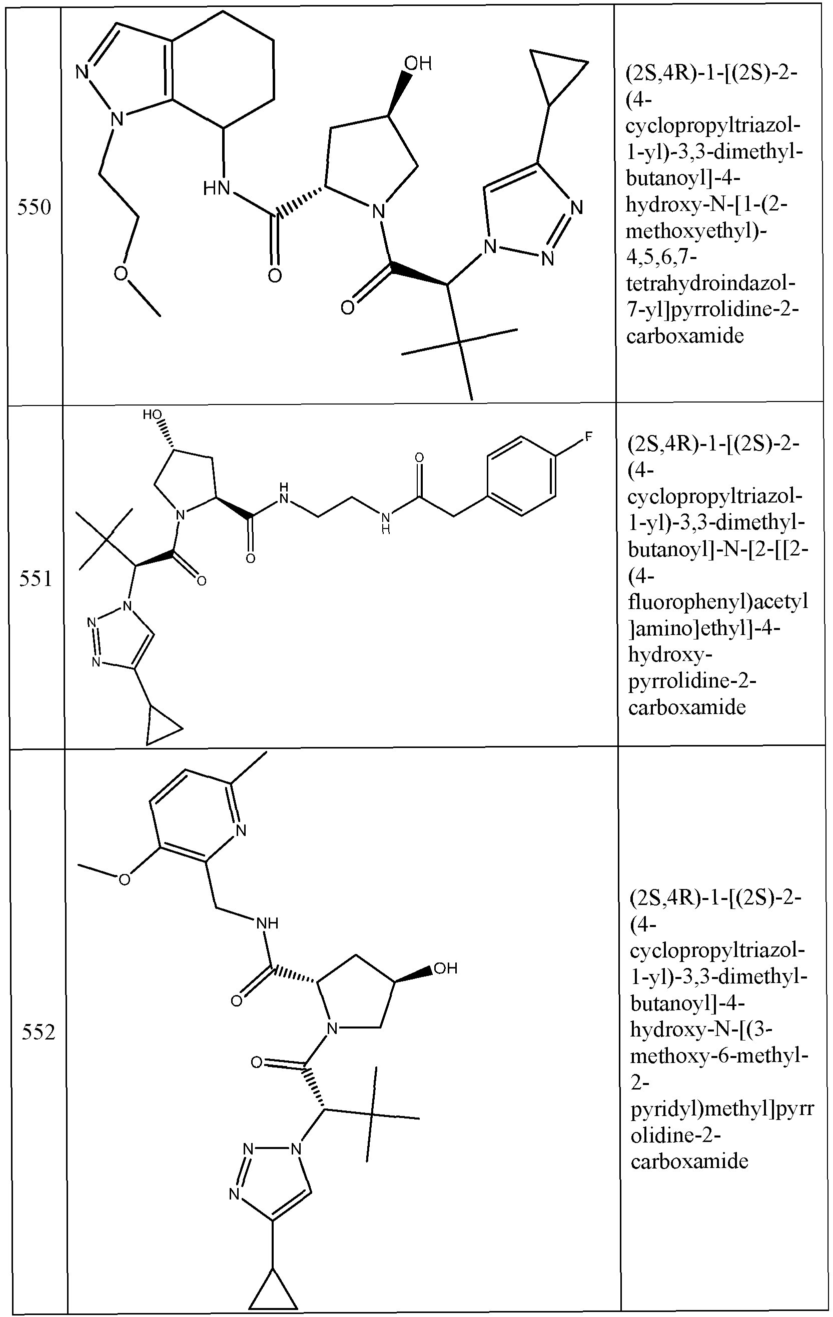

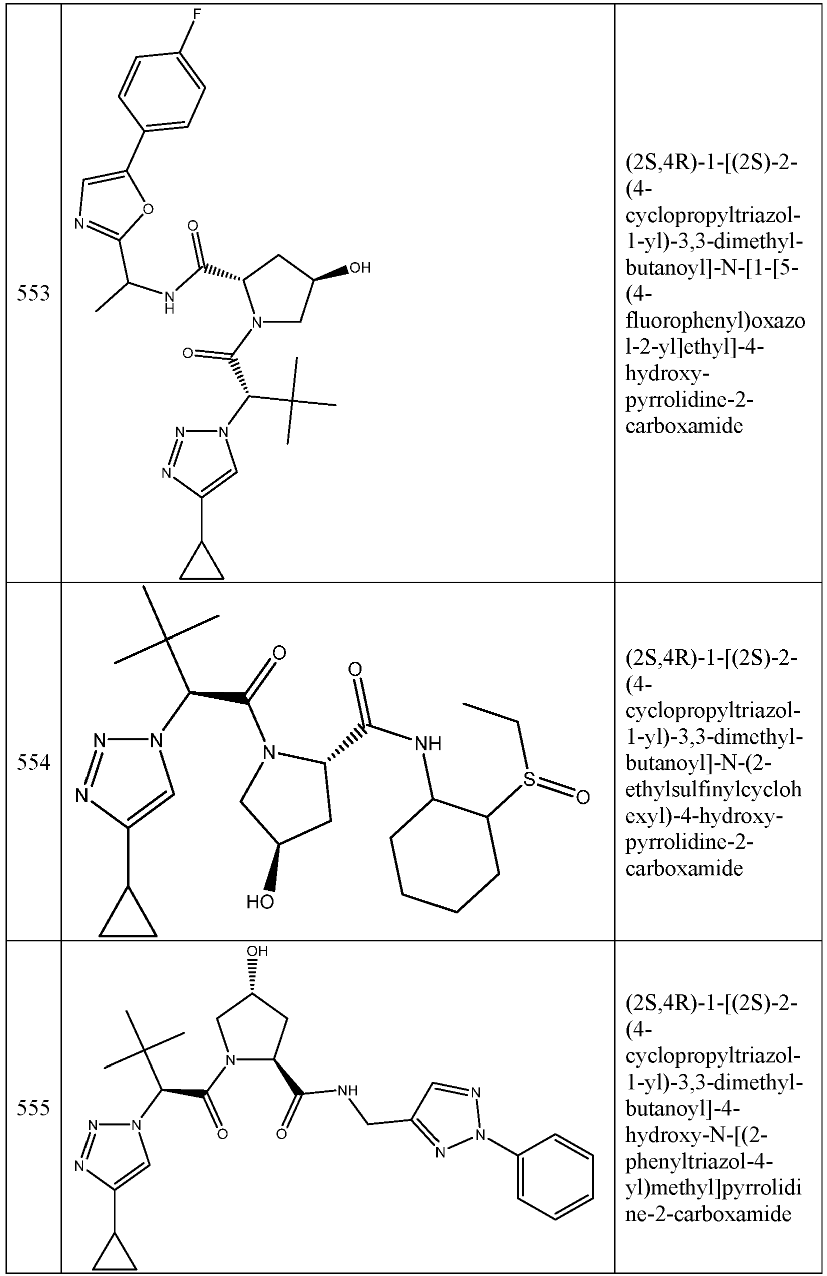

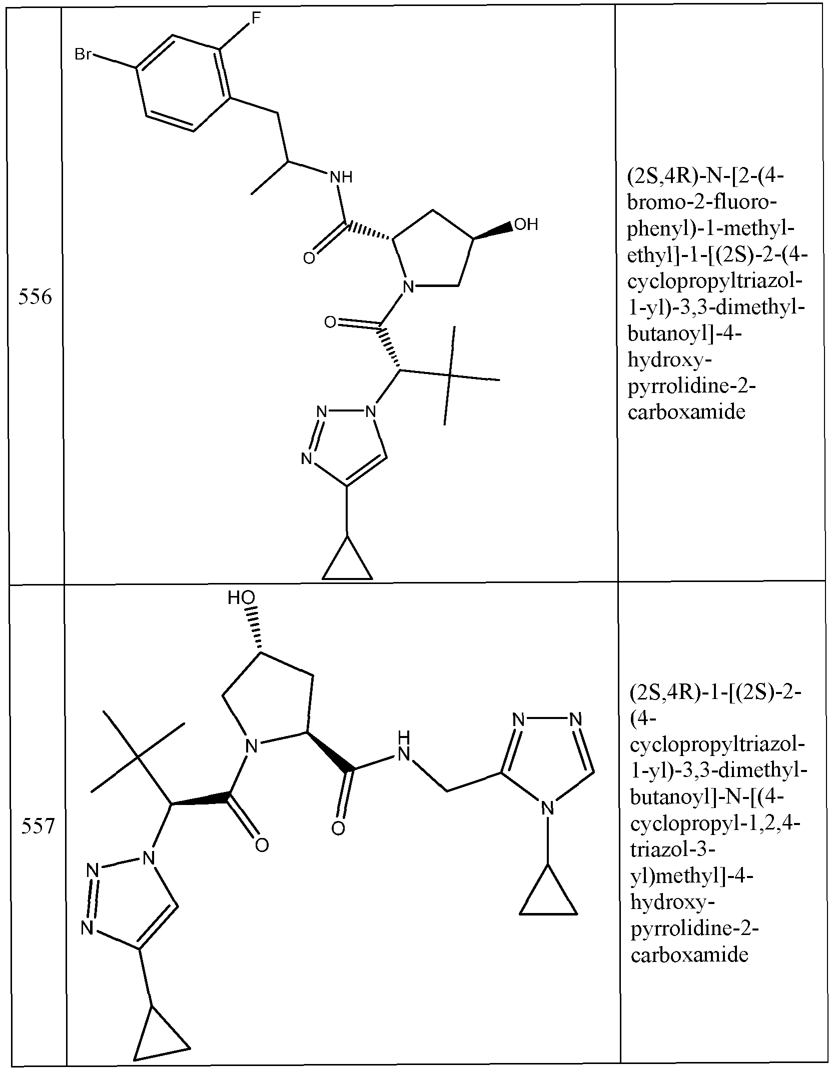

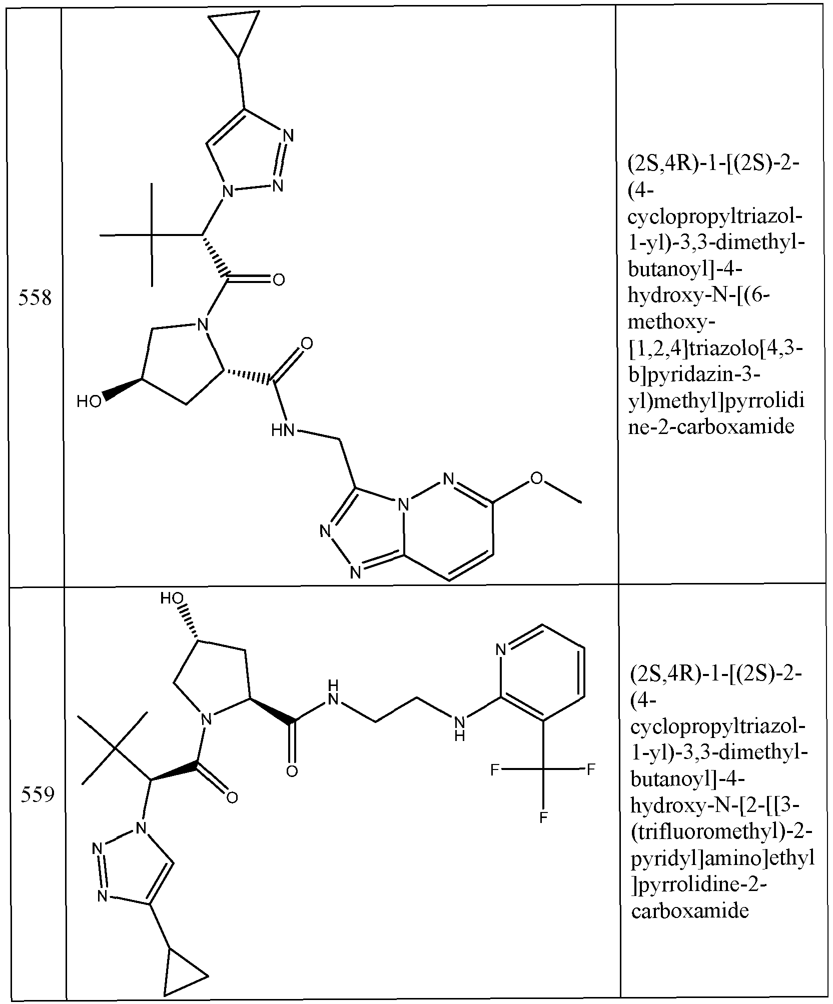

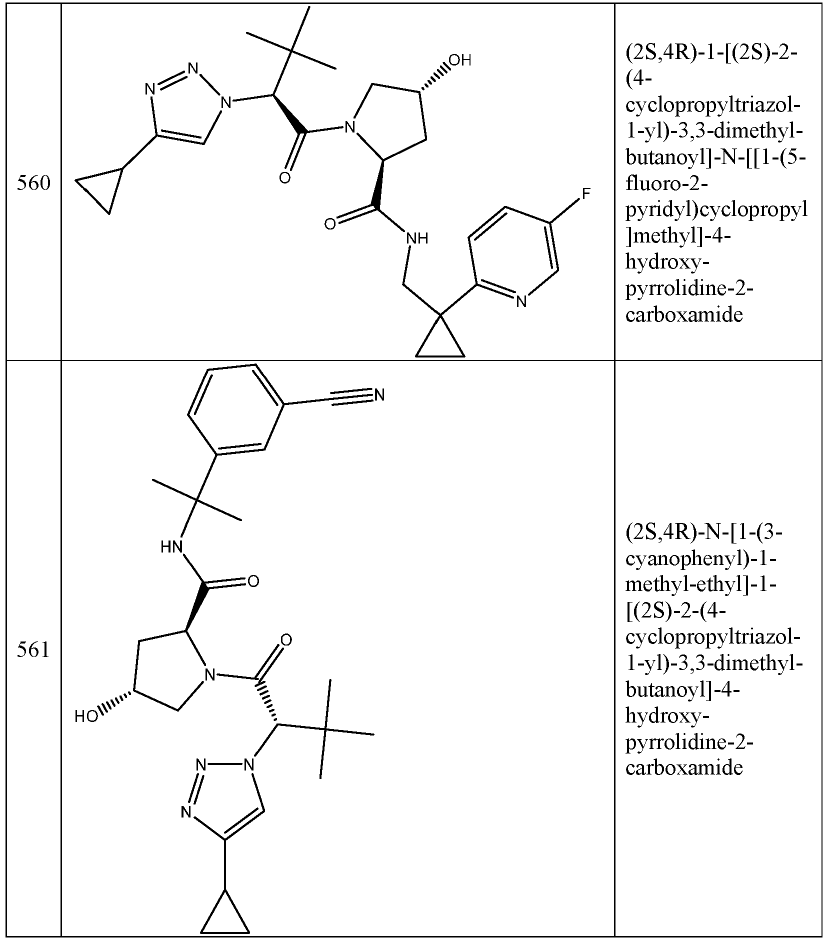

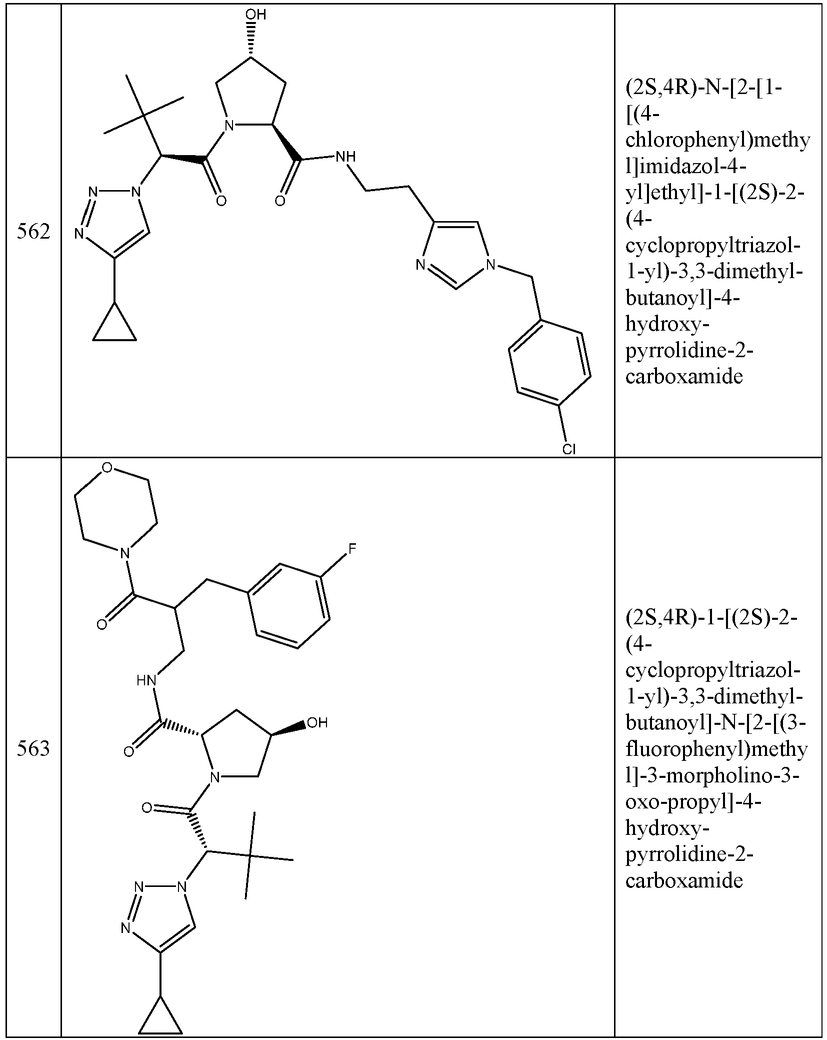

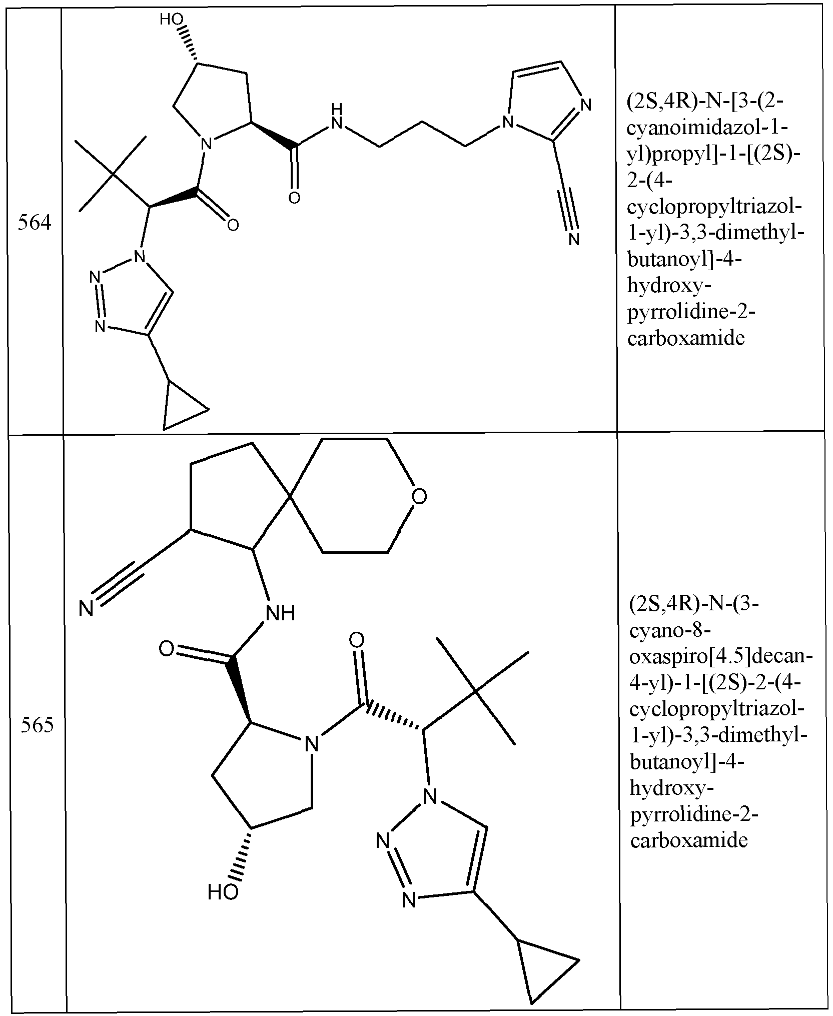

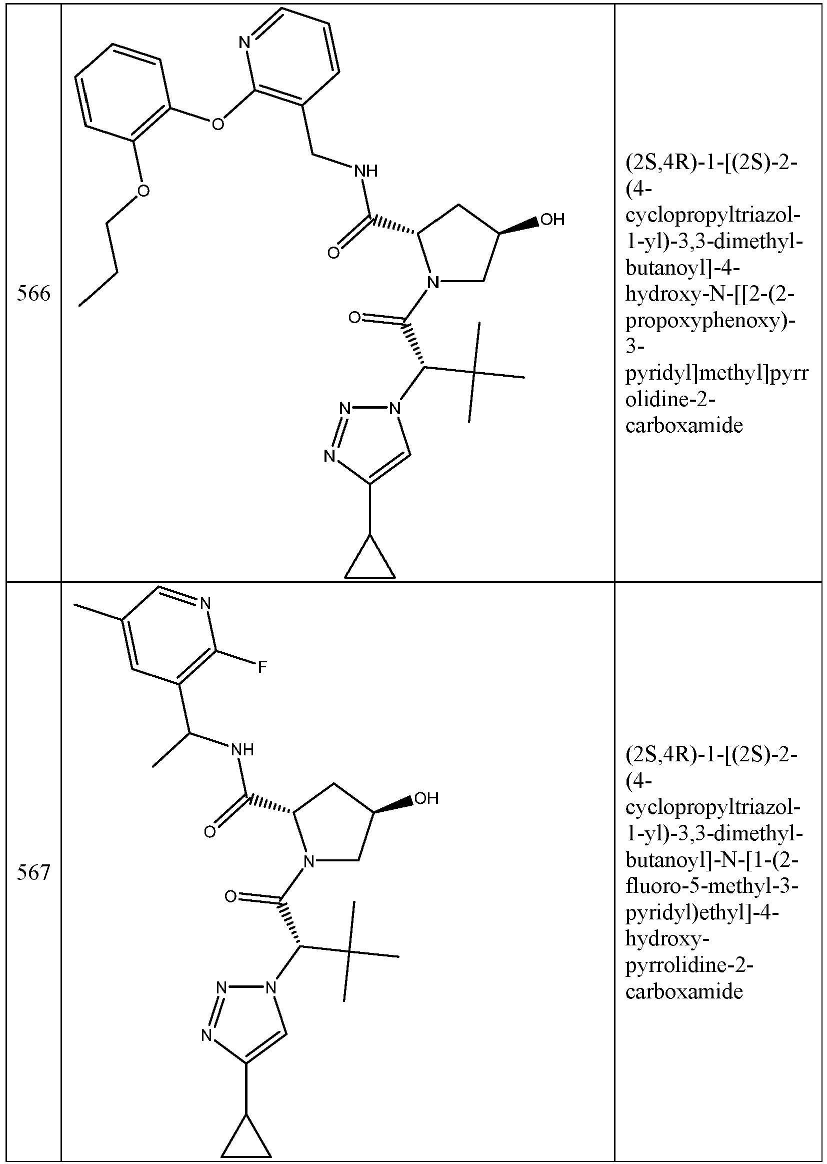

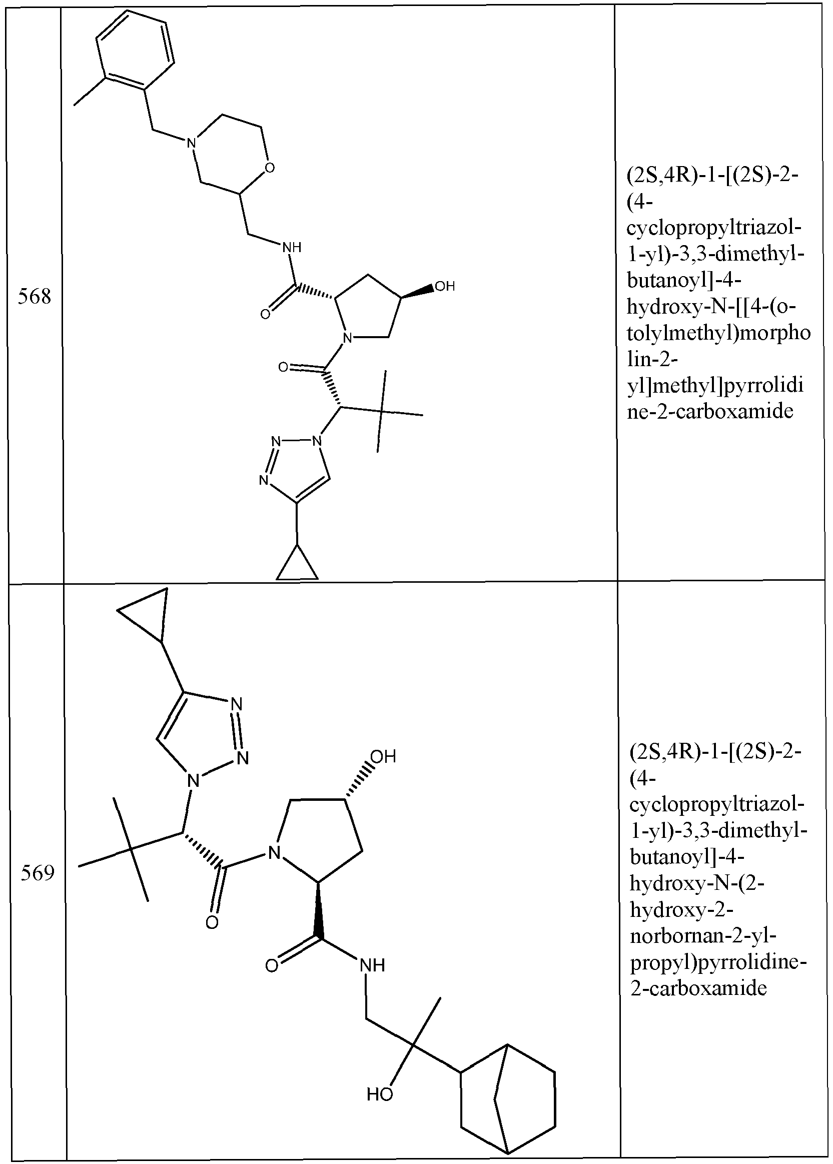

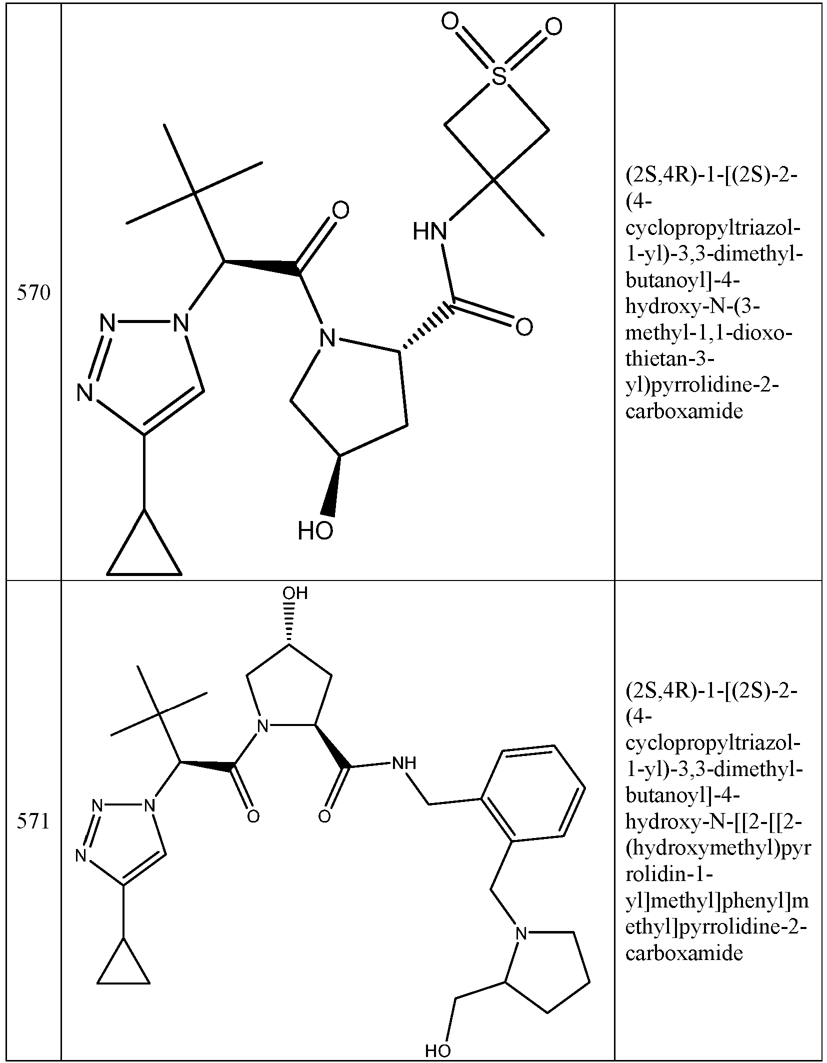

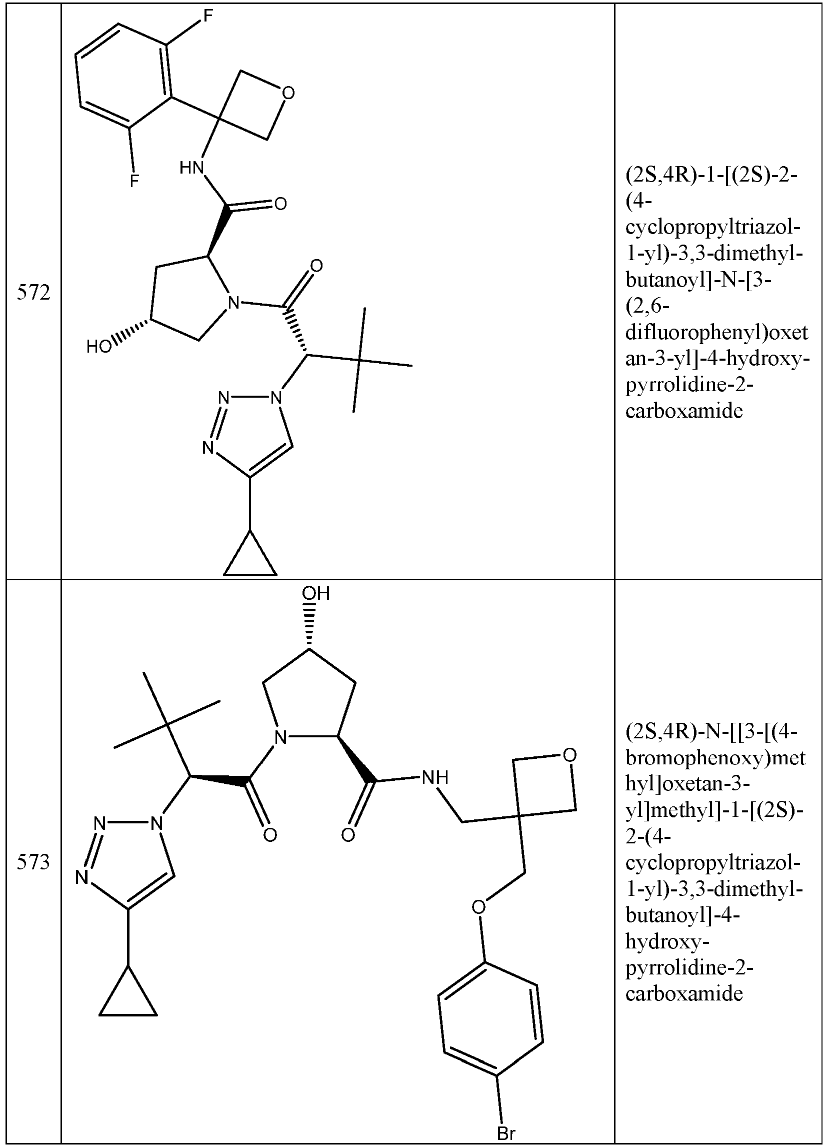

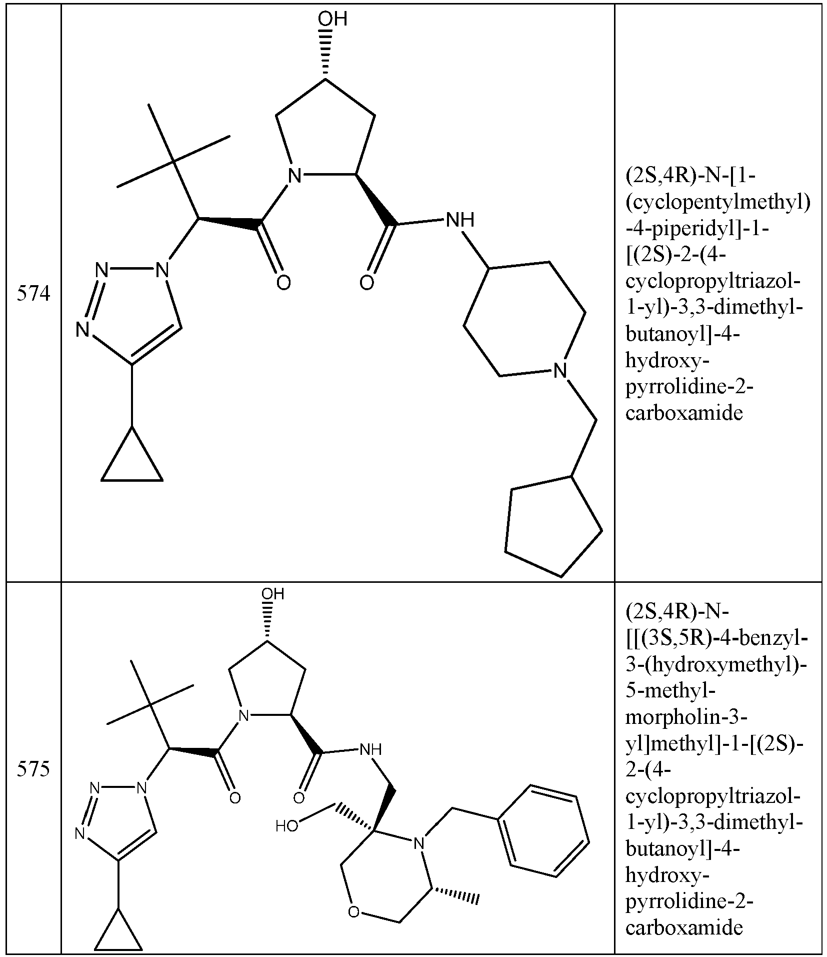

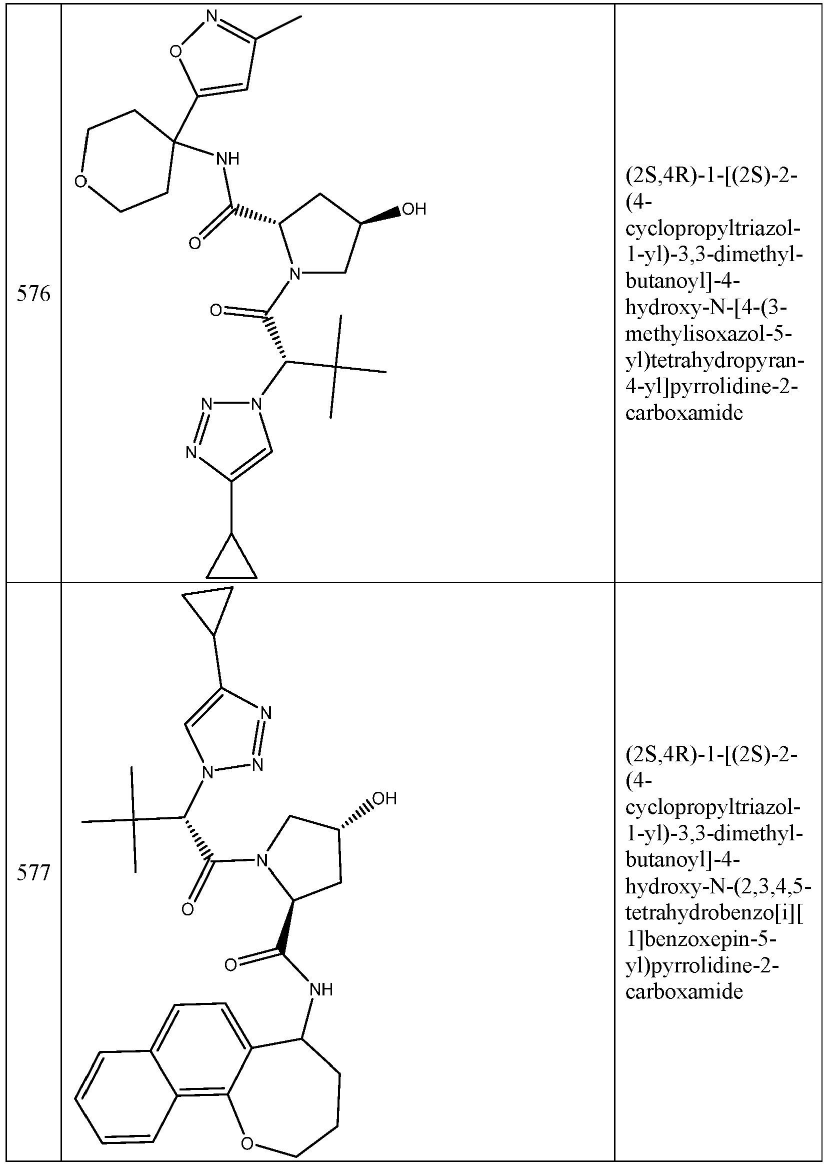

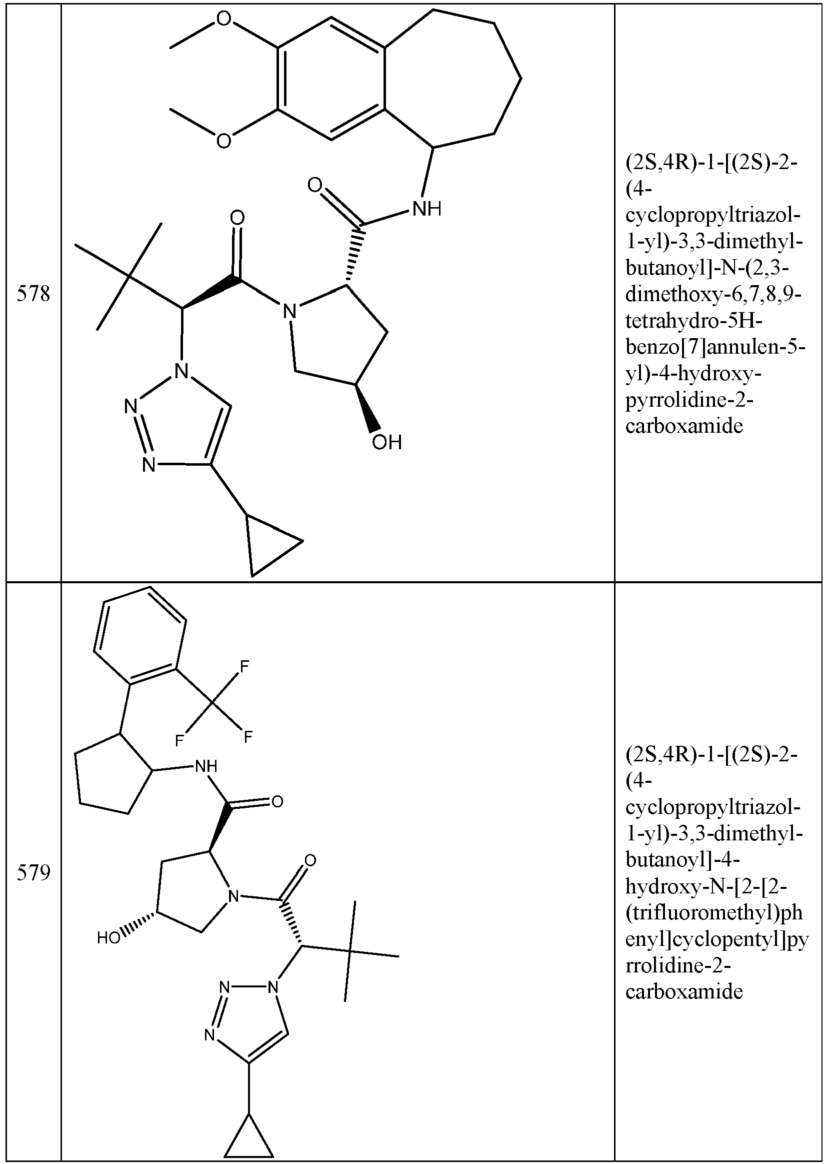

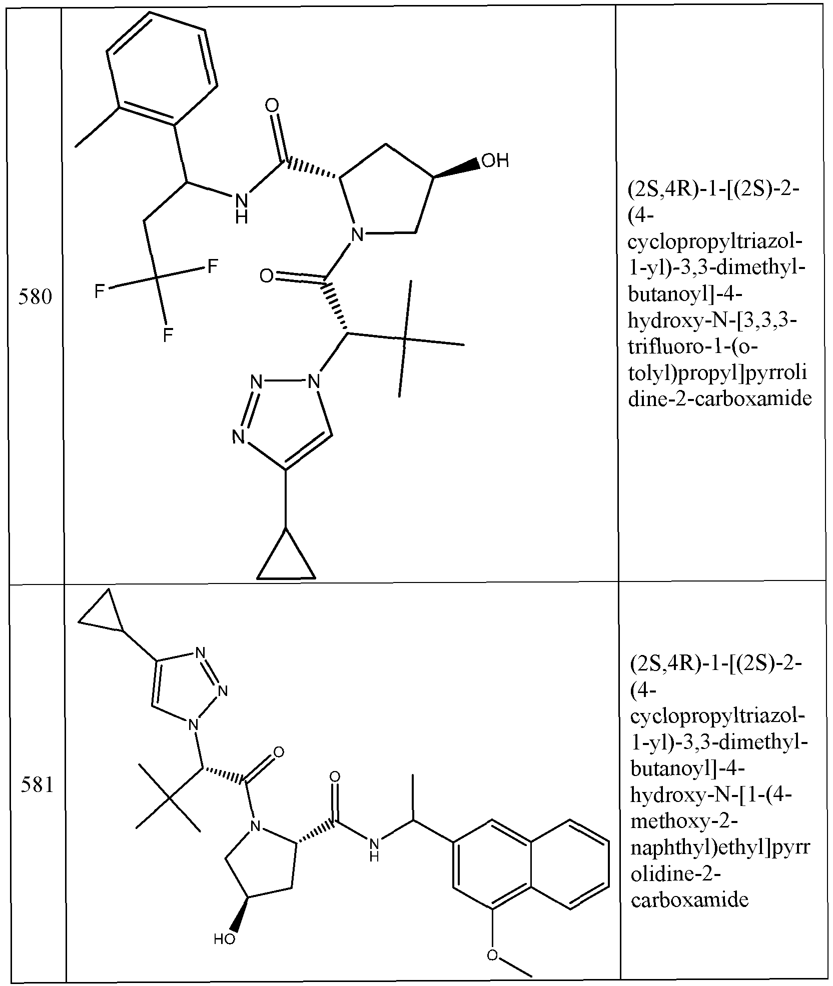

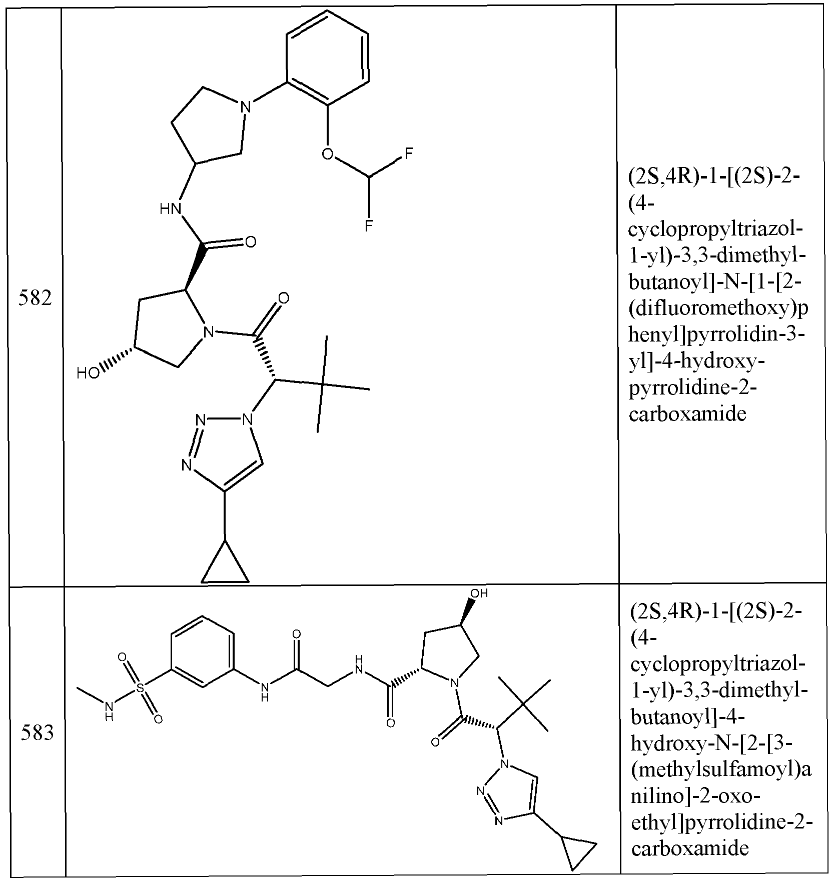

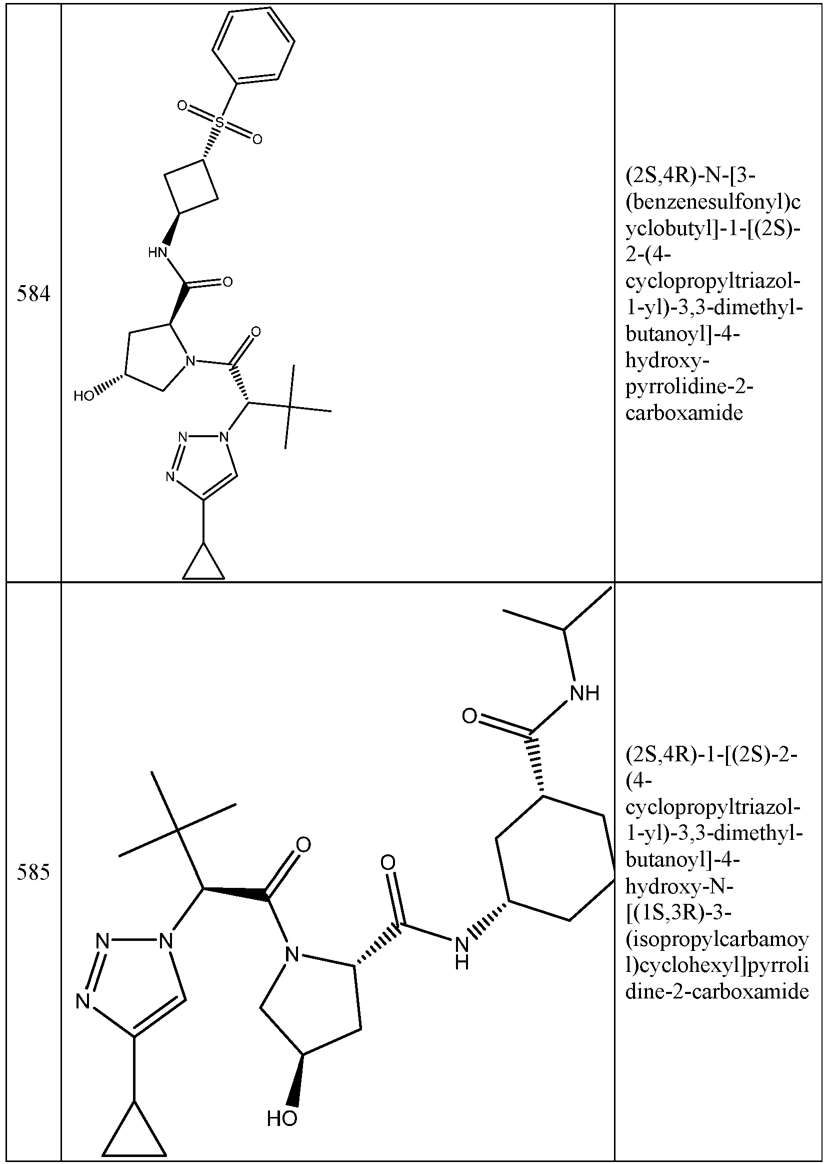

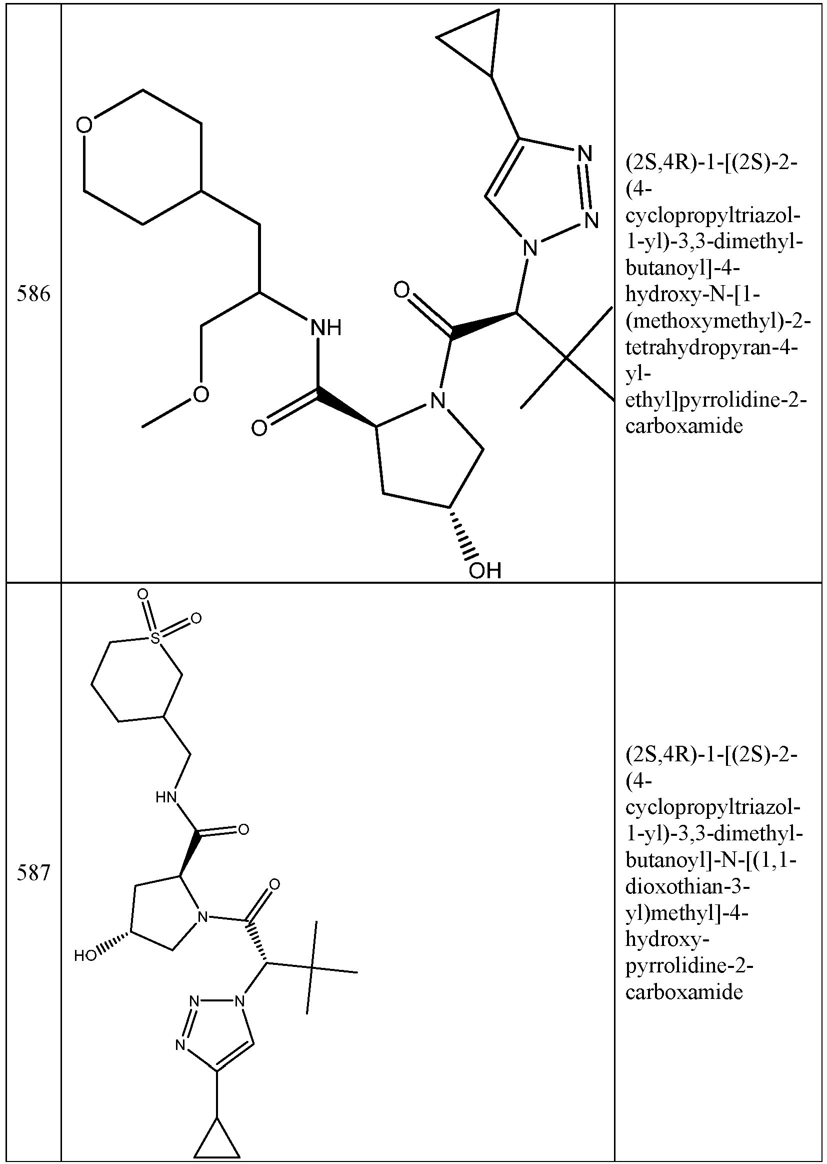

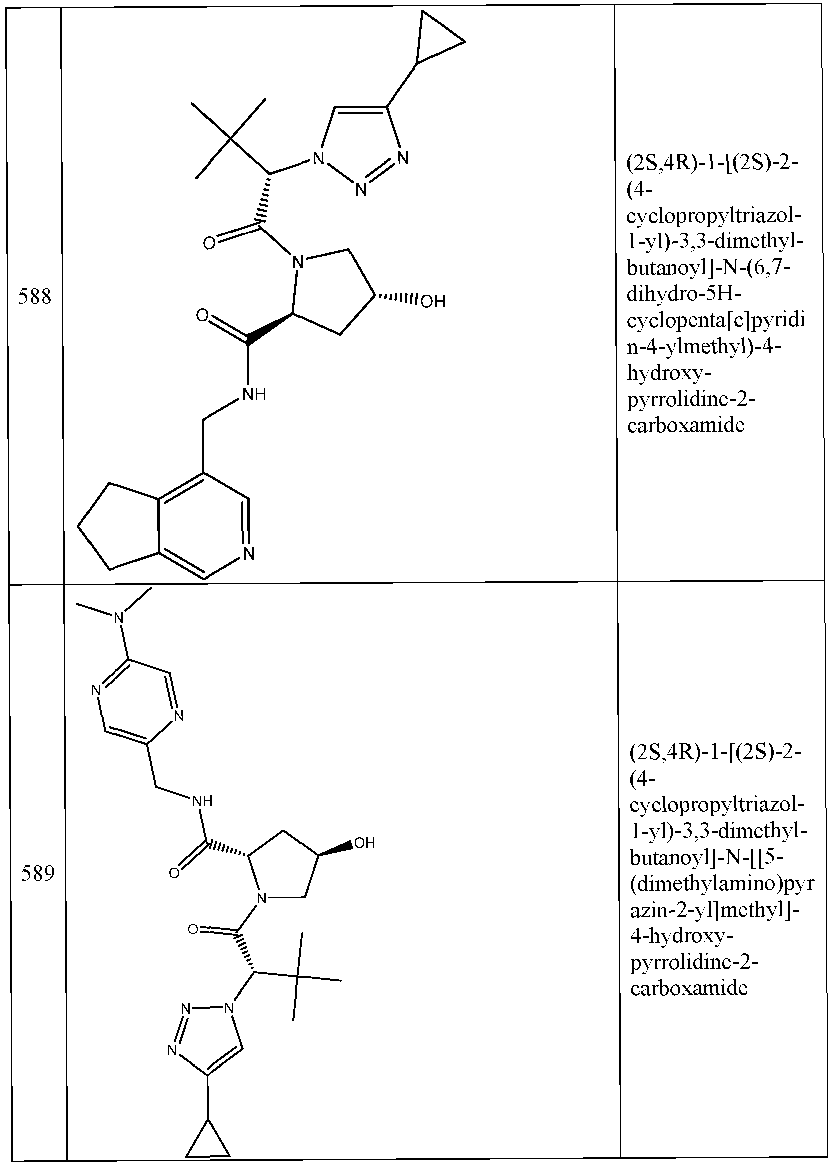

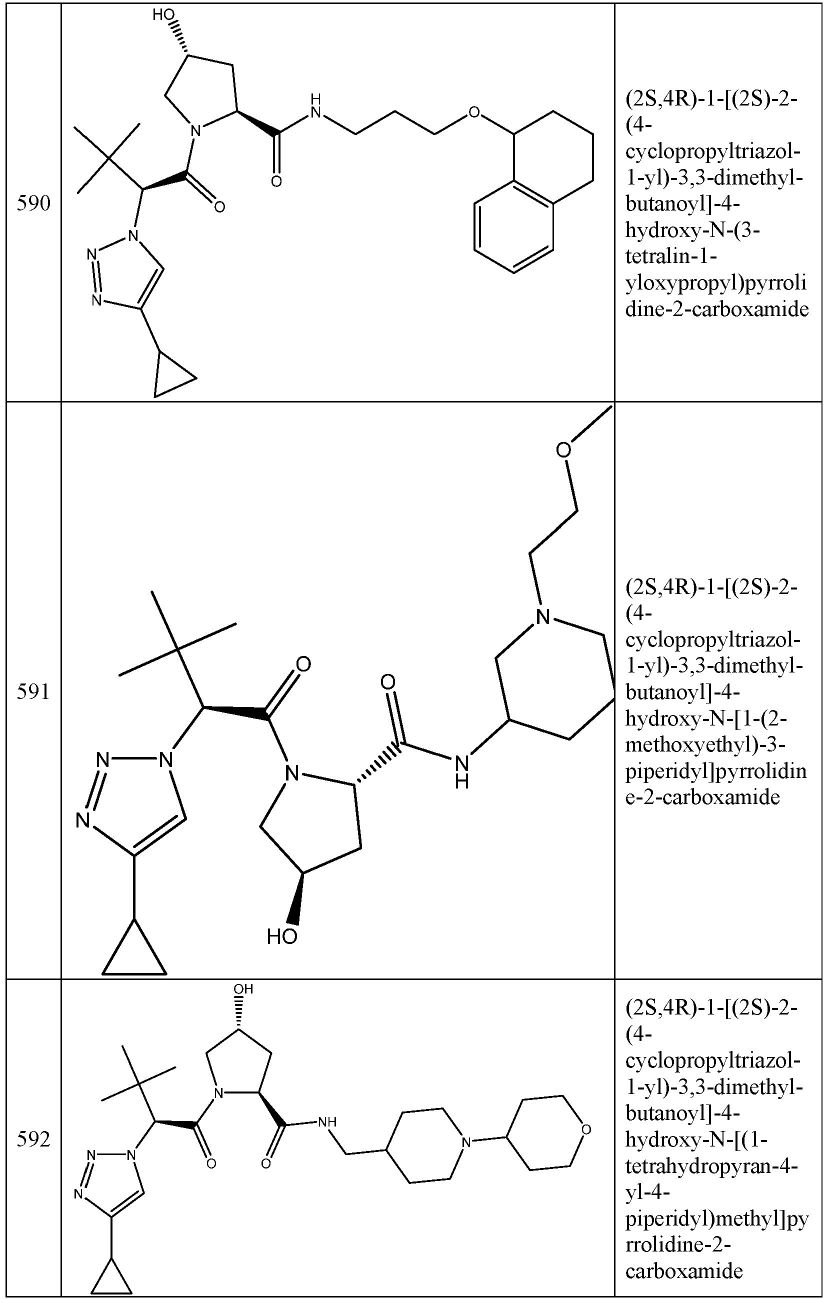

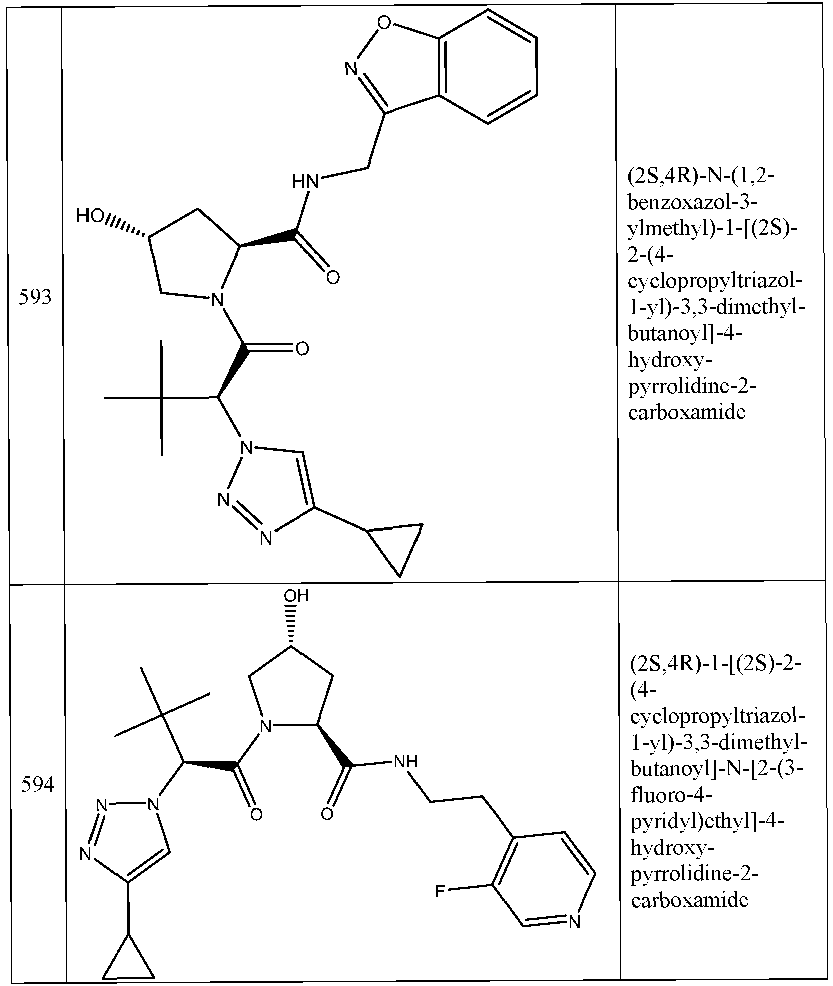

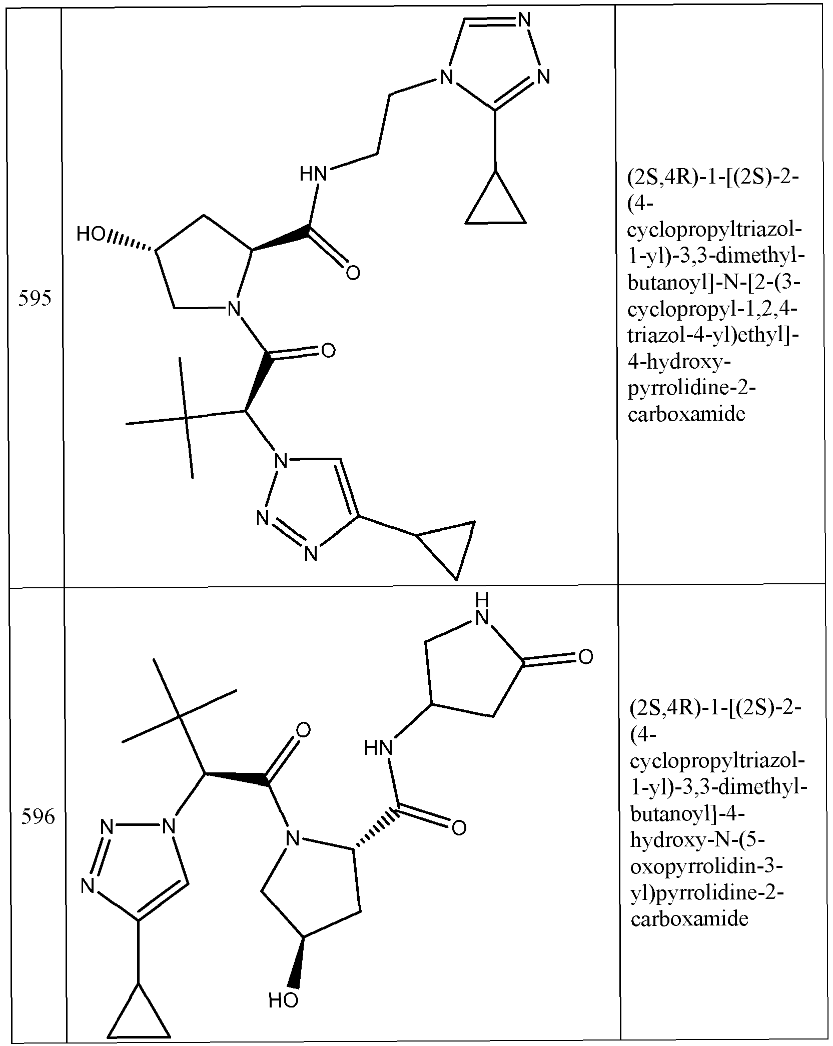

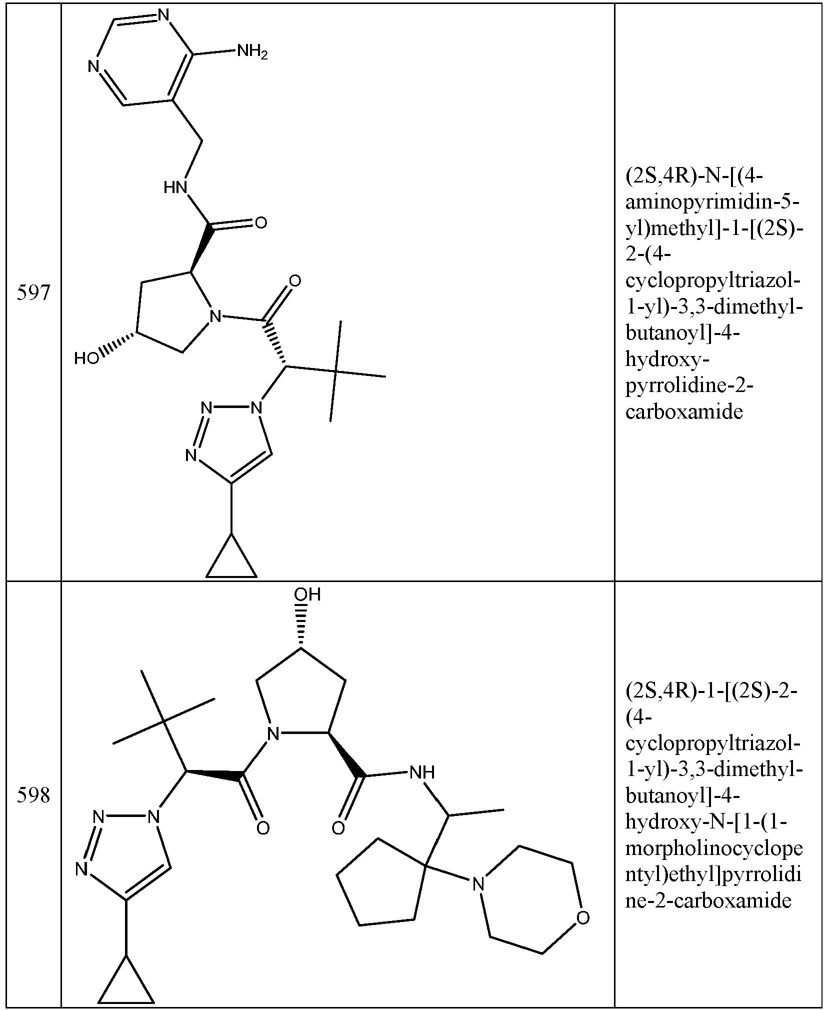

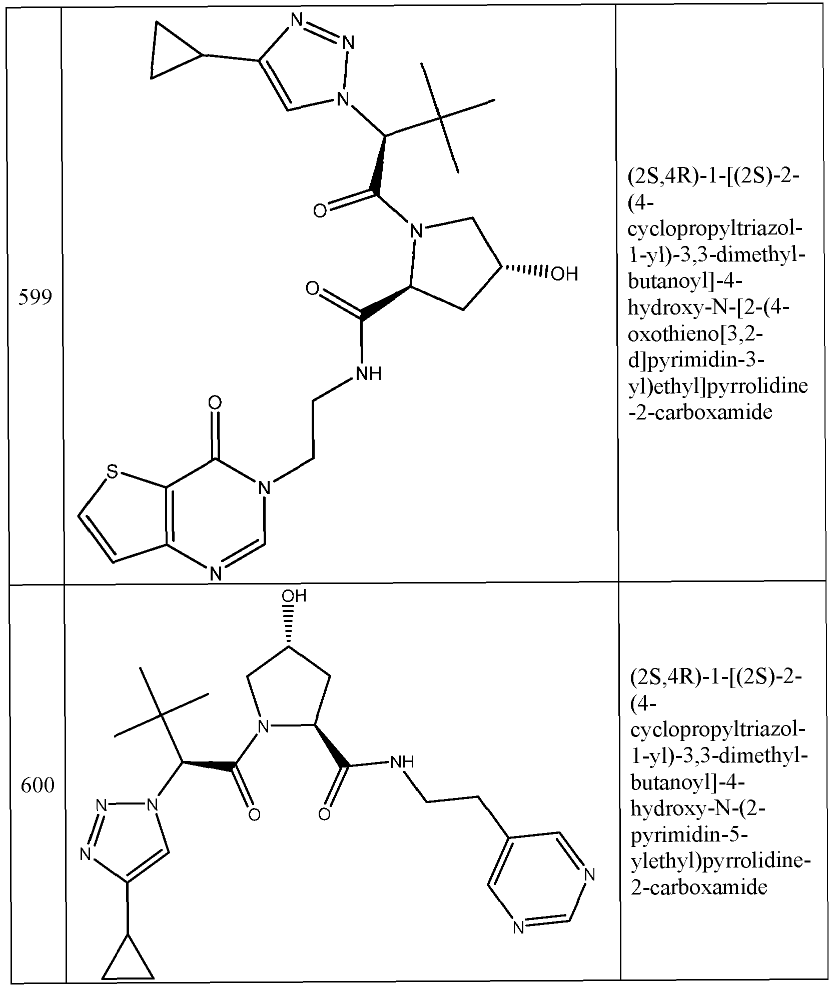

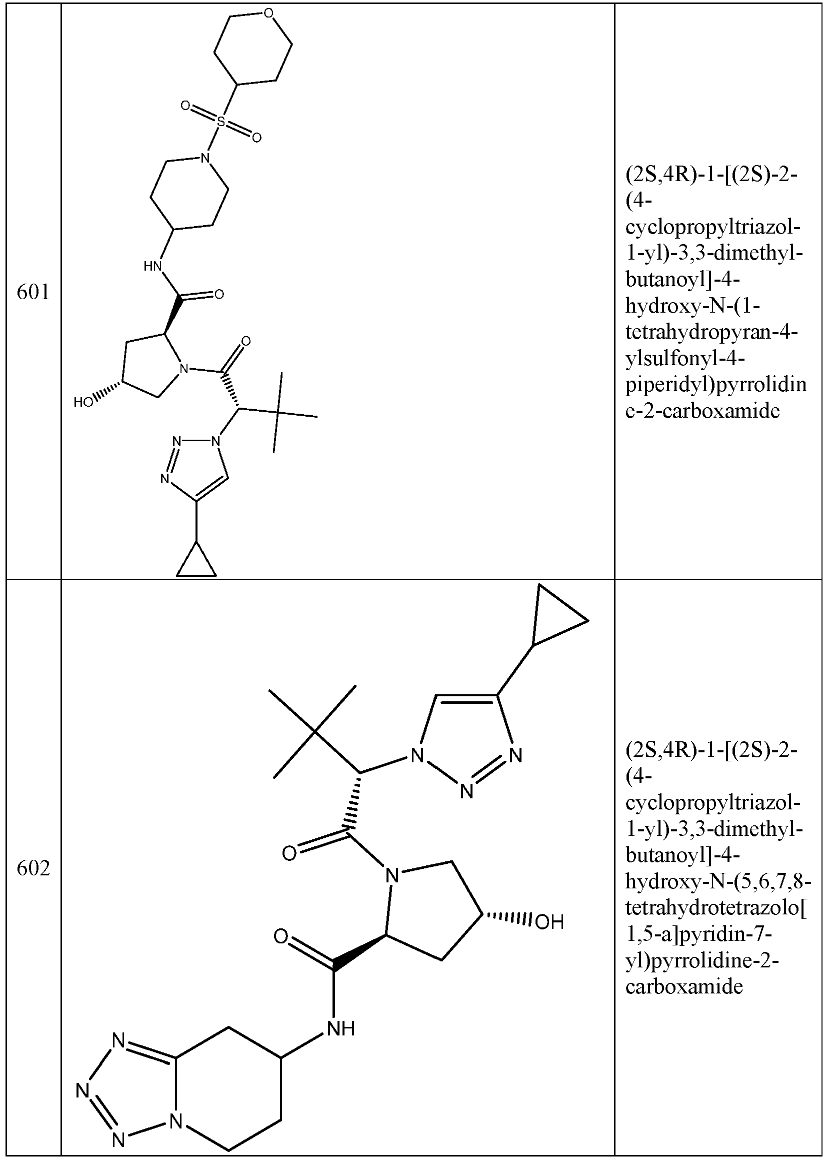

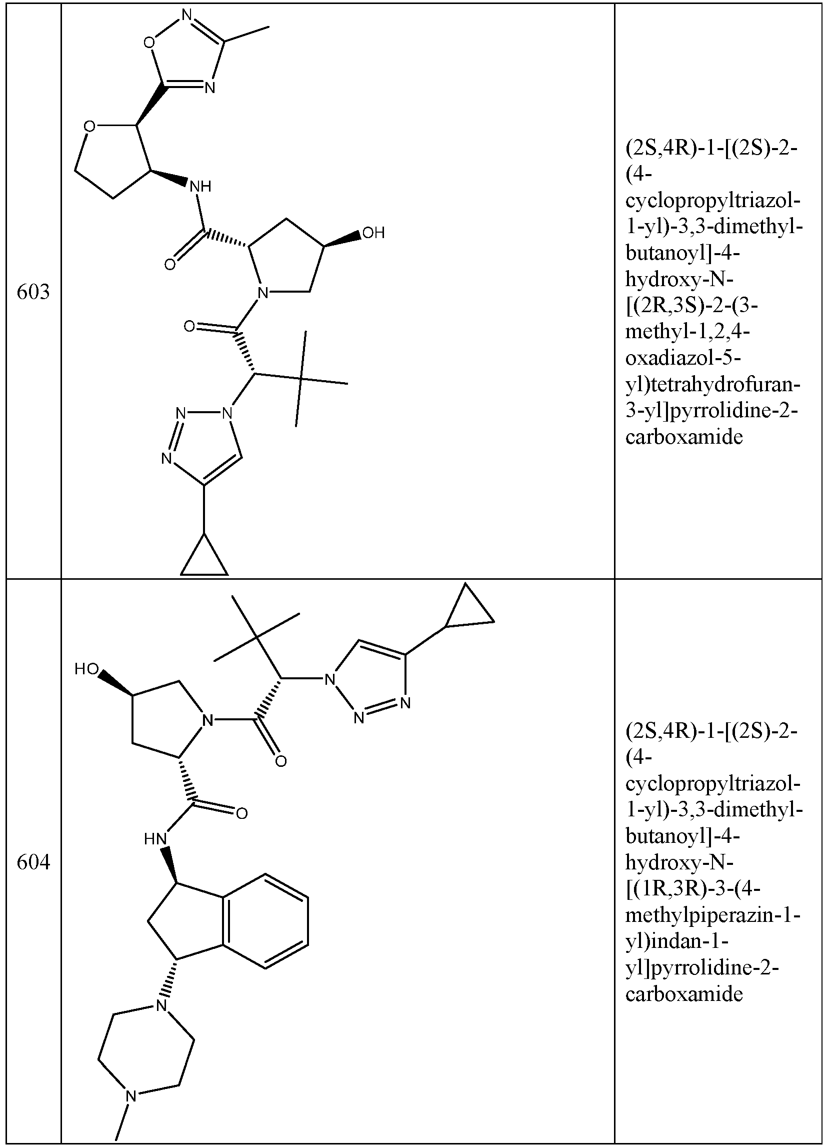

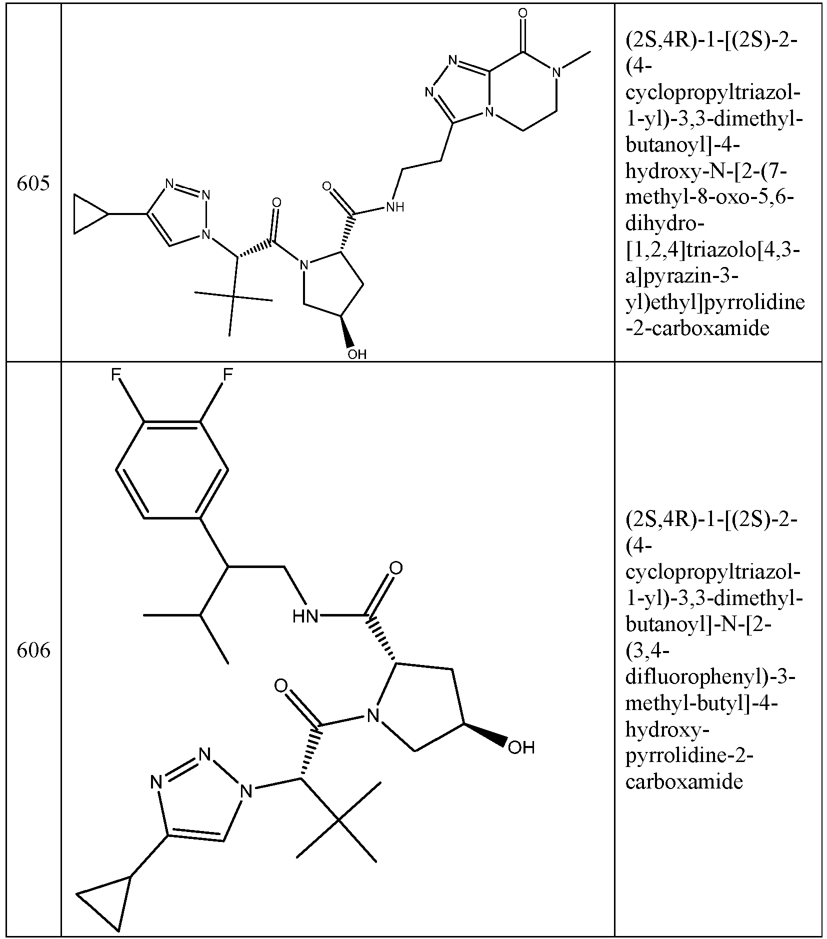

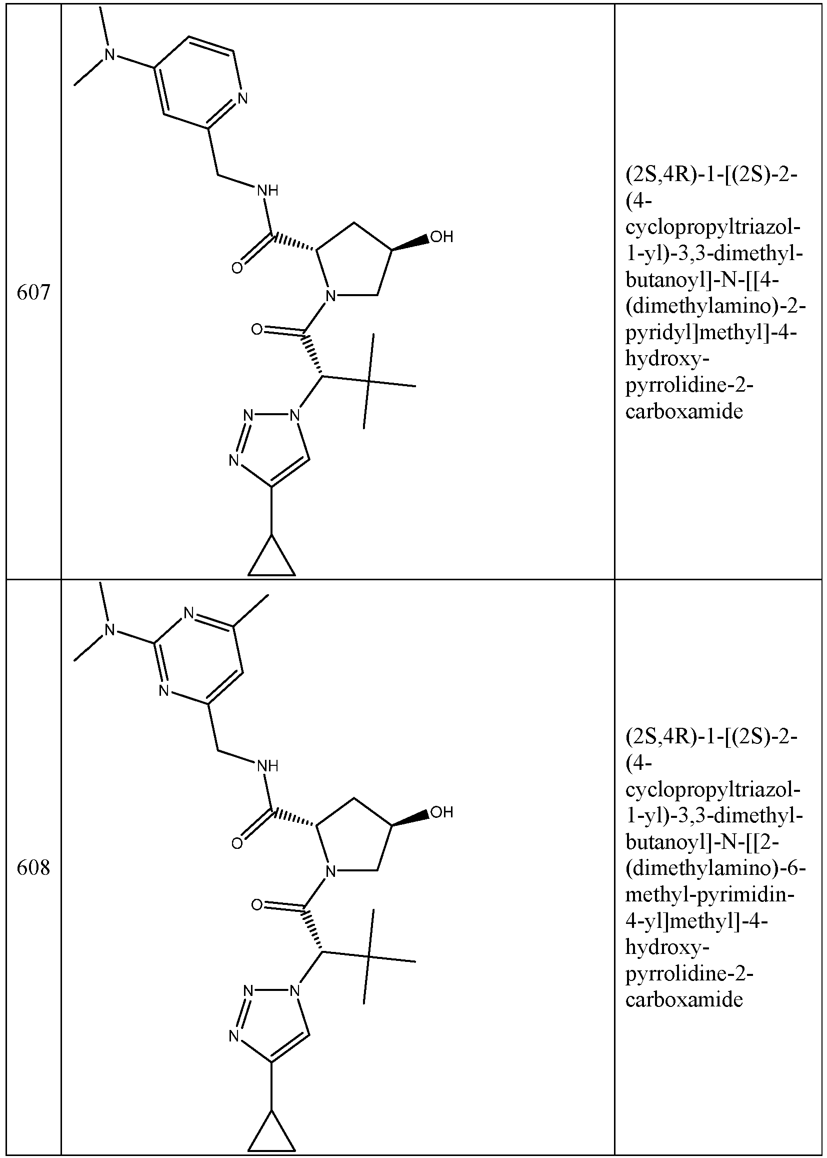

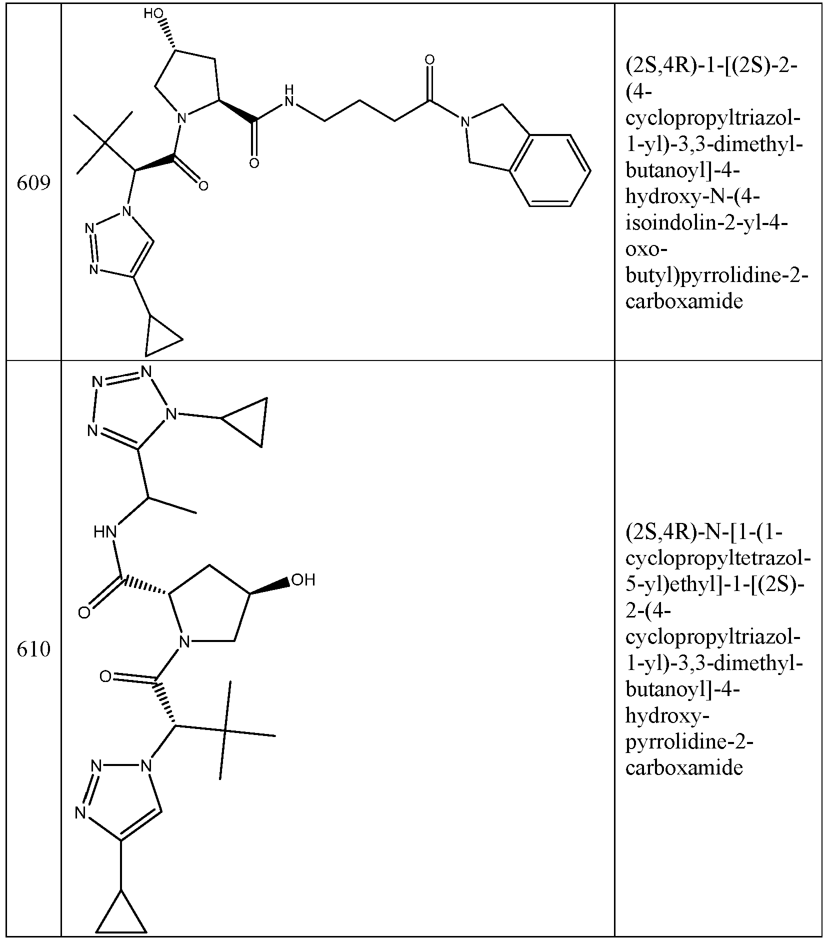

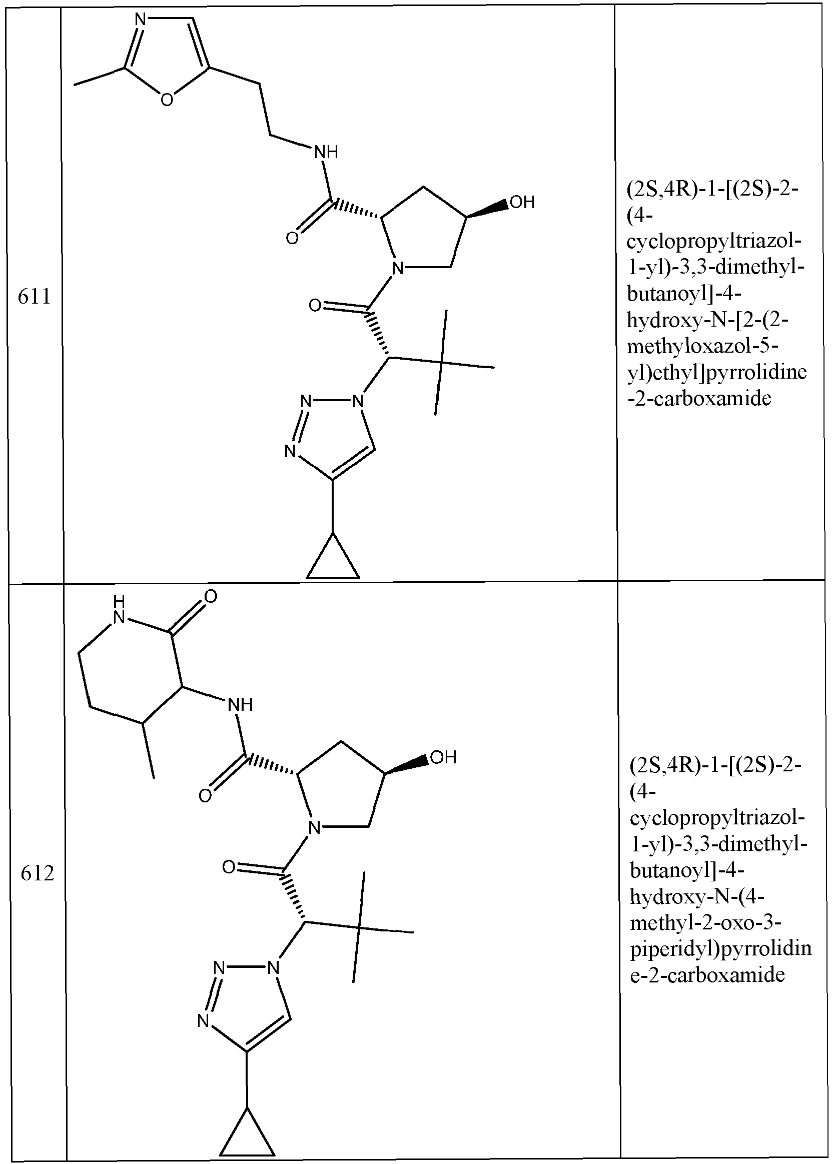

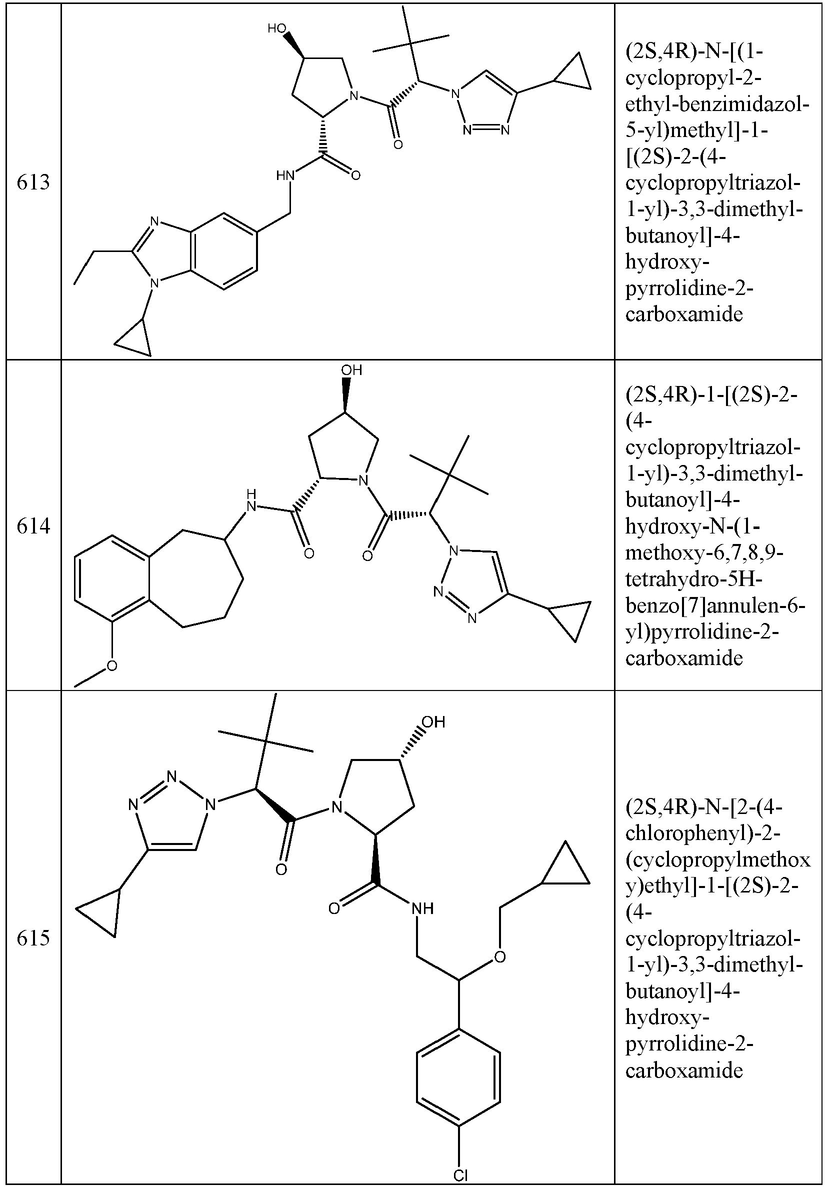

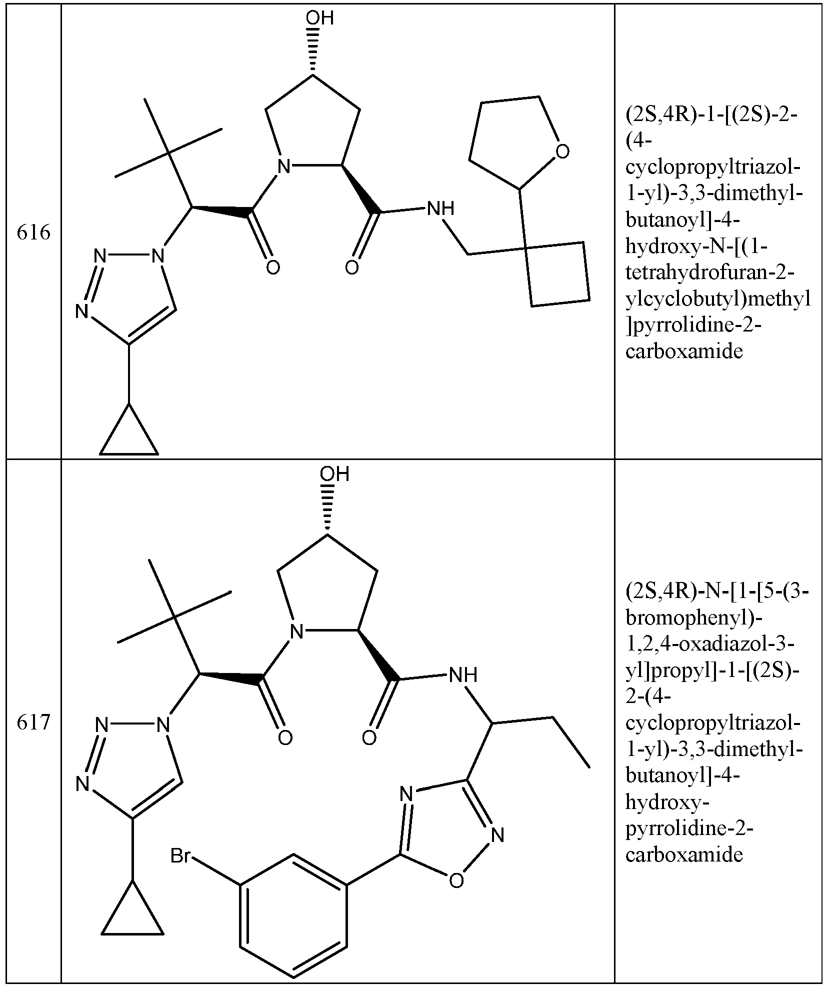

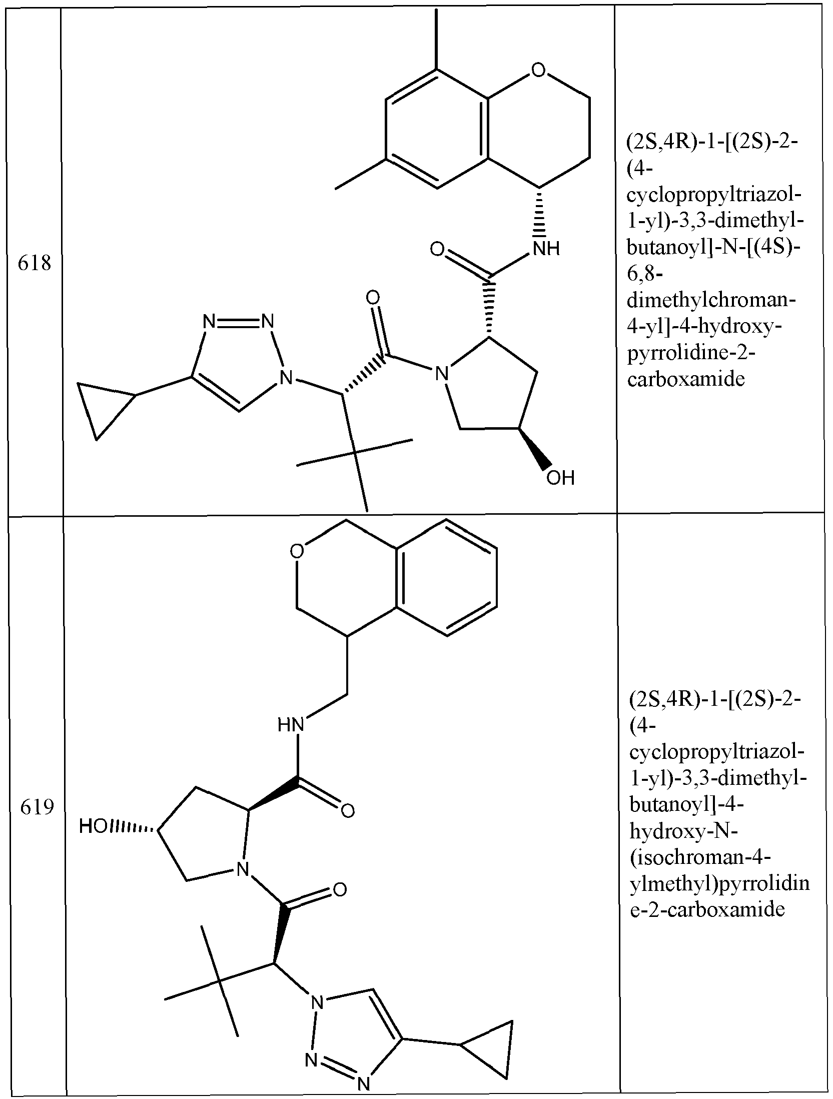

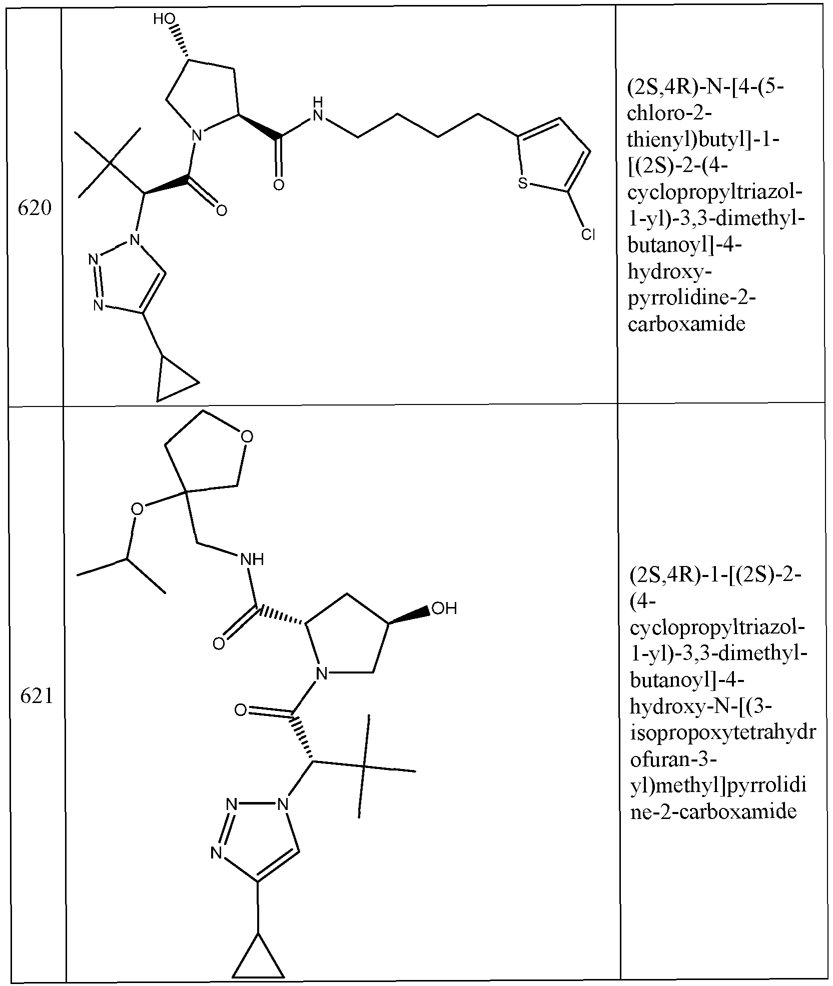

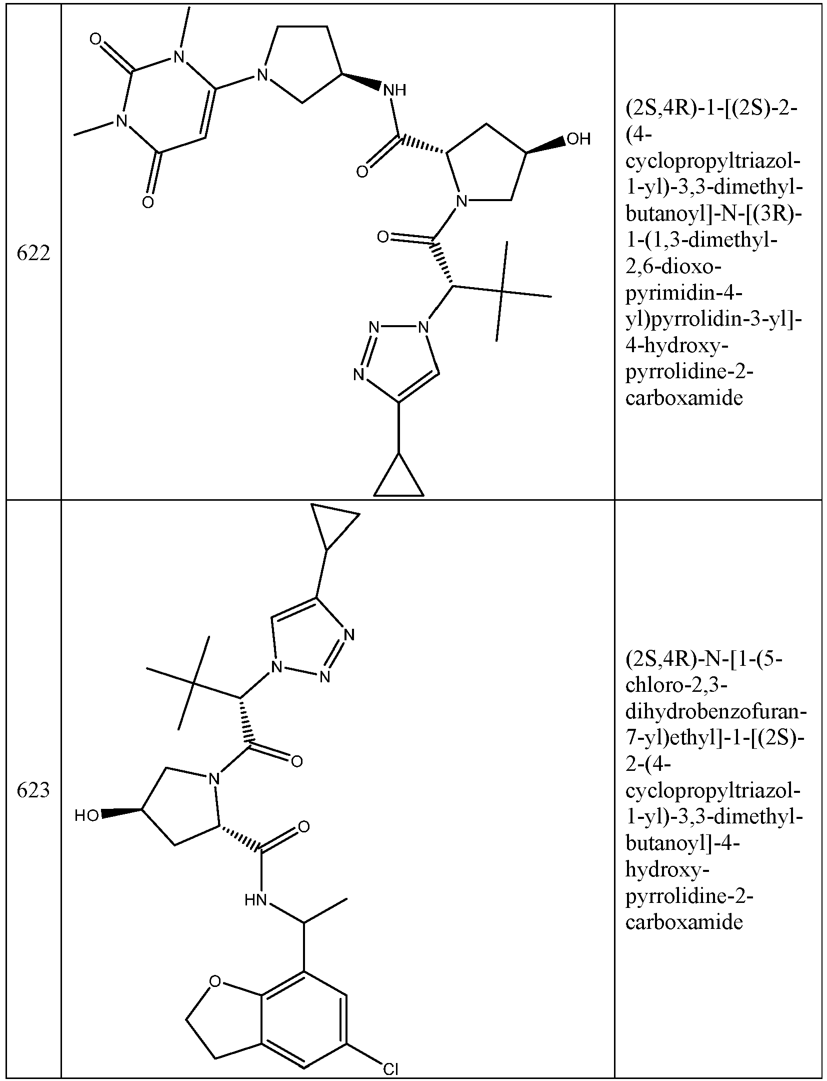

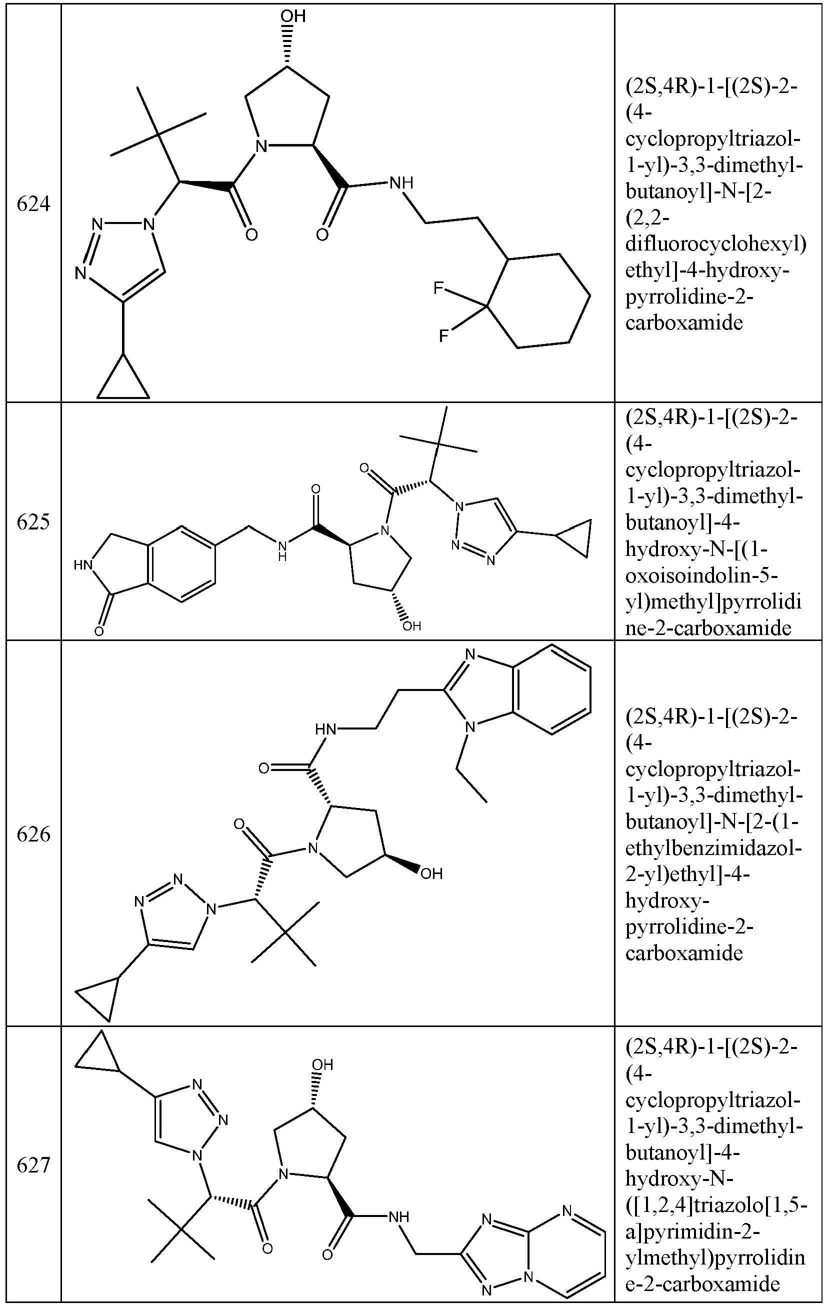

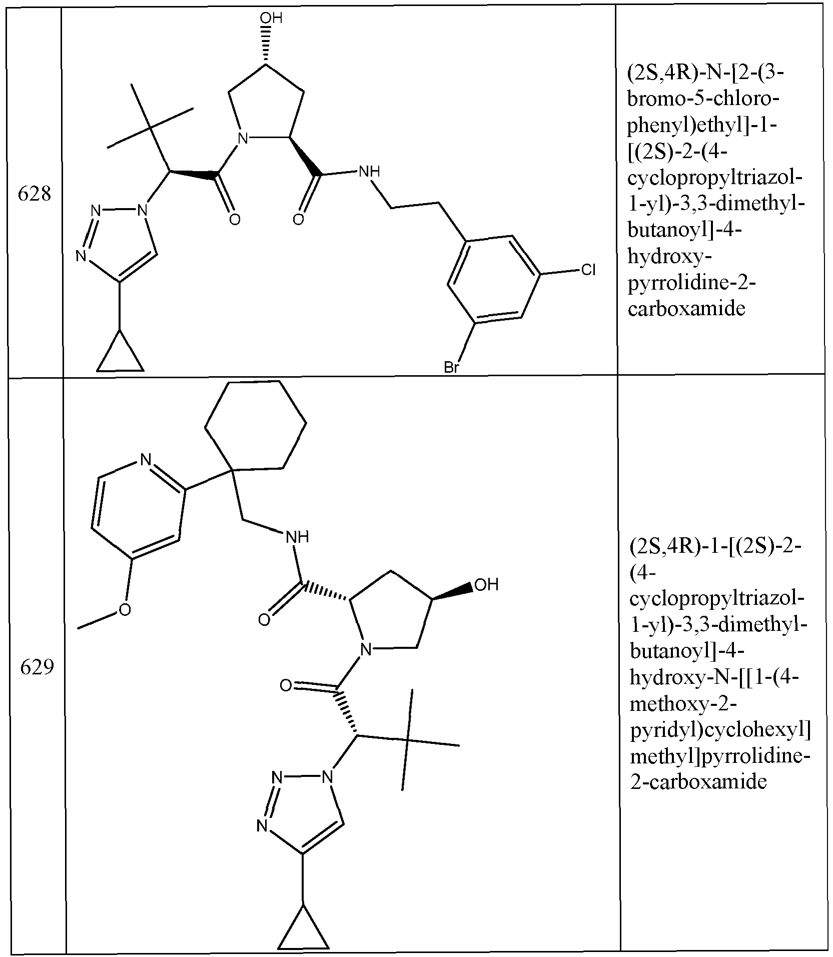

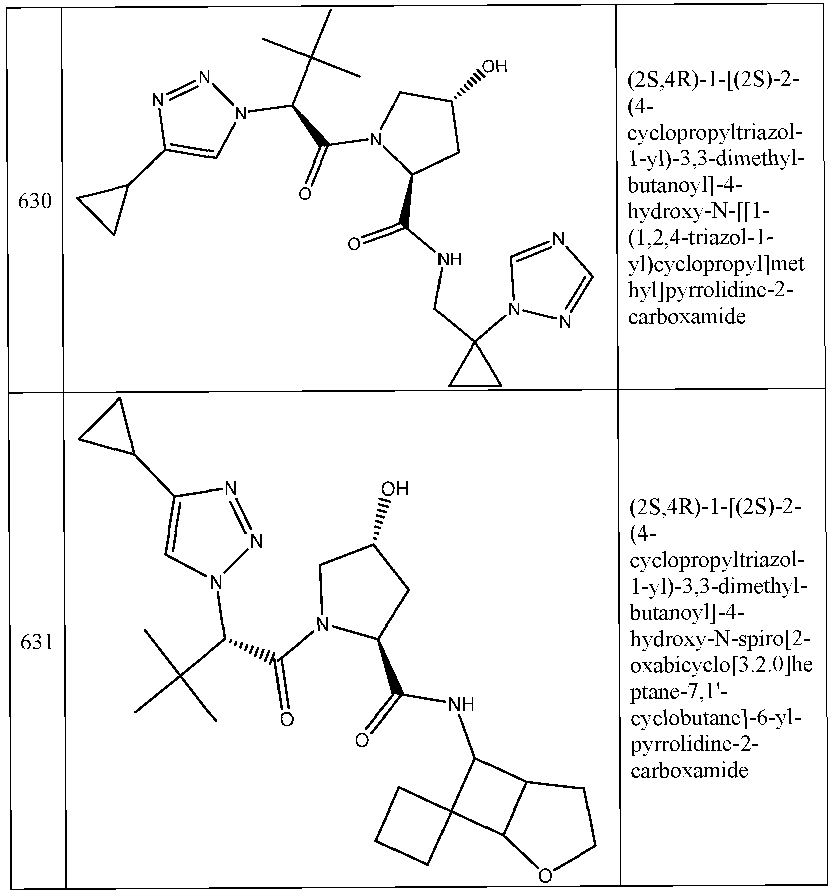

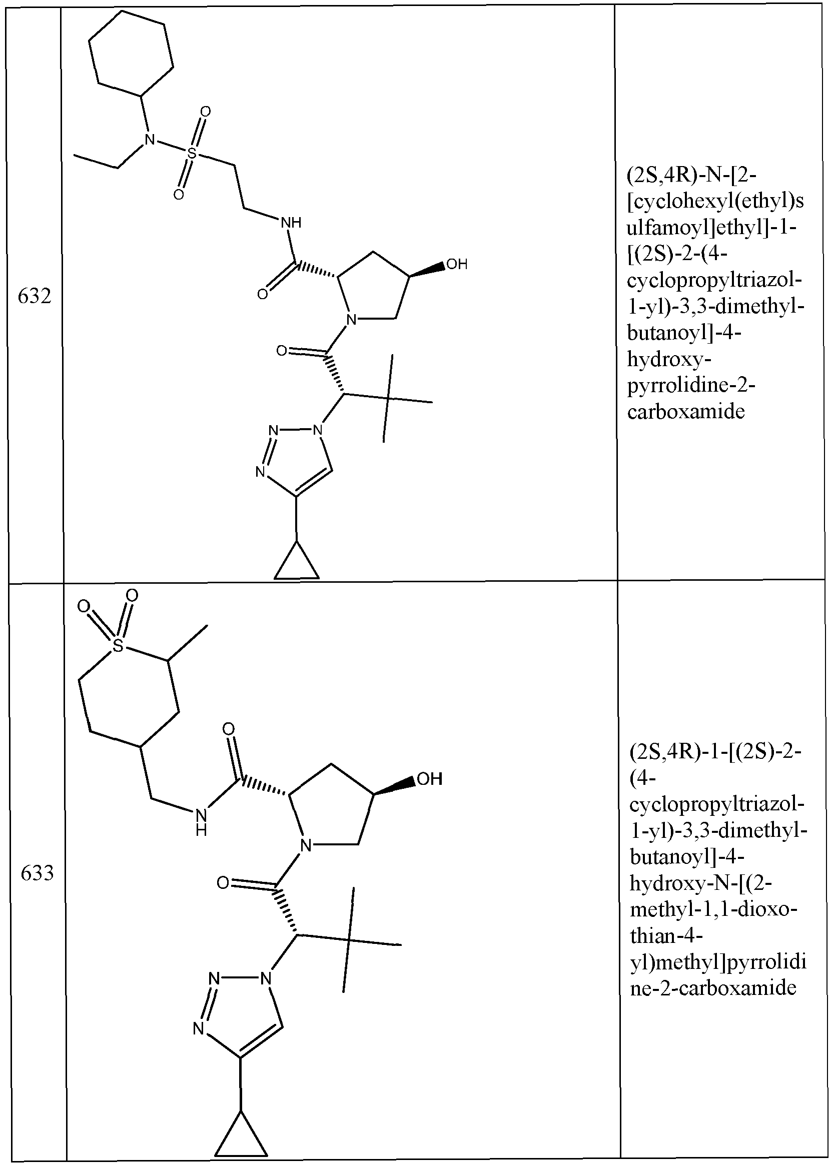

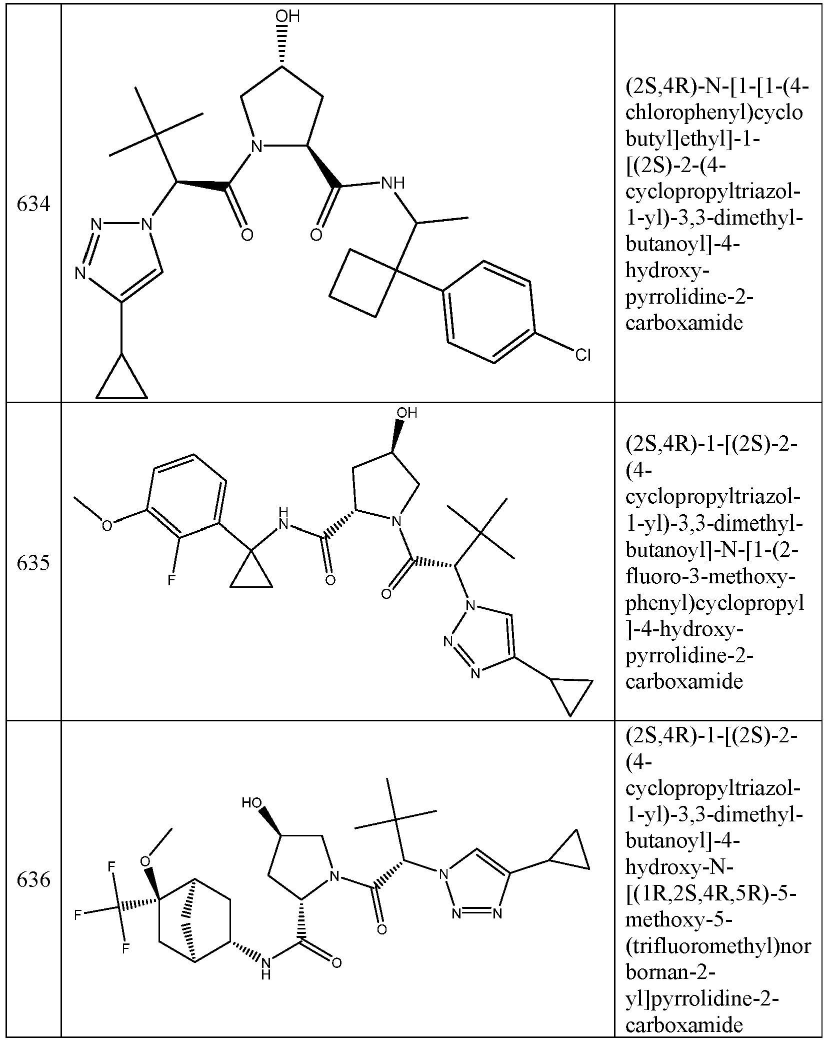

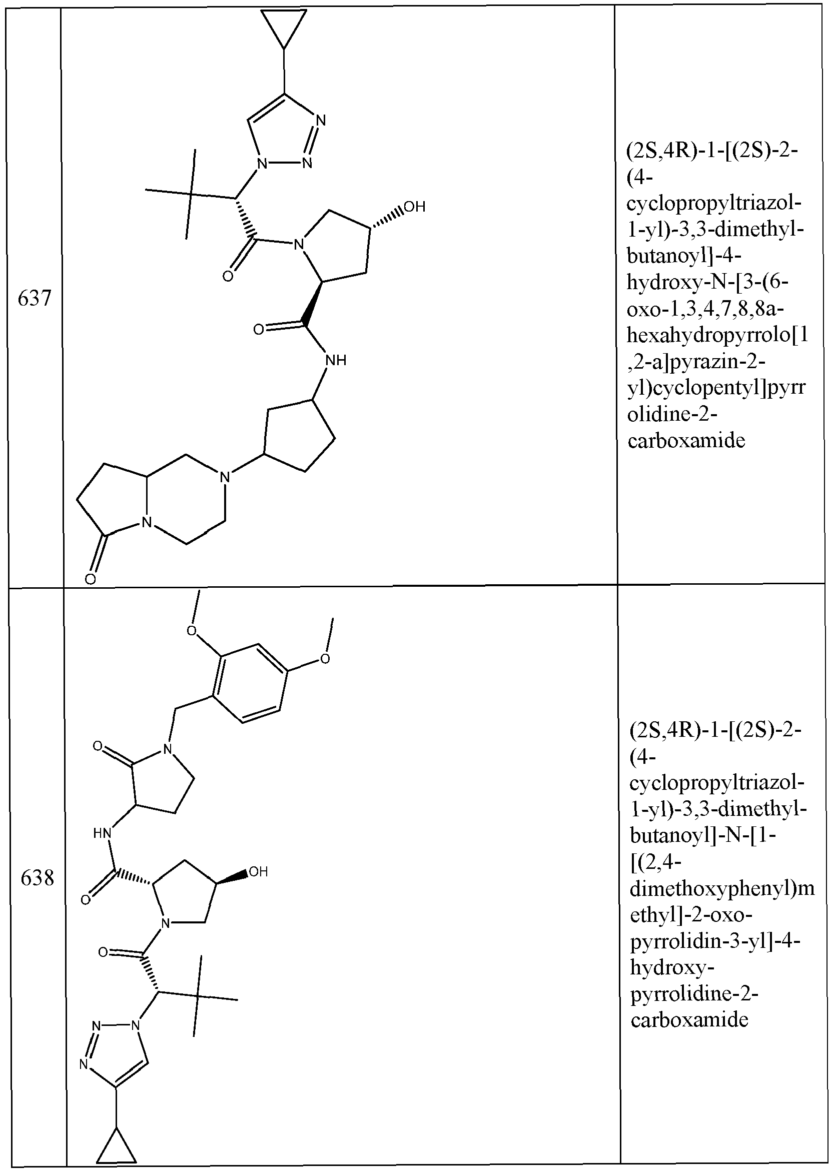

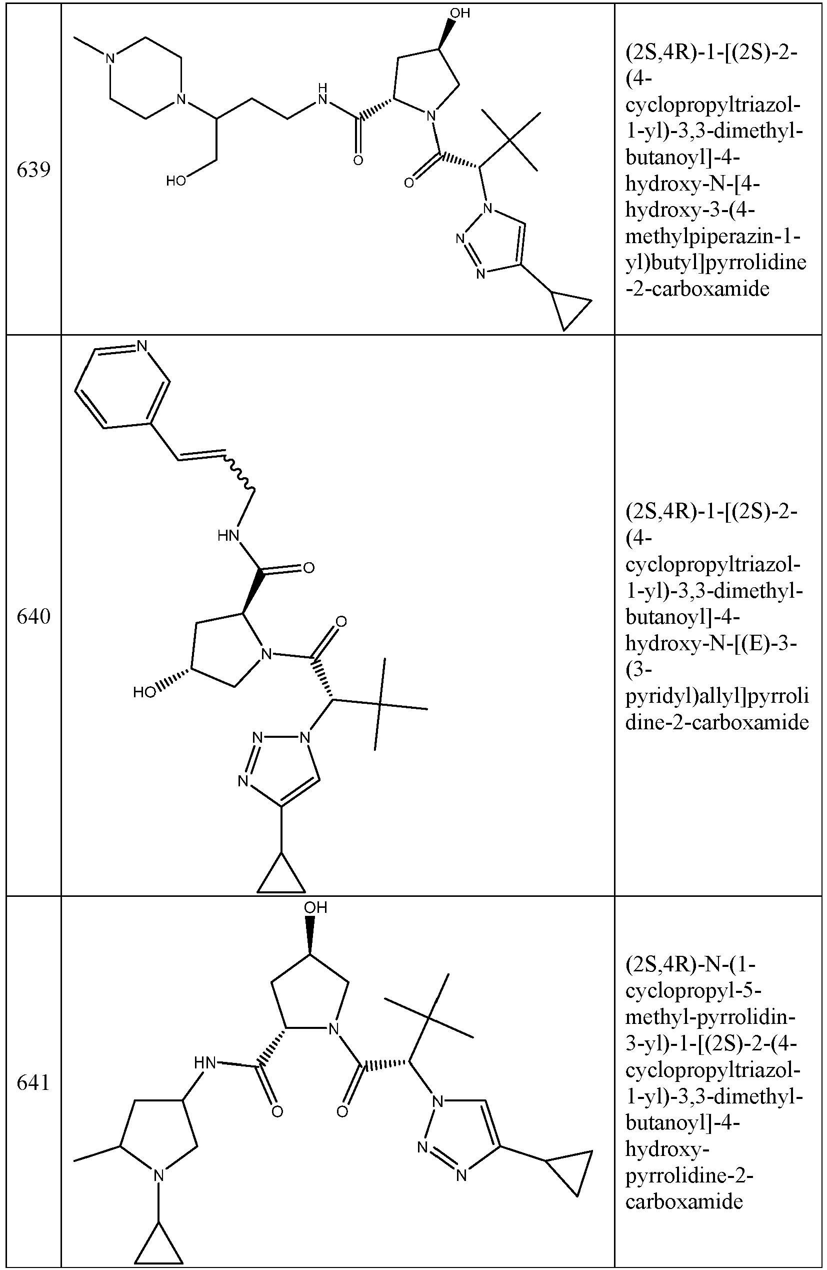

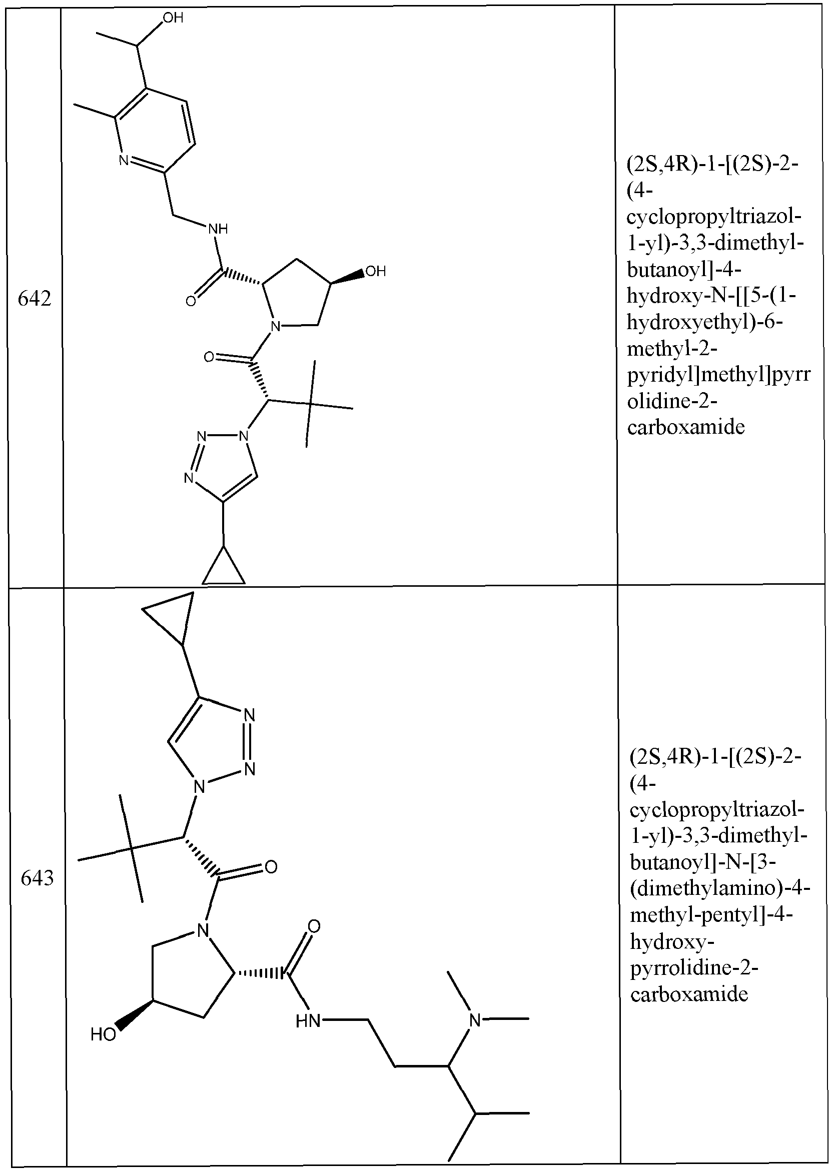

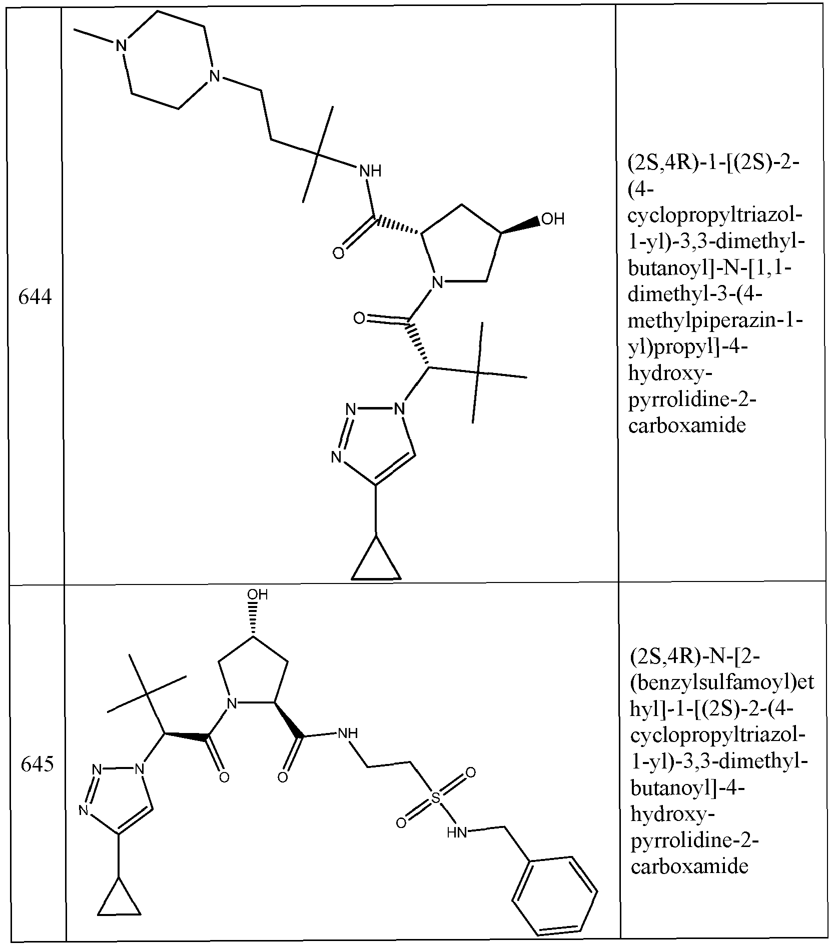

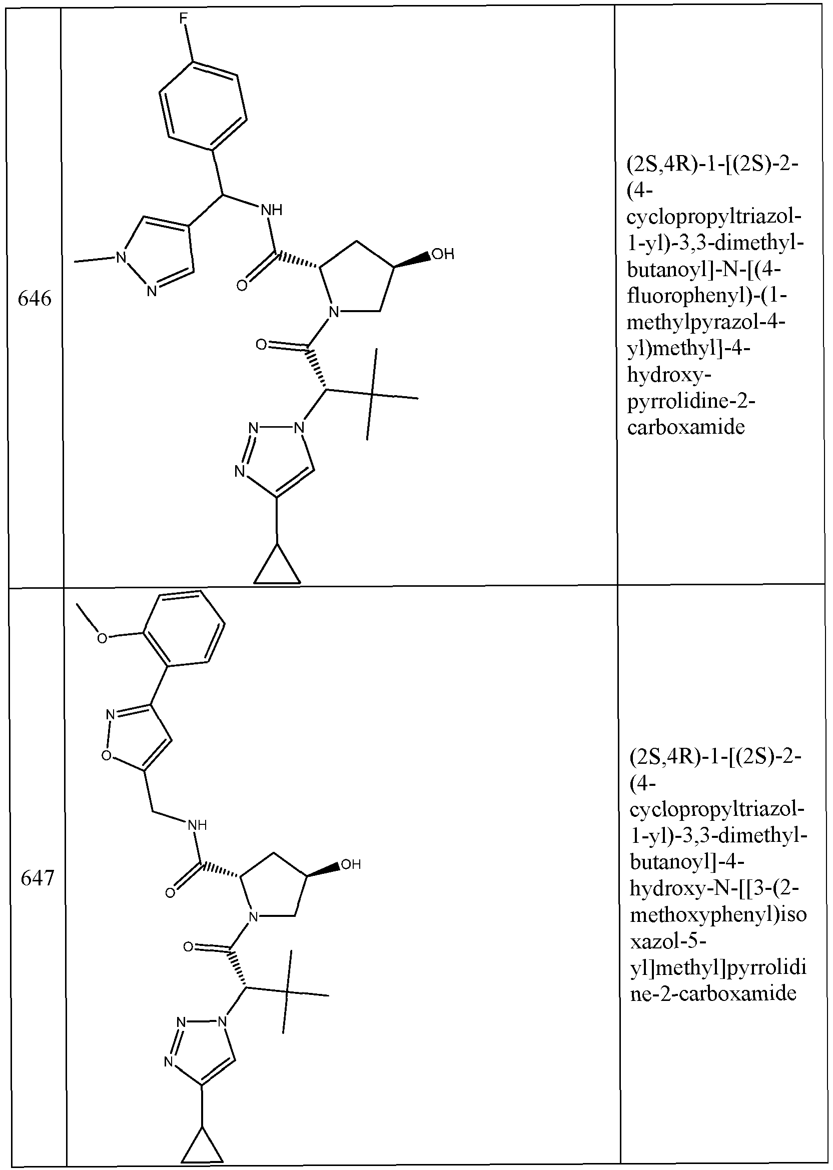

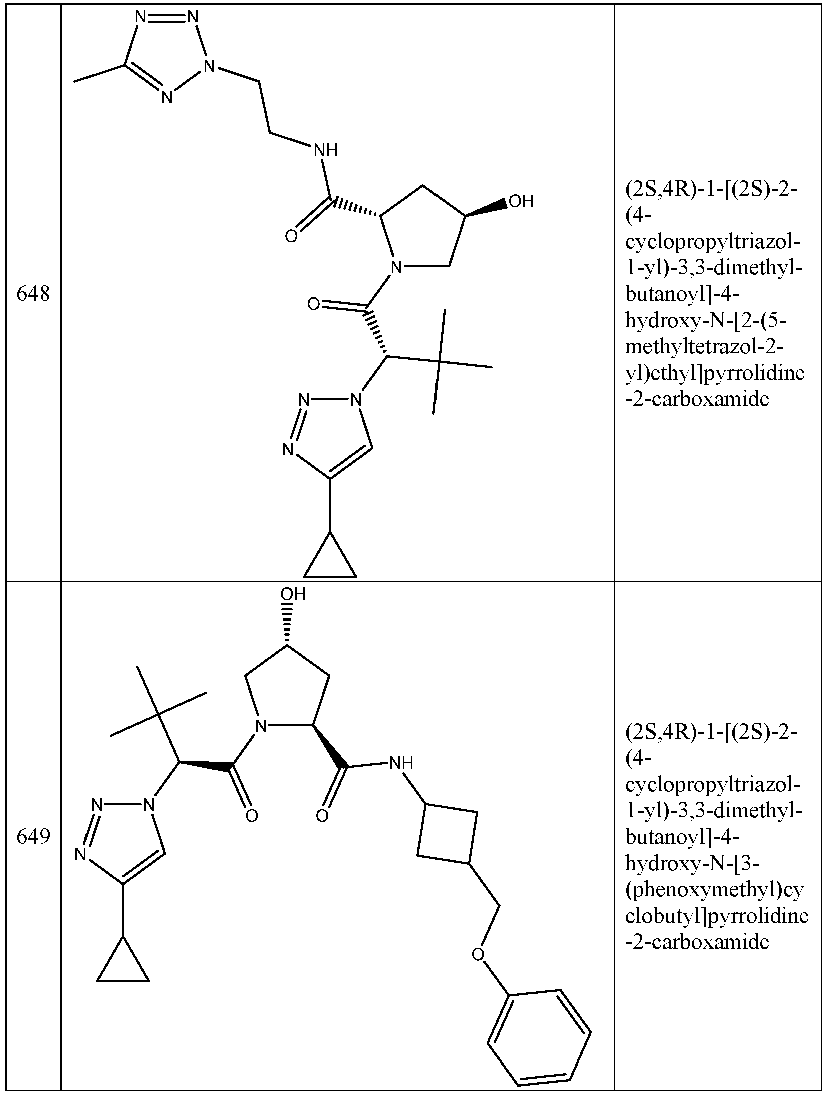

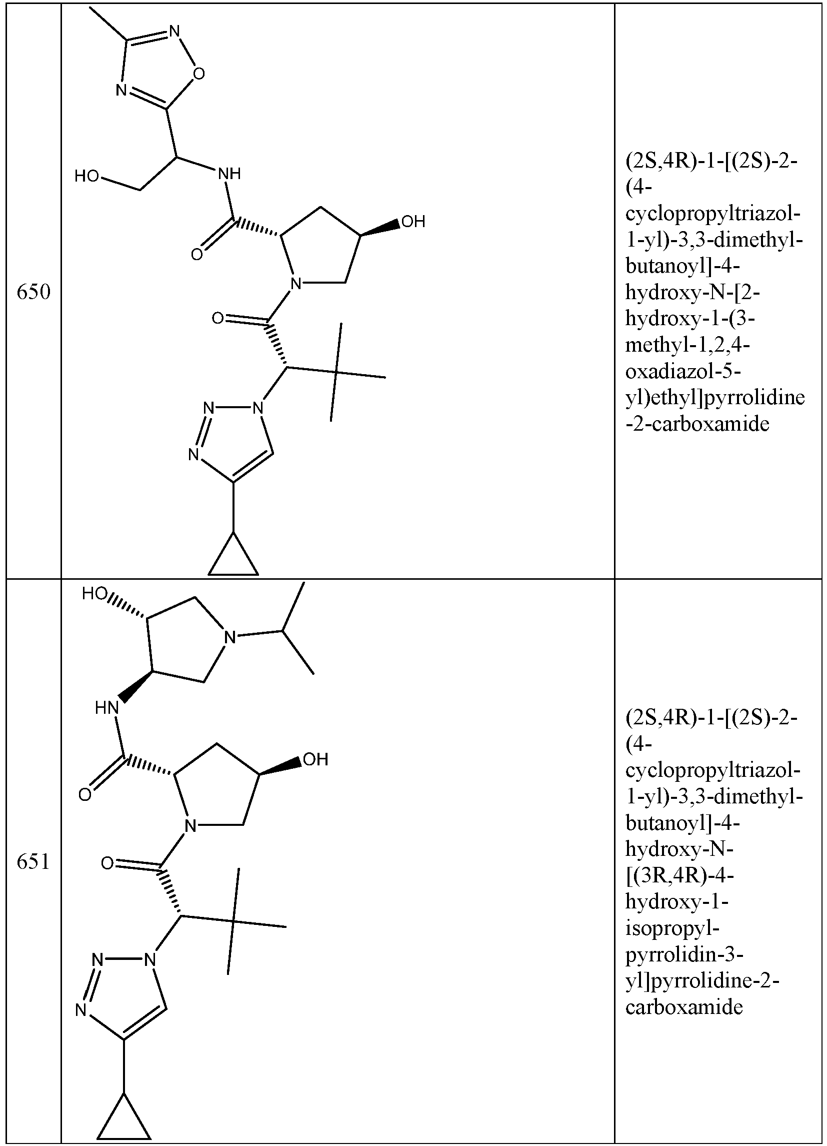

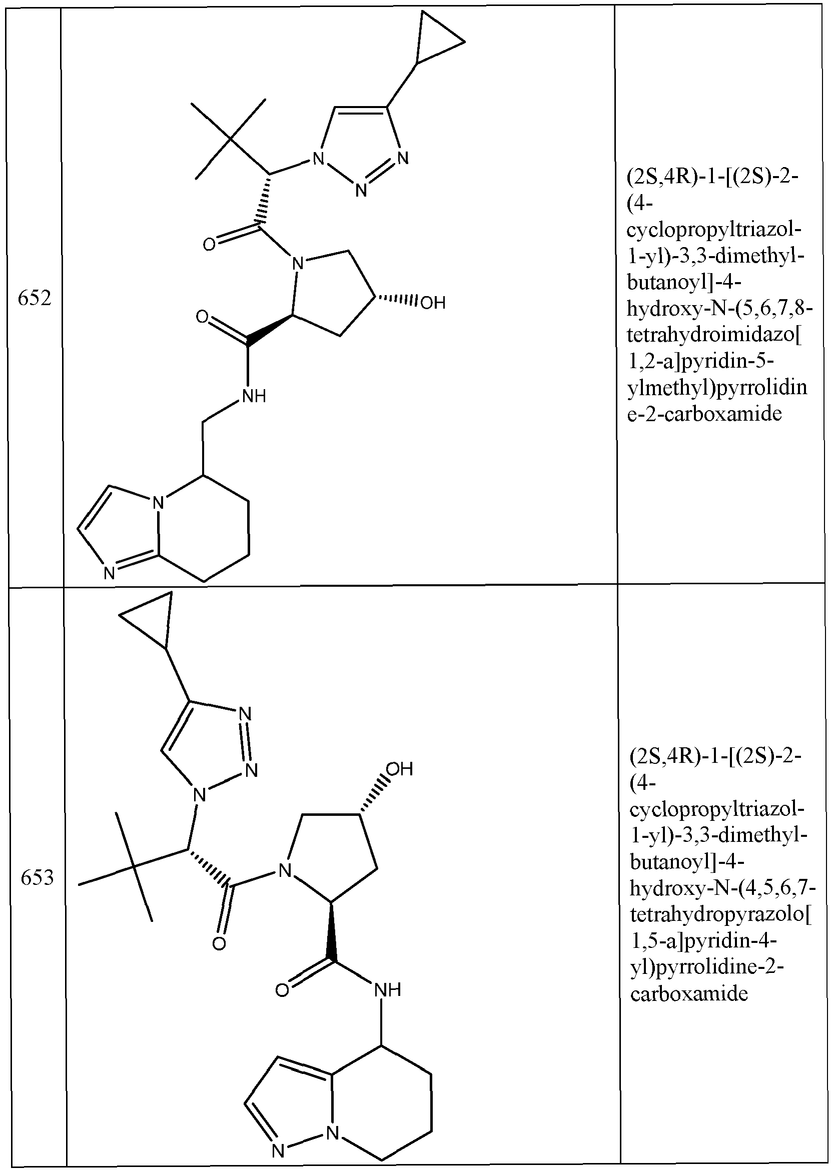

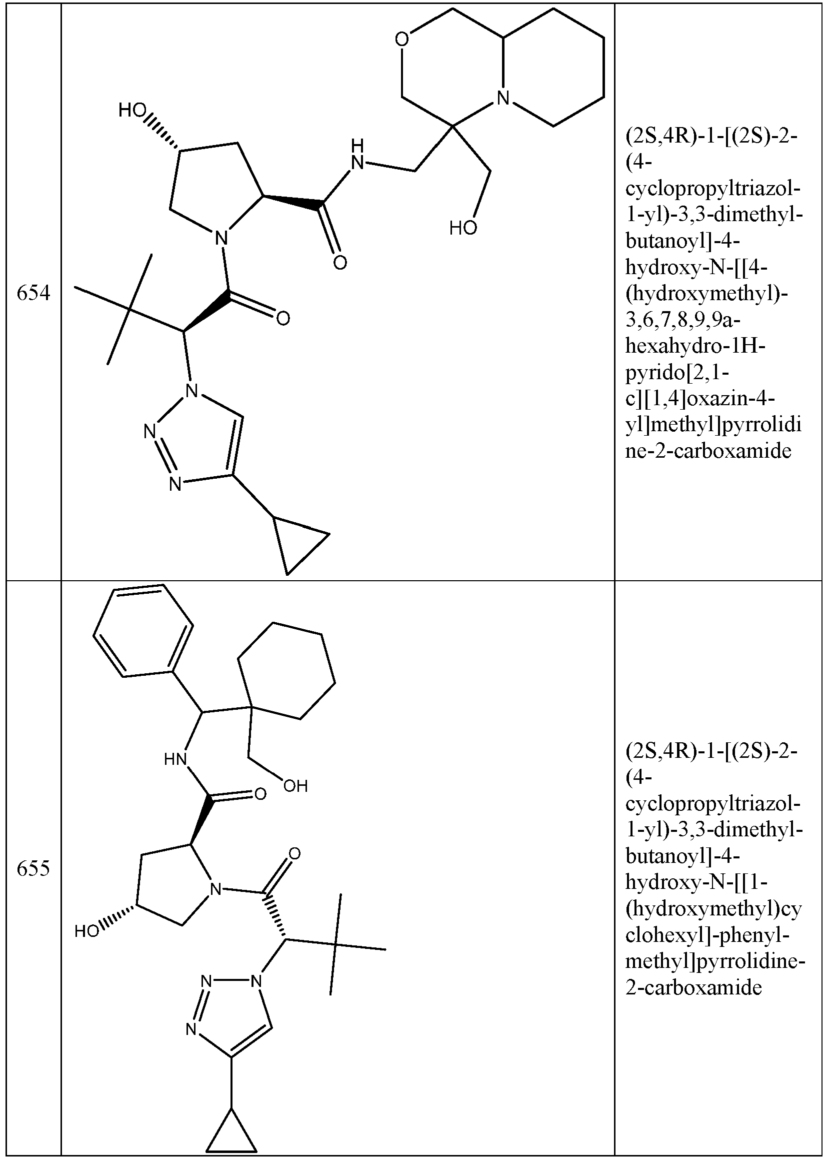

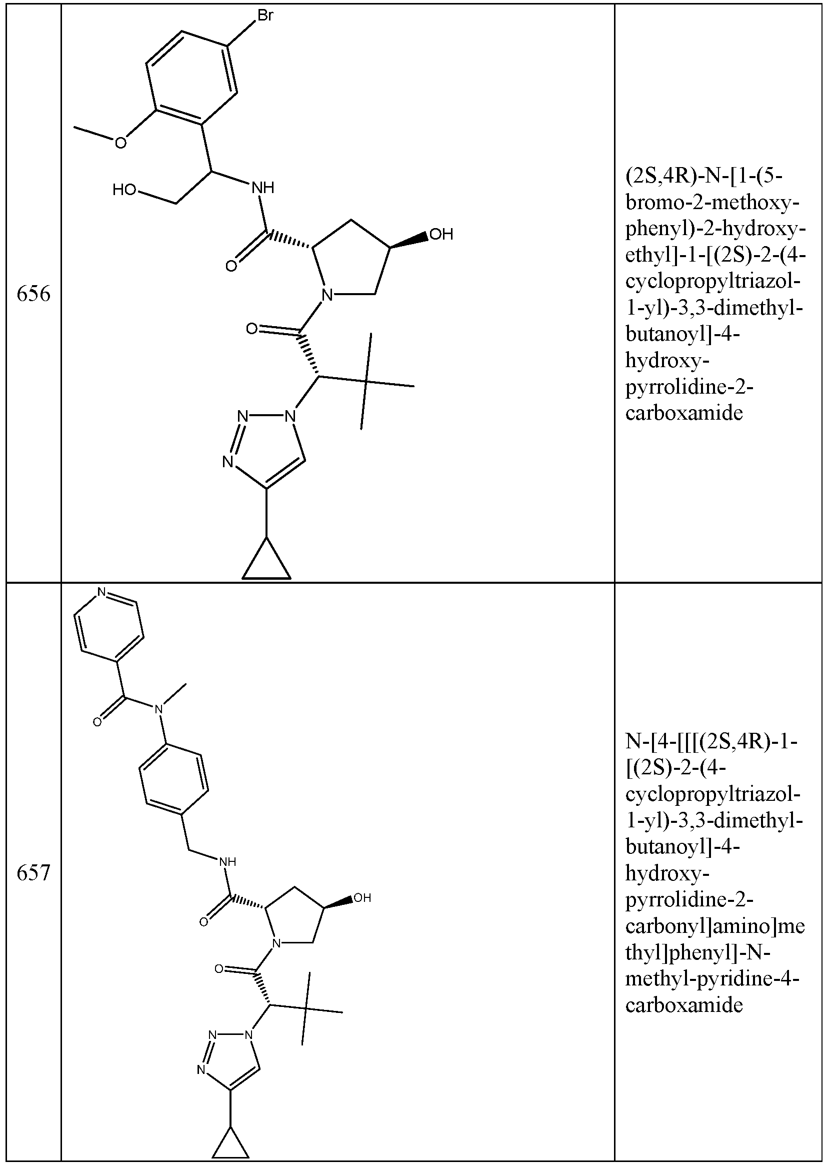

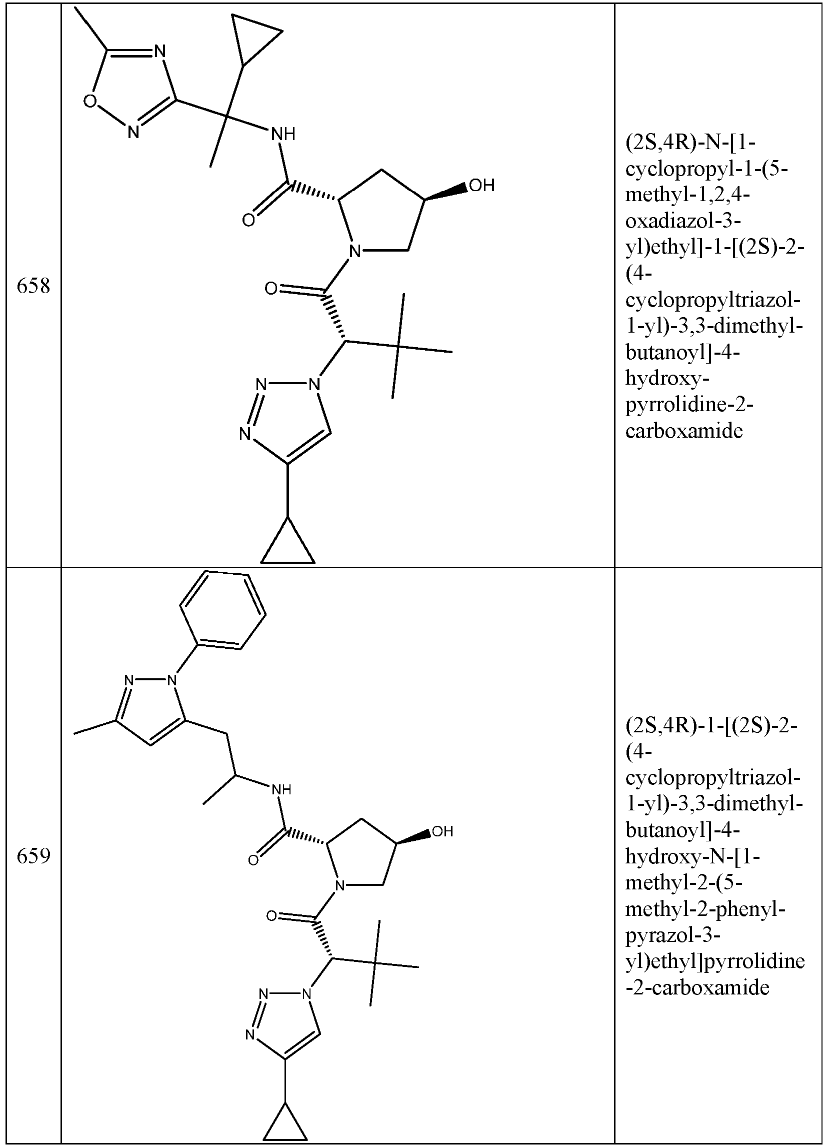

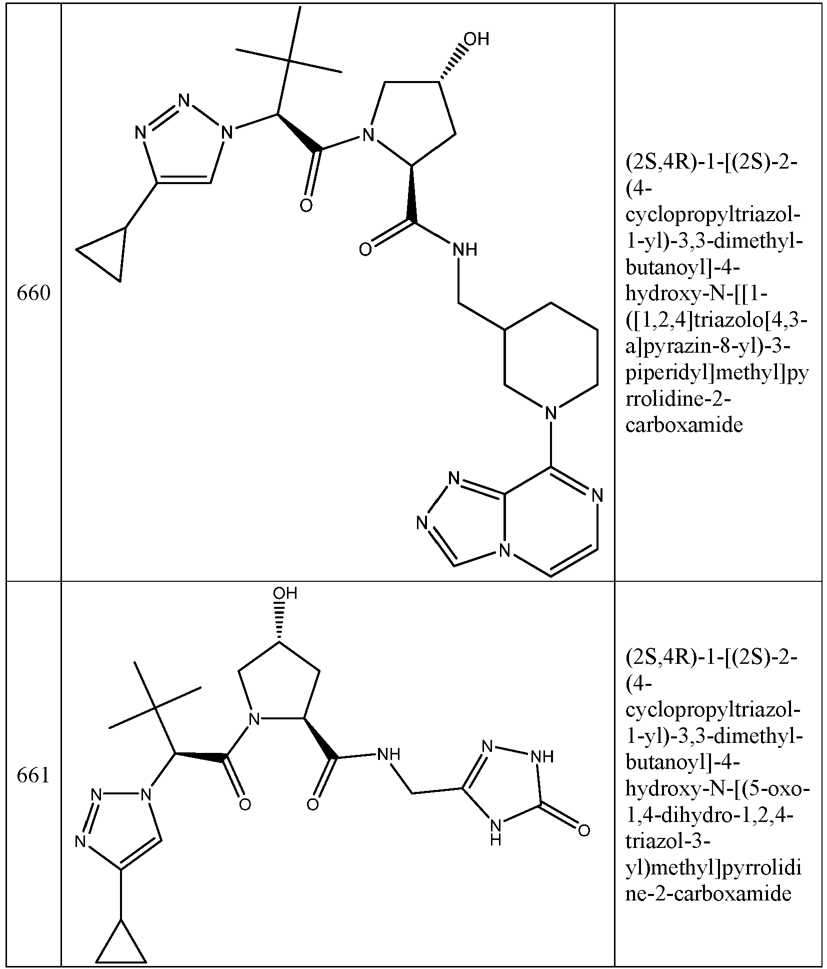

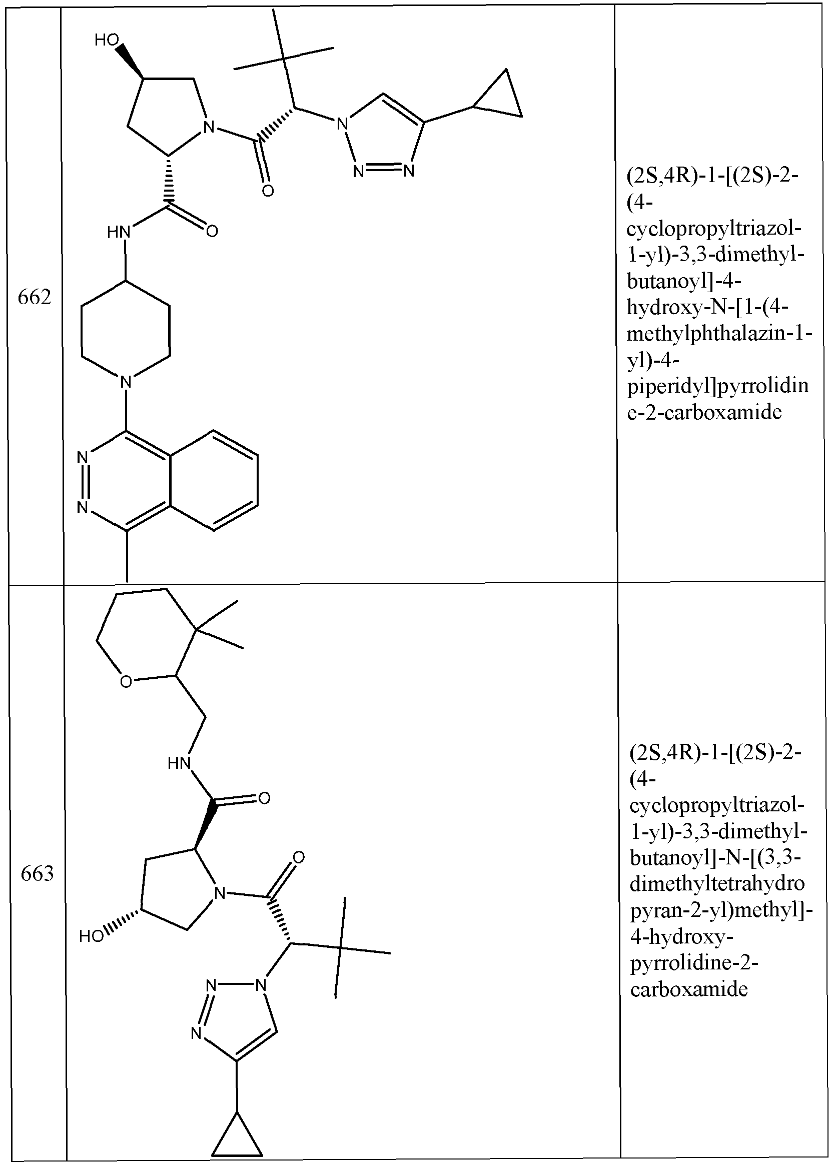

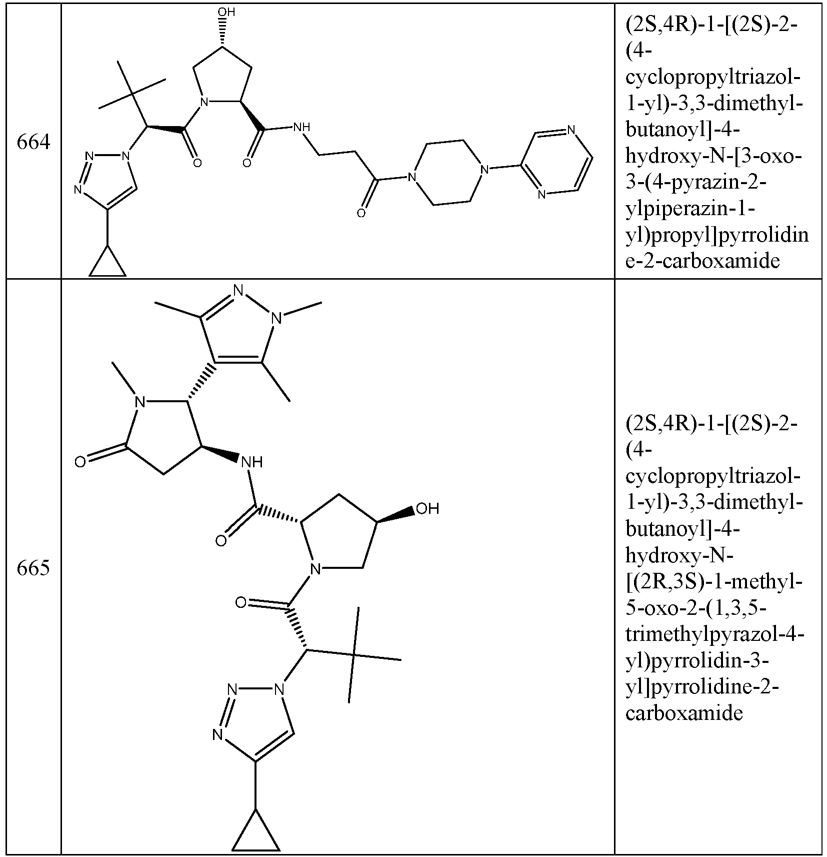

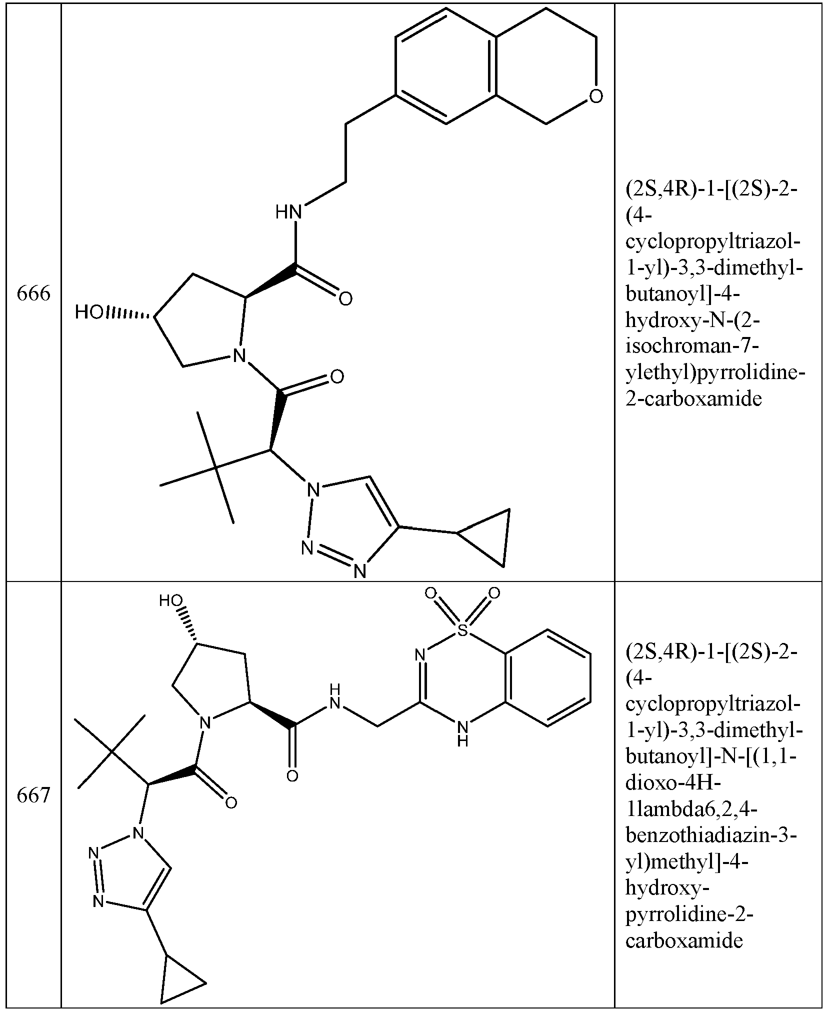

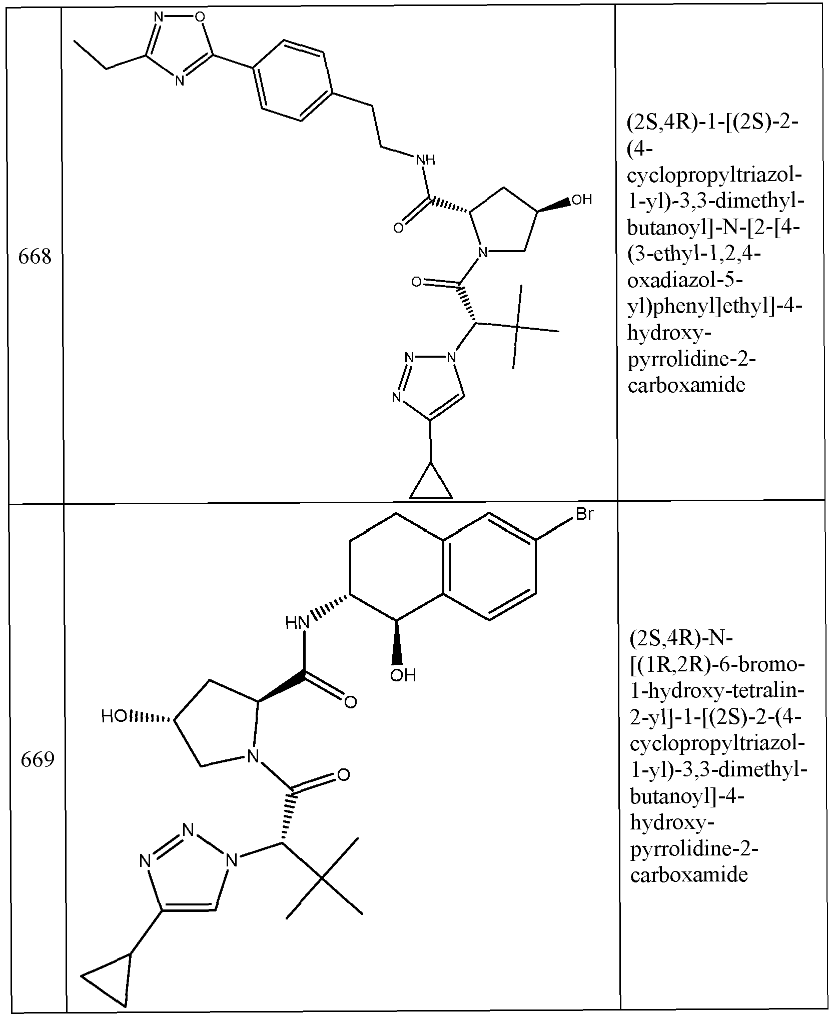

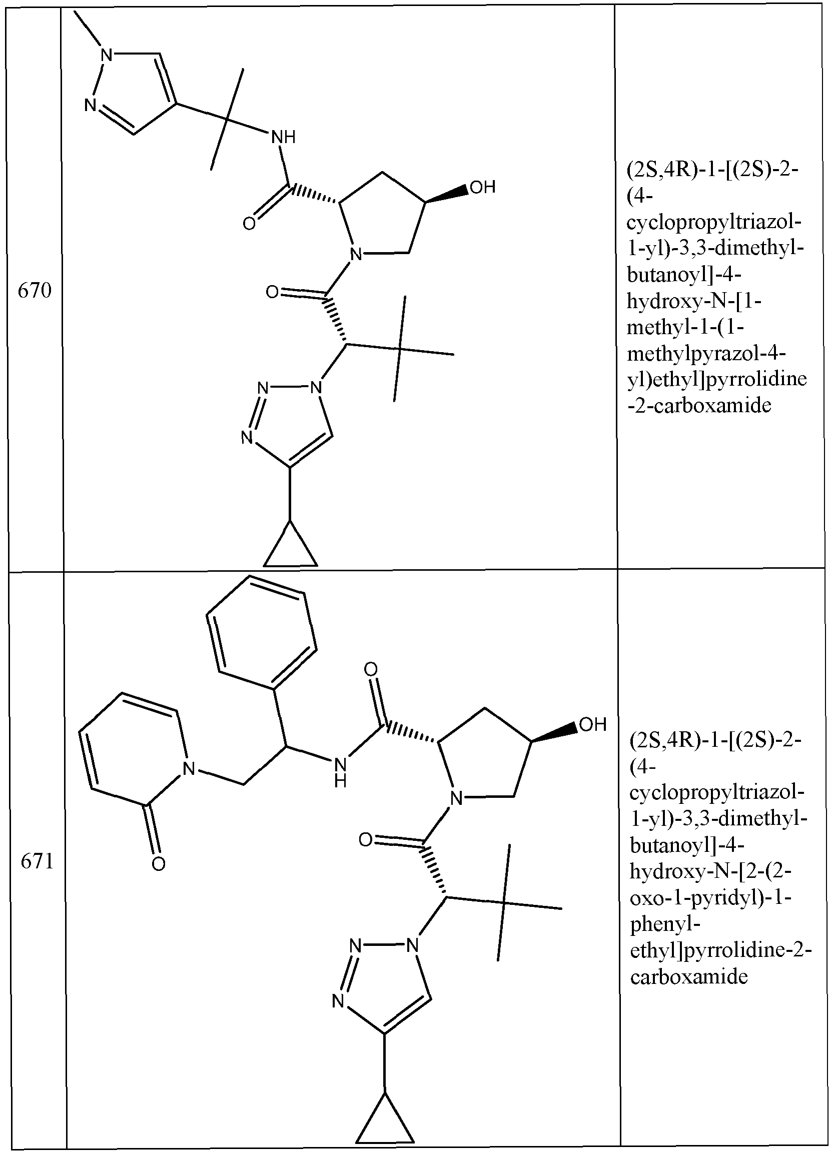

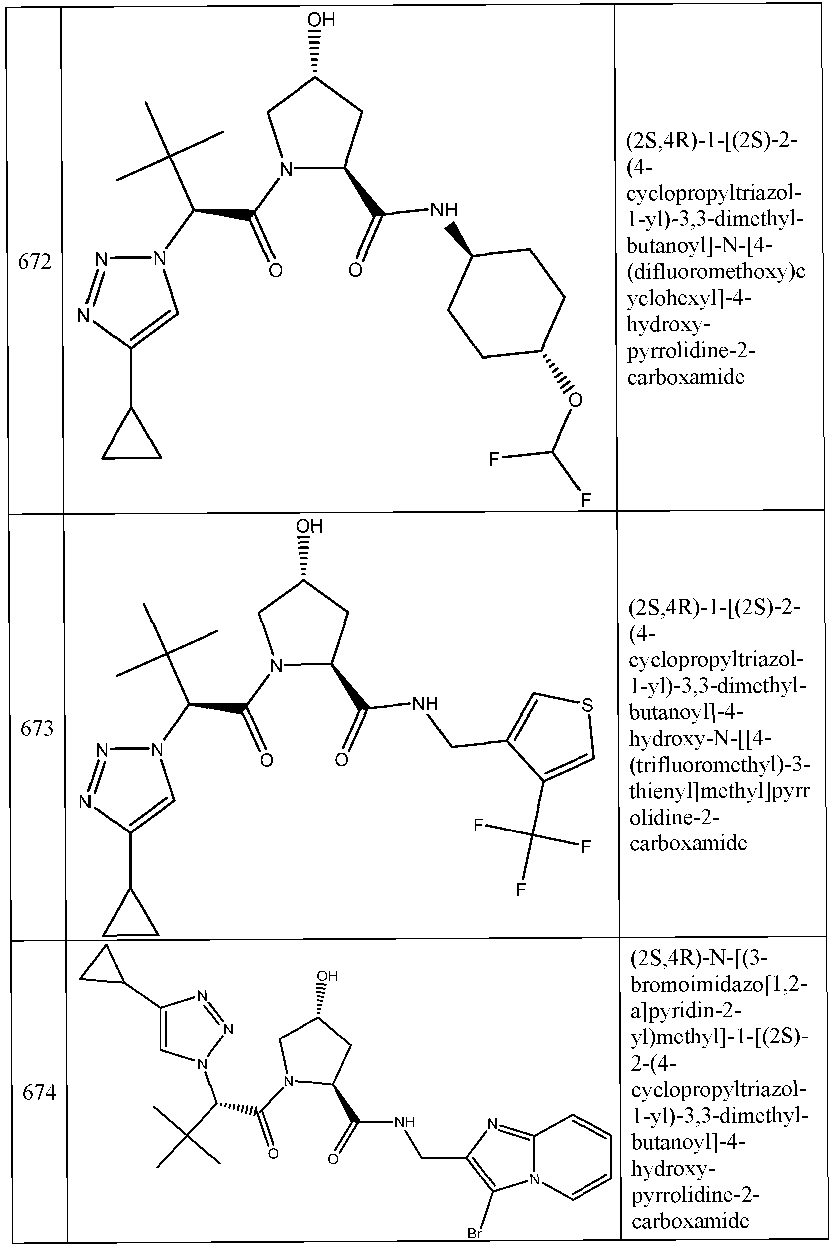

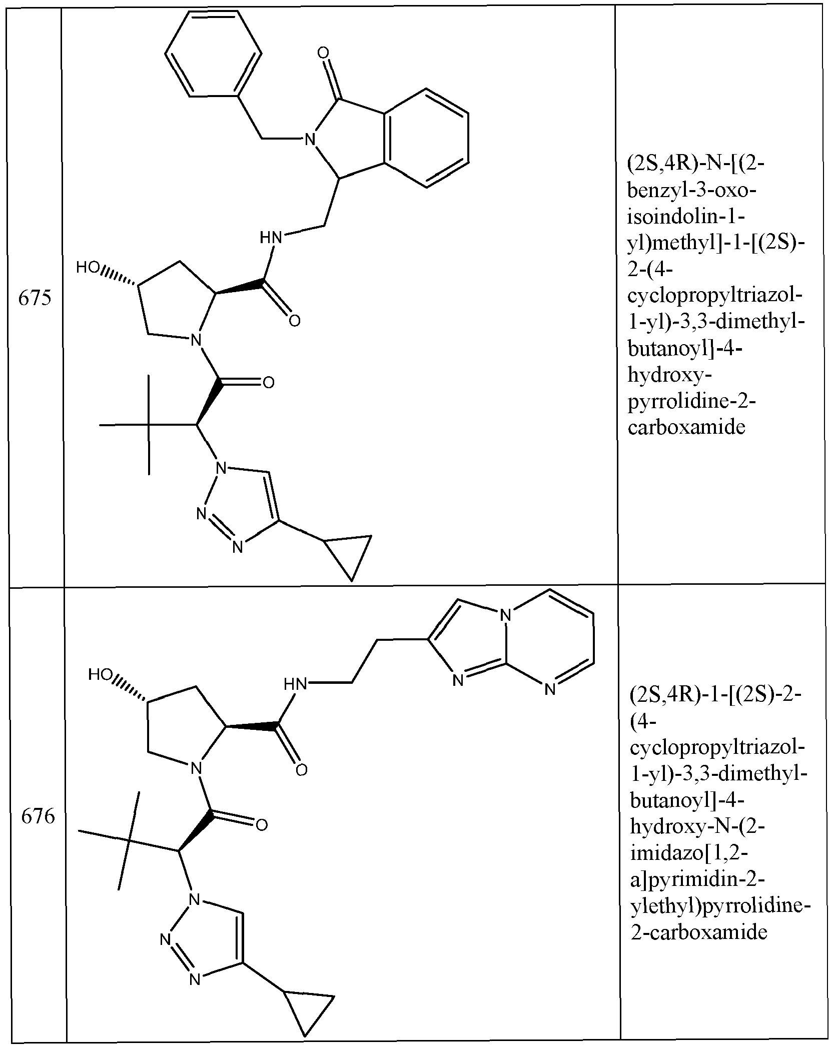

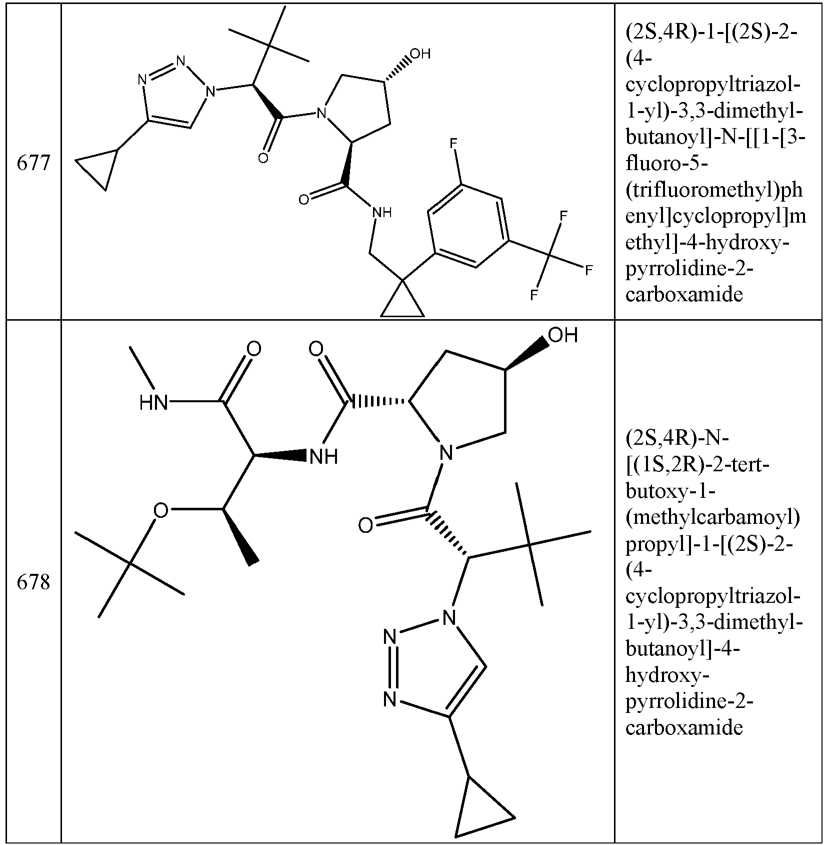

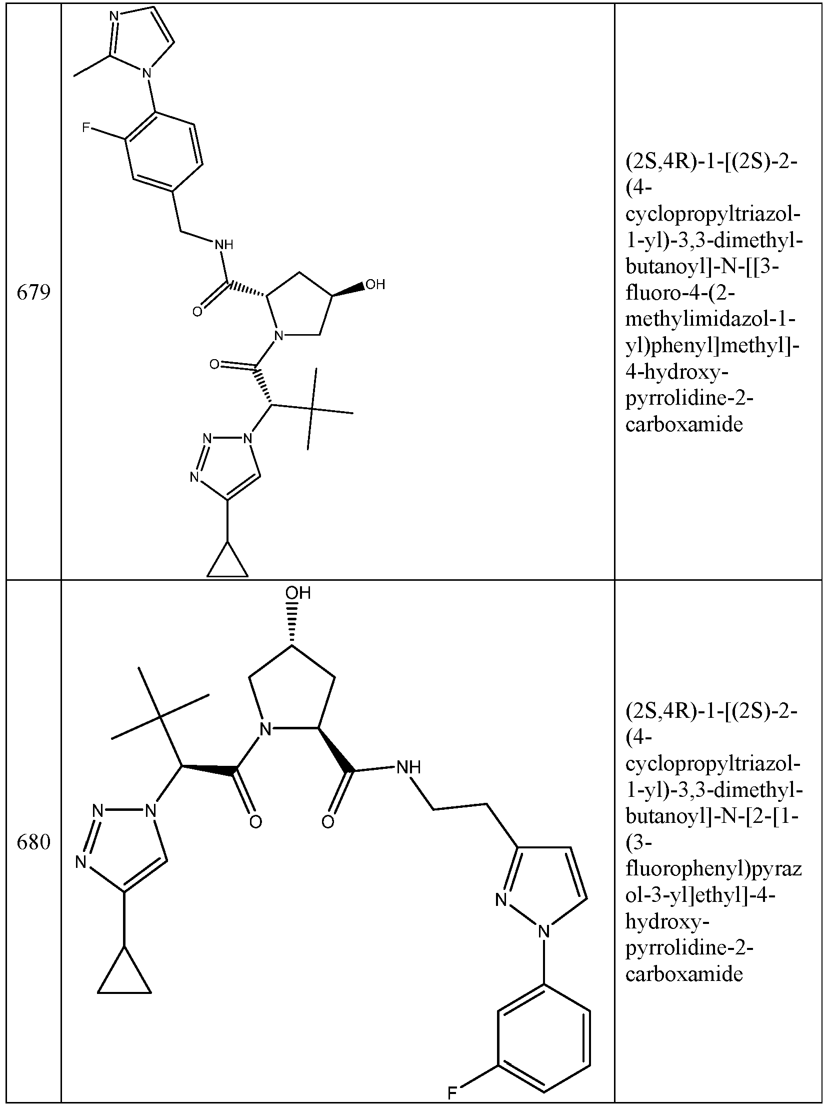

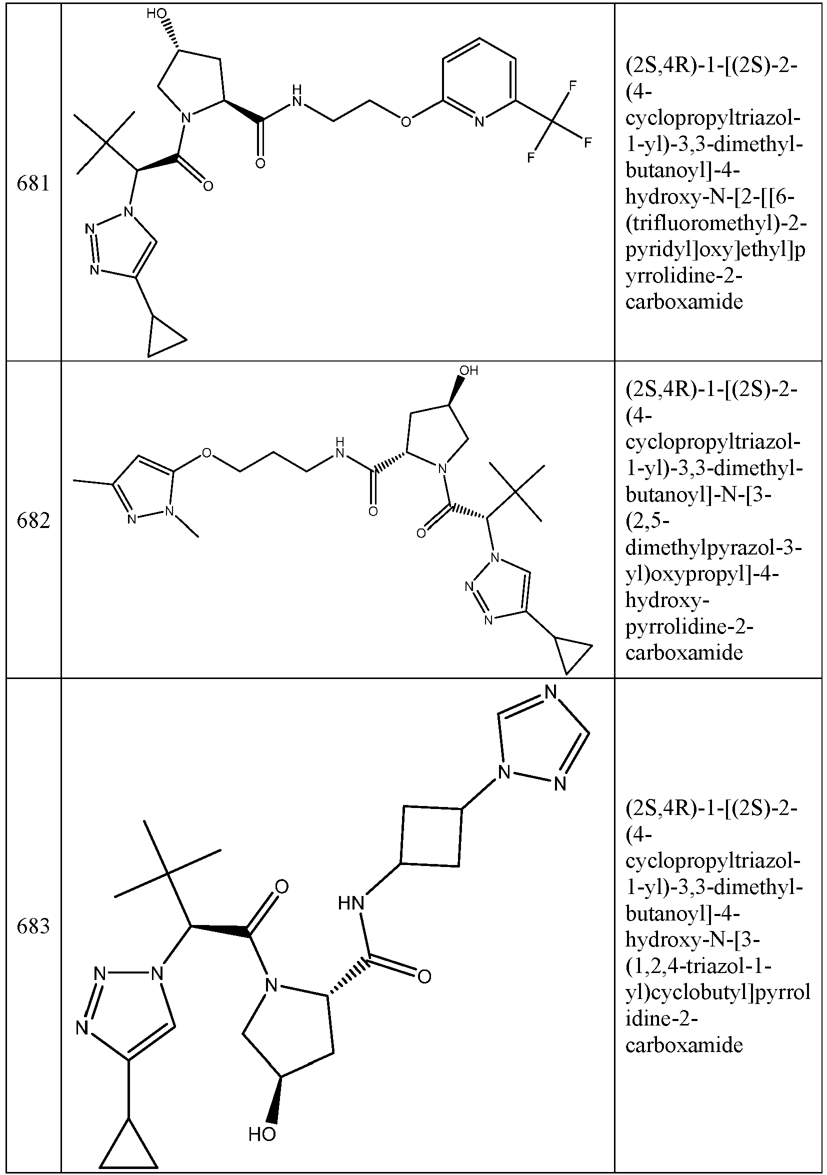

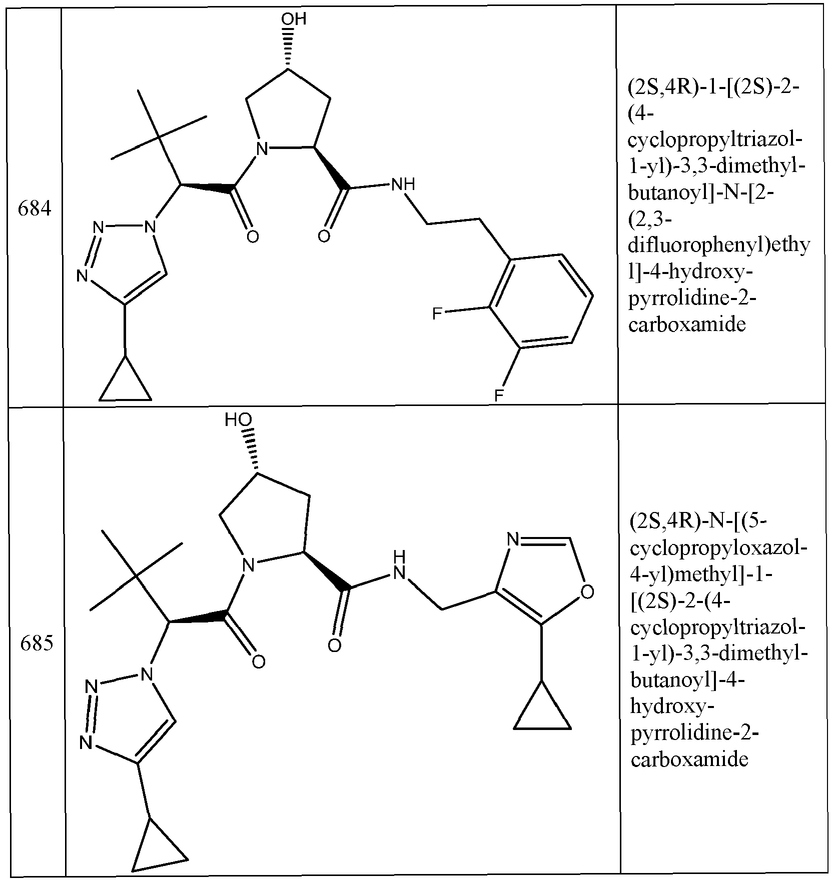

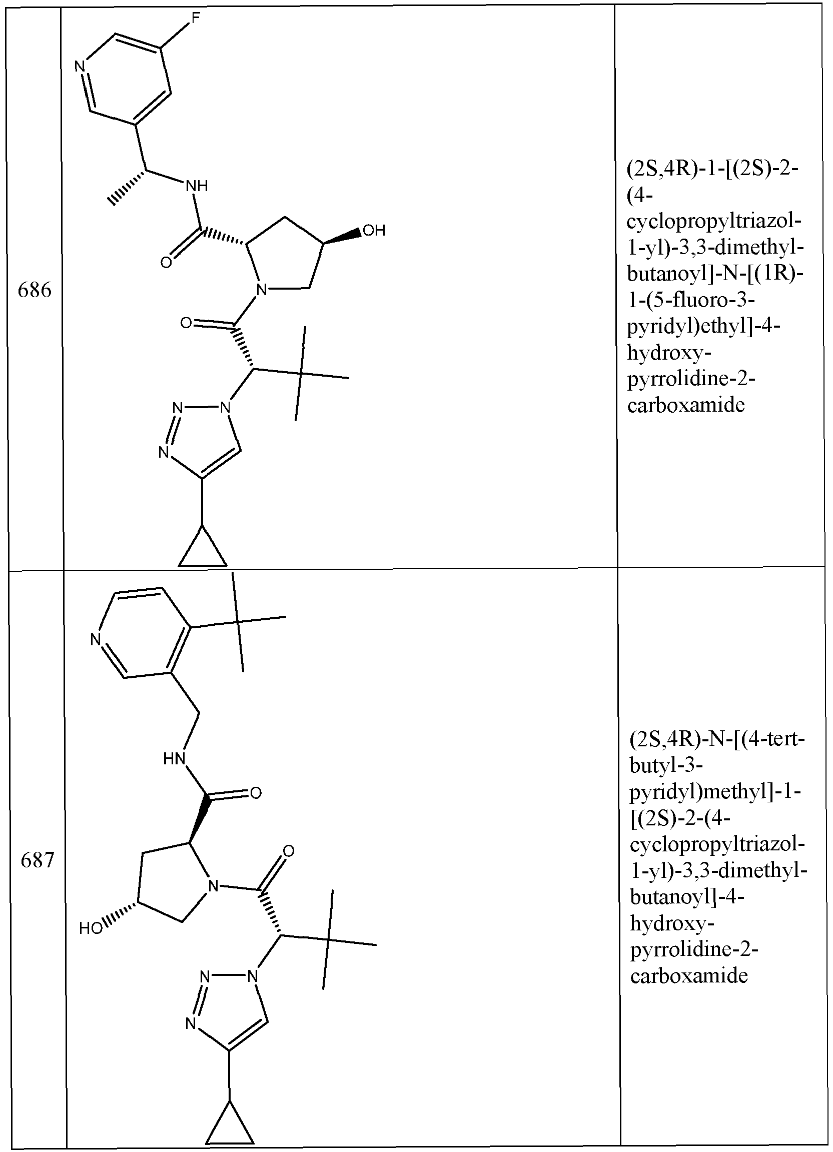

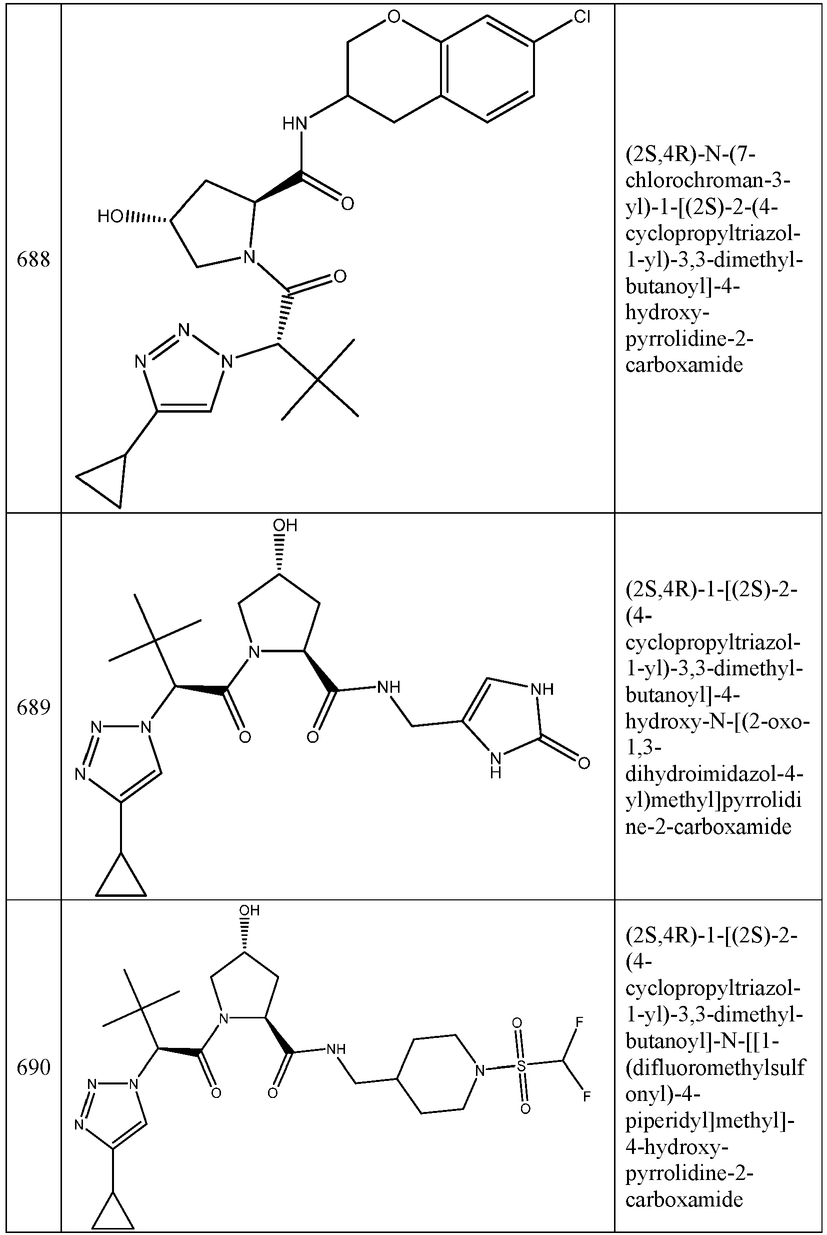

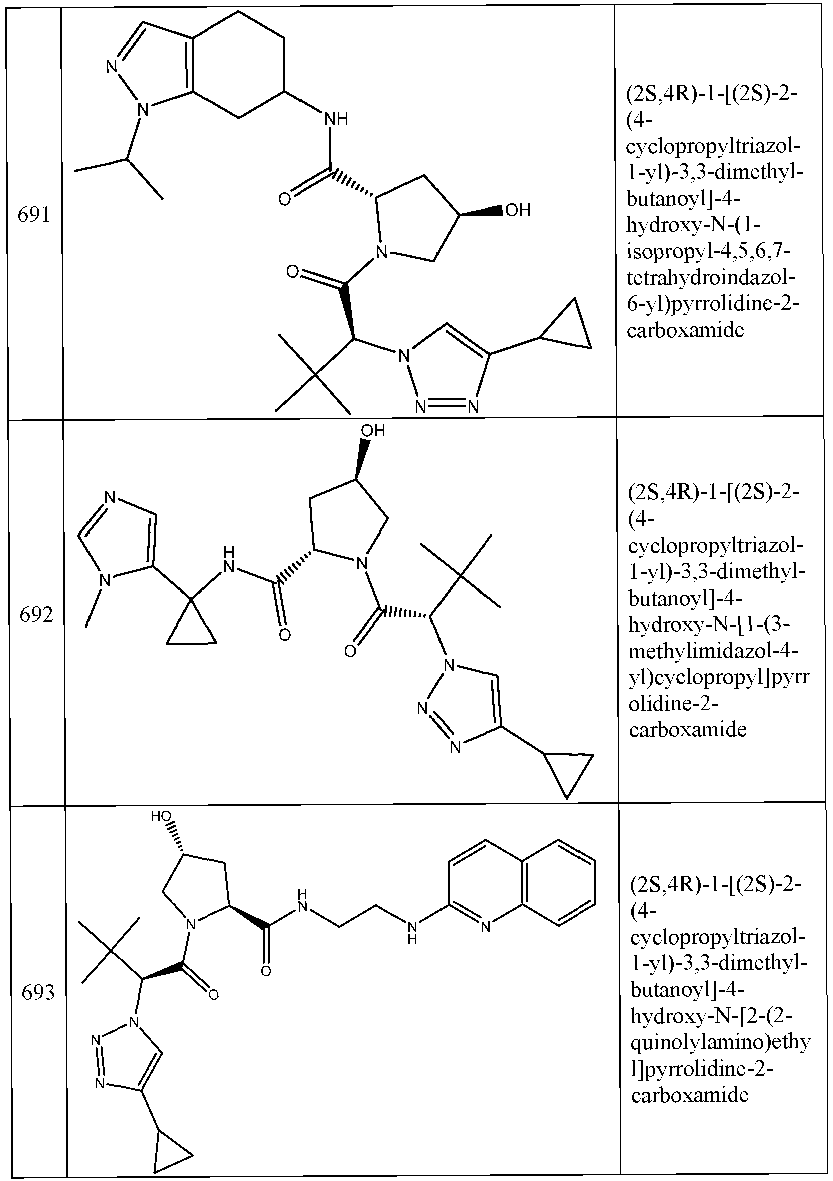

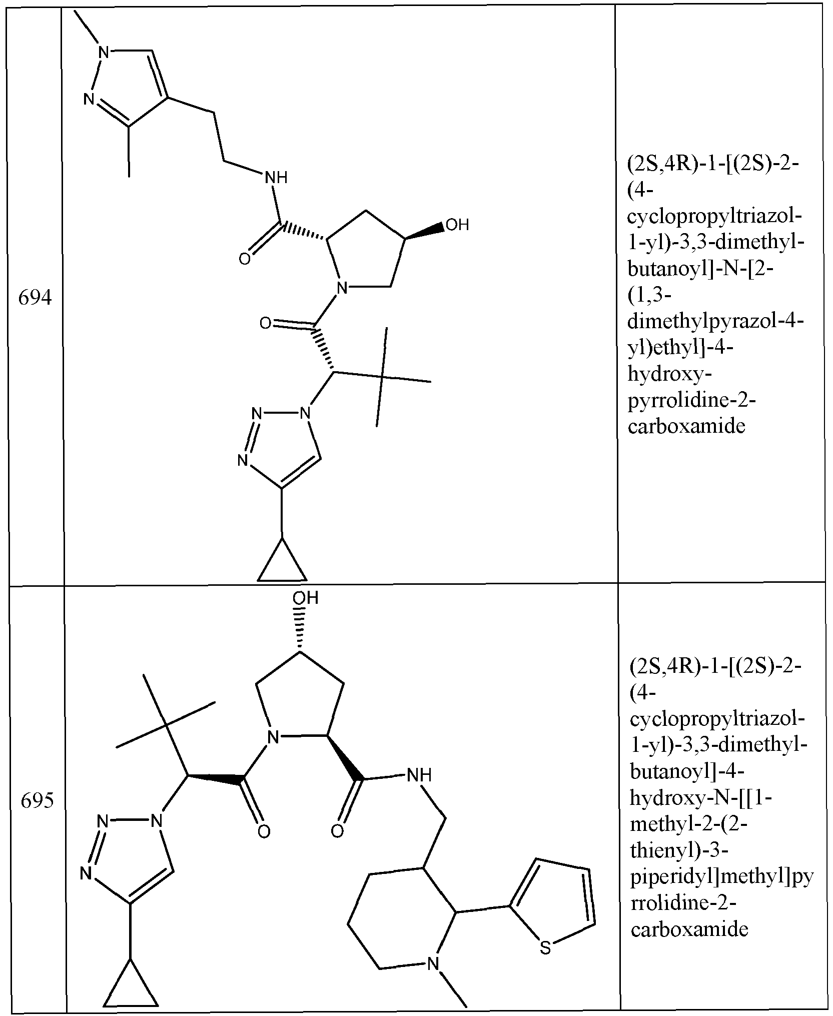

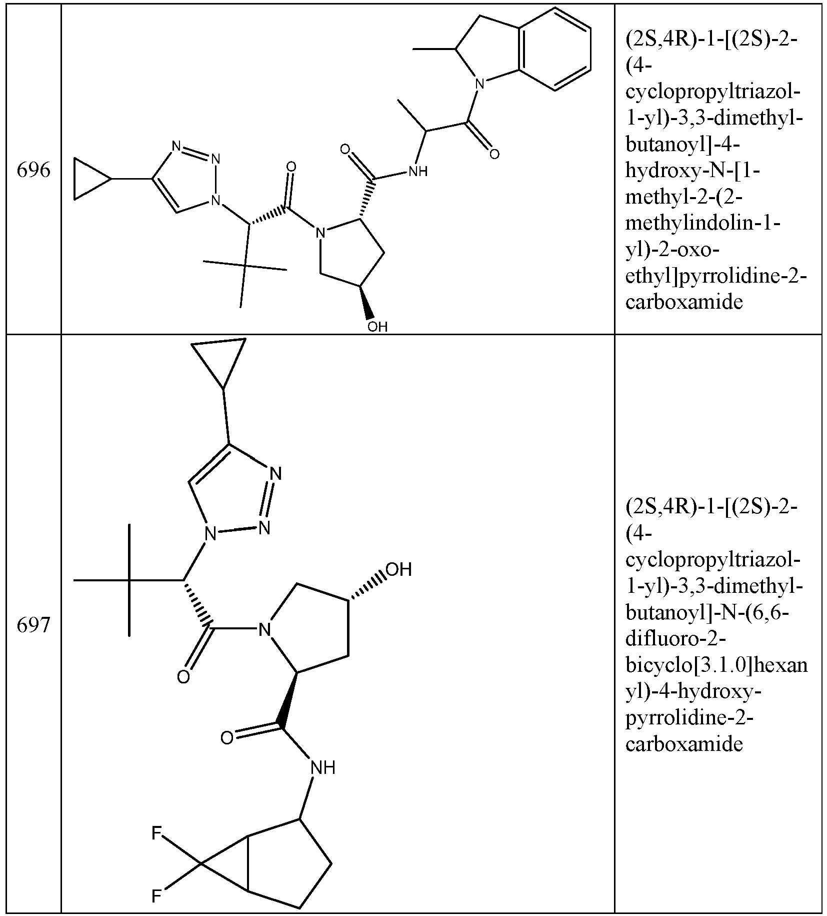

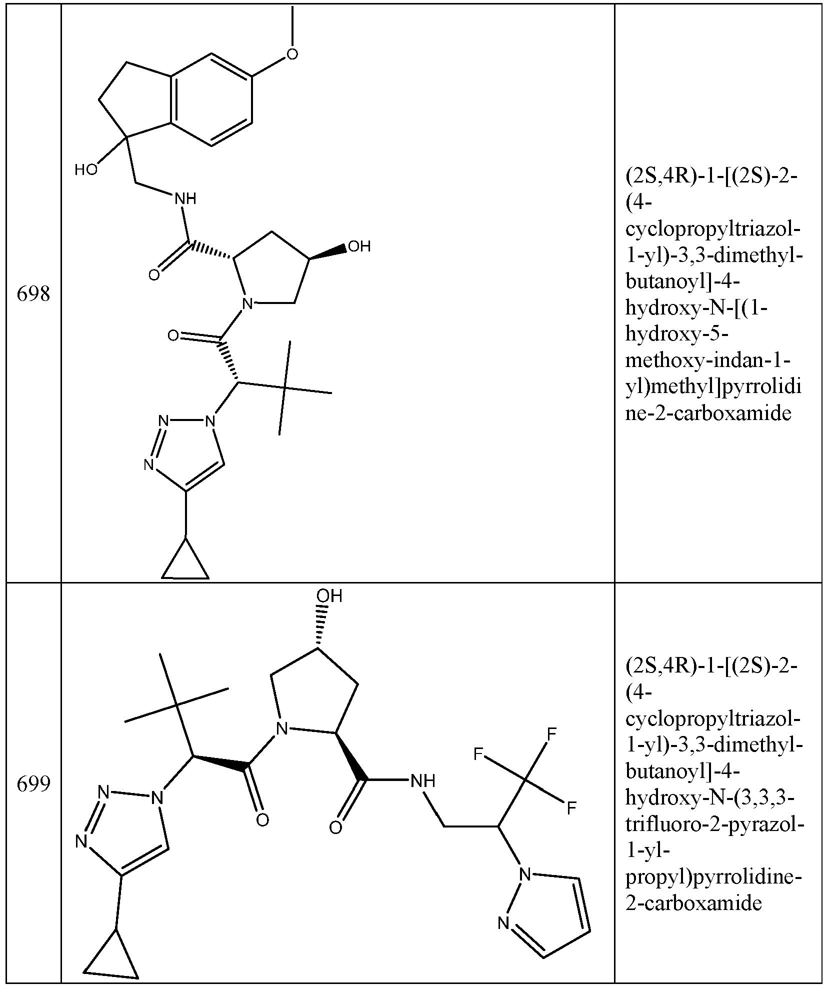

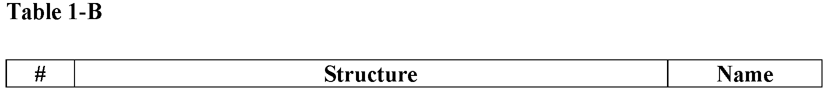

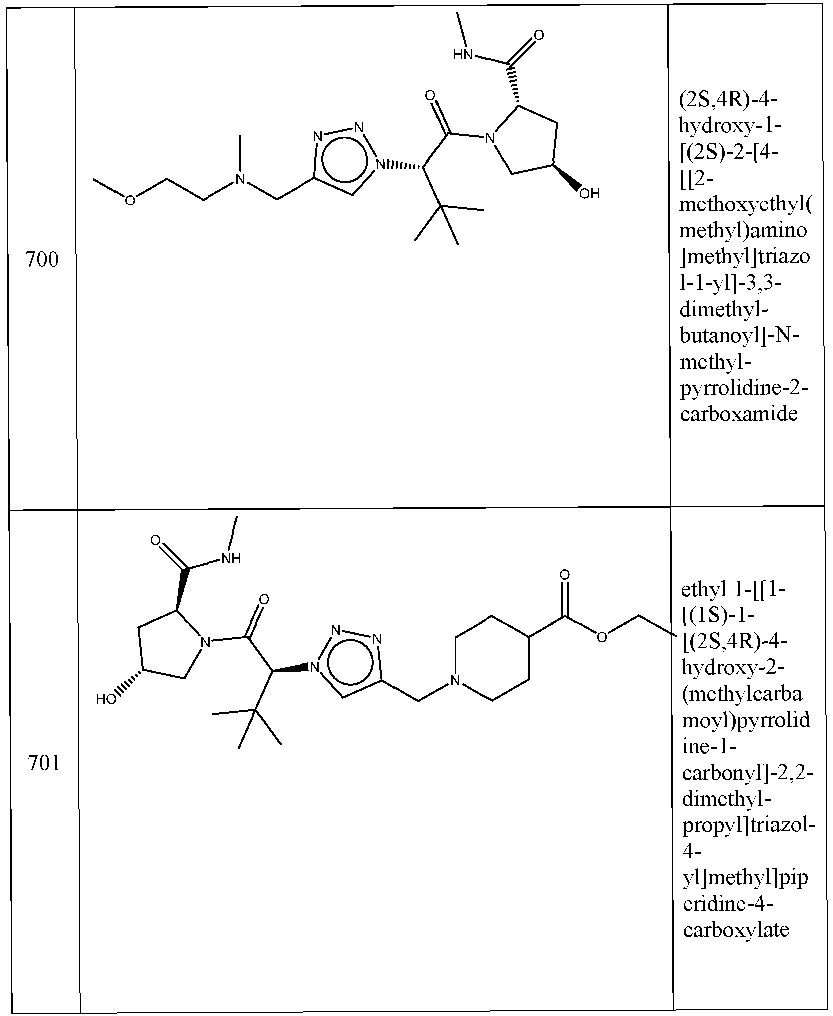

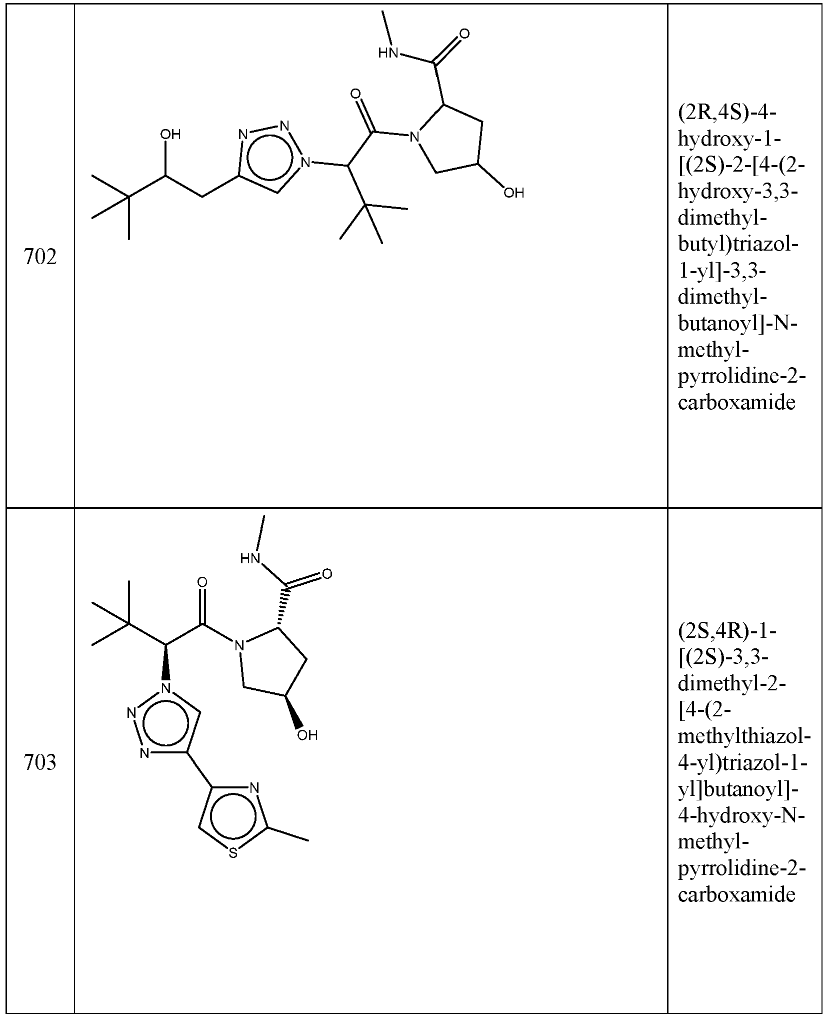

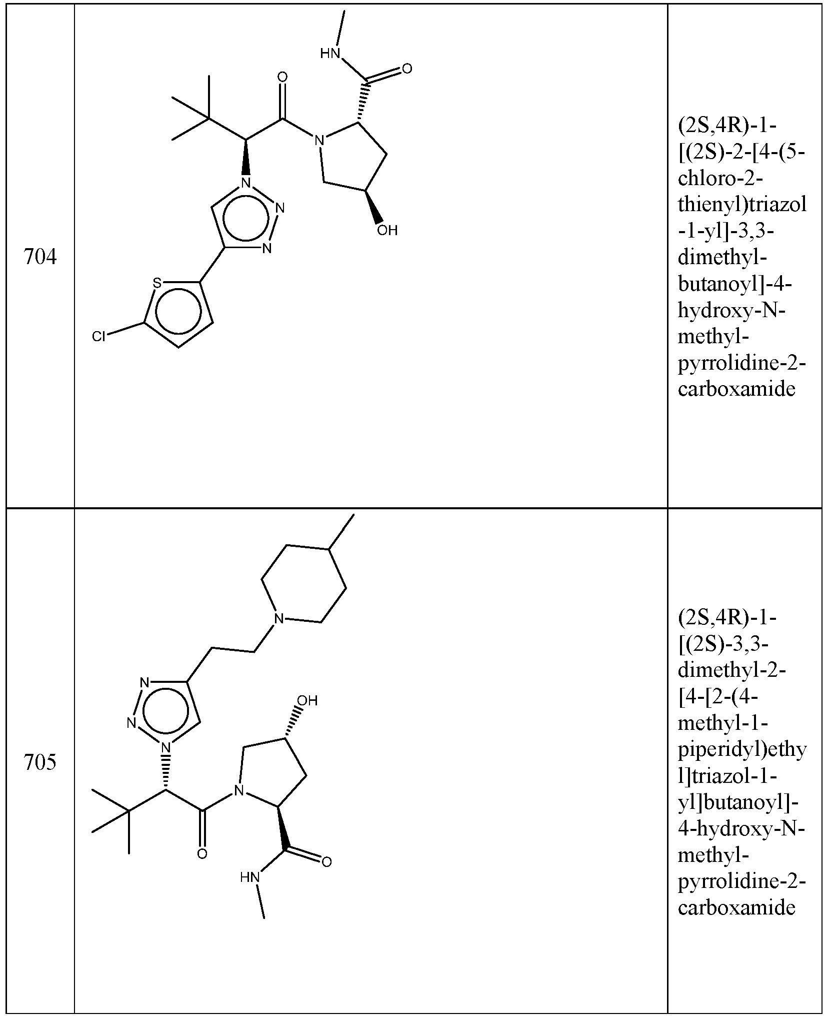

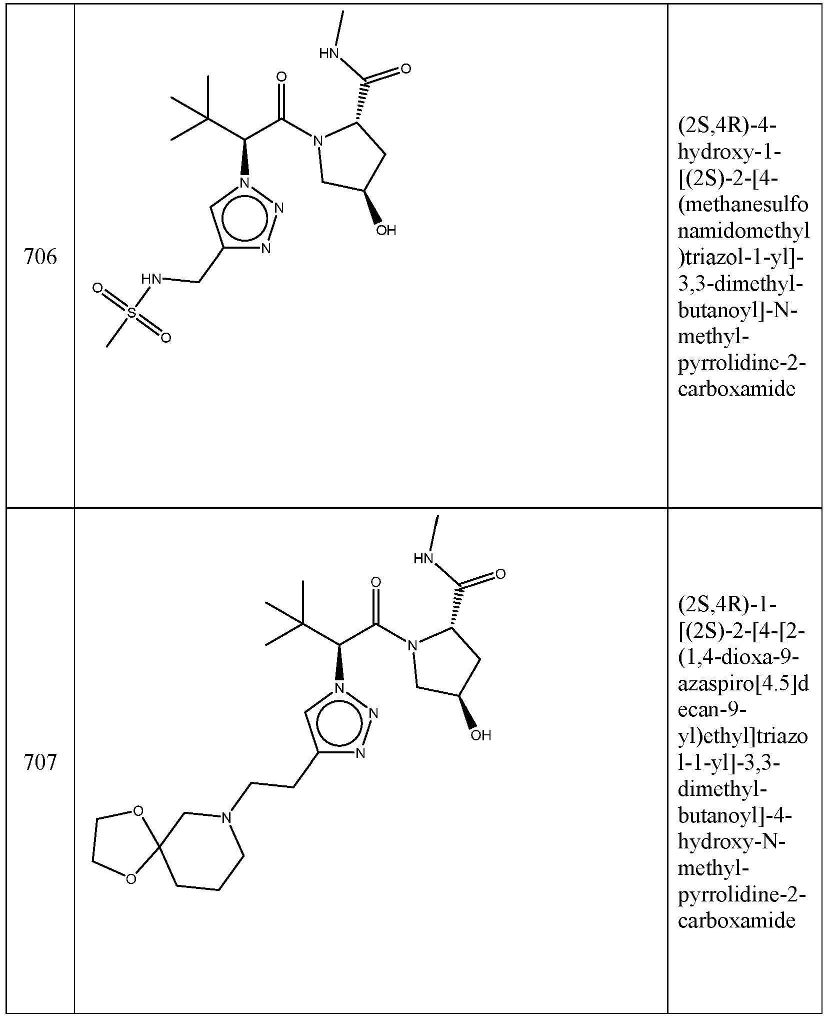

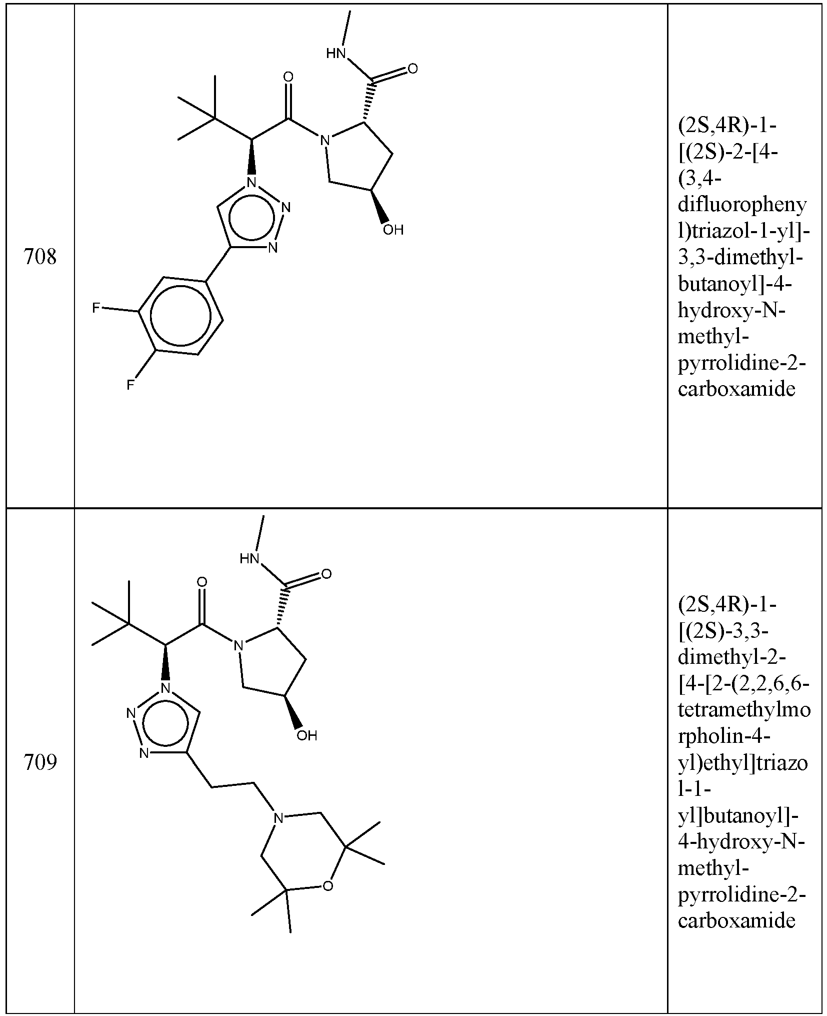

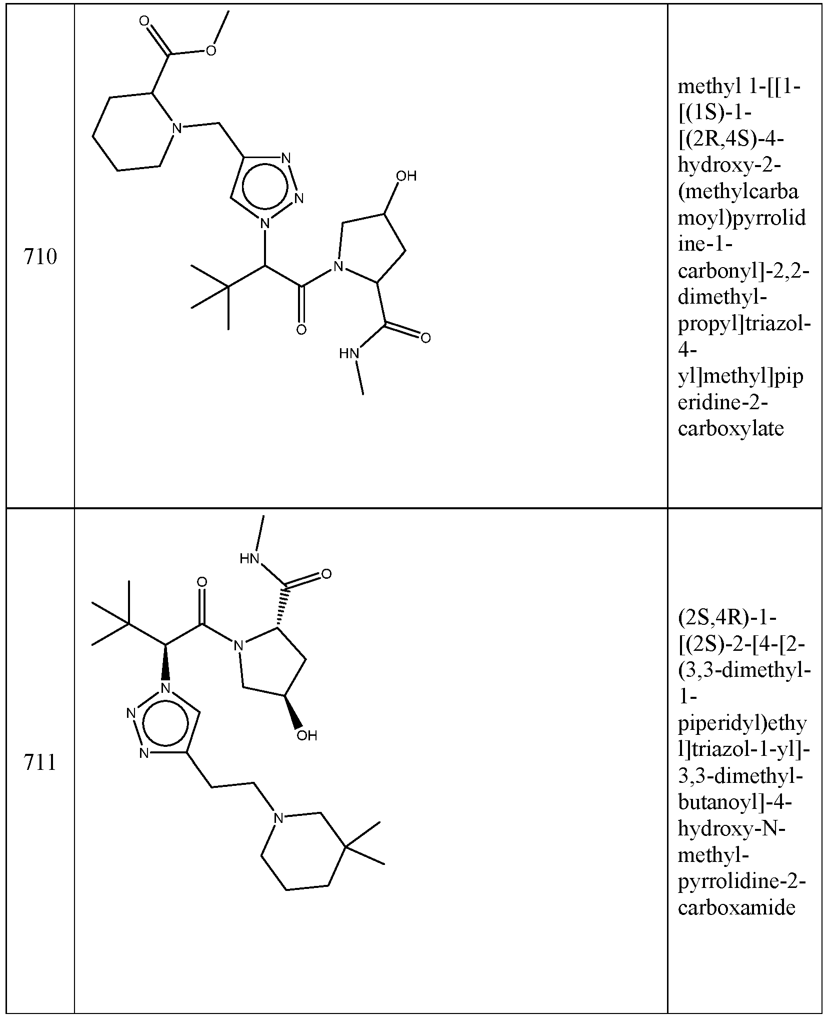

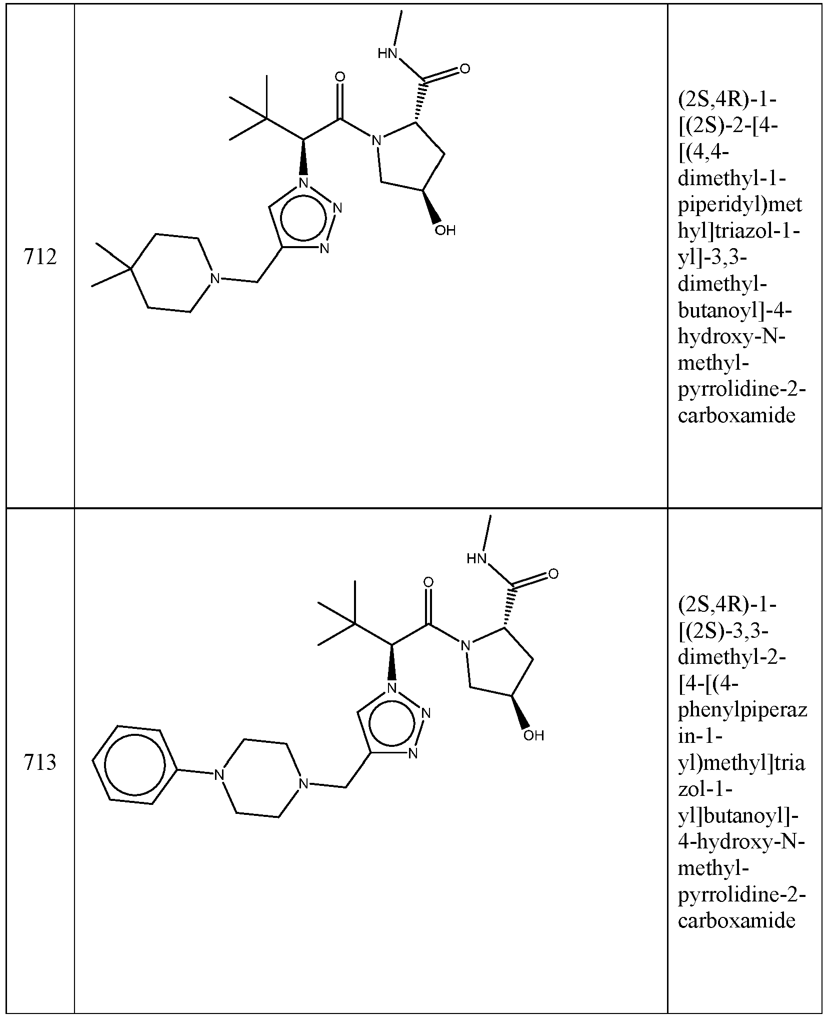

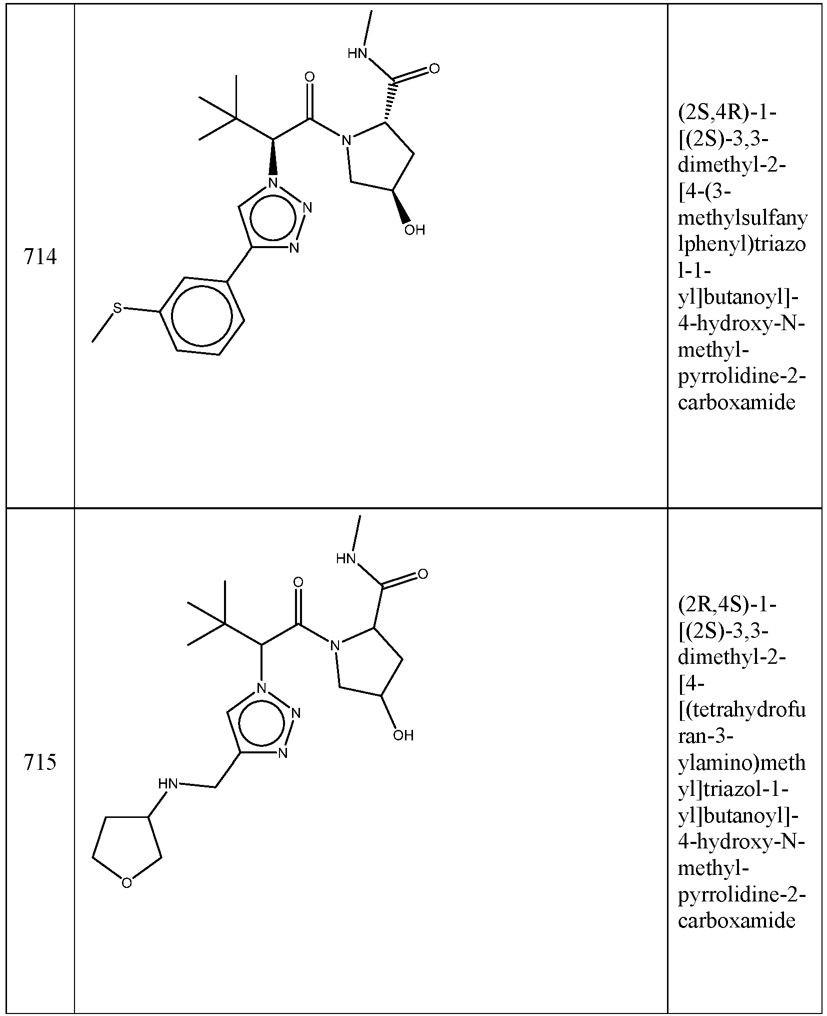

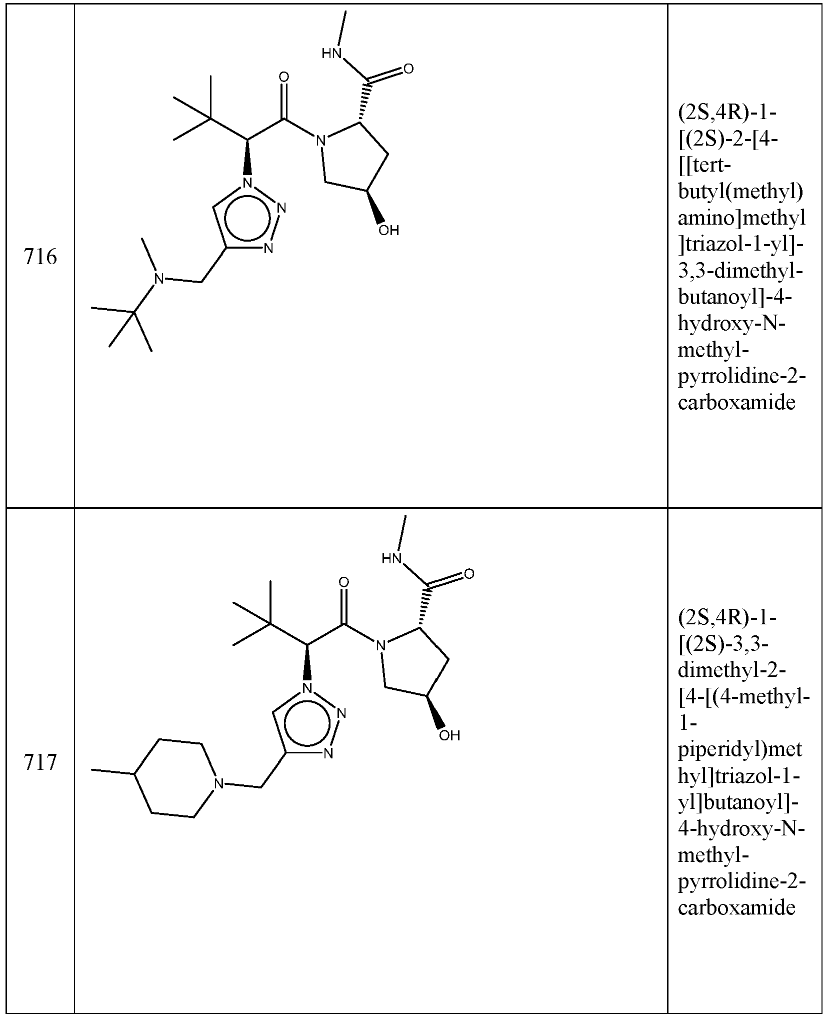

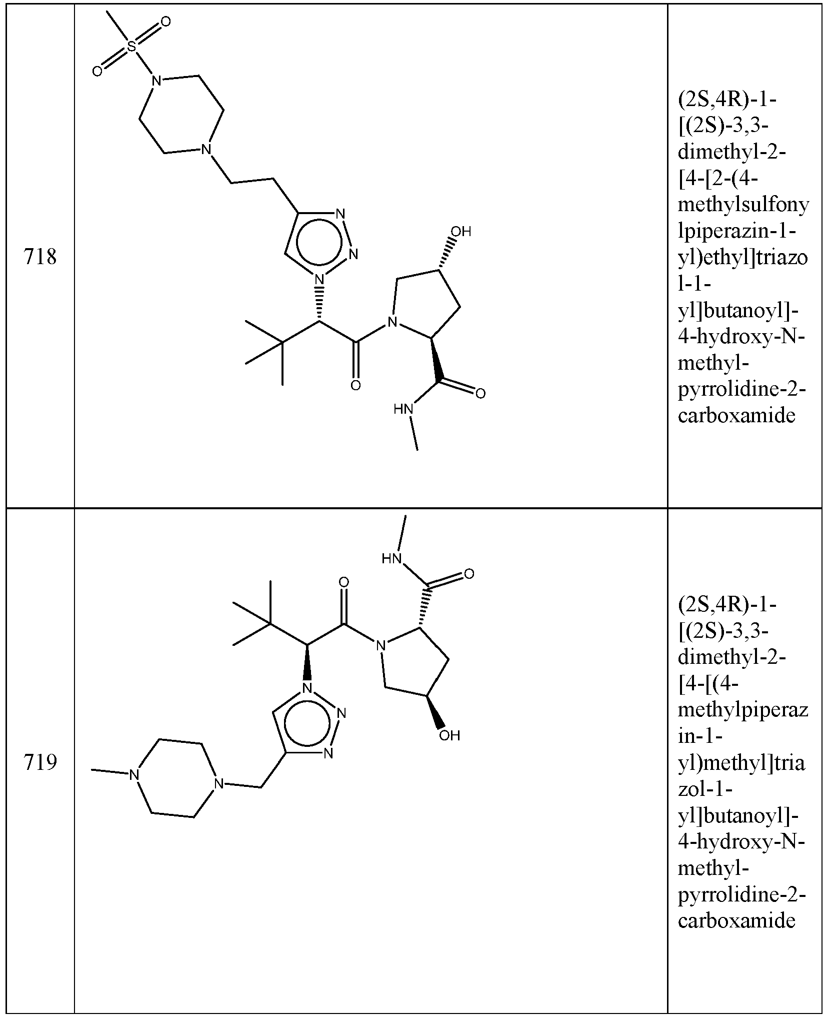

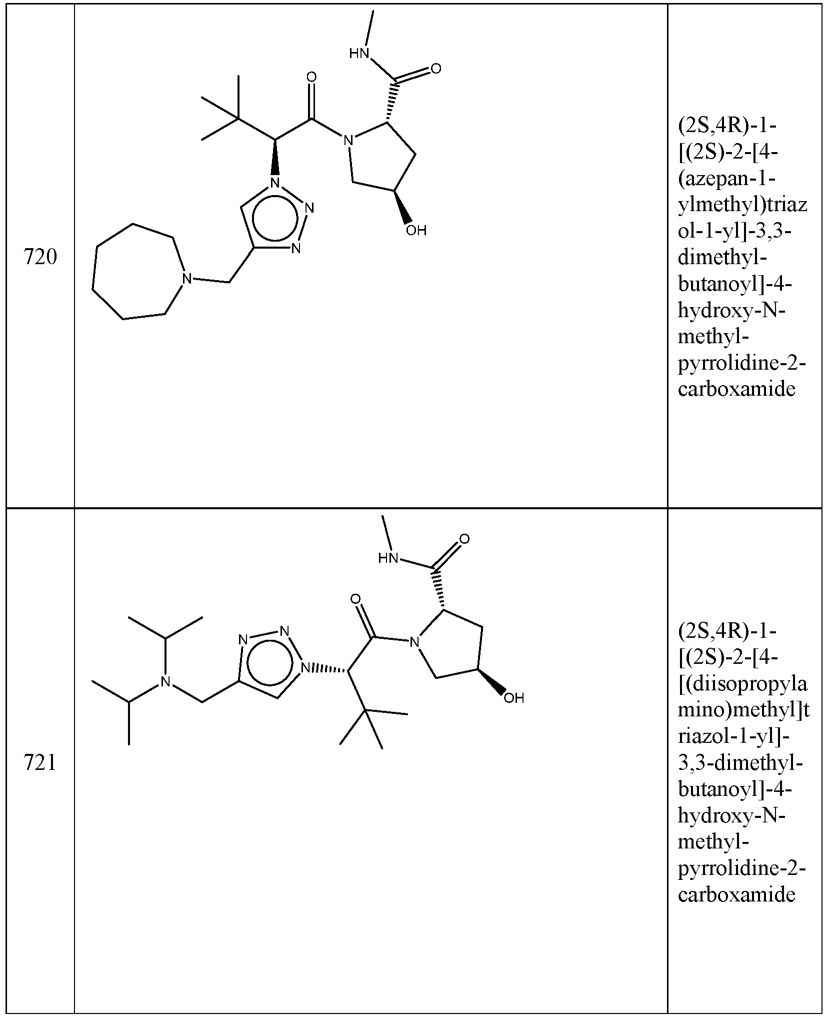

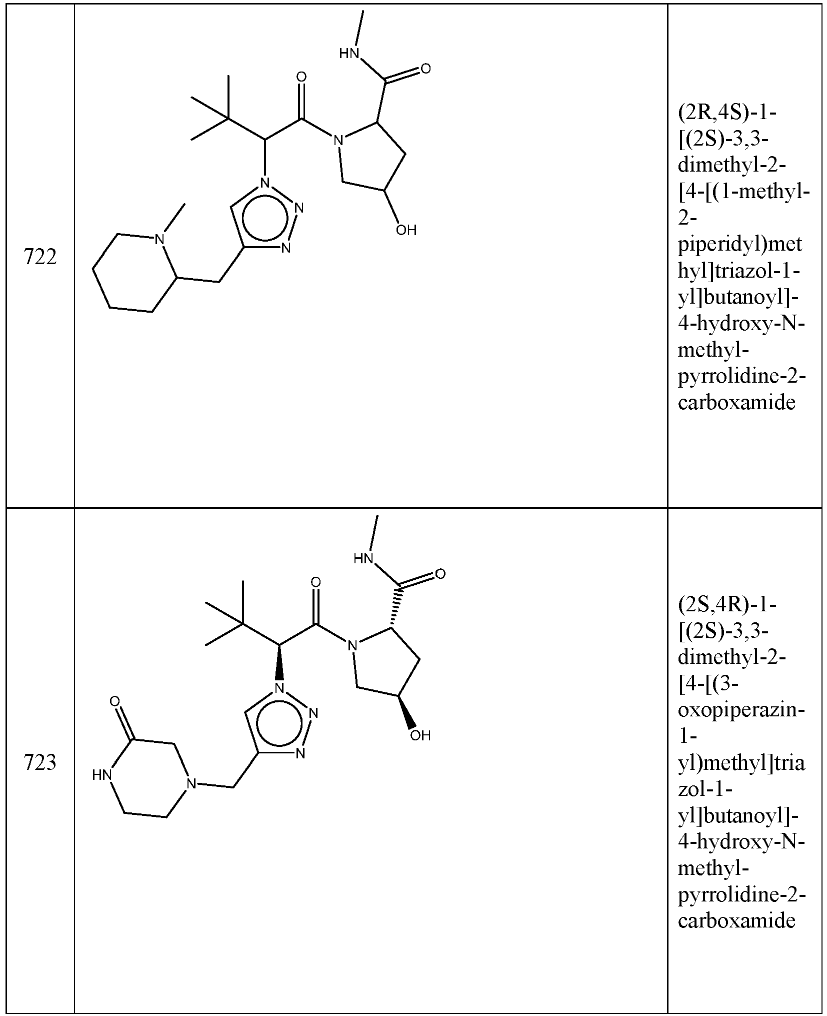

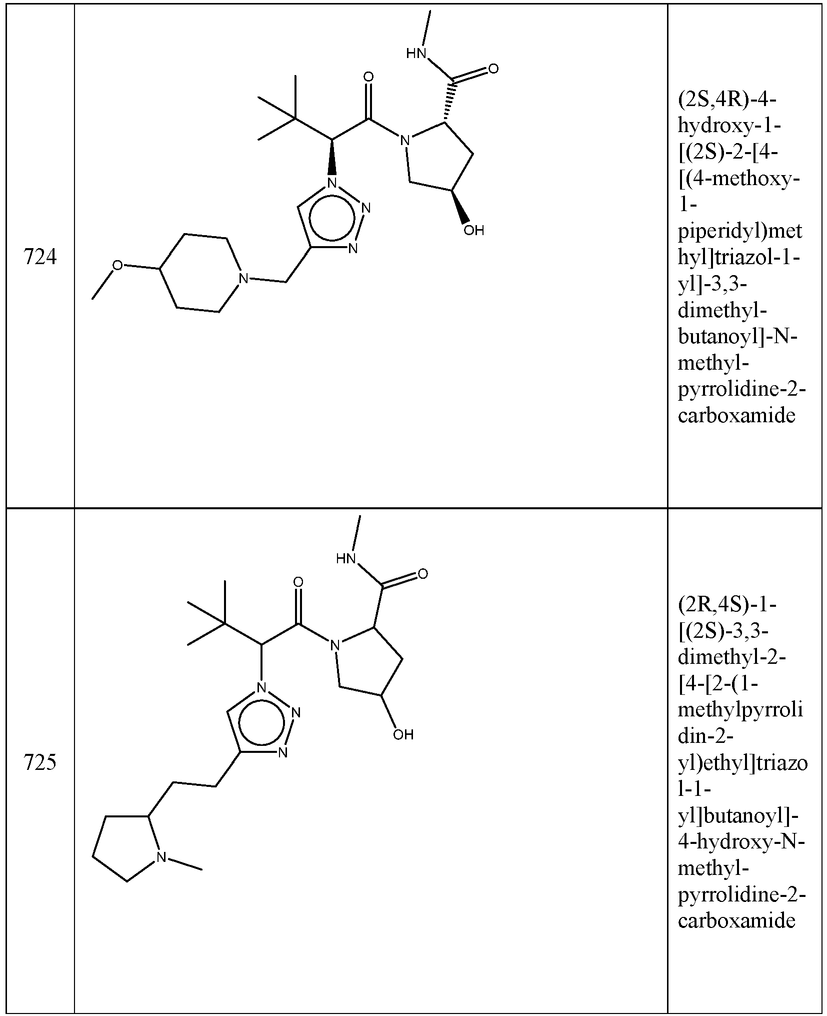

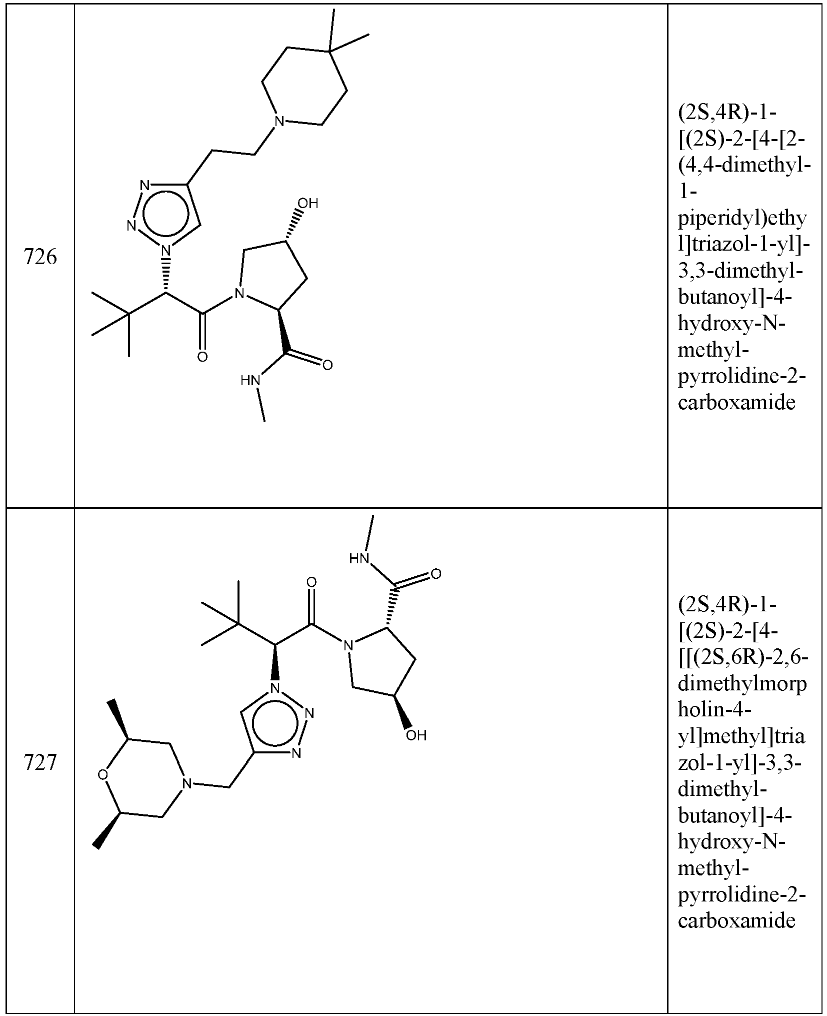

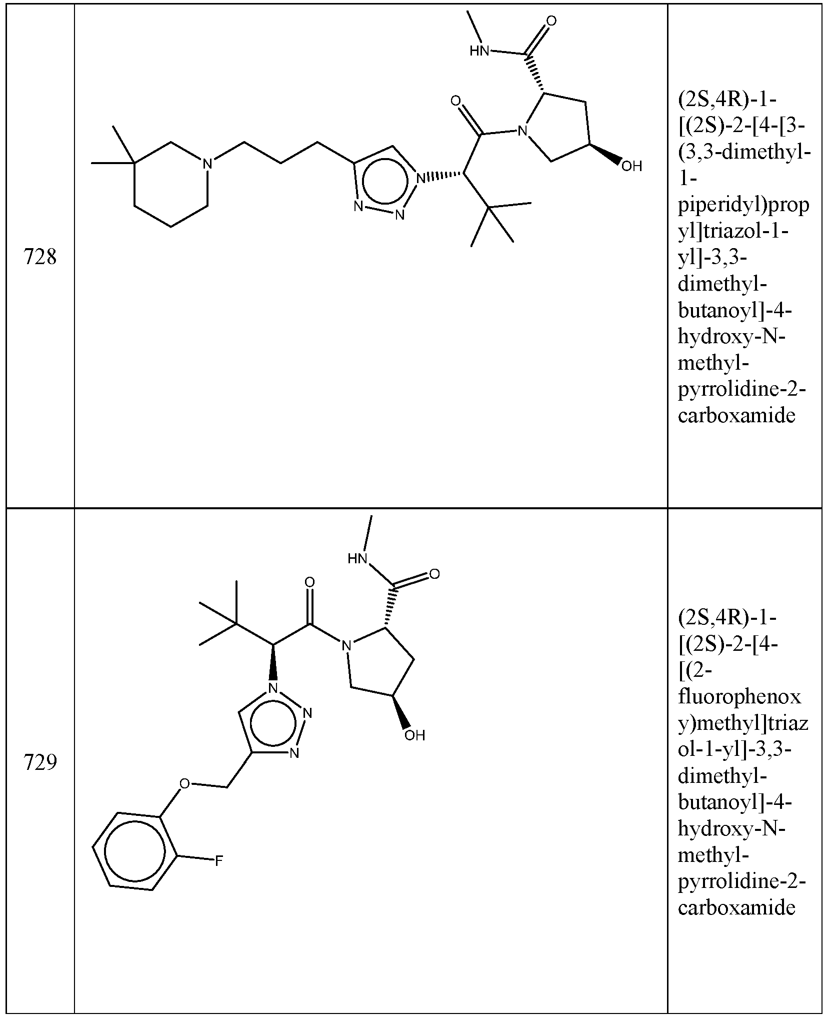

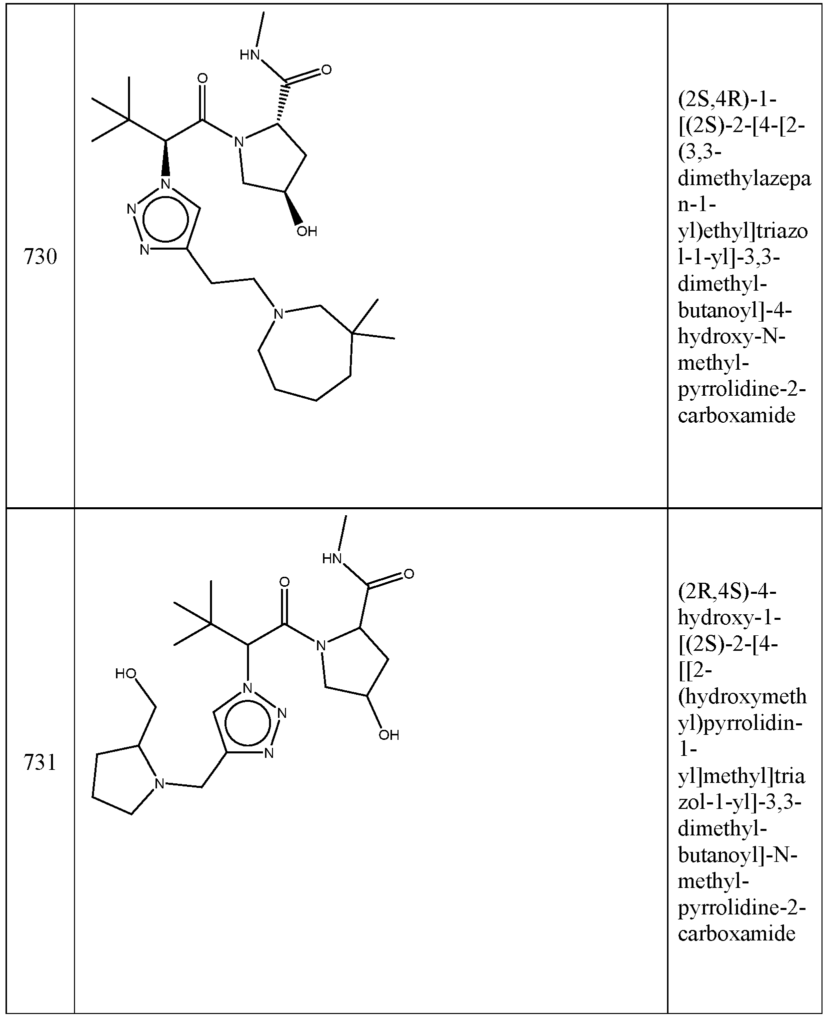

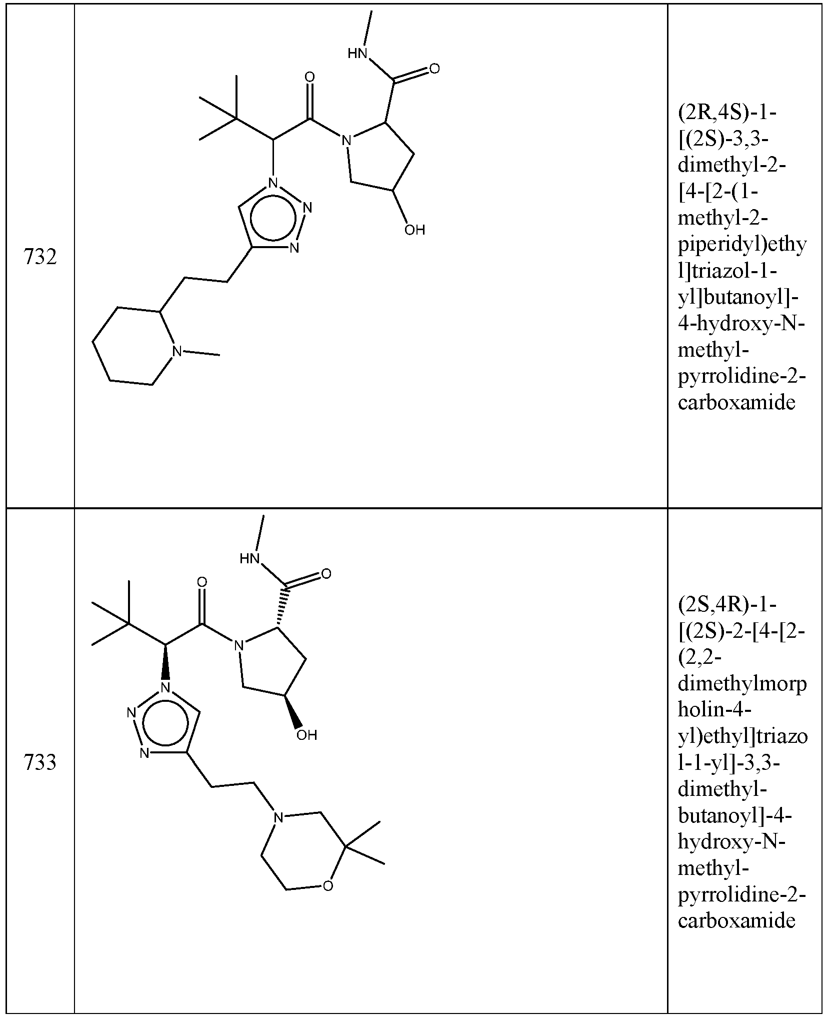

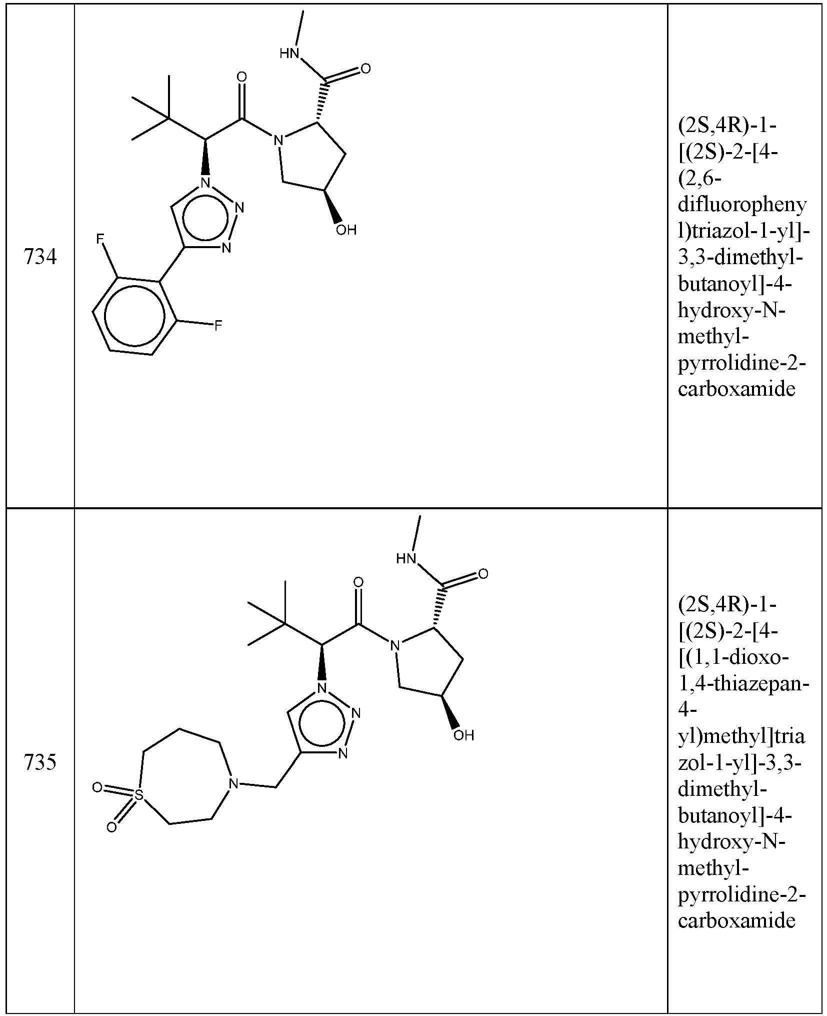

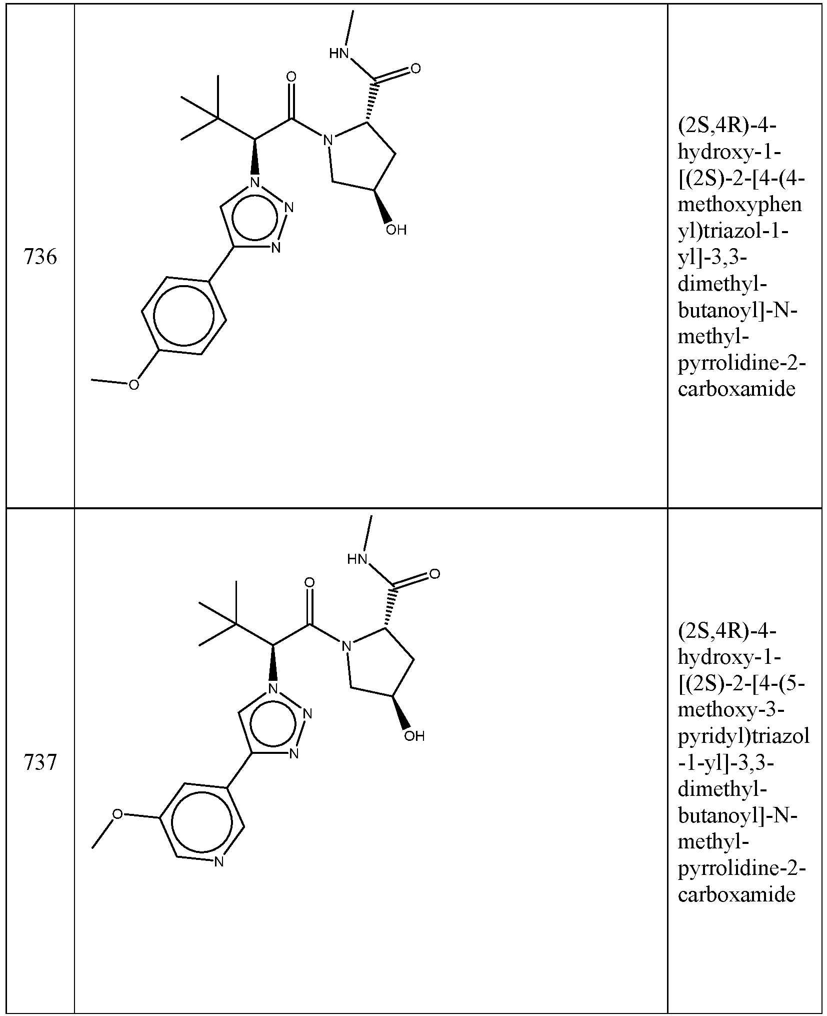

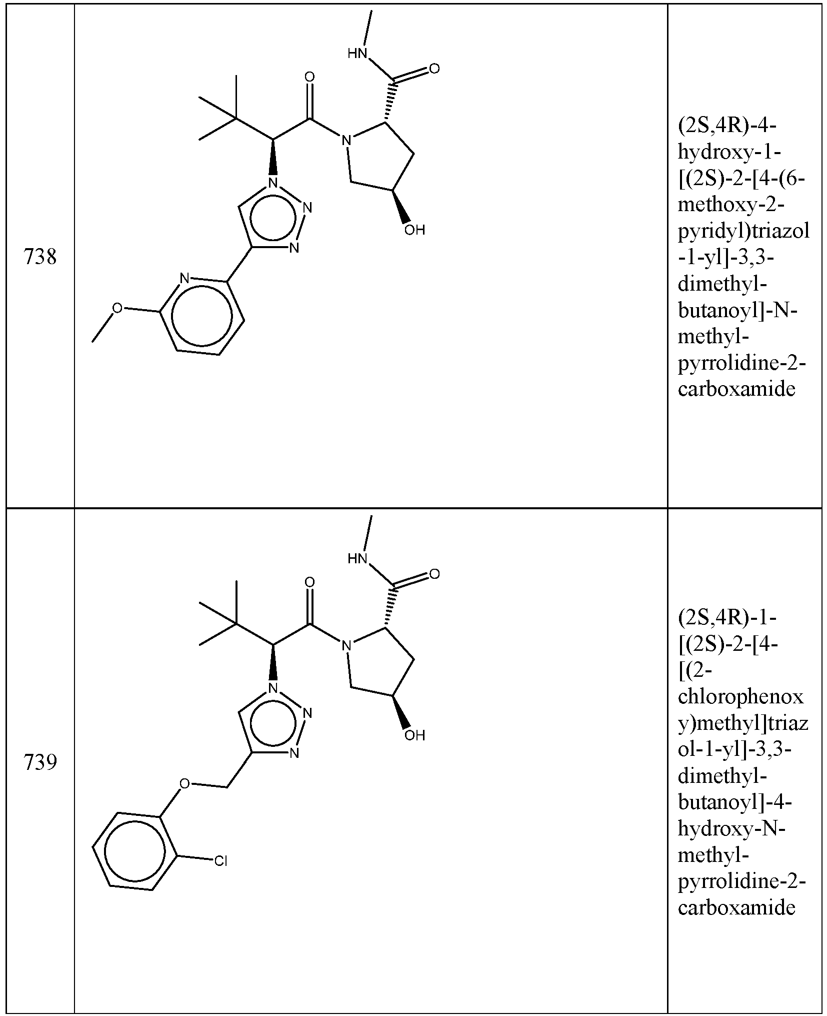

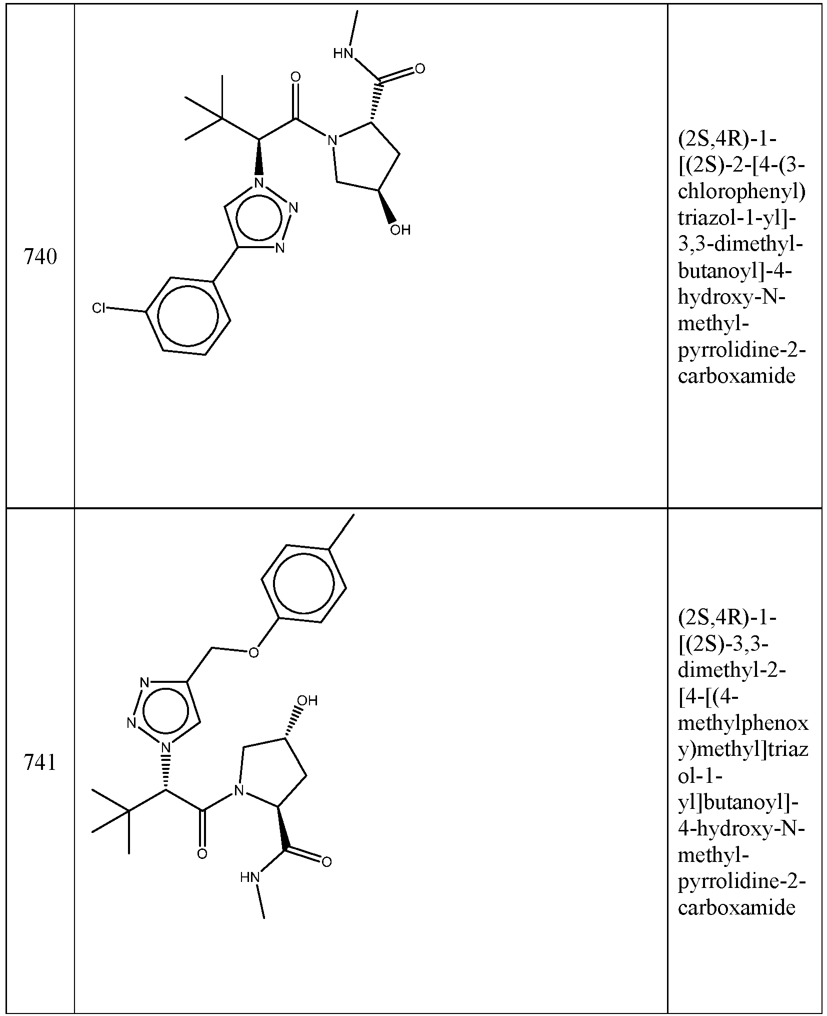

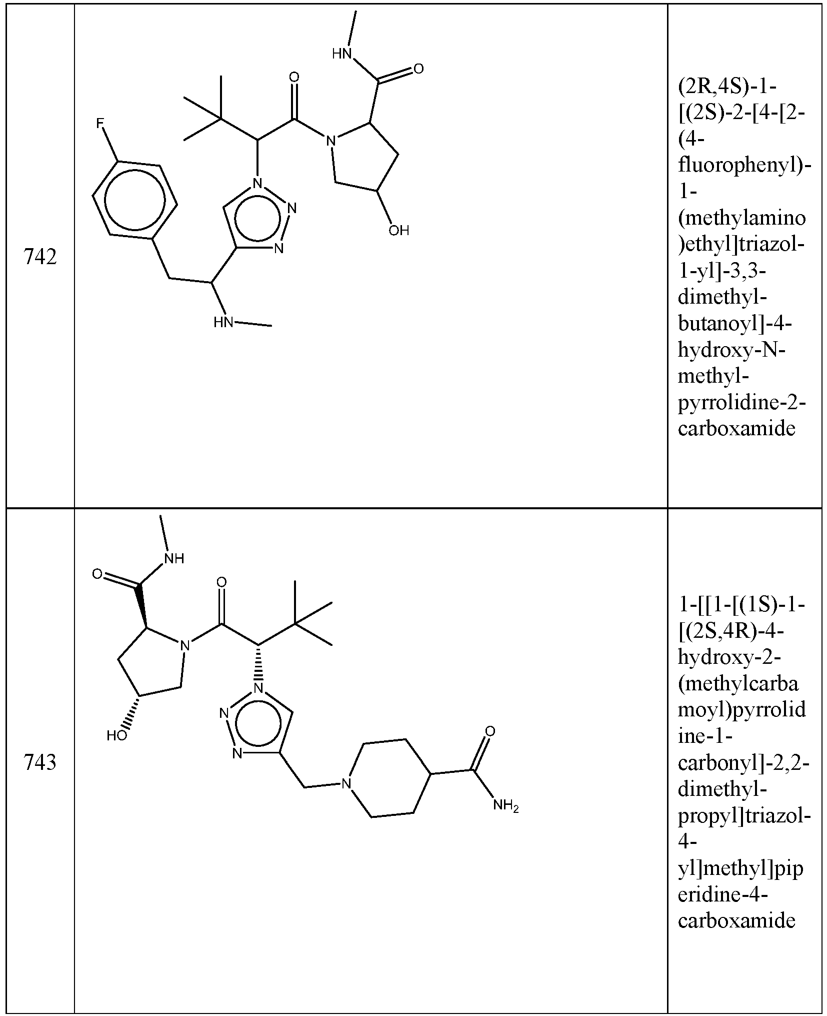

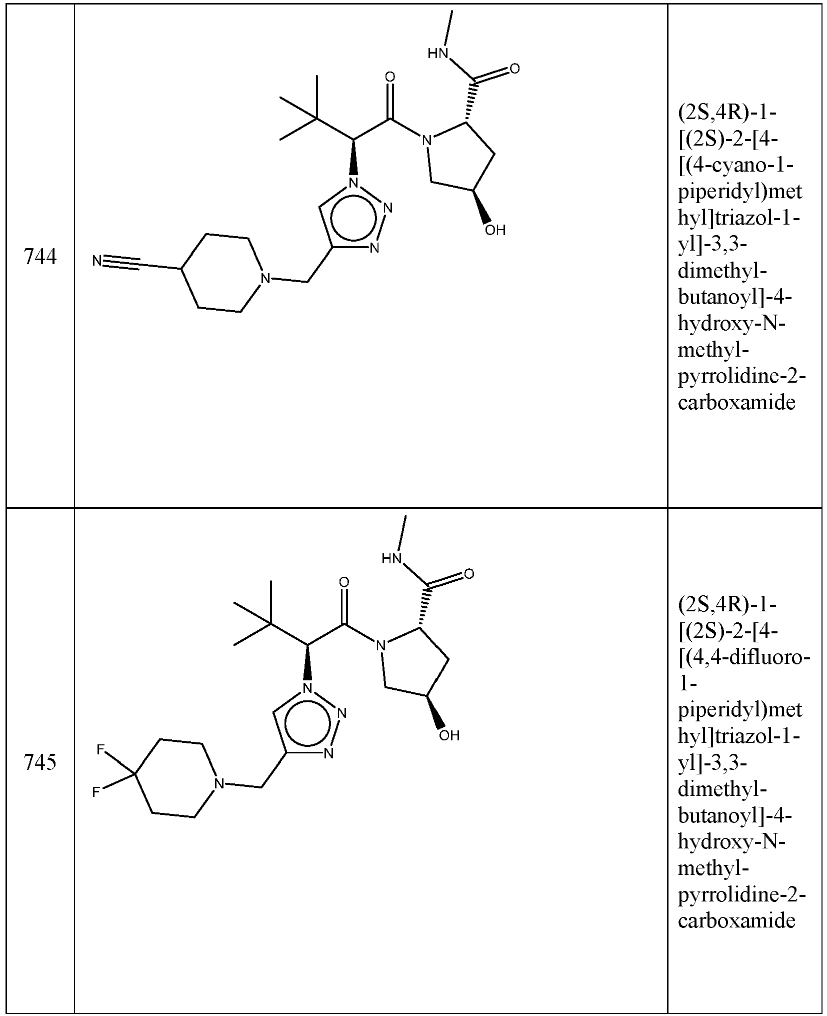

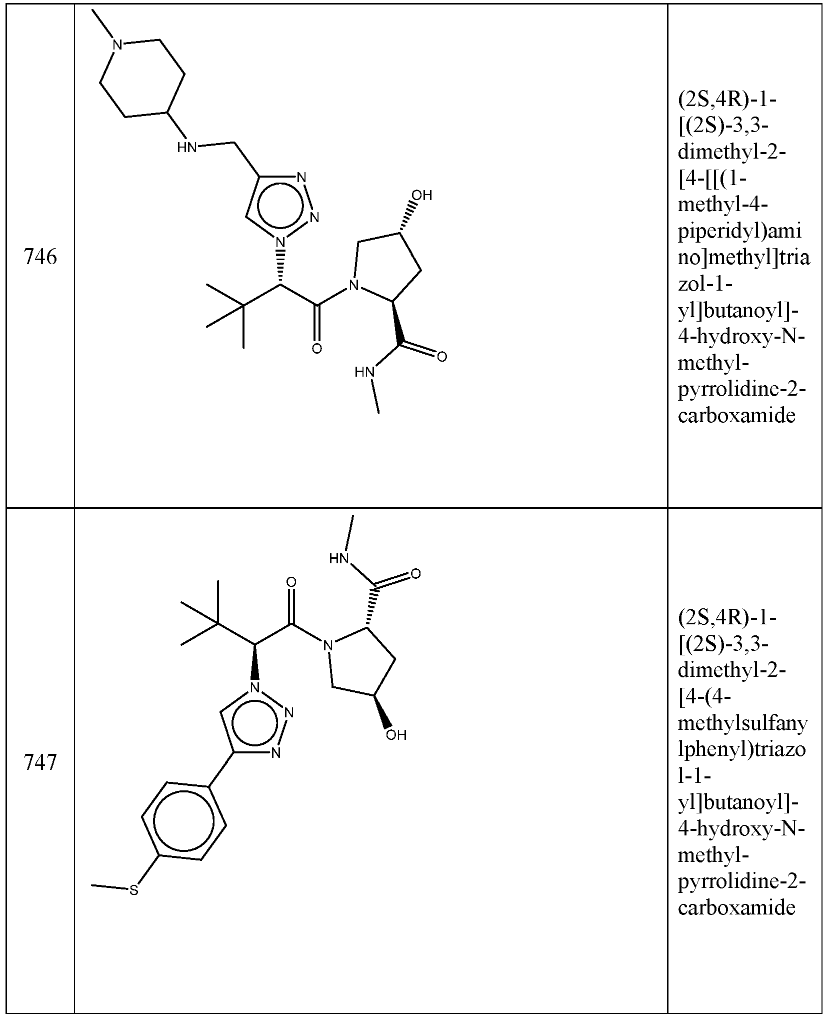

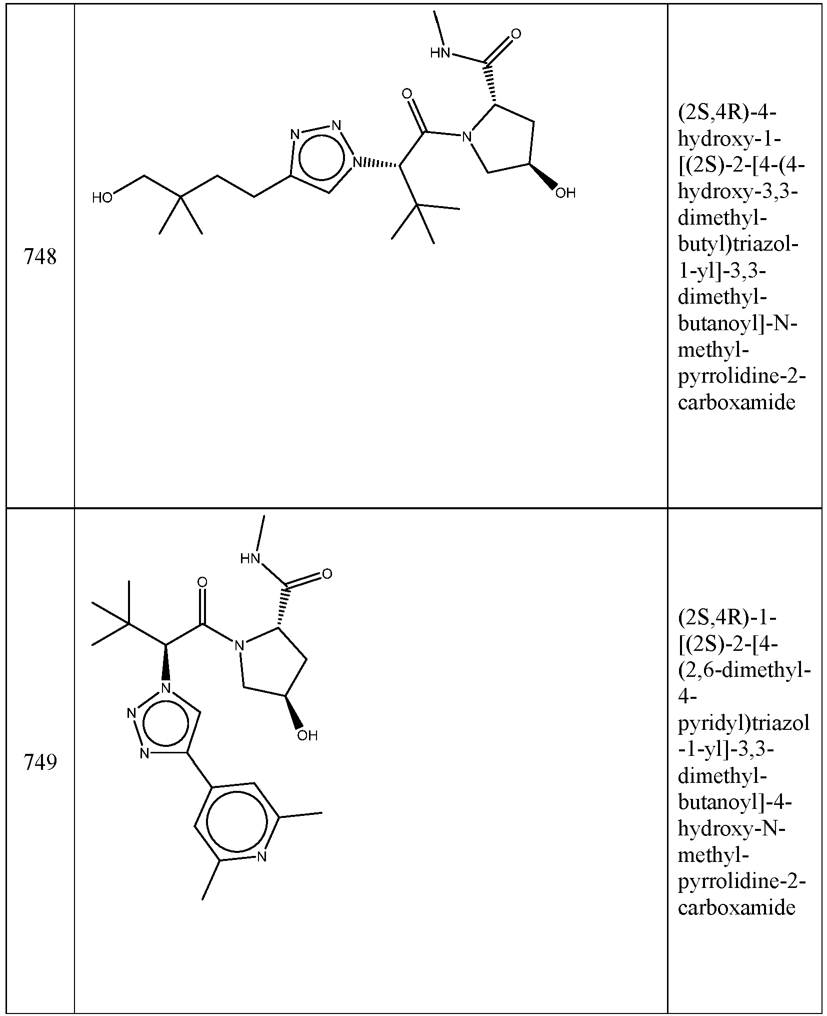

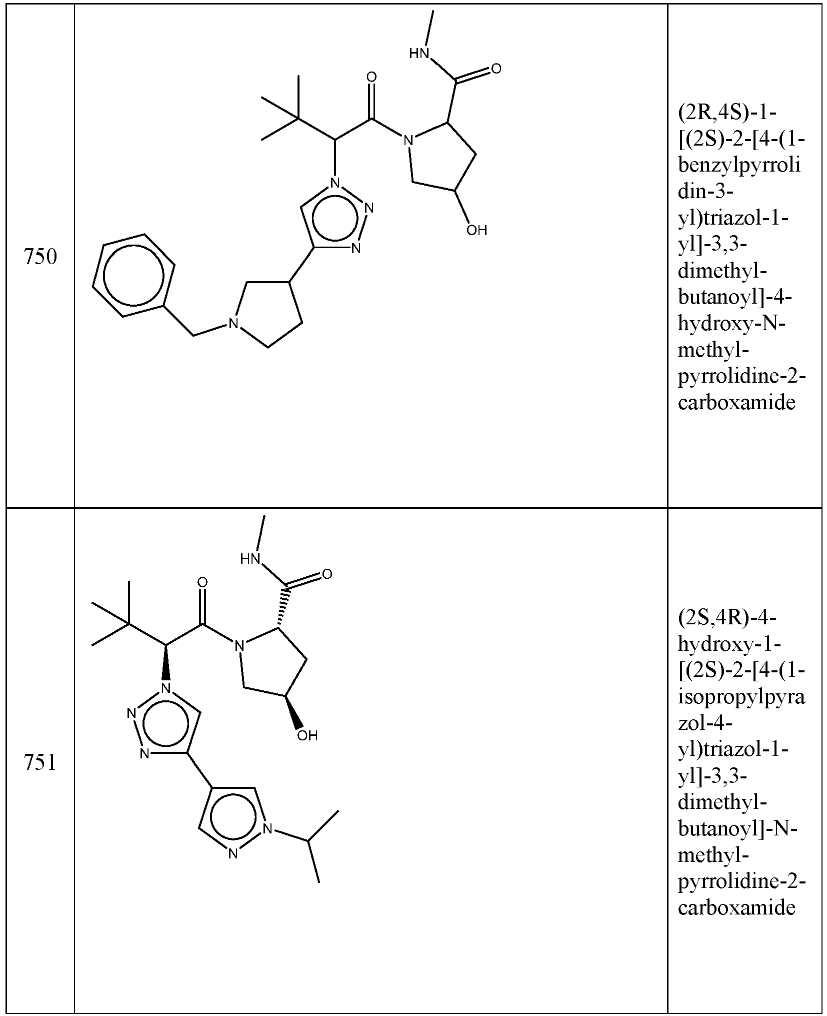

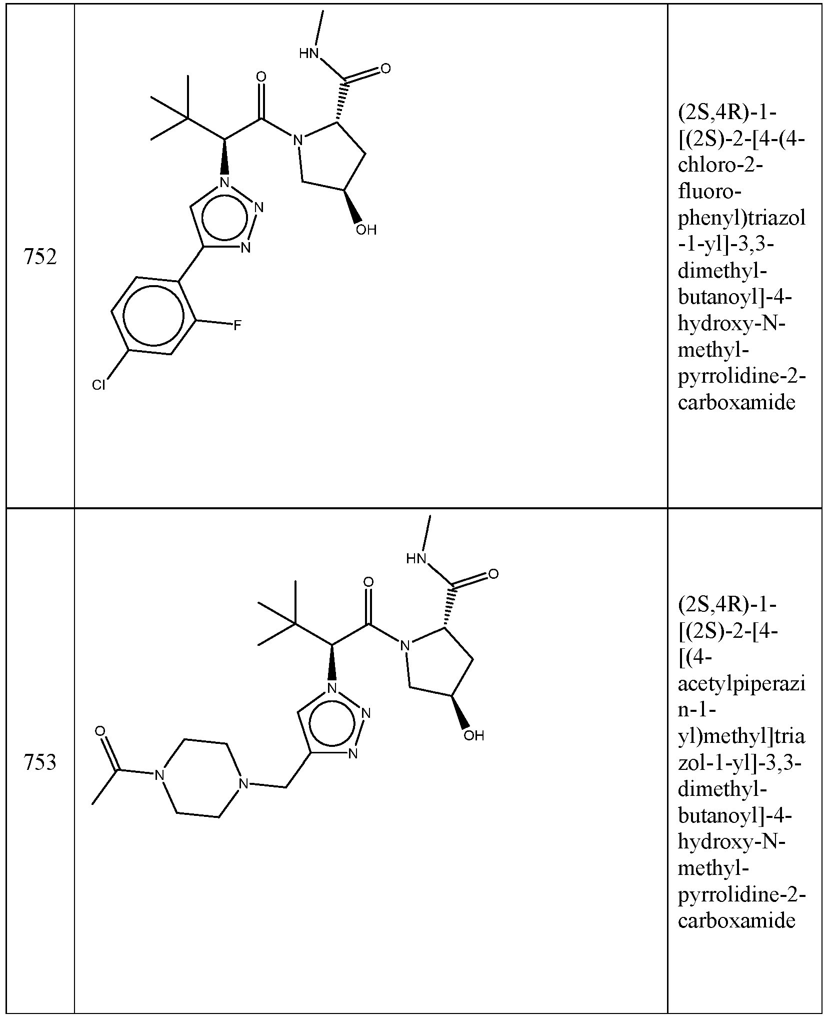

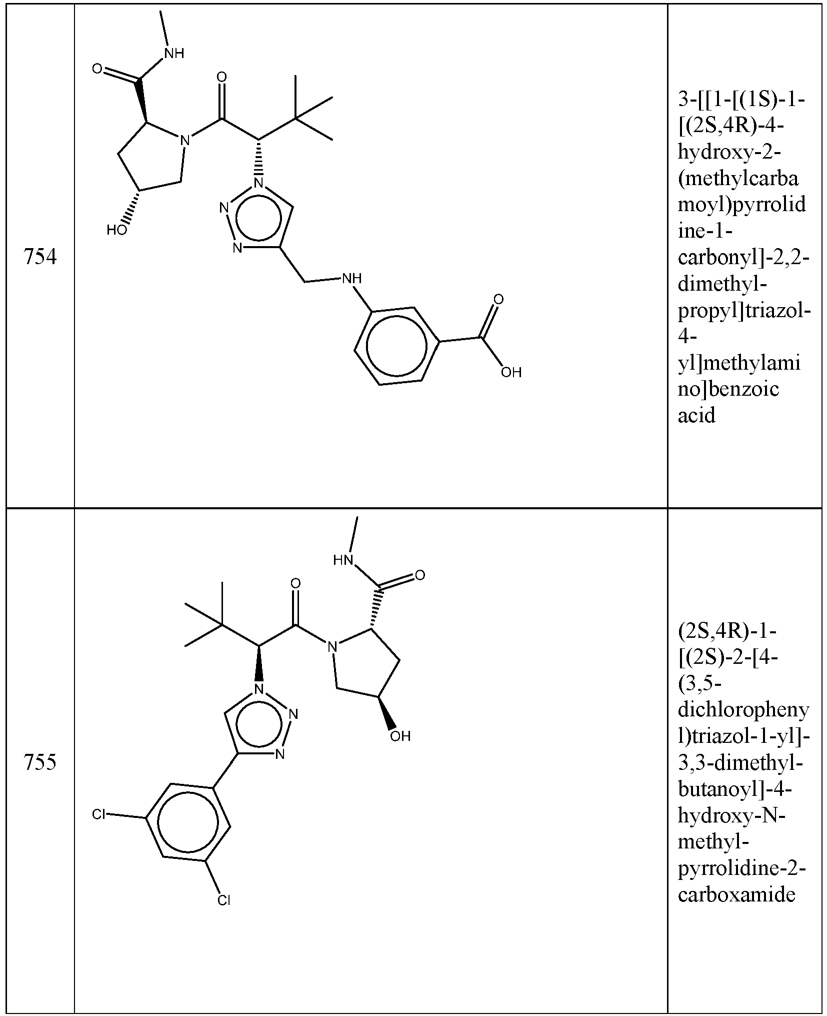

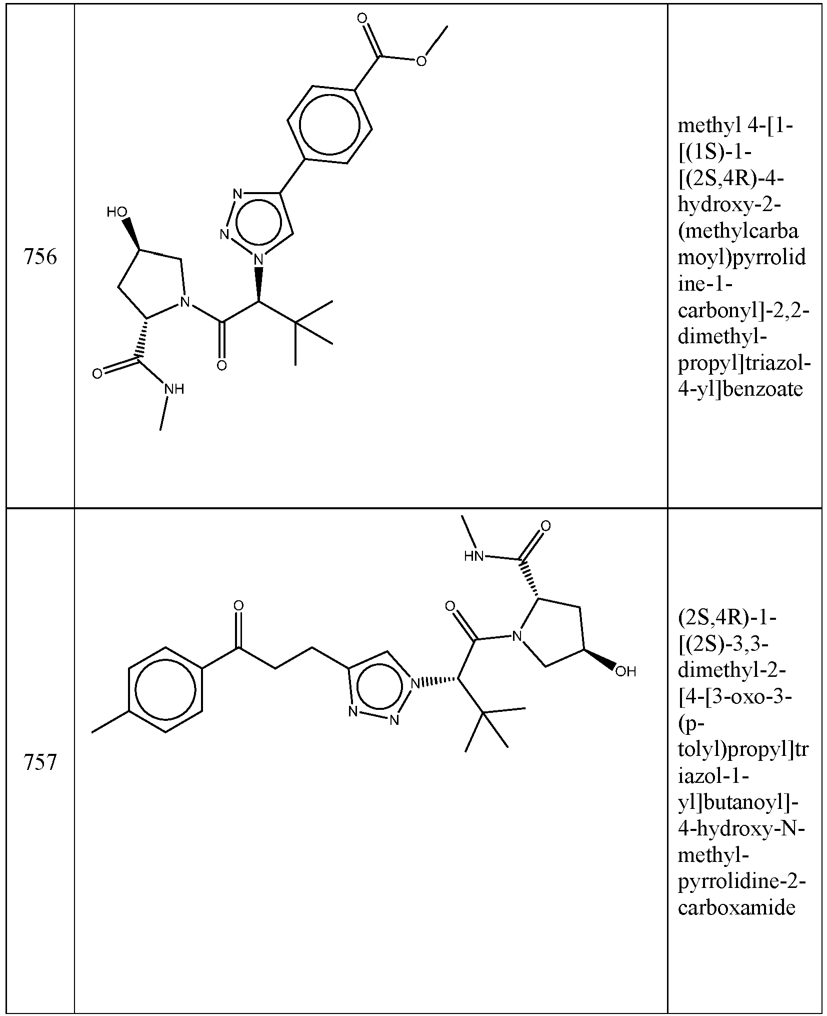

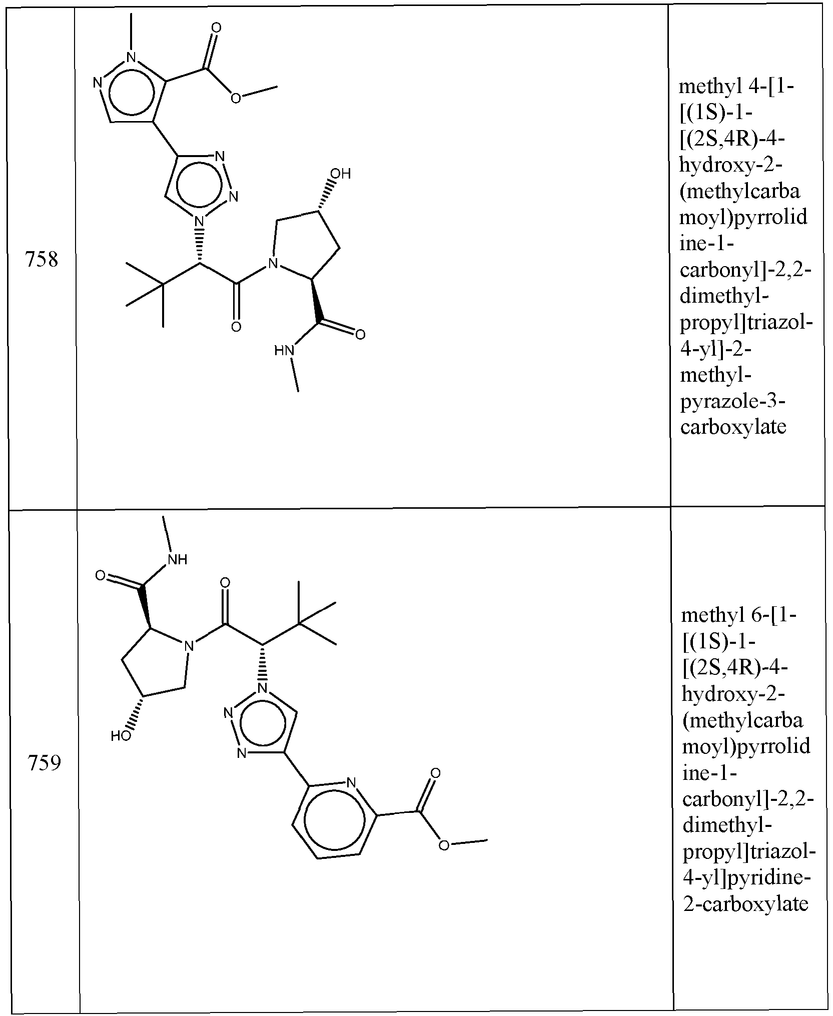

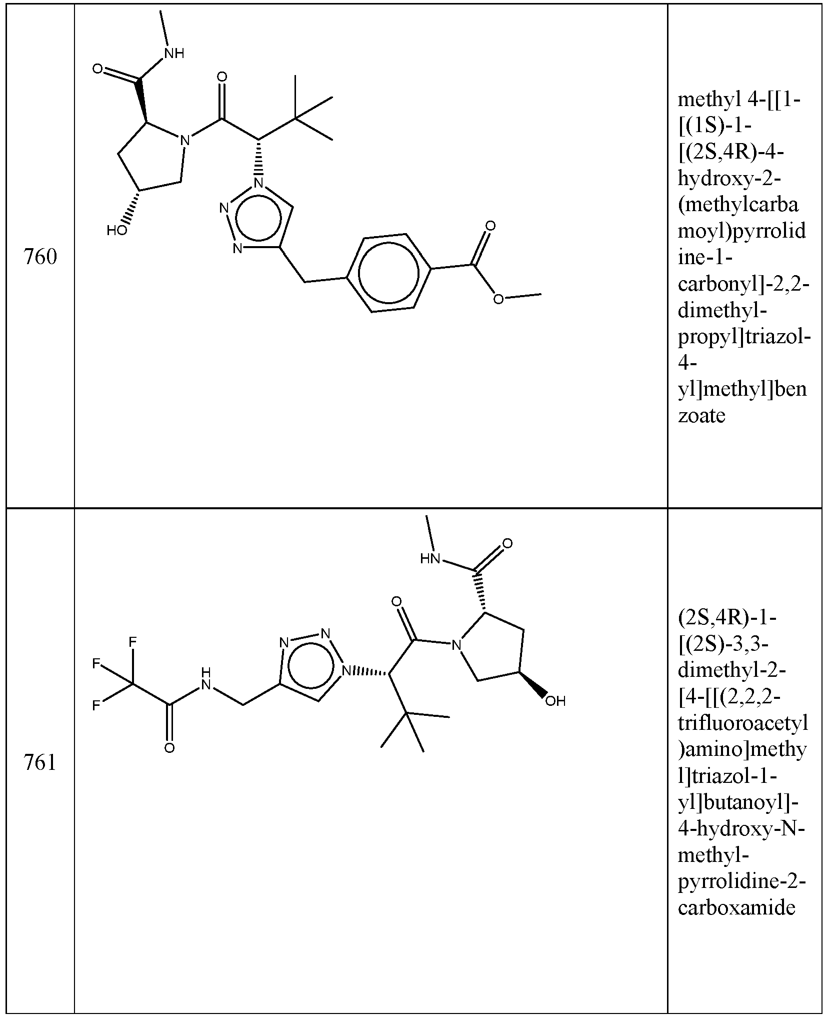

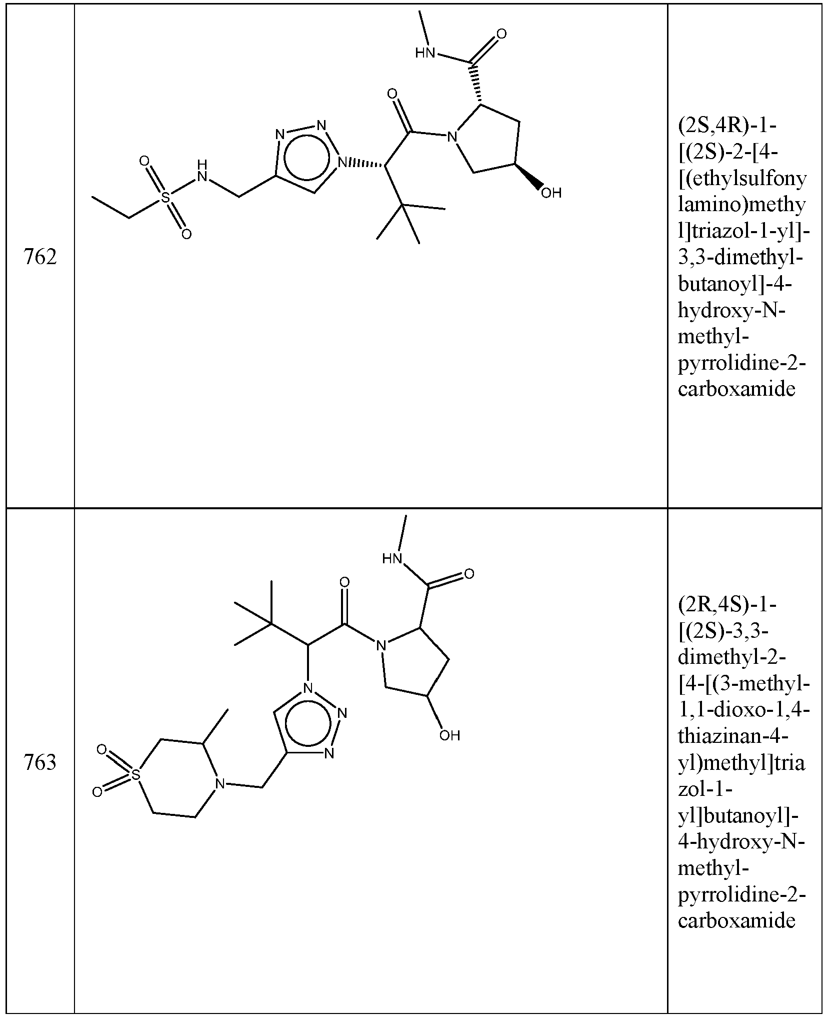

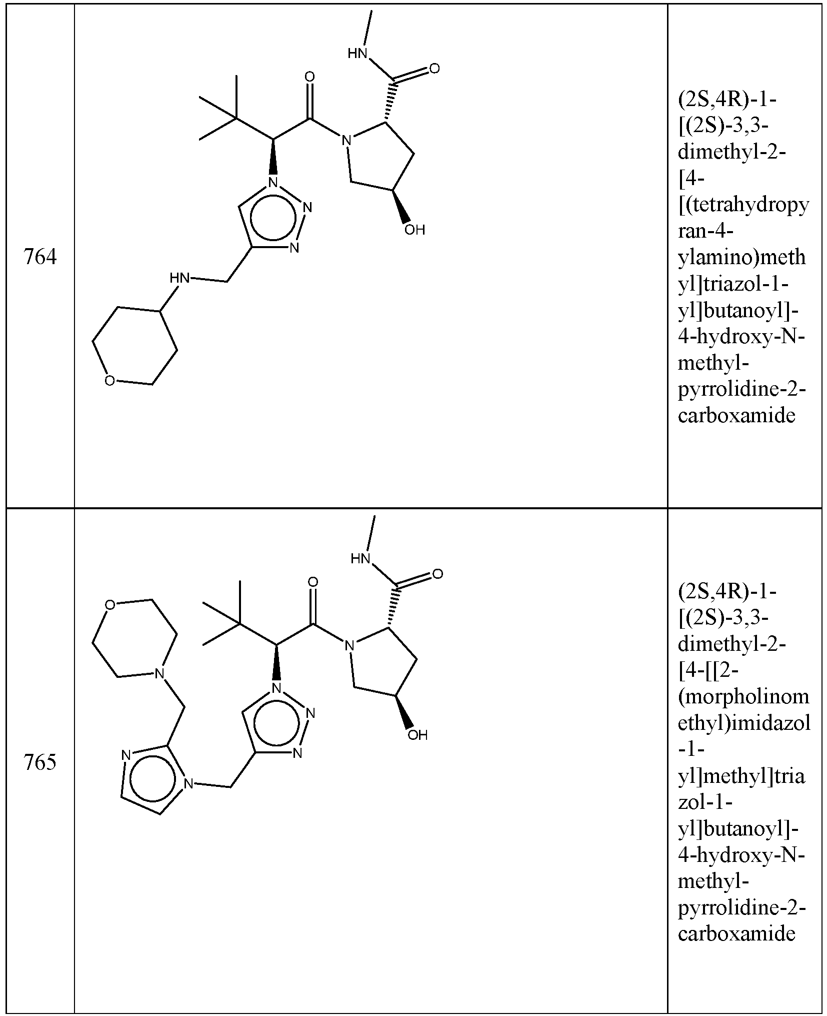

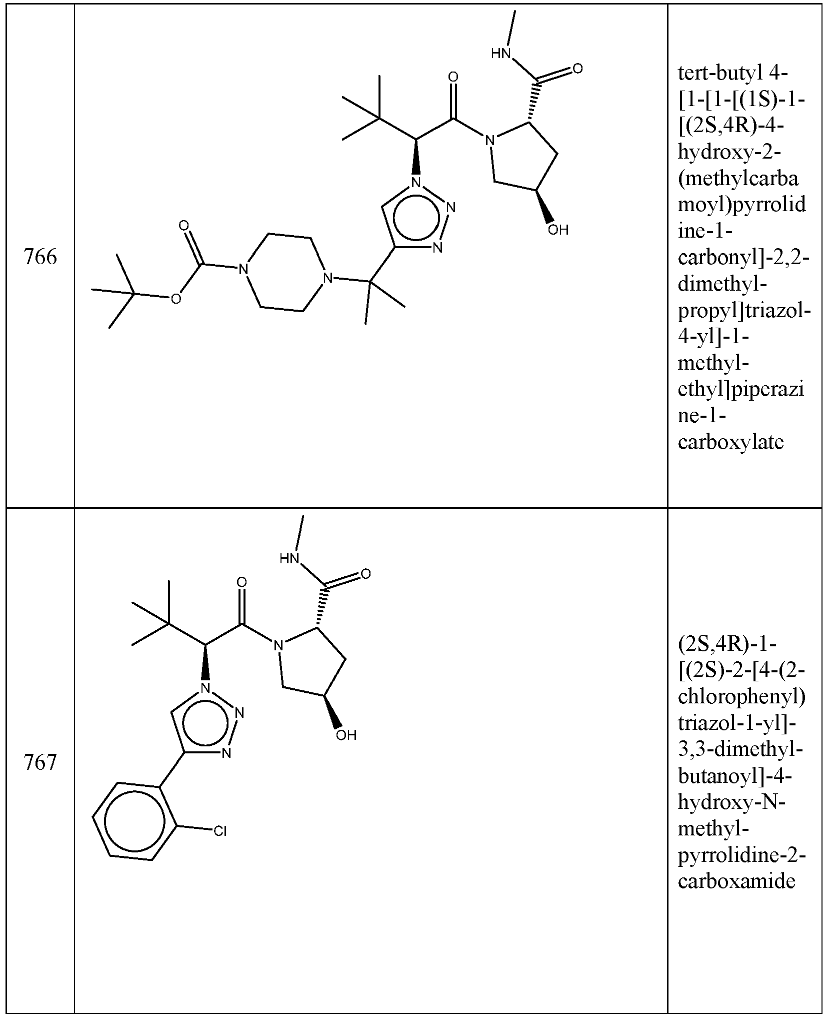

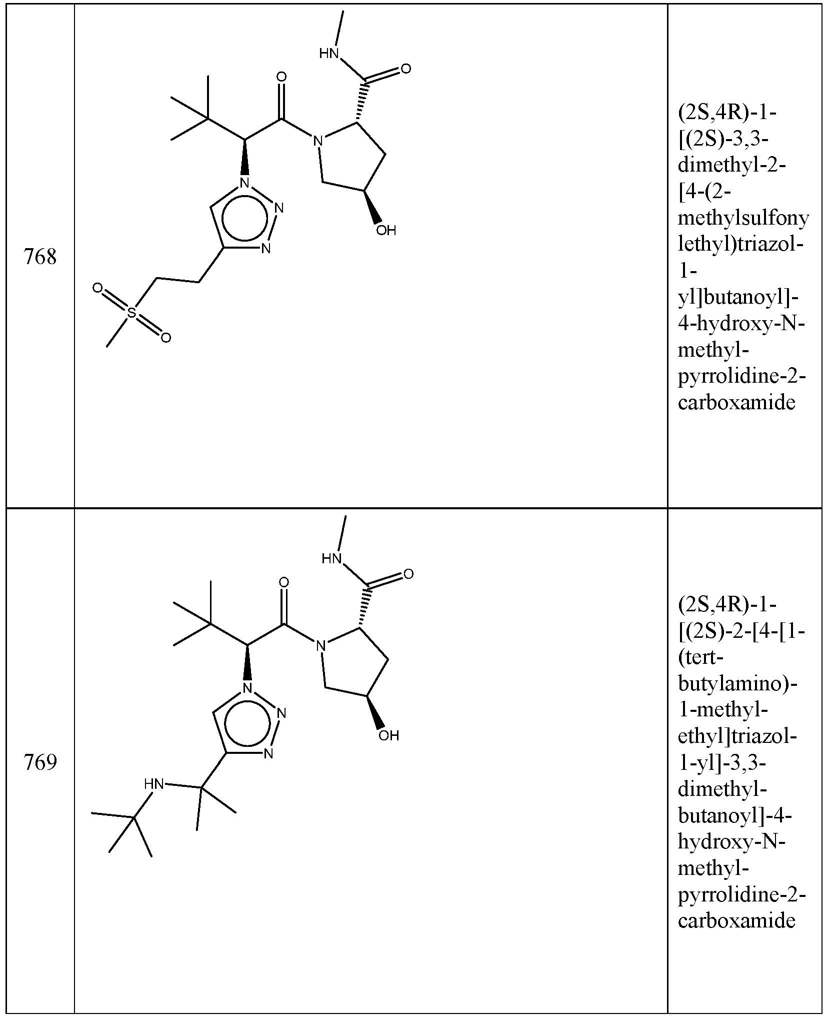

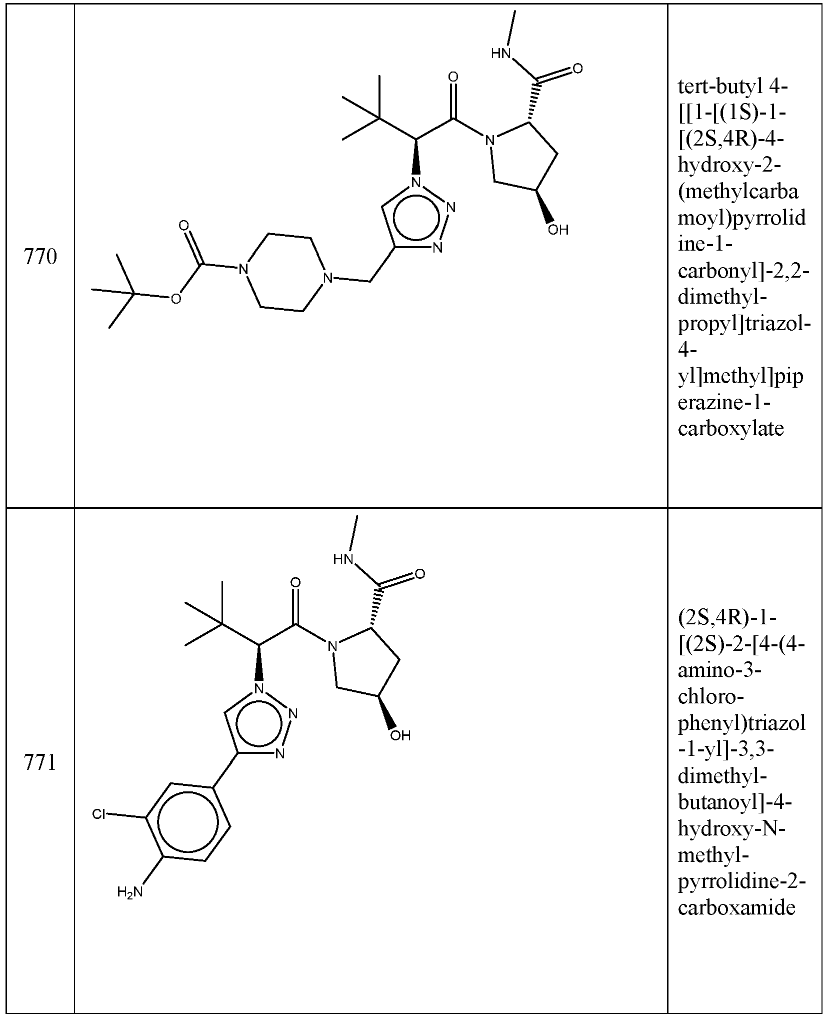

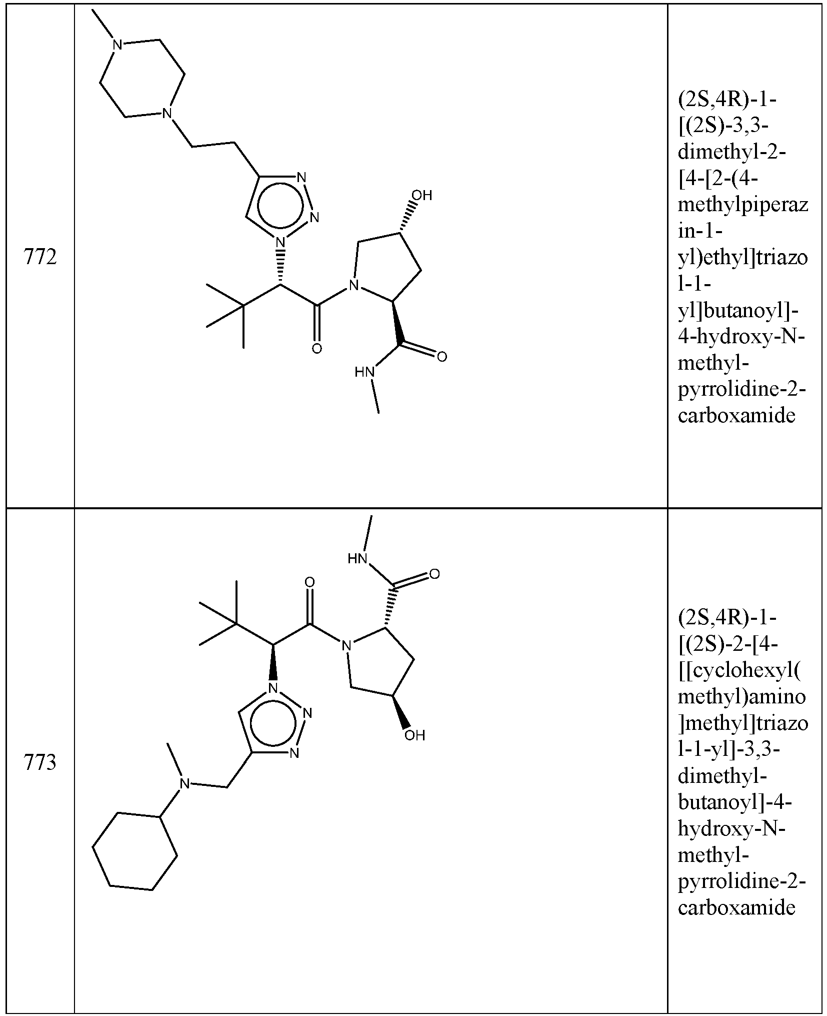

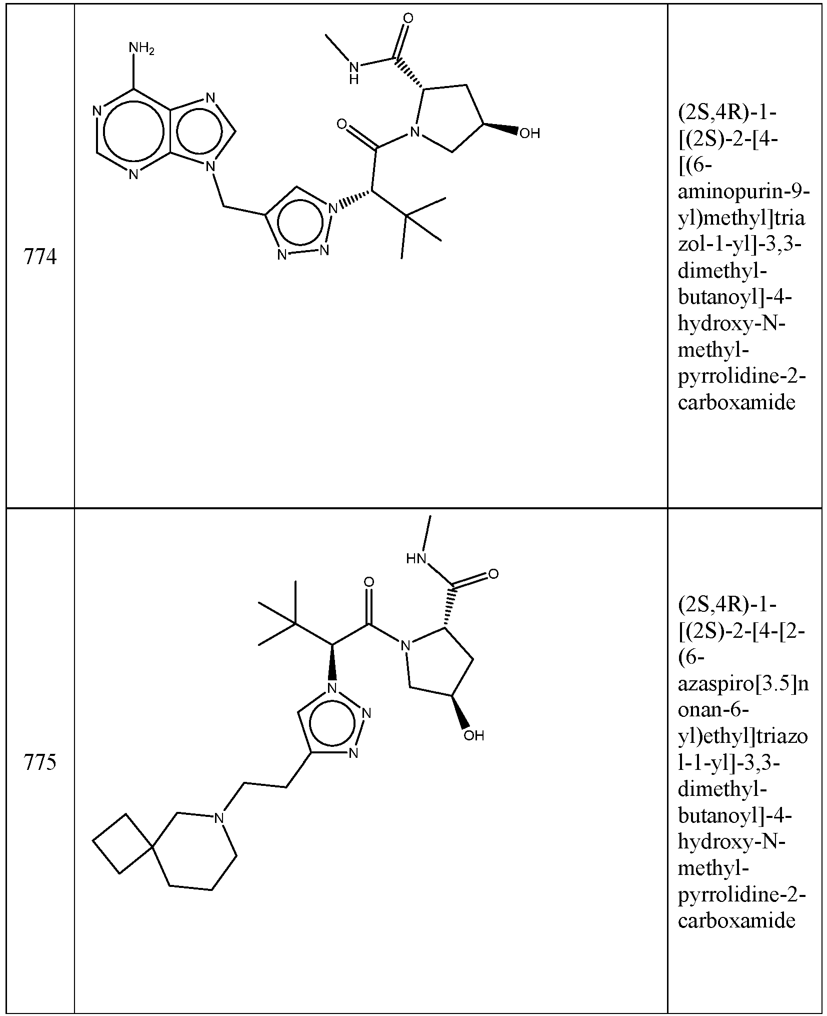

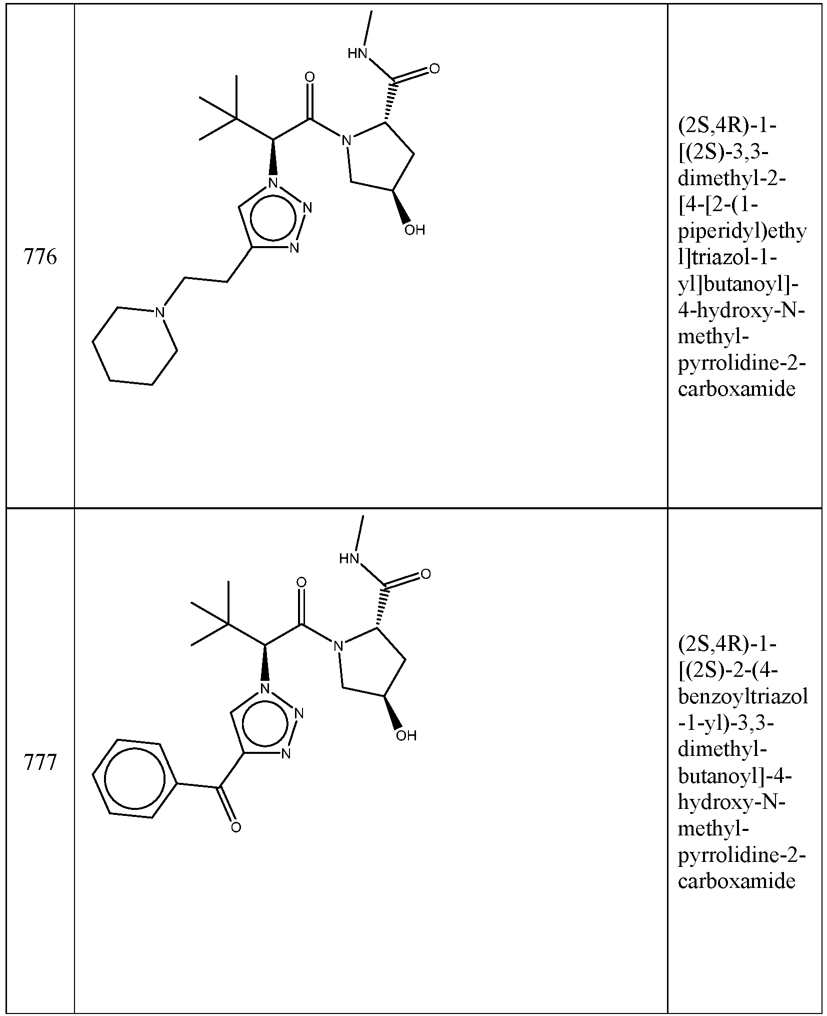

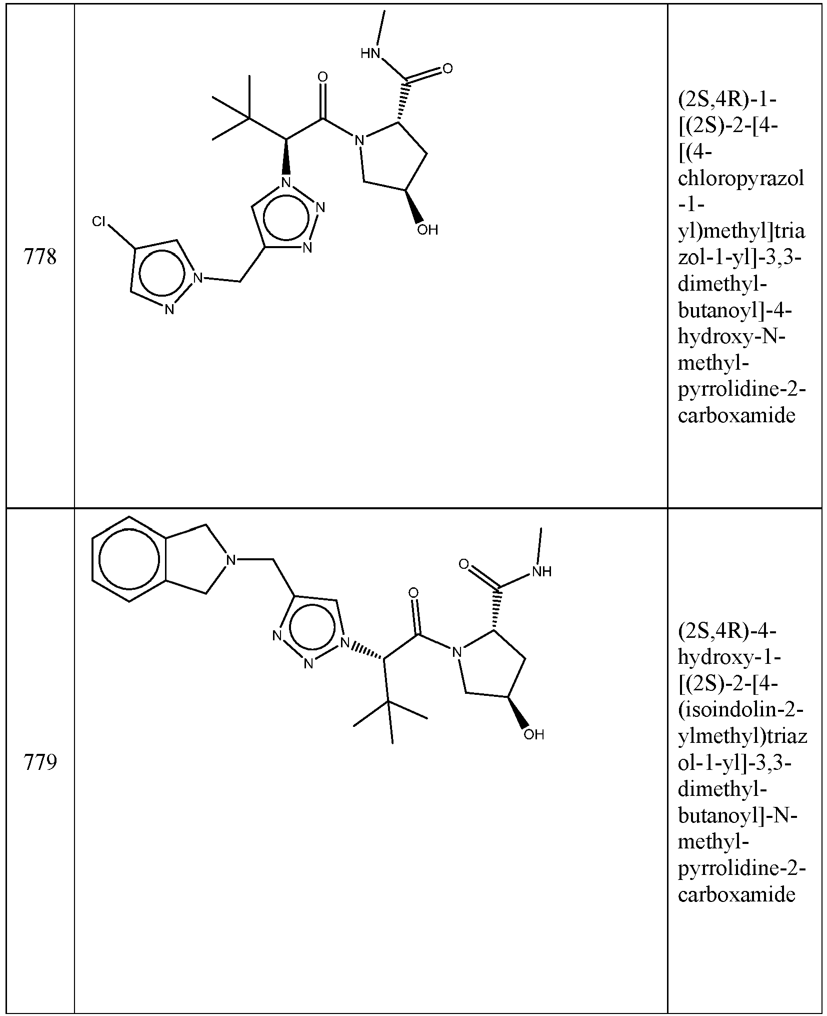

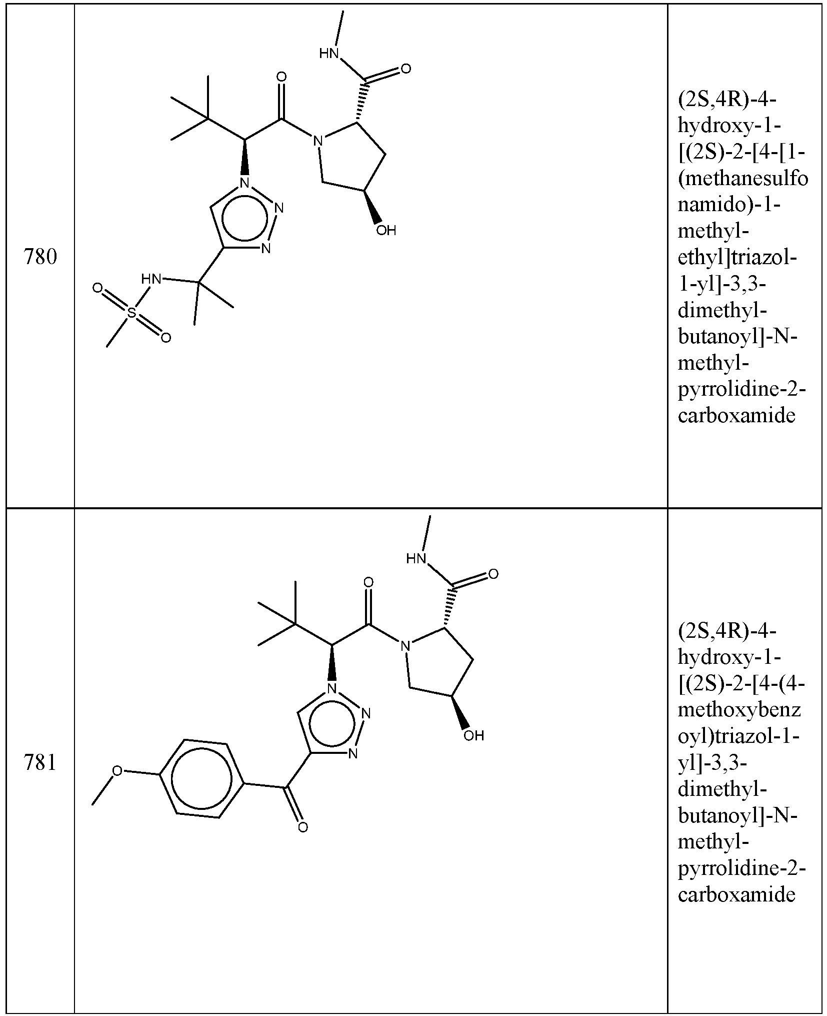

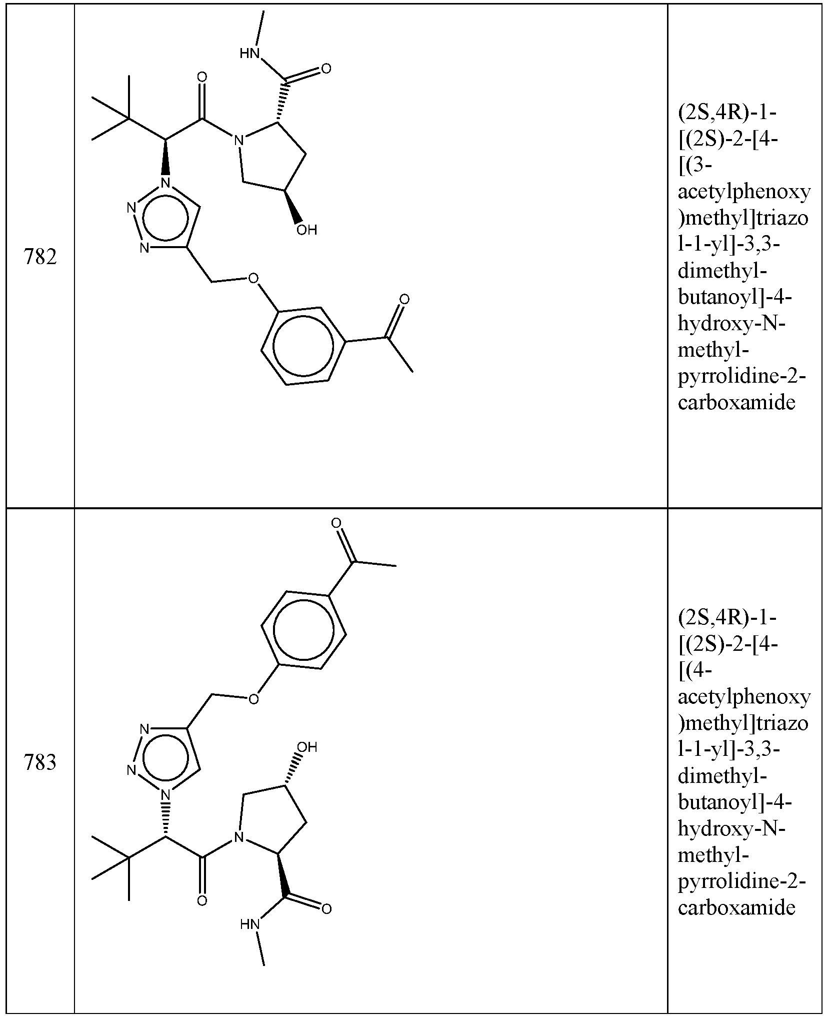

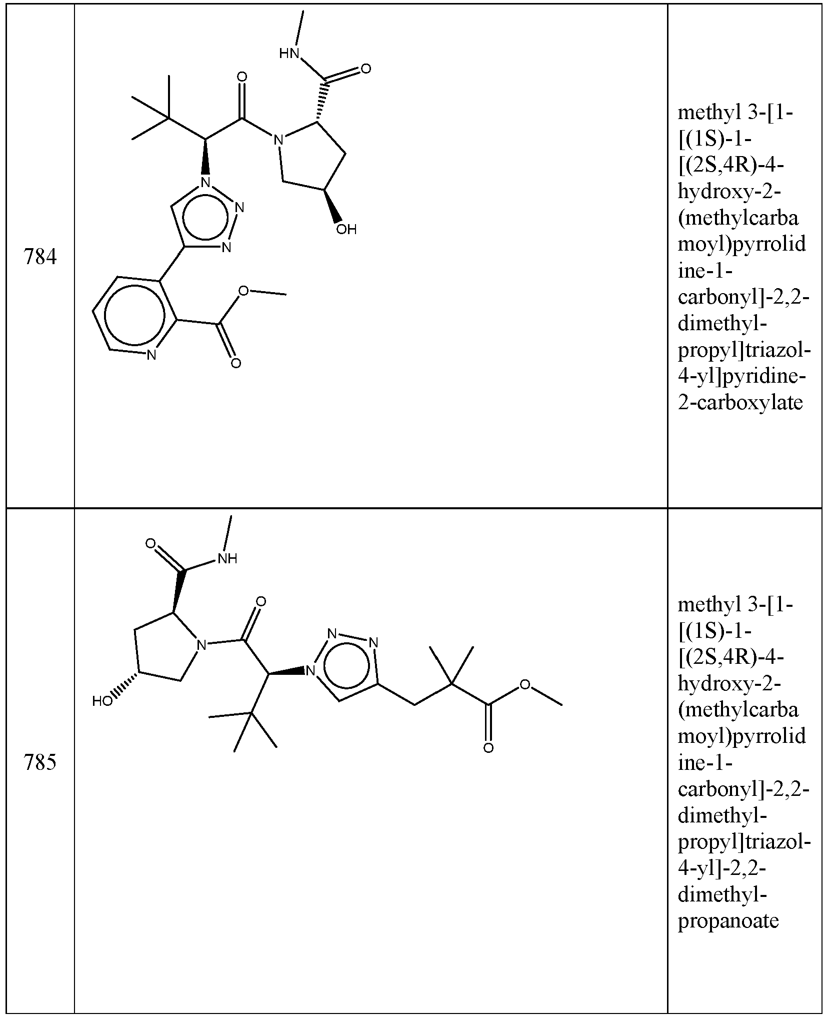

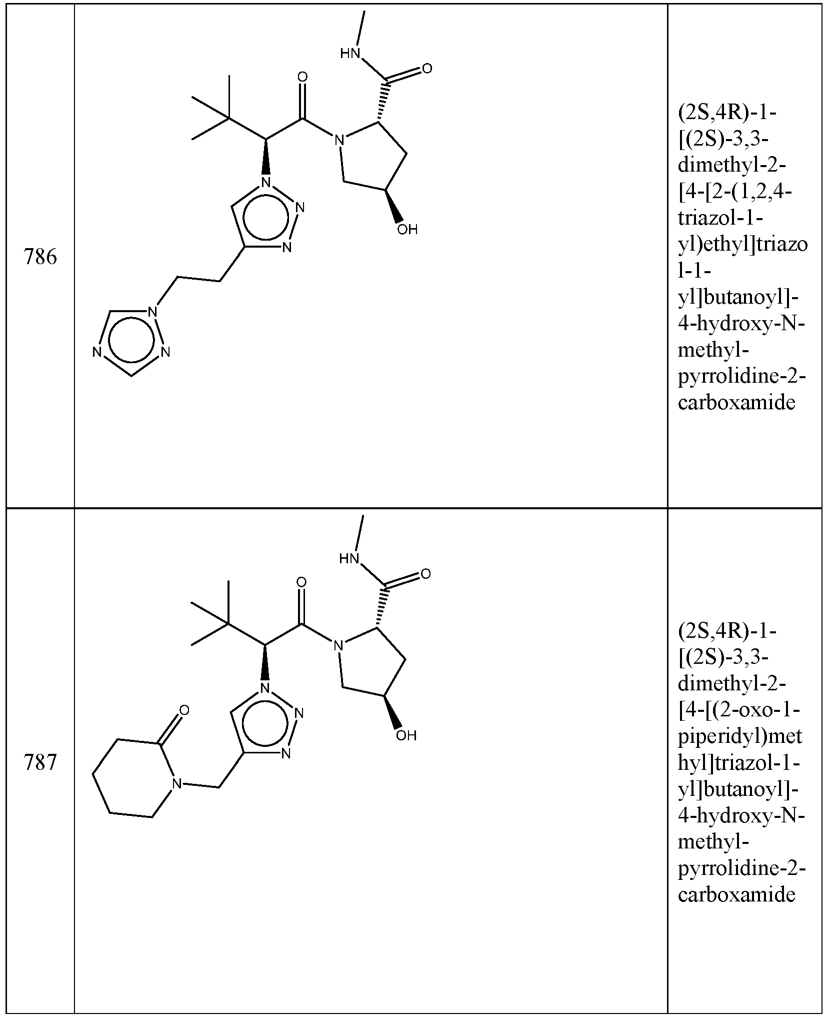

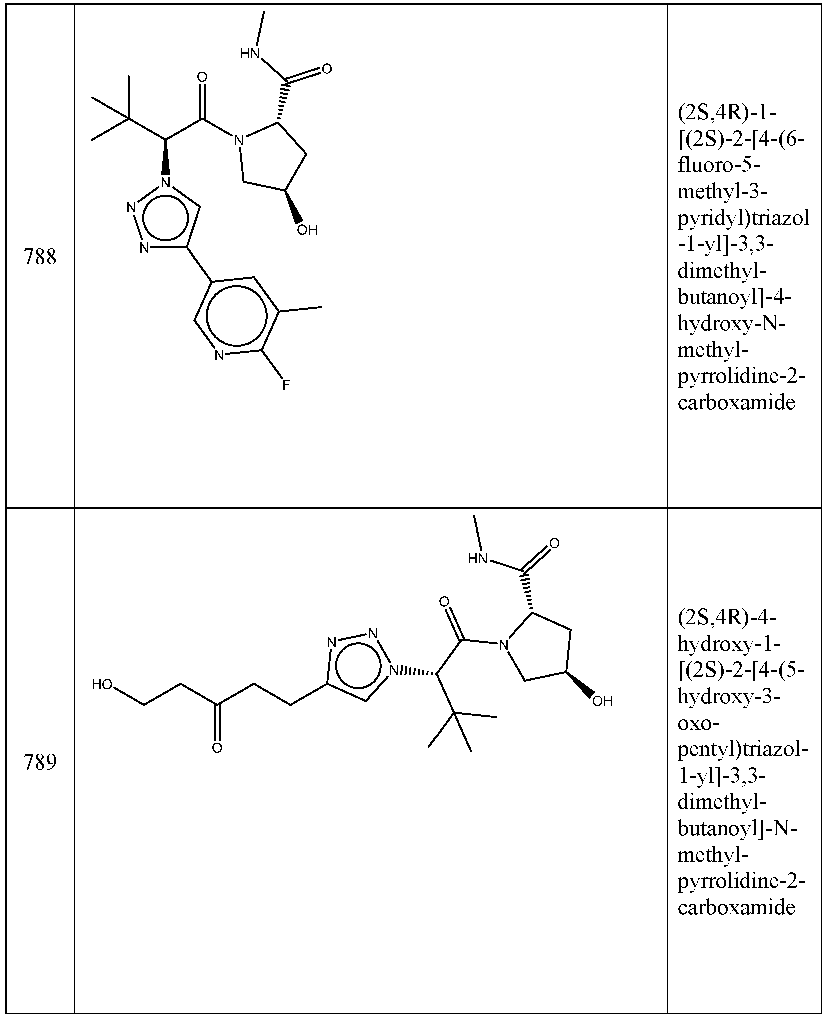

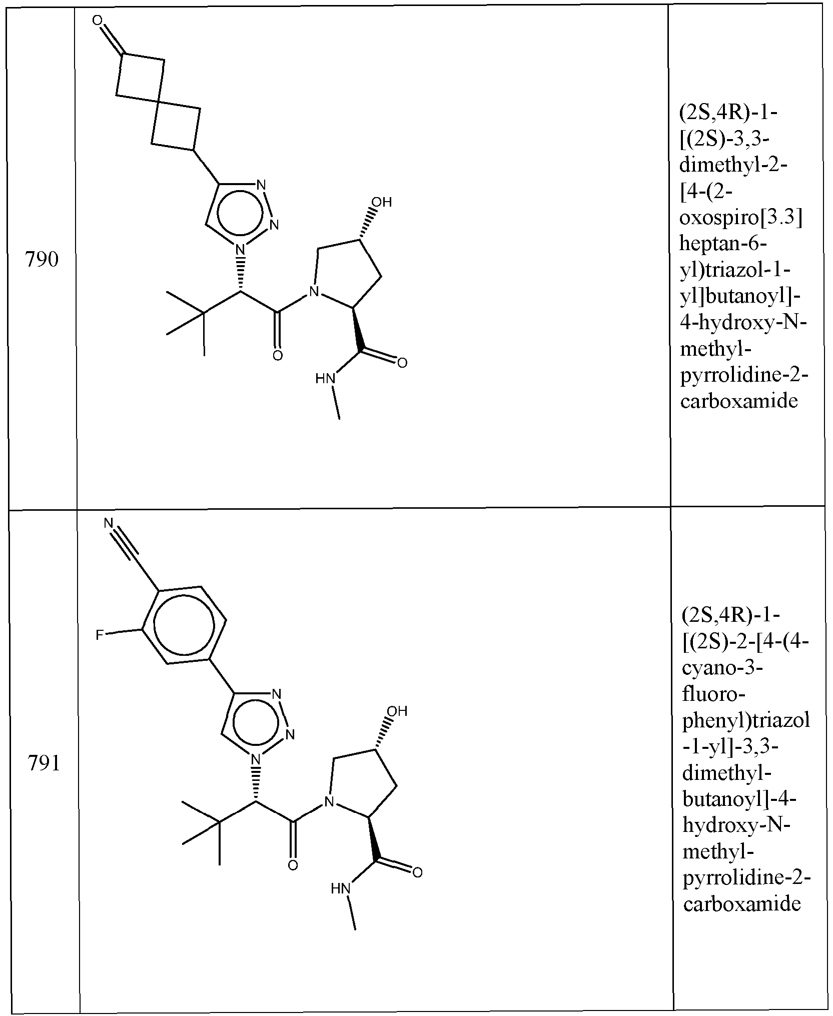

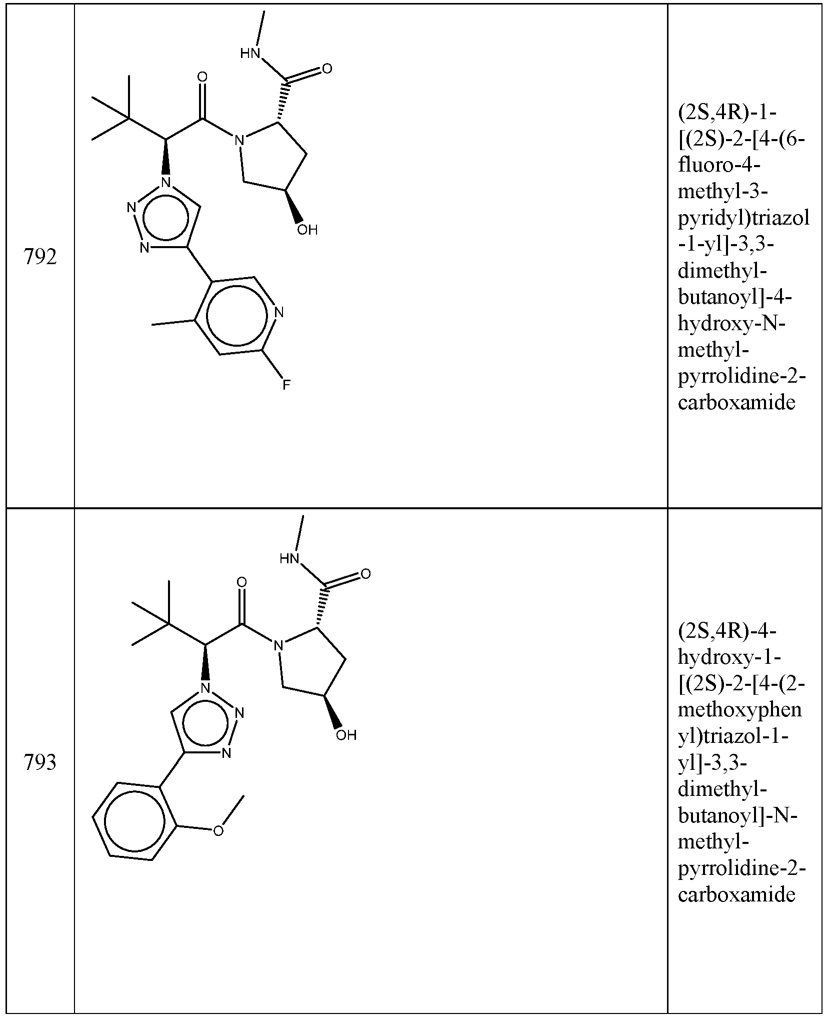

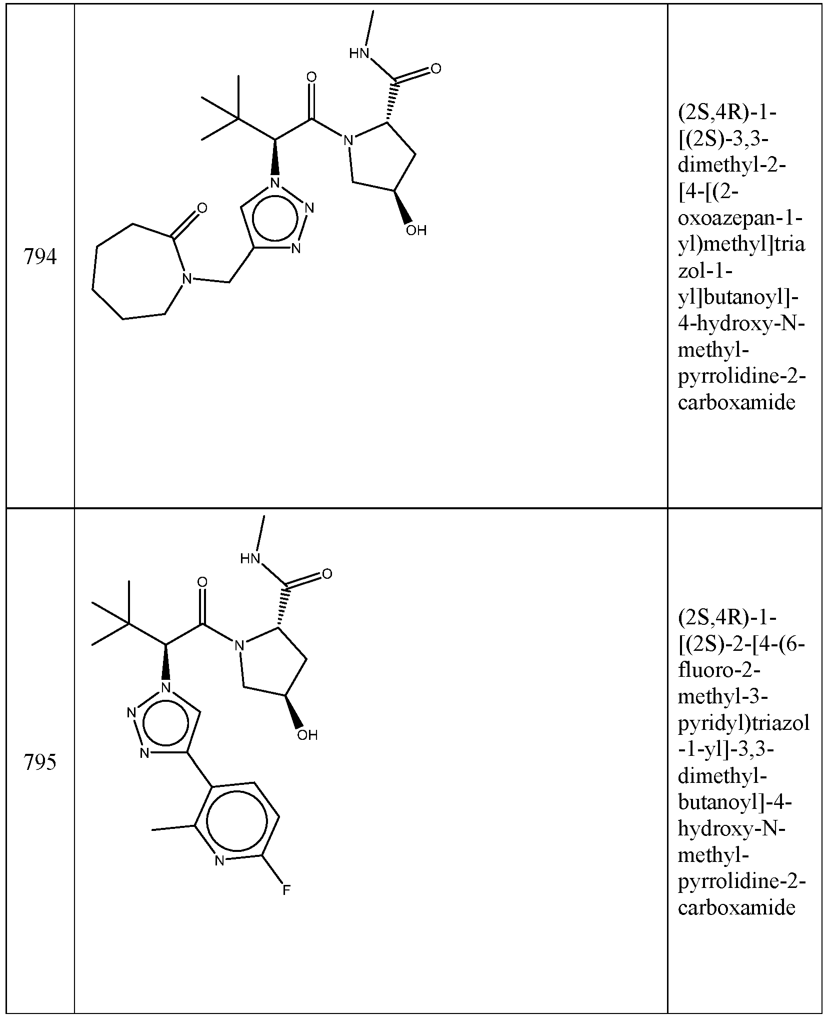

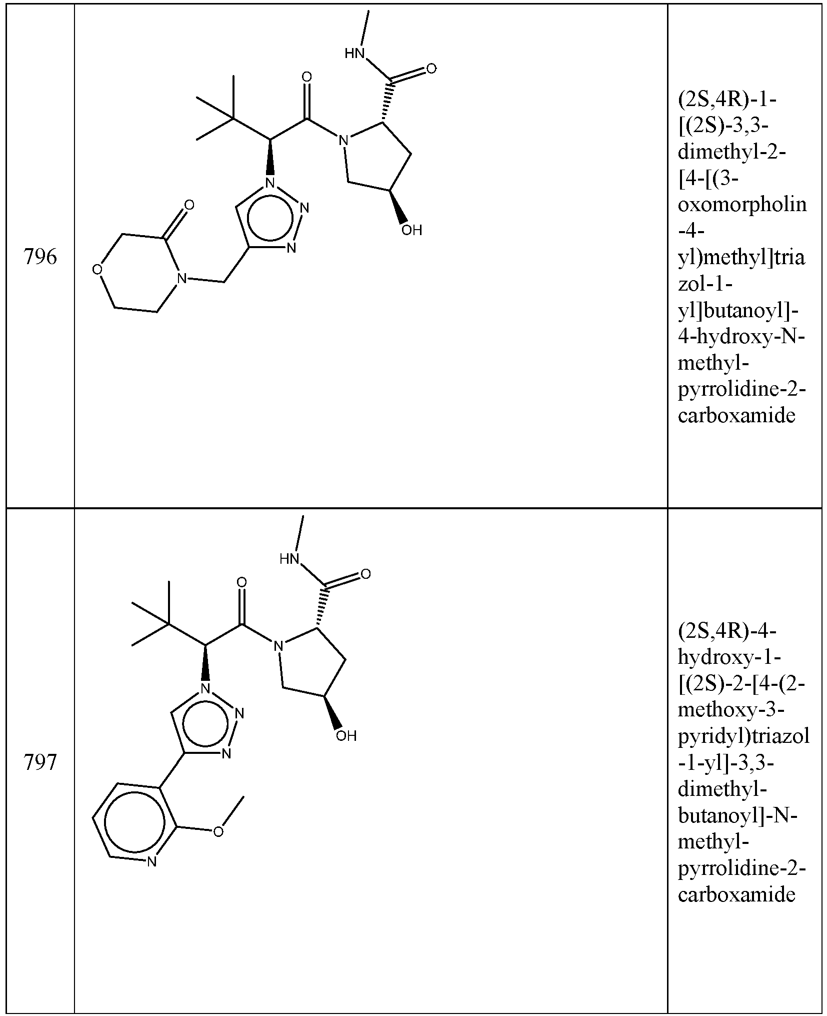

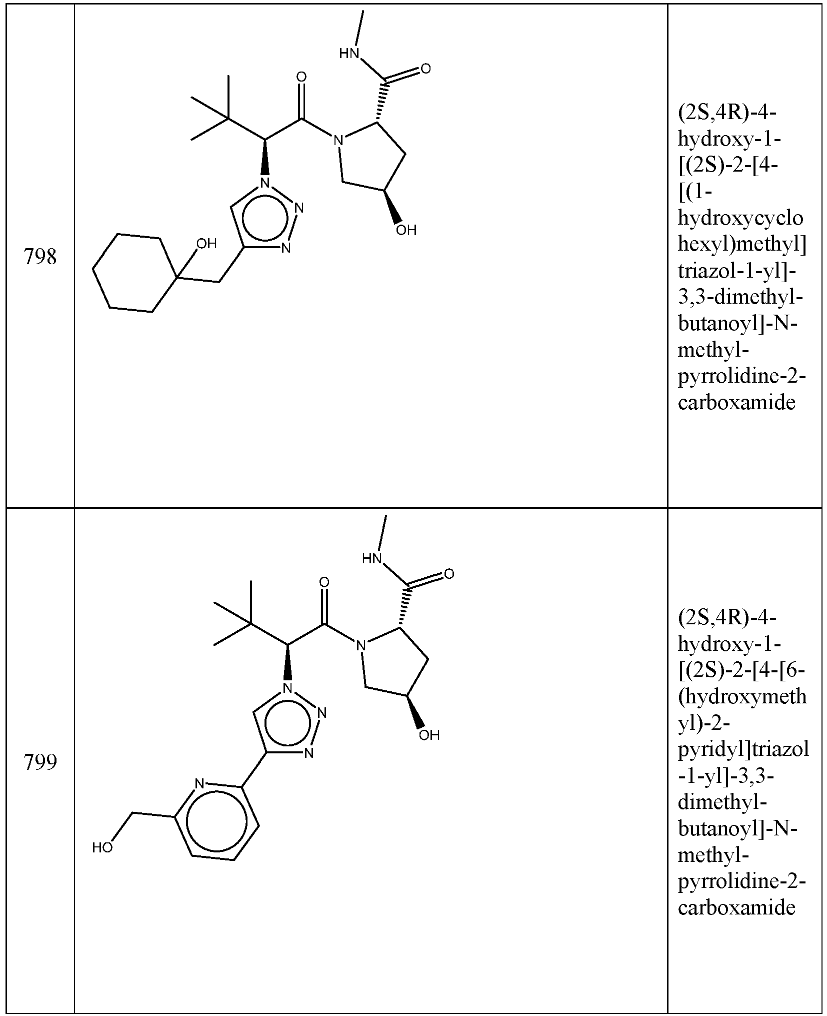

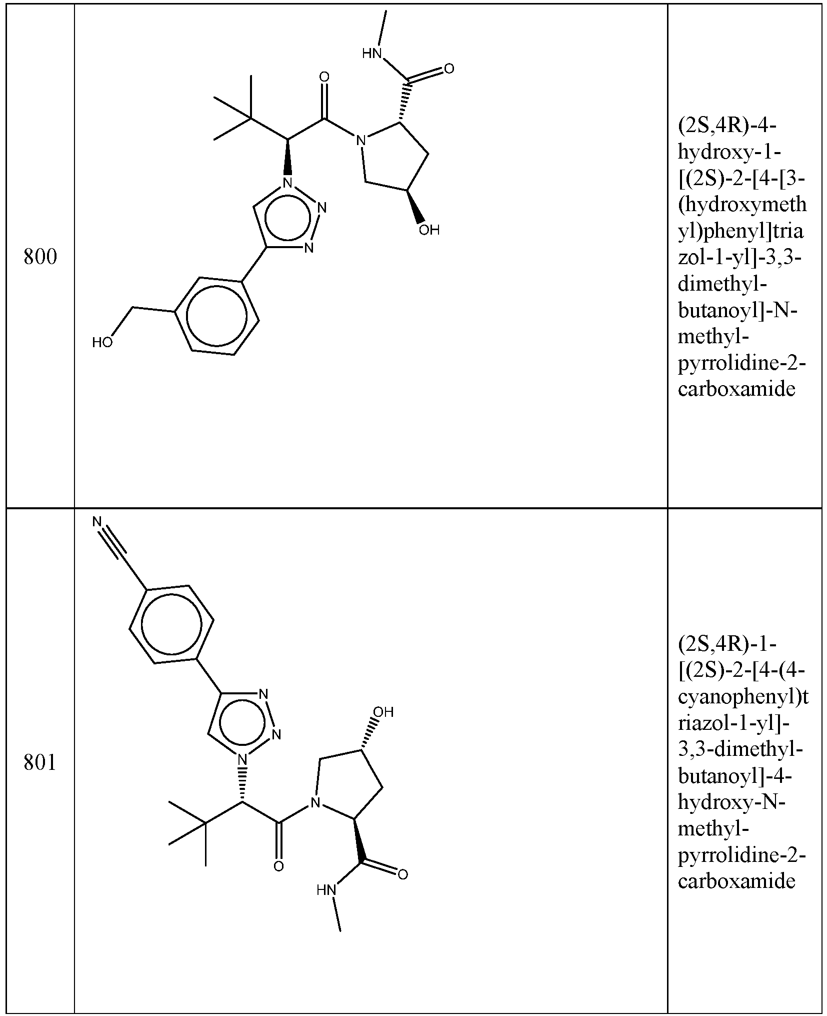

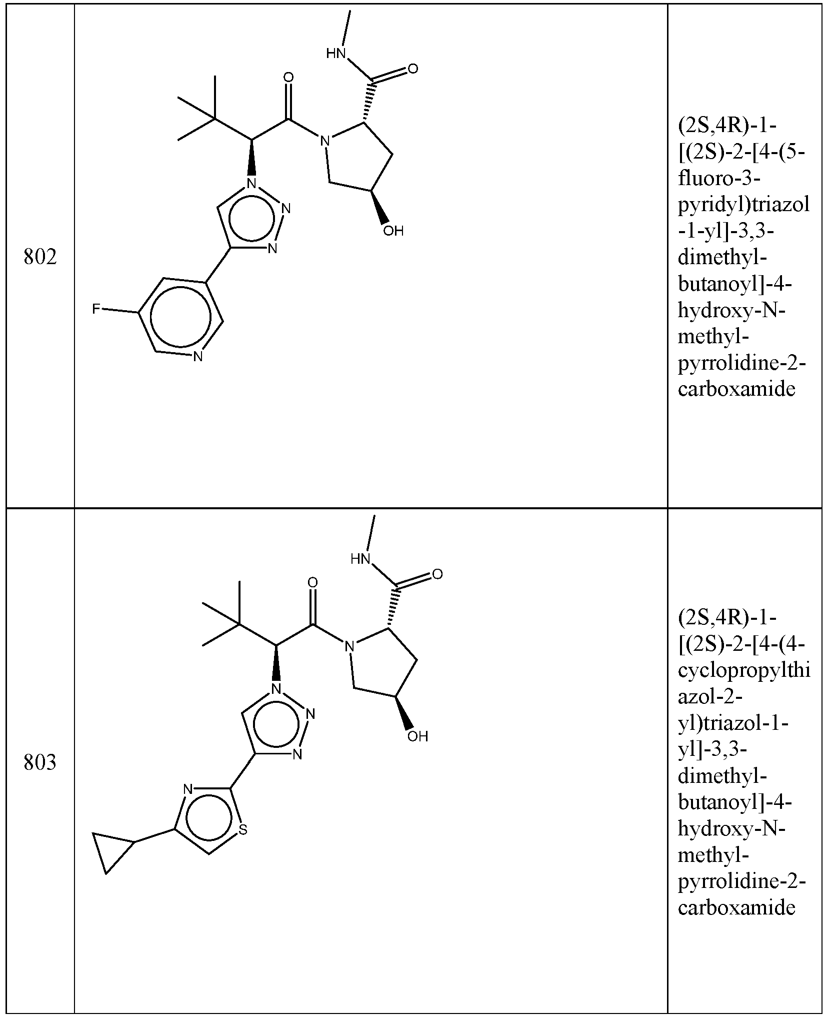

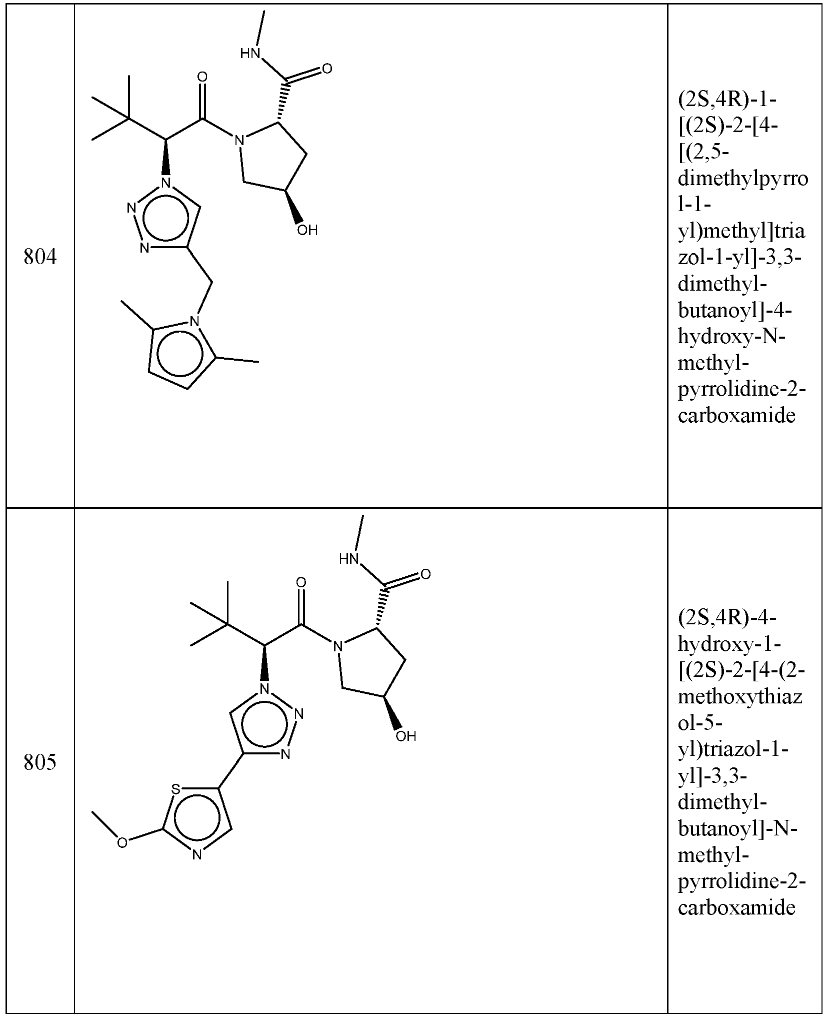

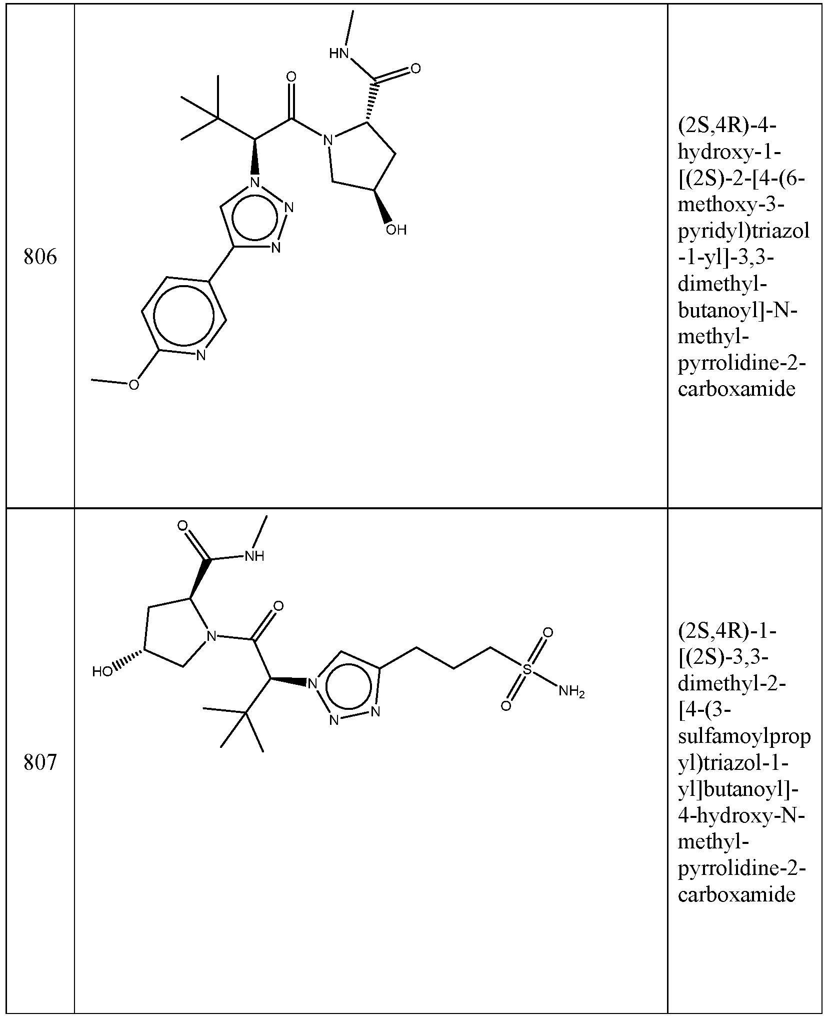

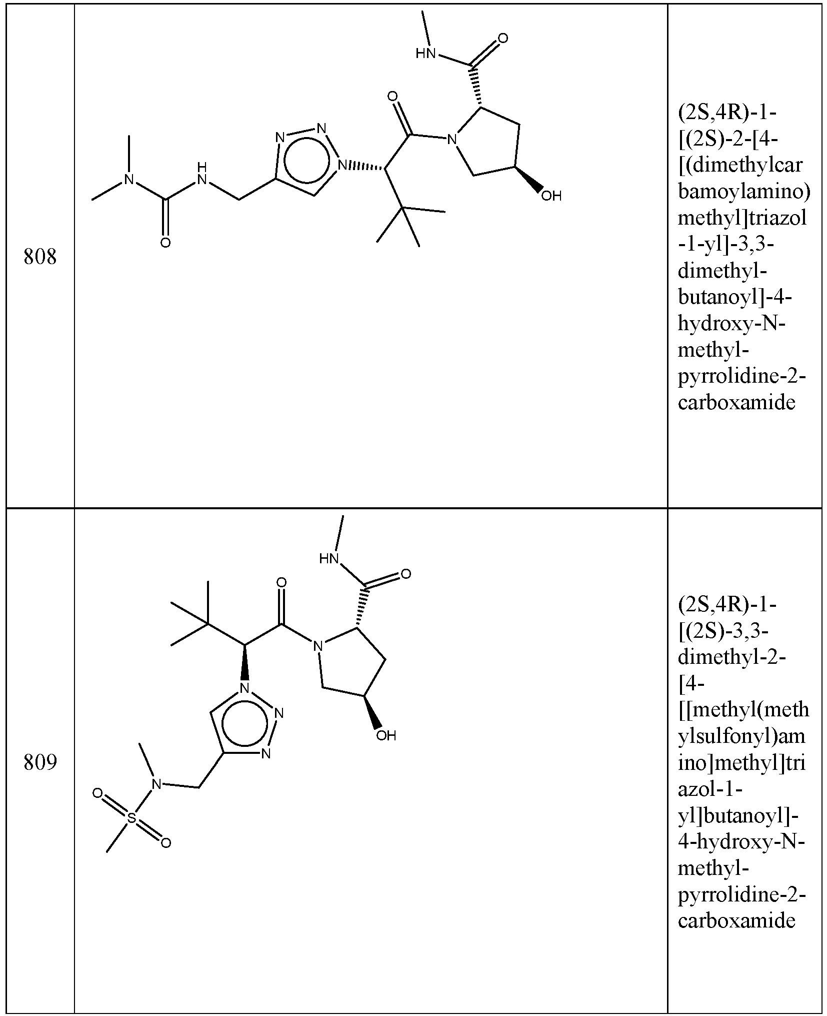

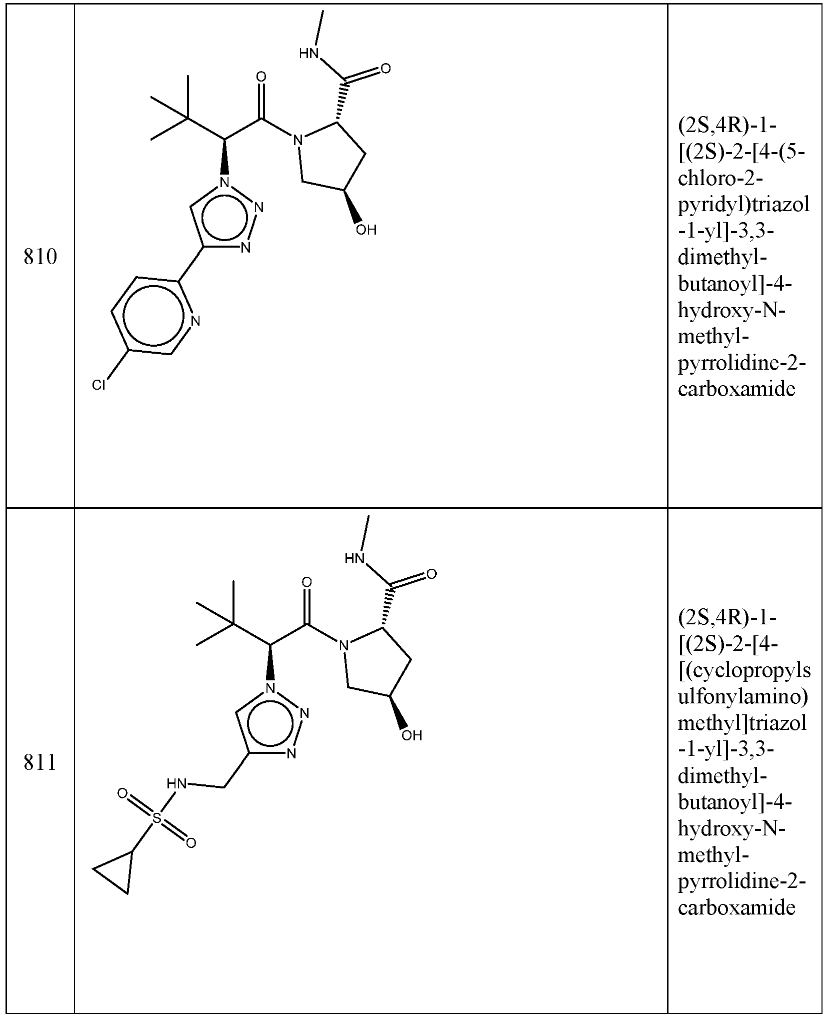

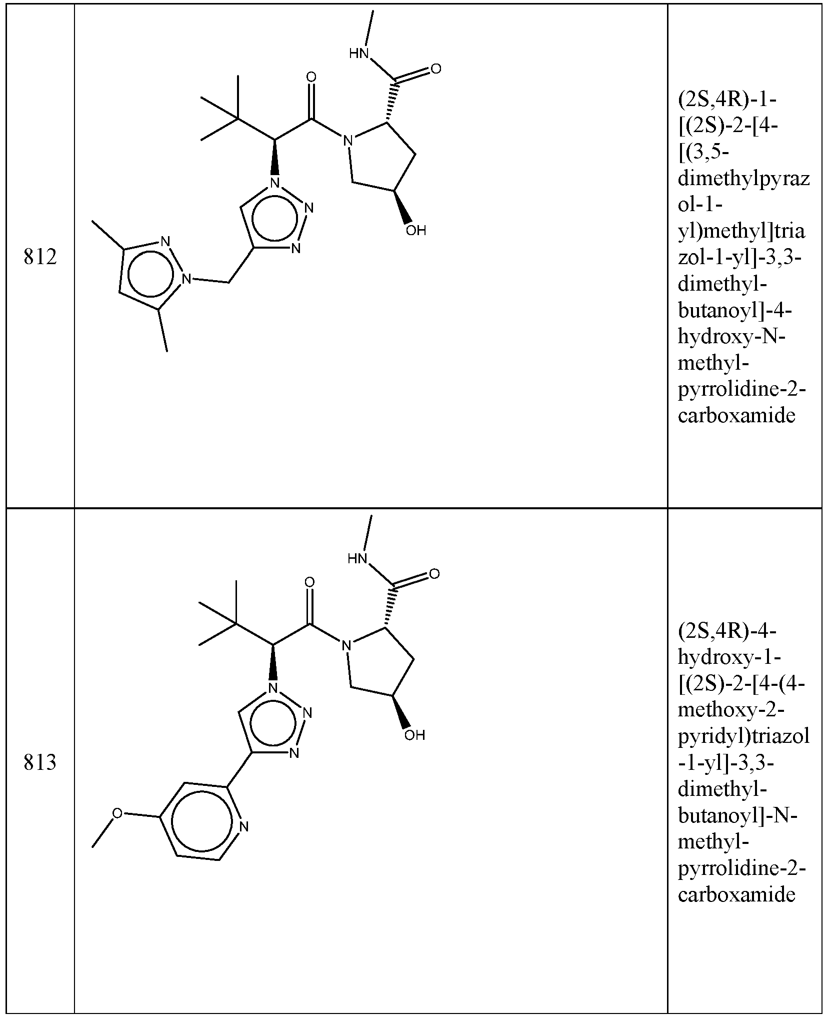

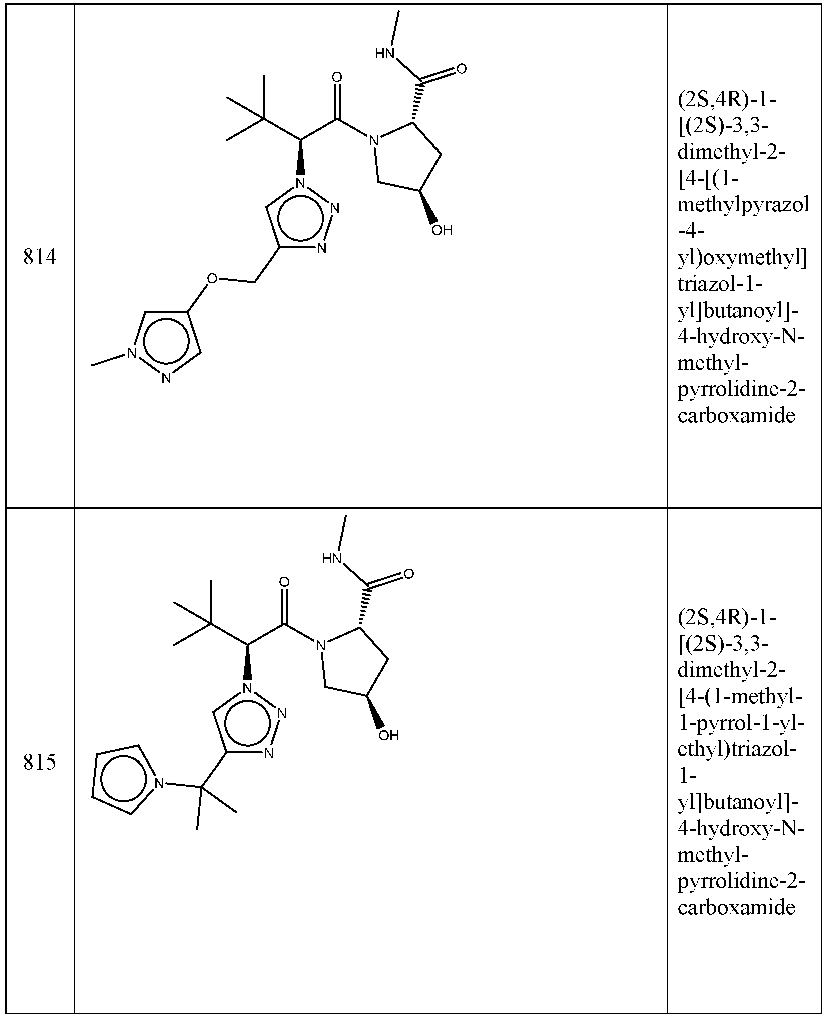

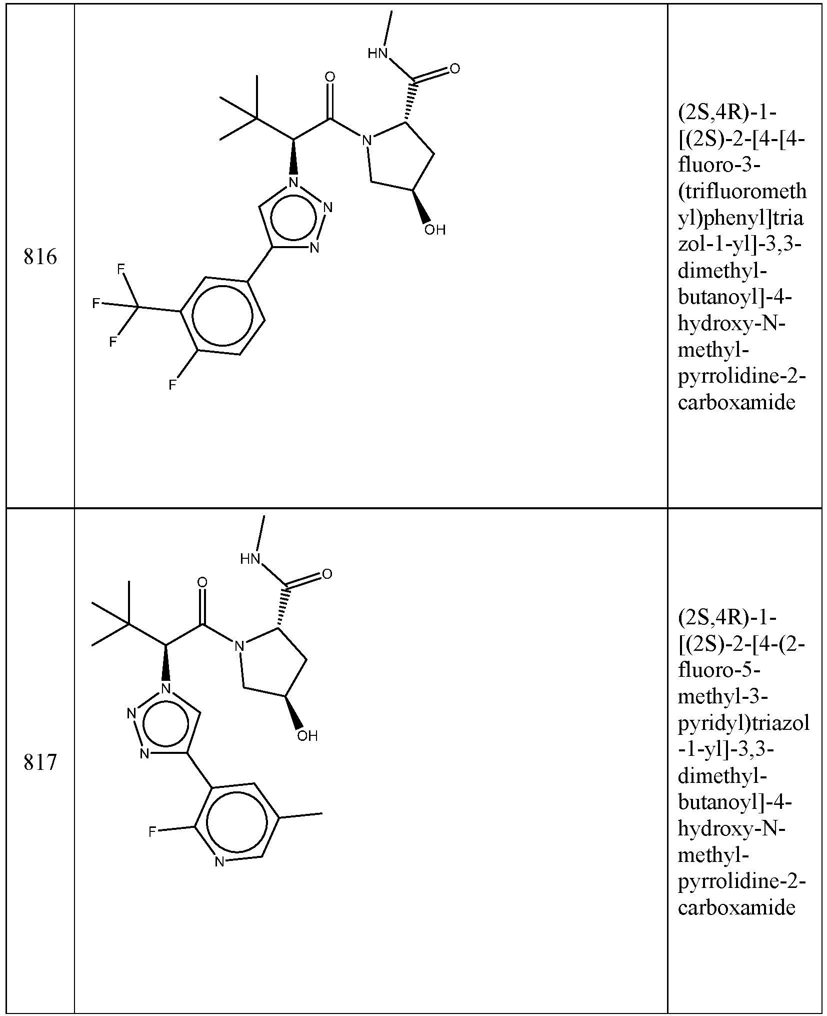

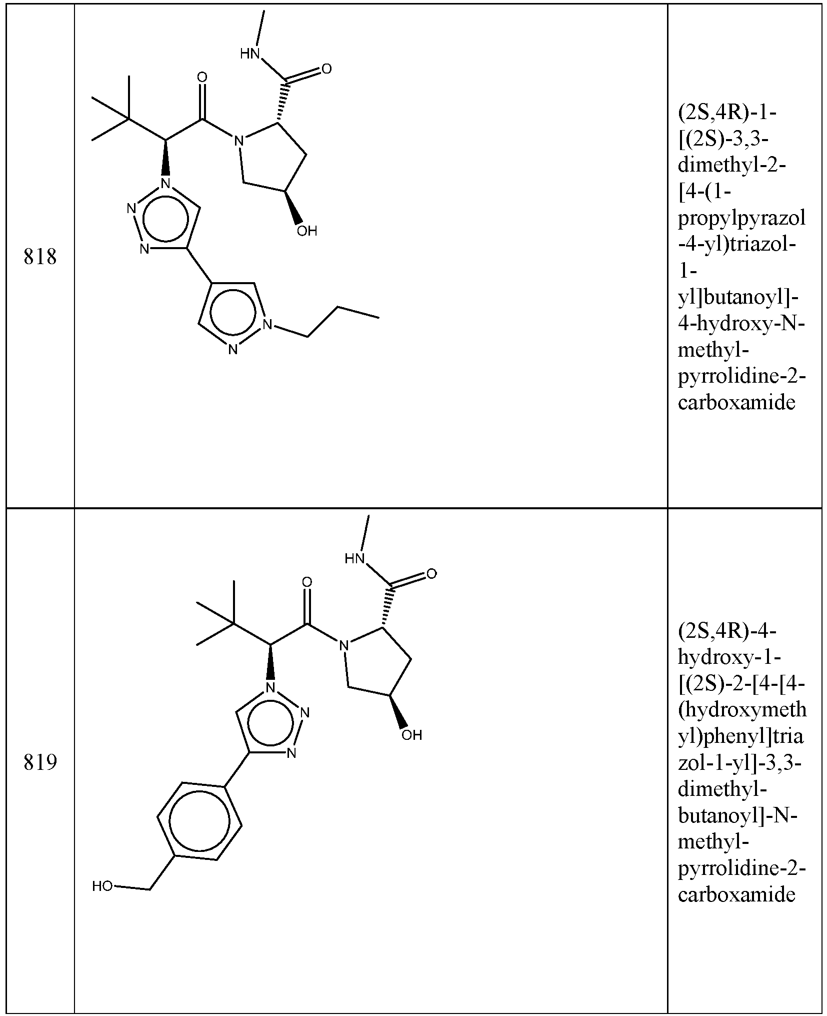

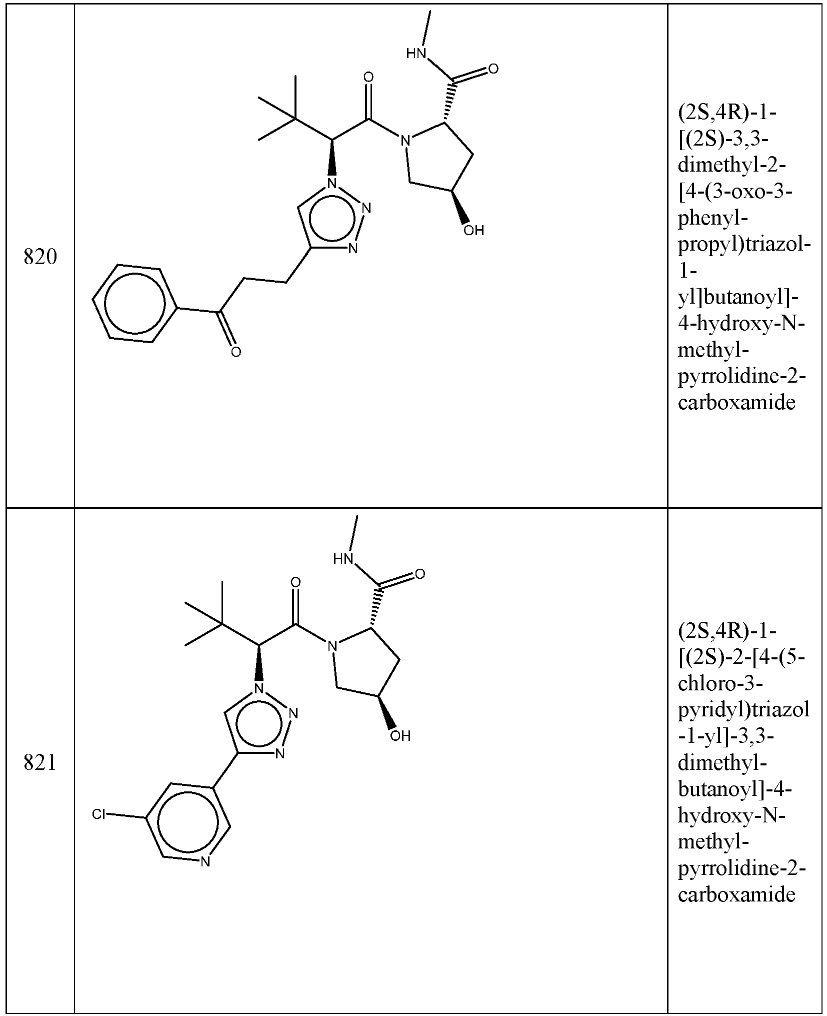

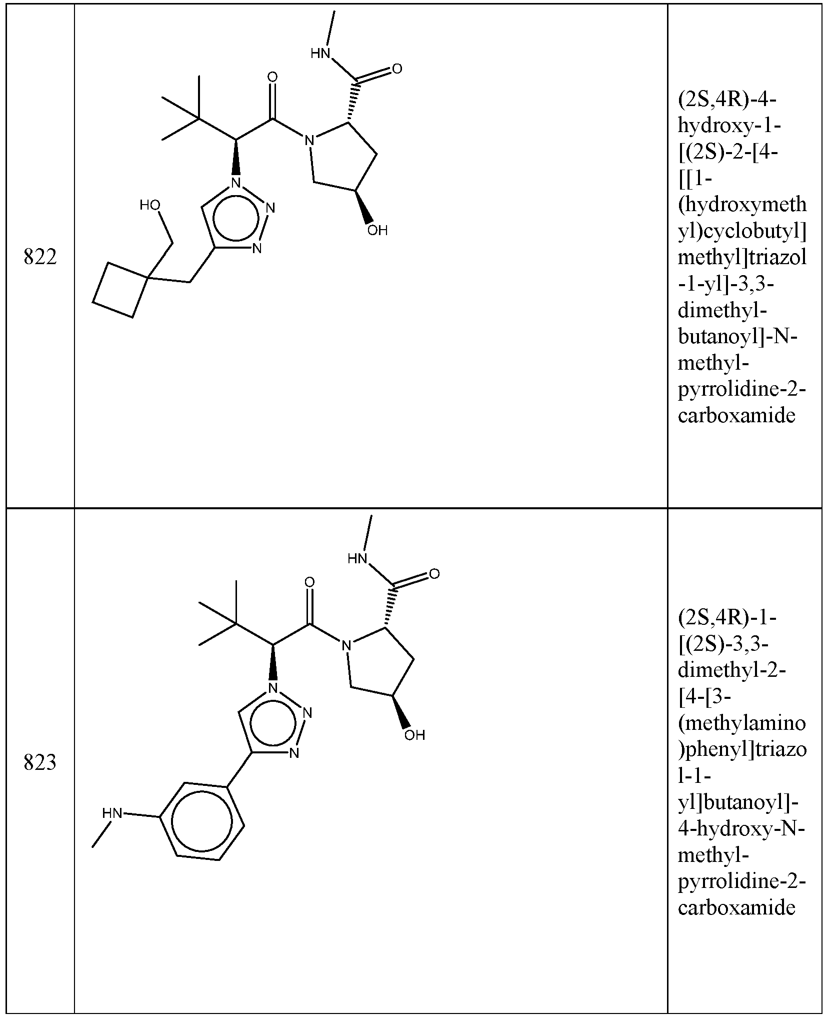

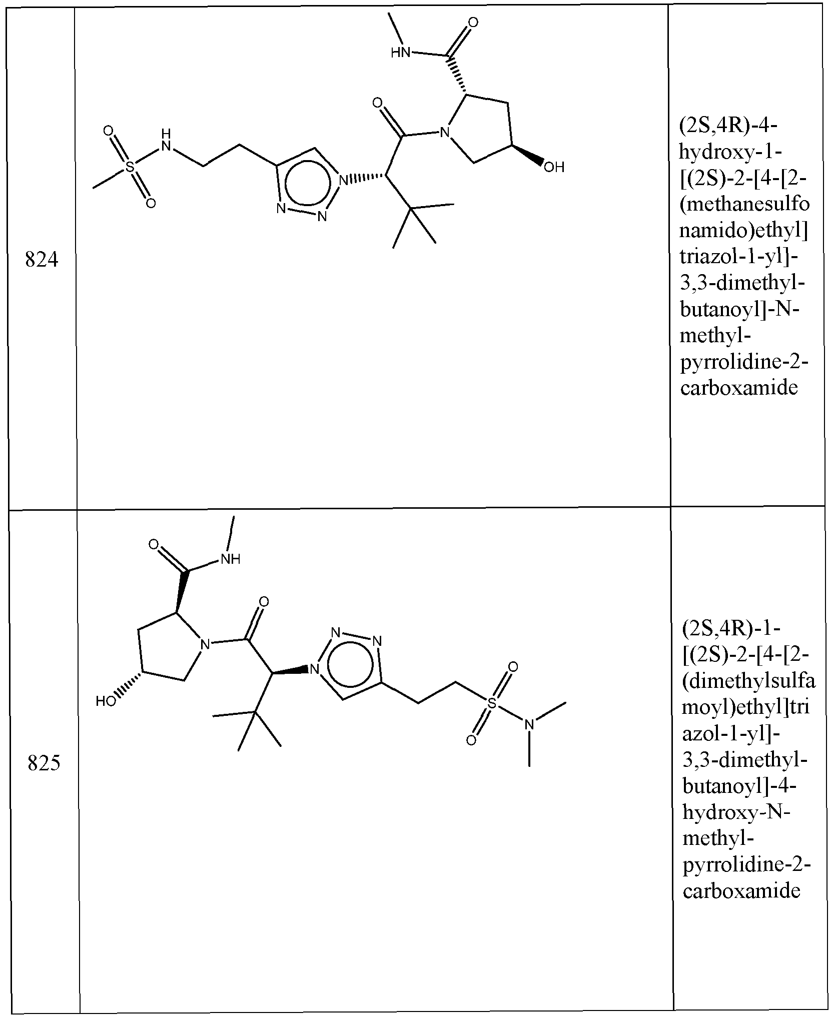

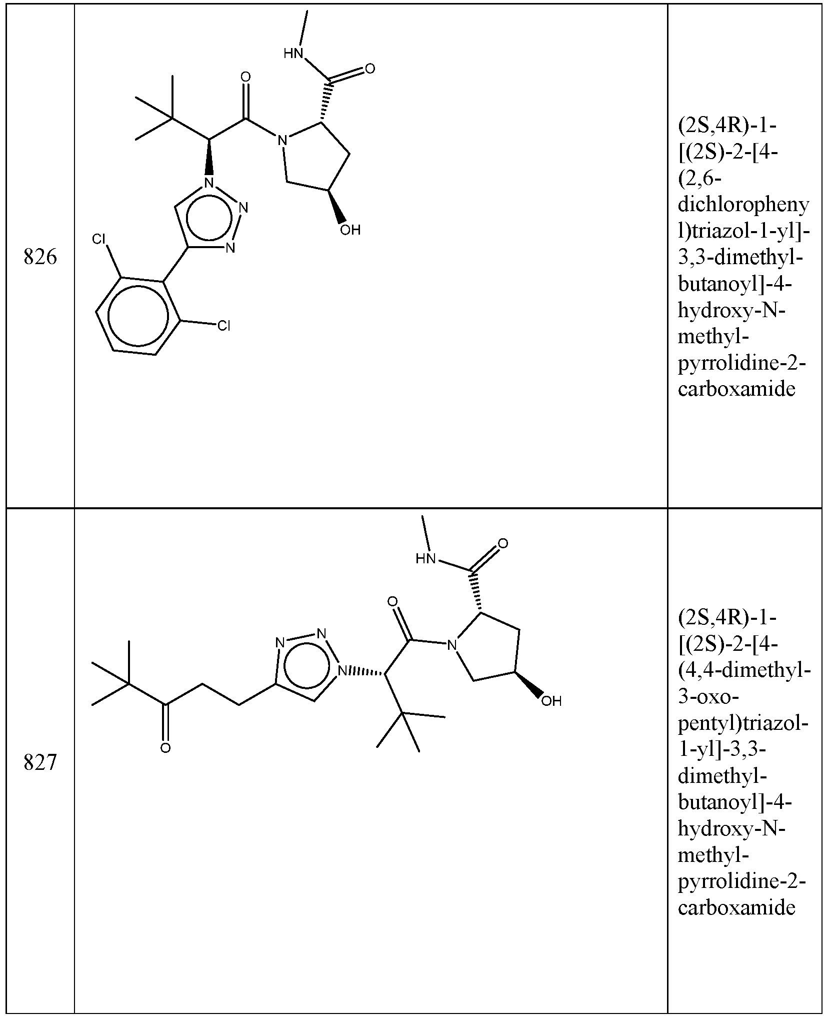

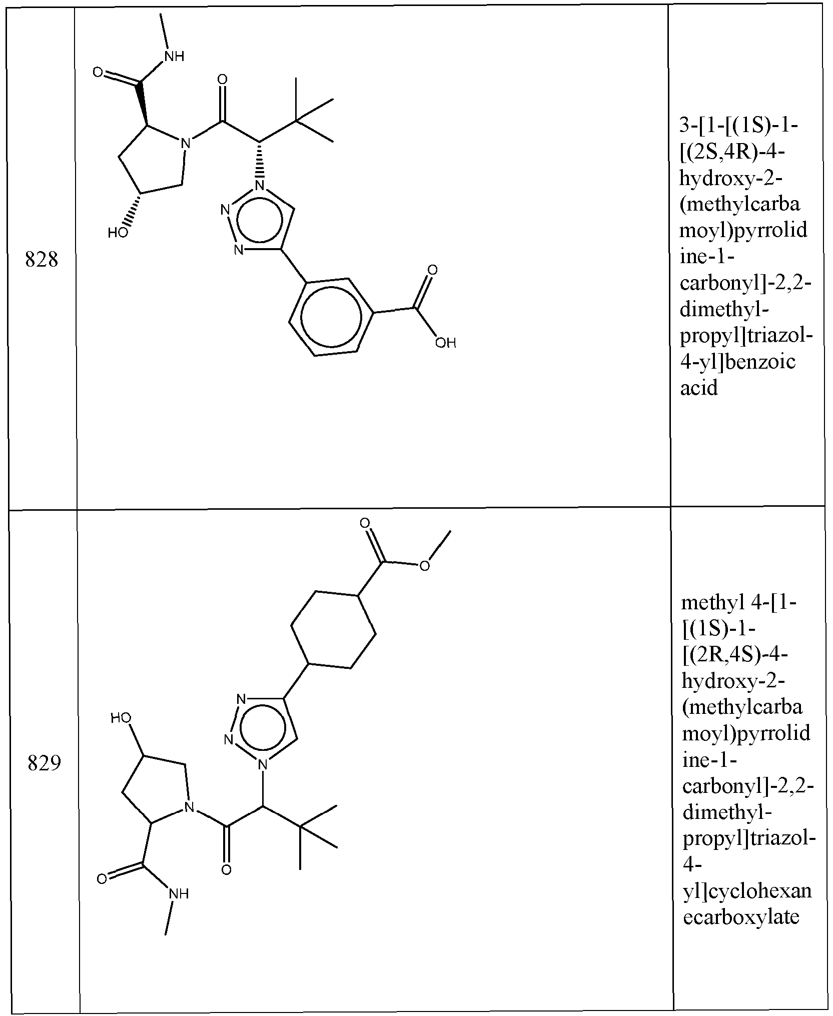

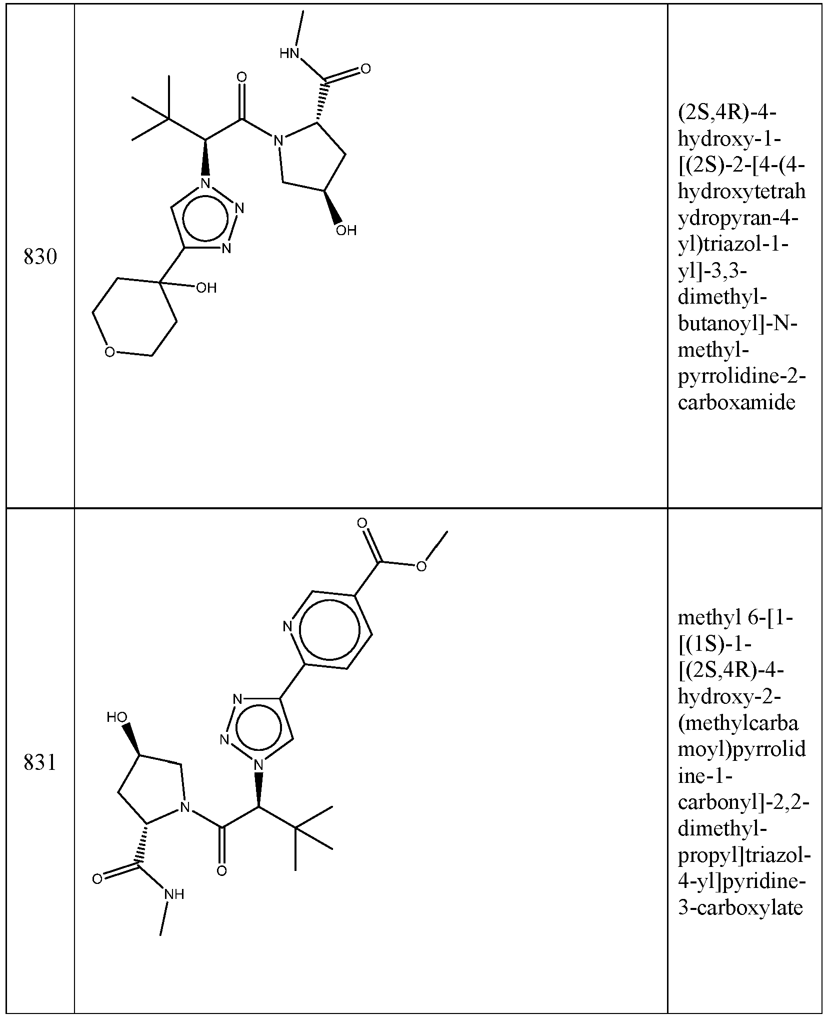

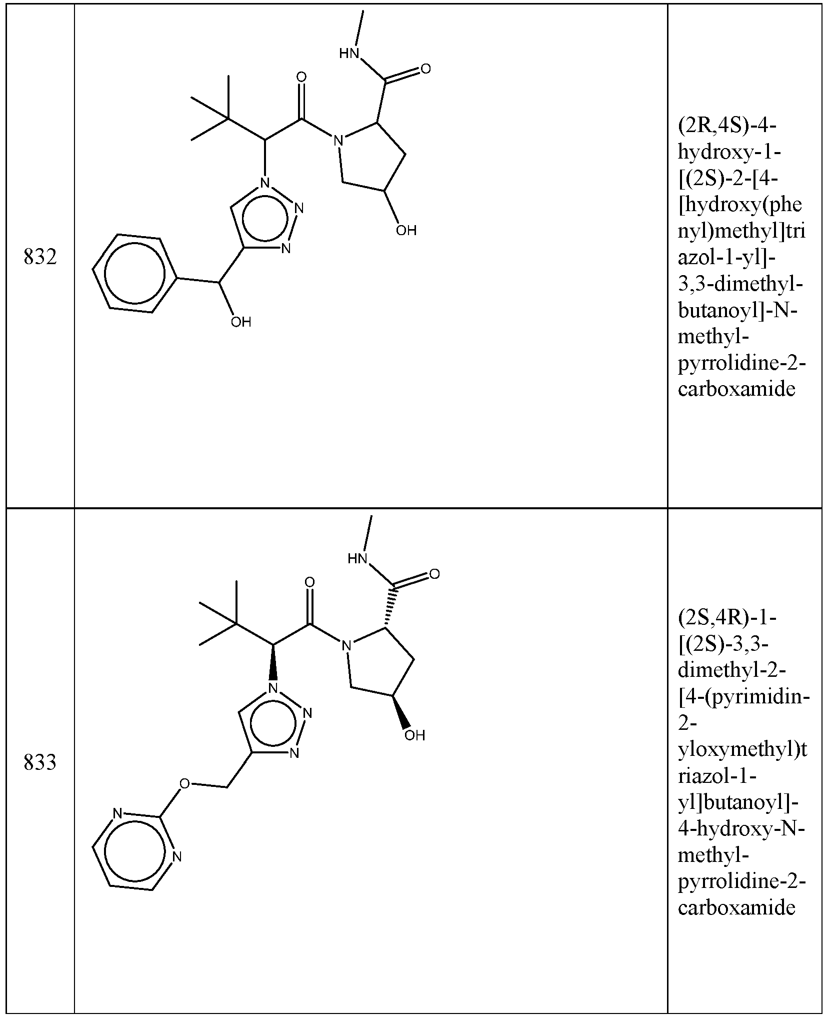

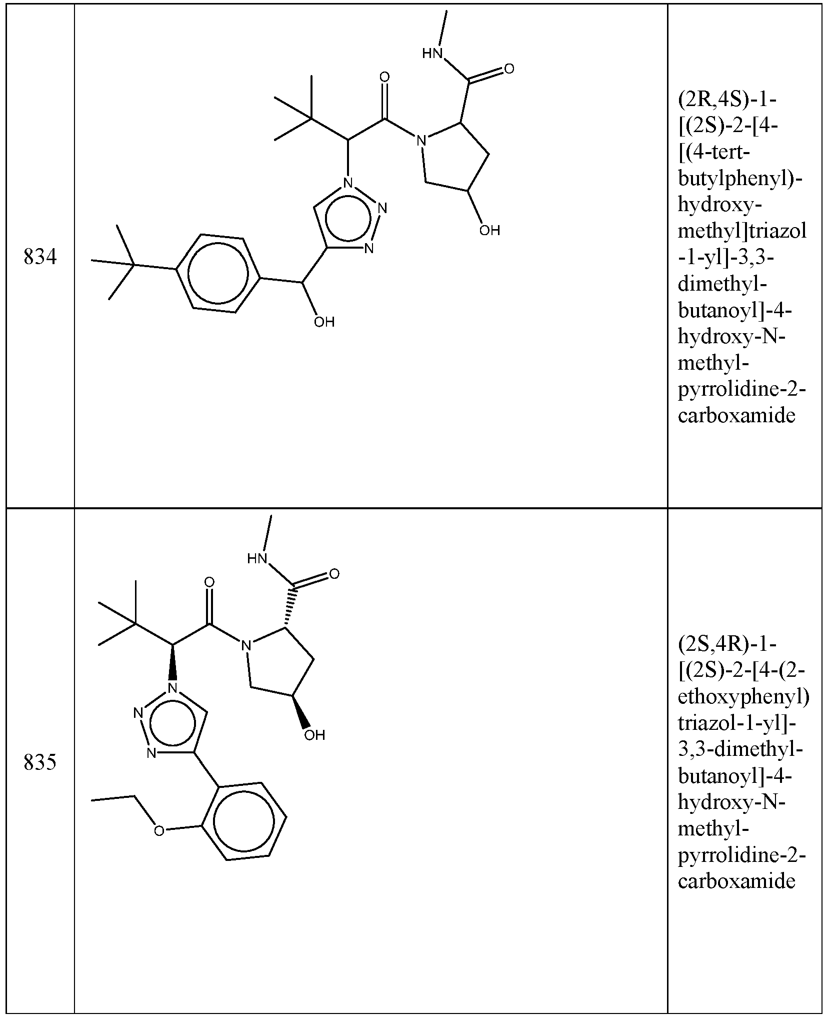

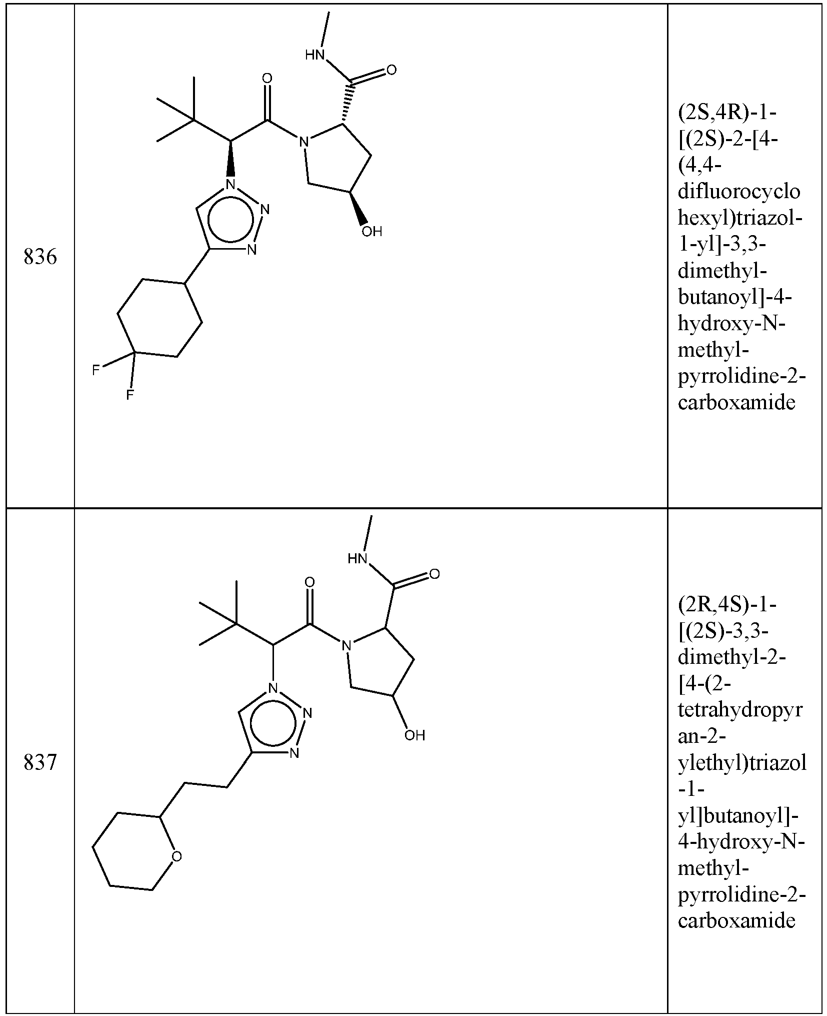

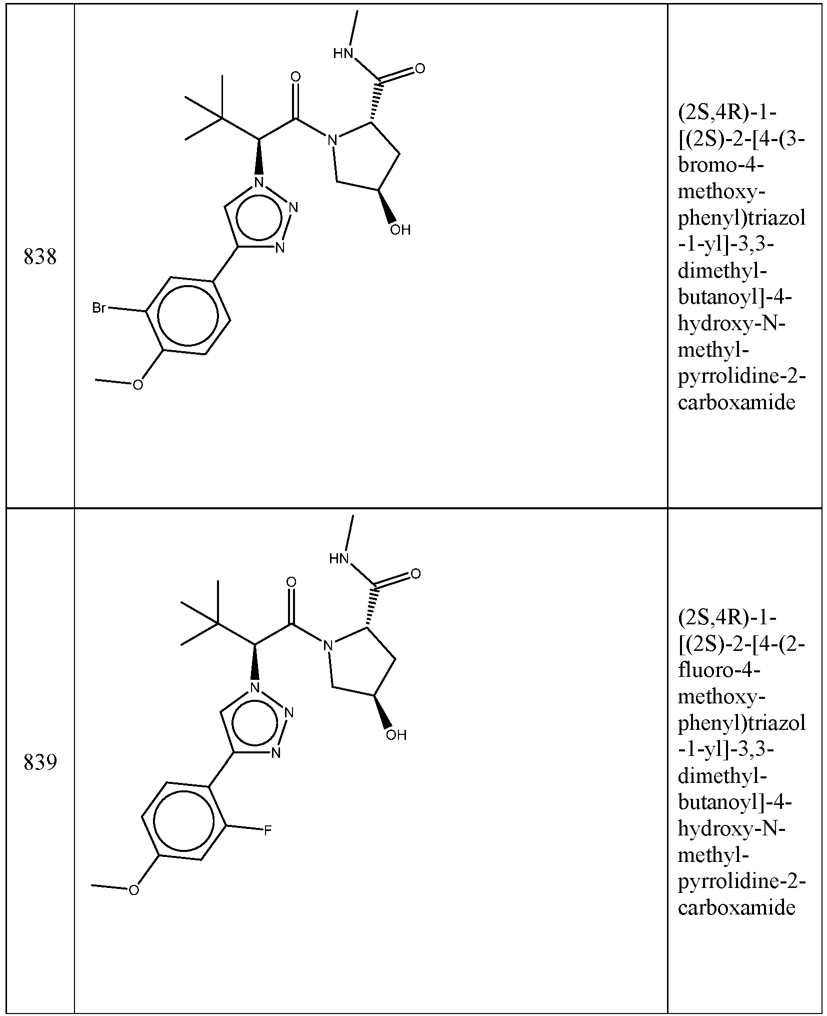

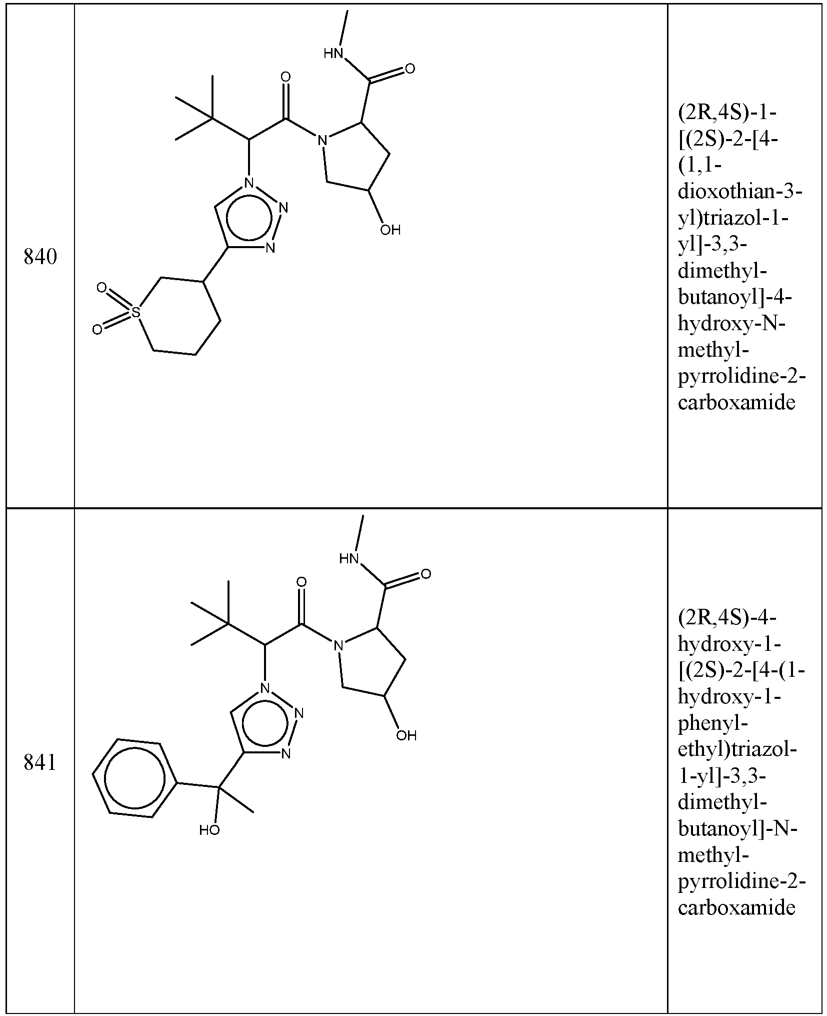

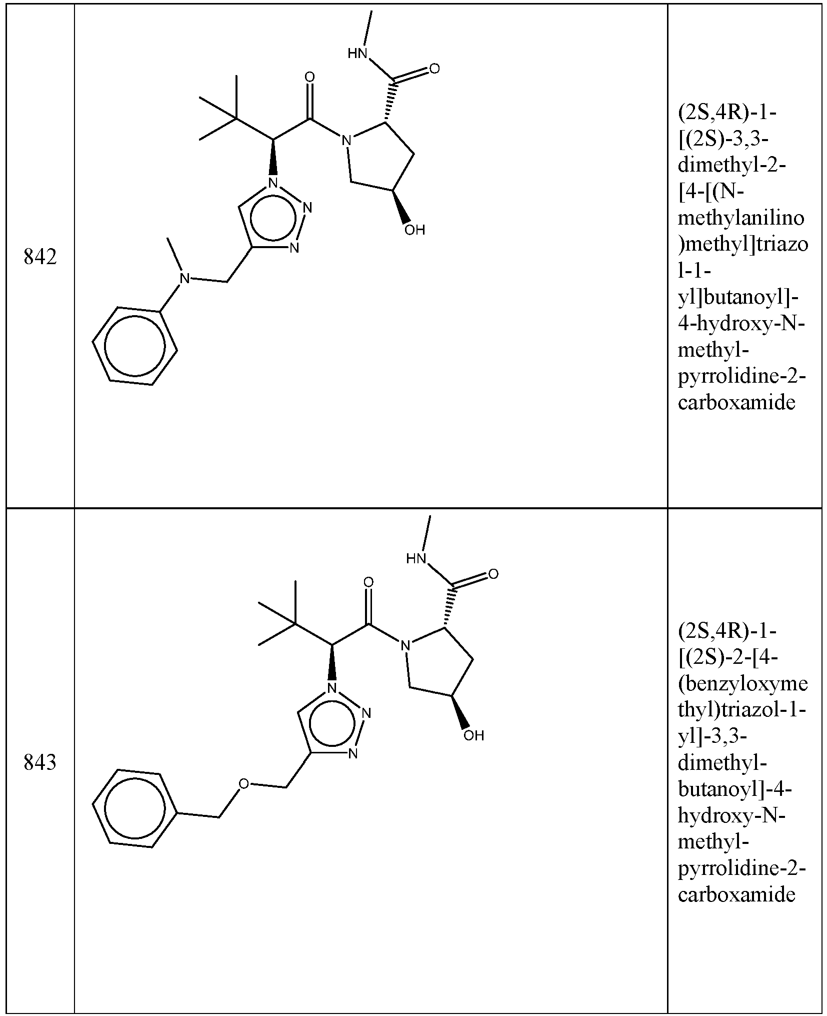

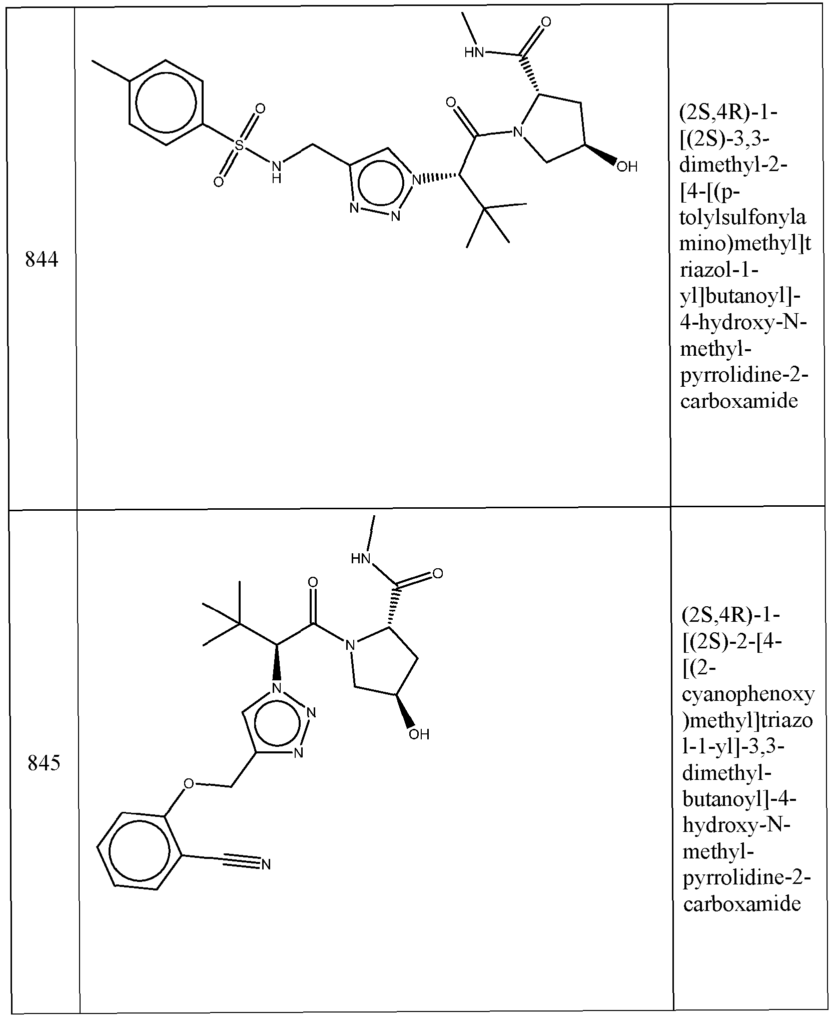

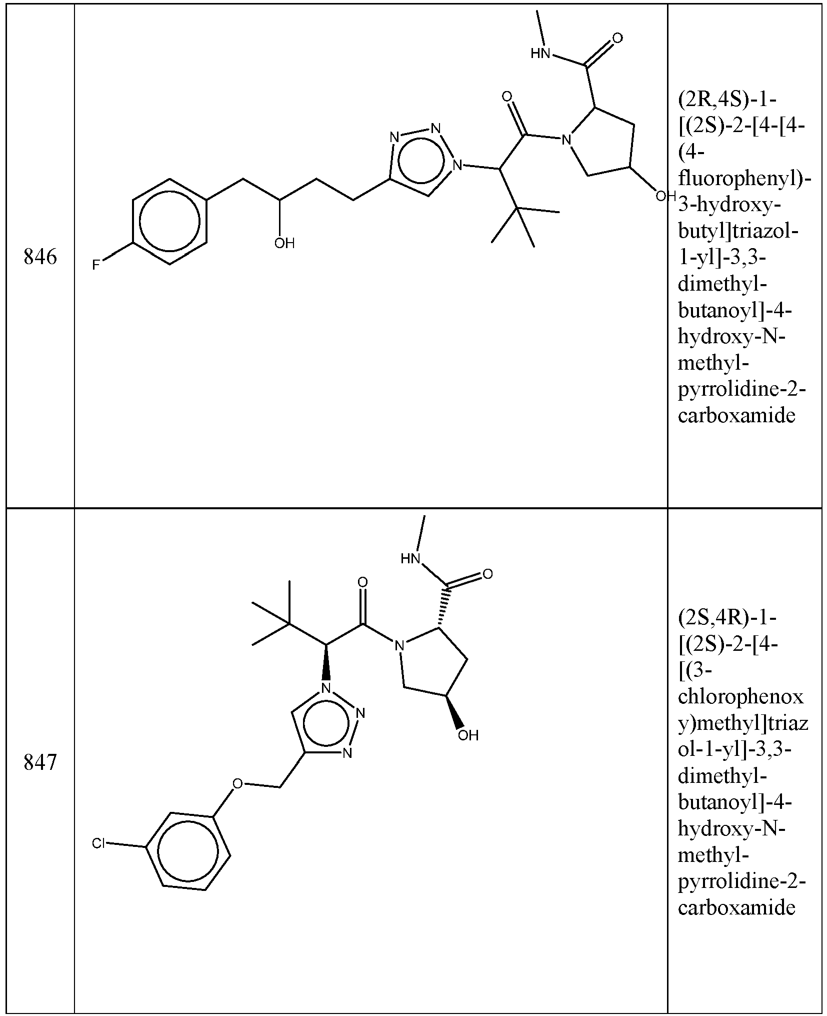

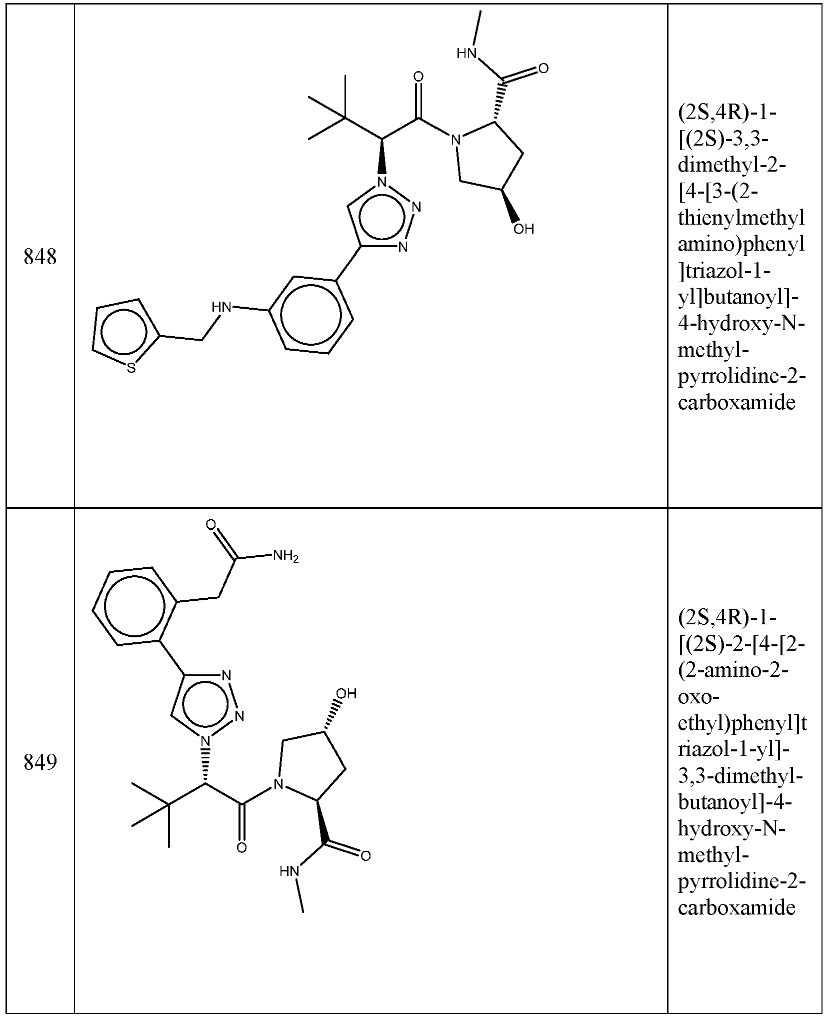

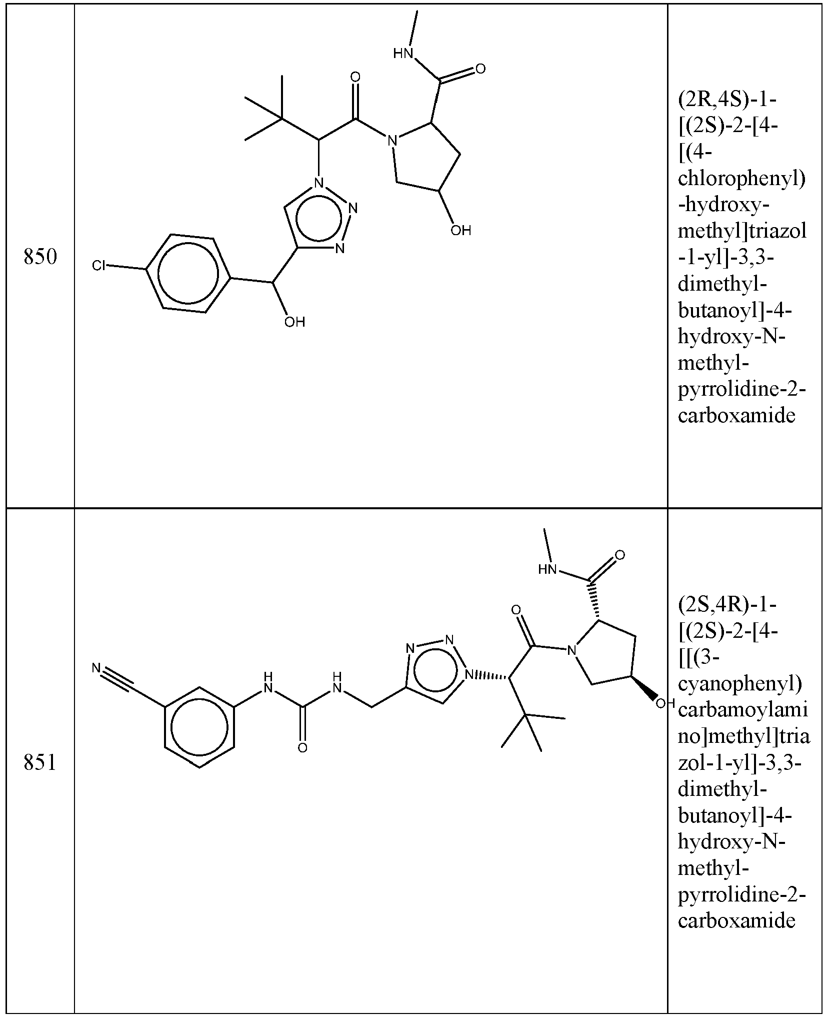

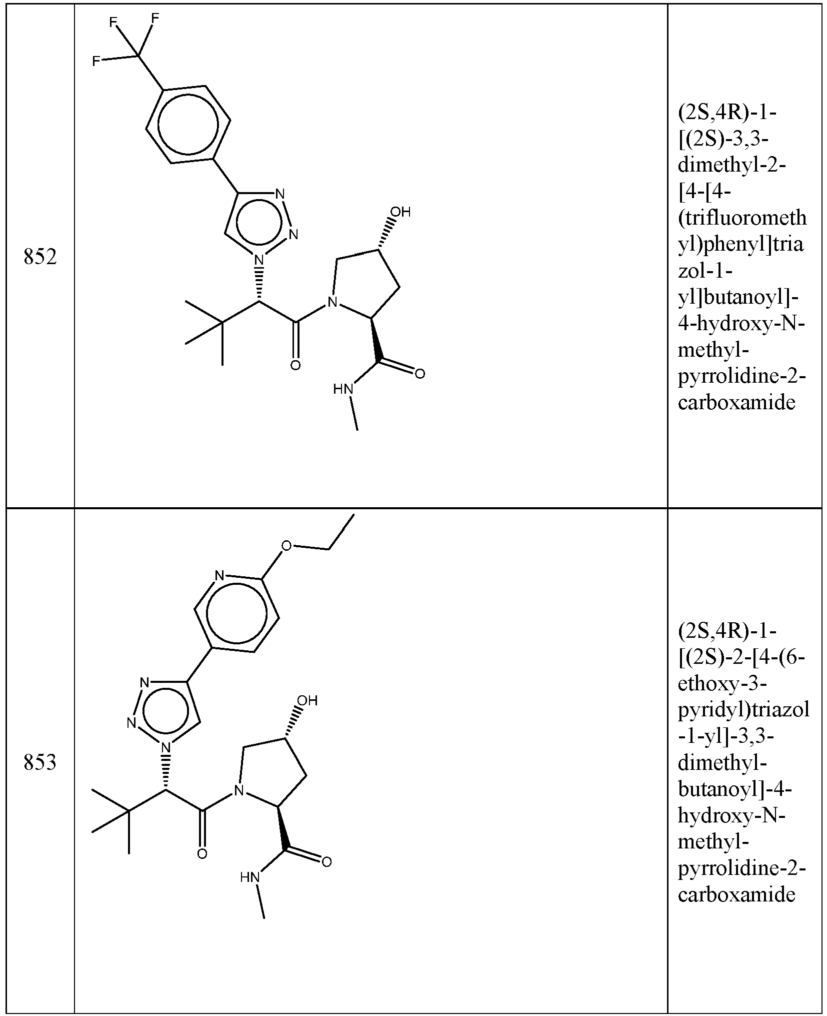

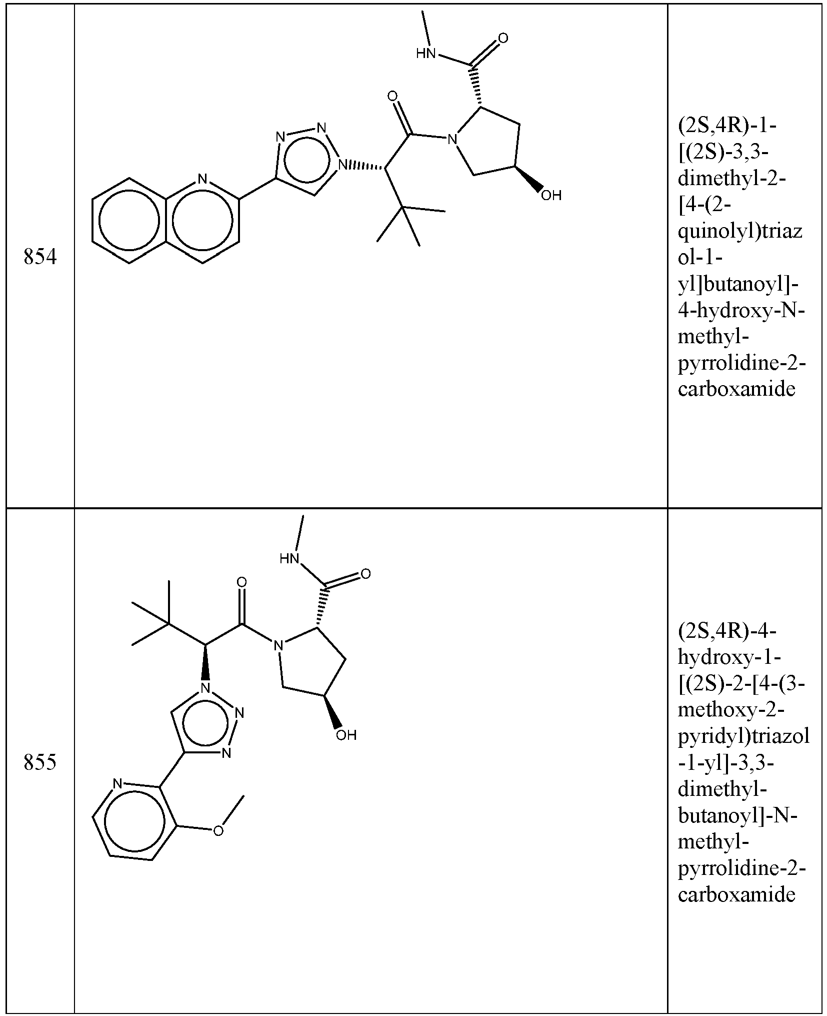

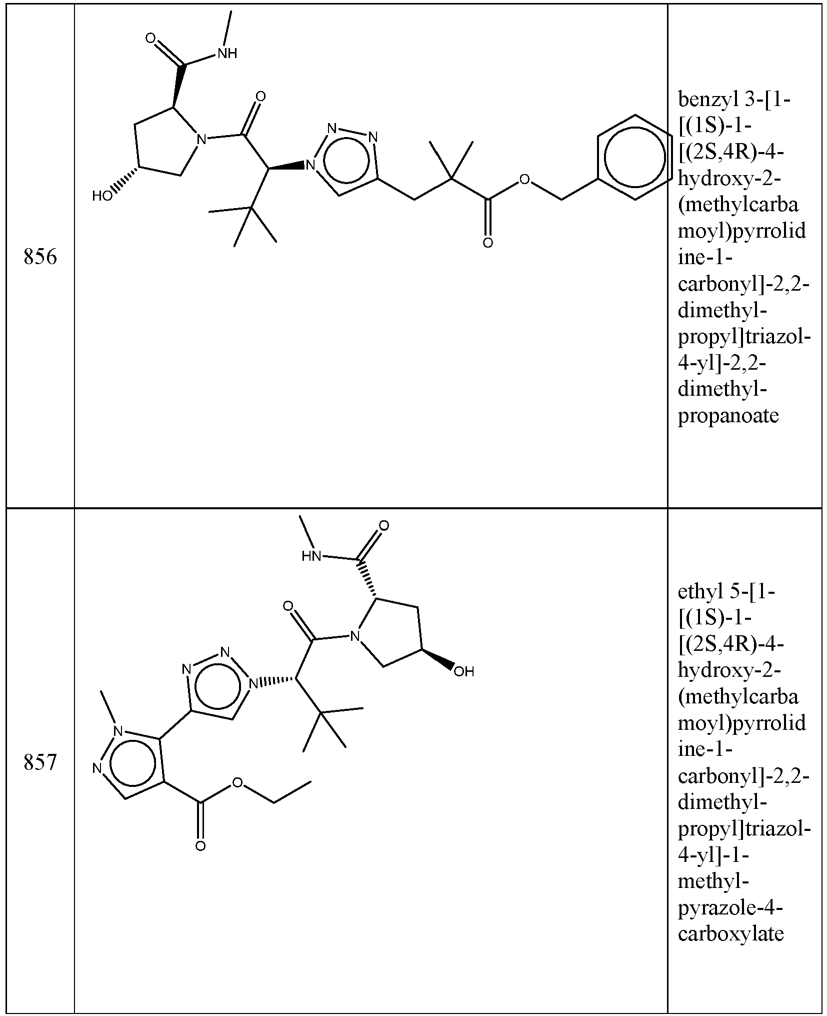

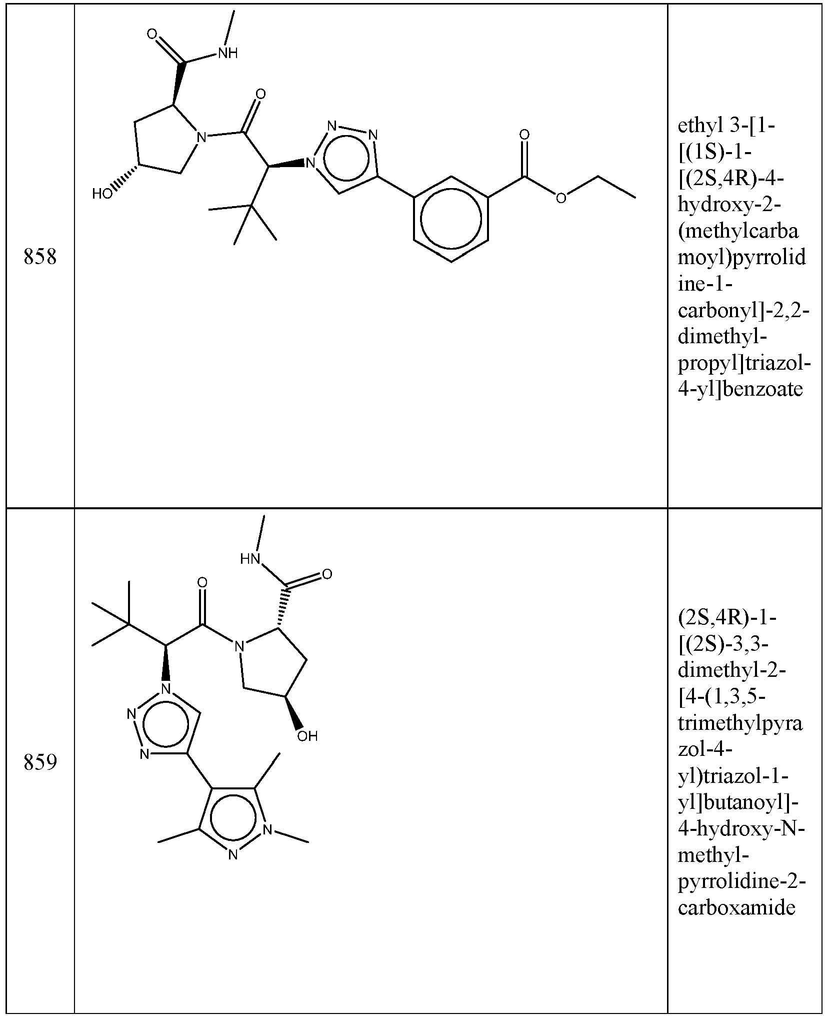

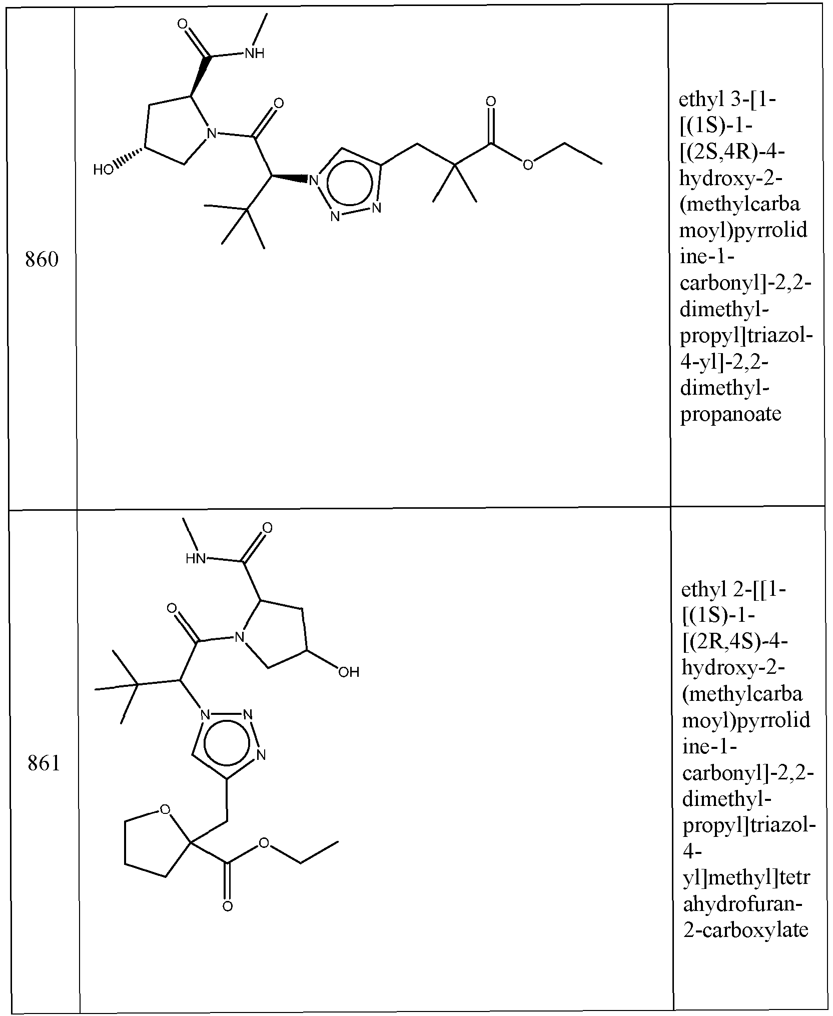

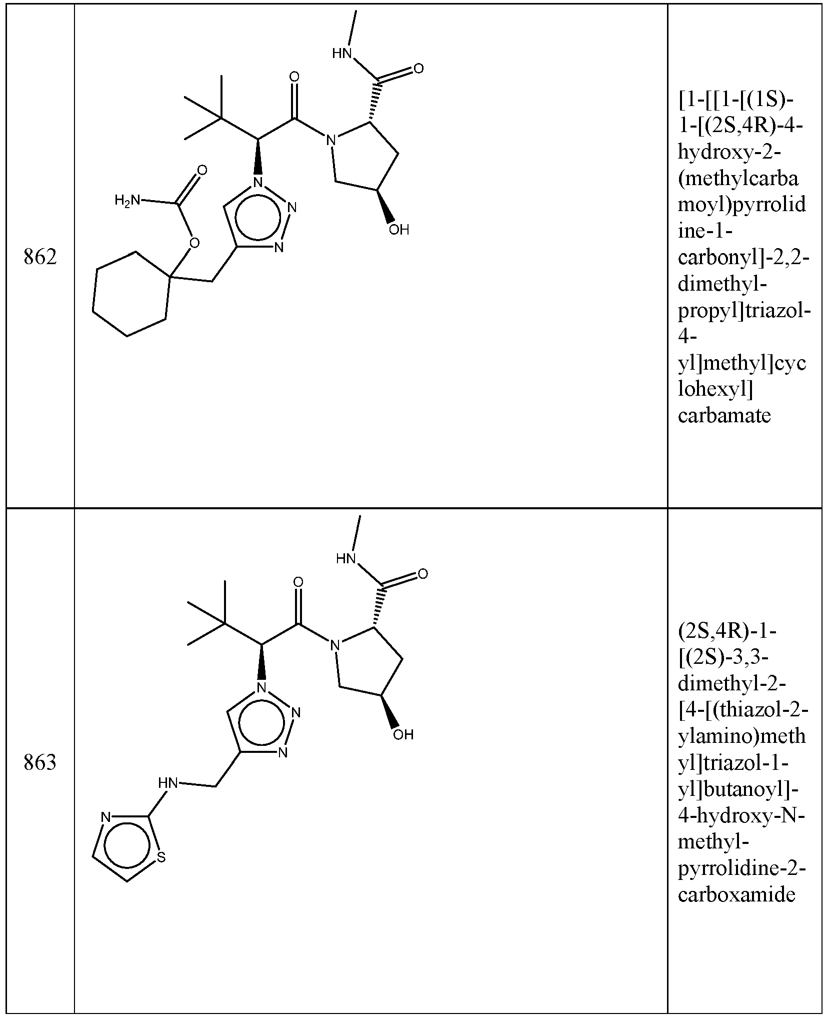

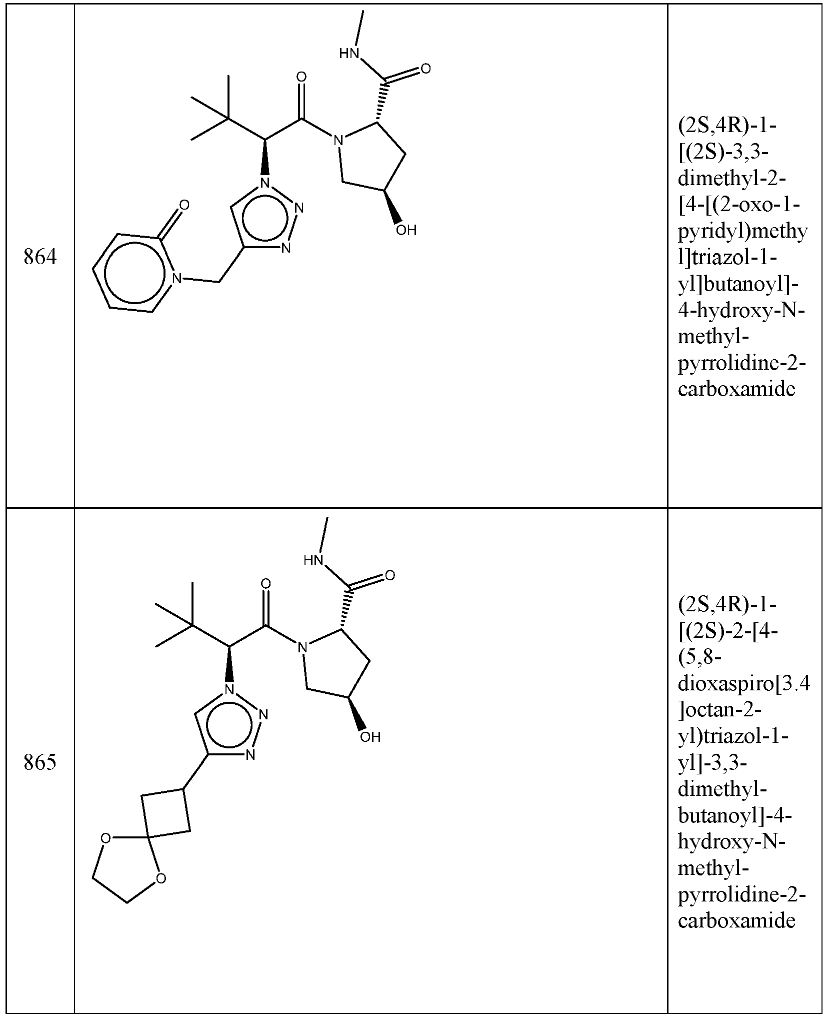

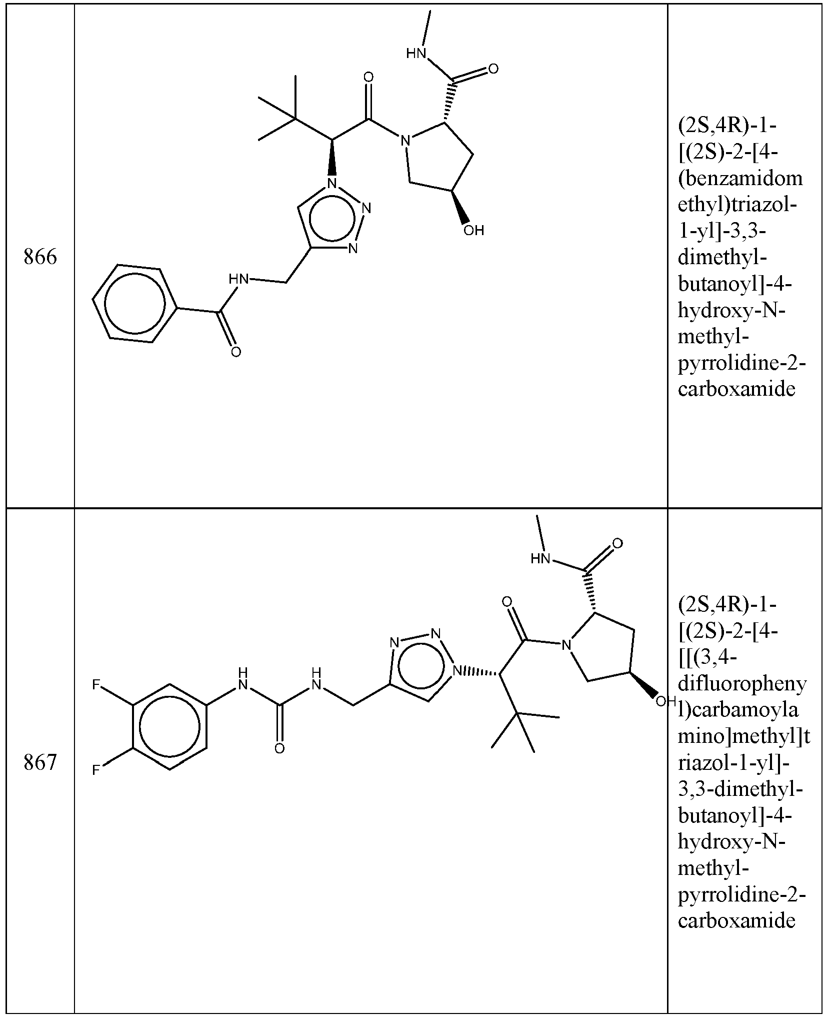

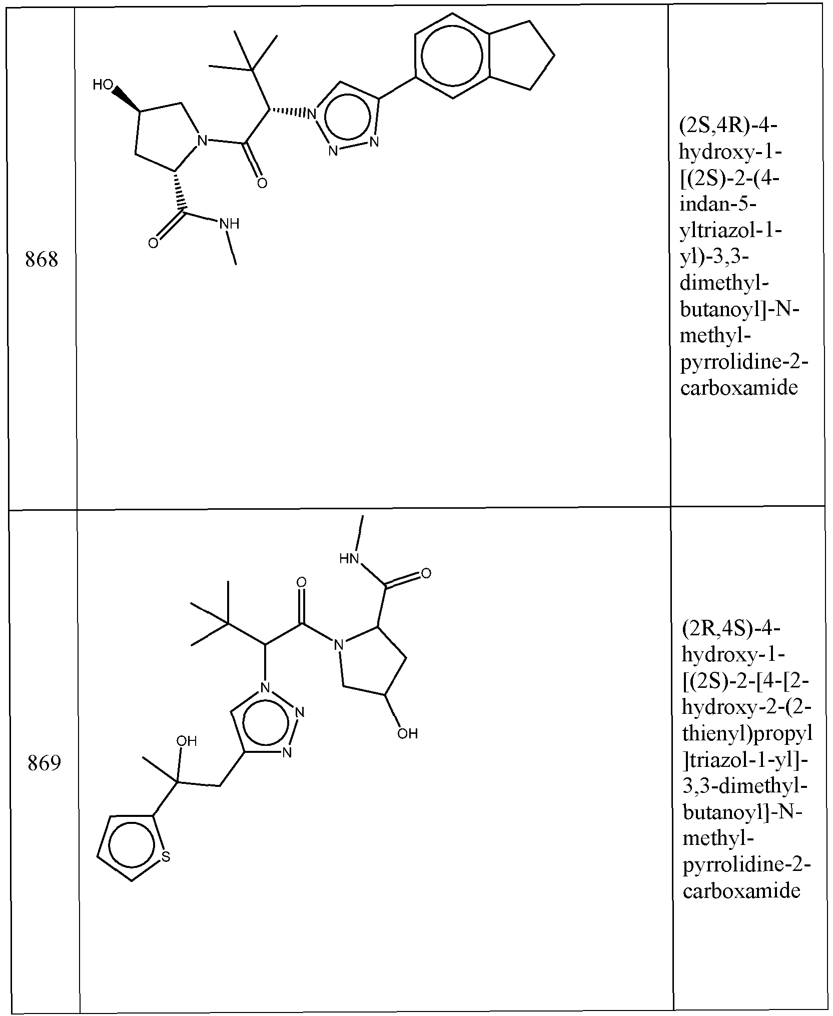

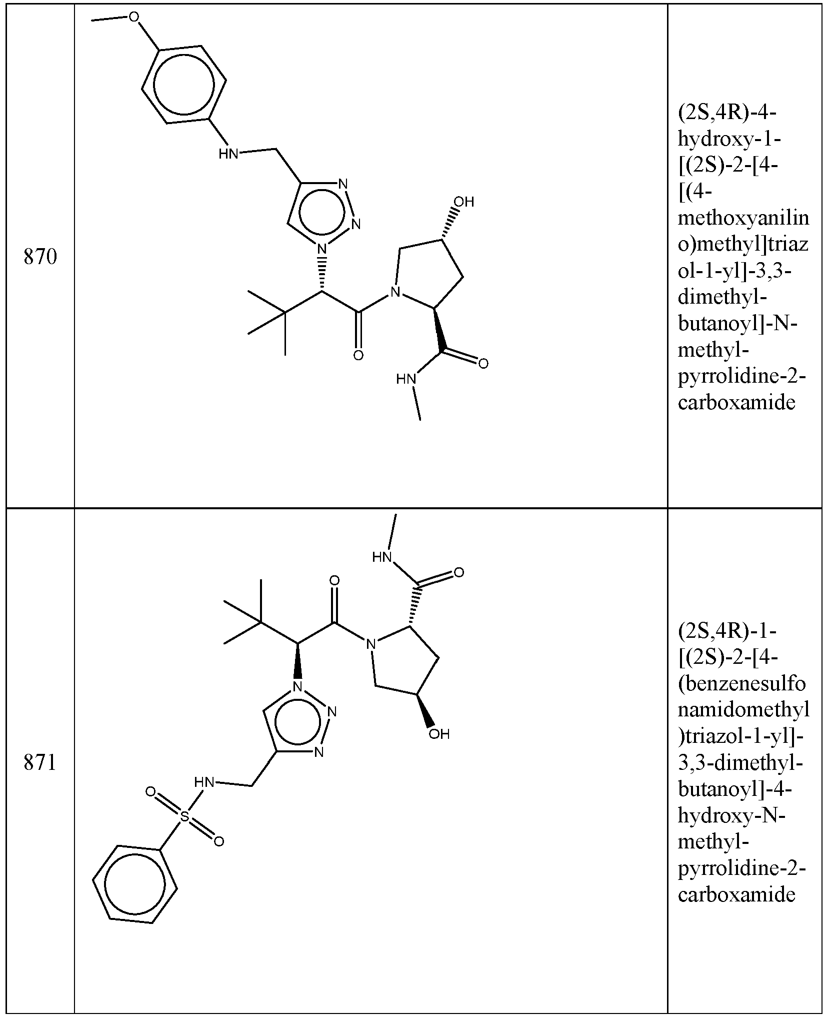

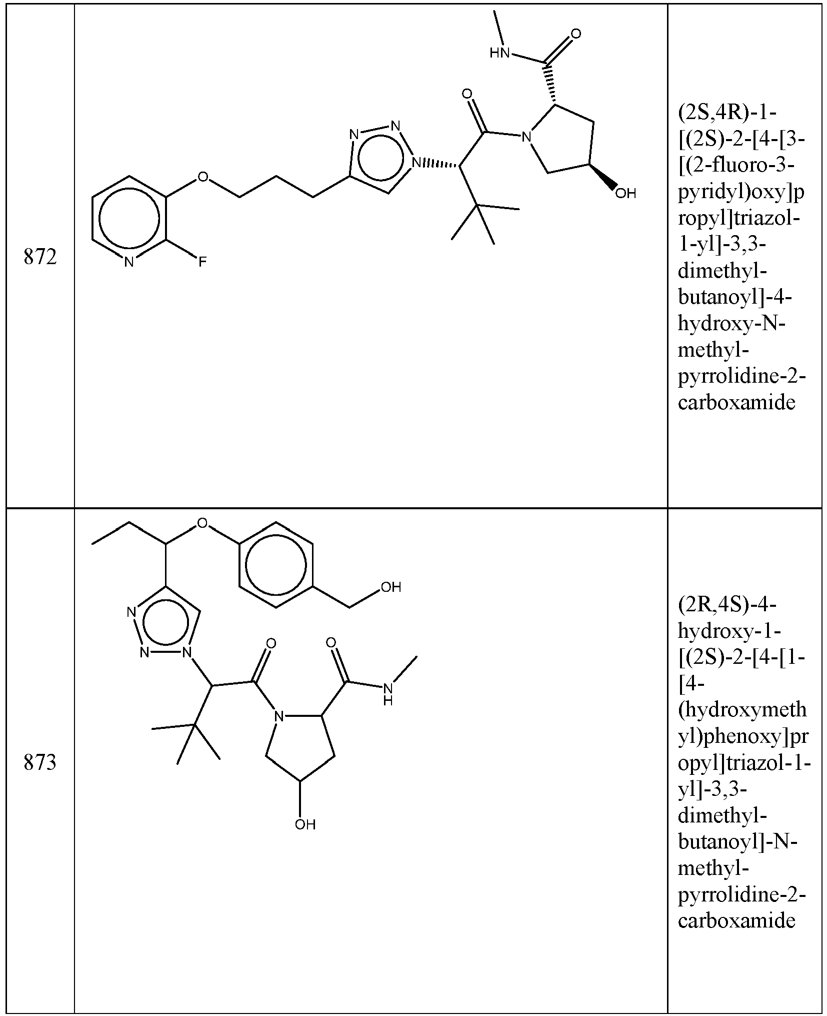

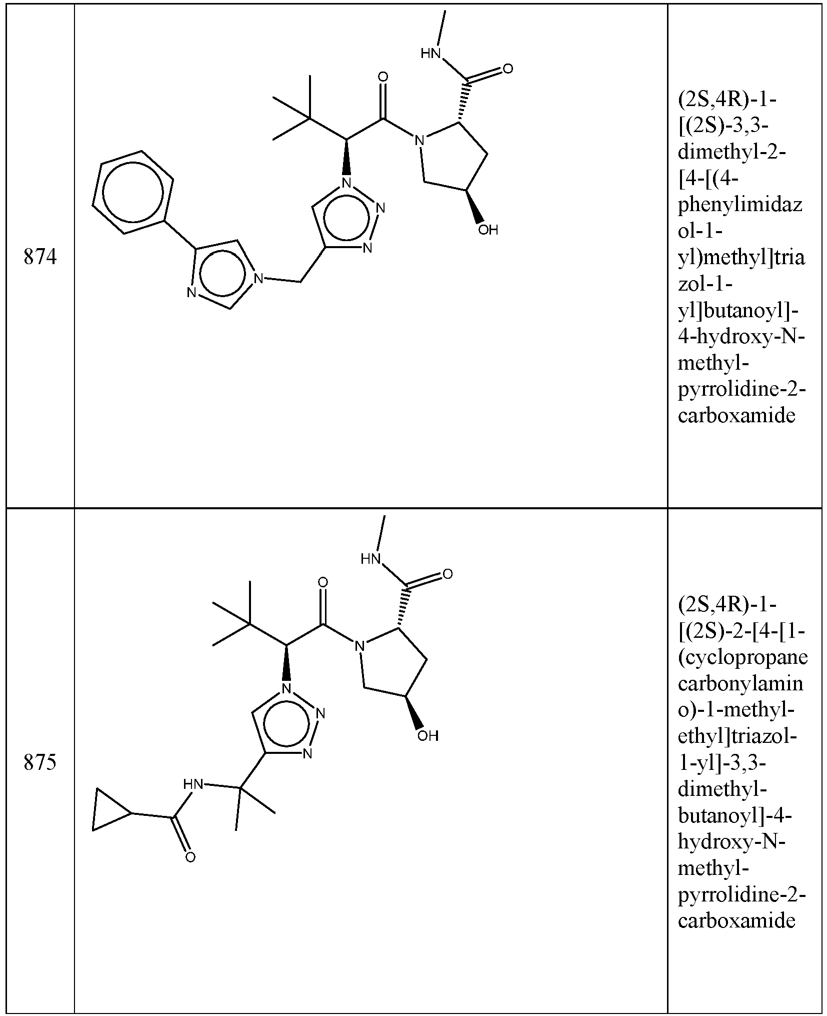

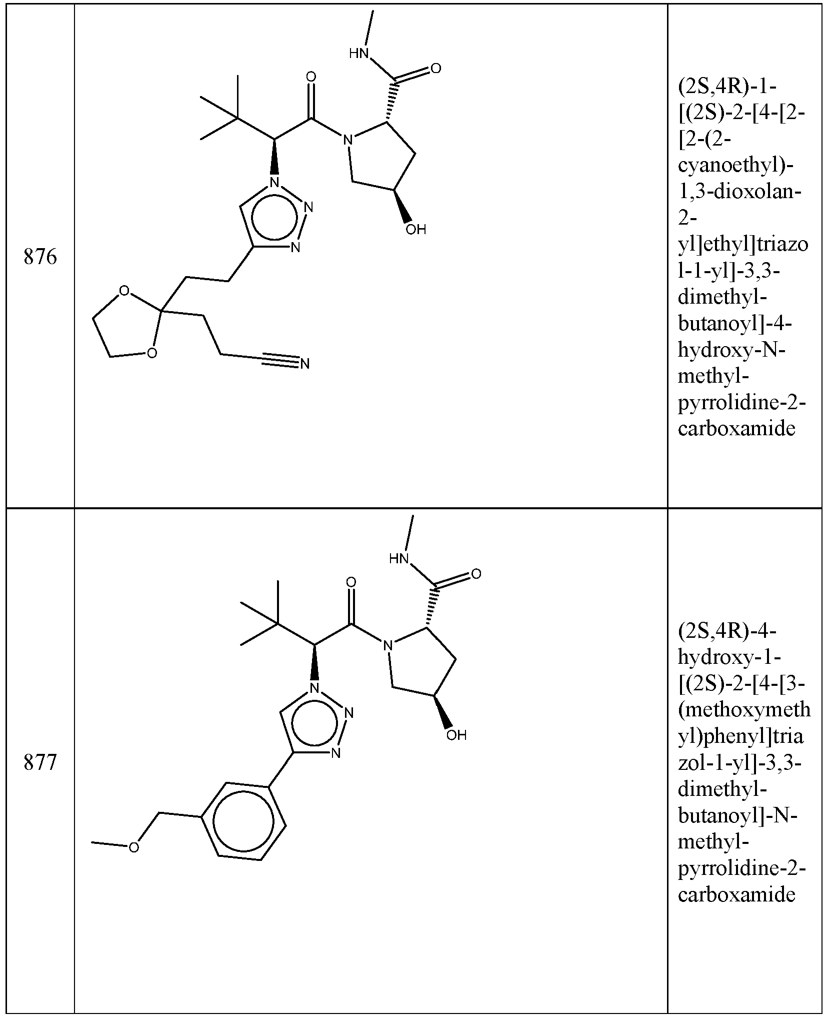

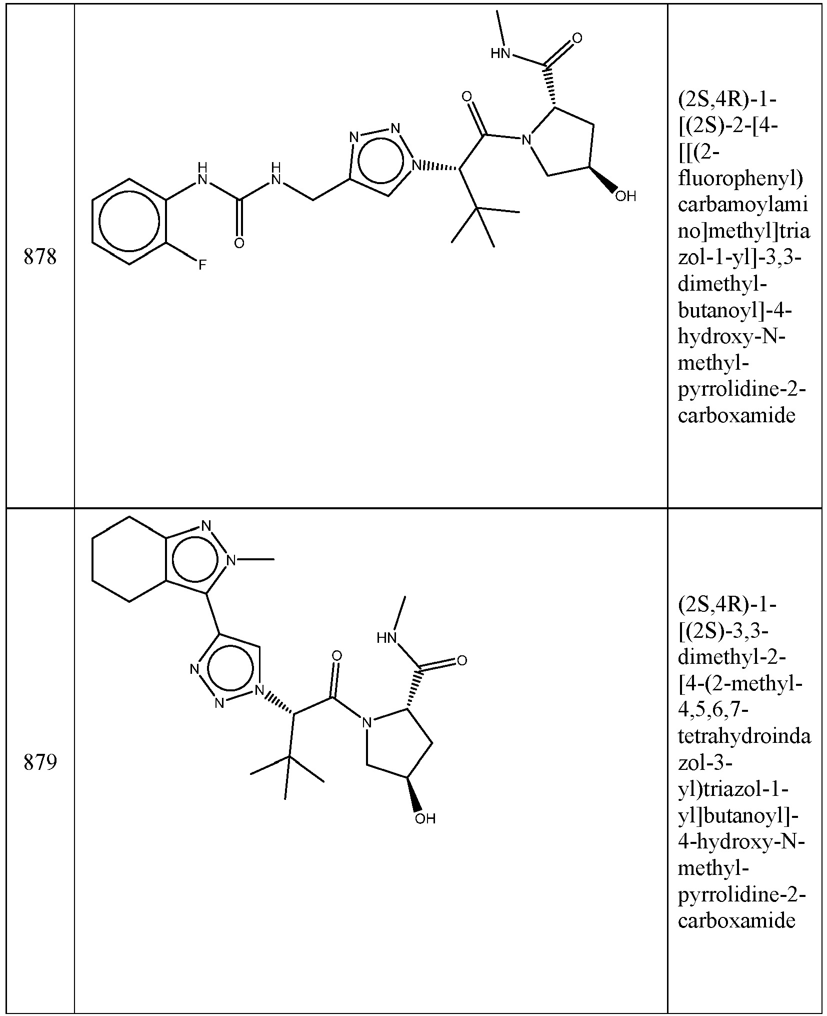

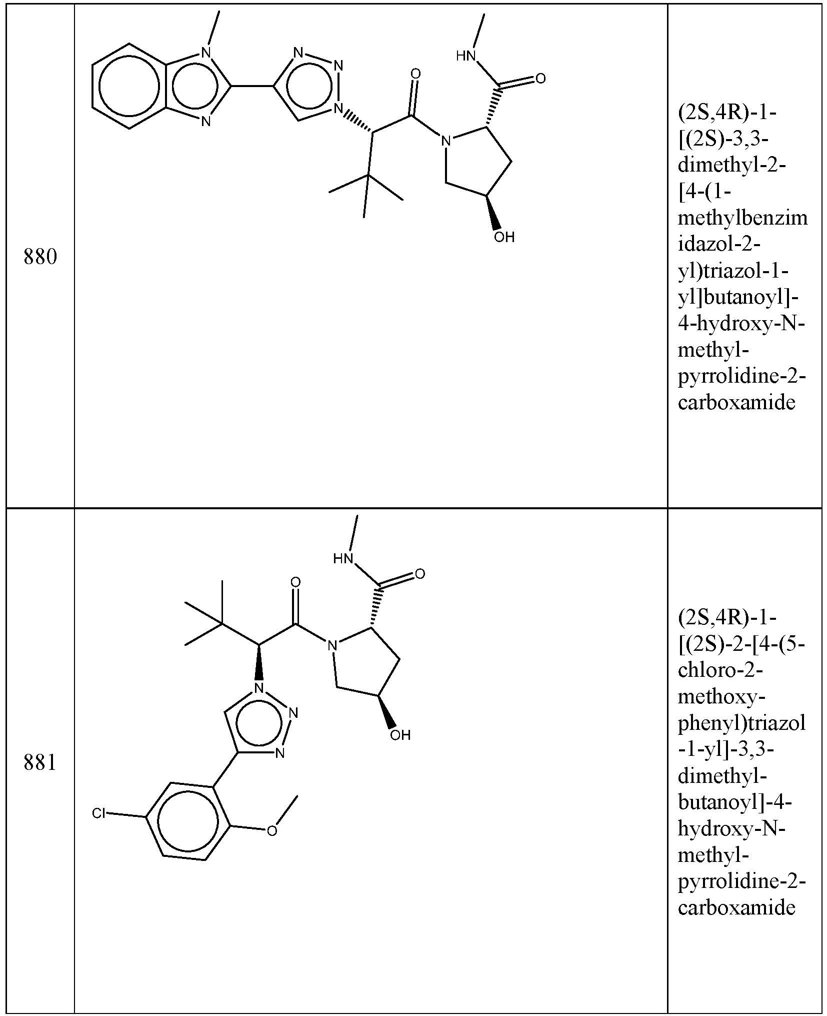

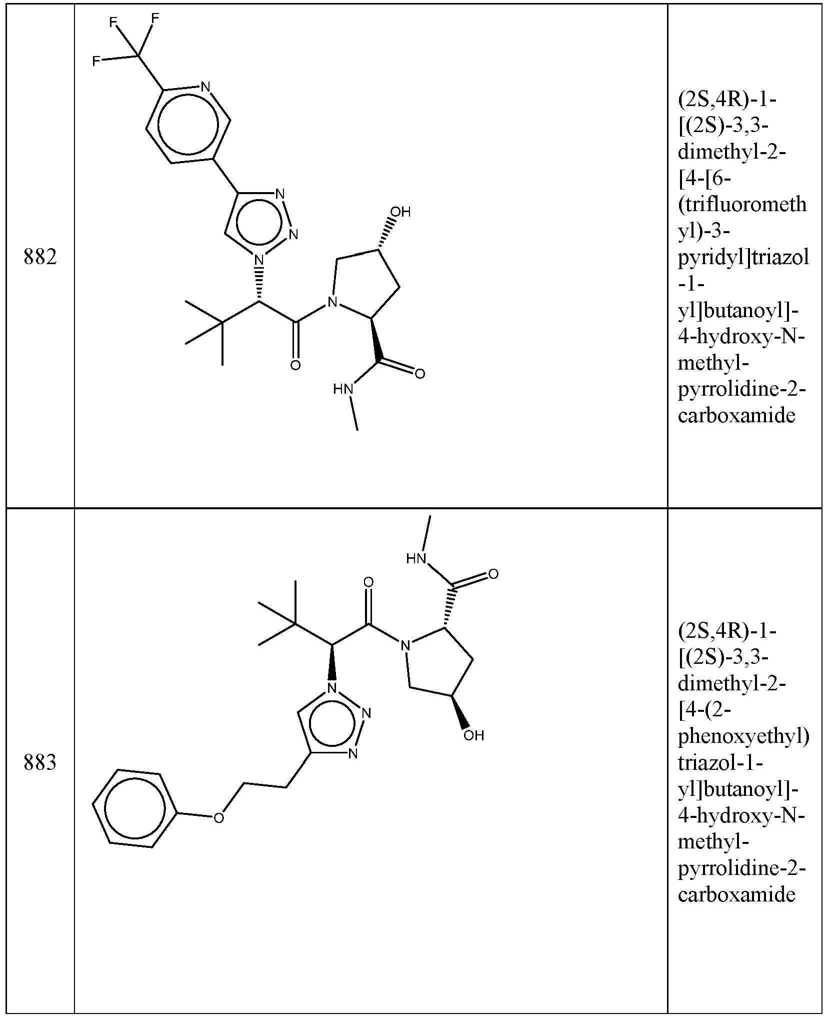

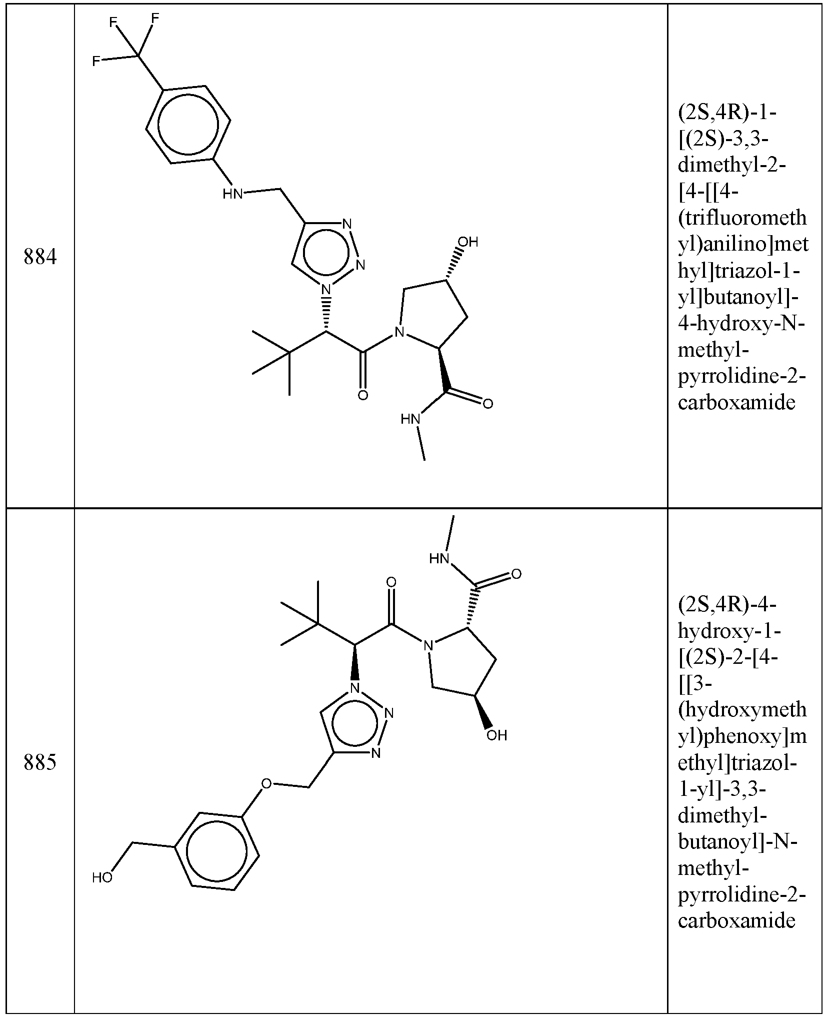

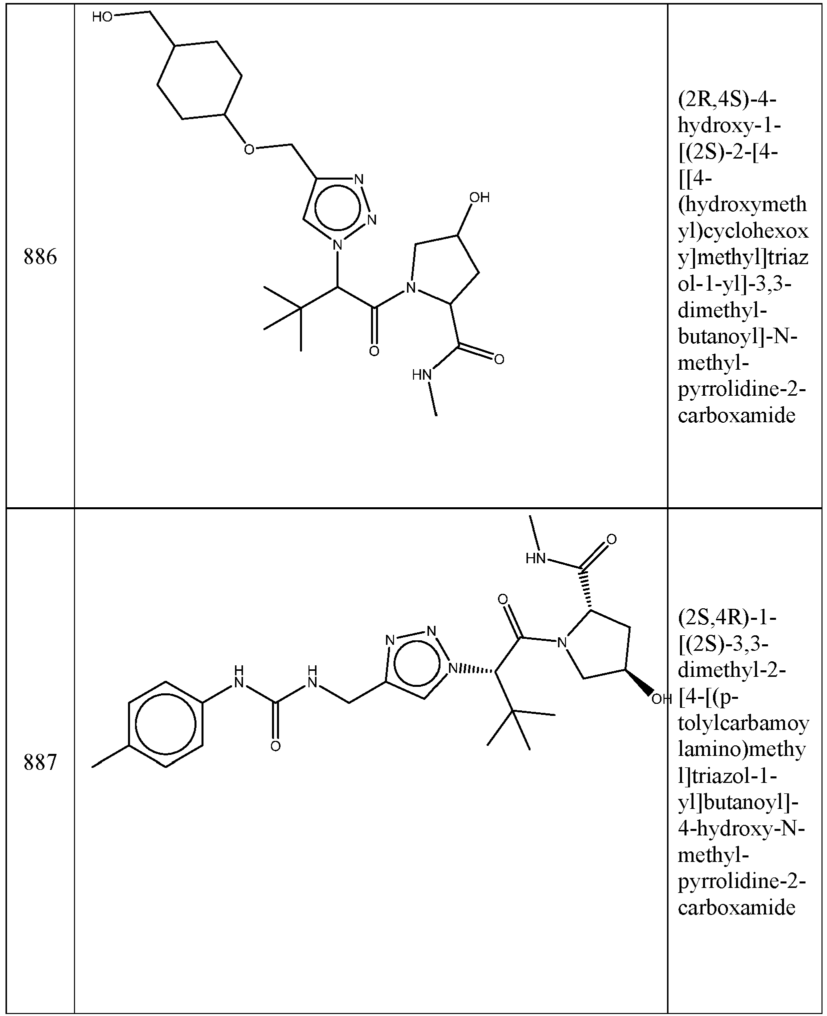

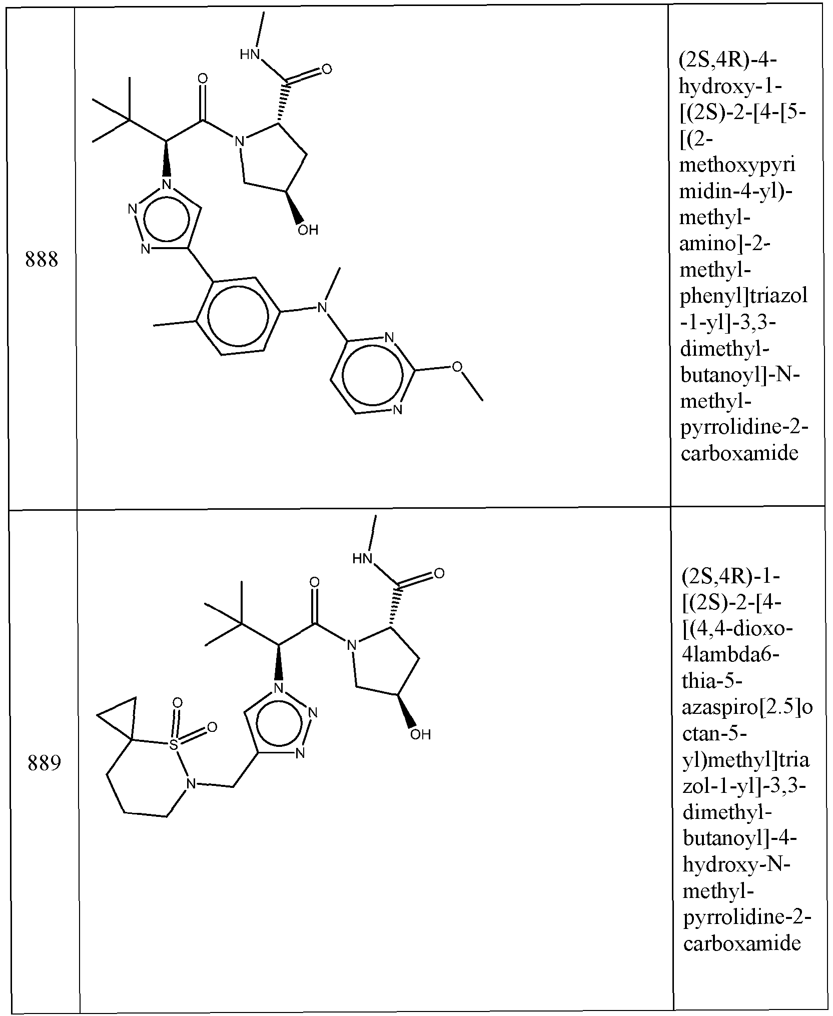

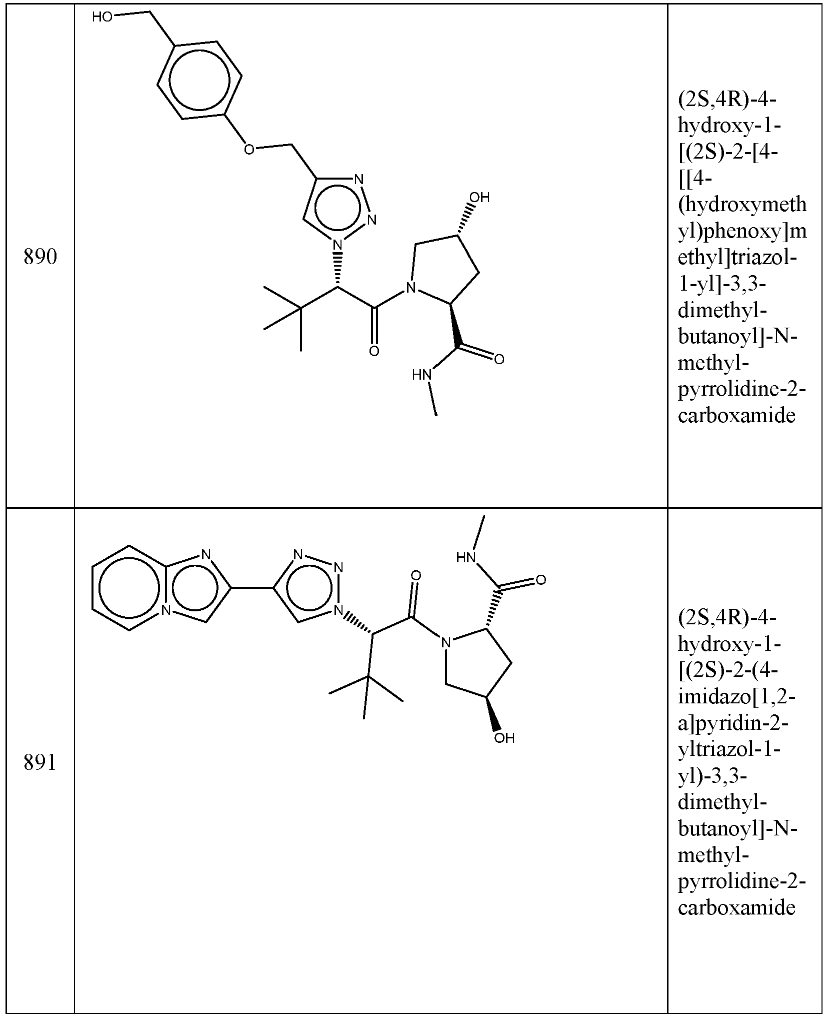

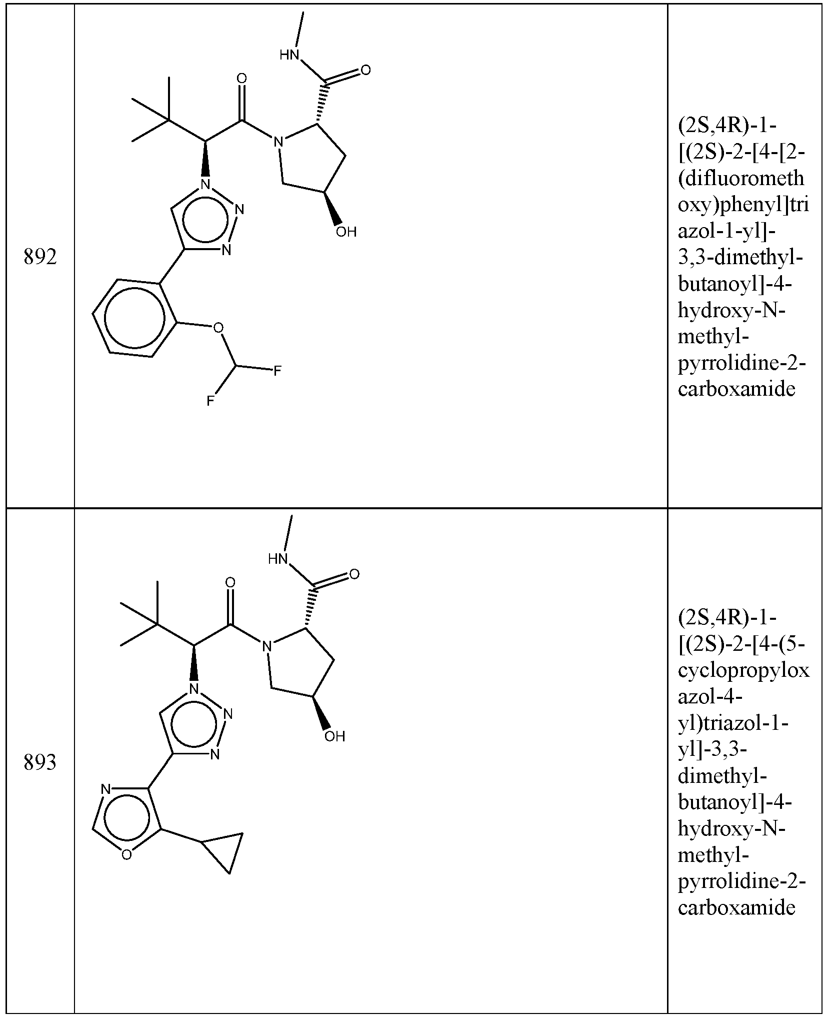

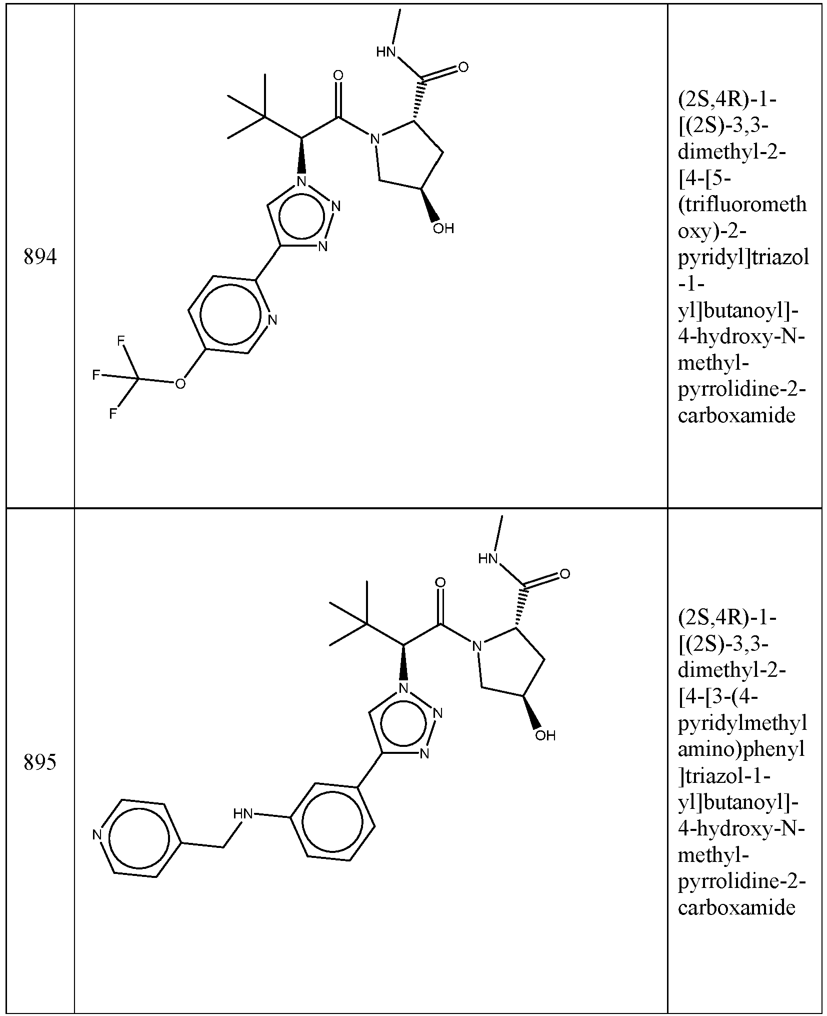

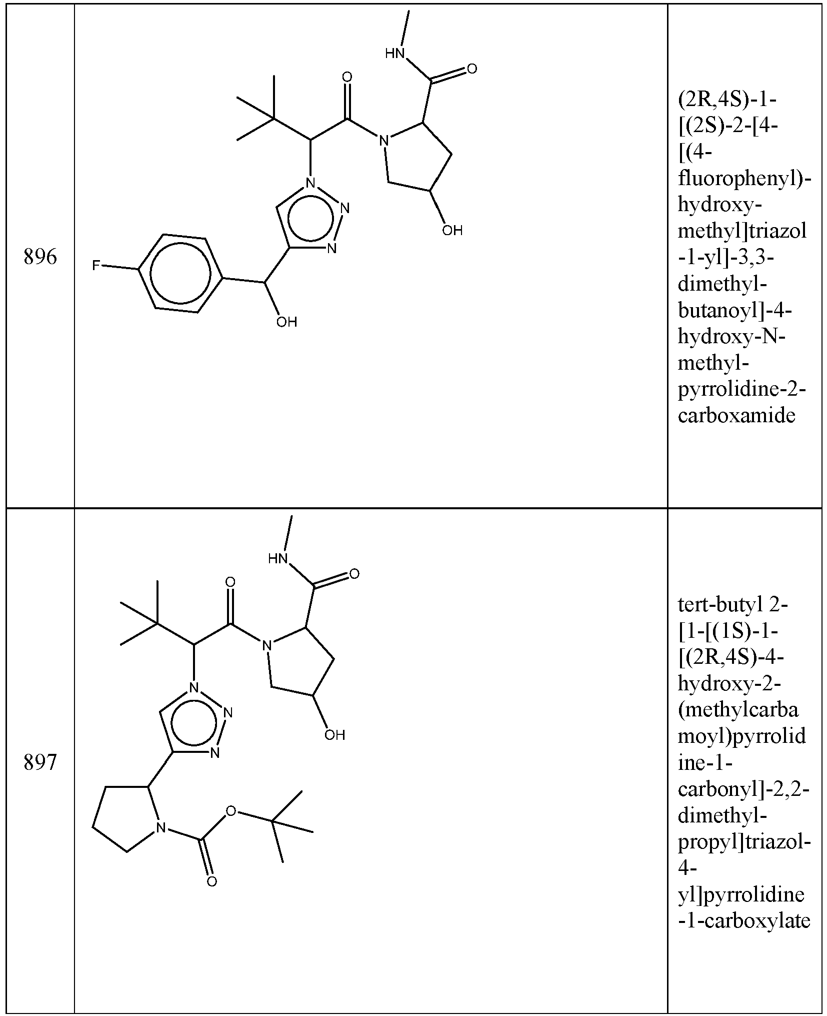

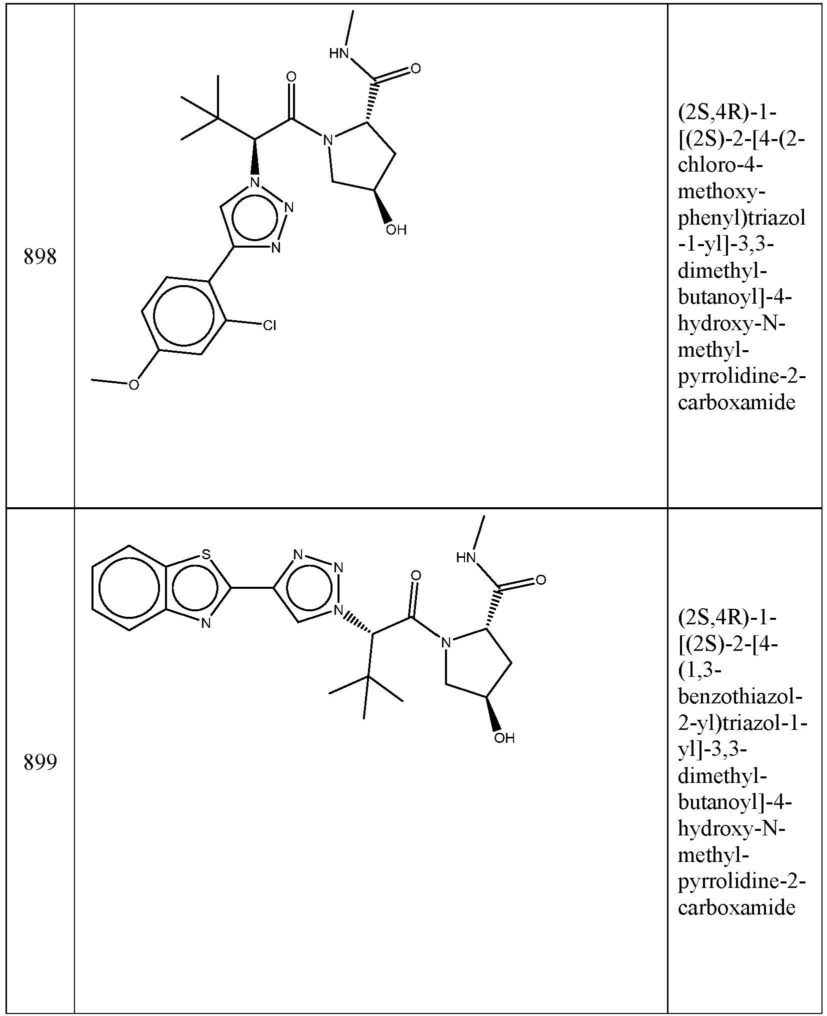

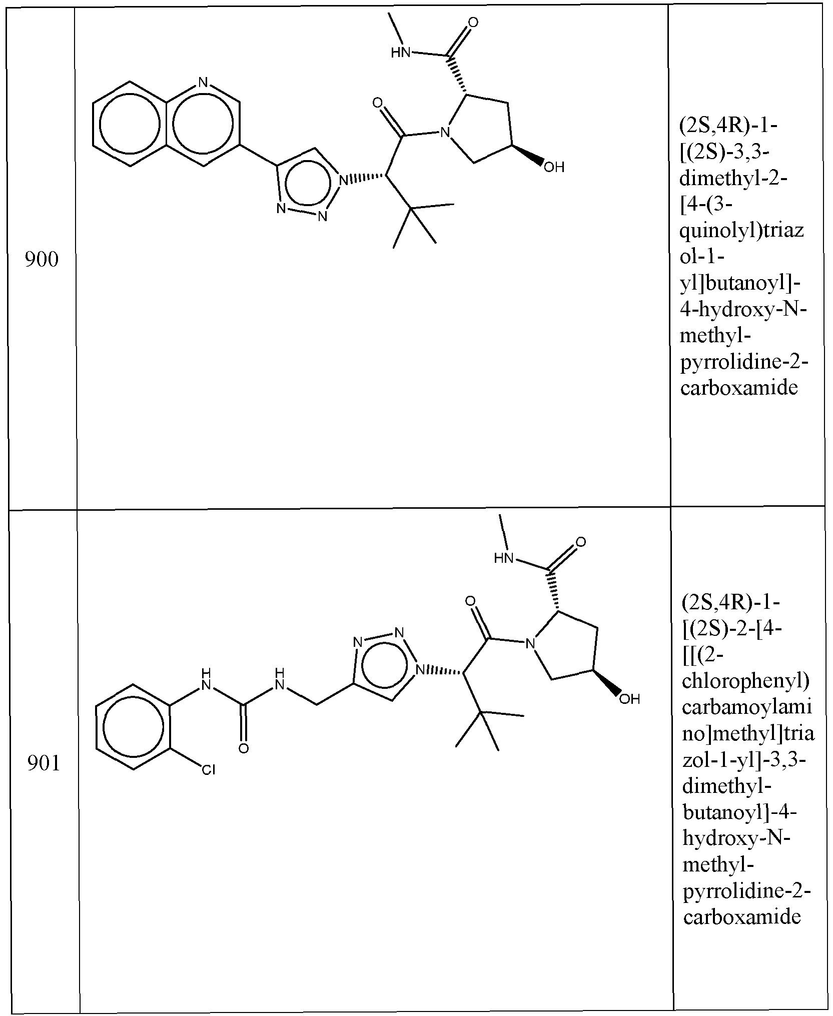

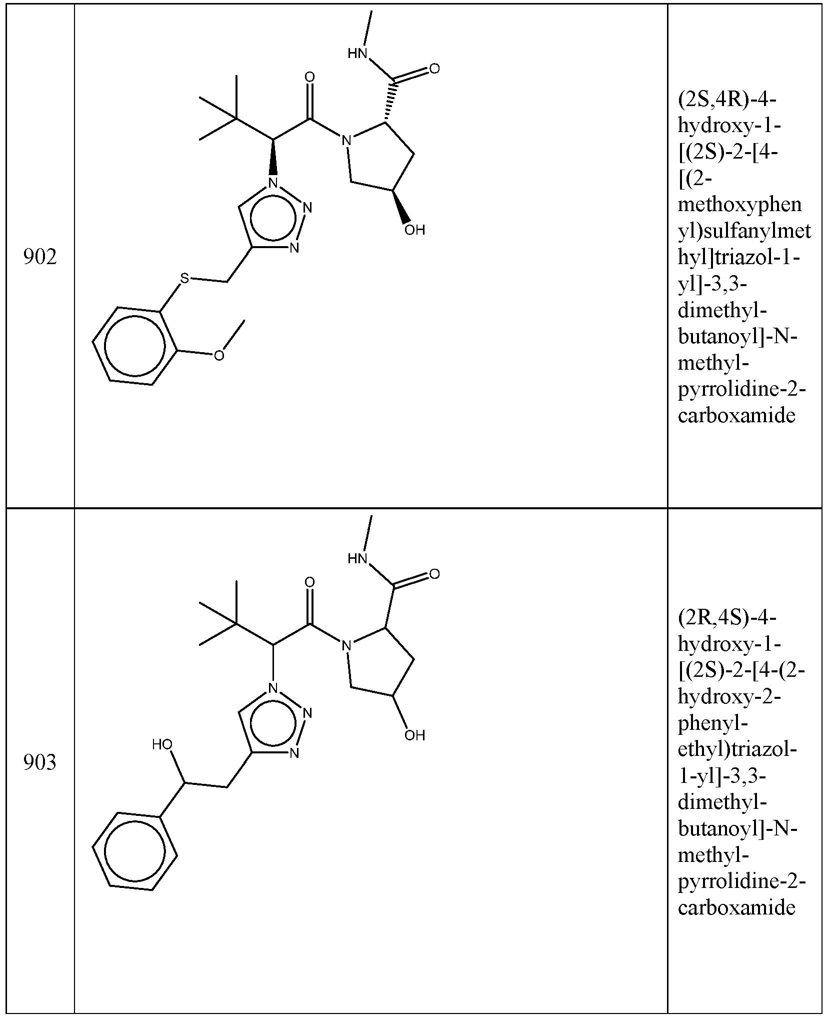

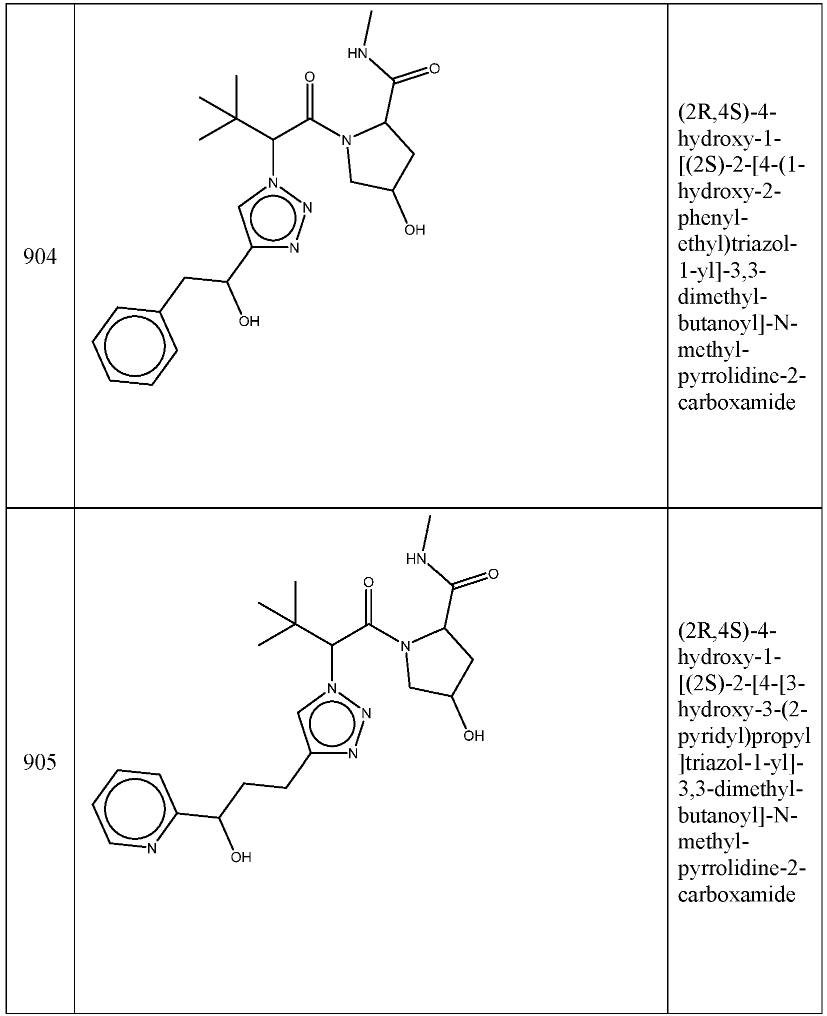

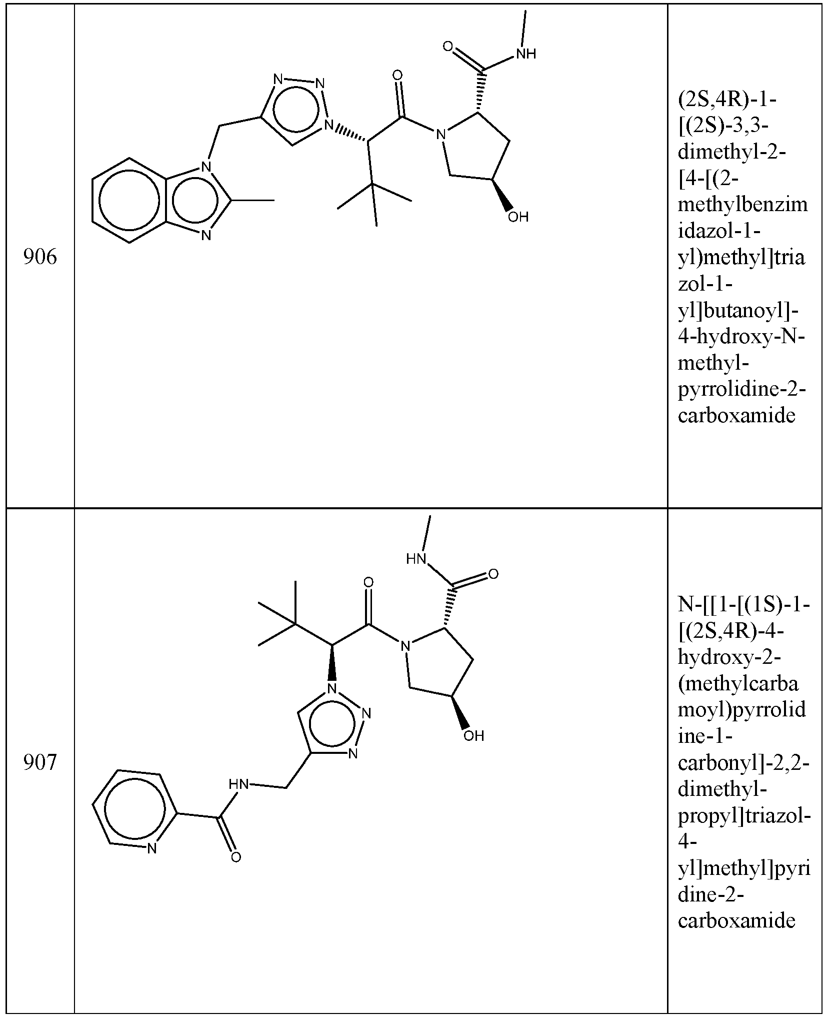

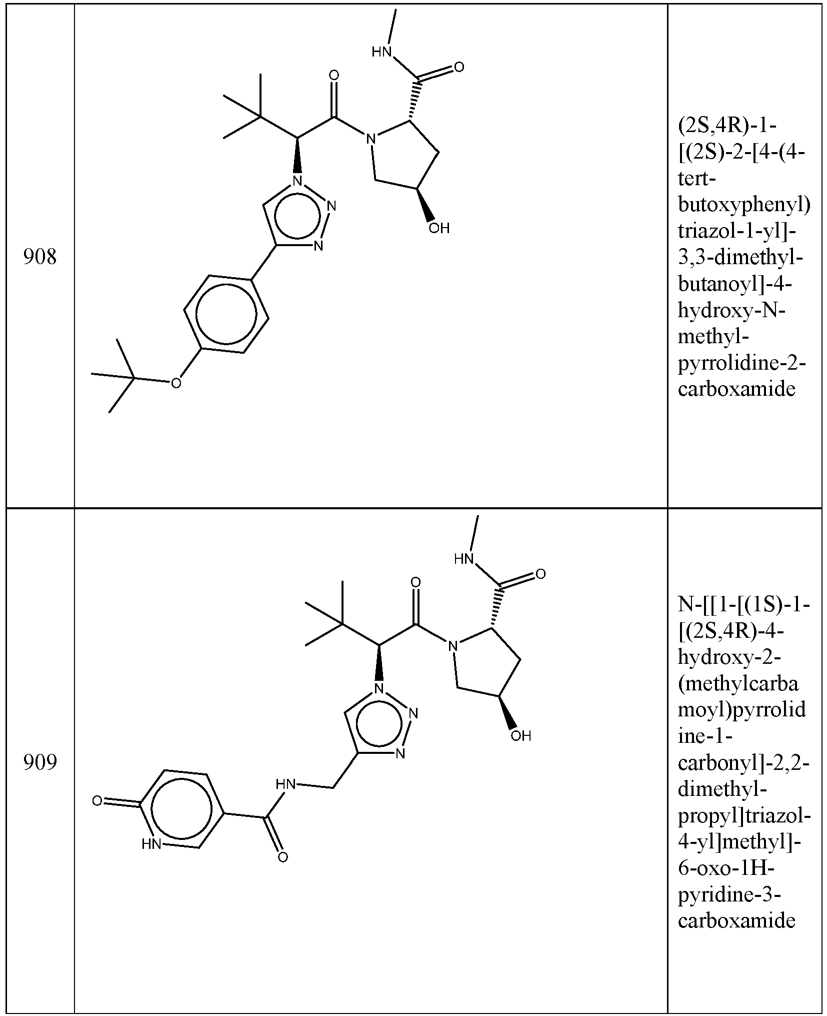

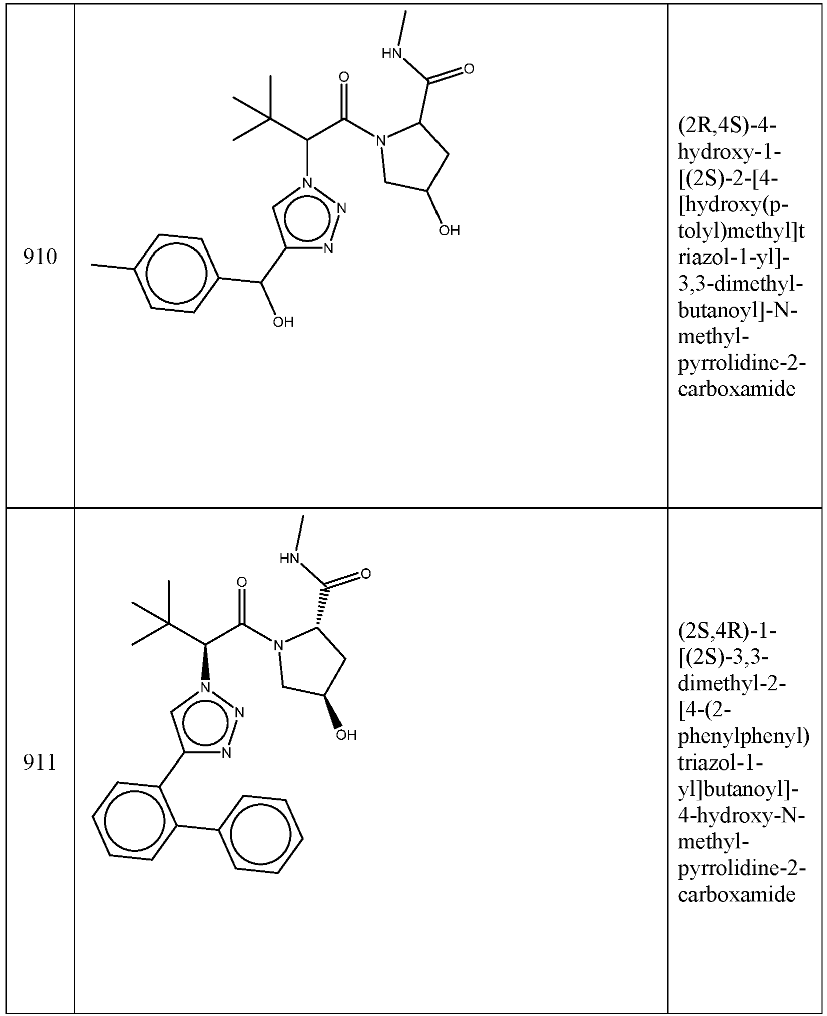

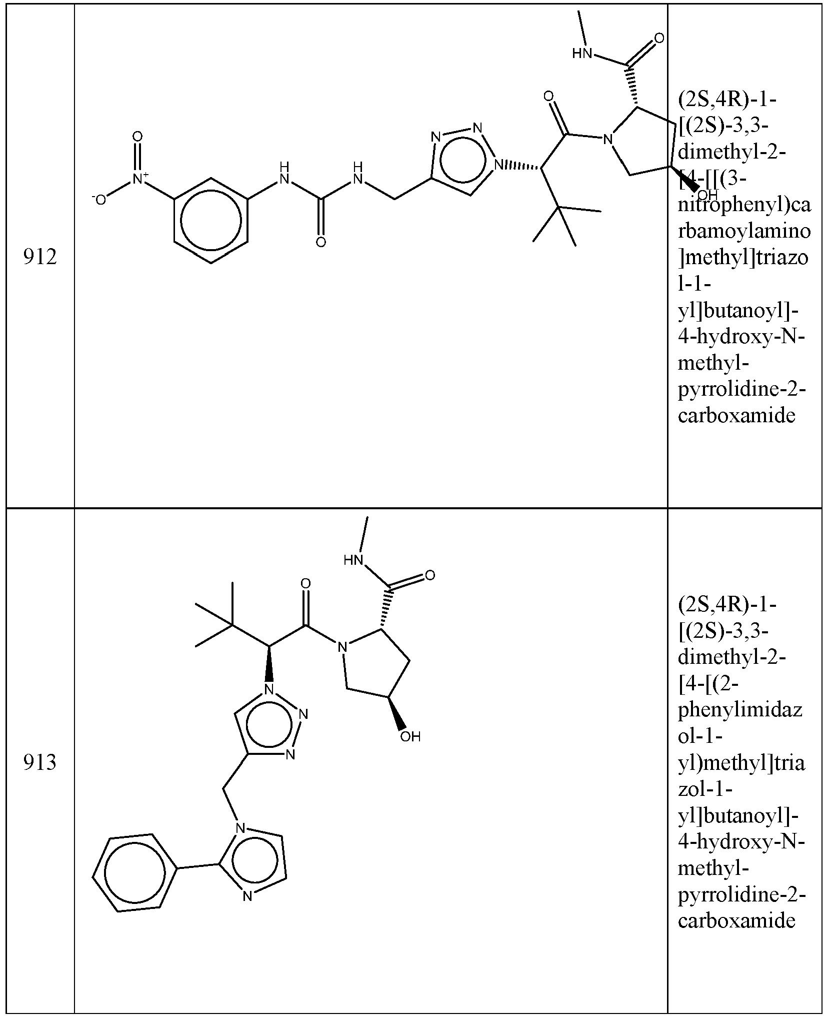

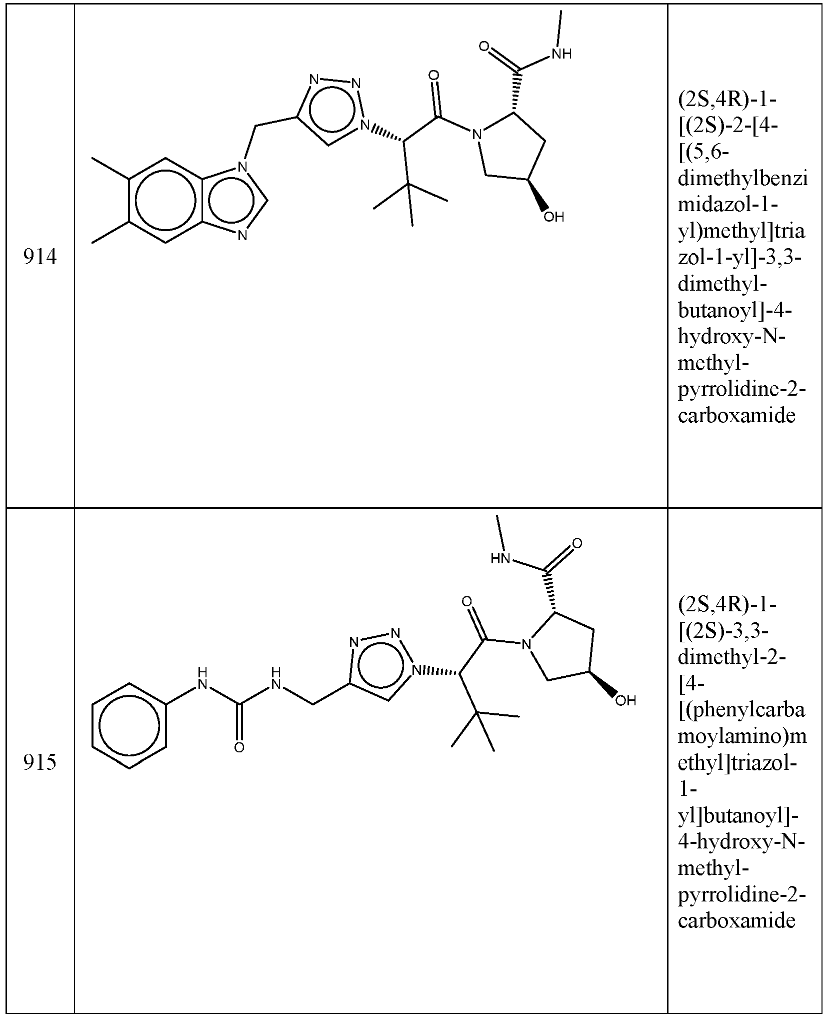

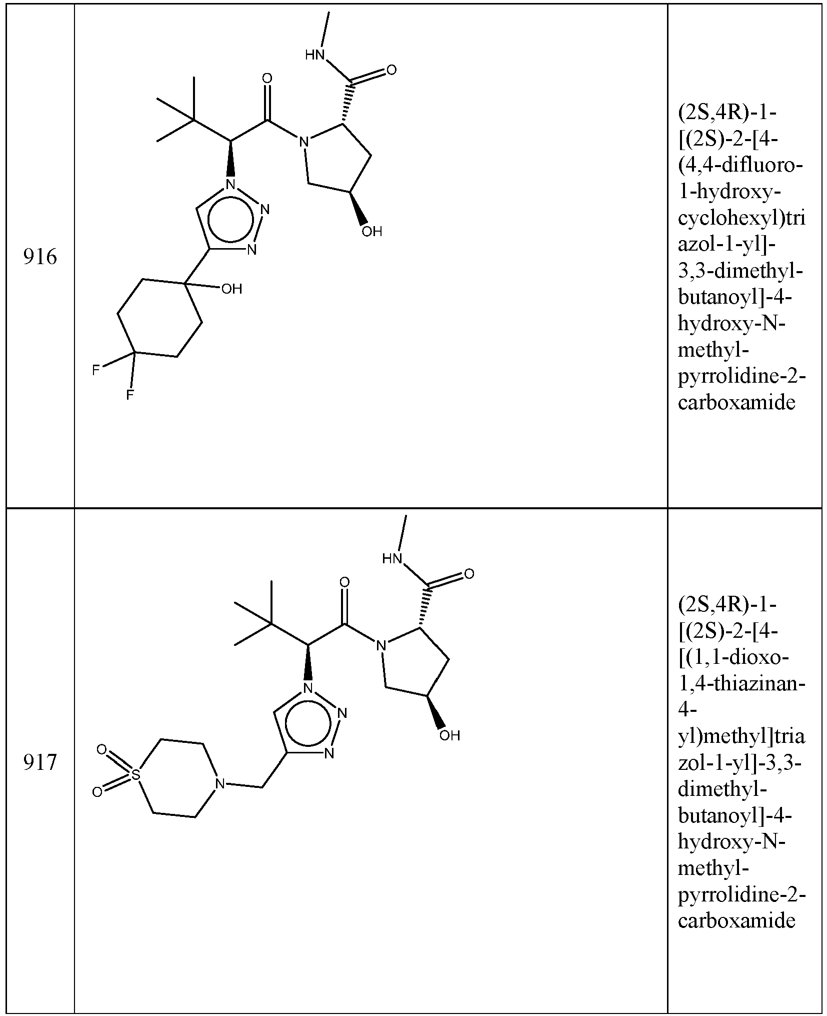

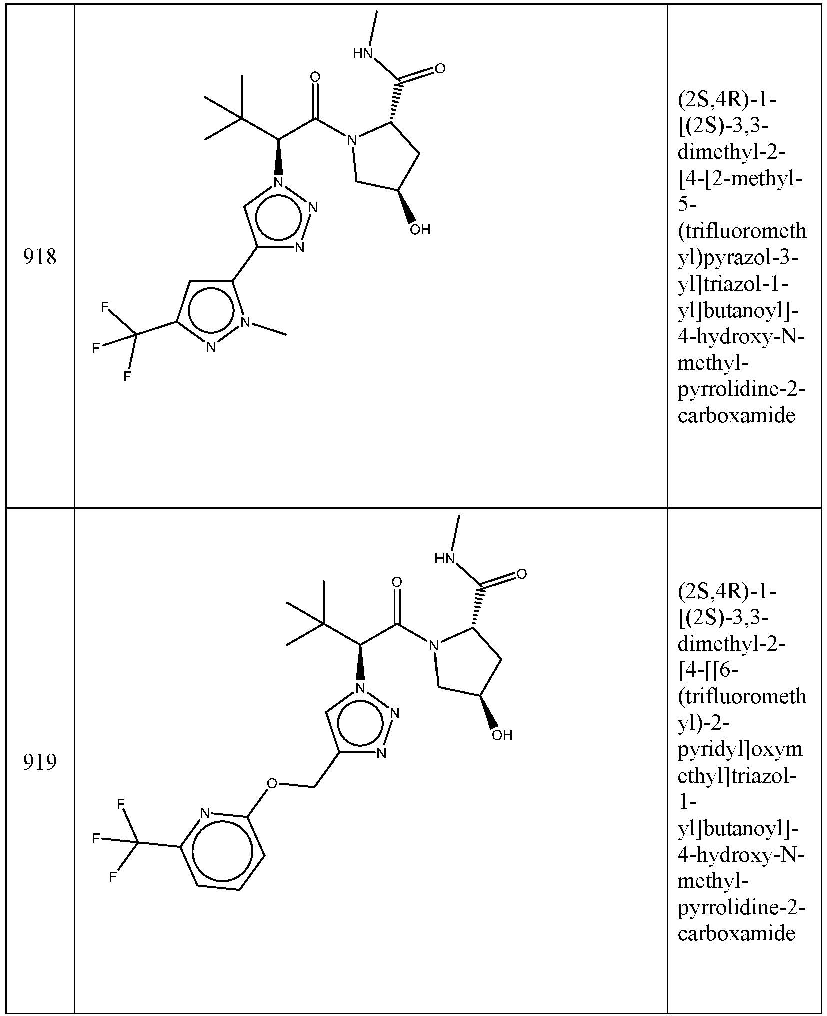

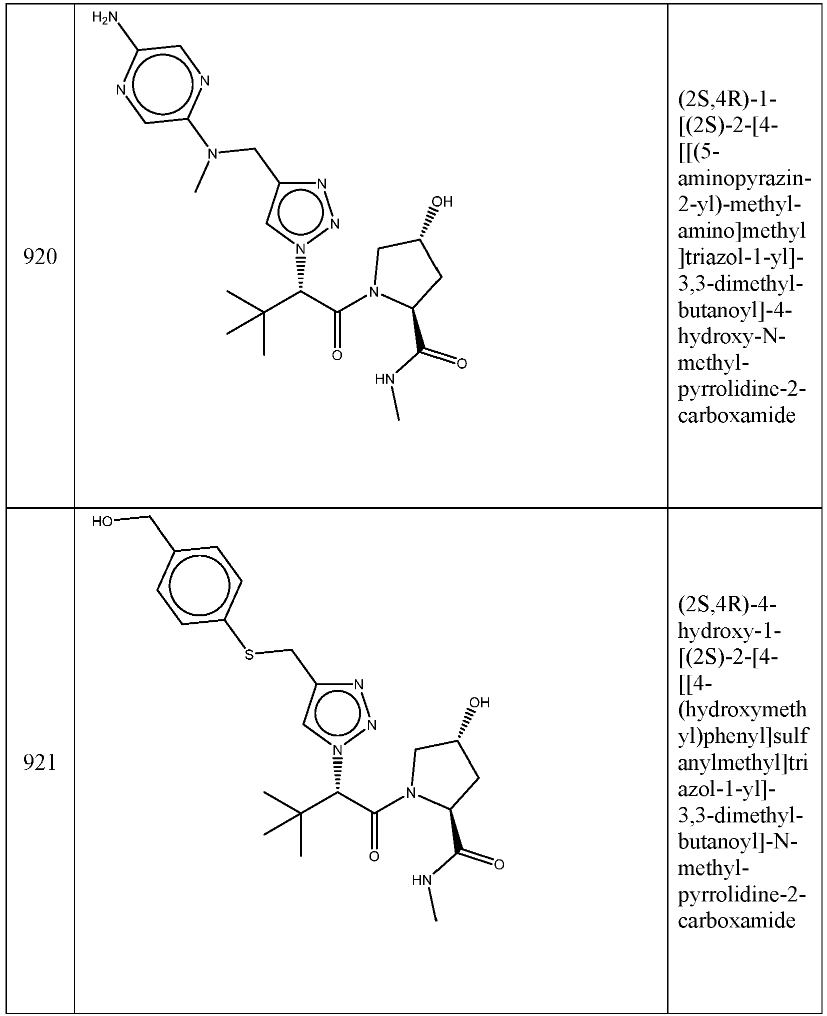

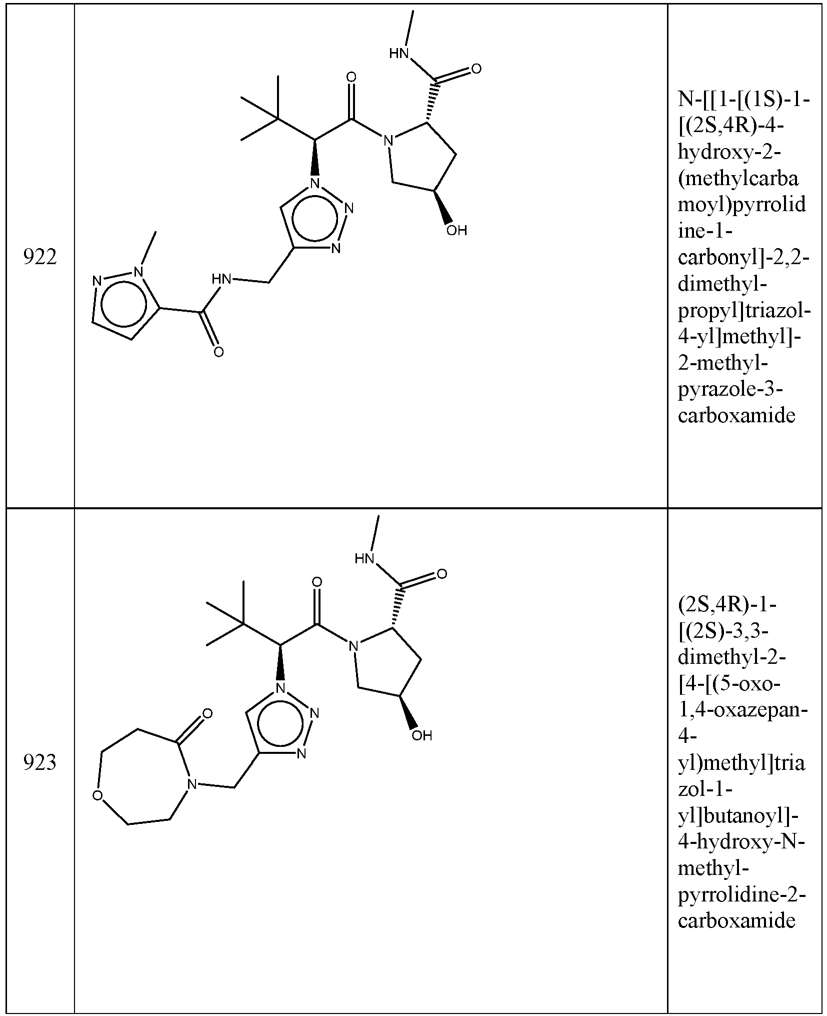

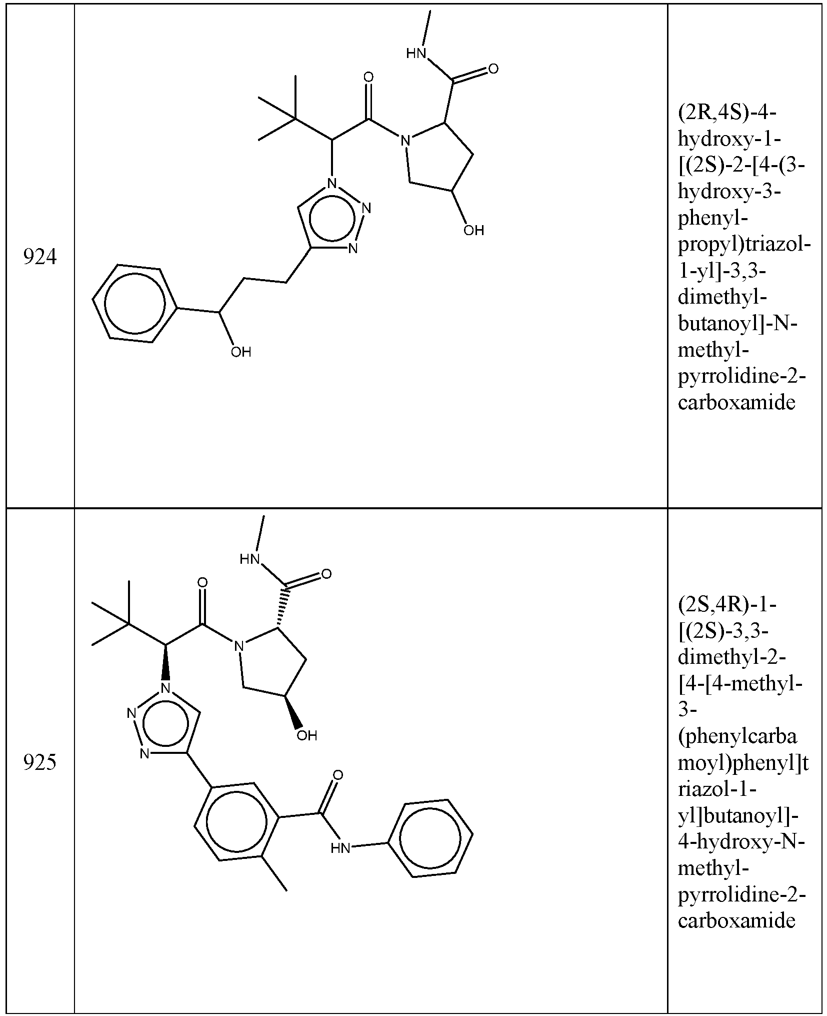

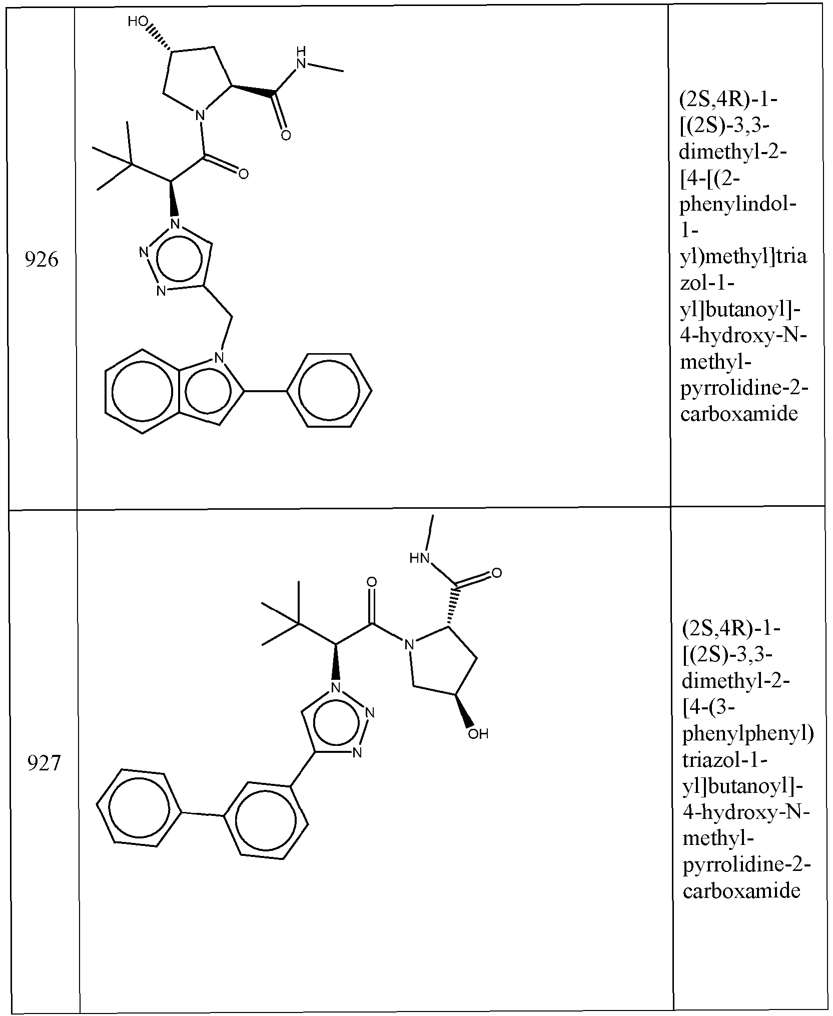

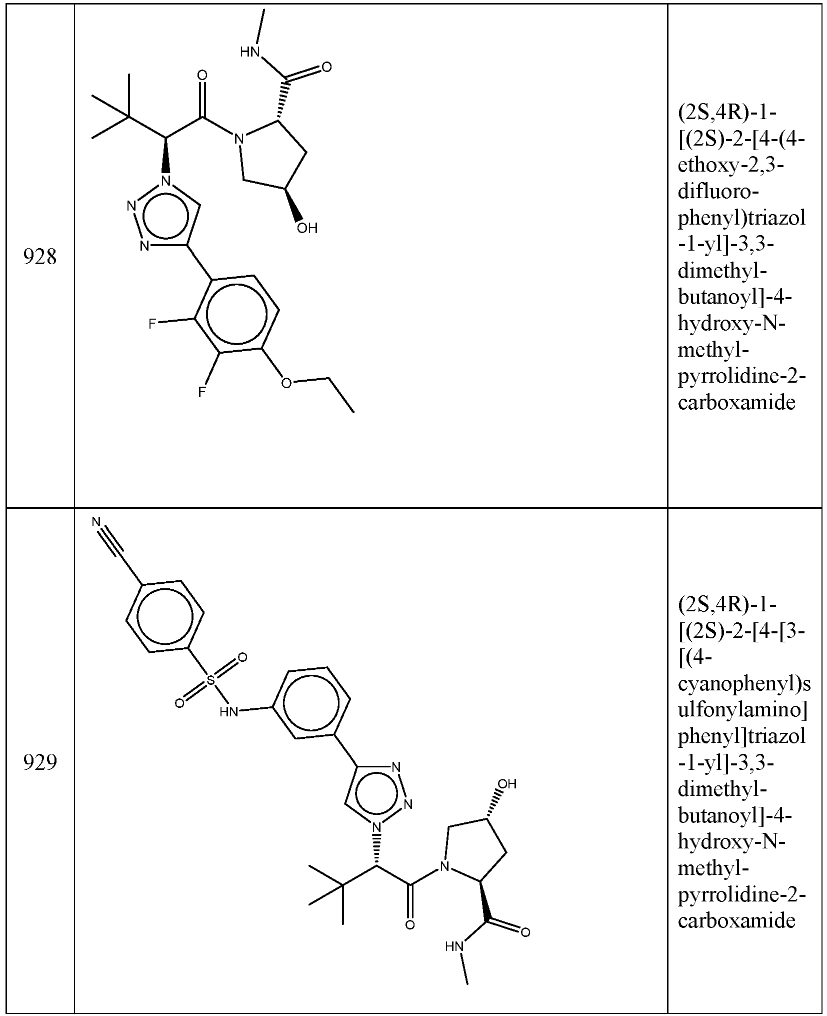

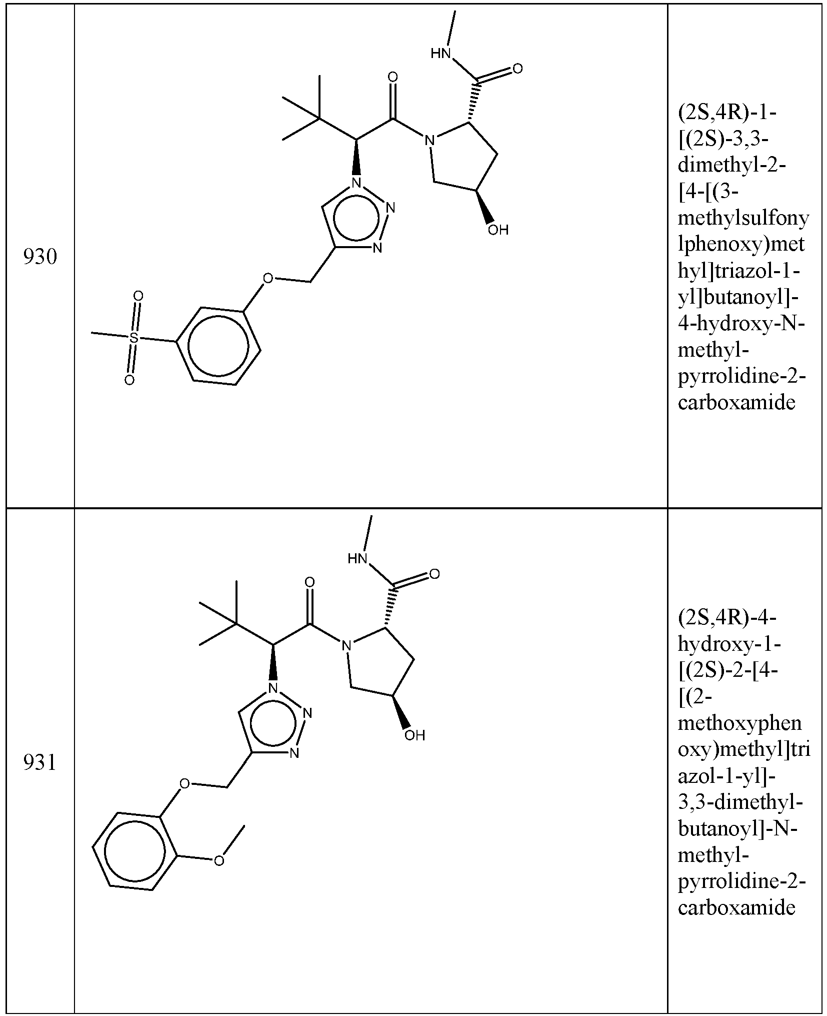

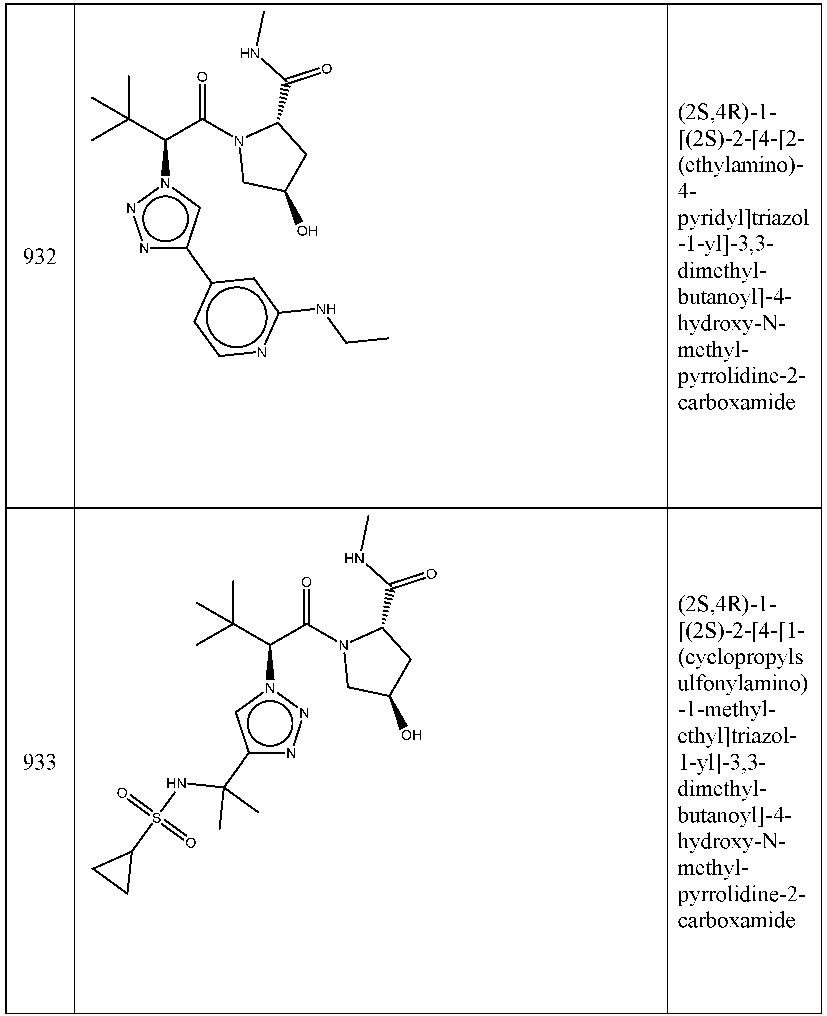

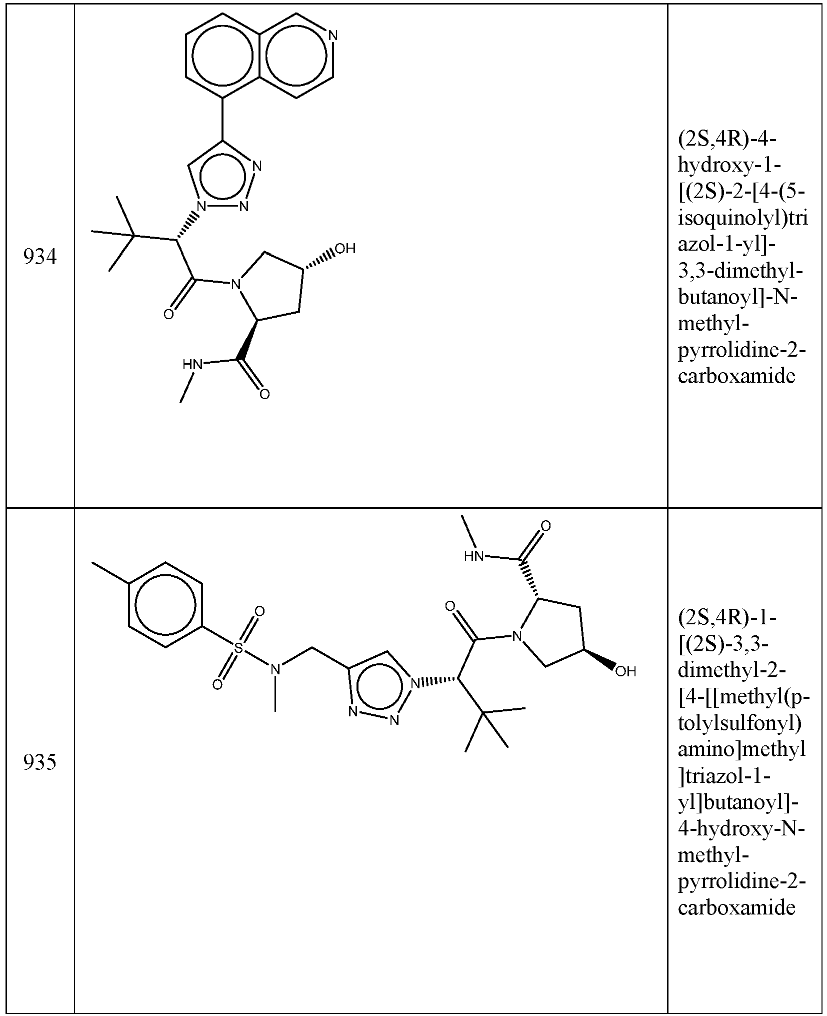

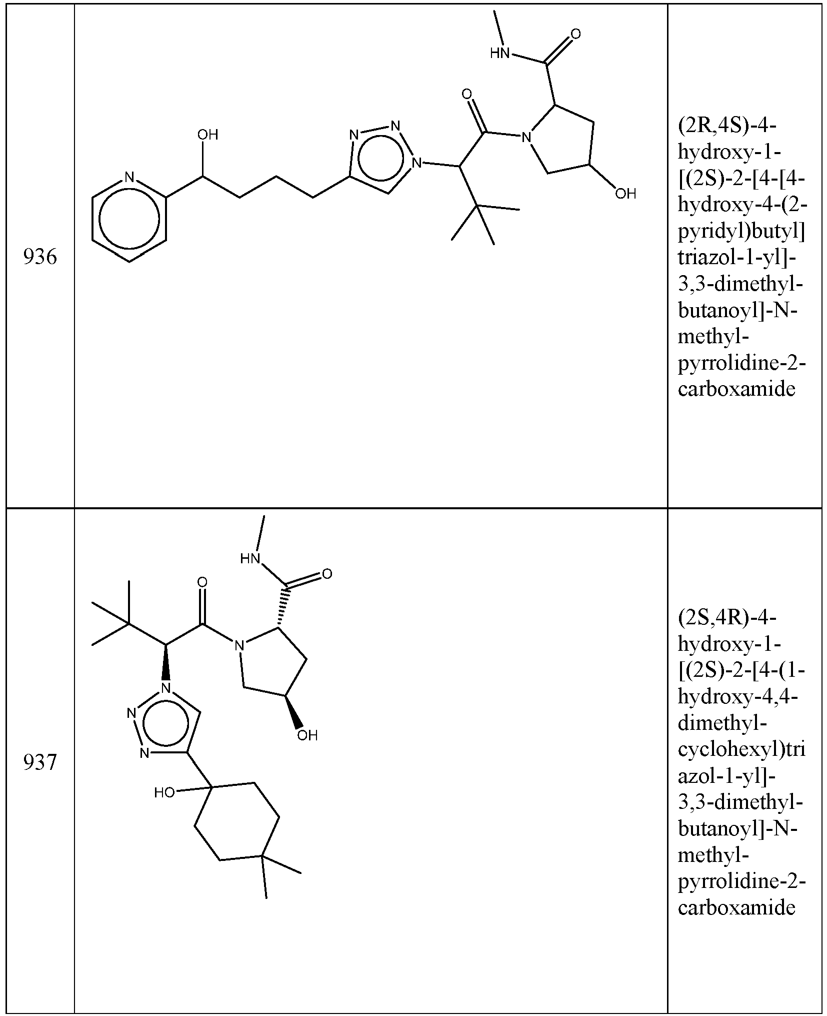

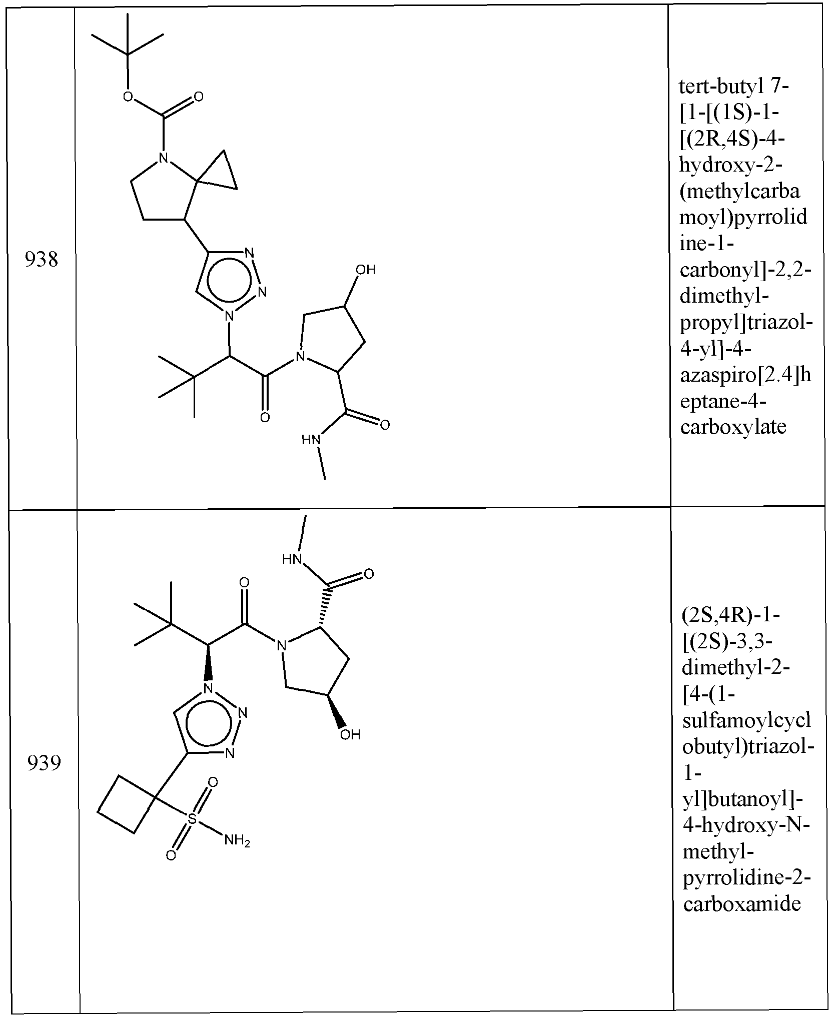

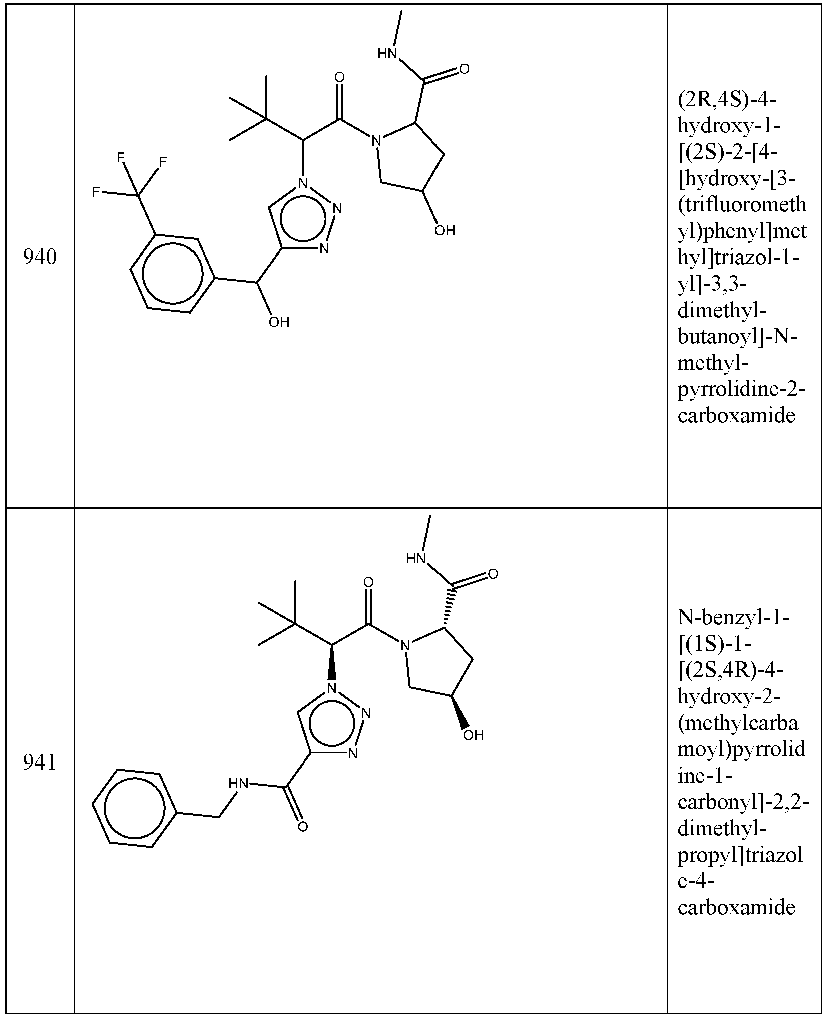

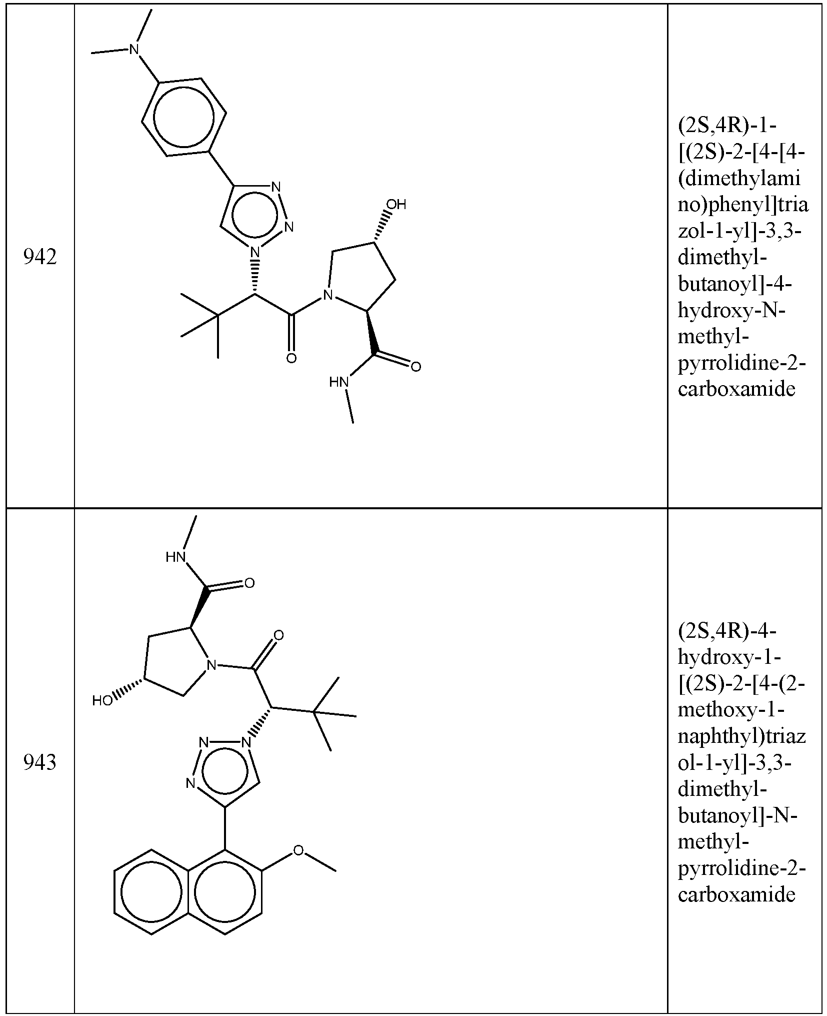

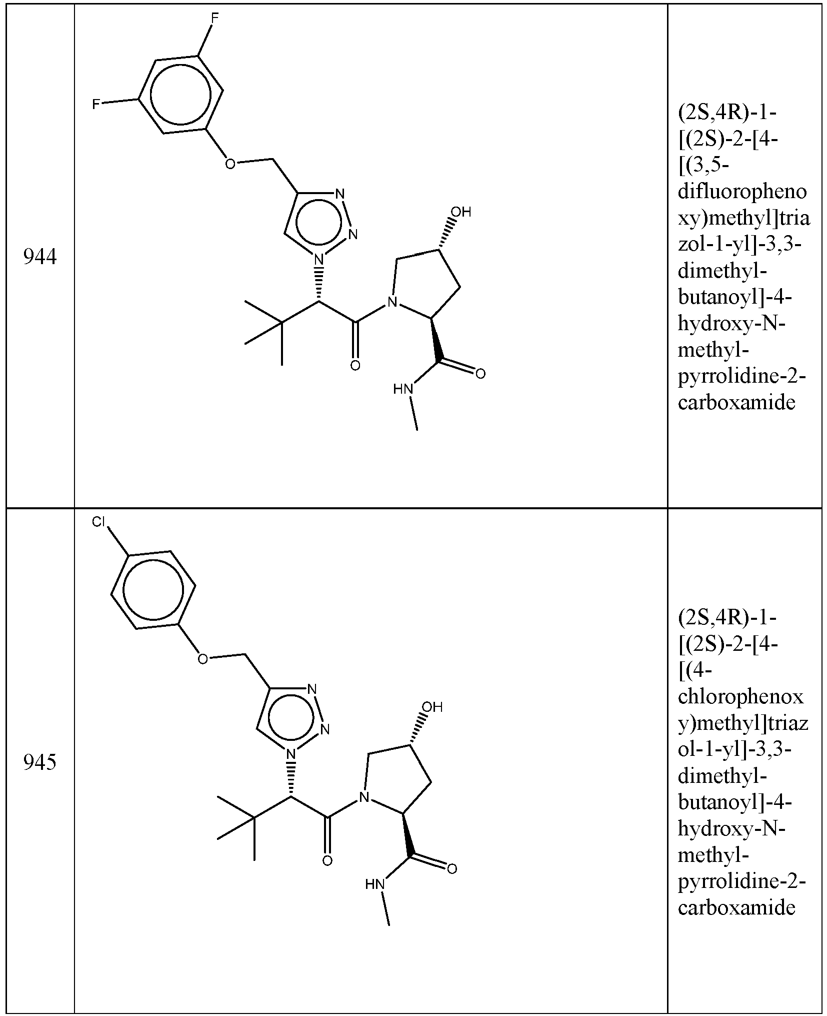

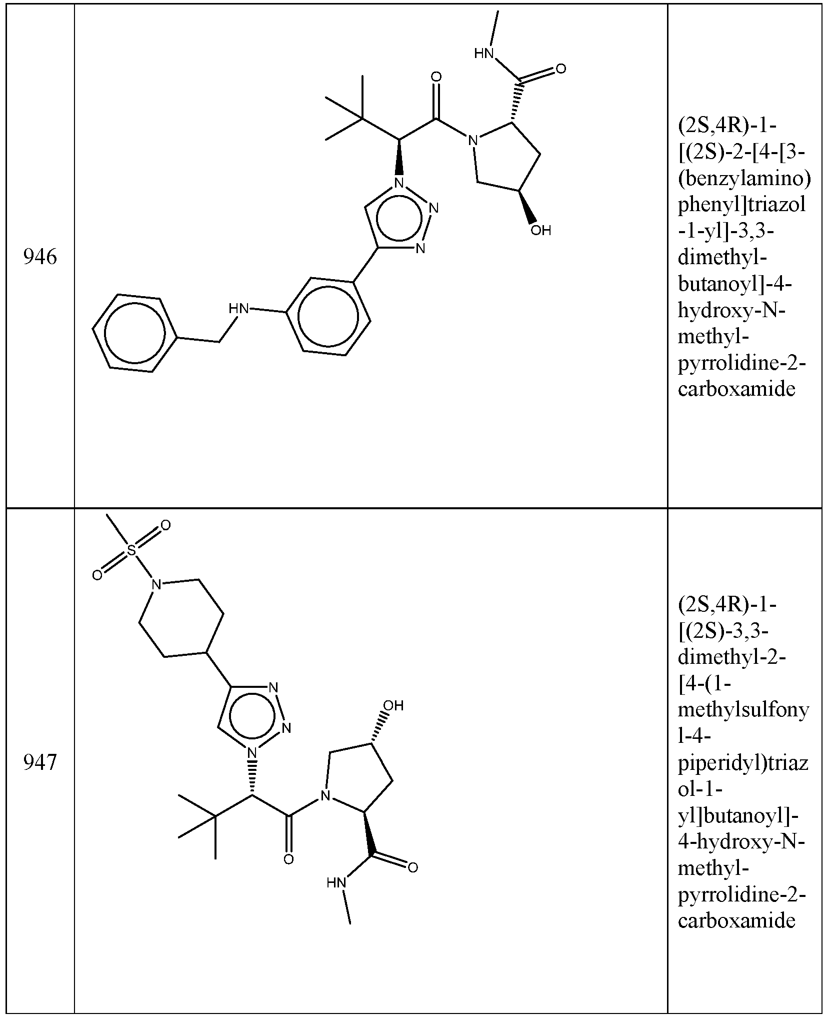

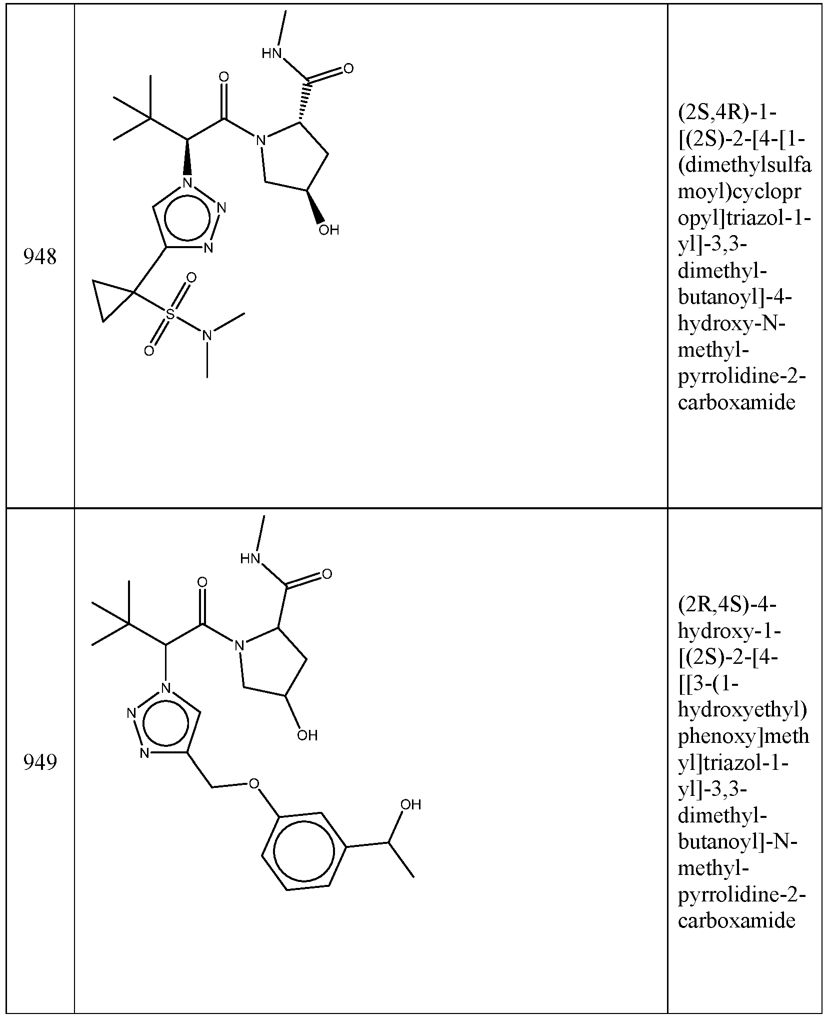

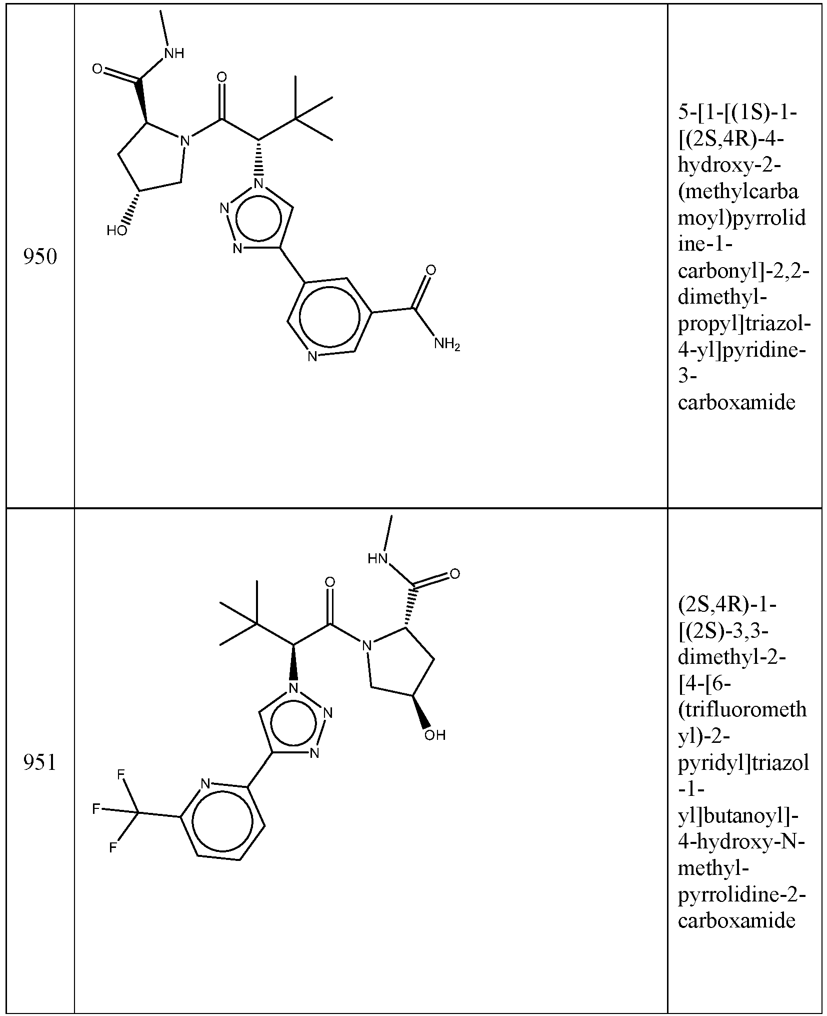

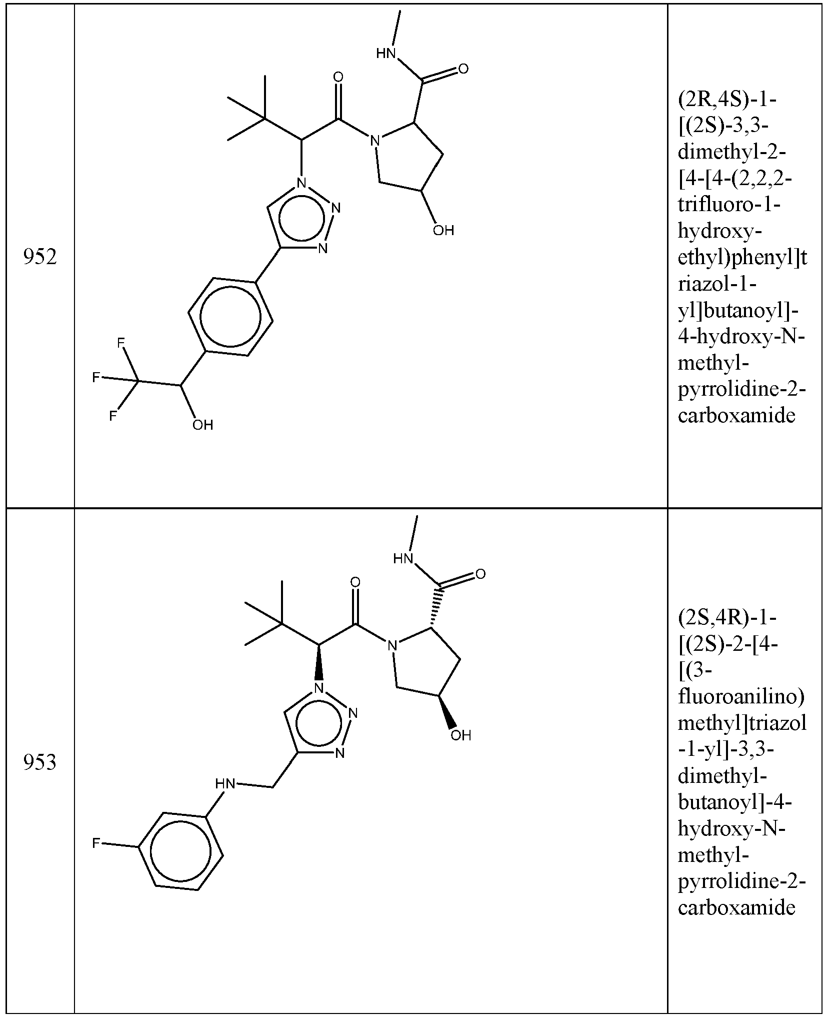

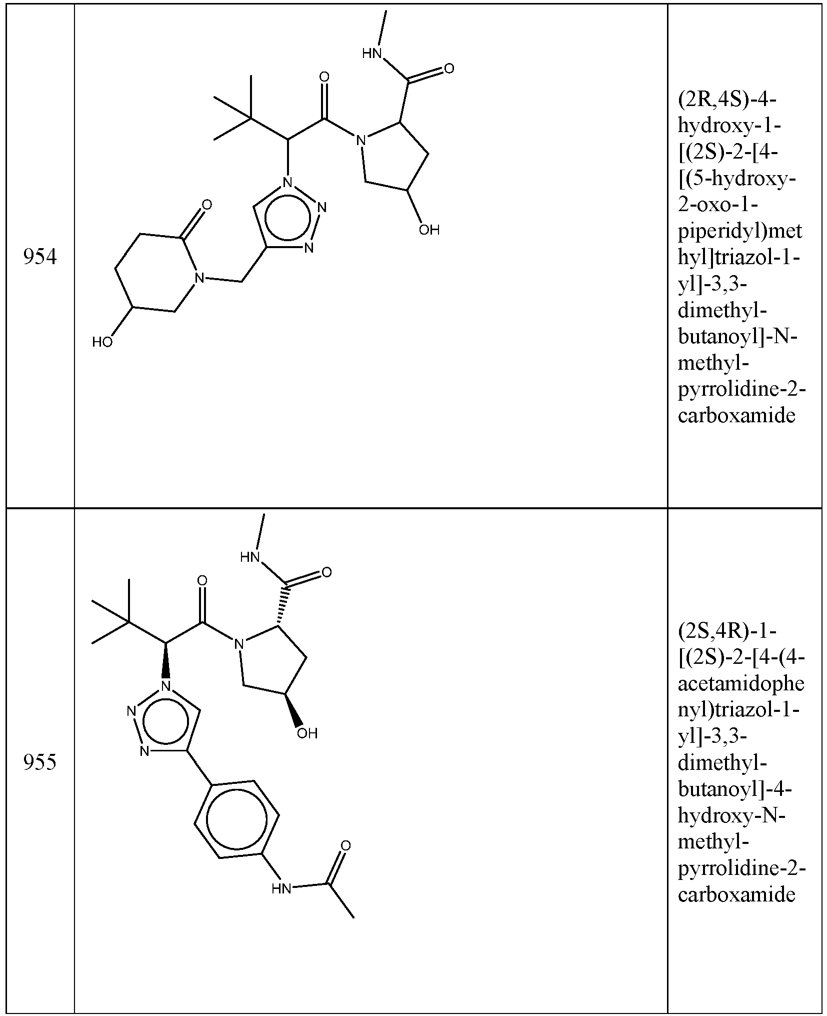

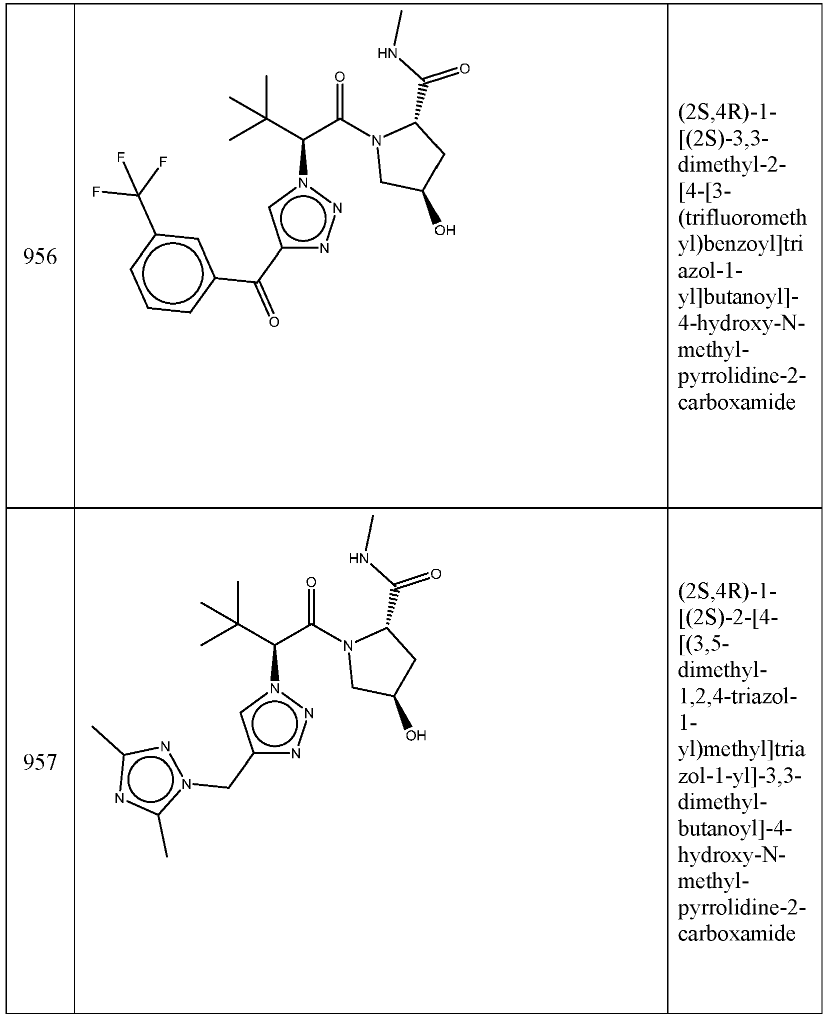

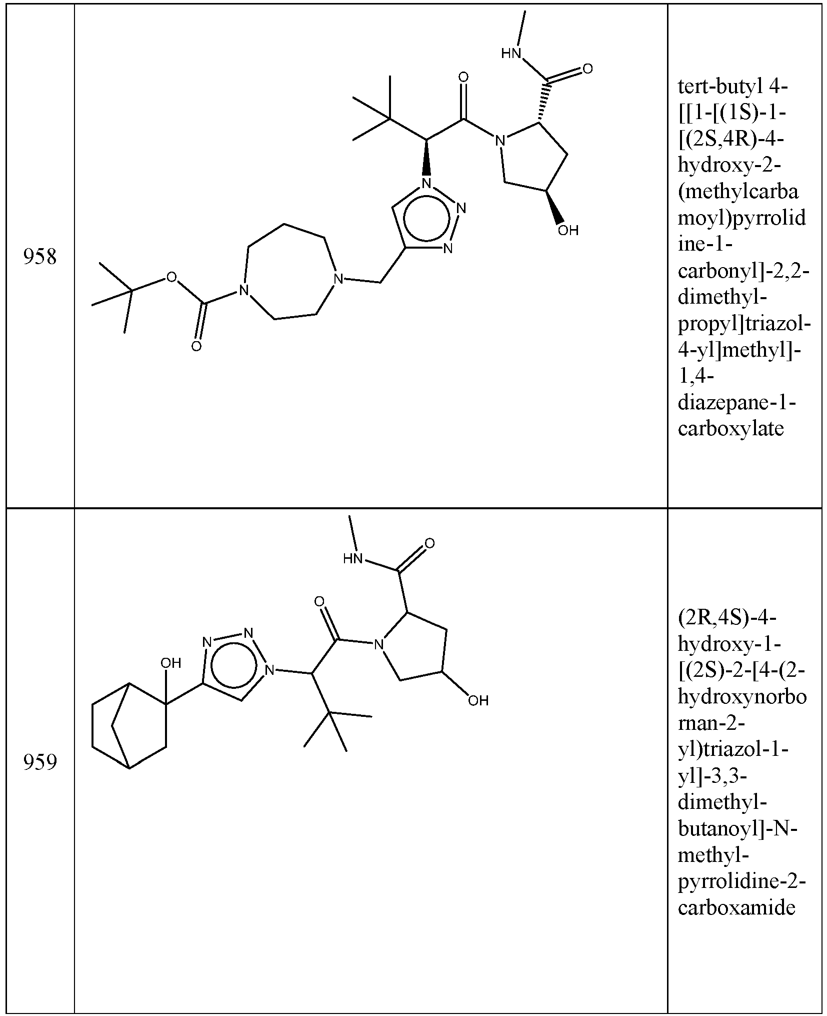

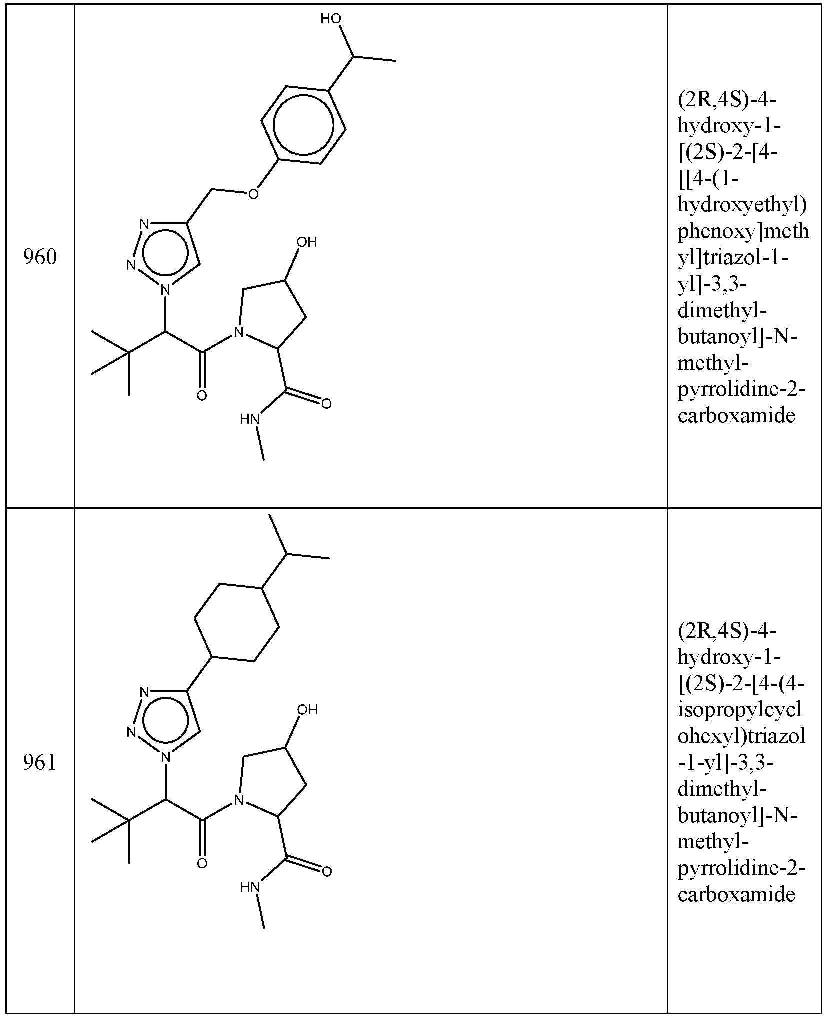

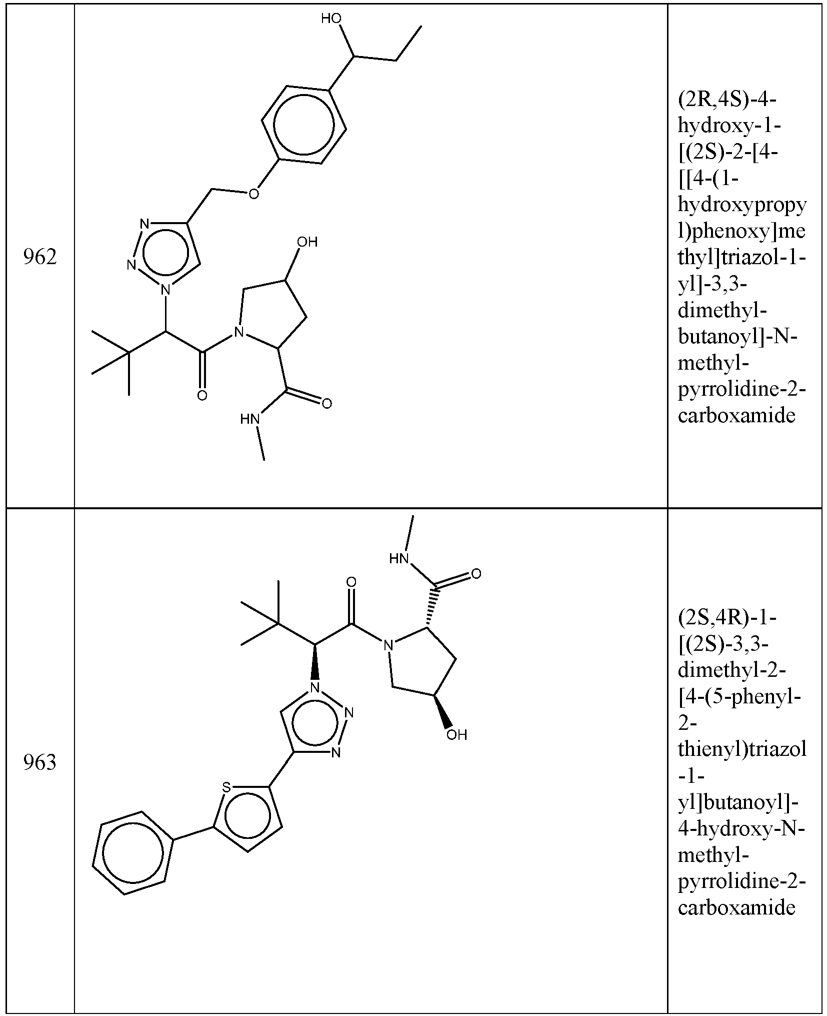

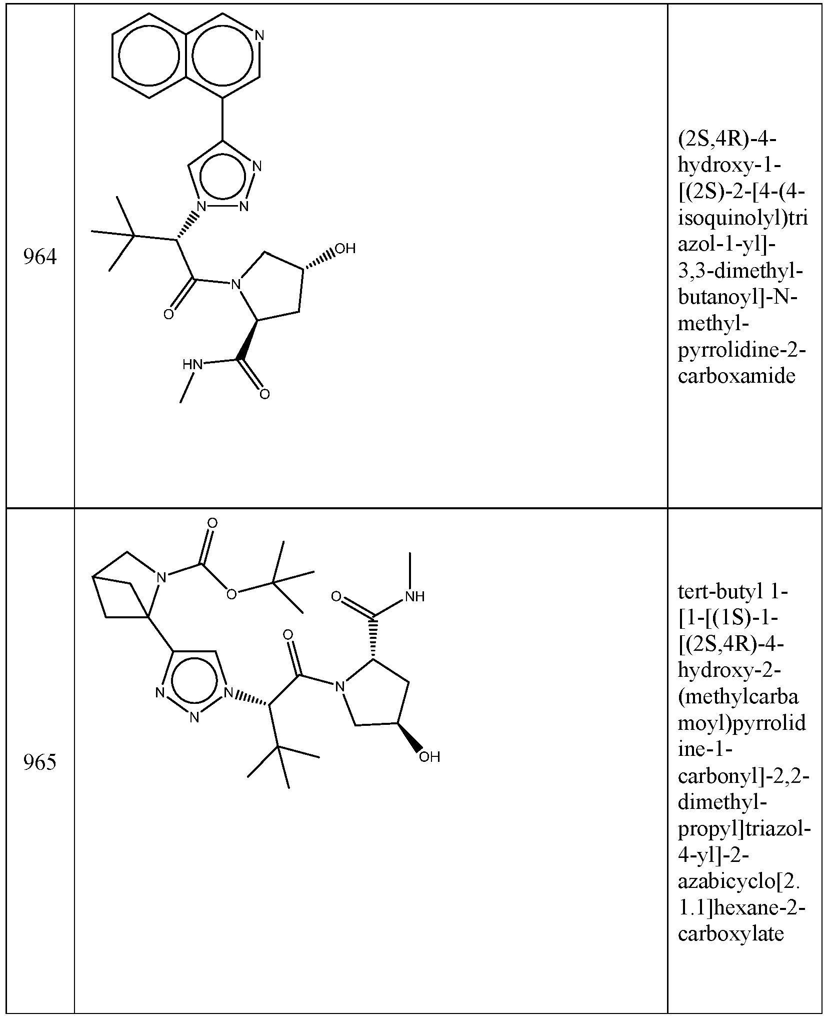

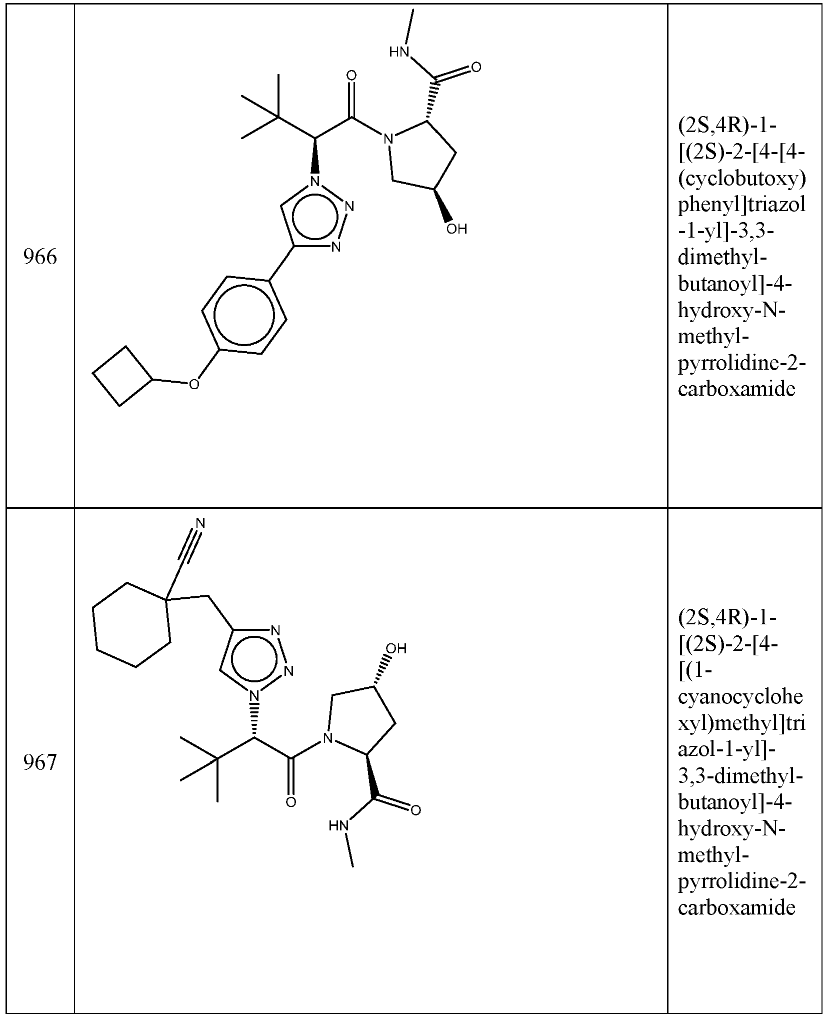

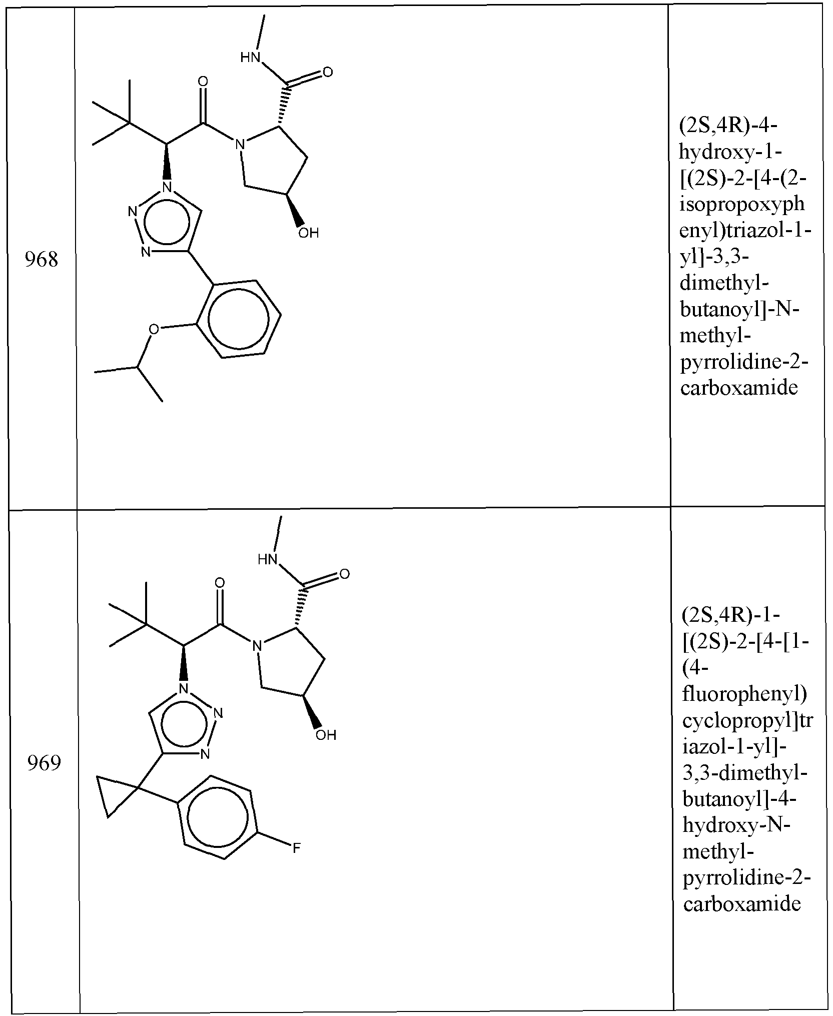

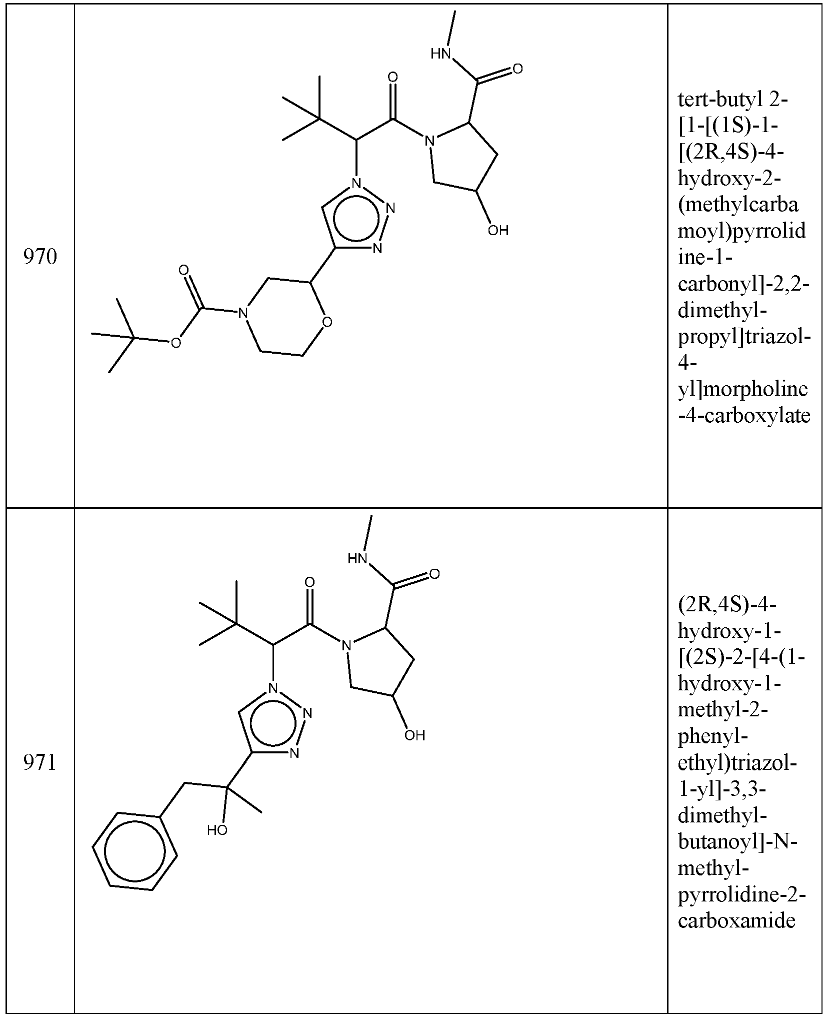

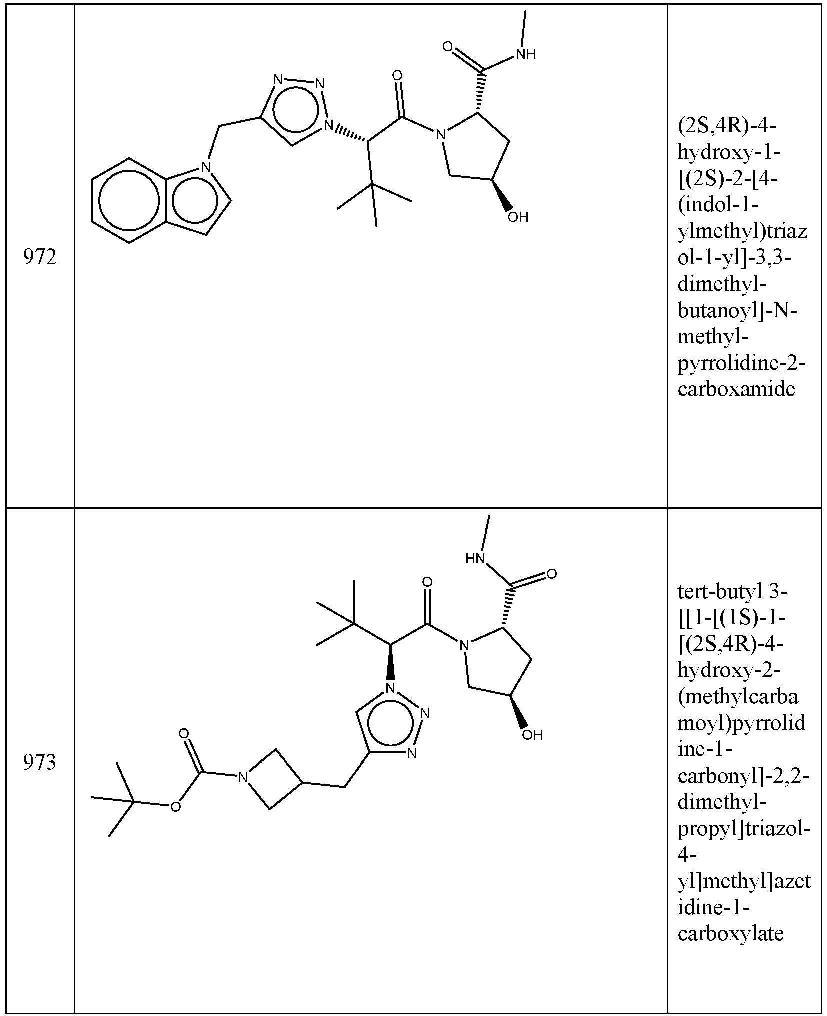

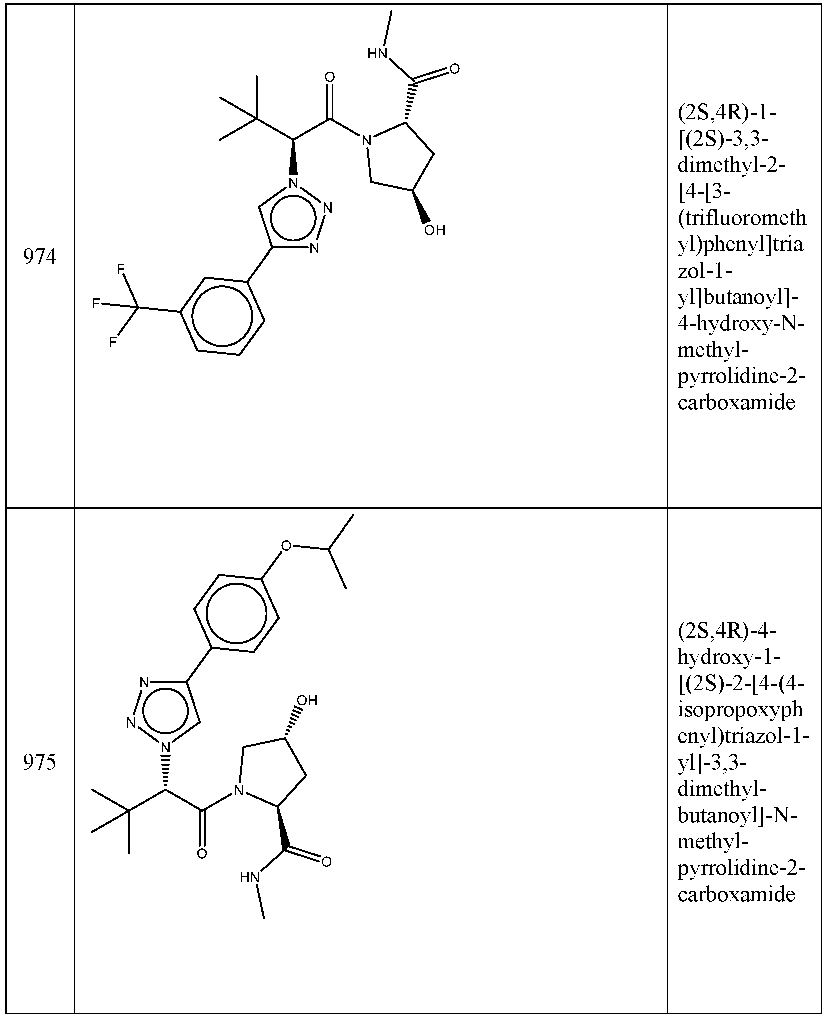

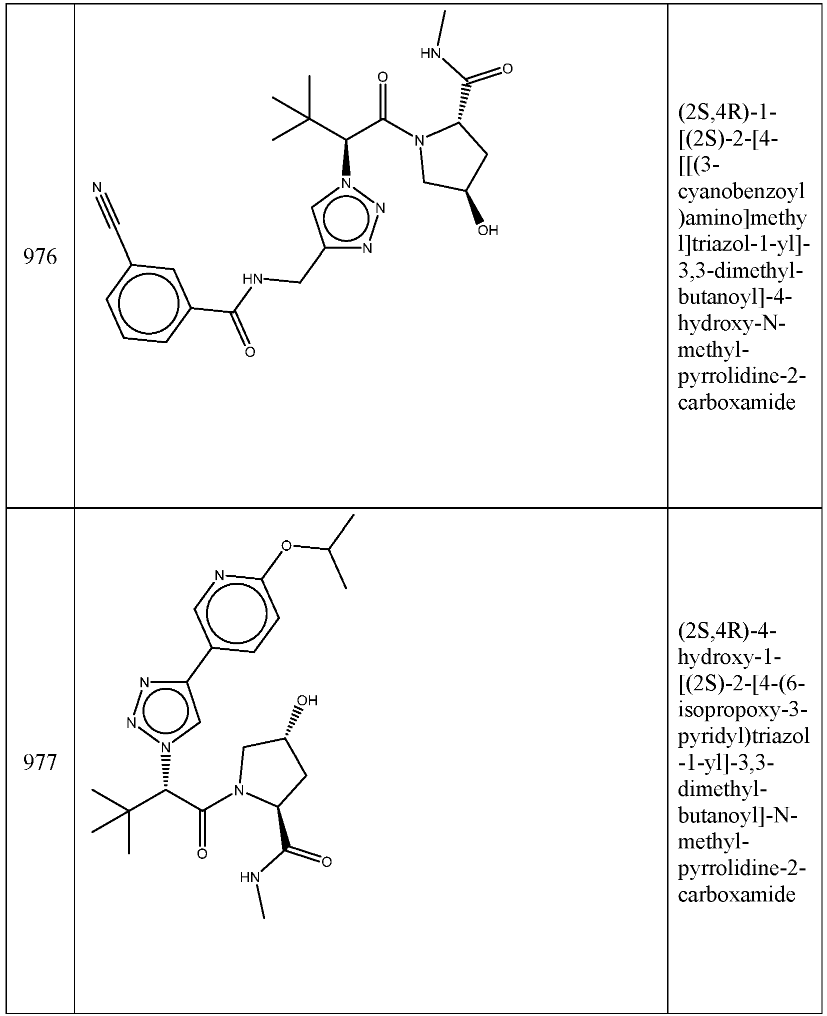

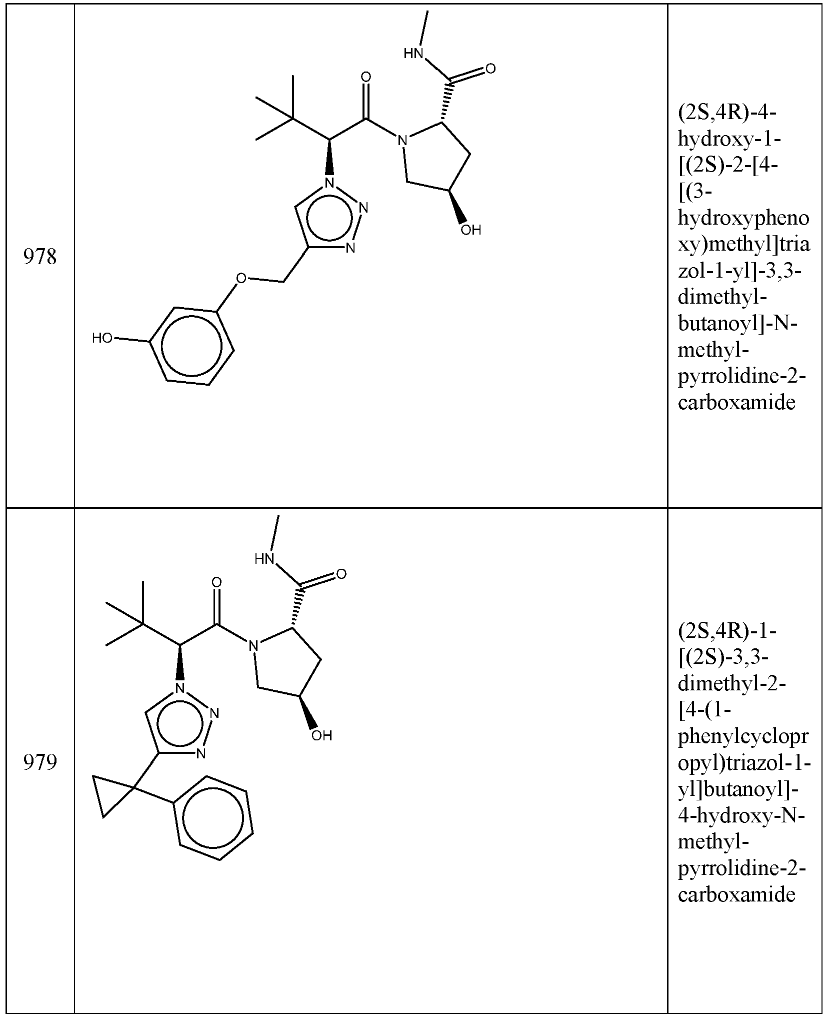

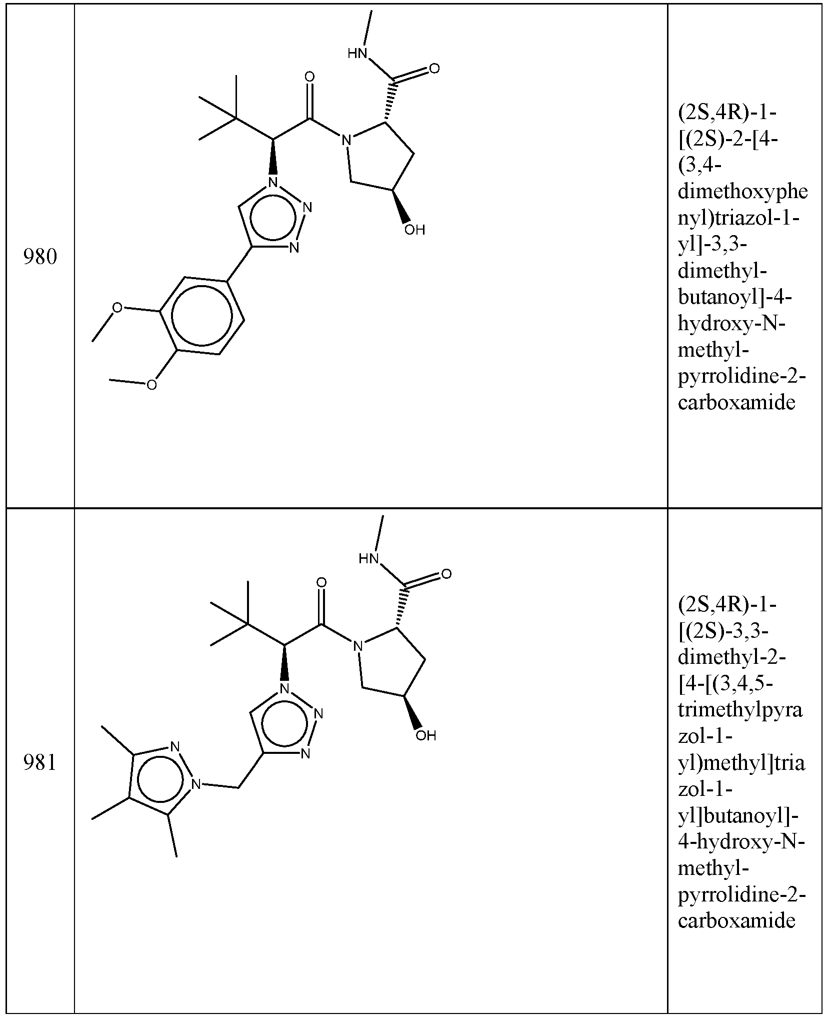

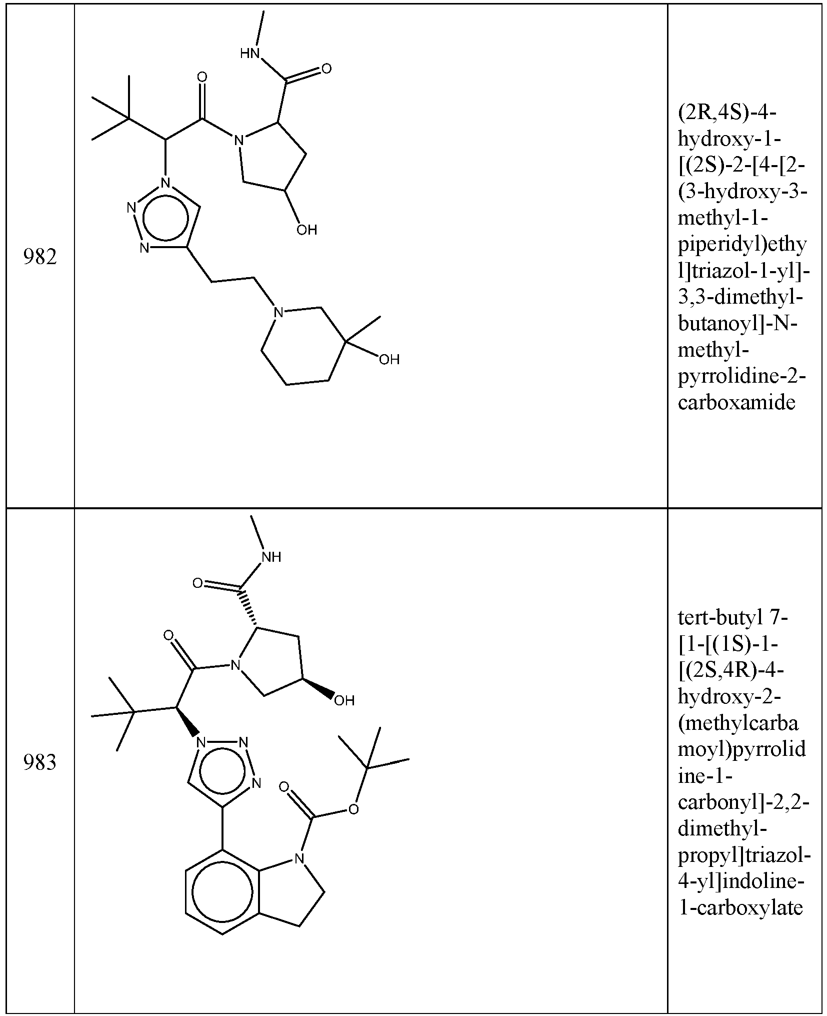

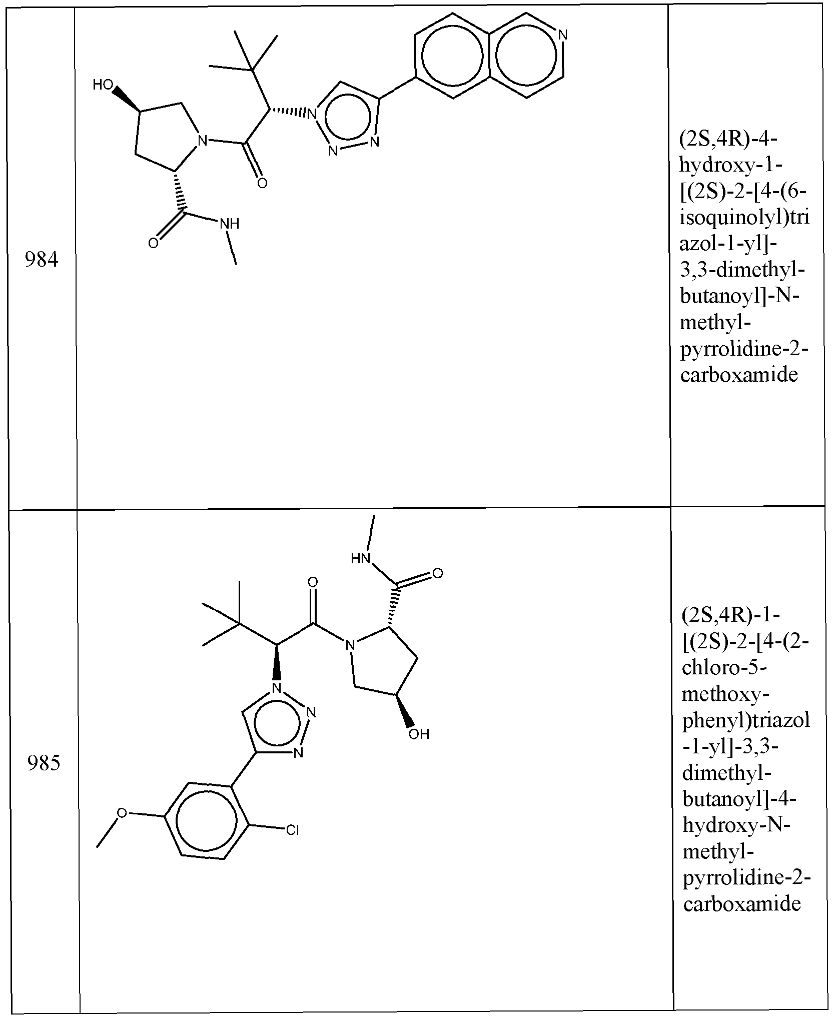

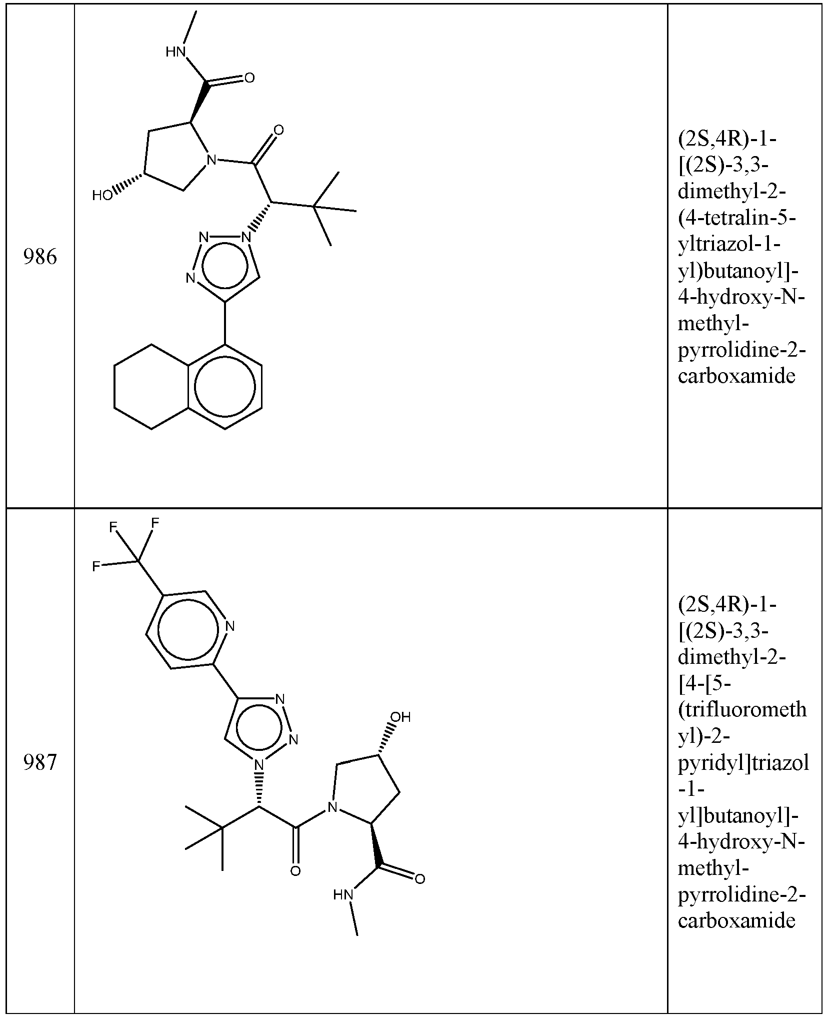

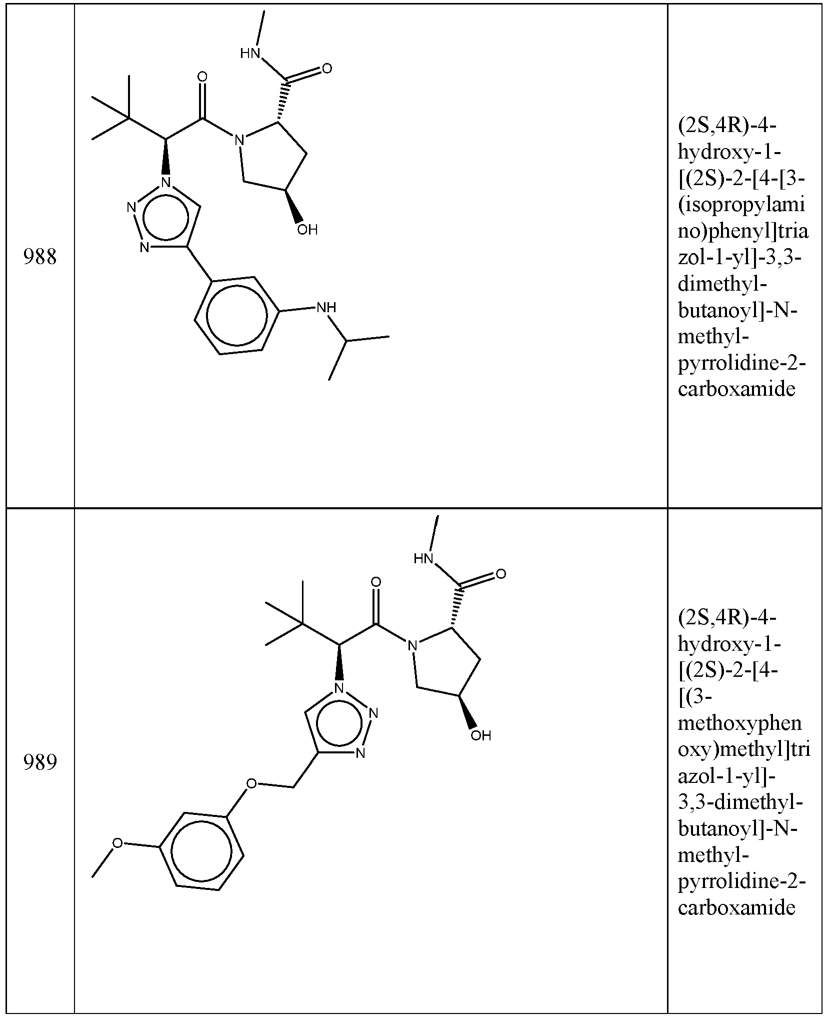

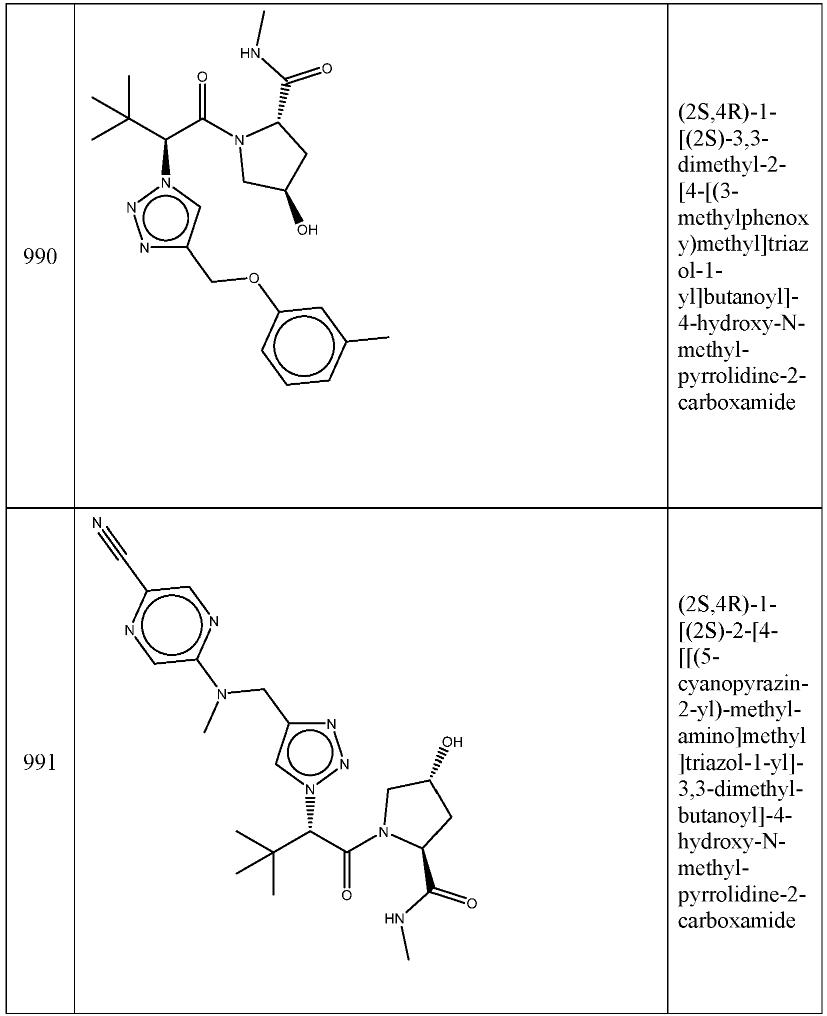

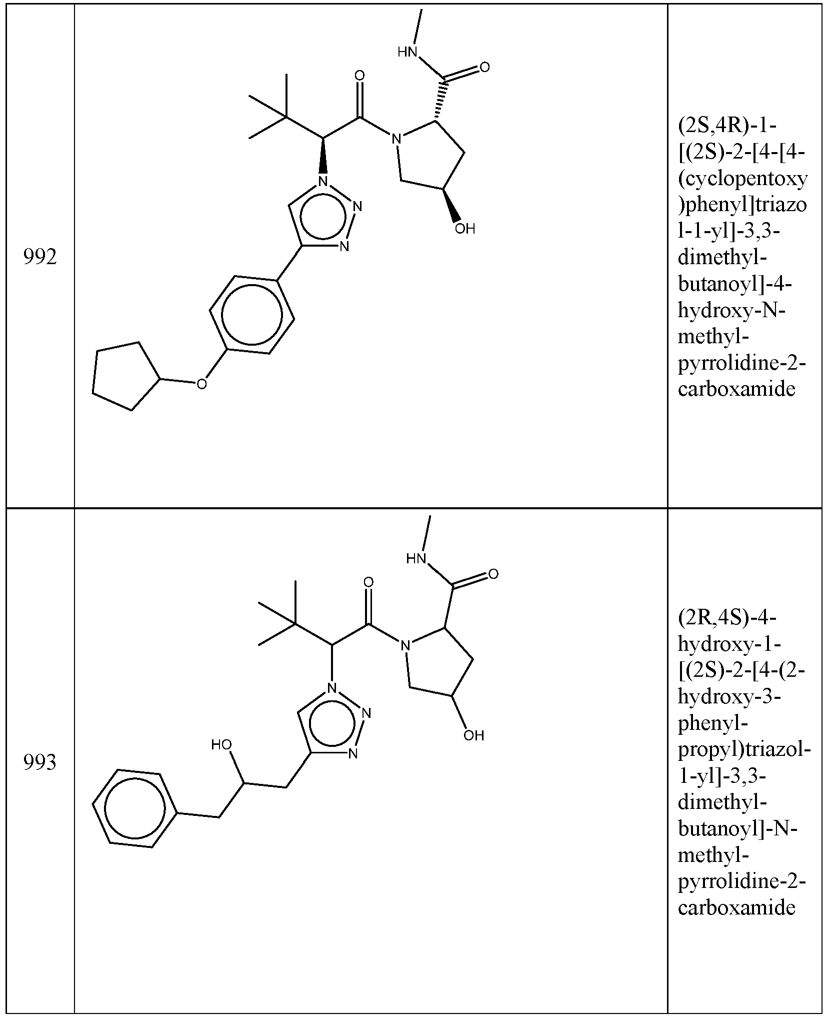

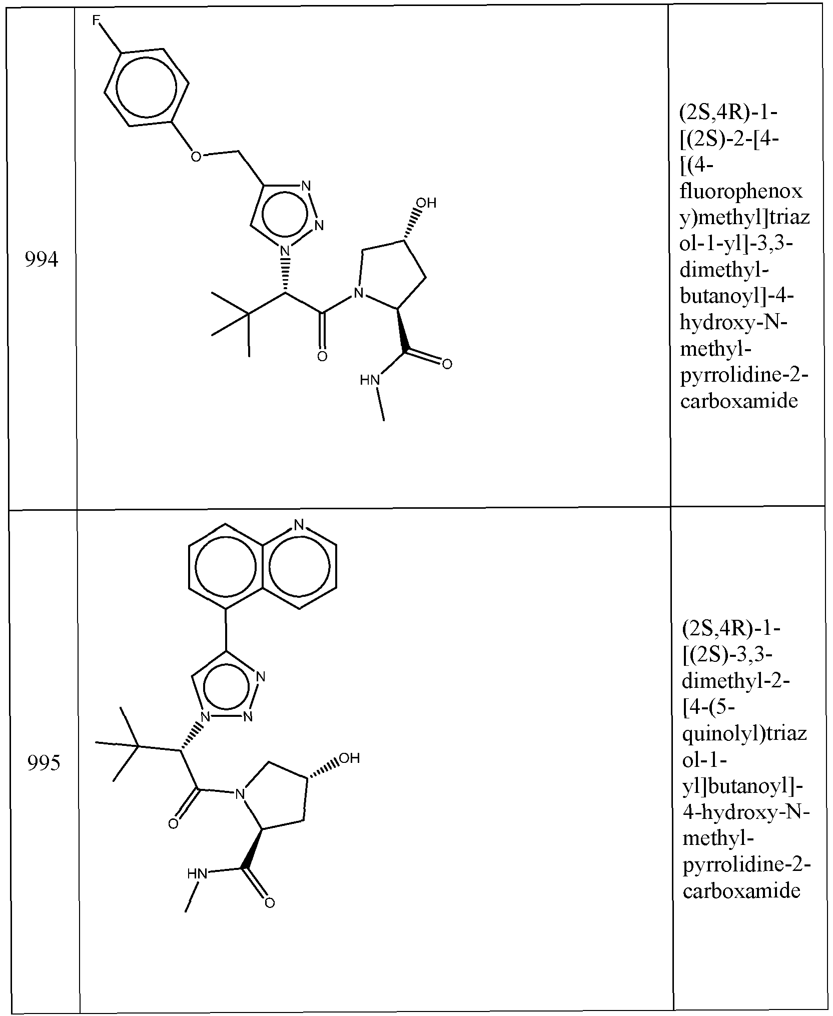

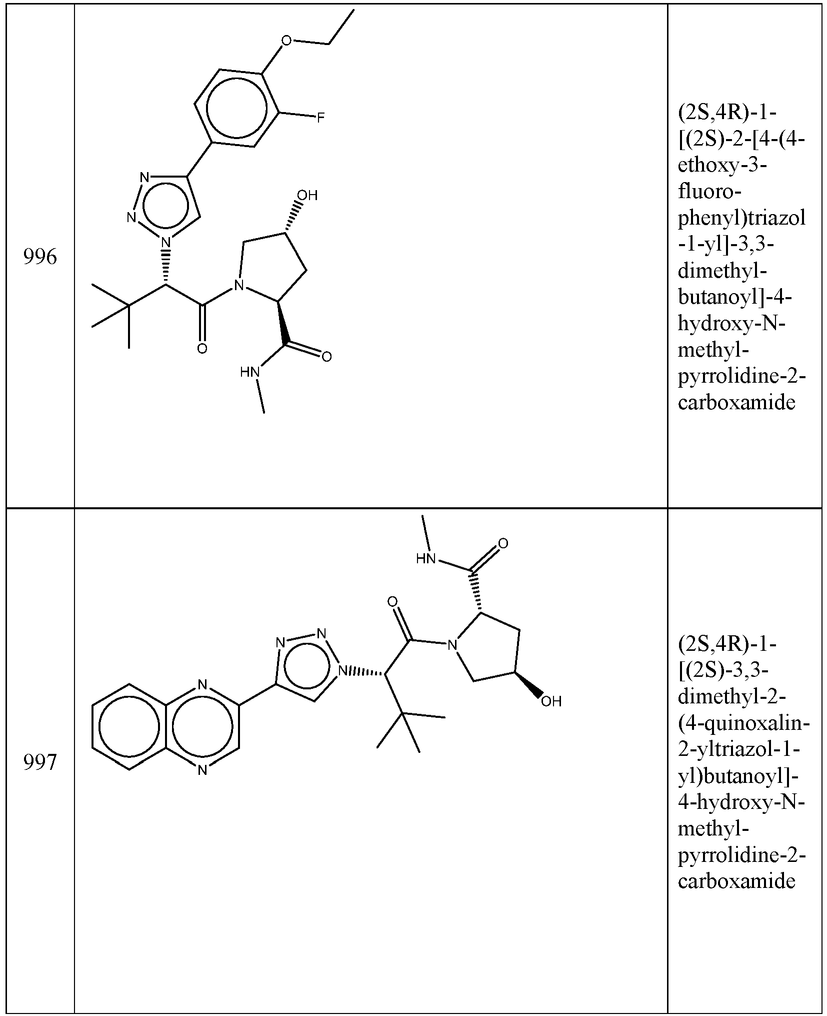

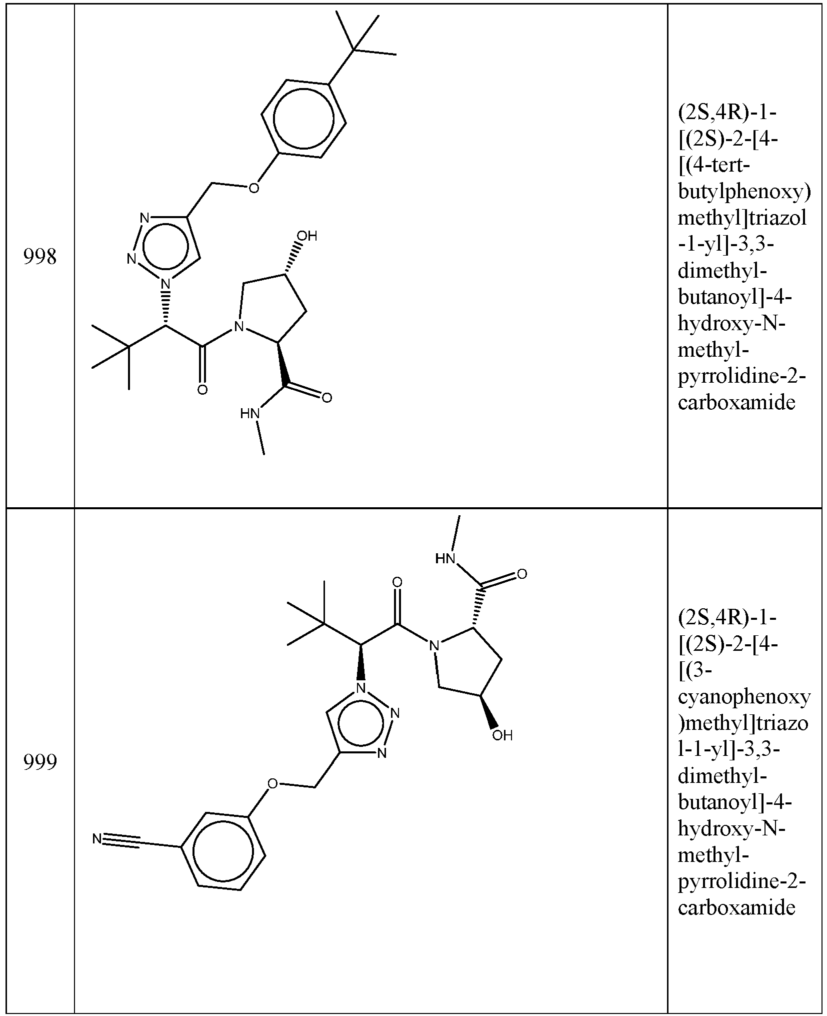

**Table 1-C**

| # | **Structure** | **Name** |
|---|---|---|
| 1000 | 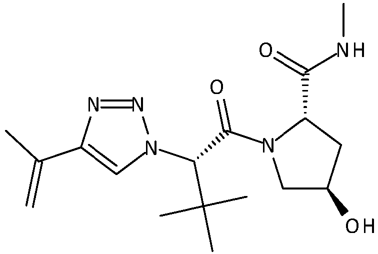 | (2S,4R)-4-hydroxy-1-[(2S)-2-(4-isopropenyltriazol-1-yl)-3,3-dimethyl-butanoyl]-N-methyl-pyrrolidine-2-carboxamide |
| 1001 | 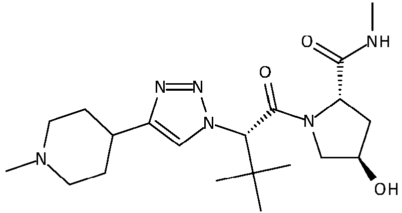 | (2S,4R)-1-[(2S)-3,3-dimethyl-2-[4-(1-methyl-4-piperidyl)triazol-1-yl]butanoyl]-4-hydroxy-N-methyl-pyrrolidine-2-carboxamide |
| 1002 | 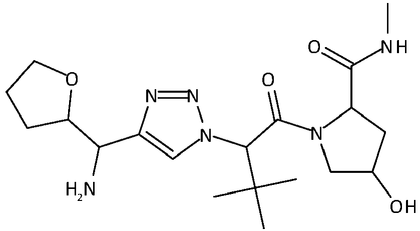 | (2R,4S)-1-[(2R)-2-[4-[amino(tetrahydrofuran-2-yl)methyl]triazol-1-yl]-3,3-dimethyl-butanoyl]-4-hydroxy-N-methyl-pyrrolidine-2-carboxamide |
| 1003 | 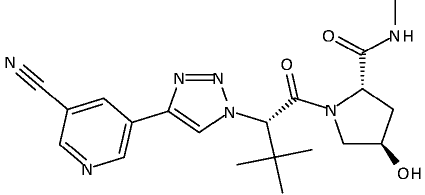 | (2S,4R)-1-[(2S)-2-[4-(5-cyano-3-pyridyl)triazol-1-yl]-3,3-dimethylbutanoyl]-4-hydroxy-N-methyl-pyrrolidine-2-carboxamide |
| 1004 | 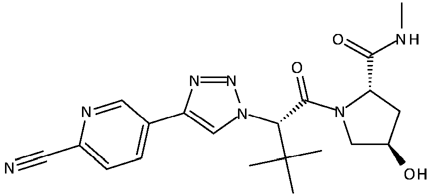 | (2S,4R)-1-[(2S)-2-[4-(6-cyano-3-pyridyl)triazol-1-yl]-3,3-dimethylbutanoyl]-4-hydroxy-N-methyl-pyrrolidine-2-carboxamide |
| 1005 | 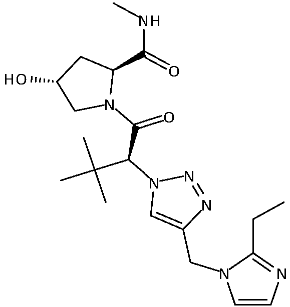 | (2S,4R)-1-[(2S)-2-[4-[(2-ethylimidazol-1-yl)methyl]triazol-1-yl]-3,3-dimethyl-butanoyl]-4-hydroxy-N-methyl-pyrrolidine-2-carboxamide |
| 1006 | 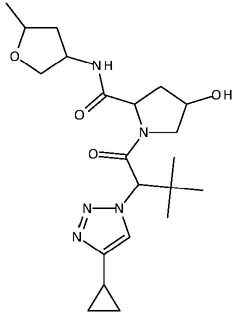 | (2R,4S)-1-[(2R)-2-(4-cyclopropyltriazol-1-yl)-3,3-dimethyl-butanoyl]-4-hydroxy-N-(5-methyltetrahydrofuran-3-yl)pyrrolidine-2-carboxamide |
| 1007 | 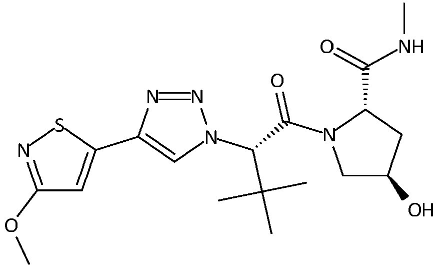 | (2S,4R)-4-hydroxy-1-[(2S)-2-[4-(3-methoxyisothiazol-5-yl)triazol-1-yl]-3,3-dimethyl-butanoyl]-N-methyl-pyrrolidine-2-carboxamide |
| 1008 | 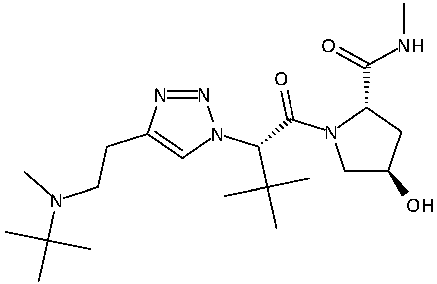 | (2S,4R)-1-[(2S)-2-[4-[2-[tert-butyl(methyl)amino]ethyl]t riazol-1-yl]-3,3-dimethylbutanoyl]-4-hydroxy-N-methyl-pyrrolidine-2-carboxamide |
| 1009 | 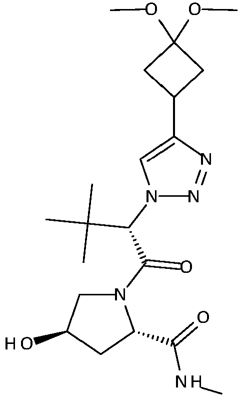 | (2S,4R)-1-[(2S)-2-[4-(3,3-dimethoxycyclobutyl)triaz ol-1-yl]-3,3-dimethylbutanoyl]-4-hydroxy-N-methyl-pyrrolidine-2-carboxamide |
| 1010 | 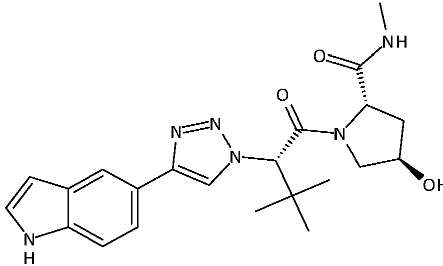 | (2S,4R)-4-hydroxy-1-[(2S)-2-[4-(1H-indol-5-yl)triazol-1-yl]-3,3-dimethyl-butanoyl]-N-methyl-pyrrolidine-2-carboxamide |
| 1011 | 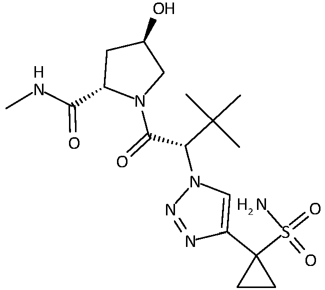 | (2S,4R)-1-[(2S)-3,3-dimethyl-2-[4-(1-sulfamoylcyclopropyl)triaz ol-1-yl]butanoyl]-4-hydroxy-N-methyl-pyrrolidine-2-carboxamide |
| 1012 | 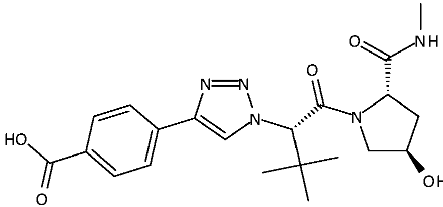 | 4-[1-[(1S)-1-[(2S,4R)-4-hydroxy-2-(methylcarbamoyl)pyrrolid ine-1-carbonyl]-2,2-dimethyl-propyl]triazol-4-yl]benzoic acid |
| 1013 | 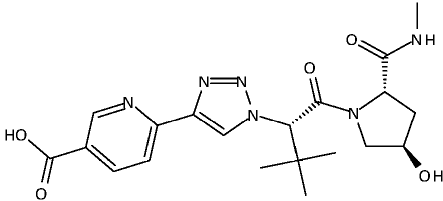 | 6-[1-[(1S)-1-[(2S,4R)-4-hydroxy-2-(methylcarbamoyl)pyrrolid ine-1-carbonyl]-2,2-dimethyl-propyl]triazol-4-yl]pyridine-3-carboxylic acid |
| 1014 | 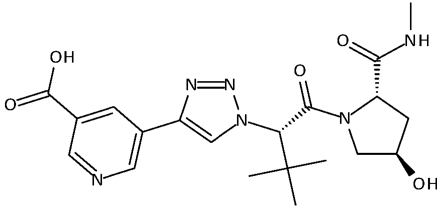 | 5-[1-[(1S)-1-[(2S,4R)-4-hydroxy-2-(methylcarbamoyl)pyrrolid ine-1-carbonyl]-2,2-dimethyl-propyl]triazol-4-yl]pyridine-3-carboxylic acid |
| 1015 | 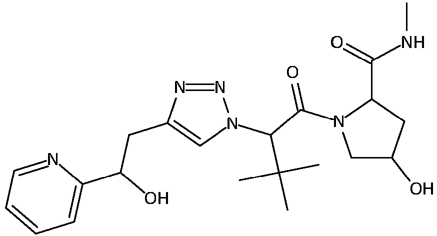 | (2R,4S)-4-hydroxy-1-[(2R)-2-[4-[2-hydroxy-2-(2-pyridyl)ethyl]triazol-1-yl]-3,3-dimethyl-butanoyl]-N-methyl-pyrrolidine-2-carboxamide |
| 1016 | 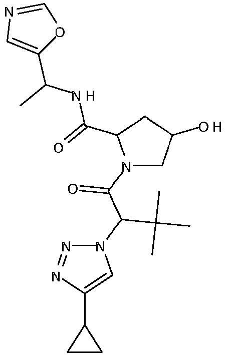 | (2R,4S)-1-[(2R)-2-(4-cyclopropyltriazol-1-yl)-3,3-dimethyl-butanoyl]-4-hydroxy-N-(1-oxazol-5-ylethyl)pyrrolidine-2-carboxamide |
| 1017 | 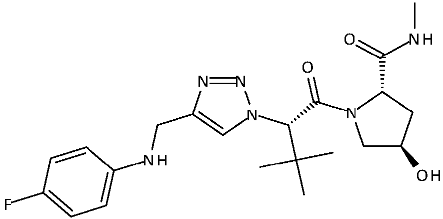 | (2S,4R)-1-[(2S)-2-[4-[(4-fluoroanilino)methyl]triazo 1-1-yl]-3,3-dimethyl-butanoyl]-4-hydroxy-N-methyl-pyrrolidine-2-carboxamide |
| 1018 | 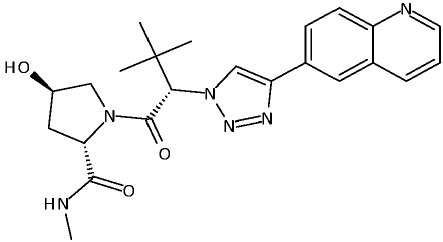 | (2S,4R)-1-[(2S)-3,3-dimethyl-2-[4-(6-quinolyl)triazol-1-yl]butanoyl]-4-hydroxy-N-methyl-pyrrolidine-2-carboxamide |
| 1019 | 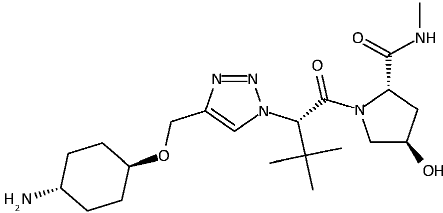 | (2S,4R)-1-[(2S)-2-[4-[(4-aminocyclohexoxy)methyl ]triazol-1-yl]-3,3-dimethyl-butanoyl]-4-hydroxy-N-methyl-pyrrolidine-2-carboxamide |
| 1020 | 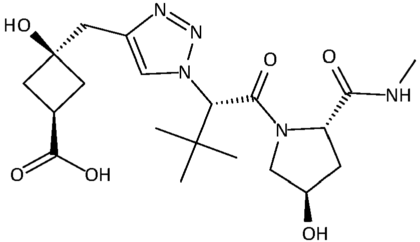 | 3-hydroxy-3-[[1-[(1S)-1-[(2S,4R)-4-hydroxy-2-(methylcarbamoyl)pyrrolid ine-1-carbonyl]-2,2-dimethyl-propyl]triazol-4-yl]methyl]cyclobutanecarb oxylic acid |
| 1021 | 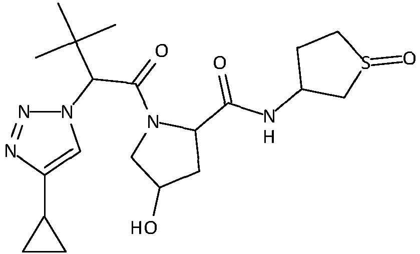 | (2S,4R)-1-[(2R)-2-(4-cyclopropyltriazol-1-yl)-3,3-dimethyl-butanoyl]-4-hydroxy-N-(1-oxothiolan-3-yl)pyrrolidine-2-carboxamide |
| 1022 | 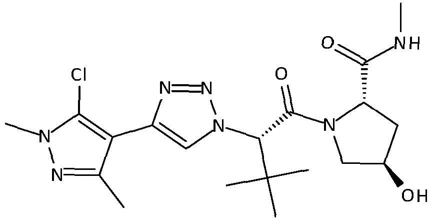 | (2S,4R)-1-[(2S)-2-[4-(5-chloro-1,3-dimethyl-pyrazol-4-yl)triazol-1-yl]-3,3-dimethyl-butanoyl]-4-hydroxy-N-methyl-pyrrolidine-2-carboxamide |
| 1023 | 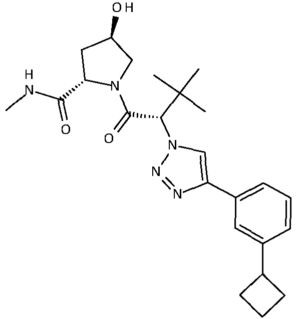 | (2S,4R)-1-[(2S)-2-[4-(3-cyclobutylphenyl)triazol-1-yl]-3,3-dimethyl-butanoyl]-4-hydroxy-N-methyl-pyrrolidine-2-carboxamide |
| 1024 | 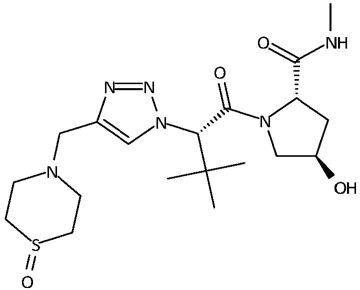 | (2S,4R)-1-[(2S)-3,3-dimethyl-2-[4-[(1-oxo-1,4-thiazinan-4-yl)methyl]triazol-1-yl]butanoyl]-4-hydroxy-N-methyl-pyrrolidine-2-carboxamide |
| 1025 | 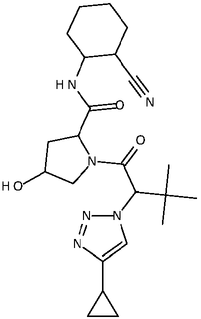 | (2S,4R)-N-(2-cyanocyclohexyl)-1-[(2R)-2-(4-cyclopropyltriazol-1-yl)-3,3-dimethyl-butanoyl]-4-hydroxy-pyrrolidine-2-carboxamide |
| 1026 | 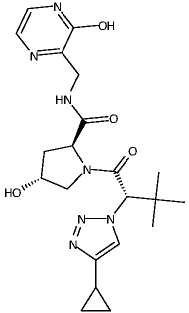 | (2S,4R)-1-[(2S)-2-(4-cyclopropyltriazol-1-yl)-3,3-dimethyl-butanoyl]-4-hydroxy-N-[(3-hydroxypyrazin-2-yl)methyl]pyrrolidine-2-carboxamide |
| 1027 | 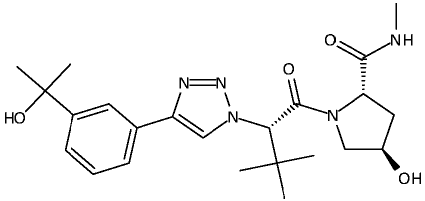 | (2S,4R)-4-hydroxy-1-[(2S)-2-[4-[3-(1-hydroxy-1-methyl-ethyl)phenyl]triazol-1-yl]-3,3-dimethyl-butanoyl]-N-methyl-pyrrolidine-2-carboxamide |
| 1028 | 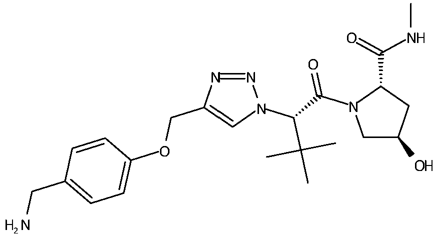 | (2S,4R)-1-[(2S)-2-[4-[[4-(aminomethyl)phenoxy]me thyl]triazol-1-yl]-3,3-dimethyl-butanoyl]-4-hydroxy-N-methyl-pyrrolidine-2-carboxamide |
| 1029 | 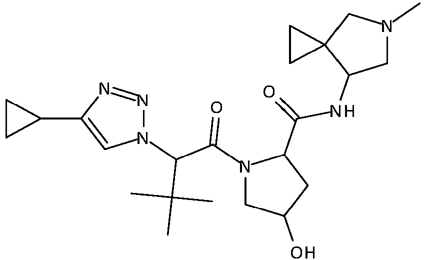 | (2R,4S)-1-[(2R)-2-(4-cyclopropyltriazol-1-yl)-3,3-dimethyl-butanoyl]-4-hydroxy-N-(5-methyl-5-azaspiro[2.4]heptan-7-yl)pyrrolidine-2-carboxamide |
| 1030 | 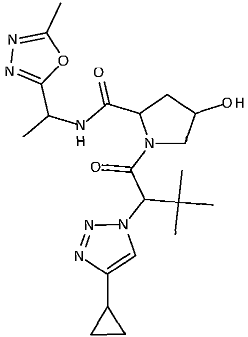 | (2R,4S)-1-[(2R)-2-(4-cyclopropyltriazol-1-yl)-3,3-dimethyl-butanoyl]-4-hydroxy-N-[1-(5-methyl-1,3,4-oxadiazol-2-yl)ethyl]pyrrolidine-2-carboxamide |
| 1031 | 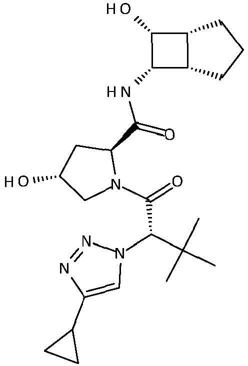 | (2S,4R)-1-[(2S)-2-(4-cyclopropyltriazol-1-yl)-3,3-dimethyl-butanoyl]-4-hydroxy-N-[(1R,5S,6S,7R)-7-hydroxy-6-bicyclo[3.2.0]heptanyl]pyr rolidine-2-carboxamide |
| 1032 | 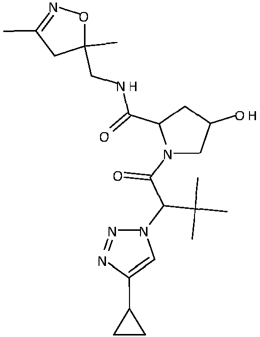 | (2R,4S)-1-[(2R)-2-(4-cyclopropyltriazol-1-yl)-3,3-dimethyl-butanoyl]-N-[(3,5-dimethyl-4H-isoxazol-5-yl)methyl]-4-hydroxy-pyrrolidine-2-carboxamide |
| 1033 | 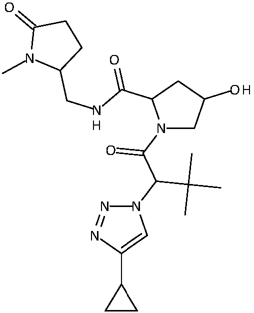 | (2R,4S)-1-[(2R)-2-(4-cyclopropyltriazol-1-yl)-3,3-dimethyl-butanoyl]-4-hydroxy-N-[(1-methyl-5-oxo-pyrrolidin-2-yl)methyl]pyrrolidine-2-carboxamide |
| 1034 | 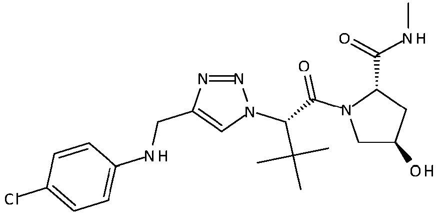 | (2S,4R)-1-[(2S)-2-[4-[(4-chloroanilino)methyl]triaz ol-1-yl]-3,3-dimethyl-butanoyl]-4-hydroxy-N-methyl-pyrrolidine-2-carboxamide |
| 1035 | 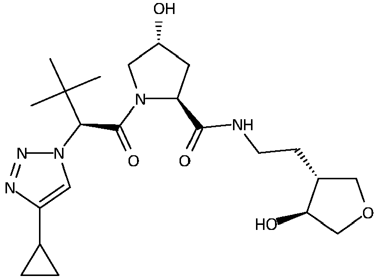 | (2S,4R)-1-[(2S)-2-(4-cyclopropyltriazol-1-yl)-3,3-dimethyl-butanoyl]-4-hydroxy-N-[2-[(3R,4S)-4-hydroxytetrahydrofuran-3-yl]ethyl]pyrrolidine-2-carboxamide |
| 1036 | 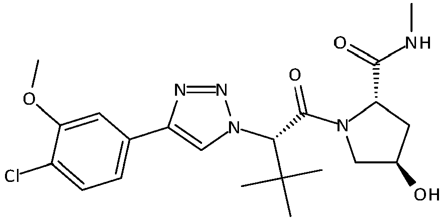 | (2S,4R)-1-[(2S)-2-[4-(4-chloro-3-methoxy-phenyl)triazol-1-yl]-3,3-dimethyl-butanoyl]-4-hydroxy-N-methyl-pyrrolidine-2-carboxamide |
| 1037 | 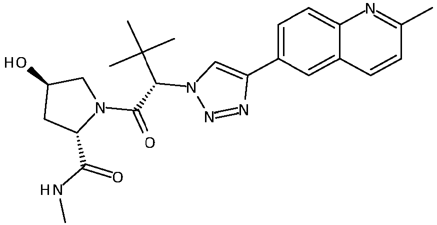 | (2S,4R)-1-[(2S)-3,3-dimethyl-2-[4-(2-methyl-6-quinolyl)triazol-1-yl]butanoyl]-4-hydroxy-N-methyl-pyrrolidine-2-carboxamide |
| 1038 | 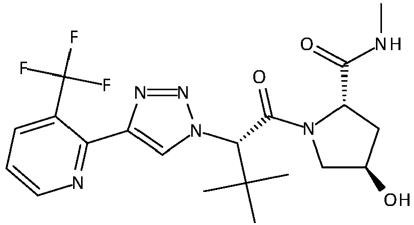 | (2S,4R)-1-[(2S)-3,3-dimethyl-2-[4-[3-(trifluoromethyl)-2-pyridyl]triazol-1-yl]butanoyl]-4-hydroxy-N-methyl-pyrrolidine-2-carboxamide |
| 1039 | 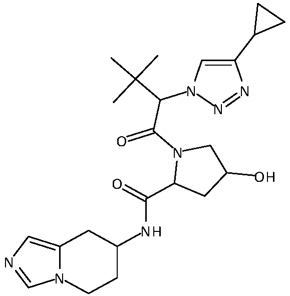 | (2S,4R)-1-[(2R)-2-(4-cyclopropyltriazol-1-yl)-3,3-dimethyl-butanoyl]-4-hydroxy-N-(5,6,7,8-tetrahydroimidazo[1,5-a]pyridin-7-yl)pyrrolidine-2-carboxamide |
| 1040 | 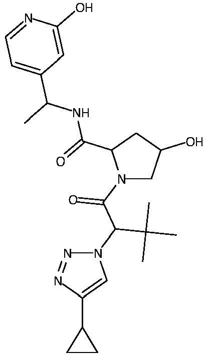 | (2R,4S)-1-[(2R)-2-(4-cyclopropyltriazol-1-yl)-3,3-dimethyl-butanoyl]-4-hydroxy-N-[1-(2-hydroxy-4-pyridyl)ethyl]pyrrolidine-2-carboxamide |
| 1041 | 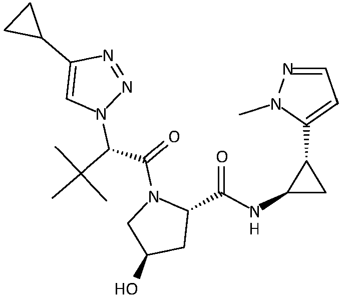 | (2S,4R)-1-[(2S)-2-(4-cyclopropyltriazol-1-yl)-3,3-dimethyl-butanoyl]-4-hydroxy-N-[(1R,2R)-2-(2-methylpyrazol-3-yl)cyclopropyl]pyrrolidine -2-carboxamide |
| 1042 | 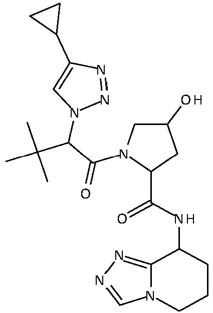 | (2S,4R)-1-[(2R)-2-(4-cyclopropyltriazol-1-yl)-3,3-dimethyl-butanoyl]-4-hydroxy-N-(5,6,7,8-tetrahydro-[1,2,4]triazolo[4,3-a]pyridin-8-yl)pyrrolidine-2-carboxamide |
| 1043 | 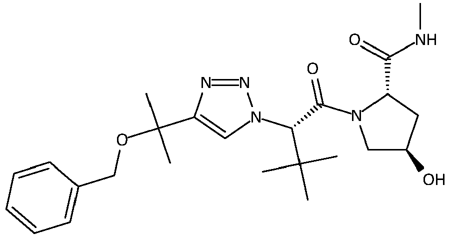 | (2S,4R)-1-[(2S)-2-[4-(1-benzyloxy-1-methyl-ethyl)triazol-1-yl]-3,3-dimethyl-butanoyl]-4-hydroxy-N-methyl-pyrrolidine-2-carboxamide |
| 1044 | 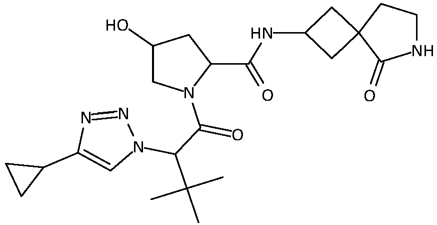 | (2S,4R)-1-[(2R)-2-(4-cyclopropyltriazol-1-yl)-3,3-dimethyl-butanoyl]-4-hydroxy-N-(5-oxo-6-azaspiro[3.4]octan-2-yl)pyrrolidine-2-carboxamide |
| 1045 | 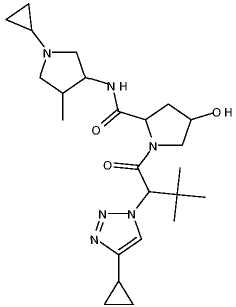 | (2R,4S)-N-(1-cyclopropyl-4-methyl-pyrrolidin-3-yl)-1-[(2R)-2-(4-cyclopropyltriazol-1-yl)-3,3-dimethyl-butanoyl]-4-hydroxy-pyrrolidine-2-carboxamide |
| 1046 | 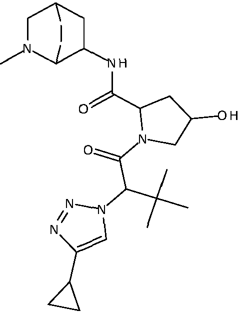 | (2R,4S)-1-[(2R)-2-(4-cyclopropyltriazol-1-yl)-3,3-dimethyl-butanoyl]-4-hydroxy-N-(2-methyl-2-azabicyclo[2.2.2]octan-6-yl)pyrrolidine-2-carboxamide |
| 1047 | 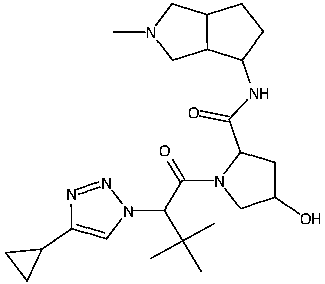 | (2R,4S)-1-[(2R)-2-(4-cyclopropyltriazol-1-yl)-3,3-dimethyl-butanoyl]-4-hydroxy-N-(2-methyl-3,3a,4,5,6,6a-hexahydro-1H-cyclopenta[c]pyrrol-4-yl)pyrrolidine-2-carboxamide |
| 1048 | 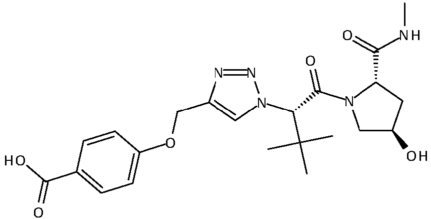 | 4-[[1-[(1S)-1-[(2S,4R)-4-hydroxy-2-(methylcarbamoyl)pyrrolid ine-1-carbonyl]-2,2-dimethyl-propyl]triazol-4-yl]methoxy]benzoic acid |
| 1049 | 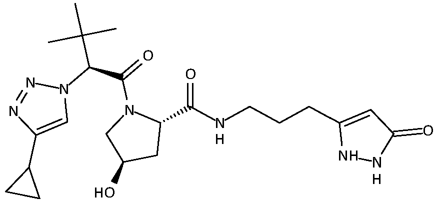 | (2S,4R)-1-[(2S)-2-(4-cyclopropyltriazol-1-yl)-3,3-dimethyl-butanoyl]-4-hydroxy-N-[3-(5-oxo-1,2-dihydropyrazol-3-yl)propyl]pyrrolidine-2-carboxamide |
| 1050 | 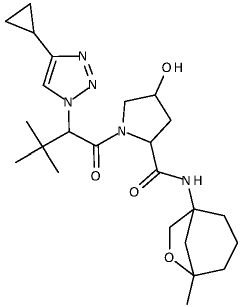 | (2S,4R)-1-[(2R)-2-(4-cyclopropyltriazol-1-yl)-3,3-dimethyl-butanoyl]-4-hydroxy-N-(5-methyl-6-oxabicyclo[3.2.1]octan-1-yl)pyrrolidine-2-carboxamide |
| 1051 | 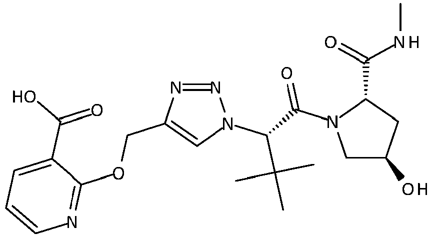 | 2-[[1-[(1S)-1-[(2S,4R)-4-hydroxy-2-(methylcarbamoyl)pyrrolid ine-1-carbonyl]-2,2-dimethyl-propyl]triazol-4-yl]methoxy]pyridine-3-carboxylic acid |
| 1052 | 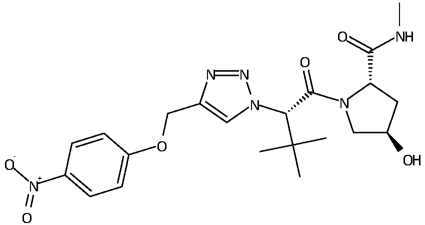 | (2S,4R)-1-[(2S)-3,3-dimethyl-2-[4-[(4-nitrophenoxy)methyl]triaz ol-1-yl]butanoyl]-4-hydroxy-N-methyl-pyrrolidine-2-carboxamide |
| 1053 | 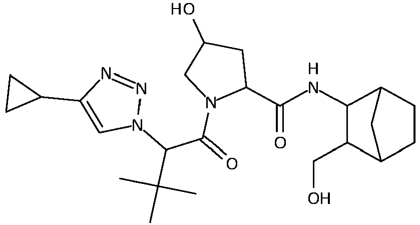 | (2S,4R)-1-[(2R)-2-(4-cyclopropyltriazol-1-yl)-3,3-dimethyl-butanoyl]-4-hydroxy-N-[3-(hydroxymethyl)norboman -2-yl]pyrrolidine-2-carboxamide |
| 1054 | 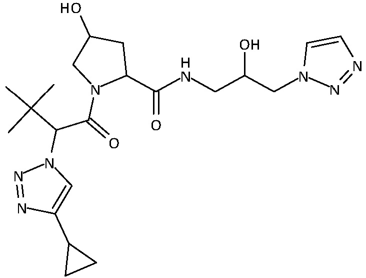 | (2S,4R)-1-[(2R)-2-(4-cyclopropyltriazol-1-yl)-3,3-dimethyl-butanoyl]-4-hydroxy-N-[2-hydroxy-3-(triazol-1-yl)propyl]pyrrolidine-2-carboxamide |
| 1055 | 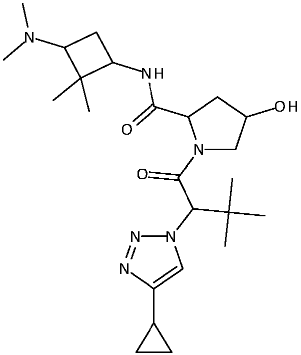 | (2R,4S)-1-[(2R)-2-(4-cyclopropyltriazol-1-yl)-3,3-dimethyl-butanoyl]-N-[3-(dimethylamino)-2,2-dimethyl-cyclobutyl]-4-hydroxy-pyrrolidine-2-carboxamide |
| 1056 | 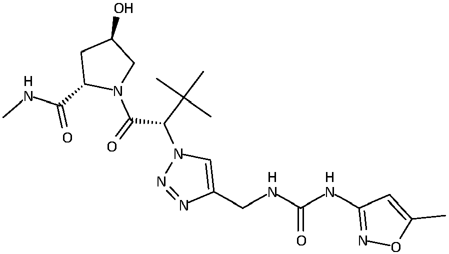 | (2S,4R)-1-[(2S)-3,3-dimethyl-2-[4-[[(5-methylisoxazol-3-yl)carbamoylamino]methyl ]triazol-1-yl]butanoyl]-4-hydroxy-N-methyl-pyrrolidine-2-carboxamide |
| 1057 | 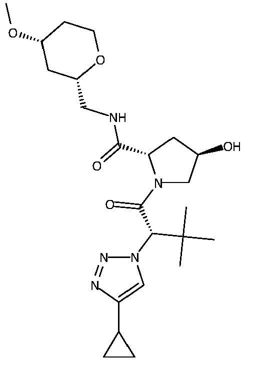 | (2S,4R)-1-[(2S)-2-(4-cyclopropyltriazol-1-yl)-3,3-dimethyl-butanoyl]-4-hydroxy-N-[[(2S,4R)-4-methoxytetrahydropyran-2-yl]methyl]pyrrolidine-2-carboxamide |
| 1058 | 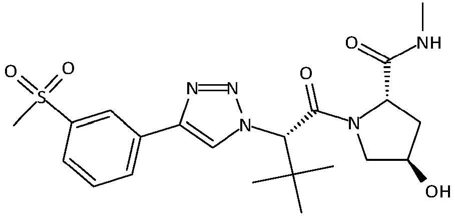 | (2S,4R)-1-[(2S)-3,3-dimethyl-2-[4-(3-methylsulfonylphenyl)triaz ol-1-yl]butanoyl]-4-hydroxy-N-methyl-pyrrolidine-2-carboxamide |
| 1059 | 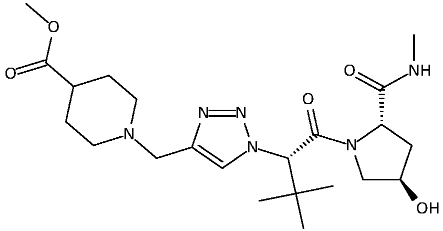 | methyl 1-[[1-[(1S)-1-[(2S,4R)-4-hydroxy-2-(methylcarbamoyl)pyrrolid ine-1-carbonyl]-2,2-dimethyl-propyl]triazol-4-yl]methyl]piperidine-4-carboxylate |
| 1060 | 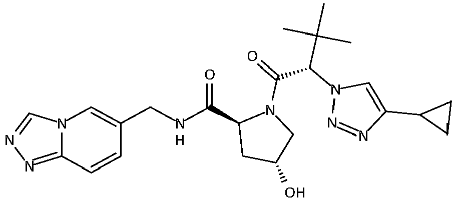 | (2S,4R)-1-[(2S)-2-(4-cyclopropyltriazol-1-yl)-3,3-dimethyl-butanoyl]-4-hydroxy-N-([1,2,4]triazolo[4,3-a]pyridin-6-ylmethyl)pyrrolidine-2-carboxamide |
| 1061 | 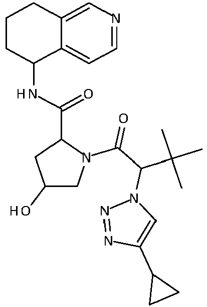 | (2S,4R)-1-[(2R)-2-(4-cyclopropyltriazol-1-yl)-3,3-dimethyl-butanoyl]-4-hydroxy-N-(5,6,7,8-tetrahydroisoquinolin-5-yl)pyrrolidine-2-carboxamide |
| 1062 | 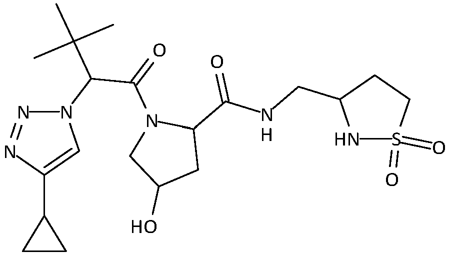 | (2S,4R)-1-[(2R)-2-(4-cyclopropyltriazol-1-yl)-3,3-dimethyl-butanoyl]-N-[(1,1-dioxo-1,2-thiazolidin-3-yl)methyl]-4-hydroxy-pyrrolidine-2-carboxamide |
| 1063 | 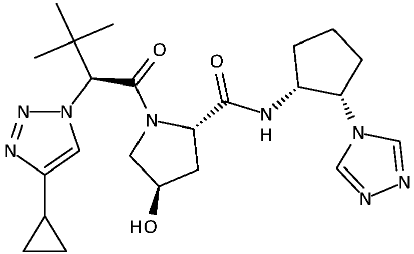 | (2S,4R)-1-[(2S)-2-(4-cyclopropyltriazol-1-yl)-3,3-dimethyl-butanoyl]-4-hydroxy-N-[(1R,2S)-2-(1,2,4-triazol-4-yl)cyclopentyl]pyrrolidine-2-carboxamide |
| 1064 | 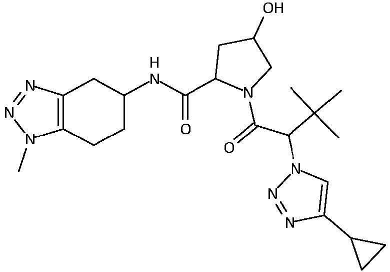 | (2R,4S)-1-[(2R)-2-(4-cyclopropyltriazol-1-yl)-3,3-dimethyl-butanoyl]-4-hydroxy-N-(1-methyl-4,5,6,7-tetrahydrobenzotriazol-5-yl)pyrrolidine-2-carboxamide |
| 1065 | 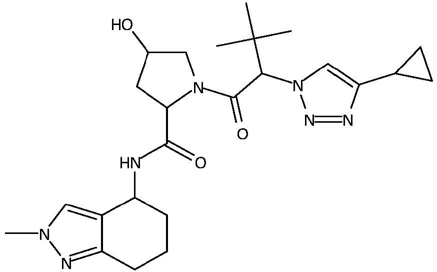 | (2R,4S)-1-[(2R)-2-(4-cyclopropyltriazol-1-yl)-3,3-dimethyl-butanoyl]-4-hydroxy-N-(2-methyl-4,5,6,7-tetrahydroindazol-4-yl)pyrrolidine-2-carboxamide |
| 1066 | 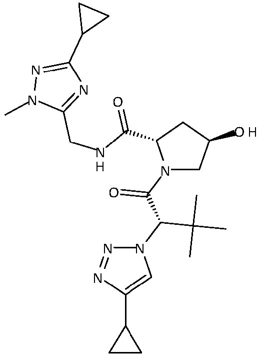 | (2S,4R)-N-[(5-cyclopropyl-2-methyl-1,2,4-triazol-3-yl)methyl]-1-[(2S)-2-(4-cyclopropyltriazol-1-yl)-3,3-dimethyl-butanoyl]-4-hydroxy-pyrrolidine-2-carboxamide |
| 1067 | 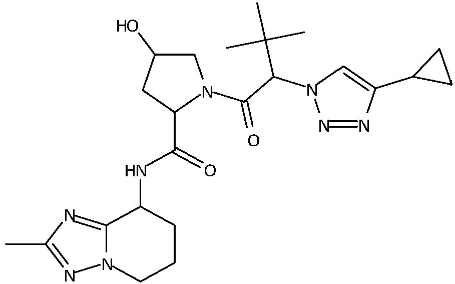 | (2R,4S)-1-[(2R)-2-(4-cyclopropyltriazol-1-yl)-3,3-dimethyl-butanoyl]-4-hydroxy-N-(2-methyl-5,6,7,8-tetrahydro-[1,2,4]triazolo[1,5-a]pyridin-8-yl)pyrrolidine-2-carboxamide |
| 1068 | 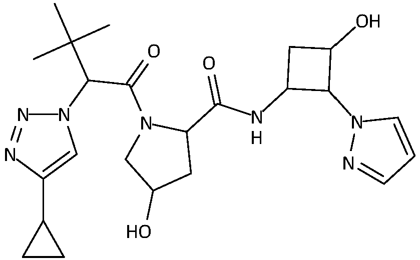 | (2S,4R)-1-[(2R)-2-(4-cyclopropyltriazol-1-yl)-3,3-dimethyl-butanoyl]-4-hydroxy-N-(3-hydroxy-2-pyrazol-1-yl-cyclobutyl)pyrrolidine-2-carboxamide |
| 1069 | 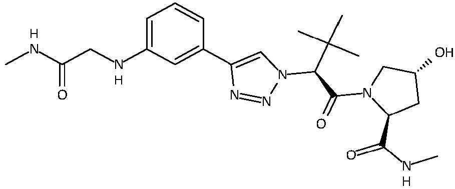 | (2S,4R)-1-[(2S)-3,3-dimethyl-2-[4-[3-[[2-(methylamino)-2-oxo-ethyl]amino]phenyl]triazol -1-yl]butanoyl]-4-hydroxy-N-methyl-pyrrolidine-2-carboxamide |
| 1070 | 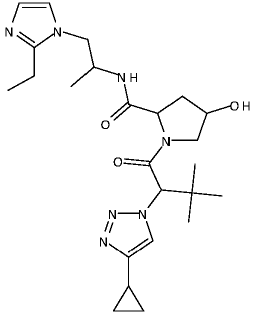 | (2R,4S)-1-[(2R)-2-(4-cyclopropyltriazol-1-yl)-3,3-dimethyl-butanoyl]-N-[2-(2-ethylimidazol-1-yl)-1-methyl-ethyl]-4-hydroxy-pyrrolidine-2-carboxamide |
| 1071 | 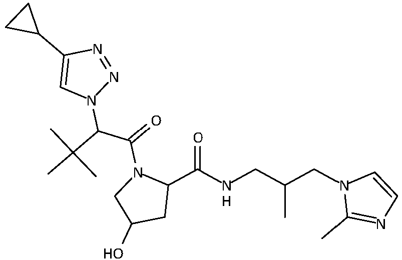 | (2R,4S)-1-[(2R)-2-(4-cyclopropyltriazol-1-yl)-3,3-dimethyl-butanoyl]-4-hydroxy-N-[2-methyl-3-(2-methylimidazol-1-yl)propyl]pyrrolidine-2-carboxamide |
| 1072 | 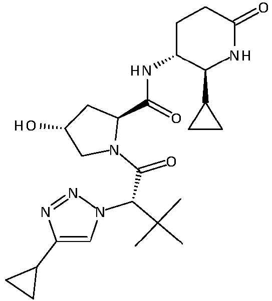 | (2S,4R)-N-[(2S,3R)-2-cyclopropyl-6-oxo-3-piperidyl]-1-[(2S)-2-(4-cyclopropyltriazol-1-yl)-3,3-dimethyl-butanoyl]-4-hydroxy-pyrrolidine-2-carboxamide |
| 1073 | 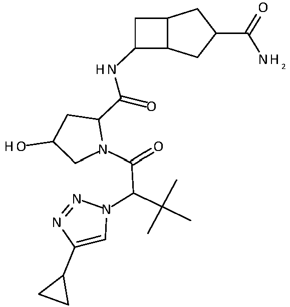 | (2S,4R)-N-[(1R,5R,6R)-3-carbamoyl-6-bicyclo[3.2.0]heptanyl]-1-[(2R)-2-(4-cyclopropyltriazol-1-yl)-3,3-dimethyl-butanoyl]-4-hydroxy-pyrrolidine-2-carboxamide |
| 1074 | 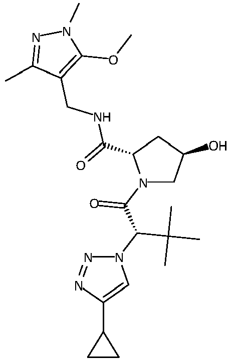 | (2S,4R)-1-[(2S)-2-(4-cyclopropyltriazol-1-yl)-3,3-dimethyl-butanoyl]-4-hydroxy-N-[(5-methoxy-1,3-dimethyl-pyrazol-4-yl)methyl]pyrrolidine-2-carboxamide |
| 1075 | 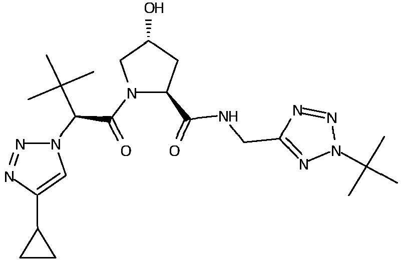 | (2S,4R)-N-[(2-tert-butyltetrazol-5-yl)methyl]-1-[(2S)-2-(4-cyclopropyltriazol-1-yl)-3,3-dimethyl-butanoyl]-4-hydroxy-pyrrolidine-2-carboxamide |
| 1076 | 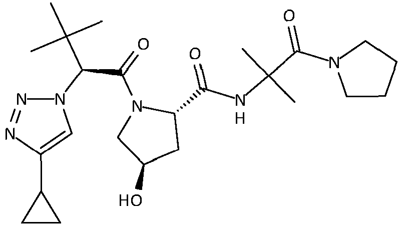 | (2S,4R)-1-[(2S)-2-(4-cyclopropyltriazol-1-yl)-3,3-dimethyl-butanoyl]-N-(1,1-dimethyl-2-oxo-2-pyrrolidin-1-yl-ethyl)-4-hydroxy-pyrrolidine-2-carboxamide |
| 1077 | 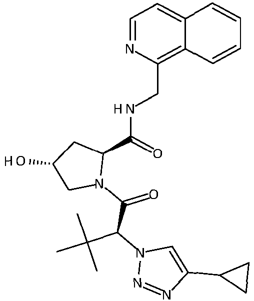 | (2S,4R)-1-[(2S)-2-(4-cyclopropyltriazol-1-yl)-3,3-dimethyl-butanoyl]-4-hydroxy-N-(1-isoquinolylmethyl)pyrrolid ine-2-carboxamide |
| 1078 | 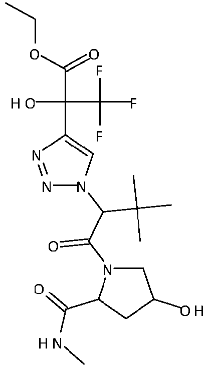 | ethyl 3,3,3-trifluoro-2-hydroxy-2-[1-[(1S)-1-[(2S,4R)-4-hydroxy-2-(methylcarbamoyl)pyrrolid ine-1-carbonyl]-2,2-dimethyl-propyl]triazol-4-yl]propanoate |
| 1079 | 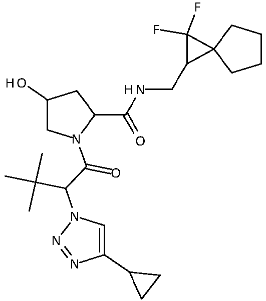 | (2S,4R)-1-[(2R)-2-(4-cyclopropyltriazol-1-yl)-3,3-dimethyl-butanoyl]-N-[(2,2-difluorospiro[2.4]heptan-1-yl)methyl]-4-hydroxy-pyrrolidine-2-carboxamide |
| 1080 | 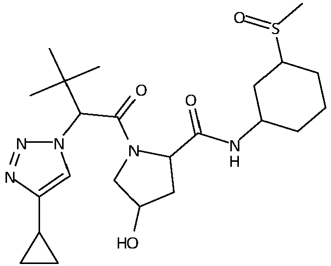 | (2R,4S)-1-[(2R)-2-(4-cyclopropyltriazol-1-yl)-3,3-dimethyl-butanoyl]-4-hydroxy-N-(3-methylsulfinylcyclohexyl) pyrrolidine-2-carboxamide |
| 1081 | 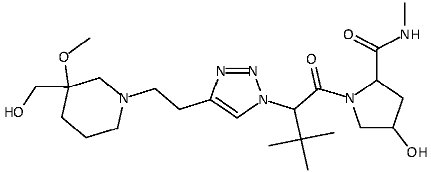 | (2R,4S)-4-hydroxy-1-[(2R)-2-[4-[2-[3-(hydroxymethyl)-3-methoxy-1-piperidyl]ethyl]triazol-1-yl]-3,3-dimethyl-butanoyl]-N-methyl-pyrrolidine-2-carboxamide |
| 1082 | 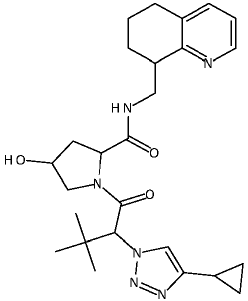 | (2S,4R)-1-[(2R)-2-(4-cyclopropyltriazol-1-yl)-3,3-dimethyl-butanoyl]-4-hydroxy-N-(5,6,7,8-tetrahydroquinolin-8-ylmethyl)pyrrolidine-2-carboxamide |
| 1083 | 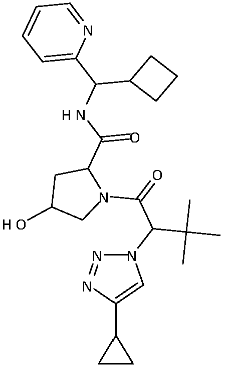 | (2S,4R)-N-[cyclobutyl(2-pyridyl)methyl]-1-[(2R)-2-(4-cyclopropyltriazol-1-yl)-3,3-dimethyl-butanoyl]-4-hydroxy-pyrrolidine-2-carboxamide |
| 1084 | 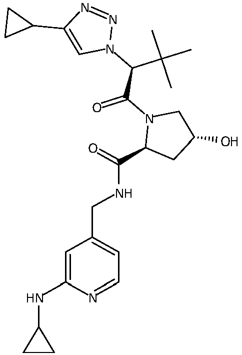 | (2S,4R)-N-[[2-(cyclopropylamino)-4-pyridyl]methyl]-1-[(2S)-2-(4-cyclopropyltriazol-1-yl)-3,3-dimethyl-butanoyl]-4-hydroxy-pyrrolidine-2-carboxamide |
| 1085 | 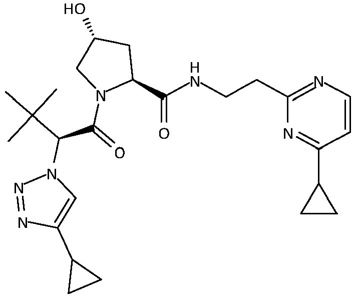 | (2S,4R)-N-[2-(4-cyclopropylpyrimidin-2-yl)ethyl]-1-[(2S)-2-(4-cyclopropyltriazol-1-yl)-3,3-dimethyl-butanoyl]-4-hydroxy-pyrrolidine-2-carboxamide |
| 1086 | 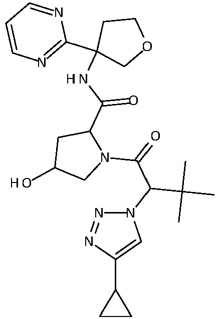 | (2S,4R)-1-[(2R)-2-(4-cyclopropyltriazol-1-yl)-3,3-dimethyl-butanoyl]-4-hydroxy-N-(3-pyrimidin-2-yltetrahydrofuran-3-yl)pyrrolidine-2-carboxamide |
| 1087 | 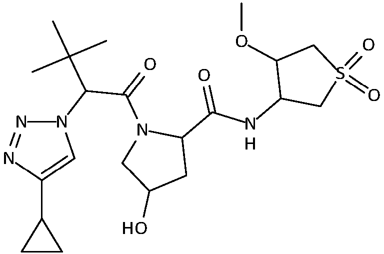 | (2R,4S)-1-[(2R)-2-(4-cyclopropyltriazol-1-yl)-3,3-dimethyl-butanoyl]-4-hydroxy-N-(4-methoxy-1,1-dioxo-thiolan-3-yl)pyrrolidine-2-carboxamide |
| 1088 | 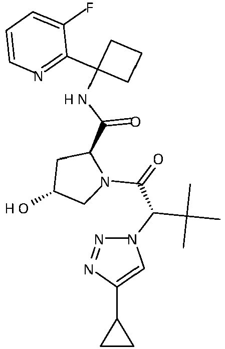 | (2S,4R)-1-[(2S)-2-(4-cyclopropyltriazol-1-yl)-3,3-dimethyl-butanoyl]-N-[1-(3-fluoro-2-pyridyl)cyclobutyl]-4-hydroxy-pyrrolidine-2-carboxamide |
| 1089 | 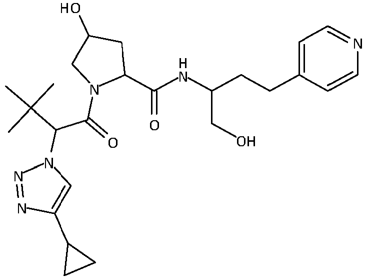 | (2S,4R)-1-[(2R)-2-(4-cyclopropyltriazol-1-yl)-3,3-dimethyl-butanoyl]-4-hydroxy-N-[1-(hydroxymethyl)-3-(4-pyridyl)propyl]pyrrolidine-2-carboxamide |
| 1090 | 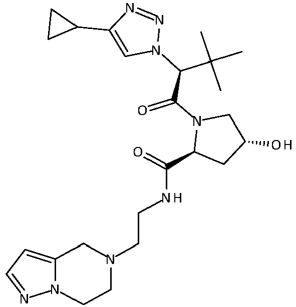 | (2S,4R)-1-[(2S)-2-(4-cyclopropyltriazol-1-yl)-3,3-dimethyl-butanoyl]-N-[2-(6,7-dihydro-4H-pyrazolo[1,5-a]pyrazin-5-yl)ethyl]-4-hydroxy-pyrrolidine-2-carboxamide |
| 1091 | 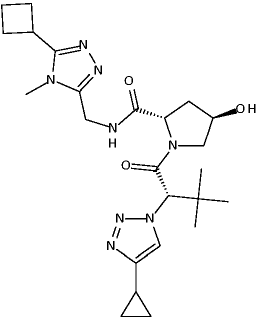 | (2S,4R)-N-[(5-cyclobutyl-4-methyl-1,2,4-triazol-3-yl)methyl]-1-[(2S)-2-(4-cyclopropyltriazol-1-yl)-3,3-dimethyl-butanoyl]-4-hydroxy-pyrrolidine-2-carboxamide |
| 1092 | 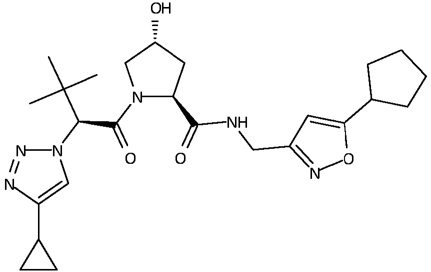 | (2S,4R)-N-[(5-cyclopentylisoxazol-3-yl)methyl]-1-[(2S)-2-(4-cyclopropyltriazol-1-yl)-3,3-dimethyl-butanoyl]-4-hydroxy-pyrrolidine-2-carboxamide |
| 1093 | 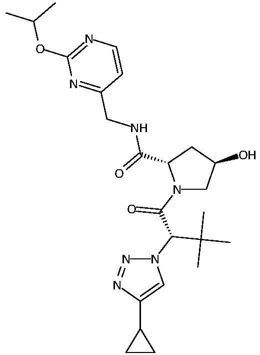 | (2S,4R)-1-[(2S)-2-(4-cyclopropyltriazol-1-yl)-3,3-dimethyl-butanoyl]-4-hydroxy-N-[(2-isopropoxypyrimidin-4-yl)methyl]pyrrolidine-2-carboxamide |
| 1094 | 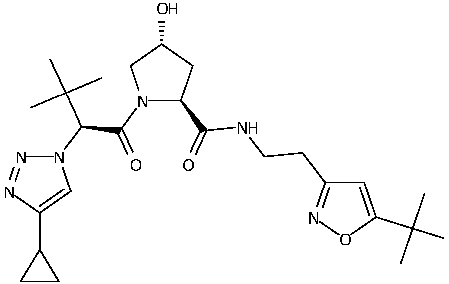 | (2S,4R)-N-[2-(5-tert-butylisoxazol-3-yl)ethyl]-1-[(2S)-2-(4-cyclopropyltriazol-1-yl)-3,3-dimethyl-butanoyl]-4-hydroxy-pyrrolidine-2-carboxamide |
| 1095 | 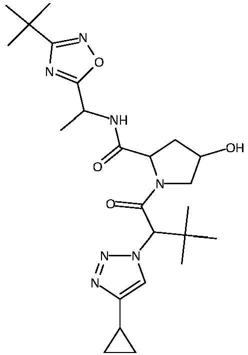 | (2R,4S)-N-[1-(3-tert-butyl-1,2,4-oxadiazol-5-yl)ethyl]-1-[(2R)-2-(4-cyclopropyltriazol-1-yl)-3,3-dimethyl-butanoyl]-4-hydroxy-pyrrolidine-2-carboxamide |
| 1096 | 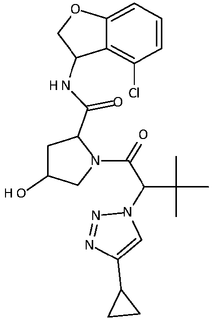 | (2S,4R)-N-(4-chloro-2,3-dihydrobenzofuran-3-yl)-1-[(2R)-2-(4-cyclopropyltriazol-1-yl)-3,3-dimethyl-butanoyl]-4-hydroxy-pyrrolidine-2-carboxamide |
| 1097 | 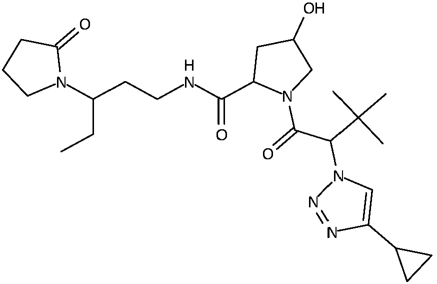 | (2R,4S)-1-[(2R)-2-(4-cyclopropyltriazol-1-yl)-3,3-dimethyl-butanoyl]-4-hydroxy-N-[3-(2-oxopyrrolidin-1-yl)pentyl]pyrrolidine-2-carboxamide |
| 1098 | 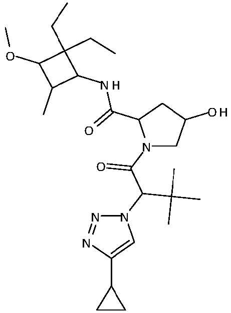 | (2R,4S)-1-[(2R)-2-(4-cyclopropyltriazol-1-yl)-3,3-dimethyl-butanoyl]-N-(2,2-diethyl-3-methoxy-4-methyl-cyclobutyl)-4-hydroxy-pyrrolidine-2-carboxamide |
| 1099 | 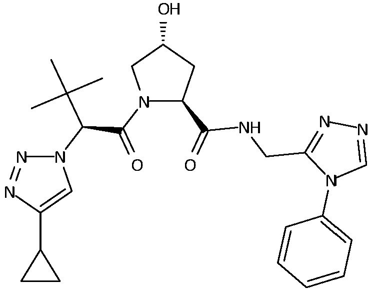 | (2S,4R)-1-[(2S)-2-(4-cyclopropyltriazol-1-yl)-3,3-dimethyl-butanoyl]-4-hydroxy-N-[(4-phenyl-1,2,4-triazol-3-yl)methyl]pyrrolidine-2-carboxamide |
| 1100 | 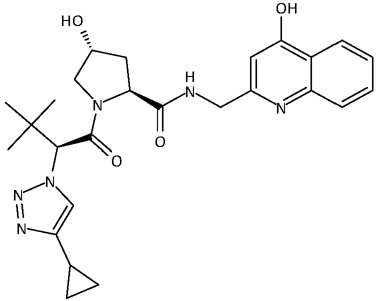 | (2S,4R)-1-[(2S)-2-(4-cyclopropyltriazol-1-yl)-3,3-dimethyl-butanoyl]-4-hydroxy-N-[(4-hydroxy-2-quinolyl)methyl]pyrrolidin e-2-carboxamide |
| 1101 | 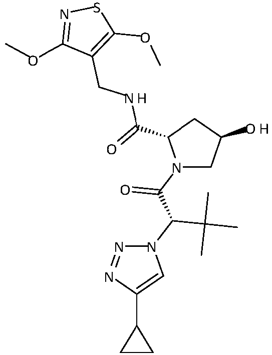 | (2S,4R)-1-[(2S)-2-(4-cyclopropyltriazol-1-yl)-3,3-dimethyl-butanoyl]-N-[(3,5-dimethoxyisothiazol-4-yl)methyl]-4-hydroxy-pyrrolidine-2-carboxamide |
| 1102 | 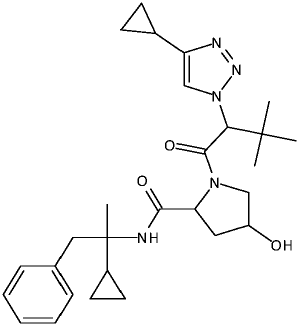 | (2S,4R)-N-(1-cyclopropyl-1-methyl-2-phenyl-ethyl)-1-[(2R)-2-(4-cyclopropyltriazol-1-yl)-3,3-dimethyl-butanoyl]-4-hydroxy-pyrrolidine-2-carboxamide |
| 1103 | 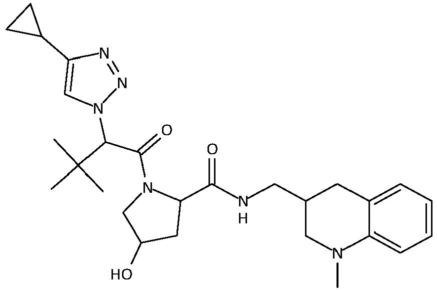 | (2R,4S)-1-[(2R)-2-(4-cyclopropyltriazol-1-yl)-3,3-dimethyl-butanoyl]-4-hydroxy-N-[(1-methyl-3,4-dihydro-2H-quinolin-3-yl)methyl]pyrrolidine-2-carboxamide |
| 1104 | 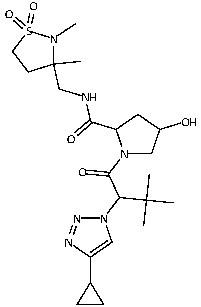 | (2R,4S)-1-[(2R)-2-(4-cyclopropyltriazol-1-yl)-3,3-dimethyl-butanoyl]-N-[(2,3-dimethyl-1,1-dioxo-1,2-thiazolidin-3-yl)methyl]-4-hydroxy-pyrrolidine-2-carboxamide |
| 1105 | 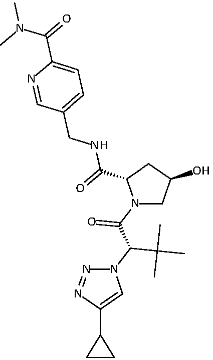 | 5-[[[(2S,4R)-1-[(2S)-2-(4-cyclopropyltriazol-1-yl)-3,3-dimethyl-butanoyl]-4-hydroxy-pyrrolidine-2-carbonyl]amino]methyl]-N,N-dimethyl-pyridine-2-carboxamide |
| 1106 | 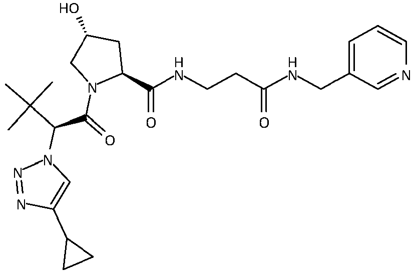 | (2S,4R)-1-[(2S)-2-(4-cyclopropyltriazol-1-yl)-3,3-dimethyl-butanoyl]-4-hydroxy-N-[3-oxo-3-(3-pyridylmethylamino)propy l]pyrrolidine-2-carboxamide |
| 1107 | 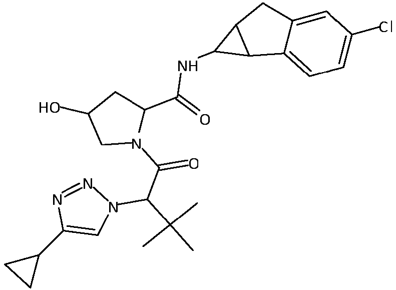 | (2S,4R)-N-(4-chloro-1,1a,6,6a-tetrahydrocyclopropa[a]ind en-1-yl)-1-[(2R)-2-(4-cyclopropyltriazol-1-yl)-3,3-dimethyl-butanoyl]-4-hydroxy-pyrrolidine-2-carboxamide |
| 1108 | 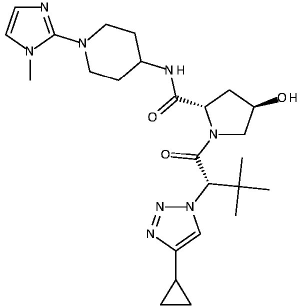 | (2S,4R)-1-[(2S)-2-(4-cyclopropyltriazol-1-yl)-3,3-dimethyl-butanoyl]-4-hydroxy-N-[1-(1-methylimidazol-2-yl)-4-piperidyl]pyrrolidine-2-carboxamide |
| 1109 | 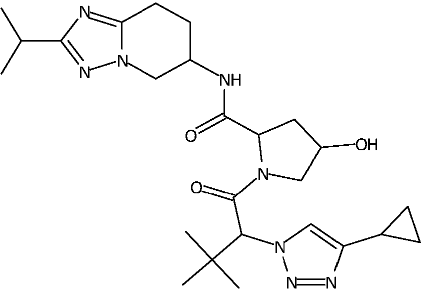 | (2R,4S)-1-[(2R)-2-(4-cyclopropyltriazol-1-yl)-3,3-dimethyl-butanoyl]-4-hydroxy-N-(2-isopropyl-5,6,7,8-tetrahydro-[1,2,4]triazolo[1,5-a]pyridin-6-yl)pyrrolidine-2-carboxamide |
| 1110 | 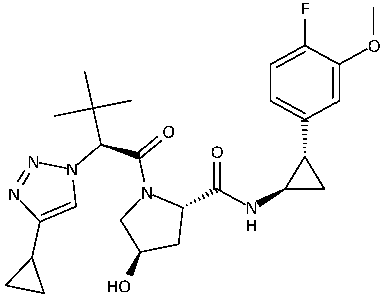 | (2S,4R)-1-[(2S)-2-(4-cyclopropyltriazol-1-yl)-3,3-dimethyl-butanoyl]-N-[(1R,2S)-2-(4-fluoro-3-methoxy-phenyl)cyclopropyl]-4-hydroxy-pyrrolidine-2-carboxamide |
| 1111 | 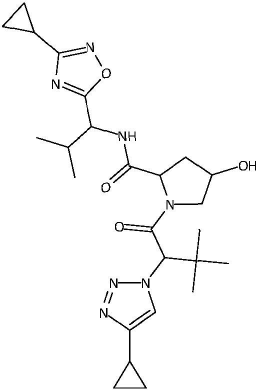 | (2R,4S)-N-[1-(3-cyclopropyl-1,2,4-oxadiazol-5-yl)-2-methyl-propyl]-1-[(2R)-2-(4-cyclopropyltriazol-1-yl)-3,3-dimethyl-butanoyl]-4-hydroxy-pyrrolidine-2-carboxamide |
| 1112 | 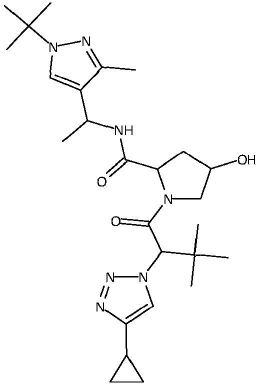 | (2R,4S)-N-[1-(1-tert-butyl-3-methyl-pyrazol-4-yl)ethyl]-1-[(2R)-2-(4-cyclopropyltriazol-1-yl)-3,3-dimethyl-butanoyl]-4-hydroxy-pyrrolidine-2-carboxamide |
| 1113 | 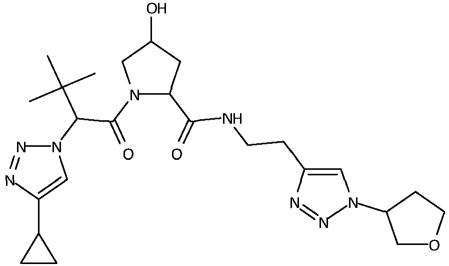 | (2S,4R)-1-[(2R)-2-(4-cyclopropyltriazol-1-yl)-3,3-dimethyl-butanoyl]-4-hydroxy-N-[2-(1-tetrahydrofuran-3-yltriazol-4-yl)ethyl]pyrrolidine-2-carboxamide |
| 1114 | 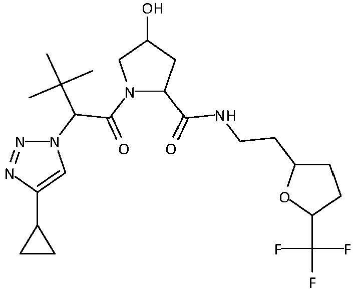 | (2S,4R)-1-[(2R)-2-(4-cyclopropyltriazol-1-yl)-3,3-dimethyl-butanoyl]-4-hydroxy-N-[2-[5-(trifluoromethyl)tetrahydro furan-2-yl]ethyl]pyrrolidine-2-carboxamide |
| 1115 | 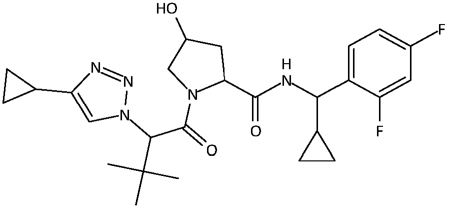 | (2S,4R)-N-[cyclopropyl-(2,4-difluorophenyl)methyl]-1-[(2R)-2-(4-cyclopropyltriazol-1-yl)-3,3-dimethyl-butanoyl]-4-hydroxy-pyrrolidine-2-carboxamide |
| 1116 | 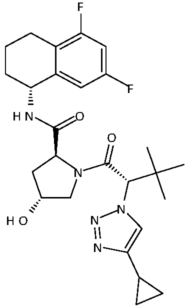 | (2S,4R)-1-[(2S)-2-(4-cyclopropyltriazol-1-yl)-3,3-dimethyl-butanoyl]-N-[(1R)-5,7-difluorotetralin-1-yl]-4-hydroxy-pyrrolidine-2-carboxamide |
| 1117 | 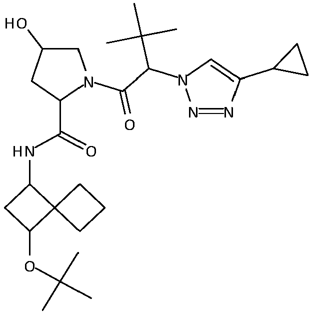 | (2R,4S)-N-(3-tert-butoxyspiro[3.3]heptan-1-yl)-1-[(2R)-2-(4-cyclopropyltriazol-1-yl)-3,3-dimethyl-butanoyl]-4-hydroxy-pyrrolidine-2-carboxamide |
| 1118 | 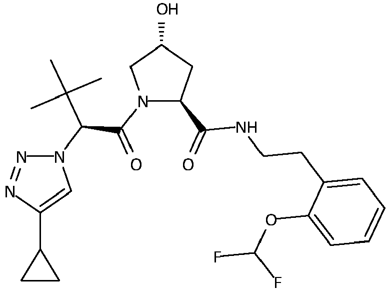 | (2S,4R)-1-[(2S)-2-(4-cyclopropyltriazol-1-yl)-3,3-dimethyl-butanoyl]-N-[2-[2-(difluoromethoxy)phenyl]e thyl]-4-hydroxy-pyrrolidine-2-carboxamide |
| 1119 | 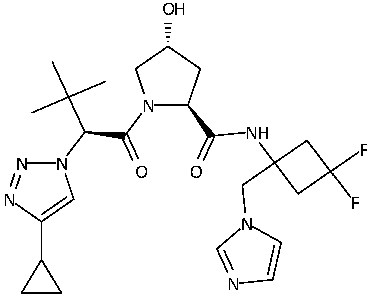 | (2S,4R)-1-[(2S)-2-(4-cyclopropyltriazol-1-yl)-3,3-dimethyl-butanoyl]-N-[3,3-difluoro-1-(imidazol-1-ylmethyl)cyclobutyl]-4-hydroxy-pyrrolidine-2-carboxamide |
| 1120 | 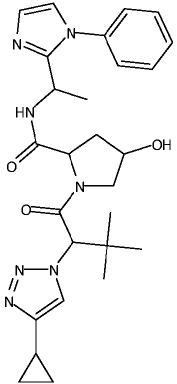 | (2R,4S)-1-[(2R)-2-(4-cyclopropyltriazol-1-yl)-3,3-dimethyl-butanoyl]-4-hydroxy-N-[1-(1-phenylimidazol-2-yl)ethyl]pyrrolidine-2-carboxamide |
| 1121 | 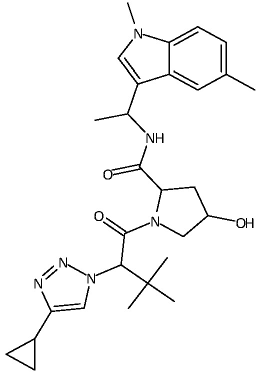 | (2R,4S)-1-[(2R)-2-(4-cyclopropyltriazol-1-yl)-3,3-dimethyl-butanoyl]-N-[1-(1,5-dimethylindol-3-yl)ethyl]-4-hydroxy-pyrrolidine-2-carboxamide |
| 1122 | 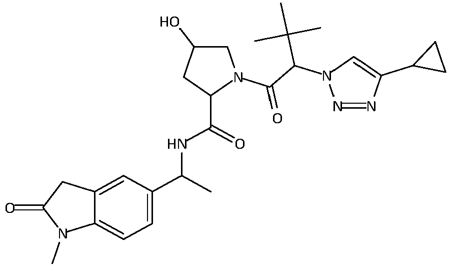 | (2R,4S)-1-[(2R)-2-(4-cyclopropyltriazol-1-yl)-3,3-dimethyl-butanoyl]-4-hydroxy-N-[1-(1-methyl-2-oxo-indolin-5-yl)ethyl]pyrrolidine-2-carboxamide |
| 1123 | 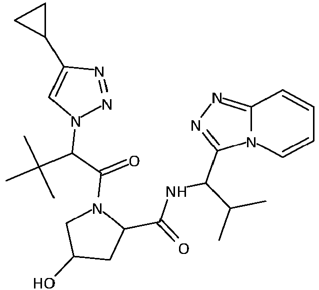 | (2R,4S)-1-[(2R)-2-(4-cyclopropyltriazol-1-yl)-3,3-dimethyl-butanoyl]-4-hydroxy-N-[2-methyl-1-([1,2,4]triazolo[4,3-a]pyridin-3-yl)propyl]pyrrolidine-2-carboxamide |
| 1124 | 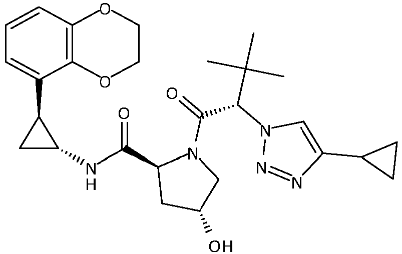 | (2S,4R)-1-[(2S)-2-(4-cyclopropyltriazol-1-yl)-3,3-dimethyl-butanoyl]-N-[(1R,2S)-2-(2,3-dihydro-1,4-benzodioxin-5-yl)cyclopropyl]-4-hydroxy-pyrrolidine-2-carboxamide |
| 1125 | 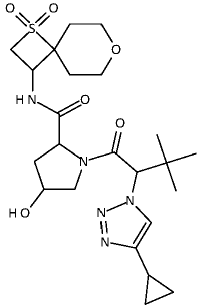 | (2S,4R)-1-[(2R)-2-(4-cyclopropyltriazol-1-yl)-3,3-dimethyl-butanoyl]-N-(1,1-dioxo-7-oxa-1lambda6-thiaspiro[3.5]nonan-3-yl)-4-hydroxy-pyrrolidine-2-carboxamide |
| 1126 | 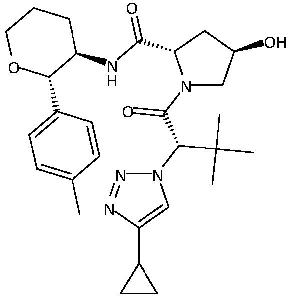 | (2S,4R)-1-[(2S)-2-(4-cyclopropyltriazol-1-yl)-3,3-dimethyl-butanoyl]-4-hydroxy-N-[(2S,3R)-2-(p-tolyl)tetrahydropyran-3-yl]pyrrolidine-2-carboxamide |
| 1127 | 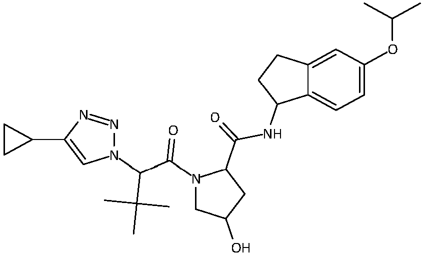 | (2R,4S)-1-[(2R)-2-(4-cyclopropyltriazol-1-yl)-3,3-dimethyl-butanoyl]-4-hydroxy-N-(5-isopropoxyindan-1-yl)pyrrolidine-2-carboxamide |
| 1128 | 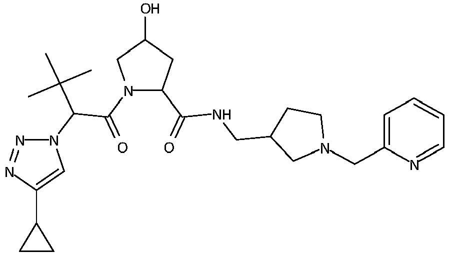 | (2S,4R)-1-[(2R)-2-(4-cyclopropyltriazol-1-yl)-3,3-dimethyl-butanoyl]-4-hydroxy-N-[[1-(2-pyridylmethyl)pyrrolidin-3-yl]methyl]pyrrolidine-2-carboxamide |
| 1129 | 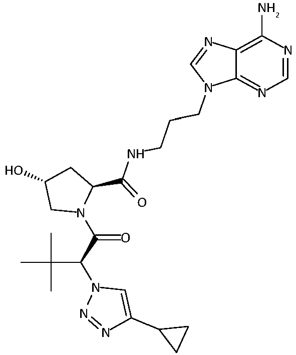 | (2S,4R)-N-[3-(6-aminopurin-9-yl)propyl]-1-[(2S)-2-(4-cyclopropyltriazol-1-yl)-3,3-dimethyl-butanoyl]-4-hydroxy-pyrrolidine-2-carboxamide |
| 1130 | 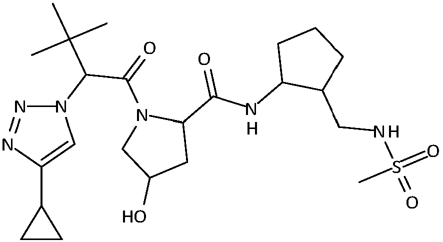 | (2S,4R)-1-[(2R)-2-(4-cyclopropyltriazol-1-yl)-3,3-dimethyl-butanoyl]-4-hydroxy-N-[2-(methanesulfonamidometh yl)cyclopentyl]pyrrolidine-2-carboxamide |
| 1131 | 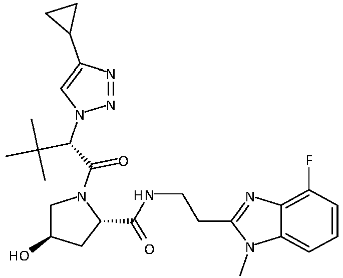 | (2S,4R)-1-[(2S)-2-(4-cyclopropyltriazol-1-yl)-3,3-dimethyl-butanoyl]-N-[2-(4-fluoro-1-methyl-benzimidazol-2-yl)ethyl]-4-hydroxy-pyrrolidine-2-carboxamide |
| 1132 | 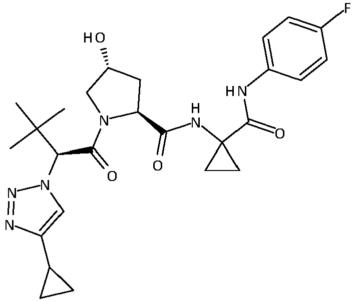 | (2S,4R)-1-[(2S)-2-(4-cyclopropyltriazol-1-yl)-3,3-dimethyl-butanoyl]-N-[1-[(4-fluorophenyl)carbamoyl]c yclopropyl]-4-hydroxy-pyrrolidine-2-carboxamide |
| 1133 | 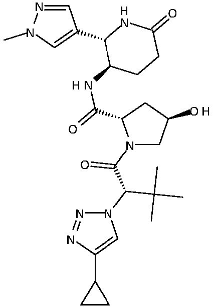 | (2S,4R)-1-[(2S)-2-(4-cyclopropyltriazol-1-yl)-3,3-dimethyl-butanoyl]-4-hydroxy-N-[(2S,3R)-2-(1-methylpyrazol-4-yl)-6-oxo-3-piperidyl]pyrrolidine-2-carboxamide |
| 1134 | 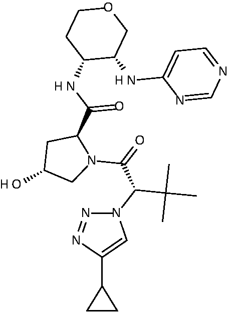 | (2S,4R)-1-[(2S)-2-(4-cyclopropyltriazol-1-yl)-3,3-dimethyl-butanoyl]-4-hydroxy-N-[(3R,4R)-3-(pyrimidin-4-ylamino)tetrahydropyran-4-yl]pyrrolidine-2-carboxamide |
| 1135 | 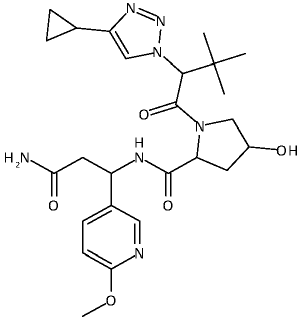 | (2R,4S)-N-[3-amino-1-(6-methoxy-3-pyridyl)-3-oxo-propyl]-1-[(2R)-2-(4-cyclopropyltriazol-1-yl)-3,3-dimethyl-butanoyl]-4-hydroxy-pyrrolidine-2-carboxamide |
| 1136 | 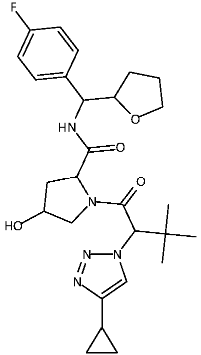 | (2S,4R)-1-[(2R)-2-(4-cyclopropyltriazol-1-yl)-3,3-dimethyl-butanoyl]-N-[(4-fluorophenyl)-tetrahydrofuran-2-yl-methyl]-4-hydroxy-pyrrolidine-2-carboxamide |
| 1137 | 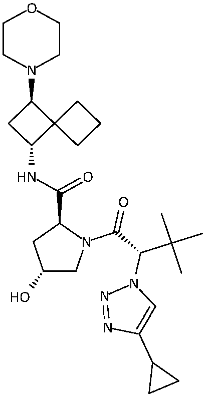 | (2S,4R)-1-[(2S)-2-(4-cyclopropyltriazol-1-yl)-3,3-dimethyl-butanoyl]-4-hydroxy-N-[(1R,3R)-3-morpholinospiro[3.3]hepta n-1-yl]pyrrolidine-2-carboxamide |
| 1138 | 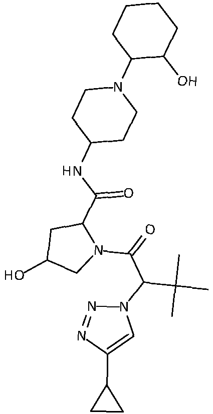 | (2S,4R)-1-[(2R)-2-(4-cyclopropyltriazol-1-yl)-3,3-dimethyl-butanoyl]-4-hydroxy-N-[1-(2-hydroxycyclohexyl)-4-piperidyl]pyrrolidine-2-carboxamide |
| 1139 | 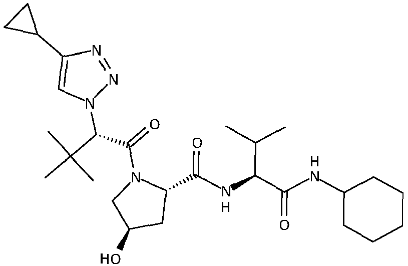 | (2S,4R)-N-[(1S)-1-(cyclohexylcarbamoyl)-2-methyl-propyl]-1-[(2S)-2-(4-cyclopropyltriazol-1-yl)-3,3-dimethyl-butanoyl]-4-hydroxy-pyrrolidine-2-carboxamide |
| 1140 | 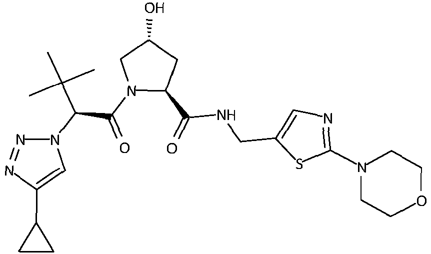 | (2S,4R)-1-[(2S)-2-(4-cyclopropyltriazol-1-yl)-3,3-dimethyl-butanoyl]-4-hydroxy-N-[(2-morpholinothiazol-5-yl)methyl]pyrrolidine-2-carboxamide |
| 1141 | 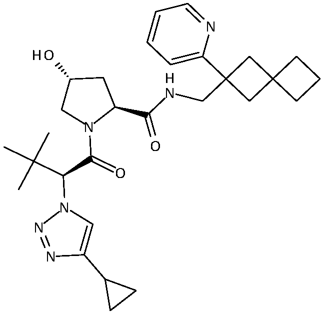 | (2S,4R)-1-[(2S)-2-(4-cyclopropyltriazol-1-yl)-3,3-dimethyl-butanoyl]-4-hydroxy-N-[[2-(2-pyridyl)spiro[3.3]heptan-2-yl]methyl]pyrrolidine-2-carboxamide |
| 1142 | 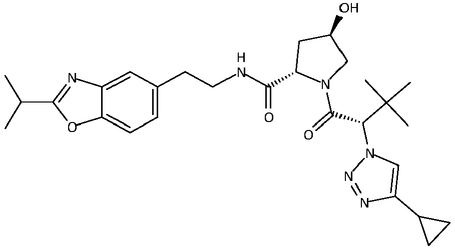 | (2S,4R)-1-[(2S)-2-(4-cyclopropyltriazol-1-yl)-3,3-dimethyl-butanoyl]-4-hydroxy-N-[2-(2-isopropyl-1,3-benzoxazol-5-yl)ethyl]pyrrolidine-2-carboxamide |
| 1143 | 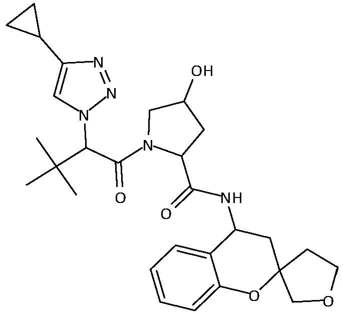 | (2S,4R)-1-[(2R)-2-(4-cyclopropyltriazol-1-yl)-3,3-dimethyl-butanoyl]-4-hydroxy-N-spiro[chromane-2,3'-tetrahydrofuran]-4-yl-pyrrolidine-2-carboxamide |
| 1144 | 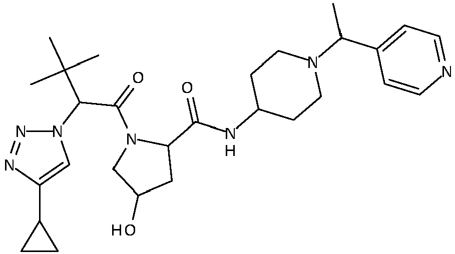 | (2R,4S)-1-[(2R)-2-(4-cyclopropyltriazol-1-yl)-3,3-dimethyl-butanoyl]-4-hydroxy-N-[1-[1-(4-pyridyl)ethyl]-4-piperidyl]pyrrolidine-2-carboxamide |
| 1145 | 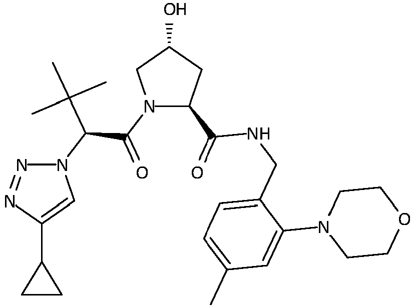 | (2S,4R)-1-[(2S)-2-(4-cyclopropyltriazol-1-yl)-3,3-dimethyl-butanoyl]-4-hydroxy-N-[(4-methyl-2-morpholino-phenyl)methyl]pyrrolidine-2-carboxamide |
| 1146 | 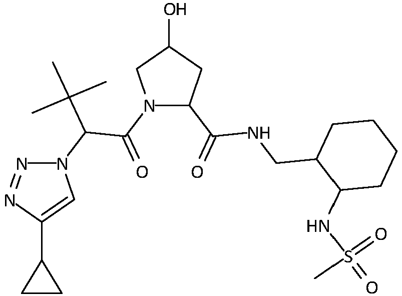 | (2S,4R)-1-[(2R)-2-(4-cyclopropyltriazol-1-yl)-3,3-dimethyl-butanoyl]-4-hydroxy-N-[[2-(methanesulfonamido) cyclohexyl]methyl]pyrrolidine-2-carboxamide |
| 1147 | 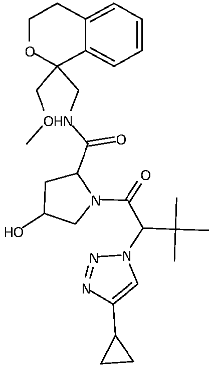 | (2R,4S)-1-[(2R)-2-(4-cyclopropyltriazol-1-yl)-3,3-dimethyl-butanoyl]-4-hydroxy-N-[[1-(methoxymethyl)isochroman -1-yl]methyl]pyrrolidine-2-carboxamide |
| 1148 | 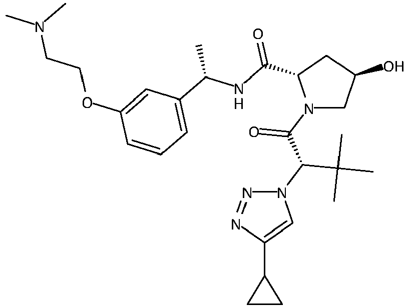 | (2S,4R)-1-[(2S)-2-(4-cyclopropyltriazol-1-yl)-3,3-dimethyl-butanoyl]-N-[(1S)-1-[3-[2-(dimethylamino)ethoxy] phenyl]ethyl]-4-hydroxy-pyrrolidine-2-carboxamide |
| 1149 | 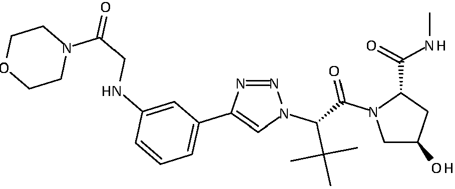 | (2S,4R)-1-[(2S)-3,3-dimethyl-2-[4-[3-[(2-morpholino-2-oxoethyl)amino]phenyl]triazol -1-yl]butanoyl]-4-hydroxy-N-methyl-pyrrolidine-2-carboxamide |
| 1150 | 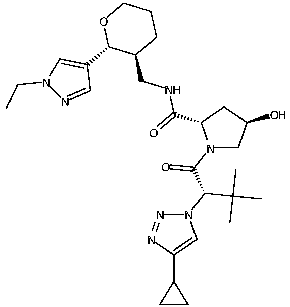 | (2S,4R)-1-[(2S)-2-(4-cyclopropyltriazol-1-yl)-3,3-dimethyl-butanoyl]-N-[[(2R,3S)-2-(1-ethylpyrazol-4-yl)tetrahydropyran-3-yl]methyl]-4-hydroxy-pyrrolidine-2-carboxamide |
| 1151 | 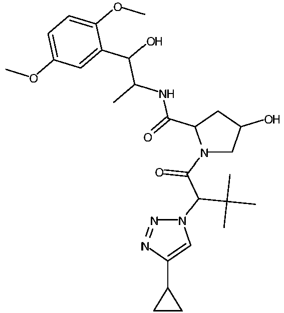 | (2R,4S)-1-[(2R)-2-(4-cyclopropyltriazol-1-yl)-3,3-dimethyl-butanoyl]-N-[2-(2,5-dimethoxyphenyl)-2-hydroxy-1-methyl-ethyl]-4-hydroxy-pyrrolidine-2-carboxamide |
| 1152 | 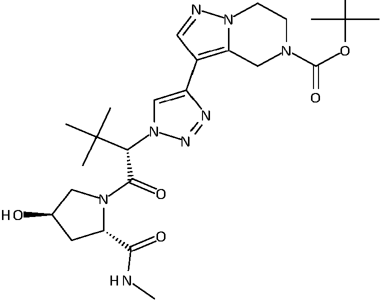 | tert-butyl 3-[1-[(1S)-1-[(2S,4R)-4-hydroxy-2-(methylcarbamoyl)pyrrolid ine-1-carbonyl]-2,2-dimethyl-propyl]triazol-4-yl]-6,7-dihydro-4H-pyrazolo[1,5-a]pyrazine-5-carboxylate |
| 1153 | 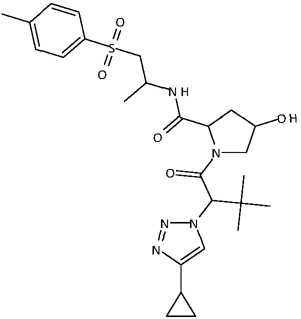 | (2R,4S)-1-[(2R)-2-(4-cyclopropyltriazol-1-yl)-3,3-dimethyl-butanoyl]-4-hydroxy-N-[1-methyl-2-(p-tolylsulfonyl)ethyl]pyrroli dine-2-carboxamide |
| 1154 | 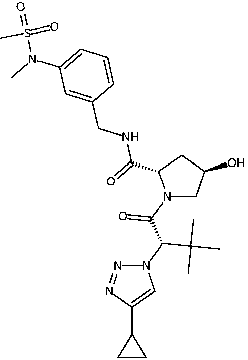 | (2S,4R)-1-[(2S)-2-(4-cyclopropyltriazol-1-yl)-3,3-dimethyl-butanoyl]-4-hydroxy-N-[[3-[methyl(methylsulfonyl) amino]phenyl]methyl]pyrro lidine-2-carboxamide |
| 1155 | 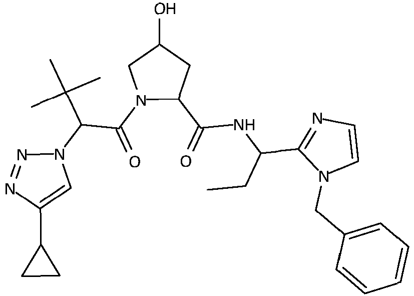 | (2R,4S)-N-[1-(1-benzylimidazol-2-yl)propyl]-1-[(2R)-2-(4-cyclopropyltriazol-1-yl)-3,3-dimethyl-butanoyl]-4-hydroxy-pyrrolidine-2-carboxamide |
| 1156 | 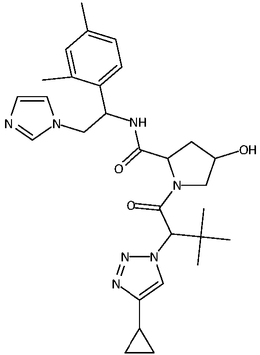 | (2R,4S)-1-[(2R)-2-(4-cyclopropyltriazol-1-yl)-3,3-dimethyl-butanoyl]-N-[1-(2,4-dimethylphenyl)-2-imidazol-1-yl-ethyl]-4-hydroxy-pyrrolidine-2-carboxamide |
| 1157 | 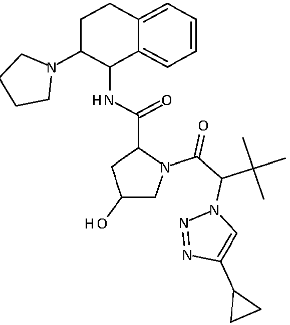 | (2S,4R)-1-[(2R)-2-(4-cyclopropyltriazol-1-yl)-3,3-dimethyl-butanoyl]-4-hydroxy-N-(2-pyrrolidin-1-yltetralin-1-yl)pyrrolidine-2-carboxamide |
| 1158 | 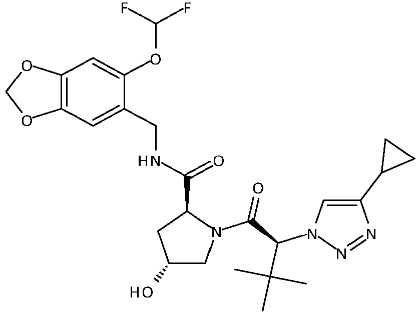 | (2S,4R)-1-[(2S)-2-(4-cyclopropyltriazol-1-yl)-3,3-dimethyl-butanoyl]-N-[[6-(difluoromethoxy)-1,3-benzodioxol-5-yl]methyl]-4-hydroxy-pyrrolidine-2-carboxamide |
| 1159 | 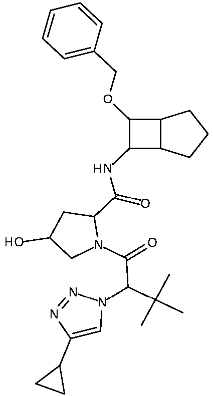 | (2S,4R)-N-(7-benzyloxy-6-bicyclo[3.2.0]heptanyl)-1-[(2R)-2-(4-cyclopropyltriazol-1-yl)-3,3-dimethyl-butanoyl]-4-hydroxy-pyrrolidine-2-carboxamide |
| 1160 | 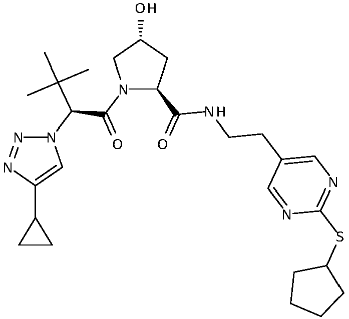 | (2S,4R)-N-[2-(2-cyclopentylsulfanylpyrimi din-5-yl)ethyl]-1-[(2S)-2-(4-cyclopropyltriazol-1-yl)-3,3-dimethyl-butanoyl]-4-hydroxy-pyrrolidine-2-carboxamide |
| 1161 | 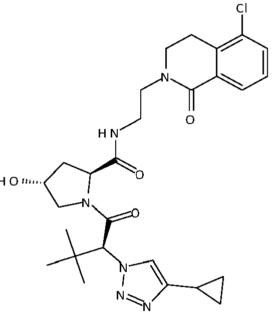 | (2S,4R)-N-[2-(5-chloro-1-oxo-3,4-dihydroisoquinolin-2-yl)ethyl]-1-[(2S)-2-(4-cyclopropyltriazol-1-yl)-3,3-dimethyl-butanoyl]-4-hydroxy-pyrrolidine-2-carboxamide |
| 1162 | 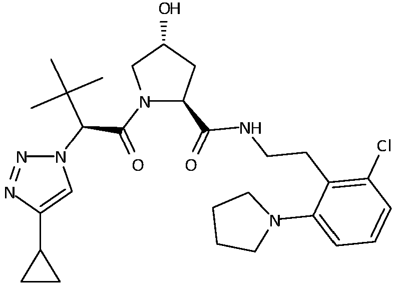 | (2S,4R)-N-[2-(2-chloro-6-pyrrolidin-1-yl-phenyl)ethyl]-1-[(2S)-2-(4-cyclopropyltriazol-1-yl)-3,3-dimethyl-butanoyl]-4-hydroxy-pyrrolidine-2-carboxamide |
| 1163 | 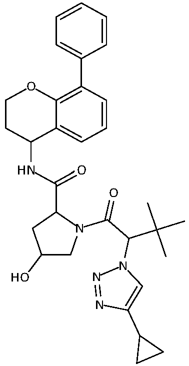 | (2S,4R)-1-[(2R)-2-(4-cyclopropyltriazol-1-yl)-3,3-dimethyl-butanoyl]-4-hydroxy-N-(8-phenylchroman-4-yl)pyrrolidine-2-carboxamide |
| 1164 | 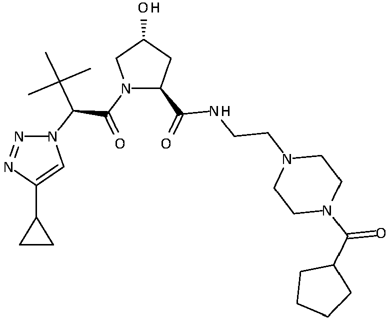 | (2S,4R)-N-[2-[4-(cyclopentanecarbonyl)pip erazin-1-yl]ethyl]-1-[(2S)-2-(4-cyclopropyltriazol-1-yl)-3,3-dimethyl-butanoyl]-4-hydroxy-pyrrolidine-2-carboxamide |
| 1165 | 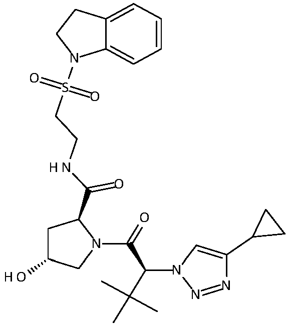 | (2S,4R)-1-[(2S)-2-(4-cyclopropyltriazol-1-yl)-3,3-dimethyl-butanoyl]-4-hydroxy-N-(2-indolin-1-ylsulfonylethyl)pyrrolidine -2-carboxamide |
| 1166 | 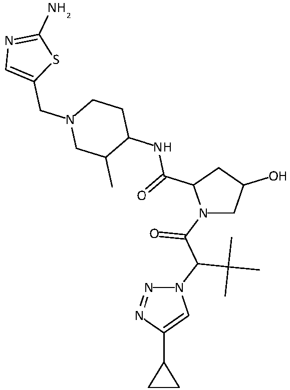 | (2R,4S)-N-[1-[(2-aminothiazol-5-yl)methyl]-3-methyl-4-piperidyl]-1-[(2R)-2-(4-cyclopropyltriazol-1-yl)-3,3-dimethyl-butanoyl]-4-hydroxy-pyrrolidine-2-carboxamide |
| 1167 | 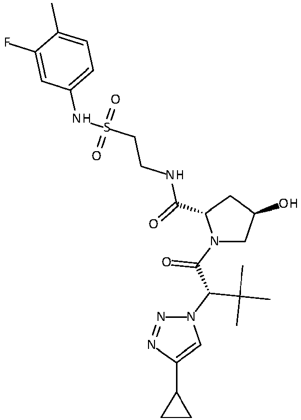 | (2S,4R)-1-[(2S)-2-(4-cyclopropyltriazol-1-yl)-3,3-dimethyl-butanoyl]-N-[2-[(3-fluoro-4-methyl-phenyl)sulfamoyl]ethyl]-4-hydroxy-pyrrolidine-2-carboxamide |
| 1168 | 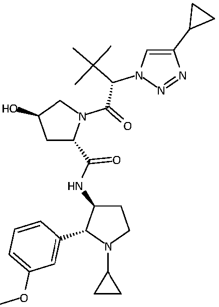 | (2S,4R)-N-[(2R,3S)-1-cyclopropyl-2-(3-methoxyphenyl)pyrrolidin-3-yl]-1-[(2S)-2-(4-cyclopropyltriazol-1-yl)-3,3-dimethyl-butanoyl]-4-hydroxy-pyrrolidine-2-carboxamide |
| 1169 | 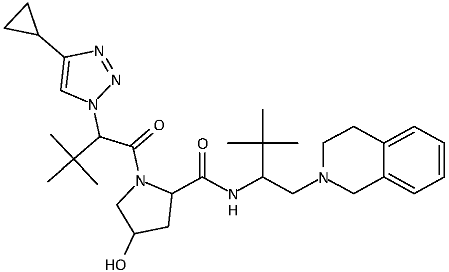 | (2R,4S)-1-[(2R)-2-(4-cyclopropyltriazol-1-yl)-3,3-dimethyl-butanoyl]-N-[1-(3,4-dihydro-1H-isoquinolin-2-ylmethyl)-2,2-dimethyl-propyl]-4-hydroxy-pyrrolidine-2-carboxamide |
| 1170 | 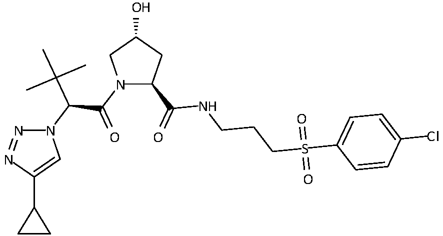 | (2S,4R)-N-[3-(4-chlorophenyl)sulfonylprop yl]-1-[(2S)-2-(4-cyclopropyltriazol-1-yl)-3,3-dimethyl-butanoyl]-4-hydroxy-pyrrolidine-2-carboxamide |
| 1171 | 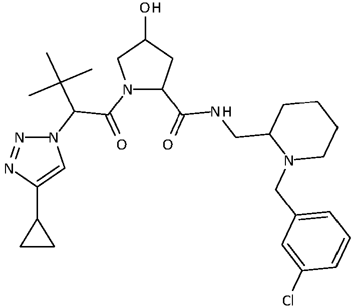 | (2S,4R)-N-[[1-[(3-chlorophenyl)methyl]-2-piperidyl]methyl]-1-[(2R)-2-(4-cyclopropyltriazol-1-yl)-3,3-dimethyl-butanoyl]-4-hydroxy-pyrrolidine-2-carboxamide |
| 1172 | 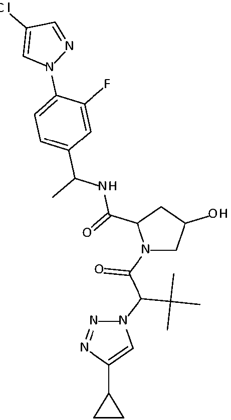 | (2R,4S)-N-[1-[4-(4-chloropyrazol-1-yl)-3-fluoro-phenyl]ethyl]-1-[(2R)-2-(4-cyclopropyltriazol-1-yl)-3,3-dimethyl-butanoyl]-4-hydroxy-pyrrolidine-2-carboxamide |
| 1173 | 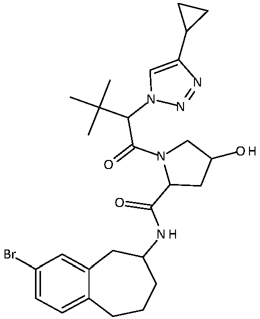 | (2S,4R)-N-(3-bromo-6,7,8,9-tetrahydro-5H-benzo[7]annulen-6-yl)-1-[(2R)-2-(4-cyclopropyltriazol-1-yl)-3,3-dimethyl-butanoyl]-4-hydroxy-pyrrolidine-2-carboxamide |
| 1174 | 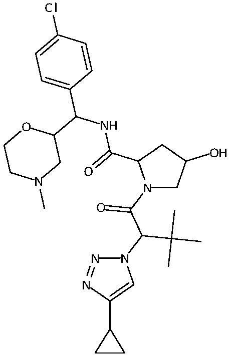 | (2R,4S)-N-[(4-chlorophenyl)-(4-methylmorpholin-2-yl)methyl]-1-[(2R)-2-(4-cyclopropyltriazol-1-yl)-3,3-dimethyl-butanoyl]-4-hydroxy-pyrrolidine-2-carboxamide |
| 1175 | 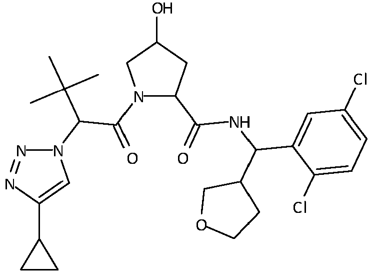 | (2S,4R)-1-[(2R)-2-(4-cyclopropyltriazol-1-yl)-3,3-dimethyl-butanoyl]-N-[(2,5-dichlorophenyl)-tetrahydrofuran-3-yl-methyl]-4-hydroxy-pyrrolidine-2-carboxamide |
| 1176 | 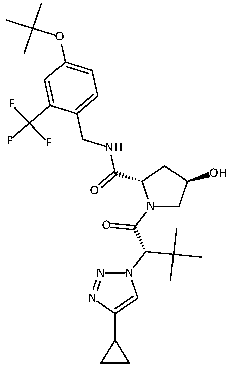 | (2S,4R)-N-[[4-tert-butoxy-2-(trifluoromethyl)phenyl]m ethyl]-1-[(2S)-2-(4-cyclopropyltriazol-1-yl)-3,3-dimethyl-butanoyl]-4-hydroxy-pyrrolidine-2-carboxamide |
| 1177 | 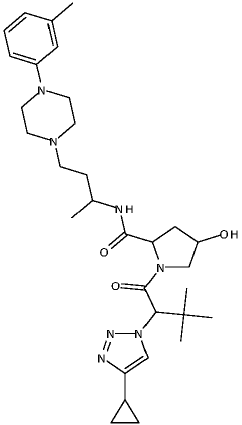 | (2R,4S)-1-[(2R)-2-(4-cyclopropyltriazol-1-yl)-3,3-dimethyl-butanoyl]-4-hydroxy-N-[1-methyl-3-[4-(m-tolyl)piperazin-1-yl]propyl]pyrrolidine-2-carboxamide |
| 1178 | 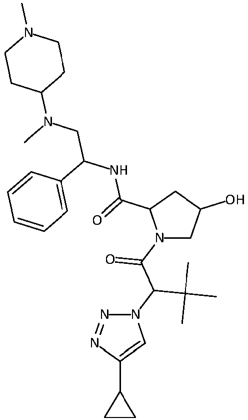 | (2R,4S)-1-[(2R)-2-(4-cyclopropyltriazol-1-yl)-3,3-dimethyl-butanoyl]-4-hydroxy-N-[2-[methyl-(1-methyl-4-piperidyl)amino]-1-phenyl-ethyl]pyrrolidine-2-carboxamide |
| 1179 | 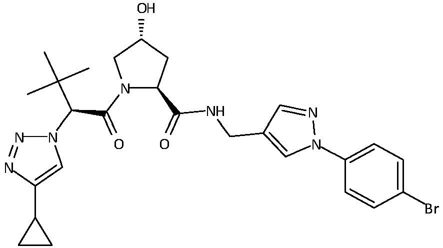 | (2S,4R)-N-[[1-(4-bromophenyl)pyrazol-4-yl]methyl]-1-[(2S)-2-(4-cyclopropyltriazol-1-yl)-3,3-dimethyl-butanoyl]-4-hydroxy-pyrrolidine-2-carboxamide |
| 1180 | 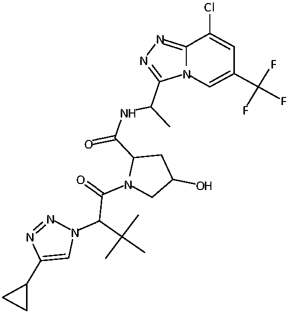 | (2R,4S)-N-[1-[8-chloro-6-(trifluoromethyl)-[1,2,4]triazolo[4,3-a]pyridin-3-yl]ethyl]-1-[(2R)-2-(4-cyclopropyltriazol-1-yl)-3,3-dimethyl-butanoyl]-4-hydroxy-pyrrolidine-2-carboxamide |
| 1181 | 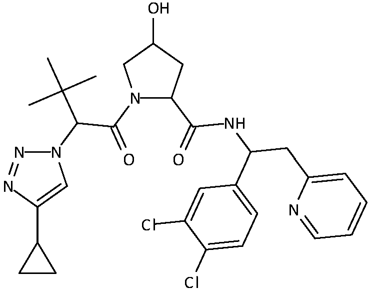 | (2S,4R)-1-[(2R)-2-(4-cyclopropyltriazol-1-yl)-3,3-dimethyl-butanoyl]-N-[1-(3,4-dichlorophenyl)-2-(2-pyridyl)ethyl]-4-hydroxy-pyrrolidine-2-carboxamide |
| 1182 | 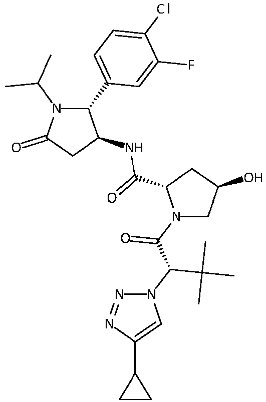 | (2S,4R)-N-[(2R,3S)-2-(4-chloro-3-fluoro-phenyl)-1-isopropyl-5-oxo-pyrrolidin-3-yl]-1-[(2S)-2-(4-cyclopropyltriazol-1-yl)-3,3-dimethyl-butanoyl]-4-hydroxy-pyrrolidine-2-carboxamide |
| 1183 | 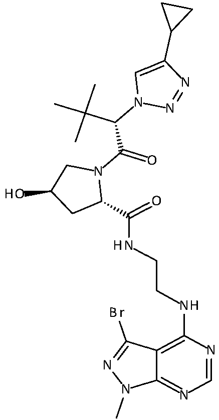 | (2S,4R)-N-[2-[(3-bromo-1-methyl-pyrazolo[3,4-d]pyrimidin-4-yl)amino]ethyl]-1-[(2S)-2-(4-cyclopropyltriazol-1-yl)-3,3-dimethyl-butanoyl]-4-hydroxy-pyrrolidine-2-carboxamide |
| 1184 | 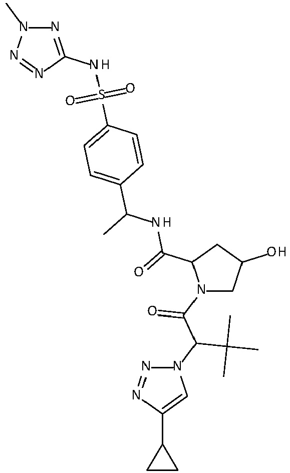 | (2R,4S)-1-[(2R)-2-(4-cyclopropyltriazol-1-yl)-3,3-dimethyl-butanoyl]-4-hydroxy-N-[1-[4-[(2-methyltetrazol-5-yl)sulfamoyl]phenyl]ethyl] pyrrolidine-2-carboxamide |
| 1185 | 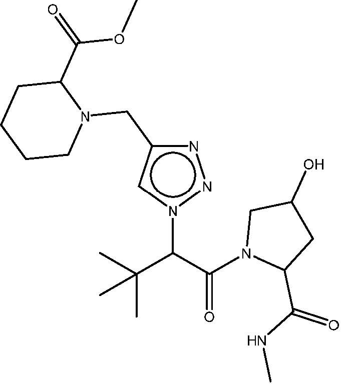 | methyl 1-[[1-[(1R)-1-[(2R,4S)-4-hydroxy-2-(methylcarbamoyl)pyrrolid ine-1-carbonyl]-2,2-dimethyl-propyl]triazol-4-yl]methyl]piperidine-2-carboxylate |
| 1186 | 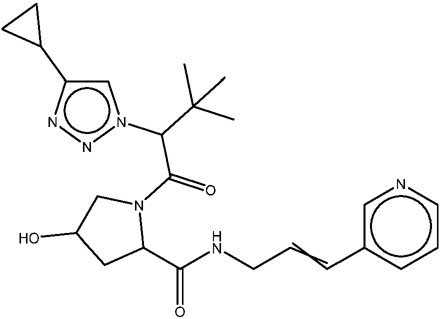 | (2R,4S)-1-[(2S)-2-(4-cyclopropyltriazol-1-yl)-3,3-dimethyl-butanoyl]-4-hydroxy-N-[3-(3-pyridyl)allyl]pyrrolidine-2-carboxamide |
| 1187 | 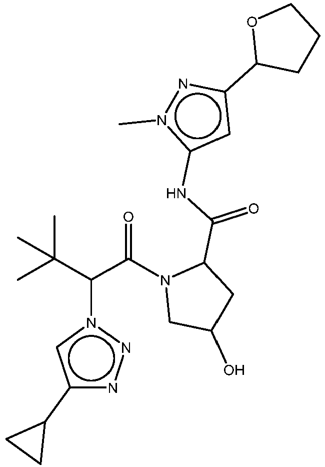 | (2R,4S)-1-[(2R)-2-(4-cyclopropyltriazol-1-yl)-3,3-dimethyl-butanoyl]-4-hydroxy-N-(2-methyl-5-tetrahydrofuran-2-yl-pyrazol-3-yl)pyrrolidine-2-carboxamide |
| 1188 | 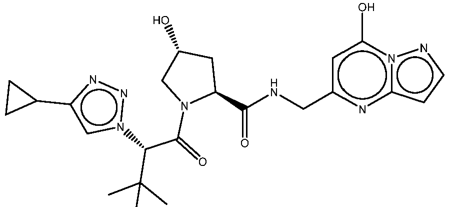 | (2S,4R)-1-[(2S)-2-(4-cyclopropyltriazol-1-yl)-3,3-dimethyl-butanoyl]-4-hydroxy-N-[(7-hydroxypyrazolo[1,5-a]pyrimidin-5-yl)methyl]pyrrolidine-2-carboxamide |
| 1189 | 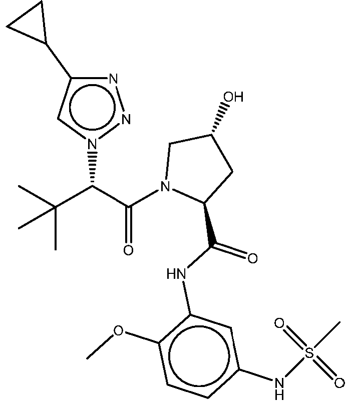 | (2S,4R)-1-[(2S)-2-(4-cyclopropyltriazol-1-yl)-3,3-dimethyl-butanoyl]-4-hydroxy-N-[5-(methanesulfonamido)-2-methoxy-phenyl]pyrrolidine-2-carboxamide |
| 1190 | 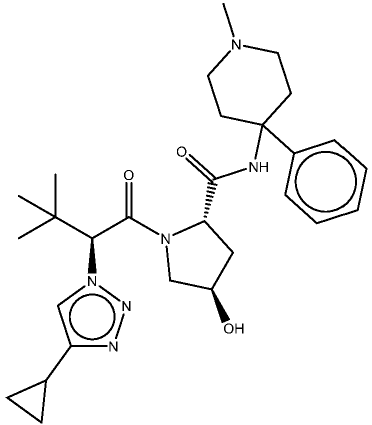 | (2S,4R)-1-[(2S)-2-(4-cyclopropyltriazol-1-yl)-3,3-dimethyl-butanoyl]-4-hydroxy-N-(1-methyl-4-phenyl-4-piperidyl)pyrrolidine-2-carboxamide |
| 1191 | 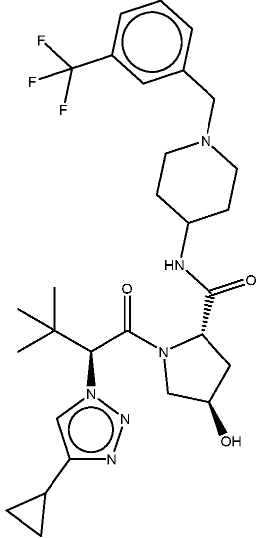 | (2S,4R)-1-[(2S)-2-(4-cyclopropyltriazol-1-yl)-3,3-dimethyl-butanoyl]-4-hydroxy-N-[1-[[3-(trifluoromethyl)phenyl]m ethyl]-4-piperidyl]pyrrolidine-2-carboxamide |

In some embodiments, provided herein is a compound of Table 1-A, Table 1-B, or Table 1-C. In some embodiments, provided herein is a stereoisomer or tautomer of a compound of Table 1-A, Table 1-B, or Table 1-C. In some embodiments, provided herein is a pharmaceutically acceptable salt of a compound of Table 1-A, Table 1-B, or Table 1-C, or a stereoisomer or tautomer thereof.

In one embodiment, provided herein is a compound selected from the group consisting of:
(2S,4R)-1-[(2S)-2-(4-cyclopropyltriazol-1-yl)-3,3-dimethyl-butanoyl]-4-hydroxy-N-[1-methyl-1-(4-methylpyrimidin-2-yl)ethyl]pyrrolidine-2-carboxamide;
(2S,4R)-N-[2-(4-bromo-N-methyl-anilino)ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-(4-phenylpiperazin-1-yl)sulfonylpropyl]pyrrolidine-2-carboxamide;
(2S,4R)-N-[(2-cyclopropyl-2-azabicyclo[2.1.1]hexan-1-yl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[(2R,5R)-5-phenyltetrahydrofuran-2-yl]methyl]pyrrolidine-2-carboxamide;
(2S,4R)-1-[(2S)-2-(4-cyclopropyltriazol-1-yl)-3,3-dimethyl-butanoyl]-4-hydroxy-N-[2-hydroxy-1-(3-methylimidazol-4-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[(2-oxo-3,4-dihydro-1H-quinolin-4-yl)methyl]pyrrolidine-2-carboxamide;
(2S,4R)-1-[(2S)-2-(4-cyclopropyltriazol-1-yl)-3,3-dimethyl-butanoyl]-N-(6,6-difluoro-3-bicyclo[3.1.0]hexanyl)-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(4-methyl-3-methylsulfonyl-phenyl)methyl]pyrrolidine-2-carboxamide;
(2S,4R)-1-[(2S)-2-(4-cyclopropyltriazol-1-yl)-3,3-dimethyl-butanoyl]-N-(6,7-dihydro-5H-pyrazolo[5,1-b][1,3]oxazin-2-ylmethyl)-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(1,2,2-trimethylpyrrolidin-3-yl)pyrrolidine-2-carboxamide;
(2S,4R)-1-[(2S)-2-(4-cyclopropyltriazol-1-yl)-3,3-dimethyl-butanoyl]-4-hydroxy-N-[(1R,2R)-2-hydroxy-4-[(2-phenylimidazol-1-yl)methyl]cyclopentyl]pyrrolidine-2-carboxamide;
(2S,4R)-1-[(2S)-2-(4-cyclopropyltriazol-1-yl)-3,3-dimethyl-butanoyl]-N-[2-(3,6-dihydro-2H-pyridin-1-yl)ethyl]-4-hydroxy-pyrrolidine-2-carboxamide;
(2S,4R)-1-[(2S)-2-(4-cyclopropyltriazol-1-yl)-3,3-dimethyl-butanoyl]-N-[[1-(4-fluorophenyl)tetrazol-5-yl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-[4-(2-pyridyl)thiazol-2-yl]ethyl]pyrrolidine-2-carboxamide;
(2S,4R)-N-[(3aS,6R,6aR)-1-benzyl-3,3a,4,5,6,6a-hexahydro-2H-cyclopenta[b]pyrrol-6-yl]-1-[(2S)-2-(4-cyclopropyltriazol-1-yl)-3,3-dimethyl-butanoyl]-4-hydroxy-pyrrolidine-2-carboxamide;
(2S,4R)-N-[(5-cyclopropylisoxazol-4-yl)methyl]-1-[(2S)-2-(4-cyclopropyltriazol-1-yl)-3,3-dimethyl-butanoyl]-4-hydroxy-pyrrolidine-2-carboxamide;
(2S,4R)-N-[2-(4-chlorophenyl)-3-(dimethylamino)propyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(2-pyrrolidin-1-ylcyclohexyl)methyl]pyrrolidine-2-carboxamide;
6-[[[(2S,4R)-1-[(2S)-2-(4-cyclopropyltriazol-1-yl)-3,3-dimethyl-butanoyl]-4-hydroxy-pyrrolidine-2-carbonyl]amino]methyl]-3,4-dihydro-1H-pyrrolo[2,1-c][1,4]oxazine-8-carboxamide;
(2S,4R)-1-[(2S)-2-(4-cyclopropyltriazol-1-yl)-3,3-dimethyl-butanoyl]-4-hydroxy-N-[[3-(3,3,3-trifluoropropoxy)-4-pyridyl]methyl]pyrrolidine-2-carboxamide;
(2S,4R)-1-[(2S)-2-(4-cyclopropyltriazol-1-yl)-3,3-dimethyl-butanoyl]-4-hydroxy-N-[(3-methyl-4-oxo-phthalazin-1-yl)methyl]pyrrolidine-2-carboxamide;
(2S,4R)-N-(8-chloro-2,3,4,5-tetrahydro-1-benzoxepin-5-yl)-1-[(2S)-2-(4-cyclopropyltriazol-1-yl)-3,3-dimethyl-butanoyl]-4-hydroxy-pyrrolidine-2-carboxamide;
(2S,4R)-N-[[1-(cyclopropylmethyl)tetrazol-5-yl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(2-methyl-5,6,7,8-tetrahydroquinazolin-6-yl)pyrrolidine-2-carboxamide;
(2S,4R)-N-[2-(2-chloro-4-fluoro-phenyl)-3,3,3-trifluoro-propyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[[4-(4-fluorophenyl)thiazol-2-yl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-[(1-phenyltetrazol-5-yl)amino]ethyl]pyrrolidine-2-carboxamide;
(2S,4R)-N-[[1-(2-chlorophenyl)triazol-4-yl]methyl]-1-[(2S)-2-(4-cyclopropyltriazol-1-yl)-3,3-dimethyl-butanoyl]-4-hydroxy-pyrrolidine-2-carboxamide;
(2S,4R)-N-[cyclohexyl(3-pyridyl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(1R,2S)-2-(1-phenylpyrazol-4-yl)cyclopropyl]pyrrolidine-2-carboxamide;
(2S,4R)-N-(3-cyclopropyl-3-hydroxy-butyl)-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[(1R,3S)-2,2-dimethyl-3-phenoxy-cyclobutyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(6-methyl-1,1-dioxo-thian-3-yl)pyrrolidine-2-carboxamide;
(2S,4R)-N-[(1R,2R,4S)-4-cyano-2-hydroxy-cyclopentyl]-1-[(2S)-2-(4-cyclopropyltriazol-1-yl)-3,3-dimethyl-butanoyl]-4-hydroxy-pyrrolidine-2-carboxamide;
(2S,4R)-N-[[3-(4-cyanophenyl)-1-methyl-pyrazol-4-yl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[1-(2-ethyl-3-oxo-4H-1,4-benzoxazin-7-yl)ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[2-methyl-4-(3-oxopiperazin-1-yl)phenyl]methyl]pyrrolidine-2-carboxamide;
(2S,4R)-1-[(2 S)-2-(4-cyclopropyltriazol-1-yl)-3,3-dimethyl-butanoyl]-4-hydroxy-N-[(2-isopropyl-1,2,4-triazol-3-yl)methyl]pyrrolidine-2-carboxamide;
(2S,4R)-1-[(2S)-2-(4-cyclopropyltriazol-1-yl)-3,3-dimethyl-butanoyl]-N-[[1-[(2-ethylthiazol-4-yl)methyl]-4-piperidyl]methyl]-4-hydroxy-pyrrolidine-2-carboxamide;
(2S,4R)-1-[(2S)-2-(4-cyclopropyltriazol-1-yl)-3,3-dimethyl-butanoyl]-N-[3-(2-fluorophenyl)-5,6,7,8-tetrahydro-[1,2,4]triazolo[4,3-a]pyridin-6-yl]-4-hydroxy-pyrrolidine-2-carboxamide;
(2S,4R)-1-[(2S)-2-(4-cyclopropyltriazol-1-yl)-3,3-dimethyl-butanoyl]-N-[2-[5-[(4-fluorophenoxy)methyl]-1,2,4-oxadiazol-3-yl]ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(1R,2S)-2-(1-naphthyl)cyclopropyl]pyrrolidine-2-carboxamide;
(2S,4R)-N-[2-cyclopropyl-1-(3-fluoro-4-methyl-phenyl)-2-hydroxy-ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[1-(2-hydroxy-2-methyl-propyl)azetidin-3-yl]pyrrolidine-2-carboxamide;
(2S,4R)-1-[(2S)-2-(4-cyclopropyltriazol-1-yl)-3,3-dimethyl-butanoyl]-N-[(1R,2R)-2-(difluoromethyl)cyclopropyl]-4-hydroxy-pyrrolidine-2-carboxamide;
(2S,4R)-1-[(2S)-2-(4-cyclopropyltriazol-1-yl)-3,3-dimethyl-butanoyl]-N-[2-(5,5-dimethyl-2-oxo-pyrrolidin-3-yl)ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-hydroxy-2-(4-phenylphenyl)ethyl]pyrrolidine-2-carboxamide;
(2S,4R)-1-[(2S)-2-(4-cyclopropyltriazol-1-yl)-3,3-dimethyl-butanoyl]-N-[1-[5-(difluoromethyl)isoxazol-3-yl]-1-methyl-ethyl]-4-hydroxy-pyrrolidine-2-carboxamide;
(2S,4R)-1-[(2S)-2-(4-cyclopropyltriazol-1-yl)-3,3-dimethyl-butanoyl]-N-[(2,4-dimethoxyphenyl)-(4-methoxyphenyl)methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-[(1-methyl-4-piperidyl)amino]-2-oxo-ethyl]pyrrolidine-2-carboxamide;
(2S,4R)-1-[(2S)-2-(4-cyclopropyltriazol-1-yl)-3,3-dimethyl-butanoyl]-N-[[2-fluoro-6-(methanesulfonamido)phenyl]methyl]-4-hydroxy-pyrrolidine-2-carboxamide;
(2S,4R)-N-[cyclopropyl(tetrahydropyran-2-yl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(4-methyl-5-phenyl-1,2,4-triazol-3-yl)methyl]pyrrolidine-2-carboxamide;
(2S,4R)-1-[(2S)-2-(4-cyclopropyltriazol-1-yl)-3,3-dimethyl-butanoyl]-N-[(3-fluoro-4-methyl-2-pyridyl)methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(3-oxabicyclo[4.1.0]heptan-7-yl)pyrrolidine-2-carboxamide;
(2S,4R)-N-[(6-amino-2,4-dimethyl-3-pyridyl)methyl]-1-[(2S)-2-(4-cyclopropyltriazol-1-yl)-3,3-dimethyl-butanoyl]-4-hydroxy-pyrrolidine-2-carboxamide;
(2S,4R)-N-[1-[1-(4-bromopyrazol-1-yl)cyclopropyl]ethyl]-1-[(2S)-2-(4-cyclopropyltriazol-1-yl)-3,3-dimethyl-butanoyl]-4-hydroxy-pyrrolidine-2-carboxamide;
(2S,4R)-N-[1-cyclopropyl-2-(1,1-dioxo-1,2-thiazolidin-2-yl)ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[5-(methoxymethyl)oxazol-4-yl]methyl]pyrrolidine-2-carboxamide;
(2S,4R)-1-[(2S)-2-(4-cyclopropyltriazol-1-yl)-3,3-dimethyl-butanoyl]-4-hydroxy-N-[(1'R,4R)-spiro[chromane-4,2'-cyclopropane]-1'-yl]pyrrolidine-2-carboxamide;
(2S,4R)-N-[2-[4-(3-chlorophenyl)piperazin-1-yl]-2-oxo-ethyl]-1-[(2S)-2-(4-cyclopropyltriazol-1-yl)-3,3-dimethyl-butanoyl]-4-hydroxy-pyrrolidine-2-carboxamide;
(2S,4R)-N-[2-[(4-chloro-2-fluoro-phenyl)sulfonylamino]ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[1-[(2-fluorophenyl)methyl]cyclopentyl]-4-hydroxy-pyrrolidine-2-carboxamide;
(2S,4R)-1-[(2S)-2-(4-cyclopropyltriazol-1-yl)-3,3-dimethyl-butanoyl]-N-[1-[2-(2,2-dimethylpropanoylamino)thiazol-4-yl]ethyl]-4-hydroxy-pyrrolidine-2-carboxamide;
(2S,4R)-N-(2-cyclopentyl-4,5,6,7-tetrahydroindazol-7-yl)-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-methyl-2-(4-pyridyl)propyl]pyrrolidine-2-carboxamide;
(2S,4R)-1-[(2S)-2-(4-cyclopropyltriazol-1-yl)-3,3-dimethyl-butanoyl]-N-[2-(2-fluorophenyl)sulfinylethyl]-4-hydroxy-pyrrolidine-2-carboxamide;
(2S,4R)-1-[(2S)-2-(4-cyclopropyltriazol-1-yl)-3,3-dimethyl-butanoyl]-N-[[5-(difluoromethyl)-2-methyl-pyrazol-3-yl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-(3-methoxyphenyl)-2-methyl-propyl]pyrrolidine-2-carboxamide;
(2S,4R)-1-[(2S)-2-(4-cyclopropyltriazol-1-yl)-3,3-dimethyl-butanoyl]-4-hydroxy-N-[(5-methylpyrimidin-2-yl)methyl]pyrrolidine-2-carboxamide;
(2S,4R)-1-[(2S)-2-(4-cyclopropyltriazol-1-yl)-3,3-dimethyl-butanoyl]-4-hydroxy-N-[2-(tetrazol-2-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-(3-isoxazol-4-ylpropyl)pyrrolidine-2-carboxamide;
(2S,4R)-1-[(2S)-2-(4-cyclopropyltriazol-1-yl)-3,3-dimethyl-butanoyl]-4-hydroxy-N-[(3-hydroxy-5,6-dimethyl-pyridazin-4-yl)methyl]pyrrolidine-2-carboxamide;
(2S,4R)-1-[(2S)-2-(4-cyclopropyltriazol-1-yl)-3,3-dimethyl-butanoyl]-N-[3-(5-fluoro-1-methyl-benzimidazol-2-yl)propyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(2-isopropyl-6-oxo-1,3,4,7,8,8a-hexahydropyrrolo[1,2-a]pyrazin-7-yl)pyrrolidine-2-carboxamide;
(2S,4R)-1-[(2S)-2-(4-cyclopropyltriazol-1-yl)-3,3-dimethyl-butanoyl]-N-[(4,4-dioxo-2,3-dihydro-1,4lambda6-benzoxathiin-2-yl)methyl]-4-hydroxy-pyrrolidine-2-carboxamide;
(2S,4R)-1-[(2S)-2-(4-cyclopropyltriazol-1-yl)-3,3-dimethyl-butanoyl]-N-(1,1-dioxothian-4-yl)-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(3-methyl-6-oxo-3-piperidyl)methyl]pyrrolidine-2-carboxamide;
(2S,4R)-1-[(2S)-2-(4-cyclopropyltriazol-1-yl)-3,3-dimethyl-butanoyl]-4-hydroxy-N-[1-(4-pyridyl)cyclopropyl]pyrrolidine-2-carboxamide;
(2S,4R)-1-[(2S)-2-(4-cyclopropyltriazol-1-yl)-3,3-dimethyl-butanoyl]-4-hydroxy-N-(5-methyl-2,3-dihydrobenzofuran-3-yl)pyrrolidine-2-carboxamide;
(2S,4R)-1-[(2S)-2-(4-cyclopropyltriazol-1-yl)-3,3-dimethyl-butanoyl]-N-[[3-(1-ethylpropyl)isoxazol-5-yl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-(4-methoxy-1-piperidyl)ethyl]pyrrolidine-2-carboxamide;
(2S,4R)-N-(1-benzyl-2-thiazol-4-yl-ethyl)-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-(1-methylimidazol-4-yl)-2-pyrrolidin-1-yl-ethyl]pyrrolidine-2-carboxamide;
(2S,4R)-1-[(2S)-2-(4-cyclopropyltriazol-1-yl)-3,3-dimethyl-butanoyl]-4-hydroxy-N-[1-(6-methoxy-2-pyridyl)ethyl]pyrrolidine-2-carboxamide;
(2S,4R)-1-[(2S)-2-(4-cyclopropyltriazol-1-yl)-3,3-dimethyl-butanoyl]-4-hydroxy-N-(1-thiazol-2-ylcyclopentyl)pyrrolidine-2-carboxamide;
(2S,4R)-1-[(2S)-2-(4-cyclopropyltriazol-1-yl)-3,3-dimethyl-butanoyl]-4-hydroxy-N-[[2-[(4-methyl-1-piperidyl)sulfonylmethyl]phenyl]methyl]pyrrolidine-2-carboxamide;
(2S,4R)-1-[(2S)-2-(4-cyclopropyltriazol-1-yl)-3,3-dimethyl-butanoyl]-N-[(1R,2S)-2-(2,5-dimethoxy-4-methyl-phenyl)cyclopropyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-(4-methyl-6-oxo-pyrimidin-1-yl)ethyl]pyrrolidine-2-carboxamide;
(2S,4R)-N-[2-(5-amino-1,3,4-oxadiazol-2-yl)ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[[6-(1,1-difluoroethyl)-2-pyridyl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(1-methyl-2-pyrazin-2-yl-ethyl)pyrrolidine-2-carboxamide;
(2S,4R)-1-[(2S)-2-(4-cyclopropyltriazol-1-yl)-3,3-dimethyl-butanoyl]-N-[2-(dimethylamino)-2-(4-ethylphenyl)ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(2-methyl-5,6,7,8-tetrahydroimidazo[1,2-a]pyridin-3-yl)methyl]pyrrolidine-2-carboxamide;
(2S,4R)-1-[(2S)-2-(4-cyclopropyltriazol-1-yl)-3,3-dimethyl-butanoyl]-N-[(4,4-difluoro-1-methyl-3-piperidyl)methyl]-4-hydroxy-pyrrolidine-2-carboxamide;
(2S,4R)-1-[(2S)-2-(4-cyclopropyltriazol-1-yl)-3,3-dimethyl-butanoyl]-N-(2,2-dimethyl-1-pyrimidin-2-yl-propyl)-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-(3-hydroxyphenyl)-1-phenyl-ethyl]pyrrolidine-2-carboxamide;
(2S,4R)-1-[(2S)-2-(4-cyclopropyltriazol-1-yl)-3,3-dimethyl-butanoyl]-4-hydroxy-N-[2-hydroxy-1-[2-(trifluoromethoxy)phenyl]ethyl]pyrrolidine-2-carboxamide;
(2S,4R)-1-[(2S)-2-(4-cyclopropyltriazol-1-yl)-3,3-dimethyl-butanoyl]-N-[2-(2,2-difluorocyclopropoxy)ethyl]-4-hydroxy-pyrrolidine-2-carboxamide;
(2S,4R)-N-[2-(4-bromotriazol-2-yl)ethyl]-1-[(2S)-2-(4-cyclopropyltriazol-1-yl)-3,3-dimethylbutanoyl]-4-hydroxy-pyrrolidine-2-carboxamide;
(2S,4R)-1-[(2S)-2-(4-cyclopropyltriazol-1-yl)-3,3-dimethyl-butanoyl]-N-[1-[3-(1,1-dioxo-1,2-thiazolidin-2-yl)phenyl]ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[2-(imidazol-1-ylmethyl)phenyl]methyl]pyrrolidine-2-carboxamide;
(2S,4R)-1-[(2S)-2-(4-cyclopropyltriazol-1-yl)-3,3-dimethyl-butanoyl]-4-hydroxy-N-[[6-(2-methylmorpholin-4-yl)-3-pyridyl]methyl]pyrrolidine-2-carboxamide;
(2S,4R)-1-[(2S)-2-(4-cyclopropyltriazol-1-yl)-3,3-dimethyl-butanoyl]-4-hydroxy-N-[2-(4-methyl-1-piperidyl)-2-phenyl-ethyl]pyrrolidine-2-carboxamide;
(2S,4R)-N-[4-(1,3-benzoxazol-2-yl)cyclohexyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-(3-oxomorpholin-4-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[[1-(4-methoxyphenyl)pyrazol-3-yl]methyl]pyrrolidine-2-carboxamide;
(2S,4R)-N-[[6-(cyclohexoxy)-3-pyridyl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(7-oxo-6-azabicyclo[3.2.1]octan-5-yl)methyl]pyrrolidine-2-carboxamide;
(2S,4R)-1-[(2S)-2-(4-cyclopropyltriazol-1-yl)-3,3-dimethyl-butanoyl]-N-[1-[5-(3-fluorophenyl)-1,3,4-thiadiazol-2-yl]ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[1-[2-(m-tolyl)cyclopropyl]ethyl]pyrrolidine-2-carboxamide;
(2S,4R)-1-[(2S)-2-(4-cyclopropyltriazol-1-yl)-3,3-dimethyl-butanoyl]-4-hydroxy-N-[5-oxo-1-(4-phenoxyphenyl)pyrrolidin-3-yl]pyrrolidine-2-carboxamide;
(2S,4R)-1-[(2S)-2-(4-cyclopropyltriazol-1-yl)-3,3-dimethyl-butanoyl]-N-[[4-fluoro-3-[(3-methoxyphenoxy)methyl]phenyl]methyl]-4-hydroxy-pyrrolidine-2-carboxamide;
(2S,4R)-N-[[4-(3-chlorophenoxy)-2-pyridyl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[[4-(4-ethoxyphenyl)tetrahydropyran-4-yl]methyl]-4-hydroxy-pyrrolidine-2-carboxamide;
(2S,4R)-N-(2-benzylsulfinylethyl)-1-[(2S)-2-(4-cyclopropyltriazol-1-yl)-3,3-dimethylbutanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[3,3,3-trifluoro-2-hydroxy-2-(1-methylimidazol-2-yl)propyl]pyrrolidine-2-carboxamide;
(2S,4R)-1-[(2S)-2-(4-cyclopropyltriazol-1-yl)-3,3-dimethyl-butanoyl]-4-hydroxy-N-[[(1R,2R,4S)-2-hydroxy-4-(4-methyl-1,2,4-triazol-3-yl)cyclopentyl]methyl]pyrrolidine-2-carboxamide;
(2S,4R)-1-[(2S)-2-(4-cyclopropyltriazol-1-yl)-3,3-dimethyl-butanoyl]-4-hydroxy-N-[4-hydroxy-2-[4-(trifluoromethyl)phenyl]butyl]pyrrolidine-2-carboxamide;
(2S,4R)-1-[(2S)-2-(4-cyclopropyltriazol-1-yl)-3,3-dimethyl-butanoyl]-4-hydroxy-N-[1-[(4-pyrimidin-2-ylpiperazin-1-yl)methyl]cyclopropyl]pyrrolidine-2-carboxamide;
(2S,4R)-N-[2-[[3-chloro-5-(trifluoromethyl)-2-pyridyl]sulfanyl]ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[[2-(difluoromethoxy)-4-propoxy-phenyl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-oxo-2-(thiazol-2-ylamino)ethyl]pyrrolidine-2-carboxamide;
(2S,4R)-1-[(2S)-2-(4-cyclopropyltriazol-1-yl)-3,3-dimethyl-butanoyl]-N-[2-(4-cyclopropyltriazol-1-yl)ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(4-hydroxy-1-methyl-pyrrolidin-2-yl)methyl]pyrrolidine-2-carboxamide;
(2S,4R)-1-[(2S)-2-(4-cyclopropyltriazol-1-yl)-3,3-dimethyl-butanoyl]-4-hydroxy-N-[[5-(1-piperidylsulfonyl)-2-thienyl]methyl]pyrrolidine-2-carboxamide;
(2S,4R)-N-[2-(3-benzyloxyphenyl)-2-hydroxy-ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[3-[[4-(trifluoromethyl)phenyl]methyl]tetrahydrofuran-3-yl]pyrrolidine-2-carboxamide;
(2S,4R)-1-[(2S)-2-(4-cyclopropyltriazol-1-yl)-3,3-dimethyl-butanoyl]-4-hydroxy-N-[3-(methylsulfonylmethyl)oxetan-3-yl]pyrrolidine-2-carboxamide;
(2S,4R)-1-[(2S)-2-(4-cyclopropyltriazol-1-yl)-3,3-dimethyl-butanoyl]-N-[[2-(1,1-dioxo-1,2-thiazolidin-2-yl)cyclopentyl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(3-phenylisothiazol-5-yl)methyl]pyrrolidine-2-carboxamide;
(2S,4R)-1-[(2S)-2-(4-cyclopropyltriazol-1-yl)-3,3-dimethyl-butanoyl]-N-[[3-fluoro-1-(2-methylpropanoyl)azetidin-3-yl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-hydroxy-3-(1-naphthyloxy)propyl]pyrrolidine-2-carboxamide;
(2S,4R)-1-[(2S)-2-(4-cyclopropyltriazol-1-yl)-3,3-dimethyl-butanoyl]-4-hydroxy-N-[[2-(4-methylpiperazin-1-yl)pyrimidin-4-yl]methyl]pyrrolidine-2-carboxamide;
(2S,4R)-N-[(5-cyano-3-pyridyl)methyl]-1-[(2S)-2-(4-cyclopropyltriazol-1-yl)-3,3-dimethylbutanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[phenyl(thiazol-2-yl)methyl]pyrrolidine-2-carboxamide;
(2S,4R)-N-(1-carbamoyl-1-cyclopropyl-propyl)-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[3-(4-methyl-3-oxo-piperazin-1-yl)pyrazin-2-yl]methyl]pyrrolidine-2-carboxamide;
(2S,4R)-1-[(2S)-2-(4-cyclopropyltriazol-1-yl)-3,3-dimethyl-butanoyl]-4-hydroxy-N-[(5-phenylthiazol-2-yl)methyl]pyrrolidine-2-carboxamide;
(2S,4R)-1-[(2S)-2-(4-cyclopropyltriazol-1-yl)-3,3-dimethyl-butanoyl]-4-hydroxy-N-[(3-oxo-4H-1,4-benzoxazin-5-yl)methyl]pyrrolidine-2-carboxamide;
(2S,4R)-1-[(2S)-2-(4-cyclopropyltriazol-1-yl)-3,3-dimethyl-butanoyl]-4-hydroxy-N-[1-(2-methylpyrazolo[1,5-a]pyrazin-4-yl)azetidin-3-yl]pyrrolidine-2-carboxamide;
(2S,4R)-1-[(2S)-2-(4-cyclopropyltriazol-1-yl)-3,3-dimethyl-butanoyl]-4-hydroxy-N-[2-(2-oxopyrimidin-1-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[3-(5-methyl-1,3,4-oxadiazol-2-yl)cyclobutyl]pyrrolidine-2-carboxamide;
(2S,4R)-1-[(2S)-2-(4-cyclopropyltriazol-1-yl)-3,3-dimethyl-butanoyl]-4-hydroxy-N-[[5-(3-pyridyl)-3-pyridyl]methyl]pyrrolidine-2-carboxamide;
(2S,4R)-N-[[6-(benzimidazol-1-yl)-3-pyridyl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[[6-(4-fluorophenoxy)-2-pyridyl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-oxo-1-[4-(trifluoromethyl)phenyl]pyrrolidin-3-yl]pyrrolidine-2-carboxamide;
(2S,4R)-1-[(2S)-2-(4-cyclopropyltriazol-1-yl)-3,3-dimethyl-butanoyl]-4-hydroxy-N-[(1R,2S)-2-(4-isopropylphenyl)cyclopropyl]pyrrolidine-2-carboxamide;
(2S,4R)-N-[(3-amino-1,2-benzoxazol-6-yl)methyl]-1-[(2S)-2-(4-cyclopropyltriazol-1-yl)-3,3-dimethyl-butanoyl]-4-hydroxy-pyrrolidine-2-carboxamide;
(2S,4R)-N-(3,4,6,7,8,8a-hexahydro-1H-pyrrolo[2,1-c][1,4]oxazin-3-ylmethyl)-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-(6,7-dihydro-4H-pyrano[4,3-d]thiazol-2-ylmethyl)-4-hydroxy-pyrrolidine-2-carboxamide;
(2S,4R)-N-[(1-carbamoylcyclopropyl)methyl]-1-[(2S)-2-(4-cyclopropyltriazol-1-yl)-3,3-dimethyl-butanoyl]-4-hydroxy-pyrrolidine-2-carboxamide;
(2S,4R)-N-[(4-cyclopropyl-3-fluoro-phenyl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[3-(4-quinolyl)propyl]pyrrolidine-2-carboxamide;
(2S,4R)-1-[(2S)-2-(4-cyclopropyltriazol-1-yl)-3,3-dimethyl-butanoyl]-4-hydroxy-N-[(1-methylazepan-3-yl)methyl]pyrrolidine-2-carboxamide;
(2S,4R)-1-[(2S)-2-(4-cyclopropyltriazol-1-yl)-3,3-dimethyl-butanoyl]-4-hydroxy-N-[2-(2-methylsulfonylphenyl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[1-(2-pyrazol-1-ylphenyl)ethyl]pyrrolidine-2-carboxamide;
(2S,4R)-1-[(2S)-2-(4-cyclopropyltriazol-1-yl)-3,3-dimethyl-butanoyl]-N-[(2,3-dimethylimidazol-4-yl)methyl]-4-hydroxy-pyrrolidine-2-carboxamide;
(2S,4R)-N-[(5-cyclopropyl-1,2,4-oxadiazol-3-yl)-phenyl-methyl]-1-[(2S)-2-(4-cyclopropyltriazol-1-yl)-3,3-dimethyl-butanoyl]-4-hydroxy-pyrrolidine-2-carboxamide;
(2S,4R)-N-[[5-(tert-butoxymethyl)isoxazol-3-yl]methyl]-1-[(2S)-2-(4-cyclopropyltriazol-1-yl)-3,3-dimethyl-butanoyl]-4-hydroxy-pyrrolidine-2-carboxamide;
(2S,4R)-N-[(2-cyclopropylthiazol-4-yl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[(4,5-dimethyloxazol-2-yl)methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[2-(2,2,2-trifluoroethoxy)cyclohexyl]methyl]pyrrolidine-2-carboxamide;
(2S,4R)-N-[(5-cyclopropyl-1,3,4-oxadiazol-2-yl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(5-methyl-2-oxo-1-phenyl-pyrrolidin-3-yl)pyrrolidine-2-carboxamide;
(2S,4R)-1-[(2S)-2-(4-cyclopropyltriazol-1-yl)-3,3-dimethyl-butanoyl]-4-hydroxy-N-[(1R)-2-(6-methoxy-2-naphthyl)-1-methyl-ethyl]pyrrolidine-2-carboxamide;
(2S,4R)-1-[(2S)-2-(4-cyclopropyltriazol-1-yl)-3,3-dimethyl-butanoyl]-4-hydroxy-N-(imidazo[1,2-a]pyridin-7-ylmethyl)pyrrolidine-2-carboxamide;
(2S,4R)-1-[(2S)-2-(4-cyclopropyltriazol-1-yl)-3,3-dimethyl-butanoyl]-N-(4,6-dichloroindan-1-yl)-4-hydroxy-pyrrolidine-2-carboxamide;
(2S,4R)-1-[(2S)-2-(4-cyclopropyltriazol-1-yl)-3,3-dimethyl-butanoyl]-N-(8-fluorothiochroman-4-yl)-4-hydroxy-pyrrolidine-2-carboxamide;
(2S,4R)-N-[[1-(tert-butylsulfamoyl)cyclopropyl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[3-(2-methyltriazol-4-yl)propyl]pyrrolidine-2-carboxamide;
(2S,4R)-1-[(2S)-2-(4-cyclopropyltriazol-1-yl)-3,3-dimethyl-butanoyl]-4-hydroxy-N-[(2-morpholinosulfonylphenyl)methyl]pyrrolidine-2-carboxamide;
(2S,4R)-N-[1-(4-bromo-3-fluoro-phenyl)-2-hydroxy-ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[3-(5-methyl-1,3,4-thiadiazol-2-yl)propyl]pyrrolidine-2-carboxamide;
(2S,4R)-1-[(2S)-2-(4-cyclopropyltriazol-1-yl)-3,3-dimethyl-butanoyl]-4-hydroxy-N-[3-(2-oxo-1-piperidyl)-1-bicyclo[1.1.1]pentanyl]pyrrolidine-2-carboxamide;
(2S,4R)-1-[(2S)-2-(4-cyclopropyltriazol-1-yl)-3,3-dimethyl-butanoyl]-4-hydroxy-N-[(5-oxo-2-phenyl-pyrrolidin-2-yl)methyl]pyrrolidine-2-carboxamide;
(2S,4R)-1-[(2S)-2-(4-cyclopropyltriazol-1-yl)-3,3-dimethyl-butanoyl]-4-hydroxy-N-[1-(2-methylcyclopropyl)-5-oxo-pyrrolidin-3-yl]pyrrolidine-2-carboxamide;
(2S,4R)-1-[(2S)-2-(4-cyclopropyltriazol-1-yl)-3,3-dimethyl-butanoyl]-N-[1-[1-(4-fluorophenyl)-1-methyl-ethyl]cyclopropyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[3-(4-methylsulfonylphenyl)tetrahydrofuran-3-yl]pyrrolidine-2-carboxamide;
(2S,4R)-1-[(2S)-2-(4-cyclopropyltriazol-1-yl)-3,3-dimethyl-butanoyl]-N-[[3-(3-fluorophenyl)-1,2,4-oxadiazol-5-yl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(1-isobutyl-5-methyl-pyrazol-4-yl)methyl]pyrrolidine-2-carboxamide;
(2S,4R)-1-[(2S)-2-(4-cyclopropyltriazol-1-yl)-3,3-dimethyl-butanoyl]-4-hydroxy-N-[2-[[2-(trifluoromethyl)benzoyl]amino]ethyl]pyrrolidine-2-carboxamide;
(2S,4R)-N-[(1R,2S)-2-(4-bromopyrazol-1-yl)cyclohexyl]-1-[(2S)-2-(4-cyclopropyltriazol-1-yl)-3,3-dimethyl-butanoyl]-4-hydroxy-pyrrolidine-2-carboxamide;
(2S,4R)-N-[(6-bromo-1,3-benzoxazol-2-yl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[1-(2,6-dichlorophenyl)-1-methyl-ethyl]-4-hydroxy-pyrrolidine-2-carboxamide;
(2S,4R)-1-[(2S)-2-(4-cyclopropyltriazol-1-yl)-3,3-dimethyl-butanoyl]-N-[2-[2,2-difluoroethyl(ethyl)amino]ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(7-methyl-6-oxaspiro[3.4]octan-2-yl)pyrrolidine-2-carboxamide;
(2S,4R)-1-[(2S)-2-(4-cyclopropyltriazol-1-yl)-3,3-dimethyl-butanoyl]-4-hydroxy-N-(3-isopropoxycyclobutyl)pyrrolidine-2-carboxamide;
(2S,4R)-N-[(3R,5R)-5-tert-butyl-2-oxo-pyrrolidin-3-yl]-1-[(2S)-2-(4-cyclopropyltriazol-1-yl)-3,3-dimethyl-butanoyl]-4-hydroxy-pyrrolidine-2-carboxamide;
(2S,4R)-N-(2-acetyl-1,3,3a,4,5,6,7,7a-octahydroisoindol-4-yl)-1-[(2S)-2-(4-cyclopropyltriazol-1-yl)-3,3-dimethyl-butanoyl]-4-hydroxy-pyrrolidine-2-carboxamide;
(2S,4R)-N-[(2-cyclohexylpyrazol-3-yl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[5-(1-piperidyl)-1,3,4-oxadiazol-2-yl]methyl]pyrrolidine-2-carboxamide;
(2S,4R)-1-[(2S)-2-(4-cyclopropyltriazol-1-yl)-3,3-dimethyl-butanoyl]-N-[(3-fluorophenyl)-(2-pyridyl)methyl]-4-hydroxy-pyrrolidine-2-carboxamide;
(2S,4R)-1-[(2S)-2-(4-cyclopropyltriazol-1-yl)-3,3-dimethyl-butanoyl]-N-(1,5-diazabicyclo[3.2.2]nonan-6-ylmethyl)-4-hydroxy-pyrrolidine-2-carboxamide;
(2S,4R)-N-[(3 S,4R)-1-benzyl-4-(3-fluorophenyl)pyrrolidin-3-yl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[1-[(3,5-dimethoxyphenyl)methyl]pyrrolidin-3-yl]-4-hydroxy-pyrrolidine-2-carboxamide;
(2S,4R)-N-[(6-chloro-8-methyl-imidazo[1,2-a]pyridin-2-yl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[[1-[2-(dimethylamino)ethyl]-4-piperidyl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-(4-oxopyrazolo[1,5-a]pyrazin-5-yl)ethyl]pyrrolidine-2-carboxamide;
(2S,4R)-N-(4-cyclopropyl-2-hydroxy-butyl)-1-[(2S)-2-(4-cyclopropyltriazol-1-yl)-3,3-dimethyl-butanoyl]-4-hydroxy-pyrrolidine-2-carboxamide;
(2S,4R)-N-[(1-benzyl-2-methyl-pyrrolidin-2-yl)methyl]-1-[(2S)-2-(4-cyclopropyltriazol-1-yl)-3,3-dimethyl-butanoyl]-4-hydroxy-pyrrolidine-2-carboxamide;
(2S,4R)-N-[3-amino-2-[(2-chlorophenyl)methyl]-3-oxo-propyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-oxo-2-(3-quinolylamino)ethyl]pyrrolidine-2-carboxamide;
(2S,4R)-1-[(2S)-2-(4-cyclopropyltriazol-1-yl)-3,3-dimethyl-butanoyl]-4-hydroxy-N-[2-(isoxazol-3-ylmethylsulfonylamino)ethyl]pyrrolidine-2-carboxamide;
(2S,4R)-1-[(2S)-2-(4-cyclopropyltriazol-1-yl)-3,3-dimethyl-butanoyl]-4-hydroxy-N-[(2R,3R)-2-(trifluoromethyl)chroman-3-yl]pyrrolidine-2-carboxamide;
(2S,4R)-1-[(2S)-2-(4-cyclopropyltriazol-1-yl)-3,3-dimethyl-butanoyl]-4-hydroxy-N-[1-methyl-2-(1-oxophthalazin-2-yl)ethyl]pyrrolidine-2-carboxamide;
(2S,4R)-N-[3-(3-cyanophenyl)cyclobutyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[(1,1-dioxo-2-phenyl-1,2-thiazolidin-5-yl)methyl]-4-hydroxy-pyrrolidine-2-carboxamide;
(2S,4R)-N-[(3-cyclopropyl-4,5-dihydroisoxazol-5-yl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[1-[(2-phenyloxazol-4-yl)methyl]-4-piperidyl]pyrrolidine-2-carboxamide;
(2S,4R)-1-[(2S)-2-(4-cyclopropyltriazol-1-yl)-3,3-dimethyl-butanoyl]-4-hydroxy-N-[[6-[2-(trifluoromethyl)phenoxy]-3-pyridyl]methyl]pyrrolidine-2-carboxamide;
(2S,4R)-N-(2-cyclopropyl-2-hydroxy-2-phenyl-ethyl)-1-[(2S)-2-(4-cyclopropyltriazol-1-yl)-3,3-dimethyl-butanoyl]-4-hydroxy-pyrrolidine-2-carboxamide;
(2S,4R)-N-[2-amino-1-methyl-2-oxo-1-(phenoxymethyl)ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[1-(3-phenylpropyl)imidazol-2-yl]methyl]pyrrolidine-2-carboxamide;
(2S,4R)-N-[6-bromo-2-(hydroxymethyl)tetralin-2-yl]-1-[(2S)-2-(4-cyclopropyltriazol-1-yl)-3,3-dimethyl-butanoyl]-4-hydroxy-pyrrolidine-2-carboxamide;
2-[4-[[[(2S,4R)-1-[(2S)-2-(4-cyclopropyltriazol-1-yl)-3,3-dimethyl-butanoyl]-4-hydroxy-pyrrolidine-2-carbonyl]amino]methyl]-1-piperidyl]pyridine-3-carboxamide;
(2S,4R)-1-[(2S)-2-(4-cyclopropyltriazol-1-yl)-3,3-dimethyl-butanoyl]-N-[[6-fluoro-2-(4-fluorophenyl)imidazo[1,2-a]pyridin-3-yl]methyl]-4-hydroxy-pyrrolidine-2-carboxamide;
(2S,4R)-N-[[1-[(5-tert-butyl-1,2,4-oxadiazol-3-yl)methyl]-3-piperidyl]methyl]-1-[(2S)-2-(4-cyclopropyltriazol-1-yl)-3,3-dimethyl-butanoyl]-4-hydroxy-pyrrolidine-2-carboxamide;
(2S,4R)-N-[(3-cyano-2-fluoro-phenyl)methyl]-1-[(2S)-2-(4-cyclopropyltriazol-1-yl)-3,3-dimethyl-butanoyl]-4-hydroxy-pyrrolidine-2-carboxamide;
3-[1-[[(2S,4R)-1-[(2S)-2-(4-cyclopropyltriazol-1-yl)-3,3-dimethyl-butanoyl]-4-hydroxy-pyrrolidine-2-carbonyl]amino]-1-methyl-ethyl]-1,2,4-oxadiazole-5-carboxamide;
(2S,4R)-1-[(2S)-2-(4-cyclopropyltriazol-1-yl)-3,3-dimethyl-butanoyl]-N-[1-[4-(difluoromethyl)-6-methyl-pyrimidin-2-yl]cyclopentyl]-4-hydroxy-pyrrolidine-2-carboxamide;
(2S,4R)-N-[(1-cyanocyclohexyl)methyl]-1-[(2S)-2-(4-cyclopropyltriazol-1-yl)-3,3-dimethylbutanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(1-methyl-2,2-dioxo-3,4-dihydro-2lambda6,1-benzothiazin-4-yl)pyrrolidine-2-carboxamide;
(2S,4R)-1-[(2S)-2-(4-cyclopropyltriazol-1-yl)-3,3-dimethyl-butanoyl]-4-hydroxy-N-[(4-methyl-2-phenyl-thiazol-5-yl)methyl]pyrrolidine-2-carboxamide;
(2S,4R)-N-[[3-(cyclopropylsulfonylamino)phenyl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(3-isopropylisoxazol-4-yl)methyl]pyrrolidine-2-carboxamide;
(2S,4R)-1-[(2S)-2-(4-cyclopropyltriazol-1-yl)-3,3-dimethyl-butanoyl]-N-[1-[3-[[2-(2-fluorophenyl)acetyl]amino]phenyl]ethyl]-4-hydroxy-pyrrolidine-2-carboxamide;
(2S,4R)-N-[2-(4-chlorophenyl)-3,3,3-trifluoro-2-hydroxy-propyl]-1-[(2S)-2-(4-cyclopropyltriazol-1-yl)-3,3-dimethyl-butanoyl]-4-hydroxy-pyrrolidine-2-carboxamide;
(2S,4R)-N-[1-(4-cyano-2-fluoro-phenyl)-3-piperidyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(2-hydroxy-5,6,7,8-tetrahydroquinolin-3-yl)methyl]pyrrolidine-2-carboxamide;
(2S,4R)-1-[(2S)-2-(4-cyclopropyltriazol-1-yl)-3,3-dimethyl-butanoyl]-4-hydroxy-N-[1-(6-morpholinopyrimidin-4-yl)-3-piperidyl]pyrrolidine-2-carboxamide;
(2S,4R)-1-[(2S)-2-(4-cyclopropyltriazol-1-yl)-3,3-dimethyl-butanoyl]-4-hydroxy-N-[3-(triazol-1-ylmethyl)cyclobutyl]pyrrolidine-2-carboxamide;
(2S,4R)-N-[2-[(1-cyanocyclopropyl)methylamino]-2-oxo-ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[4-(3-fluorophenyl)-4-hydroxy-cyclohexyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-(1-methyl-5-oxo-pyrrolidin-3-yl)ethyl]pyrrolidine-2-carboxamide;
(2S,4R)-N-(chroman-6-ylmethyl)-1-[(2S)-2-(4-cyclopropyltriazol-1-yl)-3,3-dimethylbutanoyl]-4-hydroxy-pyrrolidine-2-carboxamide;
(2S,4R)-N-[(4-cyclopropyl-2-methyl-pyrazol-3-yl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(1R)-1-(5-isoquinolyl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[(4-methylthiadiazol-5-yl)methyl]pyrrolidine-2-carboxamide;
(2S,4R)-1-[(2S)-2-(4-cyclopropyltriazol-1-yl)-3,3-dimethyl-butanoyl]-N-(2,2-dimethyl-3-pyrrolidin-1-yl-propyl)-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(2-methylcyclopropyl)-phenyl-methyl]pyrrolidine-2-carboxamide;
(2S,4R)-1-[(2S)-2-(4-cyclopropyltriazol-1-yl)-3,3-dimethyl-butanoyl]-4-hydroxy-N-[3-(1-oxo-2-isoquinolyl)propyl]pyrrolidine-2-carboxamide;
(2S,4R)-1-[(2S)-2-(4-cyclopropyltriazol-1-yl)-3,3-dimethyl-butanoyl]-4-hydroxy-N-[2-[3-(methylcarbamoyl)phenyl]ethyl]pyrrolidine-2-carboxamide;
(2S,4R)-1-[(2S)-2-(4-cyclopropyltriazol-1-yl)-3,3-dimethyl-butanoyl]-N-[1-(2,4-difluorophenyl)cyclobutyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-(5-isopropyl-2-methyl-1,2,4-triazol-3-yl)ethyl]pyrrolidine-2-carboxamide;
(2S,4R)-N-[2-(2-amino-4-pyridyl)ethyl]-1-[(2S)-2-(4-cyclopropyltriazol-1-yl)-3,3-dimethylbutanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-(3,3,4-trimethylpiperazin-1-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[3-(4-methylthiazol-5-yl)propyl]pyrrolidine-2-carboxamide;
(2S,4R)-1-[(2S)-2-(4-cyclopropyltriazol-1-yl)-3,3-dimethyl-butanoyl]-4-hydroxy-N-[(6-methyl-2-pyrrolidin-1-yl-3-pyridyl)methyl]pyrrolidine-2-carboxamide;
(2S,4R)-N-[2-(4-bromophenyl)sulfonyl-2-methyl-propyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(7-oxoazepan-4-yl)pyrrolidine-2-carboxamide;
(2S,4R)-N-(5-cyanoindan-2-yl)-1-[(2S)-2-(4-cyclopropyltriazol-1-yl)-3,3-dimethylbutanoyl]-4-hydroxy-pyrrolidine-2-carboxamide;
(2S,4R)-N-[[(2R,3S)-1-cyclopropyl-5-oxo-2-(4-pyridyl)pyrrolidin-3-yl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(oxazol-4-ylmethyl)pyrrolidine-2-carboxamide;
(2S,4R)-1-[(2S)-2-(4-cyclopropyltriazol-1-yl)-3,3-dimethyl-butanoyl]-4-hydroxy-N-[1-methyl-2-[4-(2-pyridyl)piperazin-1-yl]ethyl]pyrrolidine-2-carboxamide;
(2S,4R)-1-[(2S)-2-(4-cyclopropyltriazol-1-yl)-3,3-dimethyl-butanoyl]-N-[1-(4-fluoro-1-naphthyl)ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(4-methyl-2-pyridyl)-tetrahydropyran-4-yl-methyl]pyrrolidine-2-carboxamide;
(2S,4R)-N-[2-(3-benzyl-1,2,4-oxadiazol-5-yl)ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[[5-(difluoromethyl)-1-methyl-triazol-4-yl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[4-[(3-hydroxypyrrolidin-1-yl)methyl]phenyl]methyl]pyrrolidine-2-carboxamide;
(2S,4R)-1-[(2S)-2-(4-cyclopropyltriazol-1-yl)-3,3-dimethyl-butanoyl]-N-[1-(2-fluoro-5-methyl-phenyl)-2-oxo-3-piperidyl]-4-hydroxy-pyrrolidine-2-carboxamide;
(2S,4R)-1-[(2S)-2-(4-cyclopropyltriazol-1-yl)-3,3-dimethyl-butanoyl]-N-[[4-[2-(dimethylamino)ethyl]phenyl]methyl]-4-hydroxy-pyrrolidine-2-carboxamide;
(2S,4R)-N-(1-cyclohexyl-2-oxo-3-piperidyl)-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[4-(4-phenylthiazol-2-yl)tetrahydropyran-4-yl]methyl]pyrrolidine-2-carboxamide;
(2S,4R)-N-(5'-bromospiro[cyclopropane-2,1'-indane]-1-yl)-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[2-(3-fluoro-2-pyridyl)-2-methyl-propyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-[(4-methoxyphenyl)methylamino]-2-oxo-ethyl]pyrrolidine-2-carboxamide;
(2S,4R)-1-[(2S)-2-(4-cyclopropyltriazol-1-yl)-3,3-dimethyl-butanoyl]-4-hydroxy-N-[2-[(2-oxo-3,4-dihydro-1H-quinolin-6-yl)oxy]ethyl]pyrrolidine-2-carboxamide;
(2S,4R)-1-[(2S)-2-(4-cyclopropyltriazol-1-yl)-3,3-dimethyl-butanoyl]-N-(6-fluoro-1,1-dioxo-3,4-dihydro-2H-thiochromen-4-yl)-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-(4-hydroxytetrahydropyran-4-yl)-1-methyl-ethyl]pyrrolidine-2-carboxamide;
(2S,4R)-1-[(2S)-2-(4-cyclopropyltriazol-1-yl)-3,3-dimethyl-butanoyl]-4-hydroxy-N-[2-hydroxy-1-(2-isopropylthiazol-4-yl)ethyl]pyrrolidine-2-carboxamide;
(2S,4R)-1-[(2S)-2-(4-cyclopropyltriazol-1-yl)-3,3-dimethyl-butanoyl]-N-[1-(4,5-dimethylthiazol-2-yl)-1-methyl-ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[7-(trifluoromethyl)-5,6,7,8-tetrahydro-[1,2,4]triazolo[4,3-a]pyridin-3-yl]methyl]pyrrolidine-2-carboxamide;
(2S,4R)-1-[(2S)-2-(4-cyclopropyltriazol-1-yl)-3,3-dimethyl-butanoyl]-4-hydroxy-N-[1-(1-methyl-3,6-dihydro-2H-pyridin-4-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[2-oxo-2-(5-quinolylamino)ethyl]pyrrolidine-2-carboxamide;
(2S,4R)-1-[(2S)-2-(4-cyclopropyltriazol-1-yl)-3,3-dimethyl-butanoyl]-4-hydroxy-N-[2-oxo-2-[1-(2-pyridyl)ethylamino]ethyl]pyrrolidine-2-carboxamide;
(2S,4R)-1-[(2S)-2-(4-cyclopropyltriazol-1-yl)-3,3-dimethyl-butanoyl]-4-hydroxy-N-[3-(3-oxo-5,6,7,8-tetrahydro-[1,2,4]triazolo[4,3-a]pyridin-2-yl)propyl]pyrrolidine-2-carboxamide;
(2S,4R)-1-[(2S)-2-(4-cyclopropyltriazol-1-yl)-3,3-dimethyl-butanoyl]-4-hydroxy-N-[(1R)-1-methyl-2-(1-methyltriazol-4-yl)ethyl]pyrrolidine-2-carboxamide;
(2S,4R)-N-[2-cyclopentyl-2-(dimethylsulfamoylamino)ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[(2,2-dimethyl-1,1-dioxo-thiolan-3-yl)methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[1-(pyridine-2-carbonyl)pyrrolidin-3-yl]pyrrolidine-2-carboxamide;
(2S,4R)-N-[[2-bromo-5-(trifluoromethyl)phenyl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[1-[3-(dimethylsulfamoyl)phenyl]ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[6-(trifluoromethyl)-[1,2,4]triazolo[4,3-a]pyridin-3-yl]methyl]pyrrolidine-2-carboxamide;
(2S,4R)-1-[(2S)-2-(4-cyclopropyltriazol-1-yl)-3,3-dimethyl-butanoyl]-N-(1,2-dimethyl-3-piperidyl)-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[3-methyl-2-(2-methylpyrazol-3-yl)butyl]pyrrolidine-2-carboxamide;
(2S,4R)-1-[(2 S)-2-(4-cyclopropyltriazol-1-yl)-3,3-dimethyl-butanoyl]-4-hydroxy-N-[[4-(4-methylthiazol-5-yl)phenyl]methyl]pyrrolidine-2-carboxamide;
(2S,4R)-1-[(2S)-2-(4-cyclopropyltriazol-1-yl)-3,3-dimethyl-butanoyl]-N-(2,2-difluoronorcaran-7-yl)-4-hydroxy-pyrrolidine-2-carboxamide;
(2S,4R)-1-[(2S)-2-(4-cyclopropyltriazol-1-yl)-3,3-dimethyl-butanoyl]-N-[[3,5-dimethyl-1-(o-tolyl)pyrazol-4-yl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(1-methyl-2-oxo-5,6,7,8-tetrahydroquinolin-5-yl)pyrrolidine-2-carboxamide;
(2S,4R)-N-[[2-chloro-6-[methyl(3-pyridylmethyl)amino]phenyl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[1-hydroxy-3-(trifluoromethyl)cyclohexyl]methyl]pyrrolidine-2-carboxamide;
(2S,4R)-1-[(2S)-2-(4-cyclopropyltriazol-1-yl)-3,3-dimethyl-butanoyl]-4-hydroxy-N-[1-([1,2,4]triazolo[4,3-b]pyridazin-6-yl)pyrrolidin-3-yl]pyrrolidine-2-carboxamide;
(2S,4R)-N-[3-[(1-benzyl-4-piperidyl)oxy]propyl]-1-[(2S)-2-(4-cyclopropyltriazol-1-yl)-3,3-dimethyl-butanoyl]-4-hydroxy-pyrrolidine-2-carboxamide;
(2S,4R)-N-[2-amino-1-(3-bromophenyl)-2-oxo-ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(1R,2S)-2-[2-(trifluoromethoxy)phenyl]cyclopropyl]pyrrolidine-2-carboxamide;
(2S,4R)-1-[(2S)-2-(4-cyclopropyltriazol-1-yl)-3,3-dimethyl-butanoyl]-4-hydroxy-N-[2-oxo-2-[4-(1,2,4-triazol-1-yl)anilino]ethyl]pyrrolidine-2-carboxamide;
(2S,4R)-N-[(6R,7R)-2-benzyl-6-hydroxy-3-isopropyl-2-azaspiro[3.4]octan-7-yl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[1-(2-ethylsulfonylphenyl)-4-piperidyl]-4-hydroxy-pyrrolidine-2-carboxamide;
(2S,4R)-1-[(2S)-2-(4-cyclopropyltriazol-1-yl)-3,3-dimethyl-butanoyl]-N-[(1R,2S)-2-(2,4-difluorophenyl)cyclopropyl]-4-hydroxy-pyrrolidine-2-carboxamide;
(2S,4R)-1-[(2S)-2-(4-cyclopropyltriazol-1-yl)-3,3-dimethyl-butanoyl]-N-[[(1R,2R)-2-(2,6-difluorophenyl)cyclopropyl]methyl]-4-hydroxy-pyrrolidine-2-carboxamide;
(2S,4R)-1-[(2S)-2-(4-cyclopropyltriazol-1-yl)-3,3-dimethyl-butanoyl]-N-[2-[5-(diethylsulfamoyl)-2-thienyl]ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(2-oxo-3,4-dihydro-1H-1,8-naphthyridin-3-yl)pyrrolidine-2-carboxamide;
(2S,4R)-1-[(2S)-2-(4-cyclopropyltriazol-1-yl)-3,3-dimethyl-butanoyl]-N-[[(2S,3R)-2-(1,5-dimethylpyrazol-4-yl)-1-methyl-5-oxo-pyrrolidin-3-yl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-(3-oxo-1,5,6,7,8,8a-hexahydroimidazo[1,5-a]pyridin-2-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[(1R,2R,3R,4S)-3-(trifluoromethyl)-7-oxabicyclo[2.2.1]heptan-2-yl]pyrrolidine-2-carboxamide;
(2S,4R)-1-[(2S)-2-(4-cyclopropyltriazol-1-yl)-3,3-dimethyl-butanoyl]-4-hydroxy-N-[[3-(2-hydroxyethoxy)-1,1-dioxo-thiolan-3-yl]methyl]pyrrolidine-2-carboxamide;
(2S,4R)-1-[(2S)-2-(4-cyclopropyltriazol-1-yl)-3,3-dimethyl-butanoyl]-4-hydroxy-N-[[2-[4-(1,2,4-triazol-1-ylmethyl)phenyl]phenyl]methyl]pyrrolidine-2-carboxamide;
(2S,4R)-1-[(2S)-2-(4-cyclopropyltriazol-1-yl)-3,3-dimethyl-butanoyl]-N-[[6-(3,4-dihydro-2H-quinolin-1-yl)-3-pyridyl]methyl]-4-hydroxy-pyrrolidine-2-carboxamide;
(2S,4R)-1-[(2S)-2-(4-cyclopropyltriazol-1-yl)-3,3-dimethyl-butanoyl]-N-[[1-[(3,4-dimethylphenyl)carbamoyl]cyclopentyl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(3-methylimidazo[4,5-b]pyridin-2-yl)methyl]pyrrolidine-2-carboxamide;
(2S,4R)-1-[(2S)-2-(4-cyclopropyltriazol-1-yl)-3,3-dimethyl-butanoyl]-4-hydroxy-N-[(5-pyrrolidin-1-yl-2-pyridyl)methyl]pyrrolidine-2-carboxamide;
(2S,4R)-N-[2-(2-amino-4-methyl-thiazol-5-yl)ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-hydroxy-2-(1,3,5-trimethylpyrazol-4-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[2-hydroxy-1-(4-methylsulfonylphenyl)ethyl]pyrrolidine-2-carboxamide;
(2S,4R)-N-[3-(cyclopropylsulfonylamino)propyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[2-(2,4-dimethylphenyl)cyclobutyl]-4-hydroxy-pyrrolidine-2-carboxamide;
(2S,4R)-N-[[(2S,4R)-1-benzyl-4-methoxy-pyrrolidin-2-yl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-(4,6-difluoro-2,3-dihydrobenzofuran-3-yl)-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(2-pyrimidin-2-ylethyl)pyrrolidine-2-carboxamide;
(2S,4R)-1-[(2S)-2-(4-cyclopropyltriazol-1-yl)-3,3-dimethyl-butanoyl]-N-[1-(2-ethyl-1,2,4-triazol-3-yl)ethyl]-4-hydroxy-pyrrolidine-2-carboxamide;
(2S,4R)-N-[2-[(2-chloro-6-fluoro-phenyl)methyl]-3-hydroxy-propyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[2-[5-(difluoromethyl)-2-pyridyl]ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[3-(1-methyltetrazol-5-yl)propyl]pyrrolidine-2-carboxamide;
(2S,4R)-1-[(2S)-2-(4-cyclopropyltriazol-1-yl)-3,3-dimethyl-butanoyl]-4-hydroxy-N-(2-methyl-3,4-dihydro-1H-isoquinolin-4-yl)pyrrolidine-2-carboxamide;
(2S,4R)-1-[(2S)-2-(4-cyclopropyltriazol-1-yl)-3,3-dimethyl-butanoyl]-4-hydroxy-N-(2-isoxazol-5-ylethyl)pyrrolidine-2-carboxamide;
(2S,4R)-1-[(2S)-2-(4-cyclopropyltriazol-1-yl)-3,3-dimethyl-butanoyl]-4-hydroxy-N-[[2-(2,2,2-trifluoroethyl)pyrazol-3-yl]methyl]pyrrolidine-2-carboxamide;
(2S,4R)-N-[cyclopropyl-(4-methylthiazol-2-yl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(3-pyrrol-1-ylpropyl)pyrrolidine-2-carboxamide;
(2S,4R)-1-[(2S)-2-(4-cyclopropyltriazol-1-yl)-3,3-dimethyl-butanoyl]-N-[2-(5-ethyl-2-oxo-indolin-3-yl)ethyl]-4-hydroxy-pyrrolidine-2-carboxamide;
(2S,4R)-N-(8-cyclopropyl-8-azabicyclo[3.2.1]octan-3-yl)-1-[(2S)-2-(4-cyclopropyltriazol-1-yl)-3,3-dimethyl-butanoyl]-4-hydroxy-pyrrolidine-2-carboxamide;
N-(4-bromophenyl)-4-[[(2S,4R)-1-[(2S)-2-(4-cyclopropyltriazol-1-yl)-3,3-dimethylbutanoyl]-4-hydroxy-pyrrolidine-2-carbonyl]amino]piperidine-1-carboxamide;
(2S,4R)-1-[(2S)-2-(4-cyclopropyltriazol-1-yl)-3,3-dimethyl-butanoyl]-N-[2-(2,5-dichlorophenyl)ethyl]-4-hydroxy-pyrrolidine-2-carboxamide;
(2S,4R)-1-[(2S)-2-(4-cyclopropyltriazol-1-yl)-3,3-dimethyl-butanoyl]-N-[1-[4-(difluoromethyl)pyrimidin-2-yl]ethyl]-4-hydroxy-pyrrolidine-2-carboxamide;
(2S,4R)-1-[(2S)-2-(4-cyclopropyltriazol-1-yl)-3,3-dimethyl-butanoyl]-N-[(3S,4R)-1-[2-(dimethylamino)pyrimidin-4-yl]-4-methoxy-pyrrolidin-3-yl]-4-hydroxy-pyrrolidine-2-carboxamide;
(2S,4R)-N-[(2-chloro-6-imidazol-1-yl-phenyl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[1-phenyl-2-(trifluoromethyl)cyclopropyl]pyrrolidine-2-carboxamide;
(2S,4R)-1-[(2S)-2-(4-cyclopropyltriazol-1-yl)-3,3-dimethyl-butanoyl]-4-hydroxy-N-[1-(1-methylpyrazol-4-yl)sulfonyl-4-piperidyl]pyrrolidine-2-carboxamide;
(2S,4R)-1-[(2S)-2-(4-cyclopropyltriazol-1-yl)-3,3-dimethyl-butanoyl]-4-hydroxy-N-[1-(1,2,4-oxadiazol-3-yl)cyclopropyl]pyrrolidine-2-carboxamide;
(2S,4R)-1-[(2S)-2-(4-cyclopropyltriazol-1-yl)-3,3-dimethyl-butanoyl]-N-[[5-(dimethylcarbamoyl)tetrahydrofuran-2-yl]methyl]-4-hydroxy-pyrrolidine-2-carboxamide;
(2S,4R)-1-[(2S)-2-(4-cyclopropyltriazol-1-yl)-3,3-dimethyl-butanoyl]-N-[2-(3,4-difluorophenyl)-2-methyl-cyclopropyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(2S,4R)-2-phenylchroman-4-yl]pyrrolidine-2-carboxamide;
(2S,4R)-N-[(1S)-1-(3-bromoisoxazol-5-yl)ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[2-[2-(dimethylamino)-3-pyridyl]ethyl]-4-hydroxy-pyrrolidine-2-carboxamide;
(2S,4R)-N-[3-(4-acetylpiperazin-1-yl)cyclobutyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[(2-fluorophenyl)-(5-methyl-1,2,4-oxadiazol-3-yl)methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(4-hydroxychroman-3-yl)pyrrolidine-2-carboxamide;
(2S,4R)-N-[1-(5-cyano-2-pyridyl)-4-piperidyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[4,4,4-trifluoro-3-hydroxy-3-(5-methyloxazol-2-yl)butyl]pyrrolidine-2-carboxamide;
(2S,4R)-1-[(2S)-2-(4-cyclopropyltriazol-1-yl)-3,3-dimethyl-butanoyl]-N-[[3-[(3-fluorophenyl)methylsulfamoyl]phenyl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[1-(4,5,6,7-tetrahydropyrazolo[1,5-a]pyridin-2-yl)ethyl]pyrrolidine-2-carboxamide;
(2S,4R)-N-[(1R,3S)-3-(cyanomethyl)cyclopentyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[[3-(2,3-dihydrobenzofuran-5-yl)-4,5-dihydroisoxazol-5-yl]methyl]-4-hydroxy-pyrrolidine-2-carboxamide;
(2S,4R)-N-[[(2S,3R)-1-cyclopropyl-2-(3-methylimidazol-4-yl)-3-piperidyl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[1-methyl-1-(3-methyl-1,2,4-oxadiazol-5-yl)ethyl]pyrrolidine-2-carboxamide;
(2S,4R)-N-[(2-amino-3-methyl-benzimidazol-5-yl)methyl]-1-[(2S)-2-(4-cyclopropyltriazol-1-yl)-3,3-dimethyl-butanoyl]-4-hydroxy-pyrrolidine-2-carboxamide;
(2S,4R)-N-[[1-(1,3-benzodioxol-5-yl)cyclopropyl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(3R)-1-(3-methylisoxazole-5-carbonyl)-3-piperidyl]pyrrolidine-2-carboxamide;
(2S,4R)-1-[(2S)-2-(4-cyclopropyltriazol-1-yl)-3,3-dimethyl-butanoyl]-N-[(3R)-1-(dimethylsulfamoyl)-3-piperidyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(1-tetrahydropyran-4-ylcyclopropyl)methyl]pyrrolidine-2-carboxamide;
(2S,4R)-1-[(2S)-2-(4-cyclopropyltriazol-1-yl)-3,3-dimethyl-butanoyl]-4-hydroxy-N-[2-(1-methyltriazol-4-yl)tetrahydrofuran-3-yl]pyrrolidine-2-carboxamide;
(2S,4R)-1-[(2S)-2-(4-cyclopropyltriazol-1-yl)-3,3-dimethyl-butanoyl]-4-hydroxy-N-[1-methyl-2-(3-methylmorpholin-4-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-(2-sulfamoylpropyl)pyrrolidine-2-carboxamide;
(2S,4R)-N-[2-(4-cyanophenyl)-1,1-dimethyl-ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(4-oxazol-5-ylphenyl)methyl]pyrrolidine-2-carboxamide;
(2S,4R)-1-[(2S)-2-(4-cyclopropyltriazol-1-yl)-3,3-dimethyl-butanoyl]-N-[2-[(4-fluorophenyl)sulfonyl-methyl-amino]ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-(4-isopropylphenoxy)propyl]pyrrolidine-2-carboxamide;
(2S,4R)-1-[(2S)-2-(4-cyclopropyltriazol-1-yl)-3,3-dimethyl-butanoyl]-N-[(1R,2S)-3,3-difluoro-2-hydroxy-cyclopentyl]-4-hydroxy-pyrrolidine-2-carboxamide;
(2S,4R)-1-[(2S)-2-(4-cyclopropyltriazol-1-yl)-3,3-dimethyl-butanoyl]-N-(4,4-difluoro-3-morpholino-butyl)-4-hydroxy-pyrrolidine-2-carboxamide;
(2S,4R)-1-[(2S)-2-(4-cyclopropyltriazol-1-yl)-3,3-dimethyl-butanoyl]-N-[1-(1,1-dioxothietan-3-yl)ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(4-pyrrolidin-1-yltetrahydropyran-4-yl)methyl]pyrrolidine-2-carboxamide;
(2S,4R)-1-[(2S)-2-(4-cyclopropyltriazol-1-yl)-3,3-dimethyl-butanoyl]-4-hydroxy-N-(6-isoquinolylmethyl)pyrrolidine-2-carboxamide;
(2S,4R)-1-[(2S)-2-(4-cyclopropyltriazol-1-yl)-3,3-dimethyl-butanoyl]-N-[2-(1,1-dioxo-1,4-thiazinan-4-yl)-1-methyl-2-oxo-ethyl]-4-hydroxy-pyrrolidine-2-carboxamide;
(2S,4R)-1-[(2S)-2-(4-cyclopropyltriazol-1-yl)-3,3-dimethyl-butanoyl]-N-[2-(1,1-dioxothiolan-2-yl)ethyl]-4-hydroxy-pyrrolidine-2-carboxamide;
(2S,4R)-1-[(2S)-2-(4-cyclopropyltriazol-1-yl)-3,3-dimethyl-butanoyl]-N-[[5-fluoro-2-(methylsulfonylmethyl)phenyl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(6-methoxypyrazin-2-yl)methyl]pyrrolidine-2-carboxamide;
(2S,4R)-1-[(2S)-2-(4-cyclopropyltriazol-1-yl)-3,3-dimethyl-butanoyl]-N-[(4,4-dioxo-1,4-oxathian-2-yl)methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-(5-methylimidazo[1,2-a]pyridin-2-yl)ethyl]pyrrolidine-2-carboxamide;
(2S,4R)-1-[(2S)-2-(4-cyclopropyltriazol-1-yl)-3,3-dimethyl-butanoyl]-N-[[3-[(3,5-dimethylpyrazol-1-yl)methyl]phenyl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[4-(1,3,4-thiadiazol-2-yl)morpholin-2-yl]methyl]pyrrolidine-2-carboxamide;
(2S,4R)-N-[2-(4-cyano-2-methyl-phenyl)ethyl]-1-[(2S)-2-(4-cyclopropyltriazol-1-yl)-3,3-dimethyl-butanoyl]-4-hydroxy-pyrrolidine-2-carboxamide;
(2S,4R)-N-[(5-bromo-2-chloro-phenyl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[(2S,3R)-2-(4-fluorophenyl)-1-methyl-pyrrolidin-3-yl]-4-hydroxy-pyrrolidine-2-carboxamide;
(2S,4R)-1-[(2S)-2-(4-cyclopropyltriazol-1-yl)-3,3-dimethyl-butanoyl]-N-(8,8-dioxo-8lambda6-thiabicyclo[3.2.1]octan-3-yl)-4-hydroxy-pyrrolidine-2-carboxamide;
(2S,4R)-N-[1-(1,3-benzoxazol-2-yl)-2-phenyl-ethyl]-1-[(2S)-2-(4-cyclopropyltriazol-1-yl)-3,3-dimethyl-butanoyl]-4-hydroxy-pyrrolidine-2-carboxamide;
(2S,4R)-N-[1-(1,3-benzothiazol-2-yl)-4-piperidyl]-1-[(2S)-2-(4-cyclopropyltriazol-1-yl)-3,3-dimethyl-butanoyl]-4-hydroxy-pyrrolidine-2-carboxamide;
(2S,4R)-N-(3-cyano-1-phenyl-propyl)-1-[(2S)-2-(4-cyclopropyltriazol-1-yl)-3,3-dimethylbutanoyl]-4-hydroxy-pyrrolidine-2-carboxamide;
(2S,4R)-1-[(2S)-2-(4-cyclopropyltriazol-1-yl)-3,3-dimethyl-butanoyl]-N-[1-[5-fluoro-2-(4-hydroxy-1-piperidyl)phenyl]ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[1-[p-tolyl(1,2,4-triazol-1-yl)methyl]propyl]pyrrolidine-2-carboxamide;
(2S,4R)-1-[(2S)-2-(4-cyclopropyltriazol-1-yl)-3,3-dimethyl-butanoyl]-4-hydroxy-N-[[4-[(1-methyl-4-piperidyl)oxy]phenyl]methyl]pyrrolidine-2-carboxamide;
(2S,4R)-1-[(2S)-2-(4-cyclopropyltriazol-1-yl)-3,3-dimethyl-butanoyl]-N-[1-[5-(difluoromethyl)-1,2,4-oxadiazol-3-yl]cyclobutyl]-4-hydroxy-pyrrolidine-2-carboxamide;
(2S,4R)-N-[(1R,2R)-2-benzamidocyclohexyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[1-[3-(4-fluoro-3-methyl-phenyl)-1,2,4-oxadiazol-5-yl]ethyl]-4-hydroxy-pyrrolidine-2-carboxamide;
(2S,4R)-1-[(2S)-2-(4-cyclopropyltriazol-1-yl)-3,3-dimethyl-butanoyl]-N-[(1R,2S)-2-(3-ethoxyphenyl)cyclopropyl]-4-hydroxy-pyrrolidine-2-carboxamide;
(2S,4R)-N-[2-[(5-cyano-2-pyridyl)oxy]ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[1-[4-(trifluoromethyl)thiazol-2-yl]propyl]pyrrolidine-2-carboxamide;
N-[2-[[(2S,4R)-1-[(2S)-2-(4-cyclopropyltriazol-1-yl)-3,3-dimethyl-butanoyl]-4-hydroxy-pyrrolidine-2-carbonyl]amino]ethyl]morpholine-4-carboxamide;
(2S,4R)-N-[[3-(1-cyanopropoxy)phenyl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[[5-(dimethylamino)-1,3,4-oxadiazol-2-yl]methyl]-4-hydroxy-pyrrolidine-2-carboxamide;
(2S,4R)-N-[2-[(4-cyano-2-pyridyl)amino]ethyl]-1-[(2S)-2-(4-cyclopropyltriazol-1-yl)-3,3-dimethyl-butanoyl]-4-hydroxy-pyrrolidine-2-carboxamide;
(2S,4R)-N-[[2-(cyclopentoxy)-3-methoxy-phenyl]methyl]-1-[(2S)-2-(4-cyclopropyltriazol-1-yl)-3,3-dimethyl-butanoyl]-4-hydroxy-pyrrolidine-2-carboxamide;
(2S,4R)-N-(6-carbamoylspiro[3.3]heptan-2-yl)-1-[(2S)-2-(4-cyclopropyltriazol-1-yl)-3,3-dimethyl-butanoyl]-4-hydroxy-pyrrolidine-2-carboxamide;
(2S,4R)-N-[1-carbamoyl-3-(hydroxymethyl)cyclobutyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[(5-ethyl-3-methyl-isoxazol-4-yl)methyl]-4-hydroxy-pyrrolidine-2-carboxamide;
(2S,4R)-1-[(2S)-2-(4-cyclopropyltriazol-1-yl)-3,3-dimethyl-butanoyl]-N-[2-(1,1-dioxothiolan-3-yl)oxyethyl]-4-hydroxy-pyrrolidine-2-carboxamide;
(2S,4R)-N-[1-benzyl-2-oxo-2-(1-piperidyl)ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-(2-ethyl-3-oxo-isoxazolidin-4-yl)-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[1-[5-methyl-1-[3-(trifluoromethyl)phenyl]pyrazol-4-yl]ethyl]pyrrolidine-2-carboxamide;
(2S,4R)-N-[3-[(2-chlorophenyl)sulfonylamino]propyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[3-(methanesulfonamido)cyclobutyl]pyrrolidine-2-carboxamide;
(2S,4R)-1-[(2S)-2-(4-cyclopropyltriazol-1-yl)-3,3-dimethyl-butanoyl]-4-hydroxy-N-[(3R)-1-(4,4,4-trifluorobutanoyl)-3-piperidyl]pyrrolidine-2-carboxamide;
(2S,4R)-N-[[1-(cyclobutylmethyl)pyrazol-4-yl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[(3,3-dimethyl-5-oxo-pyrrolidin-2-yl)methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[1-(methanesulfonamido)cyclohexyl]methyl]pyrrolidine-2-carboxamide;
(2S,4R)-N-[(3-bromo-5-methoxy-phenyl)methyl]-1-[(2S)-2-(4-cyclopropyltriazol-1-yl)-3,3-dimethyl-butanoyl]-4-hydroxy-pyrrolidine-2-carboxamide;
(2S,4R)-N-[3-[cyclobutanecarbonyl(cyclopropyl)amino]propyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-(2-phenylpyrazol-3-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[3-(4-methoxyphenyl)cyclobutyl]pyrrolidine-2-carboxamide;
(2S,4R)-1-[(2S)-2-(4-cyclopropyltriazol-1-yl)-3,3-dimethyl-butanoyl]-N-[(1,1-dioxo-3,4-dihydro-2H-thiochromen-4-yl)methyl]-4-hydroxy-pyrrolidine-2-carboxamide;
5-bromo-N-[2-[[(2S,4R)-1-[(2S)-2-(4-cyclopropyltriazol-1-yl)-3,3-dimethyl-butanoyl]-4-hydroxy-pyrrolidine-2-carbonyl]amino]ethyl]pyridine-2-carboxamide;
(2S,4R)-1-[(2S)-2-(4-cyclopropyltriazol-1-yl)-3,3-dimethyl-butanoyl]-4-hydroxy-N-[[(3S)-isochroman-3-yl]methyl]pyrrolidine-2-carboxamide;
(2S,4R)-1-[(2S)-2-(4-cyclopropyltriazol-1-yl)-3,3-dimethyl-butanoyl]-N-[(8-fluoroimidazo[1,2-a]pyridin-2-yl)methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[3-(1-methylimidazol-2-yl)phenyl]methyl]pyrrolidine-2-carboxamide;
(2S,4R)-1-[(2S)-2-(4-cyclopropyltriazol-1-yl)-3,3-dimethyl-butanoyl]-N-[[1-ethyl-6-methyl-4-(tetrahydropyran-4-ylamino)pyrazolo[3,4-b]pyridin-5-yl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[1-[(1-methylimidazol-2-yl)methyl]-2-oxo-3-piperidyl]pyrrolidine-2-carboxamide;
(2S,4R)-1-[(2S)-2-(4-cyclopropyltriazol-1-yl)-3,3-dimethyl-butanoyl]-4-hydroxy-N-(4-methylsulfonylcyclohexyl)pyrrolidine-2-carboxamide;
(2S,4R)-1-[(2S)-2-(4-cyclopropyltriazol-1-yl)-3,3-dimethyl-butanoyl]-4-hydroxy-N-[[(1S,2R)-1-methylsulfonyl-2-(trifluoromethyl)cyclopropyl]methyl]pyrrolidine-2-carboxamide;
(2S,4R)-1-[(2S)-2-(4-cyclopropyltriazol-1-yl)-3,3-dimethyl-butanoyl]-4-hydroxy-N-[1-[4-(trifluoromethyl)pyrimidin-2-yl]cyclopropyl]pyrrolidine-2-carboxamide;
(2S,4R)-1-[(2S)-2-(4-cyclopropyltriazol-1-yl)-3,3-dimethyl-butanoyl]-4-hydroxy-N-[2-(5-methylisoxazol-4-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[3-(tetrazol-1-yl)propyl]pyrrolidine-2-carboxamide;
(2S,4R)-1-[(2S)-2-(4-cyclopropyltriazol-1-yl)-3,3-dimethyl-butanoyl]-4-hydroxy-N-(3-morpholinotetrahydropyran-4-yl)pyrrolidine-2-carboxamide;
(2S,4R)-1-[(2S)-2-(4-cyclopropyltriazol-1-yl)-3,3-dimethyl-butanoyl]-4-hydroxy-N-[1-(1-methylpyrazol-4-yl)-6-oxo-3-piperidyl]pyrrolidine-2-carboxamide;
(2S,4R)-1-[(2S)-2-(4-cyclopropyltriazol-1-yl)-3,3-dimethyl-butanoyl]-4-hydroxy-N-[2-(4-pyridylamino)ethyl]pyrrolidine-2-carboxamide;
(2S,4R)-1-[(2S)-2-(4-cyclopropyltriazol-1-yl)-3,3-dimethyl-butanoyl]-N-[[(1S,3R)-2,2-difluoro-3-methyl-cyclopropyl]methyl]-4-hydroxy-pyrrolidine-2-carboxamide;
(2S,4R)-1-[(2S)-2-(4-cyclopropyltriazol-1-yl)-3,3-dimethyl-butanoyl]-N-[[(1R,6R)-7,7-difluoro-2-oxabicyclo[4.1.0]heptan-1-yl]methyl]-4-hydroxy-pyrrolidine-2-carboxamide;
(2S,4R)-1-[(2S)-2-(4-cyclopropyltriazol-1-yl)-3,3-dimethyl-butanoyl]-N-[[4-(2,6-dimethylmorpholine-4-carbonyl)phenyl]methyl]-4-hydroxy-pyrrolidine-2-carboxamide;
(2S,4R)-N-[4-cyano-4-(2-pyridyl)cyclohexyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-[2-(methylcarbamoyl)pyrrolidin-1-yl]-2-oxo-ethyl]pyrrolidine-2-carboxamide;
(2S,4R)-1-[(2S)-2-(4-cyclopropyltriazol-1-yl)-3,3-dimethyl-butanoyl]-4-hydroxy-N-[3-[4-[(2-methyl-1-piperidyl)sulfonyl]phenyl]propyl]pyrrolidine-2-carboxamide;
(2S,4R)-1-[(2S)-2-(4-cyclopropyltriazol-1-yl)-3,3-dimethyl-butanoyl]-4-hydroxy-N-[(2-phenyl-1,4-dioxan-2-yl)methyl]pyrrolidine-2-carboxamide;
(2S,4R)-1-[(2S)-2-(4-cyclopropyltriazol-1-yl)-3,3-dimethyl-butanoyl]-4-hydroxy-N-[3-[isopropyl(methylsulfonyl)amino]propyl]pyrrolidine-2-carboxamide;
(2S,4R)-1-[(2S)-2-(4-cyclopropyltriazol-1-yl)-3,3-dimethyl-butanoyl]-N-(1-cyclopropyl-3,3,3-trifluoro-propyl)-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(2-methyl-4,5,6,7-tetrahydroindazol-3-yl)methyl]pyrrolidine-2-carboxamide;
(2S,4R)-1-[(2S)-2-(4-cyclopropyltriazol-1-yl)-3,3-dimethyl-butanoyl]-4-hydroxy-N-[1-(4-pyridyl)azetidin-3-yl]pyrrolidine-2-carboxamide;
(2S,4R)-1-[(2S)-2-(4-cyclopropyltriazol-1-yl)-3,3-dimethyl-butanoyl]-4-hydroxy-N-(2-hydroxy-2-thiazol-5-yl-ethyl)pyrrolidine-2-carboxamide;
(2S,4R)-1-[(2S)-2-(4-cyclopropyltriazol-1-yl)-3,3-dimethyl-butanoyl]-4-hydroxy-N-[(1R,2S)-2-isopropoxy-2-methyl-cyclopropyl]pyrrolidine-2-carboxamide;
(2S,4R)-1-[(2S)-2-(4-cyclopropyltriazol-1-yl)-3,3-dimethyl-butanoyl]-4-hydroxy-N-[2-oxo-2-(2,3,4,5-tetrahydro-1-benzazepin-1-yl)ethyl]pyrrolidine-2-carboxamide;
(2S,4R)-N-[2-(1-benzhydryl-3-hydroxy-azetidin-3-yl)ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[2-(3-methylpyrazol-1-yl)phenyl]methyl]pyrrolidine-2-carboxamide;
(2S,4R)-N-[(4-cyclopropyl-5-methyl-1,2,4-triazol-3-yl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(1-methyl-1-thiazol-2-yl-propyl)pyrrolidine-2-carboxamide;
(2S,4R)-1-[(2S)-2-(4-cyclopropyltriazol-1-yl)-3,3-dimethyl-butanoyl]-4-hydroxy-N-[2-(p-tolyl)-1-(2-pyridyl)ethyl]pyrrolidine-2-carboxamide;
(2S,4R)-1-[(2S)-2-(4-cyclopropyltriazol-1-yl)-3,3-dimethyl-butanoyl]-N-[(3,4-difluorophenyl)-(1-methylimidazol-2-yl)methyl]-4-hydroxy-pyrrolidine-2-carboxamide;
(2S,4R)-1-[(2S)-2-(4-cyclopropyltriazol-1-yl)-3,3-dimethyl-butanoyl]-N-[1-(3,5-dichlorophenyl)-3-hydroxy-propyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[3-(4-methylpiperazine-1-carbonyl)-4,5,6,7-tetrahydropyrazolo[1,5-a]pyridin-5-yl]methyl]pyrrolidine-2-carboxamide;
(2S,4R)-N-[1-(4-benzylmorpholin-2-yl)propyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[(1S,3R)-3-(dimethylamino)cyclopentyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[1-[(3-methyl-1,2,4-oxadiazol-5-yl)methyl]cyclopropyl]pyrrolidine-2-carboxamide;
(2S,4R)-1-[(2S)-2-(4-cyclopropyltriazol-1-y1)-3,3-dimethyl-butanoyl]-N-[(1S,3R)-3-[(5-ethyl-1,3,4-oxadiazol-2-yl)methyl]cyclopentyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(1-methylimidazo[1,2-b]pyrazol-7-yl)methyl]pyrrolidine-2-carboxamide;
(2S,4R)-1-[(2S)-2-(4-cyclopropyltriazol-1-yl)-3,3-dimethyl-butanoyl]-4-hydroxy-N-[[(2S)-1-(p-tolyl)pyrrolidin-2-yl]methyl]pyrrolidine-2-carboxamide;
(2S,4R)-1-[(2S)-2-(4-cyclopropyltriazol-1-yl)-3,3-dimethyl-butanoyl]-N-[1-(3,4-dichlorophenyl)pyrrolidin-3-yl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(3-phenylsulfanylcyclobutyl)pyrrolidine-2-carboxamide;
(2S,4R)-1-[(2S)-2-(4-cyclopropyltriazol-1-yl)-3,3-dimethyl-butanoyl]-N-[(4,6-dimethyl-3-pyridyl)methyl]-4-hydroxy-pyrrolidine-2-carboxamide;
(2S,4R)-N-[2-[benzyl(ethyl)amino]-2-oxo-ethyl]-1-[(2S)-2-(4-cyclopropyltriazol-1-yl)-3,3-dimethyl-butanoyl]-4-hydroxy-pyrrolidine-2-carboxamide;
(2S,4R)-N-[2-(4-acetamidophenyl)ethyl]-1-[(2S)-2-(4-cyclopropyltriazol-1-yl)-3,3-dimethylbutanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(2-isopropyl-4-methyl-oxazol-5-yl)methyl]pyrrolidine-2-carboxamide;
(2S,4R)-N-[2-(4-bromoindol-1-yl)ethyl]-1-[(2S)-2-(4-cyclopropyltriazol-1-yl)-3,3-dimethylbutanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[4-(isobutylsulfamoyl)phenyl]methyl]pyrrolidine-2-carboxamide;
(2S,4R)-1-[(2S)-2-(4-cyclopropyltriazol-1-yl)-3,3-dimethyl-butanoyl]-N-[[1-(difluoromethyl)imidazol-2-yl]methyl]-4-hydroxy-pyrrolidine-2-carboxamide;
(2S,4R)-N-[4-(4-cyanophenyl)tetrahydropyran-4-yl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-(6-methylpyrazin-2-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[(1S,2R)-2-hydroxy-1,2-diphenyl-ethyl]pyrrolidine-2-carboxamide;
(2S,4R)-N-[2-[benzyl(2-pyridyl)amino]ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(1-methylbenzimidazol-2-yl)-phenyl-methyl]pyrrolidine-2-carboxamide;
(2S,4R)-1-[(2S)-2-(4-cyclopropyltriazol-1-yl)-3,3-dimethyl-butanoyl]-4-hydroxy-N-[(4-hydroxy-1-isopropyl-4-piperidyl)methyl]pyrrolidine-2-carboxamide;
(2S,4R)-1-[(2S)-2-(4-cyclopropyltriazol-1-yl)-3,3-dimethyl-butanoyl]-4-hydroxy-N-[[3-(3-oxopiperazin-1-yl)sulfonylphenyl]methyl]pyrrolidine-2-carboxamide;
(2S,4R)-N-[3,3-bis(hydroxymethyl)cyclobutyl]-1-[(2S)-2-(4-cyclopropyltriazol-1-yl)-3,3-dimethyl-butanoyl]-4-hydroxy-pyrrolidine-2-carboxamide;
(2S,4R)-N-(2-anilino-1,1-dimethyl-2-oxo-ethyl)-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-(1,3-dimethylpyrrolidin-3-yl)-4-hydroxy-pyrrolidine-2-carboxamide;
(2S,4R)-N-[(1R,2R)-2-(cyclopropylsulfonylamino)cyclohexyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(4-oxo-3,5-dihydro-2H-1,5-benzoxazepin-3-yl)pyrrolidine-2-carboxamide;
(2S,4R)-1-[(2S)-2-(4-cyclopropyltriazol-1-yl)-3,3-dimethyl-butanoyl]-4-hydroxy-N-[2-(methylcarbamoyl)cyclohexyl]pyrrolidine-2-carboxamide;
(2S,4R)-N-[1-(5-bromo-2-pyridyl)cyclobutyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(3-methylsulfonylphenyl)-(p-tolyl)methyl]pyrrolidine-2-carboxamide;
(2S,4R)-1-[(2S)-2-(4-cyclopropyltriazol-1-yl)-3,3-dimethyl-butanoyl]-4-hydroxy-N-[[3-(trifluoromethyl)-5,6,7,8-tetrahydroimidazo[1,5-a]pyridin-1-yl]methyl]pyrrolidine-2-carboxamide;
(2S,4R)-1-[(2S)-2-(4-cyclopropyltriazol-1-yl)-3,3-dimethyl-butanoyl]-4-hydroxy-N-(4-methyl-4-phenyl-cyclohexyl)pyrrolidine-2-carboxamide;
(2S,4R)-1-[(2S)-2-(4-cyclopropyltriazol-1-yl)-3,3-dimethyl-butanoyl]-4-hydroxy-N-[2-(5-methyl-2-phenyl-oxazol-4-yl)ethyl]pyrrolidine-2-carboxamide;
(2S,4R)-1-[(2S)-2-(4-cyclopropyltriazol-1-yl)-3,3-dimethyl-butanoyl]-N-[3-(3-ethyl-1,2,4-oxadiazol-5-yl)cyclopentyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(5,6,7,8-tetrahydro-4H-cyclohepta[d]isoxazol-3-ylmethyl)pyrrolidine-2-carboxamide;
(2S,4R)-1-[(2S)-2-(4-cyclopropyltriazol-1-yl)-3,3-dimethyl-butanoyl]-4-hydroxy-N-[1-(1-isopropyltriazol-4-yl)-1-methyl-ethyl]pyrrolidine-2-carboxamide;
(2S,4R)-1-[(2S)-2-(4-cyclopropyltriazol-1-yl)-3,3-dimethyl-butanoyl]-4-hydroxy-N-[2-[3-(trifluoromethyl)phenoxy]butyl]pyrrolidine-2-carboxamide;
(2S,4R)-N-[2-amino-1-methyl-2-oxo-1-(pyrazol-1-ylmethyl)ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(3-methylquinuclidin-3-yl)pyrrolidine-2-carboxamide;
(2S,4R)-N-[(7-bromo-4-oxo-pyrido[1,2-a]pyrimidin-2-yl)methyl]-1-[(2S)-2-(4-cyclopropyltriazol-1-yl)-3,3-dimethyl-butanoyl]-4-hydroxy-pyrrolidine-2-carboxamide;
(2S,4R)-N-[2-[cyclopropyl(methyl)amino]propyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[2-[(2,4-dichlorophenyl)methoxy]ethyl]-4-hydroxy-pyrrolidine-2-carboxamide;
(2S,4R)-N-[3-(5-cyclopropyl-4-phenyl-1,2,4-triazol-3-yl)propyl]-1-[(2S)-2-(4-cyclopropyltriazol-1-yl)-3,3-dimethyl-butanoyl]-4-hydroxy-pyrrolidine-2-carboxamide;
(2S,4R)-N-[2-(5-chloroindol-1-yl)ethyl]-1-[(2S)-2-(4-cyclopropyltriazol-1-yl)-3,3-dimethylbutanoyl]-4-hydroxy-pyrrolidine-2-carboxamide;
(2S,4R)-N-[(5-bromo-1,3-benzothiazol-2-yl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[3-(4-isobutyl-5-methylsulfanyl-1,2,4-triazol-3-yl)propyl]pyrrolidine-2-carboxamide;
(2S,4R)-N-[2-(2-aminothiazol-5-yl)ethyl]-1-[(2S)-2-(4-cyclopropyltriazol-1-yl)-3,3-dimethyl-butanoyl]-4-hydroxy-pyrrolidine-2-carboxamide;
(2S,4R)-N-[(4-cyanocyclohexyl)methyl]-1-[(2S)-2-(4-cyclopropyltriazol-1-yl)-3,3-dimethylbutanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[1-(4-hydroxy-6-isopropyl-pyrimidin-2-yl)pyrrolidin-3-yl]pyrrolidine-2-carboxamide;
(2S,4R)-N-[(2-cyclobutylphenyl)methyl]-1-[(2S)-2-(4-cyclopropyltriazol-1-yl)-3,3-dimethylbutanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[5-oxo-1-[[2-(trifluoromethyl)phenyl]methyl]pyrrolidin-3-yl]pyrrolidine-2-carboxamide;
(2S,4R)-1-[(2S)-2-(4-cyclopropyltriazol-1-yl)-3,3-dimethyl-butanoyl]-N-[2-(5-fluoro-1-methyl-indol-3-yl)-1-methyl-ethyl]-4-hydroxy-pyrrolidine-2-carboxamide;
(2S,4R)-N-(1-benzyl-8-oxa-1-azaspiro[4.5]decan-3-yl)-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-(2-methoxy-1-naphthyl)ethyl]pyrrolidine-2-carboxamide;
(2S,4R)-1-[(2S)-2-(4-cyclopropyltriazol-1-yl)-3,3-dimethyl-butanoyl]-4-hydroxy-N-(2-imidazol-1-yl-2-methyl-propyl)pyrrolidine-2-carboxamide;
(2S,4R)-1-[(2S)-2-(4-cyclopropyltriazol-1-yl)-3,3-dimethyl-butanoyl]-4-hydroxy-N-[2-[4-(trifluoromethyl)-2-pyridyl]ethyl]pyrrolidine-2-carboxamide;
(2S,4R)-1-[(2S)-2-(4-cyclopropyltriazol-1-yl)-3,3-dimethyl-butanoyl]-4-hydroxy-N-[1-(1-isopropylpyrazol-4-yl)ethyl]pyrrolidine-2-carboxamide;
(2S,4R)-N-[[5-(3-bromophenyl)oxazol-2-yl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(2-methylindan-2-yl)pyrrolidine-2-carboxamide;
(2S,4R)-1-[(2S)-2-(4-cyclopropyltriazol-1-yl)-3,3-dimethyl-butanoyl]-4-hydroxy-N-(3-tetrahydropyran-2-ylprop-2-ynyl)pyrrolidine-2-carboxamide;
(2S,4R)-1-[(2S)-2-(4-cyclopropyltriazol-1-yl)-3,3-dimethyl-butanoyl]-4-hydroxy-N-(2-indolizin-2-ylethyl)pyrrolidine-2-carboxamide;
tert-butyl (4R,5R)-4-[[[(2S,4R)-1-[(2S)-2-(4-cyclopropyltriazol-1-yl)-3,3-dimethylbutanoyl]-4-hydroxy-pyrrolidine-2-carbonyl]amino]methyl]-2,2,5-trimethyl-oxazolidine-3-carboxylate;
(2S,4R)-1-[(2S)-2-(4-cyclopropyltriazol-1-yl)-3,3-dimethyl-butanoyl]-N-[2-[4-(dimethylamino)-1-piperidyl]-2-methyl-propyl]-4-hydroxy-pyrrolidine-2-carboxamide;
(2S,4R)-N-[[5-(4-chlorophenyl)-1,3,4-oxadiazol-2-yl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-(3-oxopiperazin-1-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[1-methyl-2-(1-methyl-4-oxo-pyrazolo[3,4-d]pyrimidin-5-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[1-[3-(trifluoromethyl)phenyl]cyclopropyl]pyrrolidine-2-carboxamide;
(2S,4R)-1-[(2S)-2-(4-cyclopropyltriazol-1-yl)-3,3-dimethyl-butanoyl]-4-hydroxy-N-(6-oxo-7-azaspiro[3.5]nonan-9-yl)pyrrolidine-2-carboxamide;
(2S,4R)-1-[(2S)-2-(4-cyclopropyltriazol-1-yl)-3,3-dimethyl-butanoyl]-4-hydroxy-N-[1-[2-(trifluoromethyl)phenyl]cyclobutyl]pyrrolidine-2-carboxamide;
(2S,4R)-1-[(2S)-2-(4-cyclopropyltriazol-1-yl)-3,3-dimethyl-butanoyl]-4-hydroxy-N-[(8-oxo-6,7-dihydro-5H-[1,2,4]triazolo[4,3-a]pyrazin-3-yl)methyl]pyrrolidine-2-carboxamide;
tert-butyl 2-[[[(2S,4R)-1-[(2S)-2-(4-cyclopropyltriazol-1-yl)-3,3-dimethyl-butanoyl]-4-hydroxy-pyrrolidine-2-carbonyl]amino]methyl]-4,4-difluoro-2-methyl-pyrrolidine-1-carboxylate;
(2S,4R)-1-[(2S)-2-(4-cyclopropyltriazol-1-yl)-3,3-dimethyl-butanoyl]-4-hydroxy-N-[(1R,3S)-3-(hydroxymethyl)indan-1-yl]pyrrolidine-2-carboxamide;
(2S,4R)-N-[[(2S,3R)-1-tert-butyl-2-[1-(2-methoxyethyl)pyrazol-4-yl]-5-oxo-pyrrolidin-3-yl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-[4-(2-methoxyphenyl)piperazin-1-yl]cyclohexyl]pyrrolidine-2-carboxamide;
(2S,4R)-1-[(2S)-2-(4-cyclopropyltriazol-1-yl)-3,3-dimethyl-butanoyl]-4-hydroxy-N-[(1S,2S,4R,5R)-4-(hydroxymethyl)-2-bicyclo[3.1.0]hexanyl]pyrrolidine-2-carboxamide;
(2S,4R)-1-[(2S)-2-(4-cyclopropyltriazol-1-yl)-3,3-dimethyl-butanoyl]-4-hydroxy-N-[(2-methyl-6-oxo-4-piperidyl)methyl]pyrrolidine-2-carboxamide;
(2S,4R)-1-[(2S)-2-(4-cyclopropyltriazol-1-yl)-3,3-dimethyl-butanoyl]-4-hydroxy-N-[(5-methyl-4-oxo-6,7-dihydropyrazolo[1,5-a]pyrazin-3-yl)methyl]pyrrolidine-2-carboxamide;
(2S,4R)-1-[(2S)-2-(4-cyclopropyltriazol-1-yl)-3,3-dimethyl-butanoyl]-4-hydroxy-N-[2-morpholino-2-(2-thienyl)ethyl]pyrrolidine-2-carboxamide;
(2S,4R)-N-[[2-chloro-6-(difluoromethoxy)phenyl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[2-ethyl-1-(m-tolyl)cyclopropyl]-4-hydroxy-pyrrolidine-2-carboxamide;
(2S,4R)-1-[(2S)-2-(4-cyclopropyltriazol-1-yl)-3,3-dimethyl-butanoyl]-N-[3-(3,5-difluorophenyl)cyclobutyl]-4-hydroxy-pyrrolidine-2-carboxamide;
(2S,4R)-N-[[3-bromo-2-(difluoromethoxy)phenyl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-[3-(trifluoromethyl)pyrazol-1-yl]ethyl]pyrrolidine-2-carboxamide;
(2S,4R)-N-[[5-(4-chloro-2-fluoro-phenyl)-1,2,4-oxadiazol-3-yl]methyl]-1-[(2S)-2-(4-cyclopropyltriazol-1-yl)-3,3-dimethyl-butanoyl]-4-hydroxy-pyrrolidine-2-carboxamide;
(2S,4R)-N-[(1-tert-butylpyrazol-4-yl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(5-methyl-6,7-dihydro-5H-pyrrolo[1,2-b][1,2,4]triazol-7-yl)pyrrolidine-2-carboxamide;
(2S,4R)-1-[(2S)-2-(4-cyclopropyltriazol-1-yl)-3,3-dimethyl-butanoyl]-4-hydroxy-N-(2-phenyl-5,6,7,8-tetrahydroimidazo[1,2-a]pyridin-6-yl)pyrrolidine-2-carboxamide;
(2S,4R)-1-[(2S)-2-(4-cyclopropyltriazol-1-yl)-3,3-dimethyl-butanoyl]-N-[(3S,4S)-4-(dimethylamino)tetrahydrofuran-3-yl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[2-methoxy-6-(trifluoromethoxy)phenyl]methyl]pyrrolidine-2-carboxamide;
(2S,4R)-1-[(2S)-2-(4-cyclopropyltriazol-1-yl)-3,3-dimethyl-butanoyl]-4-hydroxy-N-[2-[(1-methylpyrrol-3-yl)sulfonylamino]ethyl]pyrrolidine-2-carboxamide;
(2S,4R)-1-[(2S)-2-(4-cyclopropyltriazol-1-yl)-3,3-dimethyl-butanoyl]-4-hydroxy-N-(3-pyrimidin-2-yloxypropyl)pyrrolidine-2-carboxamide;
(2S,4R)-1-[(2S)-2-(4-cyclopropyltriazol-1-yl)-3,3-dimethyl-butanoyl]-4-hydroxy-N-[2-(1-methylcyclobutoxy)ethyl]pyrrolidine-2-carboxamide;
(2S,4R)-N-[[4-(2-amino-2-oxo-ethyl)tetrahydrofuran-2-yl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[1-(2-methoxyethyl)-4,5,6,7-tetrahydroindazol-7-yl]pyrrolidine-2-carboxamide;
(2S,4R)-1-[(2S)-2-(4-cyclopropyltriazol-1-yl)-3,3-dimethyl-butanoyl]-N-[2-[[2-(4-fluorophenyl)acetyl]amino]ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(3-methoxy-6-methyl-2-pyridyl)methyl]pyrrolidine-2-carboxamide;
(2S,4R)-1-[(2S)-2-(4-cyclopropyltriazol-1-yl)-3,3-dimethyl-butanoyl]-N-[1-[5-(4-fluorophenyl)oxazol-2-yl]ethyl]-4-hydroxy-pyrrolidine-2-carboxamide;
(2S,4R)-1-[(2S)-2-(4-cyclopropyltriazol-1-yl)-3,3-dimethyl-butanoyl]-N-(2-ethylsulfinylcyclohexyl)-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(2-phenyltriazol-4-yl)methyl]pyrrolidine-2-carboxamide;
(2S,4R)-N-[2-(4-bromo-2-fluoro-phenyl)-1-methyl-ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[(4-cyclopropyl-1,2,4-triazol-3-yl)methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(6-methoxy-[1,2,4]triazolo[4,3-b]pyridazin-3-yl)methyl]pyrrolidine-2-carboxamide;
(2S,4R)-1-[(2S)-2-(4-cyclopropyltriazol-1-yl)-3,3-dimethyl-butanoyl]-4-hydroxy-N-[2-[[3-(trifluoromethyl)-2-pyridyl]amino]ethyl]pyrrolidine-2-carboxamide;
(2S,4R)-1-[(2S)-2-(4-cyclopropyltriazol-1-yl)-3,3-dimethyl-butanoyl]-N-[[1-(5-fluoro-2-pyridyl)cyclopropyl]methyl]-4-hydroxy-pyrrolidine-2-carboxamide;
(2S,4R)-N-[1-(3-cyanophenyl)-1-methyl-ethyl]-1-[(2S)-2-(4-cyclopropyltriazol-1-yl)-3,3-dimethyl-butanoyl]-4-hydroxy-pyrrolidine-2-carboxamide;
(2S,4R)-N-[2-[1-[(4-chlorophenyl)methyl]imidazol-4-yl]ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[2-[(3-fluorophenyl)methyl]-3-morpholino-3-oxo-propyl]-4-hydroxy-pyrrolidine-2-carboxamide;
(2S,4R)-N-[3-(2-cyanoimidazol-1-yl)propyl]-1-[(2S)-2-(4-cyclopropyltriazol-1-yl)-3,3-dimethyl-butanoyl]-4-hydroxy-pyrrolidine-2-carboxamide;
(2S,4R)-N-(3-cyano-8-oxaspiro[4.5]decan-4-yl)-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[2-(2-propoxyphenoxy)-3-pyridyl]methyl]pyrrolidine-2-carboxamide;
(2S,4R)-1-[(2S)-2-(4-cyclopropyltriazol-1-yl)-3,3-dimethyl-butanoyl]-N-[1-(2-fluoro-5-methyl-3-pyridyl)ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[4-(o-tolylmethyl)morpholin-2-yl]methyl]pyrrolidine-2-carboxamide;
(2S,4R)-1-[(2S)-2-(4-cyclopropyltriazol-1-yl)-3,3-dimethyl-butanoyl]-4-hydroxy-N-(2-hydroxy-2-norbornan-2-yl-propyl)pyrrolidine-2-carboxamide;
(2S,4R)-1-[(2S)-2-(4-cyclopropyltriazol-1-yl)-3,3-dimethyl-butanoyl]-4-hydroxy-N-(3-methyl-1,1-dioxo-thietan-3-yl)pyrrolidine-2-carboxamide;
(2S,4R)-1-[(2S)-2-(4-cyclopropyltriazol-1-yl)-3,3-dimethyl-butanoyl]-4-hydroxy-N-[[2-[[2-(hydroxymethyl)pyrrolidin-1-yl]methyl]phenyl]methyl]pyrrolidine-2-carboxamide;
(2S,4R)-1-[(2S)-2-(4-cyclopropyltriazol-1-yl)-3,3-dimethyl-butanoyl]-N-[3-(2,6-difluorophenyl)oxetan-3-yl]-4-hydroxy-pyrrolidine-2-carboxamide;
(2S,4R)-N-[[3-[(4-bromophenoxy)methyl]oxetan-3-yl]methyl]-1-[(2S)-2-(4-cyclopropyltriazol-1-yl)-3,3-dimethyl-butanoyl]-4-hydroxy-pyrrolidine-2-carboxamide;
(2S,4R)-N-[1-(cyclopentylmethyl)-4-piperidyl]-1-[(2S)-2-(4-cyclopropyltriazol-1-yl)-3,3-dimethyl-butanoyl]-4-hydroxy-pyrrolidine-2-carboxamide;
(2S,4R)-N-[[(3S,5R)-4-benzyl-3-(hydroxymethyl)-5-methyl-moipholin-3-yl]methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[4-(3-methylisoxazol-5-yl)tetrahydropyran-4-yl]pyrrolidine-2-carboxamide;
(2S,4R)-1-[(2S)-2-(4-cyclopropyltriazol-1-yl)-3,3-dimethyl-butanoyl]-4-hydroxy-N-(2,3,4,5-tetrahydrobenzo[i][1]benzoxepin-5-yl)pyrrolidine-2-carboxamide;
(2S,4R)-1-[(2S)-2-(4-cyclopropyltriazol-1-yl)-3,3-dimethyl-butanoyl]-N-(2,3-dimethoxy-6,7,8,9-tetrahydro-5H-benzo[7]annulen-5-yl)-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-[2-(trifluoromethyl)phenyl]cyclopentyl]pyrrolidine-2-carboxamide;
(2S,4R)-1-[(2S)-2-(4-cyclopropyltriazol-1-yl)-3,3-dimethyl-butanoyl]-4-hydroxy-N-[3,3,3-trifluoro-1-(o-tolyl)propyl]pyrrolidine-2-carboxamide;
(2S,4R)-1-[(2S)-2-(4-cyclopropyltriazol-1-yl)-3,3-dimethyl-butanoyl]-4-hydroxy-N-[1-(4-methoxy-2-naphthyl)ethyl]pyrrolidine-2-carboxamide;
(2S,4R)-1-[(2S)-2-(4-cyclopropyltriazol-1-yl)-3,3-dimethyl-butanoyl]-N-[1-[2-(difluoromethoxy)phenyl]pyrrolidin-3-yl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-[3-(methylsulfamoyl)anilino]-2-oxo-ethyl]pyrrolidine-2-carboxamide;
(2S,4R)-N-[3-(benzenesulfonyl)cyclobutyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(1S,3R)-3-(isopropylcarbamoyl)cyclohexyl]pyrrolidine-2-carboxamide;
(2S,4R)-1-[(2S)-2-(4-cyclopropyltriazol-1-yl)-3,3-dimethyl-butanoyl]-4-hydroxy-N-[1-(methoxymethyl)-2-tetrahydropyran-4-yl-ethyl]pyrrolidine-2-carboxamide;
(2S,4R)-1-[(2S)-2-(4-cyclopropyltriazol-1-yl)-3,3-dimethyl-butanoyl]-N-[(1,1-dioxothian-3-yl)methyl]-4-hydroxy-pyrrolidine-2-carboxamide;
(2S,4R)-1-[(2S)-2-(4-cyclopropyltriazol-1-yl)-3,3-dimethyl-butanoyl]-N-(6,7-dihydro-5H-cyclopenta[c]pyridin-4-ylmethyl)-4-hydroxy-pyrrolidine-2-carboxamide;
(2S,4R)-1-[(2S)-2-(4-cyclopropyltriazol-1-yl)-3,3-dimethyl-butanoyl]-N-[[5-(dimethylamino)pyrazin-2-yl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(3-tetralin-1-yloxypropyl)pyrrolidine-2-carboxamide;
(2S,4R)-1-[(2S)-2-(4-cyclopropyltriazol-1-yl)-3,3-dimethyl-butanoyl]-4-hydroxy-N-[1-(2-methoxyethyl)-3-piperidyl]pyrrolidine-2-carboxamide;
(2S,4R)-1-[(2S)-2-(4-cyclopropyltriazol-1-yl)-3,3-dimethyl-butanoyl]-4-hydroxy-N-[(1-tetrahydropyran-4-yl-4-piperidyl)methyl]pyrrolidine-2-carboxamide;
(2S,4R)-N-(1,2-benzoxazol-3-ylmethyl)-1-[(2S)-2-(4-cyclopropyltriazol-1-yl)-3,3-dimethylbutanoyl]-4-hydroxy-pyrrolidine-2-carboxamide;
(2S,4R)-1-[(2S)-2-(4-cyclopropyltriazol-1-yl)-3,3-dimethyl-butanoyl]-N-[2-(3-fluoro-4-pyridyl)ethyl]-4-hydroxy-pyrrolidine-2-carboxamide;
(2S,4R)-1-[(2S)-2-(4-cyclopropyltriazol-1-yl)-3,3-dimethyl-butanoyl]-N-[2-(3-cyclopropyl-1,2,4-triazol-4-yl)ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(5-oxopyrrolidin-3-yl)pyrrolidine-2-carboxamide;
(2S,4R)-N-[(4-aminopyrimidin-5-yl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[1-(1-morpholinocyclopentyl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[2-(4-oxothieno[3,2-d]pyrimidin-3-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-(2-pyrimidin-5-ylethyl)pyrrolidine-2-carboxamide;
(2S,4R)-1-[(2S)-2-(4-cyclopropyltriazol-1-yl)-3,3-dimethyl-butanoyl]-4-hydroxy-N-(1-tetrahydropyran-4-ylsulfonyl-4-piperidyl)pyrrolidine-2-carboxamide;
(2S,4R)-1-[(2S)-2-(4-cyclopropyltriazol-1-yl)-3,3-dimethyl-butanoyl]-4-hydroxy-N-(5,6,7,8-tetrahydrotetrazolo[1,5-a]pyridin-7-yl)pyrrolidine-2-carboxamide;
(2S,4R)-1-[(2S)-2-(4-cyclopropyltriazol-1-yl)-3,3-dimethyl-butanoyl]-4-hydroxy-N-[(2R,3S)-2-(3-methyl-1,2,4-oxadiazol-5-yl)tetrahydrofuran-3-yl]pyrrolidine-2-carboxamide;
(2S,4R)-1-[(2S)-2-(4-cyclopropyltriazol-1-yl)-3,3-dimethyl-butanoyl]-4-hydroxy-N-[(1R,3R)-3-(4-methylpiperazin-1-yl)indan-1-yl]pyrrolidine-2-carboxamide;
(2S,4R)-1-[(2S)-2-(4-cyclopropyltriazol-1-yl)-3,3-dimethyl-butanoyl]-4-hydroxy-N-[2-(7-methyl-8-oxo-5,6-dihydro-[1,2,4]triazolo[4,3-a]pyrazin-3-yl)ethyl]pyrrolidine-2-carboxamide;
(2S,4R)-1-[(2S)-2-(4-cyclopropyltriazol-1-yl)-3,3-dimethyl-butanoyl]-N-[2-(3,4-difluorophenyl)-3-methyl-butyl]-4-hydroxy-pyrrolidine-2-carboxamide;
(2S,4R)-1-[(2S)-2-(4-cyclopropyltriazol-1-yl)-3,3-dimethyl-butanoyl]-N-[[4-(dimethylamino)-2-pyridyl]methyl]-4-hydroxy-pyrrolidine-2-carboxamide;
(2S,4R)-1-[(2S)-2-(4-cyclopropyltriazol-1-yl)-3,3-dimethyl-butanoyl]-N-[[2-(dimethylamino)-6-methyl-pyrimidin-4-yl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(4-isoindolin-2-yl-4-oxo-butyl)pyrrolidine-2-carboxamide;
(2S,4R)-N-[1-(1-cyclopropyltetrazol-5-yl)ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-(2-methyloxazol-5-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-(4-methyl-2-oxo-3-piperidyl)pyrrolidine-2-carboxamide;
(2S,4R)-N-[(1-cyclopropyl-2-ethyl-benzimidazol-5-yl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(1-methoxy-6,7,8,9-tetrahydro-5H-benzo[7]annulen-6-yl)pyrrolidine-2-carboxamide;
(2S,4R)-N-[2-(4-chlorophenyl)-2-(cyclopropylmethoxy)ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(1-tetrahydrofuran-2-ylcyclobutyl)methyl]pyrrolidine-2-carboxamide;
(2S,4R)-N-[1-[5-(3-bromophenyl)-1,2,4-oxadiazol-3-yl]propyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[(4S)-6,8-dimethylchroman-4-yl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(isochroman-4-ylmethyl)pyrrolidine-2-carboxamide;
(2S,4R)-N-[4-(5-chloro-2-thienyl)butyl]-1-[(2S)-2-(4-cyclopropyltriazol-1-yl)-3,3-dimethylbutanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(3-isopropoxytetrahydrofuran-3-yl)methyl]pyrrolidine-2-carboxamide;
(2S,4R)-1-[(2S)-2-(4-cyclopropyltriazol-1-yl)-3,3-dimethyl-butanoyl]-N-[(3R)-1-(1,3-dimethyl-2,6-dioxo-pyrimidin-4-yl)pyrrolidin-3-yl]-4-hydroxy-pyrrolidine-2-carboxamide;
(2S,4R)-N-[1-(5-chloro-2,3-dihydrobenzofuran-7-yl)ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[2-(2,2-difluorocyclohexyl)ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(1-oxoisoindolin-5-yl)methyl]pyrrolidine-2-carboxamide;
(2S,4R)-1-[(2S)-2-(4-cyclopropyltriazol-1-yl)-3,3-dimethyl-butanoyl]-N-[2-(1-ethylbenzimidazol-2-yl)ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-([1,2,4]triazolo[1,5-a]pyrimidin-2-ylmethyl)pyrrolidine-2-carboxamide;
(2S,4R)-N-[2-(3-bromo-5-chloro-phenyl)ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[1-(4-methoxy-2-pyridyl)cyclohexyl]methyl]pyrrolidine-2-carboxamide;
(2S,4R)-1-[(2S)-2-(4-cyclopropyltriazol-1-yl)-3,3-dimethyl-butanoyl]-4-hydroxy-N-[[1-(1,2,4-triazol-1-yl)cyclopropyl]methyl]pyrrolidine-2-carboxamide;
(2S,4R)-1-[(2S)-2-(4-cyclopropyltriazol-1-yl)-3,3-dimethyl-butanoyl]-4-hydroxy-N-spiro[2-oxabicyclo[3.2.0]heptane-7,1'-cyclobutane]-6-yl-pyrrolidine-2-carboxamide;
(2S,4R)-N-[2-[cyclohexyl(ethyl)sulfamoyl]ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(2-methyl-1,1-dioxo-thian-4-yl)methyl]pyrrolidine-2-carboxamide;
(2S,4R)-N-[1-[1-(4-chlorophenyl)cyclobutyl]ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[1-(2-fluoro-3-methoxy-phenyl)cyclopropyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(1R,2S,4R,SR)-5-methoxy-5-(trifluoromethyl)norbornan-2-yl]pyrrolidine-2-carboxamide;
(2S,4R)-1-[(2S)-2-(4-cyclopropyltriazol-1-yl)-3,3-dimethyl-butanoyl]-4-hydroxy-N-[3-(6-oxo-1,3,4,7,8,8a-hexahydropyrrolo[1,2-a]pyrazin-2-yl)cyclopentyl]pyrrolidine-2-carboxamide;
(2S,4R)-1-[(2S)-2-(4-cyclopropyltriazol-1-yl)-3,3-dimethyl-butanoyl]-N-[1-[(2,4-dimethoxyphenyl)methyl]-2-oxo-pyrrolidin-3-yl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[4-hydroxy-3-(4-methylpiperazin-1-yl)butyl]pyrrolidine-2-carboxamide;
(2S,4R)-1-[(2S)-2-(4-cyclopropyltriazol-1-yl)-3,3-dimethyl-butanoyl]-4-hydroxy-N-[(E)-3-(3-pyridyl)allyl]pyrrolidine-2-carboxamide;
(2S,4R)-N-(1-cyclopropyl-5-methyl-pyrrolidin-3-yl)-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[5-(1-hydroxyethyl)-6-methyl-2-pyridyl]methyl]pyrrolidine-2-carboxamide;
(2S,4R)-1-[(2S)-2-(4-cyclopropyltriazol-1-yl)-3,3-dimethyl-butanoyl]-N-[3-(dimethylamino)-4-methyl-pentyl]-4-hydroxy-pyrrolidine-2-carboxamide;
(2S,4R)-1-[(2S)-2-(4-cyclopropyltriazol-1-yl)-3,3-dimethyl-butanoyl]-N-[1,1-dimethyl-3-(4-methylpiperazin-1-yl)propyl]-4-hydroxy-pyrrolidine-2-carboxamide;
(2S,4R)-N-[2-(benzylsulfamoyl)ethyl]-1-[(2S)-2-(4-cyclopropyltriazol-1-yl)-3,3-dimethylbutanoyl]-4-hydroxy-pyrrolidine-2-carboxamide;
(2S,4R)-1-[(2S)-2-(4-cyclopropyltriazol-1-yl)-3,3-dimethyl-butanoyl]-N-[(4-fluorophenyl)-(1-methylpyrazol-4-yl)methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[3-(2-methoxyphenyl)isoxazol-5-yl]methyl]pyrrolidine-2-carboxamide;
(2S,4R)-1-[(2S)-2-(4-cyclopropyltriazol-1-yl)-3,3-dimethyl-butanoyl]-4-hydroxy-N-[2-(5-methyltetrazol-2-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[3-(phenoxymethyl)cyclobutyl]pyrrolidine-2-carboxamide;
(2S,4R)-1-[(2S)-2-(4-cyclopropyltriazol-1-yl)-3,3-dimethyl-butanoyl]-4-hydroxy-N-[2-hydroxy-1-(3-methyl-1,2,4-oxadiazol-5-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[(3R,4R)-4-hydroxy-1-isopropyl-pyrrolidin-3-yl]pyrrolidine-2-carboxamide;
(2S,4R)-1-[(2S)-2-(4-cyclopropyltriazol-1-yl)-3,3-dimethyl-butanoyl]-4-hydroxy-N-(5,6,7,8-tetrahydroimidazo[1,2-a]pyridin-5-ylmethyl)pyrrolidine-2-carboxamide;
(2S,4R)-1-[(2S)-2-(4-cyclopropyltriazol-1-yl)-3,3-dimethyl-butanoyl]-4-hydroxy-N-(4,5,6,7-tetrahydropyrazolo[1,5-a]pyridin-4-yl)pyrrolidine-2-carboxamide;
(2S,4R)-1-[(2S)-2-(4-cyclopropyltriazol-1-yl)-3,3-dimethyl-butanoyl]-4-hydroxy-N-[[4-(hydroxymethyl)-3,6,7,8,9,9a-hexahydro-1H-pyrido[2,1-c][1,4]oxazin-4-yl]methyl]pyrrolidine-2-carboxamide;
(2S,4R)-1-[(2S)-2-(4-cyclopropyltriazol-1-yl)-3,3-dimethyl-butanoyl]-4-hydroxy-N-[[1-(hydroxymethyl)cyclohexyl]-phenyl-methyl]pyrrolidine-2-carboxamide;
(2S,4R)-N-[1-(5-bromo-2-methoxy-phenyl)-2-hydroxy-ethyl]-1-[(2S)-2-(4-cyclopropyltriazol-1-yl)-3,3-dimethyl-butanoyl]-4-hydroxy-pyrrolidine-2-carboxamide;
N-[4-[[[(2S,4R)-1-[(2S)-2-(4-cyclopropyltriazol-1-yl)-3,3-dimethyl-butanoyl]-4-hydroxy-pyrrolidine-2-carbonyl]amino]methyl]pheny1]-N-methyl-pyridine-4-carboxamide;
(2S,4R)-N-[1-cyclopropyl-1-(5-methyl-1,2,4-oxadiazol-3-yl)ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[1-methyl-2-(5-methyl-2-phenyl-pyrazol-3-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[[1-([1,2,4]triazolo[4,3-a]pyrazin-8-yl)-3-piperidyl]methyl]pyrrolidine-2-carboxamide;
(2S,4R)-1-[(2S)-2-(4-cyclopropyltriazol-1-yl)-3,3-dimethyl-butanoyl]-4-hydroxy-N-[(5-oxo-1,4-dihydro-l,2,4-triazol-3-yl)methyl]pyrrolidine-2-carboxamide;
(2S,4R)-1-[(2S)-2-(4-cyclopropyltriazol-1-yl)-3,3-dimethyl-butanoyl]-4-hydroxy-N-[1-(4-methylphthalazin-1-yl)-4-piperidyl]pyrrolidine-2-carboxamide;
(2S,4R)-1-[(2S)-2-(4-cyclopropyltriazol-1-yl)-3,3-dimethyl-butanoyl]-N-[(3,3-dimethyltetrahydropyran-2-yl)methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[3-oxo-3-(4-pyrazin-2-ylpiperazin-1-yl)propyl]pyrrolidine-2-carboxamide;
(2S,4R)-1-[(2S)-2-(4-cyclopropyltriazol-1-yl)-3,3-dimethyl-butanoyl]-4-hydroxy-N-(2R,3S)-1-methyl-5-oxo-2-(1,3,5-trimethylpyrazol-4-yl)pyrrolidin-3-yl]pyrrolidine-2-carboxamide;
(2S,4R)-1-[(2S)-2-(4-cyclopropyltriazol-1-yl)-3,3-dimethyl-butanoyl]-4-hydroxy-N-(2-isochroman-7-ylethyl)pyrrolidine-2-carboxamide;
(2S,4R)-1-[(2S)-2-(4-cyclopropyltriazol-1-yl)-3,3-dimethyl-butanoyl]-N-[(1,1-dioxo-4H-1lambda6,2,4-benzothiadiazin-3-yl)methyl]-4-hydroxy-pyrrolidine-2-carboxamide;
(2S,4R)-1-[(2S)-2-(4-cyclopropyltriazol-1-yl)-3,3-dimethyl-butanoyl]-N-[2-[4-(3-ethyl-1,2,4-oxadiazol-5-yl)phenyl]ethyl]-4-hydroxy-pyrrolidine-2-carboxamide;
(2S,4R)-N-[(1R,2R)-6-bromo-1-hydroxy-tetralin-2-yl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[1-methyl-1-(1-methylpyrazol-4-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[2-(2-oxo-1-pyridyl)-1-phenyl-ethyl]pyrrolidine-2-carboxamide;
(2S,4R)-1-[(2S)-2-(4-cyclopropyltriazol-1-yl)-3,3-dimethyl-butanoyl]-N-[4-(difluoromethoxy)cyclohexyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[4-(trifluoromethyl)-3-thienyl]methyl]pyrrolidine-2-carboxamide;
(2S,4R)-N-[(3-bromoimidazo[1,2-a]pyridin-2-yl)methyl]-1-[(2S)-2-(4-cyclopropyltriazol-1-yl)-3,3-dimethyl-butanoyl]-4-hydroxy-pyrrolidine-2-carboxamide;
(2S,4R)-N-[(2-benzyl-3-oxo-isoindolin-1-yl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(2-imidazo[1,2-a]pyrimidin-2-ylethyl)pyrrolidine-2-carboxamide;
(2S,4R)-1-[(2S)-2-(4-cyclopropyltriazol-1-yl)-3,3-dimethyl-butanoyl]-N-[[1-[3-fluoro-5-(trifluoromethyl)phenyl]cyclopropyl]methyl]-4-hydroxy-pyrrolidine-2-carboxamide;
(2S,4R)-N-[(1S,2R)-2-tert-butoxy-1-(methylcarbamoyl)propyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[[3-fluoro-4-(2-methylimidazol-1-yl)phenyl]methyl]-4-hydroxy-pyrrolidine-2-carboxamide;
(2S,4R)-1-[(2S)-2-(4-cyclopropyltriazol-1-yl)-3,3-dimethyl-butanoyl]-N-[2-[1-(3-fluorophenyl)pyrazol-3-yl]ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[2-[[6-(trifluoromethyl)-2-pyridyl]oxy]ethyl]pyrrolidine-2-carboxamide;
(2S,4R)-1-[(2S)-2-(4-cyclopropyltriazol-1-yl)-3,3-dimethyl-butanoyl]-N-[3-(2,5-dimethylpyrazol-3-yl)oxypropyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[3-(1,2,4-triazol-1-yl)cyclobutyl]pyrrolidine-2-carboxamide;
(2S,4R)-1-[(2S)-2-(4-cyclopropyltriazol-1-yl)-3,3-dimethyl-butanoyl]-N-[2-(2,3-difluorophenyl)ethyl]-4-hydroxy-pyrrolidine-2-carboxamide;
(2S,4R)-N-[(5-cyclopropyloxazol-4-yl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[(1R)-1-(5-fluoro-3-pyridyl)ethyl]-4-hydroxy-pyrrolidine-2-carboxamide;
(2S,4R)-N-[(4-tert-butyl-3-pyridyl)methyl]-1-[(2S)-2-(4-cyclopropyltriazol-1-yl)-3,3-dimethyl-butanoyl]-4-hydroxy-pyrrolidine-2-carboxamide;
(2S,4R)-N-(7-chlorochroman-3-yl)-1-[(2S)-2-(4-cyclopropyltriazol-1-yl)-3,3-dimethylbutanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(2-oxo-1,3-dihydroimidazol-4-yl)methyl]pyrrolidine-2-carboxamide;
(2S,4R)-1-[(2S)-2-(4-cyclopropyltriazol-1-yl)-3,3-dimethyl-butanoyl]-N-[[1-(difluoromethylsulfonyl)-4-piperidyl]methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(1-isopropyl-4,5,6,7-tetrahydroindazol-6-yl)pyrrolidine-2-carboxamide;
(2S,4R)-1-[(2S)-2-(4-cyclopropyltriazol-1-yl)-3,3-dimethyl-butanoyl]-4-hydroxy-N-[1-(3-methylimidazol-4-yl)cyclopropyl]pyrrolidine-2-carboxamide;
(2S,4R)-1-[(2S)-2-(4-cyclopropyltriazol-1-yl)-3,3-dimethyl-butanoyl]-4-hydroxy-N-[2-(2-quinolylamino)ethyl]pyrrolidine-2-carboxamide;
(2S,4R)-1-[(2S)-2-(4-cyclopropyltriazol-1-yl)-3,3-dimethyl-butanoyl]-N-[2-(1,3-dimethylpyrazol-4-yl)ethyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[[1-methyl-2-(2-thienyl)-3-piperidyl]methyl]pyrrolidine-2-carboxamide;
(2S,4R)-1-[(2S)-2-(4-cyclopropyltriazol-1-yl)-3,3-dimethyl-butanoyl]-4-hydroxy-N-[1-methyl-2-(2-methylindolin-1-yl)-2-oxo-ethyl]pyrrolidine-2-carboxamide;
(2S,4R)-1-[(2S)-2-(4-cyclopropyltriazol-1-yl)-3,3-dimethyl-butanoyl]-N-(6,6-difluoro-2-bicyclo[3.1.0]hexanyl)-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(1-hydroxy-5-methoxy-indan-1-yl)methyl]pyrrolidine-2-carboxamide;
(2S,4R)-1-[(2S)-2-(4-cyclopropyltriazol-1-yl)-3,3-dimethyl-butanoyl]-4-hydroxy-N-(3,3,3-trifluoro-2-pyrazol-1-yl-propyl)pyrrolidine-2-carboxamide;
(2S,4R)-4-hydroxy-1-[(2S)-2-[4-[[2-methoxyethyl(methyl)amino]methyl]triazol-1-yl]-3,3-dimethyl-butanoyl]-N-methyl-pyrrolidine-2-carboxamide;
ethyl 1-[[1-[(1S)-1-[(2S,4R)-4-hydroxy-2-(methylcarbamoyl)pyrrolidine-1-carbonyl]-2,2-dimethyl-propyl]triazol-4-yl]methyl]piperidine-4-carboxylate;
(2R,4S)-4-hydroxy-1-[(2S)-2-[4-(2-hydroxy-3,3-dimethyl-butyl)triazol-1-yl]-3,3-dimethylbutanoyl]-N-methyl-pyrrolidine-2-carboxamide;
(2S,4R)-1-[(2S)-3,3-dimethyl-2-[4-(2-methylthiazol-4-yl)triazol-1-yl]butanoyl]-4-hydroxy-N-methyl-pyrrolidine-2-carboxamide;
(2S,4R)-1-[(2S)-2-[4-(5-chloro-2-thienyl)triazol-1-yl]-3,3-dimethyl-butanoyl]-4-hydroxy-N-methyl-pyrrolidine-2-carboxamide;
(2S,4R)-1-[(2S)-3,3-dimethyl-2-[4-[2-(4-methyl-1-piperidyl)ethyl]triazol-1-yl]butanoyl]-4-hydroxy-N-methyl-pyrrolidine-2-carboxamide;
(2S,4R)-4-hydroxy-1-[(2S)-2-[4-(methanesulfonamidomethyl)triazol-1-yl]-3,3-dimethylbutanoyl]-N-methyl-pyrrolidine-2-carboxamide;
(2S,4R)-1-[(2S)-2-[4-[2-(1,4-dioxa-9-azaspiro[4.5]decan-9-yl)ethyl]triazol-1-yl]-3,3-dimethyl-butanoyl]-4-hydroxy-N-methyl-pyrrolidine-2-carboxamide;
(2S,4R)-1-[(2S)-2-[4-(3,4-difluorophenyl)triazol-1-yl]-3,3-dimethyl-butanoyl]-4-hydroxy-N-methyl-pyrrolidine-2-carboxamide;
(2S,4R)-1-[(2S)-3,3-dimethyl-2-[4-[2-(2,2,6,6-tetramethylmorpholin-4-yl)ethyl]triazol-1-yl]butanoyl]-4-hydroxy-N-methyl-pyrrolidine-2-carboxamide;
methyl 1-[[1-[(1S)-1-[(2R,4S)-4-hydroxy-2-(methylcarbamoyl)pyrrolidine-1-carbonyl]-2,2-dimethyl-propyl]triazol-4-yl]methyl]piperidine-2-carboxylate;
(2S,4R)-1-[(2S)-2-[4-[2-(3,3-dimethyl-1-piperidyl)ethyl]triazol-1-yl]-3,3-dimethyl-butanoyl]-4-hydroxy-N-methyl-pyrrolidine-2-carboxamide;
(2S,4R)-1-[(2S)-2-[4-[(4,4-dimethyl-1-piperidyl)methyl]triazol-1-yl]-3,3-dimethyl-butanoyl]-4-hydroxy-N-methyl-pyrrolidine-2-carboxamide;
(2S,4R)-1-[(2S)-3,3-dimethyl-2-[4-[(4-phenylpiperazin-1-yl)methyl]triazol-1-yl]butanoyl]-4-hydroxy-N-methyl-pyrrolidine-2-carboxamide;
(2S,4R)-1-[(2S)-3,3-dimethyl-2-[4-(3-methylsulfanylphenyl)triazol-1-yl]butanoyl]-4-hydroxy-N-methyl-pyrrolidine-2-carboxamide;
(2R,4S)-1-[(2S)-3,3-dimethyl-2-[4-[(tetrahydrofuran-3-ylamino)methyl]triazol-1-yl]butanoyl]-4-hydroxy-N-methyl-pyrrolidine-2-carboxamide;
(2S,4R)-1-[(2S)-2-[4-[[tert-butyl(methyl)amino]methyl]triazol-1-yl]-3,3-dimethyl-butanoyl]-4-hydroxy-N-methyl-pyrrolidine-2-carboxamide;
(2S,4R)-1-[(2S)-3,3-dimethyl-2-[4-[(4-methyl-1-piperidyl)methyl]triazol-1-yl]butanoyl]-4-hydroxy-N-methyl-pyrrolidine-2-carboxamide;
(2S,4R)-1-[(2S)-3,3-dimethyl-2-[4-[2-(4-methylsulfonylpiperazin-1-yl)ethyl]triazol-1-yl]butanoyl]-4-hydroxy-N-methyl-pyrrolidine-2-carboxamide;
(2S,4R)-1-[(2S)-3,3-dimethyl-2-[4-[(4-methylpiperazin-1-yl)methyl]triazol-1-yl]butanoyl]-4-hydroxy-N-methyl-pyrrolidine-2-carboxamide;
(2S,4R)-1-[(2S)-2-[4-(azepan-1-ylmethyl)triazol-1-yl]-3,3-dimethyl-butanoyl]-4-hydroxy-N-methyl-pyrrolidine-2-carboxamide;
(2S,4R)-1-[(2S)-2-[4-[(diisopropylamino)methyl]triazol-1-yl]-3,3-dimethyl-butanoyl]-4-hydroxy-N-methyl-pyrrolidine-2-carboxamide;
(2R,4S)-1-[(2S)-3,3-dimethyl-2-[4-[(1-methyl-2-piperidyl)methyl]triazol-1-yl]butanoyl]-4-hydroxy-N-methyl-pyrrolidine-2-carboxamide;
(2S,4R)-1-[(2S)-3,3-dimethyl-2-[4-[(3-oxopiperazin-1-yl)methyl]triazol-1-yl]butanoyl]-4-hydroxy-N-methyl-pyrrolidine-2-carboxamide;
(2S,4R)-4-hydroxy-1-[(2S)-2-[4-[(4-methoxy-1-piperidyl)methyl]triazol-1-yl]-3,3-dimethylbutanoyl]-N-methyl-pyrrolidine-2-carboxamide;
(2R,4S)-1-[(2S)-3,3-dimethyl-2-[4-[2-(1-methylpyrrolidin-2-yl)ethyl]triazol-1-yl]butanoyl]-4-hydroxy-N-methyl-pyrrolidine-2-carboxamide;
(2S,4R)-1-[(2S)-2-[4-[2-(4,4-dimethyl-1-piperidyl)ethyl]triazol-1-yl]-3,3-dimethyl-butanoyl]-4-hydroxy-N-methyl-pyrrolidine-2-carboxamide;
(2S,4R)-1-[(2S)-2-[4-[[(2S,6R)-2,6-dimethylmorpholin-4-yl]methyl]triazol-1-yl]-3,3-dimethyl-butanoyl]-4-hydroxy-N-methyl-pyrrolidine-2-carboxamide;
(2S,4R)-1-[(2S)-2-[4-[3-(3,3-dimethyl-1-piperidyl)propyl]triazol-1-yl]-3,3-dimethylbutanoyl]-4-hydroxy-N-methyl-pyrrolidine-2-carboxamide;
(2S,4R)-1-[(2S)-2-[4-[(2-fluorophenoxy)methyl]triazol-1-yl]-3,3-dimethyl-butanoyl]-4-hydroxy-N-methyl-pyrrolidine-2-carboxamide;
(2S,4R)-1-[(2S)-2-[4-[2-(3,3-dimethylazepan-1-yl)ethyl]triazol-1-yl]-3,3-dimethyl-butanoyl]-4-hydroxy-N-methyl-pyrrolidine-2-carboxamide;
(2R,4S)-4-hydroxy-1-[(2S)-2-[4-[[2-(hydroxymethyl)pyrrolidin-1-yl]methyl]triazol-1-yl]-3,3-dimethyl-butanoyl]-N-methyl-pyrrolidine-2-carboxamide;
(2R,4S)-1-[(2S)-3,3-dimethyl-2-[4-[2-(1-methyl-2-piperidyl)ethyl]triazol-1-yl]butanoyl]-4-hydroxy-N-methyl-pyrrolidine-2-carboxamide;
(2S,4R)-1-[(2S)-2-[4-[2-(2,2-dimethylmorpholin-4-yl)ethyl]triazol-1-yl]-3,3-dimethylbutanoyl]-4-hydroxy-N-methyl-pyrrolidine-2-carboxamide;
(2S,4R)-1-[(2S)-2-[4-(2,6-difluorophenyl)triazol-1-yl]-3,3-dimethyl-butanoyl]-4-hydroxy-N-methyl-pyrrolidine-2-carboxamide;
(2S,4R)-1-[(2S)-2-[4-[(1,1-dioxo-1,4-thiazepan-4-yl)methyl]triazol-1-yl]-3,3-dimethylbutanoyl]-4-hydroxy-N-methyl-pyrrolidine-2-carboxamide;
(2S,4R)-4-hydroxy-1-[(2S)-2-[4-(4-methoxyphenyl)triazol-1-yl]-3,3-dimethyl-butanoyl]-N-methyl-pyrrolidine-2-carboxamide;
(2S,4R)-4-hydroxy-1-[(2S)-2-[4-(5-methoxy-3-pyridyl)triazol-1-yl]-3,3-dimethyl-butanoyl]-N-methyl-pyrrolidine-2-carboxamide;
(2S,4R)-4-hydroxy-1-[(2S)-2-[4-(6-methoxy-2-pyridyl)triazol-1-yl]-3,3-dimethyl-butanoyl]-N-methyl-pyrrolidine-2-carboxamide;
(2S,4R)-1-[(2S)-2-[4-[(2-chlorophenoxy)methyl]triazol-1-yl]-3,3-dimethyl-butanoyl]-4-hydroxy-N-methyl-pyrrolidine-2-carboxamide;
(2S,4R)-1-[(2S)-2-[4-(3-chlorophenyl)triazol-1-yl]-3,3-dimethyl-butanoyl]-4-hydroxy-N-methyl-pyrrolidine-2-carboxamide;
(2S,4R)-1-[(2S)-3,3-dimethyl-2-[4-[(4-methylphenoxy)methyl]triazol-1-yl]butanoyl]-4-hydroxy-N-methyl-pyrrolidine-2-carboxamide;
(2R,4S)-1-[(2S)-2-[4-[2-(4-fluorophenyl)-1-(methylamino)ethyl]triazol-1-yl]-3,3-dimethylbutanoyl]-4-hydroxy-N-methyl-pyrrolidine-2-carboxamide;
1-[[1-[(1 S)-1-[(2S,4R)-4-hydroxy-2-(methylcarbamoyl)pyrrolidine-1-carbonyl]-2,2-dimethylpropyl]triazol-4-yl]methyl]piperidine-4-carboxamide;
(2S,4R)-1-[(2S)-2-[4-[(4-cyano-1-piperidyl)methyl]triazol-1-yl]-3,3-dimethyl-butanoyl]-4-hydroxy-N-methyl-pyrrolidine-2-carboxamide;
(2S,4R)-1-[(2S)-2-[4-[(4,4-difluoro-1-piperidyl)methyl]triazol-1-yl]-3,3-dimethyl-butanoyl]-4-hydroxy-N-methyl-pyrrolidine-2-carboxamide;
(2S,4R)-1-[(2S)-3,3-dimethyl-2-[4-[[(1-methyl-4-piperidyl)amino]methyl]triazol-1-yl]butanoyl]-4-hydroxy-N-methyl-pyrrolidine-2-carboxamide;
(2S,4R)-1-[(2S)-3,3-dimethyl-2-[4-(4-methylsulfanylphenyl)triazol-1-yl]butanoyl]-4-hydroxy-N-methyl-pyrrolidine-2-carboxamide;
(2S,4R)-4-hydroxy-1-[(2S)-2-[4-(4-hydroxy-3,3-dimethyl-butyl)triazol-1-yl]-3,3-dimethylbutanoyl]-N-methyl-pyrrolidine-2-carboxamide;
(2S,4R)-1-[(2S)-2-[4-(2,6-dimethyl-4-pyridyl)triazol-1-yl]-3,3-dimethyl-butanoyl]-4-hydroxy-N-methyl-pyrrolidine-2-carboxamide;
(2R,4S)-1-[(2S)-2-[4-(1-benzylpyrrolidin-3-yl)triazol-1-yl]-3,3-dimethyl-butanoyl]-4-hydroxy-N-methyl-pyrrolidine-2-carboxamide;
(2S,4R)-4-hydroxy-1-[(2S)-2-[4-(1-isopropylpyrazol-4-yl)triazol-1-yl]-3,3-dimethylbutanoyl]-N-methyl-pyrrolidine-2-carboxamide;
(2S,4R)-1-[(2S)-2-[4-(4-chloro-2-fluoro-phenyl)triazol-1-yl]-3,3-dimethyl-butanoyl]-4-hydroxy-N-methyl-pyrrolidine-2-carboxamide;
(2S,4R)-1-[(2S)-2-[4-[(4-acetylpiperazin-1-yl)methyl]triazol-1-yl]-3,3-dimethyl-butanoyl]-4-hydroxy-N-methyl-pyrrolidine-2-carboxamide;
3-[[1-[(1S)-1-[(2S,4R)-4-hydroxy-2-(methylcarbamoyl)pyrrolidine-1-carbonyl]-2,2-dimethylpropyl]triazol-4-yl]methylamino]benzoic acid;
(2S,4R)-1-[(2S)-2-[4-(3,5-dichlorophenyl)triazol-1-yl]-3,3-dimethyl-butanoyl]-4-hydroxy-N-methyl-pyrrolidine-2-carboxamide;
methyl 4-[1-[(1S)-1-[(2S,4R)-4-hydroxy-2-(methylcarbamoyl)pyrrolidine-1-carbonyl]-2,2-dimethyl-propyl]triazol-4-yl]benzoate;
(2S,4R)-1-[(2S)-3,3-dimethyl-2-[4-[3-oxo-3-(p-tolyl)propyl]triazol-1-yl]butanoyl]-4-hydroxy-N-methyl-pyrrolidine-2-carboxamide;
methyl 4-[1-[(1S)-1-[(2S,4R)-4-hydroxy-2-(methylcarbamoyl)pyrrolidine-1-carbonyl]-2,2-dimethyl-propyl]triazol-4-yl]-2-methyl-pyrazole-3-carboxylate;
methyl 6-[1-[(1S)-1-[(2S,4R)-4-hydroxy-2-(methylcarbamoyl)pyrrolidine-1-carbonyl]-2,2-dimethyl-propyl]triazol-4-yl]pyridine-2-carboxylate;
methyl 4-[[1-[(1S)-1-[(2S,4R)-4-hydroxy-2-(methylcarbamoyl)pyrrolidine-1-carbonyl]-2,2-dimethyl-propyl]triazol-4-yl]methyl]benzoate;
(2S,4R)-1-[(2S)-3,3-dimethyl-2-[4-[[(2,2,2-trifluoroacetyl)amino]methyl]triazol-1-yl]butanoyl]-4-hydroxy-N-methyl-pyrrolidine-2-carboxamide;
(2S,4R)-1-[(2S)-2-[4-[(ethylsulfonylamino)methyl]triazol-1-yl]-3,3-dimethyl-butanoyl]-4-hydroxy-N-methyl-pyrrolidine-2-carboxamide;
(2R,4S)-1-[(2S)-3,3-dimethyl-2-[4-[(3-methyl-1,1-dioxo-1,4-thiazinan-4-yl)methyl]triazol-1-yl]butanoyl]-4-hydroxy-N-methyl-pyrrolidine-2-carboxamide;
(2S,4R)-1-[(2S)-3,3-dimethyl-2-[4-[(tetrahydropyran-4-ylamino)methyl]triazol-1-yl]butanoyl]-4-hydroxy-N-methyl-pyrrolidine-2-carboxamide;
(2S,4R)-1-[(2S)-3,3-dimethyl-2-[4-[[2-(morpholinomethyl)imidazol-1-yl]methyl]triazol-1-yl]butanoyl]-4-hydroxy-N-methyl-pyrrolidine-2-carboxamide;
tert-butyl 4-[1-[1-[(1S)-1-[(2S,4R)-4-hydroxy-2-(methylcarbamoyl)pyrrolidine-1-carbonyl]-2,2-dimethyl-propyl]triazol-4-yl]-1-methyl-ethyl]piperazine-1-carboxylate;
(2S,4R)-1-[(2S)-2-[4-(2-chlorophenyl)triazol-1-yl]-3,3-dimethyl-butanoyl]-4-hydroxy-N-methyl-pyrrolidine-2-carboxamide;
(2S,4R)-1-[(2S)-3,3-dimethyl-2-[4-(2-methylsulfonylethyl)triazol-1-yl]butanoyl]-4-hydroxy-N-methyl-pyrrolidine-2-carboxamide;
(2S,4R)-1-[(2S)-2-[4-[1-(tert-butylamino)-1-methyl-ethyl]triazol-1-yl]-3,3-dimethylbutanoyl]-4-hydroxy-N-methyl-pyrrolidine-2-carboxamide;
tert-butyl 4-[[1-[(1S)-1-[(2S,4R)-4-hydroxy-2-(methylcarbamoyl)pyrrolidine-1-carbonyl]-2,2-dimethyl-propyl]triazol-4-yl]methyl]piperazine-1-carboxylate;
(2S,4R)-1-[(2S)-2-[4-(4-amino-3-chloro-phenyl)triazol-1-yl]-3,3-dimethyl-butanoyl]-4-hydroxy-N-methyl-pyrrolidine-2-carboxamide;
(2S,4R)-1-[(2S)-3,3-dimethyl-2-[4-[2-(4-methylpiperazin-1-yl)ethyl]triazol-1-yl]butanoyl]-4-hydroxy-N-methyl-pyrrolidine-2-carboxamide;
(2S,4R)-1-[(2S)-2-[4-[[cyclohexyl(methyl)amino]methyl]triazol-1-yl]-3,3-dimethylbutanoyl]-4-hydroxy-N-methyl-pyrrolidine-2-carboxamide;
(2S,4R)-1-[(2S)-2-[4-[(6-aminopurin-9-yl)methyl]triazol-1-yl]-3,3-dimethyl-butanoyl]-4-hydroxy-N-methyl-pyrrolidine-2-carboxamide;
(2S,4R)-1-[(2S)-2-[4-[2-(6-azaspiro[3.5]nonan-6-yl)ethyl]triazol-1-yl]-3,3-dimethylbutanoyl]-4-hydroxy-N-methyl-pyrrolidine-2-carboxamide;
(2S,4R)-1-[(2S)-3,3-dimethyl-2-[4-[2-(1-piperidyl)ethyl]triazol-1-yl]butanoyl]-4-hydroxy-N-methyl-pyrrolidine-2-carboxamide;
(2S,4R)-1-[(2S)-2-(4-benzoyltriazol-1-yl)-3,3-dimethyl-butanoyl]-4-hydroxy-N-methyl-pyrrolidine-2-carboxamide;
(2S,4R)-1-[(2S)-2-[4-[(4-chloropyrazol-1-yl)methyl]triazol-1-yl]-3,3-dimethyl-butanoyl]-4-hydroxy-N-methyl-pyrrolidine-2-carboxamide;
(2S,4R)-4-hydroxy-1-[(2S)-2-[4-(isoindolin-2-ylmethyl)triazol-1-yl]-3,3-dimethyl-butanoyl]-N-methyl-pyrrolidine-2-carboxamide;
(2S,4R)-4-hydroxy-1-[(2S)-2-[4-[1-(methanesulfonamido)-1-methyl-ethyl]triazol-1-yl]-3,3-dimethyl-butanoyl]-N-methyl-pyrrolidine-2-carboxamide;
(2S,4R)-4-hydroxy-1-[(2S)-2-[4-(4-methoxybenzoyl)triazol-1-yl]-3,3-dimethyl-butanoyl]-N-methyl-pyrrolidine-2-carboxamide;
(2S,4R)-1-[(2S)-2-[4-[(3-acetylphenoxy)methyl]triazol-1-yl]-3,3-dimethyl-butanoyl]-4-hydroxy-N-methyl-pyrrolidine-2-carboxamide;
(2S,4R)-1-[(2S)-2-[4-[(4-acetylphenoxy)methyl]triazol-1-yl]-3,3-dimethyl-butanoyl]-4-hydroxy-N-methyl-pyrrolidine-2-carboxamide;
methyl 3-[1-[(1S)-1-[(2S,4R)-4-hydroxy-2-(methylcarbamoyl)pyrrolidine-1-carbonyl]-2,2-dimethyl-propyl]triazol-4-yl]pyridine-2-carboxylate;
methyl 3-[1-[(1S)-1-[(2S,4R)-4-hydroxy-2-(methylcarbamoyl)pyrrolidine-1-carbonyl]-2,2-dimethyl-propyl]triazol-4-yl]-2,2-dimethyl-propanoate;
(2S,4R)-1-[(2S)-3,3-dimethyl-2-[4-[2-(1,2,4-triazol-1-yl)ethyl]triazol-1-yl]butanoyl]-4-hydroxy-N-methyl-pyrrolidine-2-carboxamide;
(2S,4R)-1-[(2S)-3,3-dimethyl-2-[4-[(2-oxo-1-piperidyl)methyl]triazol-1-yl]butanoyl]-4-hydroxy-N-methyl-pyrrolidine-2-carboxamide;
(2S,4R)-1-[(2S)-2-[4-(6-fluoro-5-methyl-3-pyridyl)triazol-1-yl]-3,3-dimethyl-butanoyl]-4-hydroxy-N-methyl-pyrrolidine-2-carboxamide;
(2S,4R)-4-hydroxy-1-[(2S)-2-[4-(5-hydroxy-3-oxo-pentyl)triazol-1-yl]-3,3-dimethylbutanoyl]-N-methyl-pyrrolidine-2-carboxamide;
(2S,4R)-1-[(2S)-3,3-dimethyl-2-[4-(2-oxospiro[3.3]heptan-6-yl)triazol-1-yl]butanoyl]-4-hydroxy-N-methyl-pyrrolidine-2-carboxamide;
(2S,4R)-1-[(2S)-2-[4-(4-cyano-3-fluoro-phenyl)triazol-1-yl]-3,3-dimethyl-butanoyl]-4-hydroxy-N-methyl-pyrrolidine-2-carboxamide;
(2S,4R)-1-[(2S)-2-[4-(6-fluoro-4-methyl-3-pyridyl)triazol-1-yl]-3,3-dimethyl-butanoyl]-4-hydroxy-N-methyl-pyrrolidine-2-carboxamide;
(2S,4R)-4-hydroxy-1-[(2S)-2-[4-(2-methoxyphenyl)triazol-1-yl]-3,3-dimethyl-butanoyl]-N-methyl-pyrrolidine-2-carboxamide;
(2S,4R)-1-[(2S)-3,3-dimethyl-2-[4-[(2-oxoazepan-1-yl)methyl]triazol-1-yl]butanoyl]-4-hydroxy-N-methyl-pyrrolidine-2-carboxamide;
(2S,4R)-1-[(2S)-2-[4-(6-fluoro-2-methyl-3-pyridyl)triazol-1-yl]-3,3-dimethyl-butanoyl]-4-hydroxy-N-methyl-pyrrolidine-2-carboxamide;
(2S,4R)-1-[(2S)-3,3-dimethyl-2-[4-[(3-oxomorpholin-4-yl)methyl]triazol-1-yl]butanoyl]-4-hydroxy-N-methyl-pyrrolidine-2-carboxamide;
(2S,4R)-4-hydroxy-1-[(2S)-2-[4-(2-methoxy-3-pyridyl)triazol-1-yl]-3,3-dimethyl-butanoyl]-N-methyl-pyrrolidine-2-carboxamide;
(2S,4R)-4-hydroxy-1-[(2S)-2-[4-[(1-hydroxycyclohexyl)methyl]triazol-1-yl]-3,3-dimethylbutanoyl]-N-methyl-pyrrolidine-2-carboxamide;
(2S,4R)-4-hydroxy-1-[(2S)-2-[4-[6-(hydroxymethyl)-2-pyridyl]triazol-1-yl]-3,3-dimethylbutanoyl]-N-methyl-pyrrolidine-2-carboxamide;
(2S,4R)-4-hydroxy-1-[(2S)-2-[4-[3-(hydroxymethyl)phenyl]triazol-1-yl]-3,3-dimethylbutanoyl]-N-methyl-pyrrolidine-2-carboxamide;
(2S,4R)-1-[(2S)-2-[4-(4-cyanophenyl)triazol-1-yl]-3,3-dimethyl-butanoyl]-4-hydroxy-N-methyl-pyrrolidine-2-carboxamide;
(2S,4R)-1-[(2S)-2-[4-(5-fluoro-3-pyridyl)triazol-1-yl]-3,3-dimethyl-butanoyl]-4-hydroxy-N-methyl-pyrrolidine-2-carboxamide;
(2S,4R)-1-[(2S)-2-[4-(4-cyclopropylthiazol-2-yl)triazol-1-yl]-3,3-dimethyl-butanoyl]-4-hydroxy-N-methyl-pyrrolidine-2-carboxamide;
(2S,4R)-1-[(2S)-2-[4-[(2,5-dimethylpyrrol-1-yl)methyl]triazol-1-yl]-3,3-dimethyl-butanoyl]-4-hydroxy-N-methyl-pyrrolidine-2-carboxamide;
(2S,4R)-4-hydroxy-1-[(2S)-2-[4-(2-methoxythiazol-5-yl)triazol-1-yl]-3,3-dimethylbutanoyl]-N-methyl-pyrrolidine-2-carboxamide;
(2S,4R)-4-hydroxy-1-[(2S)-2-[4-(6-methoxy-3-pyridyl)triazol-1-yl]-3,3-dimethyl-butanoyl]-N-methyl-pyrrolidine-2-carboxamide;
(2S,4R)-1-[(2S)-3,3-dimethyl-2-[4-(3-sulfamoylpropyl)triazol-1-yl]butanoyl]-4-hydroxy-N-methyl-pyrrolidine-2-carboxamide;
(2S,4R)-1-[(2S)-2-[4-[(dimethylcarbamoylamino)methyl]triazol-1-yl]-3,3-dimethylbutanoyl]-4-hydroxy-N-methyl-pyrrolidine-2-carboxamide;
(2S,4R)-1-[(2S)-3,3-dimethyl-2-[4-[[methyl(methylsulfonyl)amino]methyl]triazol-1-yl]butanoyl]-4-hydroxy-N-methyl-pyrrolidine-2-carboxamide;
(2S,4R)-1-[(2S)-2-[4-(5-chloro-2-pyridyl)triazol-1-yl]-3,3-dimethyl-butanoyl]-4-hydroxy-N-methyl-pyrrolidine-2-carboxamide;
(2S,4R)-1-[(2S)-2-[4-[(cyclopropylsulfonylamino)methyl]triazol-1-yl]-3,3-dimethylbutanoyl]-4-hydroxy-N-methyl-pyrrolidine-2-carboxamide;
(2S,4R)-1-[(2S)-2-[4-[(3,5-dimethylpyrazol-1-yl)methyl]triazol-1-yl]-3,3-dimethylbutanoyl]-4-hydroxy-N-methyl-pyrrolidine-2-carboxamide;
(2S,4R)-4-hydroxy-1-[(2S)-2-[4-(4-methoxy-2-pyridyl)triazol-1-yl]-3,3-dimethyl-butanoyl]-N-methyl-pyrrolidine-2-carboxamide;
(2S,4R)-1-[(2S)-3,3-dimethyl-2-[4-[(1-methylpyrazol-4-yl)oxymethyl]triazol-1-yl]butanoyl]-4-hydroxy-N-methyl-pyrrolidine-2-carboxamide;
(2S,4R)-1-[(2S)-3,3-dimethyl-2-[4-(1-methyl-1-pyrrol-1-yl-ethyl)triazol-1-yl]butanoyl]-4-hydroxy-N-methyl-pyrrolidine-2-carboxamide;
(2S,4R)-1-[(2S)-2-[4-[4-fluoro-3-(trifluoromethyl)phenyl]triazol-1-yl]-3,3-dimethylbutanoyl]-4-hydroxy-N-methyl-pyrrolidine-2-carboxamide;
(2S,4R)-1-[(2S)-2-[4-(2-fluoro-5-methyl-3-pyridyl)triazol-1-yl]-3,3-dimethyl-butanoyl]-4-hydroxy-N-methyl-pyrrolidine-2-carboxamide;
(2S,4R)-1-[(2S)-3,3-dimethyl-2-[4-(1-propylpyrazol-4-yl)triazol-1-yl]butanoyl]-4-hydroxy-N-methyl-pyrrolidine-2-carboxamide;
(2S,4R)-4-hydroxy-1-[(2S)-2-[4-[4-(hydroxymethyl)phenyl]triazol-1-yl]-3,3-dimethylbutanoyl]-N-methyl-pyrrolidine-2-carboxamide;
(2S,4R)-1-[(2S)-3,3-dimethyl-2-[4-(3-oxo-3-phenyl-propyl)triazol-1-yl]butanoyl]-4-hydroxy-N-methyl-pyrrolidine-2-carboxamide;
(2S,4R)-1-[(2S)-2-[4-(5-chloro-3-pyridyl)triazol-1-yl]-3,3-dimethyl-butanoyl]-4-hydroxy-N-methyl-pyrrolidine-2-carboxamide;
(2S,4R)-4-hydroxy-1-[(2S)-2-[4-[[1-(hydroxymethyl)cyclobutyl]methyl]triazol-1-yl]-3,3-dimethyl-butanoyl]-N-methyl-pyrrolidine-2-carboxamide;
(2S,4R)-1-[(2S)-3,3-dimethyl-2-[4-[3-(methylamino)phenyl]triazol-1-yl]butanoyl]-4-hydroxy-N-methyl-pyrrolidine-2-carboxamide;
(2S,4R)-4-hydroxy-1-[(2S)-2-[4-[2-(methanesulfonamido)ethyl]triazol-1-yl]-3,3-dimethylbutanoyl]-N-methyl-pyrrolidine-2-carboxamide;
(2S,4R)-1-[(2S)-2-[4-[2-(dimethylsulfamoyl)ethyl]triazol-1-yl]-3,3-dimethyl-butanoyl]-4-hydroxy-N-methyl-pyrrolidine-2-carboxamide;
(2S,4R)-1-[(2S)-2-[4-(2,6-dichlorophenyl)triazol-1-yl]-3,3-dimethyl-butanoyl]-4-hydroxy-N-methyl-pyrrolidine-2-carboxamide;
(2S,4R)-1-[(2S)-2-[4-(4,4-dimethyl-3-oxo-pentyl)triazol-1-yl]-3,3-dimethyl-butanoyl]-4-hydroxy-N-methyl-pyrrolidine-2-carboxamide;
3-[1-[(1S)-1-[(2S,4R)-4-hydroxy-2-(methylcarbamoyl)pyrrolidine-1-carbonyl]-2,2-dimethylpropyl]triazol-4-yl]benzoic acid;
methyl 4-[1-[(1S)-1-[(2R,4S)-4-hydroxy-2-(methylcarbamoyl)pyrrolidine-1-carbonyl]-2,2-dimethyl-propyl]triazol-4-yl]cyclohexanecarboxylate;
(2S,4R)-4-hydroxy-1-[(2S)-2-[4-(4-hydroxytetrahydropyran-4-yl)triazol-1-yl]-3,3-dimethylbutanoyl]-N-methyl-pyrrolidine-2-carboxamide;
methyl 6-[1-[(1S)-1-[(2S,4R)-4-hydroxy-2-(methylcarbamoyl)pyrrolidine-1-carbonyl]-2,2-dimethyl-propyl]triazol-4-yl]pyridine-3-carboxylate;
(2R,4S)-4-hydroxy-1-[(2S)-2-[4-[hydroxy(phenyl)methyl]triazol-1-yl]-3,3-dimethylbutanoyl]-N-methyl-pyrrolidine-2-carboxamide;
(2S,4R)-1-[(2S)-3,3-dimethyl-2-[4-(pyrimidin-2-yloxymethyl)triazol-1-yl]butanoyl]-4-hydroxy-N-methyl-pyrrolidine-2-carboxamide;
(2R,4S)-1-[(2S)-2-[4-[(4-tert-butylphenyl)-hydroxy-methyl]triazol-1-yl]-3,3-dimethylbutanoyl]-4-hydroxy-N-methyl-pyrrolidine-2-carboxamide;
(2S,4R)-1-[(2S)-2-[4-(2-ethoxyphenyl)triazol-1-yl]-3,3-dimethyl-butanoyl]-4-hydroxy-N-methyl-pyrrolidine-2-carboxamide;
(2S,4R)-1-[(2S)-2-[4-(4,4-difluorocyclohexyl)triazol-1-yl]-3,3-dimethyl-butanoyl]-4-hydroxy-N-methyl-pyrrolidine-2-carboxamide;
(2R,4S)-1-[(2S)-3,3-dimethyl-2-[4-(2-tetrahydropyran-2-ylethyl)triazol-1-yl]butanoyl]-4-hydroxy-N-methyl-pyrrolidine-2-carboxamide;
(2S,4R)-1-[(2S)-2-[4-(3-bromo-4-methoxy-phenyl)triazol-1-yl]-3,3-dimethyl-butanoyl]-4-hydroxy-N-methyl-pyrrolidine-2-carboxamide;
(2S,4R)-1-[(2S)-2-[4-(2-fluoro-4-methoxy-phenyl)triazol-1-yl]-3,3-dimethyl-butanoyl]-4-hydroxy-N-methyl-pyrrolidine-2-carboxamide;
(2R,4S)-1-[(2S)-2-[4-(1,1-dioxothian-3-yl)triazol-1-yl]-3,3-dimethyl-butanoyl]-4-hydroxy-N-methyl-pyrrolidine-2-carboxamide;
(2R,4S)-4-hydroxy-1-[(2S)-2-[4-(1-hydroxy-1-phenyl-ethyl)triazol-1-yl]-3,3-dimethylbutanoyl]-N-methyl-pyrrolidine-2-carboxamide;
(2S,4R)-1-[(2S)-3,3-dimethyl-2-[4-[(N-methylanilino)methyl]triazol-1-yl]butanoyl]-4-hydroxy-N-methyl-pyrrolidine-2-carboxamide;
(2S,4R)-1-[(2S)-2-[4-(benzyloxymethyl)triazol-1-yl]-3,3-dimethyl-butanoyl]-4-hydroxy-N-methyl-pyrrolidine-2-carboxamide;
(2S,4R)-1-[(2S)-3,3-dimethyl-2-[4-[(p-tolylsulfonylamino)methyl]triazol-1-yl]butanoyl]-4-hydroxy-N-methyl-pyrrolidine-2-carboxamide;
(2S,4R)-1-[(2S)-2-[4-[(2-cyanophenoxy)methyl]triazol-1-yl]-3,3-dimethyl-butanoyl]-4-hydroxy-N-methyl-pyrrolidine-2-carboxamide;
(2R,4S)-1-[(2S)-2-[4-[4-(4-fluorophenyl)-3-hydroxy-butyl]triazol-1-yl]-3,3-dimethylbutanoyl]-4-hydroxy-N-methyl-pyrrolidine-2-carboxamide;
(2S,4R)-1-[(2S)-2-[4-[(3-chlorophenoxy)methyl]triazol-1-yl]-3,3-dimethyl-butanoyl]-4-hydroxy-N-methyl-pyrrolidine-2-carboxamide;
(2S,4R)-1-[(2S)-3,3-dimethyl-2-[4-[3-(2-thienylmethylamino)phenyl]triazol-1-yl]butanoyl]-4-hydroxy-N-methyl-pyrrolidine-2-carboxamide;
(2S,4R)-1-[(2S)-2-[4-[2-(2-amino-2-oxo-ethyl)phenyl]triazol-1-yl]-3,3-dimethyl-butanoyl]-4-hydroxy-N-methyl-pyrrolidine-2-carboxamide;
(2R,4S)-1-[(2S)-2-[4-[(4-chlorophenyl)-hydroxy-methyl]triazol-1-yl]-3,3-dimethylbutanoyl]-4-hydroxy-N-methyl-pyrrolidine-2-carboxamide;
(2S,4R)-1-[(2S)-2-[4-[[(3-cyanophenyl)carbamoylamino]methyl]triazol-1-yl]-3,3-dimethylbutanoyl]-4-hydroxy-N-methyl-pyrrolidine-2-carboxamide;
(2S,4R)-1-[(2S)-3,3-dimethyl-2-[4-[4-(trifluoromethyl)phenyl]triazol-1-yl]butanoyl]-4-hydroxy-N-methyl-pyrrolidine-2-carboxamide;
(2S,4R)-1-[(2S)-2-[4-(6-ethoxy-3-pyridyl)triazol-1-yl]-3,3-dimethyl-butanoyl]-4-hydroxy-N-methyl-pyrrolidine-2-carboxamide;
(2S,4R)-1-[(2S)-3,3-dimethyl-2-[4-(2-quinolyl)triazol-1-yl]butanoyl]-4-hydroxy-N-methyl-pyrrolidine-2-carboxamide;
(2S,4R)-4-hydroxy-1-[(2S)-2-[4-(3-methoxy-2-pyridyl)triazol-1-yl]-3,3-dimethyl-butanoyl]-N-methyl-pyrrolidine-2-carboxamide;
benzyl 3-[1-[(1S)-1-[(2S,4R)-4-hydroxy-2-(methylcarbamoyl)pyrrolidine-1-carbonyl]-2,2-dimethyl-propyl]triazol-4-yl]-2,2-dimethyl-propanoate;
ethyl 5-[1-[(1S)-1-[(2S,4R)-4-hydroxy-2-(methylcarbamoyl)pyrrolidine-1-carbonyl]-2,2-dimethyl-propyl]triazol-4-yl]-1-methyl-pyrazole-4-carboxylate;
ethyl 3-[1-[(1S)-1-[(2S,4R)-4-hydroxy-2-(methylcarbamoyl)pyrrolidine-1-carbonyl]-2,2-dimethyl-propyl]triazol-4-yl]benzoate;
(2S,4R)-1-[(2S)-3,3-dimethyl-2-[4-(1,3,5-trimethylpyrazol-4-yl)triazol-1-yl]butanoyl]-4-hydroxy-N-methyl-pyrrolidine-2-carboxamide;
ethyl 3-[1-[(1S)-1-[(2S,4R)-4-hydroxy-2-(methylcarbamoyl)pyrrolidine-1-carbonyl]-2,2-dimethyl-propyl]triazol-4-yl]-2,2-dimethyl-propanoate;
ethyl 2-[[1-[(1S)-1-[(2R,4S)-4-hydroxy-2-(methylcarbamoyl)pyrrolidine-1-carbonyl]-2,2-dimethyl-propyl]triazol-4-yl]methyl]tetrahydrofuran-2-carboxylate;
[1-[[1-[(1S)-1-[(2S,4R)-4-hydroxy-2-(methylcarbamoyl)pyrrolidine-1-carbonyl]-2,2-dimethyl-propyl]triazol-4-yl]methyl]cyclohexyl] carbamate;
(2S,4R)-1-[(2S)-3,3-dimethyl-2-[4-[(thiazol-2-ylamino)methyl]triazol-1-yl]butanoyl]-4-hydroxy-N-methyl-pyrrolidine-2-carboxamide;
(2S,4R)-1-[(2S)-3,3-dimethyl-2-[4-[(2-oxo-1-pyridyl)methyl]triazol-1-yl]butanoyl]-4-hydroxy-N-methyl-pyrrolidine-2-carboxamide;
(2S,4R)-1-[(2S)-2-[4-(5,8-dioxaspiro[3.4]octan-2-yl)triazol-1-yl]-3,3-dimethyl-butanoyl]-4-hydroxy-N-methyl-pyrrolidine-2-carboxamide;
(2S,4R)-1-[(2S)-2-[4-(benzamidomethyl)triazol-1-yl]-3,3-dimethyl-butanoyl]-4-hydroxy-N-methyl-pyrrolidine-2-carboxamide;
(2S,4R)-1-[(2S)-2-[4-[[(3,4-difluorophenyl)carbamoylamino]methyl]triazol-1-yl]-3,3-dimethyl-butanoyl]-4-hydroxy-N-methyl-pyrrolidine-2-carboxamide;
(2S,4R)-4-hydroxy-1-[(2S)-2-(4-indan-5-yltriazol-1-yl)-3,3-dimethyl-butanoyl]-N-methyl-pyrrolidine-2-carboxamide;
(2R,4S)-4-hydroxy-1-[(2S)-2-[4-[2-hydroxy-2-(2-thienyl)propyl]triazol-1-yl]-3,3-dimethylbutanoyl]-N-methyl-pyrrolidine-2-carboxamide;
(2S,4R)-4-hydroxy-1-[(2S)-2-[4-[(4-methoxyanilino)methyl]triazol-1-yl]-3,3-dimethylbutanoyl]-N-methyl-pyrrolidine-2-carboxamide;
(2S,4R)-1-[(2S)-2-[4-(benzenesulfonamidomethyl)triazol-1-yl]-3,3-dimethyl-butanoyl]-4-hydroxy-N-methyl-pyrrolidine-2-carboxamide;
(2S,4R)-1-[(2S)-2-[4-[3-[(2-fluoro-3-pyridyl)oxy]propyl]triazol-1-yl]-3,3-dimethylbutanoyl]-4-hydroxy-N-methyl-pyrrolidine-2-carboxamide;
(2R,4S)-4-hydroxy-1-[(2S)-2-[4-[1-[4-(hydroxymethyl)phenoxy]propyl]triazol-1-yl]-3,3-dimethyl-butanoyl]-N-methyl-pyrrolidine-2-carboxamide;
(2S,4R)-1-[(2S)-3,3-dimethyl-2-[4-[(4-phenylimidazol-1-yl)methyl]triazol-1-yl]butanoyl]-4-hydroxy-N-methyl-pyrrolidine-2-carboxamide;
(2S,4R)-1-[(2S)-2-[4-[1-(cyclopropanecarbonylamino)-1-methyl-ethyl]triazol-1-yl]-3,3-dimethyl-butanoyl]-4-hydroxy-N-methyl-pyrrolidine-2-carboxamide;
(2S,4R)-1-[(2S)-2-[4-[2-[2-(2-cyanoethyl)-1,3-dioxolan-2-yl]ethyl]triazol-1-yl]-3,3-dimethylbutanoyl]-4-hydroxy-N-methyl-pyrrolidine-2-carboxamide;
(2S,4R)-4-hydroxy-1-[(2S)-2-[4-[3-(methoxymethyl)phenyl]triazol-1-yl]-3,3-dimethylbutanoyl]-N-methyl-pyrrolidine-2-carboxamide;
(2S,4R)-1-[(2S)-2-[4-[[(2-fluorophenyl)carbamoylamino]methyl]triazol-1-yl]-3,3-dimethylbutanoyl]-4-hydroxy-N-methyl-pyrrolidine-2-carboxamide;
(2S,4R)-1-[(2S)-3,3-dimethyl-2-[4-(2-methyl-4,5,6,7-tetrahydroindazol-3-yl)triazol-1-yl]butanoyl]-4-hydroxy-N-methyl-pyrrolidine-2-carboxamide;
(2S,4R)-1-[(2S)-3,3-dimethyl-2-[4-(1-methylbenzimidazol-2-yl)triazol-1-yl]butanoyl]-4-hydroxy-N-methyl-pyrrolidine-2-carboxamide;
(2S,4R)-1-[(2S)-2-[4-(5-chloro-2-methoxy-phenyl)triazol-1-yl]-3,3-dimethyl-butanoyl]-4-hydroxy-N-methyl-pyrrolidine-2-carboxamide;
(2S,4R)-1-[(2S)-3,3-dimethyl-2-[4-[6-(trifluoromethyl)-3-pyridyl]triazol-1-yl]butanoyl]-4-hydroxy-N-methyl-pyrrolidine-2-carboxamide;
(2S,4R)-1-[(2S)-3,3-dimethyl-2-[4-(2-phenoxyethyl)triazol-1-yl]butanoyl]-4-hydroxy-N-methyl-pyrrolidine-2-carboxamide;
(2S,4R)-1-[(2S)-3,3-dimethyl-2-[4-[[4-(trifluoromethyl)anilino]methyl]triazol-1-yl]butanoyl]-4-hydroxy-N-methyl-pyrrolidine-2-carboxamide;
(2S,4R)-4-hydroxy-1-[(2S)-2-[4-[[3-(hydroxymethyl)phenoxy]methyl]triazol-1-yl]-3,3-dimethyl-butanoyl]-N-methyl-pyrrolidine-2-carboxamide;
(2R,4S)-4-hydroxy-1-[(2S)-2-[4-[[4-(hydroxymethyl)cyclohexoxy]methyl]triazol-1-yl]-3,3-dimethyl-butanoyl]-N-methyl-pyrrolidine-2-carboxamide;
(2S,4R)-1-[(2S)-3,3-dimethyl-2-[4-[(p-tolylcarbamoylamino)methyl]triazol-1-yl]butanoyl]-4-hydroxy-N-methyl-pyrrolidine-2-carboxamide;
(2S,4R)-4-hydroxy-1-[(2S)-2-[4-[5-[(2-methoxypyrimidin-4-yl)-methyl-amino]-2-methylphenyl]triazol-1-yl]-3,3-dimethyl-butanoyl]-N-methyl-pyrrolidine-2-carboxamide;
(2S,4R)-1-[(2S)-2-[4-[(4,4-dioxo-4lambda6-thia-5-azaspiro[2.5]octan-5-yl)methyl]triazol-1-yl]-3,3-dimethyl-butanoyl]-4-hydroxy-N-methyl-pyrrolidine-2-carboxamide;
(2S,4R)-4-hydroxy-1-[(2S)-2-[4-[[4-(hydroxymethyl)phenoxy]methyl]triazol-1-yl]-3,3-dimethyl-butanoyl]-N-methyl-pyrrolidine-2-carboxamide;
(2S,4R)-4-hydroxy-1-[(2S)-2-(4-imidazo[1,2-a]pyridin-2-yltriazol-1-yl)-3,3-dimethylbutanoyl]-N-methyl-pyrrolidine-2-carboxamide;
(2S,4R)-1-[(2S)-2-[4-[2-(difluoromethoxy)phenyl]triazol-1-yl]-3,3-dimethyl-butanoyl]-4-hydroxy-N-methyl-pyrrolidine-2-carboxamide;
(2S,4R)-1-[(2S)-2-[4-(5-cyclopropyloxazol-4-yl)triazol-1-yl]-3,3-dimethyl-butanoyl]-4-hydroxy-N-methyl-pyrrolidine-2-carboxamide;
(2S,4R)-1-[(2S)-3,3-dimethyl-2-[4-[5-(trifluoromethoxy)-2-pyridyl]triazol-1-yl]butanoyl]-4-hydroxy-N-methyl-pyrrolidine-2-carboxamide;
(2S,4R)-1-[(2S)-3,3-dimethyl-2-[4-[3-(4-pyridylmethylamino)phenyl]triazol-1-yl]butanoyl]-4-hydroxy-N-methyl-pyrrolidine-2-carboxamide;
(2R,4S)-1-[(2S)-2-[4-[(4-fluorophenyl)-hydroxy-methyl]triazol-1-yl]-3,3-dimethyl-butanoyl]-4-hydroxy-N-methyl-pyrrolidine-2-carboxamide;
tert-butyl 2-[1-[(1S)-1-[(2R,4S)-4-hydroxy-2-(methylcarbamoyl)pyrrolidine-1-carbonyl]-2,2-dimethyl-propyl]triazol-4-yl]pyrrolidine-1-carboxylate;
(2S,4R)-1-[(2S)-2-[4-(2-chloro-4-methoxy-phenyl)triazol-1-yl]-3,3-dimethyl-butanoyl]-4-hydroxy-N-methyl-pyrrolidine-2-carboxamide;
(2S,4R)-1-[(2S)-2-[4-(1,3-benzothiazol-2-yl)triazol-1-yl]-3,3-dimethyl-butanoyl]-4-hydroxy-N-methyl-pyrrolidine-2-carboxamide;
(2S,4R)-1-[(2S)-3,3-dimethyl-2-[4-(3-quinolyl)triazol-1-yl]butanoyl]-4-hydroxy-N-methyl-pyrrolidine-2-carboxamide;
(2S,4R)-1-[(2S)-2-[4-[[(2-chlorophenyl)carbamoylamino]methyl]triazol-1-yl]-3,3-dimethylbutanoyl]-4-hydroxy-N-methyl-pyrrolidine-2-carboxamide;
(2S,4R)-4-hydroxy-1-[(2S)-2-[4-[(2-methoxyphenyl)sulfanylmethyl]triazol-1-yl]-3,3-dimethyl-butanoyl]-N-methyl-pyrrolidine-2-carboxamide;
(2R,4S)-4-hydroxy-1-[(2S)-2-[4-(2-hydroxy-2-phenyl-ethyl)triazol-1-yl]-3,3-dimethylbutanoyl]-N-methyl-pyrrolidine-2-carboxamide;
(2R,4S)-4-hydroxy-1-[(2S)-2-[4-(1-hydroxy-2-phenyl-ethyl)triazol-1-yl]-3,3-dimethylbutanoyl]-N-methyl-pyrrolidine-2-carboxamide;
(2R,4S)-4-hydroxy-1-[(2S)-2-[4-[3-hydroxy-3-(2-pyridyl)propyl]triazol-1-yl]-3,3-dimethylbutanoyl]-N-methyl-pyrrolidine-2-carboxamide;
(2S,4R)-1-[(2S)-3,3-dimethyl-2-[4-[(2-methylbenzimidazol-1-yl)methyl]triazol-1-yl]butanoyl]-4-hydroxy-N-methyl-pyrrolidine-2-carboxamide;
N-[[1-[(1S)-1-[(2S,4R)-4-hydroxy-2-(methylcarbamoyl)pyrrolidine-1-carbonyl]-2,2-dimethyl-propyl]triazol-4-yl]methyl]pyridine-2-carboxamide;
(2S,4R)-1-[(2S)-2-[4-(4-tert-butoxyphenyl)triazol-1-yl]-3,3-dimethyl-butanoyl]-4-hydroxy-N-methyl-pyrrolidine-2-carboxamide;
N-[[1-[(1S)-1-[(2S,4R)-4-hydroxy-2-(methylcarbamoyl)pyrrolidine-1-carbonyl]-2,2-dimethyl-propyl]triazol-4-yl]methyl]-6-oxo-1H-pyridine-3-carboxamide;
(2R,4S)-4-hydroxy-1-[(2S)-2-[4-[hydroxy(p-tolyl)methyl]triazol-1-yl]-3,3-dimethylbutanoyl]-N-methyl-pyrrolidine-2-carboxamide;
(2S,4R)-1-[(2S)-3,3-dimethyl-2-[4-(2-phenylphenyl)triazol-1-yl]butanoyl]-4-hydroxy-N-methyl-pyrrolidine-2-carboxamide;
(2S,4R)-1-[(2S)-3,3-dimethyl-2-[4-[[(3-nitrophenyl)carbamoylamino]methyl]triazol-1-yl]butanoyl]-4-hydroxy-N-methyl-pyrrolidine-2-carboxamide;
(2S,4R)-1-[(2S)-3,3-dimethyl-2-[4-[(2-phenylimidazol-1-yl)methyl]triazol-1-yl]butanoyl]-4-hydroxy-N-methyl-pyrrolidine-2-carboxamide;
(2S,4R)-1-[(2S)-2-[4-[(5,6-dimethylbenzimidazol-1-yl)methyl]triazol-1-yl]-3,3-dimethylbutanoyl]-4-hydroxy-N-methyl-pyrrolidine-2-carboxamide;
(2S,4R)-1-[(2S)-3,3-dimethyl-2-[4-[(phenylcarbamoylamino)methyl]triazol-1-yl]butanoyl]-4-hydroxy-N-methyl-pyrrolidine-2-carboxamide;
(2S,4R)-1-[(2S)-2-[4-(4,4-difluoro-1-hydroxy-cyclohexyl)triazol-1-yl]-3,3-dimethylbutanoyl]-4-hydroxy-N-methyl-pyrrolidine-2-carboxamide;
(2S,4R)-1-[(2S)-2-[4-[(1,1-dioxo-1,4-thiazinan-4-yl)methyl]triazol-1-yl]-3,3-dimethylbutanoyl]-4-hydroxy-N-methyl-pyrrolidine-2-carboxamide;
(2S,4R)-1-[(2S)-3,3-dimethyl-2-[4-[2-methyl-5-(trifluoromethyl)pyrazol-3-yl]triazol-1-yl]butanoyl]-4-hydroxy-N-methyl-pyrrolidine-2-carboxamide;
(2S,4R)-1-[(2S)-3,3-dimethyl-2-[4-[[6-(trifluoromethyl)-2-pyridyl]oxymethyl]triazol-1-yl]butanoyl]-4-hydroxy-N-methyl-pyrrolidine-2-carboxamide;
(2S,4R)-1-[(2S)-2-[4-[[(5-aminopyrazin-2-yl)-methyl-amino]methyl]triazol-1-yl]-3,3-dimethyl-butanoyl]-4-hydroxy-N-methyl-pyrrolidine-2-carboxamide;
(2S,4R)-4-hydroxy-1-[(2S)-2-[4-[[4-(hydroxymethyl)phenyl]sulfanylmethyl]triazol-1-yl]-3,3-dimethyl-butanoyl]-N-methyl-pyrrolidine-2-carboxamide;
N-[[1-[(1S)-1-[(2S,4R)-4-hydroxy-2-(methylcarbamoyl)pyrrolidine-1-carbonyl]-2,2-dimethyl-propyl]triazol-4-yl]methyl]-2-methyl-pyrazole-3-carboxamide;
(2S,4R)-1-[(2S)-3,3-dimethyl-2-[4-[(5-oxo-1,4-oxazepan-4-yl)methyl]triazol-1-yl]butanoyl]-4-hydroxy-N-methyl-pyrrolidine-2-carboxamide;
(2R,4S)-4-hydroxy-1-[(2S)-2-[4-(3-hydroxy-3-phenyl-propyl)triazol-1-yl]-3,3-dimethylbutanoyl]-N-methyl-pyrrolidine-2-carboxamide;
(2S,4R)-1-[(2S)-3,3-dimethyl-2-[4-[4-methyl-3-(phenylcarbamoyl)phenyl]triazol-1-yl]butanoyl]-4-hydroxy-N-methyl-pyrrolidine-2-carboxamide;
(2S,4R)-1-[(2S)-3,3-dimethyl-2-[4-[(2-phenylindol-1-yl)methyl]triazol-1-yl]butanoyl]-4-hydroxy-N-methyl-pyrrolidine-2-carboxamide;
(2S,4R)-1-[(2S)-3,3-dimethyl-2-[4-(3-phenylphenyl)triazol-1-yl]butanoyl]-4-hydroxy-N-methyl-pyrrolidine-2-carboxamide;
(2S,4R)-1-[(2S)-2-[4-(4-ethoxy-2,3-difluoro-phenyl)triazol-1-yl]-3,3-dimethyl-butanoyl]-4-hydroxy-N-methyl-pyrrolidine-2-carboxamide;
(2S,4R)-1-[(2S)-2-[4-[3-[(4-cyanophenyl)sulfonylamino]phenyl]triazol-1-yl]-3,3-dimethylbutanoyl]-4-hydroxy-N-methyl-pyrrolidine-2-carboxamide;
(2S,4R)-1-[(2S)-3,3-dimethyl-2-[4-[(3-methylsulfonylphenoxy)methyl]triazol-1-yl]butanoyl]-4-hydroxy-N-methyl-pyrrolidine-2-carboxamide;
(2S,4R)-4-hydroxy-1-[(2S)-2-[4-[(2-methoxyphenoxy)methyl]triazol-1-yl]-3,3-dimethylbutanoyl]-N-methyl-pyrrolidine-2-carboxamide;
(2S,4R)-1-[(2S)-2-[4-[2-(ethylamino)-4-pyridyl]triazol-1-yl]-3,3-dimethyl-butanoyl]-4-hydroxy-N-methyl-pyrrolidine-2-carboxamide;
(2S,4R)-1-[(2S)-2-[4-[1-(cyclopropylsulfonylamino)-1-methyl-ethyl]triazol-1-yl]-3,3-dimethyl-butanoyl]-4-hydroxy-N-methyl-pyrrolidine-2-carboxamide;
(2S,4R)-4-hydroxy-1-[(2S)-2-[4-(5-isoquinolyl)triazol-1-yl]-3,3-dimethyl-butanoyl]-N-methyl-pyrrolidine-2-carboxamide;
(2S,4R)-1-[(2S)-3,3-dimethyl-2-[4-[[methyl(p-tolylsulfonyl)amino]methyl]triazol-1-yl]butanoyl]-4-hydroxy-N-methyl-pyrrolidine-2-carboxamide;
(2R,4S)-4-hydroxy-1-[(2S)-2-[4-[4-hydroxy-4-(2-pyridyl)butyl]triazol-1-yl]-3,3-dimethylbutanoyl]-N-methyl-pyrrolidine-2-carboxamide;
(2S,4R)-4-hydroxy-1-[(2S)-2-[4-(1-hydroxy-4,4-dimethyl-cyclohexyl)triazol-1-yl]-3,3-dimethyl-butanoyl]-N-methyl-pyrrolidine-2-carboxamide;
tert-butyl 7-[1-[(1S)-1-[(2R,4S)-4-hydroxy-2-(methylcarbamoyl)pyrrolidine-1-carbonyl]-2,2-dimethyl-propyl]triazol-4-yl]-4-azaspiro[2.4]heptane-4-carboxylate;
(2S,4R)-1-[(2S)-3,3-dimethyl-2-[4-(1-sulfamoylcyclobutyl)triazol-1-yl]butanoyl]-4-hydroxy-N-methyl-pyrrolidine-2-carboxamide;
(2R,4S)-4-hydroxy-1-[(2S)-2-[4-[hydroxy-[3-(trifluoromethyl)phenyl]methyl]triazol-1-yl]-3,3-dimethyl-butanoyl]-N-methyl-pyrrolidine-2-carboxamide;
N-benzyl-1-[(1S)-1-[(2S,4R)-4-hydroxy-2-(methylcarbamoyl)pyrrolidine-1-carbonyl]-2,2-dimethyl-propyl]triazole-4-carboxamide;
(2S,4R)-1-[(2S)-2-[4-[4-(dimethylamino)phenyl]triazol-1-yl]-3,3-dimethyl-butanoyl]-4-hydroxy-N-methyl-pyrrolidine-2-carboxamide;
(2S,4R)-4-hydroxy-1-[(2S)-2-[4-(2-methoxy-1-naphthyl)triazol-1-yl]-3,3-dimethylbutanoyl]-N-methyl-pyrrolidine-2-carboxamide;
(2S,4R)-1-[(2S)-2-[4-[(3,5-difluorophenoxy)methyl]triazol-1-yl]-3,3-dimethyl-butanoyl]-4-hydroxy-N-methyl-pyrrolidine-2-carboxamide;
(2S,4R)-1-[(2S)-2-[4-[(4-chlorophenoxy)methyl]triazol-1-yl]-3,3-dimethyl-butanoyl]-4-hydroxy-N-methyl-pyrrolidine-2-carboxamide;
(2S,4R)-1-[(2S)-2-[4-[3-(benzylamino)phenyl]triazol-1-yl]-3,3-dimethyl-butanoyl]-4-hydroxy-N-methyl-pyrrolidine-2-carboxamide;
(2S,4R)-1-[(2S)-3,3-dimethyl-2-[4-(1-methylsulfonyl-4-piperidyl)triazol-1-yl]butanoyl]-4-hydroxy-N-methyl-pyrrolidine-2-carboxamide;
(2S,4R)-1-[(2S)-2-[4-[1-(dimethylsulfamoyl)cyclopropyl]triazol-1-yl]-3,3-dimethylbutanoyl]-4-hydroxy-N-methyl-pyrrolidine-2-carboxamide;
(2R,4S)-4-hydroxy-1-[(2S)-2-[4-[[3-(1-hydroxyethyl)phenoxy]methyl]triazol-1-yl]-3,3-dimethyl-butanoyl]-N-methyl-pyrrolidine-2-carboxamide;
5-[1-[(1S)-1-[(2S,4R)-4-hydroxy-2-(methylcarbamoyl)pyrrolidine-1-carbonyl]-2,2-dimethylpropyl]triazol-4-yl]pyridine-3-carboxamide;
(2S,4R)-1-[(2S)-3,3-dimethyl-2-[4-[6-(trifluoromethyl)-2-pyridyl]triazol-1-yl]butanoyl]-4-hydroxy-N-methyl-pyrrolidine-2-carboxamide;
(2R,4S)-1-[(2S)-3,3-dimethyl-2-[4-[4-(2,2,2-trifluoro-1-hydroxy-ethyl)phenyl]triazol-1-yl]butanoyl]-4-hydroxy-N-methyl-pyrrolidine-2-carboxamide;
(2S,4R)-1-[(2S)-2-[4-[(3-fluoroanilino)methyl]triazol-1-yl]-3,3-dimethyl-butanoyl]-4-hydroxy-N-methyl-pyrrolidine-2-carboxamide;
(2R,4S)-4-hydroxy-1-[(2S)-2-[4-[(5-hydroxy-2-oxo-1-piperidyl)methyl]triazol-1-yl]-3,3-dimethyl-butanoyl]-N-methyl-pyrrolidine-2-carboxamide;
(2S,4R)-1-[(2S)-2-[4-(4-acetamidophenyl)triazol-1-yl]-3,3-dimethyl-butanoyl]-4-hydroxy-N-methyl-pyrrolidine-2-carboxamide;
(2S,4R)-1-[(2S)-3,3-dimethyl-2-[4-[3-(trifluoromethyl)benzoyl]triazol-1-yl]butanoyl]-4-hydroxy-N-methyl-pyrrolidine-2-carboxamide;
(2S,4R)-1-[(2S)-2-[4-[(3,5-dimethyl-1,2,4-triazol-1-yl)methyl]triazol-1-yl]-3,3-dimethylbutanoyl]-4-hydroxy-N-methyl-pyrrolidine-2-carboxamide;
tert-butyl 4-[[1-[(1S)-1-[(2S,4R)-4-hydroxy-2-(methylcarbamoyl)pyrrolidine-1-carbonyl]-2,2-dimethyl-propyl]triazol-4-yl]methyl]-1,4-diazepane-1-carboxylate;
(2R,4S)-4-hydroxy-1-[(2S)-2-[4-(2-hydroxynorbornan-2-yl)triazol-1-yl]-3,3-dimethylbutanoyl]-N-methyl-pyrrolidine-2-carboxamide;
(2R,4S)-4-hydroxy-1-[(2S)-2-[4-[[4-(1-hydroxyethyl)phenoxy]methyl]triazol-1-yl]-3,3-dimethyl-butanoyl]-N-methyl-pyrrolidine-2-carboxamide;
(2R,4S)-4-hydroxy-1-[(2S)-2-[4-(4-isopropylcyclohexyl)triazol-1-yl]-3,3-dimethylbutanoyl]-N-methyl-pyrrolidine-2-carboxamide;
(2R,4S)-4-hydroxy-1-[(2S)-2-[4-[[4-(1-hydroxypropyl)phenoxy]methyl]triazol-1-yl]-3,3-dimethyl-butanoyl]-N-methyl-pyrrolidine-2-carboxamide;
(2S,4R)-1-[(2S)-3,3-dimethyl-2-[4-(5-phenyl-2-thienyl)triazol-1-yl]butanoyl]-4-hydroxy-N-methyl-pyrrolidine-2-carboxamide;
(2S,4R)-4-hydroxy-1-[(2S)-2-[4-(4-isoquinolyl)triazol-1-yl]-3,3-dimethyl-butanoyl]-N-methyl-pyrrolidine-2-carboxamide;
tert-butyl 1-[1-[(1S)-1-[(2S,4R)-4-hydroxy-2-(methylcarbamoyl)pyrrolidine-1-carbonyl]-2,2-dimethyl-propyl]triazol-4-yl]-2-azabicyclo[2.1.1]hexane-2-carboxylate;
(2S,4R)-1-[(2S)-2-[4-[4-(cyclobutoxy)phenyl]triazol-1-yl]-3,3-dimethyl-butanoyl]-4-hydroxy-N-methyl-pyrrolidine-2-carboxamide;
(2S,4R)-1-[(2S)-2-[4-[(1-cyanocyclohexyl)methyl]triazol-1-yl]-3,3-dimethyl-butanoyl]-4-hydroxy-N-methyl-pyrrolidine-2-carboxamide;
(2S,4R)-4-hydroxy-1-[(2S)-2-[4-(2-isopropoxyphenyl)triazol-1-yl]-3,3-dimethyl-butanoyl]-N-methyl-pyrrolidine-2-carboxamide;
(2S,4R)-1-[(2S)-2-[4-[1-(4-fluorophenyl)cyclopropyl]triazol-1-yl]-3,3-dimethyl-butanoyl]-4-hydroxy-N-methyl-pyrrolidine-2-carboxamide;
tert-butyl 2-[1-[(1S)-1-[(2R,4S)-4-hydroxy-2-(methylcarbamoyl)pyrrolidine-1-carbonyl]-2,2-dimethyl-propyl]triazol-4-yl]morpholine-4-carboxylate;
(2R,4S)-4-hydroxy-1-[(2S)-2-[4-(1-hydroxy-1-methyl-2-phenyl-ethyl)triazol-1-yl]-3,3-dimethyl-butanoyl]-N-methyl-pyrrolidine-2-carboxamide;
(2S,4R)-4-hydroxy-1-[(2S)-2-[4-(indol-1-ylmethyl)triazol-1-yl]-3,3-dimethyl-butanoyl]-N-methyl-pyrrolidine-2-carboxamide;
tert-butyl 3-[[1-[(1S)-1-[(2S,4R)-4-hydroxy-2-(methylcarbamoyl)pyrrolidine-1-carbonyl]-2,2-dimethyl-propyl]triazol-4-yl]methyl]azetidine-1-carboxylate;
(2S,4R)-1-[(2S)-3,3-dimethyl-2-[4-[3-(trifluoromethyl)phenyl]triazol-1-yl]butanoyl]-4-hydroxy-N-methyl-pyrrolidine-2-carboxamide;
(2S,4R)-4-hydroxy-1-[(2S)-2-[4-(4-isopropoxyphenyl)triazol-1-yl]-3,3-dimethyl-butanoyl]-N-methyl-pyrrolidine-2-carboxamide;
(2S,4R)-1-[(2S)-2-[4-[[(3-cyanobenzoyl)amino]methyl]triazol-1-yl]-3,3-dimethyl-butanoyl]-4-hydroxy-N-methyl-pyrrolidine-2-carboxamide;
(2S,4R)-4-hydroxy-1-[(2S)-2-[4-(6-isopropoxy-3-pyridyl)triazol-1-yl]-3,3-dimethylbutanoyl]-N-methyl-pyrrolidine-2-carboxamide;
(2S,4R)-4-hydroxy-1-[(2S)-2-[4-[(3-hydroxyphenoxy)methyl]triazol-1-yl]-3,3-dimethylbutanoyl]-N-methyl-pyrrolidine-2-carboxamide;
(25,4R)-1-[(25)-3,3-dimethyl-2-[4-(1-phenylcyclopropyl)triazol-1-yl]butanoyl]-4-hydroxy-N-methyl-pyrrolidine-2-carboxamide;
(2S,4R)-1-[(2S)-2-[4-(3,4-dimethoxyphenyl)triazol-1-yl]-3,3-dimethyl-butanoyl]-4-hydroxy-N-methyl-pyrrolidine-2-carboxamide;
(2S,4R)-1-[(2S)-3,3-dimethyl-2-[4-[(3,4,5-trimethylpyrazol-1-yl)methyl]triazol-1-yl]butanoyl]-4-hydroxy-N-methyl-pyrrolidine-2-carboxamide;
(2R,4S)-4-hydroxy-1-[(2S)-2-[4-[2-(3-hydroxy-3-methyl-1-piperidyl)ethyl]triazol-1-yl]-3,3-dimethyl-butanoyl]-N-methyl-pyrrolidine-2-carboxamide;
tert-butyl 7-[1-[(1S)-1-[(2S,4R)-4-hydroxy-2-(methylcarbamoyl)pyrrolidine-1-carbonyl]-2,2-dimethyl-propyl]triazol-4-yl]indoline-1-carboxylate;
(2S,4R)-4-hydroxy-1-[(2S)-2-[4-(6-isoquinolyl)triazol-1-yl]-3,3-dimethyl-butanoyl]-N-methyl-pyrrolidine-2-carboxamide;
(2S,4R)-1-[(2S)-2-[4-(2-chloro-5-methoxy-phenyl)triazol-1-yl]-3,3-dimethyl-butanoyl]-4-hydroxy-N-methyl-pyrrolidine-2-carboxamide;
(2S,4R)-1-[(2S)-3,3-dimethyl-2-(4-tetralin-5-yltriazol-1-yl)butanoyl]-4-hydroxy-N-methyl-pyrrolidine-2-carboxamide;
(2S,4R)-1-[(2S)-3,3-dimethyl-2-[4-[5-(trifluoromethyl)-2-pyridyl]triazol-1-yl]butanoyl]-4-hydroxy-N-methyl-pyrrolidine-2-carboxamide;
(2S,4R)-4-hydroxy-1-[(2S)-2-[4-[3-(isopropylamino)phenyl]triazol-1-yl]-3,3-dimethylbutanoyl]-N-methyl-pyrrolidine-2-carboxamide;
(2S,4R)-4-hydroxy-1-[(2S)-2-[4-[(3-methoxyphenoxy)methyl]triazol-1-yl]-3,3-dimethylbutanoyl]-N-methyl-pyrrolidine-2-carboxamide;
(2S,4R)-1-[(2S)-3,3-dimethyl-2-[4-[(3-methylphenoxy)methyl]triazol-1-yl]butanoyl]-4-hydroxy-N-methyl-pyrrolidine-2-carboxamide;
(2S,4R)-1-[(2S)-2-[4-[[(5-cyanopyrazin-2-yl)-methyl-amino]methyl]triazol-1-yl]-3,3-dimethyl-butanoyl]-4-hydroxy-N-methyl-pyrrolidine-2-carboxamide;
(2S,4R)-1-[(2S)-2-[4-[4-(cyclopentoxy)phenyl]triazol-1-yl]-3,3-dimethyl-butanoyl]-4-hydroxy-N-methyl-pyrrolidine-2-carboxamide;
(2R,4S)-4-hydroxy-1-[(2S)-2-[4-(2-hydroxy-3-phenyl-propyl)triazol-1-yl]-3,3-dimethylbutanoyl]-N-methyl-pyrrolidine-2-carboxamide;
(2S,4R)-1-[(2S)-2-[4-[(4-fluorophenoxy)methyl]triazol-1-yl]-3,3-dimethyl-butanoyl]-4-hydroxy-N-methyl-pyrrolidine-2-carboxamide;
(2S,4R)-1-[(2S)-3,3-dimethyl-2-[4-(5-quinolyl)triazol-1-yl]butanoyl]-4-hydroxy-N-methyl-pyrrolidine-2-carboxamide;
(2S,4R)-1-[(2S)-2-[4-(4-ethoxy-3-fluoro-phenyl)triazol-1-yl]-3,3-dimethyl-butanoyl]-4-hydroxy-N-methyl-pyrrolidine-2-carboxamide;
(2S,4R)-1-[(2S)-3,3-dimethyl-2-(4-quinoxalin-2-yltriazol-1-yl)butanoyl]-4-hydroxy-N-methyl-pyrrolidine-2-carboxamide;
(2S,4R)-1-[(2S)-2-[4-[(4-tert-butylphenoxy)methyl]triazol-1-yl]-3,3-dimethyl-butanoyl]-4-hydroxy-N-methyl-pyrrolidine-2-carboxamide;
(2S,4R)-1-[(2S)-2-[4-[(3-cyanophenoxy)methyl]triazol-1-yl]-3,3-dimethyl-butanoyl]-4-hydroxy-N-methyl-pyrrolidine-2-carboxamide;
(2S,4R)-4-hydroxy-1-[(2S)-2-(4-isopropenyltriazol-1-yl)-3,3-dimethyl-butanoyl]-N-methyl-pyrrolidine-2-carboxamide;
(2S,4R)-1-[(2S)-3,3-dimethyl-2-[4-(1-methyl-4-piperidyl)triazol-1-yl]butanoyl]-4-hydroxy-N-methyl-pyrrolidine-2-carboxamide;
(2R,4S)-1-[(2R)-2-[4-[amino(tetrahydrofuran-2-yl)methyl]triazol-1-yl]-3,3-dimethylbutanoyl]-4-hydroxy-N-methyl-pyrrolidine-2-carboxamide;
(2S,4R)-1-[(2S)-2-[4-(5-cyano-3-pyridyl)triazol-1-yl]-3,3-dimethyl-butanoyl]-4-hydroxy-N-methyl-pyrrolidine-2-carboxamide;
(2S,4R)-1-[(2S)-2-[4-(6-cyano-3-pyridyl)triazol-1-yl]-3,3-dimethyl-butanoyl]-4-hydroxy-N-methyl-pyrrolidine-2-carboxamide;
(2S,4R)-1-[(2S)-2-[4-[(2-ethylimidazol-1-yl)methyl]triazol-1-yl]-3,3-dimethyl-butanoyl]-4-hydroxy-N-methyl-pyrrolidine-2-carboxamide;
(2R,4S)-1-[(2R)-2-(4-cyclopropyltriazol-1-yl)-3,3-dimethyl-butanoyl]-4-hydroxy-N-(5-methyltetrahydrofuran-3-yl)pyrrolidine-2-carboxamide;
(2S,4R)-4-hydroxy-1-[(2S)-2-[4-(3-methoxyisothiazol-5-yl)triazol-1-yl]-3,3-dimethylbutanoyl]-N-methyl-pyrrolidine-2-carboxamide;
(2S,4R)-1-[(2S)-2-[4-[2-[tert-butyl(methyl)amino]ethyl]triazol-1-yl]-3,3-dimethyl-butanoyl]-4-hydroxy-N-methyl-pyrrolidine-2-carboxamide;
(2S,4R)-1-[(2S)-2-[4-(3,3-dimethoxycyclobutyl)triazol-1-yl]-3,3-dimethyl-butanoyl]-4-hydroxy-N-methyl-pyrrolidine-2-carboxamide;
(2S,4R)-4-hydroxy-1-[(2S)-2-[4-(1H-indol-5-yl)triazol-1-yl]-3,3-dimethyl-butanoyl]-N-methyl-pyrrolidine-2-carboxamide;
(2S,4R)-1-[(2S)-3,3-dimethyl-2-[4-(1-sulfamoylcyclopropyl)triazol-1-yl]butanoyl]-4-hydroxy-N-methyl-pyrrolidine-2-carboxamide;
4-[1-[(1S)-1-[(2S,4R)-4-hydroxy-2-(methylcarbamoyl)pyrrolidine-1-carbonyl]-2,2-dimethylpropyl]triazol-4-yl]benzoic acid;
6-[1-[(1S)-1-[(2S,4R)-4-hydroxy-2-(methylcarbamoyl)pyrrolidine-1-carbonyl]-2,2-dimethylpropyl]triazol-4-yl]pyridine-3-carboxylic acid;
5-[1-[(1S)-1-[(2S,4R)-4-hydroxy-2-(methylcarbamoyl)pyrrolidine-1-carbonyl]-2,2-dimethylpropyl]triazol-4-yl]pyridine-3-carboxylic acid;
(2R,4S)-4-hydroxy-1-[(2R)-2-[4-[2-hydroxy-2-(2-pyridyl)ethyl]triazol-1-yl]-3,3-dimethylbutanoyl]-N-methyl-pyrrolidine-2-carboxamide;
(2R,4S)-1-[(2R)-2-(4-cyclopropyltriazol-1-yl)-3,3-dimethyl-butanoyl]-4-hydroxy-N-(1-oxazol-5-ylethyl)pyrrolidine-2-carboxamide;
(2S,4R)-1-[(2S)-2-[4-[(4-fluoroanilino)methyl]triazol-1-yl]-3,3-dimethyl-butanoyl]-4-hydroxy-N-methyl-pyrrolidine-2-carboxamide;
(2S,4R)-1-[(2S)-3,3-dimethyl-2-[4-(6-quinolyl)triazol-1-yl]butanoyl]-4-hydroxy-N-methyl-pyrrolidine-2-carboxamide;
(2S,4R)-1-[(2S)-2-[4-[(4-aminocyclohexoxy)methyl]triazol-1-yl]-3,3-dimethyl-butanoyl]-4-hydroxy-N-methyl-pyrrolidine-2-carboxamide;
3-hydroxy-3-[[1-[(1S)-1-[(2S,4R)-4-hydroxy-2-(methylcarbamoyl)pyrrolidine- 1 -carbonyl]-2,2-dimethyl-propyl]triazol-4-yl]methyl]cyclobutanecarboxylic acid;
(2S,4R)-1-[(2R)-2-(4-cyclopropyltriazol-1-yl)-3,3-dimethyl-butanoyl]-4-hydroxy-N-(1-oxothiolan-3-yl)pyrrolidine-2-carboxamide;
(2S,4R)-1-[(2S)-2-[4-(5-chloro-1,3-dimethyl-pyrazol-4-yl)triazol-1-yl]-3,3-dimethylbutanoyl]-4-hydroxy-N-methyl-pyrrolidine-2-carboxamide;
(2S,4R)-1-[(2S)-2-[4-(3-cyclobutylphenyl)triazol-1-yl]-3,3-dimethyl-butanoyl]-4-hydroxy-N-methyl-pyrrolidine-2-carboxamide;
(2S,4R)-1-[(2S)-3,3-dimethyl-2-[4-[(1-oxo-1,4-thiazinan-4-yl)methyl]triazol-1-yl]butanoyl]-4-hydroxy-N-methyl-pyrrolidine-2-carboxamide;
(2S,4R)-N-(2-cyanocyclohexyl)-1-[(2R)-2-(4-cyclopropyltriazol-1-yl)-3,3-dimethylbutanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(3-hydroxypyrazin-2-yl)methyl]pyrrolidine-2-carboxamide;
(2S,4R)-4-hydroxy-1-[(2S)-2-[4-[3-(1-hydroxy-1-methyl-ethyl)phenyl]triazol-1-yl]-3,3-dimethyl-butanoyl]-N-methyl-pyrrolidine-2-carboxamide;
(2S,4R)-1-[(2S)-2-[4-[[4-(aminomethyl)phenoxy]methyl]triazol-1-yl]-3,3-dimethylbutanoyl]-4-hydroxy-N-methyl-pyrrolidine-2-carboxamide;
(2R,4S)-1-[(2R)-2-(4-cyclopropyltriazol-1-yl)-3,3-dimethyl-butanoyl]-4-hydroxy-N-(5-methyl-5-azaspiro[2.4]heptan-7-yl)pyrrolidine-2-carboxamide;
(2R,4S)-1-[(2R)-2-(4-cyclopropyltriazol-1-yl)-3,3-dimethyl-butanoyl]-4-hydroxy-N-[1-(5-methyl-1,3,4-oxadiazol-2-yl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[(1R,5S,6S,7R)-7-hydroxy-6-bicyclo[3.2.0]heptanyl]pyrrolidine-2-carboxamide;
(2R,4S)-1-[(2R)-2-(4-cyclopropyltriazol-1-yl)-3,3-dimethyl-butanoyl]-N-[(3,5-dimethyl-4H-isoxazol-5-yl)methyl]-4-hydroxy-pyrrolidine-2-carboxamide;
(2R,4S)-1-[(2R)-2-(4-cyclopropyltriazol-1-yl)-3,3-dimethyl-butanoyl]-4-hydroxy-N-[(1-methyl-5-oxo-pyrrolidin-2-yl)methyl]pyrrolidine-2-carboxamide;
(2S,4R)-1-[(2S)-2-[4-[(4-chloroanilino)methyl]triazol-1-yl]-3,3-dimethyl-butanoyl]-4-hydroxy-N-methyl-pyrrolidine-2-carboxamide;
(2S,4R)-1-[(2S)-2-(4-cyclopropyltriazol-1-yl)-3,3-dimethyl-butanoyl]-4-hydroxy-N-[2-[(3R,4S)-4-hydroxytetrahydrofuran-3-yl]ethyl]pyrrolidine-2-carboxamide;
(2S,4R)-1-[(2S)-2-[4-(4-chloro-3-methoxy-phenyl)triazol-1-yl]-3,3-dimethyl-butanoyl]-4-hydroxy-N-methyl-pyrrolidine-2-carboxamide;
(2S,4R)-1-[(2S)-3,3-dimethyl-2-[4-(2-methyl-6-quinolyl)triazol-1-yl]butanoyl]-4-hydroxy-N-methyl-pyrrolidine-2-carboxamide;
(2S,4R)-1-[(2S)-3,3-dimethyl-2-[4-[3-(trifluoromethyl)-2-pyridyl]triazol-1-yl]butanoyl]-4-hydroxy-N-methyl-pyrrolidine-2-carboxamide;
(2S,4R)-1-[(2R)-2-(4-cyclopropyltriazol-1-yl)-3,3-dimethyl-butanoyl]-4-hydroxy-N-(5,6,7,8-tetrahydroimidazo[1,5-a]pyridin-7-yl)pyrrolidine-2-carboxamide;
(2R,4S)-1-[(2R)-2-(4-cyclopropyltriazol-1-yl)-3,3-dimethyl-butanoyl]-4-hydroxy-N-[1-(2-hydroxy-4-pyridyl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[(1R,2R)-2-(2-methylpyrazol-3-yl)cyclopropyl]pyrrolidine-2-carboxamide;
(2S,4R)-1-[(2R)-2-(4-cyclopropyltriazol-1-yl)-3,3-dimethyl-butanoyl]-4-hydroxy-N-(5,6,7,8-tetrahydro-[1,2,4]triazolo[4,3-a]pyridin-8-yl)pyrrolidine-2-carboxamide;
(2S,4R)-1-[(2S)-2-[4-(1-benzyloxy-1-methyl-ethyl)triazol-1-yl]-3,3-dimethyl-butanoyl]-4-hydroxy-N-methyl-pyrrolidine-2-carboxamide;
(2S,4R)-1-[(2R)-2-(4-cyclopropyltriazol-1-yl)-3,3-dimethyl-butanoyl]-4-hydroxy-N-(5-oxo-6-azaspiro[3.4]octan-2-yl)pyrrolidine-2-carboxamide;
(2R,4S)-N-(1-cyclopropyl-4-methyl-pyrrolidin-3-yl)-1-[(2R)-2-(4-cyclopropyltriazol-1-yl)-3,3-dimethyl-butanoyl]-4-hydroxy-pyrrolidine-2-carboxamide;
(2R,4S)-1-[(2R)-2-(4-cyclopropyltriazol-1-yl)-3,3-dimethyl-butanoyl]-4-hydroxy-N-(2-methyl-2-azabicyclo[2.2.2]octan-6-yl)pyrrolidine-2-carboxamide;
(2R,4S)-1-[(2R)-2-(4-cyclopropyltriazol-1-yl)-3,3-dimethyl-butanoyl]-4-hydroxy-N-(2-methyl-3,3a,4,5,6,6a-hexahydro-1H-cyclopenta[c]pyrrol-4-yl)pyrrolidine-2-carboxamide;
4-[[1-[(1S)-1-[(2S,4R)-4-hydroxy-2-(methylcarbamoyl)pyrrolidine-1-carbonyl]-2,2-dimethylpropyl]triazol-4-yl]methoxy]benzoic acid;
(2S,4R)-1-[(2S)-2-(4-cyclopropyltriazol-1-yl)-3,3-dimethyl-butanoyl]-4-hydroxy-N-[3-(5-oxo-1,2-dihydropyrazol-3-yl)propyl]pyrrolidine-2-carboxamide;
(2S,4R)-1-[(2R)-2-(4-cyclopropyltriazol-1-yl)-3,3-dimethyl-butanoyl]-4-hydroxy-N-(5-methyl-6-oxabicyclo[3.2.1]octan-1-yl)pyrrolidine-2-carboxamide;
2-[[1-[(1S)-1-[(2S,4R)-4-hydroxy-2-(methylcarbamoyl)pyrrolidine-1-carbonyl]-2,2-dimethylpropyl]triazol-4-yl]methoxy]pyridine-3-carboxylic acid;
(2S,4R)-1-[(2S)-3,3-dimethyl-2-[4-[(4-nitrophenoxy)methyl]triazol-1-yl]butanoyl]-4-hydroxy-N-methyl-pyrrolidine-2-carboxamide;
(2S,4R)-1-[(2R)-2-(4-cyclopropyltriazol-1-yl)-3,3-dimethyl-butanoyl]-4-hydroxy-N-[3-(hydroxymethyl)norbornan-2-yl]pyrrolidine-2-carboxamide;
(2S,4R)-1-[(2R)-2-(4-cyclopropyltriazol-1-yl)-3,3-dimethyl-butanoyl]-4-hydroxy-N-[2-hydroxy-3-(triazol-1-yl)propyl]pyrrolidine-2-carboxamide;
(2R,4S)-1-[(2R)-2-(4-cyclopropyltriazol-1-yl)-3,3-dimethyl-butanoyl]-N-[3-(dimethylamino)-2,2-dimethyl-cyclobutyl]-4-hydroxy-pyrrolidine-2-carboxamide;
(2S,4R)-1-[(2S)-3,3-dimethyl-2-[4-[[(5-methylisoxazol-3-yl)carbamoylamino]methyl]triazol-1-yl]butanoyl]-4-hydroxy-N-methyl-pyrrolidine-2-carboxamide;
(2S,4R)-1-[(2S)-2-(4-cyclopropyltriazol-1-yl)-3,3-dimethyl-butanoyl]-4-hydroxy-N-[[(2S,4R)-4-methoxytetrahydropyran-2-yl]methyl]pyrrolidine-2-carboxamide;
(2S,4R)-1-[(2S)-3,3-dimethyl-2-[4-(3-methylsulfonylphenyl)triazol-1-yl]butanoyl]-4-hydroxy-N-methyl-pyrrolidine-2-carboxamide;
methyl 1-[[1-[(1S)-1-[(2S,4R)-4-hydroxy-2-(methylcarbamoyl)pyrrolidine-1-carbonyl]-2,2-dimethyl-propyl]triazol-4-yl]methyl]piperidine-4-carboxylate;
(2S,4R)-1-[(2S)-2-(4-cyclopropyltriazol-1-yl)-3,3-dimethyl-butanoyl]-4-hydroxy-N-([1,2,4]triazolo[4,3-a]pyridin-6-ylmethyl)pyrrolidine-2-carboxamide;
(2S,4R)-1-[(2R)-2-(4-cyclopropyltriazol-1-yl)-3,3-dimethyl-butanoyl]-4-hydroxy-N-(5,6,7,8-tetrahydroisoquinolin-5-yl)pyrrolidine-2-carboxamide;
(2S,4R)-1-[(2R)-2-(4-cyclopropyltriazol-1-yl)-3,3-dimethyl-butanoyl]-N-[(1,1-dioxo-1,2-thiazolidin-3-yl)methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(1R,2S)-2-(1,2,4-triazol-4-yl)cyclopentyl]pyrrolidine-2-carboxamide;
(2R,4S)-1-[(2R)-2-(4-cyclopropyltriazol-1-yl)-3,3-dimethyl-butanoyl]-4-hydroxy-N-(1-methyl-4,5,6,7-tetrahydrobenzotriazol-5-yl)pyrrolidine-2-carboxamide;
(2R,4S)-1-[(2R)-2-(4-cyclopropyltriazol-1-yl)-3,3-dimethyl-butanoyl]-4-hydroxy-N-(2-methyl-4,5,6,7-tetrahydroindazol-4-yl)pyrrolidine-2-carboxamide;
(2S,4R)-N-[(5-cyclopropyl-2-methyl-1,2,4-triazol-3-yl)methyl]-1-[(2S)-2-(4-cyclopropyltriazol-1-yl)-3,3-dimethyl-butanoyl]-4-hydroxy-pyrrolidine-2-carboxamide;
(2R,4S)-1-[(2R)-2-(4-cyclopropyltriazol-1-yl)-3,3-dimethyl-butanoyl]-4-hydroxy-N-(2-methyl-5,6,7,8-tetrahydro-[1,2,4]triazolo[1,5-a]pyridin-8-yl)pyrrolidine-2-carboxamide;
(2S,4R)-1-[(2R)-2-(4-cyclopropyltriazol-1-yl)-3,3-dimethyl-butanoyl]-4-hydroxy-N-(3-hydroxy-2-pyrazol-1-yl-cyclobutyl)pyrrolidine-2-carboxamide;
(2S,4R)-1-[(2S)-3,3-dimethyl-2-[4-[3-[[2-(methylamino)-2-oxo-ethyl]amino]phenyl]triazol-1-yl]butanoyl]-4-hydroxy-N-methyl-pyrrolidine-2-carboxamide;
(2R,4S)-1-[(2R)-2-(4-cyclopropyltriazol-1-yl)-3,3-dimethyl-butanoyl]-N-[2-(2-ethylimidazol-1-yl)-1-methyl-ethyl]-4-hydroxy-pyrrolidine-2-carboxamide;
(2R,4S)-1-[(2R)-2-(4-cyclopropyltriazol-1-yl)-3,3-dimethyl-butanoyl]-4-hydroxy-N-[2-methyl-3-(2-methylimidazol-1-yl)propyl]pyrrolidine-2-carboxamide;
(2S,4R)-N-[(2S,3R)-2-cyclopropyl-6-oxo-3-piperidyl]-1-[(2S)-2-(4-cyclopropyltriazol-1-yl)-3,3-dimethyl-butanoyl]-4-hydroxy-pyrrolidine-2-carboxamide;
(2S,4R)-N-[(1R,5R,6R)-3-carbamoyl-6-bicyclo[3.2.0]heptanyl]-1-[(2R)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(5-methoxy-1,3-dimethyl-pyrazol-4-yl)methyl]pyrrolidine-2-carboxamide;
(2S,4R)-N-[(2-tert-butyltetrazol-5-yl)methyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-(1,1-dimethyl-2-oxo-2-pyrrolidin-1-yl-ethyl)-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(1-isoquinolylmethyl)pyrrolidine-2-carboxamide;
ethyl 3,3,3-trifluoro-2-hydroxy-2-[1-[(1S)-1-[(2S,4R)-4-hydroxy-2-(methylcarbamoyl)pyrrolidine-1-carbonyl]-2,2-dimethyl-propyl]triazol-4-yl]propanoate;
(2S,4R)-1-[(2R)-2-(4-cyclopropyltriazol-1-yl)-3,3-dimethyl-butanoyl]-N-[(2,2-difluorospiro[2.4]heptan-1-yl)methyl]-4-hydroxy-pyrrolidine-2-carboxamide;
(2R,4S)-1-[(2R)-2-(4-cyclopropyltriazol-1-yl)-3,3-dimethyl-butanoyl]-4-hydroxy-N-(3-methylsulfinylcyclohexyl)pyrrolidine-2-carboxamide;
(2R,4S)-4-hydroxy-1-[(2R)-2-[4-[2-[3-(hydroxymethyl)-3-methoxy-1-piperidyl]ethyl]triazol-1-yl]-3,3-dimethyl-butanoyl]-N-methyl-pyrrolidine-2-carboxamide;
(2S,4R)-1-[(2R)-2-(4-cyclopropyltriazol-1-yl)-3,3-dimethyl-butanoyl]-4-hydroxy-N-(5,6,7,8-tetrahydroquinolin-8-ylmethyl)pyrrolidine-2-carboxamide;
(2S,4R)-N-[cyclobutyl(2-pyridyl)methyl]-1-[(2R)-2-(4-cyclopropyltriazol-1-yl)-3,3-dimethyl-butanoyl] -4-hydroxy-pyrrolidine-2-carboxamide;
(2S,4R)-N-[[2-(cyclopropylamino)-4-pyridyl]methyl]-1-[(2S)-2-(4-cyclopropyltriazol-1-yl)-3,3-dimethyl-butanoyl]-4-hydroxy-pyrrolidine-2-carboxamide;
(2S,4R)-N-[2-(4-cyclopropylpyrimidin-2-yl)ethyl]-1-[(2S)-2-(4-cyclopropyltriazol-1-yl)-3,3-dimethyl-butanoyl] -4-hydroxy-pyrrolidine-2-carboxamide;
(2S,4R)-1-[(2R)-2-(4-cyclopropyltriazol-1-yl)-3,3-dimethyl-butanoyl]-4-hydroxy-N-(3-pyrimidin-2-yltetrahydrofuran-3-yl)pyrrolidine-2-carboxamide;
(2R,4S)-1-[(2R)-2-(4-cyclopropyltriazol-1-yl)-3,3-dimethyl-butanoyl]-4-hydroxy-N-(4-methoxy-1,1-dioxo-thiolan-3-yl)pyrrolidine-2-carboxamide;
(2S,4R)-1-[(2S)-2-(4-cyclopropyltriazol-1-yl)-3,3-dimethyl-butanoyl]-N-[1-(3-fluoro-2-pyridyl)cyclobutyl]-4-hydroxy-pyrrolidine-2-carboxamide;
(2S,4R)-1-[(2R)-2-(4-cyclopropyltriazol-1-yl)-3,3-dimethyl-butanoyl]-4-hydroxy-N-[1-(hydroxymethyl)-3-(4-pyridyl)propyl]pyrrolidine-2-carboxamide;
(2S,4R)-1-[(2S)-2-(4-cyclopropyltriazol-1-yl)-3,3-dimethyl-butanoyl]-N-[2-(6,7-dihydro-4H-pyrazolo[1,5-a]pyrazin-5-yl)ethyl]-4-hydroxy-pyrrolidine-2-carboxamide;
(2S,4R)-N-[(5-cyclobutyl-4-methyl-1,24-triazol-3-yl)methyl]-1-[(2S)-2-(4-cyclopropyltriazol-1-yl)-3,3-dimethyl-butanoyl]-4-hydroxy-pyrrolidine-2-carboxamide;
(2S,4R)-N-[(5-cyclopentylisoxazol-3-yl)methyl]-1-[(2S)-2-(4-cyclopropyltriazol-1-yl)-3,3-dimethyl-butanoyl] -4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(2-isopropoxypyrimidin-4-yl)methyl]pyrrolidine-2-carboxamide;
(2S,4R)-N-[2-(5-tert-butylisoxazol-3-yl)ethyl]-1-[(2S)-2-(4-cyclopropyltriazol-1-yl)-3,3-dimethyl-butanoyl] -4-hydroxy-pyrrolidine-2-carboxamide;
(2R,4S)-N-[1-(3-tert-butyl-1,2,4-oxadiazol-5-yl)ethyl]-1-[(2R)-2-(4-cyclopropyltriazol-1-yl)-3,3-dimethyl-butanoyl]-4-hydroxy-pyrrolidine-2-carboxamide;
(2S,4R)-N-(4-chloro-2,3-dihydrobenzofuran-3-yl)-1-[(2R)-2-(4-cyclopropyltriazol-1-yl)-3,3-dimethyl-butanoyl] -4-hydroxy-pyrrolidine-2-carboxamide;
(2R,4S)-1-[(2R)-2-(4-cyclopropyltriazol-l-yl)-3,3-dimethyl-butanoyl]-4-hydroxy-N-[3-(2-oxopyrrolidin-1-yl)pentyl]pyrrolidine-2-carboxamide;
(2R,4S)-1-[(2R)-2-(4-cyclopropyltriazol-1-yl)-3,3-dimethyl-butanoyl]-N-(2,2-diethyl-3-methoxy-4-methyl-cyclobutyl)-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(4-phenyl-1,2,4-triazol-3-yl)methyl]pyrrolidine-2-carboxamide;
(2S,4R)-1-[(2S)-2-(4-cyclopropyltriazol-1-yl)-3,3-dimethyl-butanoyl]-4-hydroxy-N-[(4-hydroxy-2-quinolyl)methyl]pyrrolidine-2-carboxamide;
(2S,4R)-1-[(2S)-2-(4-cyclopropyltriazol-1-yl)-3,3-dimethyl-butanoyl]-N-[(3,5-dimethoxyisothiazol-4-yl)methyl]-4-hydroxy-pyrrolidine-2-carboxamide;
(2S,4R)-N-(1-cyclopropyl-1-methyl-2-phenyl-ethyl)-1-[(2R)-2-(4-cyclopropyltriazol-1-yl)-3,3-dimethyl-butanoyl]-4-hydroxy-pyrrolidine-2-carboxamide;
(2R,4S)-1-[(2R)-2-(4-cyclopropyltriazol-1-yl)-3,3-dimethyl-butanoyl]-4-hydroxy-N-[(1-methyl-3,4-dihydro-2H-quinolin-3-yl)methyl]pyrrolidine-2-carboxamide;
(2R,4S)-1-[(2R)-2-(4-cyclopropyltriazol-1-yl)-3,3-dimethyl-butanoyl]-N-[(2,3-dimethyl-1,1-dioxo-1,2-thiazolidin-3-yl)methyl]-4-hydroxy-pyrrolidine-2-carboxamide;
5-[[[(2S,4R)-1-[(2S)-2-(4-cyclopropyltriazol-1-yl)-3,3-dimethyl-butanoyl]-4-hydroxy-pyrrolidine-2-carbonyl]amino]methyl]-N,N-dimethyl-pyridine-2-carboxamide;
(2S,4R)-1-[(2S)-2-(4-cyclopropyltriazol-1-yl)-3,3-dimethyl-butanoyl]-4-hydroxy-N-[3-oxo-3-(3-pyridylmethylamino)propyl]pyrrolidine-2-carboxamide;
(2S,4R)-N-(4-chloro-1,la,6,6a-tetrahydrocyclopropa[a]inden-1-yl)-1-[(2R)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[1-(1-methylimidazol-2-yl)-4-piperidyl]pyrrolidine-2-carboxamide;
(2R,4S)-1-[(2R)-2-(4-cyclopropyltriazol-1-yl)-3,3-dimethyl-butanoyl]-4-hydroxy-N-(2-isopropy1-5,6,7,8-tetrahydro-[1,2,4]triazolo[1,5-a]pyridin-6-yl)pyrrolidine-2-carboxamide;
(2S,4R)-1-[(2S)-2-(4-cyclopropyltriazol-1-yl)-3,3-dimethyl-butanoyl]-N-[(1R,2S)-2-(4-fluoro-3-methoxy-phenyl)cyclopropyl]-4-hydroxy-pyrrolidine-2-carboxamide;
(2R,4S)-N-[1-(3-cyclopropyl-1,2,4-oxadiazol-5-yl)-2-methyl-propyl]-1-[(2R)-2-(4-cyclopropyltriazol-1-yl)-3,3-dimethyl-butanoyl]-4-hydroxy-pyrrolidine-2-carboxamide;
(2R,4S)-N-[1-(1-tert-butyl-3-methyl-pyrazol-4-yl)ethyl]-1-[(2R)-2-(4-cyclopropyltriazol-1-yl)-3,3-dimethyl-butanoyl]-4-hydroxy-pyrrolidine-2-carboxamide;
(2S,4R)-1-[(2R)-2-(4-cyclopropyltriazol-1-yl)-3,3-dimethyl-butanoyl]-4-hydroxy-N-[2-(1-tetrahydrofuran-3-yltriazol-4-yl)ethyl]pyrrolidine-2-carboxamide;
(2S,4R)-1-[(2R)-2-(4-cyclopropyltriazol-1-yl)-3,3-dimethyl-butanoyl]-4-hydroxy-N-[2-[5-(trifluoromethyl)tetrahydrofuran-2-yl]ethyl]pyrrolidine-2-carboxamide;
(2S,4R)-N-[cyclopropyl-(2,4-difluorophenyl)methyl]-1-[(2R)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[(1R)-5,7-difluorotetralin-1-yl]-4-hydroxy-pyrrolidine-2-carboxamide;
(2R,4S)-N-(3-tert-butoxyspiro[3.3]heptan-1-yl)-1-[(2R)-2-(4-cyclopropyltriazol-1-yl)-3,3-dimethyl-butanoyl] -4-hydroxy-pyrrolidine-2-carboxamide;
(2S,4R)-1-[(2S)-2-(4-cyclopropyltriazol-l-yl)-3,3-dimethyl-butanoyl]-N-[2-[2-(difluoromethoxy)phenyl]ethyl]-4-hydroxy-pyrrolidine-2-carboxamide;
(2S,4R)-1-[(2S)-2-(4-cyclopropyltriazol-1-yl)-3,3-dimethyl-butanoyl]-N-[3,3-difluoro-1-(imidazol-1-ylmethyl)cyclobutyl]-4-hydroxy-pyrrolidine-2-carboxamide;
(2R,4S)-1-[(2R)-2-(4-cyclopropyltriazol-1-yl)-3,3-dimethyl-butanoyl]-4-hydroxy-N-[1-(1-phenylimidazol-2-yl)ethyl]pyrrolidine-2-carboxamide;
(2R,4S)-1-[(2R)-2-(4-cyclopropyltriazol-l-yl)-3,3-dimethyl-butanoyl]-N-[1-(1,5-dimethylindol-3-yl)ethyl]-4-hydroxy-pyrrolidine-2-carboxamide;
(2R,4S)-1-[(2R)-2-(4-cyclopropyltriazol-1-yl)-3,3-dimethyl-butanoyl]-4-hydroxy-N-[1-(1-methyl-2-oxo-indolin-5-yl)ethyl]pyrrolidine-2-carboxamide;
(2R,4S)-1-[(2R)-2-(4-cyclopropyltriazol-1-yl)-3,3-dimethyl-butanoyl]-4-hydroxy-N-[2-methyl-1-([1,2,4]triazolo[4,3-a]pyridin-3-yl)propyl]pyrrolidine-2-carboxamide;
(2S,4R)-1-[(2S)-2-(4-cyclopropyltriazol-1-yl)-3,3-dimethyl-butanoyl]-N-[(1R,2S)-2-(2,3-dihydro-1,4-benzodioxin-5-yl)cyclopropyl]-4-hydroxy-pyrrolidine-2-carboxamide;
(2S,4R)-1-[(2R)-2-(4-cyclopropyltriazol-1-yl)-3,3-dimethyl-butanoyl]-N-(1,1-dioxo-7-oxa-1lambda6-thiaspiro[3.5]nonan-3-yl)-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(2S,3R)-2-(p-tolyl)tetrahydropyran-3-yl]pyrrolidine-2-carboxamide;
(2R,4S)-1-[(2R)-2-(4-cyclopropyltriazol-1-yl)-3,3-dimethyl-butanoyl]-4-hydroxy-N-(5-isopropoxyindan-1-yl)pyrrolidine-2-carboxamide;
(2S,4R)-1-[(2R)-2-(4-cyclopropyltriazol-l-yl)-3,3-dimethyl-butanoyl]-4-hydroxy-N-[[1-(2-pyridylmethyl)pyrrolidin-3-yl]methyl]pyrrolidine-2-carboxamide;
(2S,4R)-N-[3-(6-aminopurin-9-yl)propyl]-1-[(2S)-2-(4-cyclopropyltriazol-1-yl)-3,3-dimethyl-butanoyl] -4-hydroxy-pyrrolidine-2-carboxamide;
(2S,4R)-1-[(2R)-2-(4-cyclopropyltriazol-1-yl)-3,3-dimethyl-butanoyl]-4-hydroxy-N-[2-(methanesulfonamidomethyl)cyclopentyl]pyrrolidine-2-carboxamide;
(2S,4R)-1-[(2S)-2-(4-cyclopropyltriazol-1-yl)-3,3-dimethyl-butanoyl]-N-[2-(4-fluoro-1-methyl-benzimidazol-2-yl)ethyl]-4-hydroxy-pyrrolidine-2-carboxamide;
(2S,4R)-1-[(2S)-2-(4-cyclopropyltriazol-1-yl)-3,3-dimethyl-butanoyl]-N-[1-[(4-fluorophenyl)carbamoyl]cyclopropyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(2S,3R)-2-(1-methylpyrazol-4-yl)-6-oxo-3-piperidyl]pyrrolidine-2-carboxamide;
(2S,4R)-1-[(2S)-2-(4-cyclopropyltriazol-1-yl)-3,3-dimethyl-butanoyl]-4-hydroxy-N-[(3R,4R)-3-(pyrimidin-4-ylamino)tetrahydropyran-4-yl]pyrrolidine-2-carboxamide;
(2R,4S)-N-[3-amino-1-(6-methoxy-3-pyridyl)-3-oxo-propyl]-1-[(2R)-2-(4-cyclopropyltriazol-1-yl)-3,3-dimethyl-butanoyl]-4-hydroxy-pyrrolidine-2-carboxamide;
(2S,4R)-1-[(2R)-2-(4-cyclopropyltriazol-1-yl)-3,3-dimethyl-butanoyl]-N-[(4-fluorophenyl)-tetrahydrofuran-2-yl-methyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(1R,3R)-3-morpholinospiro[3.3]heptan-1-yl]pyrrolidine-2-carboxamide;
(2S,4R)-1-[(2R)-2-(4-cyclopropyltriazol-1-yl)-3,3-dimethyl-butanoyl]-4-hydroxy-N-[1-(2-hydroxycyclohexyl)-4-piperidyl]pyrrolidine-2-carboxamide;
(2S,4R)-N-[(1S)-1-(cyclohexylcarbamoyl)-2-methyl-propyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-[(2-morpholinothiazol-5-yl)methyl]pyrrolidine-2-carboxamide;
(2S,4R)-1-[(2S)-2-(4-cyclopropyltriazol-1-yl)-3,3-dimethyl-butanoyl]-4-hydroxy-N-[[2-(2-pyridyl)spiro[3.3]heptan-2-yl]methyl]pyrrolidine-2-carboxamide;
(2S,4R)-1-[(2S)-2-(4-cyclopropyltriazol-1-yl)-3,3-dimethyl-butanoyl]-4-hydroxy-N-[2-(2-isopropyl-1,3-benzoxazol-5-yl)ethyl]pyrrolidine-2-carboxamide;
(2S,4R)-1-[(2R)-2-(4-cyclopropyltriazol-1-yl)-3,3-dimethyl-butanoyl]-4-hydroxy-N-spiro[chromane-2,3'-tetrahydrofuran]-4-yl-pyrrolidine-2-carboxamide;
(2R,4S)-1-[(2R)-2-(4-cyclopropyltriazol-1-yl)-3,3-dimethyl-butanoyl]-4-hydroxy-N-[1-[1-(4-pyridyl)ethyl]-4-piperidyl]pyrrolidine-2-carboxamide;
(2S,4R)-1-[(2S)-2-(4-cyclopropyltriazol-1-yl)-3,3-dimethyl-butanoyl]-4-hydroxy-N-[(4-methyl-2-morpholino-phenyl)methyl]pyrrolidine-2-carboxamide;
(2S,4R)-1-[(2R)-2-(4-cyclopropyltriazol-1-yl)-3,3-dimethyl-butanoyl]-4-hydroxy-N-[[2-(methanesulfonamido)cyclohexyl]methyl]pyrrolidine-2-carboxamide;
(2R,4S)-1-[(2R)-2-(4-cyclopropyltriazol-1-yl)-3,3-dimethyl-butanoyl]-4-hydroxy-N-[[1-(methoxymethyl)isochroman-1-yl]methyl]pyrrolidine-2-carboxamide;
(2S,4R)-1-[(2S)-2-(4-cyclopropyltriazol-1-yl)-3,3-dimethyl-butanoyl]-N-[(1S)-1-[3-[2-(dimethylamino)ethoxy]phenyl]ethyl]-4-hydroxy-pyrrolidine-2-carboxamide;
(2S,4R)-1-[(2S)-3,3-dimethyl-2-[4-[3-[(2-morpholino-2-oxo-ethyl)amino]phenyl]triazol-1-yl]butanoyl]-4-hydroxy-N-methyl-pyrrolidine-2-carboxamide;
(2S,4R)-1-[(2S)-2-(4-cyclopropyltriazol-1-yl)-3,3-dimethyl-butanoyl]-N-[[(2R,3S)-2-(1-ethylpyrazol-4-yl)tetrahydropyran-3-yl]methyl]-4-hydroxy-pyrrolidine-2-carboxamide;
(2R,4S)-1-[(2R)-2-(4-cyclopropyltriazol-1-yl)-3,3-dimethyl-butanoyl]-N-[2-(2,5-dimethoxyphenyl)-2-hydroxy-1-methyl-ethyl]-4-hydroxy-pyrrolidine-2-carboxamide;
tert-butyl 3-[1-[(1S)-1-[(2S,4R)-4-hydroxy-2-(methylcarbamoyl)pyrrolidine-1-carbonyl]-2,2-dimethyl-propyl]triazol-4-yl]-6,7-dihydro-4H-pyrazolo[1,5-a]pyrazine-5-carboxylate;
(2R,4S)-1-[(2R)-2-(4-cyclopropyltriazol-1-yl)-3,3-dimethyl-butanoyl]-4-hydroxy-N-[1-methyl-2-(p-tolylsulfonyl)ethyl]pyrrolidine-2-carboxamide;
(2S,4R)-1-[(2S)-2-(4-cyclopropyltriazol-1-yl)-3,3-dimethyl-butanoyl]-4-hydroxy-N-[[3-[methyl(methylsulfonyl)amino]phenyl]methyl]pyrrolidine-2-carboxamide;
(2R,4S)-N-[1-(1-benzylimidazol-2-yl)propyl]-1-[(2R)-2-(4-cyclopropyltriazol-1-yl)-3,3-dimethyl-butanoyl] -4-hydroxy-pyrrolidine-2-carboxamide;
(2R,4S)-1-[(2R)-2-(4-cyclopropyltriazol-1-yl)-3,3-dimethyl-butanoyl]-N-[1-(2,4-dimethylphenyl)-2-imidazol-1-yl-ethyl]-4-hydroxy-pyrrolidine-2-carboxamide;
(2S,4R)-1-[(2R)-2-(4-cyclopropyltriazol-1-yl)-3,3-dimethyl-butanoyl]-4-hydroxy-N-(2-pyrrolidin-1-yltetralin-1-yl)pyrrolidine-2-carboxamide;
(2S,4R)-1-[(2S)-2-(4-cyclopropyltriazol-1-yl)-3,3-dimethyl-butanoyl]-N-[[6-(difluoromethoxy)-1,3-benzodioxol-5-yl]methyl]-4-hydroxy-pyrrolidine-2-carboxamide;
(2S,4R)-N-(7-benzyloxy-6-bicyclo[32.0]heptanyl)-1-[(2R)-2-(4-cyclopropyltriazol-1-yl)-3,3-dimethyl-butanoyl] -4-hydroxy-pyrrolidine-2-carboxamide;
(2S,4R)-N-[2-(2-cyclopentylsulfanylpyrimidin-5-yl)ethyl]-1-[(2S)-2-(4-cyclopropyltriazol-1-yl)-3,3-dimethyl-butanoyl]-4-hydroxy-pyrrolidine-2-carboxamide;
(2S,4R)-N-[2-(5-chloro-1-oxo-3,4-dihydroisoquinolin-2-yl)ethyl]-1-[(2S)-2-(4-cyclopropyltriazol-1-yl)-3,3-dimethyl-butanoyl]-4-hydroxy-pyrrolidine-2-carboxamide;
(2S,4R)-N-[2-(2-chloro-6-pyrrolidin-1-yl-phenyl)ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2R)-2-(4-cyclopropyltriazol-1-yl)-3,3-dimethyl-butanoyl]-4-hydroxy-N-(8-phenylchroman-4-yl)pyrrolidine-2-carboxamide;
(2S,4R)-N-[2-[4-(cyclopentanecarbonyl)piperazin-1-yl]ethyl]-1-[(2S)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-4-hydroxy-N-(2-indolin-1-ylsulfonylethyl)pyrrolidine-2-carboxamide;
(2R,4S)-N-[1-[(2-aminothiazol-5-yl)methyl]-3-methyl-4-piperidyl]-1-[(2R)-2-(4-cyclopropyltriazol-1-yl)-3,3-dimethyl-butanoyl]-4-hydroxy-pyrrolidine-2-carboxamide;
(2S,4R)-1-[(2S)-2-(4-cyclopropyltriazol-1-yl)-3,3-dimethyl-butanoyl]-N-[2-[(3-fluoro-4-methyl-phenyl)sulfamoyl]ethyl]-4-hydroxy-pyrrolidine-2-carboxamide;
(2S,4R)-N-[(2R,3S)-1-cyclopropyl-2-(3-methoxyphenyl)pyrrolidin-3-yl]-1-[(2S)-2-(4-cyclopropyltriazol-1-yl)-3,3-dimethyl-butanoyl]-4-hydroxy-pyrrolidine-2-carboxamide;
(2R,4S)-1-[(2R)-2-(4-cyclopropyltriazol-1-yl)-3,3-dimethyl-butanoyl]-N-[1-(3,4-dihydro-1H-isoquinolin-2-ylmethyl)-2,2-dimethyl-propyl]-4-hydroxy-pyrrolidine-2-carboxamide;
(2S,4R)-N-[3-(4-chlorophenyl)sulfonylpropyl]-1-[(2S)-2-(4-cyclopropyltriazol-1-yl)-3,3-dimethyl-butanoyl] -4-hydroxy-pyrrolidine-2-carboxamide;
(2S,4R)-N-[[1-[(3-chlorophenyl)methyl]-2-piperidyl]methyl]-1-[(2R)-2-(4-cyclopropyltriazol-1-yl)-3,3-dimethyl-butanoyl]-4-hydroxy-pyrrolidine-2-carboxamide;
(2R,4S)-N-[1-[4-(4-chloropyrazol-1-yl)-3-fluoro-phenyl]ethyl]-1-[(2R)-2-(4-cyclopropyltriazol-1-yl)-3,3-dimethyl-butanoyl]-4-hydroxy-pyrrolidine-2-carboxamide;
(2S,4R)-N-(3-bromo-6,7,8,9-tetrahydro-5H-benzo[7]annulen-6-yl)-1-[(2R)-2-(4-cyclopropyltriazol-1-yl)-3,3-dimethyl-butanoyl]-4-hydroxy-pyrrolidine-2-carboxamide;
(2R,4S)-N-[(4-chlorophenyl)-(4-methylmorpholin-2-yl)methyl]-1-[(2R)-2-(4-cyclopropyltriazol-1-yl)-3,3-dimethyl-butanoyl]-4-hydroxy-pyrrolidine-2-carboxamide;
(2S,4R)-1-[(2R)-2-( 4-cyclopropyltriazol-1-yl)-3,3-dimethyl-butanoyl]-N-[(2,5-dichlorophenyl)-tetrahydrofuran-3-yl-methyl]-4-hydroxy-pyrrolidine-2-carboxamide;
(2S,4R)-N-[[4-tert-butoxy-2-(trifluoromethyl)phenyl]methyl]-1-[(2S)-2-(4-cyclopropyltriazol-1-yl)-3,3-dimethyl-butanoyl]-4-hydroxy-pyrrolidine-2-carboxamide;
(2R,4S)-1-[(2R)-2-(4-cyclopropyltriazol-1-yl)-3,3-dimethyl-butanoyl]-4-hydroxy-N-[1-methyl-3-[4-(m-tolyl)piperazin-1-yl]propyl]pyrrolidine-2-carboxamide;
(2R,4S)-1-[(2R)-2-(4-cyclopropyltriazol-1-yl)-3,3-dimethyl-butanoyl]-4-hydroxy-N-[2-[methyl-(1-methyl-4-piperidyl)amino]-1-phenyl-ethyl]pyrrolidine-2-carboxamide;
(2S,4R)-N-[[1-(4-bromophenyl)pyrazol-4-yl]methyl]-1-[(2S)-2-(4-cyclopropyltriazol-1-yl)-3,3-dimethyl-butanoyl]-4-hydroxy-pyrrolidine-2-carboxamide;
(2R,4S)-N-[1-[8-chloro-6-(trifluoromethyl)-[1,2,4]triazolo[4,3-a]pyridin-3-yl]ethyl]-1-[(2R)-2-(4-cyclopropyltriazol-1-yl)-3,3-dimethyl-butanoyl]-4-hydroxy-pyrrolidine-2-carboxamide;
(2S,4R)-1-[(2R)-2-(4-cyclopropyltriazol-1-yl)-3,3-dimethyl-butanoyl]-N-[1-(3,4-dichlorophenyl)-2-(2-pyridyl)ethyl]-4-hydroxy-pyrrolidine-2-carboxamide;
(2S,4R)-N-[(2R,3S)-2-(4-chloro-3-fluoro-phenyl)-1-isopropyl-5-oxo-pyrrolidin-3-yl]-1-[(2S)-2-(4-cyclopropyltriazol-1-yl)-3,3-dimethyl-butanoyl]-4-hydroxy-pyrrolidine-2-carboxamide;
(2S,4R)-N-[2-[(3-bromo-1-methyl-pyrazolo[3,4-d]pyrimidin-4-yl)amino]ethyl]-1-[(2S)-2-(4-cyclopropyltriazol-1-yl)-3,3-dimethyl-butanoyl]-4-hydroxy-pyrrolidine-2-carboxamide;
(2R,4S)-1-[(2R)-2-(4-cyclopropyltriazol-1-yl)-3,3-dimethyl-butanoyl]-4-hydroxy-N-[1-[4-[(2-methyltetrazol-5-yl)sulfamoyl]phenyl]ethyl]pyrrolidine-2-carboxamide;
methyl 1-[[1-[(1R)-1-[(2R,4S)-4-hydroxy-2-(methylcarbamoyl)pyrrolidine-1-carbonyl]-2,2-dimethyl-propyl]triazol-4-yl]methyl]piperidine-2-carboxylate;
(2R,4S)-1-[(2S)-2-(4-cyclopropyltriazol-1-yl)-3,3-dimethyl-butanoyl]-4-hydroxy-N-[3-(3-pyridyl)allyl]pyrrolidine-2-carboxamide;
(2R,4S)-1-[(2R)-2-(4-cyclopropyltriazol-1-yl)-3,3-dimethyl-butanoyl]-4-hydroxy-N-(2-methyl-5-tetrahydrofuran-2-yl-pyrazol-3-yl)pyrrolidine-2-carboxamide;
(2S,4R)-1-[(2S)-2-(4-cyclopropyltriazol-1-yl)-3,3-dimethyl-butanoyl]-4-hydroxy-N-[(7-hydroxypyrazolo[1,5-a]pyrimidin-5-yl)methyl]pyrrolidine-2-carboxamide;
(2S,4R)-1-[(2S)-2-(4-cyclopropyltriazol-1-yl)-3,3-dimethyl-butanoyl]-4-hydroxy-N-[5-(methanesulfonamido)-2-methoxy-phenyl]pyrrolidine-2-carboxamide;
(2S,4R)-1-[(2S)-2-(4-cyclopropyltriazol-1-yl)-3,3-dimethyl-butanoyl]-4-hydroxy-N-(1-methyl-4-phenyl-4-piperidyl)pyrrolidine-2-carboxamide; and
(2S,4R)-1-[(2S)-2-(4-cyclopropyltriazol-1-yl)-3,3-dimethyl-butanoyl]-4-hydroxy-N-[1-[[3-(trifluoromethyl)phenyl]methyl]-4-piperidyl]pyrrolidine-2-carboxamide,
or a stereoisomer or tautomer thereof, or a pharmaceutically acceptable salt of any of the foregoing.

In some embodiments, provided herein is a compound of Table 2. In some embodiments, provided herein is a stereoisomer or tautomer of a compound of Table 2. In some embodiments, provided herein is a pharmaceutically acceptable salt of a compound of Table 2, or a stereoisomer or tautomer thereof.

**Table 2**

| **#** | **Structure** | **Name** |
|---|---|---|
| 106 | 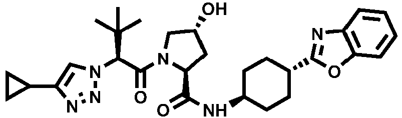 | (2S,4R)-N-[4-(1,3-benzoxazol-2-yl)cyclohexyl]-1-[(2S)-2-(4-cyclopropyltriazol-1-yl)-3,3-dimethyl-butanoyl]-4-hydroxy-pyrrolidine-2-carboxamide |
| 140 | 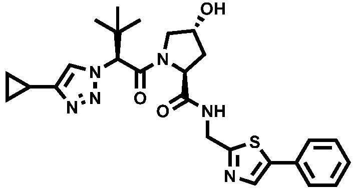 | (2S,4R)-1-[(2S)-2-(4-cyclopropyltriazol-1-yl)-3,3-dimethyl-butanoyl]-4-hydroxy-N-[(5-phenylthiazol-2-yl)methyl]pyrrolidine-2-carboxamide |
| 169 | 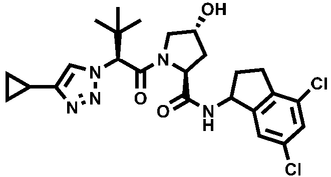 | (2S,4R)-1-[(2S)-2-(4-cyclopropyltriazol-1-yl)-3,3-dimethyl-butanoyl]-N-(4,6-dichloroindan-1-yl)-4-hydroxy-pyrrolidine-2-carboxamide |
| 174 | 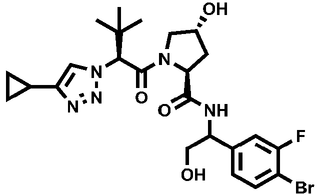 | (2S,4R)-N-[1-(4-bromo-3-fluorophenyl)-2-hydroxy-ethyl]-1-[(2S)-2-(4-cyclopropyltriazol-1-yl)-3,3-dimethyl-butanoyl]-4-hydroxy-pyrrolidine-2-carboxamide |
| 266 | 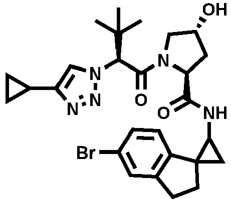 | (2S,4R)-N-(5'-bromospiro[cyclopropane-2,1'-indane]-1-yl)-1-[(25)-2-(4-cyclopropyltriazol-1-yl)-3,3-dimethyl-butanoyl]-4-hydroxy-pyrrolidine-2-carboxamide |
| 288 | 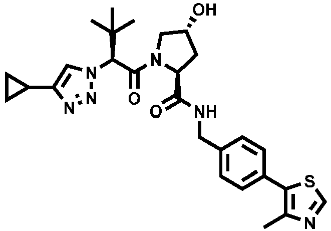 | (2S,4R)-1-[(2S)-2-(4-cyclopropyltriazol-1-yl)-3,3-dimethyl-butanoyl]-4-hydroxy-N-[[4-(4-methylthiazol-5-yl)phenyl]methyl]pyrrolidine-2-carboxamide |
| 343 | 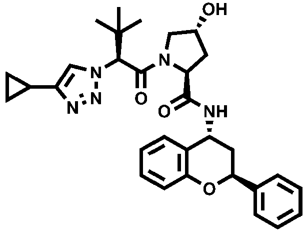 | (25,4R)-1-[(25)-2-(4-cyclopropyltriazol-1-yl)-3,3-dimethyl-butanoyl]-4-hydroxy-N-[(2S,4R)-2-phenylchroman-4-yl]pyrrol idine-2-carboxamide |
| 405 | 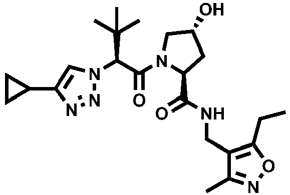 | (2S,4R)-1-[(2S)-2-(4-cyclopropyltriazol-1-yl)-3,3-dimethyl-butanoyl]-N-[(5-ethyl-3-methyl-isoxazol-4-yl)methyl]-4-hydroxy-pyrrolidine-2-carboxamide |
| 420 | 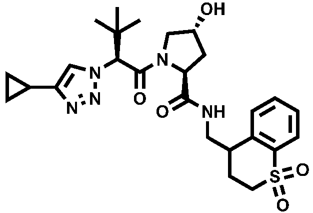 | (2S,4R)-1-[(2S)-2-(4-cyclopropyltriazol-1-yl)-3,3-dimethyl-butanoyl]-N-[(1,1-dioxo-3,4-dihydro-2H-thiochromen-4-yl)methyl]-4-hydroxy-pyrrolidine-2-carboxamide |
| 445 | 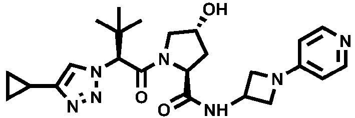 | (2S,4R)-1-[(2S)-2-(4-cyclopropyltriazol-1-yl)-3,3-dimethyl-butanoyl]-4-hydroxy-N-[1-(4-pyridyl)azetidin-3-yl]pyrrolidine-2-carboxamide |
| 449 | 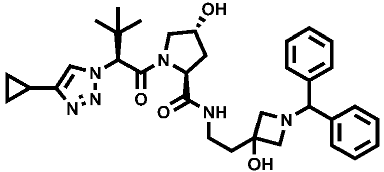 | (2S,4R)-N-[2-(1-benzhydryl-3-hydroxy-azetidin-3-yl)ethyl]-1-[(2S)-2-(4-cyclopropyltriazol-1-yl)-3,3-dimethyl-butanoyl]-4-hydroxy-pyrrolidine-2-carboxamide |
| 462 | 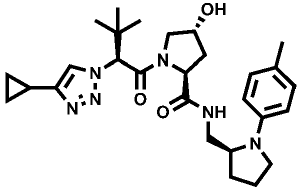 | (2S,4R)-1-[(2S)-2-(4-cyclopropyltriazol-1-yl)-3,3-dimethyl-butanoyl]-4-hydroxy-N-[[(2S)-1-(p-tolyl)pyrrolidin-2-yl]methyl]pyrrolidine-2-carboxamide |
| 463 | 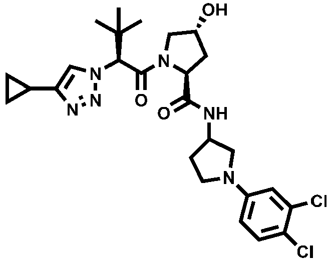 | (2S,4R)-1-[(2S)-2-(4-cyclopropyltriazol-1-yl)-3,3-dimethyl-butanoyl]-N-[1-(3,4-dichlorophenyl)pyrrolidin-3-yl]-4-hydroxy-pyrrolidine-2-carboxamide |
| 469 | 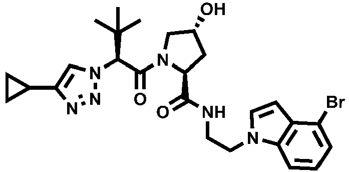 | (2S,4R)-N-[2-(4-bromoindol-1-yl)ethyl]-1-[(2S)-2-(4-cyclopropyltriazol-1-yl)-3,3-dimethyl-butanoyl]-4-hydroxy-pyrrolidine-2-carboxamide |
| 480 | 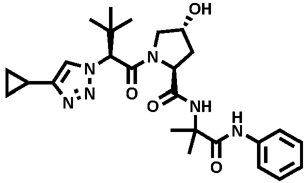 | (2S,4R)-N-(2-anilino-1,1-dimethyl-2-oxo-ethyl)-1-[(2S)-2-(4-cyclopropyltriazol-1-yl)-3,3-dimethyl-butanoyl]-4-hydroxy-pyrrolidine-2-carboxamide |
| 493 | 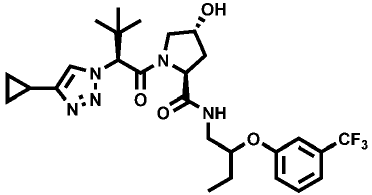 | (2S,4R)-1-[(2S)-2-(4-cyclopropyltriazol-1-yl)-3,3-dimethyl-butanoyl]-4-hydroxy-N-[2-[3-(trifluoromethyl)phenoxy]butyl] pyrrolidine-2-carboxamide |
| 554 | 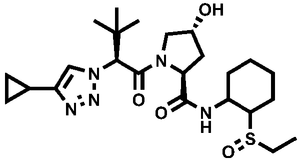 | (2S,4R)-1-[(2S)-2-(4-cyclopropyltriazol-1-yl)-3,3-dimethyl-butanoyl]-N-(2-ethylsulfinylcyclohexyl)-4-hydroxy-pyrrolidine-2-carboxamide |
| 556 | 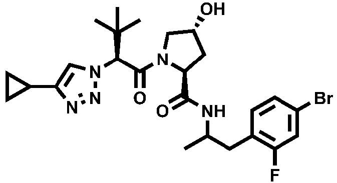 | (2S,4R)-N-[2-(4-bromo-2-fluorophenyl)-1-methyl-ethyl]-1-[(2S)-2-(4-cyclopropyltriazol-1-yl)-3,3-dimethyl-butanoyl]-4-hydroxy-pyrrolidine-2-carboxamide |
| 561 | 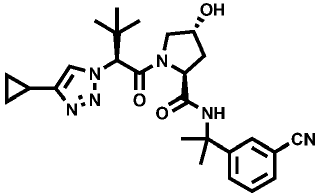 | (2S,4R)-N-[1-(3-cyanophenyl)-1-methyl-ethyl]-1-[(2S)-2-(4-cyclopropyltriazol-1-yl)-3,3-dimethyl-butanoyl]-4-hydroxy-pyrrolidine-2-carboxamide |
| 617 | 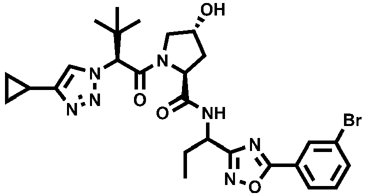 | (2S,4R)-N-[1-[5-(3-bromophenyl)-1,2,4-oxadiazol-3-yl]propyl]-1-[(2S)-2-(4-cyclopropyltriazol-1-yl)-3,3-dimethyl-butanoyl]-4-hydroxy-pyrrolidine-2-carboxamide |
| 729 | 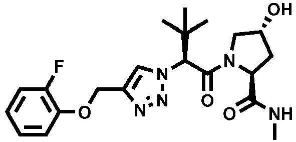 | (2S,4R)-1-[(2S)-2-[4-[(2-fluorophenoxy)methyl]triazol-1-yl]-3,3-dimethyl-butanoyl]-4-hydroxy-N-methyl-pyrrolidine-2-carboxamide |
| 792 | 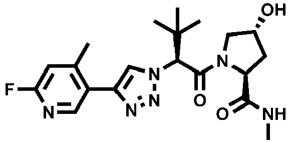 | (2S,4R)-1-[(2S)-2-[4-(6-fluoro-4-methyl-3-pyridyl)triazol-1-yl]-3,3-dimethyl-butanoyl]-4-hydroxy-N-methyl-pyrrolidine-2-carboxamide |
| 829 | 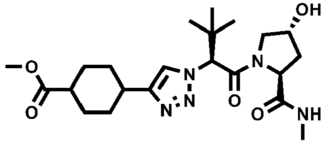 | Methyl 4-[1-[(1S)-1-[(2R,4S)-4-hydroxy-2-(methylcarbamoyl)pyrrolidine-1-carbonyl]-2,2-dimethylpropyl]triazol-4-yl]cyclohexanecarboxylate |
| 914 | 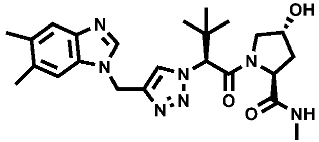 | (2S,4R)-1-[(2S)-2-[4-[(5,6-dimethylbenzimidazol-1-yl)methyl]triazol-1-yl]-3,3-dimethyl-butanoyl]-4-hydroxy-N-methyl-pyrrolidine-2-carboxamide |
| 1059 | 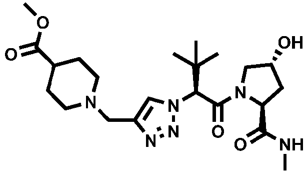 | methyl 1-[[1-[(1S)-1-[(2S,4R)-4-hydroxy-2-(methylcarbamoyl)pyrrolidine-1-carbonyl]-2,2-dimethyl-propyl]triazol-4-yl]methyl]piperidine-4-carboxylate |
| 1129 | 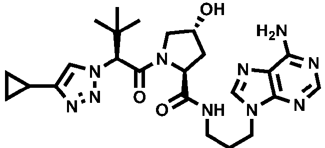 | (2S,4R)-N-[3-(6-aminopurin-9-yl)propyl]-1-[(2S)-2-(4-cyclopropyltriazol-1-yl)-3,3-dimethyl-butanoyl]-4-hydroxy-pyrrolidine-2-carboxamide |

In some embodiments, provided herein is a compound of Table 2A. In some embodiments, provided herein is a stereoisomer or tautomer of a compound of Table 2A. In some embodiments, provided herein is a pharmaceutically acceptable salt of a compound of Table 2A, or a stereoisomer or tautomer thereof.

**Table 2A**

| **#** | **Structure** | **Name** |
|---|---|---|
| 110 | 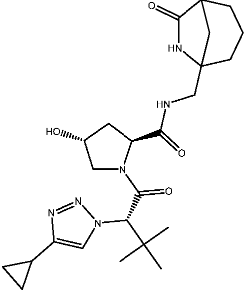 | (2S,4R)-1-[(2S)-2-(4-cyclopropyltriazol-1-yl)-3,3-dimethyl-butanoyl]-4-hydroxy-N-[(7-oxo-6-azabicyclo[3.2.1]octan-5-yl)methyl]pyrrolidine-2-carboxamide |
| 236 | 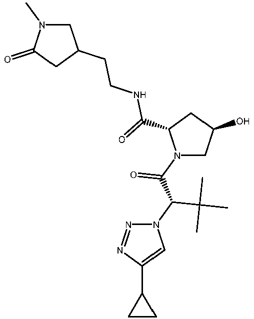 | (2S,4R)-1-[(2S)-2-(4-cyclopropyltriazol-1-yl)-3,3-dimethyl-butanoyl]-4-hydroxy-N-[2-(1-methyl-5-oxo-pyrrolidin-3-yl)ethyl]pyrrolidine-2-carboxamide |
| 842 | 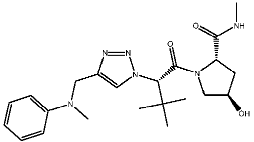 | (2S,4R)-1-[(2S)-3,3-dimethyl-2-[4-[(N-methylanilino)methyl]triazol-1-yl]butanoyl]-4-hydroxy-N-methyl-pyrrolidine-2-carboxamide |
| 974 | 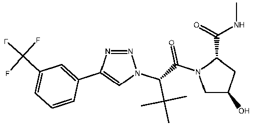 | (2S,4R)-1-[(2S)-3,3-dimethyl-2-[4-[3-(trifluoromethyl)phenyl]triazol-1-yl]butanoyl]-4-hydroxy-N-methyl-pyrrolidine-2-carboxamide |
| 1131 | 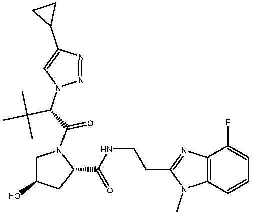 | (2S,4R)-1-[(2S)-2-(4-cyclopropyltriazol-1-yl)-3,3-dimethylbutanoyl]-N-[2-(4-fluoro-1-methyl-benzimidazol-2-yl)ethyl]-4-hydroxy-pyrrolidine-2-carboxamide |

A compound as described herein can exist in solid or liquid form. In the solid state, the compound may exist in crystalline or noncrystalline form, or as a mixture thereof. The skilled artisan will appreciate that pharmaceutically acceptable solvates may be formed for crystalline or non-crystalline compounds. In crystalline solvates, solvent molecules are incorporated into the crystalline lattice during crystallization. Solvates may involve non-aqueous solvents such as, but not limited to, ethanol, isopropanol, DMSO, acetic acid, ethanolamine, or ethyl acetate, or they may involve water as the solvent that is incorporated into the crystalline lattice. Solvates wherein water is the solvent incorporated into the crystalline lattice are typically referred to as "hydrates." Hydrates include stoichiometric hydrates as well as compositions containing variable amounts of water. The subject matter described herein includes such solvates.

The skilled artisan will further appreciate that certain compounds described herein that exist in crystalline form, including the various solvates thereof, may exhibit polymorphism (i.e. the capacity to occur in different crystalline structures). These different crystalline forms are typically known as "polymorphs." The subject matter disclosed herein includes such polymorphs. Polymorphs have the same chemical composition but differ in packing, geometrical arrangement, and other descriptive properties of the crystalline solid state. Polymorphs, therefore, may have different physical properties such as shape, density, hardness, deformability, stability, and dissolution properties. Polymorphs typically exhibit different melting points, **IR** spectra, and X-ray powder diffraction patterns, which may be used for identification. The skilled artisan will appreciate that different polymorphs may be produced, for example, by changing or adjusting the reaction conditions or reagents, used in making the compound. For example, changes in temperature, pressure, or solvent may result in polymorphs. **In** addition, one polymorph may spontaneously convert to another polymorph under certain conditions.

Compounds described herein, or a pharmaceutically acceptable salt thereof, may exist in stereoisomeric forms (e.g., it contains one or more asymmetric carbon atoms). The individual stereoisomers (enantiomers and diastereomers) and mixtures of these are included within the scope of the subject matter disclosed herein. Likewise, it is understood that a compound or salt described herein may exist in tautomeric forms other than that shown in the formula and these are also included within the scope of the subject matter disclosed herein. The scope of the subject matter disclosed herein includes mixtures of stereoisomers as well as purified enantiomers or enantiomerically/diastereomerically enriched mixtures.

The subject matter disclosed herein also includes isotopically-labelled forms of the compounds described herein, but for the fact that one or more atoms are replaced by an atom having an atomic mass or mass number different from the atomic mass or mass number usually found in nature. Examples of isotopes that can be incorporated into compounds described herein and pharmaceutically acceptable salts thereof include isotopes of hydrogen, carbon, nitrogen, oxygen, phosphorous, sulphur, fluorine, iodine, and chlorine, such as ²H, ³H, ¹¹C, ¹³C, ¹⁴C, ¹⁵N, ¹⁷O, ¹⁸O, ³¹P, ³²P, ³⁵S, ¹⁸F, ³⁶Cl, ¹²³I and ¹²⁵I.

Compounds as disclosed herein, and pharmaceutically acceptable salts thereof, that contain the aforementioned isotopes and/or other isotopes of other atoms are within the scope of the subject matter disclosed herein. Isotopically-labelled compounds are disclosed herein, for example those into which radioactive isotopes such as ³H, ¹⁴C are incorporated, are useful in drug and/or substrate tissue distribution assays. Tritiated, i.e., ³H, and carbon-14, i.e., ¹⁴C, isotopes are commonly used for their ease of preparation and detectability. ¹¹C and ¹⁸F isotopes are useful in PET (positron emission tomography), and ¹²⁵I isotopes are useful in SPECT (single photon emission computerized tomography), all useful in brain imaging. Further, substitution with heavier isotopes such as deuterium, i.e., ²H, can afford certain therapeutic advantages resulting from greater metabolic stability, for example increased in vivo half-life or reduced dosage requirements and, hence, may be preferred in some circumstances. Isotopically labelled compounds can generally be prepared by carrying out the procedures disclosed by substituting a readily available isotopically labelled reagent for a non-isotopically labelled reagent.

### III. Formulations

In an additional aspect, the description provides therapeutic or pharmaceutical compositions comprising an effective amount of at least one of the compounds as described herein. Pharmaceutical compositions comprising an effective amount of at least one compound of the present disclosure in an effective amount, in combination with a pharmaceutically effective amount of a carrier, additive, or excipient, and optionally an additional bioactive agent, represents a further aspect of the disclosure.

In certain embodiments, the compositions comprise pharmaceutically acceptable salts, in particular, acid or base addition salts of compounds as described herein. The acids that are used to prepare the pharmaceutically acceptable acid addition salts of the aforementioned base compounds include those which form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methane sulfonate, ethanesulfonate, benzenesulfonate, p-toluenesulfonate and pamoate [i.e., 1,1'-methylene-bis-(2-hydroxy-3 naphthoate)] salts, among numerous others.

Pharmaceutically acceptable base addition salts may also be used to produce pharmaceutically acceptable salt forms of the compounds or derivatives. The chemical bases that may be used as reagents to prepare pharmaceutically acceptable base salts of the present compounds that are acidic in nature are those that form non-toxic base salts with such compounds. Such non-toxic base salts include, but are not limited to those derived from such pharmacologically acceptable cations such as alkali metal cations (e.g., potassium and sodium) and alkaline earth metal cations (eg, calcium, zinc and magnesium), ammonium or water-soluble amine addition salts such as N-methylglucamine-(meglumine), and the lower alkanolammonium and other base salts of pharmaceutically acceptable organic amines, among others.

The compositions as described herein may in certain embodiments be administered in single or divided unit doses by the oral, parenteral or topical routes. Administration of the compounds may range from continuous (intravenous drip) to several oral administrations per day (for example, Q.I.D.) and may include oral, topical, parenteral, intramuscular, intravenous, sub-cutaneous, transdermal (which may include a penetration enhancement agent), buccal, sublingual and suppository administration, by inhalation spray, rectally, vaginally, or via an implanted reservoir, among other routes of administration. Enteric coated oral tablets may also be used to enhance bioavailability of the compounds from an oral route of administration. The most effective dosage form will depend upon the pharmacokinetics of the particular agent chosen as well as the severity of disease in the patient. Administration of compounds according to the present disclosure as sprays, mists, or aerosols for intra-nasal, intra-tracheal or pulmonary administration may also be used. The present disclosure therefore also is directed to pharmaceutical compositions comprising an effective amount of compound according to the present disclosure, optionally in combination with a pharmaceutically acceptable carrier, additive or excipient. Compounds according to the present disclosure may be administered in immediate release, intermediate release or sustained or controlled release forms. Sustained or controlled release forms are preferably administered orally, but may also be administered in suppository and transdermal or other topical forms. Intramuscular injections in liposomal form may also be used to control or sustain the release of compound at an injection site.

Thus in one aspect, pharmaceutical formulations of a compound as described herein can be prepared for parenteral administration with a pharmaceutically acceptable parenteral vehicle and in a unit dosage injectable form. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously.

The compositions of the present disclosure may be formulated in a conventional manner using one or more pharmaceutically acceptable carriers and may also be administered in controlled-release formulations. The compounds of the disclosure can be formulated in accordance with standard pharmaceutical practice as a pharmaceutical composition. According to this aspect, there is provided a pharmaceutical composition comprising a compound as described herein, in association with one or more pharmaceutically acceptable excipients.

A typical formulation is prepared by mixing the compounds of the disclosure with excipients, such as carriers and/or diluents. Suitable carriers, diluents and other excipients are well known to those skilled in the art and include materials such as carbohydrates, waxes, water soluble and/or swellable polymers, hydrophilic or hydrophobic materials, gelatin, oils, solvents, water and the like. The particular carrier, diluent or other excipient used will depend upon the means and purpose for which the compound is being applied. Other pharmaceutically acceptable carriers that may be used in these pharmaceutical compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as prolamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

Solvents are generally selected based on solvents recognized by persons skilled in the art as safe (GRAS) to be administered to a mammal. **In** general, safe solvents are non-toxic aqueous solvents such as water and other non-toxic solvents that are soluble or miscible in water. Suitable aqueous solvents include water, ethanol, propylene glycol, polyethylene glycols (e.g., PEG 400, PEG 300), etc. and mixtures thereof. Acceptable diluents, carriers, excipients and stabilizers are nontoxic to recipients at the dosages and concentrations employed, and include buffers such as phosphate, citrate and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{™}, PLURONICS^{™} or polyethylene glycol (PEG).

The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents and other known additives for presentation or to aid in the manufacturing of the pharmaceutical product. The formulations may be prepared using conventional dissolution and mixing procedures.

Formulation may be conducted by mixing at ambient temperature at the appropriate pH, and at the desired degree of purity, with physiologically acceptable carriers, i.e., carriers that are non-toxic to recipients at the dosages and concentrations employed. The pH of the formulation depends mainly on the particular use and the concentration of compound, but may range from about 3 to about 8. Formulation in an acetate buffer at pH 5 is a suitable embodiment.

The pharmaceutical compositions may be in the form of a sterile injectable preparation, such as a sterile injectable aqueous or oleaginous suspension. In particular, formulations to be used for in vivo administration must be sterile. Such sterilization is readily accomplished by filtration through sterile filtration membranes. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, such 1,3-butanediol. The sterile injectable preparation may also be prepared as a lyophilized powder. Among the acceptable vehicles and solvents that may be employed are water, Ringer's solution and isotonic sodium chloride solution. **In** addition, sterile fixed oils may conventionally be employed as a solvent or suspending medium. For this purpose any bland fixed oil may be employed including synthetic mono- or diglycerides. **In** addition, fatty acids such as oleic acid may likewise be used in the preparation of injectables, as well as natural pharmaceutically-acceptable oils, such as olive oil or castor oil, especially in their polyoxyethylated versions. These oil solutions or suspensions may also contain a long-chain alcohol diluent or dispersant, such as Ph. Helv or similar alcohol.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

The pharmaceutical compositions as described herein may be orally administered in any orally acceptable dosage form including, but not limited to, capsules, tablets, aqueous suspensions or solutions. **In** the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are required for oral use, the active ingredient is combined with emulsifying and suspending agents. If desired, certain sweetening, flavoring or coloring agents may also be added.

Alternatively, the pharmaceutical compositions as described herein may be administered in the form of suppositories for rectal administration. These can be prepared by mixing the agent with a suitable non-irritating excipient, which is solid at room temperature but liquid at rectal temperature and therefore will melt in the rectum to release the drug. Such materials include cocoa butter, beeswax and polyethylene glycols.

The pharmaceutical compositions as described herein may also be administered topically. Suitable topical formulations are readily prepared for each of these areas or organs. Topical application for the lower intestinal tract can be effected in a rectal suppository formulation (see above) or in a suitable enema formulation. Topically-acceptable transdermal patches may also be used.

For topical applications, the pharmaceutical compositions may be formulated in a suitable ointment containing the active component suspended or dissolved in one or more carriers. Carriers for topical administration of the compounds of this disclosure include, but are not limited to, mineral oil, liquid petrolatum, white petrolatum, propylene glycol, polyoxyethylene, polyoxypropylene compound, emulsifying wax and water. In certain preferred aspects of the disclosure, the compounds may be coated onto a stent which is to be surgically implanted into a patient in order to inhibit or reduce the likelihood of occlusion occurring in the stent in the patient.

Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with our without a preservative such as benzylalkonium chloride. Alternatively, for ophthalmic uses, the pharmaceutical compositions may be formulated in an ointment such as petrolatum.

The pharmaceutical compositions of this disclosure may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

The pharmaceutical compositions comprising a compound of the present disclosure can be formulated, dosed and administered in a fashion, i.e., amounts, concentrations, schedules, course, vehicles and route of administration, consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "therapeutically effective amount" of the compound to be administered will be governed by such considerations, and is the minimum amount necessary to prevent, ameliorate, or treat the disorder. Such amount is preferably below the amount that is toxic to the host or renders the host significantly more susceptible to unwanted side effects.

A compound described herein can be formulated into pharmaceutical dosage forms to provide an easily controllable dosage of the drug and to enable patient compliance with the prescribed regimen. The pharmaceutical composition (or formulation) for application may be packaged in a variety of ways depending upon the method used for administering the drug. Generally, an article for distribution includes a container having deposited therein the pharmaceutical formulation in an appropriate form. Suitable containers are well known to those skilled in the art and include materials such as bottles (plastic and glass), sachets, ampoules, plastic bags, metal cylinders, and the like. The container may also include a tamperproof assemblage to prevent indiscreet access to the contents of the package. In addition, the container has deposited thereon a label that describes the contents of the container. The label may also include appropriate warnings.

The formulations may be packaged in unit-dose or multi-dose containers, for example sealed ampoules and vials, and may be stored in a freeze-dried (lyophilized) condition requiring only the addition of the sterile liquid carrier, for example water, for injection immediately prior to use. Extemporaneous injection solutions and suspensions are prepared from sterile powders, granules and tablets of the kind previously described. Preferred unit dosage formulations are those containing a daily dose or unit daily sub-dose, as herein above recited, or an appropriate fraction thereof, of the active ingredient.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease or condition being treated.

A patient or subject in need of therapy using compounds according to the present disclosure can be treated by administering to the patient (subject) an effective amount of the compound according to the present disclosure including pharmaceutically acceptable salts, solvates or polymorphs, thereof optionally in a pharmaceutically acceptable carrier or diluent, either alone, or in combination with other agents.

The active compound is included in the pharmaceutically acceptable carrier or diluent in an amount sufficient to deliver to a patient a therapeutically effective amount for the desired indication, without causing serious toxic effects in the patient treated. A preferred dose of the active compound for the herein-mentioned conditions is in the range from about 10 ng/kg to 300 mg/kg, preferably 0.1 to 100 mg/kg per day, more generally 0.5 to about 25 mg per kilogram body weight of the recipient/patient per day. One typical daily dosage might range from about 1 µg/kg to 100 mg/kg or more, depending on the factors mentioned above. A typical topical dosage will range from 0.01-5% wt/wt in a suitable carrier.

The compound is conveniently administered in any suitable unit dosage form, including but not limited to one containing less than 1 mg, 1 mg to 3000 mg, preferably 5 to 500 mg of active ingredient per unit dosage form. An oral dosage of about 25-250 mg is often convenient.

The active ingredient is preferably administered to achieve peak plasma concentrations of the active compound of about 0.00001-30 mM, preferably about 0.1-30 mM. This may be achieved, for example, by the intravenous injection of a solution or formulation of the active ingredient, optionally in saline, or an aqueous medium or administered as a bolus of the active ingredient. Oral administration is also appropriate to generate effective plasma concentrations of active agent.

The concentration of active compound in the drug composition will depend on absorption, distribution, inactivation, and excretion rates of the drug as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed composition. The active ingredient may be administered at once, or may be divided into a number of smaller doses to be administered at varying intervals of time.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art.

Liposomal suspensions may also be pharmaceutically acceptable carriers. These may be prepared according to methods known to those skilled in the art, for example, as described in U.S. Pat. No.4,522,811 For example, liposome formulations may be prepared by dissolving appropriate lipid(s) (such as stearoyl phosphatidyl ethanolamine, stearoyl phosphatidyl choline, arachadoyl phosphatidyl choline, and cholesterol) in an inorganic solvent that is then evaporated, leaving behind a thin film of dried lipid on the surface of the container. An aqueous solution of the active compound are then introduced into the container. The container is then swirled by hand to free lipid material from the sides of the container and to disperse lipid aggregates, thereby forming the liposomal suspension.

The term "pharmaceutically acceptable salt" is used throughout the specification to describe, where applicable, a salt form of one or more of the compounds described herein which are presented to increase the solubility of the compound in the gastric juices of the patient's gastrointestinal tract in order to promote dissolution and the bioavailability of the compounds. Pharmaceutically acceptable salts include those derived from pharmaceutically acceptable inorganic or organic bases and acids, where applicable. Suitable salts include those derived from alkali metals such as potassium and sodium, alkaline earth metals such as calcium, magnesium and ammonium salts, among numerous other acids and bases well known in the pharmaceutical art. Sodium and potassium salts are particularly preferred as neutralization salts of the phosphates according to the present disclosure.

The subject matter further provides veterinary compositions comprising at least one active ingredient as above defined together with a veterinary carrier therefore. Veterinary carriers are materials useful for the purpose of administering the composition and may be solid, liquid or gaseous materials which are otherwise inert or acceptable in the veterinary art and are compatible with the active ingredient. These veterinary compositions may be administered parenterally or by any other desired route.

### IV. Indications and Methods of Treatment

It is contemplated that the compounds disclosed herein may be used in therapy. It is further contemplated that the compounds disclosed herein may be used to treat a disease or indication associated with VHL activity, such as the diseases and indications in Zhang et al., J. Med. Chem. 219, 62, 5725-5749,

Generally, the disease or disorder to be treated is a hyperproliferative disease such as cancer. In some embodiments, the compounds disclosed herein may be used to treat a cancer implicated by VHL modulation. In some embodiments, the compounds disclosed herein may be used to treat a solid tumor. In some embodiments, the solid tumor is breast cancer (such as triple-negative breast cancer), lung cancer, multiple myeloma or renal cell carcinoma (RCC). In some embodiments, the compounds disclosed herein may be used to treat anemia including chronic anemia such as anemia associated with chronic kidney disease, dialysis and chemotherapy. In some embodiments, the comounds disclosed herein may be used to treat ischemia including local ischemia, stroke and cardiovascular ischemia.

In an embodiment the compounds of the formula (I), or a pharmaceutically acceptable salt thereof, or of the formula (II), or a pharmaceutically acceptable salt thereof, are used for treatment of a hyperproliferative disorder selected from the group consisting of breast carcinoma, lung adenocarcinoma, lung large cell carcinoma, lung adenosquamous carcinoma, lung squamous cell carcinoma, colon adenocarcinoma and pancreatic ductal adenocarcinoma.

### V. Articles of Manufacture

In another aspect, described herein are articles of manufacture, for example, a "kit", containing materials useful for the treatment of the diseases and disorders described above is provided. The kit may further comprise a label or package insert, on or associated with the container. The term "package insert" is used to refer to instructions customarily included in commercial packages of therapeutic products, that contain information about the indications, usage, dosage, administration, contraindications and/or warnings concerning the use of such therapeutic products.

Suitable containers include, for example, bottles, vials, syringes, blister pack, etc. A "vial" is a container suitable for holding a liquid or lyophilized preparation. In one embodiment, the vial is a single-use vial, e.g. a 20-cc single-use vial with a stopper. The container may be formed from a variety of materials such as glass or plastic.

The label or package insert may indicate that the patient to be treated is one having a disorder such as a hyperproliferative disorder.

### VII. Enumerated Embodiments

1. A compound of Table 1-A, Table 1-B, or Table 1-C, or a stereoisomer or tautomer thereof, or a pharmaceutically acceptable salt of any of the foregoing.
2. A pharmaceutical composition comprising a comound of Table 1-A, Table 1-B, or Table 1-C, or a stereoisomer or tautomer thereof, or a pharmaceutically acceptable salt of any of the foregoing, and a pharmaceutically acceptable carrier.
3. A compound of Table 1-A, Table 1-B, or Table 1-C, or a stereoisomer or tautomer thereof, or a pharmaceutically acceptable salt of any of the foregoing for use in a therapeutic method.
4. The compound of embodiment 3, wherein the therapeutic method comprises administering a therapeutically effective amount of the compound, or a stereoisomer or tautomer thereof, or a pharmaceutically acceptable salt of any of the foregoing to a subject in need thereof.
5. The compound of embodiment 4, wherein the subject is a human.
6. The compound of any one of embodiments 3-5, wherein the therapeutic method is the treatment of a disease or indication associated with VHL activity.
7. The compound of embodiment 6, wherein the disease or indication is a hyperproliferative disorder, anemia or ischemia.
8. The compound of embodiment 7, wherein the disease or indication is a hyperproliferative disorder that is a solid tumor.
9. The compound of embodiment 8, wherein the solid tumor is breast cancer, lung cancer, multiple myoloma or renal cell carcinoma.
10. A method of treating a disease or indication associated with VHL actity in a subject in need thereof, comprising administering to the subject a therapeutically effective amount of a compound of Table 1-A, Table 1-B, or Table 1-C, or a stereoisomer or tautomer thereof, or a pharmaceutically acceptable salt of any of the foregoing.
11. The method of embodiment 10, wherein the disease or disorder is a hyperproliferative disorder, anemia or ischemia.
12. The method of embodiment 11, wherein the disease or indication is a hyperproliferative disorder that is a solid tumor.
13. The method of embodiment 12, wherein the solid tumor is breast cancer, lung cancer, multiple myoloma or renal cell carcinoma. A kit comprising a compound of Table 1-A, Table 1-B, or Table 1-C, or a stereoisomer or tautomer thereof, or a pharmaceutically acceptable salt of any of the foregoing, and instructions for use in treating a hyperproliferative disorder.

### VII. Examples

The following synthetic reaction schemes are merely illustrative of some of the methods by which the compounds of the present disclosure (or an embodiment or aspect thereof) can be synthesized. Various modifications to the synthetic reaction scheme can be made and will be suggested to one skilled in the art having referred to the disclosure contained herein.

The starting materials and reagents used in preparing these compounds generally are either available from commercial suppliers, such as Aldrich Chemical Co., or are prepared by methods known to those skilled in the art following procedures set forth in references such as Fieser and Fieser's Reagents for Organic Synthesis; Wiley & Sons: New York, 1991, Volumes 1-15; Rodd's Chemistry of Carbon Compounds, Elsevier Science Publishers, 1989, Volumes 1-5 and Supplementals; and Organic Reactions, Wiley & Sons: New York, 1991, Volumes 1-40.

The starting materials and the intermediates of the synthetic reaction schemes can be isolated and purified if desired using conventional techniques, including but not limited to, filtration, distillation, crystallization, chromatography, and the like. Such materials can be characterized using conventional means, including physical constants and spectral data. Synthetic steps as illustrated in schemes 1 to 6 can be carried out in any appropriate solvents and reaction conditions as understood by one skilled in the art.

Unless specified to the contrary, the reactions described herein preferably are conducted under an inert atmosphere at atmospheric pressure at a reaction temperature range of from about -78 °C to about 150 °C, more preferably from about 0 °C to about 125 °C.

Although certain exemplary embodiments are depicted and described herein, the compounds of the present disclosure (or an embodiment or aspect thereof) can be prepared using appropriate starting materials according to the methods described generally herein and/or by methods available to one of ordinary skill in the art.

The following generalized schemes are used to prepare the disclosed compounds, intermediates, and pharmaceutically acceptable salts thereof. Disclosed compounds and intermediates may be prepared using standard organic synthetic techniques and from comerically available starting materials and reagents. It will be appreciated that synthetic procedures employed in the preparation of disclosed compounds and intermediates will depend on the particular substituents present in the compound or intermediate and that various protection, deprotection, and conversion steps that are standard in organic synthesis may be required, but may not be illustrated in the following general schemes. It is also to be understood that any of the steps shown in any of the following general schemes may be used in any combination and in any order that is chemically feasible to achieve a desired intermediate or disclosed compound. Note that, in the following generalized schemes, the R₁, R₂, L₁, and L₂ moieties are as exemplified elsewhere herein, such as by the compounds of Table 1-A, Table 1-B, Table 1-C, and Table 2 and PG represents a suitable protecting group.

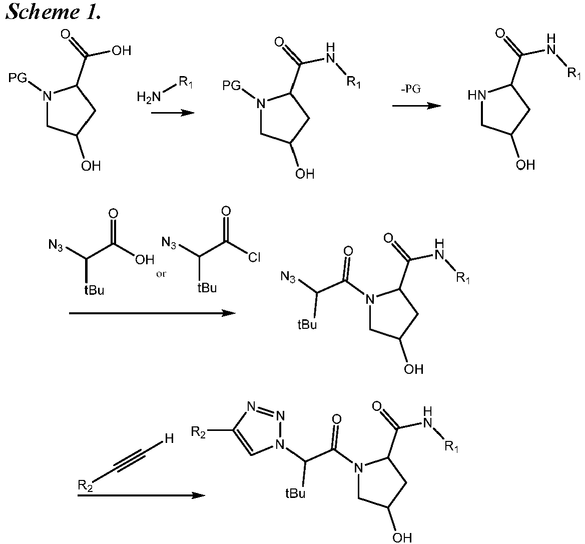

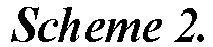

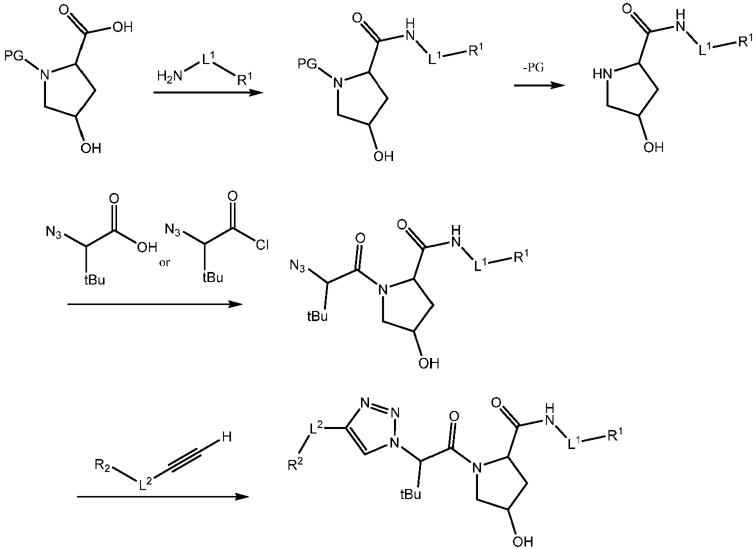

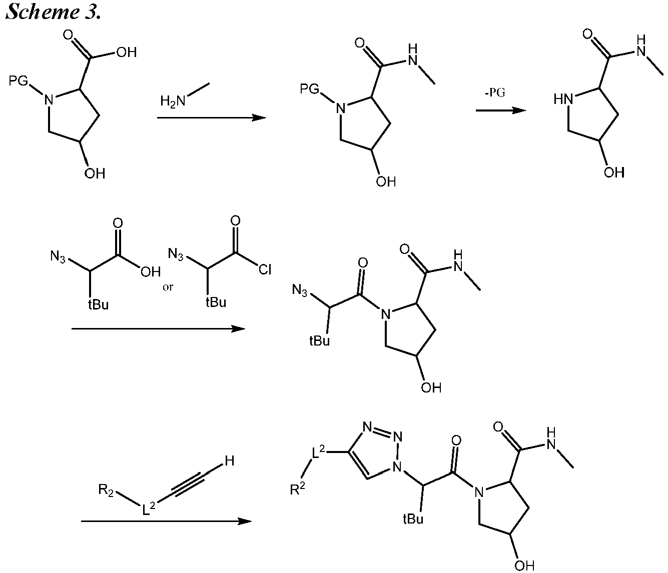

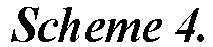

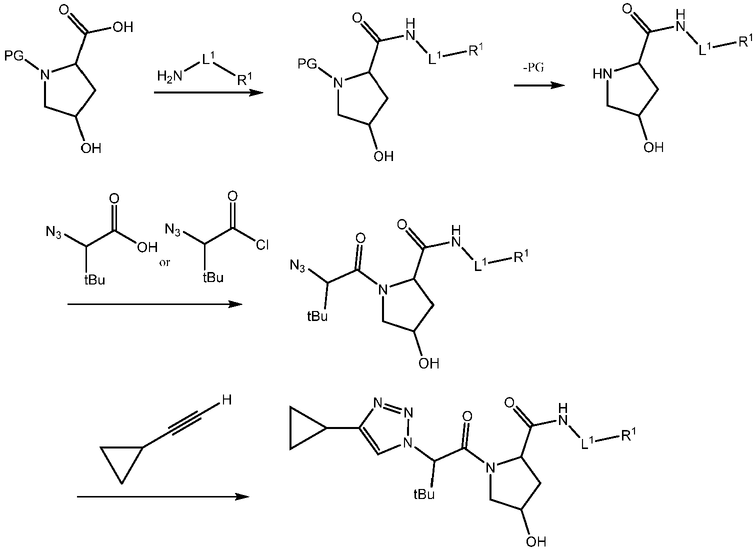

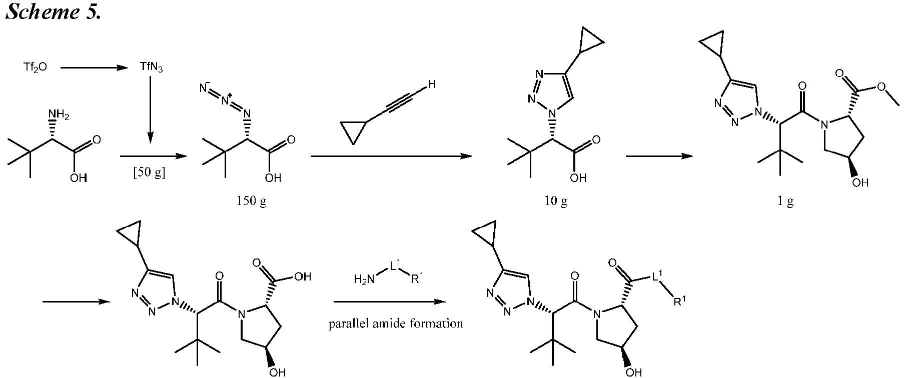

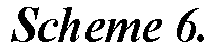

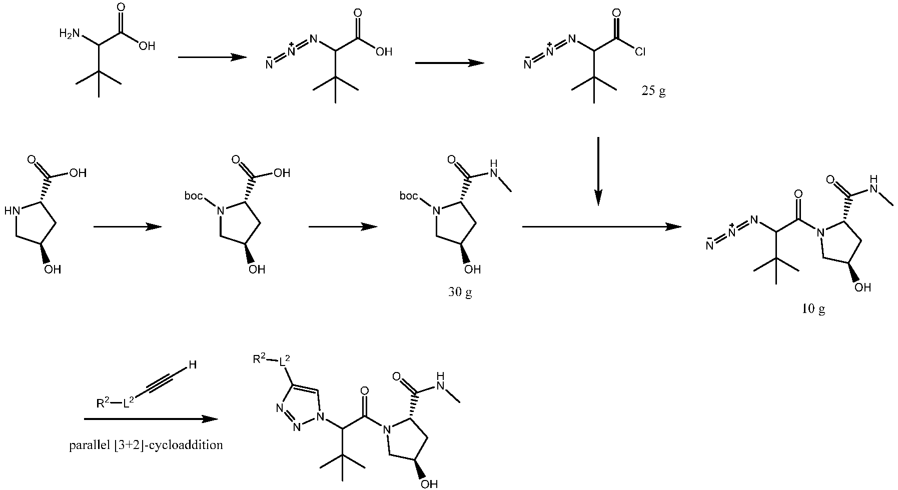

Synthetic data for selected compounds are provided in Table 3, below.

**Table 3**

| **#** | **Structure** | **Name** | **Synthesis Data** |
|---|---|---|---|
| 106 | 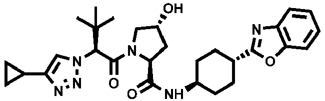 | (2S,4R)-N-[4-(1,3-benzoxazol-2-yl)cyclohexyl]-1-[(2S)-2-(4-cyclopropyltriazol-1-yl)-3,3-dimethyl-butanoyl]-4-hydroxy-pyrrolidine-2-carboxamide | The final product was 91.1% pure by LCMS (RT = 2.827 min; m/z 535.34 [M+1]⁺ |
| 140 | 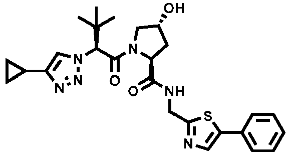 | (2S,4R)-1-[(2S)-2-(4-cyclopropyltriazol-1-yl)-3,3-dimethyl-butanoyl]-4-hydroxy-N-[(5-phenylthiazol-2-yl)methyl]pyrrolidine-2-carboxamide | The final product was 100% pure by LCMS (RT = 3.142 min; m/z 509.26 [M+1]⁺ |
| 169 | 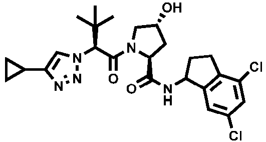 | (2S,4R)-1-[(2S)-2-(4-cyclopropyltriazol-1-yl)-3,3-dimethyl-butanoyl]-N-(4,6-dichloroindan-1-yl)-4-hydroxy-pyrrolidine-2-carboxamide | The final product was 100% pure by LCMS (mixture of 2 diastereomers; RT = 3.31; 3.41 min; m/z 520.22 [M+1]⁺ |
| 174 | 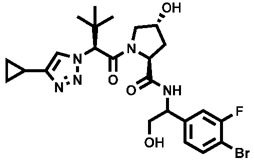 | (2S,4R)-N-[1-(4-bromo-3-fluorophenyl)-2-hydroxy-ethyl]-1-[(2S)-2-(4-cyclopropyltriazol-1-yl)-3,3-dimethyl-butanoyl]-4-hydroxy-pyrrolidine-2-carboxamide | The final product was 100% pure by LCMS (RT = 2.873 min; m/z 554.19 [M+1]⁺ |
| 266 | 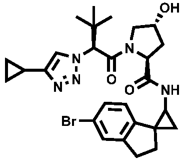 | (2S,4R)-N-(5'-bromospiro[cyclopropane-2,1'-indane]-1-yl)-1-[(2S)-2-(4-cyclopropyltriazol-1-yl)-3,3-dimethyl-butanoyl]-4-hydroxy-pyrrolidine-2-carboxamide | The final product was 100% pure by LCMS (RT = 3.452 min; m/z 558.23 [M+1]⁺ |
| 288 | 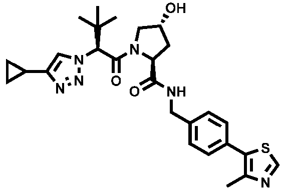 | (2S,4R)-1-[(2S)-2-(4-cyclopropyltriazol-1-yl)-3,3-dimethyl-butanoyl]-4-hydroxy-N-[[4-(4-methylthiazol-5-yl)phenyl]methyl]pyrrolidine-2-carboxamide | The final product was 100% pure by LCMS (RT = 2.403 min; m/z 523.28 [M+1]⁺ |
| 343 | 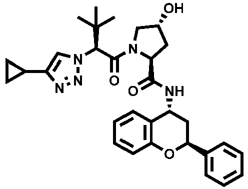 | (2S,4R)-1-[(2S)-2-(4-cyclopropyltriazol-1-yl)-3,3-dimethyl-butanoyl]-4-hydroxy-N-[(2S,4R)-2-phenylchroman-4-yl]pyrrolidine-2-carboxamide | The final product was 100% pure by LCMS (RT = 3.748 min; m/z 544.33 [M+1]⁺ |
| 405 | 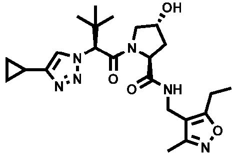 | (2S,4R)-1-[(2S)-2-(4-cyclopropyltriazol-1-yl)-3,3-dimethyl-butanoyl]-N-[(5-ethyl-3-methyl-isoxazol-4-yl)methyl]-4-hydroxy-pyrrolidine-2-carboxamide | The final product was 100% pure by LCMS (RT = 2.649 min; m/z 459.3 [M+1]⁺ |
| 420 | 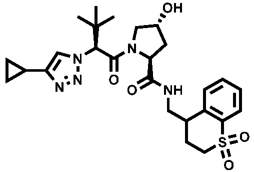 | (2S,4R)-1-[(2S)-2-(4-cyclopropyltriazol-1-yl)-3,3-dimethyl-butanoyl]-N-[(1,1-dioxo-3,4-dihydro-2H-thiochromen-4-yl)methyl]-4-hydroxy-pyrrolidine-2-carboxamide | The final product was 100% pure by LCMS (RT = 2.287 min; m/z 530.27 [M+1]⁺ |
| 445 | 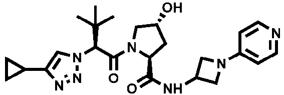 | (2S,4R)-1-[(2S)-2-(4-cyclopropyltriazol-1-yl)-3,3-dimethyl-butanoyl]-4-hydroxy-N-[1-(4-pyridyl)azetidin-3-yl]pyrrolidine-2-carboxamide | The final product was 100% pure by LCMS (RT = 1.667 min; m/z 468.3 [M+1]⁺ |
| 449 | 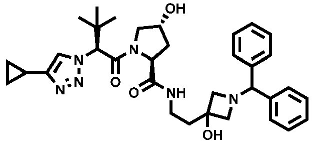 | (2S,4R)-N-[2-(1-benzhydryl-3-hydroxy-azetidin-3-yl)ethyl]-1-[(2S)-2-(4-cyclopropyltriazol-1-yl)-3,3-dimethyl-butanoyl]-4-hydroxy-pyrrolidine-2-carboxamide | The final product was 90.8% pure by LCMS (RT = 2.509 min; m/z 601.4 [M+1]⁺ |
| 462 | 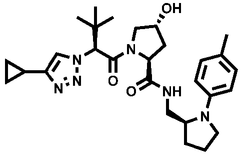 | (2S,4R)-1-[(2S)-2-(4-cyclopropyltriazol-1-yl)-3,3-dimethyl-butanoyl]-4-hydroxy-N-[[(2S)-1-(p-tolyl)pyrrolidin-2-yl]methyl]pyrrolidine-2-carboxamide | The final product was 100% pure by LCMS (RT = 2.861 min; m/z 509.37 [M+1]⁺ |
| 463 | 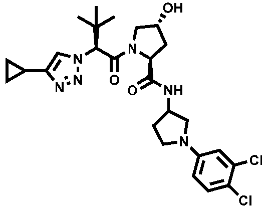 | (2S,4R)-1-[(2S)-2-(4-cyclopropyltriazol-1-yl)-3,3-dimethyl-butanoyl]-N-[1-(3,4-dichlorophenyl)pyrrolidin-3-yl]-4-hydroxy-pyrrolidine-2-carboxamide | The final product was 92.9% pure by LCMS (RT = 3.301 min; m/z 549.25 [M+1]⁺ |
| 469 | 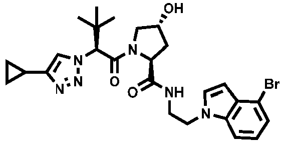 | (2S,4R)-N-[2-(4-bromoindol-1-yl)ethyl]-1-[(2S)-2-(4-cyclopropyltriazol-1-yl)-3,3-dimethyl-butanoyl]-4-hydroxy-pyrrolidine-2-carboxamide | The final product was 100% pure by LCMS (RT = 3.397 min; m/z 559.22 [M+1]⁺ |
| 480 | 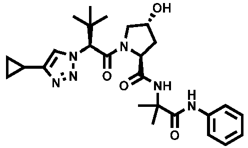 | (2S,4R)-N-(2-anilino-1,1-dimethyl-2-oxo-ethyl)-1-[(2S)-2-(4-cyclopropyltriazol-1-yl)-3,3-dimethyl-butanoyl]-4-hydroxy-pyrrolidine-2-carboxamide | The final product was 100% pure by LCMS (RT = 2.99 min; m/z 497.32 [M+1]⁺ |
| 493 | 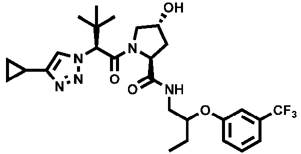 | (2S,4R)-1-[(2S)-2-(4-cyclopropyltriazol-1-yl)-3,3-dimethyl-butanoyl]-4-hydroxy-N-[2-[3-(trifluoromethyl)phenoxy]butyl] pyrrolidine-2-carboxamide | The final product was 100% pure by LCMS (RT = 3.903 min; m/z 552.32 [M+1]⁺ |
| 554 | 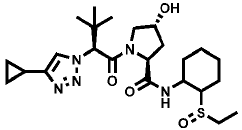 | (2S,4R)-1-[(2S)-2-(4-cyclopropyltriazol-1-yl)-3,3-dimethyl-butanoyl]-N-(2-ethylsulfinylcyclohexyl)-4-hydroxy-pyrrolidine-2-carboxamide | The final product was 100% pure by LCMS (3 diastereomer peaks RT = 2.273; 2.345; 2.472 min; m/z 494.32 [M+1]⁺ |
| 556 | 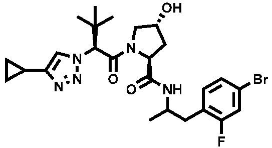 | (2S,4R)-N-[2-(4-bromo-2-fluorophenyl)-1-methyl-ethyl]-1-[(2S)-2-(4-cyclopropyltriazol-1-yl)-3,3-dimethyl-butanoyl]-4-hydroxy-pyrrolidine-2-carboxamide | The final product was 96.3% pure by LCMS (RT = 3.569 min; m/z 552.22 [M+1]⁺ |
| 561 | 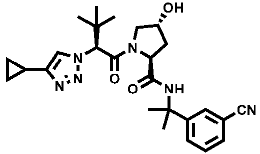 | (2S,4R)-N-[1-(3-cyanophenyl)-1-methyl-ethyl]-1-[(2S)-2-(4-cyclopropyltriazol-1-yl)-3,3-dimethyl-butanoyl]-4-hydroxy-pyrrolidine-2-carboxamide | The final product was 100% pure by LCMS (RT = 3.146 min; m/z 479.31 [M+1]⁺ |
| 617 | 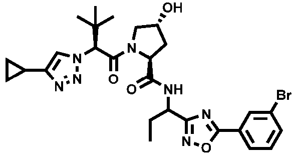 | (2S,4R)-N-[1-[5-(3-bromophenyl)-1,2,4-oxadiazol-3-yl]propyl]-1-[(2S)-2-(4-cyclopropyltriazol-1-yl)-3,3-dimethyl-butanoyl]-4-hydroxy-pyrrolidine-2-carboxamide | The final product was 100% pure by LCMS (RT = 3.018 min; m/z 602.22 [M+1]⁺ |
| 729 | 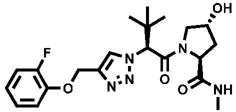 | (2S,4R)-1-[(2S)-2-[4-[(2-fluorophenoxy)methyl]triazol-1-yl]-3,3-dimethyl-butanoyl]-4-hydroxy-N-methyl-pyrrolidine-2-carboxamide | The final product was 100% pure by LCMS (RT = 1.197 min; m/z 434.24 [M+1]⁺ |
| 792 | 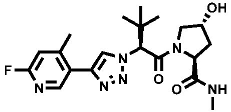 | (2S,4R)-1-[(2S)-2-[4-(6-fluoro-4-methyl-3-pyridyl)triazol-1-yl]-3,3-dimethyl-butanoyl]-4-hydroxy-N-methyl-pyrrolidine-2-carboxamide | The final product was 100% pure by LCMS (RT = 1.129 min; m/z 419.24 [M+1]⁺ |
| 829 | 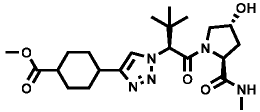 | Methyl 4-[1-[(1S)-1-[(2R,4S)-4-hydroxy-2-(methylcarbamoyl)pyrrolidine-1-carbonyl]-2,2-dimethylpropyl]triazol-4-yl]cyclohexanecarboxylate | The final product was 100% pure by LCMS (RT = 1.168 min; m/z 450.3 [M+1]⁺ |
| 914 | 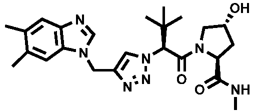 | (2S,4R)-1-[(2S)-2-[4-[(5,6-dimethylbenzimidazol-1-yl)methyl]triazol-1-yl]-3,3-dimethyl-butanoyl]-4-hydroxy-N-methyl-pyrrolidine-2-carboxamide | The final product was 85.3% pure by LCMS (RT = 0.981 min; m/z 468.3 [M+1]⁺ |
| 1059 | 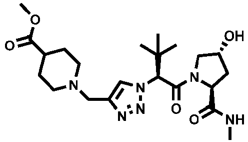 | methyl 1-[[1-[(1S)-1-[(2S,4R)-4-hydroxy-2-(methylcarbamoyl)pyrrolidine-1-carbonyl]-2,2-dimethylpropyl]triazol-4-yl]methyl]piperidine-4-carboxylate | The final product was 96.5% pure by LCMS (RT = 0.770 min; m/z 465.31 [M+1]⁺ |
| 1129 | 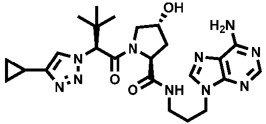 | (2S,4R)-N-[3-(6-aminopurin-9-yl)propyl]-1-[(2S)-2-(4-cyclopropyltriazol-1-yl)-3,3-dimethyl-butanoyl]-4-hydroxy-pyrrolidine-2-carboxamide | The final product was 100% pure by LCMS (RT = 1.000 min; m/z 511.31 [M+1]⁺ |

The compounds described herein may be assessed by an assay as described below.

### Biological Assays

### Example A: Fluorescence Polarization (FP) VHL Binding Assay

The binding of test compounds to the VHL Elongin B/C complex is measured using a fluorescence polarization tracer competition assay. The VHL / Elongin B/C protein complex used in the assay is generated as follows. The coding region for amino acids E55-D213 of human VHL with N-terminal His6 tag with a TEV -protease cleavage site is co-expressed with Elongin B (residues M1-Q118) and Elongin C (ResiduesM17-C112) in *E. coli.* The VHL / Elongin B/C complex is purified using an affinity nickel column, anion exchange HiTrap QP HP column chromatography, and gel filtration using a Superdex 75 26/60 column. The purified VHL / Elongin B/C complex is dialyzed into formulation buffer: 20mM Bis-Tris pH7.0, 150mM NaCl, 1mM DTT. A VHL fluorescence polarization probe consists of a VHL ligand coupled to carboxytetramethylrhodamine (TAMRA); (2S,4R)-N-(2-(2-(3',6'-bis(dimethylamino)-3-oxo-3H-spiro[isobenzofuran-1,9'-xanthene]-5-carboxamido)ethoxy)-4-(4-methylthiazol-5-yl)benzyl)-4-hydroxy-1-((R)-3-methyl-2-(3-methylisoxazol-5-yl)butanoyl)pyrrolidine-2-carboxamide. Compounds are prepared as a serial dilution in DMSO at a concentration 25-fold higher than the final desired concentration and acoustically dispensed (400 nl) into a ProxiPlate-384 Plus F, Black 384-shallow well Microplate (Part Number 6008260). DMSO is dispensed into wells designated for "VHL control" (without compound) wells. The "Assay Buffer" consists of 50 mM Tris pH 8.0, 120 mM NaCl, 0.005% Nonidet P-40, and 1% DMSO (v/v). Assay Buffer containing 5.28 µM VHL Elongin B/C complex is prepared and 5µl dispensed using a BioRapTR (Beckman Coulter) into each well of the assay plate. Assay Buffer is also dispensed into "no VHL control" wells using the same method. A "pre-assay" fluorescence measurement is made using an Infinite^{®} M1000 (Tecan) plate reader (Excitation 530 nm, Emission 574 nm, Bandwidth 10 nm). Assay Buffer containing 3.34 nM of the VHL FP probe is prepared in Assay Buffer and 5µl dispensed into each well of the assay plate using a BioRapTR (Beckman Coulter). The final VHL / Elongin B/C protein concentration is 2.64 nM and the final probe concentration is 1.67 nM. Assay plates are briefly centrifuged and incubated for 1 hour at room temperature. "Post-assay" fluorescence polarization measurements are made as described for the "pre-assay" fluorescence measurement. Fluorescence polarization is calculated for each sample; taking into account the "pre-assay" fluorescence measurements and subtracting the fluorescence signal of the compound/VHL only ("pre-assay") measurements from the "post-assay" fluorescence polarization measurements, for each plane of polarization. The data are analyzed using Genedata Screener software and normalized to the "no VHL control" and "VHL control" (without compound). IC₅₀ values are calculated using a four parameter curve fit (Robust method).

### Example B: Surface Plasmon Resonance Assay

Using a Biacore T200, Avidin tagged VHL co-expressed with Elongins B and C are immobilized to a Biacore SA chip in running buffer without DMSO. Compounds are tested individually at varying concentrations in running buffer (50 mM HEPES pH 7.2, 150 mM NaCl, 0.5 mM TCEP, 0.001% Tween 20, 0.2% PEG3350, 2% DMSO) at 20°C. Sensorgrams are run in order from low to high concentration using a flow rate of 80 µL/min. Association and disassociation times are varied depending on the estimated potency of the compound tested. Analysis of the binding curves and determination of the kinetic parameters is done using evaluation software (Version 2.0, Biacore).

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

### Example C: VHL HEK-293 BRET Assay

The VHL NanoBRET^{™} Target Engagement Assay analyzes the apparent affinity of test compounds for VHL in cells by competitive displacement of a VHL NanoBRET^{™} tracer reversibly bound to a NanoLuc^{®} VHL fusion protein stably expressed in the cells.

Test compounds were transferred to the assay plate (384 Well White Non-Binding Corning Assay Plates (Corning-3574)) using an Echo 555 Liquid Handler (Labcyte) in 2.5 nL increments and, as appropriate, intermediate stock concentrations of compounds, in order to prepare a titration series. 50 nL of control compound (10 mM; parental unlabeled VHL antagonist; see structure below) and 50 nL of DMSO (negative control) were dispensed into the appropriate control wells. DMSO was backfilled to a final volume of 50 nL as required. 50nl per well of 1 mM VHL NanoBRET^{™} Tracer in DMSO (NanoBRET^{™} Tracer-PEG2-590 (see structure below)) was transferred into each well using an Echo 555 (ultimately yielding a final concentration of 1uM). HEK 293 RT VHL-NanoLuc^{®} stable cells were cultured in DMEM High Glucose with Pyruvate, 10% fetal bovine serum, 2 mg/mL of Geneticin Selective Antibiotic (50 mg/mL) and 2 mM HEPES (1 M). Cells were seeded in Opti-MEM (Life Technologies-11058-021), 1.7 × 10⁵ cells/mL, 40 µl per well into the assay plate, centrifuged at 500 rpm for 30 seconds and incubated for 2 hours. Max Signal control wells consisted of DMSO only treated wells. Minimum Signal control wells contained of 10 uM parental unlabeled VHL antagonist (control compound - see structure below). 3X Complete Substrate plus Inhibitor Solution was prepared in Opti-MEM (consists of a 1:166 dilution of NanoBRET^{™} Nano-Glo^{®} Substrate plus a 1:500 dilution of Extracellular NanoLuc^{®} Inhibitor in Opti-MEM), and 20 ul was dispensed into each well of the 384-well plate and centrifuged at 1000 rpm for 1 minute, then incubated for 2 minutes at room temperature. Background Signal control wells were prepared without tracer for background correction steps.

Plates were read using a PerkinElmer Envision Reader (model 2104-0020) equipped with Luminescence option (Mirror: BRET2 Enh (PE Barcode 659), Emission Filter: Omega 610LP (Barcode 504), 2nd Emission Filter: Umbelliferone 460 (Barcode 207), Measurement height: 6.5 mm, Measurement time: 1s). The raw BRET ratio values were calculated by dividing the acceptor emission value (610 nm) by the donor emission value (460nm) for each sample. To correct for background, the BRET ratio in the absence of tracer (average of no-tracer control samples) was subtracted from the BRET ratio of each sample. Raw BRET units were converted to milliBRET units (mBU) by multiplying each raw BRET value by 1,000. The normalized NanoBRET^{™} signal was calculated relative to the Max Signal control wells (DMSO treated control wells) and the Minimum Signal control wells. Percentage inhibition was calculated relative to the Minimum Signal control and Maximum Signal control wells. IC₅₀ values were derived by four parameter curve fitting using the Robust method.

### NanoBRET^{™} Tracer-PEG2-590:

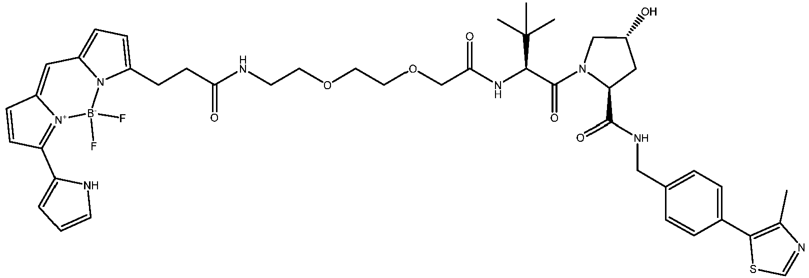

### Parental unlabeled VHL antagonist (control compound):

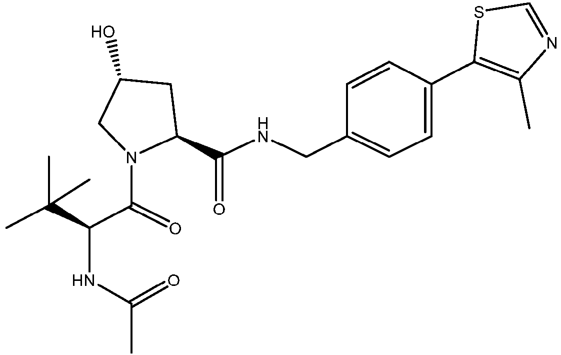

**Table 4 - Results of VHL HEK-293 BRET Assay**

| **#** | **Structure** | **Name** | **IC₅₀ + Digi (µM)*** | **IC₅₀ (µM)** |
|---|---|---|---|---|
| 106 | 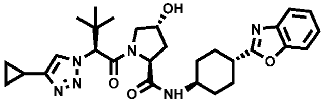 | (2S,4R)-N-[4-(1,3-benzoxazol-2-yl)cyclohexyl]-1-[(2S)-2-(4-cyclopropyltriazol-1-yl)-3,3-dimethyl-butanoyl]-4-hydroxy-pyrrolidine-2-carboxamide | 4.41 | 3.37 |
| 140 | 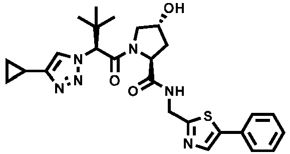 | (2S,4R)-1-[(2S)-2-(4-cyclopropyltriazol-1-yl)-3,3-dimethyl-butanoyl]-4-hydroxy-N-[(5-phenylthiazol-2-yl)methyl]pyrrolidine-2-carboxamide | 0.0142 | 0.01 |
| 169 | 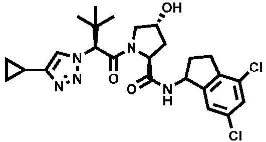 | (2S,4R)-1-[(2S)-2-(4-cyclopropyltriazol-1-yl)-3,3-dimethyl-butanoyl]-N-(4,6-dichloroindan-1-yl)-4-hydroxy-pyrrolidine-2-carboxamide | 2.93 | 1.81 |
| 174 | 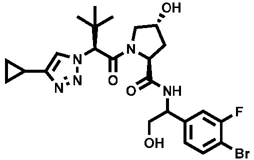 | (2S,4R)-N-[1-(4-bromo-3-fluorophenyl)-2-hydroxy-ethyl]-1-[(2S)-2-(4-cyclopropyltriazol-1-yl)-3,3-dimethyl-butanoyl]-4-hydroxy-pyrrolidine-2-carboxamide | 0.0152 | 0.0114 |
| 266 | 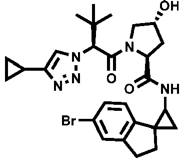 | (2S,4R)-N-(5'-bromospiro[cyclopropane-2,1'-indane]-1-yl)-1-[(2S)-2-(4-cyclopropyltriazol-1-yl)-3,3-dimethyl-butanoyl]-4-hydroxy-pyrrolidine-2-carboxamide | 9.5 | 3.69 |
| 288 | 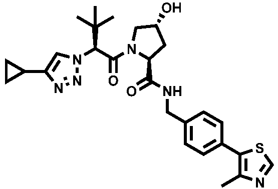 | (2S,4R)-1-[(2S)-2-(4-cyclopropyltriazol-1-yl)-3,3-dimethyl-butanoyl]-4-hydroxy-N-[[4-(4-methylthiazol-5-yl)phenyl]methyl]pyrrolidine-2-carboxamide | 0.0132 | 0.00794 |
| 343 | 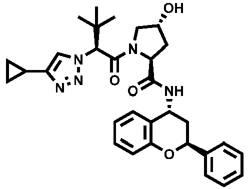 | (2S,4R)-1-[(2S)-2-(4-cyclopropyltriazol-1-yl)-3,3-dimethyl-butanoyl]-4-hydroxy-N-[(2S,4R)-2-phenylchroman-4-yl]pyrrolidine-2-carboxamide | 0.0321 | 0.0156 |
| 405 | 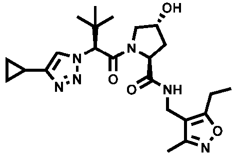 | (2S,4R)-1-[(2S)-2-(4-cyclopropyltriazol-1-yl)-3,3-dimethyl-butanoyl]-N-[(5-ethyl-3-methyl-isoxazol-4-yl)methyl]-4-hydroxy-pyrrolidine-2-carboxamide | 3.18 | 2.34 |
| 420 | 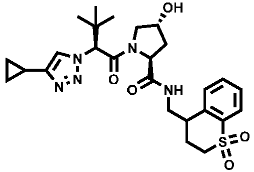 | (2S,4R)-1-[(2S)-2-(4-cyclopropyltriazol-1-yl)-3,3-dimethyl-butanoyl]-N-[(1,1-dioxo-3,4-dihydro-2H-thiochromen-4-yl)methyl]-4-hydroxy-pyrrolidine-2-carboxamide | 4.3 | 3.89 |
| 445 | 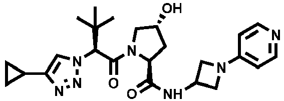 | (2S,4R)-1-[(2S)-2-(4-cyclopropyltriazol-1-yl)-3,3-dimethyl-butanoyl]-4-hydroxy-N-[1-(4-pyridyl)azetidin-3-yl]pyrrolidine-2-carboxamide | 22.3 | 26.7 |
| 449 | 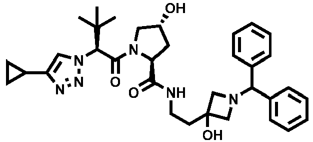 | (2S,4R)-N-[2-(1-benzhydryl-3-hydroxy-azetidin-3-yl)ethyl]-1-[(2S)-2-(4-cyclopropyltriazol-1-yl)-3,3-dimethyl-butanoyl]-4-hydroxy-pyrrolidine-2-carboxamide | 2.17 | 0.802 |
| 462 | 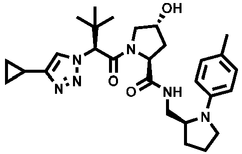 | (2S,4R)-1-[(2S)-2-(4-cyclopropyltriazol-1-yl)-3,3-dimethyl-butanoyl]-4-hydroxy-N-[[(2S)-1-(p-tolyl)pyrrolidin-2-yl]methyl]pyrrolidine-2-carboxamide | 5.35 | 2.82 |
| 463 | 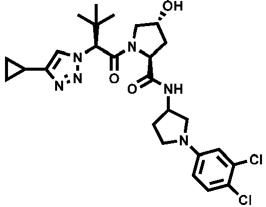 | (2S,4R)-1-[(2S)-2-(4-cyclopropyltriazol-1-yl)-3,3-dimethyl-butanoyl]-N-[1-(3,4-dichlorophenyl)pyrrolidin-3-yl]-4-hydroxy-pyrrolidine-2-carboxamide | 4.86 | 3.68 |
| 469 | 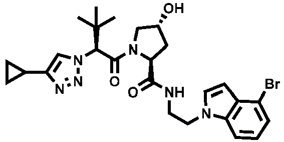 | (2S,4R)-N-[2-(4-bromoindol-1-yl)ethyl]-1-[(2S)-2-(4-cyclopropyltriazol-1-yl)-3,3-dimethyl-butanoyl]-4-hydroxy-pyrrolidine-2-carboxamide | 0.435 | 0.21 |
| 480 | 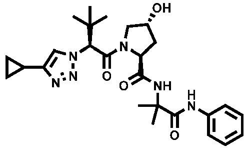 | (2S,4R)-N-(2-anilino-1,1-dimethyl-2-oxo-ethyl)-1-[(2S)-2-(4-cyclopropyltriazol-1-yl)-3,3-dimethyl-butanoyl]-4-hydroxy-pyrrolidine-2-carboxamide | 6.66 | 0.135 |
| 493 | 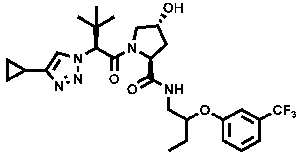 | (2S,4R)-1-[(2S)-2-(4-cyclopropyltriazol-1-yl)-3,3-dimethyl-butanoyl]-4-hydroxy-N-[2-[3-(trifluoromethyl)phenoxy]butyl] pyrrolidine-2-carboxamide | 5.56 | 3.68 |
| 554 | 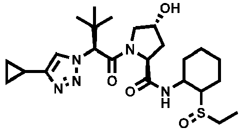 | (2S,4R)-1-[(2S)-2-(4-cyclopropyltriazol-1-yl)-3,3-dimethyl-butanoyl]-N-(2-ethylsulfinylcyclohexyl)-4-hydroxy-pyrrolidine-2-carboxamide | 22.2 | 17.2 |
| 556 | 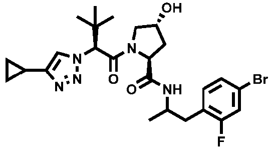 | (2S,4R)-N-[2-(4-bromo-2-fluorophenyl)-1-methyl-ethyl]-1-[(2S)-2-(4-cyclopropyltriazol-1-yl)-3,3-dimethyl-butanoyl]-4-hydroxy-pyrrolidine-2-carboxamide | 2.52 | 0.55 |
| 561 | 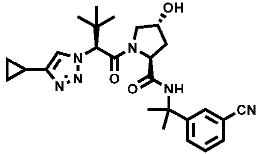 | (2S,4R)-N-[1-(3-cyanophenyl)-1-methyl-ethyl]-1-[(2S)-2-(4-cyclopropyltriazol-1-yl)-3,3-dimethyl-butanoyl]-4-hydroxy-pyrrolidine-2-carboxamide | 1.64 | 0.928 |
| 617 | 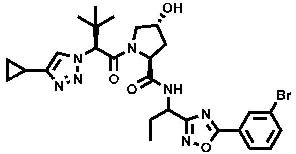 | (2S,4R)-N-[1-[5-(3-bromophenyl)-1,2,4-oxadiazol-3-yl]propyl]-1-[(2S)-2-(4-cyclopropyltriazol-1-yl)-3,3-dimethyl-butanoyl]-4-hydroxy-pyrrolidine-2-carboxamide | 1.35 | 0.829 |
| 729 | 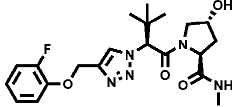 | (2S,4R)-1-[(2S)-2-[4-[(2-fluorophenoxy)methyl]triazol-1-yl]-3,3-dimethyl-butanoyl]-4-hydroxy-N-methyl-pyrrolidine-2-carboxamide | 1.48 | 0.96 |
| 792 | 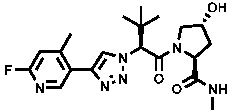 | (2S,4R)-1-[(2S)-2-[4-(6-fluoro-4-methyl-3-pyridyl)triazol-1-yl]-3,3-dimethyl-butanoyl]-4-hydroxy-N-methyl-pyrrolidine-2-carboxamide | 2.46 | 1.92 |
| 829 | 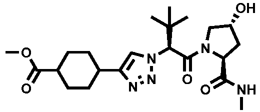 | Methyl 4-[1-[(1S)-1-[(2R,4S)-4-hydroxy-2-(methylcarbamoyl)pyrrolidine-1-carbonyl]-2,2-dimethylpropyl]triazol-4-yl]cyclohexanecarboxylate | 5.86 | 2.91 |
| 914 | 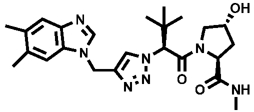 | (2S,4R)-1-[(2S)-2-[4-[(5,6-dimethylbenzimidazol-1-yl)methyl]triazol-1-yl]-3,3-dimethyl-butanoyl]-4-hydroxy-N-methyl-pyrrolidine-2-carboxamide | 3 | 5.46 |
| 1059 | 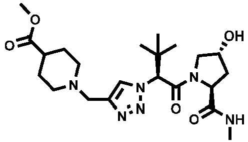 | methyl 1-[[1-[(1S)-1-[(2S,4R)-4-hydroxy-2-(methylcarbamoyl)pyrrolidine-1-carbonyl]-2,2-dimethylpropyl]triazol-4-yl]methyl]piperidine-4-carboxylate | 7.47 | 18.2 |
| 1129 | 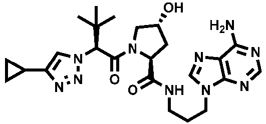 | (2S,4R)-N-[3-(6-aminopurin-9-yl)propyl]-1-[(2S)-2-(4-cyclopropyltriazol-1-yl)-3,3-dimethyl-butanoyl]-4-hydroxy-pyrrolidine-2-carboxamide | 2.33 | 17.3 |

| | | | | |
|---|---|---|---|---|
| * IC₅₀ + Digi (µM) = IC₅₀ values measured in the presence of digitonin | | | | |

### Example D: Cell Viability Assay

15 cell lines were profiled to represent a diverse genetic background and tissue types (Table 5).

**Table 5 - Summary of Cell Lines Profiled**

| cancer cell lines | cancer type |
|---|---|
| KPL-4 | Breast carcinoma |
| CAL-120 | Breast carcinoma |
| KPL-1 | breast carcinoma |
| MCF-7 | breast carcinoma |
| MDA-MB-436 | breast carcinoma |
| NCI-H23 | Lung adenocarcinoma |
| ABC-1 | Lung adenocarcinoma |
| NCI-H2030 | Lung adenocarcinoma |
| NCI-H1650 | Lung adenocarcinoma |
| HCC1438 | Lung large cell carcinoma |
| HCC366 | Lung adenosquamous carcinoma |
| KNS-62 | Lung squamous cell carcinoma |
| SW 620 | Colon adenocarcinoma |
| SUIT-2 | Pancreatic ductal adenocarcinoma |
| PANC-1 | Pancreatic ductal adenocarcinoma |

Cell lines were cultured in RPMI-1640, 5% fetal bovine serum, and 2 mM glutamine in a humidified incubator maintained at 37°C with 5% CO₂. Cells were assessed with a Vi-CELL Cell Viability Analyzer (Beckman Coulter; Brea, CA); viability of at least 90% was required for screening. A Multidropä Combi Reagent Dispenser (Thermo Scientific; Waltham, MA) was used to plate cells into Falcon^{â} 384-well, black, clear-bottom plates (Catalog No. 353962; Corning; Tewksbury, MA) using seeding densities previous determined to achieve approximately 80% confluence at the final time point of the assay. The compounds were prepared at 1000x in advance using an Echo 555 liquid handler (Labcyte; Sunnyvale, CA) and stored in -80°C until the day of compound addition. On the following day, cells were treated with compounds at final assay concentration 1uM and 10uM using a Bravo Automated Liquid-Handling Platform (Agilent; Santa Clara, CA). After 5 days, 25 mL CellTiter-Glo^{â} reagent was added using a MultiFloä Microplate Dispenser (BioTek; Winooski, VT). Cell lysis was induced by mixing for 30 minutes on an orbital shaker; plates were then incubated at room temperature for 10 minutes to stabilize the luminescent signal. Luminescence was read by a 2104 EnVision^{â} Multilabel Plate Reader (PerkinElmer; Waltham, MA).

The data was processed using Genedata Screener^{â}, Version 15 (Genedata;Basel, Switzerland) to assess compound effects on viability. Compound treated wells were normalized to the median value of 14 DMSO control wells on the same 384 well plate.

Table 6 listst selected compounds yielding a >30% effect on viability.

**Table 6 - Results of Cell Viability Assay**

| **Compound #** | **Conc. [µM]** | **Cell Line** | **Normalized viability %DMSO** |
|---|---|---|---|
| 266 | 10 | CAL-120 | 46.4 |
| | 10 | NCI-H2030 | 31.8 |
| | 10 | NCI-H23 | 37.6 |
| | 10 | PANC-1 | 27.6 |
| 445 | 10 | HCC366 | 5.2 |
| 449 | 10 | NCI-H23 | 43.4 |
| 462 | 10 | HCC366 | 11.3 |
| 463 | 10 | PANC-1 | 35.6 |
| 554 | 10 | ABC-1 | 38.2 |
| 106 | 10 | HCC366 | 6.0 |
| 288 | 10 | ABC-1 | 7.1 |
| | 1 | CAL-120 | 31.1 |
| | 10 | CAL-120 | 31.9 |
| | 10 | HCC1438 | 2.1 |
| | 10 | HCC366 | 22.5 |
| | 1 | HCC366 | 43.0 |
| | 10 | KNS-62 | 26.4 |
| | 1 | KNS-62 | 44.3 |
| | 10 | KPL-1 | 25.4 |
| | 1 | KPL-1 | 40.5 |
| | 10 | KPL-4 | 2.2 |
| | 10 | MCF-7 | 25.8 |
| | 10 | MDA-MB-436 | 15.8 |
| | 10 | NCI-H1650 | 27.7 |
| | 10 | NCI-H2030 | 16.5 |
| | 1 | NCI-H2030 | 19.2 |
| | 10 | NCI-H23 | 6.7 |
| | 1 | NCI-H23 | 8.6 |
| | 10 | PANC-1 | 31.1 |
| | 1 | PANC-1 | 33.9 |
| | 10 | SUIT-2 | 30.2 |
| | 10 | SW 620 | 28.2 |
| 110 | 1 | NCI-H23 | 18.9 |
| 561 | 10 | HCC366 | 12.5 |
| 480 | 10 | ABC-1 | 38.1 |
| | 10 | CAL-120 | 15.5 |
| | 10 | HCC1438 | 11.3 |
| | 10 | KNS-62 | 8.7 |
| | 10 | KPL-1 | 47.9 |
| | 10 | KPL-4 | 38.4 |
| | 10 | MCF-7 | 13.7 |
| | 10 | MDA-MB-436 | 23.6 |
| | 10 | NCI-H1650 | 12.6 |
| | 10 | NCI-H2030 | 5.6 |
| | 1 | NCI-H2030 | 32.4 |
| | 10 | NCI-H23 | 8.2 |
| | 1 | NCI-H23 | 37.0 |
| | 10 | PANC-1 | 22.7 |
| | 1 | PANC-1 | 38.1 |
| 140 | 10 | ABC-1 | 45.3 |
| 405 | 10 | ABC-1 | 7.6 |
| 236 | 1 | NCI-H23 | 18.7 |
| 420 | 1 | NCI-H23 | 10.8 |
| 169 | 10 | NCI-H23 | 39.3 |
| 343 | 10 | ABC-1 | 44.1 |
| 729 | 10 | ABC-1 | 35.8 |
| | 1 | NCI-H23 | 7.4 |
| 792 | 10 | ABC-1 | 13.9 |
| 829 | 1 | ABC-1 | 10.6 |
| 842 | 1 | CAL-120 | 18.5 |
| 914 | 10 | NCI-H23 | 44.5 |
| 974 | 1 | NCI-H2030 | 15.3 |
| 1059 | 10 | CAL-120 | 18.1 |
| | 10 | NCI-H2030 | 12.7 |
| 1131 | 1 | NCI-H23 | 21.7 |
| 1129 | 10 | ABC-1 | 4.2 |
| 174 | 10 | ABC-1 | 50.7 |
| 469 | 10 | CAL-120 | 62.6 |
| 493 | 10 | PANC-1 | 51.8 |
| 556 | 10 | NCI-H2030 | 50.4 |
| 617 | 10 | PANC-1 | 69.8 |

## Claims

1. A compound of formula (I) according to claim 15: 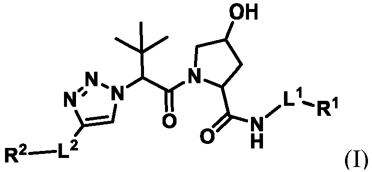 or a stereoisomer or tautomer thereof, or a pharmaceutically acceptable salt of any of the foregoing, wherein:
I) L¹-R¹ is methyl;
L² is a bond or C₁₋₁₂alkylene;
R² is
i) -O-C₆₋₂₀aryl, optionally substituted with one or more R^{s}; wherein R^{s} is, independently at each occurrence, halo;
ii) 4-20 membered heterocyclyl, optionally substituted with one or more R^{s};
wherein R^{s} is, independently at each occurrence, -C(O)O-C₁₋₆alkyl;
iii) 5-20 membered heteroaryl, wherein the 5-20 membered heteroaryl of R² is substituted with one or more R^{s}; wherein R^{s} is, independently at each occurrence, halo or C₁₋₁₂alkyl;
iv) C₆₋₂₀cycloalkyl, optionally substituted with one or more R^{s}; wherein R^{s} is, independently at each occurrence, -C(O)O-C₁₋₆alkyl; or
II) L²-R² is cyclopropyl;
L¹ is a bond or C₁₋₁₂alkylene; wherein the C₁₋₁₂alkylene of L¹ is optionally substituted with one or more R^{e}; wherein R^{e} is, independently at each occurrence, C₁₋₆alkyl or hydroxylC₁₋₆alkyl;
R¹ is
i) -L³-C₆₋₂₀aryl; wherein L³ is -O- or -C(O)NH-; the C₆₋₂₀aryl of -L³-C₆₋₂₀aryl is optionally substituted with one or more R^{s}, wherein R^{s} is, independently at each occurrence, haloC₁₋₆alkyl;
ii) C₄₋₂₀cycloalkyl optionally substituted with one or more R^{s}; wherein R^{s} is, independently at each occurrence, halo, 5-20 membered heteroaryl, or S(O)C₁₋₆alkyl;
iii) C₆₋₂₀aryl, optionally substituted with one or more R^{s}; wherein R^{s} is, independently at each occurrence, halo, cyano, or 5-20 membered heteroaryl;
wherein the 5-20 membered heteroaryl of R^{s} has one or more annular atoms, independently selected from N and O; and wherein the 5-20 membered heteroaryl of R^{s} is optionally substituted with one or more R^{t}; wherein R^{t} is, independently at each occurrence, C₁₋₆alkyl;
iv) 5-20 membered heteroaryl, optionally substituted with one or more R^{s}, wherein R^{s} is, independently at each occurrence, halo, amine, C₁₋₆alkyl, or C₆₋₂₀aryl;
wherein the C₆₋₂₀aryl of R^{s} is optionally substituted with one or more R^{t}; wherein R^{t} is, independently at each occurrence, halo; or
v) 4-20 membered heterocyclyl, optionally substituted with one or more R^{s}, wherein R^{s} is, independently at each occurrence, halo, oxo, hydroxyl, 5-20 membered heteroaryl, C₆₋₂₀aryl, or C₁₋₆alkyl;
wherein the C₁₋₆alkyl of R^{s} is optionally substituted with one or more R^{t}; wherein R^{t} is, independently at each occurrence, C₆₋₂₀aryl,
wherein the C₆₋₂₀aryl of R^{s} is optionally substituted with one or more R^{u}; wherein R^{u} is, independently at each occurrence, halo or C₁₋₆alkyl.

2. The compound of claim 1, or a stereoisomer or tautomer thereof, or a pharmaceutically acceptable salt of any of the foregoing, wherein:
L¹-R¹ is methyl;
L² is a bond or C₁₋₁₂alkylene; and
R² is
i) -O-C₆₋₂₀aryl, optionally substituted with one or more R^{s}; wherein R^{s} is, independently at each occurrence, halo;
ii) 4-20 membered heterocyclyl, optionally substituted with one or more R^{s}; wherein R^{s} is, independently at each occurrence, -C(O)O-C₁₋₆alkyl;
iii) 5-20 membered heteroaryl, wherein the 5-20 membered heteroaryl of R² is substituted with one or more R^{s}; wherein R^{s} is, independently at each occurrence, halo or C₁₋₁₂alkyl;
iv) C₆₋₂₀cycloalkyl, optionally substituted with one or more R^{s}; wherein R^{s} is, independently at each occurrence, -C(O)O-C₁₋₆alkyl.

3. The compound of claim 1, or a stereoisomer or tautomer thereof, or a pharmaceutically acceptable salt of any of the foregoing, wherein:
L²-R² is cyclopropyl;
L¹ is a bond or C₁₋₁₂alkylene; wherein the C₁₋₁₂alkylene of L¹ is optionally substituted with one or more R^{e}; wherein R^{e} is, independently at each occurrence, C₁₋₆alkyl or hydroxylC₁₋₆alkyl; and
R¹ is
i) -L³-C₆₋₂₀aryl; wherein L³ is -O- or -C(O)NH-; the C₆₋₂₀aryl of -L³-C₆₋₂₀aryl is optionally substituted with one or more R^{s}, wherein R^{s} is, independently at each occurrence, haloC₁₋₆alkyl;
ii) C₄₋₂₀cycloalkyl optionally substituted with one or more R^{s}; wherein R^{s} is, independently at each occurrence, halo, 5-20 membered heteroaryl, or S(O)C₁₋₆alkyl;
iii) C₆₋₂₀aryl, optionally substituted with one or more R^{s}; wherein R^{s} is, independently at each occurrence, halo, cyano, or 5-20 membered heteroaryl;
wherein the 5-20 membered heteroaryl of R^{s} has one or more annular atoms, independently selected from N and O; and wherein the 5-20 membered heteroaryl of R^{s} is optionally substituted with one or more R^{t}; wherein R^{t} is, independently at each occurrence, C₁₋₆alkyl;
iv) 5-20 membered heteroaryl, optionally substituted with one or more R^{s}, wherein R^{s} is, independently at each occurrence, halo, amine, C₁₋₆alkyl, or C₆₋₂₀aryl;
wherein the C₆₋₂₀aryl of R^{s} is optionally substituted with one or more R^{t}; wherein R^{t} is, independently at each occurrence, halo; or
v) 4-20 membered heterocyclyl, optionally substituted with one or more R^{s}, wherein R^{s} is, independently at each occurrence, halo, oxo, hydroxyl, 5-20 membered heteroaryl, C₆₋₂₀aryl, or C₁₋₆alkyl;
wherein the C₁₋₆alkyl of R^{s} is optionally substituted with one or more R^{t}; wherein R^{t} is, independently at each occurrence, C₆₋₂₀aryl,
wherein the C₆₋₂₀aryl of R^{s} is optionally substituted with one or more R^{u}; wherein R^{u} is, independently at each occurrence, halo or C₁₋₆alkyl.

4. The compound of any one of claims 1 or 2, or a stereoisomer or tautomer thereof, or a pharmaceutically acceptable salt of any of the foregoing, wherein:
(a)L¹-R¹ is methyl;
L² is a bond or C₁₋₁₂alkylene; and
R² is -O-C₆₋₂₀aryl, optionally substituted with one or more R^{s}; wherein R^{s} is, independently at each occurrence, halo;
(b) L¹-R¹ is methyl;
L² is a bond or C₁₋₁₂alkylene; and
R² is 4-20 membered heterocyclyl, optionally substituted with one or more R^{s};
wherein R^{s} is, independently at each occurrence, -C(O)O-C₁₋₆alkyl;
(c) L¹-R¹ is methyl;
L² is a bond or C₁₋₁₂alkylene; and
R² is 5-20 membered heteroaryl, wherein the 5-20 membered heteroaryl of R² is substituted with one or more R^{s}; wherein R^{s} is, independently at each occurrence, halo, or C₁₋₁₂alkyl;
or
(d) L¹-R¹ is methyl;
L² is a bond or C₁₋₁₂alkylene; and
R² is C₆₋₂₀cycloalkyl, optionally substituted with one or more R^{s}; wherein R^{s} is, independently at each occurrence, -C(O)O-C₁₋₆alkyl.

5. The compound of any one of claims 1 or 3, or a stereoisomer or tautomer thereof, or a pharmaceutically acceptable salt of any of the foregoing, wherein:
(a) L²-R² is cyclopropyl;
L¹ is a bond or C₁₋₁₂alkylene; wherein the C₁₋₁₂alkylene of L¹ is optionally substituted with one or more R^{e}; wherein R^{e} is, independently at each occurrence, C₁₋₆alkyl or hydroxylC₁₋₆alkyl; and
R¹ is -L³-C₆₋₂₀aryl; wherein L³ is -O- or -C(O)NH-; the C₆₋₂₀aryl of -L³-C₆₋₂₀aryl is optionally substituted with one or more R^{s}, wherein R^{s} is, independently at each occurrence, haloC₁₋₆alkyl;
(b) L²-R² is cyclopropyl;
L¹ is a bond or C₁₋₁₂alkylene; wherein the C₁₋₁₂alkylene of L¹ is optionally substituted with one or more R^{e}; wherein R^{e} is, independently at each occurrence, C₁₋₆alkyl or hydroxylC₁₋₆alkyl; and
wherein R¹ is C₄₋₂₀cycloalkyl optionally substituted with one or more R^{s}; wherein R^{s} is, independently at each occurrence, halo, 5-20 membered heteroaryl, or S(O)C₁₋₆alkyl;
(c) L²-R² is cyclopropyl;
L¹ is a bond or C₁₋₁₂alkylene; wherein the C₁₋₁₂alkylene of L¹ is optionally substituted with one or more R^{e}; wherein R^{e} is, independently at each occurrence, C₁₋₆alkyl or hydroxylC₁₋₆alkyl; and
R¹ is C₆₋₂₀aryl, optionally substituted with one or more R^{s}; wherein R^{s} is, independently at each occurrence, halo, cyano, or 5-20 membered heteroaryl;
wherein the 5-20 membered heteroaryl of R^{s} has one or more annular atoms, independently selected from N and O; and wherein the 5-20 membered heteroaryl of R^{s} is optionally substituted with one or more R^{t}; wherein R^{t} is, independently at each occurrence, C₁₋₆alkyl;
(d) L²-R² is cyclopropyl;
L¹ is a bond or C₁₋₁₂alkylene; wherein the C₁₋₁₂alkylene of L¹ is optionally substituted with one or more R^{e}; wherein R^{e} is, independently at each occurrence, C₁₋₆alkyl or hydroxylC₁₋₆alkyl; and
R¹ is 5-20 membered heteroaryl, optionally substituted with one or more R^{s}, wherein R^{s} is, independently at each occurrence, halo, amine, C₁₋₆alkyl, or C₆₋₂₀aryl;
wherein the C₆₋₂₀aryl of R^{s} is optionally substituted with one or more R^{t}; wherein R^{t} is, independently at each occurrence, halo;
or
(e) L²-R² is cyclopropyl;
L¹ is a bond or C₁₋₁₂alkylene; wherein the C₁₋₁₂alkylene of L¹ is optionally substituted with one or more R^{e}; wherein R^{e} is, independently at each occurrence, C₁₋₆alkyl or hydroxylC₁₋₆alkyl; and
R¹ is 4-20 membered heterocyclyl, optionally substituted with one or more R^{s},
wherein R^{s} is, independently at each occurrence, halo, oxo, hydroxyl, 5-20 membered heteroaryl, C₆₋₂₀aryl, or C₁₋₆alkyl;
wherein the C₁₋₆alkyl of R^{s} is optionally substituted with one or more R^{t}; wherein R^{t} is, independently at each occurrence, C₆₋₂₀aryl,
wherein the C₆₋₂₀aryl of R^{s} is optionally substituted with one or more R^{u}; wherein R^{u} is, independently at each occurrence, halo or C₁₋₆alkyl.

6. The compound of claim 1 or claim 2, or a stereoisomer or tautomer thereof, or a pharmaceutically acceptable salt of any of the foregoing, wherein:
L¹-R¹ is methyl;
L² is a bond or C₁₋₁₂alkylene; and
R² is selected from the group consisting of

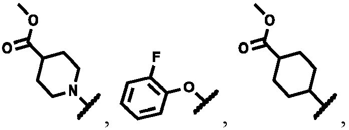

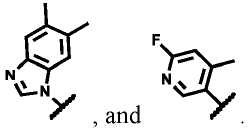

7. The compound of any one of claims 1, 2 or 6, or a stereoisomer or tautomer thereof, or a pharmaceutically acceptable salt of any of the foregoing, wherein:
L¹-R¹ is methyl;
L² is a bond; and
R² is selected from the group consisting of

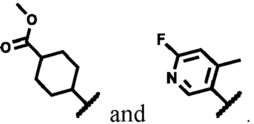

8. The compound of any one of claims 1, 2 or 6, or a stereoisomer or tautomer thereof, or a pharmaceutically acceptable salt of any of the foregoing, wherein:
L¹-R¹ is methyl;
L² is -CH₂-; and
R² is selected from the group consisting of

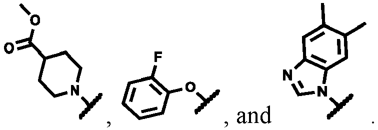

9. The compound of any one of claims 1 or 3, or a stereoisomer or tautomer thereof, or a pharmaceutically acceptable salt of any of the foregoing, wherein:
L²-R² is cyclopropyl;
L¹ is a bond or C₁₋₁₂alkylene; wherein the C₁₋₁₂alkylene of L¹ is optionally substituted with one or more R^{e}; wherein R^{e} is, independently at each occurrence, C₁₋₆alkyl or hydroxylC₁₋₆alkyl; and
R¹ is selected from the group consisting of

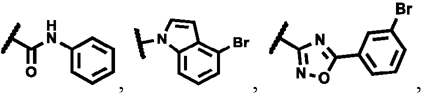

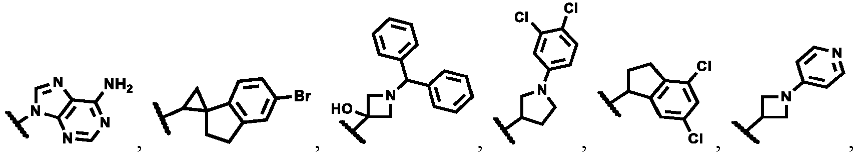

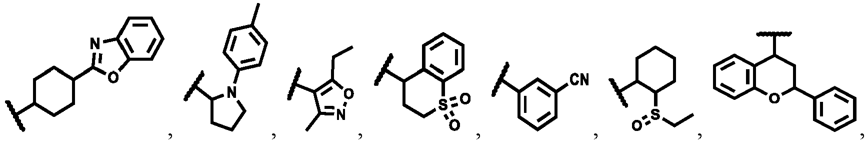

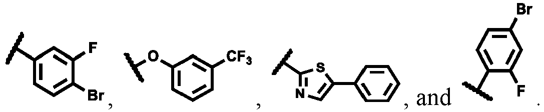

10. The compound of any one of claims 1, 3 or 9, or a stereoisomer or tautomer thereof, or a pharmaceutically acceptable salt of any of the foregoing, wherein:
L²-R² is cyclopropyl;
L¹ is a bond; and
R¹ is selected from the group consisting of

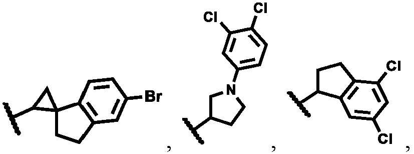

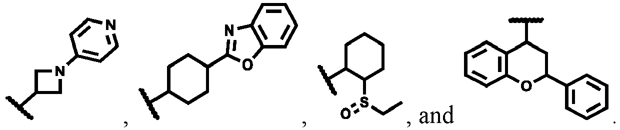

11. The compound of any one of claims 1, 3 or 9, or a stereoisomer or tautomer thereof, or a pharmaceutically acceptable salt of any of the foregoing, wherein:
L²-R² is cyclopropyl;
L¹ is selected from the group consisting of -CH₂-, -CH₂CH₂-, -CH₂CH₂CH₂-, -C (CH₃)₂-, -CH(CH₂OH)-, -CH(CH₂CH₃)-, -CH(CH₃)CH₂-, and -CH(CH₂CH₃)CH₂-; and
R¹ is selected from the group consisting of

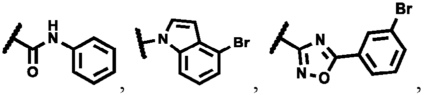

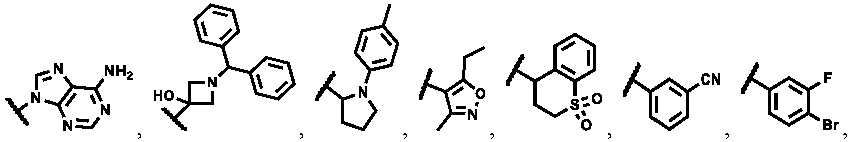

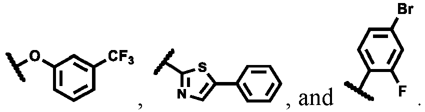

12. The compound of claim 1, or a pharmaceutically acceptable salt thereof, wherein the compound is selected from the group consisting of

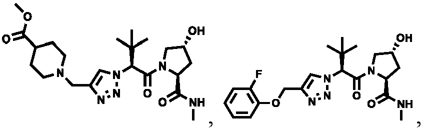

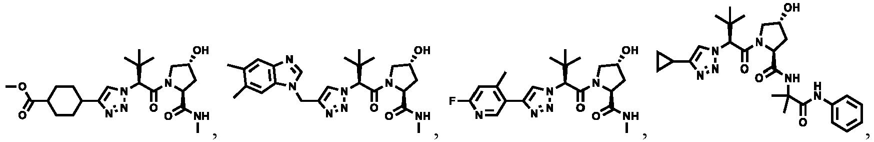

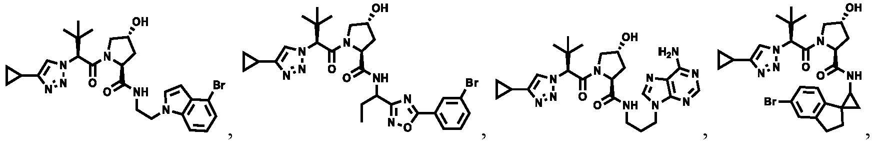

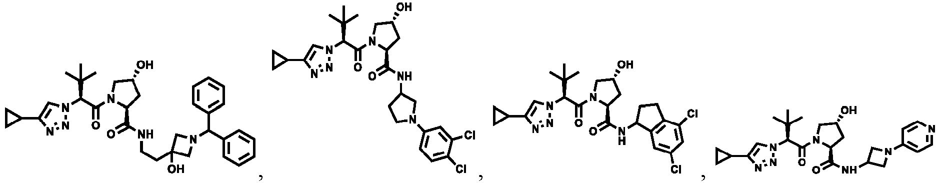

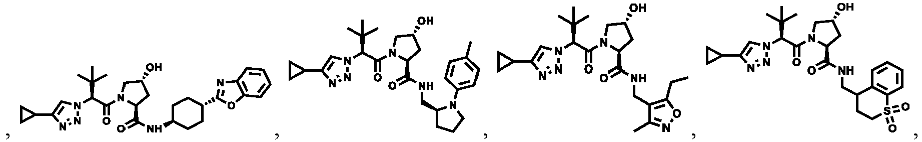

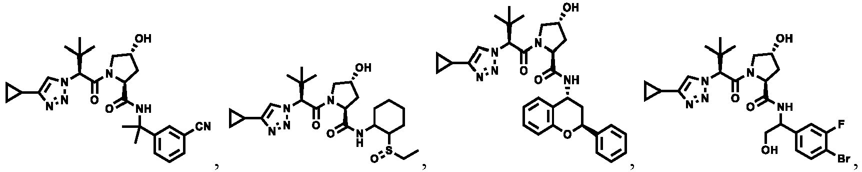

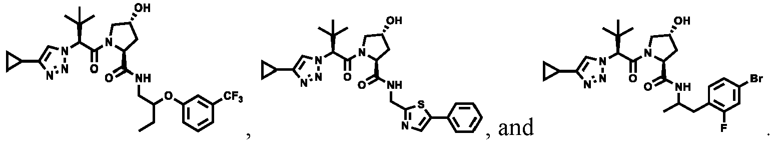

13. A pharmaceutical composition comprising a compound of any one of claims 1-12, or a stereoisomer or tautomer thereof, or a pharmaceutically acceptable salt of any of the foregoing, and one or more pharmaceutically acceptable excipients.

14. A compound according to any one of claims 1-12, or a stereoisomer or tautomer thereof, or a pharmaceutically acceptable salt of any of the foregoing, for use in treating a disease or indication associated with VHL activity.

15. A compound of formula (II): 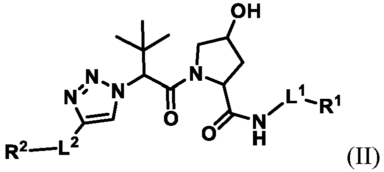 or a stereoisomer or tautomer thereof, or a pharmaceutically acceptable salt of any of the foregoing, wherein:
I) L¹-R¹ is methyl;
L² is a bond or C₁₋₁₂alkylene;
R² is
i) -O-C₆₋₂₀aryl, optionally substituted with one or more R^{s}; wherein R^{s} is, independently at each occurrence, halo;
ii) 4-20 membered heterocyclyl, optionally substituted with one or more R^{s};
wherein R^{s} is, independently at each occurrence, -C(O)O-C₁₋₆alkyl;
iii) 5-20 membered heteroaryl, wherein the 5-20 membered heteroaryl of R² is substituted with one or more R^{s}; wherein R^{s} is, independently at each occurrence, halo or C₁₋₁₂alkyl;
iv) C₆₋₂₀cycloalkyl, optionally substituted with one or more R^{s}; wherein R^{s} is, independently at each occurrence, -C(O)O-C₁₋₆alkyl;
v) -N(CH₃)-C₆₋₂₀aryl;
vi) -C₆₋₂₀aryl, optionally substituted with one or more R^{s}; wherein R^{s} is, independently at each occurrence, C₁₋₆haloalkyl; or
II) L²-R² is cyclopropyl;
L¹ is a bond or C₁₋₁₂alkylene; wherein the C₁₋₁₂alkylene of L¹ is optionally substituted with one or more R^{e}; wherein R^{e} is, independently at each occurrence, C₁₋₆alkyl or hydroxylC₁₋₆alkyl;
R¹ is
i) -L³-C₆₋₂₀aryl; wherein L³ is -O- or -C(O)NH-; the C₆₋₂₀aryl of -L³-C₆₋₂₀aryl is optionally substituted with one or more R^{s}, wherein R^{s} is, independently at each occurrence, haloC₁₋₆alkyl;
ii) C₄₋₂₀cycloalkyl optionally substituted with one or more R^{s}; wherein R^{s} is, independently at each occurrence, halo, 5-20 membered heteroaryl, or S(O)C₁₋₆alkyl;
iii) C₆₋₂₀aryl, optionally substituted with one or more R^{s}; wherein R^{s} is, independently at each occurrence, halo, cyano, or 5-20 membered heteroaryl;
wherein the 5-20 membered heteroaryl of R^{s} has one or more annular atoms, independently selected from N and O; and wherein the 5-20 membered heteroaryl of R^{s} is optionally substituted with one or more R^{t}; wherein R^{t} is, independently at each occurrence, C₁₋₆alkyl;
iv) 5-20 membered heteroaryl, optionally substituted with one or more R^{s}, wherein R^{s} is, independently at each occurrence, halo, amine, C₁₋₆alkyl, or C₆₋₂₀aryl;
wherein the C₆₋₂₀aryl of R^{s} is optionally substituted with one or more R^{t}; wherein R^{t} is, independently at each occurrence, halo; or
v) 4-20 membered heterocyclyl, optionally substituted with one or more R^{s}, wherein R^{s} is, independently at each occurrence, halo, oxo, hydroxyl, 5-20 membered heteroaryl, C₆₋₂₀aryl, or C₁₋₆alkyl;
wherein the C₁₋₆alkyl of R^{s} is optionally substituted with one or more R^{t}; wherein R^{t} is, independently at each occurrence, C₆₋₂₀aryl,
wherein the C₆₋₂₀aryl of R^{s} is optionally substituted with one or more R^{u}; wherein R^{u} is, independently at each occurrence, halo or C₁₋₆alkyl.

## Patentansprüche

1. Verbindung der Formel (I) gemäß Anspruch 15: 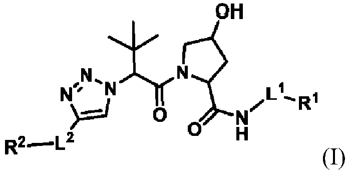 oder ein Stereoisomer oder Tautomer davon oder ein pharmazeutisch verträgliches Salz einer der vorgenannten Verbindungen, wobei:
I) L¹-R¹ Methyl ist;
L² eine Bindung oder C₁₋₁₂-Alkylen ist;
R² Folgendes ist:
i) -O-C₆₋₂₀-Aryl, gegebenenfalls substituiert mit einem oder mehreren R^{s}; wobei R^{s} bei jedem Vorkommen unabhängig voneinander Halogen ist;
ii) 4- bis 20-gliedriges Heterocyclyl, gegebenenfalls substituiert mit einem oder mehreren R^{s}; wobei R^{s} bei jedem Vorkommen unabhängig voneinander -C(O)O-C₁₋₆-Alkyl ist;
iii) 5- bis 20-gliedriges Heteroaryl, wobei das 5- bis 20-gliedrige Heteroaryl von R² mit einem oder mehreren R^{s} substituiert ist; wobei R^{s} bei jedem Vorkommen unabhängig voneinander Halogen oder C₁₋₁₂-Alkyl ist;
iv) C₆₋₂₀-Cycloalkyl, gegebenenfalls substituiert mit einem oder mehreren R^{s};
wobei R^{s} bei jedem Vorkommen unabhängig voneinander -C(O)O-C₁₋₆-Alkyl ist; oder
II) L²-R² Cyclopropyl ist;
L¹ eine Bindung oder C₁₋₁₂-Alkylen ist; wobei das C₁₋₁₂-Alkylen von L¹ gegebenenfalls mit einem oder mehreren R^{e} substituiert ist; wobei R^{e} bei jedem Vorkommen unabhängig voneinander C₁₋₆-Alkyl oder Hydroxyl-C₁₋₆-Alkyl ist;
R¹ Folgendes ist
i) -L³-C₆₋₂₀-Aryl; wobei L³ -O- oder -C(O)NH- ist; das C₆₋₂₀-Aryl von -L³-C₆₋₂₀-Aryl gegebenenfalls mit einem oder mehreren R^{s} substituiert ist, wobei R^{s} bei jedem Vorkommen unabhängig voneinander Halogen-C₁₋₆-Alkyl ist;
ii) C₄₋₂₀-Cycloalkyl, gegebenenfalls substituiert mit einem oder mehreren R^{s}; wobei R^{s} bei jedem Vorkommen unabhängig voneinander Halogen, 5- bis 20-gliedriges Heteroaryl oder S(O)C₁₋₆-Alkyl ist;
iii) C₆₋₂₀-Aryl, gegebenenfalls substituiert mit einem oder mehreren R^{s}; wobei R^{s} bei jedem Vorkommen unabhängig voneinander Halogen, Cyano oder 5- bis 20-gliedriges Heteroaryl ist;
wobei das 5- bis 20-gliedrige Heteroaryl von R^{s} ein oder mehrere Ringatome aufweist, unabhängig voneinander ausgewählt aus N und O; und wobei das 5- bis 20-gliedrige Heteroaryl von R^{s} gegebenenfalls mit einem oder mehreren R^{t} substituiert ist; wobei R^{t} bei jedem Vorkommen unabhängig voneinander C₁₋₆-Alkyl ist;
iv) 5- bis 20-gliedriges Heteroaryl, gegebenenfalls substituiert mit einem oder mehreren R^{s}, wobei R^{s} bei jedem Vorkommen unabhängig voneinander Halogen, Amin, C₁₋₆-Alkyl oder C₆₋₂₀-Aryl ist;
wobei das C₆₋₂₀-Aryl von R^{s} gegebenenfalls mit einem oder mehreren R^{t} substituiert ist; wobei R^{t} bei jedem Vorkommen unabhängig voneinander Halogen ist; oder
v) 4- bis 20-gliedriges Heterocyclyl, gegebenenfalls substituiert mit einem oder mehreren R^{s}, wobei R^{s} bei jedem Vorkommen unabhängig voneinander Halogen, Oxo, Hydroxyl, 5- bis 20-gliedriges Heteroaryl, C₆₋₂₀-Aryl oder C₁₋₆-Alkyl ist;
wobei das C₁₋₆-Alkyl von R^{s} gegebenenfalls mit einem oder mehreren R^{t} substituiert ist; wobei R^{t} bei jedem Vorkommen unabhängig voneinander C₆₋₂₀-Aryl ist,
wobei das C₆₋₂₀-Aryl von R^{s} gegebenenfalls mit einem oder mehreren R^{u} substituiert ist; wobei R^{u} bei jedem Vorkommen unabhängig voneinander Halogen oder C₁₋₆-Alkyl ist.

2. Verbindung nach Anspruch 1 oder ein Stereoisomer oder Tautomer davon oder ein pharmazeutisch verträgliches Salz einer der vorgenannten Verbindungen, wobei:
L¹-R¹ Methyl ist;
L² eine Bindung oder C₁₋₁₂-Alkylen ist; und
R² Folgendes ist:
i) -O-C₆₋₂₀-Aryl, gegebenenfalls substituiert mit einem oder mehreren R^{s}; wobei R^{s} bei jedem Vorkommen unabhängig voneinander Halogen ist;
ii) 4- bis 20-gliedriges Heterocyclyl, gegebenenfalls substituiert mit einem oder mehreren R^{s}; wobei R^{s} bei jedem Vorkommen unabhängig voneinander -C(O)O-C₁₋₆-Alkyl ist;
iii) 5- bis 20-gliedriges Heteroaryl, wobei das 5- bis 20-gliedrige Heteroaryl von R² mit einem oder mehreren R^{s} substituiert ist; wobei R^{s} bei jedem Vorkommen unabhängig voneinander Halogen oder C₁₋₁₂-Alkyl ist;
iv) C₆₋₂₀-Cycloalkyl, gegebenenfalls substituiert mit einem oder mehreren R^{s}; wobei R^{s} bei jedem Vorkommen unabhängig voneinander -C(O)O-C₁₋₆-Alkyl ist.

3. Verbindung nach Anspruch 1 oder ein Stereoisomer oder Tautomer davon oder ein pharmazeutisch verträgliches Salz einer der vorgenannten Verbindungen, wobei:
L²-R² Cyclopropyl ist;
L¹ eine Bindung oder C₁₋₁₂-Alkylen ist; wobei das C₁₋₁₂-Alkylen von L¹ gegebenenfalls mit einem oder mehreren R^{e} substituiert ist; wobei R^{e} bei jedem Vorkommen unabhängig voneinander C₁₋₆-Alkyl oder Hydroxyl-C₁₋₆-Alkyl ist; und
R¹ Folgendes ist:
i) -L³-C₆₋₂₀-Aryl; wobei L³ -O- oder -C(O)NH- ist; das C₆₋₂₀-Aryl von -L³-C₆₋₂₀-Aryl gegebenenfalls mit einem oder mehreren R^{s} substituiert ist, wobei R^{s} bei jedem Vorkommen unabhängig voneinander Halogen-C₁₋₆-Alkyl ist;
ii) C₄₋₂₀-Cycloalkyl, gegebenenfalls substituiert mit einem oder mehreren R^{s}; wobei R^{s} bei jedem Vorkommen unabhängig voneinander Halogen, 5- bis 20-gliedriges Heteroaryl oder S(O)C₁₋₆-Alkyl ist;
iii) C₆₋₂₀-Aryl, gegebenenfalls substituiert mit einem oder mehreren R^{s}; wobei R^{s} bei jedem Vorkommen unabhängig voneinander Halogen, Cyano oder 5- bis 20-gliedriges Heteroaryl ist;
wobei das 5- bis 20-gliedrige Heteroaryl von R^{s} ein oder mehrere Ringatome aufweist, unabhängig voneinander ausgewählt aus N und O; und wobei das 5- bis 20-gliedrige Heteroaryl von R^{s} gegebenenfalls mit einem oder mehreren R^{t} substituiert ist; wobei R^{t} bei jedem Vorkommen unabhängig voneinander C₁₋₆-Alkyl ist;
iv) 5- bis 20-gliedriges Heteroaryl, gegebenenfalls substituiert mit einem oder mehreren R^{s}, wobei R^{s} bei jedem Vorkommen unabhängig voneinander Halogen, Amin, C₁₋₆-Alkyl oder C₆₋₂₀-Aryl ist;
wobei das C₆₋₂₀-Aryl von R^{s} gegebenenfalls mit einem oder mehreren R^{t} substituiert ist; wobei R^{t} bei jedem Vorkommen unabhängig voneinander Halogen ist; oder
v) 4- bis 20-gliedriges Heterocyclyl, gegebenenfalls substituiert mit einem oder mehreren R^{s}, wobei R^{s} bei jedem Vorkommen unabhängig voneinander Halogen, Oxo, Hydroxyl, 5- bis 20-gliedriges Heteroaryl, C₆₋₂₀-Aryl oder C₁₋₆-Alkyl ist;
wobei das C₁₋₆-Alkyl von R^{s} gegebenenfalls mit einem oder mehreren R^{t} substituiert ist; wobei R^{t} bei jedem Vorkommen unabhängig voneinander C₆₋₂₀-Aryl ist,
wobei das C₆₋₂₀-Aryl von R^{s} gegebenenfalls mit einem oder mehreren R^{u} substituiert ist; wobei R^{u} bei jedem Vorkommen unabhängig voneinander Halogen oder C₁₋₆-Alkyl ist.

4. Verbindung nach einem der Ansprüche 1 oder 2 oder ein Stereoisomer oder Tautomer davon oder ein pharmazeutisch verträgliches Salz einer der vorgenannten Verbindungen, wobei:
(a) L¹-R¹ Methyl ist;
L² eine Bindung oder C₁₋₁₂-Alkylen ist; und
R² -O-C₆₋₂₀-Aryl ist, gegebenenfalls substituiert mit einem oder mehreren R^{s}; wobei R^{s} bei jedem Vorkommen unabhängig voneinander Halogen ist;
(b) L¹-R¹ Methyl ist;
L² eine Bindung oder C₁₋₁₂-Alkylen ist; und
R² 4- bis 20-gliedriges Heterocyclyl ist, gegebenenfalls substituiert mit einem oder mehreren R^{s}; wobei R^{s} bei jedem Vorkommen unabhängig voneinander -C(O)O-C₁₋₆-Alkyl ist;
(c) L¹-R¹ Methyl ist;
L² eine Bindung oder C₁₋₁₂-Alkylen ist; und
R² 5- bis 20-gliedriges Heteroaryl ist, wobei das 5- bis 20-gliedrige Heteroaryl von R² mit einem oder mehreren R^{s} substituiert ist; wobei R^{s} bei jedem Vorkommen unabhängig voneinander Halogen oder C₁₋₁₂-Alkyl ist;
oder
(d) L¹-R¹ Methyl ist;
L² eine Bindung oder C₁₋₁₂-Alkylen ist; und
R² C₆₋₂₀-Cycloalkyl ist, gegebenenfalls substituiert mit einem oder mehreren R^{s}; wobei R^{s} bei jedem Vorkommen unabhängig voneinander -C(O)O-C₁₋₆-Alkyl ist.

5. Verbindung nach einem der Ansprüche 1 oder 3 oder ein Stereoisomer oder Tautomer davon oder ein pharmazeutisch verträgliches Salz einer der vorgenannten Verbindungen, wobei:
(a) L²-R² Cyclopropyl ist;
L¹ eine Bindung oder C₁₋₁₂-Alkylen ist; wobei das C₁₋₁₂-Alkylen von L¹ gegebenenfalls mit einem oder mehreren R^{e} substituiert ist; wobei R^{e} bei jedem Vorkommen unabhängig voneinander C₁₋₆-Alkyl oder Hydroxyl-C₁₋₆-Alkyl ist; und
R¹ -L³-C₆₋₂₀-Aryl ist; wobei L³ -O- oder -C(O)NH- ist; das C₆₋₂₀-Aryl von -L³-C₆₋₂₀-Aryl gegebenenfalls mit einem oder mehreren R^{s} substituiert ist, wobei R^{s} bei jedem Vorkommen unabhängig voneinander Halogen-C₁₋₆-Alkyl ist;
(b) L²-R² Cyclopropyl ist;
L¹ eine Bindung oder C₁₋₁₂-Alkylen ist; wobei das C₁₋₁₂-Alkylen von L¹ gegebenenfalls mit einem oder mehreren R^{e} substituiert ist; wobei R^{e} bei jedem Vorkommen unabhängig voneinander C₁₋₆-Alkyl oder Hydroxyl-C₁₋₆-Alkyl ist; und
wobei R¹ C₄₋₂₀-Cycloalkyl ist, gegebenenfalls substituiert mit einem oder mehreren R^{s};
wobei R^{s} bei jedem Vorkommen unabhängig voneinander Halogen, 5- bis 20-gliedriges Heteroaryl oder S(O)C₁₋₆-Alkyl ist;
(c) L²-R² Cyclopropyl ist;
L¹ eine Bindung oder C₁₋₁₂-Alkylen ist; wobei das C₁₋₁₂-Alkylen von L¹ gegebenenfalls mit einem oder mehreren R^{e} substituiert ist; wobei R^{e} bei jedem Vorkommen unabhängig voneinander C₁₋₆-Alkyl oder Hydroxyl-C₁₋₆-Alkyl ist; und
R¹ C₆₋₂₀-Aryl ist, gegebenenfalls substituiert mit einem oder mehreren R^{s}; wobei R^{s} bei jedem Vorkommen unabhängig voneinander Halogen, Cyano oder 5- bis 20-gliedriges Heteroaryl ist;
wobei das 5- bis 20-gliedrige Heteroaryl von R^{s} ein oder mehrere Ringatome aufweist, unabhängig voneinander ausgewählt aus N und O; und wobei das 5- bis 20-gliedrige Heteroaryl von R^{s} gegebenenfalls mit einem oder mehreren R^{t} substituiert ist; wobei R^{t} bei jedem Vorkommen unabhängig voneinander C₁₋₆-Alkyl ist;
(d) L²-R² Cyclopropyl ist;
L¹ eine Bindung oder C₁₋₁₂-Alkylen ist; wobei das C₁₋₁₂-Alkylen von L¹ gegebenenfalls mit einem oder mehreren R^{e} substituiert ist; wobei R^{e} bei jedem Vorkommen unabhängig voneinander C₁₋₆-Alkyl oder Hydroxyl-C₁₋₆-Alkyl ist; und
R¹ 5- bis 20-gliedriges Heteroaryl ist, gegebenenfalls substituiert mit einem oder mehreren R^{s}, wobei R^{s} bei jedem Vorkommen unabhängig voneinander Halogen, Amin, C₁₋₆-Alkyl oder C₆₋₂₀-Aryl ist;
wobei das C₆₋₂₀-Aryl von R^{s} gegebenenfalls mit einem oder mehreren R^{t} substituiert ist; wobei R^{t} bei jedem Vorkommen unabhängig voneinander Halogen ist;
oder
(e) L²-R² Cyclopropyl ist;
L¹ eine Bindung oder C₁₋₁₂-Alkylen ist; wobei das C₁₋₁₂-Alkylen von L¹ gegebenenfalls mit einem oder mehreren R^{e} substituiert ist; wobei R^{e} bei jedem Vorkommen unabhängig voneinander C₁₋₆-Alkyl oder Hydroxyl-C₁₋₆-Alkyl ist; und
R¹ 4- bis 20-gliedriges Heterocyclyl ist, gegebenenfalls substituiert mit einem oder mehreren R^{s}, wobei R^{s} bei jedem Vorkommen unabhängig voneinander Halogen, Oxo, Hydroxyl, 5- bis 20-gliedriges Heteroaryl, C₆₋₂₀-Aryl oder C₁₋₆-Alkyl ist;
wobei das C₁₋₆-Alkyl von R^{s} gegebenenfalls mit einem oder mehreren R^{t} substituiert ist;
wobei R^{t} bei jedem Vorkommen unabhängig voneinander C₆₋₂₀-Aryl ist,
wobei das C₆₋₂₀-Alkyl von R^{s} gegebenenfalls mit einem oder mehreren R^{u} substituiert ist; wobei R^{u} bei jedem Vorkommen unabhängig voneinander Halogen oder C₁₋₆-Aryl ist.

6. Verbindung nach Anspruch 1 oder Anspruch 2 oder ein Stereoisomer oder Tautomer davon oder ein pharmazeutisch verträgliches Salz einer der vorgenannten Verbindungen, wobei:
L¹-R¹ Methyl ist;
L² eine Bindung oder C₁₋₁₂-Alkylen ist; und
R² aus der Gruppe ausgewählt ist, bestehend aus

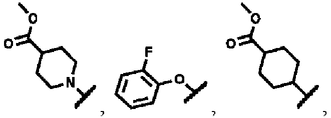

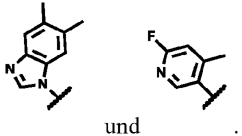

7. Verbindung nach einem der Ansprüche 1, 2 oder 6 oder ein Stereoisomer oder Tautomer davon oder ein pharmazeutisch verträgliches Salz einer der vorgenannten Verbindungen, wobei:
L¹-R¹ Methyl ist;
L² eine Bindung ist; und
R² aus der Gruppe ausgewählt ist, bestehend aus

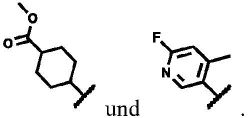

8. Verbindung nach einem der Ansprüche 1, 2 oder 6 oder ein Stereoisomer oder Tautomer davon oder ein pharmazeutisch verträgliches Salz einer der vorgenannten Verbindungen, wobei:
L¹-R¹ Methyl ist;
L² -CH₂ - ist; und
R² aus der Gruppe ausgewählt ist, bestehend aus

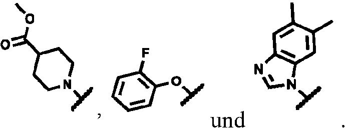

9. Verbindung nach einem der Ansprüche 1 oder 3 oder ein Stereoisomer oder Tautomer davon oder ein pharmazeutisch verträgliches Salz einer der vorgenannten Verbindungen, wobei:
L²-R² Cyclopropyl ist;
L¹ eine Bindung oder C₁₋₁₂-Alkylen ist; wobei das C₁₋₁₂-Alkylen von L¹ gegebenenfalls mit einem oder mehreren R^{e} substituiert ist; wobei R^{e} bei jedem Vorkommen unabhängig voneinander C₁₋₆-Alkyl oder Hydroxyl-C₁₋₆-Alkyl ist; und
R¹ aus der Gruppe ausgewählt ist, bestehend aus

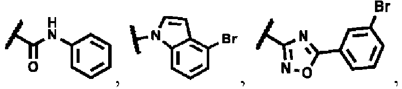

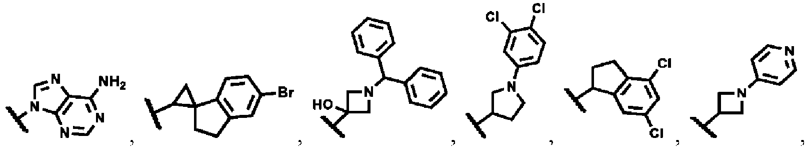

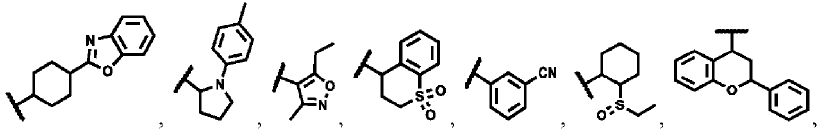

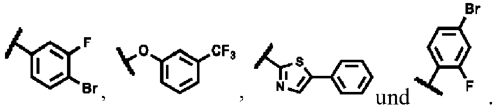

10. Verbindung nach einem der Ansprüche 1, 3 oder 9 oder ein Stereoisomer oder Tautomer davon oder ein pharmazeutisch verträgliches Salz einer der vorgenannten Verbindungen, wobei:
L²-R² Cyclopropyl ist;
L¹ eine Bindung ist; und
R¹ aus der Gruppe ausgewählt ist, bestehend aus

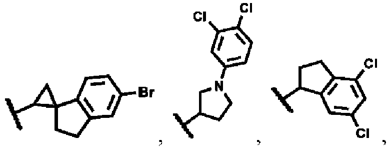

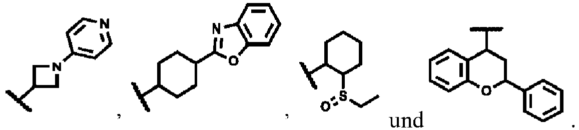

11. Verbindung nach einem der Ansprüche 1, 3 oder 9 oder ein Stereoisomer oder Tautomer davon oder ein pharmazeutisch verträgliches Salz einer der vorgenannten Verbindungen, wobei:
L²-R² Cyclopropyl ist;
L¹ aus der Gruppe ausgewählt ist, bestehend aus -CH₂-, -CH₂CH₂ -, -CH₂CH₂CH₂-, -C(CH₃)₂-, -CH(CH₂OH)-, -CH(CH₂CH₃)-, -CH(CH₃)CH₂- und -CH(CH₂CH₃)CH₂-; und
R¹ aus der Gruppe ausgewählt ist, bestehend aus

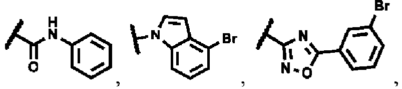

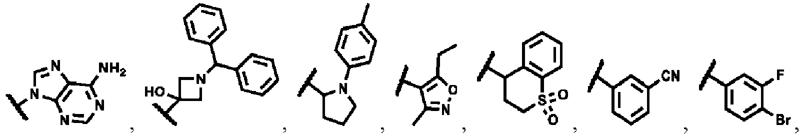

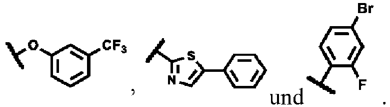

12. Verbindung nach Anspruch 1 oder ein pharmazeutisch verträgliches Salz davon, wobei die Verbindung ausgewählt ist aus der Gruppe, bestehend aus

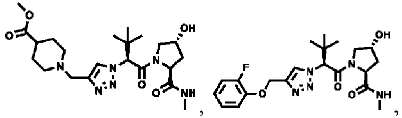

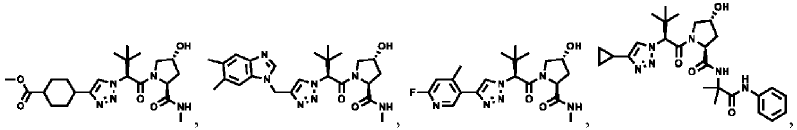

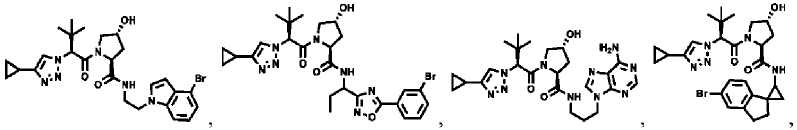

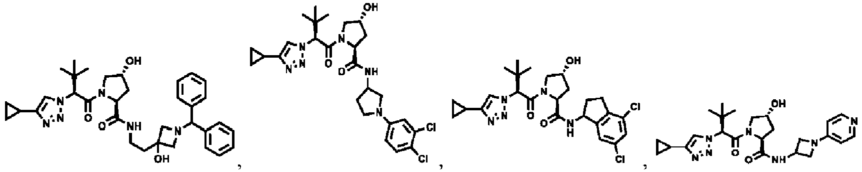

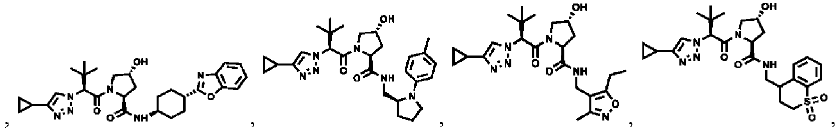

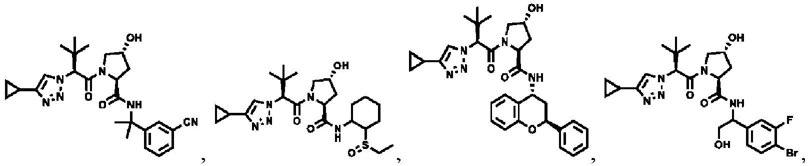

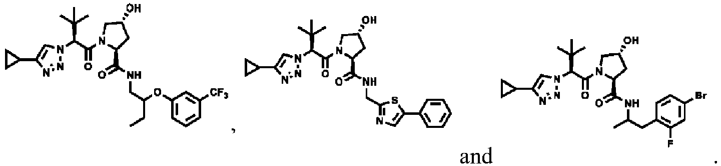

13. Pharmazeutische Zusammensetzung, umfassend eine Verbindung nach einem der Ansprüche 1 bis 12 oder ein Stereoisomer oder Tautomer davon oder ein pharmazeutisch verträgliches Salz einer der vorgenannten Verbindungen sowie einen oder mehrere pharmazeutisch verträgliche Hilfsstoffe.

14. Verbindung nach einem der Ansprüche 1 bis 12 oder ein Stereoisomer oder Tautomer davon oder ein pharmazeutisch verträgliches Salz einer der vorgenannten Verbindungen zur Verwendung bei der Behandlung einer mit der VHL-Aktivität verbundenen Erkrankung oder Indikation.

15. Verbindung der Formel (II): 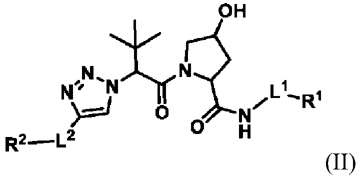 oder ein Stereoisomer oder Tautomer davon oder ein pharmazeutisch verträgliches Salz einer der vorgenannten Verbindungen, wobei:
I) L¹-R¹ Methyl ist;
L² eine Bindung oder C₁₋₁₂-Alkylen ist;
R² Folgendes ist
i) -O-C₆₋₂₀-Aryl, gegebenenfalls substituiert mit einem oder mehreren R^{s}; wobei R^{s} bei jedem Vorkommen unabhängig voneinander Halogen ist;
ii) 4- bis 20-gliedriges Heterocyclyl, gegebenenfalls mit einem oder mehreren R^{s}; wobei R^{s} bei jedem Vorkommen unabhängig voneinander -C(O)O-C₁₋₆-Alkyl ist;
iii) 5- bis 20-gliedriges Heteroaryl, wobei das 5- bis 20-gliedrige Heteroaryl von R² mit einem oder mehreren R^{s} substituiert ist; wobei R^{s} bei jedem Vorkommen unabhängig voneinander Halogen oder C₁₋₁₂-Alkyl ist;
iv) C₆₋₂₀-Cycloalkyl, gegebenenfalls substituiert mit einem oder mehreren R^{s}; wobei R^{s} bei jedem Vorkommen unabhängig voneinander -C(O)O-C₁₋₆-Alkyl ist;
v) -N(CH₃)-C₆₋₂₀-Aryl;
vi) -C₆₋₂₀-Aryl, gegebenenfalls substituiert mit einem oder mehreren R^{s}; wobei R^{s} bei jedem Vorkommen unabhängig voneinander C₁₋₆-Halogenalkyl ist; oder
II) L²-R² Cyclopropyl ist;
L¹ eine Bindung oder C₁₋₁₂-Alkylen ist; wobei das C₁₋₁₂-Alkylen von L¹ gegebenenfalls mit einem oder mehreren R^{e} substituiert ist; wobei R^{e} bei jedem Vorkommen unabhängig voneinander C₁₋₆-Alkyl oder Hydroxyl-C₁₋₆-Alkyl ist;
R¹ Folgendes ist:
i) -L³-C₆₋₂₀-Aryl; wobei L³ -O- oder -C(O)NH- ist; das C₆₋₂₀-Aryl von -L³-C₆₋₂₀-Aryl gegebenenfalls mit einem oder mehreren R^{s} substituiert ist, wobei R^{s} bei jedem Vorkommen unabhängig voneinander Halogen-C₁₋₆-Alkyl ist;
ii) C₄₋₂₀-Cycloalkyl, gegebenenfalls substituiert mit einem oder mehreren R^{s}; wobei R^{s} bei jedem Vorkommen unabhängig voneinander Halogen, 5- bis 20-gliedriges Heteroaryl oder S(O)C₁₋₆-Alkyl ist;
iii) C₆₋₂₀-Aryl, gegebenenfalls substituiert mit einem oder mehreren R^{s}; wobei R^{s} bei jedem Vorkommen unabhängig voneinander Halogen, Cyano oder 5- bis 20-gliedriges Heteroaryl ist;
wobei das 5- bis 20-gliedrige Heteroaryl von R^{s} ein oder mehrere Ringatome aufweist, unabhängig voneinander ausgewählt aus N und O; und wobei das 5- bis 20-gliedrige Heteroaryl von R^{s} gegebenenfalls mit einem oder mehreren R^{t} substituiert ist; wobei R^{t} bei jedem Vorkommen unabhängig voneinander C₁₋₆-Alkyl ist;
iv) 5- bis 20-gliedriges Heteroaryl, gegebenenfalls substituiert mit einem oder mehreren R^{s}, wobei R^{s} bei jedem Vorkommen unabhängig voneinander Halogen, Amin, C₁₋₆-Alkyl oder C₆₋₂₀-Aryl ist;
wobei das C₆₋₂₀-Aryl von R^{s} gegebenenfalls mit einem oder mehreren R^{t} substituiert ist; wobei R^{t} bei jedem Vorkommen unabhängig voneinander Halogen ist; oder
v) 4- bis 20-gliedriges Heterocyclyl, gegebenenfalls substituiert mit einem oder mehreren R^{s}, wobei R^{s} bei jedem Vorkommen unabhängig voneinander Halogen, Oxo, Hydroxyl, 5- bis 20-gliedriges Heteroaryl, C₆₋₂₀-Aryl oder C₁₋₆-Alkyl ist;
wobei das C₁₋₆-Alkyl von R^{s} gegebenenfalls mit einem oder mehreren R^{t} substituiert ist; wobei R^{t} bei jedem Vorkommen unabhängig voneinander C₆₋₂₀-Aryl ist,
wobei das C₆₋₂₀-Aryl von R^{s} gegebenenfalls mit einem oder mehreren R^{u} substituiert ist; wobei R^{u} bei jedem Vorkommen unabhängig voneinander Halogen oder C₁₋₆-Alkyl ist.

## Revendications

1. Composé de formule (I) selon la revendication 15 : 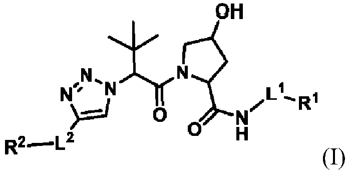 ou un stéréoisomère ou un tautomère de celui-ci, ou un sel pharmaceutiquement acceptable de l'un quelconque des précédents, dans lequel :
I) L¹-R¹ représente un méthyle ;
L² représente une liaison ou un alkylène en C₁₋₁₂ ;
R² représente
i) un -O-aryle en C₆₋₂₀, éventuellement substitué par un ou plusieurs R^{s} ; dans lequel R^{s} représente, indépendamment à chaque occurrence, un halogène ;
ii) un hétérocyclyle à 4 à 20 chaînons, éventuellement substitué par un ou plusieurs R^{s} ; dans lequel R^{s} représente, indépendamment à chaque occurrence, un -C(O)O-alkyle en C₁₋₆ ;
iii) un hétéroaryle à 5 à 20 chaînons, dans lequel l'hétéroaryle à 5 à 20 chaînons de R² est substitué par un ou plusieurs R^{s} ; dans lequel R^{s} représente, indépendamment à chaque occurrence, un halogène ou un alkyle en C₁₋₁₂ ;
iv) un cycloalkyle en C₆₋₂₀, éventuellement substitué par un ou plusieurs R^{s} ; dans lequel R^{s} représente, indépendamment à chaque occurrence, un -C(O)O-alkyle en C₁₋₆ ; ou
II) L²-R² représente un cyclopropyle ;
L¹ représente une liaison ou un alkylène en C₁₋₁₂ ; dans lequel l'alkylène en C₁₋₁₂ de L¹ est éventuellement substitué par un ou plusieurs R^{e} ; dans lequel R^{e} représente, indépendamment à chaque occurrence, un alkyle en C₁₋₆ ou un hydroxylalkyle en C₁₋₆ ;
R¹ représente
i) -L³-aryle en C₆₋₂₀ ; dans lequel L³ représente -O- ou -C(O)NH- ; l'aryle en C₆₋₂₀ de -L³-aryle en C₆₋₂₀ est éventuellement substitué par un ou plusieurs R^{s}, dans lequel R^{s} représente, indépendamment à chaque occurrence, un halogénoalkyle en C₁₋₆ ;
ii) un cycloalkyle en C₄₋₂₀ éventuellement substitué par un ou plusieurs R^{s} ; dans lequel R^{s} représente, indépendamment à chaque occurrence, un halogène, un hétéroaryle à 5 à 20 chaînons ou un S(O)alkyle en C₁₋₆ ;
iii) un aryle en C₆₋₂₀, éventuellement substitué par un ou plusieurs R^{s} ; dans lequel R^{s} représente, indépendamment à chaque occurrence, un halogène, un cyano ou un hétéroaryle à 5 à 20 chaînons ;
dans lequel l'hétéroaryle à 5 à 20 chaînons de R^{s} a un ou plusieurs atomes du cycle, indépendamment choisis parmi N et O ; et dans lequel l'hétéroaryle à 5 à 20 chaînons de R^{s} est éventuellement substitué par un ou plusieurs R^{t} ; dans lequel R^{t} représente, indépendamment à chaque occurrence, un alkyle en C₁₋₆ ;
iv) un hétéroaryle à 5 à 20 chaînons, éventuellement substitué par un ou plusieurs R^{s}, dans lequel R^{s} représente, indépendamment à chaque occurrence, un halogène, une amine, un alkyle en C₁₋₆ ou un aryle en C₆₋₂₀ ;
dans lequel l'aryle en C₆₋₂₀ de R^{s} est éventuellement substitué par un ou plusieurs R^{t} ; dans lequel R^{t} représente, indépendamment à chaque occurrence, un halogène ; ou
v) un hétérocyclyle à 4 à 20 chaînons, éventuellement substitué par un ou plusieurs R^{s}, dans lequel R^{s} représente, indépendamment à chaque occurrence, un halogène, un oxo, un hydroxyle, un hétéroaryle à 5 à 20 chaînons, un aryle en C₆₋₂₀ ou un alkyle en C₁₋₆ ;
dans lequel l'alkyle en C₁₋₆ de R^{s} est éventuellement substitué par un ou plusieurs R^{t} ; dans lequel R^{t} représente, indépendamment à chaque occurrence, un aryle en C₆₋₂₀,
dans lequel l'aryle en C₆₋₂₀ de R^{s} est éventuellement substitué par un ou plusieurs R^{u} ; dans lequel R^{u} représente, indépendamment à chaque occurrence, un halogène ou un alkyle en C₁₋₆.

2. Composé selon la revendication **1,** ou un stéréoisomère ou un tautomère de celui-ci, ou un sel pharmaceutiquement acceptable d'un quelconque des précédents, dans lequel :
L¹-R¹ représente un méthyle ;
L² représente une liaison ou un alkylène en C₁₋₁₂ ; et
R² représente
i) un -O-aryle en C₆₋₂₀, éventuellement substitué par un ou plusieurs R^{s} ; dans lequel R^{s} représente, indépendamment à chaque occurrence, un halogène ;
ii) un hétérocyclyle à 4 à 20 chaînons, éventuellement substitué par un ou plusieurs R^{s} ; dans lequel R^{s} représente, indépendamment à chaque occurrence, un -C(O)O-alkyle en C₁₋₆ ;
iii) un hétéroaryle à 5 à 20 chaînons, dans lequel l'hétéroaryle à 5 à 20 chaînons de R² est substitué par un ou plusieurs R^{s} ; dans lequel R^{s} représente, indépendamment à chaque occurrence, un halogène ou un alkyle en C₁₋₁₂ ;
iv) un cycloalkyle en C₆₋₂₀, éventuellement substitué par un ou plusieurs R^{s} ; dans lequel R^{s} représente, indépendamment à chaque occurrence, un -C(O)O-alkyle en C₁₋₆.

3. Composé selon la revendication 1, ou un stéréoisomère ou un tautomère de celui-ci, ou un sel pharmaceutiquement acceptable d'un quelconque des précédents, dans lequel :
L²-R² représente un cyclopropyle ;
L¹ représente une liaison ou un alkylène en C₁₋₁₂ ; dans lequel l'alkylène en C₁₋₁₂ de L¹ est éventuellement substitué par un ou plusieurs R^{e} ; dans lequel R^{e} représente, indépendamment à chaque occurrence, un alkyle en C₁₋₆ ou un hydroxylalkyle en C₁₋₆ ; et
R¹ représente
i) -L³-aryle en C₆₋₂₀ ; dans lequel L³ représente -O- ou -C(O)NH- ; l'aryle en C₆₋₂₀ de -L³-aryle en C₆₋₂₀ est éventuellement substitué par un ou plusieurs R^{s}, dans lequel R^{s} représente, indépendamment à chaque occurrence, un halogénoalkyle en C₁₋₆ ;
ii) un cycloalkyle en C₄₋₂₀ éventuellement substitué par un ou plusieurs R^{s} ; dans lequel R^{s} représente, indépendamment à chaque occurrence, un halogène, un hétéroaryle à 5 à 20 chaînons ou un S(O)alkyle en C₁₋₆ ;
iii) un aryle en C₆₋₂₀, éventuellement substitué par un ou plusieurs R^{s} ; dans lequel R^{s} représente, indépendamment à chaque occurrence, un halogène, un cyano ou un hétéroaryle à 5 à 20 chaînons ;
dans lequel l'hétéroaryle à 5 à 20 chaînons de R^{s} a un ou plusieurs atomes du cycle, indépendamment choisis parmi N et O ; et dans lequel l'hétéroaryle à 5 à 20 chaînons de R^{s} est éventuellement substitué par un ou plusieurs R^{t} ; dans lequel R^{t} représente, indépendamment à chaque occurrence, un alkyle en C₁₋₆ ;
iv) un hétéroaryle à 5 à 20 chaînons, éventuellement substitué par un ou plusieurs R^{s}, dans lequel R^{s} représente, indépendamment à chaque occurrence, un halogène, une amine, un alkyle en C₁₋₆ ou un aryle en C₆₋₂₀ ;
dans lequel l'aryle en C₆₋₂₀ de R^{s} est éventuellement substitué par un ou plusieurs R^{t} ; dans lequel R^{t} représente, indépendamment à chaque occurrence, un halogène ; ou
v) un hétérocyclyle à 4 à 20 chaînons, éventuellement substitué par un ou plusieurs R^{s}, dans lequel R^{s} représente, indépendamment à chaque occurrence, un halogène, un oxo, un hydroxyle, un hétéroaryle à 5 à 20 chaînons, un aryle en C₆₋₂₀ ou un alkyle en C₁₋₆ ;
dans lequel l'alkyle en C₁₋₆ de R^{s} est éventuellement substitué par un ou plusieurs R^{t} ; dans lequel R^{t} représente, indépendamment à chaque occurrence, un aryle en C₆₋₂₀,
dans lequel l'aryle en C₆₋₂₀ de R^{s} est éventuellement substitué par un ou plusieurs R^{u} ; dans lequel R^{u} représente, indépendamment à chaque occurrence, un halogène ou un alkyle en C₁₋₆.

4. Composé selon l'une quelconque des revendications 1 ou 2, ou un stéréoisomère ou un tautomère de celui-ci, ou un sel pharmaceutiquement acceptable d'un quelconque des précédents, dans lequel :
(a) L¹-R¹ représente un méthyle ;
L² représente une liaison ou un alkylène en C₁₋₁₂ ; et
R² représente un -O-aryle en C₆₋₂₀, éventuellement substitué par un ou plusieurs R^{s} ; dans lequel R^{s} représente, indépendamment à chaque occurrence, un halogène ;
(b) L¹-R¹ représente un méthyle ;
L² représente une liaison ou un alkylène en C₁₋₁₂ ; et
R² représente un hétérocyclyle à 4 à 20 chaînons, éventuellement substitué par un ou plusieurs R^{s} ; dans lequel R^{s} représente, indépendamment à chaque occurrence, un -C(O)O-alkyle en C₁₋₆ ;
(c) L¹-R¹ représente un méthyle ;
L² représente une liaison ou un alkylène en C₁₋₁₂ ; et
R² représente un hétéroaryle à 5 à 20 chaînons, dans lequel l'hétéroaryle à 5 à 20 chaînons de R² est substitué par un ou plusieurs R^{s} ; dans lequel R^{s} représente, indépendamment à chaque occurrence, un halogène ou un alkyle en C₁₋₁₂ ;
ou
(d) L¹-R¹ représente un méthyle ;
L² représente une liaison ou un alkylène en C₁₋₁₂ ; et
R² représente un cycloalkyle en C₆₋₂₀, éventuellement substitué par un ou plusieurs R^{s} ; dans lequel R^{s} représente, indépendamment à chaque occurrence, un -C(O)O-alkyle en C₁₋₆.

5. Composé selon l'une quelconque des revendications 1 ou 3, ou un stéréoisomère ou un tautomère de celui-ci, ou un sel pharmaceutiquement acceptable d'un quelconque des précédents, dans lequel :
(a) L²-R² représente un cyclopropyle ;
L¹ représente une liaison ou un alkylène en C₁₋₁₂ ; dans lequel l'alkylène en C₁₋₁₂ de L¹ est éventuellement substitué par un ou plusieurs R^{e} ; dans lequel R^{e} représente, indépendamment à chaque occurrence, un alkyle en C₁₋₆ ou un hydroxylalkyle en C₁₋₆ ; et
R¹ représente -L³-aryle en C₆₋₂₀ ; dans lequel L³ représente -O- ou -C(O)NH- ; l'aryle en C₆₋₂₀ de -L³-aryle en C₆₋₂₀ est éventuellement substitué par un ou plusieurs R^{s}, dans lequel R^{s} représente, indépendamment à chaque occurrence, un halogénoalkyle en C₁₋₆ ;
(b) L²-R² représente un cyclopropyle ;
L¹ représente une liaison ou un alkylène en C₁₋₁₂ ; dans lequel l'alkylène en C₁₋₁₂ de L¹ est éventuellement substitué par un ou plusieurs R^{e} ; dans lequel R^{e} représente, indépendamment à chaque occurrence, un alkyle en C₁₋₆ ou un hydroxylalkyle en C₁₋₄ ; et
dans lequel R¹ représente un cycloalkyle en C₄₋₂₀ éventuellement substitué par un ou plusieurs R^{s} ; dans lequel R^{s} représente, indépendamment à chaque occurrence, un halogène, un hétéroaryle à 5 à 20 chaînons ou un S(O)alkyle en C₁₋₆ ;
(c) L²-R² représente un cyclopropyle ;
L¹ représente une liaison ou un alkylène en C₁₋₁₂ ; dans lequel l'alkylène en C₁₋₁₂ de L¹ est éventuellement substitué par un ou plusieurs R^{e} ; dans lequel R^{e} représente, indépendamment à chaque occurrence, un alkyle en C₁₋₆ ou un hydroxylalkyle en C₁₋₆ ; et
R¹ représente un aryle en C₆₋₂₀, éventuellement substitué par un ou plusieurs R^{s} ; dans lequel R^{s} représente, indépendamment à chaque occurrence, un halogène, un cyano ou un hétéroaryle à 5 à 20 chaînons ;
dans lequel l'hétéroaryle à 5 à 20 chaînons de R^{s} a un ou plusieurs atomes du cycle, indépendamment choisis parmi N et O ; et dans lequel l'hétéroaryle à 5 à 20 chaînons de R^{s} est éventuellement substitué par un ou plusieurs R^{t} ; dans lequel R^{t} représente, indépendamment à chaque occurrence, un alkyle en C₁₋₆ ;
(d) L²-R² représente un cyclopropyle ;
L¹ représente une liaison ou un alkylène en C₁₋₁₂ ; dans lequel l'alkylène en C₁₋₁₂ de L¹ est éventuellement substitué par un ou plusieurs R^{e} ; dans lequel R^{e} représente, indépendamment à chaque occurrence, un alkyle en C₁₋₆ ou un hydroxylalkyle en C₁₋₆ ; et
R¹ représente un hétéroaryle à 5 à 20 chaînons, éventuellement substitué par un ou plusieurs R^{s}, dans lequel R^{s} représente, indépendamment à chaque occurrence, un halogène, une amine, un alkyle en C₁₋₆ ou un aryle en C₆₋₂₀ ;
dans lequel l'aryle en C₆₋₂₀ de R^{s} est éventuellement substitué par un ou plusieurs R^{t} ; dans lequel R^{t} représente, indépendamment à chaque occurrence, un halogène ;
ou
(e) L²-R² représente un cyclopropyle ;
L¹ représente une liaison ou un alkylène en C₁₋₁₂ ; dans lequel l'alkylène en C₁₋₁₂ de L¹ est éventuellement substitué par un ou plusieurs R^{e} ; dans lequel R^{e} représente, indépendamment à chaque occurrence, un alkyle en C₁₋₆ ou un hydroxylalkyle en C₁₋₆ ; et
R¹ représente un hétérocyclyle à 4 à 20 chaînons, éventuellement substitué par un ou plusieurs R^{s}, dans lequel R^{s} représente, indépendamment à chaque occurrence, un halogène, un oxo, un hydroxyle, un hétéroaryle à 5 à 20 chaînons, un aryle en C₆₋₂₀ ou un alkyle en C₁₋₆ ;
dans lequel l'alkyle en C₁₋₆ de R^{s} est éventuellement substitué par un ou plusieurs R^{t} ;
dans lequel R^{t} représente, indépendamment à chaque occurrence, un aryle en C₆₋₂₀, dans lequel l'alkyle en C₆₋₂₀ de R^{s} est éventuellement substitué par un ou plusieurs R^{u} ; dans lequel R^{u} représente, indépendamment à chaque occurrence, un halogène ou un aryle en C₁₋₆.

6. Composé selon la revendication 1 ou la revendication 2, ou un stéréoisomère ou un tautomère de celui-ci, ou un sel pharmaceutiquement acceptable d'un quelconque des précédents, dans lequel :
L¹-R¹ représente un méthyle ;
L² représente une liaison ou un alkylène en C₁₋₁₂ ; et
R² est choisi dans le groupe constitué par

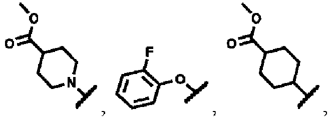

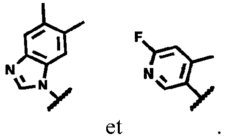

7. Composé selon l'une quelconque des revendications 1, 2 ou 6, ou un stéréoisomère ou un tautomère de celui-ci, ou un sel pharmaceutiquement acceptable d'un quelconque des précédents, dans lequel :
L¹-R¹ représente un méthyle ;
L² représente une liaison ; et
R² est choisi dans le groupe constitué par

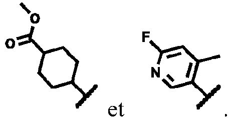

8. Composé selon l'une quelconque des revendications 1, 2 ou 6, ou un stéréoisomère ou un tautomère de celui-ci, ou un sel pharmaceutiquement acceptable d'un quelconque des précédents, dans lequel :
L¹-R¹ représente un méthyle ;
L² représente -CH₂- ; et
R² est choisi dans le groupe constitué par

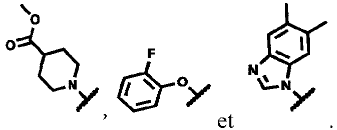

9. Composé selon l'une quelconque des revendications 1 ou 3, ou un stéréoisomère ou un tautomère de celui-ci, ou un sel pharmaceutiquement acceptable d'un quelconque des précédents, dans lequel :
L²-R² représente un cyclopropyle ;
L¹ représente une liaison ou un alkylène en C₁₋₁₂ ; dans lequel l'alkylène en C₁₋₁₂ de L¹ est éventuellement substitué par un ou plusieurs R^{e} ; dans lequel R^{e} représente, indépendamment à chaque occurrence, un alkyle en C₁₋₆ ou un hydroxylalkyle en C₁₋₆ ; et
R¹ est choisi dans le groupe constitué par

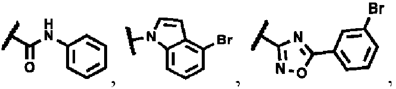

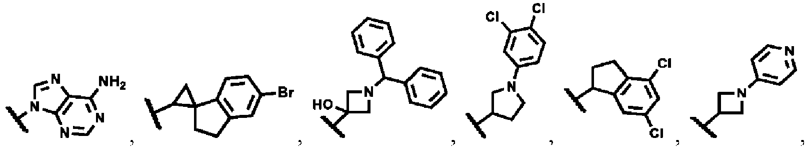

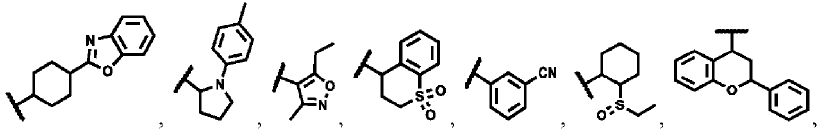

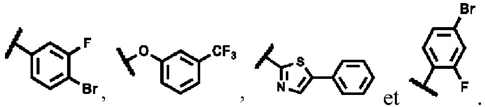

10. Composé selon l'une quelconque des revendications 1, 3 ou 9, ou un stéréoisomère ou un tautomère de celui-ci, ou un sel pharmaceutiquement acceptable d'un quelconque des précédents, dans lequel :
L²-R² représente un cyclopropyle ;
L¹ représente une liaison ; et
R¹ est choisi dans le groupe constitué par

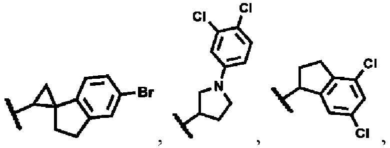

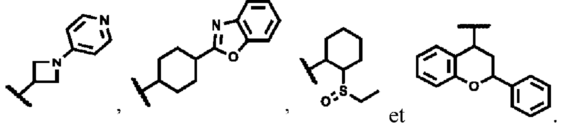

11. Composé selon l'une quelconque des revendications 1, 3 ou 9, ou un stéréoisomère ou un tautomère de celui-ci, ou un sel pharmaceutiquement acceptable d'un quelconque des précédents, dans lequel :
L²-R² représente un cyclopropyle ;
L¹ est choisi dans le groupe constitué par -CH₂-, -CH₂CH₂-, -CH₂CH₂CH₂-, -C(CH₃)₂-, -CH(CH₂OH)-, -CH(CH₂CH₃)-, -CH(CH₃)CH₂- et -CH(CH₂CH₃)CH₂- ; et
R¹ est choisi dans le groupe constitué par

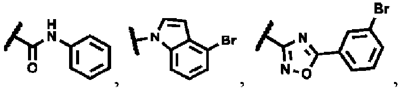

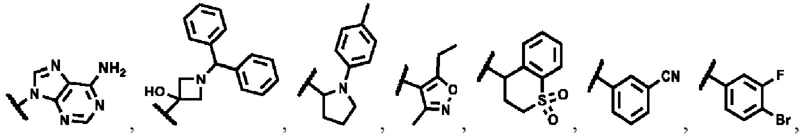

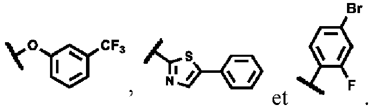

12. Composé selon la revendication 1, ou un sel pharmaceutiquement acceptable de celui-ci, dans lequel le composé est choisi dans le groupe constitué par

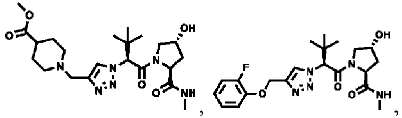

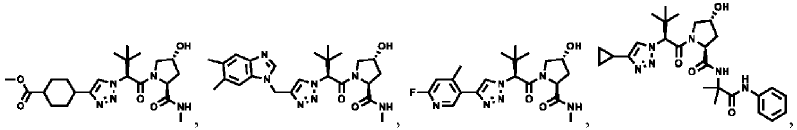

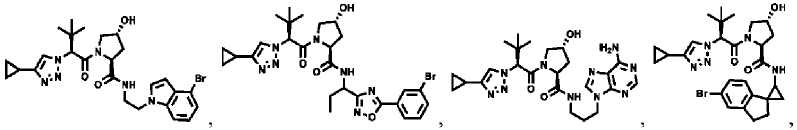

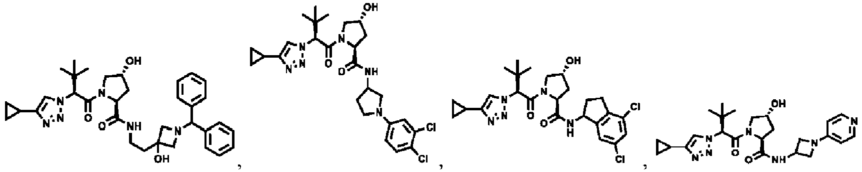

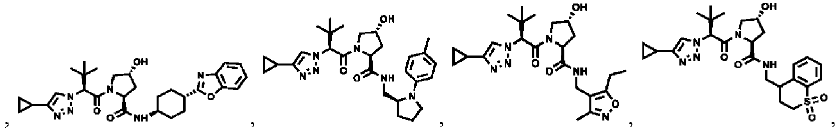

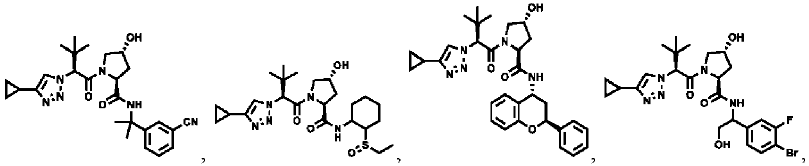

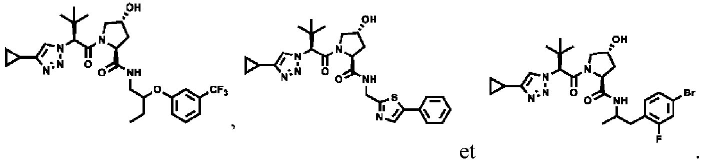

13. Composition pharmaceutique comprenant un composé selon l'une quelconque des revendications 1 à 12, ou un stéréoisomère ou un tautomère de celui-ci, ou un sel pharmaceutiquement acceptable d'un quelconque des précédents, et un ou plusieurs excipients pharmaceutiquement acceptables.

14. Composé selon l'une quelconque des revendications 1 à 12, ou un stéréoisomère ou un tautomère de celui-ci, ou un sel pharmaceutiquement acceptable d'un quelconque des précédents, pour une utilisation dans le traitement d'une maladie ou d'une indication associée à l'activité de VHL.

15. Composé de formule (II) : 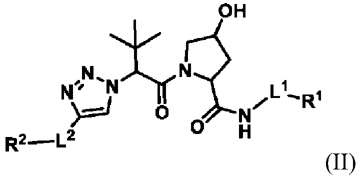 ou un stéréoisomère ou un tautomère de celui-ci, ou un sel pharmaceutiquement acceptable de l'un quelconque des précédents, dans lequel :
I) L¹-R¹ représente un méthyle ;
L² représente une liaison ou un alkylène en C₁₋₁₂ ;
R² représente
i) un -O-aryle en C₆₋₂₀, éventuellement substitué par un ou plusieurs R^{s} ; dans lequel R^{s} représente, indépendamment à chaque occurrence, un halogène ;
ii) un hétérocyclyle à 4 à 20 chaînons, éventuellement substitué par un ou plusieurs R^{s} ; dans lequel R^{s} représente, indépendamment à chaque occurrence, un -C(O)O-alkyle en C₁₋₆ ;
iii) un hétéroaryle à 5 à 20 chaînons, dans lequel l'hétéroaryle à 5 à 20 chaînons de R² est substitué par un ou plusieurs R^{s} ; dans lequel R^{s} représente, indépendamment à chaque occurrence, un halogène ou un alkyle en C₁₋₁₂ ;
iv) un cycloalkyle en C₆₋₂₀, éventuellement substitué par un ou plusieurs R^{s} ; dans lequel R^{s} représente, indépendamment à chaque occurrence, un -C(O)O-alkyle en C₁₋₆ ;
v) un -N(CH₃)-aryle en C₆₋₂₀ ;
vi) un -aryle en C₆₋₂₀, éventuellement substitué par un ou plusieurs R^{s} ; dans lequel R^{s} représente, indépendamment à chaque occurrence, un halogénoalkyle en C₁₋₆ ; ou
II) L²-R² représente un cyclopropyle ;
L¹ représente une liaison ou un alkylène en C₁₋₁₂ ; dans lequel l'alkylène en C₁₋₁₂ de L¹ est éventuellement substitué par un ou plusieurs R^{e} ; dans lequel R^{e} représente, indépendamment à chaque occurrence, un alkyle en C₁₋₆ ou un hydroxylalkyle en C₁₋₆ ;
R¹ représente
i) -L³-aryle en C₆₋₂₀ ; dans lequel L³ représente -O- ou -C(O)NH- ; l'aryle en C₆₋₂₀ de -L³-aryle en C₆₋₂₀ est éventuellement substitué par un ou plusieurs R^{s}, dans lequel R^{s} représente, indépendamment à chaque occurrence, un halogénoalkyle en C₁₋₆ ;
ii) un cycloalkyle en C₄₋₂₀ éventuellement substitué par un ou plusieurs R^{s} ; dans lequel R^{s} représente, indépendamment à chaque occurrence, un halogène, un hétéroaryle à 5 à 20 chaînons ou un S(O)alkyle en C₁₋₆ ;
iii) un aryle en C₆₋₂₀, éventuellement substitué par un ou plusieurs R^{s} ; dans lequel R^{s} représente, indépendamment à chaque occurrence, un halogène, un cyano ou un hétéroaryle à 5 à 20 chaînons ;
dans lequel l'hétéroaryle à 5 à 20 chaînons de R^{s} a un ou plusieurs atomes du cycle, indépendamment choisis parmi N et O ; et dans lequel l'hétéroaryle à 5 à 20 chaînons de R^{s} est éventuellement substitué par un ou plusieurs R^{t} ; dans lequel R^{t} représente, indépendamment à chaque occurrence, un alkyle en C₁₋₆ ;
iv) un hétéroaryle à 5 à 20 chaînons, éventuellement substitué par un ou plusieurs R^{s}, dans lequel R^{s} représente, indépendamment à chaque occurrence, un halogène, une amine, un alkyle en C₁₋₆ ou un aryle en C₆₋₂₀ ;
dans lequel l'aryle en C₆₋₂₀ de R^{s} est éventuellement substitué par un ou plusieurs R^{t} ; dans lequel R^{t} représente, indépendamment à chaque occurrence, un halogène ; ou
v) un hétérocyclyle à 4 à 20 chaînons, éventuellement substitué par un ou plusieurs R^{s}, dans lequel R^{s} représente, indépendamment à chaque occurrence, un halogène, un oxo, un hydroxyle, un hétéroaryle à 5 à 20 chaînons, un aryle en C₆₋₂₀ ou un alkyle en C₁₋₆ ;
dans lequel l'alkyle en C₁₋₆ de R^{s} est éventuellement substitué par un ou plusieurs R^{t} ; dans lequel R^{t} représente, indépendamment à chaque occurrence, un aryle en C₆₋₂₀,
dans lequel l'aryle en C₆₋₂₀ de R^{s} est éventuellement substitué par un ou plusieurs R^{u} ; dans lequel R^{u} représente, indépendamment à chaque occurrence, un halogène ou un alkyle en C₁₋₆.
